# EUROPEAN PATENT APPLICATION

(11) **EP 1 661 998 A2**
(43) Date of publication of application: **31.05.2006**
(21) Application number: 06002417.1
(22) Date of filing: 04.06.2002
(51) Int. Cl.: C12N 15/12, C07K 14/47, C07K 16/18, A61K 38/17, G01N 33/68, C12Q 1/68, G01N 33/50

(54) **Antigenic polypeptides, antibodies binding thereto, nucleic acids encoding the antigens, and methods of use**

(30) Priority: 04.06.2001 US 295607 P; 06.06.2001 US 296418 P; 06.06.2001 US 296404 P; 07.06.2001 US 296575 P; 11.06.2001 US 297414 P; 12.06.2001 US 297573 P; 12.06.2001 US 297567 P; 14.06.2001 US 298285 P; 15.06.2001 US 298556 P; 15.06.2001 US 298528 P; 18.06.2001 US 299133 P; 19.06.2001 US 299230 P; 21.06.2001 US 299949 P; 22.06.2001 US 300177 P; 28.06.2001 US 301550 P; 28.06.2001 US 301530 P; 03.07.2001 US 302951 P; 12.09.2001 US 318771 P; 25.09.2001 US 324687 P; 24.10.2001 US 339266 P; 16.11.2001 US 337524 P; 14.12.2001 US 341143 P; 21.02.2002 US 359151 P; 21.02.2002 US 358643 P; 28.02.2002 US 361195 P; 05.03.2002 US 361964 P; 10.04.2002 US 371523 P; 10.04.2002 US 371346 P; 03.06.2002 US 161493 P
(62) Divisional of application: 02732027.4
(71) Applicant: Curagen Corporation, New Haven, CT 06511 (US)
(72) Inventor: Anderson, David W., Branford, CT 06405 (US); Zerhusen, Bryan D., Branford, CT 06405 (US); Li, Li, Branford, CT 06405 (US); Zhong, Mei, Branford, CT 06405 (US); Casman, Stacie J., North Haven, CT 06473 (US); Gerlach, Valerie L., Branford, CT 06405 (US); Shimkets, Richard A., Guildford, CT 06437 (US); Gorman, Linda, Brantford, CT 06405 (US); Pena, Carol E.A., New Haven, CT 06511 (US); Kekuda, Ramesh, Danbury, CT 06810 (US); Patturajan, Meera, Branford, CT 06405 (US); Spytek, Kimberly A., New Haven, CT 06511 (US); Leite, Mario W., Milford, CT 06460 (US); Rastelli, Luca, Guildford, CT 06437 (US); Macdougall, John R., Hamden, CT 06517 (US); Taupier, Raymond J., Jr., East Haven, CT 06512 (US); Guo, Xiaojia, Branford, CT 06405 (US); Miller, Charles E., Guildford, CT 06437 (US); Shenoy, Suresh G., Branford, CT 06405 (US); Hjalt, Tord, East Haven, CT 06512 (US); Voss, Edward Z., Wallingford, CT 06492 (US); Boldog, Ferenc L., North Haven, CT 06473 (US); Malyankar, Uriel M., Branford, CT 06405 (US); Padigaru, Muralidhara, Malad, Mumbai, 400-064 (IN); Ji, Weizhen, Branford, CT 06405 (US); Smithson, Glennda, Guildford, CT 06435 (US); Edinger, Shlomit R., New Haven, CT 06515 (US); Millet, Isabelle, Milford, CT 06460 (US); Ellerman, Karen, Branford, CT 06405 (US)
(74) Representative: Naylor, Kathryn May

(57) **Abstract**

Disclosed herein are nucleic acid sequences that encode polypeptides. Also disclosed are antibodies, which immunospecifically-bind to the polypeptide, as well as derivatives, variants, mutants, or fragments of the aforementioned polypeptide, polynucleotide, or antibody. The invention further discloses therapeutic, diagnostic and research methods for diagnosis, treatment, and prevention of disorders involving any one of these novel human nucleic acids, polypeptides, or antibodies, or fragments thereof.

SEQUENCE LISTING

<110> Curagen Corporation
      Anderson, David W
      Zerhusen, Bryan D
      Li, Li
      Zhong, Mei
      Casman, Stacie J
      Gerlach, Valerie
      Shimkets, Richard A
      Gorman, Linda
      Pena, Carol EA
      Kekuda, Ramesh
      Patturajan, Meera
      Spytek, Kimberly A
      Leite, Mario W
      Rastelli, Luca
      MacDougall, John R
      Taupier Jr., Raymond J
      Guo, Xiaojia Sasha
      Miller, Charles E
      Shenoy, Suresh G
      Hjalt, Tord
      Voss, Edward Z
      Boldog, Ferenc L
      Malyankar, Uriel M
      Padigaru, Muralidhara
      Ji, Weizhen
      Smithson, Glennda
      Edinger, Shlomit R
      Millet, Isabelle
      Ellerman, Karen

<120> Novel Antibodies that Bind to Antigenic Polypeptides, Nucleic
      Acids Encoding the Antigens, and Methods of Use

<130> 21402-377A-019

<140> EP 02732027.4
<141> 2002-06-04

<150> PCT/US 02/17559
<151> 2002-06-04

<150> 10/161,493
<151> 2002-06-03

<150> 60/295,607
<151> 2001-06-04

<150> 60/337,524
<151> 2001-11-16

<150> 60/296,404
<151> 2001-06-06

<150> 60/296,418

<151> 2001-06-06

<150> 60/296,575
<151> 2001-06-07

<150> 60/359,151
<151> 2002-02-21

<150> 60/297,414
<151> 2001-06-11

<150> 60/297,573
<151> 2001-06-12

<150> 60/341,143
<151> 2001-12-14

<150> 60/297,567
<151> 2001-06-12

<150> 60/318,771
<151> 2001-09-12

<150> 60/298,285
<151> 2001-06-14

<150> 60/298,528
<151> 2001-06-15

<150> 60/298,556
<151> 2001-06-15

<150> 60/299,133
<151> 2001-06-18

<150> 60/299,230
<151> 2001-06-19

<150> 60/358,643
<151> 2002-02-21

<150> 60/299,949
<151> 2001-06-21

<150> 60/300,177
<151> 2001-06-22

<150> 60/301,550
<151> 2001-06-28

<150> 60/301,530
<151> 2001-06-28

<150> 60/302,951
<151> 2001-07-03

<150> 60/324,687

<151> 2001-09-25

<150> 60/339,266
<151> 2001-10-24

<150> 60/361,195
<151> 2002-02-28

<150> 60/361,964
<151> 2002-03-05

<150> 60/371,523
<151> 2002-04-10

<150> 60/371,346
<151> 2002-04-10

<160> 299

<210>1
<211>3430
<212>DNA
<213>Homo sapiens

<220>
<221> CDS
<222>(31)..(2611)

<400>1

gggctgcagg aattccccca cagagggagc atg act tcg gca act tca cct 51
Met Thr Ser Ala Thr Ser Pro
1               5

atc att ctg aaa tgg gac ccc aaa agt ttg gaa atc cgg acg cta aca 99
Ile Ile Leu Lys Trp Asp Pro Lys Ser Leu Glu Ile Arg Thr Leu Thr
        10                  15                  20

gtg gaa agg ctg ttg gag cca ctt gtt aca cag gtg act aca ctt gtc 147
Val Glu Arg Leu Leu Glu Pro Leu Val Thr Gln Val Thr Thr Leu Val
    25                  30                  35

aac aca agc aac aaa ggc cca tct ggt aaa aag aaa ggg agg tca aag 195
Asn Thr Ser Asn Lys Gly Pro Ser Gly Lys Lys Lys Gly Arg Ser Lys
40                  45                  50                  55

aaa gcc cat gta cta gct gcc tct gta gag caa gcc act cag aat ttc 243
Lys Ala His Val Leu Ala Ala Ser Val Glu Gln Ala Thr Gln Asn Phe
                60                  65                  70

ctg gaa aag ggt gaa cag atc gct aag gag agt caa gat ctc aaa gaa 291
Leu Glu Lys Gly Glu Gln Ile Ala Lys Glu Ser Gln Asp Leu Lys Glu
            75                  80                  85

gag ttg gtg gct gct gta gag gat gtg cgc aaa caa ggt gag acg atg 339
Glu Leu Val Ala Ala Val Glu Asp Val Arg Lys Gln Gly Glu Thr Met
        90                  95                  100

cgg atc gcc tcc tcc gag ttt gca gat gac cct tgc tcg tcg gta aag 387
Arg Ile Ala Ser Ser Glu Phe Ala Asp Asp Pro Cys Ser Ser Val Lys
    105                 110                 115

cgc ggc acc atg gta cgg gcg gca agg gct ttg ctc tcc gcg gtg aca 435
Arg Gly Thr Met Val Arg Ala Ala Arg Ala Leu Leu Ser Ala Val Thr
120                 125                 130                 135

cgc tta ctc atc ctg gcg gac atg gca gat gtc atg aga ctt tta tcc 483
Arg Leu Leu Ile Leu Ala Asp Met Ala Asp Val Met Arg Leu Leu Ser
                140                 145                 150

cat ctg aaa att gtg gaa gag gcc ctg gaa gct gtc aaa aat gct aca 531
His Leu Lys Ile Val Glu Glu Ala Leu Glu Ala Val Lys Asn Ala Thr
            155                 160                 165

aat gag caa gac ctt gca aac cgt ttt aaa gag ttt ggg aaa aag atg 579
Asn Glu Gln Asp Leu Ala Asn Arg Phe Lys Glu Phe Gly Lys Lys Met
        170                 175                 180

gtg aaa ctt aac tat gta gca gca aga aga caa cag gag ctg aag gat 627
Val Lys Leu Asn Tyr Val Ala Ala Arg Arg Gln Gln Glu Leu Lys Asp
    185                 190                 195

cct cac tgt cgg gat gag atg gca gcc gcc cga ggg gct ctg aag aag 675
Pro His Cys Arg Asp Glu Met Ala Ala Ala Arg Gly Ala Leu Lys Lys
200                 205                 210                 215

aat gcc aca atg ctg tac acg gcc tct caa gca ttt ctc cgc cac cca 723
Asn Ala Thr Met Leu Tyr Thr Ala Ser Gln Ala Phe Leu Arg His Pro
                220                 225                 230

gat gtc gcc gct acg aga gcc aac cga gat tat gtg ttc aaa caa gtc 771
Asp Val Ala Ala Thr Arg Ala Asn Arg Asp Tyr Val Phe Lys Gln Val
            235                 240                 245

cag gag gcc atc gcc ggc atc tcc aat gct gct caa gct acc tcg ccc 819
Gln Glu Ala Ile Ala Gly Ile Ser Asn Ala Ala Gln Ala Thr Ser Pro
        250                 255                 260

act gac gaa gcc aag ggc cac acg ggc atc ggc gag ctg gct gcg gct 867
Thr Asp Glu Ala Lys Gly His Thr Gly Ile Gly Glu Leu Ala Ala Ala
    265                 270                 275

ctt aat gag ttt gac aat aag att atc ctg gac ccc atg acg ttc agc 915
Leu Asn Glu Phe Asp Asn Lys Ile Ile Leu Asp Pro Met Thr Phe Ser
280                 285                 290                 295

gag gcc agg ttc cgg ccg tcc ctg gag gag agg ctg gag agc atc atc 963
Glu Ala Arg Phe Arg Pro Ser Leu Glu Glu Arg Leu Glu Ser Ile Ile
                300                 305                 310

agc ggc gca gcg ctg atg gcc gac tcc tcc tgc acg cga gac gac cgg 1011
Ser Gly Ala Ala Leu Met Ala Asp Ser Ser Cys Thr Arg Asp Asp Arg
            315                 320                 325

cgc gag agg atc gtg gcg gag tgc aac gcc gtg cgg cag gcg ctc cag 1059
Arg Glu Arg Ile Val Ala Glu Cys Asn Ala Val Arg Gln Ala Leu Gln
        330                 335                 340

gac ctg ctc agc gag tac atg aat aat act gga agg aaa gaa aaa gga 1107
Asp Leu Leu Ser Glu Tyr Met Asn Asn Thr Gly Arg Lys Glu Lys Gly
    345                 350                 355

gat cct ctc aac att gcg att gat aag atg act aag aaa aca aga gat 1155
Asp Pro Leu Asn Ile Ala Ile Asp Lys Met Thr Lys Lys Thr Arg Asp
360                 365                 370                 375

cta agg aga cag ctt cgg aaa gca gtg atg gat cac ata tct gac tct 1203
Leu Arg Arg Gln Leu Arg Lys Ala Val Met Asp His Ile Ser Asp Ser
                380                 385                 390

ttc ctg gaa acc aat gtt cct ttg cta gtt ctc att gag gct gca aag 1251
Phe Leu Glu Thr Asn Val Pro Leu Leu Val Leu Ile Glu Ala Ala Lys
            395                 400                 405

agc gga aat gaa aag gaa gtg aaa gaa tat gcc caa gtt ttc cgt gag 1299
Ser Gly Asn Glu Lys Glu Val Lys Glu Tyr Ala Gln Val Phe Arg Glu
        410                 415                 420

cat gcc aac aaa ctg gta gag gtt gcc aat ttg gcc tgt tcc atc tcc 1347
His Ala Asn Lys Leu Val Glu Val Ala Asn Leu Ala Cys Ser Ile Ser
    425                 430                 435

aac aat gaa gaa ggg gtg aaa tta gtt cgg atg gca gcc acc cag att 1395
Asn Asn Glu Glu Gly Val Lys Leu Val Arg Met Ala Ala Thr Gln Ile
440                 445                 450                 455

gac agc ctg tgt ccc cag gtc atc aat gcc gct ctg aca ctg gct gcc 1443
Asp Ser Leu Cys Pro Gln Val Ile Asn Ala Ala Leu Thr Leu Ala Ala
                460                 465                 470

cgg cca cag agc aaa gtt gct cag gat aac atg gac gtc ttc aaa gac 1491
Arg Pro Gln Ser Lys Val Ala Gln Asp Asn Met Asp Val Phe Lys Asp
            475                 480                 485

cag tgg gag aag cag gtc cga gtg ttg aca gag gcc gtg gat gac atc 1539
Gln Trp Glu Lys Gln Val Arg Val Leu Thr Glu Ala Val Asp Asp Ile
        490                 495                 500

acc tca gtg gat gac ttc ctc tct gtc tca gaa aat cac atc ttg gag 1587
Thr Ser Val Asp Asp Phe Leu Ser Val Ser Glu Asn His Ile Leu Glu
    505                 510                 515

gat gtg aac aag tgt gtg ata gcc ctc caa gag ggc gat gtg gac act 1635
Asp Val Asn Lys Cys Val Ile Ala Leu Gln Glu Gly Asp Val Asp Thr
520                 525                 530                 535

ctg gac cgg act gca ggg gcc atc agg ggc cgg gca gct cga gtc ata 1683
Leu Asp Arg Thr Ala Gly Ala Ile Arg Gly Arg Ala Ala Arg Val Ile
                540                 545                 550

cac atc atc aat gct gag atg gag aac tat gaa gct ggg gtt tat act 1731

His Ile Ile Asn Ala Glu Met Glu Asn Tyr Glu Ala Gly Val Tyr Thr
            555                 560                 565

gag aag gtg ttg gaa gct aca aaa ttg ctt tct gaa aca gtg atg cca 1779
Glu Lys Val Leu Glu Ala Thr Lys Leu Leu Ser Glu Thr Val Met Pro
        570                 575                 580

cgc ttc gct gaa caa gta gag gtt gcc att gaa gcc ctg agt gcc aac 1827
Arg Phe Ala Glu Gln Val Glu Val Ala Ile Glu Ala Leu Ser Ala Asn
    585                 590                 595

gtt cct caa ccg ttt gag gag aat gag ttc atc gat gcc tct cgc ctg 1875
Val Pro Gln Pro Phe Glu Glu Asn Glu Phe Ile Asp Ala Ser Arg Leu
600                 605                 610                 615

gtg tat gat ggc gtt cgg gac atc aga aag gct gtg ctg atg atc agg 1923
Val Tyr Asp Gly Val Arg Asp Ile Arg Lys Ala Val Leu Met Ile Arg
                620                 625                 630

acc cca gaa gaa cta gag gat gat tct gac ttt gag cag gaa gat tat 1971
Thr Pro Glu Glu Leu Glu Asp Asp Ser Asp Phe Glu Gln Glu Asp Tyr
            635                 640                 645

gat gtg cgt aga ggg aca agt gtt cag act gag gat gac cag ctc att 2019
Asp Val Arg Arg Gly Thr Ser Val Gln Thr Glu Asp Asp Gln Leu Ile
        650                 655                 660

gca ggg cag agc gca cgg gcc atc atg gcg caa cta ccg cag gag gag 2067
Ala Gly Gln Ser Ala Arg Ala Ile Met Ala Gln Leu Pro Gln Glu Glu
    665                 670                 675

aag gca aaa ata gct gag cag gtg gag ata ttc cat caa gag aaa agc 2115
Lys Ala Lys Ile Ala Glu Gln Val Glu Ile Phe His Gln Glu Lys Ser
680                 685                 690                 695

aag ctg gat gca gaa gtg gcc aaa tgg gac gac agc ggc aat gat atc 2163
Lys Leu Asp Ala Glu Val Ala Lys Trp Asp Asp Ser Gly Asn Asp Ile
                700                 705                 710

att gta ctg gcc aag cag atg tgt atg atc atg atg gaa atg aca gac 2211
Ile Val Leu Ala Lys Gln Met Cys Met Ile Met Met Glu Met Thr Asp
            715                 720                 725

ttc aca aga ggc aaa ggc cca ttg aaa aat aca tct gat gtc att aat 2259
Phe Thr Arg Gly Lys Gly Pro Leu Lys Asn Thr Ser Asp Val Ile Asn
        730                 735                 740

gct gcc aag aaa att gcc gaa gca ggt tct cga atg gac aaa tta gct 2307
Ala Ala Lys Lys Ile Ala Glu Ala Gly Ser Arg Met Asp Lys Leu Ala
    745                 750                 755

cgt gct gtg gct gat cag ctg gac agt gcc aca tcg ctt atc cag gca 2355
Arg Ala Val Ala Asp Gln Leu Asp Ser Ala Thr Ser Leu Ile Gln Ala
760                 765                 770                 775

gct aaa aac ctg atg aat gct gtt gtc ctc acg gtg aaa gca tcc tat 2403
Ala Lys Asn Leu Met Asn Ala Val Val Leu Thr Val Lys Ala Ser Tyr

                780                 785                 790

gtg gcc tca acc aaa tac cag aag gtc tat ggg aca gca gct gtc aac 2451
Val Ala Ser Thr Lys Tyr Gln Lys Val Tyr Gly Thr Ala Ala Val Asn
            795                 800                 805

tca cct gtt gtg tct tgg aag atg aag gct cca gag aag aag ccc ctt 2499
Ser Pro Val Val Ser Trp Lys Met Lys Ala Pro Glu Lys Lys Pro Leu
        810                 815                 820

gtg aag aga gaa aag cct gaa gaa ttc cag aca cga gtt cga cga ggt 2547
Val Lys Arg Glu Lys Pro Glu Glu Phe Gln Thr Arg Val Arg Arg Gly
    825                 830                 835

tct cag aag aaa cac att tcg cct gta cag gct tta agt gaa ttc aaa 2595
Ser Gln Lys Lys His Ile Ser Pro Val Gln Ala Leu Ser Glu Phe Lys
840                 845                 850                 855

gca atg gat tcc ttc taggacgata ggttttaaca agaaagcttt ttctttcttt 2650
Ala Met Asp Ser Phe
                860

tctttctttc tttttctttt taattccatt tttgtatgca tacctgccag 2700

ctcgtatgcc tctggcatgg ggaaattaag ggaacagtgt ctgtttgcat 2750

gtaagatgag atgagatcaa tactactgat ccatctgtag cctgggaagg 2800

agacaggaca ttcctgtact aaggtggcac agagctgtcc tttgcaacat 2850

tctcataaaa ttgggcacag agttcgcatt ggcgcaatat ttatgggagt 2900

gggagggatg gggaaaataa acttaactct acaaaagcaa actctaatgc 2950

atgcaagaat cattaggttg gcaggtatat gcataagtga aaaatctgga 3000

agtgtaatgg tagaacataa aacttgtatt gcttctgttt cagtgcaaaa 3050

atgtactagc caatacgctt aagtgtgtgg cccatgaatt gaacaattta 3100

accttgaagt ctatatccgt gatattatgt cgatttttaa ctgaggggaa 3150

attaactagt ccagcctaaa atgcttcttt taatctgcat tctgtttcct 3200

cttctagttg tgccattact agtgatcatg tttttttccc ccctttaatg 3250

aaaacaataa acatctattt gagacaatta aaatccttct gggggcactg 3300

gaagcacaat acggtgacca atcttgcttt catttttttt tctttttaat 3350

ttgaaccatg attttgctag aaatagaagg cccagtggtg gaatattaga 3400

gggaaggaaa ctgacaacgt gtgaaagtta 3430

<210>2
<211>860
<212>PRT
<213>Homo sapiens

<400>2

Met Thr Ser Ala Thr Ser Pro Ile Ile Leu Lys Trp Asp Pro Lys Ser
1               5                   10                  15

Leu Glu Ile Arg Thr Leu Thr Val Glu Arg Leu Leu Glu Pro Leu Val
            20                  25                  30

Thr Gln Val Thr Thr Leu Val Asn Thr Ser Asn Lys Gly Pro Ser Gly
        35                  40                  45

Lys Lys Lys Gly Arg Ser Lys Lys Ala His Val Leu Ala Ala Ser Val
    50                  55                  60

Glu Gln Ala Thr Gln Asn Phe Leu Glu Lys Gly Glu Gln Ile Ala Lys
65                  70                  75                  80

Glu Ser Gln Asp Leu Lys Glu Glu Leu Val Ala Ala Val Glu Asp Val
                85                  90                  95

Arg Lys Gln Gly Glu Thr Met Arg Ile Ala Ser Ser Glu Phe Ala Asp
            100                 105                 110

Asp Pro Cys Ser Ser Val Lys Arg Gly Thr Met Val Arg Ala Ala Arg
        115                 120                 125

Ala Leu Leu Ser Ala Val Thr Arg Leu Leu Ile Leu Ala Asp Met Ala
    130                 135                 140

Asp Val Met Arg Leu Leu Ser His Leu Lys Ile Val Glu Glu Ala Leu
145                 150                 155                 160

Glu Ala Val Lys Asn Ala Thr Asn Glu Gln Asp Leu Ala Asn Arg Phe
                165                 170                 175

Lys Glu Phe Gly Lys Lys Met Val Lys Leu Asn Tyr Val Ala Ala Arg
            180                 185                 190

Arg Gln Gln Glu Leu Lys Asp Pro His Cys Arg Asp Glu Met Ala Ala
        195                 200                 205

Ala Arg Gly Ala Leu Lys Lys Asn Ala Thr Met Leu Tyr Thr Ala Ser
    210                 215                 220

Gln Ala Phe Leu Arg His Pro Asp Val Ala Ala Thr Arg Ala Asn Arg
225                 230                 235                 240

Asp Tyr Val Phe Lys Gln Val Gln Glu Ala Ile Ala Gly Ile Ser Asn
                245                 250                 255

Ala Ala Gln Ala Thr Ser Pro Thr Asp Glu Ala Lys Gly His Thr Gly
            260                 265                 270

Ile Gly Glu Leu Ala Ala Ala Leu Asn Glu Phe Asp Asn Lys Ile Ile
        275                 280                 285

Leu Asp Pro Met Thr Phe Ser Glu Ala Arg Phe Arg Pro Ser Leu Glu
    290                 295                 300

Glu Arg Leu Glu Ser Ile Ile Ser Gly Ala Ala Leu Met Ala Asp Ser
305                 310                 315                 320

Ser Cys Thr Arg Asp Asp Arg Arg Glu Arg Ile Val Ala Glu Cys Asn
                325                 330                 335

Ala Val Arg Gln Ala Leu Gln Asp Leu Leu Ser Glu Tyr Met Asn Asn
            340                 345                 350

Thr Gly Arg Lys Glu Lys Gly Asp Pro Leu Asn Ile Ala Ile Asp Lys
        355                 360                 365

Met Thr Lys Lys Thr Arg Asp Leu Arg Arg Gln Leu Arg Lys Ala Val
    370                 375                 380

Met Asp His Ile Ser Asp Ser Phe Leu Glu Thr Asn Val Pro Leu Leu
385                 390                 395                 400

Val Leu Ile Glu Ala Ala Lys Ser Gly Asn Glu Lys Glu Val Lys Glu
                405                 410                 415

Tyr Ala Gln Val Phe Arg Glu His Ala Asn Lys Leu Val Glu Val Ala
            420                 425                 430

Asn Leu Ala Cys Ser Ile Ser Asn Asn Glu Glu Gly Val Lys Leu Val
        435                 440                 445

Arg Met Ala Ala Thr Gln Ile Asp Ser Leu Cys Pro Gln Val Ile Asn
    450                 455                 460

Ala Ala Leu Thr Leu Ala Ala Arg Pro Gln Ser Lys Val Ala Gln Asp
465                 470                 475                 480

Asn Met Asp Val Phe Lys Asp Gln Trp Glu Lys Gln Val Arg Val Leu
                485                 490                 495

Thr Glu Ala Val Asp Asp Ile Thr Ser Val Asp Asp Phe Leu Ser Val
            500                 505                 510

Ser Glu Asn His Ile Leu Glu Asp Val Asn Lys Cys Val Ile Ala Leu
        515                 520                 525

Gln Glu Gly Asp Val Asp Thr Leu Asp Arg Thr Ala Gly Ala Ile Arg
    530                 535                 540

Gly Arg Ala Ala Arg Val Ile His Ile Ile Asn Ala Glu Met Glu Asn
545                 550                 555                 560

Tyr Glu Ala Gly Val Tyr Thr Glu Lys Val Leu Glu Ala Thr Lys Leu
                565                 570                 575

Leu Ser Glu Thr Val Met Pro Arg Phe Ala Glu Gln Val Glu Val Ala
            580                 585                 590

Ile Glu Ala Leu Ser Ala Asn Val Pro Gln Pro Phe Glu Glu Asn Glu
        595                 600                 605

Phe Ile Asp Ala Ser Arg Leu Val Tyr Asp Gly Val Arg Asp Ile Arg
    610                 615                 620

Lys Ala Val Leu Met Ile Arg Thr Pro Glu Glu Leu Glu Asp Asp Ser
625                 630                 635                 640

Asp Phe Glu Gln Glu Asp Tyr Asp Val Arg Arg Gly Thr Ser Val Gln
                645                 650                 655

Thr Glu Asp Asp Gln Leu Ile Ala Gly Gln Ser Ala Arg Ala Ile Met
            660                 665                 670

Ala Gln Leu Pro Gln Glu Glu Lys Ala Lys Ile Ala Glu Gln Val Glu
        675                 680                 685

Ile Phe His Gln Glu Lys Ser Lys Leu Asp Ala Glu Val Ala Lys Trp
    690                 695                 700

Asp Asp Ser Gly Asn Asp Ile Ile Val Leu Ala Lys Gln Met Cys Met
705                 710                 715                 720

Ile Met Met Glu Met Thr Asp Phe Thr Arg Gly Lys Gly Pro Leu Lys
                725                 730                 735

Asn Thr Ser Asp Val Ile Asn Ala Ala Lys Lys Ile Ala Glu Ala Gly
            740                 745                 750

Ser Arg Met Asp Lys Leu Ala Arg Ala Val Ala Asp Gln Leu Asp Ser
        755                 760                 765

Ala Thr Ser Leu Ile Gln Ala Ala Lys Asn Leu Met Asn Ala Val Val
    770                 775                 780

Leu Thr Val Lys Ala Ser Tyr Val Ala Ser Thr Lys Tyr Gln Lys Val
785                 790                 795                 800

Tyr Gly Thr Ala Ala Val Asn Ser Pro Val Val Ser Trp Lys Met Lys
                805                 810                 815

Ala Pro Glu Lys Lys Pro Leu Val Lys Arg Glu Lys Pro Glu Glu Phe
            820                 825                 830

Gln Thr Arg Val Arg Arg Gly Ser Gln Lys Lys His Ile Ser Pro Val
        835                 840                 845

Gln Ala Leu Ser Glu Phe Lys Ala Met Asp Ser Phe
    850                 855                 860

<210>3
<211>2883
<212>DNA
<213>Homo sapiens

<220>
<221> CDS
<222>(124)..(2881)

<400>3

cgtgaatggt gtagtgagtt ctaatgaaac tttatttaca agaggagact 50

gaccaggttt ggcctggggg ccacagtgtg tagacccctg gaaagataca 100

tcctgagaag aaaaaaagaa tat atg cag gaa tgc tta act ttg tgg gtt 150
Met Gln Glu Cys Leu Thr Leu Trp Val
1               5

ttc tct cct ctt gcc ctc act gac tca gga tac aca aag acc tat caa 198
Phe Ser Pro Leu Ala Leu Thr Asp Ser Gly Tyr Thr Lys Thr Tyr Gln
10                  15                  20                  25

gct cac gca aag cag aaa ttc agc cgc tta tgg tcc agc aag tct gtc 246
Ala His Ala Lys Gln Lys Phe Ser Arg Leu Trp Ser Ser Lys Ser Val
                30                  35                  40

act gag att cac cta tac ttt gag gag gaa gtc aag caa gaa gaa tgt 294
Thr Glu Ile His Leu Tyr Phe Glu Glu Glu Val Lys Gln Glu Glu Cys
            45                  50                  55

gac cat ttg gac cgc ctt ttt gct ccc aag gaa gct ggg aaa cag cca 342
Asp His Leu Asp Arg Leu Phe Ala Pro Lys Glu Ala Gly Lys Gln Pro
        60                  65                  70

cgt aca gtg atc att caa gga cca caa gga att gga aaa acg aca ctc 390
Arg Thr Val Ile Ile Gln Gly Pro Gln Gly Ile Gly Lys Thr Thr Leu
    75                  80                  85

ctg atg aag ctg atg atg gcc tgg tcg gac aac aag atc ttt cgg gat 438
Leu Met Lys Leu Met Met Ala Trp Ser Asp Asn Lys Ile Phe Arg Asp
90                  95                  100                 105

agg ttc ctg tac acg ttc tat ttc tgc tgc aga gaa ctg agg gag ttg 486
Arg Phe Leu Tyr Thr Phe Tyr Phe Cys Cys Arg Glu Leu Arg Glu Leu
                110                 115                 120

ccg cca acg agt ttg gct gac ttg att tcc aga gag tgg cct gac ccc 534
Pro Pro Thr Ser Leu Ala Asp Leu Ile Ser Arg Glu Trp Pro Asp Pro
            125                 130                 135

gct gct cct ata aca gag atc gtg tct caa ccg gag aga ctc ttg ttc 582
Ala Ala Pro Ile Thr Glu Ile Val Ser Gln Pro Glu Arg Leu Leu Phe
        140                 145                 150

gtc atc gac agc ttc gaa gag ctg cag ggc ggc ttg aac gaa ccc gat 630
Val Ile Asp Ser Phe Glu Glu Leu Gln Gly Gly Leu Asn Glu Pro Asp
    155                 160                 165

tcg gat ctg tgt ggt gac ttg atg gag aaa cgg ccg gtg cag gtg ctt 678
Ser Asp Leu Cys Gly Asp Leu Met Glu Lys Arg Pro Val Gln Val Leu
170                 175                 180                 185

ctg agc agt ttg ctg agg aag aag atg ctc ccg gag gcc tcc ctg ctc 726
Leu Ser Ser Leu Leu Arg Lys Lys Met Leu Pro Glu Ala Ser Leu Leu
                190                 195                 200

atc gct atc aaa ccc gtg tgc ccg aag gag ctc cgg gat cag gtg acg 774
Ile Ala Ile Lys Pro Val Cys Pro Lys Glu Leu Arg Asp Gln Val Thr
            205                 210                 215

atc tca gaa atc tac cag ccc cgg gga ttc aac gag agt gat agg tta 822
Ile Ser Glu Ile Tyr Gln Pro Arg Gly Phe Asn Glu Ser Asp Arg Leu
        220                 225                 230

gtg tat ttc tgc tgt ttc ttc aaa gac ccg aaa aga gcc atg gaa gcc 870
Val Tyr Phe Cys Cys Phe Phe Lys Asp Pro Lys Arg Ala Met Glu Ala
    235                 240                 245

ttc aat ctt gta aga gaa agt gaa cag ctg ttt tcc ata tgc caa atc 918
Phe Asn Leu Val Arg Glu Ser Glu Gln Leu Phe Ser Ile Cys Gln Ile
250                 255                 260                 265

ccg ctc ctc tgc tgg atc ctg tgt acc agt ctg aag caa gag atg cag 966
Pro Leu Leu Cys Trp Ile Leu Cys Thr Ser Leu Lys Gln Glu Met Gln
                270                 275                 280

aaa gga aaa gac ctg gcc ctg acc tgc cag agc act acc tct gtg tac 1014
Lys Gly Lys Asp Leu Ala Leu Thr Cys Gln Ser Thr Thr Ser Val Tyr
            285                 290                 295

tcc tct ttc gtc ttt aac ctg ttc aca cct gag ggt gcc gag ggc ccg 1062
Ser Ser Phe Val Phe Asn Leu Phe Thr Pro Glu Gly Ala Glu Gly Pro
        300                 305                 310

act ccg caa acc cag cac cag ctg aag gcc ctg tgc tcc ctg gct gca 1110
Thr Pro Gln Thr Gln His Gln Leu Lys Ala Leu Cys Ser Leu Ala Ala
    315                 320                 325

gag ggt atg tgg aca gac aca ttt gag ttt tgt gaa gac gac ctc cgg 1158
Glu Gly Met Trp Thr Asp Thr Phe Glu Phe Cys Glu Asp Asp Leu Arg
330                 335                 340                 345

aga aat ggg gtt gtt gac gct gac atc cct gcg ctg ctg ggc acc aag 1206
Arg Asn Gly Val Val Asp Ala Asp Ile Pro Ala Leu Leu Gly Thr Lys
                350                 355                 360

ata ctt ctg aag tac ggg gag cgt gag agc tcc tac gtg ttc ctc cac 1254
Ile Leu Leu Lys Tyr Gly Glu Arg Glu Ser Ser Tyr Val Phe Leu His
            365                 370                 375

gtg tgt atc cag gag ttc tgt gcc gcc ttg ttc tat ttg ctc aag agc 1302
Val Cys Ile Gln Glu Phe Cys Ala Ala Leu Phe Tyr Leu Leu Lys Ser
        380                 385                 390

cac ctt gat cat cct cac cca gct gtg aga tgt gta cag gaa ttg cta 1350
His Leu Asp His Pro His Pro Ala Val Arg Cys Val Gln Glu Leu Leu
    395                 400                 405

gtt gcc aat ttt gaa aaa gca agg aga gca cat tgg att ttt ttg ggg 1398
Val Ala Asn Phe Glu Lys Ala Arg Arg Ala His Trp Ile Phe Leu Gly
410                 415                 420                 425

tgt ttt cta act ggc ctt tta aat aaa aag gaa caa gaa aaa ctg gat 1446
Cys Phe Leu Thr Gly Leu Leu Asn Lys Lys Glu Gln Glu Lys Leu Asp
                430                 435                 440

gcg ttt ttt ggc ttc caa ctg tcc caa gag ata aag cag caa att cac 1494
Ala Phe Phe Gly Phe Gln Leu Ser Gln Glu Ile Lys Gln Gln Ile His
            445                 450                 455

cag tgc ctg aag agc tta ggg gag cgt ggc aat cct cag gga cag gtg 1542
Gln Cys Leu Lys Ser Leu Gly Glu Arg Gly Asn Pro Gln Gly Gln Val
        460                 465                 470

gat tcc ttg gcg ata ttt tac tgt ctc ttt gaa atg cag gat cct gcc 1590
Asp Ser Leu Ala Ile Phe Tyr Cys Leu Phe Glu Met Gln Asp Pro Ala
    475                 480                 485

ttt gtg aag cag gca gtg aac ctc ctc caa gaa gct aac ttt cat att 1638

Phe Val Lys Gln Ala Val Asn Leu Leu Gln Glu Ala Asn Phe His Ile
490                 495                 500                 505

att gac aac gtg gac ttg gtg gtt tct gcc tac tgc tta aaa tac tgc     1686
Ile Asp Asn Val Asp Leu Val Val Ser Ala Tyr Cys Leu Lys Tyr Cys
                510                 515                 520

tcc agc ttg agg aaa ctc tgt ttt tcc gtt caa aat gtc ttt aag aaa     1734
Ser Ser Leu Arg Lys Leu Cys Phe Ser Val Gln Asn Val Phe Lys Lys
            525                 530                 535

gag gat gaa cac agc tct acg tcg gat tac agc ctc atc tgt tgg cat     1782
Glu Asp Glu His Ser Ser Thr Ser Asp Tyr Ser Leu Ile Cys Trp His
        540                 545                 550

cac atc tgc tct gtg ctc acc acc agc ggg cac ctc aga gag ctc cag     1830
His Ile Cys Ser Val Leu Thr Thr Ser Gly His Leu Arg Glu Leu Gln
    555                 560                 565

gtg cag gac agc acc ctc agc gag tcg acc ttt gtg acc tgg tgt aac     1878
Val Gln Asp Ser Thr Leu Ser Glu Ser Thr Phe Val Thr Trp Cys Asn
570                 575                 580                 585

cag ctg agg cat ccc agc tgt cgc ctt cag aag ctt gga ata aat aac     1926
Gln Leu Arg His Pro Ser Cys Arg Leu Gln Lys Leu Gly Ile Asn Asn
                590                 595                 600

gtt tcc ttt tct ggc cag agt gtt ctg ctc ttt gag gtg ctc ttt tat     1974
Val Ser Phe Ser Gly Gln Ser Val Leu Leu Phe Glu Val Leu Phe Tyr
            605                 610                 615

cag cca gac ttg aaa tac ctg agc ttc acc ctc acg aaa ctc tct cgt     2022
Gln Pro Asp Leu Lys Tyr Leu Ser Phe Thr Leu Thr Lys Leu Ser Arg
        620                 625                 630

gat gac atc agg tcc ctc tgt gat gcc ttg aac tac cca gca ggc aac     2070
Asp Asp Ile Arg Ser Leu Cys Asp Ala Leu Asn Tyr Pro Ala Gly Asn
    635                 640                 645

gtc aaa gag cta gcg ctg gta aat tgt cac ctc tca ccc att gat tgt     2118
Val Lys Glu Leu Ala Leu Val Asn Cys His Leu Ser Pro Ile Asp Cys
650                 655                 660                 665

gaa gtc ctt gct ggc ctt cta acc aac aac aag aag ctg acg tat ctg     2166
Glu Val Leu Ala Gly Leu Leu Thr Asn Asn Lys Lys Leu Thr Tyr Leu
                670                 675                 680

aat gta tcc tgc aac cag tta gac aca ggc gtg ccc ctt ttg tgt gaa     2214
Asn Val Ser Cys Asn Gln Leu Asp Thr Gly Val Pro Leu Leu Cys Glu
            685                 690                 695

gcc ctg tgc agc cca gac acg gtc ctg gta tac ctg atg ttg gct ttc     2262
Ala Leu Cys Ser Pro Asp Thr Val Leu Val Tyr Leu Met Leu Ala Phe
        700                 705                 710

tgc cac ctc agc gag cag tgc tgc gaa tac atc tct gaa atg ctt ctg     2310
Cys His Leu Ser Glu Gln Cys Cys Glu Tyr Ile Ser Glu Met Leu Leu

    715                 720                 725

cgt aac aag agc gtg cgc tat cta gac ctc agt gcc aat gtc ctg aag 2358
Arg Asn Lys Ser Val Arg Tyr Leu Asp Leu Ser Ala Asn Val Leu Lys
730                 735                 740                 745

gac gaa gga ctg aaa act ctc tgc gag gcc ttg aaa cat ccg gac tgc 2406
Asp Glu Gly Leu Lys Thr Leu Cys Glu Ala Leu Lys His Pro Asp Cys
                750                 755                 760

tgc ctg gat tca ctg tgt ttg gta aaa tgt ttt atc act gct gct ggc 2454
Cys Leu Asp Ser Leu Cys Leu Val Lys Cys Phe Ile Thr Ala Ala Gly
            765                 770                 775

tgt gaa gac ctc gcc tct gct ctc atc agc aat caa aac ctg aag att 2502
Cys Glu Asp Leu Ala Ser Ala Leu Ile Ser Asn Gln Asn Leu Lys Ile
        780                 785                 790

ctg caa att ggg tgc aat gaa atc gga gat gtg ggt gtg cag ctg ttg 2550
Leu Gln Ile Gly Cys Asn Glu Ile Gly Asp Val Gly Val Gln Leu Leu
    795                 800                 805

tgt cgg gct ctg acg cat acg gat tgc cgc tta gag att ctt ggg ttg 2598
Cys Arg Ala Leu Thr His Thr Asp Cys Arg Leu Glu Ile Leu Gly Leu
810                 815                 820                 825

gaa gaa tgt ggg tta acg agc acc tgc tgt aag gat ctc gcg tct gtt 2646
Glu Glu Cys Gly Leu Thr Ser Thr Cys Cys Lys Asp Leu Ala Ser Val
                830                 835                 840

ctc acc tgc agt aag acc ctg cag cag ctc aac ctg acc ttg aac acc 2694
Leu Thr Cys Ser Lys Thr Leu Gln Gln Leu Asn Leu Thr Leu Asn Thr
            845                 850                 855

ttg gac cac aca ggg gtg gtt gta ctc tgt gag gcc ctg aga cac cca 2742
Leu Asp His Thr Gly Val Val Val Leu Cys Glu Ala Leu Arg His Pro
        860                 865                 870

gag tgt gcc ctg cag gtg ctc ggg ctg aga aaa act gat ttt gat gag 2790
Glu Cys Ala Leu Gln Val Leu Gly Leu Arg Lys Thr Asp Phe Asp Glu
    875                 880                 885

gaa acc cag gca ctt ctg acg gct gag gaa gag aga aat cct aac ctg 2838
Glu Thr Gln Ala Leu Leu Thr Ala Glu Glu Glu Arg Asn Pro Asn Leu
890                 895                 900                 905

acc atc aca gac gac tgt gac aca atc aca agg gta gag atc 2880
Thr Ile Thr Asp Asp Cys Asp Thr Ile Thr Arg Val Glu Ile
                910                 915

tga 2883

<210>4
<211>919
<212>PRT

<213>Homo sapiens

<400>4

Met Gln Glu Cys Leu Thr Leu Trp Val Phe Ser Pro Leu Ala Leu Thr
1               5                   10                  15

Asp Ser Gly Tyr Thr Lys Thr Tyr Gln Ala His Ala Lys Gln Lys Phe
            20                  25                  30

Ser Arg Leu Trp Ser Ser Lys Ser Val Thr Glu Ile His Leu Tyr Phe
        35                  40                  45

Glu Glu Glu Val Lys Gln Glu Glu Cys Asp His Leu Asp Arg Leu Phe
    50                  55                  60

Ala Pro Lys Glu Ala Gly Lys Gln Pro Arg Thr Val Ile Ile Gln Gly
65                  70                  75                  80

Pro Gln Gly Ile Gly Lys Thr Thr Leu Leu Met Lys Leu Met Met Ala
                85                  90                  95

Trp Ser Asp Asn Lys Ile Phe Arg Asp Arg Phe Leu Tyr Thr Phe Tyr
            100                 105                 110

Phe Cys Cys Arg Glu Leu Arg Glu Leu Pro Pro Thr Ser Leu Ala Asp
        115                 120                 125

Leu Ile Ser Arg Glu Trp Pro Asp Pro Ala Ala Pro Ile Thr Glu Ile
    130                 135                 140

Val Ser Gln Pro Glu Arg Leu Leu Phe Val Ile Asp Ser Phe Glu Glu
145                 150                 155                 160

Leu Gln Gly Gly Leu Asn Glu Pro Asp Ser Asp Leu Cys Gly Asp Leu
                165                 170                 175

Met Glu Lys Arg Pro Val Gln Val Leu Leu Ser Ser Leu Leu Arg Lys
            180                 185                 190

Lys Met Leu Pro Glu Ala Ser Leu Leu Ile Ala Ile Lys Pro Val Cys
        195                 200                 205

Pro Lys Glu Leu Arg Asp Gln Val Thr Ile Ser Glu Ile Tyr Gln Pro
    210                 215                 220

Arg Gly Phe Asn Glu Ser Asp Arg Leu Val Tyr Phe Cys Cys Phe Phe
225                 230                 235                 240

Lys Asp Pro Lys Arg Ala Met Glu Ala Phe Asn Leu Val Arg Glu Ser
                245                 250                 255

Glu Gln Leu Phe Ser Ile Cys Gln Ile Pro Leu Leu Cys Trp Ile Leu
            260                 265                 270

Cys Thr Ser Leu Lys Gln Glu Met Gln Lys Gly Lys Asp Leu Ala Leu
        275                 280                 285

Thr Cys Gln Ser Thr Thr Ser Val Tyr Ser Ser Phe Val Phe Asn Leu
    290                 295                 300

Phe Thr Pro Glu Gly Ala Glu Gly Pro Thr Pro Gln Thr Gln His Gln
305                 310                 315                 320

Leu Lys Ala Leu Cys Ser Leu Ala Ala Glu Gly Met Trp Thr Asp Thr
                325                 330                 335

Phe Glu Phe Cys Glu Asp Asp Leu Arg Arg Asn Gly Val Val Asp Ala
            340                 345                 350

Asp Ile Pro Ala Leu Leu Gly Thr Lys Ile Leu Leu Lys Tyr Gly Glu
        355                 360                 365

Arg Glu Ser Ser Tyr Val Phe Leu His Val Cys Ile Gln Glu Phe Cys
    370                 375                 380

Ala Ala Leu Phe Tyr Leu Leu Lys Ser His Leu Asp His Pro His Pro
385                 390                 395                 400

Ala Val Arg Cys Val Gln Glu Leu Leu Val Ala Asn Phe Glu Lys Ala
                405                 410                 415

Arg Arg Ala His Trp Ile Phe Leu Gly Cys Phe Leu Thr Gly Leu Leu
            420                 425                 430

Asn Lys Lys Glu Gln Glu Lys Leu Asp Ala Phe Phe Gly Phe Gln Leu
        435                 440                 445

Ser Gln Glu Ile Lys Gln Gln Ile His Gln Cys Leu Lys Ser Leu Gly
    450                 455                 460

Glu Arg Gly Asn Pro Gln Gly Gln Val Asp Ser Leu Ala Ile Phe Tyr
465                 470                 475                 480

Cys Leu Phe Glu Met Gln Asp Pro Ala Phe Val Lys Gln Ala Val Asn
                485                 490                 495

Leu Leu Gln Glu Ala Asn Phe His Ile Ile Asp Asn Val Asp Leu Val
            500                 505                 510

Val Ser Ala Tyr Cys Leu Lys Tyr Cys Ser Ser Leu Arg Lys Leu Cys
        515                 520                 525

Phe Ser Val Gln Asn Val Phe Lys Lys Glu Asp Glu His Ser Ser Thr
    530                 535                 540

Ser Asp Tyr Ser Leu Ile Cys Trp His His Ile Cys Ser Val Leu Thr
545                 550                 555                 560

Thr Ser Gly His Leu Arg Glu Leu Gln Val Gln Asp Ser Thr Leu Ser
                565                 570                 575

Glu Ser Thr Phe Val Thr Trp Cys Asn Gln Leu Arg His Pro Ser Cys
            580                 585                 590

Arg Leu Gln Lys Leu Gly Ile Asn Asn Val Ser Phe Ser Gly Gln Ser
        595                 600                 605

Val Leu Leu Phe Glu Val Leu Phe Tyr Gln Pro Asp Leu Lys Tyr Leu
    610                 615                 620

Ser Phe Thr Leu Thr Lys Leu Ser Arg Asp Asp Ile Arg Ser Leu Cys
625                 630                 635                 640

Asp Ala Leu Asn Tyr Pro Ala Gly Asn Val Lys Glu Leu Ala Leu Val
                645                 650                 655

Asn Cys His Leu Ser Pro Ile Asp Cys Glu Val Leu Ala Gly Leu Leu
            660                 665                 670

Thr Asn Asn Lys Lys Leu Thr Tyr Leu Asn Val Ser Cys Asn Gln Leu
        675                 680                 685

Asp Thr Gly Val Pro Leu Leu Cys Glu Ala Leu Cys Ser Pro Asp Thr
    690                 695                 700

Val Leu Val Tyr Leu Met Leu Ala Phe Cys His Leu Ser Glu Gln Cys
705                 710                 715                 720

Cys Glu Tyr Ile Ser Glu Met Leu Leu Arg Asn Lys Ser Val Arg Tyr
                725                 730                 735

Leu Asp Leu Ser Ala Asn Val Leu Lys Asp Glu Gly Leu Lys Thr Leu
            740                 745                 750

Cys Glu Ala Leu Lys His Pro Asp Cys Cys Leu Asp Ser Leu Cys Leu
        755                 760                 765

Val Lys Cys Phe Ile Thr Ala Ala Gly Cys Glu Asp Leu Ala Ser Ala
    770                 775                 780

Leu Ile Ser Asn Gln Asn Leu Lys Ile Leu Gln Ile Gly Cys Asn Glu
785                 790                 795                 800

Ile Gly Asp Val Gly Val Gln Leu Leu Cys Arg Ala Leu Thr His Thr
                805                 810                 815

Asp Cys Arg Leu Glu Ile Leu Gly Leu Glu Glu Cys Gly Leu Thr Ser
            820                 825                 830

Thr Cys Cys Lys Asp Leu Ala Ser Val Leu Thr Cys Ser Lys Thr Leu
        835                 840                 845

Gln Gln Leu Asn Leu Thr Leu Asn Thr Leu Asp His Thr Gly Val Val
    850                 855                 860

Val Leu Cys Glu Ala Leu Arg His Pro Glu Cys Ala Leu Gln Val Leu
865                 870                 875                 880

Gly Leu Arg Lys Thr Asp Phe Asp Glu Glu Thr Gln Ala Leu Leu Thr
                885                 890                 895

Ala Glu Glu Glu Arg Asn Pro Asn Leu Thr Ile Thr Asp Asp Cys Asp
            900                 905                 910

Thr Ile Thr Arg Val Glu Ile
        915

<210>5
<211>2142
<212>DNA
<213>Homo sapiens

<220>
<221> CDS
<222>(34)..(2077)

<400>5

tattattcag caaacaatct caatgtgttc ctg atg gga gag aga gca tct 51
Met Gly Glu Arg Ala Ser
1               5

gga aaa act att gtt ata aat ctg gct gtg ttg agg tgg atc aag ggt 99
Gly Lys Thr Ile Val Ile Asn Leu Ala Val Leu Arg Trp Ile Lys Gly
            10                  15                  20

gag atg tgg cag aac atg atc tcg tac gtc gtt cac ctc act gct cac 147
Glu Met Trp Gln Asn Met Ile Ser Tyr Val Val His Leu Thr Ala His
        25                  30                  35

gaa ata aac cag atg acc aac agc agc ttg gct gag cta atc gcc aag 195
Glu Ile Asn Gln Met Thr Asn Ser Ser Leu Ala Glu Leu Ile Ala Lys
    40                  45                  50

gac tgg cct gac ggc cag gct ccc att gca gac atc ctg tct gat ccc 243
Asp Trp Pro Asp Gly Gln Ala Pro Ile Ala Asp Ile Leu Ser Asp Pro
55                  60                  65                  70

aag aaa ctc ctt ttc atc ctc gag gac ttg gac aac ata aga ttc gag 291
Lys Lys Leu Leu Phe Ile Leu Glu Asp Leu Asp Asn Ile Arg Phe Glu
                75                  80                  85

tta aat gtc aat gaa agt gct ttg tgt agt aac agc acc cag aaa gtt 339
Leu Asn Val Asn Glu Ser Ala Leu Cys Ser Asn Ser Thr Gln Lys Val
            90                  95                  100

ccc att cca gtt ctc ctg gtc agt ttg ctg aag aga aaa atg gct cca 387
Pro Ile Pro Val Leu Leu Val Ser Leu Leu Lys Arg Lys Met Ala Pro
        105                 110                 115

ggc tgc tgg ttc ctc atc tcc tca agg ccc aca cgt ggg aat aat gta 435
Gly Cys Trp Phe Leu Ile Ser Ser Arg Pro Thr Arg Gly Asn Asn Val
    120                 125                 130

aaa acg ttc ttg aaa gag gta gat tgc tgc acg acc ttg cag ctg tcg 483
Lys Thr Phe Leu Lys Glu Val Asp Cys Cys Thr Thr Leu Gln Leu Ser
135                 140                 145                 150

aat ggg aag agg gag ata tat ttt aac tct ttc ttt aaa gac cgc cag 531
Asn Gly Lys Arg Glu Ile Tyr Phe Asn Ser Phe Phe Lys Asp Arg Gln
                155                 160                 165

agg gcg tcg gca gcc ctc cag ctt gta cat gag gat gaa ata ctc gtg 579
Arg Ala Ser Ala Ala Leu Gln Leu Val His Glu Asp Glu Ile Leu Val
            170                 175                 180

ggt ctg tgc cga gtc gcc atc tta tgc tgg atc acg tgt act gtc ctg 627
Gly Leu Cys Arg Val Ala Ile Leu Cys Trp Ile Thr Cys Thr Val Leu
        185                 190                 195

aag cgg cag atg gac aag ggg cgt gac ttc cag ctc tgc tgc caa aca 675
Lys Arg Gln Met Asp Lys Gly Arg Asp Phe Gln Leu Cys Cys Gln Thr
    200                 205                 210

ccc act gat cta cat gcc cac ttt ctt gct gat gcg ttg aca tca gag 723
Pro Thr Asp Leu His Ala His Phe Leu Ala Asp Ala Leu Thr Ser Glu
215                 220                 225                 230

gct gga ctt act gcc aat cag tat cac cta ggt ctc cta aaa cgt ctg 771
Ala Gly Leu Thr Ala Asn Gln Tyr His Leu Gly Leu Leu Lys Arg Leu
                235                 240                 245

tgt ttg ctg gct gca gga gga ctg ttt ctg agc acc ctg aat ttc agt 819
Cys Leu Leu Ala Ala Gly Gly Leu Phe Leu Ser Thr Leu Asn Phe Ser
            250                 255                 260

ggt gaa gac ctc aga tgt gtt ggg ttt act gag gct gat gtc tct gtg 867
Gly Glu Asp Leu Arg Cys Val Gly Phe Thr Glu Ala Asp Val Ser Val
        265                 270                 275

ttg cag gcc gcg aat att ctt ttg ccg agc aac act cat aaa gac cgt 915
Leu Gln Ala Ala Asn Ile Leu Leu Pro Ser Asn Thr His Lys Asp Arg
    280                 285                 290

tac aag ttc ata cac ttg aac gtc cag gag ttt tgt aca gcc att gca 963
Tyr Lys Phe Ile His Leu Asn Val Gln Glu Phe Cys Thr Ala Ile Ala
295                 300                 305                 310

ttt ctg atg gca gta ccc aac tat ctg atc ccc tca ggc agc aga gag 1011
Phe Leu Met Ala Val Pro Asn Tyr Leu Ile Pro Ser Gly Ser Arg Glu
                315                 320                 325

tat aaa gag aag aga gaa caa tac tct gac ttt aat caa gtg ttt act 1059
Tyr Lys Glu Lys Arg Glu Gln Tyr Ser Asp Phe Asn Gln Val Phe Thr
            330                 335                 340

ttc att ttt ggt ctt cta aat gca aac agg aga aag att ctt gag aca 1107
Phe Ile Phe Gly Leu Leu Asn Ala Asn Arg Arg Lys Ile Leu Glu Thr
        345                 350                 355

tcc ttt gga tac cag cta ccg atg gta gac agc ttc aag tgg tac tcg 1155
Ser Phe Gly Tyr Gln Leu Pro Met Val Asp Ser Phe Lys Trp Tyr Ser
    360                 365                 370

gtg gga tac atg aaa cat ttg gac cgt gac ccg gaa aag ttg acg cac 1203
Val Gly Tyr Met Lys His Leu Asp Arg Asp Pro Glu Lys Leu Thr His
375                 380                 385                 390

cat atg cct ttg ttt tac tgt ctc tat gag aat cgg gaa gaa gaa ttt 1251
His Met Pro Leu Phe Tyr Cys Leu Tyr Glu Asn Arg Glu Glu Glu Phe
                395                 400                 405

gtg aag acg att gtg gat gct ctc atg gag gtt aca gtt tac ctt caa 1299
Val Lys Thr Ile Val Asp Ala Leu Met Glu Val Thr Val Tyr Leu Gln
            410                 415                 420

tca gac aag gat atg atg gtc tca tta tac tgt ctg gat tac tgc tgt 1347
Ser Asp Lys Asp Met Met Val Ser Leu Tyr Cys Leu Asp Tyr Cys Cys
        425                 430                 435

cac ctg agg aca ctt aag ttg agt gtt cag cgc atc ttt caa aac aaa 1395
His Leu Arg Thr Leu Lys Leu Ser Val Gln Arg Ile Phe Gln Asn Lys
    440                 445                 450

ctg gag aaa tgc aac ttg tcg gca gcc agc tgt cag gac cta gcc ttg 1443
Leu Glu Lys Cys Asn Leu Ser Ala Ala Ser Cys Gln Asp Leu Ala Leu
455                 460                 465                 470

ttt ctc acc agc atc caa cac gta act cga ttg tgc ctg gga ttt aat 1491
Phe Leu Thr Ser Ile Gln His Val Thr Arg Leu Cys Leu Gly Phe Asn
                475                 480                 485

cgg ctc caa gat gat ggc ata aag cta ttg tgt gcg gcc ctg act cac 1539
Arg Leu Gln Asp Asp Gly Ile Lys Leu Leu Cys Ala Ala Leu Thr His
            490                 495                 500

ccc aag tgt gcc tta gag aga ctg gag ctc tgg ttt tgc cag ctg gca 1587
Pro Lys Cys Ala Leu Glu Arg Leu Glu Leu Trp Phe Cys Gln Leu Ala
        505                 510                 515

gca ccc gct tgc aag cac ttg tca gat gct ctc ctg cag aac agg agc 1635
Ala Pro Ala Cys Lys His Leu Ser Asp Ala Leu Leu Gln Asn Arg Ser
    520                 525                 530

ctg aca cac ctg aat ctg agc aag aac agc ctg aga gac gag gga gtc 1683
Leu Thr His Leu Asn Leu Ser Lys Asn Ser Leu Arg Asp Glu Gly Val
535                 540                 545                 550

aag ttc ctg tgt gag gcc ttg ggt cgc cca gat ggt aac ctg cag agc 1731
Lys Phe Leu Cys Glu Ala Leu Gly Arg Pro Asp Gly Asn Leu Gln Ser
                555                 560                 565

ctg agt ttg tca ggt tgt tct ttc aca aga gag ggc tgt gga gag ctg 1779
Leu Ser Leu Ser Gly Cys Ser Phe Thr Arg Glu Gly Cys Gly Glu Leu
            570                 575                 580

gct aat gcc ctc agc cat aat cat aat gtg aaa atc ttg gat ttg gga 1827
Ala Asn Ala Leu Ser His Asn His Asn Val Lys Ile Leu Asp Leu Gly
        585                 590                 595

gaa aat gat ctt cag gat gat gga gtg aag cta ctg tgt gag gct ctg 1875

Glu Asn Asp Leu Gln Asp Asp Gly Val Lys Leu Leu Cys Glu Ala Leu
    600                 605                 610

aaa cca cat cgt gca ttg cac aca ctt ggg ttg gcg aaa tgc aat ctg    1923
Lys Pro His Arg Ala Leu His Thr Leu Gly Leu Ala Lys Cys Asn Leu
615                 620                 625                 630

aca act gct tgc tgc cag cat ctc ttc tct gtt ctc agc agc agt aag    1971
Thr Thr Ala Cys Cys Gln His Leu Phe Ser Val Leu Ser Ser Ser Lys
                635                 640                 645

agc ctg gtc aat ctg aac ctt cta ggc aat gaa ttg gat act gat ggt    2019
Ser Leu Val Asn Leu Asn Leu Leu Gly Asn Glu Leu Asp Thr Asp Gly
            650                 655                 660

gtc aag atg cta tgt aag gct ttg aaa aag tcg aca tgc agg ctg cag    2067
Val Lys Met Leu Cys Lys Ala Leu Lys Lys Ser Thr Cys Arg Leu Gln
        665                 670                 675

aaa ctc ggg taaacctcac tgacttttct gcaggggaga acatacaggg            2116
Lys Leu Gly
    680

acaaggctag attgactagg cttcta                                       2142

<210>6
<211>681
<212>PRT
<213>Homo sapiens

<400>6

Met Gly Glu Arg Ala Ser Gly Lys Thr Ile Val Ile Asn Leu Ala Val
1               5                   10                  15

Leu Arg Trp Ile Lys Gly Glu Met Trp Gln Asn Met Ile Ser Tyr Val
            20                  25                  30

Val His Leu Thr Ala His Glu Ile Asn Gln Met Thr Asn Ser Ser Leu
        35                  40                  45

Ala Glu Leu Ile Ala Lys Asp Trp Pro Asp Gly Gln Ala Pro Ile Ala
    50                  55                  60

Asp Ile Leu Ser Asp Pro Lys Lys Leu Leu Phe Ile Leu Glu Asp Leu
65                  70                  75                  80

Asp Asn Ile Arg Phe Glu Leu Asn Val Asn Glu Ser Ala Leu Cys Ser
                85                  90                  95

Asn Ser Thr Gln Lys Val Pro Ile Pro Val Leu Leu Val Ser Leu Leu
            100                 105                 110

Lys Arg Lys Met Ala Pro Gly Cys Trp Phe Leu Ile Ser Ser Arg Pro
        115                 120                 125

Thr Arg Gly Asn Asn Val Lys Thr Phe Leu Lys Glu Val Asp Cys Cys
    130                 135                 140

Thr Thr Leu Gln Leu Ser Asn Gly Lys Arg Glu Ile Tyr Phe Asn Ser
145                 150                 155                 160

Phe Phe Lys Asp Arg Gln Arg Ala Ser Ala Ala Leu Gln Leu Val His
                165                 170                 175

Glu Asp Glu Ile Leu Val Gly Leu Cys Arg Val Ala Ile Leu Cys Trp
            180                 185                 190

Ile Thr Cys Thr Val Leu Lys Arg Gln Met Asp Lys Gly Arg Asp Phe
        195                 200                 205

Gln Leu Cys Cys Gln Thr Pro Thr Asp Leu His Ala His Phe Leu Ala
    210                 215                 220

Asp Ala Leu Thr Ser Glu Ala Gly Leu Thr Ala Asn Gln Tyr His Leu
225                 230                 235                 240

Gly Leu Leu Lys Arg Leu Cys Leu Leu Ala Ala Gly Gly Leu Phe Leu
                245                 250                 255

Ser Thr Leu Asn Phe Ser Gly Glu Asp Leu Arg Cys Val Gly Phe Thr
            260                 265                 270

Glu Ala Asp Val Ser Val Leu Gln Ala Ala Asn Ile Leu Leu Pro Ser
        275                 280                 285

Asn Thr His Lys Asp Arg Tyr Lys Phe Ile His Leu Asn Val Gln Glu
    290                 295                 300

Phe Cys Thr Ala Ile Ala Phe Leu Met Ala Val Pro Asn Tyr Leu Ile
305                 310                 315                 320

Pro Ser Gly Ser Arg Glu Tyr Lys Glu Lys Arg Glu Gln Tyr Ser Asp
                325                 330                 335

Phe Asn Gln Val Phe Thr Phe Ile Phe Gly Leu Leu Asn Ala Asn Arg
            340                 345                 350

Arg Lys Ile Leu Glu Thr Ser Phe Gly Tyr Gln Leu Pro Met Val Asp
        355                 360                 365

Ser Phe Lys Trp Tyr Ser Val Gly Tyr Met Lys His Leu Asp Arg Asp
    370                 375                 380

Pro Glu Lys Leu Thr His His Met Pro Leu Phe Tyr Cys Leu Tyr Glu
385                 390                 395                 400

Asn Arg Glu Glu Glu Phe Val Lys Thr Ile Val Asp Ala Leu Met Glu
                405                 410                 415

Val Thr Val Tyr Leu Gln Ser Asp Lys Asp Met Met Val Ser Leu Tyr
            420                 425                 430

Cys Leu Asp Tyr Cys Cys His Leu Arg Thr Leu Lys Leu Ser Val Gln
        435                 440                 445

Arg Ile Phe Gln Asn Lys Leu Glu Lys Cys Asn Leu Ser Ala Ala Ser
    450                 455                 460

Cys Gln Asp Leu Ala Leu Phe Leu Thr Ser Ile Gln His Val Thr Arg
465                 470                 475                 480

Leu Cys Leu Gly Phe Asn Arg Leu Gln Asp Asp Gly Ile Lys Leu Leu
                485                 490                 495

Cys Ala Ala Leu Thr His Pro Lys Cys Ala Leu Glu Arg Leu Glu Leu
            500                 505                 510

Trp Phe Cys Gln Leu Ala Ala Pro Ala Cys Lys His Leu Ser Asp Ala
        515                 520                 525

Leu Leu Gln Asn Arg Ser Leu Thr His Leu Asn Leu Ser Lys Asn Ser
    530                 535                 540

Leu Arg Asp Glu Gly Val Lys Phe Leu Cys Glu Ala Leu Gly Arg Pro
545                 550                 555                 560

Asp Gly Asn Leu Gln Ser Leu Ser Leu Ser Gly Cys Ser Phe Thr Arg
                565                 570                 575

Glu Gly Cys Gly Glu Leu Ala Asn Ala Leu Ser His Asn His Asn Val
            580                 585                 590

Lys Ile Leu Asp Leu Gly Glu Asn Asp Leu Gln Asp Asp Gly Val Lys
        595                 600                 605

Leu Leu Cys Glu Ala Leu Lys Pro His Arg Ala Leu His Thr Leu Gly
    610                 615                 620

Leu Ala Lys Cys Asn Leu Thr Thr Ala Cys Cys Gln His Leu Phe Ser
625                 630                 635                 640

Val Leu Ser Ser Ser Lys Ser Leu Val Asn Leu Asn Leu Leu Gly Asn
                645                 650                 655

Glu Leu Asp Thr Asp Gly Val Lys Met Leu Cys Lys Ala Leu Lys Lys
            660                 665                 670

Ser Thr Cys Arg Leu Gln Lys Leu Gly
        675                 680

<210>7
<211>782
<212>DNA
<213>Homo sapiens

<220>
<221> CDS
<222>(76)..(694)

<400>7

tctcaaggga taatcactaa attctgccga aaggactgag gaacggtgcc          50

tggaaaaggg caagaatatc acggc atg ggc atg agt agc ttg aaa ctg ctg    102
                            Met Gly Met Ser Ser Leu Lys Leu Leu
                            1               5

aag tat gtc ctg ttt ttc ttc aac ttg ctc ttt tgg atc tgt ggc tgc    150
Lys Tyr Val Leu Phe Phe Phe Asn Leu Leu Phe Trp Ile Cys Gly Cys
10                  15                  20                  25

tgc att ttg ggc ttt ggg atc tac ctg ctg atc cac aac aac ttc gga    198
Cys Ile Leu Gly Phe Gly Ile Tyr Leu Leu Ile His Asn Asn Phe Gly
                30                  35                  40

gtg ctc ttc cat aac ctc ccc tcc ctc acg ctg ggc aat gtg ttt gtc    246
Val Leu Phe His Asn Leu Pro Ser Leu Thr Leu Gly Asn Val Phe Val
            45                  50                  55

atc gtg ggc tct atc aag gaa aac aag tgt ctg ctt atg tcg ttc ttc    294
Ile Val Gly Ser Ile Lys Glu Asn Lys Cys Leu Leu Met Ser Phe Phe
        60                  65                  70

atc ctg ctg ctg att atc ctc ctt gct gag gtg acc ttg gcc atc ctg    342
Ile Leu Leu Leu Ile Ile Leu Leu Ala Glu Val Thr Leu Ala Ile Leu
    75                  80                  85

ctc ttt gta tat gaa cag aag ctg aat gag tat gtg gct aag ggt ctg    390
Leu Phe Val Tyr Glu Gln Lys Leu Asn Glu Tyr Val Ala Lys Gly Leu
90                  95                  100                 105

acc gac agc atc cac cgt tac cac tca gac aat agc acc aag gca gcg    438
Thr Asp Ser Ile His Arg Tyr His Ser Asp Asn Ser Thr Lys Ala Ala
                110                 115                 120

tgg gac tcc atc cag tca ttt ctg cag tgt tgt ggt ata aat ggc acg    486
Trp Asp Ser Ile Gln Ser Phe Leu Gln Cys Cys Gly Ile Asn Gly Thr
            125                 130                 135

agt gat ggg acc agt ggc cca cca gca tct tgc ccc tca gat cga aaa    534
Ser Asp Gly Thr Ser Gly Pro Pro Ala Ser Cys Pro Ser Asp Arg Lys
        140                 145                 150

gtg gag ggt tgc tat gcg aaa gca aga ctg tgg ttt cat tcc aat ttc    582
Val Glu Gly Cys Tyr Ala Lys Ala Arg Leu Trp Phe His Ser Asn Phe
    155                 160                 165

ctg tat atc gga atc atc acc atc tgt gta tgt gtg att gag gtg ttg    630
Leu Tyr Ile Gly Ile Ile Thr Ile Cys Val Cys Val Ile Glu Val Leu
170                 175                 180                 185

ggg atg tcc ttt gca ctg acc ctg aac tgc cag att gac aaa acc agc    678
Gly Met Ser Phe Ala Leu Thr Leu Asn Cys Gln Ile Asp Lys Thr Ser

                190                 195                 200

cag acc ata ggg cta tgatctgcag tagttctgtg gtgaagagac ttgtttcatc 733
Gln Thr Ile Gly Leu
            205

tcctggaaat gcaaaaccat ttatagcatg agccctacat gatcatcag 782

<210>8
<211>206
<212>PRT
<213>Homo sapiens

<400>8

Met Gly Met Ser Ser Leu Lys Leu Leu Lys Tyr Val Leu Phe Phe Phe
1               5                   10                  15

Asn Leu Leu Phe Trp Ile Cys Gly Cys Cys Ile Leu Gly Phe Gly Ile
            20                  25                  30

Tyr Leu Leu Ile His Asn Asn Phe Gly Val Leu Phe His Asn Leu Pro
        35                  40                  45

Ser Leu Thr Leu Gly Asn Val Phe Val Ile Val Gly Ser Ile Lys Glu
    50                  55                  60

Asn Lys Cys Leu Leu Met Ser Phe Phe Ile Leu Leu Leu Ile Ile Leu
65                  70                  75                  80

Leu Ala Glu Val Thr Leu Ala Ile Leu Leu Phe Val Tyr Glu Gln Lys
                85                  90                  95

Leu Asn Glu Tyr Val Ala Lys Gly Leu Thr Asp Ser Ile His Arg Tyr
            100                 105                 110

His Ser Asp Asn Ser Thr Lys Ala Ala Trp Asp Ser Ile Gln Ser Phe
        115                 120                 125

Leu Gln Cys Cys Gly Ile Asn Gly Thr Ser Asp Gly Thr Ser Gly Pro
    130                 135                 140

Pro Ala Ser Cys Pro Ser Asp Arg Lys Val Glu Gly Cys Tyr Ala Lys
145                 150                 155                 160

Ala Arg Leu Trp Phe His Ser Asn Phe Leu Tyr Ile Gly Ile Ile Thr
                165                 170                 175

Ile Cys Val Cys Val Ile Glu Val Leu Gly Met Ser Phe Ala Leu Thr
            180                 185                 190

Leu Asn Cys Gln Ile Asp Lys Thr Ser Gln Thr Ile Gly Leu
        195                 200                 205

<210>9

<211>1719
<212>DNA
<213>Homo sapiens

<220>
<221> CDS
<222>(1)..(877)

<400>9

atg cgg acg ccg gtg gtg atg acg ctg ggc atg gtg ttg gcg ccc tgc 48
Met Arg Thr Pro Val Val Met Thr Leu Gly Met Val Leu Ala Pro Cys
1               5                   10                  15

ggg ctc ctg ctc aac ctg acc ggc acc ctg gcg ccc ggc tgg cgg ctg 96
Gly Leu Leu Leu Asn Leu Thr Gly Thr Leu Ala Pro Gly Trp Arg Leu
            20                  25                  30

gtg aag ggc ttc ctg aac cag cca gtg gac gtg gag ttg tac cag ggc 144
Val Lys Gly Phe Leu Asn Gln Pro Val Asp Val Glu Leu Tyr Gln Gly
        35                  40                  45

ctg tgg gac atg tgt cgc gag cag agc agc cgc gag cgc gag tgc ggc 192
Leu Trp Asp Met Cys Arg Glu Gln Ser Ser Arg Glu Arg Glu Cys Gly
    50                  55                  60

cag acg gac cag tgg ggc tac ttc gag gcc cag ccc gtg ctg gtg gcg 240
Gln Thr Asp Gln Trp Gly Tyr Phe Glu Ala Gln Pro Val Leu Val Ala
65                  70                  75                  80

cgg gca ctc atg gtc acc tcg ctg gcc gcc acg gtc ctg ggg ctt ctg 288
Arg Ala Leu Met Val Thr Ser Leu Ala Ala Thr Val Leu Gly Leu Leu
                85                  90                  95

ctg gcg tcg ctg ggc gtg cgc tgc tgg cag gac gag ccc aac ttc gtg 336
Leu Ala Ser Leu Gly Val Arg Cys Trp Gln Asp Glu Pro Asn Phe Val
            100                 105                 110

ctg gca ggg ctc tcg ggc gtc gtg ctc ttc gtc gct ggc ctc ctc ggc 384
Leu Ala Gly Leu Ser Gly Val Val Leu Phe Val Ala Gly Leu Leu Gly
        115                 120                 125

ctc atc ccg gtg tcc tgg tac aac cac ttc ttg ggg gac cgc gac gtg 432
Leu Ile Pro Val Ser Trp Tyr Asn His Phe Leu Gly Asp Arg Asp Val
    130                 135                 140

ctg ccc gcc ccg gcc agc ccg gtc acg gtg cag gtc agc tac agc ctg 480
Leu Pro Ala Pro Ala Ser Pro Val Thr Val Gln Val Ser Tyr Ser Leu
145                 150                 155                 160

gtc ctg ggc tac ctg ggc agc tgc ctc ctg ctg ctg ggc ggc ttc tcg 528
Val Leu Gly Tyr Leu Gly Ser Cys Leu Leu Leu Leu Gly Gly Phe Ser
                165                 170                 175

ctg gcg ctc agc ttc gcg ccc tgg tgc gac gag cgt tgt cgc cgc cgc 576
Leu Ala Leu Ser Phe Ala Pro Trp Cys Asp Glu Arg Cys Arg Arg Arg
            180                 185                 190

cgc aag gga ccc tcc gcc ggg cct cgc cgc agc agc gtc agc acc atc 624
Arg Lys Gly Pro Ser Ala Gly Pro Arg Arg Ser Ser Val Ser Thr Ile
        195                 200                 205

caa gtg gag tgg ccc gag ccc gac ctg gcg ccc gcc atc aag tac tac 672
Gln Val Glu Trp Pro Glu Pro Asp Leu Ala Pro Ala Ile Lys Tyr Tyr
    210                 215                 220

agc gac ggc cag cac cga ccg ccg cct gcc cag cac cgc aag ccc aag 720
Ser Asp Gly Gln His Arg Pro Pro Pro Ala Gln His Arg Lys Pro Lys
225                 230                 235                 240

ccc aag ccc aag gtc ggc ttc ccc atg ccg cgg ccg cgg ccc aag gcc 768
Pro Lys Pro Lys Val Gly Phe Pro Met Pro Arg Pro Arg Pro Lys Ala
                245                 250                 255

tac acc aac tcg gtg gac gtc ctc gac ggg gag ggg tgg gag tcc cag 816
Tyr Thr Asn Ser Val Asp Val Leu Asp Gly Glu Gly Trp Glu Ser Gln
            260                 265                 270

gac gct ccc tcg tgc agc acc cac ccc tgc gac agc tcg ctg ccc tgc 864
Asp Ala Pro Ser Cys Ser Thr His Pro Cys Asp Ser Ser Leu Pro Cys
        275                 280                 285

gac tcc gac ctc tagacgcttg tagagcctgg ggggcgccgg gtggcaaagg 916
Asp Ser Asp Leu
    290

actcaccccc gcacaggccc gcctggcttc gagttggaac ccggacactt 966

gcccctcact ggtgtggatg gaaatctgcc tttcgtggga ccaaacagga 1016

ctccttggac gattagttca ggttgggttt ggttttcttc ttaaagagtt 1066

tagttttcct ctccagaggg atcagggtcc tcttagggag tgacgggctt 1116

ttcatatatt tttgctgaag aatatatgga aagggtggca tttgcgtcac 1166

gtggaccagg gacagtgctg aaatcagcag tgctcagaaa caatttaaca 1216

tgttgaaacg acaatattct aaaatactga tgaatcttgc atcaatataa 1266

ttattgggtt ttttttcttt ttcctgctgt ataactcctt gccatgcaaa 1316

ctctcaagag gccaatatat tcctggccat gtttgaatga gcctcttaaa 1366

ataaacttag agccatgcaa atgccagcag cttaatggat ttcatggaat 1416

gaaataccgt gattaactca tagctacata tcattgcata aatgggattt 1466

atcttttttc tcacttattt ttgcggtgaa agtcgagggc atgcaagagt 1516

ttctcttcca gaagccaaga ggagaacaaa ggtcctaatg ctgtactatt 1566

ccaccctttg gacgcctcat ccaggacgca gaggactcta ggtttaacat 1616

tttgtacaaa atggaacctg ttaatcatat taaagcacat atgtatatat 1666

cttttattta taaataaaat tttaaaacaa tagtttcagt atagccacaa 1716

aaa 1719

<210>10
<211>292
<212>PRT
<213>Homo sapiens

<400>10

Met Arg Thr Pro Val Val Met Thr Leu Gly Met Val Leu Ala Pro Cys
1               5                   10                  15

Gly Leu Leu Leu Asn Leu Thr Gly Thr Leu Ala Pro Gly Trp Arg Leu
            20                  25                  30

Val Lys Gly Phe Leu Asn Gln Pro Val Asp Val Glu Leu Tyr Gln Gly
        35                  40                  45

Leu Trp Asp Met Cys Arg Glu Gln Ser Ser Arg Glu Arg Glu Cys Gly

50                  55                  60

Gln Thr Asp Gln Trp Gly Tyr Phe Glu Ala Gln Pro Val Leu Val Ala
65                  70                  75                  80

Arg Ala Leu Met Val Thr Ser Leu Ala Ala Thr Val Leu Gly Leu Leu
                85                  90                  95

Leu Ala Ser Leu Gly Val Arg Cys Trp Gln Asp Glu Pro Asn Phe Val
            100                 105                 110

Leu Ala Gly Leu Ser Gly Val Val Leu Phe Val Ala Gly Leu Leu Gly
        115                 120                 125

Leu Ile Pro Val Ser Trp Tyr Asn His Phe Leu Gly Asp Arg Asp Val
    130                 135                 140

Leu Pro Ala Pro Ala Ser Pro Val Thr Val Gln Val Ser Tyr Ser Leu
145                 150                 155                 160

Val Leu Gly Tyr Leu Gly Ser Cys Leu Leu Leu Leu Gly Gly Phe Ser
                165                 170                 175

Leu Ala Leu Ser Phe Ala Pro Trp Cys Asp Glu Arg Cys Arg Arg Arg
            180                 185                 190

Arg Lys Gly Pro Ser Ala Gly Pro Arg Arg Ser Ser Val Ser Thr Ile
        195                 200                 205

Gln Val Glu Trp Pro Glu Pro Asp Leu Ala Pro Ala Ile Lys Tyr Tyr
    210                 215                 220

Ser Asp Gly Gln His Arg Pro Pro Pro Ala Gln His Arg Lys Pro Lys
225                 230                 235                 240

Pro Lys Pro Lys Val Gly Phe Pro Met Pro Arg Pro Arg Pro Lys Ala
                245                 250                 255

Tyr Thr Asn Ser Val Asp Val Leu Asp Gly Glu Gly Trp Glu Ser Gln
            260                 265                 270

Asp Ala Pro Ser Cys Ser Thr His Pro Cys Asp Ser Ser Leu Pro Cys
        275                 280                 285

Asp Ser Asp Leu
    290

<210>11
<211>2369
<212>DNA
<213>Homo sapiens

<220>
<221> CDS
<222>(25)..(1585)

<400>11

cggccggagc gccgaggccc ggcc atg gcc acc acc agc acc acg ggc tcc 51
Met Ala Thr Thr Ser Thr Thr Gly Ser
1               5

acc ctg ctg cag ccc ctc agc aac gcc gtg cag ctg ccc atc gac cag 99
Thr Leu Leu Gln Pro Leu Ser Asn Ala Val Gln Leu Pro Ile Asp Gln
10                  15                  20                  25

gtc aac ttt gta gtg tgc caa ctc ttt gcc ttg cta gca gcc att tgg 147
Val Asn Phe Val Val Cys Gln Leu Phe Ala Leu Leu Ala Ala Ile Trp
                30                  35                  40

ttt cga act tat cta cat tca agc aaa act agc tct ttt ata aga cat 195
Phe Arg Thr Tyr Leu His Ser Ser Lys Thr Ser Ser Phe Ile Arg His
            45                  50                  55

gta gtt gct acc ctt ttg ggc ctt tat ctt gca ctt ttt tgc ttt gga 243
Val Val Ala Thr Leu Leu Gly Leu Tyr Leu Ala Leu Phe Cys Phe Gly
        60                  65                  70

tgg tat gcc tta cac ttt ctt gta caa agt gga att tcc tac tgt atc 291
Trp Tyr Ala Leu His Phe Leu Val Gln Ser Gly Ile Ser Tyr Cys Ile
    75                  80                  85

atg atc atc ata gga gtg gag aac atg cac aat tac tgc ttt gtg ttt 339
Met Ile Ile Ile Gly Val Glu Asn Met His Asn Tyr Cys Phe Val Phe
90                  95                  100                 105

gct ctg gga tac ctc aca gtg tgc caa gtt act cga gtc tat atc ttt 387
Ala Leu Gly Tyr Leu Thr Val Cys Gln Val Thr Arg Val Tyr Ile Phe
                110                 115                 120

gac tat gga caa tat tct gct gat ttt tca ggc cca atg atg atc att 435
Asp Tyr Gly Gln Tyr Ser Ala Asp Phe Ser Gly Pro Met Met Ile Ile
            125                 130                 135

act cag aag atc act agt ttg gct tgc gaa att cat gat ggg atg ttt 483
Thr Gln Lys Ile Thr Ser Leu Ala Cys Glu Ile His Asp Gly Met Phe
        140                 145                 150

cgg aag gat gaa gaa ctg act tcc tca cag agg gat tta gct gta agg 531
Arg Lys Asp Glu Glu Leu Thr Ser Ser Gln Arg Asp Leu Ala Val Arg
    155                 160                 165

cgc atg cca agc tta ctg gag tat ttg agt tac aac tgt aac ttc atg 579
Arg Met Pro Ser Leu Leu Glu Tyr Leu Ser Tyr Asn Cys Asn Phe Met
170                 175                 180                 185

ggg atc ctg gca ggc cca ctt tgc tct tac aaa gac tac att act ttc 627
Gly Ile Leu Ala Gly Pro Leu Cys Ser Tyr Lys Asp Tyr Ile Thr Phe
                190                 195                 200

att gaa ggc aga tca tac cat atc aca caa tct ggt gaa aat gga aaa 675
Ile Glu Gly Arg Ser Tyr His Ile Thr Gln Ser Gly Glu Asn Gly Lys
            205                 210                 215

gaa gag aca cag tat gaa aga aca gag cca tct cca aat agt gcg gtt 723
Glu Glu Thr Gln Tyr Glu Arg Thr Glu Pro Ser Pro Asn Ser Ala Val
        220                 225                 230

gtt cag aag ctc tta gtt tgt ggg ctg tcc ttg tta ttt cac ttg acc 771
Val Gln Lys Leu Leu Val Cys Gly Leu Ser Leu Leu Phe His Leu Thr
    235                 240                 245

atc tgt aca aca tta cct gtg gag tac aac att gat gag cat ttt caa 819
Ile Cys Thr Thr Leu Pro Val Glu Tyr Asn Ile Asp Glu His Phe Gln
250                 255                 260                 265

gct aca gct tcg tgg cca aca aag att atc tat ctg tat atc tct ctt 867
Ala Thr Ala Ser Trp Pro Thr Lys Ile Ile Tyr Leu Tyr Ile Ser Leu
                270                 275                 280

ttg gct gcc aga ccc aaa tac tat ttt gca tgg acg cta gct gat gcc 915
Leu Ala Ala Arg Pro Lys Tyr Tyr Phe Ala Trp Thr Leu Ala Asp Ala
            285                 290                 295

att aat aat gct gca ggc ttt ggt ttc aga ggg tat gac gaa aat gga 963
Ile Asn Asn Ala Ala Gly Phe Gly Phe Arg Gly Tyr Asp Glu Asn Gly
        300                 305                 310

gca gct cgc tgg gac tta att tcc aat ttg aga att caa caa ata gag 1011
Ala Ala Arg Trp Asp Leu Ile Ser Asn Leu Arg Ile Gln Gln Ile Glu
    315                 320                 325

atg tca aca agt ttc aag atg ttt ctt gat aat tgg aat att cag aca 1059
Met Ser Thr Ser Phe Lys Met Phe Leu Asp Asn Trp Asn Ile Gln Thr
330                 335                 340                 345

gct ctt tgg ctc aaa agg gtg tgt tat gaa cga acc tcc ttc agt cca 1107
Ala Leu Trp Leu Lys Arg Val Cys Tyr Glu Arg Thr Ser Phe Ser Pro
                350                 355                 360

act atc cag acg ttc att ctc tct gcc att tgg cac ggg gta tac cca 1155
Thr Ile Gln Thr Phe Ile Leu Ser Ala Ile Trp His Gly Val Tyr Pro
            365                 370                 375

gga tat tat cta acg ttt cta aca ggg gtg tta atg aca tta gca gca 1203
Gly Tyr Tyr Leu Thr Phe Leu Thr Gly Val Leu Met Thr Leu Ala Ala
        380                 385                 390

aga gct gta aga aat aac ttt aga cat tat ttc att gaa cct tcc caa 1251
Arg Ala Val Arg Asn Asn Phe Arg His Tyr Phe Ile Glu Pro Ser Gln
    395                 400                 405

ctg aaa tta ttt tat gat gtt ata aca tgg ata gta act caa gta gca 1299
Leu Lys Leu Phe Tyr Asp Val Ile Thr Trp Ile Val Thr Gln Val Ala
410                 415                 420                 425

ata agt tac aca gtt gtg cca ttt gtg ctt ctt tct ata aaa cca tca 1347
Ile Ser Tyr Thr Val Val Pro Phe Val Leu Leu Ser Ile Lys Pro Ser
                430                 435                 440

ctc acg ttt tac agc tcc tgg tat tat tgc ctg cac att ctt ggt atc 1395

Leu Thr Phe Tyr Ser Ser Trp Tyr Tyr Cys Leu His Ile Leu Gly Ile
            445                 450                 455

tta gta tta ttg ttg ttg cca gta aaa aaa act caa aga aga aag aat 1443
Leu Val Leu Leu Leu Leu Pro Val Lys Lys Thr Gln Arg Arg Lys Asn
        460                 465                 470

aca cat gaa aac att cag ctc tca caa tcc aaa aag ttt gat gaa gga 1491
Thr His Glu Asn Ile Gln Leu Ser Gln Ser Lys Lys Phe Asp Glu Gly
    475                 480                 485

gaa aat tct ttg gga cag aac agt ttt tct aca aca aac aat gtt tgc 1539
Glu Asn Ser Leu Gly Gln Asn Ser Phe Ser Thr Thr Asn Asn Val Cys
490                 495                 500                 505

aat cag aat caa gaa ata gcc tcg aga cat tca tca cta aag cag 1584
Asn Gln Asn Gln Glu Ile Ala Ser Arg His Ser Ser Leu Lys Gln
                510                 515                 520

tgatcgggaa ggctctgagg gctgtttttt ttttttgatg ttaacagaaa 1634

ccaatcttag caccttttca aggggtttga gtttgttgga aaagcagtta 1684

actgggggga aatggacagt tatagataag gaatttcctg tacaccagat 1734

tggaaatgga gtgaaacaag ccctcccatg ccatgtcccc gtgggccacg 1784

ccttatgtaa gaatatttcc atatttcagt gggcactccc aacctcagca 1834

cttgtccgta gggtcacacg cgtgccctgt tgctgaatgt atgttgcgta 1884

tcccaaggca ctgaagaggt ggaaaaataa tcgtgtcaat ctggatgata 1934

gagagaaatt aacttttcca aatgaatgtc ttgccttaaa ccctctattt 1984

cctaaaatat tgttcctaaa tggtattttc aagtgtaata ttgtgagaac 2034

gctactgcag tagttgatgt tgtgtgctgt aaaggatttt aggaggaatt 2084

tgaaacagga tatttaagag tgtggatatt tttaaaatgc aataaacatc 2134

tcagtatttg aagggttttc ttaaagtatg tcaaatgact acaatccata 2184

gtgaaactgt aaacagtaat ggacgccaaa ttataggtag ctgattttgc 2234

tggagagttt aattaccttg tgcagtcaaa gagcgcttcc agaaggaatc 2284

tcttaaaaca taatgagagg tttggtaatg tgatatttta agcttactct 2334

ttttcttaaa agagagaggt gacgaaggaa ggcag 2369

<210>12
<211>520
<212>PRT
<213>Homo sapiens

<400>12

Met Ala Thr Thr Ser Thr Thr Gly Ser Thr Leu Leu Gln Pro Leu Ser
1               5                   10                  15

Asn Ala Val Gln Leu Pro Ile Asp Gln Val Asn Phe Val Val Cys Gln
            20                  25                  30

Leu Phe Ala Leu Leu Ala Ala Ile Trp Phe Arg Thr Tyr Leu His Ser
        35                  40                  45

Ser Lys Thr Ser Ser Phe Ile Arg His Val Val Ala Thr Leu Leu Gly
    50                  55                  60

Leu Tyr Leu Ala Leu Phe Cys Phe Gly Trp Tyr Ala Leu His Phe Leu
65                  70                  75                  80

Val Gln Ser Gly Ile Ser Tyr Cys Ile Met Ile Ile Ile Gly Val Glu
                85                  90                  95

Asn Met His Asn Tyr Cys Phe Val Phe Ala Leu Gly Tyr Leu Thr Val
            100                 105                 110

Cys Gln Val Thr Arg Val Tyr Ile Phe Asp Tyr Gly Gln Tyr Ser Ala
        115                 120                 125

Asp Phe Ser Gly Pro Met Met Ile Ile Thr Gln Lys Ile Thr Ser Leu
    130                 135                 140

Ala Cys Glu Ile His Asp Gly Met Phe Arg Lys Asp Glu Glu Leu Thr
145                 150                 155                 160

Ser Ser Gln Arg Asp Leu Ala Val Arg Arg Met Pro Ser Leu Leu Glu
                165                 170                 175

Tyr Leu Ser Tyr Asn Cys Asn Phe Met Gly Ile Leu Ala Gly Pro Leu
            180                 185                 190

Cys Ser Tyr Lys Asp Tyr Ile Thr Phe Ile Glu Gly Arg Ser Tyr His
        195                 200                 205

Ile Thr Gln Ser Gly Glu Asn Gly Lys Glu Glu Thr Gln Tyr Glu Arg
    210                 215                 220

Thr Glu Pro Ser Pro Asn Ser Ala Val Val Gln Lys Leu Leu Val Cys
225                 230                 235                 240

Gly Leu Ser Leu Leu Phe His Leu Thr Ile Cys Thr Thr Leu Pro Val
                245                 250                 255

Glu Tyr Asn Ile Asp Glu His Phe Gln Ala Thr Ala Ser Trp Pro Thr
            260                 265                 270

Lys Ile Ile Tyr Leu Tyr Ile Ser Leu Leu Ala Ala Arg Pro Lys Tyr
        275                 280                 285

Tyr Phe Ala Trp Thr Leu Ala Asp Ala Ile Asn Asn Ala Ala Gly Phe
    290                 295                 300

Gly Phe Arg Gly Tyr Asp Glu Asn Gly Ala Ala Arg Trp Asp Leu Ile
305                 310                 315                 320

Ser Asn Leu Arg Ile Gln Gln Ile Glu Met Ser Thr Ser Phe Lys Met
                325                 330                 335

Phe Leu Asp Asn Trp Asn Ile Gln Thr Ala Leu Trp Leu Lys Arg Val
            340                 345                 350

Cys Tyr Glu Arg Thr Ser Phe Ser Pro Thr Ile Gln Thr Phe Ile Leu
        355                 360                 365

Ser Ala Ile Trp His Gly Val Tyr Pro Gly Tyr Tyr Leu Thr Phe Leu
    370                 375                 380

Thr Gly Val Leu Met Thr Leu Ala Ala Arg Ala Val Arg Asn Asn Phe
385                 390                 395                 400

Arg His Tyr Phe Ile Glu Pro Ser Gln Leu Lys Leu Phe Tyr Asp Val
                405                 410                 415

Ile Thr Trp Ile Val Thr Gln Val Ala Ile Ser Tyr Thr Val Val Pro
            420                 425                 430

Phe Val Leu Leu Ser Ile Lys Pro Ser Leu Thr Phe Tyr Ser Ser Trp
        435                 440                 445

Tyr Tyr Cys Leu His Ile Leu Gly Ile Leu Val Leu Leu Leu Leu Pro
    450                 455                 460

Val Lys Lys Thr Gln Arg Arg Lys Asn Thr His Glu Asn Ile Gln Leu
465                 470                 475                 480

Ser Gln Ser Lys Lys Phe Asp Glu Gly Glu Asn Ser Leu Gly Gln Asn
                485                 490                 495

Ser Phe Ser Thr Thr Asn Asn Val Cys Asn Gln Asn Gln Glu Ile Ala
            500                 505                 510

Ser Arg His Ser Ser Leu Lys Gln
        515                 520

<210>13
<211>2270
<212>DNA
<213>Homo sapiens

<220>
<221> CDS
<222>(25)..(1486)

<400>13

cggccggagc gccgaggccc ggcc atg gcc acc acc agc acc acg ggc tcc      51
                           Met Ala Thr Thr Ser Thr Thr Gly Ser
                            1               5

acc ctg ctg cag ccc ctc agc aac gcc gtg cag ctg ccc atc gac cag      99
Thr Leu Leu Gln Pro Leu Ser Asn Ala Val Gln Leu Pro Ile Asp Gln
 10                  15                  20                  25

gtc aac ttt gta gtg tgc caa ctc ttt gcc ttg cta gca gcc att tgg     147
Val Asn Phe Val Val Cys Gln Leu Phe Ala Leu Leu Ala Ala Ile Trp
                 30                  35                  40

ttt cga act tat cta cat tca agc aaa act agc tct ttt ata aga cat     195
Phe Arg Thr Tyr Leu His Ser Ser Lys Thr Ser Ser Phe Ile Arg His
             45                  50                  55

gta gtt gct acc ctt ttg ggc ctt tat ctt gca ctt ttt tgc ttt gga     243
Val Val Ala Thr Leu Leu Gly Leu Tyr Leu Ala Leu Phe Cys Phe Gly
         60                  65                  70

tgg tat gcc tta cac ttt ctt gta caa agt gga att tcc tac tgt atc     291
Trp Tyr Ala Leu His Phe Leu Val Gln Ser Gly Ile Ser Tyr Cys Ile
     75                  80                  85

atg atc atc ata gga gtg gag aac atg cag cca atg atg atc att act     339
Met Ile Ile Ile Gly Val Glu Asn Met Gln Pro Met Met Ile Ile Thr
 90                  95                 100                 105

cag aag atc act agt ttg gct tgc gaa att cat gat ggg atg ttt cgg 387
Gln Lys Ile Thr Ser Leu Ala Cys Glu Ile His Asp Gly Met Phe Arg
                110                 115                 120

aag gat gaa gaa ctg act tcc tca cag agg gat tta gct gta agg cgc 435
Lys Asp Glu Glu Leu Thr Ser Ser Gln Arg Asp Leu Ala Val Arg Arg
            125                 130                 135

atg cca agc tta ctg gag tat ttg agt tac aac tgt aac ttc atg ggg 483
Met Pro Ser Leu Leu Glu Tyr Leu Ser Tyr Asn Cys Asn Phe Met Gly
        140                 145                 150

atc ctg gca ggc cca ctt tgc tct tac aaa gac tac att act ttc att 531
Ile Leu Ala Gly Pro Leu Cys Ser Tyr Lys Asp Tyr Ile Thr Phe Ile
    155                 160                 165

gaa ggc aga tca tac cat atc aca caa tct ggt gaa aat gga aaa gaa 579
Glu Gly Arg Ser Tyr His Ile Thr Gln Ser Gly Glu Asn Gly Lys Glu
170                 175                 180                 185

gag aca cag tat gaa aga aca gag cca tct cca aat agt gcg gtt gtt 627
Glu Thr Gln Tyr Glu Arg Thr Glu Pro Ser Pro Asn Ser Ala Val Val
                190                 195                 200

cag aag ctc tta gtt tgt ggg ctg tcc ttg tta ttt cac ttg acc atc 675
Gln Lys Leu Leu Val Cys Gly Leu Ser Leu Leu Phe His Leu Thr Ile
            205                 210                 215

tgt aca aca tta cct gtg gag tac aac att gat gag cat ttt caa gct 723
Cys Thr Thr Leu Pro Val Glu Tyr Asn Ile Asp Glu His Phe Gln Ala
        220                 225                 230

aca gct tcg tgg cca aca aag att atc tat ctg tat atc tct ctt ttg 771
Thr Ala Ser Trp Pro Thr Lys Ile Ile Tyr Leu Tyr Ile Ser Leu Leu
    235                 240                 245

gct gcc aga ccc aaa tac tat ttt gca tgg acg cta gct gat gcc att 819
Ala Ala Arg Pro Lys Tyr Tyr Phe Ala Trp Thr Leu Ala Asp Ala Ile
250                 255                 260                 265

aat aat gct gca ggc ttt ggt ttc aga ggg tat gac gaa aat gga gca 867
Asn Asn Ala Ala Gly Phe Gly Phe Arg Gly Tyr Asp Glu Asn Gly Ala
                270                 275                 280

gct cgc tgg gac tta att tcc aat ttg aga att caa caa ata gag atg 915
Ala Arg Trp Asp Leu Ile Ser Asn Leu Arg Ile Gln Gln Ile Glu Met
            285                 290                 295

tca aca agt ttc aag atg ttt ctt gat aat tgg aat att cag aca gct 963
Ser Thr Ser Phe Lys Met Phe Leu Asp Asn Trp Asn Ile Gln Thr Ala
        300                 305                 310

ctt tgg ctc aaa agg gtg tgt tat gaa cga acc tcc ttc agt cca act 1011
Leu Trp Leu Lys Arg Val Cys Tyr Glu Arg Thr Ser Phe Ser Pro Thr
    315                 320                 325

atc cag acg ttc att ctc tct gcc att tgg cac ggg gta tac cca gga 1059

Ile Gln Thr Phe Ile Leu Ser Ala Ile Trp His Gly Val Tyr Pro Gly
330                 335                 340                 345

tat tat cta acg ttt cta aca ggg gtg tta atg aca tta gca gca aga     1107
Tyr Tyr Leu Thr Phe Leu Thr Gly Val Leu Met Thr Leu Ala Ala Arg
                350                 355                 360

gct gta aga aat aac ttt aga cat tat ttc att gaa cct tcc caa ctg     1155
Ala Val Arg Asn Asn Phe Arg His Tyr Phe Ile Glu Pro Ser Gln Leu
            365                 370                 375

aaa tta ttt tat gat gtt ata aca tgg ata gta act caa gta gca ata     1203
Lys Leu Phe Tyr Asp Val Ile Thr Trp Ile Val Thr Gln Val Ala Ile
        380                 385                 390

agt tac aca gtt gtg cca ttt gtg ctt ctt tct ata aaa cca tca ctc     1251
Ser Tyr Thr Val Val Pro Phe Val Leu Leu Ser Ile Lys Pro Ser Leu
    395                 400                 405

acg ttt tac agc tcc tgg tat tat tgc ctg cac att ctt ggt atc tta     1299
Thr Phe Tyr Ser Ser Trp Tyr Tyr Cys Leu His Ile Leu Gly Ile Leu
410                 415                 420                 425

gta tta ttg ttg ttg cca gta aaa aaa act caa aga aga aag aat aca     1347
Val Leu Leu Leu Leu Pro Val Lys Lys Thr Gln Arg Arg Lys Asn Thr
                430                 435                 440

cat gaa aac att cag ctc tca caa tcc aaa aag ttt gat gaa gga gaa     1395
His Glu Asn Ile Gln Leu Ser Gln Ser Lys Lys Phe Asp Glu Gly Glu
            445                 450                 455

aat tct ttg gga cag aac agt ttt tct aca aca aac aat gtt tgc aat     1443
Asn Ser Leu Gly Gln Asn Ser Phe Ser Thr Thr Asn Asn Val Cys Asn
        460                 465                 470

cag aat caa gaa ata gcc tcg aga cat tca tca cta aag cag             1485
Gln Asn Gln Glu Ile Ala Ser Arg His Ser Ser Leu Lys Gln
    475                 480                 485

tgatcgggaa ggctctgagg gctgtttttt ttttttgatg ttaacagaaa               1535

ccaatcttag caccttttca aggggtttga gtttgttgga aaagcagtta              1585

actgggggga aatggacagt tatagataag gaatttcctg tacaccagat              1635

tggaaatgga gtgaaacaag ccctcccatg ccatgtcccc gtgggccacg              1685

ccttatgtaa gaatatttcc atatttcagt gggcactccc aacctcagca              1735

cttgtccgta gggtcacacg cgtgccctgt tgctgaatgt atgttgcgta 1785

tcccaaggca ctgaagaggt ggaaaaataa tcgtgtcaat ctggatgata 1835

gagagaaatt aacttttcca aatgaatgtc ttgccttaaa ccctctattt 1885

cctaaaatat tgttcctaaa tggtattttc aagtgtaata ttgtgagaac 1935

gctactgcag tagttgatgt tgtgtgctgt aaaggatttt aggaggaatt 1985

tgaaacagga tatttaagag tgtggatatt tttaaaatgc aataaacatc 2035

tcagtatttg aagggttttc ttaaagtatg tcaaatgact acaatccata 2085

gtgaaactgt aaacagtaat ggacgccaaa ttataggtag ctgattttgc 2135

tggagagttt aattaccttg tgcagtcaaa gagcgcttcc agaaggaatc 2185

tcttaaaaca taatgagagg tttggtaatg tgatatttta agcttactct 2235

ttttcttaaa agagagaggt gacgaaggaa ggcag 2270

<210>14
<211>487
<212>PRT
<213>Homo sapiens

<400>14

Met Ala Thr Thr Ser Thr Thr Gly Ser Thr Leu Leu Gln Pro Leu Ser
1               5                   10                  15

Asn Ala Val Gln Leu Pro Ile Asp Gln Val Asn Phe Val Val Cys Gln

20                  25                  30

Leu Phe Ala Leu Leu Ala Ala Ile Trp Phe Arg Thr Tyr Leu His Ser
        35                  40                  45

Ser Lys Thr Ser Ser Phe Ile Arg His Val Val Ala Thr Leu Leu Gly
    50                  55                  60

Leu Tyr Leu Ala Leu Phe Cys Phe Gly Trp Tyr Ala Leu His Phe Leu
65                  70                  75                  80

Val Gln Ser Gly Ile Ser Tyr Cys Ile Met Ile Ile Ile Gly Val Glu
                85                  90                  95

Asn Met Gln Pro Met Met Ile Ile Thr Gln Lys Ile Thr Ser Leu Ala
            100                 105                 110

Cys Glu Ile His Asp Gly Met Phe Arg Lys Asp Glu Glu Leu Thr Ser
        115                 120                 125

Ser Gln Arg Asp Leu Ala Val Arg Arg Met Pro Ser Leu Leu Glu Tyr
    130                 135                 140

Leu Ser Tyr Asn Cys Asn Phe Met Gly Ile Leu Ala Gly Pro Leu Cys
145                 150                 155                 160

Ser Tyr Lys Asp Tyr Ile Thr Phe Ile Glu Gly Arg Ser Tyr His Ile
                165                 170                 175

Thr Gln Ser Gly Glu Asn Gly Lys Glu Glu Thr Gln Tyr Glu Arg Thr
            180                 185                 190

Glu Pro Ser Pro Asn Ser Ala Val Val Gln Lys Leu Leu Val Cys Gly
        195                 200                 205

Leu Ser Leu Leu Phe His Leu Thr Ile Cys Thr Thr Leu Pro Val Glu
    210                 215                 220

Tyr Asn Ile Asp Glu His Phe Gln Ala Thr Ala Ser Trp Pro Thr Lys
225                 230                 235                 240

Ile Ile Tyr Leu Tyr Ile Ser Leu Leu Ala Ala Arg Pro Lys Tyr Tyr
                245                 250                 255

Phe Ala Trp Thr Leu Ala Asp Ala Ile Asn Asn Ala Ala Gly Phe Gly
            260                 265                 270

Phe Arg Gly Tyr Asp Glu Asn Gly Ala Ala Arg Trp Asp Leu Ile Ser
        275                 280                 285

Asn Leu Arg Ile Gln Gln Ile Glu Met Ser Thr Ser Phe Lys Met Phe
    290                 295                 300

Leu Asp Asn Trp Asn Ile Gln Thr Ala Leu Trp Leu Lys Arg Val Cys
305                 310                 315                 320

Tyr Glu Arg Thr Ser Phe Ser Pro Thr Ile Gln Thr Phe Ile Leu Ser

325                    330                    335

Ala Ile Trp His Gly Val Tyr Pro Gly Tyr Tyr Leu Thr Phe Leu Thr
            340                    345                    350

Gly Val Leu Met Thr Leu Ala Ala Arg Ala Val Arg Asn Asn Phe Arg
        355                    360                    365

His Tyr Phe Ile Glu Pro Ser Gln Leu Lys Leu Phe Tyr Asp Val Ile
    370                    375                    380

Thr Trp Ile Val Thr Gln Val Ala Ile Ser Tyr Thr Val Val Pro Phe
385                    390                    395                    400

Val Leu Leu Ser Ile Lys Pro Ser Leu Thr Phe Tyr Ser Ser Trp Tyr
                405                    410                    415

Tyr Cys Leu His Ile Leu Gly Ile Leu Val Leu Leu Leu Leu Pro Val
            420                    425                    430

Lys Lys Thr Gln Arg Arg Lys Asn Thr His Glu Asn Ile Gln Leu Ser
        435                    440                    445

Gln Ser Lys Lys Phe Asp Glu Gly Glu Asn Ser Leu Gly Gln Asn Ser
    450                    455                    460

Phe Ser Thr Thr Asn Asn Val Cys Asn Gln Asn Gln Glu Ile Ala Ser
465                    470                    475                    480

Arg His Ser Ser Leu Lys Gln
                485

<210>15
<211>537
<212>DNA
<213>Homo sapiens

<220>
<221> CDS
<222>(201)..(480)

<400>15

attagcaacg gctcatgatg aactcaatca aagggggctt gaccagcatc          50

tcaggtctac tctatgtttt ccagtgccca tcagtgccaa gggtattgat          100

tagcctatcc gggacaaaga gagaagaaag agtgagacac caccactaaa          150

agggctgcag gtggataccg cctccctcaa gctggaaaaa gattagaaag atg gtg     206
                                                       Met Val

                                                       1

aaa aca gga aga cct tcc tca tcc cac tat cag gaa gat gag gaa aga 254
Lys Thr Gly Arg Pro Ser Ser Ser His Tyr Gln Glu Asp Glu Glu Arg
        5                   10                  15

gat cag gag gat cac agg tgg aga gga gaa gag gac cat gct cga tcc 302
Asp Gln Glu Asp His Arg Trp Arg Gly Glu Glu Asp His Ala Arg Ser
    20                  25                  30

tct ctg gta ata ggc ctg aga ttc cct ctc gta ctg ggt gat aca cat 350
Ser Leu Val Ile Gly Leu Arg Phe Pro Leu Val Leu Gly Asp Thr His
35                  40                  45                  50

ctg ctc cca gtg ttc cat cct cca ggc ttc ggg cgc ttc ttg cag agg 398
Leu Leu Pro Val Phe His Pro Pro Gly Phe Gly Arg Phe Leu Gln Arg
                55                  60                  65

ccc agg tca ctc cat gtg gcc aca aag aga acc agc atc cag cag cca 446
Pro Arg Ser Leu His Val Ala Thr Lys Arg Thr Ser Ile Gln Gln Pro
            70                  75                  80

agg ttc gcc ata atg act gct ctg cct cgg tcg tgaggagagg 489
Arg Phe Ala Ile Met Thr Ala Leu Pro Arg Ser
        85                  90

agaagctcgc ggcgccgcgg ctgtcagcga ctggctcgga ggacaggc 537

<210>16
<211>93
<212>PRT
<213>Homo sapiens

<400>16

Met Val Lys Thr Gly Arg Pro Ser Ser Ser His Tyr Gln Glu Asp Glu
1               5                   10                  15

Glu Arg Asp Gln Glu Asp His Arg Trp Arg Gly Glu Glu Asp His Ala
            20                  25                  30

Arg Ser Ser Leu Val Ile Gly Leu Arg Phe Pro Leu Val Leu Gly Asp
        35                  40                  45

Thr His Leu Leu Pro Val Phe His Pro Pro Gly Phe Gly Arg Phe Leu
    50                  55                  60

Gln Arg Pro Arg Ser Leu His Val Ala Thr Lys Arg Thr Ser Ile Gln
65                  70                  75                  80

Gln Pro Arg Phe Ala Ile Met Thr Ala Leu Pro Arg Ser
                85                  90

<210>17
<211>3653

<212>DNA
<213>Homo sapiens

<220>
<221> CDS
<222>(5)..(3647)

<400>17

cggg atg ccc ggc ttg ctg aat tgg atc acg ggg gca gcc ctg ccc 46
Met Pro Gly Leu Leu Asn Trp Ile Thr Gly Ala Ala Leu Pro
1               5                   10

ctc acc gcg tct gat gtt acc tcc tgt gtc agc ggt tat gcc ctg ggc 94
Leu Thr Ala Ser Asp Val Thr Ser Cys Val Ser Gly Tyr Ala Leu Gly
15                  20                  25                  30

cta act gcc tcc ctc acc tat ggc aac ctg gaa gcc cag ccc ttc cag 142
Leu Thr Ala Ser Leu Thr Tyr Gly Asn Leu Glu Ala Gln Pro Phe Gln
                35                  40                  45

ggt ctc ttc gtg tac ccc ctg gat gag tgc acc acg gtg atc ggc ttt 190
Gly Leu Phe Val Tyr Pro Leu Asp Glu Cys Thr Thr Val Ile Gly Phe
            50                  55                  60

gag gca gtc att gcc gac cgt gtc gtg aca gta cag atc aag gac aaa 238
Glu Ala Val Ile Ala Asp Arg Val Val Thr Val Gln Ile Lys Asp Lys
        65                  70                  75

gcc aag ctg gag agc ggc cac ttc gat gcc tcc cat gtt cga tcc cca 286
Ala Lys Leu Glu Ser Gly His Phe Asp Ala Ser His Val Arg Ser Pro
    80                  85                  90

aca gtc aca ggt aag gag acc aga agg gct gcc gcg gga cct ggg aag 334
Thr Val Thr Gly Lys Glu Thr Arg Arg Ala Ala Ala Gly Pro Gly Lys
95                  100                 105                 110

gtg acc ttg gac gag gat ttg gag cgg atc ctg ttc gtg gcc aac ctg 382
Val Thr Leu Asp Glu Asp Leu Glu Arg Ile Leu Phe Val Ala Asn Leu
                115                 120                 125

ggg acc att gcc ccc atg gag aat gtc acc atc ttc atc agc acc tcc 430
Gly Thr Ile Ala Pro Met Glu Asn Val Thr Ile Phe Ile Ser Thr Ser
            130                 135                 140

tcg gag ctc cca acg ctg ccc agc ggg gct gtg agg gtc ctt ctg cct 478
Ser Glu Leu Pro Thr Leu Pro Ser Gly Ala Val Arg Val Leu Leu Pro
        145                 150                 155

gct gtc tgt gcc cca acc gtg ccc cag ttc tgc acc aag agc act ggc 526
Ala Val Cys Ala Pro Thr Val Pro Gln Phe Cys Thr Lys Ser Thr Gly
    160                 165                 170

acc tcc aac caa cag gcc cag ggg aaa gac agg cac tgc ttc ggt gcc 574
Thr Ser Asn Gln Gln Ala Gln Gly Lys Asp Arg His Cys Phe Gly Ala
175                 180                 185                 190

tgg gcc ccg ggc tcc tgg aat aag ttg tgc ctg gcg act ctc ctg aac 622
Trp Ala Pro Gly Ser Trp Asn Lys Leu Cys Leu Ala Thr Leu Leu Asn
                195                 200                 205

acc gaa gtg tcc aac ccc atg gag tat gag ttc aac ttc cag ctg gag 670
Thr Glu Val Ser Asn Pro Met Glu Tyr Glu Phe Asn Phe Gln Leu Glu
            210                 215                 220

atc cgt ggg cca tgt ctg ctc gca ggt gtg gag agt ccc act cat gag 718
Ile Arg Gly Pro Cys Leu Leu Ala Gly Val Glu Ser Pro Thr His Glu
        225                 230                 235

att cgt gcc gac gcc gcc cca tct gcc cgc tcg gcc aag agc atc atc 766
Ile Arg Ala Asp Ala Ala Pro Ser Ala Arg Ser Ala Lys Ser Ile Ile
    240                 245                 250

atc acc ttg gcc aac aag cac acc ttt gac cgg cct gtg gag atc ctc 814
Ile Thr Leu Ala Asn Lys His Thr Phe Asp Arg Pro Val Glu Ile Leu
255                 260                 265                 270

atc cac ccc agc gag ccc cat atg ccc cat gtc ctg ata gag aaa ggg 862
Ile His Pro Ser Glu Pro His Met Pro His Val Leu Ile Glu Lys Gly
                275                 280                 285

gac atg acc ctg gga gag ttt gac cag cac ttg aag gga aga aca gat 910
Asp Met Thr Leu Gly Glu Phe Asp Gln His Leu Lys Gly Arg Thr Asp
            290                 295                 300

ttc att aaa ggg atg aag aag aag agc aga gca gag cgg aag aca gaa 958
Phe Ile Lys Gly Met Lys Lys Lys Ser Arg Ala Glu Arg Lys Thr Glu
        305                 310                 315

atc att cga aaa cgc ctc cac aaa gac att ccc cac cac tcc gtc atc 1006
Ile Ile Arg Lys Arg Leu His Lys Asp Ile Pro His His Ser Val Ile
    320                 325                 330

atg ctc aac ttc tgt ccc gac ctc cag tca gtc cag ccg tgc ctg aga 1054
Met Leu Asn Phe Cys Pro Asp Leu Gln Ser Val Gln Pro Cys Leu Arg
335                 340                 345                 350

aag gcc cac ggg gag ttc atc ttc ctc att gac agg agc agc agc atg 1102
Lys Ala His Gly Glu Phe Ile Phe Leu Ile Asp Arg Ser Ser Ser Met
                355                 360                 365

agc ggg atc agc atg cac cga gtc aag gat gcc atg ttg gtg gcc ctt 1150
Ser Gly Ile Ser Met His Arg Val Lys Asp Ala Met Leu Val Ala Leu
            370                 375                 380

aag agc ctc atg cca gcc tgc ctc ttc aat atc att ggg ttt gga tcc 1198
Lys Ser Leu Met Pro Ala Cys Leu Phe Asn Ile Ile Gly Phe Gly Ser
        385                 390                 395

aca ttt aag agc ctt ttt cct tcc agc cag acc tac agt gag gac agc 1246
Thr Phe Lys Ser Leu Phe Pro Ser Ser Gln Thr Tyr Ser Glu Asp Ser
    400                 405                 410

ttg gcc atg gct tgt gat gac atc cag aga atg aag gcc gac atg ggt 1294

Leu Ala Met Ala Cys Asp Asp Ile Gln Arg Met Lys Ala Asp Met Gly
415                 420                 425                 430

ggg acc aac atc ctt tcc cct ctc aag tgg gtc atc agg cag cca gtg     1342
Gly Thr Asn Ile Leu Ser Pro Leu Lys Trp Val Ile Arg Gln Pro Val
                435                 440                 445

cac cga ggc cac ccg cgg ctc ctc ttc gtg atc aca gat ggc gct gtc     1390
His Arg Gly His Pro Arg Leu Leu Phe Val Ile Thr Asp Gly Ala Val
            450                 455                 460

aac aac aca ggg aag gtg ctg gag ctg gtg cga aat cac gcc ttc tcc     1438
Asn Asn Thr Gly Lys Val Leu Glu Leu Val Arg Asn His Ala Phe Ser
        465                 470                 475

acc agg tgc tat agc ttt gga att gga ccc aac gtc tgc cac aga ctg     1486
Thr Arg Cys Tyr Ser Phe Gly Ile Gly Pro Asn Val Cys His Arg Leu
    480                 485                 490

gtg aaa gga ctg gca tct gtg tcc gag ggc agt gct gag ctc ctg atg     1534
Val Lys Gly Leu Ala Ser Val Ser Glu Gly Ser Ala Glu Leu Leu Met
495                 500                 505                 510

gag ggg gag cgg ctg caa ccc aag atg gtc aaa tcc ttg aag aag gcc     1582
Glu Gly Glu Arg Leu Gln Pro Lys Met Val Lys Ser Leu Lys Lys Ala
                515                 520                 525

atg gcc cca gtc ctg agc gat gtg act gtg gag tgg atc ttc cct gag     1630
Met Ala Pro Val Leu Ser Asp Val Thr Val Glu Trp Ile Phe Pro Glu
            530                 535                 540

acc act gag gtc ctg gtc tca ccc gtc agc gcc agc tcc ctc ttc cct     1678
Thr Thr Glu Val Leu Val Ser Pro Val Ser Ala Ser Ser Leu Phe Pro
        545                 550                 555

gga gaa cgg ctg gtg ggg tat ggc att gta tgt gat gct tct ttg cac     1726
Gly Glu Arg Leu Val Gly Tyr Gly Ile Val Cys Asp Ala Ser Leu His
    560                 565                 570

atc tcc aat ccc aga tct gac aag agg cgc cgg tac agc atg ctg cac     1774
Ile Ser Asn Pro Arg Ser Asp Lys Arg Arg Arg Tyr Ser Met Leu His
575                 580                 585                 590

tct cag gag tct ggc agc tct gtc ttc tac cac tct cag gat gac gga     1822
Ser Gln Glu Ser Gly Ser Ser Val Phe Tyr His Ser Gln Asp Asp Gly
                595                 600                 605

ccc ggg ctg gaa ggt gga gac tgt gcc aag aac tcg ggg gca ccc ttc     1870
Pro Gly Leu Glu Gly Gly Asp Cys Ala Lys Asn Ser Gly Ala Pro Phe
            610                 615                 620

atc cta ggg cag gcc aaa aat gcc cgg cta gcc agc gga gac tct acc     1918
Ile Leu Gly Gln Ala Lys Asn Ala Arg Leu Ala Ser Gly Asp Ser Thr
        625                 630                 635

acc aag cac ggt ctg aac ctc tct cag cga cgg agg gca tac agc acc     1966
Thr Lys His Gly Leu Asn Leu Ser Gln Arg Arg Arg Ala Tyr Ser Thr

640                    645                    650

aac cag atc acc aat cac aag ccc ctc cca aga gcc acc atg gca agt    2014
Asn Gln Ile Thr Asn His Lys Pro Leu Pro Arg Ala Thr Met Ala Ser
655                 660                 665                 670

gac ccc atg cca gct gcc aag aga tac cca ctg cgg aaa gcc agg ctg    2062
Asp Pro Met Pro Ala Ala Lys Arg Tyr Pro Leu Arg Lys Ala Arg Leu
                675                 680                 685

cag gac ctc acc aac cag acc agc ctg gat gtc cag cgg tgg cag att    2110
Gln Asp Leu Thr Asn Gln Thr Ser Leu Asp Val Gln Arg Trp Gln Ile
            690                 695                 700

gat ttg cag gta ttg ctg aac agt ggt cag gac ctg aac cag ggc ccc    2158
Asp Leu Gln Val Leu Leu Asn Ser Gly Gln Asp Leu Asn Gln Gly Pro
        705                 710                 715

aaa ctc cgt ggc cca ggg gcc cga agg ccc tct ctg ctg ccc caa ggc    2206
Lys Leu Arg Gly Pro Gly Ala Arg Arg Pro Ser Leu Leu Pro Gln Gly
    720                 725                 730

tgc cag ccc ttc ctg ccc tgg ggc cag gag acc cag gcc tgg agc cct    2254
Cys Gln Pro Phe Leu Pro Trp Gly Gln Glu Thr Gln Ala Trp Ser Pro
735                 740                 745                 750

gtg aga gag cgg act tct gac agc cga agc cct gga gat ctg ccc gca    2302
Val Arg Glu Arg Thr Ser Asp Ser Arg Ser Pro Gly Asp Leu Pro Ala
                755                 760                 765

gag ccg tcc cac cat ccc tct gcc ttc gag aca gag acg tcc tcg gac    2350
Glu Pro Ser His His Pro Ser Ala Phe Glu Thr Glu Thr Ser Ser Asp
            770                 775                 780

tgg gac ccc cca gcc gag tcc cag gag cga gcc agt ccc agc agg ccc    2398
Trp Asp Pro Pro Ala Glu Ser Gln Glu Arg Ala Ser Pro Ser Arg Pro
        785                 790                 795

gcc acc ccg gcc ccg gtg ctg ggc aag gcc ctg gtc aaa ggc ctg cac    2446
Ala Thr Pro Ala Pro Val Leu Gly Lys Ala Leu Val Lys Gly Leu His
    800                 805                 810

gac agc caa cgc ctg cag tgg gag gtg agc ttc gag ctg ggg acc cct    2494
Asp Ser Gln Arg Leu Gln Trp Glu Val Ser Phe Glu Leu Gly Thr Pro
815                 820                 825                 830

gga ccg gag cgg ggc ggc gcg cag gat gcc gac cta tgg agc gag acc    2542
Gly Pro Glu Arg Gly Gly Ala Gln Asp Ala Asp Leu Trp Ser Glu Thr
                835                 840                 845

ttc cac cac ctg gcg gcc cgc gcc atc atc cgc gac ttc gag cag ctg    2590
Phe His His Leu Ala Ala Arg Ala Ile Ile Arg Asp Phe Glu Gln Leu
            850                 855                 860

gcg gag cgc gag ggc gag atc gag cag ggt tcc aac cgc cgc tac caa    2638
Ala Glu Arg Glu Gly Glu Ile Glu Gln Gly Ser Asn Arg Arg Tyr Gln
        865                 870                 875

gtg agc gcc ttg cac acc agc aag gcc tgc aac atc att agc aaa tac 2686
Val Ser Ala Leu His Thr Ser Lys Ala Cys Asn Ile Ile Ser Lys Tyr
    880                 885                 890

aca gcc ttc gtg cct gtg gac gtg agc aag agc cgg tac ctg ccc acc 2734
Thr Ala Phe Val Pro Val Asp Val Ser Lys Ser Arg Tyr Leu Pro Thr
895                 900                 905                 910

gtg gtg gag tac ccc aac tct ggt cgt atg ctt ggc tct cgg gcc ctg 2782
Val Val Glu Tyr Pro Asn Ser Gly Arg Met Leu Gly Ser Arg Ala Leu
                915                 920                 925

gcc caa cag tgg agg ggc acc tct tct ggc ttt gga agg ccg cag acg 2830
Ala Gln Gln Trp Arg Gly Thr Ser Ser Gly Phe Gly Arg Pro Gln Thr
            930                 935                 940

atg ctt gga gaa gat tcg gca cca gga aat ggt aaa ttt cag gtc cta 2878
Met Leu Gly Glu Asp Ser Ala Pro Gly Asn Gly Lys Phe Gln Val Leu
        945                 950                 955

gac atg gag gca agt ccc act gct ctc ttc agc gag gcc agg tcc ccc 2926
Asp Met Glu Ala Ser Pro Thr Ala Leu Phe Ser Glu Ala Arg Ser Pro
    960                 965                 970

ggc cgc gag aag cac ggt gct tct gaa ggt ccc cag cgc agc ctg gct 2974
Gly Arg Glu Lys His Gly Ala Ser Glu Gly Pro Gln Arg Ser Leu Ala
975                 980                 985                 990

aca aat act ctt tct tcc atg aag gcc tca gag aat ctc ttt gga tcc 3022
Thr Asn Thr Leu Ser Ser Met Lys Ala Ser Glu Asn Leu Phe Gly Ser
                995                 1000                1005

agg cta aat ctc aac aag tcc agg cta ctg acg cga gca gcc aag ggc 3070
Arg Leu Asn Leu Asn Lys Ser Arg Leu Leu Thr Arg Ala Ala Lys Gly
            1010                1015                1020

ttc ctg agc aag cca ctg atc aaa gct gtg gag tcg acc tcc ggg aac 3118
Phe Leu Ser Lys Pro Leu Ile Lys Ala Val Glu Ser Thr Ser Gly Asn
        1025                1030                1035

cag agc ttc gac tac ata cct ctg gtg tct ctg cag ctg gcc tcc gga 3166
Gln Ser Phe Asp Tyr Ile Pro Leu Val Ser Leu Gln Leu Ala Ser Gly
    1040                1045                1050

gcc ttc ctg ctc aac gaa gcc ttc tgt gag gcc acg cac atc ccc atg 3214
Ala Phe Leu Leu Asn Glu Ala Phe Cys Glu Ala Thr His Ile Pro Met
1055                1060                1065                1070

gag aag ctc aag tgg acg tcc ccc ttc acc tgc cat cga gtg tcc ctc 3262
Glu Lys Leu Lys Trp Thr Ser Pro Phe Thr Cys His Arg Val Ser Leu
                1075                1080                1085

acc acc cgc ccg tct gag tcc aag acc ccg agt ccc cag ctg tgc acc 3310
Thr Thr Arg Pro Ser Glu Ser Lys Thr Pro Ser Pro Gln Leu Cys Thr
            1090                1095                1100

agc tcc ccg cct agg cac ccg tcc tgt gac agc ttc tcc ctg gag cct 3358
Ser Ser Pro Pro Arg His Pro Ser Cys Asp Ser Phe Ser Leu Glu Pro
        1105                1110                1115

ctg gcc aag ggc aag ctg ggc ctg gag ccg agg gca gtg gtg gag cac 3406
Leu Ala Lys Gly Lys Leu Gly Leu Glu Pro Arg Ala Val Val Glu His
    1120                1125                1130

act ggg aag ctg tgg gcc acg gtg gtg ggg ctg gca tgg ctg gag cac 3454
Thr Gly Lys Leu Trp Ala Thr Val Val Gly Leu Ala Trp Leu Glu His
1135                1140                1145                1150

agt tcg gcc tcc tac ttc act gag tgg gag ttg gtg gct gcc aag gcc 3502
Ser Ser Ala Ser Tyr Phe Thr Glu Trp Glu Leu Val Ala Ala Lys Ala
                1155                1160                1165

aac tca tgg ctg gag cag cag gaa gta ccc gag ggc cgc acg cag ggc 3550
Asn Ser Trp Leu Glu Gln Gln Glu Val Pro Glu Gly Arg Thr Gln Gly
            1170                1175                1180

aca ctc aag gcc gct gcc cgc cag ctg ttt gtg ctt ctg cgg cac tgg 3598
Thr Leu Lys Ala Ala Ala Arg Gln Leu Phe Val Leu Leu Arg His Trp
        1185                1190                1195

gat gag aat ctc gag ttc aat atg ctc tgc tat aac ccg aat tat gtg 3646
Asp Glu Asn Leu Glu Phe Asn Met Leu Cys Tyr Asn Pro Asn Tyr Val
    1200                1205                1210

tagttga 3653

<210>18
<211>1214
<212>PRT
<213>Homo sapiens

<400>18

Met Pro Gly Leu Leu Asn Trp Ile Thr Gly Ala Ala Leu Pro Leu Thr
1               5                   10                  15

Ala Ser Asp Val Thr Ser Cys Val Ser Gly Tyr Ala Leu Gly Leu Thr
            20                  25                  30

Ala Ser Leu Thr Tyr Gly Asn Leu Glu Ala Gln Pro Phe Gln Gly Leu
        35                  40                  45

Phe Val Tyr Pro Leu Asp Glu Cys Thr Thr Val Ile Gly Phe Glu Ala
    50                  55                  60

Val Ile Ala Asp Arg Val Val Thr Val Gln Ile Lys Asp Lys Ala Lys
65                  70                  75                  80

Leu Glu Ser Gly His Phe Asp Ala Ser His Val Arg Ser Pro Thr Val
                85                  90                  95

Thr Gly Lys Glu Thr Arg Arg Ala Ala Ala Gly Pro Gly Lys Val Thr
            100                 105                 110

Leu Asp Glu Asp Leu Glu Arg Ile Leu Phe Val Ala Asn Leu Gly Thr
        115                 120                 125

Ile Ala Pro Met Glu Asn Val Thr Ile Phe Ile Ser Thr Ser Ser Glu
    130                 135                 140

Leu Pro Thr Leu Pro Ser Gly Ala Val Arg Val Leu Leu Pro Ala Val
145                 150                 155                 160

Cys Ala Pro Thr Val Pro Gln Phe Cys Thr Lys Ser Thr Gly Thr Ser
                165                 170                 175

Asn Gln Gln Ala Gln Gly Lys Asp Arg His Cys Phe Gly Ala Trp Ala
            180                 185                 190

Pro Gly Ser Trp Asn Lys Leu Cys Leu Ala Thr Leu Leu Asn Thr Glu
        195                 200                 205

Val Ser Asn Pro Met Glu Tyr Glu Phe Asn Phe Gln Leu Glu Ile Arg
    210                 215                 220

Gly Pro Cys Leu Leu Ala Gly Val Glu Ser Pro Thr His Glu Ile Arg
225                 230                 235                 240

Ala Asp Ala Ala Pro Ser Ala Arg Ser Ala Lys Ser Ile Ile Ile Thr
                245                 250                 255

Leu Ala Asn Lys His Thr Phe Asp Arg Pro Val Glu Ile Leu Ile His
            260                 265                 270

Pro Ser Glu Pro His Met Pro His Val Leu Ile Glu Lys Gly Asp Met
        275                 280                 285

Thr Leu Gly Glu Phe Asp Gln His Leu Lys Gly Arg Thr Asp Phe Ile
    290                 295                 300

Lys Gly Met Lys Lys Lys Ser Arg Ala Glu Arg Lys Thr Glu Ile Ile
305                 310                 315                 320

Arg Lys Arg Leu His Lys Asp Ile Pro His His Ser Val Ile Met Leu
                325                 330                 335

Asn Phe Cys Pro Asp Leu Gln Ser Val Gln Pro Cys Leu Arg Lys Ala
            340                 345                 350

His Gly Glu Phe Ile Phe Leu Ile Asp Arg Ser Ser Ser Met Ser Gly
        355                 360                 365

Ile Ser Met His Arg Val Lys Asp Ala Met Leu Val Ala Leu Lys Ser
    370                 375                 380

Leu Met Pro Ala Cys Leu Phe Asn Ile Ile Gly Phe Gly Ser Thr Phe
385                 390                 395                 400

Lys Ser Leu Phe Pro Ser Ser Gln Thr Tyr Ser Glu Asp Ser Leu Ala
                405                 410                 415

Met Ala Cys Asp Asp Ile Gln Arg Met Lys Ala Asp Met Gly Gly Thr
            420                 425                 430

Asn Ile Leu Ser Pro Leu Lys Trp Val Ile Arg Gln Pro Val His Arg
        435                 440                 445

Gly His Pro Arg Leu Leu Phe Val Ile Thr Asp Gly Ala Val Asn Asn
    450                 455                 460

Thr Gly Lys Val Leu Glu Leu Val Arg Asn His Ala Phe Ser Thr Arg
465                 470                 475                 480

Cys Tyr Ser Phe Gly Ile Gly Pro Asn Val Cys His Arg Leu Val Lys
                485                 490                 495

Gly Leu Ala Ser Val Ser Glu Gly Ser Ala Glu Leu Leu Met Glu Gly
            500                 505                 510

Glu Arg Leu Gln Pro Lys Met Val Lys Ser Leu Lys Lys Ala Met Ala
        515                 520                 525

Pro Val Leu Ser Asp Val Thr Val Glu Trp Ile Phe Pro Glu Thr Thr
    530                 535                 540

Glu Val Leu Val Ser Pro Val Ser Ala Ser Ser Leu Phe Pro Gly Glu
545                 550                 555                 560

Arg Leu Val Gly Tyr Gly Ile Val Cys Asp Ala Ser Leu His Ile Ser
                565                 570                 575

Asn Pro Arg Ser Asp Lys Arg Arg Arg Tyr Ser Met Leu His Ser Gln
            580                 585                 590

Glu Ser Gly Ser Ser Val Phe Tyr His Ser Gln Asp Asp Gly Pro Gly
        595                 600                 605

Leu Glu Gly Gly Asp Cys Ala Lys Asn Ser Gly Ala Pro Phe Ile Leu
    610                 615                 620

Gly Gln Ala Lys Asn Ala Arg Leu Ala Ser Gly Asp Ser Thr Thr Lys
625                 630                 635                 640

His Gly Leu Asn Leu Ser Gln Arg Arg Arg Ala Tyr Ser Thr Asn Gln
                645                 650                 655

Ile Thr Asn His Lys Pro Leu Pro Arg Ala Thr Met Ala Ser Asp Pro
            660                 665                 670

Met Pro Ala Ala Lys Arg Tyr Pro Leu Arg Lys Ala Arg Leu Gln Asp
        675                 680                 685

Leu Thr Asn Gln Thr Ser Leu Asp Val Gln Arg Trp Gln Ile Asp Leu
    690                 695                 700

Gln Val Leu Leu Asn Ser Gly Gln Asp Leu Asn Gln Gly Pro Lys Leu
705                 710                 715                 720

Arg Gly Pro Gly Ala Arg Arg Pro Ser Leu Leu Pro Gln Gly Cys Gln
                725                 730                 735

Pro Phe Leu Pro Trp Gly Gln Glu Thr Gln Ala Trp Ser Pro Val Arg
            740                 745                 750

Glu Arg Thr Ser Asp Ser Arg Ser Pro Gly Asp Leu Pro Ala Glu Pro
        755                 760                 765

Ser His His Pro Ser Ala Phe Glu Thr Glu Thr Ser Ser Asp Trp Asp
    770                 775                 780

Pro Pro Ala Glu Ser Gln Glu Arg Ala Ser Pro Ser Arg Pro Ala Thr
785                 790                 795                 800

Pro Ala Pro Val Leu Gly Lys Ala Leu Val Lys Gly Leu His Asp Ser
                805                 810                 815

Gln Arg Leu Gln Trp Glu Val Ser Phe Glu Leu Gly Thr Pro Gly Pro
            820                 825                 830

Glu Arg Gly Gly Ala Gln Asp Ala Asp Leu Trp Ser Glu Thr Phe His
        835                 840                 845

His Leu Ala Ala Arg Ala Ile Ile Arg Asp Phe Glu Gln Leu Ala Glu
    850                 855                 860

Arg Glu Gly Glu Ile Glu Gln Gly Ser Asn Arg Arg Tyr Gln Val Ser
865                 870                 875                 880

Ala Leu His Thr Ser Lys Ala Cys Asn Ile Ile Ser Lys Tyr Thr Ala
                885                 890                 895

Phe Val Pro Val Asp Val Ser Lys Ser Arg Tyr Leu Pro Thr Val Val
            900                 905                 910

Glu Tyr Pro Asn Ser Gly Arg Met Leu Gly Ser Arg Ala Leu Ala Gln
        915                 920                 925

Gln Trp Arg Gly Thr Ser Ser Gly Phe Gly Arg Pro Gln Thr Met Leu
    930                 935                 940

Gly Glu Asp Ser Ala Pro Gly Asn Gly Lys Phe Gln Val Leu Asp Met
945                 950                 955                 960

Glu Ala Ser Pro Thr Ala Leu Phe Ser Glu Ala Arg Ser Pro Gly Arg
                965                 970                 975

Glu Lys His Gly Ala Ser Glu Gly Pro Gln Arg Ser Leu Ala Thr Asn
            980                 985                 990

Thr Leu Ser Ser Met Lys Ala Ser Glu Asn Leu Phe Gly Ser Arg Leu
        995                 1000                1005

Asn Leu Asn Lys Ser Arg Leu Leu Thr Arg Ala Ala Lys Gly Phe Leu
    1010                1015                1020

Ser Lys Pro Leu Ile Lys Ala Val Glu Ser Thr Ser Gly Asn Gln Ser
1025                1030                1035                1040

Phe Asp Tyr Ile Pro Leu Val Ser Leu Gln Leu Ala Ser Gly Ala Phe
                1045                1050                1055

Leu Leu Asn Glu Ala Phe Cys Glu Ala Thr His Ile Pro Met Glu Lys
            1060                1065                1070

Leu Lys Trp Thr Ser Pro Phe Thr Cys His Arg Val Ser Leu Thr Thr
        1075                1080                1085

Arg Pro Ser Glu Ser Lys Thr Pro Ser Pro Gln Leu Cys Thr Ser Ser
    1090                1095                1100

Pro Pro Arg His Pro Ser Cys Asp Ser Phe Ser Leu Glu Pro Leu Ala
1105                1110                1115                1120

Lys Gly Lys Leu Gly Leu Glu Pro Arg Ala Val Val Glu His Thr Gly
                1125                1130                1135

Lys Leu Trp Ala Thr Val Val Gly Leu Ala Trp Leu Glu His Ser Ser
            1140                1145                1150

Ala Ser Tyr Phe Thr Glu Trp Glu Leu Val Ala Ala Lys Ala Asn Ser
        1155                1160                1165

Trp Leu Glu Gln Gln Glu Val Pro Glu Gly Arg Thr Gln Gly Thr Leu
    1170                1175                1180

Lys Ala Ala Ala Arg Gln Leu Phe Val Leu Leu Arg His Trp Asp Glu
1185                1190                1195                1200

Asn Leu Glu Phe Asn Met Leu Cys Tyr Asn Pro Asn Tyr Val
                1205                1210

<210>19
<211>868
<212>DNA
<213>Homo sapiens

<220>
<221> CDS
<222>(15)..(840)

<400>19

cgttttcttc taca atg tct gaa gaa gtg acc tac gcg aca ctc aca ttt    50
                Met Ser Glu Glu Val Thr Tyr Ala Thr Leu Thr Phe
                 1               5                   10

cag gat tct gct gga gca agg aat aac cga gat gga aat aac cta aga    98
Gln Asp Ser Ala Gly Ala Arg Asn Asn Arg Asp Gly Asn Asn Leu Arg
        15                  20                  25

aaa aga ggt cat cca gct cca tct ccc att tgg cgt cat gct gct ctg 146
Lys Arg Gly His Pro Ala Pro Ser Pro Ile Trp Arg His Ala Ala Leu
    30                  35                  40

ggt ctg gta act ctt tgc ctg atg ttg ctg att ggg ctg gtg aca ttg 194
Gly Leu Val Thr Leu Cys Leu Met Leu Leu Ile Gly Leu Val Thr Leu
45                  50                  55                  60

ggg atg atg tgt ttg cag ata tct aat gac att aac tca gat tca gag 242
Gly Met Met Cys Leu Gln Ile Ser Asn Asp Ile Asn Ser Asp Ser Glu
                65                  70                  75

aaa ttg agt caa ctt cag aaa acc atc caa cag cag cag gat aac tta 290
Lys Leu Ser Gln Leu Gln Lys Thr Ile Gln Gln Gln Gln Asp Asn Leu
            80                  85                  90

tcc cag caa ctg ggc aac tcc aac aac ttg tcc atg gag gag gaa ttt 338
Ser Gln Gln Leu Gly Asn Ser Asn Asn Leu Ser Met Glu Glu Glu Phe
        95                  100                 105

ctc aag tca cag atc tcc agt gta ctg aag agg cag gaa caa atg gcc 386
Leu Lys Ser Gln Ile Ser Ser Val Leu Lys Arg Gln Glu Gln Met Ala
    110                 115                 120

atc aaa ctg tgc caa gag cta atc att cat ttt tca gac cac aga tgt 434
Ile Lys Leu Cys Gln Glu Leu Ile Ile His Phe Ser Asp His Arg Cys
125                 130                 135                 140

aat cca tgt cct aag atg tgg caa tgg tac caa aat agt tgc tac tat 482
Asn Pro Cys Pro Lys Met Trp Gln Trp Tyr Gln Asn Ser Cys Tyr Tyr
                145                 150                 155

ttt aca aca aat gag gag aaa acc tgg gct aac agt aga aag gac tgc 530
Phe Thr Thr Asn Glu Glu Lys Thr Trp Ala Asn Ser Arg Lys Asp Cys
            160                 165                 170

ata gac aag aac tcc acc cta gtg aag ata gac agt ttg gaa gaa aag 578
Ile Asp Lys Asn Ser Thr Leu Val Lys Ile Asp Ser Leu Glu Glu Lys
        175                 180                 185

gat ttt ctt atg tca cag cca tta ctc atg ttt tcg ttc ttt tgg ctg 626
Asp Phe Leu Met Ser Gln Pro Leu Leu Met Phe Ser Phe Phe Trp Leu
    190                 195                 200

gga tta tca tgg gac tcc tct ggc aga agt tgg ttc tgg gaa gat ggc 674
Gly Leu Ser Trp Asp Ser Ser Gly Arg Ser Trp Phe Trp Glu Asp Gly
205                 210                 215                 220

tct gtt ccc tct cca tcc ttg agt act aaa gaa ctt gac cag atc aat 722
Ser Val Pro Ser Pro Ser Leu Ser Thr Lys Glu Leu Asp Gln Ile Asn
                225                 230                 235

gga tcc aaa gga tgt gct tat ttt caa aaa gga aat att tat att tct 770
Gly Ser Lys Gly Cys Ala Tyr Phe Gln Lys Gly Asn Ile Tyr Ile Ser
            240                 245                 250

cgc tgt agt gct gaa att ttt tgg att tgc gag aag aca gct gcc cca    818
Arg Cys Ser Ala Glu Ile Phe Trp Ile Cys Glu Lys Thr Ala Ala Pro
        255                 260                 265

gtg aag act gag gat ttg gat tagtatgctt cttccaaatt ctccaagaa    868
Val Lys Thr Glu Asp Leu Asp
    270                 275

<210>20
<211>275
<212>PRT
<213>Homo sapiens

<400>20

Met Ser Glu Glu Val Thr Tyr Ala Thr Leu Thr Phe Gln Asp Ser Ala
1               5                   10                  15

Gly Ala Arg Asn Asn Arg Asp Gly Asn Asn Leu Arg Lys Arg Gly His
            20                  25                  30

Pro Ala Pro Ser Pro Ile Trp Arg His Ala Ala Leu Gly Leu Val Thr
        35                  40                  45

Leu Cys Leu Met Leu Leu Ile Gly Leu Val Thr Leu Gly Met Met Cys
    50                  55                  60

Leu Gln Ile Ser Asn Asp Ile Asn Ser Asp Ser Glu Lys Leu Ser Gln
65                  70                  75                  80

Leu Gln Lys Thr Ile Gln Gln Gln Gln Asp Asn Leu Ser Gln Gln Leu
                85                  90                  95

Gly Asn Ser Asn Asn Leu Ser Met Glu Glu Glu Phe Leu Lys Ser Gln
            100                 105                 110

Ile Ser Ser Val Leu Lys Arg Gln Glu Gln Met Ala Ile Lys Leu Cys
        115                 120                 125

Gln Glu Leu Ile Ile His Phe Ser Asp His Arg Cys Asn Pro Cys Pro
    130                 135                 140

Lys Met Trp Gln Trp Tyr Gln Asn Ser Cys Tyr Tyr Phe Thr Thr Asn
145                 150                 155                 160

Glu Glu Lys Thr Trp Ala Asn Ser Arg Lys Asp Cys Ile Asp Lys Asn
                165                 170                 175

Ser Thr Leu Val Lys Ile Asp Ser Leu Glu Glu Lys Asp Phe Leu Met
            180                 185                 190

Ser Gln Pro Leu Leu Met Phe Ser Phe Phe Trp Leu Gly Leu Ser Trp
        195                 200                 205

Asp Ser Ser Gly Arg Ser Trp Phe Trp Glu Asp Gly Ser Val Pro Ser
    210                 215                 220

Pro Ser Leu Ser Thr Lys Glu Leu Asp Gln Ile Asn Gly Ser Lys Gly
225                 230                 235                 240

Cys Ala Tyr Phe Gln Lys Gly Asn Ile Tyr Ile Ser Arg Cys Ser Ala
                245                 250                 255

Glu Ile Phe Trp Ile Cys Glu Lys Thr Ala Ala Pro Val Lys Thr Glu
            260                 265                 270

Asp Leu Asp
        275

<210>21
<211>819
<212>DNA
<213>Homo sapiens

<220>
<221> CDS
<222>(1)..(805)

<400>21

aca ctc aca ttt cag gat tct gct gga gca agg aat aac cga gat gga    48
Thr Leu Thr Phe Gln Asp Ser Ala Gly Ala Arg Asn Asn Arg Asp Gly
  1               5                  10                  15

aat aac cta aga aaa aga ggg cat cca gct cca tct ccc att tgg cgt    96
Asn Asn Leu Arg Lys Arg Gly His Pro Ala Pro Ser Pro Ile Trp Arg
             20                  25                  30

cat gct gct ctg ggt ctg gta act ctt tgc ctg atg ttg ctg att ggg    144
His Ala Ala Leu Gly Leu Val Thr Leu Cys Leu Met Leu Leu Ile Gly
         35                  40                  45

ctg gtg acg ttg ggg atg atg ttt ttg cag ata tct aat gac att aac    192
Leu Val Thr Leu Gly Met Met Phe Leu Gln Ile Ser Asn Asp Ile Asn
     50                  55                  60

tca gat tca gag aaa ttg agt caa ctt cag aaa acc atc caa cag cag    240
Ser Asp Ser Glu Lys Leu Ser Gln Leu Gln Lys Thr Ile Gln Gln Gln
 65                  70                  75                  80

cag gat aac tta tcc cag caa ctg ggc aac tcc aac aac ttg tcc atg    288
Gln Asp Asn Leu Ser Gln Gln Leu Gly Asn Ser Asn Asn Leu Ser Met
                 85                  90                  95

gag gag gaa ttt ctc aag tca cag atc tcc agt gta ctg aag agg cag    336
Glu Glu Glu Phe Leu Lys Ser Gln Ile Ser Ser Val Leu Lys Arg Gln
            100                 105                 110

gaa caa atg gcc atc aaa ctg tgc caa gag cta atc att cat act tca    384
Glu Gln Met Ala Ile Lys Leu Cys Gln Glu Leu Ile Ile His Thr Ser
        115                 120                 125

gac cac aga tgt aat cca tgt cct aag atg tgg caa tgg tac caa aat    432
Asp His Arg Cys Asn Pro Cys Pro Lys Met Trp Gln Trp Tyr Gln Asn

    130                 135                 140

agt tgc tac tat ttt aca aca aat gag gag aaa acc tgg gct aac agt 480
Ser Cys Tyr Tyr Phe Thr Thr Asn Glu Glu Lys Thr Trp Ala Asn Ser
145                 150                 155                 160

aga aag gac tgc ata gac aag aac tcc acc cta gtg aag ata gac agt 528
Arg Lys Asp Cys Ile Asp Lys Asn Ser Thr Leu Val Lys Ile Asp Ser
                165                 170                 175

ttg gaa gaa aag gat ttt ctt atg tca cag cca tta ctc atg ttt tcg 576
Leu Glu Glu Lys Asp Phe Leu Met Ser Gln Pro Leu Leu Met Phe Ser
            180                 185                 190

ttc ttt tgg ctg gga tta tca tgg gac tcc tct ggc aga agt tgg ttc 624
Phe Phe Trp Leu Gly Leu Ser Trp Asp Ser Ser Gly Arg Ser Trp Phe
        195                 200                 205

tgg gaa gat ggc tct gtt ccc tct cca tcc tta ttt agt act aaa gaa 672
Trp Glu Asp Gly Ser Val Pro Ser Pro Ser Leu Phe Ser Thr Lys Glu
    210                 215                 220

ctt gac cag atc aat gga tcc aaa gga tgt gct tat ttt caa aaa gga 720
Leu Asp Gln Ile Asn Gly Ser Lys Gly Cys Ala Tyr Phe Gln Lys Gly
225                 230                 235                 240

aat att tat att tct cgc tgt agt gct gaa att ttt tgg att tgc gag 768
Asn Ile Tyr Ile Ser Arg Cys Ser Ala Glu Ile Phe Trp Ile Cys Glu
                245                 250                 255

aag aca gct gcc cca gtg aag act gag gat ttg gat tagaagggcg 814
Lys Thr Ala Ala Pro Val Lys Thr Glu Asp Leu Asp
            260                 265

attcc 819

<210>22
<211>268
<212>PRT
<213>Homo sapiens

<400>22

Thr Leu Thr Phe Gln Asp Ser Ala Gly Ala Arg Asn Asn Arg Asp Gly
1               5                   10                  15

Asn Asn Leu Arg Lys Arg Gly His Pro Ala Pro Ser Pro Ile Trp Arg
            20                  25                  30

His Ala Ala Leu Gly Leu Val Thr Leu Cys Leu Met Leu Leu Ile Gly
        35                  40                  45

Leu Val Thr Leu Gly Met Met Phe Leu Gln Ile Ser Asn Asp Ile Asn
    50                  55                  60

Ser Asp Ser Glu Lys Leu Ser Gln Leu Gln Lys Thr Ile Gln Gln Gln
65                  70                  75                  80

Gln Asp Asn Leu Ser Gln Gln Leu Gly Asn Ser Asn Asn Leu Ser Met
                85                  90                  95

Glu Glu Glu Phe Leu Lys Ser Gln Ile Ser Ser Val Leu Lys Arg Gln
            100                 105                 110

Glu Gln Met Ala Ile Lys Leu Cys Gln Glu Leu Ile Ile His Thr Ser
        115                 120                 125

Asp His Arg Cys Asn Pro Cys Pro Lys Met Trp Gln Trp Tyr Gln Asn
    130                 135                 140

Ser Cys Tyr Tyr Phe Thr Thr Asn Glu Glu Lys Thr Trp Ala Asn Ser
145                 150                 155                 160

Arg Lys Asp Cys Ile Asp Lys Asn Ser Thr Leu Val Lys Ile Asp Ser
                165                 170                 175

Leu Glu Glu Lys Asp Phe Leu Met Ser Gln Pro Leu Leu Met Phe Ser
            180                 185                 190

Phe Phe Trp Leu Gly Leu Ser Trp Asp Ser Ser Gly Arg Ser Trp Phe
        195                 200                 205

Trp Glu Asp Gly Ser Val Pro Ser Pro Ser Leu Phe Ser Thr Lys Glu
    210                 215                 220

Leu Asp Gln Ile Asn Gly Ser Lys Gly Cys Ala Tyr Phe Gln Lys Gly
225                 230                 235                 240

Asn Ile Tyr Ile Ser Arg Cys Ser Ala Glu Ile Phe Trp Ile Cys Glu
                245                 250                 255

Lys Thr Ala Ala Pro Val Lys Thr Glu Asp Leu Asp
            260                 265

<210>23
<211>516
<212>DNA
<213>Homo sapiens

<220>
<221> CDS
<222>(9)..(465)

<400>23

cactgcgc atg cta ttt ggg cgc cca cct cag tgc aca tgt tca ctg      47
         Met Leu Phe Gly Arg Pro Pro Gln Cys Thr Cys Ser Leu
         1               5                   10

ggc gtc ttc tac tct acc cct tcg ccc tcg tgg ggg tgt gag ggt cgc      95
Gly Val Phe Tyr Ser Thr Pro Ser Pro Ser Trp Gly Cys Glu Gly Arg

15                  20                  25

gtt cct gct gtc tgg act ttt tct gtc cca ctg aga cgc aat gta tcg    143
Val Pro Ala Val Trp Thr Phe Ser Val Pro Leu Arg Arg Asn Val Ser
30                  35                  40                  45

ata aca aaa ctt ttt atc tgc aca cac aca cac aca cac aca cac ccc    191
Ile Thr Lys Leu Phe Ile Cys Thr His Thr His Thr His Thr His Pro
                50                  55                  60

tgg ttc cag gag ccc ggt gat gag gag cct cag caa gag gaa cca cca    239
Trp Phe Gln Glu Pro Gly Asp Glu Glu Pro Gln Gln Glu Glu Pro Pro
            65                  70                  75

act gaa agt cgg gat cct gca cct ggt cag gag aga gaa gaa gat cag    287
Thr Glu Ser Arg Asp Pro Ala Pro Gly Gln Glu Arg Glu Glu Asp Gln
        80                  85                  90

ggt gca gct gag act caa tgc ctg acc tgg aag ctg atc tcc agg agc    335
Gly Ala Ala Glu Thr Gln Cys Leu Thr Trp Lys Leu Ile Ser Arg Ser
    95                  100                 105

tgt ctc agt caa aga ctg ggg gtg aat gtg gaa atg aag att ctg cca    383
Cys Leu Ser Gln Arg Leu Gly Val Asn Val Glu Met Lys Ile Leu Pro
110                 115                 120                 125

aaa tca gaa caa ttt aaa atg cca gaa gga ggt atg cta tcc att att    431
Lys Ser Glu Gln Phe Lys Met Pro Glu Gly Gly Met Leu Ser Ile Ile
                130                 135                 140

atg tgc ttt ctg ttt tcc aca ata tta tac ttt tgataataaa             474
Met Cys Phe Leu Phe Ser Thr Ile Leu Tyr Phe
            145                 150

agagaacatt actatccctt taaaatcaga gttcaaatgc ag                     516

<210>24
<211>152
<212>PRT
<213>Homo sapiens

<400>24

Met Leu Phe Gly Arg Pro Pro Gln Cys Thr Cys Ser Leu Gly Val Phe
1               5                   10                  15

Tyr Ser Thr Pro Ser Pro Ser Trp Gly Cys Glu Gly Arg Val Pro Ala
            20                  25                  30

Val Trp Thr Phe Ser Val Pro Leu Arg Arg Asn Val Ser Ile Thr Lys
        35                  40                  45

Leu Phe Ile Cys Thr His Thr His Thr His Thr His Pro Trp Phe Gln
    50                  55                  60

Glu Pro Gly Asp Glu Glu Pro Gln Gln Glu Glu Pro Pro Thr Glu Ser
65                  70                  75                  80

Arg Asp Pro Ala Pro Gly Gln Glu Arg Glu Glu Asp Gln Gly Ala Ala
                85                  90                  95

Glu Thr Gln Cys Leu Thr Trp Lys Leu Ile Ser Arg Ser Cys Leu Ser
            100                 105                 110

Gln Arg Leu Gly Val Asn Val Glu Met Lys Ile Leu Pro Lys Ser Glu
        115                 120                 125

Gln Phe Lys Met Pro Glu Gly Gly Met Leu Ser Ile Ile Met Cys Phe
    130                 135                 140

Leu Phe Ser Thr Ile Leu Tyr Phe
145                 150

<210>25
<211>709
<212>DNA
<213>Homo sapiens

<220>
<221> CDS
<222>(59)..(686)

<400>25

cggccggttt tggtaggccc gggccgccgc caggcctccg cctgagcccg                50

cacccgcc atg gac aac tac gca gat ctt tcg gat acc gag ctg acc      97
         Met Asp Asn Tyr Ala Asp Leu Ser Asp Thr Glu Leu Thr
           1               5                  10

acc ttg ctg cgc cgg tac aac atc ccg cac ggg cct gta gta gga tca   145
Thr Leu Leu Arg Arg Tyr Asn Ile Pro His Gly Pro Val Val Gly Ser
     15                  20                  25

act cgt agg ctt tac gag aag aag atc ttc gag tac gag acc cag agg   193
Thr Arg Arg Leu Tyr Glu Lys Lys Ile Phe Glu Tyr Glu Thr Gln Arg
 30                  35                  40                  45

cgg cgg ctc tcg ccc ccc agc tcg tcc gcc gcc tcc tct tat agc ttc   241
Arg Arg Leu Ser Pro Pro Ser Ser Ser Ala Ala Ser Ser Tyr Ser Phe
                 50                  55                  60

tct gac ttg aat tcg act aga ggg gat gca gat atg tat gat ctt ccc   289
Ser Asp Leu Asn Ser Thr Arg Gly Asp Ala Asp Met Tyr Asp Leu Pro
             65                  70                  75

aag aaa gag gac gct tta ctc tac cag agc aag ggc tac aat gac gat   337
Lys Lys Glu Asp Ala Leu Leu Tyr Gln Ser Lys Gly Tyr Asn Asp Asp
         80                  85                  90

ctt ttg tct tct tct gaa gag gag tgc aag gat agg gaa cgc ccc atg 385
Leu Leu Ser Ser Ser Glu Glu Glu Cys Lys Asp Arg Glu Arg Pro Met
    95                  100                 105

tac ggc cgg gac agt gcc tac cag agc atc acg cac tac cgc cct gtt 433
Tyr Gly Arg Asp Ser Ala Tyr Gln Ser Ile Thr His Tyr Arg Pro Val
110                 115                 120                 125

tca gcc tcc agg agc tcc ctg gac ctg tcc tat tat cct act tcc tcc 481
Ser Ala Ser Arg Ser Ser Leu Asp Leu Ser Tyr Tyr Pro Thr Ser Ser
                130                 135                 140

tcc acc tct ttt atg tcc tcc tca tca tct tcc tct tca tgg ctc acc 529
Ser Thr Ser Phe Met Ser Ser Ser Ser Ser Ser Ser Ser Trp Leu Thr
            145                 150                 155

cgc cgt gcc atc cgg cct gaa aac cgt gct cct ggg gct ggg ctg ggc 577
Arg Arg Ala Ile Arg Pro Glu Asn Arg Ala Pro Gly Ala Gly Leu Gly
        160                 165                 170

cag gat cgc cag gtc ccg ctc tgg ggc cag ctg ctg ctt ttc ctg gtc 625
Gln Asp Arg Gln Val Pro Leu Trp Gly Gln Leu Leu Leu Phe Leu Val
    175                 180                 185

ttt gtg atc gtc ctc ttc ttc att tac cac ttc atg cag gct gaa gaa 673
Phe Val Ile Val Leu Phe Phe Ile Tyr His Phe Met Gln Ala Glu Glu
190                 195                 200                 205

ggc aac ccc ttc tgactgcagc caagctaatt ccgg 709
Gly Asn Pro Phe

<210>26
<211>209
<212>PRT
<213>Homo sapiens

<400>26

Met Asp Asn Tyr Ala Asp Leu Ser Asp Thr Glu Leu Thr Thr Leu Leu
1               5                   10                  15

Arg Arg Tyr Asn Ile Pro His Gly Pro Val Val Gly Ser Thr Arg Arg
            20                  25                  30

Leu Tyr Glu Lys Lys Ile Phe Glu Tyr Glu Thr Gln Arg Arg Arg Leu
        35                  40                  45

Ser Pro Pro Ser Ser Ser Ala Ala Ser Ser Tyr Ser Phe Ser Asp Leu
    50                  55                  60

Asn Ser Thr Arg Gly Asp Ala Asp Met Tyr Asp Leu Pro Lys Lys Glu
65                  70                  75                  80

Asp Ala Leu Leu Tyr Gln Ser Lys Gly Tyr Asn Asp Asp Leu Leu Ser
                85                  90                  95

Ser Ser Glu Glu Glu Cys Lys Asp Arg Glu Arg Pro Met Tyr Gly Arg
            100                 105                 110

Asp Ser Ala Tyr Gln Ser Ile Thr His Tyr Arg Pro Val Ser Ala Ser
        115                 120                 125

Arg Ser Ser Leu Asp Leu Ser Tyr Tyr Pro Thr Ser Ser Ser Thr Ser
    130                 135                 140

Phe Met Ser Ser Ser Ser Ser Ser Ser Ser Trp Leu Thr Arg Arg Ala
145                 150                 155                 160

Ile Arg Pro Glu Asn Arg Ala Pro Gly Ala Gly Leu Gly Gln Asp Arg
                165                 170                 175

Gln Val Pro Leu Trp Gly Gln Leu Leu Leu Phe Leu Val Phe Val Ile
            180                 185                 190

Val Leu Phe Phe Ile Tyr His Phe Met Gln Ala Glu Glu Gly Asn Pro
        195                 200                 205

Phe

<210>27
<211>2812
<212>DNA
<213>Homo sapiens

<220>
<221> CDS
<222>(47)..(2690)

<400>27
   cattgagtcg gcttttctac tgcttcggct agggtacctt gtgacc atg tct tcc     55
                                                      Met Ser Ser
                                                      1
   aag aag aat aga aag cgg ttg aac caa agc gcg gaa aat ggt tcg tcc     103
   Lys Lys Asn Arg Lys Arg Leu Asn Gln Ser Ala Glu Asn Gly Ser Ser
       5                   10                  15
   ttg ccc tct gct gct tcc tct tgt gcg gag gca cgg gct cct tct gct     151
   Leu Pro Ser Ala Ala Ser Ser Cys Ala Glu Ala Arg Ala Pro Ser Ala
   20                  25                  30                  35
   gga tca gac ttc gcg gca acc tcc ggg act ctg acg gtg acc aac tta     199
   Gly Ser Asp Phe Ala Ala Thr Ser Gly Thr Leu Thr Val Thr Asn Leu
                   40                  45                  50
   tta gaa aag ggt aaa att cct aaa aca ttc cag aat tcc ctt att cat     247
   Leu Glu Lys Gly Lys Ile Pro Lys Thr Phe Gln Asn Ser Leu Ile His
               55                  60                  65
   ctt gga ctc aac act atg aag tct gca aat ata tgt ata ggt cga cca     295
   Leu Gly Leu Asn Thr Met Lys Ser Ala Asn Ile Cys Ile Gly Arg Pro
   70                    75                    80
   gtg ttg ctt act agt ttg aac gga aag caa gag gta tat aca gcc tgg    343
   Val Leu Leu Thr Ser Leu Asn Gly Lys Gln Glu Val Tyr Thr Ala Trp
       85                  90                  95
   cct atg gca gga ttt cct gga ggc aag gtc ggc ctg agt gaa atg gca    391
   Pro Met Ala Gly Phe Pro Gly Gly Lys Val Gly Leu Ser Glu Met Ala
   100                 105                 110                 115
   cag aaa aat gtg ggt gtg agg cct ggt gat gcc atc cag gtc cag cct    439
   Gln Lys Asn Val Gly Val Arg Pro Gly Asp Ala Ile Gln Val Gln Pro
                   120                 125                 130
   ctt gtg ggt gct gtg cta cag gct gag gaa atg gat gtg gca ctg agt    487
   Leu Val Gly Ala Val Leu Gln Ala Glu Glu Met Asp Val Ala Leu Ser
               135                 140                 145
   gac aaa gat atg gaa att aat gaa gaa gaa ctg act ggt tgt atc ctg    535
   Asp Lys Asp Met Glu Ile Asn Glu Glu Glu Leu Thr Gly Cys Ile Leu
           150                 155                 160
   aga aaa cta gat ggc aag att gtt tta cca ggc aac ttt ctg tat tgt    583
   Arg Lys Leu Asp Gly Lys Ile Val Leu Pro Gly Asn Phe Leu Tyr Cys
       165                 170                 175
   aca ttc tat gga cga ccg tac aag ctg caa gta ttg cga gtg aaa ggg    631
   Thr Phe Tyr Gly Arg Pro Tyr Lys Leu Gln Val Leu Arg Val Lys Gly
   180                 185                 190                 195
   gca gat ggc atg ata ttg gga ggg cct cag agt gac tct gac act gat    679
   Ala Asp Gly Met Ile Leu Gly Gly Pro Gln Ser Asp Ser Asp Thr Asp
                   200                 205                 210
   gcc caa aga atg gcc ttt gaa cag tcc agc atg gaa acc agt agc ctg    727
   Ala Gln Arg Met Ala Phe Glu Gln Ser Ser Met Glu Thr Ser Ser Leu
               215                 220                 225
   gag tta tcc tta cag cta agc cag tta gat ctg gag gat acc cag atc    775
   Glu Leu Ser Leu Gln Leu Ser Gln Leu Asp Leu Glu Asp Thr Gln Ile
           230                 235                 240
   cca aca tca aga agt act cct tat aaa cca att gat gac aga att aca    823
   Pro Thr Ser Arg Ser Thr Pro Tyr Lys Pro Ile Asp Asp Arg Ile Thr
       245                 250                 255
   aat aaa gcc agt gat gtt ttg ctg gat gtt aca cag agc cct gga gat    871
   Asn Lys Ala Ser Asp Val Leu Leu Asp Val Thr Gln Ser Pro Gly Asp
   260                 265                 270                 275
   ggc agt gga ctt atg cta gag gaa gtc aca ggt ctt aaa tgt aat ttt    919
   Gly Ser Gly Leu Met Leu Glu Glu Val Thr Gly Leu Lys Cys Asn Phe
                   280                 285                 290
   gaa tct gcc aga gaa gga aat gag caa ctt act gaa gaa gag aga ctg    967
   Glu Ser Ala Arg Glu Gly Asn Glu Gln Leu Thr Glu Glu Glu Arg Leu
               295                 300                 305
   cta aag ttc agc ata gga gca aag tgc aat act gat act ttt tat ttt 1015
   Leu Lys Phe Ser Ile Gly Ala Lys Cys Asn Thr Asp Thr Phe Tyr Phe
   310 315 320
   att tct tca aca aca aga gtc aat ttt aca gag att gat aaa aat tca 1063
   Ile Ser Ser Thr Thr Arg Val Asn Phe Thr Glu Ile Asp Lys Asn Ser
   325 330 335
   aaa gag caa gac agt gat gtt aaa agt aac tat gac cat gat aga gga 1111
   Lys Glu Gln Asp Ser Asp Val Lys Ser Asn Tyr Asp His Asp Arg Gly
   340 345 350 355
   tta agt agc cag ctg aaa gca att aga gaa ata att gaa ttg ccc ctc 1159
   Leu Ser Ser Gln Leu Lys Ala Ile Arg Glu Ile Ile Glu Leu Pro Leu
   360 365 370
   aaa att cct gcc cct aga gga ttg tta ctt tat ggt cct cca tgt act 1207
   Lys Ile Pro Ala Pro Arg Gly Leu Leu Leu Tyr Gly Pro Pro Cys Thr
   375 380 385
   gga aaa aca atg atc gcc agg gct gtt gct aat gaa ttt gga gcc tat 1255
   Gly Lys Thr Met Ile Ala Arg Ala Val Ala Asn Glu Phe Gly Ala Tyr
   390 395 400
   gtt tct gta att aat ggt cct gaa att ata agc aag ttc tat ggt gag 1303
   Val Ser Val Ile Asn Gly Pro Glu Ile Ile Ser Lys Phe Tyr Gly Glu
   405 410 415
   act gaa gca aag tta cgt cag ata ttt gct gaa gcc act cta aga cac 1351
   Thr Glu Ala Lys Leu Arg Gln Ile Phe Ala Glu Ala Thr Leu Arg His
   420 425 430 435
   cca tca att att ttt att gat gag ctg gat gca ctt tgt ccg aaa aga 1399
   Pro Ser Ile Ile Phe Ile Asp Glu Leu Asp Ala Leu Cys Pro Lys Arg
   440 445 450
   gag ggg gcc cag aat gaa gtg gaa aaa aga gtt gtg gct tca ctc tta 1447
   Glu Gly Ala Gln Asn Glu Val Glu Lys Arg Val Val Ala Ser Leu Leu
   455 460 465
   aca ctg atg gat ggc att ggt tca gaa gta agt gaa gga caa gtg ttg 1495
   Thr Leu Met Asp Gly Ile Gly Ser Glu Val Ser Glu Gly Gln Val Leu
   470 475 480
   gtt ctt ggg gcc aca aat cgc cct cat gcc ttg gat gct gct ctc cga 1543
   Val Leu Gly Ala Thr Asn Arg Pro His Ala Leu Asp Ala Ala Leu Arg
   485 490 495
   aga cct ggg cga ttt gat aaa gag att gag att gga gtt ccc aat gct 1591
   Arg Pro Gly Arg Phe Asp Lys Glu Ile Glu Ile Gly Val Pro Asn Ala
   500 505 510 515
   cag gac cgg cta gat att ctc cag aaa ctg ctt cga agg gta ccc cat 1639
   Gln Asp Arg Leu Asp Ile Leu Gln Lys Leu Leu Arg Arg Val Pro His
   520 525 530
   ttg ctc act gag gct gag ctg ctg cag ctg gca aat agt gct cat gga 1687
   Leu Leu Thr Glu Ala Glu Leu Leu Gln Leu Ala Asn Ser Ala His Gly
   535 540 545
   tac gtt gga gca gac ttg aaa gtc ttg tgt aat gaa gca ggt ctc tgt 1735
   Tyr Val Gly Ala Asp Leu Lys Val Leu Cys Asn Glu Ala Gly Leu Cys
   550 555 560
   gcc ttg cgg aga atc ctg aaa aaa cag cct aac ctc cct gat gtc aag 1783
   Ala Leu Arg Arg Ile Leu Lys Lys Gln Pro Asn Leu Pro Asp Val Lys
   565 570 575
   gtg gct gga ctg gtg aag att act ctg aag gat ttc ttg cag gca atg 1831
   Val Ala Gly Leu Val Lys Ile Thr Leu Lys Asp Phe Leu Gln Ala Met
   580 585 590 595
   aat gat atc aga ccc agt gcc atg agg gaa ata gca att gat gtc cca 1879
   Asn Asp Ile Arg Pro Ser Ala Met Arg Glu Ile Ala Ile Asp Val Pro
   600 605 610
   aat gta agt tat gat gat gtt ggt gga gtt aga aag caa atg gcc caa 1927
   Asn Val Ser Tyr Asp Asp Val Gly Gly Val Arg Lys Gln Met Ala Gln
   615 620 625
   atc aga gag ctt gtt gag ctt cca cta cgc cat cct caa ctt ttc aaa 1975
   Ile Arg Glu Leu Val Glu Leu Pro Leu Arg His Pro Gln Leu Phe Lys
   630 635 640
   tct att ggt att cct gcc cct aga gga ttg tta ctt tat ggt cct cca 2023
   Ser Ile Gly Ile Pro Ala Pro Arg Gly Leu Leu Leu Tyr Gly Pro Pro
   645 650 655
   tgt act gga aaa aca atg atc gcc agg gct gtt gct aat gaa ttt gga 2071
   Cys Thr Gly Lys Thr Met Ile Ala Arg Ala Val Ala Asn Glu Phe Gly
   660 665 670 675
   gcc tat gtt tct gta att aat ggt cct gaa att ata agc aag tat gtt 2119
   Ala Tyr Val Ser Val Ile Asn Gly Pro Glu Ile Ile Ser Lys Tyr Val
   680 685 690
   ggt gag agt gaa cgt gct gtg cga caa gtt ttt caa cga gcc aag aac 2167
   Gly Glu Ser Glu Arg Ala Val Arg Gln Val Phe Gln Arg Ala Lys Asn
   695 700 705
   tca gca cca tca att att ttt att gat gag ctg gat gca ctt tgt ccg 2215
   Ser Ala Pro Ser Ile Ile Phe Ile Asp Glu Leu Asp Ala Leu Cys Pro
   710 715 720
   aaa aga gag ggg gcc cag aat gaa gtg gaa aaa aga gtt gtg gct tca 2263
   Lys Arg Glu Gly Ala Gln Asn Glu Val Glu Lys Arg Val Val Ala Ser
   725 730 735
   ctc tta aca ctg atg gat ggc att ggt tca gta agt ata gtg ttg gtt 2311
   Leu Leu Thr Leu Met Asp Gly Ile Gly Ser Val Ser Ile Val Leu Val
   740 745 750 755
   ctt ggg gcc aca aat cgc cct cat gcc ttg gat gct gct ctc cga aga 2359
   Leu Gly Ala Thr Asn Arg Pro His Ala Leu Asp Ala Ala Leu Arg Arg
   760 765 770
   cct ggg cga ttt gat aaa gag att gag att gga gtt ccc aat gct cag 2407
   Pro Gly Arg Phe Asp Lys Glu Ile Glu Ile Gly Val Pro Asn Ala Gln
   775 780 785
   gac cgg cta gat att ctc cag aaa ctg ctt cga agg gta ccc cat ttg 2455
   Asp Arg Leu Asp Ile Leu Gln Lys Leu Leu Arg Arg Val Pro His Leu
   790 795 800
   ctc act gag gct gag ctg ctg cag ctg gca aat agt gct cat gga tac 2503
   Leu Thr Glu Ala Glu Leu Leu Gln Leu Ala Asn Ser Ala His Gly Tyr
   805 810 815
   gtt gga gca gac ttg aaa gtc ttg tgt aat gaa gca ggt gag tgt ggt 2551
   Val Gly Ala Asp Leu Lys Val Leu Cys Asn Glu Ala Gly Glu Cys Gly
   820 825 830 835
   ttg cta tgg gac att caa gcc aat ctc atc atg aaa aga cat ttc act 2599
   Leu Leu Trp Asp Ile Gln Ala Asn Leu Ile Met Lys Arg His Phe Thr
   840 845 850
   cag gcc ttg agc act gtg aca cct aga att cct gag tca ttg aga cgt 2647
   Gln Ala Leu Ser Thr Val Thr Pro Arg Ile Pro Glu Ser Leu Arg Arg
   855 860 865
   ttt tat gaa gat tat caa gag aag agt ggg ctg cat aca ctc 2689
   Phe Tyr Glu Asp Tyr Gln Glu Lys Ser Gly Leu His Thr Leu
   870 875 880
   tgagaaaata tatatattca agatgctgaa aatcctttcc agagaaaatt 2739
   gtttcttttt aaaatttttg agagtgttaa aaaaaatttt actaggcaaa 2789
   atgtttgaag tatgttcagt aga 2812

<210>28
<211>881
<212>PRT
<213>Homo sapiens

<400>28

Met Ser Ser Lys Lys Asn Arg Lys Arg Leu Asn Gln Ser Ala Glu Asn
1               5                   10                  15

Gly Ser Ser Leu Pro Ser Ala Ala Ser Ser Cys Ala Glu Ala Arg Ala
            20                  25                  30

Pro Ser Ala Gly Ser Asp Phe Ala Ala Thr Ser Gly Thr Leu Thr Val

35                  40                  45

Thr Asn Leu Leu Glu Lys Gly Lys Ile Pro Lys Thr Phe Gln Asn Ser
    50                  55                  60

Leu Ile His Leu Gly Leu Asn Thr Met Lys Ser Ala Asn Ile Cys Ile
65                  70                  75                  80

Gly Arg Pro Val Leu Leu Thr Ser Leu Asn Gly Lys Gln Glu Val Tyr
                85                  90                  95

Thr Ala Trp Pro Met Ala Gly Phe Pro Gly Gly Lys Val Gly Leu Ser
            100                 105                 110

Glu Met Ala Gln Lys Asn Val Gly Val Arg Pro Gly Asp Ala Ile Gln
        115                 120                 125

Val Gln Pro Leu Val Gly Ala Val Leu Gln Ala Glu Glu Met Asp Val
    130                 135                 140

Ala Leu Ser Asp Lys Asp Met Glu Ile Asn Glu Glu Glu Leu Thr Gly
145                 150                 155                 160

Cys Ile Leu Arg Lys Leu Asp Gly Lys Ile Val Leu Pro Gly Asn Phe
                165                 170                 175

Leu Tyr Cys Thr Phe Tyr Gly Arg Pro Tyr Lys Leu Gln Val Leu Arg
            180                 185                 190

Val Lys Gly Ala Asp Gly Met Ile Leu Gly Gly Pro Gln Ser Asp Ser
        195                 200                 205

Asp Thr Asp Ala Gln Arg Met Ala Phe Glu Gln Ser Ser Met Glu Thr
    210                 215                 220

Ser Ser Leu Glu Leu Ser Leu Gln Leu Ser Gln Leu Asp Leu Glu Asp
225                 230                 235                 240

Thr Gln Ile Pro Thr Ser Arg Ser Thr Pro Tyr Lys Pro Ile Asp Asp
                245                 250                 255

Arg Ile Thr Asn Lys Ala Ser Asp Val Leu Leu Asp Val Thr Gln Ser
            260                 265                 270

Pro Gly Asp Gly Ser Gly Leu Met Leu Glu Glu Val Thr Gly Leu Lys
        275                 280                 285

Cys Asn Phe Glu Ser Ala Arg Glu Gly Asn Glu Gln Leu Thr Glu Glu
    290                 295                 300

Glu Arg Leu Leu Lys Phe Ser Ile Gly Ala Lys Cys Asn Thr Asp Thr
305                 310                 315                 320

Phe Tyr Phe Ile Ser Ser Thr Thr Arg Val Asn Phe Thr Glu Ile Asp
                325                 330                 335

Lys Asn Ser Lys Glu Gln Asp Ser Asp Val Lys Ser Asn Tyr Asp His

            340                 345                 350

Asp Arg Gly Leu Ser Ser Gln Leu Lys Ala Ile Arg Glu Ile Ile Glu
        355                 360                 365

Leu Pro Leu Lys Ile Pro Ala Pro Arg Gly Leu Leu Leu Tyr Gly Pro
    370                 375                 380

Pro Cys Thr Gly Lys Thr Met Ile Ala Arg Ala Val Ala Asn Glu Phe
385                 390                 395                 400

Gly Ala Tyr Val Ser Val Ile Asn Gly Pro Glu Ile Ile Ser Lys Phe
                405                 410                 415

Tyr Gly Glu Thr Glu Ala Lys Leu Arg Gln Ile Phe Ala Glu Ala Thr
            420                 425                 430

Leu Arg His Pro Ser Ile Ile Phe Ile Asp Glu Leu Asp Ala Leu Cys
        435                 440                 445

Pro Lys Arg Glu Gly Ala Gln Asn Glu Val Glu Lys Arg Val Val Ala
    450                 455                 460

Ser Leu Leu Thr Leu Met Asp Gly Ile Gly Ser Glu Val Ser Glu Gly
465                 470                 475                 480

Gln Val Leu Val Leu Gly Ala Thr Asn Arg Pro His Ala Leu Asp Ala
                485                 490                 495

Ala Leu Arg Arg Pro Gly Arg Phe Asp Lys Glu Ile Glu Ile Gly Val
            500                 505                 510

Pro Asn Ala Gln Asp Arg Leu Asp Ile Leu Gln Lys Leu Leu Arg Arg
        515                 520                 525

Val Pro His Leu Leu Thr Glu Ala Glu Leu Leu Gln Leu Ala Asn Ser
    530                 535                 540

Ala His Gly Tyr Val Gly Ala Asp Leu Lys Val Leu Cys Asn Glu Ala
545                 550                 555                 560

Gly Leu Cys Ala Leu Arg Arg Ile Leu Lys Lys Gln Pro Asn Leu Pro
                565                 570                 575

Asp Val Lys Val Ala Gly Leu Val Lys Ile Thr Leu Lys Asp Phe Leu
            580                 585                 590

Gln Ala Met Asn Asp Ile Arg Pro Ser Ala Met Arg Glu Ile Ala Ile
        595                 600                 605

Asp Val Pro Asn Val Ser Tyr Asp Asp Val Gly Gly Val Arg Lys Gln
    610                 615                 620

Met Ala Gln Ile Arg Glu Leu Val Glu Leu Pro Leu Arg His Pro Gln
625                 630                 635                 640

Leu Phe Lys Ser Ile Gly Ile Pro Ala Pro Arg Gly Leu Leu Leu Tyr

645                 650                 655

Gly Pro Pro Cys Thr Gly Lys Thr Met Ile Ala Arg Ala Val Ala Asn
            660                 665                 670

Glu Phe Gly Ala Tyr Val Ser Val Ile Asn Gly Pro Glu Ile Ile Ser
        675                 680                 685

Lys Tyr Val Gly Glu Ser Glu Arg Ala Val Arg Gln Val Phe Gln Arg
    690                 695                 700

Ala Lys Asn Ser Ala Pro Ser Ile Ile Phe Ile Asp Glu Leu Asp Ala
705                 710                 715                 720

Leu Cys Pro Lys Arg Glu Gly Ala Gln Asn Glu Val Glu Lys Arg Val
                725                 730                 735

Val Ala Ser Leu Leu Thr Leu Met Asp Gly Ile Gly Ser Val Ser Ile
            740                 745                 750

Val Leu Val Leu Gly Ala Thr Asn Arg Pro His Ala Leu Asp Ala Ala
        755                 760                 765

Leu Arg Arg Pro Gly Arg Phe Asp Lys Glu Ile Glu Ile Gly Val Pro
    770                 775                 780

Asn Ala Gln Asp Arg Leu Asp Ile Leu Gln Lys Leu Leu Arg Arg Val
785                 790                 795                 800

Pro His Leu Leu Thr Glu Ala Glu Leu Leu Gln Leu Ala Asn Ser Ala
                805                 810                 815

His Gly Tyr Val Gly Ala Asp Leu Lys Val Leu Cys Asn Glu Ala Gly
            820                 825                 830

Glu Cys Gly Leu Leu Trp Asp Ile Gln Ala Asn Leu Ile Met Lys Arg
        835                 840                 845

His Phe Thr Gln Ala Leu Ser Thr Val Thr Pro Arg Ile Pro Glu Ser
    850                 855                 860

Leu Arg Arg Phe Tyr Glu Asp Tyr Gln Glu Lys Ser Gly Leu His Thr
865                 870                 875                 880

Leu

<210>29
<211>2789
<212>DNA
<213>Homo sapiens

<220>
<221> CDS
<222>(61)..(2740)

<400>29

cagagttcgc ccttcattga gtcggctttt ctactgcttc ggctagggta 50

ccttgtgacc atg tct tcc aag aag aat aga aag cgg ttg aac caa agc 99
Met Ser Ser Lys Lys Asn Arg Lys Arg Leu Asn Gln Ser
1               5                   10

gcg gaa aat ggt tcg tcc ttg ccc tct gct gct tcc tct tgt gtg gag 147
Ala Glu Asn Gly Ser Ser Leu Pro Ser Ala Ala Ser Ser Cys Val Glu
    15                  20                  25

gca cgg gct cct tct gct gga tca gac ttc gcg gca acc tcc ggg act 195
Ala Arg Ala Pro Ser Ala Gly Ser Asp Phe Ala Ala Thr Ser Gly Thr
30                  35                  40                  45

ctg acg gtg acc aac tta tta gaa aag gta gat gac aaa att cct aaa 243
Leu Thr Val Thr Asn Leu Leu Glu Lys Val Asp Asp Lys Ile Pro Lys
                50                  55                  60

aca ttc cag aat tcc ctt att cat ctt gga ctc aac act atg aag tct 291
Thr Phe Gln Asn Ser Leu Ile His Leu Gly Leu Asn Thr Met Lys Ser
            65                  70                  75

gca aat ata tgt ata ggt cga cca gtg ttg ctt act agt ttg aac gga 339
Ala Asn Ile Cys Ile Gly Arg Pro Val Leu Leu Thr Ser Leu Asn Gly
        80                  85                  90

aag caa gag gtg tat aca gcc tgg cct atg gca gga ttt cct gga ggc 387
Lys Gln Glu Val Tyr Thr Ala Trp Pro Met Ala Gly Phe Pro Gly Gly
    95                  100                 105

aag gtc ggc ctg agt gaa atg gca cag aaa aat gtg ggt gtg agg cct 435
Lys Val Gly Leu Ser Glu Met Ala Gln Lys Asn Val Gly Val Arg Pro
110                 115                 120                 125

ggt gat gcc atc cag gtc cag cct ctt gtg ggt gct gtg cta cag gct 483
Gly Asp Ala Ile Gln Val Gln Pro Leu Val Gly Ala Val Leu Gln Ala
                130                 135                 140

gag gaa atg gat gtg gca ctg agt gac aaa gat atg gaa att aat gaa 531
Glu Glu Met Asp Val Ala Leu Ser Asp Lys Asp Met Glu Ile Asn Glu
            145                 150                 155

gaa gaa ctg act ggt tgt atc ctg aga aaa cta gat ggc aag att gtt 579
Glu Glu Leu Thr Gly Cys Ile Leu Arg Lys Leu Asp Gly Lys Ile Val
        160                 165                 170

tta cca ggc aac ttt ctg tat tgt aca ttc tat gga cga ccg tac aag 627
Leu Pro Gly Asn Phe Leu Tyr Cys Thr Phe Tyr Gly Arg Pro Tyr Lys
    175                 180                 185

ctg caa gta ttg cga gtg aaa ggg gca gat ggc atg ata ttg gga ggg 675
Leu Gln Val Leu Arg Val Lys Gly Ala Asp Gly Met Ile Leu Gly Gly
190                 195                 200                 205

cct cag agt gac tct gac act gat gcc caa aga atg gcc ttt gaa cag 723
Pro Gln Ser Asp Ser Asp Thr Asp Ala Gln Arg Met Ala Phe Glu Gln
                210                 215                 220

tcc agc atg gaa acc agt agc ctg gag tta tcc tta cag cta agc cag 771
Ser Ser Met Glu Thr Ser Ser Leu Glu Leu Ser Leu Gln Leu Ser Gln
            225                 230                 235

tta gat ctg gag gat acc cag atc cca aca tca aga agt act cct tat 819
Leu Asp Leu Glu Asp Thr Gln Ile Pro Thr Ser Arg Ser Thr Pro Tyr
        240                 245                 250

aaa cca att gat gac aga att aca aat aaa gcc agt gat gtt ttg ctg 867
Lys Pro Ile Asp Asp Arg Ile Thr Asn Lys Ala Ser Asp Val Leu Leu
    255                 260                 265

gat gtt aca cag agc cct gga gat ggc agt gga ctt atg cta gag gaa 915
Asp Val Thr Gln Ser Pro Gly Asp Gly Ser Gly Leu Met Leu Glu Glu
270                 275                 280                 285

gtc aca ggt ctt aaa tgt aat ttt gaa tct gcc aga gaa gga aat gag 963
Val Thr Gly Leu Lys Cys Asn Phe Glu Ser Ala Arg Glu Gly Asn Glu
                290                 295                 300

caa ctt act gaa gaa gag aga ctg cta aag ttc agc ata gga gca aag 1011
Gln Leu Thr Glu Glu Glu Arg Leu Leu Lys Phe Ser Ile Gly Ala Lys
            305                 310                 315

tgc aat act gat act ttt tat ttt att tct tca aca aca aga gtc aat 1059
Cys Asn Thr Asp Thr Phe Tyr Phe Ile Ser Ser Thr Thr Arg Val Asn
        320                 325                 330

ttt aca gag att gat aaa aat tca aaa gag caa gac aac caa ttt aaa 1107
Phe Thr Glu Ile Asp Lys Asn Ser Lys Glu Gln Asp Asn Gln Phe Lys
    335                 340                 345

gta act tat gac atg ata gga gga tta agt agc cag ctg aaa gca att 1155
Val Thr Tyr Asp Met Ile Gly Gly Leu Ser Ser Gln Leu Lys Ala Ile
350                 355                 360                 365

aga gaa ata att gaa ttg ccc ctc aaa cag cct gag ctt ttc aag agt 1203
Arg Glu Ile Ile Glu Leu Pro Leu Lys Gln Pro Glu Leu Phe Lys Ser
                370                 375                 380

tat gga att cct gcc cct aga gga gtg tta ctt tat ggt cct cca ggt 1251
Tyr Gly Ile Pro Ala Pro Arg Gly Val Leu Leu Tyr Gly Pro Pro Gly
            385                 390                 395

act gga aaa aca atg atc gcc agg gct gtt gct aat gaa gtt gga gcc 1299
Thr Gly Lys Thr Met Ile Ala Arg Ala Val Ala Asn Glu Val Gly Ala
        400                 405                 410

tat gtt tct gta att aat ggt cct gaa att ata agc aaa ttc tat ggt 1347
Tyr Val Ser Val Ile Asn Gly Pro Glu Ile Ile Ser Lys Phe Tyr Gly
    415                 420                 425

gag act gaa gca aag tta cgt cag ata ttt gct gaa gcc act cta cga 1395

Glu Thr Glu Ala Lys Leu Arg Gln Ile Phe Ala Glu Ala Thr Leu Arg
430                 435                 440                 445

cac cca tca att att ttt att gat gag ctg gat gca ctt tgt ccg aaa     1443
His Pro Ser Ile Ile Phe Ile Asp Glu Leu Asp Ala Leu Cys Pro Lys
                450                 455                 460

aga gag ggg gcc cag aat gaa gtg gaa aaa aga gtt gtg gct tca ctc     1491
Arg Glu Gly Ala Gln Asn Glu Val Glu Lys Arg Val Val Ala Ser Leu
            465                 470                 475

tta aca ctg atg gat ggc att ggt tca gaa gta agt gaa gga caa gtg     1539
Leu Thr Leu Met Asp Gly Ile Gly Ser Glu Val Ser Glu Gly Gln Val
        480                 485                 490

ttg gtt ctt ggg gcc aca aat cgc cct cat gcc ttg gat gct gct ctc     1587
Leu Val Leu Gly Ala Thr Asn Arg Pro His Ala Leu Asp Ala Ala Leu
    495                 500                 505

cga aga cct ggg cga ttt gat aaa gag att gag att gga gtt ccc aat     1635
Arg Arg Pro Gly Arg Phe Asp Lys Glu Ile Glu Ile Gly Val Pro Asn
510                 515                 520                 525

gct cag gac cgg cta gat att ctc cag aaa ctg ctt cga agg gta ccc     1683
Ala Gln Asp Arg Leu Asp Ile Leu Gln Lys Leu Leu Arg Arg Val Pro
                530                 535                 540

cat ttg ctc act gag gct gag ctg ctg cag ctg gca aat agt gct cat     1731
His Leu Leu Thr Glu Ala Glu Leu Leu Gln Leu Ala Asn Ser Ala His
            545                 550                 555

gga tac gtt gga gca gac ttg aaa gtc ttg tgt aat gaa gca ggt ctc     1779
Gly Tyr Val Gly Ala Asp Leu Lys Val Leu Cys Asn Glu Ala Gly Leu
        560                 565                 570

tgt gcc ttg cgg aga atc ctg aaa aaa cag cct aac ctc cct gat gtc     1827
Cys Ala Leu Arg Arg Ile Leu Lys Lys Gln Pro Asn Leu Pro Asp Val
    575                 580                 585

aag gtg gct gga ctg gtg aag att act ctg aag gat ttc ttg cag gca     1875
Lys Val Ala Gly Leu Val Lys Ile Thr Leu Lys Asp Phe Leu Gln Ala
590                 595                 600                 605

atg aat gat atc aga ccc agt gcc atg agg gaa ata gca att gat gtc     1923
Met Asn Asp Ile Arg Pro Ser Ala Met Arg Glu Ile Ala Ile Asp Val
                610                 615                 620

cca aat gta tcc tgg tca gat ata gga gga ctg gaa agt atc aaa ctg     1971
Pro Asn Val Ser Trp Ser Asp Ile Gly Gly Leu Glu Ser Ile Lys Leu
            625                 630                 635

aag ttg gaa cag gct gtg gaa tgg ccc tta aaa cat cca gag tct ttc     2019
Lys Leu Glu Gln Ala Val Glu Trp Pro Leu Lys His Pro Glu Ser Phe
        640                 645                 650

att cga atg ggt att cag cca cct aaa gga gtt ctt ctc tat ggg cca     2067
Ile Arg Met Gly Ile Gln Pro Pro Lys Gly Val Leu Leu Tyr Gly Pro

655                 660                 665

cct ggg tgc tct aaa aca atg ata gca aag gct ttg gcc aat gag agt    2115
Pro Gly Cys Ser Lys Thr Met Ile Ala Lys Ala Leu Ala Asn Glu Ser
670                 675                 680                 685

gga ctg aat ttt cta gct ata aag ggg cct gaa tta atg aat aaa tat    2163
Gly Leu Asn Phe Leu Ala Ile Lys Gly Pro Glu Leu Met Asn Lys Tyr
                690                 695                 700

gtt ggt gaa tct gaa aga gca gtt aga gag acc ttc cga aaa gca aga    2211
Val Gly Glu Ser Glu Arg Ala Val Arg Glu Thr Phe Arg Lys Ala Arg
            705                 710                 715

gca gtg gcg cct tcc att att ttc ttt gat gaa ctg gat gcc tta gca    2259
Ala Val Ala Pro Ser Ile Ile Phe Phe Asp Glu Leu Asp Ala Leu Ala
        720                 725                 730

gtt gaa agg ggc agt tct tta ggt gct ggg aat gta gcc gat cgt gtt    2307
Val Glu Arg Gly Ser Ser Leu Gly Ala Gly Asn Val Ala Asp Arg Val
    735                 740                 745

ttg gct cag ctc tta aca gaa atg gat ggg att gaa cag cta aag gat    2355
Leu Ala Gln Leu Leu Thr Glu Met Asp Gly Ile Glu Gln Leu Lys Asp
750                 755                 760                 765

gtg acc att ttg gca gct act aac cgt cca gat agg ata gac aag gct    2403
Val Thr Ile Leu Ala Ala Thr Asn Arg Pro Asp Arg Ile Asp Lys Ala
                770                 775                 780

ttg atg cgg cct gga aga att gat aga atc atc tat gtg cct tta ccg    2451
Leu Met Arg Pro Gly Arg Ile Asp Arg Ile Ile Tyr Val Pro Leu Pro
            785                 790                 795

gat gca gca aca aga agg gaa ata ttt aag ctg cag ttt cac tcc atg    2499
Asp Ala Ala Thr Arg Arg Glu Ile Phe Lys Leu Gln Phe His Ser Met
        800                 805                 810

cct gtc agt aat gaa gtt gac ctg gat gaa ctc atc ctt caa acc gac    2547
Pro Val Ser Asn Glu Val Asp Leu Asp Glu Leu Ile Leu Gln Thr Asp
    815                 820                 825

gca tac tca gga gca gag att gta gct gtc tgc aga gag gca gct ctt    2595
Ala Tyr Ser Gly Ala Glu Ile Val Ala Val Cys Arg Glu Ala Ala Leu
830                 835                 840                 845

ctg gct ctg gaa gaa gac att caa gcc aat ctc atc atg aaa aga cat    2643
Leu Ala Leu Glu Glu Asp Ile Gln Ala Asn Leu Ile Met Lys Arg His
                850                 855                 860

ttc act cag gcc ttg agc act gtg aca cct aga att cct gag tca ttg    2691
Phe Thr Gln Ala Leu Ser Thr Val Thr Pro Arg Ile Pro Glu Ser Leu
            865                 870                 875

aga cgt ttt tat gaa gat tat caa gag aag agt ggg ctg cat aca ctc    2739
Arg Arg Phe Tyr Glu Asp Tyr Gln Glu Lys Ser Gly Leu His Thr Leu
        880                 885                 890

tgagaaaata tatatattca agatgctgaa aatcctttcc agagaaaatt 2789

<210>30
<211>893
<212>PRT
<213>Homo sapiens

<400>30

Met Ser Ser Lys Lys Asn Arg Lys Arg Leu Asn Gln Ser Ala Glu Asn
1               5                   10                  15

Gly Ser Ser Leu Pro Ser Ala Ala Ser Ser Cys Val Glu Ala Arg Ala
            20                  25                  30

Pro Ser Ala Gly Ser Asp Phe Ala Ala Thr Ser Gly Thr Leu Thr Val
        35                  40                  45

Thr Asn Leu Leu Glu Lys Val Asp Asp Lys Ile Pro Lys Thr Phe Gln
    50                  55                  60

Asn Ser Leu Ile His Leu Gly Leu Asn Thr Met Lys Ser Ala Asn Ile
65                  70                  75                  80

Cys Ile Gly Arg Pro Val Leu Leu Thr Ser Leu Asn Gly Lys Gln Glu
                85                  90                  95

Val Tyr Thr Ala Trp Pro Met Ala Gly Phe Pro Gly Gly Lys Val Gly
            100                 105                 110

Leu Ser Glu Met Ala Gln Lys Asn Val Gly Val Arg Pro Gly Asp Ala
        115                 120                 125

Ile Gln Val Gln Pro Leu Val Gly Ala Val Leu Gln Ala Glu Glu Met
    130                 135                 140

Asp Val Ala Leu Ser Asp Lys Asp Met Glu Ile Asn Glu Glu Glu Leu
145                 150                 155                 160

Thr Gly Cys Ile Leu Arg Lys Leu Asp Gly Lys Ile Val Leu Pro Gly
                165                 170                 175

Asn Phe Leu Tyr Cys Thr Phe Tyr Gly Arg Pro Tyr Lys Leu Gln Val
            180                 185                 190

Leu Arg Val Lys Gly Ala Asp Gly Met Ile Leu Gly Gly Pro Gln Ser
        195                 200                 205

Asp Ser Asp Thr Asp Ala Gln Arg Met Ala Phe Glu Gln Ser Ser Met
    210                 215                 220

Glu Thr Ser Ser Leu Glu Leu Ser Leu Gln Leu Ser Gln Leu Asp Leu
225                 230                 235                 240

Glu Asp Thr Gln Ile Pro Thr Ser Arg Ser Thr Pro Tyr Lys Pro Ile
                245                 250                 255

Asp Asp Arg Ile Thr Asn Lys Ala Ser Asp Val Leu Leu Asp Val Thr
            260                 265                 270

Gln Ser Pro Gly Asp Gly Ser Gly Leu Met Leu Glu Glu Val Thr Gly
        275                 280                 285

Leu Lys Cys Asn Phe Glu Ser Ala Arg Glu Gly Asn Glu Gln Leu Thr
    290                 295                 300

Glu Glu Glu Arg Leu Leu Lys Phe Ser Ile Gly Ala Lys Cys Asn Thr
305                 310                 315                 320

Asp Thr Phe Tyr Phe Ile Ser Ser Thr Thr Arg Val Asn Phe Thr Glu
                325                 330                 335

Ile Asp Lys Asn Ser Lys Glu Gln Asp Asn Gln Phe Lys Val Thr Tyr
            340                 345                 350

Asp Met Ile Gly Gly Leu Ser Ser Gln Leu Lys Ala Ile Arg Glu Ile
        355                 360                 365

Ile Glu Leu Pro Leu Lys Gln Pro Glu Leu Phe Lys Ser Tyr Gly Ile
    370                 375                 380

Pro Ala Pro Arg Gly Val Leu Leu Tyr Gly Pro Pro Gly Thr Gly Lys
385                 390                 395                 400

Thr Met Ile Ala Arg Ala Val Ala Asn Glu Val Gly Ala Tyr Val Ser
                405                 410                 415

Val Ile Asn Gly Pro Glu Ile Ile Ser Lys Phe Tyr Gly Glu Thr Glu
            420                 425                 430

Ala Lys Leu Arg Gln Ile Phe Ala Glu Ala Thr Leu Arg His Pro Ser
        435                 440                 445

Ile Ile Phe Ile Asp Glu Leu Asp Ala Leu Cys Pro Lys Arg Glu Gly
    450                 455                 460

Ala Gln Asn Glu Val Glu Lys Arg Val Val Ala Ser Leu Leu Thr Leu
465                 470                 475                 480

Met Asp Gly Ile Gly Ser Glu Val Ser Glu Gly Gln Val Leu Val Leu
                485                 490                 495

Gly Ala Thr Asn Arg Pro His Ala Leu Asp Ala Ala Leu Arg Arg Pro
            500                 505                 510

Gly Arg Phe Asp Lys Glu Ile Glu Ile Gly Val Pro Asn Ala Gln Asp
        515                 520                 525

Arg Leu Asp Ile Leu Gln Lys Leu Leu Arg Arg Val Pro His Leu Leu
    530                 535                 540

Thr Glu Ala Glu Leu Leu Gln Leu Ala Asn Ser Ala His Gly Tyr Val
545                 550                 555                 560

Gly Ala Asp Leu Lys Val Leu Cys Asn Glu Ala Gly Leu Cys Ala Leu
                565                 570                 575

Arg Arg Ile Leu Lys Lys Gln Pro Asn Leu Pro Asp Val Lys Val Ala
            580                 585                 590

Gly Leu Val Lys Ile Thr Leu Lys Asp Phe Leu Gln Ala Met Asn Asp
        595                 600                 605

Ile Arg Pro Ser Ala Met Arg Glu Ile Ala Ile Asp Val Pro Asn Val
    610                 615                 620

Ser Trp Ser Asp Ile Gly Gly Leu Glu Ser Ile Lys Leu Lys Leu Glu
625                 630                 635                 640

Gln Ala Val Glu Trp Pro Leu Lys His Pro Glu Ser Phe Ile Arg Met
                645                 650                 655

Gly Ile Gln Pro Pro Lys Gly Val Leu Leu Tyr Gly Pro Pro Gly Cys
            660                 665                 670

Ser Lys Thr Met Ile Ala Lys Ala Leu Ala Asn Glu Ser Gly Leu Asn
        675                 680                 685

Phe Leu Ala Ile Lys Gly Pro Glu Leu Met Asn Lys Tyr Val Gly Glu
    690                 695                 700

Ser Glu Arg Ala Val Arg Glu Thr Phe Arg Lys Ala Arg Ala Val Ala
705                 710                 715                 720

Pro Ser Ile Ile Phe Phe Asp Glu Leu Asp Ala Leu Ala Val Glu Arg
                725                 730                 735

Gly Ser Ser Leu Gly Ala Gly Asn Val Ala Asp Arg Val Leu Ala Gln
            740                 745                 750

Leu Leu Thr Glu Met Asp Gly Ile Glu Gln Leu Lys Asp Val Thr Ile
        755                 760                 765

Leu Ala Ala Thr Asn Arg Pro Asp Arg Ile Asp Lys Ala Leu Met Arg
    770                 775                 780

Pro Gly Arg Ile Asp Arg Ile Ile Tyr Val Pro Leu Pro Asp Ala Ala
785                 790                 795                 800

Thr Arg Arg Glu Ile Phe Lys Leu Gln Phe His Ser Met Pro Val Ser
                805                 810                 815

Asn Glu Val Asp Leu Asp Glu Leu Ile Leu Gln Thr Asp Ala Tyr Ser
            820                 825                 830

Gly Ala Glu Ile Val Ala Val Cys Arg Glu Ala Ala Leu Leu Ala Leu
        835                 840                 845

Glu Glu Asp Ile Gln Ala Asn Leu Ile Met Lys Arg His Phe Thr Gln
    850                 855                 860

Ala Leu Ser Thr Val Thr Pro Arg Ile Pro Glu Ser Leu Arg Arg Phe
865                 870                 875                 880

Tyr Glu Asp Tyr Gln Glu Lys Ser Gly Leu His Thr Leu
                885                 890

<210>31
<211>420
<212>DNA
<213>Homo sapiens

<220>
<221> CDS
<222>(3)..(261)

<400>31

tg cag aag gtg acc ctg ggc ctg ctt gtg ttc ctg gca ggc ttt cct 47
Gln Lys Val Thr Leu Gly Leu Leu Val Phe Leu Ala Gly Phe Pro
1               5                   10                  15

gtc ctg gac gcc aat gac cta gaa gat aaa aac agt cct ttc tac tat 95
Val Leu Asp Ala Asn Asp Leu Glu Asp Lys Asn Ser Pro Phe Tyr Tyr
                20                  25                  30

gac tgg cac agc ctc cag gtt ggc ggg ctc atc tgc gct ggg gtt ctg 143
Asp Trp His Ser Leu Gln Val Gly Gly Leu Ile Cys Ala Gly Val Leu
            35                  40                  45

tgc gcc atg ggc atc atc atc gtc atg agt gca aaa tgc aaa tgc aag 191
Cys Ala Met Gly Ile Ile Ile Val Met Ser Ala Lys Cys Lys Cys Lys
        50                  55                  60

ttt ggc cag aag tcc ggt cac cat cca ggg gag act cca cct ctc atc 239
Phe Gly Gln Lys Ser Gly His His Pro Gly Glu Thr Pro Pro Leu Ile
    65                  70                  75

acc cca ggc tca gcc caa agc tgatgaggac agaccagctg aaattgggtg 290
Thr Pro Gly Ser Ala Gln Ser
80                  85

gaggaccgtt ctctgtcccc aggtcctgtc tctgcacaga aacttgaact 340

ccaggatgga attcttcctc ctctgctggg actcctttgc atggcagggc 390

ctcatctcac ctctcgcaag agggtctctt 420

<210>32

<211>86
<212>PRT
<213>Homo sapiens

<400>32

Gln Lys Val Thr Leu Gly Leu Leu Val Phe Leu Ala Gly Phe Pro Val
1               5                   10                  15

Leu Asp Ala Asn Asp Leu Glu Asp Lys Asn Ser Pro Phe Tyr Tyr Asp
            20                  25                  30

Trp His Ser Leu Gln Val Gly Gly Leu Ile Cys Ala Gly Val Leu Cys
        35                  40                  45

Ala Met Gly Ile Ile Ile Val Met Ser Ala Lys Cys Lys Cys Lys Phe
    50                  55                  60

Gly Gln Lys Ser Gly His His Pro Gly Glu Thr Pro Pro Leu Ile Thr
65                  70                  75                  80

Pro Gly Ser Ala Gln Ser
                85

<210>33
<211>462
<212>DNA
<213>Homo sapiens

<220>
<221> CDS
<222>(71)..(419)

<400>33

tcagcctggt gaaccacaca gaggctgggg cgaggaggat accatctgtc          50

agtcttggct ggatgacatc atg gga agg ggg tat agt ggg gcc ttg cag    100
                      Met Gly Arg Gly Tyr Ser Gly Ala Leu Gln
                      1               5                   10

gcc aga ggt ggc ttg gag gag ccc ctg gaa aga ggc tta aga ggc cag  148
Ala Arg Gly Gly Leu Glu Glu Pro Leu Glu Arg Gly Leu Arg Gly Gln
                15                  20                  25

cgc tct gac atg cag aag gtg acc ctg ggc ctg ctt gtg ttc ctg gca  196
Arg Ser Asp Met Gln Lys Val Thr Leu Gly Leu Leu Val Phe Leu Ala
            30                  35                  40

ggc ttt cct gtc ctg gac gcc aat gac cta gaa gat aaa aac agt cct  244
Gly Phe Pro Val Leu Asp Ala Asn Asp Leu Glu Asp Lys Asn Ser Pro
        45                  50                  55

ttc tac tat gac tgg cac agc ctc cag gtt ggc ggg ctc atc tgc gct  292
Phe Tyr Tyr Asp Trp His Ser Leu Gln Val Gly Gly Leu Ile Cys Ala

60                  65                  70

ggg gtt ctg tgc gcc atg ggc atc atc atc gtc atg agt gca aaa tgc     340
Gly Val Leu Cys Ala Met Gly Ile Ile Ile Val Met Ser Ala Lys Cys
 75                  80                  85                  90

aaa tgc aag ttt ggc cag aag tcc ggt cac cat cca ggg gag act cca     388
Lys Cys Lys Phe Gly Gln Lys Ser Gly His His Pro Gly Glu Thr Pro
                 95                 100                 105

cct ctc atc acc cca ggc tca gcc caa agc tgatgaggac agaccagctg      438
Pro Leu Ile Thr Pro Gly Ser Ala Gln Ser
            110                 115

aaattgggtg gaggaccgtt ctct                                          462

<210>34
<211>116
<212>PRT
<213>Homo sapiens

<400>34

Met Gly Arg Gly Tyr Ser Gly Ala Leu Gln Ala Arg Gly Gly Leu Glu
  1               5                  10                  15

Glu Pro Leu Glu Arg Gly Leu Arg Gly Gln Arg Ser Asp Met Gln Lys
             20                  25                  30

Val Thr Leu Gly Leu Leu Val Phe Leu Ala Gly Phe Pro Val Leu Asp
         35                  40                  45

Ala Asn Asp Leu Glu Asp Lys Asn Ser Pro Phe Tyr Tyr Asp Trp His
     50                  55                  60

Ser Leu Gln Val Gly Gly Leu Ile Cys Ala Gly Val Leu Cys Ala Met
 65                  70                  75                  80

Gly Ile Ile Ile Val Met Ser Ala Lys Cys Lys Cys Lys Phe Gly Gln
                 85                  90                  95

Lys Ser Gly His His Pro Gly Glu Thr Pro Pro Leu Ile Thr Pro Gly
            100                 105                 110

Ser Ala Gln Ser
        115

<210>35
<211>1638
<212>DNA
<213>Homo sapiens

<220>
<221> CDS
<222>(10)..(1546)

<400>35

ttatctaat atg ttt gtt tta gct aca tct tta tca agc caa gtg aat     48
          Met Phe Val Leu Ala Thr Ser Leu Ser Ser Gln Val Asn
            1               5                   10

cct gac tgg cga aca tat atc atg ctt gca gta tat ttt cta att tta     96
Pro Asp Trp Arg Thr Tyr Ile Met Leu Ala Val Tyr Phe Leu Ile Leu
     15                  20                  25

ctt gtt att gga tat tat ggt tat aag caa gca acc gga gat tta agt    144
Leu Val Ile Gly Tyr Tyr Gly Tyr Lys Gln Ala Thr Gly Asp Leu Ser
 30                  35                  40                  45

gaa tat atg ctt ggc gaa aga aat att ggt cca tat gtc act gcc tta    192
Glu Tyr Met Leu Gly Glu Arg Asn Ile Gly Pro Tyr Val Thr Ala Leu
                 50                  55                  60

tct gcc gga gct tca gat atg agc ggt tgg atg att atg gga tta cct    240
Ser Ala Gly Ala Ser Asp Met Ser Gly Trp Met Ile Met Gly Leu Pro
             65                  70                  75

gga gaa gtt tat act aca ggt tta tca gca gca tgg tta gct att ggg    288
Gly Glu Val Tyr Thr Thr Gly Leu Ser Ala Ala Trp Leu Ala Ile Gly
         80                  85                  90

tta act atc gga gct tat gtt aac tac ata ctt gta gca cca aga ctt    336
Leu Thr Ile Gly Ala Tyr Val Asn Tyr Ile Leu Val Ala Pro Arg Leu
     95                 100                 105

cgt gtg tac act gaa aaa gcc aat gac tca att aca ttg cct aat tac    384
Arg Val Tyr Thr Glu Lys Ala Asn Asp Ser Ile Thr Leu Pro Asn Tyr
110                 115                 120                 125

ttt aca cat cgt ctt aat gat aat tcc aat att att aaa att atc tct    432
Phe Thr His Arg Leu Asn Asp Asn Ser Asn Ile Ile Lys Ile Ile Ser
                130                 135                 140

ggt ggt atc att gtt gta ttt ttt aca ctc tat act cat tca ggt atg    480
Gly Gly Ile Ile Val Val Phe Phe Thr Leu Tyr Thr His Ser Gly Met
            145                 150                 155

gta tca ggt ggt aaa tta ttt gat agt gct ttt ggt tta gac tat cat    528
Val Ser Gly Gly Lys Leu Phe Asp Ser Ala Phe Gly Leu Asp Tyr His
        160                 165                 170

att gga ctt att tta atc tct gtc att gta att tta tat act ttt ttt    576
Ile Gly Leu Ile Leu Ile Ser Val Ile Val Ile Leu Tyr Thr Phe Phe
    175                 180                 185

ggt ggc tat tta gca gtg tcg tta act gac ttt ttc caa ggg gtt gtc    624
Gly Gly Tyr Leu Ala Val Ser Leu Thr Asp Phe Phe Gln Gly Val Val
190                 195                 200                 205

atg tta att gcg atg gtt atg gta cct att gta gcc atg atg cag ctc    672
Met Leu Ile Ala Met Val Met Val Pro Ile Val Ala Met Met Gln Leu

                210                 215                 220

gga ggt atg gat gct ttt tca caa gca gca aca tta aaa cct act aat 720
Gly Gly Met Asp Ala Phe Ser Gln Ala Ala Thr Leu Lys Pro Thr Asn
            225                 230                 235

tta gat tta ttt aaa gga aca act att ata ggc atc att tca ttc ttt 768
Leu Asp Leu Phe Lys Gly Thr Thr Ile Ile Gly Ile Ile Ser Phe Phe
        240                 245                 250

gct tgg gga tta ggc tat ttt ggc cag cct cat atc att gta cga ttt 816
Ala Trp Gly Leu Gly Tyr Phe Gly Gln Pro His Ile Ile Val Arg Phe
    255                 260                 265

atg tct atc aaa tcc gta cga caa tta aaa acg tct aga aga ttt ggt 864
Met Ser Ile Lys Ser Val Arg Gln Leu Lys Thr Ser Arg Arg Phe Gly
270                 275                 280                 285

att agt tgg atg gct att agt tta atc ggt gca gta tgt gtt gga tta 912
Ile Ser Trp Met Ala Ile Ser Leu Ile Gly Ala Val Cys Val Gly Leu
                290                 295                 300

att ggc att tcg ttt gta caa gat aaa ggt gtt gaa tta aaa gat cca 960
Ile Gly Ile Ser Phe Val Gln Asp Lys Gly Val Glu Leu Lys Asp Pro
            305                 310                 315

gaa aca cta ttt att tta atg gga caa att tta ttc cat cct ctt gta 1008
Glu Thr Leu Phe Ile Leu Met Gly Gln Ile Leu Phe His Pro Leu Val
        320                 325                 330

ggt ggg ttc cta ctt gca gcc att ttg gca gca att atg agt acg att 1056
Gly Gly Phe Leu Leu Ala Ala Ile Leu Ala Ala Ile Met Ser Thr Ile
    335                 340                 345

tct tcc caa tta ctt gtg act tca agt tca ctt aca gaa gat ttt tac 1104
Ser Ser Gln Leu Leu Val Thr Ser Ser Ser Leu Thr Glu Asp Phe Tyr
350                 355                 360                 365

aag tta att cgt ggt gaa gaa gca gca aag caa cat aag aaa gaa ttt 1152
Lys Leu Ile Arg Gly Glu Glu Ala Ala Lys Gln His Lys Lys Glu Phe
                370                 375                 380

tta tta gtg ggt cga tta tct gtt gta gtc gtt gcg att atc tcc atc 1200
Leu Leu Val Gly Arg Leu Ser Val Val Val Val Ala Ile Ile Ser Ile
            385                 390                 395

ctc att gca tgg acg cca aat gac act atc tta aat ctt gtt ggt aac 1248
Leu Ile Ala Trp Thr Pro Asn Asp Thr Ile Leu Asn Leu Val Gly Asn
        400                 405                 410

gct tgg gct gga ttc ggt gca gca ttt ggt cca ctg gta tta tta tct 1296
Ala Trp Ala Gly Phe Gly Ala Ala Phe Gly Pro Leu Val Leu Leu Ser
    415                 420                 425

ctc tat tcg aaa ggt tta agt cgt act gga gct att tct gga atg tta 1344
Leu Tyr Ser Lys Gly Leu Ser Arg Thr Gly Ala Ile Ser Gly Met Leu
430                 435                 440                 445

tca gga gca att gtc gtc att ctt tgg att gtg ttt gtt aaa cca tta 1392
Ser Gly Ala Ile Val Val Ile Leu Trp Ile Val Phe Val Lys Pro Leu
                450                 455                 460

gga gca tat aat gat ttc ttt aat tta tat gaa att att cct ggt ttc 1440
Gly Ala Tyr Asn Asp Phe Phe Asn Leu Tyr Glu Ile Ile Pro Gly Phe
            465                 470                 475

tta aca agt ctt att gtg aca tat gta gtg agt ctt gta act aaa aag 1488
Leu Thr Ser Leu Ile Val Thr Tyr Val Val Ser Leu Val Thr Lys Lys
        480                 485                 490

cca gat ctc aat gtt caa aaa gat tta gaa gac gtc aaa cgt att gta 1536
Pro Asp Leu Asn Val Gln Lys Asp Leu Glu Asp Val Lys Arg Ile Val
    495                 500                 505

aaa gga caa taaattaata atattcaacg atgcttaatg tcaatattat 1585
Lys Gly Gln
510

ttcaattagt gcattactct tataatatga aacacaaata aatttttata 1635

cat 1638

<210>36
<211>512
<212>PRT
<213>Homo sapiens

<400>36

Met Phe Val Leu Ala Thr Ser Leu Ser Ser Gln Val Asn Pro Asp Trp
1               5                   10                  15

Arg Thr Tyr Ile Met Leu Ala Val Tyr Phe Leu Ile Leu Leu Val Ile
            20                  25                  30

Gly Tyr Tyr Gly Tyr Lys Gln Ala Thr Gly Asp Leu Ser Glu Tyr Met
        35                  40                  45

Leu Gly Glu Arg Asn Ile Gly Pro Tyr Val Thr Ala Leu Ser Ala Gly
    50                  55                  60

Ala Ser Asp Met Ser Gly Trp Met Ile Met Gly Leu Pro Gly Glu Val
65                  70                  75                  80

Tyr Thr Thr Gly Leu Ser Ala Ala Trp Leu Ala Ile Gly Leu Thr Ile
                85                  90                  95

Gly Ala Tyr Val Asn Tyr Ile Leu Val Ala Pro Arg Leu Arg Val Tyr
            100                 105                 110

Thr Glu Lys Ala Asn Asp Ser Ile Thr Leu Pro Asn Tyr Phe Thr His
        115                 120                 125

Arg Leu Asn Asp Asn Ser Asn Ile Ile Lys Ile Ile Ser Gly Gly Ile
    130                 135                 140

Ile Val Val Phe Phe Thr Leu Tyr Thr His Ser Gly Met Val Ser Gly
145                 150                 155                 160

Gly Lys Leu Phe Asp Ser Ala Phe Gly Leu Asp Tyr His Ile Gly Leu
                165                 170                 175

Ile Leu Ile Ser Val Ile Val Ile Leu Tyr Thr Phe Phe Gly Gly Tyr
            180                 185                 190

Leu Ala Val Ser Leu Thr Asp Phe Phe Gln Gly Val Val Met Leu Ile
        195                 200                 205

Ala Met Val Met Val Pro Ile Val Ala Met Met Gln Leu Gly Gly Met
    210                 215                 220

Asp Ala Phe Ser Gln Ala Ala Thr Leu Lys Pro Thr Asn Leu Asp Leu
225                 230                 235                 240

Phe Lys Gly Thr Thr Ile Ile Gly Ile Ile Ser Phe Phe Ala Trp Gly
                245                 250                 255

Leu Gly Tyr Phe Gly Gln Pro His Ile Ile Val Arg Phe Met Ser Ile
            260                 265                 270

Lys Ser Val Arg Gln Leu Lys Thr Ser Arg Arg Phe Gly Ile Ser Trp
        275                 280                 285

Met Ala Ile Ser Leu Ile Gly Ala Val Cys Val Gly Leu Ile Gly Ile
    290                 295                 300

Ser Phe Val Gln Asp Lys Gly Val Glu Leu Lys Asp Pro Glu Thr Leu
305                 310                 315                 320

Phe Ile Leu Met Gly Gln Ile Leu Phe His Pro Leu Val Gly Gly Phe
                325                 330                 335

Leu Leu Ala Ala Ile Leu Ala Ala Ile Met Ser Thr Ile Ser Ser Gln
            340                 345                 350

Leu Leu Val Thr Ser Ser Ser Leu Thr Glu Asp Phe Tyr Lys Leu Ile
        355                 360                 365

Arg Gly Glu Glu Ala Ala Lys Gln His Lys Lys Glu Phe Leu Leu Val
    370                 375                 380

Gly Arg Leu Ser Val Val Val Val Ala Ile Ile Ser Ile Leu Ile Ala
385                 390                 395                 400

Trp Thr Pro Asn Asp Thr Ile Leu Asn Leu Val Gly Asn Ala Trp Ala
                405                 410                 415

Gly Phe Gly Ala Ala Phe Gly Pro Leu Val Leu Leu Ser Leu Tyr Ser
            420                 425                 430

Lys Gly Leu Ser Arg Thr Gly Ala Ile Ser Gly Met Leu Ser Gly Ala
        435                 440                 445

Ile Val Val Ile Leu Trp Ile Val Phe Val Lys Pro Leu Gly Ala Tyr
    450                 455                 460

Asn Asp Phe Phe Asn Leu Tyr Glu Ile Ile Pro Gly Phe Leu Thr Ser
465                 470                 475                 480

Leu Ile Val Thr Tyr Val Val Ser Leu Val Thr Lys Lys Pro Asp Leu
                485                 490                 495

Asn Val Gln Lys Asp Leu Glu Asp Val Lys Arg Ile Val Lys Gly Gln
            500                 505                 510

<210>37
<211>1146
<212>DNA
<213>Homo sapiens

<220>
<221> CDS
<222>(31)..(1117)

<400>37

ctagctcgac agcttcccgg cggctgcgcg atg gac agc ccc gag gtg acc      51
                                 Met Asp Ser Pro Glu Val Thr
                                 1               5

ttc act ctc gcc tat ctg gtg ttc gcc gtg tgc ttc gtg ttc acg ccc      99
Phe Thr Leu Ala Tyr Leu Val Phe Ala Val Cys Phe Val Phe Thr Pro
        10                  15                  20

aac gag ttc cac gcg gcg ggg ctc acg gtg cag aac ctg ctg tcg ggc      147
Asn Glu Phe His Ala Ala Gly Leu Thr Val Gln Asn Leu Leu Ser Gly
    25                  30                  35

tgg ctg ggc agc gag gac gcc gcc ttc gtg ccc ttc cac ttg cgc cgc      195
Trp Leu Gly Ser Glu Asp Ala Ala Phe Val Pro Phe His Leu Arg Arg
40                  45                  50                  55

acg gcc gcc acg ctg ttg tgc cac tcg ctg ctg ccg ctc ggc tac tat      243
Thr Ala Ala Thr Leu Leu Cys His Ser Leu Leu Pro Leu Gly Tyr Tyr
                60                  65                  70

gtg ggc atg tgc ctt gcg gct tca gaa aag cgg ctc cac gcc ctc agc      291
Val Gly Met Cys Leu Ala Ala Ser Glu Lys Arg Leu His Ala Leu Ser
            75                  80                  85

cag gcc cct gag gcc tgg cgg ctc ttc ctg ctg ctg gcc gtg acc ctc      339
Gln Ala Pro Glu Ala Trp Arg Leu Phe Leu Leu Leu Ala Val Thr Leu
        90                  95                  100

ccc tcc atc gcc tgc atc ctg atc tac tac tgg tcc cgt gac cgg tgg 387
Pro Ser Ile Ala Cys Ile Leu Ile Tyr Tyr Trp Ser Arg Asp Arg Trp
    105                 110                 115

gcc tgc cac cca ctg gcg cgc acc ctg gcc ctc tac gcc ctc cca cag 435
Ala Cys His Pro Leu Ala Arg Thr Leu Ala Leu Tyr Ala Leu Pro Gln
120                 125                 130                 135

tct ggc tgg cag gct gtt gcc tcc tct gtc aac act gag ttc cgg cgg 483
Ser Gly Trp Gln Ala Val Ala Ser Ser Val Asn Thr Glu Phe Arg Arg
                140                 145                 150

att gac aag ttt gcc acc ggt gca cca ggt gcc cgt gtg att gtg aca 531
Ile Asp Lys Phe Ala Thr Gly Ala Pro Gly Ala Arg Val Ile Val Thr
            155                 160                 165

gac acg tgg gtg atg aag gta acc acc tac cga gtg cac gtg gcc cag 579
Asp Thr Trp Val Met Lys Val Thr Thr Tyr Arg Val His Val Ala Gln
        170                 175                 180

cag cag gac gtg cac ctg act gtg acg gag tct cgg cag cat gag ctc 627
Gln Gln Asp Val His Leu Thr Val Thr Glu Ser Arg Gln His Glu Leu
    185                 190                 195

tcg cca gac tcg aac ttg ccc gtg cag ctc ctc acc atc cgt gtg gcc 675
Ser Pro Asp Ser Asn Leu Pro Val Gln Leu Leu Thr Ile Arg Val Ala
200                 205                 210                 215

agc acc aac cct gct gtg cag gcc ttt gac atc agg ctg aac tcc act 723
Ser Thr Asn Pro Ala Val Gln Ala Phe Asp Ile Arg Leu Asn Ser Thr
                220                 225                 230

gag tac ggg gag ctc tgc gag aag ctc cgg gca ccc atc cgc agg gca 771
Glu Tyr Gly Glu Leu Cys Glu Lys Leu Arg Ala Pro Ile Arg Arg Ala
            235                 240                 245

gcc cat gtg gtc atc cac cag agc ctg ggc gac ctg ttc ctg gag aca 819
Ala His Val Val Ile His Gln Ser Leu Gly Asp Leu Phe Leu Glu Thr
        250                 255                 260

ttt gcc tcc ctg gta gag gtc aac ccg gcc tac tca gtg ccc agc agc 867
Phe Ala Ser Leu Val Glu Val Asn Pro Ala Tyr Ser Val Pro Ser Ser
    265                 270                 275

cag gag ctg gag gcc tgc ata ggc tgc atg cag aca cgt gcc agc gtg 915
Gln Glu Leu Glu Ala Cys Ile Gly Cys Met Gln Thr Arg Ala Ser Val
280                 285                 290                 295

aag ctg gtg aag acc tgc cag gag gca gcc aca ggc gag tgc cag cag 963
Lys Leu Val Lys Thr Cys Gln Glu Ala Ala Thr Gly Glu Cys Gln Gln
                300                 305                 310

tgt tac tgc cgc ccc atg tgg tgc ctc acc tgc atg ggc aag tgg ttc 1011
Cys Tyr Cys Arg Pro Met Trp Cys Leu Thr Cys Met Gly Lys Trp Phe
            315                 320                 325

gcc agc cgc cag gac ccc ctg cgc cct gac acc tgg ctg gcc agc cgc 1059

Ala Ser Arg Gln Asp Pro Leu Arg Pro Asp Thr Trp Leu Ala Ser Arg
        330                 335                 340

gtg ccc tgc ccc acc tgc cgc gca cgc ttc tgc atc ctg gat gtg tgc 1107
Val Pro Cys Pro Thr Cys Arg Ala Arg Phe Cys Ile Leu Asp Val Cys
    345                 350                 355

acc gtg cgc tgagtgggct ggggccttga ggtgactctg 1146
Thr Val Arg
360

<210>38
<211>362
<212>PRT
<213>Homo sapiens

<400>38

Met Asp Ser Pro Glu Val Thr Phe Thr Leu Ala Tyr Leu Val Phe Ala
1               5                   10                  15

Val Cys Phe Val Phe Thr Pro Asn Glu Phe His Ala Ala Gly Leu Thr
            20                  25                  30

Val Gln Asn Leu Leu Ser Gly Trp Leu Gly Ser Glu Asp Ala Ala Phe
        35                  40                  45

Val Pro Phe His Leu Arg Arg Thr Ala Ala Thr Leu Leu Cys His Ser
    50                  55                  60

Leu Leu Pro Leu Gly Tyr Tyr Val Gly Met Cys Leu Ala Ala Ser Glu
65                  70                  75                  80

Lys Arg Leu His Ala Leu Ser Gln Ala Pro Glu Ala Trp Arg Leu Phe
                85                  90                  95

Leu Leu Leu Ala Val Thr Leu Pro Ser Ile Ala Cys Ile Leu Ile Tyr
            100                 105                 110

Tyr Trp Ser Arg Asp Arg Trp Ala Cys His Pro Leu Ala Arg Thr Leu
        115                 120                 125

Ala Leu Tyr Ala Leu Pro Gln Ser Gly Trp Gln Ala Val Ala Ser Ser
    130                 135                 140

Val Asn Thr Glu Phe Arg Arg Ile Asp Lys Phe Ala Thr Gly Ala Pro
145                 150                 155                 160

Gly Ala Arg Val Ile Val Thr Asp Thr Trp Val Met Lys Val Thr Thr
                165                 170                 175

Tyr Arg Val His Val Ala Gln Gln Gln Asp Val His Leu Thr Val Thr
            180                 185                 190

Glu Ser Arg Gln His Glu Leu Ser Pro Asp Ser Asn Leu Pro Val Gln
        195                 200                 205

Leu Leu Thr Ile Arg Val Ala Ser Thr Asn Pro Ala Val Gln Ala Phe
    210                 215                 220

Asp Ile Arg Leu Asn Ser Thr Glu Tyr Gly Glu Leu Cys Glu Lys Leu
225                 230                 235                 240

Arg Ala Pro Ile Arg Arg Ala Ala His Val Val Ile His Gln Ser Leu
                245                 250                 255

Gly Asp Leu Phe Leu Glu Thr Phe Ala Ser Leu Val Glu Val Asn Pro
            260                 265                 270

Ala Tyr Ser Val Pro Ser Ser Gln Glu Leu Glu Ala Cys Ile Gly Cys
        275                 280                 285

Met Gln Thr Arg Ala Ser Val Lys Leu Val Lys Thr Cys Gln Glu Ala
    290                 295                 300

Ala Thr Gly Glu Cys Gln Gln Cys Tyr Cys Arg Pro Met Trp Cys Leu
305                 310                 315                 320

Thr Cys Met Gly Lys Trp Phe Ala Ser Arg Gln Asp Pro Leu Arg Pro
                325                 330                 335

Asp Thr Trp Leu Ala Ser Arg Val Pro Cys Pro Thr Cys Arg Ala Arg
            340                 345                 350

Phe Cys Ile Leu Asp Val Cys Thr Val Arg
        355                 360

<210>39
<211>1115
<212>DNA
<213>Homo sapiens

<220>
<221> CDS
<222>(19)..(1105)

<400>39

ttcgcccttg gctgcgcg atg gac agc ccc gag gtg acc ttc act ctc gcc      51
                    Met Asp Ser Pro Glu Val Thr Phe Thr Leu Ala
                    1               5                   10

tat ctg gtg ttc gcc gtg tgc ttc gtg ttc acg ccc aac gag ttc cac      99
Tyr Leu Val Phe Ala Val Cys Phe Val Phe Thr Pro Asn Glu Phe His
            15                  20                  25

gcg gcg ggg ctc acg gtg cag aac ctg ctg tcg ggc tgg ctg ggc agc      147
Ala Ala Gly Leu Thr Val Gln Asn Leu Leu Ser Gly Trp Leu Gly Ser
        30                  35                  40

gag gac gcc gcc ttc gtg ccc ttc cac ttg cgc cgc acg gcc gcc acg      195
Glu Asp Ala Ala Phe Val Pro Phe His Leu Arg Arg Thr Ala Ala Thr
    45                  50                  55

ctg ttg tgc cac tcg ctg ctg ccg ctc ggc tac tac gtg ggc atg tgc 243
Leu Leu Cys His Ser Leu Leu Pro Leu Gly Tyr Tyr Val Gly Met Cys
60                  65                  70                  75

ctt gcg gct tca gaa aag cgg ctc cac gcc ctc agc cag gcc cct gag 291
Leu Ala Ala Ser Glu Lys Arg Leu His Ala Leu Ser Gln Ala Pro Glu
                80                  85                  90

gcc tgg cgg ctc ttc ctg ctg ctg gcc gtg acc ctc ccc tcc att gcc 339
Ala Trp Arg Leu Phe Leu Leu Leu Ala Val Thr Leu Pro Ser Ile Ala
            95                  100                 105

tgc atc ctg atc tac tac tgg tcc cgt gac cgg tgg gcc tgc cac cca 387
Cys Ile Leu Ile Tyr Tyr Trp Ser Arg Asp Arg Trp Ala Cys His Pro
        110                 115                 120

ctg gcg cgc acc ctg gcc ctc tac gcc ctc cca cag tct ggc tgg cag 435
Leu Ala Arg Thr Leu Ala Leu Tyr Ala Leu Pro Gln Ser Gly Trp Gln
    125                 130                 135

gct gtt gcc tcc tct gtc aac act gag ttc cgg cgg att gac aag ttt 483
Ala Val Ala Ser Ser Val Asn Thr Glu Phe Arg Arg Ile Asp Lys Phe
140                 145                 150                 155

gcc acc ggt gca cca ggt gcc cgt gtg att gtg aca gac acg tgg gtg 531
Ala Thr Gly Ala Pro Gly Ala Arg Val Ile Val Thr Asp Thr Trp Val
                160                 165                 170

atg aag gta acc acc tac cga gtg cac gtg gcc cag cag cag gac gtg 579
Met Lys Val Thr Thr Tyr Arg Val His Val Ala Gln Gln Gln Asp Val
            175                 180                 185

cac ctg act gtg acg gag tct cgg cag cat gag ctc tcg cca gac tcg 627
His Leu Thr Val Thr Glu Ser Arg Gln His Glu Leu Ser Pro Asp Ser
        190                 195                 200

aac ttg ccc gtg cag ctc ctc acc atc cgt gtg gcc agc acc aac cct 675
Asn Leu Pro Val Gln Leu Leu Thr Ile Arg Val Ala Ser Thr Asn Pro
    205                 210                 215

gct gtg cag gcc ttt gac atc tgg ctg aac tcc act gag tac ggg gag 723
Ala Val Gln Ala Phe Asp Ile Trp Leu Asn Ser Thr Glu Tyr Gly Glu
220                 225                 230                 235

ctc tgc gag aag ctc cgg gca ccc atc cgc agg gca gcc cat gtg gtc 771
Leu Cys Glu Lys Leu Arg Ala Pro Ile Arg Arg Ala Ala His Val Val
                240                 245                 250

atc cac cag agc ctg ggc gac ctg ttc ctg gag aca ttt gcc tcc ctg 819
Ile His Gln Ser Leu Gly Asp Leu Phe Leu Glu Thr Phe Ala Ser Leu
            255                 260                 265

gta gag gtc aac ccg gcc tac tca gtg ccc agc agc cag gag ctg gag 867
Val Glu Val Asn Pro Ala Tyr Ser Val Pro Ser Ser Gln Glu Leu Glu
        270                 275                 280

gcc tgc ata ggc tgc atg cag aca cgt gcc agc gtg aag ctg gtg aag 915

Ala Cys Ile Gly Cys Met Gln Thr Arg Ala Ser Val Lys Leu Val Lys
    285                 290                 295

acc tgc cag gag gca gcc aca ggc gag tgc cag cag tgt tac tgc cgc     963
Thr Cys Gln Glu Ala Ala Thr Gly Glu Cys Gln Gln Cys Tyr Cys Arg
300                 305                 310                 315

ccc atg tgg tgc ctc acc tgc atg ggc aag tgg ttc gcc agc cgc cag     1011
Pro Met Trp Cys Leu Thr Cys Met Gly Lys Trp Phe Ala Ser Arg Gln
                320                 325                 330

gac ccc ctg cgc cct gac acc tgg ctg gcc agc cgc gtg ccc tgc ccc     1059
Asp Pro Leu Arg Pro Asp Thr Trp Leu Ala Ser Arg Val Pro Cys Pro
            335                 340                 345

acc tgc cgc gca cgc ttc tgc atc ctg gat gtg tgc acc gtg cgc         1104
Thr Cys Arg Ala Arg Phe Cys Ile Leu Asp Val Cys Thr Val Arg
        350                 355                 360

tgatgtggcg g                                                        1115

<210>40
<211>362
<212>PRT
<213>Homo sapiens

<400>40

Met Asp Ser Pro Glu Val Thr Phe Thr Leu Ala Tyr Leu Val Phe Ala
  1               5                  10                  15

Val Cys Phe Val Phe Thr Pro Asn Glu Phe His Ala Ala Gly Leu Thr
             20                  25                  30

Val Gln Asn Leu Leu Ser Gly Trp Leu Gly Ser Glu Asp Ala Ala Phe
         35                  40                  45

Val Pro Phe His Leu Arg Arg Thr Ala Ala Thr Leu Leu Cys His Ser
     50                  55                  60

Leu Leu Pro Leu Gly Tyr Tyr Val Gly Met Cys Leu Ala Ala Ser Glu
 65                  70                  75                  80

Lys Arg Leu His Ala Leu Ser Gln Ala Pro Glu Ala Trp Arg Leu Phe
                 85                  90                  95

Leu Leu Leu Ala Val Thr Leu Pro Ser Ile Ala Cys Ile Leu Ile Tyr
            100                 105                 110

Tyr Trp Ser Arg Asp Arg Trp Ala Cys His Pro Leu Ala Arg Thr Leu
        115                 120                 125

Ala Leu Tyr Ala Leu Pro Gln Ser Gly Trp Gln Ala Val Ala Ser Ser
    130                 135                 140

Val Asn Thr Glu Phe Arg Arg Ile Asp Lys Phe Ala Thr Gly Ala Pro
145                 150                 155                 160

Gly Ala Arg Val Ile Val Thr Asp Thr Trp Val Met Lys Val Thr Thr
                165                 170                 175

Tyr Arg Val His Val Ala Gln Gln Gln Asp Val His Leu Thr Val Thr
            180                 185                 190

Glu Ser Arg Gln His Glu Leu Ser Pro Asp Ser Asn Leu Pro Val Gln
        195                 200                 205

Leu Leu Thr Ile Arg Val Ala Ser Thr Asn Pro Ala Val Gln Ala Phe
    210                 215                 220

Asp Ile Trp Leu Asn Ser Thr Glu Tyr Gly Glu Leu Cys Glu Lys Leu
225                 230                 235                 240

Arg Ala Pro Ile Arg Arg Ala Ala His Val Val Ile His Gln Ser Leu
                245                 250                 255

Gly Asp Leu Phe Leu Glu Thr Phe Ala Ser Leu Val Glu Val Asn Pro
            260                 265                 270

Ala Tyr Ser Val Pro Ser Ser Gln Glu Leu Glu Ala Cys Ile Gly Cys
        275                 280                 285

Met Gln Thr Arg Ala Ser Val Lys Leu Val Lys Thr Cys Gln Glu Ala
    290                 295                 300

Ala Thr Gly Glu Cys Gln Gln Cys Tyr Cys Arg Pro Met Trp Cys Leu
305                 310                 315                 320

Thr Cys Met Gly Lys Trp Phe Ala Ser Arg Gln Asp Pro Leu Arg Pro
                325                 330                 335

Asp Thr Trp Leu Ala Ser Arg Val Pro Cys Pro Thr Cys Arg Ala Arg
            340                 345                 350

Phe Cys Ile Leu Asp Val Cys Thr Val Arg
        355                 360

<210>41
<211>2765
<212>DNA
<213>Homo sapiens

<220>
<221> CDS
<222>(14)..(1745)

<400>41

ctggcggcgt cgc atg gag ggc tct ggg ggc ggt gcg ggc gag cgg gcg      49
               
                1               5                   10

ccg ctg ctg ggc gcg cgg cgg gcg gcg gcg gcc gcg gcg gcg gct ggg 97

        15                  20                  25

gcg ttc gcg ggc cgg cgc gcg gcg tgc ggg gcc gtg ctg ctg acg gag 145

    30                  35                  40

ctg ctg gag cgc gcc gct ttc tac ggc atc acg tcc aac ctg gtg cta 193

45                  50                  55                  60

ttc ctg aac ggg gcg ccg ttc tgc tgg gag ggc gcg cag gcc agc gag 241

                65                  70                  75

gcg ctg ctg ctc ttc atg ggc ctc acc tac ctg ggc tcg ccg ttc gga 289

            80                  85                  90

ggc tgg ctg gcc gac gcg cgg ctg ggc cgg gcg cgc gcc atc ctg ctg 337

        95                  100                 105

agc ctg gcg ctc tac ctg ctg ggc atg ctg gcc ttc ccg ctg ctg gcc 385

    110                 115                 120

gcg ccc gcc acg cga gcc gcg ctc tgc ggt tcc gcg cgc ctg ctc aac 433

125                 130                 135                 140

tgc acg gcg cct ggt ccc gac gcc gcc gcc cgc tgc tgc tca ccg gcc 481

                145                 150                 155

acc ttc gcg ggg ctg gtg ctg gtg ggc ctg ggc gtg gcc acc gtc aag 529

            160                 165                 170

gcc aac atc acg ccc ttc ggc gcc gac cag gtt aaa gat cga ggt ccg 577

        175                 180                 185

gaa gcc act agg aga ttt ttt aat tgg ttt tat tgg agc att aac ctg 625

    190                 195                 200

gga gcg atc ctg tcg tta ggt ggc att gcc tat att cag cag aac gtc 673

205                 210                 215                 220

agc ttt gtc act ggt tat gcg atc ccc act gtc tgc gtc ggc ctt gct 721

                225                 230                 235

ttt gtg gcc ttc ctc tgt ggc cag agc gtt ttc atc acc aag cct cct 769


            240                 245                 250

gat ggc agt gcc ttc acc gat atg ttc aag ata ctg acg tat tcc tgc 817

        255                 260                 265

tgt tcc cag aag cga agt gga gag cgc cag agt aat ggt gaa ggc att 865

    270                 275                 280

gga gtc ttt cag caa tct tct aaa caa agt ctg ttt gat tca tgt aag 913

285                 290                 295                 300

atg tct cat ggt ggg cca ttt aca gaa gag aaa gtg gaa gat gtg aaa 961

                305                 310                 315

gct ctg gtc aag att gtc cct gtt ttc ttg gct ttg ata cct tac tgg 1009

            320                 325                 330

aca gtg tat ttc caa atg cag aca aca tat gtt tta cag agt ctt cat 1057

        335                 340                 345

ttg agg att cca gaa att tca aat att aca acc act cct cac acg ctc 1105

    350                 355                 360

cct gca gcc tgg ctg acc atg ttt gat gct gtg ctc atc ctc ctg ctc 1153

365                 370                 375                 380

atc cct ctg aag gac aaa ctg gtc gat ccc att ttg aga aga cat ggc 1201

                385                 390                 395

ctg ctc cca tcc tcc ctg aag agg atc gcc gtg ggc atg ttc ttt gtc 1249

            400                 405                 410

atg tgc tca gcc ttt gct gca gga att ttg gag agt aaa agg ctg aac 1297

        415                 420                 425

ctt gtt aaa gag aaa acc att aat cag acc atc ggc aac gtc gtc tac 1345

    430                 435                 440

cat gct gcc gat ctg tcg ctg tgg tgg cag gtg ccg cag tac ttg ctg 1393

445                 450                 455                 460

att ggg atc agc gag atc ttt gca agt atc gca ggc ctg gaa ttt gca 1441


                465                 470                 475

tac tca gct gcc ccc aag tcc atg cag agt gcc ata atg ggc ttg ttc 1489

            480                 485                 490

ttt ttc ttc tct ggc gtc ggg tcg ttc gtg ggt tct gga ctg ctg gca 1537

        495                 500                 505

ctg gtg tct atc aaa gcc atc gga tgg atg agc agt cac aca gac ttt 1585

    510                 515                 520

ggt aat att aac ggc tgc tat ttg aac tat tac ttt ttt ctt ctg gct 1633

525                 530                 535                 540

gct att caa gga gct acc ctc ctg ctt ttc ctc att att tct gtg aaa 1681

                545                 550                 555

tat gac cat cat cga gac cat cag cga tca aga gcc aat ggc gtg ccc 1729

            560                 565                 570

acc agc agg agg gcc tgaccttcct gaggccatgt gcggtttctg aggctgacat 1784

        575

gtcagtaact gactggggtg cactgagaac aggcaagact ttaaattccc 1834

ataaaatgtc tgacttcact gaaacttgca tgttgcctgg attgatttct 1884

tctttccctc tatccaaagg agcttggtaa gtgccttact gcagcgtgtc 1934

tcctggcacg ctgggccctc cgggaggaga gctgcagatt tcgagtatgt 1984

cgcttgtcat tcaaggtctc tgtgaatcct ctagctgggt tccctttttt 2034

acagaaactc acaaatggag attgcaaagt cttggggaac tccacgtgtt 2084

agttggcatc ccagtttctt aaacaaatag tatcacctgc ttcccatagc 2134

catatctcac tgtaaaaaaa aaaattaata aactgttact tatatttaag 2184

aaagtgagga tttttttttt ttaaagataa aagcatggtc agatgctgca 2234

aggattttac ataaatgcca tatttatggt ttccttcctg agaacaatct 2284

tgctcttgcc atgttctttg atttaggctg gtagtaaaca catttcatct 2334

gctgcttcaa aaagtactta ctttttaaac catcaacatt acttttcttt 2384

cttaaggcaa ggcatgcata/agagtcattt gagaccatgt gtcccatctc 2434

aagccacaga gcaactcacg gggtacttca caccttacct agtcagagtg 2484

cttatatata gctttatttt ggtacgattg agactaaaga ctgatcatgg 2534

ttgtatgtaa ggaaaacatt cttttgaaca gaaatagtgt aattaaaaat 2584

aattgaaagt gttaaatgtg aacttgagct gtttgaccag tcacattttt 2634

gtattgttac tgtacgtgta tctggggctt ctccgtttgt taatactttt 2684

tctgtatttg ttgctgtatt tttggcataa ctttattata aaaagcatct 2734

caaatgcgaa aaaaaaaaaa aaaaaaaaaa a 2765

<210>42
<211>577
<212>PRT
<213>Homo sapiens

<400>42

<210>43
<211>1393
<212>DNA
<213>Homo sapiens

<220>
<221> CDS
<222>(4)..(523)

<400>43
   ccc atg gag gct ccg gga ccc cgc gcc ttg cgg act gcg ctc tgt ggc    48
       Met Glu Ala Pro Gly Pro Arg Ala Leu Arg Thr Ala Leu Cys Gly
       1               5                   10                  15
   ggc tgt tgc tgc ctc ctc cta tgt gcc cag ctg gct gtg gct ggt aaa    96
   Gly Cys Cys Cys Leu Leu Leu Cys Ala Gln Leu Ala Val Ala Gly Lys
                   20                  25                  30
   gga gct cga ggc ttt ggg agg gga gcc ctg atc cgc ctg aat atc tgg    144
   Gly Ala Arg Gly Phe Gly Arg Gly Ala Leu Ile Arg Leu Asn Ile Trp
               35                  40                  45
   ccg gcg gtc caa ggg gcc tgc aaa cag ctg gag gtc tgt gag cac tgc    192
   Pro Ala Val Gln Gly Ala Cys Lys Gln Leu Glu Val Cys Glu His Cys
           50                  55                  60
   gtg gag gga gac aga gcg cgc aat ctc tcc agc tgc atg tgg gag cag    240
   Val Glu Gly Asp Arg Ala Arg Asn Leu Ser Ser Cys Met Trp Glu Gln
       65                  70                  75
   tgc cgg cca gag gag cca ggt cac tgt gtg gcc caa tct gag gtg gtc    288
   Cys Arg Pro Glu Glu Pro Gly His Cys Val Ala Gln Ser Glu Val Val
   80                  85                  90                  95
   aag gaa ggt tgc tcc atc tac aac cgc tca gag gca tgt cca gct gct    336
   Lys Glu Gly Cys Ser Ile Tyr Asn Arg Ser Glu Ala Cys Pro Ala Ala
                   100                 105                 110
   cac cac cac ccc acc tat gaa ccg aag aca gtc aca aca ggt agc ccc    384
   His His His Pro Thr Tyr Glu Pro Lys Thr Val Thr Thr Gly Ser Pro
               115                 120                 125
   cca gtc cct gag gcc cac agc cct gga ttt gac ggg gcc agc ttt atc    432
   Pro Val Pro Glu Ala His Ser Pro Gly Phe Asp Gly Ala Ser Phe Ile
           130                 135                 140
   gga ggt gtc gtg ctg gtg ttg agc cta cag gcg gtg gct ttc ttt gtg    480
   Gly Gly Val Val Leu Val Leu Ser Leu Gln Ala Val Ala Phe Phe Val
       145                 150                 155
   ctg cac ttc ctc aag gcc aag gac agc acc tac cag acg ctg            522
   Leu His Phe Leu Lys Ala Lys Asp Ser Thr Tyr Gln Thr Leu
   160                 165                 170
   tgagtacctg gccagcagca agtacctgag tcccagctca cctcctggtt             572
   cctgccccac cgttcccctt cagtacccag ggtgctgtct tctccactgg 622
   caagccctca ggacggtgac agcgtgctcc atgtgagcca cacccctttt 672
   gtctcctcca gttggggtgt ttcctttgtc agatgttggc tgggaccagg 722
   actcagcctg ggccagtcta ggagcccagc tgagccctcc tgtgtctttt 772
   cccttcatgc tgccagcagg gaagagaacc agtaggtgcc agcccagcaa 822
   cctgtggccc gcgtttctgt ggctgtgggc aggagctggg ccttgtgtct 872
   agttgggttt tgctctgaga aggggagctg tgctgaggcc ctctgtgtgc 922
   cgtgtgtgct gtggggcggg tcgccacagc ctgtgttaaa gtgtttgctc 972
   ttcctctgct gcctcctctc gaggcagggg gtccttggct ggctgaggca 1022
   gtgtcacctt cctgagtgtc ctctttggcc tctgcagaat ctgacccctt 1072
   tgggcctgga ctccatcctg aggggaaagg aggatgcaga gggtggcctc 1122
   tgggcaccct tgtgggtaag cggggggcgg gggcgggaaa aactctggcc 1172
   gccagttttt ggctcctgcg ggcaccaagc aggctcagtg tctgatgctt 1222
   gacatctcct cctgtcctgg gcctggaacc tgcagctgag aaaatccctc 1272
   aaccacctcg tctcctccat cgcccctgct gggcccccca gcctgacagt 1322
   gggttgtatg cctgcctctt tccaccaact ggcctgggca ctgcccccaa 1372
   ataaaggaac tctgcactgc a 1393

<210>44
<211>173
<212>PRT
<213>Homo sapiens

<400>44

Met Glu Ala Pro Gly Pro Arg Ala Leu Arg Thr Ala Leu Cys Gly Gly
1               5                   10                  15

Cys Cys Cys Leu Leu Leu Cys Ala Gln Leu Ala Val Ala Gly Lys Gly
            20                  25                  30

Ala Arg Gly Phe Gly Arg Gly Ala Leu Ile Arg Leu Asn Ile Trp Pro
        35                  40                  45

Ala Val Gln Gly Ala Cys Lys Gln Leu Glu Val Cys Glu His Cys Val
    50                  55                  60

Glu Gly Asp Arg Ala Arg Asn Leu Ser Ser Cys Met Trp Glu Gln Cys
65                  70                  75                  80

Arg Pro Glu Glu Pro Gly His Cys Val Ala Gln Ser Glu Val Val Lys
                85                  90                  95

Glu Gly Cys Ser Ile Tyr Asn Arg Ser Glu Ala Cys Pro Ala Ala His
            100                 105                 110

His His Pro Thr Tyr Glu Pro Lys Thr Val Thr Thr Gly Ser Pro Pro
        115                 120                 125

Val Pro Glu Ala His Ser Pro Gly Phe Asp Gly Ala Ser Phe Ile Gly
    130                 135                 140

Gly Val Val Leu Val Leu Ser Leu Gln Ala Val Ala Phe Phe Val Leu
145                 150                 155                 160

His Phe Leu Lys Ala Lys Asp Ser Thr Tyr Gln Thr Leu
                165                 170

<210>45
<211>561
<212>DNA
<213>Homo sapiens

<220>
<221> CDS
<222>(4)..(523)

<400>45

ccc atg gag gct ccg gga ccc cgc gcc ttg cgg act gcg ctc tgt ggc    48
    Met Glu Ala Pro Gly Pro Arg Ala Leu Arg Thr Ala Leu Cys Gly
    1               5                   10                  15

ggc tgt tgc tgc ctc ctc cta tgt gcc cag ctg gct gtg gct ggt aaa    96
Gly Cys Cys Cys Leu Leu Leu Cys Ala Gln Leu Ala Val Ala Gly Lys
                20                  25                  30

gga gct cga ggc ttt ggg agg gga gcc ctg atc cgc ctg aat atc tgg    144
Gly Ala Arg Gly Phe Gly Arg Gly Ala Leu Ile Arg Leu Asn Ile Trp
            35                  40                  45

ccg gcg gtc caa ggg gcc tgc aaa cag ctg gag gtc tgt gag cac tgc    192
Pro Ala Val Gln Gly Ala Cys Lys Gln Leu Glu Val Cys Glu His Cys
        50                  55                  60

gtg gag gga gac aga gcg cgc aat ctc tcc agc tgc gtg tgg gag cag    240
Val Glu Gly Asp Arg Ala Arg Asn Leu Ser Ser Cys Val Trp Glu Gln
    65                  70                  75

tgc cgg cca gag gag cca gga cac tgt gtg gcc caa tct gag gtg gtc    288
Cys Arg Pro Glu Glu Pro Gly His Cys Val Ala Gln Ser Glu Val Val
80                  85                  90                  95

aag gaa ggt tgc tcc atc tac aac cgc tca gag gca tgt cca gct gct    336
Lys Glu Gly Cys Ser Ile Tyr Asn Arg Ser Glu Ala Cys Pro Ala Ala
                100                 105                 110

cac cac cac ccc acc tat gaa ccg aag aca gtc aca aca ggg agc ccc    384
His His His Pro Thr Tyr Glu Pro Lys Thr Val Thr Thr Gly Ser Pro
            115                 120                 125

cca gtc cct gag gcc cac agc cct gga ttt gac ggg gcc agc ttt atc    432
Pro Val Pro Glu Ala His Ser Pro Gly Phe Asp Gly Ala Ser Phe Ile
        130                 135                 140

gga ggt gtc gtg ctg gtg ttg agc cta cag gcg gtg gct ttc ttt gtg    480
Gly Gly Val Val Leu Val Leu Ser Leu Gln Ala Val Ala Phe Phe Val
    145                 150                 155

ctg cac ttc ctc aag gcc aag gac agc acc tac cag acg ctg            522
Leu His Phe Leu Lys Ala Lys Asp Ser Thr Tyr Gln Thr Leu
160                 165                 170

tgagtacctg gccagcagca agtacctgag tcccagctc                         561


<210>46
<211>173

<212>PRT
<213>Homo sapiens

<400>46

Met Glu Ala Pro Gly Pro Arg Ala Leu Arg Thr Ala Leu Cys Gly Gly
1               5                   10                  15

Cys Cys Cys Leu Leu Leu Cys Ala Gln Leu Ala Val Ala Gly Lys Gly
            20                  25                  30

Ala Arg Gly Phe Gly Arg Gly Ala Leu Ile Arg Leu Asn Ile Trp Pro
        35                  40                  45

Ala Val Gln Gly Ala Cys Lys Gln Leu Glu Val Cys Glu His Cys Val
    50                  55                  60

Glu Gly Asp Arg Ala Arg Asn Leu Ser Ser Cys Val Trp Glu Gln Cys
65                  70                  75                  80

Arg Pro Glu Glu Pro Gly His Cys Val Ala Gln Ser Glu Val Val Lys
                85                  90                  95

Glu Gly Cys Ser Ile Tyr Asn Arg Ser Glu Ala Cys Pro Ala Ala His
            100                 105                 110

His His Pro Thr Tyr Glu Pro Lys Thr Val Thr Thr Gly Ser Pro Pro
        115                 120                 125

Val Pro Glu Ala His Ser Pro Gly Phe Asp Gly Ala Ser Phe Ile Gly
    130                 135                 140

Gly Val Val Leu Val Leu Ser Leu Gln Ala Val Ala Phe Phe Val Leu
145                 150                 155                 160

His Phe Leu Lys Ala Lys Asp Ser Thr Tyr Gln Thr Leu
                165                 170

<210>47
<211>349
<212>DNA
<213>Homo sapiens

<220>
<221> CDS
<222>(2)..(349)

<400>47

c acc gga tcc ggt aaa gga gct cga ggc ttt ggg agg gga gcc ctg      46
  Thr Gly Ser Gly Lys Gly Ala Arg Gly Phe Gly Arg Gly Ala Leu
  1               5                   10                  15

atc cgc ctg aat atc tgg ccg gcg gtc caa ggg gcc tgc aaa cag ctg    94
Ile Arg Leu Asn Ile Trp Pro Ala Val Gln Gly Ala Cys Lys Gln Leu
                20                  25                  30

gag gtc tgt gag cac tgc gtg gag gga gac aga gcg cgc aat ctc tcc 142
Glu Val Cys Glu His Cys Val Glu Gly Asp Arg Ala Arg Asn Leu Ser
            35                  40                  45

agc tgc atg tgg gag cag tgc cgg cca gag gag cca gga cac tgt gtg 190
Ser Cys Met Trp Glu Gln Cys Arg Pro Glu Glu Pro Gly His Cys Val
        50                  55                  60

gcc caa tct gag gtg gtc aag gaa ggt tgc tcc atc tac aac cgc tca 238
Ala Gln Ser Glu Val Val Lys Glu Gly Cys Ser Ile Tyr Asn Arg Ser
    65                  70                  75

gag gca tgt cca gct gct cac cac cac ccc acc tat gaa ccg aag 'aca 286
Glu Ala Cys Pro Ala Ala His His His Pro Thr Tyr Glu Pro Lys Thr
80                  85                  90                  95

gtc aca aca ggg agc ccc cca gtc cct gag gcc cac agc cct gga ttt 334
Val Thr Thr Gly Ser Pro Pro Val Pro Glu Ala His Ser Pro Gly Phe
                100                 105                 110

gac ggg gtc gac ggc 349
Asp Gly Val Asp Gly
            115

<210>48
<211>116
<212>PRT
<213>Homo sapiens

<400>48

Thr Gly Ser Gly Lys Gly Ala Arg Gly Phe Gly Arg Gly Ala Leu Ile
1               5                   10                  15

Arg Leu Asn Ile Trp Pro Ala Val Gln Gly Ala Cys Lys Gln Leu Glu
            20                  25                  30

Val Cys Glu His Cys Val Glu Gly Asp Arg Ala Arg Asn Leu Ser Ser
        35                  40                  45

Cys Met Trp Glu Gln Cys Arg Pro Glu Glu Pro Gly His Cys Val Ala
    50                  55                  60

Gln Ser Glu Val Val Lys Glu Gly Cys Ser Ile Tyr Asn Arg Ser Glu
65                  70                  75                  80

Ala Cys Pro Ala Ala His His His Pro Thr Tyr Glu Pro Lys Thr Val
                85                  90                  95

Thr Thr Gly Ser Pro Pro Val Pro Glu Ala His Ser Pro Gly Phe Asp
            100                 105                 110

Gly Val Asp Gly
        115

<210>49
<211>788

<212>DNA
<213>Homo sapiens

<220>
<221> CDS
<222>(23)..(464)

<400>49

cttttgcctt tatgcaacca ac atg gag att ttg tac cat gtc ctg ttc 49
Met Glu Ile Leu Tyr His Val Leu Phe
1               5

tta gtg ctt gaa tgt cct aac ctg aag ctg aag aag ccg ccc tgg ctg 97
Leu Val Leu Glu Cys Pro Asn Leu Lys Leu Lys Lys Pro Pro Trp Leu
10                  15                  20                  25

cac atg ctg tcg gcc atg act gta tgc tct ggt ggt ggt gtc ttc ctc 145
His Met Leu Ser Ala Met Thr Val Cys Ser Gly Gly Gly Val Phe Leu
                30                  35                  40

att acc gga gga atc att tat gat gtt att gtt gaa cct cca agt gtt 193
Ile Thr Gly Gly Ile Ile Tyr Asp Val Ile Val Glu Pro Pro Ser Val
            45                  50                  55

ggc tct atg act gat gaa cat ggg cat cag agg cca gta gct ttc ttt 241
Gly Ser Met Thr Asp Glu His Gly His Gln Arg Pro Val Ala Phe Phe
        60                  65                  70

gcc tat aga gta aat gga caa tat att atg gaa gga ctt gca tcc agc 289
Ala Tyr Arg Val Asn Gly Gln Tyr Ile Met Glu Gly Leu Ala Ser Ser
    75                  80                  85

ttc ctg ttt aca atg gga ggt tta ggt ttc ata atc ctg gac caa ttg 337
Phe Leu Phe Thr Met Gly Gly Leu Gly Phe Ile Ile Leu Asp Gln Leu
90                  95                  100                 105

aat gca cca aat atc cca aaa ctc aat aga ttt ctt ctt cta ttc att 385
Asn Ala Pro Asn Ile Pro Lys Leu Asn Arg Phe Leu Leu Leu Phe Ile
                110                 115                 120

gga ttt gtc tgt gtt cta ttg agt att ttc atg gct aga gta ttc atg 433
Gly Phe Val Cys Val Leu Leu Ser Ile Phe Met Ala Arg Val Phe Met
            125                 130                 135

aga atg aaa ctg ccg agc tat ctg atg ggt tagagtgcct ttgagaagaa 483
Arg Met Lys Leu Pro Ser Tyr Leu Met Gly
        140                 145

atcagtggat actggatttt ttcttgtcaa tgaagtttta aaggctgtac 533

caatcctcta atatgaaatg tggaaaagaa tgaagagcag cagtaaaaga 583

aatatctagt gaaaaaacag gaagcgtatt gaagcttgga ctagaatttc 633

ttcttggtat taaagagaca agtttatcac agaatttttt ttcctgctgg 683

cctattgcta taccaatgat gttgagtggc attttctttt tagtttttca 733

ttaaaatata ttccatatct acaactataa tatcaaataa agtgattatt 783

tttta 788

<210>50
<211>147
<212>PRT
<213>Homo sapiens

<400>50

Met Glu Ile Leu Tyr His Val Leu Phe Leu Val Leu Glu Cys Pro Asn
1               5                   10                  15

Leu Lys Leu Lys Lys Pro Pro Trp Leu His Met Leu Ser Ala Met Thr
            20                  25                  30

Val Cys Ser Gly Gly Gly Val Phe Leu Ile Thr Gly Gly Ile Ile Tyr
        35                  40                  45

Asp Val Ile Val Glu Pro Pro Ser Val Gly Ser Met Thr Asp Glu His
    50                  55                  60

Gly His Gln Arg Pro Val Ala Phe Phe Ala Tyr Arg Val Asn Gly Gln
65                  70                  75                  80

Tyr Ile Met Glu Gly Leu Ala Ser Ser Phe Leu Phe Thr Met Gly Gly
                85                  90                  95

Leu Gly Phe Ile Ile Leu Asp Gln Leu Asn Ala Pro Asn Ile Pro Lys
            100                 105                 110

Leu Asn Arg Phe Leu Leu Leu Phe Ile Gly Phe Val Cys Val Leu Leu
        115                 120                 125

Ser Ile Phe Met Ala Arg Val Phe Met Arg Met Lys Leu Pro Ser Tyr
    130                 135                 140

Leu Met Gly
145

<210>51
<211>3761
<212>DNA
<213>Homo sapiens

<220>
<221> CDS
<222>(61)..(3634)

<400>51

gggcgcgccg agccgggcgc gggggcgctg aacggcggag cgggagcggc 50

cggaggagcc atg gac tgc agc ctc gtg cgg acg ctc gtg cac aga tac 99
Met Asp Cys Ser Leu Val Arg Thr Leu Val His Arg Tyr
1               5                   10

tgt gca gga gaa gag aat tgg gtg gac agc agg acc atc tac gtg gga 147
Cys Ala Gly Glu Glu Asn Trp Val Asp Ser Arg Thr Ile Tyr Val Gly
    15                  20                  25

cac agg gag cca cct ccg ggc gca gag gcc tac atc cca cag aga tac 195
His Arg Glu Pro Pro Pro Gly Ala Glu Ala Tyr Ile Pro Gln Arg Tyr
30                  35                  40                  45

cca gac aac agg atc gtc tcg tcc aag tac aca ttt tgg aac ttt ata 243
Pro Asp Asn Arg Ile Val Ser Ser Lys Tyr Thr Phe Trp Asn Phe Ile
                50                  55                  60

ccc aag aat tta ttt gaa caa ttc aga aga gta gcc aac ttt tat ttc 291
Pro Lys Asn Leu Phe Glu Gln Phe Arg Arg Val Ala Asn Phe Tyr Phe
            65                  70                  75

ctt atc ata ttt ctg gtg cag ttg att att gat aca ccc aca agt cca 339
Leu Ile Ile Phe Leu Val Gln Leu Ile Ile Asp Thr Pro Thr Ser Pro
        80                  85                  90

gtg aca agc gga ctt cca ctc ttc ttt gtc att act gtg acg gct atc 387
Val Thr Ser Gly Leu Pro Leu Phe Phe Val Ile Thr Val Thr Ala Ile
    95                  100                 105

aaa cag ggt tat gaa gac tgg ctt cga cat aaa gca gac aat gcc atg 435
Lys Gln Gly Tyr Glu Asp Trp Leu Arg His Lys Ala Asp Asn Ala Met
110                 115                 120                 125

aac cag tgt cct gtt cat ttc att cag cac ggc aag ctc gtt cgg aaa 483
Asn Gln Cys Pro Val His Phe Ile Gln His Gly Lys Leu Val Arg Lys
                130                 135                 140

caa agt cga aag ctg cga gtt ggg gac att gtc atg gtt aag gag gac 531
Gln Ser Arg Lys Leu Arg Val Gly Asp Ile Val Met Val Lys Glu Asp
            145                 150                 155

gag acc ttt ccc tgc gac ttg atc ttc ctt tcc agc aac cgg gga gat 579
Glu Thr Phe Pro Cys Asp Leu Ile Phe Leu Ser Ser Asn Arg Gly Asp

        160                 165                 170

ggg acg tgc cac gtc acc acc gcc agc ttg gat gga gaa tcc agc cat 627
Gly Thr Cys His Val Thr Thr Ala Ser Leu Asp Gly Glu Ser Ser His
    175                 180                 185

aaa acg cat tac gcg gtc cag gac acc aaa ggc ttc cac aca gag gag 675
Lys Thr His Tyr Ala Val Gln Asp Thr Lys Gly Phe His Thr Glu Glu
190                 195                 200                 205

gat atc ggc gga ctt cac gcc acc atc gag tgt gag cag ccc cag ccc 723
Asp Ile Gly Gly Leu His Ala Thr Ile Glu Cys Glu Gln Pro Gln Pro
                210                 215                 220

gac ctc tac aag ttc gtg ggt cgc atc aac gtt tac agt gac ctg aat 771
Asp Leu Tyr Lys Phe Val Gly Arg Ile Asn Val Tyr Ser Asp Leu Asn
            225                 230                 235

gac ccc gtg gtg agg ccc tta gga tcg gaa aac ctg ctg ctt aga gga 819
Asp Pro Val Val Arg Pro Leu Gly Ser Glu Asn Leu Leu Leu Arg Gly
        240                 245                 250

gct aca ctg aag aac act gag aaa atc ttt ggt gtg gct att tac acg 867
Ala Thr Leu Lys Asn Thr Glu Lys Ile Phe Gly Val Ala Ile Tyr Thr
    255                 260                 265

gga atg gaa acc aag atg gca tta aat tat caa tca aaa tct cag aag 915
Gly Met Glu Thr Lys Met Ala Leu Asn Tyr Gln Ser Lys Ser Gln Lys
270                 275                 280                 285

cga tct gcc gtg gaa aaa tcg atg aat gcg ttc ctc att gtg tat ctc 963
Arg Ser Ala Val Glu Lys Ser Met Asn Ala Phe Leu Ile Val Tyr Leu
                290                 295                 300

tgc att ctg atc agc aaa gcc ctg ata aac act gtg ctg aaa tac atg 1011
Cys Ile Leu Ile Ser Lys Ala Leu Ile Asn Thr Val Leu Lys Tyr Met
            305                 310                 315

tgg cag agt gag ccc ttt cgg gat gag ccg tgg tat aat cag aaa acg 1059
Trp Gln Ser Glu Pro Phe Arg Asp Glu Pro Trp Tyr Asn Gln Lys Thr
        320                 325                 330

gag tcg gaa agg cag agg aat ctg ttc ctc aag gca ttc acg gac ttc 1107
Glu Ser Glu Arg Gln Arg Asn Leu Phe Leu Lys Ala Phe Thr Asp Phe
    335                 340                 345

ctg gcc ttc atg gtc ctc ttt aac tac atc atc cct gtg tcc atg tac 1155
Leu Ala Phe Met Val Leu Phe Asn Tyr Ile Ile Pro Val Ser Met Tyr
350                 355                 360                 365

gtc acg gtc gag atg cag aag ttc ctc ggc tct tac ttc atc acc tgg 1203
Val Thr Val Glu Met Gln Lys Phe Leu Gly Ser Tyr Phe Ile Thr Trp
                370                 375                 380

gac gaa gac atg ttt gac gag gag act ggc gag ggg cct ctg gtg aac 1251
Asp Glu Asp Met Phe Asp Glu Glu Thr Gly Glu Gly Pro Leu Val Asn
            385                 390                 395

acg tcg gac ctc aat gaa gag ctg gga cag gtg gag tac atc ttc aca 1299
Thr Ser Asp Leu Asn Glu Glu Leu Gly Gln Val Glu Tyr Ile Phe Thr
        400                 405                 410

gac aag acc ggc acc ctc acg gaa aac aac atg gag ttc aag gag tgc 1347
Asp Lys Thr Gly Thr Leu Thr Glu Asn Asn Met Glu Phe Lys Glu Cys
    415                 420                 425

tgc atc gaa ggc cat gtc tac gtg ccc cac gtc atc tgc aac ggg cag 1395
Cys Ile Glu Gly His Val Tyr Val Pro His Val Ile Cys Asn Gly Gln
430                 435                 440                 445

gtc ctc cca gag tcg tca gga atc gac atg att gac tcg tcc ccc agc 1443
Val Leu Pro Glu Ser Ser Gly Ile Asp Met Ile Asp Ser Ser Pro Ser
                450                 455                 460

gtc aac ggg agg gag cgc gag gag ctg ttt ttc cgg gcc ctc tgt ctc 1491
Val Asn Gly Arg Glu Arg Glu Glu Leu Phe Phe Arg Ala Leu Cys Leu
            465                 470                 475

tgc cac acc gtc cag gtg aaa gac gat gac agc gta gac ggc ccc agg 1539
Cys His Thr Val Gln Val Lys Asp Asp Asp Ser Val Asp Gly Pro Arg
        480                 485                 490

aaa tcg ccg gac ggg ggg aaa tcc tgt gtg tac atc tca tcc tcg ccc 1587
Lys Ser Pro Asp Gly Gly Lys Ser Cys Val Tyr Ile Ser Ser Ser Pro
    495                 500                 505

gac gag gtg gcg ctg gtc gaa ggt gtc cag aga ctt ggc ttt acc tac 1635
Asp Glu Val Ala Leu Val Glu Gly Val Gln Arg Leu Gly Phe Thr Tyr
510                 515                 520                 525

cta agg ctg aag gac aat tac atg gag ata tta aac agg gag aac cac 1683
Leu Arg Leu Lys Asp Asn Tyr Met Glu Ile Leu Asn Arg Glu Asn His
                530                 535                 540

atc gaa agg ttt gaa ttg ctg gaa att ttg agt ttt gac tca gtc aga 1731
Ile Glu Arg Phe Glu Leu Leu Glu Ile Leu Ser Phe Asp Ser Val Arg
            545                 550                 555

agg aga atg agt gta att gta aaa tct gct aca gga gaa att tat ctg 1779
Arg Arg Met Ser Val Ile Val Lys Ser Ala Thr Gly Glu Ile Tyr Leu
        560                 565                 570

ttt tgc aaa gga gca gat tct tcg ata ttc ccc cga gtg ata gaa ggc 1827
Phe Cys Lys Gly Ala Asp Ser Ser Ile Phe Pro Arg Val Ile Glu Gly
    575                 580                 585

aaa gtt gac cag atc cga gcc aga gtg gag cgt aac gca gtg gag ggg 1875
Lys Val Asp Gln Ile Arg Ala Arg Val Glu Arg Asn Ala Val Glu Gly
590                 595                 600                 605

ctc cga act ttg tgt gtt gct tat aaa agg ctg atc caa gaa gaa tat 1923
Leu Arg Thr Leu Cys Val Ala Tyr Lys Arg Leu Ile Gln Glu Glu Tyr
                610                 615                 620

gaa ggc att tgt aag ctg ctg cag gct gcc aaa gtg gcc ctt caa gat 1971
Glu Gly Ile Cys Lys Leu Leu Gln Ala Ala Lys Val Ala Leu Gln Asp
            625                 630                 635

cga gag aaa aag tta gca gaa gcc tat gag caa ata gag aaa gat ctt 2019
Arg Glu Lys Lys Leu Ala Glu Ala Tyr Glu Gln Ile Glu Lys Asp Leu
        640                 645                 650

act ctg ctt ggt gct aca gct gtt gag gac cgg ctg cag gag aaa gct 2067
Thr Leu Leu Gly Ala Thr Ala Val Glu Asp Arg Leu Gln Glu Lys Ala
    655                 660                 665

gca gac acc atc gag gcc ctg cag aag gcc ggg atc aaa gtc tgg gtt 2115
Ala Asp Thr Ile Glu Ala Leu Gln Lys Ala Gly Ile Lys Val Trp Val
670                 675                 680                 685

ctc acg gga gac aag atg gag acg gcc gcg gcc acg tgc tac gcc tgc 2163
Leu Thr Gly Asp Lys Met Glu Thr Ala Ala Ala Thr Cys Tyr Ala Cys
                690                 695                 700

aag ctc ttc cgc agg aac acg cag ctg ctg gag ctg acc acc aag agg 2211
Lys Leu Phe Arg Arg Asn Thr Gln Leu Leu Glu Leu Thr Thr Lys Arg
            705                 710                 715

atc gag gag cag agc ctg cac gac gtc ctg ttc gag ctg agc aag acg 2259
Ile Glu Glu Gln Ser Leu His Asp Val Leu Phe Glu Leu Ser Lys Thr
        720                 725                 730

gtc ctg cgc cac agc ggg agc ctg acc aga gac aac ctg tcc gga ctt 2307
Val Leu Arg His Ser Gly Ser Leu Thr Arg Asp Asn Leu Ser Gly Leu
    735                 740                 745

tca gca gat atg cag gac tac ggt tta att atc gac gga gct gca ctg 2355
Ser Ala Asp Met Gln Asp Tyr Gly Leu Ile Ile Asp Gly Ala Ala Leu
750                 755                 760                 765

tct ctg ata atg aag cct cga gaa gac ggg agt tcc ggc aac tac agg 2403
Ser Leu Ile Met Lys Pro Arg Glu Asp Gly Ser Ser Gly Asn Tyr Arg
                770                 775                 780

gag ctc ttc ctg gaa atc tgc cgg agc tgc agc gcg gtg ctc tgc tgc 2451
Glu Leu Phe Leu Glu Ile Cys Arg Ser Cys Ser Ala Val Leu Cys Cys
            785                 790                 795

cgc atg gcg ccc ttg cag aag gct cag att gtt aaa tta atc aaa ttt 2499
Arg Met Ala Pro Leu Gln Lys Ala Gln Ile Val Lys Leu Ile Lys Phe
        800                 805                 810

tca aaa gag cac cca atc acg tta gca att ggc gat ggt gca aat gat 2547
Ser Lys Glu His Pro Ile Thr Leu Ala Ile Gly Asp Gly Ala Asn Asp
    815                 820                 825

gtc agc atg att ctg gaa gcg cac gtg ggc ata ggt gtc atc ggc aag 2595
Val Ser Met Ile Leu Glu Ala His Val Gly Ile Gly Val Ile Gly Lys
830                 835                 840                 845

gaa ggc cgc cag gct gcc agg aac agc gac tat gca atc cca aag ttt 2643

Glu Gly Arg Gln Ala Ala Arg Asn Ser Asp Tyr Ala Ile Pro Lys Phe
                850                 855                 860

aag cat ttg aag aag atg ctg ctt gtt cac ggg cat ttt tat tac att 2691
Lys His Leu Lys Lys Met Leu Leu Val His Gly His Phe Tyr Tyr Ile
            865                 870                 875

agg atc tct gag ctc gtg cag tac ttc ttc tat aag aac gtc tgc ttc 2739
Arg Ile Ser Glu Leu Val Gln Tyr Phe Phe Tyr Lys Asn Val Cys Phe
        880                 885                 890

atc ttc cct cag ttt tta tac cag ttc ttc tgt ggg ttt tca caa cag 2787
Ile Phe Pro Gln Phe Leu Tyr Gln Phe Phe Cys Gly Phe Ser Gln Gln
    895                 900                 905

act ttg tac gac acc gcg tat ctg acc ctc tac aac atc agc ttc acc 2835
Thr Leu Tyr Asp Thr Ala Tyr Leu Thr Leu Tyr Asn Ile Ser Phe Thr
910                 915                 920                 925

tcc ctc ccc atc ctc ctg tac agc ctc atg gag cag cat gtt ggc att 2883
Ser Leu Pro Ile Leu Leu Tyr Ser Leu Met Glu Gln His Val Gly Ile
                930                 935                 940

gac gtg ctc aag aga gac ccg acc ctg tac agg gac gtc gcc aag aat 2931
Asp Val Leu Lys Arg Asp Pro Thr Leu Tyr Arg Asp Val Ala Lys Asn
            945                 950                 955

gcc ctg ctg cgc tgg cgc gtg ttc atc tac tgg acg ctc ctg gga ctg 2979
Ala Leu Leu Arg Trp Arg Val Phe Ile Tyr Trp Thr Leu Leu Gly Leu
        960                 965                 970

ttt gac gca ctg gtg ttc ttc ttt ggt gct tat ttc gtg ttt gaa aat 3027
Phe Asp Ala Leu Val Phe Phe Phe Gly Ala Tyr Phe Val Phe Glu Asn
    975                 980                 985

aca act gtg aca agc aac ggg cag ata ttt gga aac tgg acg ttt gga 3075
Thr Thr Val Thr Ser Asn Gly Gln Ile Phe Gly Asn Trp Thr Phe Gly
990                 995                 1000                1005

acg ctg gta ttc acc gtg atg gtg ttc aca gtt aca cta aag ctt gca 3123
Thr Leu Val Phe Thr Val Met Val Phe Thr Val Thr Leu Lys Leu Ala
                1010                1015                1020

ttg gac aca cac tac tgg act tgg atc aac cat ttt gtc atc tgg ggg 3171
Leu Asp Thr His Tyr Trp Thr Trp Ile Asn His Phe Val Ile Trp Gly
            1025                1030                1035

tcg ctg ctg ttc tac gtt gtc ttt tcg ctt ctc tgg gga gga gtg atc 3219
Ser Leu Leu Phe Tyr Val Val Phe Ser Leu Leu Trp Gly Gly Val Ile
        1040                1045                1050

tgg ccg ttc ctc aac tac cag agg atg tac tac gtg ttc atc cag atg 3267
Trp Pro Phe Leu Asn Tyr Gln Arg Met Tyr Tyr Val Phe Ile Gln Met
    1055                1060                1065

ctg tcc agc ggg ccc gcc tgg ctg gcc atc gtg ctg ctg gtg acc atc 3315
Leu Ser Ser Gly Pro Ala Trp Leu Ala Ile Val Leu Leu Val Thr Ile

1070                    1075                    1080                    1085

agc ctc ctt ccc gac gtc ctc aag aaa gtc ctg tgc cgg cag ctg tgg     3363
Ser Leu Leu Pro Asp Val Leu Lys Lys Val Leu Cys Arg Gln Leu Trp
                1090                    1095                    1100

cca aca gca aca gag aga gtc cag aat ggg tgc gca cag cct cgg gac     3411
Pro Thr Ala Thr Glu Arg Val Gln Asn Gly Cys Ala Gln Pro Arg Asp
            1105                    1110                    1115

cgc gac tca gaa ttc acc cct ctt gcc tct ctg cag agc cca ggc tac     3459
Arg Asp Ser Glu Phe Thr Pro Leu Ala Ser Leu Gln Ser Pro Gly Tyr
        1120                    1125                    1130

cag agc acc tgt ccc tcg gcc gcc tgg tac agc tcc cac tct cag cag     3507
Gln Ser Thr Cys Pro Ser Ala Ala Trp Tyr Ser Ser His Ser Gln Gln
    1135                    1140                    1145

gtg aca ctc gcg gcc tgg aag gag aag gtg tcc acg gag ccc cca ccc     3555
Val Thr Leu Ala Ala Trp Lys Glu Lys Val Ser Thr Glu Pro Pro Pro
1150                    1155                    1160                    1165

atc ctc ggc ggt tcc cat cac cac tgc agt tcc atc cca agt cac agc     3603
Ile Leu Gly Gly Ser His His His Cys Ser Ser Ile Pro Ser His Ser
                1170                    1175                    1180

tgc cct agg tcc cgt gtg gga atg ctc gtg tgatggatgg tcctaagcct     3653
Cys Pro Arg Ser Arg Val Gly Met Leu Val
            1185                    1190

gtggagactg tgcacgtgcc tcttcctggc ccccagcagg caaggagggg     3703

ggtcacaggc cttgccctcg agcatggcac cctggccgcc tggacccagc     3753

actgtggt     3761

<210>52
<211>1191
<212>PRT
<213>Homo sapiens

<400>52

Met Asp Cys Ser Leu Val Arg Thr Leu Val His Arg Tyr Cys Ala Gly
  1               5                  10                  15

Glu Glu Asn Trp Val Asp Ser Arg Thr Ile Tyr Val Gly His Arg Glu
             20                  25                  30

Pro Pro Pro Gly Ala Glu Ala Tyr Ile Pro Gln Arg Tyr Pro Asp Asn
         35                  40                  45

Arg Ile Val Ser Ser Lys Tyr Thr Phe Trp Asn Phe Ile Pro Lys Asn
     50                  55                  60

Leu Phe Glu Gln Phe Arg Arg Val Ala Asn Phe Tyr Phe Leu Ile Ile
65                  70                  75                  80

Phe Leu Val Gln Leu Ile Ile Asp Thr Pro Thr Ser Pro Val Thr Ser
                85                  90                  95

Gly Leu Pro Leu Phe Phe Val Ile Thr Val Thr Ala Ile Lys Gln Gly
            100                 105                 110

Tyr Glu Asp Trp Leu Arg His Lys Ala Asp Asn Ala Met Asn Gln Cys
        115                 120                 125

Pro Val His Phe Ile Gln His Gly Lys Leu Val Arg Lys Gln Ser Arg
    130                 135                 140

Lys Leu Arg Val Gly Asp Ile Val Met Val Lys Glu Asp Glu Thr Phe
145                 150                 155                 160

Pro Cys Asp Leu Ile Phe Leu Ser Ser Asn Arg Gly Asp Gly Thr Cys
                165                 170                 175

His Val Thr Thr Ala Ser Leu Asp Gly Glu Ser Ser His Lys Thr His
            180                 185                 190

Tyr Ala Val Gln Asp Thr Lys Gly Phe His Thr Glu Glu Asp Ile Gly
        195                 200                 205

Gly Leu His Ala Thr Ile Glu Cys Glu Gln Pro Gln Pro Asp Leu Tyr
    210                 215                 220

Lys Phe Val Gly Arg Ile Asn Val Tyr Ser Asp Leu Asn Asp Pro Val
225                 230                 235                 240

Val Arg Pro Leu Gly Ser Glu Asn Leu Leu Leu Arg Gly Ala Thr Leu
                245                 250                 255

Lys Asn Thr Glu Lys Ile Phe Gly Val Ala Ile Tyr Thr Gly Met Glu
            260                 265                 270

Thr Lys Met Ala Leu Asn Tyr Gln Ser Lys Ser Gln Lys Arg Ser Ala
        275                 280                 285

Val Glu Lys Ser Met Asn Ala Phe Leu Ile Val Tyr Leu Cys Ile Leu
    290                 295                 300

Ile Ser Lys Ala Leu Ile Asn Thr Val Leu Lys Tyr Met Trp Gln Ser
305                 310                 315                 320

Glu Pro Phe Arg Asp Glu Pro Trp Tyr Asn Gln Lys Thr Glu Ser Glu
                325                 330                 335

Arg Gln Arg Asn Leu Phe Leu Lys Ala Phe Thr Asp Phe Leu Ala Phe
            340                 345                 350

Met Val Leu Phe Asn Tyr Ile Ile Pro Val Ser Met Tyr Val Thr Val
        355                 360                 365

Glu Met Gln Lys Phe Leu Gly Ser Tyr Phe Ile Thr Trp Asp Glu Asp
    370                 375                 380

Met Phe Asp Glu Glu Thr Gly Glu Gly Pro Leu Val Asn Thr Ser Asp
385                 390                 395                 400

Leu Asn Glu Glu Leu Gly Gln Val Glu Tyr Ile Phe Thr Asp Lys Thr
                405                 410                 415

Gly Thr Leu Thr Glu Asn Asn Met Glu Phe Lys Glu Cys Cys Ile Glu
            420                 425                 430

Gly His Val Tyr Val Pro His Val Ile Cys Asn Gly Gln Val Leu Pro
        435                 440                 445

Glu Ser Ser Gly Ile Asp Met Ile Asp Ser Ser Pro Ser Val Asn Gly
    450                 455                 460

Arg Glu Arg Glu Glu Leu Phe Phe Arg Ala Leu Cys Leu Cys His Thr
465                 470                 475                 480

Val Gln Val Lys Asp Asp Asp Ser Val Asp Gly Pro Arg Lys Ser Pro
                485                 490                 495

Asp Gly Gly Lys Ser Cys Val Tyr Ile Ser Ser Ser Pro Asp Glu Val
            500                 505                 510

Ala Leu Val Glu Gly Val Gln Arg Leu Gly Phe Thr Tyr Leu Arg Leu
        515                 520                 525

Lys Asp Asn Tyr Met Glu Ile Leu Asn Arg Glu Asn His Ile Glu Arg
    530                 535                 540

Phe Glu Leu Leu Glu Ile Leu Ser Phe Asp Ser Val Arg Arg Arg Met
545                 550                 555                 560

Ser Val Ile Val Lys Ser Ala Thr Gly Glu Ile Tyr Leu Phe Cys Lys
                565                 570                 575

Gly Ala Asp Ser Ser Ile Phe Pro Arg Val Ile Glu Gly Lys Val Asp
            580                 585                 590

Gln Ile Arg Ala Arg Val Glu Arg Asn Ala Val Glu Gly Leu Arg Thr
        595                 600                 605

Leu Cys Val Ala Tyr Lys Arg Leu Ile Gln Glu Glu Tyr Glu Gly Ile
    610                 615                 620

Cys Lys Leu Leu Gln Ala Ala Lys Val Ala Leu Gln Asp Arg Glu Lys
625                 630                 635                 640

Lys Leu Ala Glu Ala Tyr Glu Gln Ile Glu Lys Asp Leu Thr Leu Leu
                645                 650                 655

Gly Ala Thr Ala Val Glu Asp Arg Leu Gln Glu Lys Ala Ala Asp Thr
            660                 665                 670

Ile Glu Ala Leu Gln Lys Ala Gly Ile Lys Val Trp Val Leu Thr Gly
        675                 680                 685

Asp Lys Met Glu Thr Ala Ala Ala Thr Cys Tyr Ala Cys Lys Leu Phe
    690                 695                 700

Arg Arg Asn Thr Gln Leu Leu Glu Leu Thr Thr Lys Arg Ile Glu Glu
705                 710                 715                 720

Gln Ser Leu His Asp Val Leu Phe Glu Leu Ser Lys Thr Val Leu Arg
                725                 730                 735

His Ser Gly Ser Leu Thr Arg Asp Asn Leu Ser Gly Leu Ser Ala Asp
            740                 745                 750

Met Gln Asp Tyr Gly Leu Ile Ile Asp Gly Ala Ala Leu Ser Leu Ile
        755                 760                 765

Met Lys Pro Arg Glu Asp Gly Ser Ser Gly Asn Tyr Arg Glu Leu Phe
    770                 775                 780

Leu Glu Ile Cys Arg Ser Cys Ser Ala Val Leu Cys Cys Arg Met Ala
785                 790                 795                 800

Pro Leu Gln Lys Ala Gln Ile Val Lys Leu Ile Lys Phe Ser Lys Glu
                805                 810                 815

His Pro Ile Thr Leu Ala Ile Gly Asp Gly Ala Asn Asp Val Ser Met
            820                 825                 830

Ile Leu Glu Ala His Val Gly Ile Gly Val Ile Gly Lys Glu Gly Arg
        835                 840                 845

Gln Ala Ala Arg Asn Ser Asp Tyr Ala Ile Pro Lys Phe Lys His Leu
    850                 855                 860

Lys Lys Met Leu Leu Val His Gly His Phe Tyr Tyr Ile Arg Ile Ser
865                 870                 875                 880

Glu Leu Val Gln Tyr Phe Phe Tyr Lys Asn Val Cys Phe Ile Phe Pro
                885                 890                 895

Gln Phe Leu Tyr Gln Phe Phe Cys Gly Phe Ser Gln Gln Thr Leu Tyr
            900                 905                 910

Asp Thr Ala Tyr Leu Thr Leu Tyr Asn Ile Ser Phe Thr Ser Leu Pro
        915                 920                 925

Ile Leu Leu Tyr Ser Leu Met Glu Gln His Val Gly Ile Asp Val Leu
    930                 935                 940

Lys Arg Asp Pro Thr Leu Tyr Arg Asp Val Ala Lys Asn Ala Leu Leu
945                 950                 955                 960

Arg Trp Arg Val Phe Ile Tyr Trp Thr Leu Leu Gly Leu Phe Asp Ala
                965                 970                 975

Leu Val Phe Phe Phe Gly Ala Tyr Phe Val Phe Glu Asn Thr Thr Val
            980                 985                 990

Thr Ser Asn Gly Gln Ile Phe Gly Asn Trp Thr Phe Gly Thr Leu Val
        995                 1000                1005

Phe Thr Val Met Val Phe Thr Val Thr Leu Lys Leu Ala Leu Asp Thr
    1010                1015                1020

His Tyr Trp Thr Trp Ile Asn His Phe Val Ile Trp Gly Ser Leu Leu
1025                1030                1035                1040

Phe Tyr Val Val Phe Ser Leu Leu Trp Gly Gly Val Ile Trp Pro Phe
                1045                1050                1055

Leu Asn Tyr Gln Arg Met Tyr Tyr Val Phe Ile Gln Met Leu Ser Ser
            1060                1065                1070

Gly Pro Ala Trp Leu Ala Ile Val Leu Leu Val Thr Ile Ser Leu Leu
        1075                1080                1085

Pro Asp Val Leu Lys Lys Val Leu Cys Arg Gln Leu Trp Pro Thr Ala
    1090                1095                1100

Thr Glu Arg Val Gln Asn Gly Cys Ala Gln Pro Arg Asp Arg Asp Ser
1105                1110                1115                1120

Glu Phe Thr Pro Leu Ala Ser Leu Gln Ser Pro Gly Tyr Gln Ser Thr
                1125                1130                1135

Cys Pro Ser Ala Ala Trp Tyr Ser Ser His Ser Gln Gln Val Thr Leu
            1140                1145                1150

Ala Ala Trp Lys Glu Lys Val Ser Thr Glu Pro Pro Pro Ile Leu Gly
        1155                1160                1165

Gly Ser His His His Cys Ser Ser Ile Pro Ser His Ser Cys Pro Arg
    1170                1175                1180

Ser Arg Val Gly Met Leu Val
1185                1190

<210>53
<211>2588
<212>DNA
<213>Homo sapiens

<220>
<221> CDS
<222>(94)..(2488)

<400>53

agttccgaac agaaggctgt gtattctctg ccgcttattg tggcctcgac 50

aggccatggt tactttggcc actgccagag cagccttggc act atg gag gag cct 105
Met Glu Glu Pro
1

agg gct acc cct cag ctg tac ttg ggg ctg gtc ctg cag ttg cta ccc 153
Arg Ala Thr Pro Gln Leu Tyr Leu Gly Leu Val Leu Gln Leu Leu Pro
5                   10                  15                  20

agg gtt atg gca gca ctg cct gaa ggt gtg aga cca aat tcg aat cct 201
Arg Val Met Ala Ala Leu Pro Glu Gly Val Arg Pro Asn Ser Asn Pro
                25                  30                  35

tat ggt ttt cca tgg gaa ttg gtg ata tgt gca gct gtc ctt gga ttt 249
Tyr Gly Phe Pro Trp Glu Leu Val Ile Cys Ala Ala Val Leu Gly Phe
            40                  45                  50

gtt gct gtt ccc ttt ttt ttg tgg aga agt ttt aga tcg gtt agg agt 297
Val Ala Val Pro Phe Phe Leu Trp Arg Ser Phe Arg Ser Val Arg Ser
        55                  60                  65

cgg ctt tat gtg gga aga gag aaa gag ctt gct ata gcg ctt tct gga 345
Arg Leu Tyr Val Gly Arg Glu Lys Glu Leu Ala Ile Ala Leu Ser Gly
    70                  75                  80

cta att gaa gaa aaa tgt aga cta ctt gaa aaa ttt agc ctt gtt caa 393
Leu Ile Glu Glu Lys Cys Arg Leu Leu Glu Lys Phe Ser Leu Val Gln
85                  90                  95                  100

aaa gag tat gaa ggc tat gaa gta gag tca tct tta gag gat gcc agc 441
Lys Glu Tyr Glu Gly Tyr Glu Val Glu Ser Ser Leu Glu Asp Ala Ser
                105                 110                 115

ttt gag aag gag gca aca gaa gca caa agt ctg gag gca aac tgt gaa 489
Phe Glu Lys Glu Ala Thr Glu Ala Gln Ser Leu Glu Ala Asn Cys Glu
            120                 125                 130

aag ctg aac agg tcc aat tct gaa ctg gag cat gaa ata ctc tgt cta 537
Lys Leu Asn Arg Ser Asn Ser Glu Leu Glu His Glu Ile Leu Cys Leu
        135                 140                 145

gaa aag ggg ata aaa gaa gag aaa tct aaa cat tct gaa caa gat gag 585
Glu Lys Gly Ile Lys Glu Glu Lys Ser Lys His Ser Glu Gln Asp Glu
    150                 155                 160

gtg atg gca gat att tcc aaa aag ata cag tct cta gaa gat gag tca 633
Val Met Ala Asp Ile Ser Lys Lys Ile Gln Ser Leu Glu Asp Glu Ser
165                 170                 175                 180

aaa tcc ctc aaa tca cta cta act gaa gcc aaa atg acc ttc aag gga 681
Lys Ser Leu Lys Ser Leu Leu Thr Glu Ala Lys Met Thr Phe Lys Gly
                185                 190                 195

ttt caa atg aat gaa gaa aaa ctg gag ata gga ata caa gat gct tcg 729

Phe Gln Met Asn Glu Glu Lys Leu Glu Ile Gly Ile Gln Asp Ala Ser
            200                 205                 210

agt gaa aat tgt caa ctt cag gaa agc cag aaa cag ctt ttg caa gaa 777
Ser Glu Asn Cys Gln Leu Gln Glu Ser Gln Lys Gln Leu Leu Gln Glu
        215                 220                 225

gct gaa gta tgg aaa gaa caa gtg agt gaa ctt aat aaa cag aaa ata 825
Ala Glu Val Trp Lys Glu Gln Val Ser Glu Leu Asn Lys Gln Lys Ile
    230                 235                 240

aca ttt gaa gac tcc aaa gta cac gca gaa caa gtt cta aat gat aaa 873
Thr Phe Glu Asp Ser Lys Val His Ala Glu Gln Val Leu Asn Asp Lys
245                 250                 255                 260

gaa aat cac atc gag act ctg act gaa cgc ttg cta aag atc aaa gat 921
Glu Asn His Ile Glu Thr Leu Thr Glu Arg Leu Leu Lys Ile Lys Asp
                265                 270                 275

cag gct gct gtg ctg gaa gaa gac ata acg gat gat ggt aac ttg gaa 969
Gln Ala Ala Val Leu Glu Glu Asp Ile Thr Asp Asp Gly Asn Leu Glu
            280                 285                 290

tta gaa atg aac agt gaa ttg aaa gat ggt gct tac tta gat aat cct 1017
Leu Glu Met Asn Ser Glu Leu Lys Asp Gly Ala Tyr Leu Asp Asn Pro
        295                 300                 305

cca aaa gga gct ttg aag aaa ctg att cat gct gct aag tta aat gct 1065
Pro Lys Gly Ala Leu Lys Lys Leu Ile His Ala Ala Lys Leu Asn Ala
    310                 315                 320

tct tta aca acc tta gaa gga gaa aga aac caa ttt ata ttc agt tat 1113
Ser Leu Thr Thr Leu Glu Gly Glu Arg Asn Gln Phe Ile Phe Ser Tyr
325                 330                 335                 340

ctg aag ttg ata aaa cca agg aag agc tta gag agc ata tta aaa atc 1161
Leu Lys Leu Ile Lys Pro Arg Lys Ser Leu Glu Ser Ile Leu Lys Ile
                345                 350                 355

ttc aga cgg aac aag cat ctt tgc agt cgg aaa aca cac att ttg aaa 1209
Phe Arg Arg Asn Lys His Leu Cys Ser Arg Lys Thr His Ile Leu Lys
            360                 365                 370

gtg aga atc aga aac ttc aac aga aag tta atg act gag tta tat caa 1257
Val Arg Ile Arg Asn Phe Asn Arg Lys Leu Met Thr Glu Leu Tyr Gln
        375                 380                 385

gaa aat gaa atg aaa ctc tac agg aaa tta ata gta gag gaa aat aac 1305
Glu Asn Glu Met Lys Leu Tyr Arg Lys Leu Ile Val Glu Glu Asn Asn
    390                 395                 400

cgg tta gag aaa gag aaa ctt tct aaa gta gac gaa atg atc agc cat 1353
Arg Leu Glu Lys Glu Lys Leu Ser Lys Val Asp Glu Met Ile Ser His
405                 410                 415                 420

gcc act gaa gag ctg gag acc tgc aga aag cga gcc aaa gat ctt gaa 1401
Ala Thr Glu Glu Leu Glu Thr Cys Arg Lys Arg Ala Lys Asp Leu Glu

                425                 430                 435

gaa gaa ctt gag aga act att ctt ttt tat caa ggg aag att ata tac 1449
Glu Glu Leu Glu Arg Thr Ile Leu Phe Tyr Gln Gly Lys Ile Ile Tyr
            440                 445                 450

cat gag aaa aaa gca cat gat aat tgt ttg gca gca tgg act gct gaa 1497
His Glu Lys Lys Ala His Asp Asn Cys Leu Ala Ala Trp Thr Ala Glu
        455                 460                 465

aga aac ctc aat gat tta agg aaa gaa aat gct cac aaa aga caa aaa 1545
Arg Asn Leu Asn Asp Leu Arg Lys Glu Asn Ala His Lys Arg Gln Lys
    470                 475                 480

tta gct gaa aca gag ttt aaa att aaa ctt tta gaa aaa gat cct tat 1593
Leu Ala Glu Thr Glu Phe Lys Ile Lys Leu Leu Glu Lys Asp Pro Tyr
485                 490                 495                 500

gca ctt gat gtt cca aat aca gca ttt ggc aga gag cat tcc tca tat 1641
Ala Leu Asp Val Pro Asn Thr Ala Phe Gly Arg Glu His Ser Ser Tyr
                505                 510                 515

ggt ccc tca cca ttg ggt cgg cct tca tct gaa acg aga gct ttt ctc 1689
Gly Pro Ser Pro Leu Gly Arg Pro Ser Ser Glu Thr Arg Ala Phe Leu
            520                 525                 530

tat ctt ccg act ttg ttg gag ggt cca ctg aga ctc tca cct ttg ctt 1737
Tyr Leu Pro Thr Leu Leu Glu Gly Pro Leu Arg Leu Ser Pro Leu Leu
        535                 540                 545

cca ggg gga gga gga aga gac cca aga ggc cca ggg aat cct ctg gac 1785
Pro Gly Gly Gly Gly Arg Asp Pro Arg Gly Pro Gly Asn Pro Leu Asp
    550                 555                 560

cac cag att acc aag gaa aga gga gaa tca agc tgt gat agg ttt act 1833
His Gln Ile Thr Lys Glu Arg Gly Glu Ser Ser Cys Asp Arg Phe Thr
565                 570                 575                 580

gat cct cac aag gct cct tct gac act ggg ccc ctg tca cct ccg tgg 1881
Asp Pro His Lys Ala Pro Ser Asp Thr Gly Pro Leu Ser Pro Pro Trp
                585                 590                 595

gaa cag gac cgt agg atg atg ttt cct cca cca gga caa tca tat cct 1929
Glu Gln Asp Arg Arg Met Met Phe Pro Pro Pro Gly Gln Ser Tyr Pro
            600                 605                 610

gat tca gct ctt cct cca caa agg caa gac aga ttt tat tct aat tct 1977
Asp Ser Ala Leu Pro Pro Gln Arg Gln Asp Arg Phe Tyr Ser Asn Ser
        615                 620                 625

gct aga cgc tct gga cta gca gaa ctc aga agt ttt aat ata cct tct 2025
Ala Arg Arg Ser Gly Leu Ala Glu Leu Arg Ser Phe Asn Ile Pro Ser
    630                 635                 640

ttg gat aaa atg gat ggg tca atg cct tca gaa atg gaa tcc agt gga 2073
Leu Asp Lys Met Asp Gly Ser Met Pro Ser Glu Met Glu Ser Ser Gly
645                 650                 655                 660

aat gat acc aaa gat aat ctt ggt aat tta aat gtg gct gat tca tct 2121
Asn Asp Thr Lys Asp Asn Leu Gly Asn Leu Asn Val Ala Asp Ser Ser
                665                 670                 675

ctc cct gct gga aat gaa gtg agt ggc cct ggc ttt gtt cct cca cct 2169
Leu Pro Ala Gly Asn Glu Val Ser Gly Pro Gly Phe Val Pro Pro Pro
            680                 685                 690

ctt gct tca atc aga ggt cca ttg ttt cca gtg gat acg agg ggc ccg 2217
Leu Ala Ser Ile Arg Gly Pro Leu Phe Pro Val Asp Thr Arg Gly Pro
        695                 700                 705

ttc atg aga aga gga cct cct ttc cct cca cct cct cca gga acc atg 2265
Phe Met Arg Arg Gly Pro Pro Phe Pro Pro Pro Pro Pro Gly Thr Met
    710                 715                 720

ttt gga gct tct cca gat tat ttt cca cca agg gat gtc cca ggt cca 2313
Phe Gly Ala Ser Pro Asp Tyr Phe Pro Pro Arg Asp Val Pro Gly Pro
725                 730                 735                 740

cca cgt gct cca ttt gca atg aga aat gtc tgt cca ccg agg ggt ttt 2361
Pro Arg Ala Pro Phe Ala Met Arg Asn Val Cys Pro Pro Arg Gly Phe
                745                 750                 755

cct cct tac ctt ccc cca aga cct gga ttt tgc ccc cac ccc cac ccc 2409
Pro Pro Tyr Leu Pro Pro Arg Pro Gly Phe Cys Pro His Pro His Pro
            760                 765                 770

cac agt gag ttc cct tta ggg ttg agt ctg cct tca aat gag cct gct 2457
His Ser Glu Phe Pro Leu Gly Leu Ser Leu Pro Ser Asn Glu Pro Ala
        775                 780                 785

gct gaa gat cca gaa cca cgg caa gaa acc tgataatatt tttgctgtct 2507
Ala Glu Asp Pro Glu Pro Arg Gln Glu Thr
    790                 795

tcaaaagtca ttttgactat tctcattttc agttgaagta actgctgtta 2557

cttcagtgat tacacttttg ctcaaattga a 2588

<210>54
<211>798
<212>PRT
<213>Homo sapiens

<400>54

Met Glu Glu Pro Arg Ala Thr Pro Gln Leu Tyr Leu Gly Leu Val Leu
1               5                   10                  15

Gln Leu Leu Pro Arg Val Met Ala Ala Leu Pro Glu Gly Val Arg Pro
            20                  25                  30

Asn Ser Asn Pro Tyr Gly Phe Pro Trp Glu Leu Val Ile Cys Ala Ala
        35                  40                  45

Val Leu Gly Phe Val Ala Val Pro Phe Phe Leu Trp Arg Ser Phe Arg
    50                  55                  60

Ser Val Arg Ser Arg Leu Tyr Val Gly Arg Glu Lys Glu Leu Ala Ile
65                  70                  75                  80

Ala Leu Ser Gly Leu Ile Glu Glu Lys Cys Arg Leu Leu Glu Lys Phe
                85                  90                  95

Ser Leu Val Gln Lys Glu Tyr Glu Gly Tyr Glu Val Glu Ser Ser Leu
            100                 105                 110

Glu Asp Ala Ser Phe Glu Lys Glu Ala Thr Glu Ala Gln Ser Leu Glu
        115                 120                 125

Ala Asn Cys Glu Lys Leu Asn Arg Ser Asn Ser Glu Leu Glu His Glu
    130                 135                 140

Ile Leu Cys Leu Glu Lys Gly Ile Lys Glu Glu Lys Ser Lys His Ser
145                 150                 155                 160

Glu Gln Asp Glu Val Met Ala Asp Ile Ser Lys Lys Ile Gln Ser Leu
                165                 170                 175

Glu Asp Glu Ser Lys Ser Leu Lys Ser Leu Leu Thr Glu Ala Lys Met
            180                 185                 190

Thr Phe Lys Gly Phe Gln Met Asn Glu Glu Lys Leu Glu Ile Gly Ile
        195                 200                 205

Gln Asp Ala Ser Ser Glu Asn Cys Gln Leu Gln Glu Ser Gln Lys Gln
    210                 215                 220

Leu Leu Gln Glu Ala Glu Val Trp Lys Glu Gln Val Ser Glu Leu Asn
225                 230                 235                 240

Lys Gln Lys Ile Thr Phe Glu Asp Ser Lys Val His Ala Glu Gln Val
                245                 250                 255

Leu Asn Asp Lys Glu Asn His Ile Glu Thr Leu Thr Glu Arg Leu Leu
            260                 265                 270

Lys Ile Lys Asp Gln Ala Ala Val Leu Glu Glu Asp Ile Thr Asp Asp
        275                 280                 285

Gly Asn Leu Glu Leu Glu Met Asn Ser Glu Leu Lys Asp Gly Ala Tyr
    290                 295                 300

Leu Asp Asn Pro Pro Lys Gly Ala Leu Lys Lys Leu Ile His Ala Ala
305                 310                 315                 320

Lys Leu Asn Ala Ser Leu Thr Thr Leu Glu Gly Glu Arg Asn Gln Phe
                325                 330                 335

Ile Phe Ser Tyr Leu Lys Leu Ile Lys Pro Arg Lys Ser Leu Glu Ser
            340                 345                 350

Ile Leu Lys Ile Phe Arg Arg Asn Lys His Leu Cys Ser Arg Lys Thr
        355                 360                 365

His Ile Leu Lys Val Arg Ile Arg Asn Phe Asn Arg Lys Leu Met Thr
    370                 375                 380

Glu Leu Tyr Gln Glu Asn Glu Met Lys Leu Tyr Arg Lys Leu Ile Val
385                 390                 395                 400

Glu Glu Asn Asn Arg Leu Glu Lys Glu Lys Leu Ser Lys Val Asp Glu
                405                 410                 415

Met Ile Ser His Ala Thr Glu Glu Leu Glu Thr Cys Arg Lys Arg Ala
            420                 425                 430

Lys Asp Leu Glu Glu Glu Leu Glu Arg Thr Ile Leu Phe Tyr Gln Gly
        435                 440                 445

Lys Ile Ile Tyr His Glu Lys Lys Ala His Asp Asn Cys Leu Ala Ala
    450                 455                 460

Trp Thr Ala Glu Arg Asn Leu Asn Asp Leu Arg Lys Glu Asn Ala His
465                 470                 475                 480

Lys Arg Gln Lys Leu Ala Glu Thr Glu Phe Lys Ile Lys Leu Leu Glu
                485                 490                 495

Lys Asp Pro Tyr Ala Leu Asp Val Pro Asn Thr Ala Phe Gly Arg Glu
            500                 505                 510

His Ser Ser Tyr Gly Pro Ser Pro Leu Gly Arg Pro Ser Ser Glu Thr
        515                 520                 525

Arg Ala Phe Leu Tyr Leu Pro Thr Leu Leu Glu Gly Pro Leu Arg Leu
    530                 535                 540

Ser Pro Leu Leu Pro Gly Gly Gly Gly Arg Asp Pro Arg Gly Pro Gly
545                 550                 555                 560

Asn Pro Leu Asp His Gln Ile Thr Lys Glu Arg Gly Glu Ser Ser Cys
                565                 570                 575

Asp Arg Phe Thr Asp Pro His Lys Ala Pro Ser Asp Thr Gly Pro Leu
            580                 585                 590

Ser Pro Pro Trp Glu Gln Asp Arg Arg Met Met Phe Pro Pro Pro Gly
        595                 600                 605

Gln Ser Tyr Pro Asp Ser Ala Leu Pro Pro Gln Arg Gln Asp Arg Phe
    610                 615                 620

Tyr Ser Asn Ser Ala Arg Arg Ser Gly Leu Ala Glu Leu Arg Ser Phe
625                 630                 635                 640

Asn Ile Pro Ser Leu Asp Lys Met Asp Gly Ser Met Pro Ser Glu Met
                645                 650                 655

Glu Ser Ser Gly Asn Asp Thr Lys Asp Asn Leu Gly Asn Leu Asn Val
            660                 665                 670

Ala Asp Ser Ser Leu Pro Ala Gly Asn Glu Val Ser Gly Pro Gly Phe
        675                 680                 685

Val Pro Pro Pro Leu Ala Ser Ile Arg Gly Pro Leu Phe Pro Val Asp
    690                 695                 700

Thr Arg Gly Pro Phe Met Arg Arg Gly Pro Pro Phe Pro Pro Pro Pro
705                 710                 715                 720

Pro Gly Thr Met Phe Gly Ala Ser Pro Asp Tyr Phe Pro Pro Arg Asp
                725                 730                 735

Val Pro Gly Pro Pro Arg Ala Pro Phe Ala Met Arg Asn Val Cys Pro
            740                 745                 750

Pro Arg Gly Phe Pro Pro Tyr Leu Pro Pro Arg Pro Gly Phe Cys Pro
        755                 760                 765

His Pro His Pro His Ser Glu Phe Pro Leu Gly Leu Ser Leu Pro Ser
    770                 775                 780

Asn Glu Pro Ala Ala Glu Asp Pro Glu Pro Arg Gln Glu Thr
785                 790                 795

<210>55
<211>2483
<212>DNA
<213>Homo sapiens

<220>
<221> CDS
<222>(67)..(2401)

<400>55

agctggaatt cgcccttctc gacaggccat ggttactttg gccactgcca 50

gagcagcctt ggcact atg gag gag cct agg gct acc cct cag ctg tac 99
Met Glu Glu Pro Arg Ala Thr Pro Gln Leu Tyr
1               5                   10

ttg ggg ctg gtc ctg cag ttg cta ccc agg gtt atg gca gca ctg cct 147
Leu Gly Leu Val Leu Gln Leu Leu Pro Arg Val Met Ala Ala Leu Pro
            15                  20                  25

gaa ggt gtg aga cca aat tcg aat cct tat ggt ttt cca tgg gaa ttg 195
Glu Gly Val Arg Pro Asn Ser Asn Pro Tyr Gly Phe Pro Trp Glu Leu
        30                  35                  40

gtg ata tgt gca gct gtc ctt gga ttt gtt gct gtt ccc ttt ttt ttg 243
Val Ile Cys Ala Ala Val Leu Gly Phe Val Ala Val Pro Phe Phe Leu
    45                  50                  55

tgg aga agt ttt aga tcg gtt agg agt cgg ctt tat gtg gga aga gag 291
Trp Arg Ser Phe Arg Ser Val Arg Ser Arg Leu Tyr Val Gly Arg Glu
60                  65                  70                  75

aaa gag ctt gct ata gcg ctt tct gga cta att gaa gaa aaa tgt aga 339
Lys Glu Leu Ala Ile Ala Leu Ser Gly Leu Ile Glu Glu Lys Cys Arg
                80                  85                  90

cta ctt gaa aaa ttt agc ctt gtt caa aaa gag tat gaa ggc tat gaa 387
Leu Leu Glu Lys Phe Ser Leu Val Gln Lys Glu Tyr Glu Gly Tyr Glu
            95                  100                 105

gta gag tca tct tta gag gat gcc agc ttt gag aag gag gca aca gaa 435
Val Glu Ser Ser Leu Glu Asp Ala Ser Phe Glu Lys Glu Ala Thr Glu
        110                 115                 120

gca caa agt ctg gag gca aac tgt gaa aag ctg aac agg tcc aat tct 483
Ala Gln Ser Leu Glu Ala Asn Cys Glu Lys Leu Asn Arg Ser Asn Ser
    125                 130                 135

gaa ctg gag cat gaa ata ctc tgt cta gaa aag ggg ata aaa gaa gag 531
Glu Leu Glu His Glu Ile Leu Cys Leu Glu Lys Gly Ile Lys Glu Glu
140                 145                 150                 155

aaa tct aaa cat tct gaa caa gat gag gtg atg gca gat att tcc aaa 579
Lys Ser Lys His Ser Glu Gln Asp Glu Val Met Ala Asp Ile Ser Lys
                160                 165                 170

aag ata cag tct cta gaa gat gag tca aaa tcc ctc aaa tca cta cta 627
Lys Ile Gln Ser Leu Glu Asp Glu Ser Lys Ser Leu Lys Ser Leu Leu
            175                 180                 185

act gaa gct aaa atg acc ttc aag gga ttt caa atg aat gaa gaa aaa 675
Thr Glu Ala Lys Met Thr Phe Lys Gly Phe Gln Met Asn Glu Glu Lys
        190                 195                 200

ctg gag ata gga ata caa gat gct tcg agt gaa aat tgt caa ctt cag 723
Leu Glu Ile Gly Ile Gln Asp Ala Ser Ser Glu Asn Cys Gln Leu Gln
    205                 210                 215

gaa agc cag aaa cag ctt ttg caa gaa gct gaa gta tgg aaa gaa caa 771
Glu Ser Gln Lys Gln Leu Leu Gln Glu Ala Glu Val Trp Lys Glu Gln
220                 225                 230                 235

gtg agt gaa ctt aat aaa cag aaa ata aca ttt gaa gac tcc aaa gta 819
Val Ser Glu Leu Asn Lys Gln Lys Ile Thr Phe Glu Asp Ser Lys Val
                240                 245                 250

cac gca gaa caa gtt cta aat gat aaa gaa aat cac atc gag act ctg 867
His Ala Glu Gln Val Leu Asn Asp Lys Glu Asn His Ile Glu Thr Leu
            255                 260                 265

act gaa cgc ttg cta aag atc aaa gat cag gct gct gtg ctg gaa gaa 915
Thr Glu Arg Leu Leu Lys Ile Lys Asp Gln Ala Ala Val Leu Glu Glu
        270                 275                 280

gac ata acg gat gat ggt aac ttg gaa tta gaa atg aac agt gaa ttg 963
Asp Ile Thr Asp Asp Gly Asn Leu Glu Leu Glu Met Asn Ser Glu Leu
    285                 290                 295

aaa gat ggt gct tac tta gat aat cct cca aaa gga gct ttg aag aaa 1011
Lys Asp Gly Ala Tyr Leu Asp Asn Pro Pro Lys Gly Ala Leu Lys Lys
300                 305                 310                 315

ctg att cat gct gct aag tta aat gct tct tta aca acc tta gaa gga 1059
Leu Ile His Ala Ala Lys Leu Asn Ala Ser Leu Thr Thr Leu Glu Gly
                320                 325                 330

gaa aga aac caa ttt ata ttc agt tat ctg aag ttg ata aaa cca agg 1107
Glu Arg Asn Gln Phe Ile Phe Ser Tyr Leu Lys Leu Ile Lys Pro Arg
            335                 340                 345

aag agc tta gag agc ata tta aaa atc ttc aga cgg aac aag cat ctt 1155
Lys Ser Leu Glu Ser Ile Leu Lys Ile Phe Arg Arg Asn Lys His Leu
        350                 355                 360

tgc agt cgg aaa aca cac att ttg aaa gtg aga atc aga aac ttc aac 1203
Cys Ser Arg Lys Thr His Ile Leu Lys Val Arg Ile Arg Asn Phe Asn
    365                 370                 375

aga aag tta atg act gag tta tat caa gaa aat gaa atg aaa ctc tac 1251
Arg Lys Leu Met Thr Glu Leu Tyr Gln Glu Asn Glu Met Lys Leu Tyr
380                 385                 390                 395

agg aaa tta ata gta gag gaa aat aac cgg tta gag aaa gag aaa ctt 1299
Arg Lys Leu Ile Val Glu Glu Asn Asn Arg Leu Glu Lys Glu Lys Leu
                400                 405                 410

tct aaa gta gac gaa atg atc agc cat gcc act gaa gag ctg gag acc 1347
Ser Lys Val Asp Glu Met Ile Ser His Ala Thr Glu Glu Leu Glu Thr
            415                 420                 425

tgc aga aag cga gcc aaa gat ctt gaa gaa gaa ctt gag aga act att 1395
Cys Arg Lys Arg Ala Lys Asp Leu Glu Glu Glu Leu Glu Arg Thr Ile
        430                 435                 440

ctt ttt tat caa ggg aag att ata tac cat gag aaa aaa gca cat gat 1443
Leu Phe Tyr Gln Gly Lys Ile Ile Tyr His Glu Lys Lys Ala His Asp
    445                 450                 455

aat tgt ttg gca gca tgg act gct gaa aga aac ctc aat gat tta agg 1491
Asn Cys Leu Ala Ala Trp Thr Ala Glu Arg Asn Leu Asn Asp Leu Arg
460                 465                 470                 475

aaa gaa aat gct cac aaa aga caa aaa tta gct gaa aca gag ttt aaa 1539
Lys Glu Asn Ala His Lys Arg Gln Lys Leu Ala Glu Thr Glu Phe Lys
                480                 485                 490

att aaa ctt tta gaa aaa gat cct tat gca ctt gat gtt cca aat aca 1587

Ile Lys Leu Leu Glu Lys Asp Pro Tyr Ala Leu Asp Val Pro Asn Thr
            495                 500                 505

gca ttt ggc aga gag cat tcc tca tat ggt ccc tca cca ttg ggt cgg 1635
Ala Phe Gly Arg Glu His Ser Ser Tyr Gly Pro Ser Pro Leu Gly Arg
        510                 515                 520

cct tca tct gaa acg aga gct ttt ctc tat ctt ccg act ttg ttg gag 1683
Pro Ser Ser Glu Thr Arg Ala Phe Leu Tyr Leu Pro Thr Leu Leu Glu
    525                 530                 535

ggt cca ctg aga ctc tca cct ttg ctt cca ggg gga gga gga aga ggc 1731
Gly Pro Leu Arg Leu Ser Pro Leu Leu Pro Gly Gly Gly Gly Arg Gly
540                 545                 550                 555

cca aga ggc cca ggg aat cct ctg gac cac cag att acc aag gaa aga 1779
Pro Arg Gly Pro Gly Asn Pro Leu Asp His Gln Ile Thr Lys Glu Arg
                560                 565                 570

gga gaa tca agc tgt gat agg ttt act gat cct cac aag gct cct tct 1827
Gly Glu Ser Ser Cys Asp Arg Phe Thr Asp Pro His Lys Ala Pro Ser
            575                 580                 585

gac act ggg ccc ctg tca cct ccg tgg gaa cag gac cgt agg atg atg 1875
Asp Thr Gly Pro Leu Ser Pro Pro Trp Glu Gln Asp Arg Arg Met Met
        590                 595                 600

ttt cct cca cca gga caa tca tat cct gat tca gct ctt cct cca caa 1923
Phe Pro Pro Pro Gly Gln Ser Tyr Pro Asp Ser Ala Leu Pro Pro Gln
    605                 610                 615

agg caa gac aga ttt tat tct aat tct gct aga cgc tct gga cta gca 1971
Arg Gln Asp Arg Phe Tyr Ser Asn Ser Ala Arg Arg Ser Gly Leu Ala
620                 625                 630                 635

gaa ctc aga agt ttt aat ata cct tct ttg gat aaa atg gat ggg tca 2019
Glu Leu Arg Ser Phe Asn Ile Pro Ser Leu Asp Lys Met Asp Gly Ser
                640                 645                 650

atg cct tca gaa atg gaa tcc agt gga aat gat acc aaa gat aat ctt 2067
Met Pro Ser Glu Met Glu Ser Ser Gly Asn Asp Thr Lys Asp Asn Leu
            655                 660                 665

ggt aat tta aat gtg gct gat tca tct ctc cct gct gga aat gaa gtg 2115
Gly Asn Leu Asn Val Ala Asp Ser Ser Leu Pro Ala Gly Asn Glu Val
        670                 675                 680

agt ggc cct ggc ttt gtt cct cca cct ctt gct cca atc aga ggt ccg 2163
Ser Gly Pro Gly Phe Val Pro Pro Pro Leu Ala Pro Ile Arg Gly Pro
    685                 690                 695

ttg ttt cca gtg gat acg agg ggc ccg ttc atg aga aga gga cct cct 2211
Leu Phe Pro Val Asp Thr Arg Gly Pro Phe Met Arg Arg Gly Pro Pro
700                 705                 710                 715

ttc cct cca cct cct cca gga acc atg ttt gga gct tct cca gat tat 2259
Phe Pro Pro Pro Pro Pro Gly Thr Met Phe Gly Ala Ser Pro Asp Tyr

                720                 725                 730

ttt cca cca agg gat gtc cca ggt cta cca cgt gct cca ttt gca atg 2307
Phe Pro Pro Arg Asp Val Pro Gly Leu Pro Arg Ala Pro Phe Ala Met
            735                 740                 745

aga aat gtc tgt cca ccg agg ggt ttt cct cct tac ctt ccc cca aga 2355
Arg Asn Val Cys Pro Pro Arg Gly Phe Pro Pro Tyr Leu Pro Pro Arg
        750                 755                 760

cct gga ttt tgc ccc cac ccc cac ccc cac att ctg aag ata gag 2400
Pro Gly Phe Cys Pro His Pro His Pro His Ile Leu Lys Ile Glu
    765                 770                 775

tgagttccct ttagggttga gtgccttcaa atgagcctgc tgctgaagat 2450

ccagaaccac ggcaagaaac ctgataatat ttt 2483

<210>56
<211>778
<212>PRT
<213>Homo sapiens

<400>56

Met Glu Glu Pro Arg Ala Thr Pro Gln Leu Tyr Leu Gly Leu Val Leu
1               5                   10                  15

Gln Leu Leu Pro Arg Val Met Ala Ala Leu Pro Glu Gly Val Arg Pro
            20                  25                  30

Asn Ser Asn Pro Tyr Gly Phe Pro Trp Glu Leu Val Ile Cys Ala Ala
        35                  40                  45

Val Leu Gly Phe Val Ala Val Pro Phe Phe Leu Trp Arg Ser Phe Arg
    50                  55                  60

Ser Val Arg Ser Arg Leu Tyr Val Gly Arg Glu Lys Glu Leu Ala Ile
65                  70                  75                  80

Ala Leu Ser Gly Leu Ile Glu Glu Lys Cys Arg Leu Leu Glu Lys Phe
                85                  90                  95

Ser Leu Val Gln Lys Glu Tyr Glu Gly Tyr Glu Val Glu Ser Ser Leu
            100                 105                 110

Glu Asp Ala Ser Phe Glu Lys Glu Ala Thr Glu Ala Gln Ser Leu Glu
        115                 120                 125

Ala Asn Cys Glu Lys Leu Asn Arg Ser Asn Ser Glu Leu Glu His Glu
    130                 135                 140

Ile Leu Cys Leu Glu Lys Gly Ile Lys Glu Glu Lys Ser Lys His Ser

145                 150                 155                 160

Glu Gln Asp Glu Val Met Ala Asp Ile Ser Lys Lys Ile Gln Ser Leu
                165                 170                 175

Glu Asp Glu Ser Lys Ser Leu Lys Ser Leu Leu Thr Glu Ala Lys Met
            180                 185                 190

Thr Phe Lys Gly Phe Gln Met Asn Glu Glu Lys Leu Glu Ile Gly Ile
        195                 200                 205

Gln Asp Ala Ser Ser Glu Asn Cys Gln Leu Gln Glu Ser Gln Lys Gln
    210                 215                 220

Leu Leu Gln Glu Ala Glu Val Trp Lys Glu Gln Val Ser Glu Leu Asn
225                 230                 235                 240

Lys Gln Lys Ile Thr Phe Glu Asp Ser Lys Val His Ala Glu Gln Val
                245                 250                 255

Leu Asn Asp Lys Glu Asn His Ile Glu Thr Leu Thr Glu Arg Leu Leu
            260                 265                 270

Lys Ile Lys Asp Gln Ala Ala Val Leu Glu Glu Asp Ile Thr Asp Asp
        275                 280                 285

Gly Asn Leu Glu Leu Glu Met Asn Ser Glu Leu Lys Asp Gly Ala Tyr
    290                 295                 300

Leu Asp Asn Pro Pro Lys Gly Ala Leu Lys Lys Leu Ile His Ala Ala
305                 310                 315                 320

Lys Leu Asn Ala Ser Leu Thr Thr Leu Glu Gly Glu Arg Asn Gln Phe
                325                 330                 335

Ile Phe Ser Tyr Leu Lys Leu Ile Lys Pro Arg Lys Ser Leu Glu Ser
            340                 345                 350

Ile Leu Lys Ile Phe Arg Arg Asn Lys His Leu Cys Ser Arg Lys Thr
        355                 360                 365

His Ile Leu Lys Val Arg Ile Arg Asn Phe Asn Arg Lys Leu Met Thr
    370                 375                 380

Glu Leu Tyr Gln Glu Asn Glu Met Lys Leu Tyr Arg Lys Leu Ile Val
385                 390                 395                 400

Glu Glu Asn Asn Arg Leu Glu Lys Glu Lys Leu Ser Lys Val Asp Glu
                405                 410                 415

Met Ile Ser His Ala Thr Glu Glu Leu Glu Thr Cys Arg Lys Arg Ala
            420                 425                 430

Lys Asp Leu Glu Glu Glu Leu Glu Arg Thr Ile Leu Phe Tyr Gln Gly
        435                 440                 445

Lys Ile Ile Tyr His Glu Lys Lys Ala His Asp Asn Cys Leu Ala Ala

450                 455                 460

Trp Thr Ala Glu Arg Asn Leu Asn Asp Leu Arg Lys Glu Asn Ala His
465                 470                 475                 480

Lys Arg Gln Lys Leu Ala Glu Thr Glu Phe Lys Ile Lys Leu Leu Glu
                485                 490                 495

Lys Asp Pro Tyr Ala Leu Asp Val Pro Asn Thr Ala Phe Gly Arg Glu
            500                 505                 510

His Ser Ser Tyr Gly Pro Ser Pro Leu Gly Arg Pro Ser Ser Glu Thr
        515                 520                 525

Arg Ala Phe Leu Tyr Leu Pro Thr Leu Leu Glu Gly Pro Leu Arg Leu
    530                 535                 540

Ser Pro Leu Leu Pro Gly Gly Gly Gly Arg Gly Pro Arg Gly Pro Gly
545                 550                 555                 560

Asn Pro Leu Asp His Gln Ile Thr Lys Glu Arg Gly Glu Ser Ser Cys
                565                 570                 575

Asp Arg Phe Thr Asp Pro His Lys Ala Pro Ser Asp Thr Gly Pro Leu
            580                 585                 590

Ser Pro Pro Trp Glu Gln Asp Arg Arg Met Met Phe Pro Pro Pro Gly
        595                 600                 605

Gln Ser Tyr Pro Asp Ser Ala Leu Pro Pro Gln Arg Gln Asp Arg Phe
    610                 615                 620

Tyr Ser Asn Ser Ala Arg Arg Ser Gly Leu Ala Glu Leu Arg Ser Phe
625                 630                 635                 640

Asn Ile Pro Ser Leu Asp Lys Met Asp Gly Ser Met Pro Ser Glu Met
                645                 650                 655

Glu Ser Ser Gly Asn Asp Thr Lys Asp Asn Leu Gly Asn Leu Asn Val
            660                 665                 670

Ala Asp Ser Ser Leu Pro Ala Gly Asn Glu Val Ser Gly Pro Gly Phe
        675                 680                 685

Val Pro Pro Pro Leu Ala Pro Ile Arg Gly Pro Leu Phe Pro Val Asp
    690                 695                 700

Thr Arg Gly Pro Phe Met Arg Arg Gly Pro Pro Phe Pro Pro Pro Pro
705                 710                 715                 720

Pro Gly Thr Met Phe Gly Ala Ser Pro Asp Tyr Phe Pro Pro Arg Asp
                725                 730                 735

Val Pro Gly Leu Pro Arg Ala Pro Phe Ala Met Arg Asn Val Cys Pro
            740                 745                 750

Pro Arg Gly Phe Pro Pro Tyr Leu Pro Pro Arg Pro Gly Phe Cys Pro

755                 760                 765

His Pro His Pro His Ile Leu Lys Ile Glu
    770                 775

<210>57
<211>1269
<212>DNA
<213>Homo sapiens

<220>
<221> CDS
<222>(26)..(1004)

<400>57

agcgggggc gctgcctcga gcctc atg gct gcc cct gct tcc gtc atg ggc    52
                           
                           1               5

cca ctc ggg ccc tct gcc ctg ggc ctt ctg ctg ctg ctc ctg gtg gtg    100

10                  15                  20                  25

gcc cct ccc cgg gtc gca gca ttg gtc cac aga cag cca gag aac cag    148

                30                  35                  40

gga atc tcc cta act ggc agc gtg gcc tgt ggt cgg ccc agc atg gag    196

            45                  50                  55

ggg aaa atc ctg ggc ggc gtc cct gcg ccc gag agg aag tgg ccg tgg    244

        60                  65                  70

cag gtc agc gtg cac tac gca ggc ctc cac gtc tgc ggc ggc tcc atc    292

    75                  80                  85

ctc aat gag tac tgg gtg ctg tca gct gcg cac tgc ttt cac agg gac    340

90                  95                  100                 105

aag aat atc aaa atc tat gac atg tac gta ggc ctc gta aac ctc agg    388

                110                 115                 120

gtg gcc ggc aac cac acc cag tgg tat gag gtg aac agg gtg atc ctg    436

            125                 130                 135

cac ccc aca tat gag atg tac cac ccc atc gga ggt gac gtg gcc ctg    484

        140                 145                 150

gtg cag ctg aag acc cgc att gtg ttt tct gag tcc gtg ctc ccg gtt    532


    155                 160                 165

tgc ctt gca act cca gaa gtg aac ctt acc agt gcc aat tgc tgg gct     580

170                 175                 180                 185

acg gga tgg gga cta gtc tca aaa caa ggt gag acc tca gac gag ctg     628

                190                 195                 200

cag gag gtg cag ctc ccg ctg atc ctg gag ccc tgg tgc cac ctg ctc     676

            205                 210                 215

tac gga cac atg tcc tac atc atg ccc gac atg ctg tgt gct ggg gac     724

        220                 225                 230

atc ctg aat gct aag acc gtg tgt gag ggc gac tcc ggg ggc cca ctt     772

    235                 240                 245

gtc tgt gaa ttc aac cgc agc tgg ttg cag att gga att gtg agc tgg     820

250                 255                 260                 265

ggc cga ggc tgc tcc aac cct ctg tac cct gga gtg tat gcc agt gtt     868

                270                 275                 280

tcc tat ttc tca aaa tgg ata tgt gat aac ata gaa atc acg ccc act     916

            285                 290                 295

cct gct cag cca gcc cct gct ctc tct cca gct ctg ggg ccc act ctc     964

        300                 305                 310

agc gtc cta atg gcc atg ctg gct ggc tgg tca gtg ctg tgaggtcagg     1013

    315                 320                 325

atacccactc taggattctc atggctgcac accctgcccc agcccagctg     1063

cctccagacc cctaagcatc tcctgtcctg gcctctctga agcagacaag     1113

ggccacctat cccgggggtg gatgctgagt ccaggaggtg atgagcaagt     1163

gtacaaaaga aaaaagggaa gggggagagg ggctggtcag ggagaaccca     1213

gcttgggcag agtgcacctg agatttgata agatcattaa atatttacaa 1263

agcaaa 1269

<210>58
<211>326
<212>PRT
<213>Homo sapiens

<400>58

<210>59
<211>4131
<212>DNA
<213>Homo sapiens

<220>
<221> CDS
<222>(10)..(4120)

<400>59

gtgccgagg atg gcc agg cag cca ccg ccg ccc tgg atc cat gca gcc      48
          Met Ala Arg Gln Pro Pro Pro Pro Trp Ile His Ala Ala
            1               5                   10

ttc ctc ctc tgc ctc ctc agt ctt ggc gga gcc atc gaa att cct atg      96
Phe Leu Leu Cys Leu Leu Ser Leu Gly Gly Ala Ile Glu Ile Pro Met
     15                  20                  25

gtt cca agc att cag aat gag ctg acg cag ccg cca acc atc acc aag      144
Val Pro Ser Ile Gln Asn Glu Leu Thr Gln Pro Pro Thr Ile Thr Lys
 30                  35                  40                  45

cag tca gcg aag gat cac atc gtg gac ccc cgt gat aac atc ctg att      192
Gln Ser Ala Lys Asp His Ile Val Asp Pro Arg Asp Asn Ile Leu Ile
                 50                  55                  60

gag tgt gaa gca aaa ggg aac cct gcc ccc agc ttc cac tgg aca cga      240
Glu Cys Glu Ala Lys Gly Asn Pro Ala Pro Ser Phe His Trp Thr Arg
             65                  70                  75

aac agc aga ttc ttc aac atc gcc aag gac ccc cgg gtg tcc atg agg      288
Asn Ser Arg Phe Phe Asn Ile Ala Lys Asp Pro Arg Val Ser Met Arg
         80                  85                  90

agg agg tct ggg acc ctg gtg att gac ttc cgc agt ggc ggg cgg ccg 336
Arg Arg Ser Gly Thr Leu Val Ile Asp Phe Arg Ser Gly Gly Arg Pro
    95                  100                 105

gag gaa tat gag ggg gaa tat cag tgc ttc gcc cgc aac aaa ttt ggc 384
Glu Glu Tyr Glu Gly Glu Tyr Gln Cys Phe Ala Arg Asn Lys Phe Gly
110                 115                 120                 125

acg gcc ctg tcc aat agg atc cgc ctg cag gtg tct aaa tct cct ctg 432
Thr Ala Leu Ser Asn Arg Ile Arg Leu Gln Val Ser Lys Ser Pro Leu
                130                 135                 140

tgg ccc aag gaa aac cta gac cct gtc gtg gtc caa gag ggc gct cct 480
Trp Pro Lys Glu Asn Leu Asp Pro Val Val Val Gln Glu Gly Ala Pro
            145                 150                 155

ttg acg ctc cag tgc aac ccc ccg cct gga ctt cca tcc ccg gtc atc 528
Leu Thr Leu Gln Cys Asn Pro Pro Pro Gly Leu Pro Ser Pro Val Ile
        160                 165                 170

ttc tgg atg agc agc gcc atg gag ccc atc acc caa gac aaa cgt gtc 576
Phe Trp Met Ser Ser Ala Met Glu Pro Ile Thr Gln Asp Lys Arg Val
    175                 180                 185

tct cag ggc cat aac gga gac cta tac ttc tcc aac gtg atg ctg cag 624
Ser Gln Gly His Asn Gly Asp Leu Tyr Phe Ser Asn Val Met Leu Gln
190                 195                 200                 205

gac atg cag acc gac tac agt tgt aac gcc cgc ttc cac ttc acc cac 672
Asp Met Gln Thr Asp Tyr Ser Cys Asn Ala Arg Phe His Phe Thr His
                210                 215                 220

acc atc cag cag aag aac cct ttc acc ctc aag gtc ctc acc agt aag 720
Thr Ile Gln Gln Lys Asn Pro Phe Thr Leu Lys Val Leu Thr Ser Lys
            225                 230                 235

cct tat aat gac tcg tcc tta aga aac cac cct gac atg tac agt gcc 768
Pro Tyr Asn Asp Ser Ser Leu Arg Asn His Pro Asp Met Tyr Ser Ala
        240                 245                 250

cga gga gtt gca gaa aga aca cca agc ttc atg tat ccc cag ggc acc 816
Arg Gly Val Ala Glu Arg Thr Pro Ser Phe Met Tyr Pro Gln Gly Thr
    255                 260                 265

gcg agc agc cag atg gtg ctt cgt ggc atg gac ctc ctg ctg gaa tgc 864
Ala Ser Ser Gln Met Val Leu Arg Gly Met Asp Leu Leu Leu Glu Cys
270                 275                 280                 285

atc gcc tcc ggg gtc cca aca cca gac atc gca tgg tac aag aaa ggt 912
Ile Ala Ser Gly Val Pro Thr Pro Asp Ile Ala Trp Tyr Lys Lys Gly
                290                 295                 300

ggg gac ctc cca tct gat aag gcc aag ttt gag aac ttt aat aag gcc 960
Gly Asp Leu Pro Ser Asp Lys Ala Lys Phe Glu Asn Phe Asn Lys Ala
            305                 310                 315

ctg cgt atc aca aat gtc tct gag gaa gac tcc ggg gag tat ttc tgc 1008
Leu Arg Ile Thr Asn Val Ser Glu Glu Asp Ser Gly Glu Tyr Phe Cys
        320                 325                 330

ctg gcc tcc aac aag atg ggc agc atc cgg cac acg atc tcg gtg aga 1056
Leu Ala Ser Asn Lys Met Gly Ser Ile Arg His Thr Ile Ser Val Arg
    335                 340                 345

gta aag gct gct ccc tac tgg ctg gac gaa ccc aag aac ctt att ctg 1104
Val Lys Ala Ala Pro Tyr Trp Leu Asp Glu Pro Lys Asn Leu Ile Leu
350                 355                 360                 365

gct cct ggc gag gat ggg aga ctg gtg tgt cga gcc aat gga aac ccc 1152
Ala Pro Gly Glu Asp Gly Arg Leu Val Cys Arg Ala Asn Gly Asn Pro
                370                 375                 380

aaa ccc act gtc cag tgg atg gtg aat ggg gaa cct ttg caa gcg gca 1200
Lys Pro Thr Val Gln Trp Met Val Asn Gly Glu Pro Leu Gln Ala Ala
            385                 390                 395

cca cct aac cca aac cgt gag gtg gcc gga gac acc atc atc ttc cgg 1248
Pro Pro Asn Pro Asn Arg Glu Val Ala Gly Asp Thr Ile Ile Phe Arg
        400                 405                 410

gac acc cag atc agc agc agg gct gtg tac cag tgc aac acc tcc aac 1296
Asp Thr Gln Ile Ser Ser Arg Ala Val Tyr Gln Cys Asn Thr Ser Asn
    415                 420                 425

gag cat ggc tac ctg ctg gcc aac gcc ttt gtc agt gtg ctg gat gtg 1344
Glu His Gly Tyr Leu Leu Ala Asn Ala Phe Val Ser Val Leu Asp Val
430                 435                 440                 445

ccg cct cgg atg ctg tcg ccc cgg aac cag ctc att cga gtg att ctt 1392
Pro Pro Arg Met Leu Ser Pro Arg Asn Gln Leu Ile Arg Val Ile Leu
                450                 455                 460

tac aac cgg acg cgg ctg gac tgc cct ttc ttt ggg tct ccc atc ccc 1440
Tyr Asn Arg Thr Arg Leu Asp Cys Pro Phe Phe Gly Ser Pro Ile Pro
            465                 470                 475

aca ctg cga tgg ttt aag aat ggg caa gga agc aac ctg gat ggt ggc 1488
Thr Leu Arg Trp Phe Lys Asn Gly Gln Gly Ser Asn Leu Asp Gly Gly
        480                 485                 490

aac tac cat gtt tat gag aac ggc agt ctg gaa att aag atg atc cgc 1536
Asn Tyr His Val Tyr Glu Asn Gly Ser Leu Glu Ile Lys Met Ile Arg
    495                 500                 505

aaa gag gac cag ggc atc tac acc tgt gtc gcc acc aac atc ctg ggc 1584
Lys Glu Asp Gln Gly Ile Tyr Thr Cys Val Ala Thr Asn Ile Leu Gly
510                 515                 520                 525

aaa gct gaa aac caa gtc cgc ctg gag gta aaa gac ccc acc agg atc 1632
Lys Ala Glu Asn Gln Val Arg Leu Glu Val Lys Asp Pro Thr Arg Ile
                530                 535                 540

tac cgg atg ccc gag gac cag gtg gcc aga agg ggc acc acg gtg caa 1680

Tyr Arg Met Pro Glu Asp Gln Val Ala Arg Arg Gly Thr Thr Val Gln
            545                 550                 555

ctg gag tgt cgg gtg aag cac gac ccc tcc ctg aaa ctc acc gtc tac 1728
Leu Glu Cys Arg Val Lys His Asp Pro Ser Leu Lys Leu Thr Val Tyr
        560                 565                 570

tgg ctg aag gat gac gag ccg ctc tat att gga aac agg atg aag aag 1776
Trp Leu Lys Asp Asp Glu Pro Leu Tyr Ile Gly Asn Arg Met Lys Lys
    575                 580                 585

gaa gac gac tcc ctg acc atc ttt ggg gtg gca gag cgg gac cag ggc 1824
Glu Asp Asp Ser Leu Thr Ile Phe Gly Val Ala Glu Arg Asp Gln Gly
590                 595                 600                 605

agt tac acg tgt gtc gcc agc acc gag cta gac caa gac ctg gcc aag 1872
Ser Tyr Thr Cys Val Ala Ser Thr Glu Leu Asp Gln Asp Leu Ala Lys
                610                 615                 620

gcc tac ctc acc gtg cta gga cgg cca gac cgg ccc cgg gac ctg gag 1920
Ala Tyr Leu Thr Val Leu Gly Arg Pro Asp Arg Pro Arg Asp Leu Glu
            625                 630                 635

ctg acc gac ctg gcc gag agg agc gtg cgg ctg acc tgg atc ccc ggg 1968
Leu Thr Asp Leu Ala Glu Arg Ser Val Arg Leu Thr Trp Ile Pro Gly
        640                 645                 650

gat gct aac aac agc ccc atc aca gac tac gtc gtc cag ttt gaa gaa 2016
Asp Ala Asn Asn Ser Pro Ile Thr Asp Tyr Val Val Gln Phe Glu Glu
    655                 660                 665

gac cag ttc caa cct ggg gtc tgg cat gac cat tcc aag tac ccc ggc 2064
Asp Gln Phe Gln Pro Gly Val Trp His Asp His Ser Lys Tyr Pro Gly
670                 675                 680                 685

agc gtt aac tca gcc gtc ctc cgg ctg tcc ccg tat gtc aac tac cag 2112
Ser Val Asn Ser Ala Val Leu Arg Leu Ser Pro Tyr Val Asn Tyr Gln
                690                 695                 700

ttc cgt gtc att gcc atc aac gag gtt ggg agc agc cac ccc agc ctc 2160
Phe Arg Val Ile Ala Ile Asn Glu Val Gly Ser Ser His Pro Ser Leu
            705                 710                 715

cca tcc gag cgc tac cga acc agt gga gca ccc ccc gag tcc aat cct 2208
Pro Ser Glu Arg Tyr Arg Thr Ser Gly Ala Pro Pro Glu Ser Asn Pro
        720                 725                 730

ggt gac gtg aag gga gag ggg acc aga aag aac aac atg gag atc acg 2256
Gly Asp Val Lys Gly Glu Gly Thr Arg Lys Asn Asn Met Glu Ile Thr
    735                 740                 745

tgg acg ccc atg aat gcc acc tcg gcc ttt ggc ccc aac ctg cgc tac 2304
Trp Thr Pro Met Asn Ala Thr Ser Ala Phe Gly Pro Asn Leu Arg Tyr
750                 755                 760                 765

att gtc aag tgg agg cgg aga gag act cga gag gcc tgg aac aac gtc 2352
Ile Val Lys Trp Arg Arg Arg Glu Thr Arg Glu Ala Trp Asn Asn Val

                770                 775                 780

aca gtg tgg ggc tct cgc tac gtg gtg ggg cag acc cca gtc tac gtg 2400
Thr Val Trp Gly Ser Arg Tyr Val Val Gly Gln Thr Pro Val Tyr Val
            785                 790                 795

ccc tat gag atc cga gtc cag gct gaa aat gac ttc ggg aag ggc cct 2448
Pro Tyr Glu Ile Arg Val Gln Ala Glu Asn Asp Phe Gly Lys Gly Pro
        800                 805                 810

gag cca gag tcc gtc atc ggt tac tcc gga gaa gat tat ccc agg gct 2496
Glu Pro Glu Ser Val Ile Gly Tyr Ser Gly Glu Asp Tyr Pro Arg Ala
    815                 820                 825

gcg ccc act gaa gtt aaa gtc cga gtc atg aac agc aca gcc atc agc 2544
Ala Pro Thr Glu Val Lys Val Arg Val Met Asn Ser Thr Ala Ile Ser
830                 835                 840                 845

ctt cag tgg aac cgc gtc tac tcc gac acg gtc cag ggc cag ctc aga 2592
Leu Gln Trp Asn Arg Val Tyr Ser Asp Thr Val Gln Gly Gln Leu Arg
                850                 855                 860

gag tac cga gcc tac tac tgg agg gag agc agc ttg ctg aag aac ctg 2640
Glu Tyr Arg Ala Tyr Tyr Trp Arg Glu Ser Ser Leu Leu Lys Asn Leu
            865                 870                 875

tgg gtg tct cag aag aga cag caa gcc agc ttc cct ggt gac cgc ctc 2688
Trp Val Ser Gln Lys Arg Gln Gln Ala Ser Phe Pro Gly Asp Arg Leu
        880                 885                 890

cgt ggc gtg gtg tcc cgc ctc ttc ccc tac agt aac tac aag ctg gag 2736
Arg Gly Val Val Ser Arg Leu Phe Pro Tyr Ser Asn Tyr Lys Leu Glu
    895                 900                 905

atg gtt gtg gtc aat ggg aga ggt gat ggg cct cgc agt gag acc aag 2784
Met Val Val Val Asn Gly Arg Gly Asp Gly Pro Arg Ser Glu Thr Lys
910                 915                 920                 925

gag ttc acc acc ccg gaa gga gta ccc agt gcc cct agg cgt ttc cga 2832
Glu Phe Thr Thr Pro Glu Gly Val Pro Ser Ala Pro Arg Arg Phe Arg
                930                 935                 940

gtc cgg cag ccc aac ctg gag aca atc aac ctg gaa tgg gat cat cct 2880
Val Arg Gln Pro Asn Leu Glu Thr Ile Asn Leu Glu Trp Asp His Pro
            945                 950                 955

gag cat cca aat ggg atc atg att gga tac act ctc aaa tat gtg gcc 2928
Glu His Pro Asn Gly Ile Met Ile Gly Tyr Thr Leu Lys Tyr Val Ala
        960                 965                 970

tgt acg ttc tcc cca gtt aac ggg acc aaa gta gga aag cag ata gtg 2976
Cys Thr Phe Ser Pro Val Asn Gly Thr Lys Val Gly Lys Gln Ile Val
    975                 980                 985

gaa aac ttc tct ccc aat cag acc aag ttc acg gtg caa aga acg gac 3024
Glu Asn Phe Ser Pro Asn Gln Thr Lys Phe Thr Val Gln Arg Thr Asp
990                 995                 1000                1005

ccc gtg tca cgc tac cgc ttt acc ctc agc gcc agg acg cag gtg ggc 3072
Pro Val Ser Arg Tyr Arg Phe Thr Leu Ser Ala Arg Thr Gln Val Gly
                1010                1015                1020

tct ggg gaa gcc gtc aca gag gag tca cca gca ccc ccg aat gaa ggt 3120
Ser Gly Glu Ala Val Thr Glu Glu Ser Pro Ala Pro Pro Asn Glu Gly
            1025                1030                1035

agg tgc atg gca gca gcc cct ggg gta aaa ccc ccg act acc gtg ggt 3168
Arg Cys Met Ala Ala Ala Pro Gly Val Lys Pro Pro Thr Thr Val Gly
        1040                1045                1050

gcg acg ggc gct gtg agc agt acc gat gct act gcc att gct gcc acc 3216
Ala Thr Gly Ala Val Ser Ser Thr Asp Ala Thr Ala Ile Ala Ala Thr
    1055                1060                1065

acc gaa gcc aca aca gtc ccc atc atc cca act gtc gca cct acc acc 3264
Thr Glu Ala Thr Thr Val Pro Ile Ile Pro Thr Val Ala Pro Thr Thr
1070                1075                1080                1085

atg gcc acc acc acc acc gtc gcc aca act act aca acc act gct gcc 3312
Met Ala Thr Thr Thr Thr Val Ala Thr Thr Thr Thr Thr Thr Ala Ala
                1090                1095                1100

gcc acc acc acc acg gag agt cct ccc acc acc acc tcc ggg act aag 3360
Ala Thr Thr Thr Thr Glu Ser Pro Pro Thr Thr Thr Ser Gly Thr Lys
            1105                1110                1115

ata cac gaa tcc ggt act gcg cat cgc cca tgc tcc cca gcc cct gat 3408
Ile His Glu Ser Gly Thr Ala His Arg Pro Cys Ser Pro Ala Pro Asp
        1120                1125                1130

gag cag tcc ata tgg aac gtc acg gtg ctc ccc aac agt aaa tgg gcc 3456
Glu Gln Ser Ile Trp Asn Val Thr Val Leu Pro Asn Ser Lys Trp Ala
    1135                1140                1145

aac atc acc tgg aag cac aat ttc ggg ccc gga act gac ttt gtg gtt 3504
Asn Ile Thr Trp Lys His Asn Phe Gly Pro Gly Thr Asp Phe Val Val
1150                1155                1160                1165

gag tac atc gac agt aac cat acg aaa aaa act gtc cca gtt aag gcc 3552
Glu Tyr Ile Asp Ser Asn His Thr Lys Lys Thr Val Pro Val Lys Ala
                1170                1175                1180

cag gct cag cct ata cag ctg aca gac ctc tat ccc ggg atg aca tac 3600
Gln Ala Gln Pro Ile Gln Leu Thr Asp Leu Tyr Pro Gly Met Thr Tyr
            1185                1190                1195

acg ttg cgg gtt tat tcc cgg gac aac gag ggc atc agc aat cat tca 3648
Thr Leu Arg Val Tyr Ser Arg Asp Asn Glu Gly Ile Ser Asn His Ser
        1200                1205                1210

cgg gtt tgc ttc cgg ccc tcc ccg cca gct tac acc aac aac caa gcg 3696
Arg Val Cys Phe Arg Pro Ser Pro Pro Ala Tyr Thr Asn Asn Gln Ala
    1215                1220                1225

gac atc gcc acc cag ggc tgg ttc att ggg ctt atg tgc gcc atc gcc    3744
Asp Ile Ala Thr Gln Gly Trp Phe Ile Gly Leu Met Cys Ala Ile Ala
1230                1235                1240                1245

ctc ctg gtg ctg atc ctg ctc atc gtc tgt ttc atc aag agg agt cgc    3792
Leu Leu Val Leu Ile Leu Leu Ile Val Cys Phe Ile Lys Arg Ser Arg
                1250                1255                1260

ggc ggc aag tac cca gta cga gaa aag aag gat gtt ccc ctt ggc cct    3840
Gly Gly Lys Tyr Pro Val Arg Glu Lys Lys Asp Val Pro Leu Gly Pro
            1265                1270                1275

gaa gac ccc aag gaa gag gat ggc tca ttt gac tat agg tct ttg gcc    3888
Glu Asp Pro Lys Glu Glu Asp Gly Ser Phe Asp Tyr Arg Ser Leu Ala
        1280                1285                1290

agt gat gag gac aac aag ccc ctg cag ggc agt cag aca tct ctg gac    3936
Ser Asp Glu Asp Asn Lys Pro Leu Gln Gly Ser Gln Thr Ser Leu Asp
    1295                1300                1305

ggc acc atc aag cag cag gag agt gac gac agc ctg gtg gac tat ggc    3984
Gly Thr Ile Lys Gln Gln Glu Ser Asp Asp Ser Leu Val Asp Tyr Gly
1310                1315                1320                1325

gag ggt ggc gag ggt cag ttc aat gaa gac ggc tcc ttc atc ggc cag    4032
Glu Gly Gly Glu Gly Gln Phe Asn Glu Asp Gly Ser Phe Ile Gly Gln
                1330                1335                1340

tac acg gtc aaa aag gac aag gag gaa aca gag ggc aac gaa agc tca    4080
Tyr Thr Val Lys Lys Asp Lys Glu Glu Thr Glu Gly Asn Glu Ser Ser
            1345                1350                1355

gag gcc acg tca cct gtc aat gct atc tac tct ctg gcc taacggagcc    4129
Glu Ala Thr Ser Pro Val Asn Ala Ile Tyr Ser Leu Ala
        1360                1365                1370

ca                                                                 4131

<210>60
<211>1370
<212>PRT
<213>Homo sapiens

<400>60

Met Ala Arg Gln Pro Pro Pro Pro Trp Ile His Ala Ala Phe Leu Leu
  1               5                  10                  15

Cys Leu Leu Ser Leu Gly Gly Ala Ile Glu Ile Pro Met Val Pro Ser
             20                  25                  30

Ile Gln Asn Glu Leu Thr Gln Pro Pro Thr Ile Thr Lys Gln Ser Ala
         35                  40                  45

Lys Asp His Ile Val Asp Pro Arg Asp Asn Ile Leu Ile Glu Cys Glu

50                  55                  60

Ala Lys Gly Asn Pro Ala Pro Ser Phe His Trp Thr Arg Asn Ser Arg
65                  70                  75                  80

Phe Phe Asn Ile Ala Lys Asp Pro Arg Val Ser Met Arg Arg Arg Ser
                85                  90                  95

Gly Thr Leu Val Ile Asp Phe Arg Ser Gly Gly Arg Pro Glu Glu Tyr
            100                 105                 110

Glu Gly Glu Tyr Gln Cys Phe Ala Arg Asn Lys Phe Gly Thr Ala Leu
        115                 120                 125

Ser Asn Arg Ile Arg Leu Gln Val Ser Lys Ser Pro Leu Trp Pro Lys
    130                 135                 140

Glu Asn Leu Asp Pro Val Val Val Gln Glu Gly Ala Pro Leu Thr Leu
145                 150                 155                 160

Gln Cys Asn Pro Pro Pro Gly Leu Pro Ser Pro Val Ile Phe Trp Met
                165                 170                 175

Ser Ser Ala Met Glu Pro Ile Thr Gln Asp Lys Arg Val Ser Gln Gly
            180                 185                 190

His Asn Gly Asp Leu Tyr Phe Ser Asn Val Met Leu Gln Asp Met Gln
        195                 200                 205

Thr Asp Tyr Ser Cys Asn Ala Arg Phe His Phe Thr His Thr Ile Gln
    210                 215                 220

Gln Lys Asn Pro Phe Thr Leu Lys Val Leu Thr Ser Lys Pro Tyr Asn
225                 230                 235                 240

Asp Ser Ser Leu Arg Asn His Pro Asp Met Tyr Ser Ala Arg Gly Val
                245                 250                 255

Ala Glu Arg Thr Pro Ser Phe Met Tyr Pro Gln Gly Thr Ala Ser Ser
            260                 265                 270

Gln Met Val Leu Arg Gly Met Asp Leu Leu Leu Glu Cys Ile Ala Ser
        275                 280                 285

Gly Val Pro Thr Pro Asp Ile Ala Trp Tyr Lys Lys Gly Gly Asp Leu
    290                 295                 300

Pro Ser Asp Lys Ala Lys Phe Glu Asn Phe Asn Lys Ala Leu Arg Ile
305                 310                 315                 320

Thr Asn Val Ser Glu Glu Asp Ser Gly Glu Tyr Phe Cys Leu Ala Ser
                325                 330                 335

Asn Lys Met Gly Ser Ile Arg His Thr Ile Ser Val Arg Val Lys Ala
            340                 345                 350

Ala Pro Tyr Trp Leu Asp Glu Pro Lys Asn Leu Ile Leu Ala Pro Gly

        355                 360                 365

Glu Asp Gly Arg Leu Val Cys Arg Ala Asn Gly Asn Pro Lys Pro Thr
    370                 375                 380

Val Gln Trp Met Val Asn Gly Glu Pro Leu Gln Ala Ala Pro Pro Asn
385                 390                 395                 400

Pro Asn Arg Glu Val Ala Gly Asp Thr Ile Ile Phe Arg Asp Thr Gln
                405                 410                 415

Ile Ser Ser Arg Ala Val Tyr Gln Cys Asn Thr Ser Asn Glu His Gly
            420                 425                 430

Tyr Leu Leu Ala Asn Ala Phe Val Ser Val Leu Asp Val Pro Pro Arg
        435                 440                 445

Met Leu Ser Pro Arg Asn Gln Leu Ile Arg Val Ile Leu Tyr Asn Arg
    450                 455                 460

Thr Arg Leu Asp Cys Pro Phe Phe Gly Ser Pro Ile Pro Thr Leu Arg
465                 470                 475                 480

Trp Phe Lys Asn Gly Gln Gly Ser Asn Leu Asp Gly Gly Asn Tyr His
                485                 490                 495

Val Tyr Glu Asn Gly Ser Leu Glu Ile Lys Met Ile Arg Lys Glu Asp
            500                 505                 510

Gln Gly Ile Tyr Thr Cys Val Ala Thr Asn Ile Leu Gly Lys Ala Glu
        515                 520                 525

Asn Gln Val Arg Leu Glu Val Lys Asp Pro Thr Arg Ile Tyr Arg Met
    530                 535                 540

Pro Glu Asp Gln Val Ala Arg Arg Gly Thr Thr Val Gln Leu Glu Cys
545                 550                 555                 560

Arg Val Lys His Asp Pro Ser Leu Lys Leu Thr Val Tyr Trp Leu Lys
                565                 570                 575

Asp Asp Glu Pro Leu Tyr Ile Gly Asn Arg Met Lys Lys Glu Asp Asp
            580                 585                 590

Ser Leu Thr Ile Phe Gly Val Ala Glu Arg Asp Gln Gly Ser Tyr Thr
        595                 600                 605

Cys Val Ala Ser Thr Glu Leu Asp Gln Asp Leu Ala Lys Ala Tyr Leu
    610                 615                 620

Thr Val Leu Gly Arg Pro Asp Arg Pro Arg Asp Leu Glu Leu Thr Asp
625                 630                 635                 640

Leu Ala Glu Arg Ser Val Arg Leu Thr Trp Ile Pro Gly Asp Ala Asn
                645                 650                 655

Asn Ser Pro Ile Thr Asp Tyr Val Val Gln Phe Glu Glu Asp Gln Phe

            660                 665                 670

Gln Pro Gly Val Trp His Asp His Ser Lys Tyr Pro Gly Ser Val Asn
        675                 680                 685

Ser Ala Val Leu Arg Leu Ser Pro Tyr Val Asn Tyr Gln Phe Arg Val
    690                 695                 700

Ile Ala Ile Asn Glu Val Gly Ser Ser His Pro Ser Leu Pro Ser Glu
705                 710                 715                 720

Arg Tyr Arg Thr Ser Gly Ala Pro Pro Glu Ser Asn Pro Gly Asp Val
                725                 730                 735

Lys Gly Glu Gly Thr Arg Lys Asn Asn Met Glu Ile Thr Trp Thr Pro
            740                 745                 750

Met Asn Ala Thr Ser Ala Phe Gly Pro Asn Leu Arg Tyr Ile Val Lys
        755                 760                 765

Trp Arg Arg Arg Glu Thr Arg Glu Ala Trp Asn Asn Val Thr Val Trp
    770                 775                 780

Gly Ser Arg Tyr Val Val Gly Gln Thr Pro Val Tyr Val Pro Tyr Glu
785                 790                 795                 800

Ile Arg Val Gln Ala Glu Asn Asp Phe Gly Lys Gly Pro Glu Pro Glu
                805                 810                 815

Ser Val Ile Gly Tyr Ser Gly Glu Asp Tyr Pro Arg Ala Ala Pro Thr
            820                 825                 830

Glu Val Lys Val Arg Val Met Asn Ser Thr Ala Ile Ser Leu Gln Trp
        835                 840                 845

Asn Arg Val Tyr Ser Asp Thr Val Gln Gly Gln Leu Arg Glu Tyr Arg
    850                 855                 860

Ala Tyr Tyr Trp Arg Glu Ser Ser Leu Leu Lys Asn Leu Trp Val Ser
865                 870                 875                 880

Gln Lys Arg Gln Gln Ala Ser Phe Pro Gly Asp Arg Leu Arg Gly Val
                885                 890                 895

Val Ser Arg Leu Phe Pro Tyr Ser Asn Tyr Lys Leu Glu Met Val Val
            900                 905                 910

Val Asn Gly Arg Gly Asp Gly Pro Arg Ser Glu Thr Lys Glu Phe Thr
        915                 920                 925

Thr Pro Glu Gly Val Pro Ser Ala Pro Arg Arg Phe Arg Val Arg Gln
    930                 935                 940

Pro Asn Leu Glu Thr Ile Asn Leu Glu Trp Asp His Pro Glu His Pro
945                 950                 955                 960

Asn Gly Ile Met Ile Gly Tyr Thr Leu Lys Tyr Val Ala Cys Thr Phe

                965                 970                 975

Ser Pro Val Asn Gly Thr Lys Val Gly Lys Gln Ile Val Glu Asn Phe
            980                 985                 990

Ser Pro Asn Gln Thr Lys Phe Thr Val Gln Arg Thr Asp Pro Val Ser
        995                 1000                1005

Arg Tyr Arg Phe Thr Leu Ser Ala Arg Thr Gln Val Gly Ser Gly Glu
    1010                1015                1020

Ala Val Thr Glu Glu Ser Pro Ala Pro Pro Asn Glu Gly Arg Cys Met
1025                1030                1035                1040

Ala Ala Ala Pro Gly Val Lys Pro Pro Thr Thr Val Gly Ala Thr Gly
                1045                1050                1055

Ala Val Ser Ser Thr Asp Ala Thr Ala Ile Ala Ala Thr Thr Glu Ala
            1060                1065                1070

Thr Thr Val Pro Ile Ile Pro Thr Val Ala Pro Thr Thr Met Ala Thr
        1075                1080                1085

Thr Thr Thr Val Ala Thr Thr Thr Thr Thr Thr Ala Ala Ala Thr Thr
    1090                1095                1100

Thr Thr Glu Ser Pro Pro Thr Thr Thr Ser Gly Thr Lys Ile His Glu
1105                1110                1115                1120

Ser Gly Thr Ala His Arg Pro Cys Ser Pro Ala Pro Asp Glu Gln Ser
                1125                1130                1135

Ile Trp Asn Val Thr Val Leu Pro Asn Ser Lys Trp Ala Asn Ile Thr
            1140                1145                1150

Trp Lys His Asn Phe Gly Pro Gly Thr Asp Phe Val Val Glu Tyr Ile
        1155                1160                1165

Asp Ser Asn His Thr Lys Lys Thr Val Pro Val Lys Ala Gln Ala Gln
    1170                1175                1180

Pro Ile Gln Leu Thr Asp Leu Tyr Pro Gly Met Thr Tyr Thr Leu Arg
1185                1190                1195                1200

Val Tyr Ser Arg Asp Asn Glu Gly Ile Ser Asn His Ser Arg Val Cys
                1205                1210                1215

Phe Arg Pro Ser Pro Pro Ala Tyr Thr Asn Asn Gln Ala Asp Ile Ala
            1220                1225                1230

Thr Gln Gly Trp Phe Ile Gly Leu Met Cys Ala Ile Ala Leu Leu Val
        1235                1240                1245

Leu Ile Leu Leu Ile Val Cys Phe Ile Lys Arg Ser Arg Gly Gly Lys
    1250                1255                1260

Tyr Pro Val Arg Glu Lys Lys Asp Val Pro Leu Gly Pro Glu Asp Pro

1265                    1270                    1275                    1280

Lys Glu Glu Asp Gly Ser Phe Asp Tyr Arg Ser Leu Ala Ser Asp Glu
                1285                    1290                    1295

Asp Asn Lys Pro Leu Gln Gly Ser Gln Thr Ser Leu Asp Gly Thr Ile
            1300                    1305                    1310

Lys Gln Gln Glu Ser Asp Asp Ser Leu Val Asp Tyr Gly Glu Gly Gly
        1315                    1320                    1325

Glu Gly Gln Phe Asn Glu Asp Gly Ser Phe Ile Gly Gln Tyr Thr Val
    1330                    1335                    1340

Lys Lys Asp Lys Glu Glu Thr Glu Gly Asn Glu Ser Ser Glu Ala Thr
1345                    1350                    1355                    1360

Ser Pro Val Asn Ala Ile Tyr Ser Leu Ala
                1365                    1370

<210>61
<211>2497
<212>DNA
<213>Homo sapiens

<220>
<221> CDS
<222>(7)..(2464)

<400>61

gccccg atg gac gcc gcg ttc ctc ctc gtc ctc ggg ctg ttg gcc cag     48
       Met Asp Ala Ala Phe Leu Leu Val Leu Gly Leu Leu Ala Gln
         1               5                  10

agc ctc tgc ctg tct ttg ggg gtt cct gga tgg agg agg ccc acc acc     96
Ser Leu Cys Leu Ser Leu Gly Val Pro Gly Trp Arg Arg Pro Thr Thr
 15                  20                  25                  30

ctg tac ccc tgg cgc cgg gcg cct gcc ctg agc cgc gtg cgg agg gcc    144
Leu Tyr Pro Trp Arg Arg Ala Pro Ala Leu Ser Arg Val Arg Arg Ala
                 35                  40                  45

tgg gtc atc ccc ccg atc agc gta tcc gag aac cac aag cgt ctc ccc    192
Trp Val Ile Pro Pro Ile Ser Val Ser Glu Asn His Lys Arg Leu Pro
             50                  55                  60

tac ccc ctg gtt cag gtg agc agg tgg aag cac cag ttg gcc agc gtc    240
Tyr Pro Leu Val Gln Val Ser Arg Trp Lys His Gln Leu Ala Ser Val
         65                  70                  75

atc tcc agc atc cag ggc ccc ggc gtg gat gag gag ccc cgg ggc gtc    288
Ile Ser Ser Ile Gln Gly Pro Gly Val Asp Glu Glu Pro Arg Gly Val
     80                  85                  90

ttc tct atc gcc cag ttc aca ggg aag gtc ttc ctc aat gcc atg ctg    336
Phe Ser Ile Ala Gln Phe Thr Gly Lys Val Phe Leu Asn Ala Met Leu

95                  100                 105                 110

gac cgc gag aag act gat cgc ttc agg cta aga ggg ttt gcc ctg gac    384
Asp Arg Glu Lys Thr Asp Arg Phe Arg Leu Arg Gly Phe Ala Leu Asp
                115                 120                 125

ctg gga gga tcc acc ctg gag gac ccc acg gac ctg gag att gta gtt    432
Leu Gly Gly Ser Thr Leu Glu Asp Pro Thr Asp Leu Glu Ile Val Val
            130                 135                 140

gtg gat cag aat gac aac cgg cca gcc ttc ctg cag gag gcg ttc act    480
Val Asp Gln Asn Asp Asn Arg Pro Ala Phe Leu Gln Glu Ala Phe Thr
        145                 150                 155

ggc cgc gtg ctg gag ggt gca gtc cca ggt acc tat gtg acc agg gca    528
Gly Arg Val Leu Glu Gly Ala Val Pro Gly Thr Tyr Val Thr Arg Ala
    160                 165                 170

gag gcc aca gat gcc gac gac ccc gag acg gac aac gca gcg ctg cgg    576
Glu Ala Thr Asp Ala Asp Asp Pro Glu Thr Asp Asn Ala Ala Leu Arg
175                 180                 185                 190

ttc tcc atc ctg cag cag ggc agc ccc gag ctc ttc agc atc gac gag    624
Phe Ser Ile Leu Gln Gln Gly Ser Pro Glu Leu Phe Ser Ile Asp Glu
                195                 200                 205

ctc aca gga gag atc cgc aca gtg caa gtg ggg ctg gac cgc gag gtg    672
Leu Thr Gly Glu Ile Arg Thr Val Gln Val Gly Leu Asp Arg Glu Val
            210                 215                 220

gtc gcg gtg tac aat ctg acc ctg cag gtg gcg gac atg tct gga gac    720
Val Ala Val Tyr Asn Leu Thr Leu Gln Val Ala Asp Met Ser Gly Asp
        225                 230                 235

ggc ctc aca gcc act gct tca gcc atc atc acc ttt gat gac atc aat    768
Gly Leu Thr Ala Thr Ala Ser Ala Ile Ile Thr Phe Asp Asp Ile Asn
    240                 245                 250

gac aat gcc ccc gag ttc acc agg gat gag ttc ttc atg gag gcc ata    816
Asp Asn Ala Pro Glu Phe Thr Arg Asp Glu Phe Phe Met Glu Ala Ile
255                 260                 265                 270

gag gcc gtc agc gga gtg gat gtg gga cgc ctg gaa gtg gag gac agg    864
Glu Ala Val Ser Gly Val Asp Val Gly Arg Leu Glu Val Glu Asp Arg
                275                 280                 285

gac ctg cca ggc tcc cca aac tgg gtg gcc agg ttc acc atc ctg gaa    912
Asp Leu Pro Gly Ser Pro Asn Trp Val Ala Arg Phe Thr Ile Leu Glu
            290                 295                 300

ggc gac ccc gat ggg cag ttc acc atc cgc acg gac ccc aag acc aac    960
Gly Asp Pro Asp Gly Gln Phe Thr Ile Arg Thr Asp Pro Lys Thr Asn
        305                 310                 315

gag ggt gtt ctg tcc att gtg aag gcc ctg gac tat gaa agc tgt gaa    1008
Glu Gly Val Leu Ser Ile Val Lys Ala Leu Asp Tyr Glu Ser Cys Glu
    320                 325                 330

cac tac gaa act aaa aca cac ggg cag gat aag aca gag aac gca cgg 1056
His Tyr Glu Thr Lys Thr His Gly Gln Asp Lys Thr Glu Asn Ala Arg
335                 340                 345                 350

gca ggg ctg agg gct gag cgg ggc cag gcc aag gtc cgc gtg cat gtg 1104
Ala Gly Leu Arg Ala Glu Arg Gly Gln Ala Lys Val Arg Val His Val
                355                 360                 365

cag gac acc aac gag ccc ccc gtg ttc cag gag aac cca ctt cgg acc 1152
Gln Asp Thr Asn Glu Pro Pro Val Phe Gln Glu Asn Pro Leu Arg Thr
            370                 375                 380

agc cta gca gag ggg gca ccc cca ggc act ctg gtg gcc acc ttc tct 1200
Ser Leu Ala Glu Gly Ala Pro Pro Gly Thr Leu Val Ala Thr Phe Ser
        385                 390                 395

gcc cgg gac cct gac aca gag cag ctg cag agg ctc agc tac tcc aag 1248
Ala Arg Asp Pro Asp Thr Glu Gln Leu Gln Arg Leu Ser Tyr Ser Lys
    400                 405                 410

gac tac gac ccg gaa gac tgg ctg caa gtg gac gca gcc act ggc cgg 1296
Asp Tyr Asp Pro Glu Asp Trp Leu Gln Val Asp Ala Ala Thr Gly Arg
415                 420                 425                 430

atc cag acc cag cac gtg ctc agc ccg gcg tcc cct ttc ctc aag ggc 1344
Ile Gln Thr Gln His Val Leu Ser Pro Ala Ser Pro Phe Leu Lys Gly
                435                 440                 445

ggc tgg tac aga gcc atc gtc ttg gcc cag gat gcc tcc cag ccc cgc 1392
Gly Trp Tyr Arg Ala Ile Val Leu Ala Gln Asp Ala Ser Gln Pro Arg
            450                 455                 460

acc gcc acc ggc acc ctg tcc atc gag atc ctg gag gtg aac gac cat 1440
Thr Ala Thr Gly Thr Leu Ser Ile Glu Ile Leu Glu Val Asn Asp His
        465                 470                 475

gca cct gtg ctg gcc ccg ccg ccg ccg ggc agc ctg tgc agc gag cca 1488
Ala Pro Val Leu Ala Pro Pro Pro Pro Gly Ser Leu Cys Ser Glu Pro
    480                 485                 490

cac caa ggc cca ggc ctc ctc ctg ggc gcc acg gat gag gac ctg ccc 1536
His Gln Gly Pro Gly Leu Leu Leu Gly Ala Thr Asp Glu Asp Leu Pro
495                 500                 505                 510

ccc cac ggg gcc ccc ttc cac ttc cag ctg agc ccc agg ctc cca gag 1584
Pro His Gly Ala Pro Phe His Phe Gln Leu Ser Pro Arg Leu Pro Glu
                515                 520                 525

ctc ggc cgg aac tgg agc ctc agc cag gtc aac cct ctc tcc cat cac 1632
Leu Gly Arg Asn Trp Ser Leu Ser Gln Val Asn Pro Leu Ser His His
            530                 535                 540

cgt ctc cac cca gac ccc cac ctg ccc cat ggc ccc cat ttc atg tct 1680
Arg Leu His Pro Asp Pro His Leu Pro His Gly Pro His Phe Met Ser
        545                 550                 555

gtg gct cac cag ctt ttc ccc aga ccc agc tcc gga gcc cac agg cgt    1728
Val Ala His Gln Leu Phe Pro Arg Pro Ser Ser Gly Ala His Arg Arg
    560                 565                 570

ggc cga tgc aga aac ctc agg aag gtg tgt tgt gaa tgt ggg agg gag    1776
Gly Arg Cys Arg Asn Leu Arg Lys Val Cys Cys Glu Cys Gly Arg Glu
575                 580                 585                 590

ggt gtg gcg gtc gtg ggc tgt gcg gga gtt ctg act agg gga agt ggg    1824
Gly Val Ala Val Val Gly Cys Ala Gly Val Leu Thr Arg Gly Ser Gly
                595                 600                 605

ctc agc ctg ggc gca ctg gtc atc gtg ctg gcc agc gcc ctc ctg ctg    1872
Leu Ser Leu Gly Ala Leu Val Ile Val Leu Ala Ser Ala Leu Leu Leu
            610                 615                 620

ctg gtg ctg gtc ctg ctc gtg gca ctc cgg gcg cgg ttc tgg aag cag    1920
Leu Val Leu Val Leu Leu Val Ala Leu Arg Ala Arg Phe Trp Lys Gln
        625                 630                 635

tct cgg ggc aag ggg ctg ctg cac ggc ccc cag gac gac ctt cga gac    1968
Ser Arg Gly Lys Gly Leu Leu His Gly Pro Gln Asp Asp Leu Arg Asp
    640                 645                 650

aat gtc ctc aac tac gat gag caa gga ggc ggg gag gag gac cag gac    2016
Asn Val Leu Asn Tyr Asp Glu Gln Gly Gly Gly Glu Glu Asp Gln Asp
655                 660                 665                 670

gcc tac gac atc agc cag ctg cgt cac ccg aca gcg ctg agc ctg cct    2064
Ala Tyr Asp Ile Ser Gln Leu Arg His Pro Thr Ala Leu Ser Leu Pro
                675                 680                 685

ctg gga ccg ccg cca ctt cgc aga gat gcc ccg cag ggc cgc ctg cac    2112
Leu Gly Pro Pro Pro Leu Arg Arg Asp Ala Pro Gln Gly Arg Leu His
            690                 695                 700

ccc cag cca ccc cga gtg ctg ccc acc agc ccc ctg gac atc gcc gac    2160
Pro Gln Pro Pro Arg Val Leu Pro Thr Ser Pro Leu Asp Ile Ala Asp
        705                 710                 715

ttc atc aat gat ggc ttg gag gct gca gat agt gac ccc agt gtg ccg    2208
Phe Ile Asn Asp Gly Leu Glu Ala Ala Asp Ser Asp Pro Ser Val Pro
    720                 725                 730

cct tac gac aca gcc ctc atc tat gac tac gag ggt gac ggc tcg gtg    2256
Pro Tyr Asp Thr Ala Leu Ile Tyr Asp Tyr Glu Gly Asp Gly Ser Val
735                 740                 745                 750

gcg ggg acg ctg agc tcc atc ctg tcc agc cag ggc gat gag gac cag    2304
Ala Gly Thr Leu Ser Ser Ile Leu Ser Ser Gln Gly Asp Glu Asp Gln
                755                 760                 765

gac tac gac tac ctc aga gac tgg ggg ccc cgc ttc gcc cgg ctg gca    2352
Asp Tyr Asp Tyr Leu Arg Asp Trp Gly Pro Arg Phe Ala Arg Leu Ala
            770                 775                 780

gac atg tat ggg cac ccg tgc ggg ttg gag tac ggg gcc aga tgg gac    2400

Asp Met Tyr Gly His Pro Cys Gly Leu Glu Tyr Gly Ala Arg Trp Asp
        785                 790                 795

cac cag gcc agg gag ggt ctt tct cct ggg gca ctg cta ccc aga cac 2448
His Gln Ala Arg Glu Gly Leu Ser Pro Gly Ala Leu Leu Pro Arg His
    800                 805                 810

aga ggc cgg aca gcc tgaccctggg gcgcaactgg acatgccact cccc 2497
Arg Gly Arg Thr Ala
815

<210>62
<211>819
<212>PRT
<213>Homo sapiens

<400>62

Met Asp Ala Ala Phe Leu Leu Val Leu Gly Leu Leu Ala Gln Ser Leu
1               5                   10                  15

Cys Leu Ser Leu Gly Val Pro Gly Trp Arg Arg Pro Thr Thr Leu Tyr
            20                  25                  30

Pro Trp Arg Arg Ala Pro Ala Leu Ser Arg Val Arg Arg Ala Trp Val
        35                  40                  45

Ile Pro Pro Ile Ser Val Ser Glu Asn His Lys Arg Leu Pro Tyr Pro
    50                  55                  60

Leu Val Gln Val Ser Arg Trp Lys His Gln Leu Ala Ser Val Ile Ser
65                  70                  75                  80

Ser Ile Gln Gly Pro Gly Val Asp Glu Glu Pro Arg Gly Val Phe Ser
                85                  90                  95

Ile Ala Gln Phe Thr Gly Lys Val Phe Leu Asn Ala Met Leu Asp Arg
            100                 105                 110

Glu Lys Thr Asp Arg Phe Arg Leu Arg Gly Phe Ala Leu Asp Leu Gly
        115                 120                 125

Gly Ser Thr Leu Glu Asp Pro Thr Asp Leu Glu Ile Val Val Val Asp
    130                 135                 140

Gln Asn Asp Asn Arg Pro Ala Phe Leu Gln Glu Ala Phe Thr Gly Arg
145                 150                 155                 160

Val Leu Glu Gly Ala Val Pro Gly Thr Tyr Val Thr Arg Ala Glu Ala
                165                 170                 175

Thr Asp Ala Asp Asp Pro Glu Thr Asp Asn Ala Ala Leu Arg Phe Ser
            180                 185                 190

Ile Leu Gln Gln Gly Ser Pro Glu Leu Phe Ser Ile Asp Glu Leu Thr
        195                 200                 205

Gly Glu Ile Arg Thr Val Gln Val Gly Leu Asp Arg Glu Val Val Ala
    210                 215                 220

Val Tyr Asn Leu Thr Leu Gln Val Ala Asp Met Ser Gly Asp Gly Leu
225                 230                 235                 240

Thr Ala Thr Ala Ser Ala Ile Ile Thr Phe Asp Asp Ile Asn Asp Asn
                245                 250                 255

Ala Pro Glu Phe Thr Arg Asp Glu Phe Phe Met Glu Ala Ile Glu Ala
            260                 265                 270

Val Ser Gly Val Asp Val Gly Arg Leu Glu Val Glu Asp Arg Asp Leu
        275                 280                 285

Pro Gly Ser Pro Asn Trp Val Ala Arg Phe Thr Ile Leu Glu Gly Asp
    290                 295                 300

Pro Asp Gly Gln Phe Thr Ile Arg Thr Asp Pro Lys Thr Asn Glu Gly
305                 310                 315                 320

Val Leu Ser Ile Val Lys Ala Leu Asp Tyr Glu Ser Cys Glu His Tyr
                325                 330                 335

Glu Thr Lys Thr His Gly Gln Asp Lys Thr Glu Asn Ala Arg Ala Gly
            340                 345                 350

Leu Arg Ala Glu Arg Gly Gln Ala Lys Val Arg Val His Val Gln Asp
        355                 360                 365

Thr Asn Glu Pro Pro Val Phe Gln Glu Asn Pro Leu Arg Thr Ser Leu
    370                 375                 380

Ala Glu Gly Ala Pro Pro Gly Thr Leu Val Ala Thr Phe Ser Ala Arg
385                 390                 395                 400

Asp Pro Asp Thr Glu Gln Leu Gln Arg Leu Ser Tyr Ser Lys Asp Tyr
                405                 410                 415

Asp Pro Glu Asp Trp Leu Gln Val Asp Ala Ala Thr Gly Arg Ile Gln
            420                 425                 430

Thr Gln His Val Leu Ser Pro Ala Ser Pro Phe Leu Lys Gly Gly Trp
        435                 440                 445

Tyr Arg Ala Ile Val Leu Ala Gln Asp Ala Ser Gln Pro Arg Thr Ala
    450                 455                 460

Thr Gly Thr Leu Ser Ile Glu Ile Leu Glu Val Asn Asp His Ala Pro
465                 470                 475                 480

Val Leu Ala Pro Pro Pro Pro Gly Ser Leu Cys Ser Glu Pro His Gln
                485                 490                 495

Gly Pro Gly Leu Leu Leu Gly Ala Thr Asp Glu Asp Leu Pro Pro His
            500                 505                 510

Gly Ala Pro Phe His Phe Gln Leu Ser Pro Arg Leu Pro Glu Leu Gly
        515                 520                 525

Arg Asn Trp Ser Leu Ser Gln Val Asn Pro Leu Ser His His Arg Leu
    530                 535                 540

His Pro Asp Pro His Leu Pro His Gly Pro His Phe Met Ser Val Ala
545                 550                 555                 560

His Gln Leu Phe Pro Arg Pro Ser Ser Gly Ala His Arg Arg Gly Arg
                565                 570                 575

Cys Arg Asn Leu Arg Lys Val Cys Cys Glu Cys Gly Arg Glu Gly Val
            580                 585                 590

Ala Val Val Gly Cys Ala Gly Val Leu Thr Arg Gly Ser Gly Leu Ser
        595                 600                 605

Leu Gly Ala Leu Val Ile Val Leu Ala Ser Ala Leu Leu Leu Val
    610                 615                 620

Leu Val Leu Leu Val Ala Leu Arg Ala Arg Phe Trp Lys Gln Ser Arg
625                 630                 635                 640

Gly Lys Gly Leu Leu His Gly Pro Gln Asp Asp Leu Arg Asp Asn Val
                645                 650                 655

Leu Asn Tyr Asp Glu Gln Gly Gly Gly Glu Glu Asp Gln Asp Ala Tyr
            660                 665                 670

Asp Ile Ser Gln Leu Arg His Pro Thr Ala Leu Ser Leu Pro Leu Gly
        675                 680                 685

Pro Pro Pro Leu Arg Arg Asp Ala Pro Gln Gly Arg Leu His Pro Gln
    690                 695                 700

Pro Pro Arg Val Leu Pro Thr Ser Pro Leu Asp Ile Ala Asp Phe Ile
705                 710                 715                 720

Asn Asp Gly Leu Glu Ala Ala Asp Ser Asp Pro Ser Val Pro Pro Tyr
                725                 730                 735

Asp Thr Ala Leu Ile Tyr Asp Tyr Glu Gly Asp Gly Ser Val Ala Gly
            740                 745                 750

Thr Leu Ser Ser Ile Leu Ser Ser Gln Gly Asp Glu Asp Gln Asp Tyr
        755                 760                 765

Asp Tyr Leu Arg Asp Trp Gly Pro Arg Phe Ala Arg Leu Ala Asp Met
    770                 775                 780

Tyr Gly His Pro Cys Gly Leu Glu Tyr Gly Ala Arg Trp Asp His Gln
785                 790                 795                 800

Ala Arg Glu Gly Leu Ser Pro Gly Ala Leu Leu Pro Arg His Arg Gly
                805                 810                 815

Arg Thr Ala

<210>63
<211>3617
<212>DNA
<213>Homo sapiens

<220>
<221> CDS
<222>(355)..(3544)

<400>63

gagatgggac tgcaatagaa atccgggcag cccgaagagg cacccagcgc 50

tccagccacc agctgggccg cccgggagtc cctggctcta gaccagccgc 100

gaggaggcgc cgcgagagag ctggtccctg cccgcggccg gaggagggct 150

agagcccctg ggccagcccc ccgagccggc tgggcgggcg ggcgggtggg 200

agcagacgcc gggcactgtc accacgggtg cgccgagcgc accgacccgg 250

gacacgggca gctggggacc gccagattcc accagccccc cttgccccgc 300

aggggtcctc ggctcgcgct cctgggtagc agccacccac cggggcggag 350

ggag atg tcg ccc ggg gcc agc cgc ggt ccc cgg gga agc cag gcg 396
Met Ser Pro Gly Ala Ser Arg Gly Pro Arg Gly Ser Gln Ala
1               5                   10

ccg ctg atc gcg ccc ctc tgc tgc gcc gcg gcc gcg ctg ggg atg ttg 444
Pro Leu Ile Ala Pro Leu Cys Cys Ala Ala Ala Ala Leu Gly Met Leu
15                  20                  25                  30

ctg tgg tcc ccc gcc tgt cag gcg ttc aac ctg gac gtg gaa aag ctc 492
Leu Trp Ser Pro Ala Cys Gln Ala Phe Asn Leu Asp Val Glu Lys Leu
                35                  40                  45

aca gtg tac agc ggc ccc aag ggc agc tac ttc ggc tac gcc gtg gac 540
Thr Val Tyr Ser Gly Pro Lys Gly Ser Tyr Phe Gly Tyr Ala Val Asp
            50                  55                  60

ttc cac ata ccc gac gcc cgc aca gcg agt gtc ttg gtg ggg gcg ccc 588
Phe His Ile Pro Asp Ala Arg Thr Ala Ser Val Leu Val Gly Ala Pro
        65                  70                  75

aaa gcc aac acc agc cag ccc gat atc gtg gaa ggg gga gcc gtc tat 636
Lys Ala Asn Thr Ser Gln Pro Asp Ile Val Glu Gly Gly Ala Val Tyr
    80                  85                  90

tac tgt cct tgg ccc gcg gag ggg tct gcg cag tgc agg cag ata ccg 684
Tyr Cys Pro Trp Pro Ala Glu Gly Ser Ala Gln Cys Arg Gln Ile Pro
95                  100                 105                 110

ttt gac acc acc aac aac aga aag atc aga gtt aat gga acc aaa gaa 732
Phe Asp Thr Thr Asn Asn Arg Lys Ile Arg Val Asn Gly Thr Lys Glu
                115                 120                 125

cct atc gag ttc aaa tcc aat cag tgg ttt gga gca aca gtg aaa gct 780
Pro Ile Glu Phe Lys Ser Asn Gln Trp Phe Gly Ala Thr Val Lys Ala
            130                 135                 140

cac aaa gga aaa gtt gtg gcc tgt gct cct tta tat cac tgg aga act 828
His Lys Gly Lys Val Val Ala Cys Ala Pro Leu Tyr His Trp Arg Thr
        145                 150                 155

ctt aaa ccg aca cca gaa aag gac cca gtt ggc acc tgc tat gta gca 876
Leu Lys Pro Thr Pro Glu Lys Asp Pro Val Gly Thr Cys Tyr Val Ala
    160                 165                 170

att cag aac ttc agc gcc tat gcc gag ttc tct cct tgc cgg aac agc 924
Ile Gln Asn Phe Ser Ala Tyr Ala Glu Phe Ser Pro Cys Arg Asn Ser
175                 180                 185                 190

aat gct gat ccg gaa ggc cag ggt tac tgc caa gca gga ttt agt ctg 972
Asn Ala Asp Pro Glu Gly Gln Gly Tyr Cys Gln Ala Gly Phe Ser Leu
                195                 200                 205

gat ttt tat aag aat gga gac ctt att gtg gga gga cct ggg agt ttc 1020
Asp Phe Tyr Lys Asn Gly Asp Leu Ile Val Gly Gly Pro Gly Ser Phe
            210                 215                 220

tac tgg caa gga caa gtg atc act gcc agt gtt gca gat atc att gca 1068
Tyr Trp Gln Gly Gln Val Ile Thr Ala Ser Val Ala Asp Ile Ile Ala
        225                 230                 235

aat tac tca ttc aag gat atc ctc agg aaa ctg gca gga gaa aag cag 1116
Asn Tyr Ser Phe Lys Asp Ile Leu Arg Lys Leu Ala Gly Glu Lys Gln
    240                 245                 250

acg gaa gtg gct cca gct tcc tat gat gac agt tac ctt gga tac tca 1164
Thr Glu Val Ala Pro Ala Ser Tyr Asp Asp Ser Tyr Leu Gly Tyr Ser
255                 260                 265                 270

gtt gct gct ggg gag ttt act ggg gat tct cag caa gaa ttg gtt gct 1212
Val Ala Ala Gly Glu Phe Thr Gly Asp Ser Gln Gln Glu Leu Val Ala
                275                 280                 285

gga att cca aga gga gca cag aat ttt gga tat gtt tcc atc att aac 1260
Gly Ile Pro Arg Gly Ala Gln Asn Phe Gly Tyr Val Ser Ile Ile Asn
            290                 295                 300

tct acg gat atg acg ttt att cag aat ttc acg gga gaa cag atg gca 1308
Ser Thr Asp Met Thr Phe Ile Gln Asn Phe Thr Gly Glu Gln Met Ala
        305                 310                 315

tct tat ttt gga tat acc gtt gtc gta tca gat gtt aac agt gat gga 1356
Ser Tyr Phe Gly Tyr Thr Val Val Val Ser Asp Val Asn Ser Asp Gly
    320                 325                 330

ctg gat gat gtc ctg gtt ggg gca cct ctc ttt atg gaa cgt gaa ttt 1404
Leu Asp Asp Val Leu Val Gly Ala Pro Leu Phe Met Glu Arg Glu Phe
335                 340                 345                 350

gag agc aac ccc aga gaa gta ggg caa atc tac ctg tat ttg caa gtg 1452
Glu Ser Asn Pro Arg Glu Val Gly Gln Ile Tyr Leu Tyr Leu Gln Val
                355                 360                 365

agc tct ctc ctc ttc aga gac ccc cag atc ctc act ggc acc gag acg 1500
Ser Ser Leu Leu Phe Arg Asp Pro Gln Ile Leu Thr Gly Thr Glu Thr
            370                 375                 380

ttt ggg aga ttc ggt agt gct atg gca cac tta gga gac ctg aac caa 1548
Phe Gly Arg Phe Gly Ser Ala Met Ala His Leu Gly Asp Leu Asn Gln
        385                 390                 395

gat gga tac aat gac att gcc atc gga gtg cct ttt gca ggc aag gat 1596
Asp Gly Tyr Asn Asp Ile Ala Ile Gly Val Pro Phe Ala Gly Lys Asp
    400                 405                 410

caa aga ggc aaa gtg ctc att tat aat ggg aac aaa gat ggc tta aac 1644
Gln Arg Gly Lys Val Leu Ile Tyr Asn Gly Asn Lys Asp Gly Leu Asn
415                 420                 425                 430

acc aag cct tcc caa gtt ctg caa gga gtg tgg gcc tca cat gct gtc 1692
Thr Lys Pro Ser Gln Val Leu Gln Gly Val Trp Ala Ser His Ala Val
                435                 440                 445

cct tcc gga ttt ggc ttt act tta aga gga gat tca gac ata gac aag 1740
Pro Ser Gly Phe Gly Phe Thr Leu Arg Gly Asp Ser Asp Ile Asp Lys
            450                 455                 460

aat gat tac cca gat ttg att gtg ggt gca ttt gga aca gga aaa gtc 1788
Asn Asp Tyr Pro Asp Leu Ile Val Gly Ala Phe Gly Thr Gly Lys Val
        465                 470                 475

gct gtt tac aga gca aga ccg gtt gtg act gta gat gcc cag ctt ctg 1836
Ala Val Tyr Arg Ala Arg Pro Val Val Thr Val Asp Ala Gln Leu Leu
    480                 485                 490

ctg cac cca atg att atc aat ctt gaa aat aaa act tgc cag gtt cca 1884
Leu His Pro Met Ile Ile Asn Leu Glu Asn Lys Thr Cys Gln Val Pro
495                 500                 505                 510

gac tct atg aca tct gct gcc tgc ttt tct tta aga gta tgt gca tct 1932

Asp Ser Met Thr Ser Ala Ala Cys Phe Ser Leu Arg Val Cys Ala Ser
                515                 520                 525

gtc aca ggc cag agc att gca aac aca ata gtc ttg atg gca gag gtg 1980
Val Thr Gly Gln Ser Ile Ala Asn Thr Ile Val Leu Met Ala Glu Val
            530                 535                 540

caa tta gat tcc ctg aaa cag aaa gga gct att aaa cgg acg ctc ttc 2028
Gln Leu Asp Ser Leu Lys Gln Lys Gly Ala Ile Lys Arg Thr Leu Phe
        545                 550                 555

ctt gat aac cat cag gct cat cgc gtc ttc cct ctt gtg ata aaa agg 2076
Leu Asp Asn His Gln Ala His Arg Val Phe Pro Leu Val Ile Lys Arg
    560                 565                 570

cag aaa tcc cac cag tgc cag gat ttc atc gtt tac ctt cga gat gaa 2124
Gln Lys Ser His Gln Cys Gln Asp Phe Ile Val Tyr Leu Arg Asp Glu
575                 580                 585                 590

act gaa ttc cga gat aaa tta tct cca atc aac att agt ttg aat tac 2172
Thr Glu Phe Arg Asp Lys Leu Ser Pro Ile Asn Ile Ser Leu Asn Tyr
                595                 600                 605

agt ttg gac gaa tcc acc ttt aaa gaa ggc ctg gaa gtg aaa cca ata 2220
Ser Leu Asp Glu Ser Thr Phe Lys Glu Gly Leu Glu Val Lys Pro Ile
            610                 615                 620

ttg aac tac tac aga gaa aac att gtt agt gaa cag gct cac att ctg 2268
Leu Asn Tyr Tyr Arg Glu Asn Ile Val Ser Glu Gln Ala His Ile Leu
        625                 630                 635

gtg gac tgt gga gaa gac aat ctg tgt gtt cct gac ttg aag ctg tcg 2316
Val Asp Cys Gly Glu Asp Asn Leu Cys Val Pro Asp Leu Lys Leu Ser
    640                 645                 650

gct aga cca gat aag cat cag gta atc att gga gat gaa aat cac ctt 2364
Ala Arg Pro Asp Lys His Gln Val Ile Ile Gly Asp Glu Asn His Leu
655                 660                 665                 670

atg ctc ata ata aat gca aga aat gaa ggg gaa gga gca tat gaa gct 2412
Met Leu Ile Ile Asn Ala Arg Asn Glu Gly Glu Gly Ala Tyr Glu Ala
                675                 680                 685

gaa ctc ttt gta atg ata cca gaa gag gca gat tat gtt gga atc gaa 2460
Glu Leu Phe Val Met Ile Pro Glu Glu Ala Asp Tyr Val Gly Ile Glu
            690                 695                 700

cgc aac aac aag gga ttt cga cca ctg agc tgt gag tac aag atg gaa 2508
Arg Asn Asn Lys Gly Phe Arg Pro Leu Ser Cys Glu Tyr Lys Met Glu
        705                 710                 715

aat gta acc agg atg gtg gtg tgt gac ctt ggg aac cct atg gtg tct 2556
Asn Val Thr Arg Met Val Val Cys Asp Leu Gly Asn Pro Met Val Ser
    720                 725                 730

gga aca aat tat tcc ctg ggc ctc cga ttt gca gtt cca cgt ctt gag 2604
Gly Thr Asn Tyr Ser Leu Gly Leu Arg Phe Ala Val Pro Arg Leu Glu

735                 740                 745                 750

aaa aca aac atg agc att aac ttc gat ctc caa atc aga agt tcc aac     2652
Lys Thr Asn Met Ser Ile Asn Phe Asp Leu Gln Ile Arg Ser Ser Asn
                755                 760                 765

aag gac aat cca gac agc aat ttt gtg agc ctg caa atc aac atc act     2700
Lys Asp Asn Pro Asp Ser Asn Phe Val Ser Leu Gln Ile Asn Ile Thr
            770                 775                 780

gct gta gcg cag gtg gaa ata aga gga gtg tca cac cct ccg cag att     2748
Ala Val Ala Gln Val Glu Ile Arg Gly Val Ser His Pro Pro Gln Ile
        785                 790                 795

gtt ctg ccc att cat aac tgg gaa cca gaa gag gag ccc cac aaa gag     2796
Val Leu Pro Ile His Asn Trp Glu Pro Glu Glu Glu Pro His Lys Glu
    800                 805                 810

gag gag gtt gga cca ttg gtg gaa cat att tat gag ctg cac aat att     2844
Glu Glu Val Gly Pro Leu Val Glu His Ile Tyr Glu Leu His Asn Ile
815                 820                 825                 830

gga cca agt acc atc agt gac acc atc ctg gag gtg ggc tgg cct ttc     2892
Gly Pro Ser Thr Ile Ser Asp Thr Ile Leu Glu Val Gly Trp Pro Phe
                835                 840                 845

tct gcc cgg gat gaa ttt ctt ctc tat att ttc cat att caa act ctg     2940
Ser Ala Arg Asp Glu Phe Leu Leu Tyr Ile Phe His Ile Gln Thr Leu
            850                 855                 860

gga cct ctg cag tgc caa cca aat cct aat atc aat cca cag gat ata     2988
Gly Pro Leu Gln Cys Gln Pro Asn Pro Asn Ile Asn Pro Gln Asp Ile
        865                 870                 875

aag cct gct gcc tcc cca gag gac acc cct gag ctc agc gcc ttt ttg     3036
Lys Pro Ala Ala Ser Pro Glu Asp Thr Pro Glu Leu Ser Ala Phe Leu
    880                 885                 890

cga aac tct act att cct cat ctt gtc agg aag agg gat gta cat gtg     3084
Arg Asn Ser Thr Ile Pro His Leu Val Arg Lys Arg Asp Val His Val
895                 900                 905                 910

gtc gaa ttc cac aga cag agc cct gca aaa ata ctg aat tgt aca aat     3132
Val Glu Phe His Arg Gln Ser Pro Ala Lys Ile Leu Asn Cys Thr Asn
                915                 920                 925

atc gag tgt tta caa atc tcc tgt gca gtg gga cga ctc gaa gga gga     3180
Ile Glu Cys Leu Gln Ile Ser Cys Ala Val Gly Arg Leu Glu Gly Gly
            930                 935                 940

gaa agc gca gtc ctg aaa gtc agg tca cga tta tgg gcc cac acc ttc     3228
Glu Ser Ala Val Leu Lys Val Arg Ser Arg Leu Trp Ala His Thr Phe
        945                 950                 955

ctc cag aga aaa aat gat ccc tat gct ctt gca tcc ctg gtg tcc ttt     3276
Leu Gln Arg Lys Asn Asp Pro Tyr Ala Leu Ala Ser Leu Val Ser Phe
    960                 965                 970

gaa gtt aag aag atg cct tat aca gat cag cca gca aaa ctc cca gaa    3324
Glu Val Lys Lys Met Pro Tyr Thr Asp Gln Pro Ala Lys Leu Pro Glu
975                 980                 985                 990

gga agc ata gta att aag aca tca gtt att tgg gca act ccg aat gtt    3372
Gly Ser Ile Val Ile Lys Thr Ser Val Ile Trp Ala Thr Pro Asn Val
                995                 1000                1005

tcc ttc tca atc cca tta tgg gta ata ata cta gca ata ctt ctt gga    3420
Ser Phe Ser Ile Pro Leu Trp Val Ile Ile Leu Ala Ile Leu Leu Gly
            1010                1015                1020

ttg ttg gtt ctc gcc att tta acc tta gct tta tgg aag tgt gga ttc    3468
Leu Leu Val Leu Ala Ile Leu Thr Leu Ala Leu Trp Lys Cys Gly Phe
        1025                1030                1035

ttt gac aga gcc aga cct cct cag gag gac atg acc gac agg gaa cag    3516
Phe Asp Arg Ala Arg Pro Pro Gln Glu Asp Met Thr Asp Arg Glu Gln
    1040                1045                1050

ctg aca aat gac aag acc cct gag gca tgacaagaaa aaaaaagaag          3563
Leu Thr Asn Asp Lys Thr Pro Glu Ala
1055                1060

accaaagacc tcaaacactg gtcctgttca aagaaaaaga aagaacatga             3613

ggcc                                                               3617

<210>64
<211>1063
<212>PRT
<213>Homo sapiens

<400>64

Met Ser Pro Gly Ala Ser Arg Gly Pro Arg Gly Ser Gln Ala Pro Leu
  1               5                  10                  15

Ile Ala Pro Leu Cys Cys Ala Ala Ala Ala Leu Gly Met Leu Leu Trp
             20                  25                  30

Ser Pro Ala Cys Gln Ala Phe Asn Leu Asp Val Glu Lys Leu Thr Val
         35                  40                  45

Tyr Ser Gly Pro Lys Gly Ser Tyr Phe Gly Tyr Ala Val Asp Phe His
     50                  55                  60

Ile Pro Asp Ala Arg Thr Ala Ser Val Leu Val Gly Ala Pro Lys Ala
 65                  70                  75                  80

Asn Thr Ser Gln Pro Asp Ile Val Glu Gly Gly Ala Val Tyr Tyr Cys
                 85                  90                  95

Pro Trp Pro Ala Glu Gly Ser Ala Gln Cys Arg Gln Ile Pro Phe Asp
            100                 105                 110

Thr Thr Asn Asn Arg Lys Ile Arg Val Asn Gly Thr Lys Glu Pro Ile
        115                 120                 125

Glu Phe Lys Ser Asn Gln Trp Phe Gly Ala Thr Val Lys Ala His Lys
    130                 135                 140

Gly Lys Val Val Ala Cys Ala Pro Leu Tyr His Trp Arg Thr Leu Lys
145                 150                 155                 160

Pro Thr Pro Glu Lys Asp Pro Val Gly Thr Cys Tyr Val Ala Ile Gln
                165                 170                 175

Asn Phe Ser Ala Tyr Ala Glu Phe Ser Pro Cys Arg Asn Ser Asn Ala
            180                 185                 190

Asp Pro Glu Gly Gln Gly Tyr Cys Gln Ala Gly Phe Ser Leu Asp Phe
        195                 200                 205

Tyr Lys Asn Gly Asp Leu Ile Val Gly Gly Pro Gly Ser Phe Tyr Trp
    210                 215                 220

Gln Gly Gln Val Ile Thr Ala Ser Val Ala Asp Ile Ile Ala Asn Tyr
225                 230                 235                 240

Ser Phe Lys Asp Ile Leu Arg Lys Leu Ala Gly Glu Lys Gln Thr Glu
                245                 250                 255

Val Ala Pro Ala Ser Tyr Asp Asp Ser Tyr Leu Gly Tyr Ser Val Ala
            260                 265                 270

Ala Gly Glu Phe Thr Gly Asp Ser Gln Gln Glu Leu Val Ala Gly Ile
        275                 280                 285

Pro Arg Gly Ala Gln Asn Phe Gly Tyr Val Ser Ile Ile Asn Ser Thr
    290                 295                 300

Asp Met Thr Phe Ile Gln Asn Phe Thr Gly Glu Gln Met Ala Ser Tyr
305                 310                 315                 320

Phe Gly Tyr Thr Val Val Val Ser Asp Val Asn Ser Asp Gly Leu Asp
                325                 330                 335

Asp Val Leu Val Gly Ala Pro Leu Phe Met Glu Arg Glu Phe Glu Ser
            340                 345                 350

Asn Pro Arg Glu Val Gly Gln Ile Tyr Leu Tyr Leu Gln Val Ser Ser
        355                 360                 365

Leu Leu Phe Arg Asp Pro Gln Ile Leu Thr Gly Thr Glu Thr Phe Gly
    370                 375                 380

Arg Phe Gly Ser Ala Met Ala His Leu Gly Asp Leu Asn Gln Asp Gly
385                 390                 395                 400

Tyr Asn Asp Ile Ala Ile Gly Val Pro Phe Ala Gly Lys Asp Gln Arg
                405                 410                 415

Gly Lys Val Leu Ile Tyr Asn Gly Asn Lys Asp Gly Leu Asn Thr Lys
            420                 425                 430

Pro Ser Gln Val Leu Gln Gly Val Trp Ala Ser His Ala Val Pro Ser
        435                 440                 445

Gly Phe Gly Phe Thr Leu Arg Gly Asp Ser Asp Ile Asp Lys Asn Asp
    450                 455                 460

Tyr Pro Asp Leu Ile Val Gly Ala Phe Gly Thr Gly Lys Val Ala Val
465                 470                 475                 480

Tyr Arg Ala Arg Pro Val Val Thr Val Asp Ala Gln Leu Leu Leu His
                485                 490                 495

Pro Met Ile Ile Asn Leu Glu Asn Lys Thr Cys Gln Val Pro Asp Ser
            500                 505                 510

Met Thr Ser Ala Ala Cys Phe Ser Leu Arg Val Cys Ala Ser Val Thr
        515                 520                 525

Gly Gln Ser Ile Ala Asn Thr Ile Val Leu Met Ala Glu Val Gln Leu
    530                 535                 540

Asp Ser Leu Lys Gln Lys Gly Ala Ile Lys Arg Thr Leu Phe Leu Asp
545                 550                 555                 560

Asn His Gln Ala His Arg Val Phe Pro Leu Val Ile Lys Arg Gln Lys
                565                 570                 575

Ser His Gln Cys Gln Asp Phe Ile Val Tyr Leu Arg Asp Glu Thr Glu
            580                 585                 590

Phe Arg Asp Lys Leu Ser Pro Ile Asn Ile Ser Leu Asn Tyr Ser Leu
        595                 600                 605

Asp Glu Ser Thr Phe Lys Glu Gly Leu Glu Val Lys Pro Ile Leu Asn
    610                 615                 620

Tyr Tyr Arg Glu Asn Ile Val Ser Glu Gln Ala His Ile Leu Val Asp
625                 630                 635                 640

Cys Gly Glu Asp Asn Leu Cys Val Pro Asp Leu Lys Leu Ser Ala Arg
                645                 650                 655

Pro Asp Lys His Gln Val Ile Ile Gly Asp Glu Asn His Leu Met Leu
            660                 665                 670

Ile Ile Asn Ala Arg Asn Glu Gly Glu Gly Ala Tyr Glu Ala Glu Leu
        675                 680                 685

Phe Val Met Ile Pro Glu Glu Ala Asp Tyr Val Gly Ile Glu Arg Asn
    690                 695                 700

Asn Lys Gly Phe Arg Pro Leu Ser Cys Glu Tyr Lys Met Glu Asn Val
705                 710                 715                 720

Thr Arg Met Val Val Cys Asp Leu Gly Asn Pro Met Val Ser Gly Thr
                725                 730                 735

Asn Tyr Ser Leu Gly Leu Arg Phe Ala Val Pro Arg Leu Glu Lys Thr
            740                 745                 750

Asn Met Ser Ile Asn Phe Asp Leu Gln Ile Arg Ser Ser Asn Lys Asp
        755                 760                 765

Asn Pro Asp Ser Asn Phe Val Ser Leu Gln Ile Asn Ile Thr Ala Val
    770                 775                 780

Ala Gln Val Glu Ile Arg Gly Val Ser His Pro Pro Gln Ile Val Leu
785                 790                 795                 800

Pro Ile His Asn Trp Glu Pro Glu Glu Glu Pro His Lys Glu Glu Glu
                805                 810                 815

Val Gly Pro Leu Val Glu His Ile Tyr Glu Leu His Asn Ile Gly Pro
            820                 825                 830

Ser Thr Ile Ser Asp Thr Ile Leu Glu Val Gly Trp Pro Phe Ser Ala
        835                 840                 845

Arg Asp Glu Phe Leu Leu Tyr Ile Phe His Ile Gln Thr Leu Gly Pro
    850                 855                 860

Leu Gln Cys Gln Pro Asn Pro Asn Ile Asn Pro Gln Asp Ile Lys Pro
865                 870                 875                 880

Ala Ala Ser Pro Glu Asp Thr Pro Glu Leu Ser Ala Phe Leu Arg Asn
                885                 890                 895

Ser Thr Ile Pro His Leu Val Arg Lys Arg Asp Val His Val Val Glu
            900                 905                 910

Phe His Arg Gln Ser Pro Ala Lys Ile Leu Asn Cys Thr Asn Ile Glu
        915                 920                 925

Cys Leu Gln Ile Ser Cys Ala Val Gly Arg Leu Glu Gly Gly Glu Ser
    930                 935                 940

Ala Val Leu Lys Val Arg Ser Arg Leu Trp Ala His Thr Phe Leu Gln
945                 950                 955                 960

Arg Lys Asn Asp Pro Tyr Ala Leu Ala Ser Leu Val Ser Phe Glu Val
                965                 970                 975

Lys Lys Met Pro Tyr Thr Asp Gln Pro Ala Lys Leu Pro Glu Gly Ser
            980                 985                 990

Ile Val Ile Lys Thr Ser Val Ile Trp Ala Thr Pro Asn Val Ser Phe
        995                 1000                1005

Ser Ile Pro Leu Trp Val Ile Ile Leu Ala Ile Leu Leu Gly Leu Leu
    1010                1015                1020

Val Leu Ala Ile Leu Thr Leu Ala Leu Trp Lys Cys Gly Phe Phe Asp
1025                1030                1035                1040

Arg Ala Arg Pro Pro Gln Glu Asp Met Thr Asp Arg Glu Gln Leu Thr
                1045                1050                1055

Asn Asp Lys Thr Pro Glu Ala
            1060

<210>65
<211>3617
<212>DNA
<213>Homo sapiens

<220>
<221> CDS
<222>(355)..(3544)

<400>65

gagatgggac tgcaatagaa atccgggcag cccgaagagg cacccagcgc          50

tccagccacc agctgggccg cccgggagtc cctggctcta gaccagccgc          100

gaggaggcgc cgcgagagag ctggtccctg cccgcggccg gaggagggct          150

agagcccctg ggccagcccc ccgagccggc tgggcgggcg ggcgggtggg          200

agcagacgcc gggcactgtc accacgggtg cgccgagcgc accgacccgg          250

gacacgggca gctggggacc gccagattcc accagccccc cttgccccgc          300

aggggtcctc ggctcgcgct cctgggtagc agccacccac cggggcggag          350

ggag atg tcg ccc ggg gcc agc cgc ggt ccc cgg gga agc cag gcg    396
     Met Ser Pro Gly Ala Ser Arg Gly Pro Arg Gly Ser Gln Ala
     1               5                   10

ccg ctg atc gcg ccc ctc tgc tgc gcc gcg gcc gcg ctg ggg atg ttg    444

Pro Leu Ile Ala Pro Leu Cys Cys Ala Ala Ala Ala Leu Gly Met Leu
15                  20                  25                  30

ctg tgg tcc ccc gcc tgt cag gcg ttc aac ctg gac gtg gaa aag ctc     492
Leu Trp Ser Pro Ala Cys Gln Ala Phe Asn Leu Asp Val Glu Lys Leu
                35                  40                  45

aca gtg tac agc ggc ccc aag ggc agc tac ttc ggc tac gcc gtg gac     540
Thr Val Tyr Ser Gly Pro Lys Gly Ser Tyr Phe Gly Tyr Ala Val Asp
            50                  55                  60

ttc cac ata ccc gac gcc cgc aca gcg agt gtc ttg gtg ggg gcg ccc     588
Phe His Ile Pro Asp Ala Arg Thr Ala Ser Val Leu Val Gly Ala Pro
        65                  70                  75

aaa gcc aac acc agc cag ccc gat atc gtg gaa ggg gga gcc gtc tat     636
Lys Ala Asn Thr Ser Gln Pro Asp Ile Val Glu Gly Gly Ala Val Tyr
    80                  85                  90

tac tgt cct tgg ccc gcg gag ggg tcc gcg cag tgc agg cag ata ccg     684
Tyr Cys Pro Trp Pro Ala Glu Gly Ser Ala Gln Cys Arg Gln Ile Pro
95                  100                 105                 110

ttt gac acc acc aac aac aga aag atc aga gtt aat gga acc aaa gaa     732
Phe Asp Thr Thr Asn Asn Arg Lys Ile Arg Val Asn Gly Thr Lys Glu
                115                 120                 125

cct atc gag ttc aaa tcc aat cag tgg ttt gga gca aca gtg aaa gct     780
Pro Ile Glu Phe Lys Ser Asn Gln Trp Phe Gly Ala Thr Val Lys Ala
            130                 135                 140

cac aaa gga aaa gtt gtg gcc tgt gct cct tta tat cac tgg aga act     828
His Lys Gly Lys Val Val Ala Cys Ala Pro Leu Tyr His Trp Arg Thr
        145                 150                 155

ctt aaa ccg aca cca gaa aag gac cca gtt ggc acc tgc tat gta gca     876
Leu Lys Pro Thr Pro Glu Lys Asp Pro Val Gly Thr Cys Tyr Val Ala
    160                 165                 170

att cag aac ttc agc gcc tat gcc gag ttc tct cct tgc cgg aac agc     924
Ile Gln Asn Phe Ser Ala Tyr Ala Glu Phe Ser Pro Cys Arg Asn Ser
175                 180                 185                 190

aat gct gat ccg gaa ggc cag ggt tac tgc caa gca gga ttt agt ctg     972
Asn Ala Asp Pro Glu Gly Gln Gly Tyr Cys Gln Ala Gly Phe Ser Leu
                195                 200                 205

gat ttt tat aag aat gga gac ctt att gtg gga gga cct ggg agt ttc     1020
Asp Phe Tyr Lys Asn Gly Asp Leu Ile Val Gly Gly Pro Gly Ser Phe
            210                 215                 220

tac tgg caa gga caa gtg atc act gcc agt gtt gca gat atc att gca     1068
Tyr Trp Gln Gly Gln Val Ile Thr Ala Ser Val Ala Asp Ile Ile Ala
        225                 230                 235

aat tac tca ttc aag gat atc ctc agg aaa ctg gca gga gaa aag cag     1116
Asn Tyr Ser Phe Lys Asp Ile Leu Arg Lys Leu Ala Gly Glu Lys Gln

240                 245                 250

acg gaa gtg gct cca gct tcc tat gat gac agt tac ctt gga tac tca    1164
Thr Glu Val Ala Pro Ala Ser Tyr Asp Asp Ser Tyr Leu Gly Tyr Ser
255                 260                 265                 270

gtt gct gct ggg gag ttt act ggg gat tct cag caa gaa ttg gtt gct    1212
Val Ala Ala Gly Glu Phe Thr Gly Asp Ser Gln Gln Glu Leu Val Ala
                275                 280                 285

gga att cca aga gga gca cag aat ttt gga tat gtt tcc atc att aac    1260
Gly Ile Pro Arg Gly Ala Gln Asn Phe Gly Tyr Val Ser Ile Ile Asn
            290                 295                 300

tct acg gat atg acg ttt att cag aat ttc acg gga gaa cag atg gca    1308
Ser Thr Asp Met Thr Phe Ile Gln Asn Phe Thr Gly Glu Gln Met Ala
        305                 310                 315

tct tat ttt gga tat acc gtt gtc gta tca gat gtt aac agt gat gga    1356
Ser Tyr Phe Gly Tyr Thr Val Val Val Ser Asp Val Asn Ser Asp Gly
    320                 325                 330

ctg gat gat gtc ctg gtt ggg gca cct ctc ttt atg gaa cgt gaa ttt    1404
Leu Asp Asp Val Leu Val Gly Ala Pro Leu Phe Met Glu Arg Glu Phe
335                 340                 345                 350

gag agc aac ccc aga gaa gta ggg caa atc tac ctg tat ttg caa gtg    1452
Glu Ser Asn Pro Arg Glu Val Gly Gln Ile Tyr Leu Tyr Leu Gln Val
                355                 360                 365

agc tct ctc ctc ttc aga gac ccc cag atc ctc act ggc acc gag acg    1500
Ser Ser Leu Leu Phe Arg Asp Pro Gln Ile Leu Thr Gly Thr Glu Thr
            370                 375                 380

ttt ggg aga ttc ggt agt gct atg gca cac tta gga gac ctg aac caa    1548
Phe Gly Arg Phe Gly Ser Ala Met Ala His Leu Gly Asp Leu Asn Gln
        385                 390                 395

gat gga tac aat gac att gcc atc gga gtg cct ttt gca ggc aag gat    1596
Asp Gly Tyr Asn Asp Ile Ala Ile Gly Val Pro Phe Ala Gly Lys Asp
    400                 405                 410

caa aga ggc aaa gtg ctc att tat aat ggg aac aaa gat ggc tta aac    1644
Gln Arg Gly Lys Val Leu Ile Tyr Asn Gly Asn Lys Asp Gly Leu Asn
415                 420                 425                 430

acc aag cct tcc caa gtt ctg caa gga gtg tgg gcc tca cat gct gtc    1692
Thr Lys Pro Ser Gln Val Leu Gln Gly Val Trp Ala Ser His Ala Val
                435                 440                 445

cct tcc gga ttt ggc ttt act tta aga gga gat tca gac ata gac aag    1740
Pro Ser Gly Phe Gly Phe Thr Leu Arg Gly Asp Ser Asp Ile Asp Lys
            450                 455                 460

aat gat tac cca gat ttg att gtg ggt gca ttt gga aca gga aaa gtc    1788
Asn Asp Tyr Pro Asp Leu Ile Val Gly Ala Phe Gly Thr Gly Lys Val
        465                 470                 475

gct gtt tac aga gca aga ccg gtt gtg act gta gat gcc cag ctt ctg 1836
Ala Val Tyr Arg Ala Arg Pro Val Val Thr Val Asp Ala Gln Leu Leu
    480                 485                 490

ctg cac cca atg att atc aat ctt gaa aat aaa act tgc cag gtt cca 1884
Leu His Pro Met Ile Ile Asn Leu Glu Asn Lys Thr Cys Gln Val Pro
495                 500                 505                 510

gac tct atg aca tct gct gcc tgc ttt tct tta aga gta tgt gca tct 1932
Asp Ser Met Thr Ser Ala Ala Cys Phe Ser Leu Arg Val Cys Ala Ser
                515                 520                 525

gtc aca ggc cag agc att gca aac aca ata gtc ttg atg gca gag gtg 1980
Val Thr Gly Gln Ser Ile Ala Asn Thr Ile Val Leu Met Ala Glu Val
            530                 535                 540

caa tta gat tcc ctg aaa cag aaa gga gct att aaa cgg acg ctc ttc 2028
Gln Leu Asp Ser Leu Lys Gln Lys Gly Ala Ile Lys Arg Thr Leu Phe
        545                 550                 555

ctt gat aac cat cag gct cat cgc gtc ttc cct ctt gtg ata aaa agg 2076
Leu Asp Asn His Gln Ala His Arg Val Phe Pro Leu Val Ile Lys Arg
    560                 565                 570

cag aaa tcc cac cag tgc cag gat ttc atc gtt tac ctt cga gat gaa 2124
Gln Lys Ser His Gln Cys Gln Asp Phe Ile Val Tyr Leu Arg Asp Glu
575                 580                 585                 590

act gaa ttc cga gat aaa tta tct cca atc aac att agt ttg aat tac 2172
Thr Glu Phe Arg Asp Lys Leu Ser Pro Ile Asn Ile Ser Leu Asn Tyr
                595                 600                 605

agt ttg gac gaa tcc acc ttt aaa gaa ggc ctg gaa gtg aaa cca ata 2220
Ser Leu Asp Glu Ser Thr Phe Lys Glu Gly Leu Glu Val Lys Pro Ile
            610                 615                 620

ttg aac tac tac aga gaa aac att gtt agt gaa cag gct cac att ctg 2268
Leu Asn Tyr Tyr Arg Glu Asn Ile Val Ser Glu Gln Ala His Ile Leu
        625                 630                 635

gtg gac tgt gga gaa gac aat ctg tgt gtt cct gac ttg aag ctg tcg 2316
Val Asp Cys Gly Glu Asp Asn Leu Cys Val Pro Asp Leu Lys Leu Ser
    640                 645                 650

gct aga cca gat aag cat cag gta atc att gga gat gaa aat cac ctt 2364
Ala Arg Pro Asp Lys His Gln Val Ile Ile Gly Asp Glu Asn His Leu
655                 660                 665                 670

atg ctc ata ata aat gca aga aat gaa ggg gag gga gca tat gaa gct 2412
Met Leu Ile Ile Asn Ala Arg Asn Glu Gly Glu Gly Ala Tyr Glu Ala
                675                 680                 685

gaa ctc ttt gta atg ata cca gaa gag gca gat tat gtt gga atc gaa 2460
Glu Leu Phe Val Met Ile Pro Glu Glu Ala Asp Tyr Val Gly Ile Glu
            690                 695                 700

cgc aac aac aag gga ttt cga cca ctg agc tgt gag tac aag atg gaa 2508
Arg Asn Asn Lys Gly Phe Arg Pro Leu Ser Cys Glu Tyr Lys Met Glu
        705                 710                 715

aat gta acc agg atg gtg gtg tgt gac ctt ggg aac cct atg gtg tct 2556
Asn Val Thr Arg Met Val Val Cys Asp Leu Gly Asn Pro Met Val Ser
    720                 725                 730

gga aca aat tat tcc ctg ggc ctc cga ttt gca gtt cca cgt ctt gag 2604
Gly Thr Asn Tyr Ser Leu Gly Leu Arg Phe Ala Val Pro Arg Leu Glu
735                 740                 745                 750

aaa aca aac atg agc att aac ttc gat ctc caa atc aga agt tcc aac 2652
Lys Thr Asn Met Ser Ile Asn Phe Asp Leu Gln Ile Arg Ser Ser Asn
                755                 760                 765

aag gac aat cca gac agc aat ttt gtg agc ctg caa atc aac atc act 2700
Lys Asp Asn Pro Asp Ser Asn Phe Val Ser Leu Gln Ile Asn Ile Thr
            770                 775                 780

gct gta gcg cag gtg gaa ata aga gga gtg tca cac cct ccg cag att 2748
Ala Val Ala Gln Val Glu Ile Arg Gly Val Ser His Pro Pro Gln Ile
        785                 790                 795

gtt ctg ccc att cat aac tgg gaa cca gaa gag gag ccc cac aaa gag 2796
Val Leu Pro Ile His Asn Trp Glu Pro Glu Glu Glu Pro His Lys Glu
    800                 805                 810

gag gag gtt gga cca ttg gtg gaa cat att tat gag ctg cac aat att 2844
Glu Glu Val Gly Pro Leu Val Glu His Ile Tyr Glu Leu His Asn Ile
815                 820                 825                 830

gga cca agt acc atc agt gac acc atc ctg gag gtg ggc tgg cct ttc 2892
Gly Pro Ser Thr Ile Ser Asp Thr Ile Leu Glu Val Gly Trp Pro Phe
                835                 840                 845

tct gcc cgg gat gaa ttt ctt ctc tat att ttc cat att caa act ctg 2940
Ser Ala Arg Asp Glu Phe Leu Leu Tyr Ile Phe His Ile Gln Thr Leu
            850                 855                 860

gga cct ctg cag tgc caa cca aat cct aat atc aat cca cag gat ata 2988
Gly Pro Leu Gln Cys Gln Pro Asn Pro Asn Ile Asn Pro Gln Asp Ile
        865                 870                 875

aag cct gct gcc tcc cca gag gac acc cct gag ctc agc gcc ttt ttg 3036
Lys Pro Ala Ala Ser Pro Glu Asp Thr Pro Glu Leu Ser Ala Phe Leu
    880                 885                 890

cga aac tct act att cct cat ctt gtc agg aag agg gat gta cat gtg 3084
Arg Asn Ser Thr Ile Pro His Leu Val Arg Lys Arg Asp Val His Val
895                 900                 905                 910

gtc gaa ttc cac aga cag agc cct gca aaa ata ctg aat tgt aca aat 3132
Val Glu Phe His Arg Gln Ser Pro Ala Lys Ile Leu Asn Cys Thr Asn
                915                 920                 925

atc gag tgt tta caa atc tcc tgt gca gtg gga cga ctc gaa gga gga 3180

Ile Glu Cys Leu Gln Ile Ser Cys Ala Val Gly Arg Leu Glu Gly Gly
            930                 935                 940

gaa agc gca gtc ctg aaa gtc agg tca cga tta tgg gcc cac acc ttc 3228
Glu Ser Ala Val Leu Lys Val Arg Ser Arg Leu Trp Ala His Thr Phe
        945                 950                 955

ctc cag aga aaa aat gat ccc tat gct ctt gca tcc ctg gtg tcc ttt 3276
Leu Gln Arg Lys Asn Asp Pro Tyr Ala Leu Ala Ser Leu Val Ser Phe
    960                 965                 970

gaa gtt aag aag atg cct tat aca gat cag cca gca aaa ctc cca gaa 3324
Glu Val Lys Lys Met Pro Tyr Thr Asp Gln Pro Ala Lys Leu Pro Glu
975                 980                 985                 990

gga agc ata gca att aag aca tca gtt att tgg gca act ccg aat gtt 3372
Gly Ser Ile Ala Ile Lys Thr Ser Val Ile Trp Ala Thr Pro Asn Val
                995                 1000                1005

tcc ttc tca atc cca tta tgg gta ata ata cta gca ata ctt ctt gga 3420
Ser Phe Ser Ile Pro Leu Trp Val Ile Ile Leu Ala Ile Leu Leu Gly
            1010                1015                1020

ttg ttg gtt ctc gcc att tta acc tta gct tta tgg aag tgt gga ttc 3468
Leu Leu Val Leu Ala Ile Leu Thr Leu Ala Leu Trp Lys Cys Gly Phe
        1025                1030                1035

ttt gac aga gcc aga cct cct cag gag gac atg acc gac agg gaa cag 3516
Phe Asp Arg Ala Arg Pro Pro Gln Glu Asp Met Thr Asp Arg Glu Gln
    1040                1045                1050

ctg aca aat gac aag acc cct gag gca tgacaagaaa aaaaaagaag 3563
Leu Thr Asn Asp Lys Thr Pro Glu Ala
1055                1060

accaaagacc tcaaacactg gtcctgttca aagaaaaaga aagaacatga 3613

ggcc 3617

<210>66
<211>1063
<212>PRT
<213>Homo sapiens

<400>66

Met Ser Pro Gly Ala Ser Arg Gly Pro Arg Gly Ser Gln Ala Pro Leu
1               5                   10                  15

Ile Ala Pro Leu Cys Cys Ala Ala Ala Ala Leu Gly Met Leu Leu Trp
            20                  25                  30

Ser Pro Ala Cys Gln Ala Phe Asn Leu Asp Val Glu Lys Leu Thr Val

        35                  40                  45

Tyr Ser Gly Pro Lys Gly Ser Tyr Phe Gly Tyr Ala Val Asp Phe His
    50                  55                  60

Ile Pro Asp Ala Arg Thr Ala Ser Val Leu Val Gly Ala Pro Lys Ala
65                  70                  75                  80

Asn Thr Ser Gln Pro Asp Ile Val Glu Gly Gly Ala Val Tyr Tyr Cys
                85                  90                  95

Pro Trp Pro Ala Glu Gly Ser Ala Gln Cys Arg Gln Ile Pro Phe Asp
            100                 105                 110

Thr Thr Asn Asn Arg Lys Ile Arg Val Asn Gly Thr Lys Glu Pro Ile
        115                 120                 125

Glu Phe Lys Ser Asn Gln Trp Phe Gly Ala Thr Val Lys Ala His Lys
    130                 135                 140

Gly Lys Val Val Ala Cys Ala Pro Leu Tyr His Trp Arg Thr Leu Lys
145                 150                 155                 160

Pro Thr Pro Glu Lys Asp Pro Val Gly Thr Cys Tyr Val Ala Ile Gln
                165                 170                 175

Asn Phe Ser Ala Tyr Ala Glu Phe Ser Pro Cys Arg Asn Ser Asn Ala
            180                 185                 190

Asp Pro Glu Gly Gln Gly Tyr Cys Gln Ala Gly Phe Ser Leu Asp Phe
        195                 200                 205

Tyr Lys Asn Gly Asp Leu Ile Val Gly Gly Pro Gly Ser Phe Tyr Trp
    210                 215                 220

Gln Gly Gln Val Ile Thr Ala Ser Val Ala Asp Ile Ile Ala Asn Tyr
225                 230                 235                 240

Ser Phe Lys Asp Ile Leu Arg Lys Leu Ala Gly Glu Lys Gln Thr Glu
                245                 250                 255

Val Ala Pro Ala Ser Tyr Asp Asp Ser Tyr Leu Gly Tyr Ser Val Ala
            260                 265                 270

Ala Gly Glu Phe Thr Gly Asp Ser Gln Gln Glu Leu Val Ala Gly Ile
        275                 280                 285

Pro Arg Gly Ala Gln Asn Phe Gly Tyr Val Ser Ile Ile Asn Ser Thr
    290                 295                 300

Asp Met Thr Phe Ile Gln Asn Phe Thr Gly Glu Gln Met Ala Ser Tyr
305                 310                 315                 320

Phe Gly Tyr Thr Val Val Val Ser Asp Val Asn Ser Asp Gly Leu Asp
                325                 330                 335

Asp Val Leu Val Gly Ala Pro Leu Phe Met Glu Arg Glu Phe Glu Ser

            340                 345                 350

Asn Pro Arg Glu Val Gly Gln Ile Tyr Leu Tyr Leu Gln Val Ser Ser
        355                 360                 365

Leu Leu Phe Arg Asp Pro Gln Ile Leu Thr Gly Thr Glu Thr Phe Gly
    370                 375                 380

Arg Phe Gly Ser Ala Met Ala His Leu Gly Asp Leu Asn Gln Asp Gly
385                 390                 395                 400

Tyr Asn Asp Ile Ala Ile Gly Val Pro Phe Ala Gly Lys Asp Gln Arg
                405                 410                 415

Gly Lys Val Leu Ile Tyr Asn Gly Asn Lys Asp Gly Leu Asn Thr Lys
            420                 425                 430

Pro Ser Gln Val Leu Gln Gly Val Trp Ala Ser His Ala Val Pro Ser
        435                 440                 445

Gly Phe Gly Phe Thr Leu Arg Gly Asp Ser Asp Ile Asp Lys Asn Asp
    450                 455                 460

Tyr Pro Asp Leu Ile Val Gly Ala Phe Gly Thr Gly Lys Val Ala Val
465                 470                 475                 480

Tyr Arg Ala Arg Pro Val Val Thr Val Asp Ala Gln Leu Leu Leu His
                485                 490                 495

Pro Met Ile Ile Asn Leu Glu Asn Lys Thr Cys Gln Val Pro Asp Ser
            500                 505                 510

Met Thr Ser Ala Ala Cys Phe Ser Leu Arg Val Cys Ala Ser Val Thr
        515                 520                 525

Gly Gln Ser Ile Ala Asn Thr Ile Val Leu Met Ala Glu Val Gln Leu
    530                 535                 540

Asp Ser Leu Lys Gln Lys Gly Ala Ile Lys Arg Thr Leu Phe Leu Asp
545                 550                 555                 560

Asn His Gln Ala His Arg Val Phe Pro Leu Val Ile Lys Arg Gln Lys
                565                 570                 575

Ser His Gln Cys Gln Asp Phe Ile Val Tyr Leu Arg Asp Glu Thr Glu
            580                 585                 590

Phe Arg Asp Lys Leu Ser Pro Ile Asn Ile Ser Leu Asn Tyr Ser Leu
        595                 600                 605

Asp Glu Ser Thr Phe Lys Glu Gly Leu Glu Val Lys Pro Ile Leu Asn
    610                 615                 620

Tyr Tyr Arg Glu Asn Ile Val Ser Glu Gln Ala His Ile Leu Val Asp
625                 630                 635                 640

Cys Gly Glu Asp Asn Leu Cys Val Pro Asp Leu Lys Leu Ser Ala Arg

                645                 650                 655

Pro Asp Lys His Gln Val Ile Ile Gly Asp Glu Asn His Leu Met Leu
            660                 665                 670

Ile Ile Asn Ala Arg Asn Glu Gly Glu Gly Ala Tyr Glu Ala Glu Leu
        675                 680                 685

Phe Val Met Ile Pro Glu Glu Ala Asp Tyr Val Gly Ile Glu Arg Asn
    690                 695                 700

Asn Lys Gly Phe Arg Pro Leu Ser Cys Glu Tyr Lys Met Glu Asn Val
705                 710                 715                 720

Thr Arg Met Val Val Cys Asp Leu Gly Asn Pro Met Val Ser Gly Thr
                725                 730                 735

Asn Tyr Ser Leu Gly Leu Arg Phe Ala Val Pro Arg Leu Glu Lys Thr
            740                 745                 750

Asn Met Ser Ile Asn Phe Asp Leu Gln Ile Arg Ser Ser Asn Lys Asp
        755                 760                 765

Asn Pro Asp Ser Asn Phe Val Ser Leu Gln Ile Asn Ile Thr Ala Val
    770                 775                 780

Ala Gln Val Glu Ile Arg Gly Val Ser His Pro Pro Gln Ile Val Leu
785                 790                 795                 800

Pro Ile His Asn Trp Glu Pro Glu Glu Glu Pro His Lys Glu Glu Glu
                805                 810                 815

Val Gly Pro Leu Val Glu His Ile Tyr Glu Leu His Asn Ile Gly Pro
            820                 825                 830

Ser Thr Ile Ser Asp Thr Ile Leu Glu Val Gly Trp Pro Phe Ser Ala
        835                 840                 845

Arg Asp Glu Phe Leu Leu Tyr Ile Phe His Ile Gln Thr Leu Gly Pro
    850                 855                 860

Leu Gln Cys Gln Pro Asn Pro Asn Ile Asn Pro Gln Asp Ile Lys Pro
865                 870                 875                 880

Ala Ala Ser Pro Glu Asp Thr Pro Glu Leu Ser Ala Phe Leu Arg Asn
                885                 890                 895

Ser Thr Ile Pro His Leu Val Arg Lys Arg Asp Val His Val Val Glu
            900                 905                 910

Phe His Arg Gln Ser Pro Ala Lys Ile Leu Asn Cys Thr Asn Ile Glu
        915                 920                 925

Cys Leu Gln Ile Ser Cys Ala Val Gly Arg Leu Glu Gly Gly Glu Ser
    930                 935                 940

Ala Val Leu Lys Val Arg Ser Arg Leu Trp Ala His Thr Phe Leu Gln

945                 950                 955                 960

Arg Lys Asn Asp Pro Tyr Ala Leu Ala Ser Leu Val Ser Phe Glu Val
                965                 970                 975

Lys Lys Met Pro Tyr Thr Asp Gln Pro Ala Lys Leu Pro Glu Gly Ser
            980                 985                 990

Ile Ala Ile Lys Thr Ser Val Ile Trp Ala Thr Pro Asn Val Ser Phe
        995                 1000                1005

Ser Ile Pro Leu Trp Val Ile Ile Leu Ala Ile Leu Leu Gly Leu Leu
    1010                1015                1020

Val Leu Ala Ile Leu Thr Leu Ala Leu Trp Lys Cys Gly Phe Phe Asp
1025                1030                1035                1040

Arg Ala Arg Pro Pro Gln Glu Asp Met Thr Asp Arg Glu Gln Leu Thr
                1045                1050                1055

Asn Asp Lys Thr Pro Glu Ala
            1060

<210>67
<211>1524
<212>DNA
<213>Homo sapiens

<220>
<221> CDS
<222>(10)..(1522)

<400>67
tttgccatc atg ttg cgg ttg gtg gca gct tgc cct gag tca tgt gtg      48
gtg tgc acc aaa gat gta acc ctc tgt cac cag cta acc tat ata gta    96
gca gcc cct atg acc acg agg gtt tta atc atc acc gat gga tat ctc    144
tcc tct att gag agc aca aac ctg tct ctc ttg ttt aat ctt gcc ctg    192
ctc tcc cta agc aga aat ggt atc gag gat gtt cag gaa gat gcc ctg    240
cat ggg ctt acg atg ttg cgg acc ttg ttg ctg gag cac aac caa ata    288
tcc agc tct tcg ctc act gat cac acc ttc agc aag ctt cac agc ctg 336
cag gta ctg gtg ctg agc aat aat gct ctc cgc acc cta cga ggg tct 384
tgg ttc cga aac aca agc ggc ctg acc cgg ctc cag ctg gat ggg aat 432
cag att act aat ctc aca gac agt tct ttc gga ggc acg aat ctc cac 480
agt ctc agg tat ctg gat tta tcc aac aat ttt att tcc tac att ggg 528
aaa gat gcc ttc cgg ccc ctg cct caa cta cag gaa gtg gac ctt tcc 576
cga aat agg tta gcc cac atg ccg gat gtg ttt act cca ctg aag cag 624
tta atc ctt ctg agc tta gat aag aac cag tgg agc tgc act tgt gat 672
ctc cat ccc ctt gct cgg ttt tta aga aac tac att aag tct tct gct 720
cac acg ctc agg aat gcc aag gac cta aat tgc cag cca tct acc gca 768
gct gtg gca gct gca cag agt gtg ctg agg ctg tct gag acc aac tgt 816
gat tcc aaa gct ccc aac ttc act ctg gtt cta aag gac aga agt ccc 864
ctc ctc cca gga cca gat gtg gcc ctg ctg act gtc ctt ggc ttc gca 912
ggt gct gtt ggt ctc act tgc cta ggt tta gtt gta ttt aac tgg aaa 960
ctc cac caa ggc aaa gca aat gaa cac aca tca gaa aac ctt tgt tgc 1008
aga acc ttc gat gaa ccc ctg tgt gct cat gag gca aga aat tac cac 1056
act aag gga tac tgc aac tgc cac tta act cag gaa aac gag ata aag 1104
gtc atg tcc att gtg ggg tcc aga aaa gaa atg cca ctt tta cag gaa 1152
aat agc cat caa gca aca tcg gcc tct gag tct gca acc ctt gac aga 1200
tca ttt aga aac ctg aaa aag aaa gac cgt ggg gta ggc agc act tta 1248
ttt tgc cag gat ggt aga ttg ctg cat tcg gaa tgt tca gag cct cct 1296
gga aat atg aga gct ttt aat gaa gca ggc tta ctt aca aca tat aat 1344
cca agg aaa gtt caa aag cta tgg aat ctt gag cct gga gaa gtc cag 1392
cct caa act ctg caa cac cat ata ata aga aca gaa gat atc agc agt 1440
gac ata ttt aga aga aga tat gca aca ccc gct tca gcc ttg gca gga 1488
gaa agt ctt gag aag cgt tta aca aat gaa tca tga 1524

<210>68
<211>504
<212>PRT
<213>Homo sapiens

<400>68

<210>69
<211>3757
<212>DNA
<213>Homo sapiens

<220>
<221> CDS
<222>(13)..(3754)

<400>69

ctctttgcca tc atg ttg cgg ttg gtg gca gct tgc cct gag tca tgt      48
              Met Leu Arg Leu Val Ala Ala Cys Pro Glu Ser Cys
              1               5                   10

gtg gtg tgc acc aaa gat gta acc ctc tgt cac cag cta acc tat ata      96
Val Val Cys Thr Lys Asp Val Thr Leu Cys His Gln Leu Thr Tyr Ile
        15                  20                  25

gta gca gcc cct atg acc acg agg gtt tta atc atc acc gat gga tat     144

Val Ala Ala Pro Met Thr Thr Arg Val Leu Ile Ile Thr Asp Gly Tyr
    30                  35                  40

ctc tcc tct att gag agc aca aac ctg tct ctc ttg ttt aat ctt gcc 192
Leu Ser Ser Ile Glu Ser Thr Asn Leu Ser Leu Leu Phe Asn Leu Ala
45                  50                  55                  60

ctg ctc tcc cta agc aga aat ggt atc gag gat gtt cag gaa gat gcc 240
Leu Leu Ser Leu Ser Arg Asn Gly Ile Glu Asp Val Gln Glu Asp Ala
                65                  70                  75

ctg cat ggg ctt acg atg ttg cgg acc ttg ttg ctg gag cac aac caa 288
Leu His Gly Leu Thr Met Leu Arg Thr Leu Leu Leu Glu His Asn Gln
            80                  85                  90

ata tcc agc tct tcg ctc act gat cac acc ttc agc aag ctt cac agc 336
Ile Ser Ser Ser Ser Leu Thr Asp His Thr Phe Ser Lys Leu His Ser
        95                  100                 105

ctg cag gta ctg gtg ctg agc aat aat gct ctc cgc acc cta cga ggg 384
Leu Gln Val Leu Val Leu Ser Asn Asn Ala Leu Arg Thr Leu Arg Gly
    110                 115                 120

tct tgg ttc cga aac aca agc ggc ctg acc cgg ctc cag ctg gat ggg 432
Ser Trp Phe Arg Asn Thr Ser Gly Leu Thr Arg Leu Gln Leu Asp Gly
125                 130                 135                 140

aat cag att act aat ctc aca gac agt tct ttc gga ggc acg aat ctc 480
Asn Gln Ile Thr Asn Leu Thr Asp Ser Ser Phe Gly Gly Thr Asn Leu
                145                 150                 155

cac agt ctc agg tat ctg gat tta tcc aac aat ttt att tcc tac att 528
His Ser Leu Arg Tyr Leu Asp Leu Ser Asn Asn Phe Ile Ser Tyr Ile
            160                 165                 170

ggg aaa gat gcc ttc cgg ccc ctg cct caa cta cag gaa gtg gac ctt 576
Gly Lys Asp Ala Phe Arg Pro Leu Pro Gln Leu Gln Glu Val Asp Leu
        175                 180                 185

tcc cga aat agg tta gcc cac atg ccg gat gtg ttt act cca ctg aag 624
Ser Arg Asn Arg Leu Ala His Met Pro Asp Val Phe Thr Pro Leu Lys
    190                 195                 200

cag tta atc ctt ctg agc tta gat aag aac cag tgg agc tgc act tgt 672
Gln Leu Ile Leu Leu Ser Leu Asp Lys Asn Gln Trp Ser Cys Thr Cys
205                 210                 215                 220

gat ctc cat ccc ctt gct cgg ttt tta aga aac tac att aag tct tct 720
Asp Leu His Pro Leu Ala Arg Phe Leu Arg Asn Tyr Ile Lys Ser Ser
                225                 230                 235

gct cac acg ctc agg aat gcc aag gac cta aat tgc cag cca tct acc 768
Ala His Thr Leu Arg Asn Ala Lys Asp Leu Asn Cys Gln Pro Ser Thr
            240                 245                 250

gca gct gtg gca gct gca cag agt gtg ctg agg ctg tct gag acc aac 816
Ala Ala Val Ala Ala Ala Gln Ser Val Leu Arg Leu Ser Glu Thr Asn

        255                 260                 265

tgt gat tcc aaa gct ccc aac ttc act ctg gtt cta aag gac aga agt 864
Cys Asp Ser Lys Ala Pro Asn Phe Thr Leu Val Leu Lys Asp Arg Ser
    270                 275                 280

ccc ctc ctc cca gga cca gat gtg gcc ctg ctg act gtc ctt ggc ttc 912
Pro Leu Leu Pro Gly Pro Asp Val Ala Leu Leu Thr Val Leu Gly Phe
285                 290                 295                 300

gca ggt gct gtt ggt ctc act tgc cta ggt tta gtt gta ttt aac tgg 960
Ala Gly Ala Val Gly Leu Thr Cys Leu Gly Leu Val Val Phe Asn Trp
                305                 310                 315

aaa ctc cac caa ggc aaa gca aat gaa cac aca tca gaa aac ctt tgt 1008
Lys Leu His Gln Gly Lys Ala Asn Glu His Thr Ser Glu Asn Leu Cys
            320                 325                 330

tgc aga acc ttc gat gaa ccc ctg tgt gct cat gag gca aga aat tac 1056
Cys Arg Thr Phe Asp Glu Pro Leu Cys Ala His Glu Ala Arg Asn Tyr
        335                 340                 345

cac act aag gga tac tgc aac tgc cac tta act cag gaa aac gag ata 1104
His Thr Lys Gly Tyr Cys Asn Cys His Leu Thr Gln Glu Asn Glu Ile
    350                 355                 360

aag gtc atg tcc att gtg ggg tcc aga aaa gaa atg cca ctt tta cag 1152
Lys Val Met Ser Ile Val Gly Ser Arg Lys Glu Met Pro Leu Leu Gln
365                 370                 375                 380

gaa aat agc cat caa gca aca tcg gcc tct gag tct gca acc ctt gac 1200
Glu Asn Ser His Gln Ala Thr Ser Ala Ser Glu Ser Ala Thr Leu Asp
                385                 390                 395

aga tca ttt aga aac ctg aaa aag aaa gac cgt ggg gta ggc agc act 1248
Arg Ser Phe Arg Asn Leu Lys Lys Lys Asp Arg Gly Val Gly Ser Thr
            400                 405                 410

tta ttt tgc cag gat ggt aga ttg ctg cat tcg gaa tgt tca gag cct 1296
Leu Phe Cys Gln Asp Gly Arg Leu Leu His Ser Glu Cys Ser Glu Pro
        415                 420                 425

cct gga aat atg aga gct ttt aat gaa gca ggc tta ctt aca aca tat 1344
Pro Gly Asn Met Arg Ala Phe Asn Glu Ala Gly Leu Leu Thr Thr Tyr
    430                 435                 440

aat cca agg aaa gtt caa aag cta tgg aat ctt gag cct gga gaa gtc 1392
Asn Pro Arg Lys Val Gln Lys Leu Trp Asn Leu Glu Pro Gly Glu Val
445                 450                 455                 460

cag cct caa act ctg caa cac cat ata ata aga aca gaa gat atc agc 1440
Gln Pro Gln Thr Leu Gln His His Ile Ile Arg Thr Glu Asp Ile Ser
                465                 470                 475

agt gac ata ttt aga aga aga tat gca aca ccc gct tca gcc ttg gca 1488
Ser Asp Ile Phe Arg Arg Arg Tyr Ala Thr Pro Ala Ser Ala Leu Ala
            480                 485                 490

gga gaa agt ctt gag aag cgt tta aca aat gaa tca tgg cag cct cca 1536
Gly Glu Ser Leu Glu Lys Arg Leu Thr Asn Glu Ser Trp Gln Pro Pro
        495                 500                 505

ata gaa aaa gaa gac aat ggc tta cac cct cac agg caa aga cat ttt 1584
Ile Glu Lys Glu Asp Asn Gly Leu His Pro His Arg Gln Arg His Phe
    510                 515                 520

att aca agc tca tca tcc aag cct tgt gag cct gag gaa cac tat gta 1632
Ile Thr Ser Ser Ser Ser Lys Pro Cys Glu Pro Glu Glu His Tyr Val
525                 530                 535                 540

caa aat atc gta caa aaa aat aga tca aaa tat gat gat cct tgt gga 1680
Gln Asn Ile Val Gln Lys Asn Arg Ser Lys Tyr Asp Asp Pro Cys Gly
                545                 550                 555

ctg tta aaa cag agc aaa cct agg tat ttt cag cca aac aat tct ctt 1728
Leu Leu Lys Gln Ser Lys Pro Arg Tyr Phe Gln Pro Asn Asn Ser Leu
            560                 565                 570

atc tgt aaa tat gtg ccc tgt gag caa ttt gaa gat tac atg aaa gaa 1776
Ile Cys Lys Tyr Val Pro Cys Glu Gln Phe Glu Asp Tyr Met Lys Glu
        575                 580                 585

aag aag cca aat cgt aga caa cac tca aag cct gag aaa gag caa atc 1824
Lys Lys Pro Asn Arg Arg Gln His Ser Lys Pro Glu Lys Glu Gln Ile
    590                 595                 600

caa att aac agt gca ata gaa aaa ttt ctt atg agt gag gac aac ata 1872
Gln Ile Asn Ser Ala Ile Glu Lys Phe Leu Met Ser Glu Asp Asn Ile
605                 610                 615                 620

gat tta tca gga tta tca aca aaa acc aag aaa gca tat tcc cca aag 1920
Asp Leu Ser Gly Leu Ser Thr Lys Thr Lys Lys Ala Tyr Ser Pro Lys
                625                 630                 635

agg gtt atc ttc cat gat cct gat tta gta gaa ata aat agg tcg atg 1968
Arg Val Ile Phe His Asp Pro Asp Leu Val Glu Ile Asn Arg Ser Met
            640                 645                 650

atg tca ccc aaa ata tca acc cct tgg aaa cga cag aaa aat caa agt 2016
Met Ser Pro Lys Ile Ser Thr Pro Trp Lys Arg Gln Lys Asn Gln Ser
        655                 660                 665

aac caa ctg act aag ttg gat gtt aaa aaa ttt agc aac act ggg gag 2064
Asn Gln Leu Thr Lys Leu Asp Val Lys Lys Phe Ser Asn Thr Gly Glu
    670                 675                 680

aga aac aaa gga gaa aaa tgg ttt act aat tca tgg gtt ctg aaa agg 2112
Arg Asn Lys Gly Glu Lys Trp Phe Thr Asn Ser Trp Val Leu Lys Arg
685                 690                 695                 700

aag aga acc cct cag tct gac ctc aaa ggg aaa att aaa gga caa aac 2160
Lys Arg Thr Pro Gln Ser Asp Leu Lys Gly Lys Ile Lys Gly Gln Asn
                705                 710                 715

tta aaa tta aat tta cat cct ttt aga aaa gtc aga gtc cat cca gaa    2208
Leu Lys Leu Asn Leu His Pro Phe Arg Lys Val Arg Val His Pro Glu
        720                 725                 730

aaa tcc ttg tca agt ctc cca aag caa tgc aag cag gta ttg ttg cct    2256
Lys Ser Leu Ser Ser Leu Pro Lys Gln Cys Lys Gln Val Leu Leu Pro
        735                 740                 745

cct aag aaa tta tcc aaa act tct gag aca gaa gcc aaa ata aat act    2304
Pro Lys Lys Leu Ser Lys Thr Ser Glu Thr Glu Ala Lys Ile Asn Thr
    750                 755                 760

gtg tgt tct gca gat ttt ctt caa cag tca gag agt agc aac tat gtt    2352
Val Cys Ser Ala Asp Phe Leu Gln Gln Ser Glu Ser Ser Asn Tyr Val
765                 770                 775                 780

aga ctc act tca aag agg ctg cct ctg aaa cat gac tca aag cag acc    2400
Arg Leu Thr Ser Lys Arg Leu Pro Leu Lys His Asp Ser Lys Gln Thr
                785                 790                 795

cca tat tat caa cga aac act aaa cgt gcc ccc ctg ctc agt gct aac    2448
Pro Tyr Tyr Gln Arg Asn Thr Lys Arg Ala Pro Leu Leu Ser Ala Asn
            800                 805                 810

aac ttg cgt gta gtc aac cag agc tct ata gaa agc agc tgt tac tca    2496
Asn Leu Arg Val Val Asn Gln Ser Ser Ile Glu Ser Ser Cys Tyr Ser
        815                 820                 825

gct ggc cac att cct gat gga aac aca tca aaa ttg ccc caa cct aca    2544
Ala Gly His Ile Pro Asp Gly Asn Thr Ser Lys Leu Pro Gln Pro Thr
    830                 835                 840

ccc act gat gct gag cac agg cac tca cat tct caa ttc tca act gag    2592
Pro Thr Asp Ala Glu His Arg His Ser His Ser Gln Phe Ser Thr Glu
845                 850                 855                 860

caa atg gaa gat gca act cag ctt gaa tca aaa gtg ctt agt tat tta    2640
Gln Met Glu Asp Ala Thr Gln Leu Glu Ser Lys Val Leu Ser Tyr Leu
                865                 870                 875

gca act act tgg gaa aat aca gga agt gat gtt tta cca ttc caa cat    2688
Ala Thr Thr Trp Glu Asn Thr Gly Ser Asp Val Leu Pro Phe Gln His
            880                 885                 890

tcc agg agg gct act gac caa ggg aca acg gag tcc act gag cac atg    2736
Ser Arg Arg Ala Thr Asp Gln Gly Thr Thr Glu Ser Thr Glu His Met
        895                 900                 905

gga cag aat gta tca aag acc agt gag tta aat cag ttt tct ttg tcc    2784
Gly Gln Asn Val Ser Lys Thr Ser Glu Leu Asn Gln Phe Ser Leu Ser
    910                 915                 920

ccg agg aat caa aca caa ctt tta gat gct cac aag act gac agc tac    2832
Pro Arg Asn Gln Thr Gln Leu Leu Asp Ala His Lys Thr Asp Ser Tyr
925                 930                 935                 940

aac aag gaa tac act tta gac caa aat gaa ggc tta caa cac aga gag    2880

Asn Lys Glu Tyr Thr Leu Asp Gln Asn Glu Gly Leu Gln His Arg Glu
                945                 950                 955

caa aat tca agt cat gca cag ctt gaa aat aaa gaa aaa aca tta atg 2928
Gln Asn Ser Ser His Ala Gln Leu Glu Asn Lys Glu Lys Thr Leu Met
            960                 965                 970

aca aaa ccc caa ata cca cat caa att gtg gaa aat tgt att atg gat 2976
Thr Lys Pro Gln Ile Pro His Gln Ile Val Glu Asn Cys Ile Met Asp
        975                 980                 985

aag gaa gaa aat gat gta gaa aaa aaa ctt tca aaa aca gaa act tat 3024
Lys Glu Glu Asn Asp Val Glu Lys Lys Leu Ser Lys Thr Glu Thr Tyr
    990                 995                 1000

gat tcc tct ctc att ccc caa aca caa tcc aag aac aac cta tca ttt 3072
Asp Ser Ser Leu Ile Pro Gln Thr Gln Ser Lys Asn Asn Leu Ser Phe
1005                1010                1015                1020

atg aag aca aat tca att cca tac caa aat aga ata gaa ctt ccc aag 3120
Met Lys Thr Asn Ser Ile Pro Tyr Gln Asn Arg Ile Glu Leu Pro Lys
                1025                1030                1035

gat atc agt act tct cct gtt agt agt caa gcc gtt tgg cac cta acc 3168
Asp Ile Ser Thr Ser Pro Val Ser Ser Gln Ala Val Trp His Leu Thr
            1040                1045                1050

aat agt agc gaa aaa gga att gac agc aca aat gca ttg ccc aga aat 3216
Asn Ser Ser Glu Lys Gly Ile Asp Ser Thr Asn Ala Leu Pro Arg Asn
        1055                1060                1065

gac ggc act gaa gca cta gag ata aaa ata gta ggg aaa gaa gag aaa 3264
Asp Gly Thr Glu Ala Leu Glu Ile Lys Ile Val Gly Lys Glu Glu Lys
    1070                1075                1080

aat atg ctt gat gaa agc aag aca gat tct agt atg tta act cag atc 3312
Asn Met Leu Asp Glu Ser Lys Thr Asp Ser Ser Met Leu Thr Gln Ile
1085                1090                1095                1100

tca caa atg acc tta aaa ggc atc aca aaa gaa agg cag caa act tgg 3360
Ser Gln Met Thr Leu Lys Gly Ile Thr Lys Glu Arg Gln Gln Thr Trp
                1105                1110                1115

gaa aat gga aca agt gaa aaa tat ata tta cat gat gca agc tct gcc 3408
Glu Asn Gly Thr Ser Glu Lys Tyr Ile Leu His Asp Ala Ser Ser Ala
            1120                1125                1130

gag gag acc att aca gct aaa gat tta agt atc aca agt tcc cat gaa 3456
Glu Glu Thr Ile Thr Ala Lys Asp Leu Ser Ile Thr Ser Ser His Glu
        1135                1140                1145

acc caa aat aga ata ctt tgc agt gaa gta gat cct gaa gtt aac agt 3504
Thr Gln Asn Arg Ile Leu Cys Ser Glu Val Asp Pro Glu Val Asn Ser
    1150                1155                1160

aat gta cat aat ttt aga gaa gtt caa aat att caa cca gat aaa gat 3552
Asn Val His Asn Phe Arg Glu Val Gln Asn Ile Gln Pro Asp Lys Asp

1165                1170                1175                1180

agg gca cat aaa gaa ggc gca atg aca gtg gag aca cat gaa gcg ctt    3600
Arg Ala His Lys Glu Gly Ala Met Thr Val Glu Thr His Glu Ala Leu
                1185                1190                1195

tcc ttc tta cca ggg tta aaa gac agt ttt gag gca gaa aat gag gtg    3648
Ser Phe Leu Pro Gly Leu Lys Asp Ser Phe Glu Ala Glu Asn Glu Val
            1200                1205                1210

ttt tta gtt cct agc aga ata aat gaa gct gaa aac tct gct cca aaa    3696
Phe Leu Val Pro Ser Arg Ile Asn Glu Ala Glu Asn Ser Ala Pro Lys
        1215                1220                1225

cct gta ctg tat cca cca tct gct gaa tat gct act aca tca cct tta    3744
Pro Val Leu Tyr Pro Pro Ser Ala Glu Tyr Ala Thr Thr Ser Pro Leu
    1230                1235                1240

gaa aca gaa taaa                                                   3757
Glu Thr Glu
1245

<210>70
<211>1247
<212>PRT
<213>Homo sapiens

<400>70

Met Leu Arg Leu Val Ala Ala Cys Pro Glu Ser Cys Val Val Cys Thr
 1               5                  10                  15

Lys Asp Val Thr Leu Cys His Gln Leu Thr Tyr Ile Val Ala Ala Pro
            20                  25                  30

Met Thr Thr Arg Val Leu Ile Ile Thr Asp Gly Tyr Leu Ser Ser Ile
        35                  40                  45

Glu Ser Thr Asn Leu Ser Leu Leu Phe Asn Leu Ala Leu Leu Ser Leu
    50                  55                  60

Ser Arg Asn Gly Ile Glu Asp Val Gln Glu Asp Ala Leu His Gly Leu
65                  70                  75                  80

Thr Met Leu Arg Thr Leu Leu Leu Glu His Asn Gln Ile Ser Ser Ser
                85                  90                  95

Ser Leu Thr Asp His Thr Phe Ser Lys Leu His Ser Leu Gln Val Leu
            100                 105                 110

Val Leu Ser Asn Asn Ala Leu Arg Thr Leu Arg Gly Ser Trp Phe Arg
        115                 120                 125

Asn Thr Ser Gly Leu Thr Arg Leu Gln Leu Asp Gly Asn Gln Ile Thr
    130                 135                 140

Asn Leu Thr Asp Ser Ser Phe Gly Gly Thr Asn Leu His Ser Leu Arg

145                 150                 155                 160

Tyr Leu Asp Leu Ser Asn Asn Phe Ile Ser Tyr Ile Gly Lys Asp Ala
                165                 170                 175

Phe Arg Pro Leu Pro Gln Leu Gln Glu Val Asp Leu Ser Arg Asn Arg
            180                 185                 190

Leu Ala His Met Pro Asp Val Phe Thr Pro Leu Lys Gln Leu Ile Leu
        195                 200                 205

Leu Ser Leu Asp Lys Asn Gln Trp Ser Cys Thr Cys Asp Leu His Pro
    210                 215                 220

Leu Ala Arg Phe Leu Arg Asn Tyr Ile Lys Ser Ser Ala His Thr Leu
225                 230                 235                 240

Arg Asn Ala Lys Asp Leu Asn Cys Gln Pro Ser Thr Ala Ala Val Ala
                245                 250                 255

Ala Ala Gln Ser Val Leu Arg Leu Ser Glu Thr Asn Cys Asp Ser Lys
            260                 265                 270

Ala Pro Asn Phe Thr Leu Val Leu Lys Asp Arg Ser Pro Leu Leu Pro
        275                 280                 285

Gly Pro Asp Val Ala Leu Leu Thr Val Leu Gly Phe Ala Gly Ala Val
    290                 295                 300

Gly Leu Thr Cys Leu Gly Leu Val Val Phe Asn Trp Lys Leu His Gln
305                 310                 315                 320

Gly Lys Ala Asn Glu His Thr Ser Glu Asn Leu Cys Cys Arg Thr Phe
                325                 330                 335

Asp Glu Pro Leu Cys Ala His Glu Ala Arg Asn Tyr His Thr Lys Gly
            340                 345                 350

Tyr Cys Asn Cys His Leu Thr Gln Glu Asn Glu Ile Lys Val Met Ser
        355                 360                 365

Ile Val Gly Ser Arg Lys Glu Met Pro Leu Leu Gln Glu Asn Ser His
    370                 375                 380

Gln Ala Thr Ser Ala Ser Glu Ser Ala Thr Leu Asp Arg Ser Phe Arg
385                 390                 395                 400

Asn Leu Lys Lys Lys Asp Arg Gly Val Gly Ser Thr Leu Phe Cys Gln
                405                 410                 415

Asp Gly Arg Leu Leu His Ser Glu Cys Ser Glu Pro Pro Gly Asn Met
            420                 425                 430

Arg Ala Phe Asn Glu Ala Gly Leu Leu Thr Thr Tyr Asn Pro Arg Lys
        435                 440                 445

Val Gln Lys Leu Trp Asn Leu Glu Pro Gly Glu Val Gln Pro Gln Thr

450                 455                 460

Leu Gln His His Ile Ile Arg Thr Glu Asp Ile Ser Ser Asp Ile Phe
465                 470                 475                 480

Arg Arg Arg Tyr Ala Thr Pro Ala Ser Ala Leu Ala Gly Glu Ser Leu
                485                 490                 495

Glu Lys Arg Leu Thr Asn Glu Ser Trp Gln Pro Pro Ile Glu Lys Glu
            500                 505                 510

Asp Asn Gly Leu His Pro His Arg Gln Arg His Phe Ile Thr Ser Ser
        515                 520                 525

Ser Ser Lys Pro Cys Glu Pro Glu Glu His Tyr Val Gln Asn Ile Val
    530                 535                 540

Gln Lys Asn Arg Ser Lys Tyr Asp Asp Pro Cys Gly Leu Leu Lys Gln
545                 550                 555                 560

Ser Lys Pro Arg Tyr Phe Gln Pro Asn Asn Ser Leu Ile Cys Lys Tyr
                565                 570                 575

Val Pro Cys Glu Gln Phe Glu Asp Tyr Met Lys Glu Lys Lys Pro Asn
            580                 585                 590

Arg Arg Gln His Ser Lys Pro Glu Lys Glu Gln Ile Gln Ile Asn Ser
        595                 600                 605

Ala Ile Glu Lys Phe Leu Met Ser Glu Asp Asn Ile Asp Leu Ser Gly
    610                 615                 620

Leu Ser Thr Lys Thr Lys Lys Ala Tyr Ser Pro Lys Arg Val Ile Phe
625                 630                 635                 640

His Asp Pro Asp Leu Val Glu Ile Asn Arg Ser Met Met Ser Pro Lys
                645                 650                 655

Ile Ser Thr Pro Trp Lys Arg Gln Lys Asn Gln Ser Asn Gln Leu Thr
            660                 665                 670

Lys Leu Asp Val Lys Lys Phe Ser Asn Thr Gly Glu Arg Asn Lys Gly
        675                 680                 685

Glu Lys Trp Phe Thr Asn Ser Trp Val Leu Lys Arg Lys Arg Thr Pro
    690                 695                 700

Gln Ser Asp Leu Lys Gly Lys Ile Lys Gly Gln Asn Leu Lys Leu Asn
705                 710                 715                 720

Leu His Pro Phe Arg Lys Val Arg Val His Pro Glu Lys Ser Leu Ser
                725                 730                 735

Ser Leu Pro Lys Gln Cys Lys Gln Val Leu Leu Pro Pro Lys Lys Leu
            740                 745                 750

Ser Lys Thr Ser Glu Thr Glu Ala Lys Ile Asn Thr Val Cys Ser Ala

755                 760                 765

Asp Phe Leu Gln Gln Ser Glu Ser Ser Asn Tyr Val Arg Leu Thr Ser
    770                 775                 780

Lys Arg Leu Pro Leu Lys His Asp Ser Lys Gln Thr Pro Tyr Tyr Gln
785                 790                 795                 800

Arg Asn Thr Lys Arg Ala Pro Leu Leu Ser Ala Asn Asn Leu Arg Val
                805                 810                 815

Val Asn Gln Ser Ser Ile Glu Ser Ser Cys Tyr Ser Ala Gly His Ile
            820                 825                 830

Pro Asp Gly Asn Thr Ser Lys Leu Pro Gln Pro Thr Pro Thr Asp Ala
        835                 840                 845

Glu His Arg His Ser His Ser Gln Phe Ser Thr Glu Gln Met Glu Asp
    850                 855                 860

Ala Thr Gln Leu Glu Ser Lys Val Leu Ser Tyr Leu Ala Thr Thr Trp
865                 870                 875                 880

Glu Asn Thr Gly Ser Asp Val Leu Pro Phe Gln His Ser Arg Arg Ala
                885                 890                 895

Thr Asp Gln Gly Thr Thr Glu Ser Thr Glu His Met Gly Gln Asn Val
            900                 905                 910

Ser Lys Thr Ser Glu Leu Asn Gln Phe Ser Leu Ser Pro Arg Asn Gln
        915                 920                 925

Thr Gln Leu Leu Asp Ala His Lys Thr Asp Ser Tyr Asn Lys Glu Tyr
    930                 935                 940

Thr Leu Asp Gln Asn Glu Gly Leu Gln His Arg Glu Gln Asn Ser Ser
945                 950                 955                 960

His Ala Gln Leu Glu Asn Lys Glu Lys Thr Leu Met Thr Lys Pro Gln
                965                 970                 975

Ile Pro His Gln Ile Val Glu Asn Cys Ile Met Asp Lys Glu Glu Asn
            980                 985                 990

Asp Val Glu Lys Lys Leu Ser Lys Thr Glu Thr Tyr Asp Ser Ser Leu
        995                 1000                1005

Ile Pro Gln Thr Gln Ser Lys Asn Asn Leu Ser Phe Met Lys Thr Asn
    1010                1015                1020

Ser Ile Pro Tyr Gln Asn Arg Ile Glu Leu Pro Lys Asp Ile Ser Thr
1025                1030                1035                1040

Ser Pro Val Ser Ser Gln Ala Val Trp His Leu Thr Asn Ser Ser Glu
                1045                1050                1055

Lys Gly Ile Asp Ser Thr Asn Ala Leu Pro Arg Asn Asp Gly Thr Glu

1060                    1065                    1070

Ala Leu Glu Ile Lys Ile Val Gly Lys Glu Glu Lys Asn Met Leu Asp
        1075                    1080                    1085

Glu Ser Lys Thr Asp Ser Ser Met Leu Thr Gln Ile Ser Gln Met Thr
    1090                    1095                    1100

Leu Lys Gly Ile Thr Lys Glu Arg Gln Gln Thr Trp Glu Asn Gly Thr
1105                    1110                    1115                    1120

Ser Glu Lys Tyr Ile Leu His Asp Ala Ser Ser Ala Glu Glu Thr Ile
                1125                    1130                    1135

Thr Ala Lys Asp Leu Ser Ile Thr Ser Ser His Glu Thr Gln Asn Arg
            1140                    1145                    1150

Ile Leu Cys Ser Glu Val Asp Pro Glu Val Asn Ser Asn Val His Asn
        1155                    1160                    1165

Phe Arg Glu Val Gln Asn Ile Gln Pro Asp Lys Asp Arg Ala His Lys
    1170                    1175                    1180

Glu Gly Ala Met Thr Val Glu Thr His Glu Ala Leu Ser Phe Leu Pro
1185                    1190                    1195                    1200

Gly Leu Lys Asp Ser Phe Glu Ala Glu Asn Glu Val Phe Leu Val Pro
                1205                    1210                    1215

Ser Arg Ile Asn Glu Ala Glu Asn Ser Ala Pro Lys Pro Val Leu Tyr
            1220                    1225                    1230

Pro Pro Ser Ala Glu Tyr Ala Thr Thr Ser Pro Leu Glu Thr Glu
        1235                    1240                    1245

<210>71
<211>645
<212>DNA
<213>Homo sapiens

<220>
<221> CDS
<222>(1)..(645)

<400>71

gga tcc gcc ctg ctc tcc cta agc aga aat ggt atc gag gat gtt cag      48
Gly Ser Ala Leu Leu Ser Leu Ser Arg Asn Gly Ile Glu Asp Val Gln
1               5                   10                  15

gaa gat gcc ctg cat ggg ctt acg atg ttg cgg acc ttg ttg ctg gag      96
Glu Asp Ala Leu His Gly Leu Thr Met Leu Arg Thr Leu Leu Leu Glu
            20                  25                  30

cac aac caa ata tcc agc tct tcg ctc act gat cac acc ttc agc aag      144
His Asn Gln Ile Ser Ser Ser Ser Leu Thr Asp His Thr Phe Ser Lys
        35                  40                  45

ctt cac agc ctg cag gta ctg gtg ctg agc aat aat gct ctc cgc acc 192
Leu His Ser Leu Gln Val Leu Val Leu Ser Asn Asn Ala Leu Arg Thr
    50                  55                  60

cta cga ggg tct tgg ttc cga aac aca agc ggc ctg acc cgg ctc cag 240
Leu Arg Gly Ser Trp Phe Arg Asn Thr Ser Gly Leu Thr Arg Leu Gln
65                  70                  75                  80

ctg gat ggg aat cag att act aat ctc aca gac agt tct ttc gga ggc 288
Leu Asp Gly Asn Gln Ile Thr Asn Leu Thr Asp Ser Ser Phe Gly Gly
                85                  90                  95

acg aat ctc cac agt ctc agg tat ctg gat tta tcc aac aat ttt att 336
Thr Asn Leu His Ser Leu Arg Tyr Leu Asp Leu Ser Asn Asn Phe Ile
            100                 105                 110

tcc tac att ggg aaa gat gcc ttc cgg ccc ctg cct caa cta cag gaa 384
Ser Tyr Ile Gly Lys Asp Ala Phe Arg Pro Leu Pro Gln Leu Gln Glu
        115                 120                 125

gtg gac ctt tcc cga aat agg tta gcc cac atg ccg gat gtg ttt act 432
Val Asp Leu Ser Arg Asn Arg Leu Ala His Met Pro Asp Val Phe Thr
    130                 135                 140

cca ctg aag cag tta atc ctt ctg agc tta gat aag aac cag tgg agc 480
Pro Leu Lys Gln Leu Ile Leu Leu Ser Leu Asp Lys Asn Gln Trp Ser
145                 150                 155                 160

tgc act tgt gat ctc cat ccc ctt gct cgg ttt tta aga aac tac att 528
Cys Thr Cys Asp Leu His Pro Leu Ala Arg Phe Leu Arg Asn Tyr Ile
                165                 170                 175

aag tct tct gct cac acg ctc agg aat gcc aag gac cta aat tgc cag 576
Lys Ser Ser Ala His Thr Leu Arg Asn Ala Lys Asp Leu Asn Cys Gln
            180                 185                 190

cca tct acc gca gct gtg gca gct gca cag agt gtg ctg agg ctg tct 624
Pro Ser Thr Ala Ala Val Ala Ala Ala Gln Ser Val Leu Arg Leu Ser
        195                 200                 205

gag acc aac tgt gat ctc gag 645
Glu Thr Asn Cys Asp Leu Glu
    210                 215

<210>72
<211>215
<212>PRT
<213>Homo sapiens

<400>72

Gly Ser Ala Leu Leu Ser Leu Ser Arg Asn Gly Ile Glu Asp Val Gln
1               5                   10                  15

Glu Asp Ala Leu His Gly Leu Thr Met Leu Arg Thr Leu Leu Leu Glu
            20                  25                  30

His Asn Gln Ile Ser Ser Ser Ser Leu Thr Asp His Thr Phe Ser Lys
        35                  40                  45

Leu His Ser Leu Gln Val Leu Val Leu Ser Asn Asn Ala Leu Arg Thr
    50                  55                  60

Leu Arg Gly Ser Trp Phe Arg Asn Thr Ser Gly Leu Thr Arg Leu Gln
65                  70                  75                  80

Leu Asp Gly Asn Gln Ile Thr Asn Leu Thr Asp Ser Ser Phe Gly Gly
                85                  90                  95

Thr Asn Leu His Ser Leu Arg Tyr Leu Asp Leu Ser Asn Asn Phe Ile
            100                 105                 110

Ser Tyr Ile Gly Lys Asp Ala Phe Arg Pro Leu Pro Gln Leu Gln Glu
        115                 120                 125

Val Asp Leu Ser Arg Asn Arg Leu Ala His Met Pro Asp Val Phe Thr
    130                 135                 140

Pro Leu Lys Gln Leu Ile Leu Leu Ser Leu Asp Lys Asn Gln Trp Ser
145                 150                 155                 160

Cys Thr Cys Asp Leu His Pro Leu Ala Arg Phe Leu Arg Asn Tyr Ile
                165                 170                 175

Lys Ser Ser Ala His Thr Leu Arg Asn Ala Lys Asp Leu Asn Cys Gln
            180                 185                 190

Pro Ser Thr Ala Ala Val Ala Ala Ala Gln Ser Val Leu Arg Leu Ser
        195                 200                 205

Glu Thr Asn Cys Asp Leu Glu
    210                 215

<210>73
<211>2358
<212>DNA
<213>Homo sapiens

<220>
<221> CDS
<222>(977)..(2261)

<400>73

gacttcctgg ctcgccagcc ccttccttcc ggagcctgac ccgggcccgg 50

gcgacctccc cgcgcgcttc ccggccgctg cccagggggt agagcgggcg 100

cagccgatca ctacctgacg gcctttttgg cggcctggcc gggctgtgca 150

gggtggtagg gcaagacgcg cggctcccaa ttctccccgg cgccttcgcc 200

ggccccgggc ttctcgcgct ccgctccggg ctgcaccgag ttgggccggc 250

gcgccgcgtt ggtgttgccg cgcggcggca gctcagagtc tccaggttgg 300

ggcgggcctg ggccgcacgg ctcctccacc caggtgacgc tgagcaggct 350

cagggtgaag cccagggaga tgcccacggc cacgggccct gcgggccgca 400

gcaccgacag cagcagcgat gcccgcatgg cgcccggacc gcgggtcccc 450

ggccccggcg aacccccaga gcagccagag gagtctccga gggggcggga 500

ccggggaggg ggcggatccg gagggctcgg gccccgcggg cgggcccgct 550

ccctccccgc agagcagagc cagcggcccg agccgaatcc ccggagccgc 600

gcctcgattc ccctccagca gctgctctgg gctgcgcagg gttcttgcgc 650

tcggcactgg agcctcagcc gcggccgcag ctgtccgacg tgtcactgca 700

agggccccgc ccccggggtg gggtctcggg ctctcgctac cggagaggga 750

ggagaagggg gaggttaaag gggaaggacc cccggaagtg ccccctcctc 800

agtgcgggag agggagacgc cgggggcgga gtcccctgcc tcccgcggcg 850

tggttggtgc gtcccatgtg acgtcagaag cagcccgccc ctgcctggat 900

ggtgcgccct gagtgacgtc aggagcagag gccggagctg tccatcagca 950

ccaaaggccg cgggcgggct cagggc atg ggg ccg cgg ttc tgg ggc ggc 1000
Met Gly Pro Arg Phe Trp Gly Gly
1               5

ccg agc ccc ggc tcc tgc gcc ttc ccc ttc ctc agg ccc agc ccg agt 1048
Pro Ser Pro Gly Ser Cys Ala Phe Pro Phe Leu Arg Pro Ser Pro Ser
    10                  15                  20

tcc cgg acg ccg cgg gac tgg agt gcc agc cgg tgt tgg acg tgg agc 1096
Ser Arg Thr Pro Arg Asp Trp Ser Ala Ser Arg Cys Trp Thr Trp Ser
25                  30                  35                  40

ggc gcc gcc acc gcg ccg aca cca ttc tct ccg gcc cag cag ccc cct 1144
Gly Ala Ala Thr Ala Pro Thr Pro Phe Ser Pro Ala Gln Gln Pro Pro
                45                  50                  55

tcc tcg cac qac gga ctt tcc ctg gac ccc agc act atg ccg ggg act 1192
Ser Ser His Asp Gly Leu Ser Leu Asp Pro Ser Thr Met Pro Gly Thr
            60                  65                  70

gtg gca aca ctg cgg ttc cag ctg ctg ccc cct gag cca gat gat gcc 1240
Val Ala Thr Leu Arg Phe Gln Leu Leu Pro Pro Glu Pro Asp Asp Ala
        75                  80                  85

ttc tgg ggt gca cct tgt gaa cag ccc ctg gag cgc agg tac cag gca 1288
Phe Trp Gly Ala Pro Cys Glu Gln Pro Leu Glu Arg Arg Tyr Gln Ala
    90                  95                  100

ctg ccg gcc ctc gtc tgc atc atg tgc tgt ttg ttt gga gtc gtc tac 1336
Leu Pro Ala Leu Val Cys Ile Met Cys Cys Leu Phe Gly Val Val Tyr
105                 110                 115                 120

tgc ttc ttc ggt tac cgc tgc ttc aag gca gtg ctc ttt ctc act ggg 1384
Cys Phe Phe Gly Tyr Arg Cys Phe Lys Ala Val Leu Phe Leu Thr Gly
                125                 130                 135

ttg ctg ttt ggc tcg gtg gtc atc ttc ctc ctc tgc tac cga gag cgg 1432
Leu Leu Phe Gly Ser Val Val Ile Phe Leu Leu Cys Tyr Arg Glu Arg
            140                 145                 150

gtg cta gag aca cag ctg agt gct ggg gcg agc gcg ggc atc gct ctg 1480
Val Leu Glu Thr Gln Leu Ser Ala Gly Ala Ser Ala Gly Ile Ala Leu
        155                 160                 165

ggc atc ggg ctg ctc tgc ggg ctg gtg gcc atg cta gtg cgc agc gtg 1528
Gly Ile Gly Leu Leu Cys Gly Leu Val Ala Met Leu Val Arg Ser Val
    170                 175                 180

ggc ctc ttc ctg gtg ggg ctg ctg ctc ggc ctg ctg ctc gca gct gct 1576
Gly Leu Phe Leu Val Gly Leu Leu Leu Gly Leu Leu Leu Ala Ala Ala
185                 190                 195                 200

gcc ctg ctg ggc tcc gca ccc tac tac cag cca ggc tcc gtg tgg ggt 1624
Ala Leu Leu Gly Ser Ala Pro Tyr Tyr Gln Pro Gly Ser Val Trp Gly
                205                 210                 215

cca ctg ggg ctg ttg ctg ggg ggc ggc ctg ctc tgt gcc ctg ctc act 1672
Pro Leu Gly Leu Leu Leu Gly Gly Gly Leu Leu Cys Ala Leu Leu Thr
            220                 225                 230

ctg cgc tgg ccc cgc cca ctc acc acc ctg gcc acc gcc gtg act ggt 1720
Leu Arg Trp Pro Arg Pro Leu Thr Thr Leu Ala Thr Ala Val Thr Gly
        235                 240                 245

gct gcg ctg atc gcc act gcc gct gac tac ttc gcc gag ctg cta ctg 1768
Ala Ala Leu Ile Ala Thr Ala Ala Asp Tyr Phe Ala Glu Leu Leu Leu
    250                 255                 260

ctg ggg cgc tac gtg gtg gag cga ctc cgg gct gct cct gtg ccc cca 1816
Leu Gly Arg Tyr Val Val Glu Arg Leu Arg Ala Ala Pro Val Pro Pro
265                 270                 275                 280

ctc tgc tgg cga agc tgg gcc ctg ctg gca ctc tgg ccc ctg ctc agc 1864
Leu Cys Trp Arg Ser Trp Ala Leu Leu Ala Leu Trp Pro Leu Leu Ser
                285                 290                 295

ctg atg ggc gtt ctg gtg cag tgg agg gtg aca gct gag ggg gac tcc 1912
Leu Met Gly Val Leu Val Gln Trp Arg Val Thr Ala Glu Gly Asp Ser
            300                 305                 310

cac acg gaa gtg gtc atc agc cgg cag cgc cga cgc gtg caa ctg atg 1960
His Thr Glu Val Val Ile Ser Arg Gln Arg Arg Arg Val Gln Leu Met
        315                 320                 325

cgg att cgg cag cag gaa gat cgc aag gag aaa agg cgg aaa aag aga 2008
Arg Ile Arg Gln Gln Glu Asp Arg Lys Glu Lys Arg Arg Lys Lys Arg
    330                 335                 340

cct cct cgg gct ccc ctc aga ggt ccc cgg gct cct ccc agg cct ggg 2056
Pro Pro Arg Ala Pro Leu Arg Gly Pro Arg Ala Pro Pro Arg Pro Gly
345                 350                 355                 360

cca cca gat cct gct tat cgg cgc agg cca gtg ccc atc aaa cgc ttc 2104
Pro Pro Asp Pro Ala Tyr Arg Arg Arg Pro Val Pro Ile Lys Arg Phe
                365                 370                 375

aat gga gac gtc ctc tcc ccg agc tat atc cag agc ttc cga gac cgg 2152
Asn Gly Asp Val Leu Ser Pro Ser Tyr Ile Gln Ser Phe Arg Asp Arg
            380                 385                 390

cag acc ggg agc tcc ctg agc tcc ttc atg gcc tca ccc aca gat gcg 2200
Gln Thr Gly Ser Ser Leu Ser Ser Phe Met Ala Ser Pro Thr Asp Ala
        395                 400                 405

gac tat gag tat ggg tcc cgg gga cct ctg aca gcc tgc tca ggc ccc 2248
Asp Tyr Glu Tyr Gly Ser Arg Gly Pro Leu Thr Ala Cys Ser Gly Pro
    410                 415                 420

cca gtg cgg gta tagccatatc tgtctgtcta gactctgcag tcaccagctc 2300
Pro Val Arg Val
425

tgacagctcg aggaggccgg taggctgcaa tcagcttccg gtttggtggt 2350

ccttccca 2358

<210>74
<211>428
<212>PRT
<213>Homo sapiens

<400>74

Met Gly Pro Arg Phe Trp Gly Gly Pro Ser Pro Gly Ser Cys Ala Phe
1               5                   10                  15

Pro Phe Leu Arg Pro Ser Pro Ser Ser Arg Thr Pro Arg Asp Trp Ser
            20                  25                  30

Ala Ser Arg Cys Trp Thr Trp Ser Gly Ala Ala Thr Ala Pro Thr Pro
        35                  40                  45

Phe Ser Pro Ala Gln Gln Pro Pro Ser Ser His Asp Gly Leu Ser Leu
    50                  55                  60

Asp Pro Ser Thr Met Pro Gly Thr Val Ala Thr Leu Arg Phe Gln Leu
65                  70                  75                  80

Leu Pro Pro Glu Pro Asp Asp Ala Phe Trp Gly Ala Pro Cys Glu Gln
                85                  90                  95

Pro Leu Glu Arg Arg Tyr Gln Ala Leu Pro Ala Leu Val Cys Ile Met
            100                 105                 110

Cys Cys Leu Phe Gly Val Val Tyr Cys Phe Phe Gly Tyr Arg Cys Phe
        115                 120                 125

Lys Ala Val Leu Phe Leu Thr Gly Leu Leu Phe Gly Ser Val Val Ile
    130                 135                 140

Phe Leu Leu Cys Tyr Arg Glu Arg Val Leu Glu Thr Gln Leu Ser Ala
145                 150                 155                 160

Gly Ala Ser Ala Gly Ile Ala Leu Gly Ile Gly Leu Leu Cys Gly Leu
                165                 170                 175

Val Ala Met Leu Val Arg Ser Val Gly Leu Phe Leu Val Gly Leu Leu

180                 185                 190

Leu Gly Leu Leu Leu Ala Ala Ala Ala Leu Leu Gly Ser Ala Pro Tyr
        195                 200                 205

Tyr Gln Pro Gly Ser Val Trp Gly Pro Leu Gly Leu Leu Leu Gly Gly
    210                 215                 220

Gly Leu Leu Cys Ala Leu Leu Thr Leu Arg Trp Pro Arg Pro Leu Thr
225                 230                 235                 240

Thr Leu Ala Thr Ala Val Thr Gly Ala Ala Leu Ile Ala Thr Ala Ala
                245                 250                 255

Asp Tyr Phe Ala Glu Leu Leu Leu Leu Gly Arg Tyr Val Val Glu Arg
            260                 265                 270

Leu Arg Ala Ala Pro Val Pro Pro Leu Cys Trp Arg Ser Trp Ala Leu
        275                 280                 285

Leu Ala Leu Trp Pro Leu Leu Ser Leu Met Gly Val Leu Val Gln Trp
    290                 295                 300

Arg Val Thr Ala Glu Gly Asp Ser His Thr Glu Val Val Ile Ser Arg
305                 310                 315                 320

Gln Arg Arg Arg Val Gln Leu Met Arg Ile Arg Gln Gln Glu Asp Arg
                325                 330                 335

Lys Glu Lys Arg Arg Lys Lys Arg Pro Pro Arg Ala Pro Leu Arg Gly
            340                 345                 350

Pro Arg Ala Pro Pro Arg Pro Gly Pro Pro Asp Pro Ala Tyr Arg Arg
        355                 360                 365

Arg Pro Val Pro Ile Lys Arg Phe Asn Gly Asp Val Leu Ser Pro Ser
    370                 375                 380

Tyr Ile Gln Ser Phe Arg Asp Arg Gln Thr Gly Ser Ser Leu Ser Ser
385                 390                 395                 400

Phe Met Ala Ser Pro Thr Asp Ala Asp Tyr Glu Tyr Gly Ser Arg Gly
                405                 410                 415

Pro Leu Thr Ala Cys Ser Gly Pro Pro Val Arg Val
            420                 425

<210>75
<211>2177
<212>DNA
<213>Homo sapiens

<220>
<221> CDS
<222>(27)..(2154)

<400>75
   attccccttg ccgacccaca tacacc atg aag agg tgc aga tcg gac gag 50
   Met Lys Arg Cys Arg Ser Asp Glu
   1 5
   ctg cag caa caa cag ggc gag gag gat gga gct ggg ctg gaa gat gcc 98
   Leu Gln Gln Gln Gln Gly Glu Glu Asp Gly Ala Gly Leu Glu Asp Ala
   10 15 20
   gct tcc cac ctg ccg ggc gcg gac ctc cgg cct ggg gag acc acg ggt 146
   Ala Ser His Leu Pro Gly Ala Asp Leu Arg Pro Gly Glu Thr Thr Gly
   25 30 35 40
   gct aac tct gct ggc ggg cca act tca gac gcc ggc gct gcc gcg gcg 194
   Ala Asn Ser Ala Gly Gly Pro Thr Ser Asp Ala Gly Ala Ala Ala Ala
   45 50 55
   ccc aac cca ggt ccc cga agc aag cct cct gat tta aag aaa atc cag 242
   Pro Asn Pro Gly Pro Arg Ser Lys Pro Pro Asp Leu Lys Lys Ile Gln
   60 65 70
   cag ctg tca gag ggc tcc atg ttt ggc cac ggt ctg aag cac ctg ttc 290
   Gln Leu Ser Glu Gly Ser Met Phe Gly His Gly Leu Lys His Leu Phe
   75 80 85
   cac agc cgc cgt cgg tct cgg gaa agg gag cac cag acg tct cag gat 338
   His Ser Arg Arg Arg Ser Arg Glu Arg Glu His Gln Thr Ser Gln Asp
   90 95 100
   tcc cag cag cat cag cag cag cag ggt atg tcc gac cat gac tcc cca 386
   Ser Gln Gln His Gln Gln Gln Gln Gly Met Ser Asp His Asp Ser Pro
   105 110 115 120
   gat gag aag gag cgc tct ccg gag atg cat cgc gtc tcc tac gcc atg 434
   Asp Glu Lys Glu Arg Ser Pro Glu Met His Arg Val Ser Tyr Ala Met
   125 130 135
   tcc ctg cac gac ctg ccc gcc cgg ccc acc gcc ttc aac cgc gtg ctg 482
   Ser Leu His Asp Leu Pro Ala Arg Pro Thr Ala Phe Asn Arg Val Leu
   140 145 150
   cag cag atc cgc tcc cgg ccc tcc atc aag cgg ggc gcc agc ctg cac 530
   Gln Gln Ile Arg Ser Arg Pro Ser Ile Lys Arg Gly Ala Ser Leu His
   155 160 165
   agc agc agt ggg ggc ggc agc agc ggg agc agc agc cgg cgc acc aag 578
   Ser Ser Ser Gly Gly Gly Ser Ser Gly Ser Ser Ser Arg Arg Thr Lys
   170 175 180
   agt agc tcc ctg gag ccc cag cgt ggc agc cct cac ctg ctg cgc aag 626
   Ser Ser Ser Leu Glu Pro Gln Arg Gly Ser Pro His Leu Leu Arg Lys
   185 190 195 200
   gcc ccc cag gac agc agc ctg gcc gcc atc ctg cac cag cac cag tgc 674
   Ala Pro Gln Asp Ser Ser Leu Ala Ala Ile Leu His Gln His Gln Cys
   205 210 215
   cgt ccc cgc tct tcc tcc acc acc gac act gct ctg ctg ctg gcc gac 722
   Arg Pro Arg Ser Ser Ser Thr Thr Asp Thr Ala Leu Leu Leu Ala Asp
   220 225 230
   ggc agc aac gtg tac ctc ctg gct gag gag gcc gaa ggc atc ggg gac 770
   Gly Ser Asn Val Tyr Leu Leu Ala Glu Glu Ala Glu Gly Ile Gly Asp
   235 240 245
   aag gtg gat aag gga gac ctg gtg gcc ctg agc ctc ccc gcc ggc cat 818
   Lys Val Asp Lys Gly Asp Leu Val Ala Leu Ser Leu Pro Ala Gly His
   250 255 260
   ggt gac acc gac ggc ccc atc agc ctg gac gtg ccc gat ggg gca ccg 866
   Gly Asp Thr Asp Gly Pro Ile Ser Leu Asp Val Pro Asp Gly Ala Pro
   265 270 275 280
   gac ccc cag cgg acc aag gcc gcc att gac cac ctg cac cag aag atc 914
   Asp Pro Gln Arg Thr Lys Ala Ala Ile Asp His Leu His Gln Lys Ile
   285 290 295
   ctg aag atc acc gag cag atc aag att gag cag gag gct cgc gac gac 962
   Leu Lys Ile Thr Glu Gln Ile Lys Ile Glu Gln Glu Ala Arg Asp Asp
   300 305 310
   aat gtg gca gag tat ctg aaa ctg gcc aac aac gcg gac aag cag cag 1010
   Asn Val Ala Glu Tyr Leu Lys Leu Ala Asn Asn Ala Asp Lys Gln Gln
   315 320 325
   gtg tca cgc atc aag caa gtg ttc gag aag aag aac cag aag tca gcc 1058
   Val Ser Arg Ile Lys Gln Val Phe Glu Lys Lys Asn Gln Lys Ser Ala
   330 335 340
   cag acc atc gcc cag ctg cac aag aag ctg gag cac tac cgc cgg cgc 1106
   Gln Thr Ile Ala Gln Leu His Lys Lys Leu Glu His Tyr Arg Arg Arg
   345 350 355 360
   ctg aag gag att gag cag aac ggg ccc tcg cgg cag ccc aag gac gtg 1154
   Leu Lys Glu Ile Glu Gln Asn Gly Pro Ser Arg Gln Pro Lys Asp Val
   365 370 375
   ctg cgg gac atg cag cag ggg ctg aag gac gtg ggc gcc aac gtg cgc 1202
   Leu Arg Asp Met Gln Gln Gly Leu Lys Asp Val Gly Ala Asn Val Arg
   380 385 390
   gca ggc atc agc ggc ttt ggg ggc ggc gtg gtg gag ggc gtc aag ggc 1250
   Ala Gly Ile Ser Gly Phe Gly Gly Gly Val Val Glu Gly Val Lys Gly
   395 400 405
   agc ctc tct ggc ctc tca cag gcc acc cac acc gcc gtg gtg tcc aag 1298
   Ser Leu Ser Gly Leu Ser Gln Ala Thr His Thr Ala Val Val Ser Lys
   410 415 420
   ccc cgg gag ttt gcc agc ctc atc cgc aac aag ttt ggc agt gct gac 1346
   Pro Arg Glu Phe Ala Ser Leu Ile Arg Asn Lys Phe Gly Ser Ala Asp
   425 430 435 440
   aac atc gcc cac ctg aag gac ccc ctg gaa gat ggg ccc cct gag gag 1394
   Asn Ile Ala His Leu Lys Asp Pro Leu Glu Asp Gly Pro Pro Glu Glu
   445 450 455
   gca gcc cgg gca ctg agc ggc agt gcc aca ctc gtc tcc agc ccc aag 1442
   Ala Ala Arg Ala Leu Ser Gly Ser Ala Thr Leu Val Ser Ser Pro Lys
   460 465 470
   tat ggc agc gat gat gag tgc tcc agc gcc acg ctc agc tca gcc ggg 1490
   Tyr Gly Ser Asp Asp Glu Cys Ser Ser Ala Thr Leu Ser Ser Ala Gly
   475 480 485
   gca ggc agc aac tct ggg gct ggg cct ggt ggg gcg ctg ggg agc cct 1538
   Ala Gly Ser Asn Ser Gly Ala Gly Pro Gly Gly Ala Leu Gly Ser Pro
   490 495 500
   aag tcc aat gca ctg tat ggt gct cct gga aac ctg gat gct ctg ctg 1586
   Lys Ser Asn Ala Leu Tyr Gly Ala Pro Gly Asn Leu Asp Ala Leu Leu
   505 510 515 520
   gaa gag cta cgg gag atc aag gag gga cag tct cac ctg gag gac tcc 1634
   Glu Glu Leu Arg Glu Ile Lys Glu Gly Gln Ser His Leu Glu Asp Ser
   525 530 535
   atg gaa gac ctg aag act cag ctg cag agg gac tac acc tac atg acc 1682
   Met Glu Asp Leu Lys Thr Gln Leu Gln Arg Asp Tyr Thr Tyr Met Thr
   540 545 550
   cag tgc ctg cag gag gag cgc tac agg tac gag cgg ctg gag gag cag 1730
   Gln Cys Leu Gln Glu Glu Arg Tyr Arg Tyr Glu Arg Leu Glu Glu Gln
   555 560 565
   ctc aac gac ctg act gag ctt cat cag aac gag atg acg aac ctg aag 1778
   Leu Asn Asp Leu Thr Glu Leu His Gln Asn Glu Met Thr Asn Leu Lys
   570 575 580
   cag gag ctg gcc agc atg gag gag aag gtg gcc tac cag tcc tat gag 1826
   Gln Glu Leu Ala Ser Met Glu Glu Lys Val Ala Tyr Gln Ser Tyr Glu
   585 590 595 600
   agg gca cgg gac atc cag gag gcc gtg gag tcc tgc ctg acc cgg gtc 1874
   Arg Ala Arg Asp Ile Gln Glu Ala Val Glu Ser Cys Leu Thr Arg Val
   605 610 615
   acc aag ctg gag ctg cag cag caa cag cag cag gtg gta cag ctg gag 1922
   Thr Lys Leu Glu Leu Gln Gln Gln Gln Gln Gln Val Val Gln Leu Glu
   620 625 630
   ggc gtg gag aat gcc aac gcg cgg gcg ctg ctg ggc aag ttc atc aac 1970
   Gly Val Glu Asn Ala Asn Ala Arg Ala Leu Leu Gly Lys Phe Ile Asn
   635 640 645
   gtg atc ctg gcg ctc atg gcc gtg ctg ctg gtg ttc gtg tcc acc atc 2018
   Val Ile Leu Ala Leu Met Ala Val Leu Leu Val Phe Val Ser Thr Ile
   650 655 660
   gcc aac ttc atc acg ccc ctc atg aag aca cgc ctg cgc atc acc agc 2066
   Ala Asn Phe Ile Thr Pro Leu Met Lys Thr Arg Leu Arg Ile Thr Ser
   665                 670                 675                 680
   acc acc ctc ctg gtc ctc gtc ctg ttc ctc ctc tgg aag cac tgg gac    2114
   Thr Thr Leu Leu Val Leu Val Leu Phe Leu Leu Trp Lys His Trp Asp
                   685                 690                 695
   tcc ctc acc tac ctc ctg gag cac gtg ttg ctg ccc agc tgagtggcca    2163
   Ser Leu Thr Tyr Leu Leu Glu His Val Leu Leu Pro Ser
               700                 705
   gccacaccaa ccct                                                   2177

<210>76
<211>709
<212>PRT
<213>Homo sapiens

<400>76

Met Lys Arg Cys Arg Ser Asp Glu Leu Gln Gln Gln Gln Gly Glu Glu
1               5                   10                  15

Asp Gly Ala Gly Leu Glu Asp Ala Ala Ser His Leu Pro Gly Ala Asp
            20                  25                  30

Leu Arg Pro Gly Glu Thr Thr Gly Ala Asn Ser Ala Gly Gly Pro Thr
        35                  40                  45

Ser Asp Ala Gly Ala Ala Ala Ala Pro Asn Pro Gly Pro Arg Ser Lys
    50                  55                  60

Pro Pro Asp Leu Lys Lys Ile Gln Gln Leu Ser Glu Gly Ser Met Phe
65                  70                  75                  80

Gly His Gly Leu Lys His Leu Phe His Ser Arg Arg Arg Ser Arg Glu
                85                  90                  95

Arg Glu His Gln Thr Ser Gln Asp Ser Gln Gln His Gln Gln Gln
            100                 105                 110

Gly Met Ser Asp His Asp Ser Pro Asp Glu Lys Glu Arg Ser Pro Glu
        115                 120                 125

Met His Arg Val Ser Tyr Ala Met Ser Leu His Asp Leu Pro Ala Arg
    130                 135                 140

Pro Thr Ala Phe Asn Arg Val Leu Gln Gln Ile Arg Ser Arg Pro Ser
145                 150                 155                 160

Ile Lys Arg Gly Ala Ser Leu His Ser Ser Ser Gly Gly Gly Ser Ser
                165                 170                 175

Gly Ser Ser Ser Arg Arg Thr Lys Ser Ser Ser Leu Glu Pro Gln Arg
            180                 185                 190

Gly Ser Pro His Leu Leu Arg Lys Ala Pro Gln Asp Ser Ser Leu Ala
            195                 200                 205

Ala Ile Leu His Gln His Gln Cys Arg Pro Arg Ser Ser Ser Thr Thr
        210                 215                 220

Asp Thr Ala Leu Leu Leu Ala Asp Gly Ser Asn Val Tyr Leu Leu Ala
225 230 235 240

Glu Glu Ala Glu Gly Ile Gly Asp Lys Val Asp Lys Gly Asp Leu Val
245 250 255

Ala Leu Ser Leu Pro Ala Gly His Gly Asp Thr Asp Gly Pro Ile Ser
                260                 265                 270

Leu Asp Val Pro Asp Gly Ala Pro Asp Pro Gln Arg Thr Lys Ala Ala
            275                 280                 285

Ile Asp His Leu His Gln Lys Ile Leu Lys Ile Thr Glu Gln Ile Lys
        290                 295                 300

Ile Glu Gln Glu Ala Arg Asp Asp Asn Val Ala Glu Tyr Leu Lys Leu
305 310 315 320

Ala Asn Asn Ala Asp Lys Gln Gln Val Ser Arg Ile Lys Gln Val Phe
325 330 335

Glu Lys Lys Asn Gln Lys Ser Ala Gln Thr Ile Ala Gln Leu His Lys
                340                 345                 350

Lys Leu Glu His Tyr Arg Arg Arg Leu Lys Glu Ile Glu Gln Asn Gly
            355                 360                 365

Pro Ser Arg Gln Pro Lys Asp Val Leu Arg Asp Met Gln Gln Gly Leu
        370                 375                 380

Lys Asp Val Gly Ala Asn Val Arg Ala Gly Ile Ser Gly Phe Gly Gly
385 390 395 400

Gly Val Val Glu Gly Val Lys Gly Ser Leu Ser Gly Leu Ser Gln Ala
405 410 415

Thr His Thr Ala Val Val Ser Lys Pro Arg Glu Phe Ala Ser Leu Ile
                420                 425                 430

Arg Asn Lys Phe Gly Ser Ala Asp Asn Ile Ala His Leu Lys Asp Pro
            435                 440                 445

Leu Glu Asp Gly Pro Pro Glu Glu Ala Ala Arg Ala Leu Ser Gly Ser
        450                 455                 460

Ala Thr Leu Val Ser Ser Pro Lys Tyr Gly Ser Asp Asp Glu Cys Ser
465 470 475 480

Ser Ala Thr Leu Ser Ser Ala Gly Ala Gly Ser Asn Ser Gly Ala Gly
485 490 495

Pro Gly Gly Ala Leu Gly Ser Pro Lys Ser Asn Ala Leu Tyr Gly Ala
                500                 505                 510

Pro Gly Asn Leu Asp Ala Leu Leu Glu Glu Leu Arg Glu Ile Lys Glu
            515                 520                 525

Gly Gln Ser His Leu Glu Asp Ser Met Glu Asp Leu Lys Thr Gln Leu
        530                 535                 540

Gln Arg Asp Tyr Thr Tyr Met Thr Gln Cys Leu Gln Glu Glu Arg Tyr
545 550 555 560

Arg Tyr Glu Arg Leu Glu Glu Gln Leu Asn Asp Leu Thr Glu Leu His
565 570 575

Gln Asn Glu Met Thr Asn Leu Lys Gln Glu Leu Ala Ser Met Glu Glu
                580                 585                 590

Lys Val Ala Tyr Gln Ser Tyr Glu Arg Ala Arg Asp Ile Gln Glu Ala
            595                 600                 605

Val Glu Ser Cys Leu Thr Arg Val Thr Lys Leu Glu Leu Gln Gln Gln
        610                 615                 620

Gln Gln Gln Val Val Gln Leu Glu Gly Val Glu Asn Ala Asn Ala Arg
625 630 635 640

Ala Leu Leu Gly Lys Phe Ile Asn Val Ile Leu Ala Leu Met Ala Val
645 650 655

Leu Leu Val Phe Val Ser Thr Ile Ala Asn Phe Ile Thr Pro Leu Met
                660                 665                 670

Lys Thr Arg Leu Arg Ile Thr Ser Thr Thr Leu Leu Val Leu Val Leu
            675                 680                 685

Phe Leu Leu Trp Lys His Trp Asp Ser Leu Thr Tyr Leu Leu Glu His
        690                 695                 700

Val Leu Leu Pro Ser
    705

<210>77
<211>664
<212>DNA
<213>Homo sapiens

<220>
<221> CDS
<222>(40)..(334)

<400>77

atcgcccctc ctgcgctagc ggaggtgatc gccgcggcg atg ccg gag gag ggt 54
Met Pro Glu Glu Gly
1               5

tcg ggc tgc tcg gtg cgg cgc agg ccc tat ggg tgc gtc ctg cgg gct     102
Ser Gly Cys Ser Val Arg Arg Arg Pro Tyr Gly Cys Val Leu Arg Ala
                10                  15                  20

gct ttg gtc cca ttg gtc gcg ggc ttg gtg atc tgc ctc gtg gtg tgc     150
Ala Leu Val Pro Leu Val Ala Gly Leu Val Ile Cys Leu Val Val Cys
            25                  30                  35

atc cag cgc ttc gca cag gct cag cag cag ctg ccg ctc gag tca ctt     198
Ile Gln Arg Phe Ala Gln Ala Gln Gln Gln Leu Pro Leu Glu Ser Leu
        40                  45                  50

ggg gac ctc agc agg acc cca ggc tat act ggc agg ggg gcc cag cac     246
Gly Asp Leu Ser Arg Thr Pro Gly Tyr Thr Gly Arg Gly Ala Gln His
    55                  60                  65

tgg gcc gct cct tcc tgc atg gac cag agc tgg aca agg ggc agc tac     294
Trp Ala Ala Pro Ser Cys Met Asp Gln Ser Trp Thr Arg Gly Ser Tyr
70                  75                  80                  85

gta tcc atc gtg atg gca tct aca tgg tac aca tcc agg tgacgctggc      343
Val Ser Ile Val Met Ala Ser Thr Trp Tyr Thr Ser Arg
                90                  95

catctgctcc tccacgacgg cctccaggca ccaccccacc accctggccg              393

tgggaatctg ctctcccgcc tcccgtagca tcagcctgct gcgtctcagc              443

ttccaccaag gttgtaccat tgcctcccag cgcctgacgc ccctggcccg              493

aggggacaca ctctgcacca acctcactgg gacacttttg ccttcccgaa              543

acactgatga gaccttcttt ggagtgcagt gggtgcgccc ctgaccactg              593

ctgctgatta gggtttttta aattttattt tattttattt aagttcaaga              643

gaaaaagtgt acacacaggg g                                             664

<210>78
<211>98
<212>PRT
<213>Homo sapiens

<400>78

Met Pro Glu Glu Gly Ser Gly Cys Ser Val Arg Arg Arg Pro Tyr Gly
1               5                   10                  15

Cys Val Leu Arg Ala Ala Leu Val Pro Leu Val Ala Gly Leu Val Ile
            20                  25                  30

Cys Leu Val Val Cys Ile Gln Arg Phe Ala Gln Ala Gln Gln Gln Leu
        35                  40                  45

Pro Leu Glu Ser Leu Gly Asp Leu Ser Arg Thr Pro Gly Tyr Thr Gly
    50                  55                  60

Arg Gly Ala Gln His Trp Ala Ala Pro Ser Cys Met Asp Gln Ser Trp
65                  70                  75                  80

Thr Arg Gly Ser Tyr Val Ser Ile Val Met Ala Ser Thr Trp Tyr Thr
                85                  90                  95

Ser Arg

<210>79
<211>2840
<212>DNA
<213>Homo sapiens

<220>
<221> CDS
<222>(380)..(2678)

<400>79

cggcagtggc aggagccgcc tttccgattc cctacgatgc gggtgctgag          50

ctatggcaaa gggcagcgaa gtgacgagcg agacccgcgt acgactgtga          100

aagccacctg gagccacctt gccgggattg tacctgcagg cagaaagtct          150

tcctacgacc gtcttttccc ttagaggcac cagaatccct gtaaccattc          200

atccaggtgt tgagaagata tgtagcagcc gagcacccat cttttgacac          250

cgtcctctga aatcagcttt ggagatgctt tcactctgtc cgtcttctgc          300

agcagccagg cagagtgccg actccttcac agccgtgagg aactcttcag 350

gctccagaag ctcttaaacc tgatctaca atg gaa aaa att ctt ttt tat ctg 403
Met Glu Lys Ile Leu Phe Tyr Leu
1               5

ttt ctc att ggc ata gca gtg aaa gct cag atc tgt cca aag cgt tgt 451
Phe Leu Ile Gly Ile Ala Val Lys Ala Gln Ile Cys Pro Lys Arg Cys
    10                  15                  20

gtc tgt cag att ttg tct cct aat ctt gca acc ctt tgt gcc aag aaa 499
Val Cys Gln Ile Leu Ser Pro Asn Leu Ala Thr Leu Cys Ala Lys Lys
25                  30                  35                  40

ggg ctt tta ttt gtt cca cca aac att gac aga aga act gtg gaa ctg 547
Gly Leu Leu Phe Val Pro Pro Asn Ile Asp Arg Arg Thr Val Glu Leu
                45                  50                  55

cgg ttg gca gac aat ttt gtt aca aat att aaa agg aaa gat ttt gcc 595
Arg Leu Ala Asp Asn Phe Val Thr Asn Ile Lys Arg Lys Asp Phe Ala
            60                  65                  70

aat atg acc agc ttg gtg gac ctg act cta tcc agg aat aca ata agt 643
Asn Met Thr Ser Leu Val Asp Leu Thr Leu Ser Arg Asn Thr Ile Ser
        75                  80                  85

ttt att aca cct cat gct ttc gct gac cta cga aat ttg agg gct ttg 691
Phe Ile Thr Pro His Ala Phe Ala Asp Leu Arg Asn Leu Arg Ala Leu
    90                  95                  100

cat ttg aat agc aac aga ttg act aaa att aca aat gat atg ttc agt 739
His Leu Asn Ser Asn Arg Leu Thr Lys Ile Thr Asn Asp Met Phe Ser
105                 110                 115                 120

ggt ctt tcc aat ctt cat cat ttg ata ctg aac aac aat cag ctg act 787
Gly Leu Ser Asn Leu His His Leu Ile Leu Asn Asn Asn Gln Leu Thr
                125                 130                 135

tta att tcc tct aca gcg ttt gat gat gtc ttc gcc ctt gag gag ctg 835
Leu Ile Ser Ser Thr Ala Phe Asp Asp Val Phe Ala Leu Glu Glu Leu
            140                 145                 150

gat ctg tcc tat aat aat cta gaa acc att cct tgg gat gct gtt gag 883
Asp Leu Ser Tyr Asn Asn Leu Glu Thr Ile Pro Trp Asp Ala Val Glu
        155                 160                 165

aag atg gtt agc ttg cat acc ctt agt ttg gat cac aat atg att gat 931
Lys Met Val Ser Leu His Thr Leu Ser Leu Asp His Asn Met Ile Asp
    170                 175                 180

aac att cct aag ggg acc ttc tcc cat ttg cac aag atg act cgg tta 979
Asn Ile Pro Lys Gly Thr Phe Ser His Leu His Lys Met Thr Arg Leu
185                 190                 195                 200

gat gtg aca tca aat aaa ttg cag aag cta cca cct gac cct ctc ttt 1027
Asp Val Thr Ser Asn Lys Leu Gln Lys Leu Pro Pro Asp Pro Leu Phe
                205                 210                 215

cag cga gct cag gta cta gca acc tca gga atc ata agc cca tct act 1075
Gln Arg Ala Gln Val Leu Ala Thr Ser Gly Ile Ile Ser Pro Ser Thr
            220                 225                 230

ttt gca tta agt ttt ggt gga aac ccc ttg cat tgc aat tgt gaa ttg 1123
Phe Ala Leu Ser Phe Gly Gly Asn Pro Leu His Cys Asn Cys Glu Leu
        235                 240                 245

ttg tgg ttg agg cgt ctg tcc aga gaa gat gac tta gag acc tgt gct 1171
Leu Trp Leu Arg Arg Leu Ser Arg Glu Asp Asp Leu Glu Thr Cys Ala
    250                 255                 260

tct cct cca ctt tta act ggc cgc tac ttt tgg tca att cct gaa gaa 1219
Ser Pro Pro Leu Leu Thr Gly Arg Tyr Phe Trp Ser Ile Pro Glu Glu
265                 270                 275                 280

gag ttt ttg tgt gag cct cct ctc att act cgt cat aca cat gag atg 1267
Glu Phe Leu Cys Glu Pro Pro Leu Ile Thr Arg His Thr His Glu Met
                285                 290                 295

aga gtc ctg gag gga caa agg gca aca ctg agg tgc aaa gcc agg gga 1315
Arg Val Leu Glu Gly Gln Arg Ala Thr Leu Arg Cys Lys Ala Arg Gly
            300                 305                 310

gac cct gag cct gca att cac tgg att tct cct gaa ggg aag ctt att 1363
Asp Pro Glu Pro Ala Ile His Trp Ile Ser Pro Glu Gly Lys Leu Ile
        315                 320                 325

tca aat gca aca aga tct ctg gtg tat gat aac gga aca ctt gac att 1411
Ser Asn Ala Thr Arg Ser Leu Val Tyr Asp Asn Gly Thr Leu Asp Ile
    330                 335                 340

ctt atc aca act gta aag gat aca ggt gct ttt acc tgc att gct tcc 1459
Leu Ile Thr Thr Val Lys Asp Thr Gly Ala Phe Thr Cys Ile Ala Ser
345                 350                 355                 360

aat cct gct ggg gaa gca aca caa ata gtg gat ctt cat ata att aag 1507
Asn Pro Ala Gly Glu Ala Thr Gln Ile Val Asp Leu His Ile Ile Lys
                365                 370                 375

ctc cct cac tta cta aat agt aca aac cat atc cat gag cct gat cct 1555
Leu Pro His Leu Leu Asn Ser Thr Asn His Ile His Glu Pro Asp Pro
            380                 385                 390

ggt tct tca gat atc tca act tct acc aag tca ggt tct aat aca agc 1603
Gly Ser Ser Asp Ile Ser Thr Ser Thr Lys Ser Gly Ser Asn Thr Ser
        395                 400                 405

agt agt aat ggt gat act aaa ttg agt caa gat aaa att gtg gtg gca 1651
Ser Ser Asn Gly Asp Thr Lys Leu Ser Gln Asp Lys Ile Val Val Ala
    410                 415                 420

gaa gct aca tca tca acg gca cta ctt aaa ttt aat ttt caa aga aat 1699

Glu Ala Thr Ser Ser Thr Ala Leu Leu Lys Phe Asn Phe Gln Arg Asn
425                 430                 435                 440

atc cct gga ata cgt atg ttt caa atc cag tac aat ggt act tat gat    1747
Ile Pro Gly Ile Arg Met Phe Gln Ile Gln Tyr Asn Gly Thr Tyr Asp
                445                 450                 455

gac acc ctt gtt tac agg atg ata cct cct acg agc aaa act ttt ctg    1795
Asp Thr Leu Val Tyr Arg Met Ile Pro Pro Thr Ser Lys Thr Phe Leu
            460                 465                 470

gtc aat aat ctg gct gct gga act atg tat gac ttg tgt gtc ttg gcc    1843
Val Asn Asn Leu Ala Ala Gly Thr Met Tyr Asp Leu Cys Val Leu Ala
        475                 480                 485

ata tat gat gat ggc atc act tcc ctc act gcc aca aga gtc gtg ggt    1891
Ile Tyr Asp Asp Gly Ile Thr Ser Leu Thr Ala Thr Arg Val Val Gly
    490                 495                 500

tgc atc cag ttt act acg gaa cag gat tat gtg cgt tgc cat ttc atg    1939
Cys Ile Gln Phe Thr Thr Glu Gln Asp Tyr Val Arg Cys His Phe Met
505                 510                 515                 520

cag tcc cag ttt ttg gga ggc acc atg att att att att ggt gga atc    1987
Gln Ser Gln Phe Leu Gly Gly Thr Met Ile Ile Ile Ile Gly Gly Ile
                525                 530                 535

att gta gca tct gtg ctg gta ttc atc att att ctg atg atc cgg tat    2035
Ile Val Ala Ser Val Leu Val Phe Ile Ile Ile Leu Met Ile Arg Tyr
            540                 545                 550

aag gtt tgc aac aat aat ggg caa cac aag gtc acc aag gtt agc aat    2083
Lys Val Cys Asn Asn Asn Gly Gln His Lys Val Thr Lys Val Ser Asn
        555                 560                 565

gtt tat tcc caa act aac ggg gct caa ata caa ggc tgt agt gta acg    2131
Val Tyr Ser Gln Thr Asn Gly Ala Gln Ile Gln Gly Cys Ser Val Thr
    570                 575                 580

ctg ccc cag tcc gtg tcc aaa caa gct gtg gga cac gaa gag aat gcc    2179
Leu Pro Gln Ser Val Ser Lys Gln Ala Val Gly His Glu Glu Asn Ala
585                 590                 595                 600

cag tgt tgt aaa gct acc agt gac aat gtg att caa tct tca gaa act    2227
Gln Cys Cys Lys Ala Thr Ser Asp Asn Val Ile Gln Ser Ser Glu Thr
                605                 610                 615

tgt tcg agt caa gac tcc tct acc act acc tct gct ttg cct cct tcc    2275
Cys Ser Ser Gln Asp Ser Ser Thr Thr Thr Ser Ala Leu Pro Pro Ser
            620                 625                 630

tgg act tca agc act tct gtg tcc caa aag cag aaa aga aag act ggc    2323
Trp Thr Ser Ser Thr Ser Val Ser Gln Lys Gln Lys Arg Lys Thr Gly
        635                 640                 645

aca aag cca agt aca gaa cca cag aat gaa gcc gtc aca aat gtt gaa    2371
Thr Lys Pro Ser Thr Glu Pro Gln Asn Glu Ala Val Thr Asn Val Glu

650                 655                 660

tcc caa aac act aac agg aac aac tca act gcc ttg cag tta gct agc    2419
Ser Gln Asn Thr Asn Arg Asn Asn Ser Thr Ala Leu Gln Leu Ala Ser
665                 670                 675                 680

cgt cct ccc gat tct gtc aca gag ggg ccc acg tct aaa aga gca cat    2467
Arg Pro Pro Asp Ser Val Thr Glu Gly Pro Thr Ser Lys Arg Ala His
                685                 690                 695

ata aag cca agt aag ttt atc act ttg cct gct gag aga tcc gga gca    2515
Ile Lys Pro Ser Lys Phe Ile Thr Leu Pro Ala Glu Arg Ser Gly Ala
            700                 705                 710

agg cac aag tac tcc ctc aat gga gaa tta aag gaa tac tat tgt tat    2563
Arg His Lys Tyr Ser Leu Asn Gly Glu Leu Lys Glu Tyr Tyr Cys Tyr
        715                 720                 725

att aac tcg ccg aac aca tgt gga ctg ttt cct aaa aga agc atg tct    2611
Ile Asn Ser Pro Asn Thr Cys Gly Leu Phe Pro Lys Arg Ser Met Ser
    730                 735                 740

atg aat gtg atg ttt att cag tct gac tgt tct gat ggt cat agt gga    2659
Met Asn Val Met Phe Ile Gln Ser Asp Cys Ser Asp Gly His Ser Gly
745                 750                 755                 760

aag gca act ctc aaa ttc tgagggacta ctggaaagct ctgtgtaatt           2707
Lys Ala Thr Leu Lys Phe
                765

tataatttct ttttcatgaa aaatcatttt gagaactcac atagaagatt             2757

ggaatttgca attccaatgc tgtgtataaa tcaaccttct cagatgcttt             2807

gctgactaat gttgaccaga ttgtccagga aac                               2840

<210>80
<211>766
<212>PRT
<213>Homo sapiens

<400>80

Met Glu Lys Ile Leu Phe Tyr Leu Phe Leu Ile Gly Ile Ala Val Lys
1               5                   10                  15

Ala Gln Ile Cys Pro Lys Arg Cys Val Cys Gln Ile Leu Ser Pro Asn
            20                  25                  30

Leu Ala Thr Leu Cys Ala Lys Lys Gly Leu Leu Phe Val Pro Pro Asn
        35                  40                  45

Ile Asp Arg Arg Thr Val Glu Leu Arg Leu Ala Asp Asn Phe Val Thr
    50                  55                  60

Asn Ile Lys Arg Lys Asp Phe Ala Asn Met Thr Ser Leu Val Asp Leu
65                  70                  75                  80

Thr Leu Ser Arg Asn Thr Ile Ser Phe Ile Thr Pro His Ala Phe Ala
                85                  90                  95

Asp Leu Arg Asn Leu Arg Ala Leu His Leu Asn Ser Asn Arg Leu Thr
            100                 105                 110

Lys Ile Thr Asn Asp Met Phe Ser Gly Leu Ser Asn Leu His His Leu
        115                 120                 125

Ile Leu Asn Asn Asn Gln Leu Thr Leu Ile Ser Ser Thr Ala Phe Asp
    130                 135                 140

Asp Val Phe Ala Leu Glu Glu Leu Asp Leu Ser Tyr Asn Asn Leu Glu
145                 150                 155                 160

Thr Ile Pro Trp Asp Ala Val Glu Lys Met Val Ser Leu His Thr Leu
                165                 170                 175

Ser Leu Asp His Asn Met Ile Asp Asn Ile Pro Lys Gly Thr Phe Ser
            180                 185                 190

His Leu His Lys Met Thr Arg Leu Asp Val Thr Ser Asn Lys Leu Gln
        195                 200                 205

Lys Leu Pro Pro Asp Pro Leu Phe Gln Arg Ala Gln Val Leu Ala Thr
    210                 215                 220

Ser Gly Ile Ile Ser Pro Ser Thr Phe Ala Leu Ser Phe Gly Gly Asn
225                 230                 235                 240

Pro Leu His Cys Asn Cys Glu Leu Leu Trp Leu Arg Arg Leu Ser Arg
                245                 250                 255

Glu Asp Asp Leu Glu Thr Cys Ala Ser Pro Pro Leu Leu Thr Gly Arg
            260                 265                 270

Tyr Phe Trp Ser Ile Pro Glu Glu Glu Phe Leu Cys Glu Pro Pro Leu
        275                 280                 285

Ile Thr Arg His Thr His Glu Met Arg Val Leu Glu Gly Gln Arg Ala
    290                 295                 300

Thr Leu Arg Cys Lys Ala Arg Gly Asp Pro Glu Pro Ala Ile His Trp
305                 310                 315                 320

Ile Ser Pro Glu Gly Lys Leu Ile Ser Asn Ala Thr Arg Ser Leu Val
                325                 330                 335

Tyr Asp Asn Gly Thr Leu Asp Ile Leu Ile Thr Thr Val Lys Asp Thr
            340                 345                 350

Gly Ala Phe Thr Cys Ile Ala Ser Asn Pro Ala Gly Glu Ala Thr Gln
        355                 360                 365

Ile Val Asp Leu His Ile Ile Lys Leu Pro His Leu Leu Asn Ser Thr
    370                 375                 380

Asn His Ile His Glu Pro Asp Pro Gly Ser Ser Asp Ile Ser Thr Ser
385                 390                 395                 400

Thr Lys Ser Gly Ser Asn Thr Ser Ser Ser Asn Gly Asp Thr Lys Leu
                405                 410                 415

Ser Gln Asp Lys Ile Val Val Ala Glu Ala Thr Ser Ser Thr Ala Leu
            420                 425                 430

Leu Lys Phe Asn Phe Gln Arg Asn Ile Pro Gly Ile Arg Met Phe Gln
        435                 440                 445

Ile Gln Tyr Asn Gly Thr Tyr Asp Asp Thr Leu Val Tyr Arg Met Ile
    450                 455                 460

Pro Pro Thr Ser Lys Thr Phe Leu Val Asn Asn Leu Ala Ala Gly Thr
465                 470                 475                 480

Met Tyr Asp Leu Cys Val Leu Ala Ile Tyr Asp Asp Gly Ile Thr Ser
                485                 490                 495

Leu Thr Ala Thr Arg Val Val Gly Cys Ile Gln Phe Thr Thr Glu Gln
            500                 505                 510

Asp Tyr Val Arg Cys His Phe Met Gln Ser Gln Phe Leu Gly Gly Thr
        515                 520                 525

Met Ile Ile Ile Ile Gly Gly Ile Ile Val Ala Ser Val Leu Val Phe
    530                 535                 540

Ile Ile Ile Leu Met Ile Arg Tyr Lys Val Cys Asn Asn Asn Gly Gln
545                 550                 555                 560

His Lys Val Thr Lys Val Ser Asn Val Tyr Ser Gln Thr Asn Gly Ala
                565                 570                 575

Gln Ile Gln Gly Cys Ser Val Thr Leu Pro Gln Ser Val Ser Lys Gln
            580                 585                 590

Ala Val Gly His Glu Glu Asn Ala Gln Cys Cys Lys Ala Thr Ser Asp
        595                 600                 605

Asn Val Ile Gln Ser Ser Glu Thr Cys Ser Ser Gln Asp Ser Ser Thr
    610                 615                 620

Thr Thr Ser Ala Leu Pro Pro Ser Trp Thr Ser Ser Thr Ser Val Ser
625                 630                 635                 640

Gln Lys Gln Lys Arg Lys Thr Gly Thr Lys Pro Ser Thr Glu Pro Gln
                645                 650                 655

Asn Glu Ala Val Thr Asn Val Glu Ser Gln Asn Thr Asn Arg Asn Asn
            660                 665                 670

Ser Thr Ala Leu Gln Leu Ala Ser Arg Pro Pro Asp Ser Val Thr Glu
        675                 680                 685

Gly Pro Thr Ser Lys Arg Ala His Ile Lys Pro Ser Lys Phe Ile Thr
    690                 695                 700

Leu Pro Ala Glu Arg Ser Gly Ala Arg His Lys Tyr Ser Leu Asn Gly
705                 710                 715                 720

Glu Leu Lys Glu Tyr Tyr Cys Tyr Ile Asn Ser Pro Asn Thr Cys Gly
                725                 730                 735

Leu Phe Pro Lys Arg Ser Met Ser Met Asn Val Met Phe Ile Gln Ser
            740                 745                 750

Asp Cys Ser Asp Gly His Ser Gly Lys Ala Thr Leu Lys Phe
        755                 760                 765

<210>81
<211>2388
<212>DNA
<213>Homo sapiens

<220>
<221> CDS
<222>(21)..(2178)

<400>81

gctcttaaac ctgatctaca atg gaa aaa att ctt ttt tat ctg ttt ctc 50
Met Glu Lys Ile Leu Phe Tyr Leu Phe Leu
1               5                   10

att ggc ata gca gtg aaa gct cag atc tgt cca aag cgt tgt gtc tgt 98
Ile Gly Ile Ala Val Lys Ala Gln Ile Cys Pro Lys Arg Cys Val Cys
                15                  20                  25

cag att ttg tct cct aat ctt gca acc ctt tgt gcc aag aaa ggg ctt 146
Gln Ile Leu Ser Pro Asn Leu Ala Thr Leu Cys Ala Lys Lys Gly Leu
            30                  35                  40

tta ttt gtt cca cca aac att gac aga aga act gtg gaa ctg cgg ttg 194
Leu Phe Val Pro Pro Asn Ile Asp Arg Arg Thr Val Glu Leu Arg Leu
        45                  50                  55

gca gac aat ttt gtt aca aat att aaa agg aaa gat ttt gcc aat atg 242
Ala Asp Asn Phe Val Thr Asn Ile Lys Arg Lys Asp Phe Ala Asn Met
    60                  65                  70

acc agc ttg gtg gac ctg act cta tcc agg aat aca ata agt ttt att 290
Thr Ser Leu Val Asp Leu Thr Leu Ser Arg Asn Thr Ile Ser Phe Ile
75                  80                  85                  90

aca cct cat gct ttc gct gac cta cga aat ttg agg gct ttg cat ttg 338
Thr Pro His Ala Phe Ala Asp Leu Arg Asn Leu Arg Ala Leu His Leu
                95                  100                 105

aat agc aac aga ttg act aaa att aca aat gat atg ttc agt ggt ctt 386
Asn Ser Asn Arg Leu Thr Lys Ile Thr Asn Asp Met Phe Ser Gly Leu
            110                 115                 120

tcc aat ctt cat cat ttg ata ctg aac aac aat cag ctg act tta att 434
Ser Asn Leu His His Leu Ile Leu Asn Asn Asn Gln Leu Thr Leu Ile
        125                 130                 135

tcc tct aca gcg ttt gat gat gtc ttc gcc ctt gag gag ctg gat ctg 482
Ser Ser Thr Ala Phe Asp Asp Val Phe Ala Leu Glu Glu Leu Asp Leu
    140                 145                 150

tcc tat aat aat cta gaa acc att cct tgg gat gct gtt gag aag atg 530
Ser Tyr Asn Asn Leu Glu Thr Ile Pro Trp Asp Ala Val Glu Lys Met
155                 160                 165                 170

gtt agc ttg cat acc ctt agt ttg gat cac aat atg att gat aac att 578
Val Ser Leu His Thr Leu Ser Leu Asp His Asn Met Ile Asp Asn Ile
                175                 180                 185

cct aag ggg acc ttc tcc cat ttg cac aag atg act cgg tta gat gtg 626
Pro Lys Gly Thr Phe Ser His Leu His Lys Met Thr Arg Leu Asp Val
            190                 195                 200

aca tca aat aaa ttg cag aag cta cca cct gac cct ctc ttt cag cga 674
Thr Ser Asn Lys Leu Gln Lys Leu Pro Pro Asp Pro Leu Phe Gln Arg
        205                 210                 215

gct cag gta cta gca acc tca gga atc ata agc cca tct act ttt gca 722
Ala Gln Val Leu Ala Thr Ser Gly Ile Ile Ser Pro Ser Thr Phe Ala
    220                 225                 230

tta agt ttt ggt gga aac ccc ttg cat tgc aat tgt gaa ttg ttg tgg 770
Leu Ser Phe Gly Gly Asn Pro Leu His Cys Asn Cys Glu Leu Leu Trp
235                 240                 245                 250

ttg agg cgt ctg tcc aga gaa gat gac tta gag acc tgt gct tct cct 818
Leu Arg Arg Leu Ser Arg Glu Asp Asp Leu Glu Thr Cys Ala Ser Pro
                255                 260                 265

cca ctt tta act ggc cgc tac ttt tgg tca att cct gaa gaa gag ttt 866
Pro Leu Leu Thr Gly Arg Tyr Phe Trp Ser Ile Pro Glu Glu Glu Phe
            270                 275                 280

ttg tgt gag cct cct ctc att act cgt cat aca cat gag atg aga gtc 914
Leu Cys Glu Pro Pro Leu Ile Thr Arg His Thr His Glu Met Arg Val
        285                 290                 295

ctg gag gga caa agg gca aca ctg agg tgc aaa gcc agg gga gac cct 962
Leu Glu Gly Gln Arg Ala Thr Leu Arg Cys Lys Ala Arg Gly Asp Pro
    300                 305                 310

gag cct gca att cac tgg att tct cct gaa ggg aag ctt att tca aat 1010

Glu Pro Ala Ile His Trp Ile Ser Pro Glu Gly Lys Leu Ile Ser Asn
315                 320                 325                 330

gca aca aga tct ctg gtg tat gat aac gga aca ctt gac att ctt atc     1058
Ala Thr Arg Ser Leu Val Tyr Asp Asn Gly Thr Leu Asp Ile Leu Ile
                335                 340                 345

aca act gta aag gat aca ggt gct ttt acc tgc att gct tcc aat cct     1106
Thr Thr Val Lys Asp Thr Gly Ala Phe Thr Cys Ile Ala Ser Asn Pro
            350                 355                 360

gct ggg gaa gca aca caa ata gtg gat ctt cat ata att aag ctc cct     1154
Ala Gly Glu Ala Thr Gln Ile Val Asp Leu His Ile Ile Lys Leu Pro
        365                 370                 375

cac tta cta aat agt aca aac cat atc cat gag cct gat cct ggt tct     1202
His Leu Leu Asn Ser Thr Asn His Ile His Glu Pro Asp Pro Gly Ser
    380                 385                 390

tca gat atc tca act tct acc aag tca ggt tct aat aca agc agt agt     1250
Ser Asp Ile Ser Thr Ser Thr Lys Ser Gly Ser Asn Thr Ser Ser Ser
395                 400                 405                 410

aat ggt gat act aaa ttg agt caa gat aaa att gtg gtg gca gaa gct     1298
Asn Gly Asp Thr Lys Leu Ser Gln Asp Lys Ile Val Val Ala Glu Ala
                415                 420                 425

aca tca tca acg gca cta ctt aac ttt act ttt caa aga act atc cct     1346
Thr Ser Ser Thr Ala Leu Leu Asn Phe Thr Phe Gln Arg Thr Ile Pro
            430                 435                 440

gga ata cgt atg ttt caa atc cag tac aat ggt act tat gat gac acc     1394
Gly Ile Arg Met Phe Gln Ile Gln Tyr Asn Gly Thr Tyr Asp Asp Thr
        445                 450                 455

ctt gtt tac agg atg ata cct cct acg agc aaa act ttt ctg gtc aat     1442
Leu Val Tyr Arg Met Ile Pro Pro Thr Ser Lys Thr Phe Leu Val Asn
    460                 465                 470

aat ctg gct gct gga act atg tat gac ttg tgt gtc ttg gcc ata tat     1490
Asn Leu Ala Ala Gly Thr Met Tyr Asp Leu Cys Val Leu Ala Ile Tyr
475                 480                 485                 490

gat gat ggc atc act tcc ctc act gcc aca aga gtc gtg ggt tgc atc     1538
Asp Asp Gly Ile Thr Ser Leu Thr Ala Thr Arg Val Val Gly Cys Ile
                495                 500                 505

cag ttt act acg gaa cag gat tat gtg cgt tgc cat ttc atg cag tcc     1586
Gln Phe Thr Thr Glu Gln Asp Tyr Val Arg Cys His Phe Met Gln Ser
            510                 515                 520

cag ttt ttg gga ggc acc atg att att att att ggt gga atc att gta     1634
Gln Phe Leu Gly Gly Thr Met Ile Ile Ile Ile Gly Gly Ile Ile Val
        525                 530                 535

gca tct gtg ctg gta ttc atc att att ctg atg atc cgg tat aag gtt     1682
Ala Ser Val Leu Val Phe Ile Ile Ile Leu Met Ile Arg Tyr Lys Val

540                 545                 550

tgc aac aat aat ggg caa cac aag gtc acc aag gtt agc aat gtt tat    1730
Cys Asn Asn Asn Gly Gln His Lys Val Thr Lys Val Ser Asn Val Tyr
555                 560                 565                 570

tcc caa act aac ggg gct caa ata caa ggc tgt agt gta acg ctg ccc    1778
Ser Gln Thr Asn Gly Ala Gln Ile Gln Gly Cys Ser Val Thr Leu Pro
                575                 580                 585

cag tcc gtg tcc aaa caa gct gtg gga cac gaa gag att gcc cag tgt    1826
Gln Ser Val Ser Lys Gln Ala Val Gly His Glu Glu Ile Ala Gln Cys
            590                 595                 600

tgt aaa gct acc agt gac aat gtg att caa tct tca gaa act tgt tcg    1874
Cys Lys Ala Thr Ser Asp Asn Val Ile Gln Ser Ser Glu Thr Cys Ser
        605                 610                 615

agt cag gac tcc tct acc act acc tct gct ttg cct cct tcc tgg act    1922
Ser Gln Asp Ser Ser Thr Thr Thr Ser Ala Leu Pro Pro Ser Trp Thr
    620                 625                 630

tca agc act tct gtg tcc caa aag cag aaa aga aag act ggc aca aag    1970
Ser Ser Thr Ser Val Ser Gln Lys Gln Lys Arg Lys Thr Gly Thr Lys
635                 640                 645                 650

cca agt aca gaa cca cag aat gaa gcc gtc aca aat gtt gaa tcc caa    2018
Pro Ser Thr Glu Pro Gln Asn Glu Ala Val Thr Asn Val Glu Ser Gln
                655                 660                 665

aac act aac agg aac aac tca act gcc ttg cag tta gct agc cgt cct    2066
Asn Thr Asn Arg Asn Asn Ser Thr Ala Leu Gln Leu Ala Ser Arg Pro
            670                 675                 680

ccc gat tct gtc aca gag ggg ccc acg tct aaa aga gca cat ata aag    2114
Pro Asp Ser Val Thr Glu Gly Pro Thr Ser Lys Arg Ala His Ile Lys
        685                 690                 695

cca aat gct ttg ctg act aat gtt gac cag att gtc cag gaa aca cag    2162
Pro Asn Ala Leu Leu Thr Asn Val Asp Gln Ile Val Gln Glu Thr Gln
    700                 705                 710

agg ctg gag tta atc tgaagagcac cacttctcct ctctctcctg aaaaaatttg    2217
Arg Leu Glu Leu Ile
715

ccactgatat ttttactgga taaaattcaa aaatgtttca attcacaaag    2267

gctaattgtt gaactggtgt cgtagaagaa attgtctaca ggagccaagg    2317

tgaaagtctc tgatgacggc ggaactggct ccattagacc atggttcatc    2367

ctcttttaaa aacaaatttt t                                                2388

<210>82
<211>719
<212>PRT
<213>Homo sapiens

<400>82

Met Glu Lys Ile Leu Phe Tyr Leu Phe Leu Ile Gly Ile Ala Val Lys
 1               5                  10                  15

Ala Gln Ile Cys Pro Lys Arg Cys Val Cys Gln Ile Leu Ser Pro Asn
            20                  25                  30

Leu Ala Thr Leu Cys Ala Lys Lys Gly Leu Leu Phe Val Pro Pro Asn
        35                  40                  45

Ile Asp Arg Arg Thr Val Glu Leu Arg Leu Ala Asp Asn Phe Val Thr
    50                  55                  60

Asn Ile Lys Arg Lys Asp Phe Ala Asn Met Thr Ser Leu Val Asp Leu
65                  70                  75                  80

Thr Leu Ser Arg Asn Thr Ile Ser Phe Ile Thr Pro His Ala Phe Ala
                85                  90                  95

Asp Leu Arg Asn Leu Arg Ala Leu His Leu Asn Ser Asn Arg Leu Thr
            100                 105                 110

Lys Ile Thr Asn Asp Met Phe Ser Gly Leu Ser Asn Leu His His Leu
        115                 120                 125

Ile Leu Asn Asn Asn Gln Leu Thr Leu Ile Ser Ser Thr Ala Phe Asp
    130                 135                 140

Asp Val Phe Ala Leu Glu Glu Leu Asp Leu Ser Tyr Asn Asn Leu Glu
145                 150                 155                 160

Thr Ile Pro Trp Asp Ala Val Glu Lys Met Val Ser Leu His Thr Leu
                165                 170                 175

Ser Leu Asp His Asn Met Ile Asp Asn Ile Pro Lys Gly Thr Phe Ser
            180                 185                 190

His Leu His Lys Met Thr Arg Leu Asp Val Thr Ser Asn Lys Leu Gln
        195                 200                 205

Lys Leu Pro Pro Asp Pro Leu Phe Gln Arg Ala Gln Val Leu Ala Thr
    210                 215                 220

Ser Gly Ile Ile Ser Pro Ser Thr Phe Ala Leu Ser Phe Gly Gly Asn
225                 230                 235                 240

Pro Leu His Cys Asn Cys Glu Leu Leu Trp Leu Arg Arg Leu Ser Arg
                245                 250                 255

Glu Asp Asp Leu Glu Thr Cys Ala Ser Pro Pro Leu Leu Thr Gly Arg
            260                 265                 270

Tyr Phe Trp Ser Ile Pro Glu Glu Glu Phe Leu Cys Glu Pro Pro Leu
        275                 280                 285

Ile Thr Arg His Thr His Glu Met Arg Val Leu Glu Gly Gln Arg Ala
    290                 295                 300

Thr Leu Arg Cys Lys Ala Arg Gly Asp Pro Glu Pro Ala Ile His Trp
305                 310                 315                 320

Ile Ser Pro Glu Gly Lys Leu Ile Ser Asn Ala Thr Arg Ser Leu Val
                325                 330                 335

Tyr Asp Asn Gly Thr Leu Asp Ile Leu Ile Thr Thr Val Lys Asp Thr
            340                 345                 350

Gly Ala Phe Thr Cys Ile Ala Ser Asn Pro Ala Gly Glu Ala Thr Gln
        355                 360                 365

Ile Val Asp Leu His Ile Ile Lys Leu Pro His Leu Leu Asn Ser Thr
    370                 375                 380

Asn His Ile His Glu Pro Asp Pro Gly Ser Ser Asp Ile Ser Thr Ser
385                 390                 395                 400

Thr Lys Ser Gly Ser Asn Thr Ser Ser Ser Asn Gly Asp Thr Lys Leu
                405                 410                 415

Ser Gln Asp Lys Ile Val Val Ala Glu Ala Thr Ser Ser Thr Ala Leu
            420                 425                 430

Leu Asn Phe Thr Phe Gln Arg Thr Ile Pro Gly Ile Arg Met Phe Gln
        435                 440                 445

Ile Gln Tyr Asn Gly Thr Tyr Asp Asp Thr Leu Val Tyr Arg Met Ile
    450                 455                 460

Pro Pro Thr Ser Lys Thr Phe Leu Val Asn Asn Leu Ala Ala Gly Thr
465                 470                 475                 480

Met Tyr Asp Leu Cys Val Leu Ala Ile Tyr Asp Asp Gly Ile Thr Ser
                485                 490                 495

Leu Thr Ala Thr Arg Val Val Gly Cys Ile Gln Phe Thr Thr Glu Gln
            500                 505                 510

Asp Tyr Val Arg Cys His Phe Met Gln Ser Gln Phe Leu Gly Gly Thr
        515                 520                 525

Met Ile Ile Ile Ile Gly Gly Ile Ile Val Ala Ser Val Leu Val Phe
    530                 535                 540

Ile Ile Ile Leu Met Ile Arg Tyr Lys Val Cys Asn Asn Asn Gly Gln
545                 550                 555                 560

His Lys Val Thr Lys Val Ser Asn Val Tyr Ser Gln Thr Asn Gly Ala
                565                 570                 575

Gln Ile Gln Gly Cys Ser Val Thr Leu Pro Gln Ser Val Ser Lys Gln
            580                 585                 590

Ala Val Gly His Glu Glu Ile Ala Gln Cys Cys Lys Ala Thr Ser Asp
        595                 600                 605

Asn Val Ile Gln Ser Ser Glu Thr Cys Ser Ser Gln Asp Ser Ser Thr
    610                 615                 620

Thr Thr Ser Ala Leu Pro Pro Ser Trp Thr Ser Ser Thr Ser Val Ser
625                 630                 635                 640

Gln Lys Gln Lys Arg Lys Thr Gly Thr Lys Pro Ser Thr Glu Pro Gln
                645                 650                 655

Asn Glu Ala Val Thr Asn Val Glu Ser Gln Asn Thr Asn Arg Asn Asn
            660                 665                 670

Ser Thr Ala Leu Gln Leu Ala Ser Arg Pro Pro Asp Ser Val Thr Glu
        675                 680                 685

Gly Pro Thr Ser Lys Arg Ala His Ile Lys Pro Asn Ala Leu Leu Thr
    690                 695                 700

Asn Val Asp Gln Ile Val Gln Glu Thr Gln Arg Leu Glu Leu Ile
705                 710                 715

<210>83
<211>1545
<212>DNA
<213>Homo sapiens

<220>
<221> CDS
<222>(1)..(1545)

<400>83

gga tcc cag atc tgt cca aag cgt tgt gtc tgt cag att ttg tct cct    48
Gly Ser Gln Ile Cys Pro Lys Arg Cys Val Cys Gln Ile Leu Ser Pro
  1               5                  10                  15

aat ctt gca acc ctt tgt gcc aag aaa ggg ctt tta ttt gtt cca cca    96
Asn Leu Ala Thr Leu Cys Ala Lys Lys Gly Leu Leu Phe Val Pro Pro
             20                  25                  30

aac att gac aga aga act gtg gaa ctg cgg ttg gca gac aat ttt gtt    144
Asn Ile Asp Arg Arg Thr Val Glu Leu Arg Leu Ala Asp Asn Phe Val
         35                  40                  45

aca aat att aaa agg aaa gat ttt gcc aat atg acc agc ttg gtg gac    192

Thr Asn Ile Lys Arg Lys Asp Phe Ala Asn Met Thr Ser Leu Val Asp
    50                  55                  60

ctg act cta tcc agg aat aca ata agt ttt att aca cct cat gct ttc 240
Leu Thr Leu Ser Arg Asn Thr Ile Ser Phe Ile Thr Pro His Ala Phe
65                  70                  75                  80

gct gac cta cga aat ttg agg gct ttg cat ttg aat agc aac aga ttg 288
Ala Asp Leu Arg Asn Leu Arg Ala Leu His Leu Asn Ser Asn Arg Leu
                85                  90                  95

act aaa att aca aat gat atg ttc agt ggt ctt tcc aat ctt cat cat 336
Thr Lys Ile Thr Asn Asp Met Phe Ser Gly Leu Ser Asn Leu His His
            100                 105                 110

ttg ata ctg aac aac aat cag ctg act tta att tcc tct aca gcg ttt 384
Leu Ile Leu Asn Asn Asn Gln Leu Thr Leu Ile Ser Ser Thr Ala Phe
        115                 120                 125

gat gat gtc ttc gcc ctt gag gag ctg gat ctg tcc tat aat aat cta 432
Asp Asp Val Phe Ala Leu Glu Glu Leu Asp Leu Ser Tyr Asn Asn Leu
    130                 135                 140

gaa acc att cct tgg gat gct gtt gag aag atg gtt agc ttg cat acc 480
Glu Thr Ile Pro Trp Asp Ala Val Glu Lys Met Val Ser Leu His Thr
145                 150                 155                 160

ctt agt ttg gat cac aat atg att gat aac att cct aag ggg acc ttc 528
Leu Ser Leu Asp His Asn Met Ile Asp Asn Ile Pro Lys Gly Thr Phe
                165                 170                 175

tcc cat ttg cac aag atg act cgg tta gat gtg aca tca aat aaa ttg 576
Ser His Leu His Lys Met Thr Arg Leu Asp Val Thr Ser Asn Lys Leu
            180                 185                 190

cag aag cta cca cct gac cct ctc ttt cag cga gct cag gta cta gca 624
Gln Lys Leu Pro Pro Asp Pro Leu Phe Gln Arg Ala Gln Val Leu Ala
        195                 200                 205

acc tca gga atc ata agc cca tct act ttt gca tta agt ttt ggt gga 672
Thr Ser Gly Ile Ile Ser Pro Ser Thr Phe Ala Leu Ser Phe Gly Gly
    210                 215                 220

aac ccc ttg cat tgc aat tgt gaa ttg ttg tgg ttg agg cgt ctg tcc 720
Asn Pro Leu His Cys Asn Cys Glu Leu Leu Trp Leu Arg Arg Leu Ser
225                 230                 235                 240

aga gaa gat gac tta gag acc tgt gct tct cct cca ctt tta act ggc 768
Arg Glu Asp Asp Leu Glu Thr Cys Ala Ser Pro Pro Leu Leu Thr Gly
                245                 250                 255

cgc tac ttt tgg tca att cct gaa gaa gag ttt ttg tgt gag cct cct 816
Arg Tyr Phe Trp Ser Ile Pro Glu Glu Glu Phe Leu Cys Glu Pro Pro
            260                 265                 270

ctc att act cgt cat aca cat gag atg aga gtc ctg gag gga caa agg 864
Leu Ile Thr Arg His Thr His Glu Met Arg Val Leu Glu Gly Gln Arg

        275                 280                 285

gca aca ctg agg tgc aaa gcc agg gga gac cct gag cct gca att cac 912
Ala Thr Leu Arg Cys Lys Ala Arg Gly Asp Pro Glu Pro Ala Ile His
    290                 295                 300

tgg att tct cct gaa ggg aag ctt att tca aat gca aca aga tct ctg 960
Trp Ile Ser Pro Glu Gly Lys Leu Ile Ser Asn Ala Thr Arg Ser Leu
305                 310                 315                 320

gtg tat gat aac gga aca ctt gac att ctt atc aca act gta aag gat 1008
Val Tyr Asp Asn Gly Thr Leu Asp Ile Leu Ile Thr Thr Val Lys Asp
                325                 330                 335

aca ggt gct ttt acc tgc att gct tcc aat cct gct ggg gaa gca aca 1056
Thr Gly Ala Phe Thr Cys Ile Ala Ser Asn Pro Ala Gly Glu Ala Thr
            340                 345                 350

caa ata gtg gat ctt cat ata att aag ctc cct cac tta cta aat agt 1104
Gln Ile Val Asp Leu His Ile Ile Lys Leu Pro His Leu Leu Asn Ser
        355                 360                 365

aca aac cat atc cat gag cct gat cct ggt tct tca gat atc tca act 1152
Thr Asn His Ile His Glu Pro Asp Pro Gly Ser Ser Asp Ile Ser Thr
    370                 375                 380

tct acc aag tca ggt tct aat aca agc agt agt aat ggt gat act aaa 1200
Ser Thr Lys Ser Gly Ser Asn Thr Ser Ser Ser Asn Gly Asp Thr Lys
385                 390                 395                 400

ttg agt caa gat aaa att gtg gtg gca gaa gct aca tca tca acg gca 1248
Leu Ser Gln Asp Lys Ile Val Val Ala Glu Ala Thr Ser Ser Thr Ala
                405                 410                 415

cta ctt aaa ttt aat ttt cga aga aat atc cct gga ata cgt atg ttt 1296
Leu Leu Lys Phe Asn Phe Arg Arg Asn Ile Pro Gly Ile Arg Met Phe
            420                 425                 430

caa atc cag tac aat ggt act tat gat gac acc ctt gtt tac aga atg 1344
Gln Ile Gln Tyr Asn Gly Thr Tyr Asp Asp Thr Leu Val Tyr Arg Met
        435                 440                 445

ata cct cct acg agc aaa act ttt ctg gtc aat aat ctg gct gct gga 1392
Ile Pro Pro Thr Ser Lys Thr Phe Leu Val Asn Asn Leu Ala Ala Gly
    450                 455                 460

act atg tat gac ttg tgt gtc ttg gcc ata tat gat gat ggc atc act 1440
Thr Met Tyr Asp Leu Cys Val Leu Ala Ile Tyr Asp Asp Gly Ile Thr
465                 470                 475                 480

tcc ctc act gcc aca aga gtc gtg ggt tgc atc cag ttt act acg gaa 1488
Ser Leu Thr Ala Thr Arg Val Val Gly Cys Ile Gln Phe Thr Thr Glu
                485                 490                 495

cag gat tat gtg cgt tgc cat ttc atg cag tcc cag ttt ttg gga ggc 1536
Gln Asp Tyr Val Arg Cys His Phe Met Gln Ser Gln Phe Leu Gly Gly
            500                 505                 510

acc ctc gag                                                          1545
Thr Leu Glu
        515

<210>84
<211>515
<212>PRT
<213>Homo sapiens

<400>84

Gly Ser Gln Ile Cys Pro Lys Arg Cys Val Cys Gln Ile Leu Ser Pro
  1               5                  10                  15

Asn Leu Ala Thr Leu Cys Ala Lys Lys Gly Leu Leu Phe Val Pro Pro
             20                  25                  30

Asn Ile Asp Arg Arg Thr Val Glu Leu Arg Leu Ala Asp Asn Phe Val
         35                  40                  45

Thr Asn Ile Lys Arg Lys Asp Phe Ala Asn Met Thr Ser Leu Val Asp
     50                  55                  60

Leu Thr Leu Ser Arg Asn Thr Ile Ser Phe Ile Thr Pro His Ala Phe
 65                  70                  75                  80

Ala Asp Leu Arg Asn Leu Arg Ala Leu His Leu Asn Ser Asn Arg Leu
                 85                  90                  95

Thr Lys Ile Thr Asn Asp Met Phe Ser Gly Leu Ser Asn Leu His His
            100                 105                 110

Leu Ile Leu Asn Asn Asn Gln Leu Thr Leu Ile Ser Ser Thr Ala Phe
        115                 120                 125

Asp Asp Val Phe Ala Leu Glu Glu Leu Asp Leu Ser Tyr Asn Asn Leu
    130                 135                 140

Glu Thr Ile Pro Trp Asp Ala Val Glu Lys Met Val Ser Leu His Thr
145                 150                 155                 160

Leu Ser Leu Asp His Asn Met Ile Asp Asn Ile Pro Lys Gly Thr Phe
                165                 170                 175

Ser His Leu His Lys Met Thr Arg Leu Asp Val Thr Ser Asn Lys Leu
            180                 185                 190

Gln Lys Leu Pro Pro Asp Pro Leu Phe Gln Arg Ala Gln Val Leu Ala
        195                 200                 205

Thr Ser Gly Ile Ile Ser Pro Ser Thr Phe Ala Leu Ser Phe Gly Gly
    210                 215                 220

Asn Pro Leu His Cys Asn Cys Glu Leu Leu Trp Leu Arg Arg Leu Ser
225                 230                 235                 240

Arg Glu Asp Asp Leu Glu Thr Cys Ala Ser Pro Pro Leu Leu Thr Gly
                245                 250                 255

Arg Tyr Phe Trp Ser Ile Pro Glu Glu Glu Phe Leu Cys Glu Pro Pro
            260                 265                 270

Leu Ile Thr Arg His Thr His Glu Met Arg Val Leu Glu Gly Gln Arg
        275                 280                 285

Ala Thr Leu Arg Cys Lys Ala Arg Gly Asp Pro Glu Pro Ala Ile His
    290                 295                 300

Trp Ile Ser Pro Glu Gly Lys Leu Ile Ser Asn Ala Thr Arg Ser Leu
305                 310                 315                 320

Val Tyr Asp Asn Gly Thr Leu Asp Ile Leu Ile Thr Thr Val Lys Asp
                325                 330                 335

Thr Gly Ala Phe Thr Cys Ile Ala Ser Asn Pro Ala Gly Glu Ala Thr
            340                 345                 350

Gln Ile Val Asp Leu His Ile Ile Lys Leu Pro His Leu Leu Asn Ser
        355                 360                 365

Thr Asn His Ile His Glu Pro Asp Pro Gly Ser Ser Asp Ile Ser Thr
    370                 375                 380

Ser Thr Lys Ser Gly Ser Asn Thr Ser Ser Ser Asn Gly Asp Thr Lys
385                 390                 395                 400

Leu Ser Gln Asp Lys Ile Val Val Ala Glu Ala Thr Ser Ser Thr Ala
                405                 410                 415

Leu Leu Lys Phe Asn Phe Arg Arg Asn Ile Pro Gly Ile Arg Met Phe
            420                 425                 430

Gln Ile Gln Tyr Asn Gly Thr Tyr Asp Asp Thr Leu Val Tyr Arg Met
        435                 440                 445

Ile Pro Pro Thr Ser Lys Thr Phe Leu Val Asn Asn Leu Ala Ala Gly
    450                 455                 460

Thr Met Tyr Asp Leu Cys Val Leu Ala Ile Tyr Asp Asp Gly Ile Thr
465                 470                 475                 480

Ser Leu Thr Ala Thr Arg Val Val Gly Cys Ile Gln Phe Thr Thr Glu
                485                 490                 495

Gln Asp Tyr Val Arg Cys His Phe Met Gln Ser Gln Phe Leu Gly Gly
            500                 505                 510

Thr Leu Glu
        515

<210>85
<211>1545
<212>DNA

<213>Homo sapiens

<220>
<221> CDS
<222>(1)..(1545)

<400>85

gga tcc cag atc tgt cca aag cgt tgt gtc tgt cag att ttg tct cct    48
Gly Ser Gln Ile Cys Pro Lys Arg Cys Val Cys Gln Ile Leu Ser Pro
1               5                   10                  15

aat ctt gca acc ctt tgt gcc aag aaa ggg ctt tta ttt gtt cca cca    96
Asn Leu Ala Thr Leu Cys Ala Lys Lys Gly Leu Leu Phe Val Pro Pro
            20                  25                  30

aac att gac aga aga act gtg gaa ctg cgg ttg gca gac aat ttt gtt    144
Asn Ile Asp Arg Arg Thr Val Glu Leu Arg Leu Ala Asp Asn Phe Val
        35                  40                  45

aca aat att aaa agg aaa gat ttt gcc aat atg acc agc ttg gtg gac    192
Thr Asn Ile Lys Arg Lys Asp Phe Ala Asn Met Thr Ser Leu Val Asp
    50                  55                  60

ctg act cta tcc agg aat aca ata agt ttt att aca cct cat gct ttc    240
Leu Thr Leu Ser Arg Asn Thr Ile Ser Phe Ile Thr Pro His Ala Phe
65                  70                  75                  80

gct gac cta cga aat ttg agg gct ttg cat ttg aat agc aac aga ttg    288
Ala Asp Leu Arg Asn Leu Arg Ala Leu His Leu Asn Ser Asn Arg Leu
                85                  90                  95

act aaa att aca aat gat atg ttc agt ggt ctt tcc aat ctt cat cat    336
Thr Lys Ile Thr Asn Asp Met Phe Ser Gly Leu Ser Asn Leu His His
            100                 105                 110

ttg ata, ctg aac aac aat cag ctg act tta att tcc tct aca gcg ttt    384
Leu Ile Leu Asn Asn Asn Gln Leu Thr Leu Ile Ser Ser Thr Ala Phe
        115                 120                 125

gat gat gtc ttc acc ctt gag gag ctg gat ctg tcc tat aat aat cta    432
Asp Asp Val Phe Thr Leu Glu Glu Leu Asp Leu Ser Tyr Asn Asn Leu
    130                 135                 140

gaa acc att cct tgg gat gct gtt gag aag atg gtt agc ttg cat acc    480
Glu Thr Ile Pro Trp Asp Ala Val Glu Lys Met Val Ser Leu His Thr
145                 150                 155                 160

ctt agt ttg gat cac aat atg att gat aac att cct aag ggg acc ttc    528
Leu Ser Leu Asp His Asn Met Ile Asp Asn Ile Pro Lys Gly Thr Phe
                165                 170                 175

tcc cat ttg cac aag atg act cgg tta gat gtg aca tca aat aaa ttg    576
Ser His Leu His Lys Met Thr Arg Leu Asp Val Thr Ser Asn Lys Leu
            180                 185                 190

cag aag cta cca cct gac cct ctc ttt cag cga gct cag gta cta gca    624

Gln Lys Leu Pro Pro Asp Pro Leu Phe Gln Arg Ala Gln Val Leu Ala
        195                 200                 205

acc tca gga atc ata agc cca tct act ttt gca tta agt ttt ggt gga 672
Thr Ser Gly Ile Ile Ser Pro Ser Thr Phe Ala Leu Ser Phe Gly Gly
    210                 215                 220

aac ccc ttg cat tgc aat tgt gaa ttg ttg tgg ttg agg cgt ctg tcc 720
Asn Pro Leu His Cys Asn Cys Glu Leu Leu Trp Leu Arg Arg Leu Ser
225                 230                 235                 240

aga gaa gat gac tta gag acc tgt gct tct cct cca ctt tta act ggc 768
Arg Glu Asp Asp Leu Glu Thr Cys Ala Ser Pro Pro Leu Leu Thr Gly
                245                 250                 255

cgc tac ttt tgg tca att cct gaa gaa gag ttt ttg tgt gag cct cct 816
Arg Tyr Phe Trp Ser Ile Pro Glu Glu Glu Phe Leu Cys Glu Pro Pro
            260                 265                 270

ctc att act cgt cat aca cat gag atg aga gtc ctg gag gga caa agg 864
Leu Ile Thr Arg His Thr His Glu Met Arg Val Leu Glu Gly Gln Arg
        275                 280                 285

gca aca ctg agg tgc aaa gcc agg gga gac cct gag cct gca att cac 912
Ala Thr Leu Arg Cys Lys Ala Arg Gly Asp Pro Glu Pro Ala Ile His
    290                 295                 300

tgg att tct cct gaa ggg aag ctt att tca aat gca aca aga tct ctg 960
Trp Ile Ser Pro Glu Gly Lys Leu Ile Ser Asn Ala Thr Arg Ser Leu
305                 310                 315                 320

gtg tat gat aac gga aca ctt gac att ctt atc aca act gta aag gat 1008
Val Tyr Asp Asn Gly Thr Leu Asp Ile Leu Ile Thr Thr Val Lys Asp
                325                 330                 335

aca ggt gct ttt acc tgc att gct tcc aat cct gct ggg gaa gca aca 1056
Thr Gly Ala Phe Thr Cys Ile Ala Ser Asn Pro Ala Gly Glu Ala Thr
            340                 345                 350

caa ata gtg gat ctt cat ata att aag ctc cct cac tta cta aat agt 1104
Gln Ile Val Asp Leu His Ile Ile Lys Leu Pro His Leu Leu Asn Ser
        355                 360                 365

aca aac cat atc cat gag cct gat cct ggt tct tca gat atc tca act 1152
Thr Asn His Ile His Glu Pro Asp Pro Gly Ser Ser Asp Ile Ser Thr
    370                 375                 380

tct acc aag tca ggt tct aat aca agc agt agt aat ggt gat act aaa 1200
Ser Thr Lys Ser Gly Ser Asn Thr Ser Ser Ser Asn Gly Asp Thr Lys
385                 390                 395                 400

ttg agt caa gat aaa att gtg gtg gca gaa gct aca tca tca acg gca 1248
Leu Ser Gln Asp Lys Ile Val Val Ala Glu Ala Thr Ser Ser Thr Ala
                405                 410                 415

cta ctt aaa ttt aat ttt caa aga aat atc cct gga ata cgt atg ttt 1296
Leu Leu Lys Phe Asn Phe Gln Arg Asn Ile Pro Gly Ile Arg Met Phe

            420                 425                 430

caa atc cag tac aat ggt act tat gat gac acc ctt gtt tac aga atg 1344
Gln Ile Gln Tyr Asn Gly Thr Tyr Asp Asp Thr Leu Val Tyr Arg Met
        435                 440                 445

ata cct cct acg agc aaa act ttt ctg gtc aat aat ctg gct gct gga 1392
Ile Pro Pro Thr Ser Lys Thr Phe Leu Val Asn Asn Leu Ala Ala Gly
    450                 455                 460

act atg tat gac ttg tgt gtc ttg gcc ata tat gat gat ggc atc act 1440
Thr Met Tyr Asp Leu Cys Val Leu Ala Ile Tyr Asp Asp Gly Ile Thr
465                 470                 475                 480

tcc ctc act gcc aca aga gtc gtg ggt tgc atc cag ttt act acg gaa 1488
Ser Leu Thr Ala Thr Arg Val Val Gly Cys Ile Gln Phe Thr Thr Glu
                485                 490                 495

cag gat tat gtg cgt tgc cat ttc atg cag tcc cag ttt ttg gga ggc 1536
Gln Asp Tyr Val Arg Cys His Phe Met Gln Ser Gln Phe Leu Gly Gly
            500                 505                 510

acc ctc gag 1545
Thr Leu Glu
        515

<210>86
<211>515
<212>PRT
<213>Homo sapiens

<400>86

Gly Ser Gln Ile Cys Pro Lys Arg Cys Val Cys Gln Ile Leu Ser Pro
1               5                   10                  15

Asn Leu Ala Thr Leu Cys Ala Lys Lys Gly Leu Leu Phe Val Pro Pro
            20                  25                  30

Asn Ile Asp Arg Arg Thr Val Glu Leu Arg Leu Ala Asp Asn Phe Val
        35                  40                  45

Thr Asn Ile Lys Arg Lys Asp Phe Ala Asn Met Thr Ser Leu Val Asp
    50                  55                  60

Leu Thr Leu Ser Arg Asn Thr Ile Ser Phe Ile Thr Pro His Ala Phe
65                  70                  75                  80

Ala Asp Leu Arg Asn Leu Arg Ala Leu His Leu Asn Ser Asn Arg Leu
                85                  90                  95

Thr Lys Ile Thr Asn Asp Met Phe Ser Gly Leu Ser Asn Leu His His
            100                 105                 110

Leu Ile Leu Asn Asn Asn Gln Leu Thr Leu Ile Ser Ser Thr Ala Phe
        115                 120                 125

Asp Asp Val Phe Thr Leu Glu Glu Leu Asp Leu Ser Tyr Asn Asn Leu
    130                 135                 140

Glu Thr Ile Pro Trp Asp Ala Val Glu Lys Met Val Ser Leu His Thr
145                 150                 155                 160

Leu Ser Leu Asp His Asn Met Ile Asp Asn Ile Pro Lys Gly Thr Phe
                165                 170                 175

Ser His Leu His Lys Met Thr Arg Leu Asp Val Thr Ser Asn Lys Leu
            180                 185                 190

Gln Lys Leu Pro Pro Asp Pro Leu Phe Gln Arg Ala Gln Val Leu Ala
        195                 200                 205

Thr Ser Gly Ile Ile Ser Pro Ser Thr Phe Ala Leu Ser Phe Gly Gly
    210                 215                 220

Asn Pro Leu His Cys Asn Cys Glu Leu Leu Trp Leu Arg Arg Leu Ser
225                 230                 235                 240

Arg Glu Asp Asp Leu Glu Thr Cys Ala Ser Pro Pro Leu Leu Thr Gly
                245                 250                 255

Arg Tyr Phe Trp Ser Ile Pro Glu Glu Glu Phe Leu Cys Glu Pro Pro
            260                 265                 270

Leu Ile Thr Arg His Thr His Glu Met Arg Val Leu Glu Gly Gln Arg
        275                 280                 285

Ala Thr Leu Arg Cys Lys Ala Arg Gly Asp Pro Glu Pro Ala Ile His
    290                 295                 300

Trp Ile Ser Pro Glu Gly Lys Leu Ile Ser Asn Ala Thr Arg Ser Leu
305                 310                 315                 320

Val Tyr Asp Asn Gly Thr Leu Asp Ile Leu Ile Thr Thr Val Lys Asp
                325                 330                 335

Thr Gly Ala Phe Thr Cys Ile Ala Ser Asn Pro Ala Gly Glu Ala Thr
            340                 345                 350

Gln Ile Val Asp Leu His Ile Ile Lys Leu Pro His Leu Leu Asn Ser
        355                 360                 365

Thr Asn His Ile His Glu Pro Asp Pro Gly Ser Ser Asp Ile Ser Thr
    370                 375                 380

Ser Thr Lys Ser Gly Ser Asn Thr Ser Ser Ser Asn Gly Asp Thr Lys
385                 390                 395                 400

Leu Ser Gln Asp Lys Ile Val Val Ala Glu Ala Thr Ser Ser Thr Ala
                405                 410                 415

Leu Leu Lys Phe Asn Phe Gln Arg Asn Ile Pro Gly Ile Arg Met Phe
            420                 425                 430

Gln Ile Gln Tyr Asn Gly Thr Tyr Asp Asp Thr Leu Val Tyr Arg Met
        435                 440                 445

Ile Pro Pro Thr Ser Lys Thr Phe Leu Val Asn Asn Leu Ala Ala Gly
    450                 455                 460

Thr Met Tyr Asp Leu Cys Val Leu Ala Ile Tyr Asp Asp Gly Ile Thr
465                 470                 475                 480

Ser Leu Thr Ala Thr Arg Val Val Gly Cys Ile Gln Phe Thr Thr Glu
                485                 490                 495

Gln Asp Tyr Val Arg Cys His Phe Met Gln Ser Gln Phe Leu Gly Gly
            500                 505                 510

Thr Leu Glu
        515

<210>87
<211>1545
<212>DNA
<213>Homo sapiens

<220>
<221> CDS
<222>(1)..(1545)

<400>87

gga tcc cag atc tgt cca aag cgt tgt gtc tgt cag att ttg tct cct 48
Gly Ser Gln Ile Cys Pro Lys Arg Cys Val Cys Gln Ile Leu Ser Pro
1               5                   10                  15

aat ctt gca acc ctt tgt gcc aag aaa ggg ctt tta ttt gtt cca cca 96
Asn Leu Ala Thr Leu Cys Ala Lys Lys Gly Leu Leu Phe Val Pro Pro
            20                  25                  30

aac att gac aga aga act gtg gaa ctg cgg ttg gca gac aat ttt gtt 144
Asn Ile Asp Arg Arg Thr Val Glu Leu Arg Leu Ala Asp Asn Phe Val
        35                  40                  45

aca aat att aaa agg aaa gat ttt gcc aat atg acc agc ttg gtg gac 192
Thr Asn Ile Lys Arg Lys Asp Phe Ala Asn Met Thr Ser Leu Val Asp
    50                  55                  60

ctg act cta tcc agg aat aca ata agt ttt att aca cct cat gct ttc 240
Leu Thr Leu Ser Arg Asn Thr Ile Ser Phe Ile Thr Pro His Ala Phe
65                  70                  75                  80

gct gac cta cga aat ttg agg gct ttg cat ttg aat agc aac aga ttg 288
Ala Asp Leu Arg Asn Leu Arg Ala Leu His Leu Asn Ser Asn Arg Leu
                85                  90                  95

act aaa att aca aat gat atg ttc agt ggt ctt tcc aat ctt cat cat 336
Thr Lys Ile Thr Asn Asp Met Phe Ser Gly Leu Ser Asn Leu His His
            100                 105                 110

ttg ata ctg aac aac aat cag ctg act tta att tcc tct aca gcg ttt 384
Leu Ile Leu Asn Asn Asn Gln Leu Thr Leu Ile Ser Ser Thr Ala Phe
        115                 120                 125

gat gat gtc ttc acc ctt gag gag ctg gat ctg tcc tat aat aat cta 432
Asp Asp Val Phe Thr Leu Glu Glu Leu Asp Leu Ser Tyr Asn Asn Leu
    130                 135                 140

gaa acc att cct tgg gat gct gtt gag aag atg gtt agc ttg cat acc 480
Glu Thr Ile Pro Trp Asp Ala Val Glu Lys Met Val Ser Leu His Thr
145                 150                 155                 160

ctt agt ttg gat cac aat atg att gat aac att cct aag ggg acc ttc 528
Leu Ser Leu Asp His Asn Met Ile Asp Asn Ile Pro Lys Gly Thr Phe
                165                 170                 175

tcc cat ttg cac aag atg act cgg tta gat gtg aca tca aat aaa ttg 576
Ser His Leu His Lys Met Thr Arg Leu Asp Val Thr Ser Asn Lys Leu
            180                 185                 190

cag aag cta cca cct gac cct ctc ttt cag cga gct cag gta cta gca 624
Gln Lys Leu Pro Pro Asp Pro Leu Phe Gln Arg Ala Gln Val Leu Ala
        195                 200                 205

acc tca gga atc ata agc cca tct act ttt gca tta agt ttt ggt gga 672
Thr Ser Gly Ile Ile Ser Pro Ser Thr Phe Ala Leu Ser Phe Gly Gly
    210                 215                 220

aac ccc ttg cat tgc aat tgt gaa ttg ttg tgg ttg agg cgt ctg tcc 720
Asn Pro Leu His Cys Asn Cys Glu Leu Leu Trp Leu Arg Arg Leu Ser
225                 230                 235                 240

aga gaa gat gac tta gag acc tgt gct tct cct cca ctt tta act ggc 768
Arg Glu Asp Asp Leu Glu Thr Cys Ala Ser Pro Pro Leu Leu Thr Gly
                245                 250                 255

cgc tac ttt tgg tca att cct gaa gaa gag ttt ttg tgt gag cct cct 816
Arg Tyr Phe Trp Ser Ile Pro Glu Glu Glu Phe Leu Cys Glu Pro Pro
            260                 265                 270

ctc att act cgt cat aca cat gag atg aga gtc ctg gag gga caa agg 864
Leu Ile Thr Arg His Thr His Glu Met Arg Val Leu Glu Gly Gln Arg
        275                 280                 285

gca aca ctg agg tgc aaa gcc agg gga gac cct gag cct gca att cac 912
Ala Thr Leu Arg Cys Lys Ala Arg Gly Asp Pro Glu Pro Ala Ile His
    290                 295                 300

tgg att tct cct gaa ggg aag ctt att tca aat gca aca aga tct ctg 960
Trp Ile Ser Pro Glu Gly Lys Leu Ile Ser Asn Ala Thr Arg Ser Leu
305                 310                 315                 320

gtg tat gat aac gga aca ctt gac att ctt atc aca act gta aag gat 1008
Val Tyr Asp Asn Gly Thr Leu Asp Ile Leu Ile Thr Thr Val Lys Asp
                325                 330                 335

aca ggt gct ttt acc tgc att gct tcc aat cct gct ggg gaa gca aca 1056

Thr Gly Ala Phe Thr Cys Ile Ala Ser Asn Pro Ala Gly Glu Ala Thr
            340                 345                 350

caa ata gtg gat ctt cat ata att aag ctc cct cac tta cta aat agt 1104
Gln Ile Val Asp Leu His Ile Ile Lys Leu Pro His Leu Leu Asn Ser
        355                 360                 365

aca aac cat atc cat gag cct gat cct ggt tct tca gat atc tca act 1152
Thr Asn His Ile His Glu Pro Asp Pro Gly Ser Ser Asp Ile Ser Thr
    370                 375                 380

tct acc aag tca ggt tct aat aca agc agt agt aat ggt gat act aaa 1200
Ser Thr Lys Ser Gly Ser Asn Thr Ser Ser Ser Asn Gly Asp Thr Lys
385                 390                 395                 400

ttg agt caa gat aaa att gtg gtg gca gaa gct aca tca tca acg gca 1248
Leu Ser Gln Asp Lys Ile Val Val Ala Glu Ala Thr Ser Ser Thr Ala
                405                 410                 415

cta ctt aaa ttt aat ttt caa aga aat atc cct gga ata cgt atg ttt 1296
Leu Leu Lys Phe Asn Phe Gln Arg Asn Ile Pro Gly Ile Arg Met Phe
            420                 425                 430

caa atc cag tac aat ggt act tat gat gac acc ctt gtt cac aga atg 1344
Gln Ile Gln Tyr Asn Gly Thr Tyr Asp Asp Thr Leu Val His Arg Met
        435                 440                 445

ata cct cct acg agc aaa act ttt ctg gtc aat aat ctg gct gct gga 1392
Ile Pro Pro Thr Ser Lys Thr Phe Leu Val Asn Asn Leu Ala Ala Gly
    450                 455                 460

act atg tat gac ttg tgt gtc ttg gcc ata tat gat gat ggc atc act 1440
Thr Met Tyr Asp Leu Cys Val Leu Ala Ile Tyr Asp Asp Gly Ile Thr
465                 470                 475                 480

tcc ctc act gcc aca aga gtc gtg ggt tgc atc cag ttt act acg gaa 1488
Ser Leu Thr Ala Thr Arg Val Val Gly Cys Ile Gln Phe Thr Thr Glu
                485                 490                 495

cag gat tat gtg cgt tgc cat ttc atg cag tcc cag ttt ttg gga ggc 1536
Gln Asp Tyr Val Arg Cys His Phe Met Gln Ser Gln Phe Leu Gly Gly
            500                 505                 510

acc ctc gag 1545
Thr Leu Glu
        515

<210>88
<211>515
<212>PRT
<213>Homo sapiens

<400>88

Gly Ser Gln Ile Cys Pro Lys Arg Cys Val Cys Gln Ile Leu Ser Pro
1               5                   10                  15

Asn Leu Ala Thr Leu Cys Ala Lys Lys Gly Leu Leu Phe Val Pro Pro
            20                  25                  30

Asn Ile Asp Arg Arg Thr Val Glu Leu Arg Leu Ala Asp Asn Phe Val
        35                  40                  45

Thr Asn Ile Lys Arg Lys Asp Phe Ala Asn Met Thr Ser Leu Val Asp
    50                  55                  60

Leu Thr Leu Ser Arg Asn Thr Ile Ser Phe Ile Thr Pro His Ala Phe
65                  70                  75                  80

Ala Asp Leu Arg Asn Leu Arg Ala Leu His Leu Asn Ser Asn Arg Leu
                85                  90                  95

Thr Lys Ile Thr Asn Asp Met Phe Ser Gly Leu Ser Asn Leu His His
            100                 105                 110

Leu Ile Leu Asn Asn Asn Gln Leu Thr Leu Ile Ser Ser Thr Ala Phe
        115                 120                 125

Asp Asp Val Phe Thr Leu Glu Glu Leu Asp Leu Ser Tyr Asn Asn Leu
    130                 135                 140

Glu Thr Ile Pro Trp Asp Ala Val Glu Lys Met Val Ser Leu His Thr
145                 150                 155                 160

Leu Ser Leu Asp His Asn Met Ile Asp Asn Ile Pro Lys Gly Thr Phe
                165                 170                 175

Ser His Leu His Lys Met Thr Arg Leu Asp Val Thr Ser Asn Lys Leu
            180                 185                 190

Gln Lys Leu Pro Pro Asp Pro Leu Phe Gln Arg Ala Gln Val Leu Ala
        195                 200                 205

Thr Ser Gly Ile Ile Ser Pro Ser Thr Phe Ala Leu Ser Phe Gly Gly
    210                 215                 220

Asn Pro Leu His Cys Asn Cys Glu Leu Leu Trp Leu Arg Arg Leu Ser
225                 230                 235                 240

Arg Glu Asp Asp Leu Glu Thr Cys Ala Ser Pro Pro Leu Leu Thr Gly
                245                 250                 255

Arg Tyr Phe Trp Ser Ile Pro Glu Glu Glu Phe Leu Cys Glu Pro Pro
            260                 265                 270

Leu Ile Thr Arg His Thr His Glu Met Arg Val Leu Glu Gly Gln Arg
        275                 280                 285

Ala Thr Leu Arg Cys Lys Ala Arg Gly Asp Pro Glu Pro Ala Ile His
    290                 295                 300

Trp Ile Ser Pro Glu Gly Lys Leu Ile Ser Asn Ala Thr Arg Ser Leu
305                 310                 315                 320

Val Tyr Asp Asn Gly Thr Leu Asp Ile Leu Ile Thr Thr Val Lys Asp
                325                 330                 335

Thr Gly Ala Phe Thr Cys Ile Ala Ser Asn Pro Ala Gly Glu Ala Thr
            340                 345                 350

Gln Ile Val Asp Leu His Ile Ile Lys Leu Pro His Leu Leu Asn Ser
        355                 360                 365

Thr Asn His Ile His Glu Pro Asp Pro Gly Ser Ser Asp Ile Ser Thr
    370                 375                 380

Ser Thr Lys Ser Gly Ser Asn Thr Ser Ser Ser Asn Gly Asp Thr Lys
385                 390                 395                 400

Leu Ser Gln Asp Lys Ile Val Val Ala Glu Ala Thr Ser Ser Thr Ala
                405                 410                 415

Leu Leu Lys Phe Asn Phe Gln Arg Asn Ile Pro Gly Ile Arg Met Phe
            420                 425                 430

Gln Ile Gln Tyr Asn Gly Thr Tyr Asp Asp Thr Leu Val His Arg Met
        435                 440                 445

Ile Pro Pro Thr Ser Lys Thr Phe Leu Val Asn Asn Leu Ala Ala Gly
    450                 455                 460

Thr Met Tyr Asp Leu Cys Val Leu Ala Ile Tyr Asp Asp Gly Ile Thr
465                 470                 475                 480

Ser Leu Thr Ala Thr Arg Val Val Gly Cys Ile Gln Phe Thr Thr Glu
                485                 490                 495

Gln Asp Tyr Val Arg Cys His Phe Met Gln Ser Gln Phe Leu Gly Gly
            500                 505                 510

Thr Leu Glu
        515

<210>89
<211>1220
<212>DNA
<213>Homo sapiens

<220>
<221> CDS
<222>(3)..(1212)

<400>89

tc atg gcg ctg gcg ggc ttg gcc atg ggc tgc atc gac acc gtg gcc 47
Met Ala Leu Ala Gly Leu Ala Met Gly Cys Ile Asp Thr Val Ala
1               5                   10                  15

aac atg cag ctg gta agg atg tac cag aag gac tcg gcc gtc ttc ctc 95
Asn Met Gln Leu Val Arg Met Tyr Gln Lys Asp Ser Ala Val Phe Leu
                20                  25                  30

cag gtg ctc cat ttc ttc gtg ggc ttt ggt gct ctg ctg agc ccc ctt 143
Gln Val Leu His Phe Phe Val Gly Phe Gly Ala Leu Leu Ser Pro Leu
            35                  40                  45

att gct gac cct ttc ctg tct gag gcc aac tgc ttg cct gcc aat agc 191
Ile Ala Asp Pro Phe Leu Ser Glu Ala Asn Cys Leu Pro Ala Asn Ser
        50                  55                  60

acg gcc aac acc acc tcc cga ggc cac ctg ttc cat gtc tcc agg gtg 239
Thr Ala Asn Thr Thr Ser Arg Gly His Leu Phe His Val Ser Arg Val
    65                  70                  75

ctg ggc cag cac cac gta gat gcc aag cct tgg tcc aac cag acg ttc 287
Leu Gly Gln His His Val Asp Ala Lys Pro Trp Ser Asn Gln Thr Phe
80                  85                  90                  95

cca ggg ctg act cca aag gac ggg gca ggg acc cga gtg tcc tat gcc 335
Pro Gly Leu Thr Pro Lys Asp Gly Ala Gly Thr Arg Val Ser Tyr Ala
                100                 105                 110

ttc tgg atc atg gcc ctc atc aat ctt cca gtg ccc atg gct gtg ctg 383
Phe Trp Ile Met Ala Leu Ile Asn Leu Pro Val Pro Met Ala Val Leu
            115                 120                 125

atg ctg ctg tcc aag gag cgg ctg ctg acc tgc tgt ccc cag agg agg 431
Met Leu Leu Ser Lys Glu Arg Leu Leu Thr Cys Cys Pro Gln Arg Arg
        130                 135                 140

ccc ctg ctt ctg tct gct gat gag ctt gcc ttg gag aca cag cct cct 479
Pro Leu Leu Leu Ser Ala Asp Glu Leu Ala Leu Glu Thr Gln Pro Pro
    145                 150                 155

gag aag gaa gat gcc tcc tca ctg ccc cca aag ttt cag tca cac cta 527
Glu Lys Glu Asp Ala Ser Ser Leu Pro Pro Lys Phe Gln Ser His Leu
160                 165                 170                 175

ggt cat gag gac ctg ttc agc tgc tgc caa agg aag aac ctc aga gga 575
Gly His Glu Asp Leu Phe Ser Cys Cys Gln Arg Lys Asn Leu Arg Gly
                180                 185                 190

gcc cct tat tcc ttc ttt gcc atc cac atc acg ggc gcc ctg gta ctg 623
Ala Pro Tyr Ser Phe Phe Ala Ile His Ile Thr Gly Ala Leu Val Leu
            195                 200                 205

ttc atg acg gat ggg ttg acg ggt gcc tat tcc gcc ttc gtg tac agc 671
Phe Met Thr Asp Gly Leu Thr Gly Ala Tyr Ser Ala Phe Val Tyr Ser
        210                 215                 220

tat gct gtg gag aag ccc ctg tct gtg gga cac aag gtg gct ggc tac 719
Tyr Ala Val Glu Lys Pro Leu Ser Val Gly His Lys Val Ala Gly Tyr
    225                 230                 235

ctc ccc agc ctc ttc tgg ggc ttc atc aca ctg ggc cgg ctc ctc tcc 767
Leu Pro Ser Leu Phe Trp Gly Phe Ile Thr Leu Gly Arg Leu Leu Ser
240                 245                 250                 255

att ccc ata tcc tca aga atg aag ccg gcc acc atg gtt ttc atc aac 815
Ile Pro Ile Ser Ser Arg Met Lys Pro Ala Thr Met Val Phe Ile Asn
                260                 265                 270

gtg gta ggc gtg gtg gtg acg ttc ctg gtg ctg ctt att ttc tcc tac 863
Val Val Gly Val Val Val Thr Phe Leu Val Leu Leu Ile Phe Ser Tyr
            275                 280                 285

aac gtc gtg ttc ctg ttc gtg ggg acg gca agc ctg ggc ctg ttt ctc 911
Asn Val Val Phe Leu Phe Val Gly Thr Ala Ser Leu Gly Leu Phe Leu
        290                 295                 300

agc agc acc ttc ccc agc atg ctg gcc tac acg gag gac tcg ctg cag 959
Ser Ser Thr Phe Pro Ser Met Leu Ala Tyr Thr Glu Asp Ser Leu Gln
    305                 310                 315

tac aaa ggc tgt gca acc aca gtg ctg gtg aca ggg gca gga gtt ggc 1007
Tyr Lys Gly Cys Ala Thr Thr Val Leu Val Thr Gly Ala Gly Val Gly
320                 325                 330                 335

gag atg gtg ctg cag atg ctg gtt ggt tcg ata ttc cag gct cag ggc 1055
Glu Met Val Leu Gln Met Leu Val Gly Ser Ile Phe Gln Ala Gln Gly
                340                 345                 350

agc tat agt ttc ctg gtc tgt ggc gtg atc ttt ggt tgt ctg gct ttt 1103
Ser Tyr Ser Phe Leu Val Cys Gly Val Ile Phe Gly Cys Leu Ala Phe
            355                 360                 365

acc ttc tat atc ttg ctc ctg ttt ttc cac agg atg cac cct gga ctc 1151
Thr Phe Tyr Ile Leu Leu Leu Phe Phe His Arg Met His Pro Gly Leu
        370                 375                 380

cca tca gtt cct acc caa gac aga tca att gga atg gaa aac tct gag 1199
Pro Ser Val Pro Thr Gln Asp Arg Ser Ile Gly Met Glu Asn Ser Glu
    385                 390                 395

tgc tac cag agg taaaactgg 1220
Cys Tyr Gln Arg
400

<210>90
<211>403
<212>PRT
<213>Homo sapiens

<400>90

Met Ala Leu Ala Gly Leu Ala Met Gly Cys Ile Asp Thr Val Ala Asn
1               5                   10                  15

Met Gln Leu Val Arg Met Tyr Gln Lys Asp Ser Ala Val Phe Leu Gln
            20                  25                  30

Val Leu His Phe Phe Val Gly Phe Gly Ala Leu Leu Ser Pro Leu Ile
        35                  40                  45

Ala Asp Pro Phe Leu Ser Glu Ala Asn Cys Leu Pro Ala Asn Ser Thr

    50                  55                  60

Ala Asn Thr Thr Ser Arg Gly His Leu Phe His Val Ser Arg Val Leu
65                  70                  75                  80

Gly Gln His His Val Asp Ala Lys Pro Trp Ser Asn Gln Thr Phe Pro
                85                  90                  95

Gly Leu Thr Pro Lys Asp Gly Ala Gly Thr Arg Val Ser Tyr Ala Phe
            100                 105                 110

Trp Ile Met Ala Leu Ile Asn Leu Pro Val Pro Met Ala Val Leu Met
        115                 120                 125

Leu Leu Ser Lys Glu Arg Leu Leu Thr Cys Cys Pro Gln Arg Arg Pro
    130                 135                 140

Leu Leu Leu Ser Ala Asp Glu Leu Ala Leu Glu Thr Gln Pro Pro Glu
145                 150                 155                 160

Lys Glu Asp Ala Ser Ser Leu Pro Pro Lys Phe Gln Ser His Leu Gly
                165                 170                 175

His Glu Asp Leu Phe Ser Cys Cys Gln Arg Lys Asn Leu Arg Gly Ala
            180                 185                 190

Pro Tyr Ser Phe Phe Ala Ile His Ile Thr Gly Ala Leu Val Leu Phe
        195                 200                 205

Met Thr Asp Gly Leu Thr Gly Ala Tyr Ser Ala Phe Val Tyr Ser Tyr
    210                 215                 220

Ala Val Glu Lys Pro Leu Ser Val Gly His Lys Val Ala Gly Tyr Leu
225                 230                 235                 240

Pro Ser Leu Phe Trp Gly Phe Ile Thr Leu Gly Arg Leu Leu Ser Ile
                245                 250                 255

Pro Ile Ser Ser Arg Met Lys Pro Ala Thr Met Val Phe Ile Asn Val
            260                 265                 270

Val Gly Val Val Val Thr Phe Leu Val Leu Leu Ile Phe Ser Tyr Asn
        275                 280                 285

Val Val Phe Leu Phe Val Gly Thr Ala Ser Leu Gly Leu Phe Leu Ser
    290                 295                 300

Ser Thr Phe Pro Ser Met Leu Ala Tyr Thr Glu Asp Ser Leu Gln Tyr
305                 310                 315                 320

Lys Gly Cys Ala Thr Thr Val Leu Val Thr Gly Ala Gly Val Gly Glu
                325                 330                 335

Met Val Leu Gln Met Leu Val Gly Ser Ile Phe Gln Ala Gln Gly Ser
            340                 345                 350

Tyr Ser Phe Leu Val Cys Gly Val Ile Phe Gly Cys Leu Ala Phe Thr

355                 360                 365

Phe Tyr Ile Leu Leu Leu Phe Phe His Arg Met His Pro Gly Leu Pro
    370                 375                 380

Ser Val Pro Thr Gln Asp Arg Ser Ile Gly Met Glu Asn Ser Glu Cys
385                 390                 395                 400

Tyr Gln Arg


<210>91
<211>1217
<212>DNA
<213>Homo sapiens

<220>
<221> CDS
<222>(1)..(1210)

<400>91

atg gcg ctg gcg ggc ttg gcc atg ggc tgc atc gac acc gtg gcc aac    48
Met Ala Leu Ala Gly Leu Ala Met Gly Cys Ile Asp Thr Val Ala Asn
  1               5                  10                  15

atg cag cag gta agg atg tac cag aag gac tcg gcc gtc ttc ctc cag    96
Met Gln Gln Val Arg Met Tyr Gln Lys Asp Ser Ala Val Phe Leu Gln
             20                  25                  30

gtg ctc cat ttc ttc gtg ggc ttt ggt gct ctg ctg agc ccc ctt att    144
Val Leu His Phe Phe Val Gly Phe Gly Ala Leu Leu Ser Pro Leu Ile
         35                  40                  45

gct gac cct ttc ctg tct gag gcc aac tgc ttg cct gcc aat agc acg    192
Ala Asp Pro Phe Leu Ser Glu Ala Asn Cys Leu Pro Ala Asn Ser Thr
     50                  55                  60

gcc aac acc acc tcc cga ggc cac ctg ttc cat gtc tcc agg gtg ctg    240
Ala Asn Thr Thr Ser Arg Gly His Leu Phe His Val Ser Arg Val Leu
 65                  70                  75                  80

ggc cag cac cac gta gat gcc aag cct tgg tcc aac cag acg ttc cca    288
Gly Gln His His Val Asp Ala Lys Pro Trp Ser Asn Gln Thr Phe Pro
                 85                  90                  95

ggg ctg act cca aag gac ggg gca ggg acc cga gtg tcc tat gcc ttc    336
Gly Leu Thr Pro Lys Asp Gly Ala Gly Thr Arg Val Ser Tyr Ala Phe
            100                 105                 110

tgg atc atg gcc ctc atc aat ctt cca gtg ccc atg gct gtg ctg atg    384
Trp Ile Met Ala Leu Ile Asn Leu Pro Val Pro Met Ala Val Leu Met
        115                 120                 125

ctg ctg tcc aag gag cgg ctg ctg acc tgc tgt ccc cag agg agg ccc    432
Leu Leu Ser Lys Glu Arg Leu Leu Thr Cys Cys Pro Gln Arg Arg Pro
    130                 135                 140

ctg ctt ctg tct gct gat gag ctt gcc ttg gag aca cag cct cct gag 480
Leu Leu Leu Ser Ala Asp Glu Leu Ala Leu Glu Thr Gln Pro Pro Glu
145                 150                 155                 160

aag gaa gat gcc tcc tca ctg ccc cca aag ttt cag tca cac cta ggg 528
Lys Glu Asp Ala Ser Ser Leu Pro Pro Lys Phe Gln Ser His Leu Gly
                165                 170                 175

cat gag gac ctg ttc agc tgc tgc caa agg aag aac ctc aga gga gcc 576
His Glu Asp Leu Phe Ser Cys Cys Gln Arg Lys Asn Leu Arg Gly Ala
            180                 185                 190

cct tat tcc ttc ttt gcc atc cac atc acg gcc gcc ctg gtc ctg ttc 624
Pro Tyr Ser Phe Phe Ala Ile His Ile Thr Ala Ala Leu Val Leu Phe
        195                 200                 205

atg acg gat ggg ttg acg ggt gcc tat tcc gcc ttc gtg tac agc tat 672
Met Thr Asp Gly Leu Thr Gly Ala Tyr Ser Ala Phe Val Tyr Ser Tyr
    210                 215                 220

gct gtg gag aag ccc ctg tct gtg gga cac aag gtg gct ggc tac ctc 720
Ala Val Glu Lys Pro Leu Ser Val Gly His Lys Val Ala Gly Tyr Leu
225                 230                 235                 240

ccc agc ctc ttc tgg ggc ttc atc aca ctg ggc cgg ctc ctc tcc att 768
Pro Ser Leu Phe Trp Gly Phe Ile Thr Leu Gly Arg Leu Leu Ser Ile
                245                 250                 255

ccc ata tcc tca aga atg aag ccg gcc acc atg gtt ttc atc aac gtg 816
Pro Ile Ser Ser Arg Met Lys Pro Ala Thr Met Val Phe Ile Asn Val
            260                 265                 270

gtt ggc gtg gtg gtg acg ttc ctg gtg ctg ctt att ttc tcc tac aac 864
Val Gly Val Val Val Thr Phe Leu Val Leu Leu Ile Phe Ser Tyr Asn
        275                 280                 285

gtc gtc ttc ctg ttc gtg ggg acg gca agc ctg ggc ctg ttt ctc agc 912
Val Val Phe Leu Phe Val Gly Thr Ala Ser Leu Gly Leu Phe Leu Ser
    290                 295                 300

agc acc ttc ccc agc atg ctg gcc tac acg gag gac tcg ctg cag tac 960
Ser Thr Phe Pro Ser Met Leu Ala Tyr Thr Glu Asp Ser Leu Gln Tyr
305                 310                 315                 320

aaa ggc tgt gca acc aca gtg ctg gtg aca ggg gca gga gtt ggc gag 1008
Lys Gly Cys Ala Thr Thr Val Leu Val Thr Gly Ala Gly Val Gly Glu
                325                 330                 335

atg gtg ctg cag atg ctg gtt ggt tcg ata ttc cag gct cag ggc agc 1056
Met Val Leu Gln Met Leu Val Gly Ser Ile Phe Gln Ala Gln Gly Ser
            340                 345                 350

tat agt ttc ctg gtc tgt ggc gtg atc ttt ggt tgt ctg gct ttt acc 1104
Tyr Ser Phe Leu Val Cys Gly Val Ile Phe Gly Cys Leu Ala Phe Thr
        355                 360                 365

ttc tat atc ttg ctc ctg ttt ttc cac agg atg cac cct gga ctc cca 1152
Phe Tyr Ile Leu Leu Leu Phe Phe His Arg Met His Pro Gly Leu Pro
    370                 375                 380

tca gtt cct acc caa gac aga tca att gga atg gaa aac tct gag tgc 1200
Ser Val Pro Thr Gln Asp Arg Ser Ile Gly Met Glu Asn Ser Glu Cys
385                 390                 395                 400

tac cag agg taaaactg 1217
Tyr Gln Arg

<210>92
<211>403
<212>PRT
<213>Homo sapiens

<400>92

Met Ala Leu Ala Gly Leu Ala Met Gly Cys Ile Asp Thr Val Ala Asn
1               5                   10                  15

Met Gln Gln Val Arg Met Tyr Gln Lys Asp Ser Ala Val Phe Leu Gln
            20                  25                  30

Val Leu His Phe Phe Val Gly Phe Gly Ala Leu Leu Ser Pro Leu Ile
        35                  40                  45

Ala Asp Pro Phe Leu Ser Glu Ala Asn Cys Leu Pro Ala Asn Ser Thr
    50                  55                  60

Ala Asn Thr Thr Ser Arg Gly His Leu Phe His Val Ser Arg Val Leu
65                  70                  75                  80

Gly Gln His His Val Asp Ala Lys Pro Trp Ser Asn Gln Thr Phe Pro
                85                  90                  95

Gly Leu Thr Pro Lys Asp Gly Ala Gly Thr Arg Val Ser Tyr Ala Phe
            100                 105                 110

Trp Ile Met Ala Leu Ile Asn Leu Pro Val Pro Met Ala Val Leu Met
        115                 120                 125

Leu Leu Ser Lys Glu Arg Leu Leu Thr Cys Cys Pro Gln Arg Arg Pro
    130                 135                 140

Leu Leu Leu Ser Ala Asp Glu Leu Ala Leu Glu Thr Gln Pro Pro Glu
145                 150                 155                 160

Lys Glu Asp Ala Ser Ser Leu Pro Pro Lys Phe Gln Ser His Leu Gly
                165                 170                 175

His Glu Asp Leu Phe Ser Cys Cys Gln Arg Lys Asn Leu Arg Gly Ala
            180                 185                 190

Pro Tyr Ser Phe Phe Ala Ile His Ile Thr Ala Ala Leu Val Leu Phe
        195                 200                 205

Met Thr Asp Gly Leu Thr Gly Ala Tyr Ser Ala Phe Val Tyr Ser Tyr
    210                 215                 220

Ala Val Glu Lys Pro Leu Ser Val Gly His Lys Val Ala Gly Tyr Leu
225                 230                 235                 240

Pro Ser Leu Phe Trp Gly Phe Ile Thr Leu Gly Arg Leu Leu Ser Ile
                245                 250                 255

Pro Ile Ser Ser Arg Met Lys Pro Ala Thr Met Val Phe Ile Asn Val
            260                 265                 270

Val Gly Val Val Val Thr Phe Leu Val Leu Leu Ile Phe Ser Tyr Asn
        275                 280                 285

Val Val Phe Leu Phe Val Gly Thr Ala Ser Leu Gly Leu Phe Leu Ser
    290                 295                 300

Ser Thr Phe Pro Ser Met Leu Ala Tyr Thr Glu Asp Ser Leu Gln Tyr
305                 310                 315                 320

Lys Gly Cys Ala Thr Thr Val Leu Val Thr Gly Ala Gly Val Gly Glu
                325                 330                 335

Met Val Leu Gln Met Leu Val Gly Ser Ile Phe Gln Ala Gln Gly Ser
            340                 345                 350

Tyr Ser Phe Leu Val Cys Gly Val Ile Phe Gly Cys Leu Ala Phe Thr
        355                 360                 365

Phe Tyr Ile Leu Leu Leu Phe Phe His Arg Met His Pro Gly Leu Pro
    370                 375                 380

Ser Val Pro Thr Gln Asp Arg Ser Ile Gly Met Glu Asn Ser Glu Cys
385                 390                 395                 400

Tyr Gln Arg


<210>93
<211>1190
<212>DNA
<213>Homo sapiens

<220>
<221> CDS
<222>(1)..(1183)

<400>93

atg gcg ctg gcg ggc ttg gcc atg ggc tgc atc gac acc gtg gcc aac     48
Met Ala Leu Ala Gly Leu Ala Met Gly Cys Ile Asp Thr Val Ala Asn
  1               5                  10                  15

atg cag ctg gta agg atg tac cag aag gac tcg gcc gtc ttc ctc cag     96
Met Gln Leu Val Arg Met Tyr Gln Lys Asp Ser Ala Val Phe Leu Gln

                20                  25                  30

gtg ctc cat ttc ttc gtg ggc ttt ggt gct ctg ctg agc ccc ctt att    144
Val Leu His Phe Phe Val Gly Phe Gly Ala Leu Leu Ser Pro Leu Ile
            35                  40                  45

gct gac cct ttc ctg tct gag gcc aac tgc ttg cct gcc aat agc acg    192
Ala Asp Pro Phe Leu Ser Glu Ala Asn Cys Leu Pro Ala Asn Ser Thr
        50                  55                  60

gcc aac acc acc tcc cga ggc cac ctg ttc cat gtc tcc agg gtg ctg    240
Ala Asn Thr Thr Ser Arg Gly His Leu Phe His Val Ser Arg Val Leu
 65                  70                  75                  80

ggc cag cac cac gta gat gcc aag cct tgg tcc aac cag acg ttc cca    288
Gly Gln His His Val Asp Ala Lys Pro Trp Ser Asn Gln Thr Phe Pro
                 85                  90                  95

ggg ctg act cca aag gac ggg gca ggg acc cga gtg tcc tat gcc ttc    336
Gly Leu Thr Pro Lys Asp Gly Ala Gly Thr Arg Val Ser Tyr Ala Phe
            100                 105                 110

tgg atc atg gcc ctc atc aat ctt cca gtg ccc atg gct gtg ctg atg    384
Trp Ile Met Ala Leu Ile Asn Leu Pro Val Pro Met Ala Val Leu Met
        115                 120                 125

ctg ctg tcc aag gag cgg ctg ctg acc tgc tgt ccc cag agg agg ccc    432
Leu Leu Ser Lys Glu Arg Leu Leu Thr Cys Cys Pro Gln Arg Arg Pro
    130                 135                 140

ctg ctt ctg tct gct gat gag ctt gcc ttg gag aca cag cct cct gag    480
Leu Leu Leu Ser Ala Asp Glu Leu Ala Leu Glu Thr Gln Pro Pro Glu
145                 150                 155                 160

aag gaa gat gcc tcc tca ctg ccc cca aag ttt cag tca cac cta ggg    528
Lys Glu Asp Ala Ser Ser Leu Pro Pro Lys Phe Gln Ser His Leu Gly
                165                 170                 175

cat gag gac ctg ttc agc tgc tgc caa agg aag aac ctc aga gga gcc    576
His Glu Asp Leu Phe Ser Cys Cys Gln Arg Lys Asn Leu Arg Gly Ala
            180                 185                 190

cct tat tcc ttc ttt gcc atc cac atc acg ggc gcc ctg ggt gcc tat    624
Pro Tyr Ser Phe Phe Ala Ile His Ile Thr Gly Ala Leu Gly Ala Tyr
        195                 200                 205

tcc gcc ttc gtg tac agc tat gct gtg gag aag ccc ctg tct gtg gga    672
Ser Ala Phe Val Tyr Ser Tyr Ala Val Glu Lys Pro Leu Ser Val Gly
    210                 215                 220

cac aag gtg gct ggc tac ctc ccc agc ctc ttc tgg ggc ttc atc aca    720
His Lys Val Ala Gly Tyr Leu Pro Ser Leu Phe Trp Gly Phe Ile Thr
225                 230                 235                 240

ctg ggc cgg ctc ctc tcc att ccc ata tcc tca aga atg aag ccg gcc    768
Leu Gly Arg Leu Leu Ser Ile Pro Ile Ser Ser Arg Met Lys Pro Ala
                245                 250                 255

acc atg gtt ttc atc aac gtg gtt ggc gtg gtg gtg acg ttc ctg gtg 816
Thr Met Val Phe Ile Asn Val Val Gly Val Val Val Thr Phe Leu Val
            260                 265                 270

ctg ctt att ttc tcc tac aac gtc gtc ttc ctg ttc gtg ggg acg gca 864
Leu Leu Ile Phe Ser Tyr Asn Val Val Phe Leu Phe Val Gly Thr Ala
        275                 280                 285

agc ctg ggc ctg ttt ctc agc agc acc ttc ccc agc atg ctg gcc tac 912
Ser Leu Gly Leu Phe Leu Ser Ser Thr Phe Pro Ser Met Leu Ala Tyr
    290                 295                 300

acg gag gac tcg ctg cag tac aaa ggc tgt gca acc aca gtg ctg gtg 960
Thr Glu Asp Ser Leu Gln Tyr Lys Gly Cys Ala Thr Thr Val Leu Val
305                 310                 315                 320

aca ggg gca gga gtt ggc gag atg gtg ctg cag atg ctg gtt ggt tcg 1008
Thr Gly Ala Gly Val Gly Glu Met Val Leu Gln Met Leu Val Gly Ser
                325                 330                 335

ata ttc cag gct cag ggc agc tat agt ttc ctg gtc tgt ggc gtg atc 1056
Ile Phe Gln Ala Gln Gly Ser Tyr Ser Phe Leu Val Cys Gly Val Ile
            340                 345                 350

ttt ggt tgt ctg gct ttt acc ttc tat acc ttg ctc ctg ttt ttc cac 1104
Phe Gly Cys Leu Ala Phe Thr Phe Tyr Thr Leu Leu Leu Phe Phe His
        355                 360                 365

agg atg cac cct gga ctc cca tca gtt cct acc caa gac aga tca att 1152
Arg Met His Pro Gly Leu Pro Ser Val Pro Thr Gln Asp Arg Ser Ile
    370                 375                 380

gga atg gaa aac tct gag tgc tac cag agg taaaactg 1190
Gly Met Glu Asn Ser Glu Cys Tyr Gln Arg
385                 390

<210>94
<211>394
<212>PRT
<213>Homo sapiens

<400>94

Met Ala Leu Ala Gly Leu Ala Met Gly Cys Ile Asp Thr Val Ala Asn
1               5                   10                  15

Met Gln Leu Val Arg Met Tyr Gln Lys Asp Ser Ala Val Phe Leu Gln
            20                  25                  30

Val Leu His Phe Phe Val Gly Phe Gly Ala Leu Leu Ser Pro Leu Ile
        35                  40                  45

Ala Asp Pro Phe Leu Ser Glu Ala Asn Cys Leu Pro Ala Asn Ser Thr
    50                  55                  60

Ala Asn Thr Thr Ser Arg Gly His Leu Phe His Val Ser Arg Val Leu

65                  70                  75                  80

Gly Gln His His Val Asp Ala Lys Pro Trp Ser Asn Gln Thr Phe Pro
                85                  90                  95

Gly Leu Thr Pro Lys Asp Gly Ala Gly Thr Arg Val Ser Tyr Ala Phe
            100                 105                 110

Trp Ile Met Ala Leu Ile Asn Leu Pro Val Pro Met Ala Val Leu Met
        115                 120                 125

Leu Leu Ser Lys Glu Arg Leu Leu Thr Cys Cys Pro Gln Arg Arg Pro
    130                 135                 140

Leu Leu Leu Ser Ala Asp Glu Leu Ala Leu Glu Thr Gln Pro Pro Glu
145                 150                 155                 160

Lys Glu Asp Ala Ser Ser Leu Pro Pro Lys Phe Gln Ser His Leu Gly
                165                 170                 175

His Glu Asp Leu Phe Ser Cys Cys Gln Arg Lys Asn Leu Arg Gly Ala
            180                 185                 190

Pro Tyr Ser Phe Phe Ala Ile His Ile Thr Gly Ala Leu Gly Ala Tyr
        195                 200                 205

Ser Ala Phe Val Tyr Ser Tyr Ala Val Glu Lys Pro Leu Ser Val Gly
    210                 215                 220

His Lys Val Ala Gly Tyr Leu Pro Ser Leu Phe Trp Gly Phe Ile Thr
225                 230                 235                 240

Leu Gly Arg Leu Leu Ser Ile Pro Ile Ser Ser Arg Met Lys Pro Ala
                245                 250                 255

Thr Met Val Phe Ile Asn Val Val Gly Val Val Val Thr Phe Leu Val
            260                 265                 270

Leu Leu Ile Phe Ser Tyr Asn Val Val Phe Leu Phe Val Gly Thr Ala
        275                 280                 285

Ser Leu Gly Leu Phe Leu Ser Ser Thr Phe Pro Ser Met Leu Ala Tyr
    290                 295                 300

Thr Glu Asp Ser Leu Gln Tyr Lys Gly Cys Ala Thr Thr Val Leu Val
305                 310                 315                 320

Thr Gly Ala Gly Val Gly Glu Met Val Leu Gln Met Leu Val Gly Ser
                325                 330                 335

Ile Phe Gln Ala Gln Gly Ser Tyr Ser Phe Leu Val Cys Gly Val Ile
            340                 345                 350

Phe Gly Cys Leu Ala Phe Thr Phe Tyr Thr Leu Leu Leu Phe Phe His
        355                 360                 365

Arg Met His Pro Gly Leu Pro Ser Val Pro Thr Gln Asp Arg Ser Ile

370                 375                 380

Gly Met Glu Asn Ser Glu Cys Tyr Gln Arg
385                 390

<210>95
<211>1557
<212>DNA
<213>Homo sapiens

<220>
<221> CDS
<222>(51)..(1482)

<400>95

ccctgaggaa cagacgttcc ctggcagccc tggcacctac aaccccagac atg ctg     56
                                                       Met Leu
                                                       1

ctg ctg ctg ccc ctg ctc tgg ggg agg gag ggg gtg gag gga cag gga     104
Leu Leu Leu Pro Leu Leu Trp Gly Arg Glu Gly Val Glu Gly Gln Gly
        5                   10                  15

cag caa gag aat ggt tac aca ctg caa gtg cag agg gag gtg agg gtg     152
Gln Gln Glu Asn Gly Tyr Thr Leu Gln Val Gln Arg Glu Val Arg Val
    20                  25                  30

cag gag ggc ctg tgt gtc cac gtg ccc tcc tcc ttc tcc cac ccc cag     200
Gln Glu Gly Leu Cys Val His Val Pro Ser Ser Phe Ser His Pro Gln
35                  40                  45                  50

gtt gcc ttg act aac tct ccc caa gtt cat ggc tac tgg ttc cag gaa     248
Val Ala Leu Thr Asn Ser Pro Gln Val His Gly Tyr Trp Phe Gln Glu
                55                  60                  65

ggg gct gac aca gcc cag gat gct cca atg gct acg aac aac cca aaa     296
Gly Ala Asp Thr Ala Gln Asp Ala Pro Met Ala Thr Asn Asn Pro Lys
            70                  75                  80

cga aaa gtg aag aag gag acc cag ggc cga ttc cgt ctc tct gga aac     344
Arg Lys Val Lys Lys Glu Thr Gln Gly Arg Phe Arg Leu Ser Gly Asn
        85                  90                  95

ctg cag atg aac gac tgc tcc ctg agc atc gga gac gcc agg agg aag     392
Leu Gln Met Asn Asp Cys Ser Leu Ser Ile Gly Asp Ala Arg Arg Lys
    100                 105                 110

gac cag ggg tca ttt tct ttc ttt cgc atg gag aga gga agc atg aga     440
Asp Gln Gly Ser Phe Ser Phe Phe Arg Met Glu Arg Gly Ser Met Arg
115                 120                 125                 130

tgg aat tac gcg tct aac cag ctc cac gtg ttg gtg acg gcc ctg acc     488
Trp Asn Tyr Ala Ser Asn Gln Leu His Val Leu Val Thr Ala Leu Thr
                135                 140                 145

cac agg ccc aat atc tcc tcc ctg ggg acc atg gag tcc ggc cgc ccg     536

His Arg Pro Asn Ile Ser Ser Leu Gly Thr Met Glu Ser Gly Arg Pro
            150                 155                 160

gga aac ctg acc tgc tct gtg tcc tgg gcc tgt gag cag ggg ata ccc 584
Gly Asn Leu Thr Cys Ser Val Ser Trp Ala Cys Glu Gln Gly Ile Pro
        165                 170                 175

cta cca tct atc tcc tgg atg ggg acc tcc gtg tcc ttc ccg ggc cgc 632
Leu Pro Ser Ile Ser Trp Met Gly Thr Ser Val Ser Phe Pro Gly Arg
    180                 185                 190

acc aca gcc cgc tcc tca gtg ctc acc ctc atc cca aag ccc cag gac 680
Thr Thr Ala Arg Ser Ser Val Leu Thr Leu Ile Pro Lys Pro Gln Asp
195                 200                 205                 210

cat ggc acc aac ctc acc tgt cag gtg acc ctg cct gag gct ggt gtg 728
His Gly Thr Asn Leu Thr Cys Gln Val Thr Leu Pro Glu Ala Gly Val
                215                 220                 225

acc ttg acc agg act gtc caa ttc aat gcg tcc gac cct cct cag aac 776
Thr Leu Thr Arg Thr Val Gln Phe Asn Ala Ser Asp Pro Pro Gln Asn
            230                 235                 240

ttg act gtg gct atc ttc caa gca gac ggc aca gca tcc aca gcc ttg 824
Leu Thr Val Ala Ile Phe Gln Ala Asp Gly Thr Ala Ser Thr Ala Leu
        245                 250                 255

ggg aac agc tca tcc ctc tca gtc ctg gag ggc cag tct ctg cgc ctg 872
Gly Asn Ser Ser Ser Leu Ser Val Leu Glu Gly Gln Ser Leu Arg Leu
    260                 265                 270

gtc tgt gct gtc gac agc aat ccc cct gcc agg ctg agc tgg acc caa 920
Val Cys Ala Val Asp Ser Asn Pro Pro Ala Arg Leu Ser Trp Thr Gln
275                 280                 285                 290

ggg agc ctg acc ctg agc ccc tca cag tcc tcg aac cat ggg ctg ctg 968
Gly Ser Leu Thr Leu Ser Pro Ser Gln Ser Ser Asn His Gly Leu Leu
                295                 300                 305

aag ctg cct cga gtg cac gcg agg gat gaa ggg gaa ttc acc tgc cga 1016
Lys Leu Pro Arg Val His Ala Arg Asp Glu Gly Glu Phe Thr Cys Arg
            310                 315                 320

gct cag aac cct cgg ggc tcc cag cac att tcc ctg agc ctc tcc ctg 1064
Ala Gln Asn Pro Arg Gly Ser Gln His Ile Ser Leu Ser Leu Ser Leu
        325                 330                 335

cag aat gag ggc aca ggt acc aca tgg cct gta tca gga gtg atg ctg 1112
Gln Asn Glu Gly Thr Gly Thr Thr Trp Pro Val Ser Gly Val Met Leu
    340                 345                 350

ggg gtg gtc ggg gga gct gga gcc aca gcc ctg gtc ttc ctg tcc ttc 1160
Gly Val Val Gly Gly Ala Gly Ala Thr Ala Leu Val Phe Leu Ser Phe
355                 360                 365                 370

tgc gtc atc ttc atc gtg gtg agg tcc tgc agg aag aaa tca gca agg 1208
Cys Val Ile Phe Ile Val Val Arg Ser Cys Arg Lys Lys Ser Ala Arg

375                 380                 385

cca gca gcg ggc atc agg gat atg ggc atg gag gat gca aac gct gtc    1256
Pro Ala Ala Gly Ile Arg Asp Met Gly Met Glu Asp Ala Asn Ala Val
            390                 395                 400

agg ggc tca gcc tat cag cag gga ccc ctg act gaa tcc tgg aca gac    1304
Arg Gly Ser Ala Tyr Gln Gln Gly Pro Leu Thr Glu Ser Trp Thr Asp
        405                 410                 415

ggc agc ccc ccg aag cat cct ccc atg gct gcc tcc tcc tta gga gaa    1352
Gly Ser Pro Pro Lys His Pro Pro Met Ala Ala Ser Ser Leu Gly Glu
    420                 425                 430

gga gag ctc cag cat gca acc ctc agc ttc cat aag gtc agg cct cag    1400
Gly Glu Leu Gln His Ala Thr Leu Ser Phe His Lys Val Arg Pro Gln
435                 440                 445                 450

aac gcg cag gga cag gag gcc atg gac agt gaa tac ttg gag atc aag    1448
Asn Ala Gln Gly Gln Glu Ala Met Asp Ser Glu Tyr Leu Glu Ile Lys
            455                 460                 465

atc cac aag cga gaa act gca gag act cgg gcc tgattggggg             1491
Ile His Lys Arg Glu Thr Ala Glu Thr Arg Ala
            470                 475

atcacggtcc ctccaggcaa aggagaagtc agaagctgat tcttctaaaa             1541

ttaacagccc tctaga                                                  1557

<210>96
<211>477
<212>PRT
<213>Homo sapiens

<400>96

Met Leu Leu Leu Leu Pro Leu Leu Trp Gly Arg Glu Gly Val Glu Gly
 1               5                  10                  15

Gln Gly Gln Gln Glu Asn Gly Tyr Thr Leu Gln Val Gln Arg Glu Val
            20                  25                  30

Arg Val Gln Glu Gly Leu Cys Val His Val Pro Ser Ser Phe Ser His
        35                  40                  45

Pro Gln Val Ala Leu Thr Asn Ser Pro Gln Val His Gly Tyr Trp Phe
    50                  55                  60

Gln Glu Gly Ala Asp Thr Ala Gln Asp Ala Pro Met Ala Thr Asn Asn
65                  70                  75                  80

Pro Lys Arg Lys Val Lys Lys Glu Thr Gln Gly Arg Phe Arg Leu Ser

85                  90                  95

Gly Asn Leu Gln Met Asn Asp Cys Ser Leu Ser Ile Gly Asp Ala Arg
            100                 105                 110

Arg Lys Asp Gln Gly Ser Phe Ser Phe Phe Arg Met Glu Arg Gly Ser
        115                 120                 125

Met Arg Trp Asn Tyr Ala Ser Asn Gln Leu His Val Leu Val Thr Ala
    130                 135                 140

Leu Thr His Arg Pro Asn Ile Ser Ser Leu Gly Thr Met Glu Ser Gly
145                 150                 155                 160

Arg Pro Gly Asn Leu Thr Cys Ser Val Ser Trp Ala Cys Glu Gln Gly
                165                 170                 175

Ile Pro Leu Pro Ser Ile Ser Trp Met Gly Thr Ser Val Ser Phe Pro
            180                 185                 190

Gly Arg Thr Thr Ala Arg Ser Ser Val Leu Thr Leu Ile Pro Lys Pro
        195                 200                 205

Gln Asp His Gly Thr Asn Leu Thr Cys Gln Val Thr Leu Pro Glu Ala
    210                 215                 220

Gly Val Thr Leu Thr Arg Thr Val Gln Phe Asn Ala Ser Asp Pro Pro
225                 230                 235                 240

Gln Asn Leu Thr Val Ala Ile Phe Gln Ala Asp Gly Thr Ala Ser Thr
                245                 250                 255

Ala Leu Gly Asn Ser Ser Ser Leu Ser Val Leu Glu Gly Gln Ser Leu
            260                 265                 270

Arg Leu Val Cys Ala Val Asp Ser Asn Pro Pro Ala Arg Leu Ser Trp
        275                 280                 285

Thr Gln Gly Ser Leu Thr Leu Ser Pro Ser Gln Ser Ser Asn His Gly
    290                 295                 300

Leu Leu Lys Leu Pro Arg Val His Ala Arg Asp Glu Gly Glu Phe Thr
305                 310                 315                 320

Cys Arg Ala Gln Asn Pro Arg Gly Ser Gln His Ile Ser Leu Ser Leu
                325                 330                 335

Ser Leu Gln Asn Glu Gly Thr Gly Thr Thr Trp Pro Val Ser Gly Val
            340                 345                 350

Met Leu Gly Val Val Gly Gly Ala Gly Ala Thr Ala Leu Val Phe Leu
        355                 360                 365

Ser Phe Cys Val Ile Phe Ile Val Val Arg Ser Cys Arg Lys Lys Ser
    370                 375                 380

Ala Arg Pro Ala Ala Gly Ile Arg Asp Met Gly Met Glu Asp Ala Asn

385                 390                 395                 400

Ala Val Arg Gly Ser Ala Tyr Gln Gln Gly Pro Leu Thr Glu Ser Trp
                405                 410                 415

Thr Asp Gly Ser Pro Pro Lys His Pro Pro Met Ala Ala Ser Ser Leu
            420                 425                 430

Gly Glu Gly Glu Leu Gln His Ala Thr Leu Ser Phe His Lys Val Arg
        435                 440                 445

Pro Gln Asn Ala Gln Gly Gln Glu Ala Met Asp Ser Glu Tyr Leu Glu
    450                 455                 460

Ile Lys Ile His Lys Arg Glu Thr Ala Glu Thr Arg Ala
465                 470                 475

<210>97
<211>1494
<212>DNA
<213>Homo sapiens

<220>
<221> CDS
<222>(28)..(1288)

<400>97
tttaatgcac agatataata caggagt atg cta ggt ggg tct gtt ttt caa      51
ctt gga tct ctc ctt aca ggg cta ctt tct cat gtt ttg atg gaa gtc    99
aag aag ata cca gtg gaa gag ggg ctg tgc act aca atc cct tgt gca    147
ttt gaa ttt ccc aga gaa ccc cca agc aat tcc atg atc atg cac tac    195
tgg ctc aac aaa aac atc agc tcc ctg gta gct aca aat aaa ccc aat    243
gct acc att ggt gat aac acc aag gac aaa ttt tac atg act ggg aat    291
ctt gat gaa gaa gac tgt acc cta ctc atc cac gat ata ctc aaa ggg    339
aac agc ata aca tat tta ttc tac gca gat cta gga gaa caa aaa agt    387
gct ttc ctg ggg gag aat atc aaa ctt acc caa aag cca gag ctc cac    435
atg cca gag att ctt ttg gct gag aag act gtg acc ttg aac tgt acc    483
ctc aaa ggc acc tgc aaa gaa acc aaa gcc ctc ttc cac tcc cgg aag    531
aac cca gcc atc tcc agc agc tcc tcc tcg gtg ctg cac ttc acc ctg    579
agg cct gag gac cat ggc aac acc ctt gga tgt cac ttg aac tta tcc    627
cta gcc aac gtg acc aga agt agc ttg gtc aag ctc caa gtg gtc tgt    675
gag tgc tgg gca cct gcc agg ttg ttc aat tcc tct tgt tcc ttg gag    723
aag aca gtt ctg tgc agc tgt tcc ttc cac ggg atc ccc acg ccc tcc    771
gtg cag tgg tgg atg gga ggt gtc cct gtg gct gtg aac agc atg gat    819
aac atc ctc cgg gtg act tct tcc aca tgt gcc ccc tgg gcc aac agc    867
acc atc agc ctc att ggg gag cca gaa aga gtc atg aga ctt cac tgt    915
gag ggg aag aac caa tat gga att cac act tcc agc atc ttc ctg ctt    963
aga atg aag atg ctt acc tgg tgg gag gag cat cag agt ccc aag gcc    1011
aaa gaa ggc ttg ccc ctt aag aaa cca gag ctg ctg gag gaa aca gaa    1059
gta ccc aaa atg cct gaa gct gac acc cca cca gac cgg gct gga ggt    1107
aag tcc ctg gga cag tca cag gca gac tgt caa gca aca ggg ccg cag    1155
gcc agg gct ccc acc cca tca cct ggc aga acc ccg ggg agg cac ttg    1203
ttc agg acg gag aga ccc agg ccc cct cgc ccc aca cag cag atg cta    1251
cat ttg cca aaa caa agc ccc aca gac tgg gcg gct tgaacggcag         1297
atactgattt tctccccctc tggaggctgg aaatcccagg tcaaggtgcc             1347
agcagcagag cttccttctg aggcctctca ccttggccgg tagatgctgt             1397
cttctccctg tgtcctcaca gggtcatccc tctgcgtgtg cctgggtcct             1447
aatttccttc ccttgtaagg acaccagtca ttgaattcag gcccatc                1494

<210>98
<211>420
<212>PRT
<213>Homo sapiens

<400>98

<210>99
<211>1152
<212>DNA
<213>Homo sapiens

<220>
<221> CDS
<222>(16)..(1123)

<400>99

tgggaccacg tgggg atg ctg ccg tgg ctt ctt gtc ttc tct gct ctg      48
                 Met Leu Pro Trp Leu Leu Val Phe Ser Ala Leu
                  1               5                  10

ggt ctc cag gcc tgg ggt tct ttc tcc tgg agt gag acc caa gcc aga    96
Gly Leu Gln Ala Trp Gly Ser Phe Ser Trp Ser Glu Thr Gln Ala Arg
            15                  20                  25

ggc ttg tcc cag agg ctt atg gac ctg ttt gtc agc atc tca cag ttc    144
Gly Leu Ser Gln Arg Leu Met Asp Leu Phe Val Ser Ile Ser Gln Phe
        30                  35                  40

att cac aag gat act ccc acc atc gtc tcc cgc aag gag tgg ggg gca    192
Ile His Lys Asp Thr Pro Thr Ile Val Ser Arg Lys Glu Trp Gly Ala
    45                  50                  55

aga ccg ctc gcc tgc agg gcc ctg ctg acc ctg cct gtg gcc tac atc    240
Arg Pro Leu Ala Cys Arg Ala Leu Leu Thr Leu Pro Val Ala Tyr Ile
60                  65                  70                  75

atc aca gac cag ctc cca ggg atg cag tgc cag cag cag agc gtt tgc    288
Ile Thr Asp Gln Leu Pro Gly Met Gln Cys Gln Gln Gln Ser Val Cys
                80                  85                  90

agc cag atg ctg cgg ggg ttg cag tcc cat tcc gtc tac acc ata ggc    336
Ser Gln Met Leu Arg Gly Leu Gln Ser His Ser Val Tyr Thr Ile Gly
            95                  100                 105

tgg tgc gac gtg gcg tac agc ttc ctg gtt ggg gat gat ggc agg gtg    384
Trp Cys Asp Val Ala Tyr Ser Phe Leu Val Gly Asp Asp Gly Arg Val
        110                 115                 120

tat gaa ggt gtt ggc tgg aac atc caa ggc ttg cac acc cag ggc tac    432
Tyr Glu Gly Val Gly Trp Asn Ile Gln Gly Leu His Thr Gln Gly Tyr

125                 130                 135

aac aac att tcc ctg ggc atc gcc ttc ttt ggc aat aag aag ggt cac     480
Asn Asn Ile Ser Leu Gly Ile Ala Phe Phe Gly Asn Lys Lys Gly His
140                 145                 150                 155

tcc ccc agc cct gct gcc tta tca gct gca gag ggt ctg atc tcc tat     528
Ser Pro Ser Pro Ala Ala Leu Ser Ala Ala Glu Gly Leu Ile Ser Tyr
                160                 165                 170

gcc atc cag aag ggt cac ctg tcg ccc agg tat att cag cca ctt ctt     576
Ala Ile Gln Lys Gly His Leu Ser Pro Arg Tyr Ile Gln Pro Leu Leu
            175                 180                 185

ctg aaa gaa gag acc tgc ctg gac cct caa cat cca gtg atg ccc agg     624
Leu Lys Glu Glu Thr Cys Leu Asp Pro Gln His Pro Val Met Pro Arg
        190                 195                 200

aag cag ctt gcc ccg gcg ttg tcc cac ggt ctg tgt ggg gag cca ggg     672
Lys Gln Leu Ala Pro Ala Leu Ser His Gly Leu Cys Gly Glu Pro Gly
    205                 210                 215

aga ccc tta tca aaa atg aac ctc cca gcc aaa tat gtc atc atc atc     720
Arg Pro Leu Ser Lys Met Asn Leu Pro Ala Lys Tyr Val Ile Ile Ile
220                 225                 230                 235

cac acc gct ggc aca agc tgc act gta tcc aca gac tgc cag act gtc     768
His Thr Ala Gly Thr Ser Cys Thr Val Ser Thr Asp Cys Gln Thr Val
                240                 245                 250

gtc cga aac ata cag tcc ttt cac atg gac aca cgg aac ttt tgt gac     816
Val Arg Asn Ile Gln Ser Phe His Met Asp Thr Arg Asn Phe Cys Asp
            255                 260                 265

att gga tat agc ttc ctg gtg ggc cag gat ggt ggc gtg tat gaa ggg     864
Ile Gly Tyr Ser Phe Leu Val Gly Gln Asp Gly Gly Val Tyr Glu Gly
        270                 275                 280

gtt gga tgg cac atc caa ggc tct cac act tat gga ttc aac gat att     912
Val Gly Trp His Ile Gln Gly Ser His Thr Tyr Gly Phe Asn Asp Ile
    285                 290                 295

gcc cta gga att gcc ttc atc ggc atc ccc tac ttt gta ggt cca aat     960
Ala Leu Gly Ile Ala Phe Ile Gly Ile Pro Tyr Phe Val Gly Pro Asn
300                 305                 310                 315

gct gca gcg ctg gag gcg gcc cag gac ctg atc cag tgt gcc gtg gtt     1008
Ala Ala Ala Leu Glu Ala Ala Gln Asp Leu Ile Gln Cys Ala Val Val
                320                 325                 330

gag ggg tac ctg act cca aac tac ctg ctg atg ggc cac agt gac gtg     1056
Glu Gly Tyr Leu Thr Pro Asn Tyr Leu Leu Met Gly His Ser Asp Val
            335                 340                 345

gtc aac atc ctg tcc cct ggg cag gct ttg tat aac atc atc agc acc     1104
Val Asn Ile Leu Ser Pro Gly Gln Ala Leu Tyr Asn Ile Ile Ser Thr
        350                 355                 360

tgg cct cat ttc aag cac tgaaggaggc cccactccct ttgagactgc 1152
Trp Pro His Phe Lys His
    365

<210>100
<211>369
<212>PRT
<213>Homo sapiens

<400>100

Met Leu Pro Trp Leu Leu Val Phe Ser Ala Leu Gly Leu Gln Ala Trp
1               5                   10                  15

Gly Ser Phe Ser Trp Ser Glu Thr Gln Ala Arg Gly Leu Ser Gln Arg
            20                  25                  30

Leu Met Asp Leu Phe Val Ser Ile Ser Gln Phe Ile His Lys Asp Thr
        35                  40                  45

Pro Thr Ile Val Ser Arg Lys Glu Trp Gly Ala Arg Pro Leu Ala Cys
    50                  55                  60

Arg Ala Leu Leu Thr Leu Pro Val Ala Tyr Ile Ile Thr Asp Gln Leu
65                  70                  75                  80

Pro Gly Met Gln Cys Gln Gln Gln Ser Val Cys Ser Gln Met Leu Arg
                85                  90                  95

Gly Leu Gln Ser His Ser Val Tyr Thr Ile Gly Trp Cys Asp Val Ala
            100                 105                 110

Tyr Ser Phe Leu Val Gly Asp Asp Gly Arg Val Tyr Glu Gly Val Gly
        115                 120                 125

Trp Asn Ile Gln Gly Leu His Thr Gln Gly Tyr Asn Asn Ile Ser Leu
    130                 135                 140

Gly Ile Ala Phe Phe Gly Asn Lys Lys Gly His Ser Pro Ser Pro Ala
145                 150                 155                 160

Ala Leu Ser Ala Ala Glu Gly Leu Ile Ser Tyr Ala Ile Gln Lys Gly
                165                 170                 175

His Leu Ser Pro Arg Tyr Ile Gln Pro Leu Leu Leu Lys Glu Glu Thr
            180                 185                 190

Cys Leu Asp Pro Gln His Pro Val Met Pro Arg Lys Gln Leu Ala Pro
        195                 200                 205

Ala Leu Ser His Gly Leu Cys Gly Glu Pro Gly Arg Pro Leu Ser Lys
    210                 215                 220

Met Asn Leu Pro Ala Lys Tyr Val Ile Ile Ile His Thr Ala Gly Thr
225                 230                 235                 240

Ser Cys Thr Val Ser Thr Asp Cys Gln Thr Val Val Arg Asn Ile Gln
                245                 250                 255

Ser Phe His Met Asp Thr Arg Asn Phe Cys Asp Ile Gly Tyr Ser Phe
            260                 265                 270

Leu Val Gly Gln Asp Gly Gly Val Tyr Glu Gly Val Gly Trp His Ile
        275                 280                 285

Gln Gly Ser His Thr Tyr Gly Phe Asn Asp Ile Ala Leu Gly Ile Ala
    290                 295                 300

Phe Ile Gly Ile Pro Tyr Phe Val Gly Pro Asn Ala Ala Ala Leu Glu
305                 310                 315                 320

Ala Ala Gln Asp Leu Ile Gln Cys Ala Val Val Glu Gly Tyr Leu Thr
                325                 330                 335

Pro Asn Tyr Leu Leu Met Gly His Ser Asp Val Val Asn Ile Leu Ser
            340                 345                 350

Pro Gly Gln Ala Leu Tyr Asn Ile Ile Ser Thr Trp Pro His Phe Lys
        355                 360                 365

His

<210>101
<211>3950
<212>DNA
<213>Homo sapiens

<220>
<221> CDS
<222>(1)..(3655)

<400>101

atg cgc ggg gcg ggg gcg gcg ggg ctg ctg gcg ctg ctg ctg ctg ctg    48
Met Arg Gly Ala Gly Ala Ala Gly Leu Leu Ala Leu Leu Leu Leu Leu
  1               5                  10                  15

ctg ctg ctg ctg ctg ggc ctg ggc ggc agg gtc gag ggg ggg ccg gcc    96
Leu Leu Leu Leu Leu Gly Leu Gly Gly Arg Val Glu Gly Gly Pro Ala
             20                  25                  30

ggc gag cgg ggc gca ggc ggg ggc ggg gcg ctg gcc cgc gag cgc ttc    144
Gly Glu Arg Gly Ala Gly Gly Gly Gly Ala Leu Ala Arg Glu Arg Phe
         35                  40                  45

aag gtg gtc ttt gcg ccg gtg atc tgc aag cgg acc tgt ctc aag ggc    192
Lys Val Val Phe Ala Pro Val Ile Cys Lys Arg Thr Cys Leu Lys Gly
     50                  55                  60

cag tgt cgg gac agt tgt cag cag ggc tcc aac atg acg ctc atc gga 240
Gln Cys Arg Asp Ser Cys Gln Gln Gly Ser Asn Met Thr Leu Ile Gly
65                  70                  75                  80

gag aac ggc cac agc aca gac acg ctc acg ggc tcc ggc ttc cgc gtg 288
Glu Asn Gly His Ser Thr Asp Thr Leu Thr Gly Ser Gly Phe Arg Val
                85                  90                  95

gtg gtg tgc cct ctc ccc tgc atg aat ggc ggc cag tgc tcc tcg cga 336
Val Val Cys Pro Leu Pro Cys Met Asn Gly Gly Gln Cys Ser Ser Arg
            100                 105                 110

aac cag tgc ctg tgt ccc ccg gac ttc act ggg cgc ttc tgc cag gtg 384
Asn Gln Cys Leu Cys Pro Pro Asp Phe Thr Gly Arg Phe Cys Gln Val
        115                 120                 125

ccc gca gga gga gcc ggt ggg ggt acc ggc ggc tca ggc ccc ggc ctg 432
Pro Ala Gly Gly Ala Gly Gly Gly Thr Gly Gly Ser Gly Pro Gly Leu
    130                 135                 140

agc agg aca ggg gcc ctg tcc aca ggg gcg ctg ccg ccc ctg gct ccg 480
Ser Arg Thr Gly Ala Leu Ser Thr Gly Ala Leu Pro Pro Leu Ala Pro
145                 150                 155                 160

gag ggc gac tct gtg gcc agc aag cac gcc atc tac gcc gtc cag gtg 528
Glu Gly Asp Ser Val Ala Ser Lys His Ala Ile Tyr Ala Val Gln Val
                165                 170                 175

atc gct gac cct cct ggg ccc ggg gag ggg cct cct gcc cag cac gca 576
Ile Ala Asp Pro Pro Gly Pro Gly Glu Gly Pro Pro Ala Gln His Ala
            180                 185                 190

gcc ttc ctg gtg ccc cta ggc ccg gga cag atc tca gca gaa gtg cag 624
Ala Phe Leu Val Pro Leu Gly Pro Gly Gln Ile Ser Ala Glu Val Gln
        195                 200                 205

gcc ccg ccc ccc gtg gtg aat gtg cgc gtc cat cac ccg ccc gag gcc 672
Ala Pro Pro Pro Val Val Asn Val Arg Val His His Pro Pro Glu Ala
    210                 215                 220

tca gtc cag gtg cac cgc att gag agc tcg aac gcc gag agc gca gcc 720
Ser Val Gln Val His Arg Ile Glu Ser Ser Asn Ala Glu Ser Ala Ala
225                 230                 235                 240

ccc tcc cag cac ctg ctg ccg cac ccc aag ccc tcg cac ccc cgg ccg 768
Pro Ser Gln His Leu Leu Pro His Pro Lys Pro Ser His Pro Arg Pro
                245                 250                 255

ccc acc cag aag ccc ctg ggc cgc tgc ttt cag gac act ctg ccc aag 816
Pro Thr Gln Lys Pro Leu Gly Arg Cys Phe Gln Asp Thr Leu Pro Lys
            260                 265                 270

cag ccg tgt ggc agc aac ccc ctc ccc ggc ctc acc aag cag gaa gac 864
Gln Pro Cys Gly Ser Asn Pro Leu Pro Gly Leu Thr Lys Gln Glu Asp
        275                 280                 285

tgc tgc ggt agc atc ggc act gcc tgg ggc cag agc aag tgc cac aag 912
Cys Cys Gly Ser Ile Gly Thr Ala Trp Gly Gln Ser Lys Cys His Lys
    290                 295                 300

tgt ccc cag ctg cag tac aca gga gtg cag aag cca ggg cct gta cgt 960
Cys Pro Gln Leu Gln Tyr Thr Gly Val Gln Lys Pro Gly Pro Val Arg
305                 310                 315                 320

ggg gaa gtg ggc gct gac tgt ccc cag ggc tac aag agg ctt aac agc 1008
Gly Glu Val Gly Ala Asp Cys Pro Gln Gly Tyr Lys Arg Leu Asn Ser
                325                 330                 335

acc cac tgc cag gac atc aac gag tgc gca atg ccg ggc gtg tgt cgc 1056
Thr His Cys Gln Asp Ile Asn Glu Cys Ala Met Pro Gly Val Cys Arg
            340                 345                 350

cat ggt gac tgc ctc aac aac cct ggc tcc tat cgc tgt gtc tgc cca 1104
His Gly Asp Cys Leu Asn Asn Pro Gly Ser Tyr Arg Cys Val Cys Pro
        355                 360                 365

cct ggc cat agt tta ggc ccc tcc cgt aca cag tgc att gca gac aaa 1152
Pro Gly His Ser Leu Gly Pro Ser Arg Thr Gln Cys Ile Ala Asp Lys
    370                 375                 380

ccg gag gag aag agc ctg tgt ttc cgc ctg gtg agc cct gag cac cag 1200
Pro Glu Glu Lys Ser Leu Cys Phe Arg Leu Val Ser Pro Glu His Gln
385                 390                 395                 400

tgc cag cac cca ctg acc acc cgc ctg acc cgc cag ctc tgc tgc tgc 1248
Cys Gln His Pro Leu Thr Thr Arg Leu Thr Arg Gln Leu Cys Cys Cys
                405                 410                 415

agt gtc ggc aag gcc tgg ggc gcg cgg tgt cag cgc tgc cca aca gat 1296
Ser Val Gly Lys Ala Trp Gly Ala Arg Cys Gln Arg Cys Pro Thr Asp
            420                 425                 430

ggc acc gct gcg ttc aag gag atc tgc cca gct ggg aag gga tac cac 1344
Gly Thr Ala Ala Phe Lys Glu Ile Cys Pro Ala Gly Lys Gly Tyr His
        435                 440                 445

att ctc acc tcc cac cag acg ctc acc att cag ggc gag agt gac ttt 1392
Ile Leu Thr Ser His Gln Thr Leu Thr Ile Gln Gly Glu Ser Asp Phe
    450                 455                 460

tcc ctt ttc ctg cac cct gac ggg cca ccc aag ccc cag cag ctt ccg 1440
Ser Leu Phe Leu His Pro Asp Gly Pro Pro Lys Pro Gln Gln Leu Pro
465                 470                 475                 480

gag agc cct agc cag gct cca cca cct gag gac aca gag gaa gag aga 1488
Glu Ser Pro Ser Gln Ala Pro Pro Pro Glu Asp Thr Glu Glu Glu Arg
                485                 490                 495

ggg gtg acc acg gac tca ccg gtg agt gag gag agg tca gtg cag cag 1536
Gly Val Thr Thr Asp Ser Pro Val Ser Glu Glu Arg Ser Val Gln Gln
            500                 505                 510

agc cac cca act gcc acc acg act cct gcc cgg ccc tac ccc gag ctg 1584

Ser His Pro Thr Ala Thr Thr Thr Pro Ala Arg Pro Tyr Pro Glu Leu
        515                 520                 525

atc tcc cgt ccc tcg ccc ccg acc atg cgc tgg ttc ctg ccg gac ttg 1632
Ile Ser Arg Pro Ser Pro Pro Thr Met Arg Trp Phe Leu Pro Asp Leu
    530                 535                 540

cct cct tcc cgc agc gcc gta gag atc gct ccc act cag gtc aca gag 1680
Pro Pro Ser Arg Ser Ala Val Glu Ile Ala Pro Thr Gln Val Thr Glu
545                 550                 555                 560

act gat gag tgc cga ctg aac cag aac atc tgt ggc cac gga gag tgc 1728
Thr Asp Glu Cys Arg Leu Asn Gln Asn Ile Cys Gly His Gly Glu Cys
                565                 570                 575

gtg ccg ggc ccc cct gac tac tcc tgc cac tgc aac ccc ggc tac cgg 1776
Val Pro Gly Pro Pro Asp Tyr Ser Cys His Cys Asn Pro Gly Tyr Arg
            580                 585                 590

tca cat ccc cag cac cgc tac tgc gtg gat gtg aac gag tgc gag gca 1824
Ser His Pro Gln His Arg Tyr Cys Val Asp Val Asn Glu Cys Glu Ala
        595                 600                 605

gag ccc tgt ggc ccg ggg agg ggc atc tgc atg aac acc ggc ggc tcc 1872
Glu Pro Cys Gly Pro Gly Arg Gly Ile Cys Met Asn Thr Gly Gly Ser
    610                 615                 620

tac aat tgc cac tgc aac cgc ggc tac cgc ctg cac gtg ggc gcc ggg 1920
Tyr Asn Cys His Cys Asn Arg Gly Tyr Arg Leu His Val Gly Ala Gly
625                 630                 635                 640

ggg cgc tcg tgc gtg gac ctg aac gaa tgc gcc aag ccc cac ctg tgc 1968
Gly Arg Ser Cys Val Asp Leu Asn Glu Cys Ala Lys Pro His Leu Cys
                645                 650                 655

ggc gac ggc ggc ttc tgc atc aac ttt ccc ggt cac tac aag tgc aac 2016
Gly Asp Gly Gly Phe Cys Ile Asn Phe Pro Gly His Tyr Lys Cys Asn
            660                 665                 670

tgc tac ccc ggc tac cgg ctc aaa gcc tcc cgg cct cct gtg tgc gaa 2064
Cys Tyr Pro Gly Tyr Arg Leu Lys Ala Ser Arg Pro Pro Val Cys Glu
        675                 680                 685

gac atc gac gag tgc cgg gac cca agc tct tgc ccg gat ggc aaa tgc 2112
Asp Ile Asp Glu Cys Arg Asp Pro Ser Ser Cys Pro Asp Gly Lys Cys
    690                 695                 700

gag aac aag ccc ggg agc ttc aag tgc atc gcc tgt cag cct ggc tgg 2160
Glu Asn Lys Pro Gly Ser Phe Lys Cys Ile Ala Cys Gln Pro Gly Trp
705                 710                 715                 720

tgc gag aac ctc ccg ggc tcc ttc cgc tgc acc tgt gcc cag ggc tac 2208
Cys Glu Asn Leu Pro Gly Ser Phe Arg Cys Thr Cys Ala Gln Gly Tyr
                725                 730                 735

gcg ccc gcg ccc gac ggc cgc agt tgc ttg gat gtg gac gag tgt gag 2256
Ala Pro Ala Pro Asp Gly Arg Ser Cys Leu Asp Val Asp Glu Cys Glu

            740                 745                 750

gct ggg gac gtg tgt gac aat ggc atc tgc agc aac acg cca gga tct 2304
Ala Gly Asp Val Cys Asp Asn Gly Ile Cys Ser Asn Thr Pro Gly Ser
        755                 760                 765

ttc cag tgt cag tgc ctc tct ggc tac cat ctg tcc agg gac cgg agc 2352
Phe Gln Cys Gln Cys Leu Ser Gly Tyr His Leu Ser Arg Asp Arg Ser
    770                 775                 780

cac tgc gag gac att gat gag tgt gac ttc cct gca gcc tgc att ggg 2400
His Cys Glu Asp Ile Asp Glu Cys Asp Phe Pro Ala Ala Cys Ile Gly
785                 790                 795                 800

ggt gac tgc atc aat acc aat ggc tcc tac aga tgt ctt tgc ccc cag 2448
Gly Asp Cys Ile Asn Thr Asn Gly Ser Tyr Arg Cys Leu Cys Pro Gln
                805                 810                 815

ggg cat cgg ctg gtg ggt ggc agg aaa tgc caa gac ata gat gag tgc 2496
Gly His Arg Leu Val Gly Gly Arg Lys Cys Gln Asp Ile Asp Glu Cys
            820                 825                 830

agc cag gac ccg agc ctg tgc ctt ccc cat ggg gcc tgc aag aac ctt 2544
Ser Gln Asp Pro Ser Leu Cys Leu Pro His Gly Ala Cys Lys Asn Leu
        835                 840                 845

cag ggc tcc tat gtg tgt gtc tgc gat gag ggc ttc act ccc acc cag 2592
Gln Gly Ser Tyr Val Cys Val Cys Asp Glu Gly Phe Thr Pro Thr Gln
    850                 855                 860

gac cag cac ggt tgt gag gag gtg gag cag ccc cac cac aag aag gag 2640
Asp Gln His Gly Cys Glu Glu Val Glu Gln Pro His His Lys Lys Glu
865                 870                 875                 880

tgc tac ctg aac ttc gat gac aca gtg ttc tgc gac agc gta ttg gcc 2688
Cys Tyr Leu Asn Phe Asp Asp Thr Val Phe Cys Asp Ser Val Leu Ala
                885                 890                 895

acc aac gtg acc cag cag gag tgc tgc tgc tct ctg ggg gcc ggc tgg 2736
Thr Asn Val Thr Gln Gln Glu Cys Cys Cys Ser Leu Gly Ala Gly Trp
            900                 905                 910

ggc gac cac tgc gaa atc tac ccc tgc cca gtc tac agc tca gcc gag 2784
Gly Asp His Cys Glu Ile Tyr Pro Cys Pro Val Tyr Ser Ser Ala Glu
        915                 920                 925

ttc cac agc ctc tgc cca gac gga aag ggc tac acc cag gac aac aac 2832
Phe His Ser Leu Cys Pro Asp Gly Lys Gly Tyr Thr Gln Asp Asn Asn
    930                 935                 940

atc gtc aac tac ggc atc cca gcc cac cgt gac atc gac gag tgc atg 2880
Ile Val Asn Tyr Gly Ile Pro Ala His Arg Asp Ile Asp Glu Cys Met
945                 950                 955                 960

ttg ttc ggg tcg gag att tgc aag gag ggc aag tgc gtg aac acg cag 2928
Leu Phe Gly Ser Glu Ile Cys Lys Glu Gly Lys Cys Val Asn Thr Gln
                965                 970                 975

cct ggc tac gag tgc tac tgc aag cag ggc ttc tac tac gac ggg aac 2976
Pro Gly Tyr Glu Cys Tyr Cys Lys Gln Gly Phe Tyr Tyr Asp Gly Asn
            980                 985                 990

ctg ctg gaa tgc gtg gac gtg gac gag tgc ctg gac gag tcc aac tgc 3024
Leu Leu Glu Cys Val Asp Val Asp Glu Cys Leu Asp Glu Ser Asn Cys
        995                 1000                1005

cgg aac gga gtg tgt gag aac acg cgc ggc ggc tac cgc tgt gcc tgc 3072
Arg Asn Gly Val Cys Glu Asn Thr Arg Gly Gly Tyr Arg Cys Ala Cys
    1010                1015                1020

acg ccc cct gcc gag tac agt ccc gcg cag cgc cag tgc ctg agc ccg 3120
Thr Pro Pro Ala Glu Tyr Ser Pro Ala Gln Arg Gln Cys Leu Ser Pro
1025                1030                1035                1040

gaa gag atg gag cgt gcc ccg gag cgg cgc gac gtg tgc tgg agc cag 3168
Glu Glu Met Glu Arg Ala Pro Glu Arg Arg Asp Val Cys Trp Ser Gln
                1045                1050                1055

cgc gga gag gac ggc atg tgc gct ggc ccc ctg gcc ggg cct gcc ctc 3216
Arg Gly Glu Asp Gly Met Cys Ala Gly Pro Leu Ala Gly Pro Ala Leu
            1060                1065                1070

acc ttc gac gac tgc tgc tgc cgc cag ggc cgc ggc tgg ggc gcc caa 3264
Thr Phe Asp Asp Cys Cys Cys Arg Gln Gly Arg Gly Trp Gly Ala Gln
        1075                1080                1085

tgc cga ccg tgc ccg ccg cgc ggc gcg ggg tcc cat tgc ccg aca tcg 3312
Cys Arg Pro Cys Pro Pro Arg Gly Ala Gly Ser His Cys Pro Thr Ser
    1090                1095                1100

cag agc gag agc aat tcc ttc tgg gac aca agc ccc ctg ctg ttg ggg 3360
Gln Ser Glu Ser Asn Ser Phe Trp Asp Thr Ser Pro Leu Leu Leu Gly
1105                1110                1115                1120

aag ccc cca aga gat gag gac agt tca gag gag gat tca gac gag tgt 3408
Lys Pro Pro Arg Asp Glu Asp Ser Ser Glu Glu Asp Ser Asp Glu Cys
                1125                1130                1135

cgc tgc gtg agt ggc cgc tgc gtg ccg cgg ccg ggc ggc gcc gtg tgc 3456
Arg Cys Val Ser Gly Arg Cys Val Pro Arg Pro Gly Gly Ala Val Cys
            1140                1145                1150

gag tgt ccc ggc ggc ttc cag ctc gac gcc tcc cgc gcc cgc tgc gtg 3504
Glu Cys Pro Gly Gly Phe Gln Leu Asp Ala Ser Arg Ala Arg Cys Val
        1155                1160                1165

gat atc gac gag tgc cga gag ctg aac cag cgc ggg ctg ctg tgc aag 3552
Asp Ile Asp Glu Cys Arg Glu Leu Asn Gln Arg Gly Leu Leu Cys Lys
    1170                1175                1180

agc gag cgc tgc gtg aac acc agc ggc tcc ttc cgc tgc gtc tgc aaa 3600
Ser Glu Arg Cys Val Asn Thr Ser Gly Ser Phe Arg Cys Val Cys Lys
1185                1190                1195                1200

gcc ggc ttc gcg cgc agc cgc ccg cac ggg gcc tgc gtt ccc cag cgc    3648
Ala Gly Phe Ala Arg Ser Arg Pro His Gly Ala Cys Val Pro Gln Arg
                1205                1210                1215

cgc cgc tgacgccgcc gacgccgccc tcggcccaga cctcggtgat cactgaggga    3704
Arg Arg

tttccgcgag ctcggcctca cttctgcccc gacttgtggc tcggacccag    3754

ggaccttcag ggcccgcaga ccctcccggc gccttgagac ccgaggcgcc    3804

cctaccggcc cccctccccg gttagcgggc ggttgtaagg tctccggcgg    3854

gcgctgcctg ccttcctccc agagggtgtt tcctagaaac tgataaatca    3904

gatcgtgcct ctttaccctt ggctttcgaa aaaaaaaaaa aaaaaa    3950

<210>102
<211>1218
<212>PRT
<213>Homo sapiens

<400>102
   Met Arg Gly Ala Gly Ala Ala Gly Leu Leu Ala Leu Leu Leu Leu Leu
   1 5 10 15
   Leu Leu Leu Leu Leu Gly Leu Gly Gly Arg Val Glu Gly Gly Pro Ala
   20 25 30
   Gly Glu Arg Gly Ala Gly Gly Gly Gly Ala Leu Ala Arg Glu Arg Phe
   35 40 45
   Lys Val Val Phe Ala Pro Val Ile Cys Lys Arg Thr Cys Leu Lys Gly
   50 55 60
   Gln Cys Arg Asp Ser Cys Gln Gln Gly Ser Asn Met Thr Leu Ile Gly
   65 70 75 80
   Glu Asn Gly His Ser Thr Asp Thr Leu Thr Gly Ser Gly Phe Arg Val
   85 90 95
   Val Val Cys Pro Leu Pro Cys Met Asn Gly Gly Gln Cys Ser Ser Arg
   100 105 110
   Asn Gln Cys Leu Cys Pro Pro Asp Phe Thr Gly Arg Phe Cys Gln Val
   115                 120                 125
   Pro Ala Gly Gly Ala Gly Gly Gly Thr Gly Gly Ser Gly Pro Gly Leu
       130                 135                 140
   Ser Arg Thr Gly Ala Leu Ser Thr Gly Ala Leu Pro Pro Leu Ala Pro
   145                 150                 155                 160
   Glu Gly Asp Ser Val Ala Ser Lys His Ala Ile Tyr Ala Val Gln Val
                   165                 170                 175
   Ile Ala Asp Pro Pro Gly Pro Gly Glu Gly Pro Pro Ala Gln His Ala
               180                 185                 190
   Ala Phe Leu Val Pro Leu Gly Pro Gly Gln Ile Ser Ala Glu Val Gln
           195                 200                 205
   Ala Pro Pro Pro Val Val Asn Val Arg Val His His Pro Pro Glu Ala
       210                 215                 220
   Ser Val Gln Val His Arg Ile Glu Ser Ser Asn Ala Glu Ser Ala Ala
   225                 230                 235                 240
   Pro Ser Gln His Leu Leu Pro His Pro Lys Pro Ser His Pro Arg Pro
                   245                 250                 255
   Pro Thr Gln Lys Pro Leu Gly Arg Cys Phe Gln Asp Thr Leu Pro Lys
               260                 265                 270
   Gln Pro Cys Gly Ser Asn Pro Leu Pro Gly Leu Thr Lys Gln Glu Asp
           275                 280                 285
   Cys Cys Gly Ser Ile Gly Thr Ala Trp Gly Gln Ser Lys Cys His Lys
       290                 295                 300
   Cys Pro Gln Leu Gln Tyr Thr Gly Val Gln Lys Pro Gly Pro Val Arg
   305                 310                 315                 320
   Gly Glu Val Gly Ala Asp Cys Pro Gln Gly Tyr Lys Arg Leu Asn Ser
                   325                 330                 335
   Thr His Cys Gln Asp Ile Asn Glu Cys Ala Met Pro Gly Val Cys Arg
               340                 345                 350
   His Gly Asp Cys Leu Asn Asn Pro Gly Ser Tyr Arg Cys Val Cys Pro
           355                 360                 365
   Pro Gly His Ser Leu Gly Pro Ser Arg Thr Gln Cys Ile Ala Asp Lys
       370                 375                 380
   Pro Glu Glu Lys Ser Leu Cys Phe Arg Leu Val Ser Pro Glu His Gln
   385                 390                 395                 400
   Cys Gln His Pro Leu Thr Thr Arg Leu Thr Arg Gln Leu Cys Cys Cys
                   405                 410                 415
   Ser Val Gly Lys Ala Trp Gly Ala Arg Cys Gln Arg Cys Pro Thr Asp
   420 425 430
   Gly Thr Ala Ala Phe Lys Glu Ile Cys Pro Ala Gly Lys Gly Tyr His
   435 440 445
   Ile Leu Thr Ser His Gln Thr Leu Thr Ile Gln Gly Glu Ser Asp Phe
   450 455 460
   Ser Leu Phe Leu His Pro Asp Gly Pro Pro Lys Pro Gln Gln Leu Pro
   465 470 475 480
   Glu Ser Pro Ser Gln Ala Pro Pro Pro Glu Asp Thr Glu Glu Glu Arg
   485 490 495
   Gly Val Thr Thr Asp Ser Pro Val Ser Glu Glu Arg Ser Val Gln Gln
   500 505 510
   Ser His Pro Thr Ala Thr Thr Thr Pro Ala Arg Pro Tyr Pro Glu Leu
   515 520 525
   Ile Ser Arg Pro Ser Pro Pro Thr Met Arg Trp Phe Leu Pro Asp Leu
   530 535 540
   Pro Pro Ser Arg Ser Ala Val Glu Ile Ala Pro Thr Gln Val Thr Glu
   545 550 555 560
   Thr Asp Glu Cys Arg Leu Asn Gln Asn Ile Cys Gly His Gly Glu Cys
   565 570 575
   Val Pro Gly Pro Pro Asp Tyr Ser Cys His Cys Asn Pro Gly Tyr Arg
   580 585 590
   Ser His Pro Gln His Arg Tyr Cys Val Asp Val Asn Glu Cys Glu Ala
   595 600 605
   Glu Pro Cys Gly Pro Gly Arg Gly Ile Cys Met Asn Thr Gly Gly Ser
   610 615 620
   Tyr Asn Cys His Cys Asn Arg Gly Tyr Arg Leu His Val Gly Ala Gly
   625 630 635 640
   Gly Arg Ser Cys Val Asp Leu Asn Glu Cys Ala Lys Pro His Leu Cys
   645 650 655
   Gly Asp Gly Gly Phe Cys Ile Asn Phe Pro Gly His Tyr Lys Cys Asn
   660 665 670
   Cys Tyr Pro Gly Tyr Arg Leu Lys Ala Ser Arg Pro Pro Val Cys Glu
   675 680 685
   Asp Ile Asp Glu Cys Arg Asp Pro Ser Ser Cys Pro Asp Gly Lys Cys
   690 695 700
   Glu Asn Lys Pro Gly Ser Phe Lys Cys Ile Ala Cys Gln Pro Gly Trp
   705 710 715 720
   Cys Glu Asn Leu Pro Gly Ser Phe Arg Cys Thr Cys Ala Gln Gly Tyr
   725 730 735
   Ala Pro Ala Pro Asp Gly Arg Ser Cys Leu Asp Val Asp Glu Cys Glu
   740 745 750
   Ala Gly Asp Val Cys Asp Asn Gly Ile Cys Ser Asn Thr Pro Gly Ser
   755 760 765
   Phe Gln Cys Gln Cys Leu Ser Gly Tyr His Leu Ser Arg Asp Arg Ser
   770 775 780
   His Cys Glu Asp Ile Asp Glu Cys Asp Phe Pro Ala Ala Cys Ile Gly
   785 790 795 800
   Gly Asp Cys Ile Asn Thr Asn Gly Ser Tyr Arg Cys Leu Cys Pro Gln
   805 810 815
   Gly His Arg Leu Val Gly Gly Arg Lys Cys Gln Asp Ile Asp Glu Cys
   820 825 830
   Ser Gln Asp Pro Ser Leu Cys Leu Pro His Gly Ala Cys Lys Asn Leu
   835 840 845
   Gln Gly Ser Tyr Val Cys Val Cys Asp Glu Gly Phe Thr Pro Thr Gln
   850 855 860
   Asp Gln His Gly Cys Glu Glu Val Glu Gln Pro His His Lys Lys Glu
   865 870 875 880
   Cys Tyr Leu Asn Phe Asp Asp Thr Val Phe Cys Asp Ser Val Leu Ala
   885 890 895
   Thr Asn Val Thr Gln Gln Glu Cys Cys Cys Ser Leu Gly Ala Gly Trp
   900 905 910
   Gly Asp His Cys Glu Ile Tyr Pro Cys Pro Val Tyr Ser Ser Ala Glu
   915 920 925
   Phe His Ser Leu Cys Pro Asp Gly Lys Gly Tyr Thr Gln Asp Asn Asn
   930 935 940
   Ile Val Asn Tyr Gly Ile Pro Ala His Arg Asp Ile Asp Glu Cys Met
   945 950 955 960
   Leu Phe Gly Ser Glu Ile Cys Lys Glu Gly Lys Cys Val Asn Thr Gln
   965 970 975
   Pro Gly Tyr Glu Cys Tyr Cys Lys Gln Gly Phe Tyr Tyr Asp Gly Asn
   980 985 990
   Leu Leu Glu Cys Val Asp Val Asp Glu Cys Leu Asp Glu Ser Asn Cys
   995 1000 1005
   Arg Asn Gly Val Cys Glu Asn Thr Arg Gly Gly Tyr Arg Cys Ala Cys
   1010 1015 1020
   Thr Pro Pro Ala Glu Tyr Ser Pro Ala Gln Arg Gln Cys Leu Ser Pro
   1025                    1030                    1035                    1040
   Glu Glu Met Glu Arg Ala Pro Glu Arg Arg Asp Val Cys Trp Ser Gln
                   1045                    1050                    1055
   Arg Gly Glu Asp Gly Met Cys Ala Gly Pro Leu Ala Gly Pro Ala Leu
               1060                    1065                    1070
   Thr Phe Asp Asp Cys Cys Cys Arg Gln Gly Arg Gly Trp Gly Ala Gln
           1075                    1080                    1085
   Cys Arg Pro Cys Pro Pro Arg Gly Ala Gly Ser His Cys Pro Thr Ser
       1090                    1095                    1100
   Gln Ser Glu Ser Asn Ser Phe Trp Asp Thr Ser Pro Leu Leu Leu Gly
   1105                    1110                    1115                    1120
   Lys Pro Pro Arg Asp Glu Asp Ser Ser Glu Glu Asp Ser Asp Glu Cys
                   1125                    1130                    1135
   Arg Cys Val Ser Gly Arg Cys Val Pro Arg Pro Gly Gly Ala Val Cys
               1140                    1145                    1150
   Glu Cys Pro Gly Gly Phe Gln Leu Asp Ala Ser Arg Ala Arg Cys Val
           1155                    1160                    1165
   Asp Ile Asp Glu Cys Arg Glu Leu Asn Gln Arg Gly Leu Leu Cys Lys
       1170                    1175                    1180
   Ser Glu Arg Cys Val Asn Thr Ser Gly Ser Phe Arg Cys Val Cys Lys
   1185                    1190                    1195                    1200
   Ala Gly Phe Ala Arg Ser Arg Pro His Gly Ala Cys Val Pro Gln Arg
                   1205                    1210                    1215
   Arg Arg

<210>103
<211>603
<212>DNA
<213>Homo sapiens

<220>
<221> CDS
<222>(1)..(603)

<400>103

gga tcc cgc ttc aag gtg gtc ttt gcg ccg gtg atc tgc aag cgg acc      48
Gly Ser Arg Phe Lys Val Val Phe Ala Pro Val Ile Cys Lys Arg Thr
  1               5                   10                  15

tgt ctc aag ggc cag tgt cgg gac agt tgt cag cag ggc tcc aac atg      96
Cys Leu Lys Gly Gln Cys Arg Asp Ser Cys Gln Gln Gly Ser Asn Met
            20                  25                  30

acg ctc atc gga gag aac ggc cac agc aca gac acg ctc acg ggc tcc 144
Thr Leu Ile Gly Glu Asn Gly His Ser Thr Asp Thr Leu Thr Gly Ser
        35                  40                  45

ggc ttc cgc gtg gtg gtg tgc cct ctc ccc tgc atg aat ggc ggc cag 192
Gly Phe Arg Val Val Val Cys Pro Leu Pro Cys Met Asn Gly Gly Gln
    50                  55                  60

tgc tcc tcg cga aac cag tgc ctg tgt ccc ccg gac ttc act ggg cgc 240
Cys Ser Ser Arg Asn Gln Cys Leu Cys Pro Pro Asp Phe Thr Gly Arg
65                  70                  75                  80

ttc tgc cag gtg ccc gca gga gga gcc ggt ggg ggt acc ggc ggc tca 288
Phe Cys Gln Val Pro Ala Gly Gly Ala Gly Gly Gly Thr Gly Gly Ser
                85                  90                  95

ggc ccc ggc ctg agc agg aca ggg gcc ctg tcc aca ggg gcg ctg ccg 336
Gly Pro Gly Leu Ser Arg Thr Gly Ala Leu Ser Thr Gly Ala Leu Pro
            100                 105                 110

ccc ctg gct ccg gag ggc ggc tct gtg gcc agc aag cac gcc atc tac 384
Pro Leu Ala Pro Glu Gly Gly Ser Val Ala Ser Lys His Ala Ile Tyr
        115                 120                 125

gcc gtc cag gtg atc gct gac cct cct ggg ccc ggg gag ggg cct cct 432
Ala Val Gln Val Ile Ala Asp Pro Pro Gly Pro Gly Glu Gly Pro Pro
    130                 135                 140

gcc cag cac gca gcc ttc ctg gtg ccc cta ggc ccg gga cag atc tca 480
Ala Gln His Ala Ala Phe Leu Val Pro Leu Gly Pro Gly Gln Ile Ser
145                 150                 155                 160

gca gaa gtg cag gcc ccg ccc ccc gtg gtg aat gtg cgc gtc cat cac 528
Ala Glu Val Gln Ala Pro Pro Pro Val Val Asn Val Arg Val His His
                165                 170                 175

ccg ccc gag gcc tca gtc cag gtg cac cgc att gag agc tcg aac gcc 576
Pro Pro Glu Ala Ser Val Gln Val His Arg Ile Glu Ser Ser Asn Ala
            180                 185                 190

gag agc gca gcc ccc tcc cag ctc gag 603
Glu Ser Ala Ala Pro Ser Gln Leu Glu
        195                 200

<210>104
<211>201
<212>PRT
<213>Homo sapiens

<400>104

Gly Ser Arg Phe Lys Val Val Phe Ala Pro Val Ile Cys Lys Arg Thr
1               5                   10                  15

Cys Leu Lys Gly Gln Cys Arg Asp Ser Cys Gln Gln Gly Ser Asn Met
            20                  25                  30

Thr Leu Ile Gly Glu Asn Gly His Ser Thr Asp Thr Leu Thr Gly Ser
        35                  40                  45

Gly Phe Arg Val Val Val Cys Pro Leu Pro Cys Met Asn Gly Gly Gln
    50                  55                  60

Cys Ser Ser Arg Asn Gln Cys Leu Cys Pro Pro Asp Phe Thr Gly Arg
65                  70                  75                  80

Phe Cys Gln Val Pro Ala Gly Gly Ala Gly Gly Gly Thr Gly Gly Ser
                85                  90                  95

Gly Pro Gly Leu Ser Arg Thr Gly Ala Leu Ser Thr Gly Ala Leu Pro
            100                 105                 110

Pro Leu Ala Pro Glu Gly Gly Ser Val Ala Ser Lys His Ala Ile Tyr
        115                 120                 125

Ala Val Gln Val Ile Ala Asp Pro Pro Gly Pro Gly Glu Gly Pro Pro
    130                 135                 140

Ala Gln His Ala Ala Phe Leu Val Pro Leu Gly Pro Gly Gln Ile Ser
145                 150                 155                 160

Ala Glu Val Gln Ala Pro Pro Pro Val Val Asn Val Arg Val His His
                165                 170                 175

Pro Pro Glu Ala Ser Val Gln Val His Arg Ile Glu Ser Ser Asn Ala
            180                 185                 190

Glu Ser Ala Ala Pro Ser Gln Leu Glu
        195                 200

<210>105
<211>603
<212>DNA
<213>Homo sapiens

<220>
<221> CDS
<222>(1)..(603)

<400>105

gga tcc cgc ttc aag gtg gtc ttt gcg ccg gtg atc tgc aag cgg acc 48
Gly Ser Arg Phe Lys Val Val Phe Ala Pro Val Ile Cys Lys Arg Thr
1               5                   10                  15

tgt ctc aag ggc cag tgt cgg gac agt tgt cag cag ggc tcc aac atg 96
Cys Leu Lys Gly Gln Cys Arg Asp Ser Cys Gln Gln Gly Ser Asn Met
            20                  25                  30

acg ctc atc gga gag aac ggc cac agc aca gac acg ctc acg ggc tcc 144
Thr Leu Ile Gly Glu Asn Gly His Ser Thr Asp Thr Leu Thr Gly Ser
        35                  40                  45

ggc ttc cgc gtg gtg gtg tgc cct ctc ccc tgc atg aat ggc ggc cag 192

Gly Phe Arg Val Val Val Cys Pro Leu Pro Cys Met Asn Gly Gly Gln
    50                  55                  60

tgc tcc tcg cga aac cag tgc ctg tgt ccc ccg gac ttc act ggg cgc 240
Cys Ser Ser Arg Asn Gln Cys Leu Cys Pro Pro Asp Phe Thr Gly Arg
65                  70                  75                  80

ttc tgc cag gtg ccc gca gga gga gcc ggt ggg ggt acc ggc ggc tca 288
Phe Cys Gln Val Pro Ala Gly Gly Ala Gly Gly Gly Thr Gly Gly Ser
                85                  90                  95

ggc ccc ggc ctg agc agg aca ggg gcc ctg tcc aca ggg gcg ctg ccg 336
Gly Pro Gly Leu Ser Arg Thr Gly Ala Leu Ser Thr Gly Ala Leu Pro
            100                 105                 110

ccc ctg gct ccg gag ggc gac tct gtg gcc agc aag cac gcc atc tac 384
Pro Leu Ala Pro Glu Gly Asp Ser Val Ala Ser Lys His Ala Ile Tyr
        115                 120                 125

gcc gtc cag gtg atc gct gac cct cct ggg ccc ggg gag ggg cct cct 432
Ala Val Gln Val Ile Ala Asp Pro Pro Gly Pro Gly Glu Gly Pro Pro
    130                 135                 140

gcc cag cac gca gcc ttc ctg gtg ccc cta ggc ccg gga cag atc tca 480
Ala Gln His Ala Ala Phe Leu Val Pro Leu Gly Pro Gly Gln Ile Ser
145                 150                 155                 160

aca gaa gtg cag gcc ccg ccc ccc gtg gtg aat gtg cgc gtc cat cac 528
Thr Glu Val Gln Ala Pro Pro Pro Val Val Asn Val Arg Val His His
                165                 170                 175

ccg ccc gag gcc tca gtc cag gtg cac cgc att gag agc tcg aac gcc 576
Pro Pro Glu Ala Ser Val Gln Val His Arg Ile Glu Ser Ser Asn Ala
            180                 185                 190

gag agc gca gcc ccc tcc cag ctc gag 603
Glu Ser Ala Ala Pro Ser Gln Leu Glu
        195                 200

<210>106
<211>201
<212>PRT
<213>Homo sapiens

<400>106

Gly Ser Arg Phe Lys Val Val Phe Ala Pro Val Ile Cys Lys Arg Thr
1               5                   10                  15

Cys Leu Lys Gly Gln Cys Arg Asp Ser Cys Gln Gln Gly Ser Asn Met
            20                  25                  30

Thr Leu Ile Gly Glu Asn Gly His Ser Thr Asp Thr Leu Thr Gly Ser
        35                  40                  45

Gly Phe Arg Val Val Val Cys Pro Leu Pro Cys Met Asn Gly Gly Gln
    50                  55                  60

Cys Ser Ser Arg Asn Gln Cys Leu Cys Pro Pro Asp Phe Thr Gly Arg
65                  70                  75                  80

Phe Cys Gln Val Pro Ala Gly Gly Ala Gly Gly Gly Thr Gly Gly Ser
                85                  90                  95

Gly Pro Gly Leu Ser Arg Thr Gly Ala Leu Ser Thr Gly Ala Leu Pro
            100                 105                 110

Pro Leu Ala Pro Glu Gly Asp Ser Val Ala Ser Lys His Ala Ile Tyr
        115                 120                 125

Ala Val Gln Val Ile Ala Asp Pro Pro Gly Pro Gly Glu Gly Pro Pro
    130                 135                 140

Ala Gln His Ala Ala Phe Leu Val Pro Leu Gly Pro Gly Gln Ile Ser
145                 150                 155                 160

Thr Glu Val Gln Ala Pro Pro Pro Val Val Asn Val Arg Val His His
                165                 170                 175

Pro Pro Glu Ala Ser Val Gln Val His Arg Ile Glu Ser Ser Asn Ala
            180                 185                 190

Glu Ser Ala Ala Pro Ser Gln Leu Glu
        195                 200

<210>107
<211>603
<212>DNA
<213>Homo sapiens

<220>
<221> CDS
<222>(1)..(603)

<400>107

gga tcc cgc ttc aag gtg gtc ttt gcg ccg gtg atc tgc aag cgg acc     48
Gly Ser Arg Phe Lys Val Val Phe Ala Pro Val Ile Cys Lys Arg Thr
  1               5                  10                  15

tgt ctc aag ggc cag tgt cgg gac agt tgt ccg cag ggc tcc aac atg     96
Cys Leu Lys Gly Gln Cys Arg Asp Ser Cys Pro Gln Gly Ser Asn Met
             20                  25                  30

acg ctc atc gga gag aac ggc cac agc aca gac acg ctc acg ggc tcc     144
Thr Leu Ile Gly Glu Asn Gly His Ser Thr Asp Thr Leu Thr Gly Ser
         35                  40                  45

ggc ttc cgc gtg gtg gtg tgc cct ctc ccc tgc acg aat ggc ggc cag     192
Gly Phe Arg Val Val Val Cys Pro Leu Pro Cys Thr Asn Gly Gly Gln
     50                  55                  60

tgc tcc tcg cga aac cag tgc ctg tgt ccc ccg gac ttc act ggg cgc     240
Cys Ser Ser Arg Asn Gln Cys Leu Cys Pro Pro Asp Phe Thr Gly Arg

65                  70                  75                  80

ttc tgc cag gtg ccc gca gga gga gcc ggt ggg ggt acc ggc ggc tca     288
Phe Cys Gln Val Pro Ala Gly Gly Ala Gly Gly Gly Thr Gly Gly Ser
                85                  90                  95

ggc ccc ggc ctg agc agg aca ggg gcc ctg tcc aca ggg gcg ctg ccg     336
Gly Pro Gly Leu Ser Arg Thr Gly Ala Leu Ser Thr Gly Ala Leu Pro
            100                 105                 110

ccc ctg gct ccg gag ggc gac tct gtg gcc agc aag cac gcc atc tac     384
Pro Leu Ala Pro Glu Gly Asp Ser Val Ala Ser Lys His Ala Ile Tyr
        115                 120                 125

gcc gtc cag gtg atc gct gac cct cct ggg ccc ggg gag ggg cct cct     432
Ala Val Gln Val Ile Ala Asp Pro Pro Gly Pro Gly Glu Gly Pro Pro
    130                 135                 140

gcc cag cac gca gcc ttc ctg gtg ccc cta ggc ccg gga cag atc tca     480
Ala Gln His Ala Ala Phe Leu Val Pro Leu Gly Pro Gly Gln Ile Ser
145                 150                 155                 160

gca gaa gtg cag gcc ccg ccc ccc gtg gtg aat gtg cgc gtc cat cac     528
Ala Glu Val Gln Ala Pro Pro Pro Val Val Asn Val Arg Val His His
                165                 170                 175

ccg ccc gag gcc tca gtc cag gtg cac cgc att gag agc tcg aac gcc     576
Pro Pro Glu Ala Ser Val Gln Val His Arg Ile Glu Ser Ser Asn Ala
            180                 185                 190

gag agc gca gcc ccc tcc cag ctc gag                                 603
Glu Ser Ala Ala Pro Ser Gln Leu Glu
        195                 200

<210>108
<211>201
<212>PRT
<213>Homo sapiens

<400>108

Gly Ser Arg Phe Lys Val Val Phe Ala Pro Val Ile Cys Lys Arg Thr
  1               5                  10                  15

Cys Leu Lys Gly Gln Cys Arg Asp Ser Cys Pro Gln Gly Ser Asn Met
             20                  25                  30

Thr Leu Ile Gly Glu Asn Gly His Ser Thr Asp Thr Leu Thr Gly Ser
         35                  40                  45

Gly Phe Arg Val Val Val Cys Pro Leu Pro Cys Thr Asn Gly Gly Gln
     50                  55                  60

Cys Ser Ser Arg Asn Gln Cys Leu Cys Pro Pro Asp Phe Thr Gly Arg
 65                  70                  75                  80

Phe Cys Gln Val Pro Ala Gly Gly Ala Gly Gly Gly Thr Gly Gly Ser

85                    90                    95

Gly Pro Gly Leu Ser Arg Thr Gly Ala Leu Ser Thr Gly Ala Leu Pro
            100                 105                 110

Pro Leu Ala Pro Glu Gly Asp Ser Val Ala Ser Lys His Ala Ile Tyr
        115                 120                 125

Ala Val Gln Val Ile Ala Asp Pro Pro Gly Pro Gly Glu Gly Pro Pro
    130                 135                 140

Ala Gln His Ala Ala Phe Leu Val Pro Leu Gly Pro Gly Gln Ile Ser
145                 150                 155                 160

Ala Glu Val Gln Ala Pro Pro Pro Val Val Asn Val Arg Val His His
                165                 170                 175

Pro Pro Glu Ala Ser Val Gln Val His Arg Ile Glu Ser Ser Asn Ala
            180                 185                 190

Glu Ser Ala Ala Pro Ser Gln Leu Glu
        195                 200

<210>109
<211>603
<212>DNA
<213>Homo sapiens

<220>
<221> CDS
<222>(1)..(603)

<400>109

gga tcc cgc ttc aag gtg gtc ttt gcg ccg gtg atc tgc aag cgg acc      48
Gly Ser Arg Phe Lys Val Val Phe Ala Pro Val Ile Cys Lys Arg Thr
  1               5                  10                  15

tgt ctc gag ggc cag tgt cgg gac agt tgt cag cag ggc tcc aac atg      96
Cys Leu Glu Gly Gln Cys Arg Asp Ser Cys Gln Gln Gly Ser Asn Met
             20                  25                  30

acg ctc atc gga gag aac ggc cac agc aca gac acg ctc acg ggc tcc     144
Thr Leu Ile Gly Glu Asn Gly His Ser Thr Asp Thr Leu Thr Gly Ser
         35                  40                  45

ggc ttc cgc gtg gtg gtg tgc cct ctc ccc tgc atg aat ggc ggc cag     192
Gly Phe Arg Val Val Val Cys Pro Leu Pro Cys Met Asn Gly Gly Gln
     50                  55                  60

tgc tcc tcg cga aac cag tgc ctg tgt ccc ccg gac ttc act ggg cgc     240
Cys Ser Ser Arg Asn Gln Cys Leu Cys Pro Pro Asp Phe Thr Gly Arg
 65                  70                  75                  80

ttc tgc cag gtg ccc gca gga gga gcc ggt ggg ggt acc ggc ggc tca     288
Phe Cys Gln Val Pro Ala Gly Gly Ala Gly Gly Gly Thr Gly Gly Ser
                 85                  90                  95

ggc ccc ggc ctg agc agg aca ggg gcc ctg tcc aca ggg gcg ctg ccg 336
Gly Pro Gly Leu Ser Arg Thr Gly Ala Leu Ser Thr Gly Ala Leu Pro
            100                 105                 110

ccc ctg gct ccg gag ggc gac tct gtg gcc agc aag cac gcc atc tac 384
Pro Leu Ala Pro Glu Gly Asp Ser Val Ala Ser Lys His Ala Ile Tyr
        115                 120                 125

gcc gtc cag gtg atc gct gac cct cct ggg ccc ggg gag ggg cct cct 432
Ala Val Gln Val Ile Ala Asp Pro Pro Gly Pro Gly Glu Gly Pro Pro
    130                 135                 140

gcc cag cac gca gcc ttc ctg gtg ccc cta ggc ccg gga cag atc tca 480
Ala Gln His Ala Ala Phe Leu Val Pro Leu Gly Pro Gly Gln Ile Ser
145                 150                 155                 160

gca gaa gtg cag gcc ccg ccc ccc gtg gtg aat gtg cgc gtc cat cac 528
Ala Glu Val Gln Ala Pro Pro Pro Val Val Asn Val Arg Val His His
                165                 170                 175

ccg ccc gag gcc tca gtc cag gtg cac cgc att gag agc tcg aac gcc 576
Pro Pro Glu Ala Ser Val Gln Val His Arg Ile Glu Ser Ser Asn Ala
            180                 185                 190

gag agc gca gcc ccc tcc cag ctc gag 603
Glu Ser Ala Ala Pro Ser Gln Leu Glu
        195                 200

<210>110
<211>201
<212>PRT
<213>Homo sapiens

<400>110

Gly Ser Arg Phe Lys Val Val Phe Ala Pro Val Ile Cys Lys Arg Thr
1               5                   10                  15

Cys Leu Glu Gly Gln Cys Arg Asp Ser Cys Gln Gln Gly Ser Asn Met
            20                  25                  30

Thr Leu Ile Gly Glu Asn Gly His Ser Thr Asp Thr Leu Thr Gly Ser
        35                  40                  45

Gly Phe Arg Val Val Val Cys Pro Leu Pro Cys Met Asn Gly Gly Gln
    50                  55                  60

Cys Ser Ser Arg Asn Gln Cys Leu Cys Pro Pro Asp Phe Thr Gly Arg
65                  70                  75                  80

Phe Cys Gln Val Pro Ala Gly Gly Ala Gly Gly Gly Thr Gly Gly Ser
                85                  90                  95

Gly Pro Gly Leu Ser Arg Thr Gly Ala Leu Ser Thr Gly Ala Leu Pro
            100                 105                 110

Pro Leu Ala Pro Glu Gly Asp Ser Val Ala Ser Lys His Ala Ile Tyr
        115                 120                 125

Ala Val Gln Val Ile Ala Asp Pro Pro Gly Pro Gly Glu Gly Pro Pro
    130                 135                 140

Ala Gln His Ala Ala Phe Leu Val Pro Leu Gly Pro Gly Gln Ile Ser
145                 150                 155                 160

Ala Glu Val Gln Ala Pro Pro Pro Val Val Asn Val Arg Val His His
                165                 170                 175

Pro Pro Glu Ala Ser Val Gln Val His Arg Ile Glu Ser Ser Asn Ala
            180                 185                 190

Glu Ser Ala Ala Pro Ser Gln Leu Glu
        195                 200

<210>111
<211>1846
<212>DNA
<213>Homo sapiens

<220>
<221> CDS
<222>(4)..(1828)

<400>111

atc atg cac tcc cag aga cct cca ctt gca ctc ctt gag ggc tcc acc 48
Met His Ser Gln Arg Pro Pro Leu Ala Leu Leu Glu Gly Ser Thr
1               5                   10                  15

ctc gat aga aaa gga gaa atc gca gct tca ctc gtc tct agg ctg tgg 96
Leu Asp Arg Lys Gly Glu Ile Ala Ala Ser Leu Val Ser Arg Leu Trp
                20                  25                  30

aaa gtc tcc aat tca act ctg ttc caa atg atg ctg gtc act gtt ttg 144
Lys Val Ser Asn Ser Thr Leu Phe Gln Met Met Leu Val Thr Val Leu
            35                  40                  45

ttg gct acc att ctt ggt gac tgt ggt cct cca cct gag tta cca ttt 192
Leu Ala Thr Ile Leu Gly Asp Cys Gly Pro Pro Pro Glu Leu Pro Phe
        50                  55                  60

gct ttt cca ata aat ccg ttg tat gat act gaa ttc aaa act gga act 240
Ala Phe Pro Ile Asn Pro Leu Tyr Asp Thr Glu Phe Lys Thr Gly Thr
    65                  70                  75

act ctg aag tac acc tgc cac cct ggg cat ggt aaa atc aat tca agt 288
Thr Leu Lys Tyr Thr Cys His Pro Gly His Gly Lys Ile Asn Ser Ser
80                  85                  90                  95

cga ctg att tgt gat gcc aaa gac tcg tgg aac tat agt atc ttt tgt 336
Arg Leu Ile Cys Asp Ala Lys Asp Ser Trp Asn Tyr Ser Ile Phe Cys
                100                 105                 110

gca agt aag cga tgc aga aat cca gaa tta atc aat ggg ata gtg gaa 384
Ala Ser Lys Arg Cys Arg Asn Pro Glu Leu Ile Asn Gly Ile Val Glu
            115                 120                 125

gtt aaa aaa gat ctt ctc ctt ggt tca acc ata aaa ttc agc tgc tca 432
Val Lys Lys Asp Leu Leu Leu Gly Ser Thr Ile Lys Phe Ser Cys Ser
        130                 135                 140

gag ggg ttt ttc tta att ggc tca acc acc agt cat tgt cag atc caa 480
Glu Gly Phe Phe Leu Ile Gly Ser Thr Thr Ser His Cys Gln Ile Gln
    145                 150                 155

ggt aaa gga gtt gat tgg agt gat cct ctc cca gaa acc tca gtt gcc 528
Gly Lys Gly Val Asp Trp Ser Asp Pro Leu Pro Glu Thr Ser Val Ala
160                 165                 170                 175

aag tgc gag ccc cct cca gac atc agg aat ggg aag cac agc ggt gga 576
Lys Cys Glu Pro Pro Pro Asp Ile Arg Asn Gly Lys His Ser Gly Gly
                180                 185                 190

gat caa gaa ttc tac aca tat gcc tcc tct gtc acc tac agc tgc aac 624
Asp Gln Glu Phe Tyr Thr Tyr Ala Ser Ser Val Thr Tyr Ser Cys Asn
            195                 200                 205

ccc tac ttc tca ctc ata ggc aac gtc tcc atc tcc tgc acc gtg gag 672
Pro Tyr Phe Ser Leu Ile Gly Asn Val Ser Ile Ser Cys Thr Val Glu
        210                 215                 220

aat gaa aca ata ggt gtc tgg agc cca aac cct cct atc tgt gaa gaa 720
Asn Glu Thr Ile Gly Val Trp Ser Pro Asn Pro Pro Ile Cys Glu Glu
    225                 230                 235

att gtc tgt cgt cga cca cag att cca aag gca atc ttt gtt tct gga 768
Ile Val Cys Arg Arg Pro Gln Ile Pro Lys Ala Ile Phe Val Ser Gly
240                 245                 250                 255

ttt gga ccc ctc tat act tac aaa gac tct att atg gtt aac tgt gag 816
Phe Gly Pro Leu Tyr Thr Tyr Lys Asp Ser Ile Met Val Asn Cys Glu
                260                 265                 270

gaa ggt tat atc ctc aga ggc agc agt tta atc tat tgt gaa acg aat 864
Glu Gly Tyr Ile Leu Arg Gly Ser Ser Leu Ile Tyr Cys Glu Thr Asn
            275                 280                 285

aat gag tgg tat cct tct gtt ccc tct tgc aga gtg aat ggt tgc act 912
Asn Glu Trp Tyr Pro Ser Val Pro Ser Cys Arg Val Asn Gly Cys Thr
        290                 295                 300

gtc cta ccg gac att tcc tat gct tcc tgg gag aga aat gac tac aac 960
Val Leu Pro Asp Ile Ser Tyr Ala Ser Trp Glu Arg Asn Asp Tyr Asn
    305                 310                 315

cta agt gat cac gaa ata ttt gaa att gga act gag ttg aaa tat cta 1008
Leu Ser Asp His Glu Ile Phe Glu Ile Gly Thr Glu Leu Lys Tyr Leu
320                 325                 330                 335

tgc aaa cct ggc tat aga cct gtt tta gat gag cct ctg act gtg act 1056

Cys Lys Pro Gly Tyr Arg Pro Val Leu Asp Glu Pro Leu Thr Val Thr
                340                 345                 350

tgt cag gaa aat ttg aca tgg aca tct tcc aat gag tgt gag agt gta 1104
Cys Gln Glu Asn Leu Thr Trp Thr Ser Ser Asn Glu Cys Glu Ser Val
            355                 360                 365

tgt tgc cca aca cca gat ctg gag aat atc aga atc ata aat gaa agg 1152
Cys Cys Pro Thr Pro Asp Leu Glu Asn Ile Arg Ile Ile Asn Glu Arg
        370                 375                 380

agg tat ttc act ggt aga tgt gtc tat gcc tat gga gac tat att tca 1200
Arg Tyr Phe Thr Gly Arg Cys Val Tyr Ala Tyr Gly Asp Tyr Ile Ser
    385                 390                 395

tat atg tgt gat gaa ggc tat tac cct att tct gtt gac ggg gag agt 1248
Tyr Met Cys Asp Glu Gly Tyr Tyr Pro Ile Ser Val Asp Gly Glu Ser
400                 405                 410                 415

tcc tgc cac aca gat ggc aca tgg aag cct aaa atg cca gca tgt gag 1296
Ser Cys His Thr Asp Gly Thr Trp Lys Pro Lys Met Pro Ala Cys Glu
                420                 425                 430

cca ggt tgc agt ttt gcc cct agt ttt gcc cat ggg cat cct aaa caa 1344
Pro Gly Cys Ser Phe Ala Pro Ser Phe Ala His Gly His Pro Lys Gln
            435                 440                 445

gtt aat tta tgc aac tgt ttc aaa aat gag gct gta tat aaa tgt gat 1392
Val Asn Leu Cys Asn Cys Phe Lys Asn Glu Ala Val Tyr Lys Cys Asp
        450                 455                 460

gaa ggc tac act gtg atc gga cag gtg aaa ctc acc tgc att tct tcc 1440
Glu Gly Tyr Thr Val Ile Gly Gln Val Lys Leu Thr Cys Ile Ser Ser
    465                 470                 475

tgc tgg tca tct cca gcc cct caa tgt aaa agt ctg tgt ctg aaa cca 1488
Cys Trp Ser Ser Pro Ala Pro Gln Cys Lys Ser Leu Cys Leu Lys Pro
480                 485                 490                 495

gaa ata gtg aat gga agg ctg tct gtg gat aag gat cag tat gtt gag 1536
Glu Ile Val Asn Gly Arg Leu Ser Val Asp Lys Asp Gln Tyr Val Glu
                500                 505                 510

tct gaa aat gtt acc att gaa tgt gat tct ggc tat ggt gtg gtt ggt 1584
Ser Glu Asn Val Thr Ile Glu Cys Asp Ser Gly Tyr Gly Val Val Gly
            515                 520                 525

ctc aaa agt atc act tgc tca gag aag aga acc tgg tac cca gaa gtg 1632
Leu Lys Ser Ile Thr Cys Ser Glu Lys Arg Thr Trp Tyr Pro Glu Val
        530                 535                 540

ccc agg tgt gag tgg gag gca cct gaa ggt tgt gag caa gtg ctc aca 1680
Pro Arg Cys Glu Trp Glu Ala Pro Glu Gly Cys Glu Gln Val Leu Thr
    545                 550                 555

ggc aga aaa ctc atg cag tgt ctc cca agc cca gag gat gtg aaa gtg 1728
Gly Arg Lys Leu Met Gln Cys Leu Pro Ser Pro Glu Asp Val Lys Val

560                 565                 570                 575

gcc ctg gag gtg tat aag ctg tct ctg gag ata aaa caa ctt gaa aaa    1776
Ala Leu Glu Val Tyr Lys Leu Ser Leu Glu Ile Lys Gln Leu Glu Lys
                580                 585                 590

gag aga gac aaa ttg atg aac acc cat cag aaa ttt tct gaa aaa gag    1824
Glu Arg Asp Lys Leu Met Asn Thr His Gln Lys Phe Ser Glu Lys Glu
            595                 600                 605

gaa tgaaggactt atttttccc                                           1846
Glu

<210>112
<211>608
<212>PRT
<213>Homo sapiens

<400>112

Met His Ser Gln Arg Pro Pro Leu Ala Leu Leu Glu Gly Ser Thr Leu
 1               5                  10                  15

Asp Arg Lys Gly Glu Ile Ala Ala Ser Leu Val Ser Arg Leu Trp Lys
            20                  25                  30

Val Ser Asn Ser Thr Leu Phe Gln Met Met Leu Val Thr Val Leu Leu
        35                  40                  45

Ala Thr Ile Leu Gly Asp Cys Gly Pro Pro Pro Glu Leu Pro Phe Ala
    50                  55                  60

Phe Pro Ile Asn Pro Leu Tyr Asp Thr Glu Phe Lys Thr Gly Thr Thr
65                  70                  75                  80

Leu Lys Tyr Thr Cys His Pro Gly His Gly Lys Ile Asn Ser Ser Arg
                85                  90                  95

Leu Ile Cys Asp Ala Lys Asp Ser Trp Asn Tyr Ser Ile Phe Cys Ala
            100                 105                 110

Ser Lys Arg Cys Arg Asn Pro Glu Leu Ile Asn Gly Ile Val Glu Val
        115                 120                 125

Lys Lys Asp Leu Leu Leu Gly Ser Thr Ile Lys Phe Ser Cys Ser Glu
    130                 135                 140

Gly Phe Phe Leu Ile Gly Ser Thr Thr Ser His Cys Gln Ile Gln Gly
145                 150                 155                 160

Lys Gly Val Asp Trp Ser Asp Pro Leu Pro Glu Thr Ser Val Ala Lys
                165                 170                 175

Cys Glu Pro Pro Pro Asp Ile Arg Asn Gly Lys His Ser Gly Gly Asp
            180                 185                 190

Gln Glu Phe Tyr Thr Tyr Ala Ser Ser Val Thr Tyr Ser Cys Asn Pro
        195                 200                 205

Tyr Phe Ser Leu Ile Gly Asn Val Ser Ile Ser Cys Thr Val Glu Asn
    210                 215                 220

Glu Thr Ile Gly Val Trp Ser Pro Asn Pro Pro Ile Cys Glu Glu Ile
225                 230                 235                 240

Val Cys Arg Arg Pro Gln Ile Pro Lys Ala Ile Phe Val Ser Gly Phe
                245                 250                 255

Gly Pro Leu Tyr Thr Tyr Lys Asp Ser Ile Met Val Asn Cys Glu Glu
            260                 265                 270

Gly Tyr Ile Leu Arg Gly Ser Ser Leu Ile Tyr Cys Glu Thr Asn Asn
        275                 280                 285

Glu Trp Tyr Pro Ser Val Pro Ser Cys Arg Val Asn Gly Cys Thr Val
    290                 295                 300

Leu Pro Asp Ile Ser Tyr Ala Ser Trp Glu Arg Asn Asp Tyr Asn Leu
305                 310                 315                 320

Ser Asp His Glu Ile Phe Glu Ile Gly Thr Glu Leu Lys Tyr Leu Cys
                325                 330                 335

Lys Pro Gly Tyr Arg Pro Val Leu Asp Glu Pro Leu Thr Val Thr Cys
            340                 345                 350

Gln Glu Asn Leu Thr Trp Thr Ser Ser Asn Glu Cys Glu Ser Val Cys
        355                 360                 365

Cys Pro Thr Pro Asp Leu Glu Asn Ile Arg Ile Ile Asn Glu Arg Arg
    370                 375                 380

Tyr Phe Thr Gly Arg Cys Val Tyr Ala Tyr Gly Asp Tyr Ile Ser Tyr
385                 390                 395                 400

Met Cys Asp Glu Gly Tyr Tyr Pro Ile Ser Val Asp Gly Glu Ser Ser
                405                 410                 415

Cys His Thr Asp Gly Thr Trp Lys Pro Lys Met Pro Ala Cys Glu Pro
            420                 425                 430

Gly Cys Ser Phe Ala Pro Ser Phe Ala His Gly His Pro Lys Gln Val
        435                 440                 445

Asn Leu Cys Asn Cys Phe Lys Asn Glu Ala Val Tyr Lys Cys Asp Glu
    450                 455                 460

Gly Tyr Thr Val Ile Gly Gln Val Lys Leu Thr Cys Ile Ser Ser Cys
465                 470                 475                 480

Trp Ser Ser Pro Ala Pro Gln Cys Lys Ser Leu Cys Leu Lys Pro Glu
                485                 490                 495

Ile Val Asn Gly Arg Leu Ser Val Asp Lys Asp Gln Tyr Val Glu Ser
            500                 505                 510

Glu Asn Val Thr Ile Glu Cys Asp Ser Gly Tyr Gly Val Val Gly Leu
        515                 520                 525

Lys Ser Ile Thr Cys Ser Glu Lys Arg Thr Trp Tyr Pro Glu Val Pro
    530                 535                 540

Arg Cys Glu Trp Glu Ala Pro Glu Gly Cys Glu Gln Val Leu Thr Gly
545                 550                 555                 560

Arg Lys Leu Met Gln Cys Leu Pro Ser Pro Glu Asp Val Lys Val Ala
                565                 570                 575

Leu Glu Val Tyr Lys Leu Ser Leu Glu Ile Lys Gln Leu Glu Lys Glu
            580                 585                 590

Arg Asp Lys Leu Met Asn Thr His Gln Lys Phe Ser Glu Lys Glu Glu
        595                 600                 605

<210>113
<211>2974
<212>DNA
<213>Homo sapiens

<220>
<221> CDS
<222>(193)..(2119)

<400>113

cggggactcg gaggtactgg gcgcgcgcgg ctccggctcg ggacgcctcg 50

ggacgcctcg gggtcgggtt ccggttgcgg ctgctgctgc ggcgcccgcg 100

cttccgtagc gttccgcctc ctgtgcccgc cgcggagcaa gtctgcgcgc 150

ccgccgtgcg cccctaagct ccttttacct gagcccgccg cg atg gga gct gcg 204
Met Gly Ala Ala
1

cgg gga tcc ccg gcc aga ccc cgc cgg ttg cct ctg ctc agc gtc ctg 252
Arg Gly Ser Pro Ala Arg Pro Arg Arg Leu Pro Leu Leu Ser Val Leu
5                   10                  15                  20

ctg ctg ccg ctg ctg ggc ggt acc cag aca gcc att gtc ttc atc aag 300
Leu Leu Pro Leu Leu Gly Gly Thr Gln Thr Ala Ile Val Phe Ile Lys
                25                  30                  35

cag ccg tcc tcc cag gat gca ctg cag ggg cgc cgg gcg ctg ctt cgc 348

Gln Pro Ser Ser Gln Asp Ala Leu Gln Gly Arg Arg Ala Leu Leu Arg
            40                  45                  50

tgt gag gtt gag gct ccg ggc ccg gta cat gtg tac tgg ctg ctc gat 396
Cys Glu Val Glu Ala Pro Gly Pro Val His Val Tyr Trp Leu Leu Asp
        55                  60                  65

ggg gcc cct gtc cag gac acg gag cgg cgt ttc gcc cag ggc agc agc 444
Gly Ala Pro Val Gln Asp Thr Glu Arg Arg Phe Ala Gln Gly Ser Ser
    70                  75                  80

ctg agc ttt gca gct gtg gac ccg ctg cag gac tct ggc acc ttc cag 492
Leu Ser Phe Ala Ala Val Asp Pro Leu Gln Asp Ser Gly Thr Phe Gln
85                  90                  95                  100

tgt gtg gct cgg gat gat gtc act gga gaa gaa gcc cgc agt gcc aac 540
Cys Val Ala Arg Asp Asp Val Thr Gly Glu Glu Ala Arg Ser Ala Asn
                105                 110                 115

gcc tcc ttc aac atc aaa tgg att gag gca ggt cct gtg gtc ctg aag 588
Ala Ser Phe Asn Ile Lys Trp Ile Glu Ala Gly Pro Val Val Leu Lys
            120                 125                 130

cat cca gcc tcg gaa gct gag atc cag cca cag acc cag gtc aaa ctt 636
His Pro Ala Ser Glu Ala Glu Ile Gln Pro Gln Thr Gln Val Lys Leu
        135                 140                 145

cgt tgc cac att gat ggg cac cct cgg ccc acc tac caa tgg ttc cga 684
Arg Cys His Ile Asp Gly His Pro Arg Pro Thr Tyr Gln Trp Phe Arg
    150                 155                 160

gat ggg acc ccc ctt tct gat ggt cag agc aac cac aca gtc agc agc 732
Asp Gly Thr Pro Leu Ser Asp Gly Gln Ser Asn His Thr Val Ser Ser
165                 170                 175                 180

aag gag cgg aac ctg acg ctc cgg cca gct ggt cct gag cat agt ggg 780
Lys Glu Arg Asn Leu Thr Leu Arg Pro Ala Gly Pro Glu His Ser Gly
                185                 190                 195

ctg tat tcc tgc tgc gcc cac agt gct ttt ggc cag gct tgc agc agc 828
Leu Tyr Ser Cys Cys Ala His Ser Ala Phe Gly Gln Ala Cys Ser Ser
            200                 205                 210

cag aac ttc acc ttg agc att gct gat gaa agc ttt gcc agg gtg gtg 876
Gln Asn Phe Thr Leu Ser Ile Ala Asp Glu Ser Phe Ala Arg Val Val
        215                 220                 225

ctg gca ccc cag gac gtg gta gta gcg agg tat gag gag gcc atg ttc 924
Leu Ala Pro Gln Asp Val Val Val Ala Arg Tyr Glu Glu Ala Met Phe
    230                 235                 240

cat tgc cag ttc tca gcc cag cca ccc ccg agc ctg cag tgg ctc ttt 972
His Cys Gln Phe Ser Ala Gln Pro Pro Pro Ser Leu Gln Trp Leu Phe
245                 250                 255                 260

gag gat gag act ccc atc act aac cgc agt cgc ccc cca cac ctc cgc 1020
Glu Asp Glu Thr Pro Ile Thr Asn Arg Ser Arg Pro Pro His Leu Arg

                265                 270                 275

aga gcc aca gtg ttt gcc aac ggg tct ctg ctg ctg acc cag gtc cgg 1068
Arg Ala Thr Val Phe Ala Asn Gly Ser Leu Leu Leu Thr Gln Val Arg
            280                 285                 290

cca cgc aat gca ggg atc tac cgc tgc att ggc cag ggg cag agg ggc 1116
Pro Arg Asn Ala Gly Ile Tyr Arg Cys Ile Gly Gln Gly Gln Arg Gly
        295                 300                 305

cca ccc atc atc ctg gaa gcc aca ctt cac cta gca gag att gaa gac 1164
Pro Pro Ile Ile Leu Glu Ala Thr Leu His Leu Ala Glu Ile Glu Asp
    310                 315                 320

atg ccg cta ttt gag cca cgg gtg ttt aca gct ggc agc gag gag cgt 1212
Met Pro Leu Phe Glu Pro Arg Val Phe Thr Ala Gly Ser Glu Glu Arg
325                 330                 335                 340

gtg acc tgc ctt ccc ccc aag ggt ctg cca gag ccc agc gtg tgg tgg 1260
Val Thr Cys Leu Pro Pro Lys Gly Leu Pro Glu Pro Ser Val Trp Trp
                345                 350                 355

gag cac gcg gga gtc cgg ctg ccc acc cat ggc agg gtc tac cag aag 1308
Glu His Ala Gly Val Arg Leu Pro Thr His Gly Arg Val Tyr Gln Lys
            360                 365                 370

ggc cac gag ctg gtg ttg gcc aat att gct gaa agt gat gct ggt gtc 1356
Gly His Glu Leu Val Leu Ala Asn Ile Ala Glu Ser Asp Ala Gly Val
        375                 380                 385

tac acc tgc cac gcg gcc aac ctg gct ggt cag cgg aga cag gat gcc 1404
Tyr Thr Cys His Ala Ala Asn Leu Ala Gly Gln Arg Arg Gln Asp Ala
    390                 395                 400

aac atc act gtg gcc act gtg ccc tcc tgg ctg aag aag ccc caa gac 1452
Asn Ile Thr Val Ala Thr Val Pro Ser Trp Leu Lys Lys Pro Gln Asp
405                 410                 415                 420

agc cag ctg gag gag ggc aaa ccc ggc tac ttg gat tgc ctg acc cag 1500
Ser Gln Leu Glu Glu Gly Lys Pro Gly Tyr Leu Asp Cys Leu Thr Gln
                425                 430                 435

gcc aca cca aaa cct aca gtt gtc tgg tac aga aac cag atg ctc atc 1548
Ala Thr Pro Lys Pro Thr Val Val Trp Tyr Arg Asn Gln Met Leu Ile
            440                 445                 450

tca gag gac tca cgg ttc gag gtc ttc aag aat ggg acc ttg cgc atc 1596
Ser Glu Asp Ser Arg Phe Glu Val Phe Lys Asn Gly Thr Leu Arg Ile
        455                 460                 465

aac agc gtg gag gtg tat gat ggg aca tgg tac cgt tgt atg agc agc 1644
Asn Ser Val Glu Val Tyr Asp Gly Thr Trp Tyr Arg Cys Met Ser Ser
    470                 475                 480

acc cca gcc ggc agc atc gag gcg caa gcc cgt gtc caa gtg ctg gaa 1692
Thr Pro Ala Gly Ser Ile Glu Ala Gln Ala Arg Val Gln Val Leu Glu
485                 490                 495                 500

aag ctc aag ttc aca cca cca ccc cag cca cag cag tgc atg gag ttt 1740
Lys Leu Lys Phe Thr Pro Pro Pro Gln Pro Gln Gln Cys Met Glu Phe
                505                 510                 515

gac aag gag gcc acg gtg ccc tgt tca gcc aca ggc cga gag aag ccc 1788
Asp Lys Glu Ala Thr Val Pro Cys Ser Ala Thr Gly Arg Glu Lys Pro
            520                 525                 530

act att aag tgg gaa cgg gca gat ggg agc agc ctc cca gag tgg gtg 1836
Thr Ile Lys Trp Glu Arg Ala Asp Gly Ser Ser Leu Pro Glu Trp Val
        535                 540                 545

aca gac aac gct ggg acc ctg cat ttt gcc cgg gtg act cga gat gac 1884
Thr Asp Asn Ala Gly Thr Leu His Phe Ala Arg Val Thr Arg Asp Asp
    550                 555                 560

gct ggc aac tac act tgc att gcc tcc aac ggg ccg cag ggc cag att 1932
Ala Gly Asn Tyr Thr Cys Ile Ala Ser Asn Gly Pro Gln Gly Gln Ile
565                 570                 575                 580

cgt gcc cat gtc cag ctc act gtg gca gtt ttt atc acc ttc aaa gtg 1980
Arg Ala His Val Gln Leu Thr Val Ala Val Phe Ile Thr Phe Lys Val
                585                 590                 595

gaa cca gag cgt acg act gtg tac cct tcc aaa ctc tat cgg ctg atg 2028
Glu Pro Glu Arg Thr Thr Val Tyr Pro Ser Lys Leu Tyr Arg Leu Met
            600                 605                 610

cag cgc tgc tgg gcc ctc agc ccc aag gac cgg ccc tcc ttc agt gag 2076
Gln Arg Cys Trp Ala Leu Ser Pro Lys Asp Arg Pro Ser Phe Ser Glu
        615                 620                 625

att gcc agc gcc ctg gga gac agc acc gtg gac agc aag ccg 2118
Ile Ala Ser Ala Leu Gly Asp Ser Thr Val Asp Ser Lys Pro
    630                 635                 640

tgaggaggga gcccgctcag gatggcctgg gcaggggagg acatctctag 2168

agggaagctc acagcatgat gggcaagatc cctgtcctcc tgggccctga 2218

ggcccctgcc ctagtgcaac aggcattgct gaggtctgag cagggcctgg 2268

cctttcctcc tcttcctcac cctcatcctt tgggaggctg acttggaccc 2318

aaactgggcg actagggctt tgagctgggc agttttccct gccacctctt 2368

cctctatcag ggacagtgtg ggtgccacag gtaaccccaa tttctggcct 2418

tcaacttctc cccttgaccg ggtccaactc tgccactcat ctgccaactt 2468

tgcctgggga gggctaggct tgggatgagc tgggtttgtg gggagttcct 2518

taatattctc aagttctggg cacacagggt taatgagtct cttggcccac 2568

tggtcccact tgggggtcta gaccaggatt atagaggaca cagcaagtga 2618

gtcctcccca ctctgggctt gtgcacactg acccagaccc acggtcttcc 2668

ccacccttct ctcctttcct catcctaagt gcctggcaga tgaaggagtt 2718

ttcaggagct tttgacacta tataaaccgc cctttttgta tgcaccacgg 2768

gcggctttta tatgtaattg cagcgtgggg tgggtgggca tgggaggtag 2818

gggtgggccc tggagatgag gagggtgggc catccttacc ccacactttt 2868

attgttgtcg ttttttgtgt gtttgtgttt ttttgttttt gtttttgttt 2918

ttacactcgc tgctctcaat aaataagcct ttttaaaaaa aaaaaaaaaa 2968

aaaaaa 2974

<210>114
<211>642
<212>PRT
<213>Homo sapiens

<400>114

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Pro Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Lys Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln

    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Ala Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly
    450                 455                 460

Thr Leu Arg Ile Asn Ser Val Glu Val Tyr Asp Gly Thr Trp Tyr Arg
465                 470                 475                 480

Cys Met Ser Ser Thr Pro Ala Gly Ser Ile Glu Ala Gln Ala Arg Val
                485                 490                 495

Gln Val Leu Glu Lys Leu Lys Phe Thr Pro Pro Pro Gln Pro Gln Gln
            500                 505                 510

Cys Met Glu Phe Asp Lys Glu Ala Thr Val Pro Cys Ser Ala Thr Gly
        515                 520                 525

Arg Glu Lys Pro Thr Ile Lys Trp Glu Arg Ala Asp Gly Ser Ser Leu
    530                 535                 540

Pro Glu Trp Val Thr Asp Asn Ala Gly Thr Leu His Phe Ala Arg Val
545                 550                 555                 560

Thr Arg Asp Asp Ala Gly Asn Tyr Thr Cys Ile Ala Ser Asn Gly Pro
                565                 570                 575

Gln Gly Gln Ile Arg Ala His Val Gln Leu Thr Val Ala Val Phe Ile
            580                 585                 590

Thr Phe Lys Val Glu Pro Glu Arg Thr Thr Val Tyr Pro Ser Lys Leu

        595                 600                 605

Tyr Arg Leu Met Gln Arg Cys Trp Ala Leu Ser Pro Lys Asp Arg Pro
    610                 615                 620

Ser Phe Ser Glu Ile Ala Ser Ala Leu Gly Asp Ser Thr Val Asp Ser
625                 630                 635                 640

Lys Pro

<210>115
<211>3693
<212>DNA
<213>Homo sapiens

<220>
<221> CDS
<222>(478)..(3040)

<400>115

cttttcctga gcccgccgcg atgggagctg cgcggggatc cccggccaga 50

ccccgccggt tgcctctgct cagcgtcctg ctgctgccgc tgctgggcgg 100

tacccagaca gccattgtct tcatcaagca gccgtcctcc caggatgcac 150

tgcaggggcg ccgggcgctg cttcgctgtg aggttgaggc tccgggcccg 200

gtacatgtgt actggctgct cgatggggcc cctgtccagg acacggagcg 250

gcgtttcgcc cagggcagca gcctgagctt tgcagctgtg gaccggctgc 300

aggactctgg caccttccag tgtgtggctc gggatgatgt cactggagaa 350

gaagcccgca gtgccaacgc ctccttcaac atcaaatgga ttgaggcagg 400

tcctgtggtc ctgaagcatc cagcctcgga agctgagatc cagccacaga 450

cccaggtcac acttcgttgc cacattg atg ggc acc ctc gat gaa agc ttt 501
Met Gly Thr Leu Asp Glu Ser Phe
1               5

gcc agg gtg gtg ctg gca ccc cag gac gtg gta gta gcg agg tat gag 549
Ala Arg Val Val Leu Ala Pro Gln Asp Val Val Val Ala Arg Tyr Glu
    10                  15                  20

gag gcc atg ttc cat tgc cag ttc tca gcc cag cca ccc ccg agc ctg 597
Glu Ala Met Phe His Cys Gln Phe Ser Ala Gln Pro Pro Pro Ser Leu
25                  30                  35                  40

cag tgg ctc ttt gag gat gag act ccc atc act aac cgc agt cgc ccc 645
Gln Trp Leu Phe Glu Asp Glu Thr Pro Ile Thr Asn Arg Ser Arg Pro
                45                  50                  55

cca cac ctc cgc aga gcc aca gtg ttt gcc aac ggg tct ctg ctg ctg 693
Pro His Leu Arg Arg Ala Thr Val Phe Ala Asn Gly Ser Leu Leu Leu
            60                  65                  70

acc cag gtc cgg cca cgc aat gca ggg atc tac cgc tgc att ggc cag 741
Thr Gln Val Arg Pro Arg Asn Ala Gly Ile Tyr Arg Cys Ile Gly Gln
        75                  80                  85

ggg cag agg ggc cca ccc atc atc ctg gaa gcc aca ctt cac cta gca 789
Gly Gln Arg Gly Pro Pro Ile Ile Leu Glu Ala Thr Leu His Leu Ala
    90                  95                  100

gag att gaa gac atg ccg cta ttt gag cca cgg gtg ttt aca gct ggc 837
Glu Ile Glu Asp Met Pro Leu Phe Glu Pro Arg Val Phe Thr Ala Gly
105                 110                 115                 120

agc gag gag cgt gtg acc tgc ctt ccc ccc aag ggt ctg cca gag ccc 885
Ser Glu Glu Arg Val Thr Cys Leu Pro Pro Lys Gly Leu Pro Glu Pro
                125                 130                 135

agc gtg tgg tgg gag cac gcg gga gtc cgg ctg ccc acc cat ggc agg 933
Ser Val Trp Trp Glu His Ala Gly Val Arg Leu Pro Thr His Gly Arg
            140                 145                 150

gtc tac cag aag ggc cac gag ctg gtg ttg gcc aat att gct gaa agt 981
Val Tyr Gln Lys Gly His Glu Leu Val Leu Ala Asn Ile Ala Glu Ser
        155                 160                 165

gat gct ggt gtc tac acc tgc cac gcg gcc aac ctg gct ggt cag cgg 1029
Asp Ala Gly Val Tyr Thr Cys His Ala Ala Asn Leu Ala Gly Gln Arg
    170                 175                 180

aga cag gat gtc aac atc act gtg gcc act gtg ccc tcc tgg ctg aag 1077
Arg Gln Asp Val Asn Ile Thr Val Ala Thr Val Pro Ser Trp Leu Lys
185                 190                 195                 200

aag ccc caa gac agc cag ctg gag gag ggc aaa ccc ggc tac ttg gat 1125
Lys Pro Gln Asp Ser Gln Leu Glu Glu Gly Lys Pro Gly Tyr Leu Asp
                205                 210                 215

tgc ctg acc cag gcc aca cca aaa cct aca gtt gtc tgg tac aga aac 1173
Cys Leu Thr Gln Ala Thr Pro Lys Pro Thr Val Val Trp Tyr Arg Asn
            220                 225                 230

cag atg ctc atc tca gag gac tca cgg ttc gag gtc ttc aag aat ggg 1221
Gln Met Leu Ile Ser Glu Asp Ser Arg Phe Glu Val Phe Lys Asn Gly
        235                 240                 245

acc ttg cgc atc aac agc gtg gag gtg tat gat ggg aca tgg tac cgt 1269
Thr Leu Arg Ile Asn Ser Val Glu Val Tyr Asp Gly Thr Trp Tyr Arg
    250                 255                 260

tgt atg agc agc acc cca gcc ggc agc atc gag gcg caa gcc cgt gtc 1317
Cys Met Ser Ser Thr Pro Ala Gly Ser Ile Glu Ala Gln Ala Arg Val
265                 270                 275                 280

caa gtg ctg gaa aag ctc aag ttc aca cca cca ccc cag cca cag cag 1365
Gln Val Leu Glu Lys Leu Lys Phe Thr Pro Pro Pro Gln Pro Gln Gln
                285                 290                 295

tgc atg gag ttt gac aag gag gcc acg gtg ccc tgt tca gcc aca ggc 1413
Cys Met Glu Phe Asp Lys Glu Ala Thr Val Pro Cys Ser Ala Thr Gly
            300                 305                 310

cga gag aag ccc act att aag tgg gaa cgg gca gat ggg agc agc ctc 1461
Arg Glu Lys Pro Thr Ile Lys Trp Glu Arg Ala Asp Gly Ser Ser Leu
        315                 320                 325

cca gag tgg gtg aca gac aac gct ggg acc ctg cat ttt gcc cgg gtg 1509
Pro Glu Trp Val Thr Asp Asn Ala Gly Thr Leu His Phe Ala Arg Val
    330                 335                 340

act cga gat gac gct ggc aac tac act tgc att gcc tcc aac ggg ccg 1557
Thr Arg Asp Asp Ala Gly Asn Tyr Thr Cys Ile Ala Ser Asn Gly Pro
345                 350                 355                 360

cag ggc cag att cgt gcc cat gtc cag ctc act gtg gca gtt ttt atc 1605
Gln Gly Gln Ile Arg Ala His Val Gln Leu Thr Val Ala Val Phe Ile
                365                 370                 375

acc ttc aaa gtg gaa cca gag cgt acg act gtg tac cag ggc cac aca 1653
Thr Phe Lys Val Glu Pro Glu Arg Thr Thr Val Tyr Gln Gly His Thr
            380                 385                 390

gcc cta ctg cag tgc gag gcc cag gga gac ccc aag ccg ctg att cag 1701
Ala Leu Leu Gln Cys Glu Ala Gln Gly Asp Pro Lys Pro Leu Ile Gln
        395                 400                 405

tgg aaa ggc aag gac cgc atc ctg gac ccc acc aag ctg gga ccc agg 1749
Trp Lys Gly Lys Asp Arg Ile Leu Asp Pro Thr Lys Leu Gly Pro Arg
    410                 415                 420

atg cac atc ttc cag aat ggc tcc ctg gtg atc cat gac gtg gcc cct 1797
Met His Ile Phe Gln Asn Gly Ser Leu Val Ile His Asp Val Ala Pro
425                 430                 435                 440

gag gac tca ggc cgc tac acc tgc att gca ggc aac agc tgc aac atc 1845

Glu Asp Ser Gly Arg Tyr Thr Cys Ile Ala Gly Asn Ser Cys Asn Ile
                445                 450                 455

aag cac acg gag gcc ccc ctc tat gtc gtg gac aag cct gtg ccg gag 1893
Lys His Thr Glu Ala Pro Leu Tyr Val Val Asp Lys Pro Val Pro Glu
            460                 465                 470

gag tcg gag ggc cct ggc agc cct ccc ccc tac aag atg atc cag acc 1941
Glu Ser Glu Gly Pro Gly Ser Pro Pro Pro Tyr Lys Met Ile Gln Thr
        475                 480                 485

att ggg ttg tcg gtg ggt gcc gct gtg gcc tac atc att gcc gtg ctg 1989
Ile Gly Leu Ser Val Gly Ala Ala Val Ala Tyr Ile Ile Ala Val Leu
    490                 495                 500

ggc ctc atg ttc tac tgc aag aag cgc tgc aaa gcc aag cgg ctg cag 2037
Gly Leu Met Phe Tyr Cys Lys Lys Arg Cys Lys Ala Lys Arg Leu Gln
505                 510                 515                 520

aag cag ccc gag ggc gag gag cca gag atg gaa tgc ctc aac ggt ggg 2085
Lys Gln Pro Glu Gly Glu Glu Pro Glu Met Glu Cys Leu Asn Gly Gly
                525                 530                 535

cct ttg cag aac ggg cag ccc tca gca gag atc caa gaa gaa gtg gcc 2133
Pro Leu Gln Asn Gly Gln Pro Ser Ala Glu Ile Gln Glu Glu Val Ala
            540                 545                 550

ttg acc agc ttg ggc tcc ggc ccc gcg gcc ccc aac aaa cgc cac agc 2181
Leu Thr Ser Leu Gly Ser Gly Pro Ala Ala Pro Asn Lys Arg His Ser
        555                 560                 565

aca agt gat aag atg cac ttc cca cgg tct agc ctg cag ccc atc acc 2229
Thr Ser Asp Lys Met His Phe Pro Arg Ser Ser Leu Gln Pro Ile Thr
    570                 575                 580

acg ctg ggg aag agt gag ttt ggg gag gtg ttc ctg gca aag gct cag 2277
Thr Leu Gly Lys Ser Glu Phe Gly Glu Val Phe Leu Ala Lys Ala Gln
585                 590                 595                 600

ggc ttg gag gag gga gtg gca gag acc ctg gta ctt gtg aag agc ctg 2325
Gly Leu Glu Glu Gly Val Ala Glu Thr Leu Val Leu Val Lys Ser Leu
                605                 610                 615

cag agc aag gat gag cag cag cag ctg gac ttc cgg agg gag ttg gag 2373
Gln Ser Lys Asp Glu Gln Gln Gln Leu Asp Phe Arg Arg Glu Leu Glu
            620                 625                 630

atg ttt ggg aag ctg aac cac gcc aac gtg gtg cgg ctc ctg ggg ctg 2421
Met Phe Gly Lys Leu Asn His Ala Asn Val Val Arg Leu Leu Gly Leu
        635                 640                 645

tgc cgg gag gct gag ccc cac tac atg gtg ctg gaa tat gtg gat ctg 2469
Cys Arg Glu Ala Glu Pro His Tyr Met Val Leu Glu Tyr Val Asp Leu
    650                 655                 660

gga gac ctc aag cag ttc ctg agg att tcc aag agc aag gat gaa aaa 2517
Gly Asp Leu Lys Gln Phe Leu Arg Ile Ser Lys Ser Lys Asp Glu Lys

665                 670                 675                 680

ttg aag tca cgg ccc ctc agc acc aag cag aag gtg gcc cta tgc acc    2565
Leu Lys Ser Arg Pro Leu Ser Thr Lys Gln Lys Val Ala Leu Cys Thr
                685                 690                 695

cag gta gcc ctg ggc atg gag cac ctg tcc aac aac cgc ttt gtg cat    2613
Gln Val Ala Leu Gly Met Glu His Leu Ser Asn Asn Arg Phe Val His
            700                 705                 710

aag gac ttg gct gcg cgt aac tgc ctg gtc agt gcc cag aga caa gtg    2661
Lys Asp Leu Ala Ala Arg Asn Cys Leu Val Ser Ala Gln Arg Gln Val
        715                 720                 725

aag gtg tct gcc ctg ggc ctc agc aag gat gtg tac aac agt gag tac    2709
Lys Val Ser Ala Leu Gly Leu Ser Lys Asp Val Tyr Asn Ser Glu Tyr
    730                 735                 740

tac cac ttc cgc cag gcc tgg gtg ccg ctg cgc tgg atg tcc ccc gag    2757
Tyr His Phe Arg Gln Ala Trp Val Pro Leu Arg Trp Met Ser Pro Glu
745                 750                 755                 760

gcc atc ctg gag ggt gac ttc tct acc aag tct gat gtc tgg gcc ttc    2805
Ala Ile Leu Glu Gly Asp Phe Ser Thr Lys Ser Asp Val Trp Ala Phe
                765                 770                 775

ggt gtg ctg atg tgg gaa gtg ttt aca cat gga gag atg ccc cat ggt    2853
Gly Val Leu Met Trp Glu Val Phe Thr His Gly Glu Met Pro His Gly
            780                 785                 790

ggg cag gca gat gat gaa gta ctg gca gat ttg cag gct ggg aag gct    2901
Gly Gln Ala Asp Asp Glu Val Leu Ala Asp Leu Gln Ala Gly Lys Ala
        795                 800                 805

aga ctt cct cag ccc gag ggc tgc cct tcc aaa ctc tat cgg ctg atg    2949
Arg Leu Pro Gln Pro Glu Gly Cys Pro Ser Lys Leu Tyr Arg Leu Met
    810                 815                 820

cag cgc tgc tgg gcc ctc agc ccc aag gac cgg ccc tcc ttc agt gag    2997
Gln Arg Cys Trp Ala Leu Ser Pro Lys Asp Arg Pro Ser Phe Ser Glu
825                 830                 835                 840

att gcc agc gcc ctg gga gac agc acc gtg gac agc aag ccg            3039
Ile Ala Ser Ala Leu Gly Asp Ser Thr Val Asp Ser Lys Pro
                845                 850

tgaggaggga gcccgctcag gatggcctgg gcaggggagg acatctctag             3089

agggaagctc acagcatgat gggcaagatc cctgtcctcc tgggccctga             3139

ggcccctgcc ctagtgcaac aggcattgct gaggtctgag cagggcctgg             3189

cctttcctcc tcttcctcac cctcatcctt tgggaggctg acttggaccc 3239

aaactgggcg actagggctt tgagctgggc agttttccct gccacctctt 3289

cctctatcag ggacagtgtg ggtgccacag gtaaccccaa tttctggcct 3339

tcaacttctc cccttgaccg ggtccaactc tgccactcat ctgccaactt 3389

tgcctgggga gggctaggct tgggatgagc tgggtttgtg gggagttcct 3439

taatattctc aagttctggg cacacagggt taatgagtct cttggcccac 3489

tggtcccact tgggggtcta gaccaggatt atagaggaca cagcaagtga 3539

gtcctcccca ctctgggctt gtgcacactg acccagaccc acgtcttccc 3589

cacccttctc tcctttcctc atcctaagtg cctggcagat gaaggagttt 3639

tcaggagctt ttgacactat ataaaccgcc cttttcgtat gcaccacggg 3689

cggc 3693

<210>116
<211>854
<212>PRT
<213>Homo sapiens

<400>116
   Met Gly Thr Leu Asp Glu Ser Phe Ala Arg Val Val Leu Ala Pro Gln
   1               5                   10                  15
   Asp Val Val Val Ala Arg Tyr Glu Glu Ala Met Phe His Cys Gln Phe
               20                  25                  30
   Ser Ala Gln Pro Pro Pro Ser Leu Gln Trp Leu Phe Glu Asp Glu Thr
           35                  40                  45
   Pro Ile Thr Asn Arg Ser Arg Pro Pro His Leu Arg Arg Ala Thr Val
       50                  55                  60
   Phe Ala Asn Gly Ser Leu Leu Leu Thr Gln Val Arg Pro Arg Asn Ala
   65                  70                  75                  80
   Gly Ile Tyr Arg Cys Ile Gly Gln Gly Gln Arg Gly Pro Pro Ile Ile
                   85                  90                  95
   Leu Glu Ala Thr Leu His Leu Ala Glu Ile Glu Asp Met Pro Leu Phe
               100                 105                 110
   Glu Pro Arg Val Phe Thr Ala Gly Ser Glu Glu Arg Val Thr Cys Leu
           115                 120                 125
   Pro Pro Lys Gly Leu Pro Glu Pro Ser Val Trp Trp Glu His Ala Gly
       130                 135                 140
   Val Arg Leu Pro Thr His Gly Arg Val Tyr Gln Lys Gly His Glu Leu
   145                 150                 155                 160
   Val Leu Ala Asn Ile Ala Glu Ser Asp Ala Gly Val Tyr Thr Cys His
                   165                 170                 175
   Ala Ala Asn Leu Ala Gly Gln Arg Arg Gln Asp Val Asn Ile Thr Val
               180                 185                 190
   Ala Thr Val Pro Ser Trp Leu Lys Lys Pro Gln Asp Ser Gln Leu Glu
           195                 200                 205
   Glu Gly Lys Pro Gly Tyr Leu Asp Cys Leu Thr Gln Ala Thr Pro Lys
       210                 215                 220
   Pro Thr Val Val Trp Tyr Arg Asn Gln Met Leu Ile Ser Glu Asp Ser
   225                 230                 235                 240
   Arg Phe Glu Val Phe Lys Asn Gly Thr Leu Arg Ile Asn Ser Val Glu
                   245                 250                 255
   Val Tyr Asp Gly Thr Trp Tyr Arg Cys Met Ser Ser Thr Pro Ala Gly
               260                 265                 270
   Ser Ile Glu Ala Gln Ala Arg Val Gln Val Leu Glu Lys Leu Lys Phe
           275                 280                 285
   Thr Pro Pro Pro Gln Pro Gln Gln Cys Met Glu Phe Asp Lys Glu Ala
       290                 295                 300
   Thr Val Pro Cys Ser Ala Thr Gly Arg Glu Lys Pro Thr Ile Lys Trp
   305                 310                 315                 320
   Glu Arg Ala Asp Gly Ser Ser Leu Pro Glu Trp Val Thr Asp Asn Ala
                   325                 330                 335
   Gly Thr Leu His Phe Ala Arg Val Thr Arg Asp Asp Ala Gly Asn Tyr
               340                 345                 350
   Thr Cys Ile Ala Ser Asn Gly Pro Gln Gly Gln Ile Arg Ala His Val
           355                 360                 365
   Gln Leu Thr Val Ala Val Phe Ile Thr Phe Lys Val Glu Pro Glu Arg
       370                 375                 380
   Thr Thr Val Tyr Gln Gly His Thr Ala Leu Leu Gln Cys Glu Ala Gln
   385                 390                 395                 400
   Gly Asp Pro Lys Pro Leu Ile Gln Trp Lys Gly Lys Asp Arg Ile Leu
                   405                 410                 415
   Asp Pro Thr Lys Leu Gly Pro Arg Met His Ile Phe Gln Asn Gly Ser
               420                 425                 430
   Leu Val Ile His Asp Val Ala Pro Glu Asp Ser Gly Arg Tyr Thr Cys
           435                 440                 445
   Ile Ala Gly Asn Ser Cys Asn Ile Lys His Thr Glu Ala Pro Leu Tyr
       450                 455                 460
   Val Val Asp Lys Pro Val Pro Glu Glu Ser Glu Gly Pro Gly Ser Pro
   465                 470                 475                 480
   Pro Pro Tyr Lys Met Ile Gln Thr Ile Gly Leu Ser Val Gly Ala Ala
                   485                 490                 495
   Val Ala Tyr Ile Ile Ala Val Leu Gly Leu Met Phe Tyr Cys Lys Lys
               500                 505                 510
   Arg Cys Lys Ala Lys Arg Leu Gln Lys Gln Pro Glu Gly Glu Glu Pro
           515                 520                 525
   Glu Met Glu Cys Leu Asn Gly Gly Pro Leu Gln Asn Gly Gln Pro Ser
       530                 535                 540
   Ala Glu Ile Gln Glu Glu Val Ala Leu Thr Ser Leu Gly Ser Gly Pro
   545                 550                 555                 560
   Ala Ala Pro Asn Lys Arg His Ser Thr Ser Asp Lys Met His Phe Pro
                   565                 570                 575
   Arg Ser Ser Leu Gln Pro Ile Thr Thr Leu Gly Lys Ser Glu Phe Gly
               580                 585                 590
   Glu Val Phe Leu Ala Lys Ala Gln Gly Leu Glu Glu Gly Val Ala Glu
           595                 600                 605
   Thr Leu Val Leu Val Lys Ser Leu Gln Ser Lys Asp Glu Gln Gln Gln
       610                 615                 620
   Leu Asp Phe Arg Arg Glu Leu Glu Met Phe Gly Lys Leu Asn His Ala
   625                 630                 635                 640
   Asn Val Val Arg Leu Leu Gly Leu Cys Arg Glu Ala Glu Pro His Tyr
                   645                 650                 655
   Met Val Leu Glu Tyr Val Asp Leu Gly Asp Leu Lys Gln Phe Leu Arg
               660                 665                 670
   Ile Ser Lys Ser Lys Asp Glu Lys Leu Lys Ser Arg Pro Leu Ser Thr
           675                 680                 685
   Lys Gln Lys Val Ala Leu Cys Thr Gln Val Ala Leu Gly Met Glu His
       690                 695                 700
   Leu Ser Asn Asn Arg Phe Val His Lys Asp Leu Ala Ala Arg Asn Cys
   705                 710                 715                 720
   Leu Val Ser Ala Gln Arg Gln Val Lys Val Ser Ala Leu Gly Leu Ser
                   725                 730                 735
   Lys Asp Val Tyr Asn Ser Glu Tyr Tyr His Phe Arg Gln Ala Trp Val
               740                 745                 750
   Pro Leu Arg Trp Met Ser Pro Glu Ala Ile Leu Glu Gly Asp Phe Ser
           755                 760                 765
   Thr Lys Ser Asp Val Trp Ala Phe Gly Val Leu Met Trp Glu Val Phe
       770                 775                 780
   Thr His Gly Glu Met Pro His Gly Gly Gln Ala Asp Asp Glu Val Leu
   785                 790                 795                 800
   Ala Asp Leu Gln Ala Gly Lys Ala Arg Leu Pro Gln Pro Glu Gly Cys
                   805                 810                 815
   Pro Ser Lys Leu Tyr Arg Leu Met Gln Arg Cys Trp Ala Leu Ser Pro
               820                 825                 830
   Lys Asp Arg Pro Ser Phe Ser Glu Ile Ala Ser Ala Leu Gly Asp Ser
           835                 840                 845
   Thr Val Asp Ser Lys Pro
       850

<210>117
<211>2866
<212>DNA
<213>Homo sapiens

<220>
<221> CDS
<222>(29)..(2189)

<400>117

ctcagctcct tttcctgagc ccgccgcg atg gga gct gcg cgg gga tcc ccg     52
                               Met Gly Ala Ala Arg Gly Ser Pro
                               1               5

gcc aga ccc cgc cgg ttg cct ctg ctc agc gtc ctg ctg ctg ccg ctg 100
Ala Arg Pro Arg Arg Leu Pro Leu Leu Ser Val Leu Leu Leu Pro Leu
    10                  15                  20

ctg ggc ggt acc cag aca gcc att gtc ttc atc aag cag ccg tcc tcc 148
Leu Gly Gly Thr Gln Thr Ala Ile Val Phe Ile Lys Gln Pro Ser Ser
25                  30                  35                  40

cag gat gca ctg cag ggg cgc cgg gcg ctg ctt cgc tgt gag gtt gag 196
Gln Asp Ala Leu Gln Gly Arg Arg Ala Leu Leu Arg Cys Glu Val Glu
                45                  50                  55

gct ccg ggc ccg gta cat gtg tac tgg ctg ctc gat ggg gcc cct gtc 244
Ala Pro Gly Pro Val His Val Tyr Trp Leu Leu Asp Gly Ala Pro Val
            60                  65                  70

cag gac acg gag cgg cgt ttc gcc cag ggc agc agc ctg agc ttt gca 292
Gln Asp Thr Glu Arg Arg Phe Ala Gln Gly Ser Ser Leu Ser Phe Ala
        75                  80                  85

gct gtg gac cgg ctg cag gac tct ggc acc ttc cag tgt gtg gct cgg 340
Ala Val Asp Arg Leu Gln Asp Ser Gly Thr Phe Gln Cys Val Ala Arg
    90                  95                  100

gat gat gtc act gga gaa gaa gcc cgc agt gcc aac gcc tcc ttc aac 388
Asp Asp Val Thr Gly Glu Glu Ala Arg Ser Ala Asn Ala Ser Phe Asn
105                 110                 115                 120

atc aaa tgg att gag gca ggt cct gtg gtc ctg aag cat cca gcc tcg 436
Ile Lys Trp Ile Glu Ala Gly Pro Val Val Leu Lys His Pro Ala Ser
                125                 130                 135

gaa gct gag atc cag cca cag acc cag gtc aca ctt cgt tgc cac att 484
Glu Ala Glu Ile Gln Pro Gln Thr Gln Val Thr Leu Arg Cys His Ile
            140                 145                 150

gat ggg cac cct cgg ccc acc tac caa tgg ttc cga gat ggg acc ccc 532
Asp Gly His Pro Arg Pro Thr Tyr Gln Trp Phe Arg Asp Gly Thr Pro
        155                 160                 165

ctt tct gat ggt cag agc aac cac aca gtc agc agc aag gag cgg aac 580
Leu Ser Asp Gly Gln Ser Asn His Thr Val Ser Ser Lys Glu Arg Asn
    170                 175                 180

ctg acg ctc cgg cca gct ggt cct gag cat agt ggg ctg tat tcc tgc 628
Leu Thr Leu Arg Pro Ala Gly Pro Glu His Ser Gly Leu Tyr Ser Cys
185                 190                 195                 200

tgc gcc cac agt gct ttt ggc cag gct tgc agc agc cag aac ttc acc 676
Cys Ala His Ser Ala Phe Gly Gln Ala Cys Ser Ser Gln Asn Phe Thr
                205                 210                 215

ttg agc att gct gat gaa agc ttt gcc agg gtg gtg ctg gca ccc cag 724
Leu Ser Ile Ala Asp Glu Ser Phe Ala Arg Val Val Leu Ala Pro Gln
            220                 225                 230

gac gtg gta gta gcg agg tat gag gag gcc atg ttc cat tgc cag ttc 772
Asp Val Val Val Ala Arg Tyr Glu Glu Ala Met Phe His Cys Gln Phe
        235                 240                 245

tca gcc cag cca ccc ccg agc ctg cag tgg ctc ttt gag gat gag act 820
Ser Ala Gln Pro Pro Pro Ser Leu Gln Trp Leu Phe Glu Asp Glu Thr
    250                 255                 260

ccc atc act aac cgc agt cgc ccc cca cac ctc cgc aga gcc aca gtg 868
Pro Ile Thr Asn Arg Ser Arg Pro Pro His Leu Arg Arg Ala Thr Val
265                 270                 275                 280

ttt gcc aac ggg tct ctg ctg ctg acc cag gtc cgg cca cgc aat gca 916
Phe Ala Asn Gly Ser Leu Leu Leu Thr Gln Val Arg Pro Arg Asn Ala
                285                 290                 295

ggg atc tac cgc tgc att ggc cag ggg cag agg ggc cca ccc atc atc 964
Gly Ile Tyr Arg Cys Ile Gly Gln Gly Gln Arg Gly Pro Pro Ile Ile
            300                 305                 310

ctg gaa gcc aca ctt cac cta gca gag att gaa gac atg ccg cta ttt 1012
Leu Glu Ala Thr Leu His Leu Ala Glu Ile Glu Asp Met Pro Leu Phe
        315                 320                 325

gag cca cgg gtg ttt aca gct ggc agc gag gag cgt gtg acc tgc ctt 1060
Glu Pro Arg Val Phe Thr Ala Gly Ser Glu Glu Arg Val Thr Cys Leu
    330                 335                 340

ccc ccc aag ggt ctg cca gag ccc agc gtg tgg tgg gag cac gcg gga 1108
Pro Pro Lys Gly Leu Pro Glu Pro Ser Val Trp Trp Glu His Ala Gly
345                 350                 355                 360

gtc cgg ctg ccc acc cat ggc agg gtc tac cag aag ggc cac gag ctg 1156
Val Arg Leu Pro Thr His Gly Arg Val Tyr Gln Lys Gly His Glu Leu
                365                 370                 375

gtg ttg gcc aat att gct gaa agt gat gct ggt gtc tac acc tgc cac 1204
Val Leu Ala Asn Ile Ala Glu Ser Asp Ala Gly Val Tyr Thr Cys His
            380                 385                 390

gcg gcc aac ctg gct ggt cag cgg aga cag gat gtc aac atc act gtg 1252
Ala Ala Asn Leu Ala Gly Gln Arg Arg Gln Asp Val Asn Ile Thr Val
        395                 400                 405

gcc act gtg ccc tcc tgg ctg aag aag ccc caa gac agc cag ctg gag 1300
Ala Thr Val Pro Ser Trp Leu Lys Lys Pro Gln Asp Ser Gln Leu Glu
    410                 415                 420

gag ggc aaa ccc ggc tac ttg gat tgc ctg acc cag gcc aca cca aaa 1348
Glu Gly Lys Pro Gly Tyr Leu Asp Cys Leu Thr Gln Ala Thr Pro Lys
425                 430                 435                 440

cct aca gtt gtc tgg tac aga aac cag atg ctc atc tca gag gac tca 1396
Pro Thr Val Val Trp Tyr Arg Asn Gln Met Leu Ile Ser Glu Asp Ser
                445                 450                 455

cgg ttc gag gtg ttc ctg gca aag gct cag ggc ttg gag gag gga gtg 1444

Arg Phe Glu Val Phe Leu Ala Lys Ala Gln Gly Leu Glu Glu Gly Val
            460                 465                 470

gca gag acc ctg gta ctt gtg aag agc ctg cag agc aag gat gag cag 1492
Ala Glu Thr Leu Val Leu Val Lys Ser Leu Gln Ser Lys Asp Glu Gln
        475                 480                 485

cag cag ctg gac ttc cgg agg gag ttg gag atg ttt ggg aag ctg aac 1540
Gln Gln Leu Asp Phe Arg Arg Glu Leu Glu Met Phe Gly Lys Leu Asn
    490                 495                 500

cac gcc aac gtg gtg cgg ctc ctg ggg ctg tgc cgg gag gct gag ccc 1588
His Ala Asn Val Val Arg Leu Leu Gly Leu Cys Arg Glu Ala Glu Pro
505                 510                 515                 520

cac tac atg gtg ctg gaa tat gtg gat ctg gga gac ctc aag cag ttc 1636
His Tyr Met Val Leu Glu Tyr Val Asp Leu Gly Asp Leu Lys Gln Phe
                525                 530                 535

ctg agg att tcc aag agc aag gat gaa aaa ttg aag tca cag ccc ctc 1684
Leu Arg Ile Ser Lys Ser Lys Asp Glu Lys Leu Lys Ser Gln Pro Leu
            540                 545                 550

agc acc aag cag aag gtg gcc cta tgc acc cag gta gcc ctg ggc atg 1732
Ser Thr Lys Gln Lys Val Ala Leu Cys Thr Gln Val Ala Leu Gly Met
        555                 560                 565

gag cac ctg tcc aac aac cgc ttt gtg cat aag gac ttg gct gcg cgt 1780
Glu His Leu Ser Asn Asn Arg Phe Val His Lys Asp Leu Ala Ala Arg
    570                 575                 580

aac tgc ctg gtc agt gcc cag aga caa gtg aag gtg tct gcc ctg ggc 1828
Asn Cys Leu Val Ser Ala Gln Arg Gln Val Lys Val Ser Ala Leu Gly
585                 590                 595                 600

ctc agc aag gat gtg tac aac agt gag tac tac cac ttc cgc cag gcc 1876
Leu Ser Lys Asp Val Tyr Asn Ser Glu Tyr Tyr His Phe Arg Gln Ala
                605                 610                 615

tgg gtg ccg ctg cgc tgg atg tcc ccc gag gcc atc ctg gag ggt gac 1924
Trp Val Pro Leu Arg Trp Met Ser Pro Glu Ala Ile Leu Glu Gly Asp
            620                 625                 630

ttc tct acc aag tct gat gtc tgg gcc ttc ggt gtg ctg atg tgg gaa 1972
Phe Ser Thr Lys Ser Asp Val Trp Ala Phe Gly Val Leu Met Trp Glu
        635                 640                 645

gtg ttt aca cat gga gag atg ccc cat ggt ggg cag gca gat gat gaa 2020
Val Phe Thr His Gly Glu Met Pro His Gly Gly Gln Ala Asp Asp Glu
    650                 655                 660

gta ctg gca gat ttg cag gct ggg aag gct aga ctt cct cag ccc gag 2068
Val Leu Ala Asp Leu Gln Ala Gly Lys Ala Arg Leu Pro Gln Pro Glu
665                 670                 675                 680

ggc tgc cct tcc aaa ctc tat cgg ctg atg cag cgc tgc tgg gcc ctc 2116
Gly Cys Pro Ser Lys Leu Tyr Arg Leu Met Gln Arg Cys Trp Ala Leu

                685                 690                 695

agc ccc aag gac cgg ccc tcc ttc agt gag att gcc agc gcc ctg gga 2164
Ser Pro Lys Asp Arg Pro Ser Phe Ser Glu Ile Ala Ser Ala Leu Gly
            700                 705                 710

gac agc acc gtg gac agc aag ccg tgaggaggga gcccgctcag 2208
Asp Ser Thr Val Asp Ser Lys Pro
        715                 720

gatggcctgg gcaggggagg acatctctag agggaagctc acagcatgat 2258

gggcaagatc cctgtcctcc tgggccctga ggcccctgcc ctagtgcaac 2308

aggcattgct gaggtctgag cagggcctgg cctttcctcc tcttcctcac 2358

cctcatcctt tgggaggctg acttggaccc aaactgggcg actagggctt 2408

tgagctgggc agttttccct gccacctctt cctctatcag ggacagtgtg 2458

ggtgccacag gtaaccccaa tttctggcct tcaacttctc cccttgaccg 2508

ggtccaactc tgccactcat ctgccaactt tgcctgggga gggctaggct 2558

tgggatgagc tgggtttgtg gggagttcct taatattctc aagttctggg 2608

cacacagggt taatgagtct cttggcccac tggtcccact tgggggtcta 2658

gaccaggatt atagaggaca cagcaagtga gtcctcccca ctctgggctt 2708

gtgcacactg acccagaccc acgtcttccc caccettctc tcctttcctc 2758

atcctaagtg cctggcagat gaaggagttt tcaggagctt ttgacactat 2808

ataaaccgcc ctttttgtat gcaccacggg cggcttttat atgtaattgc 2858

agcgtggg 2866

<210>118
<211>720
<212>PRT
<213>Homo sapiens

<400>118

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Leu Ala Lys
    450                 455                 460

Ala Gln Gly Leu Glu Glu Gly Val Ala Glu Thr Leu Val Leu Val Lys
465                 470                 475                 480

Ser Leu Gln Ser Lys Asp Glu Gln Gln Gln Leu Asp Phe Arg Arg Glu
                485                 490                 495

Leu Glu Met Phe Gly Lys Leu Asn His Ala Asn Val Val Arg Leu Leu
            500                 505                 510

Gly Leu Cys Arg Glu Ala Glu Pro His Tyr Met Val Leu Glu Tyr Val
        515                 520                 525

Asp Leu Gly Asp Leu Lys Gln Phe Leu Arg Ile Ser Lys Ser Lys Asp
    530                 535                 540

Glu Lys Leu Lys Ser Gln Pro Leu Ser Thr Lys Gln Lys Val Ala Leu
545                 550                 555                 560

Cys Thr Gln Val Ala Leu Gly Met Glu His Leu Ser Asn Asn Arg Phe
                565                 570                 575

Val His Lys Asp Leu Ala Ala Arg Asn Cys Leu Val Ser Ala Gln Arg
            580                 585                 590

Gln Val Lys Val Ser Ala Leu Gly Leu Ser Lys Asp Val Tyr Asn Ser
        595                 600                 605

Glu Tyr Tyr His Phe Arg Gln Ala Trp Val Pro Leu Arg Trp Met Ser
    610                 615                 620

Pro Glu Ala Ile Leu Glu Gly Asp Phe Ser Thr Lys Ser Asp Val Trp
625                 630                 635                 640

Ala Phe Gly Val Leu Met Trp Glu Val Phe Thr His Gly Glu Met Pro
                645                 650                 655

His Gly Gly Gln Ala Asp Asp Glu Val Leu Ala Asp Leu Gln Ala Gly
            660                 665                 670

Lys Ala Arg Leu Pro Gln Pro Glu Gly Cys Pro Ser Lys Leu Tyr Arg
        675                 680                 685

Leu Met Gln Arg Cys Trp Ala Leu Ser Pro Lys Asp Arg Pro Ser Phe
    690                 695                 700

Ser Glu Ile Ala Ser Ala Leu Gly Asp Ser Thr Val Asp Ser Lys Pro
705                 710                 715                 720

<210>119
<211>2894
<212>DNA
<213>Homo sapiens

<220>
<221> CDS
<222>(2)..(2891)

<400>119

g atg gtg ggc tgt ggg gtg gca gtt tta tgt ttg tgg gtt tcc tgc 46
Met Val Gly Cys Gly Val Ala Val Leu Cys Leu Trp Val Ser Cys
1               5                   10                  15

ggc gct gca gcg gga cag ctc gag tac tca gtg ccg gag gag acg gag 94
Gly Ala Ala Ala Gly Gln Leu Glu Tyr Ser Val Pro Glu Glu Thr Glu
                20                  25                  30

cgg ggc gta gcc gta ggc aat ctc tcc gcg gac ttg agg ctg cca gcg 142

Arg Gly Val Ala Val Gly Asn Leu Ser Ala Asp Leu Arg Leu Pro Ala
            35                  40                  45

gcc gct atg tcc tcg cgg aac ttt cgc ttc ctt tcc agc cac cgc gag 190
Ala Ala Met Ser Ser Arg Asn Phe Arg Phe Leu Ser Ser His Arg Glu
        50                  55                  60

ctc tac ttc ggg gtg gat cta ccc agc ggc aat ttg gtg gtc aga gag 238
Leu Tyr Phe Gly Val Asp Leu Pro Ser Gly Asn Leu Val Val Arg Glu
    65                  70                  75

ccg gcg gac cgc gaa cag ctg tgc agg gcc aaa gct gcc tgc gtc ttg 286
Pro Ala Asp Arg Glu Gln Leu Cys Arg Ala Lys Ala Ala Cys Val Leu
80                  85                  90                  95

acc tac gac ctg gtg ctc gag gac ccg ctg gag ctg cac aag att cgg 334
Thr Tyr Asp Leu Val Leu Glu Asp Pro Leu Glu Leu His Lys Ile Arg
                100                 105                 110

att cac gtc ctg gac acc aat gac aac tca cct ctc ttt cct gcc ggc 382
Ile His Val Leu Asp Thr Asn Asp Asn Ser Pro Leu Phe Pro Ala Gly
            115                 120                 125

gac gtg cag ctg cac atc ccc gag ttc ctg acg ccc gga gcc cgc ttt 430
Asp Val Gln Leu His Ile Pro Glu Phe Leu Thr Pro Gly Ala Arg Phe
        130                 135                 140

act ctc ccg aat gcc caa gat gac gac gag gga agc aat ggg ata cta 478
Thr Leu Pro Asn Ala Gln Asp Asp Asp Glu Gly Ser Asn Gly Ile Leu
    145                 150                 155

agc tac agc cta agc ccc agt cag cac ttt cgc ctg gac atg gga tcg 526
Ser Tyr Ser Leu Ser Pro Ser Gln His Phe Arg Leu Asp Met Gly Ser
160                 165                 170                 175

cgg gtt gac ggc agc gaa tac ccg gag ttg gtg ttg gag aaa gca ctg 574
Arg Val Asp Gly Ser Glu Tyr Pro Glu Leu Val Leu Glu Lys Ala Leu
                180                 185                 190

gat cgc gaa cag cgc gcc acc cac ctg ctg gtg ctt aca gct cgg gac 622
Asp Arg Glu Gln Arg Ala Thr His Leu Leu Val Leu Thr Ala Arg Asp
            195                 200                 205

ggc ggg cta cct gcc cgc tca gga gac gca caa gtc acc atc att gtg 670
Gly Gly Leu Pro Ala Arg Ser Gly Asp Ala Gln Val Thr Ile Ile Val
        210                 215                 220

gtg gac aca aat gac aac gcg cct gta ttt gag cgc tcc gta tac cgc 718
Val Asp Thr Asn Asp Asn Ala Pro Val Phe Glu Arg Ser Val Tyr Arg
    225                 230                 235

acc aag gtt cca gag act gca ccc aat ggg act gtg tta ttc cga gtt 766
Thr Lys Val Pro Glu Thr Ala Pro Asn Gly Thr Val Leu Phe Arg Val
240                 245                 250                 255

caa gcc ttg gat cca gat gaa ggg tcc aat ggg gaa gtc caa tac tcc 814
Gln Ala Leu Asp Pro Asp Glu Gly Ser Asn Gly Glu Val Gln Tyr Ser

                260                 265                 270

cta agc aac agc acg caa gca gag ctg cga cac cgc ttt cac gtt cac 862
Leu Ser Asn Ser Thr Gln Ala Glu Leu Arg His Arg Phe His Val His
            275                 280                 285

cct aaa agt ggg gag gtg caa gta gct gct tca cta ggt ccg cct gaa 910
Pro Lys Ser Gly Glu Val Gln Val Ala Ala Ser Leu Gly Pro Pro Glu
        290                 295                 300

acg ctc ttg gag gca tac att gag gcg agg gac gaa ggt gtc ttt ggt 958
Thr Leu Leu Glu Ala Tyr Ile Glu Ala Arg Asp Glu Gly Val Phe Gly
    305                 310                 315

tta gct agc acc gct aaa ctg ctg gtg gag gtg act gac gtg aac gat 1006
Leu Ala Ser Thr Ala Lys Leu Leu Val Glu Val Thr Asp Val Asn Asp
320                 325                 330                 335

cat gcc ccc gaa ctg gac ttc ctg act ctt tcg aac cca gta cct gag 1054
His Ala Pro Glu Leu Asp Phe Leu Thr Leu Ser Asn Pro Val Pro Glu
                340                 345                 350

gac gct gcc cct ggc aca gtg att gct ctc ttt agt gta aag gat gaa 1102
Asp Ala Ala Pro Gly Thr Val Ile Ala Leu Phe Ser Val Lys Asp Glu
            355                 360                 365

gac ctc gat tct aat ggt agg gtc att tgt ggc atg tct agt gca ggc 1150
Asp Leu Asp Ser Asn Gly Arg Val Ile Cys Gly Met Ser Ser Ala Gly
        370                 375                 380

cct ttt cag ctg acg gct tcc ttt gac aac tac tac agc ctg ctg att 1198
Pro Phe Gln Leu Thr Ala Ser Phe Asp Asn Tyr Tyr Ser Leu Leu Ile
    385                 390                 395

gat ggg ccc ctg gac cgg gag cag atc agt gaa tac caa gtc ctg atc 1246
Asp Gly Pro Leu Asp Arg Glu Gln Ile Ser Glu Tyr Gln Val Leu Ile
400                 405                 410                 415

acg gcc tca gat agt ggc tca ccc cca ctt agc acc cga agg aca atc 1294
Thr Ala Ser Asp Ser Gly Ser Pro Pro Leu Ser Thr Arg Arg Thr Ile
                420                 425                 430

act gtg tca gtt gct gat gtg aat gac aat aca cca aac ttt cct caa 1342
Thr Val Ser Val Ala Asp Val Asn Asp Asn Thr Pro Asn Phe Pro Gln
            435                 440                 445

ccc cag cag gaa ctt ttc gtt gct gaa aac aat ggc cct ggg gcc tct 1390
Pro Gln Gln Glu Leu Phe Val Ala Glu Asn Asn Gly Pro Gly Ala Ser
        450                 455                 460

cta ggc cga gtg ttt gcc cag gac ccc gac ctg ggg aag aat ggc ctt 1438
Leu Gly Arg Val Phe Ala Gln Asp Pro Asp Leu Gly Lys Asn Gly Leu
    465                 470                 475

gtc tct tat gag ctg ttg gat gtt atc tct gaa cgg cca tca gcc tct 1486
Val Ser Tyr Glu Leu Leu Asp Val Ile Ser Glu Arg Pro Ser Ala Ser
480                 485                 490                 495

agc ttg gtg gca gtg gaa tca gcc agt ggg gcc atc act gcc aaa act 1534
Ser Leu Val Ala Val Glu Ser Ala Ser Gly Ala Ile Thr Ala Lys Thr
                500                 505                 510

tcc ttt gac ttt gag cag ctc agg ggg ttt cat ttc caa gta gag ggc 1582
Ser Phe Asp Phe Glu Gln Leu Arg Gly Phe His Phe Gln Val Glu Gly
            515                 520                 525

cgg gat ggt ggc att cct ccc aga agt gca aca gtg act ata aac ttg 1630
Arg Asp Gly Gly Ile Pro Pro Arg Ser Ala Thr Val Thr Ile Asn Leu
        530                 535                 540

ttt gtg gta gat agg aat gac aat tat ccg gtt atc ttg gtt ccc ttg 1678
Phe Val Val Asp Arg Asn Asp Asn Tyr Pro Val Ile Leu Val Pro Leu
    545                 550                 555

ccc aga aat ggt tct gtc cca gtg gaa att gtg ccc cgc tct gcc agg 1726
Pro Arg Asn Gly Ser Val Pro Val Glu Ile Val Pro Arg Ser Ala Arg
560                 565                 570                 575

act gga cac ttg gtc aca aaa gtg gta gca gag gat gct gac agt ggt 1774
Thr Gly His Leu Val Thr Lys Val Val Ala Glu Asp Ala Asp Ser Gly
                580                 585                 590

tct aat gcc tgg ctt tcc tac cac atc tcc cgg gcg tct gac tct agt 1822
Ser Asn Ala Trp Leu Ser Tyr His Ile Ser Arg Ala Ser Asp Ser Ser
            595                 600                 605

ctc ttt aga att tca gcc aat ata ggt gag ctc cgt act gct cgc tta 1870
Leu Phe Arg Ile Ser Ala Asn Ile Gly Glu Leu Arg Thr Ala Arg Leu
        610                 615                 620

gtt ctt ccc act gat gca gtt aag cag agg gtg gtg gta gtg gtt cgg 1918
Val Leu Pro Thr Asp Ala Val Lys Gln Arg Val Val Val Val Val Arg
    625                 630                 635

gac cat gga gac cca cca ctt tcc tcc tct gtc act ctg ggt gtg ctg 1966
Asp His Gly Asp Pro Pro Leu Ser Ser Ser Val Thr Leu Gly Val Leu
640                 645                 650                 655

ttg agc aac tct gtc cct cag tta ctt cca gac ttt gaa gat gtc tgg 2014
Leu Ser Asn Ser Val Pro Gln Leu Leu Pro Asp Phe Glu Asp Val Trp
                660                 665                 670

gaa cca gga ggg cag ctt tct gcc cag aac ttg tat tta gta att gcc 2062
Glu Pro Gly Gly Gln Leu Ser Ala Gln Asn Leu Tyr Leu Val Ile Ala
            675                 680                 685

ttg gct tgt att tcc ttt tta ttt ctg ggg tgc tta ctt ttc ttc gtg 2110
Leu Ala Cys Ile Ser Phe Leu Phe Leu Gly Cys Leu Leu Phe Phe Val
        690                 695                 700

tgt acc aag ttg cac cag agc cca ggc tgt tgc gct cag agc tgc tgt 2158
Cys Thr Lys Leu His Gln Ser Pro Gly Cys Cys Ala Gln Ser Cys Cys
    705                 710                 715

cgc tct aca gag gat ctg agg tat gga agt aag atg gtt tca aat cct 2206
Arg Ser Thr Glu Asp Leu Arg Tyr Gly Ser Lys Met Val Ser Asn Pro
720                 725                 730                 735

tgc atg aca tca gcc acc ata gat gtc act aca gtt gag aga ctt tct 2254
Cys Met Thr Ser Ala Thr Ile Asp Val Thr Thr Val Glu Arg Leu Ser
                740                 745                 750

cag act tat ctc tat cgg gcc tct ctg gga ctt ggt tct gat aat aac 2302
Gln Thr Tyr Leu Tyr Arg Ala Ser Leu Gly Leu Gly Ser Asp Asn Asn
            755                 760                 765

agt ttg ctg ttg cgt ggg gag tac aat gct gcc gac ctg cga aat ctt 2350
Ser Leu Leu Leu Arg Gly Glu Tyr Asn Ala Ala Asp Leu Arg Asn Leu
        770                 775                 780

gcc act ggg gta gga ctg aat ttg cca ata tcc tgt att cag att cgg 2398
Ala Thr Gly Val Gly Leu Asn Leu Pro Ile Ser Cys Ile Gln Ile Arg
    785                 790                 795

aat agg aaa ggg gat cac gct aat gtc aat gcc atg cca cga cag ccc 2446
Asn Arg Lys Gly Asp His Ala Asn Val Asn Ala Met Pro Arg Gln Pro
800                 805                 810                 815

aac cct gac tgg cgt tac tct gcc tcc ctg aga gca ggc atg cac agc 2494
Asn Pro Asp Trp Arg Tyr Ser Ala Ser Leu Arg Ala Gly Met His Ser
                820                 825                 830

tct gtg cac cta gag gag gct ggc att cta cgg gct ggt cca gga ggg 2542
Ser Val His Leu Glu Glu Ala Gly Ile Leu Arg Ala Gly Pro Gly Gly
            835                 840                 845

cct gat cag cag tgg cca aca gta tcc agt gca aca cca gaa cca gag 2590
Pro Asp Gln Gln Trp Pro Thr Val Ser Ser Ala Thr Pro Glu Pro Glu
        850                 855                 860

gca gga gaa gtg tcc cct cca gtc ggt gcg ggt gtc aac agc aac agc 2638
Ala Gly Glu Val Ser Pro Pro Val Gly Ala Gly Val Asn Ser Asn Ser
    865                 870                 875

tgg acc ttt aaa tac gga cca ggc aac ccc aaa caa tcc ggt ccc ggt 2686
Trp Thr Phe Lys Tyr Gly Pro Gly Asn Pro Lys Gln Ser Gly Pro Gly
880                 885                 890                 895

gag ttg ccc gac aaa ttc att atc cca gga tct cct gca atc atc tcc 2734
Glu Leu Pro Asp Lys Phe Ile Ile Pro Gly Ser Pro Ala Ile Ile Ser
                900                 905                 910

atc cgg cag gag cct act aac agc caa att gac aaa agt gac ttc ata 2782
Ile Arg Gln Glu Pro Thr Asn Ser Gln Ile Asp Lys Ser Asp Phe Ile
            915                 920                 925

acc ttc ggc aaa aag gag gag acc aag aaa aag aag aaa aag aag aag 2830
Thr Phe Gly Lys Lys Glu Glu Thr Lys Lys Lys Lys Lys Lys Lys Lys
        930                 935                 940

ggt aac aag acc cag gag aaa aaa gag aaa ggg aac agc acg act gac 2878

Gly Asn Lys Thr Gln Glu Lys Lys Glu Lys Gly Asn Ser Thr Thr Asp
    945                 950                 955

aac agt gac cag tgag 2894
Asn Ser Asp Gln
960

<210>120
<211>963
<212>PRT
<213>Homo sapiens

<400>120
   Met Val Gly Cys Gly Val Ala Val Leu Cys Leu Trp Val Ser Cys Gly
   1 5 10 15
   Ala Ala Ala Gly Gln Leu Glu Tyr Ser Val Pro Glu Glu Thr Glu Arg
   20 25 30
   Gly Val Ala Val Gly Asn Leu Ser Ala Asp Leu Arg Leu Pro Ala Ala
   35 40 45
   Ala Met Ser Ser Arg Asn Phe Arg Phe Leu Ser Ser His Arg Glu Leu
   50 55 60
   Tyr Phe Gly Val Asp Leu Pro Ser Gly Asn Leu Val Val Arg Glu Pro
   65 70 75 80
   Ala Asp Arg Glu Gln Leu Cys Arg Ala Lys Ala Ala Cys Val Leu Thr
   85 90 95
   Tyr Asp Leu Val Leu Glu Asp Pro Leu Glu Leu His Lys Ile Arg Ile
   100 105 110
   His Val Leu Asp Thr Asn Asp Asn Ser Pro Leu Phe Pro Ala Gly Asp
   115 120 125
   Val Gln Leu His Ile Pro Glu Phe Leu Thr Pro Gly Ala Arg Phe Thr
   130 135 140
   Leu Pro Asn Ala Gln Asp Asp Asp Glu Gly Ser Asn Gly Ile Leu Ser
   145 150 155 160
   Tyr Ser Leu Ser Pro Ser Gln His Phe Arg Leu Asp Met Gly Ser Arg
   165 170 175
   Val Asp Gly Ser Glu Tyr Pro Glu Leu Val Leu Glu Lys Ala Leu Asp
   180 185 190
   Arg Glu Gln Arg Ala Thr His Leu Leu Val Leu Thr Ala Arg Asp Gly
   195 200 205
   Gly Leu Pro Ala Arg Ser Gly Asp Ala Gln Val Thr Ile Ile Val Val
   210 215 220
   Asp Thr Asn Asp Asn Ala Pro Val Phe Glu Arg Ser Val Tyr Arg Thr
   225                 230                 235                 240
   Lys Val Pro Glu Thr Ala Pro Asn Gly Thr Val Leu Phe Arg Val Gln
                   245                 250                 255
   Ala Leu Asp Pro Asp Glu Gly Ser Asn Gly Glu Val Gln Tyr Ser Leu
               260                 265                 270
   Ser Asn Ser Thr Gln Ala Glu Leu Arg His Arg Phe His Val His Pro
           275                 280                 285
   Lys Ser Gly Glu Val Gln Val Ala Ala Ser Leu Gly Pro Pro Glu Thr
       290                 295                 300
   Leu Leu Glu Ala Tyr Ile Glu Ala Arg Asp Glu Gly Val Phe Gly Leu
   305                 310                 315                 320
   Ala Ser Thr Ala Lys Leu Leu Val Glu Val Thr Asp Val Asn Asp His
                   325                 330                 335
   Ala Pro Glu Leu Asp Phe Leu Thr Leu Ser Asn Pro Val Pro Glu Asp
               340                 345                 350
   Ala Ala Pro Gly Thr Val Ile Ala Leu Phe Ser Val Lys Asp Glu Asp
           355                 360                 365
   Leu Asp Ser Asn Gly Arg Val Ile Cys Gly Met Ser Ser Ala Gly Pro
       370                 375                 380
   Phe Gln Leu Thr Ala Ser Phe Asp Asn Tyr Tyr Ser Leu Leu Ile Asp
   385                 390                 395                 400
   Gly Pro Leu Asp Arg Glu Gln Ile Ser Glu Tyr Gln Val Leu Ile Thr
                   405                 410                 415
   Ala Ser Asp Ser Gly Ser Pro Pro Leu Ser Thr Arg Arg Thr Ile Thr
               420                 425                 430
   Val Ser Val Ala Asp Val Asn Asp Asn Thr Pro Asn Phe Pro Gln Pro
           435                 440                 445
   Gln Gln Glu Leu Phe Val Ala Glu Asn Asn Gly Pro Gly Ala Ser Leu
       450                 455                 460
   Gly Arg Val Phe Ala Gln Asp Pro Asp Leu Gly Lys Asn Gly Leu Val
   465                 470                 475                 480
   Ser Tyr Glu Leu Leu Asp Val Ile Ser Glu Arg Pro Ser Ala Ser Ser
                   485                 490                 495
   Leu Val Ala Val Glu Ser Ala Ser Gly Ala Ile Thr Ala Lys Thr Ser
               500                 505                 510
   Phe Asp Phe Glu Gln Leu Arg Gly Phe His Phe Gln Val Glu Gly Arg
           515                 520                 525
   Asp Gly Gly Ile Pro Pro Arg Ser Ala Thr Val Thr Ile Asn Leu Phe
   530                 535                 540
   Val Val Asp Arg Asn Asp Asn Tyr Pro Val Ile Leu Val Pro Leu Pro
   545                 550                 555                 560
   Arg Asn Gly Ser Val Pro Val Glu Ile Val Pro Arg Ser Ala Arg Thr
                   565                 570                 575
   Gly His Leu Val Thr Lys Val Val Ala Glu Asp Ala Asp Ser Gly Ser
               580                 585                 590
   Asn Ala Trp Leu Ser Tyr His Ile Ser Arg Ala Ser Asp Ser Ser Leu
           595                 600                 605
   Phe Arg Ile Ser Ala Asn Ile Gly Glu Leu Arg Thr Ala Arg Leu Val
       610                 615                 620
   Leu Pro Thr Asp Ala Val Lys Gln Arg Val Val Val Val Val Arg Asp
   625                 630                 635                 640
   His Gly Asp Pro Pro Leu Ser Ser Ser Val Thr Leu Gly Val Leu Leu
                   645                 650                 655
   Ser Asn Ser Val Pro Gln Leu Leu Pro Asp Phe Glu Asp Val Trp Glu
               660                 665                 670
   Pro Gly Gly Gln Leu Ser Ala Gln Asn Leu Tyr Leu Val Ile Ala Leu
           675                 680                 685
   Ala Cys Ile Ser Phe Leu Phe Leu Gly Cys Leu Leu Phe Phe Val Cys
       690                 695                 700
   Thr Lys Leu His Gln Ser Pro Gly Cys Cys Ala Gln Ser Cys Cys Arg
   705                 710                 715                 720
   Ser Thr Glu Asp Leu Arg Tyr Gly Ser Lys Met Val Ser Asn Pro Cys
                   725                 730                 735
   Met Thr Ser Ala Thr Ile Asp Val Thr Thr Val Glu Arg Leu Ser Gln
               740                 745                 750
   Thr Tyr Leu Tyr Arg Ala Ser Leu Gly Leu Gly Ser Asp Asn Asn Ser
           755                 760                 765
   Leu Leu Leu Arg Gly Glu Tyr Asn Ala Ala Asp Leu Arg Asn Leu Ala
       770                 775                 780
   Thr Gly Val Gly Leu Asn Leu Pro Ile Ser Cys Ile Gln Ile Arg Asn
   785                 790                 795                 800
   Arg Lys Gly Asp His Ala Asn Val Asn Ala Met Pro Arg Gln Pro Asn
                   805                 810                 815
   Pro Asp Trp Arg Tyr Ser Ala Ser Leu Arg Ala Gly Met His Ser Ser
               820                 825                 830
   Val His Leu Glu Glu Ala Gly Ile Leu Arg Ala Gly Pro Gly Gly Pro
   835                 840                 845
   Asp Gln Gln Trp Pro Thr Val Ser Ser Ala Thr Pro Glu Pro Glu Ala
       850                 855                 860
   Gly Glu Val Ser Pro Pro Val Gly Ala Gly Val Asn Ser Asn Ser Trp
   865                 870                 875                 880
   Thr Phe Lys Tyr Gly Pro Gly Asn Pro Lys Gln Ser Gly Pro Gly Glu
                   885                 890                 895
   Leu Pro Asp Lys Phe Ile Ile Pro Gly Ser Pro Ala Ile Ile Ser Ile
               900                 905                 910
   Arg Gln Glu Pro Thr Asn Ser Gln Ile Asp Lys Ser Asp Phe Ile Thr
           915                 920                 925
   Phe Gly Lys Lys Glu Glu Thr Lys Lys Lys Lys Lys Lys Lys Lys Gly
       930                 935                 940
   Asn Lys Thr Gln Glu Lys Lys Glu Lys Gly Asn Ser Thr Thr Asp Asn
   945                 950                 955                 960
   Ser Asp Gln


<210>121
<211>2010
<212>DNA
<213>Homo sapiens

<220>
<221> CDS
<222>(7)..(2010)

<400>121

agatct gcg gga cag ctc gag tac tca gtg cgg gag gag acg gag cgg     48
       Ala Gly Gln Leu Glu Tyr Ser Val Arg Glu Glu Thr Glu Arg
       1               5                   10

ggc gta gcc gta ggc aat ctc tcc gcg gac ttg agg ctg cca gcg gcc     96
Gly Val Ala Val Gly Asn Leu Ser Ala Asp Leu Arg Leu Pro Ala Ala
15                  20                  25                  30

gct atg tcc tcg cgg aac ttt cgc ttc ctt tcc agc cac cgc gag ctc     144
Ala Met Ser Ser Arg Asn Phe Arg Phe Leu Ser Ser His Arg Glu Leu
                35                  40                  45

tac ttc ggg gtg gat cta ccc agc ggc aat ttg gtg gtc aga gag ccg     192
Tyr Phe Gly Val Asp Leu Pro Ser Gly Asn Leu Val Val Arg Glu Pro
            50                  55                  60

gcg gac cgc gaa cag ctg tgc agg gcc aaa gct gcc tgc gtc ttg acc     240
Ala Asp Arg Glu Gln Leu Cys Arg Ala Lys Ala Ala Cys Val Leu Thr
        65                  70                  75

tac gac ctg gtg ctc gag gac ccg ctg gag ctg cac aag att cgg att 288
Tyr Asp Leu Val Leu Glu Asp Pro Leu Glu Leu His Lys Ile Arg Ile
    80                  85                  90

cac gtc ctg gac acc aat gac aac tca cct ctc ttt cct gcc ggc gac 336
His Val Leu Asp Thr Asn Asp Asn Ser Pro Leu Phe Pro Ala Gly Asp
95                  100                 105                 110

gtg cag ctg cac atc ccc gag ttc ctg acg ccc gga gcc cgc ttt act 384
Val Gln Leu His Ile Pro Glu Phe Leu Thr Pro Gly Ala Arg Phe Thr
                115                 120                 125

ctc ccg aat gcc caa gat gac gac gag gga agc aat ggg ata cta agc 432
Leu Pro Asn Ala Gln Asp Asp Asp Glu Gly Ser Asn Gly Ile Leu Ser
            130                 135                 140

tac agc cta agc ccc agt cag cac ttt cgc ctg gac atg gga tcg cgg 480
Tyr Ser Leu Ser Pro Ser Gln His Phe Arg Leu Asp Met Gly Ser Arg
        145                 150                 155

gtt gac ggc agc gaa tac ccg gag ttg gtg ttg gag aaa gca ctg gat 528
Val Asp Gly Ser Glu Tyr Pro Glu Leu Val Leu Glu Lys Ala Leu Asp
    160                 165                 170

cgc gaa cag cgc gcc acc cac ctg ctg gtg ctt aca gct cgg gac ggc 576
Arg Glu Gln Arg Ala Thr His Leu Leu Val Leu Thr Ala Arg Asp Gly
175                 180                 185                 190

ggg cta cct gcc cgc tca gga gac gca caa gtc acc atc att gtg gtg 624
Gly Leu Pro Ala Arg Ser Gly Asp Ala Gln Val Thr Ile Ile Val Val
                195                 200                 205

gac aca aat gac aac gcg cct gta ttt gag cgc tcc gta tac cgc acc 672
Asp Thr Asn Asp Asn Ala Pro Val Phe Glu Arg Ser Val Tyr Arg Thr
            210                 215                 220

aag gtt cca gag act gca ccc aat ggg act gtg tta ttc cga gtt caa 720
Lys Val Pro Glu Thr Ala Pro Asn Gly Thr Val Leu Phe Arg Val Gln
        225                 230                 235

gcc ttg gat cca gat gaa ggg tcc aat ggg gaa gtc caa tac tcc cta 768
Ala Leu Asp Pro Asp Glu Gly Ser Asn Gly Glu Val Gln Tyr Ser Leu
    240                 245                 250

agc aac agc acg caa gca gag ctg cga cac cgc ttt cac gtg cac cct 816
Ser Asn Ser Thr Gln Ala Glu Leu Arg His Arg Phe His Val His Pro
255                 260                 265                 270

aaa agt ggg gag gtg caa gta gct gct tca cta ggt ccg cct gaa acg 864
Lys Ser Gly Glu Val Gln Val Ala Ala Ser Leu Gly Pro Pro Glu Thr
                275                 280                 285

ctc ttg gag gca tac att gag gcg agg gac gaa ggt gtc ttt ggt tta 912
Leu Leu Glu Ala Tyr Ile Glu Ala Arg Asp Glu Gly Val Phe Gly Leu
            290                 295                 300

gct agc acc gct aaa ctg ctg gtg gag gtg act gac gtg aac gat cat 960

Ala Ser Thr Ala Lys Leu Leu Val Glu Val Thr Asp Val Asn Asp His
        305                 310                 315

gcc ccc gaa ctg gac ttc ctg act ctt tcg aac cca gta cct gag gac    1008
Ala Pro Glu Leu Asp Phe Leu Thr Leu Ser Asn Pro Val Pro Glu Asp
    320                 325                 330

gct gcc cct ggc aca gtg att gct ctc ttt agt gta aag gat gaa gac    1056
Ala Ala Pro Gly Thr Val Ile Ala Leu Phe Ser Val Lys Asp Glu Asp
335                 340                 345                 350

ctc gat tct aat ggt agg gtc att tgt ggc atg tct agt gca ggc cct    1104
Leu Asp Ser Asn Gly Arg Val Ile Cys Gly Met Ser Ser Ala Gly Pro
                355                 360                 365

ttt cag ctg acg gct tcc ttt gac aac tac tac agc ctg ctg att gat    1152
Phe Gln Leu Thr Ala Ser Phe Asp Asn Tyr Tyr Ser Leu Leu Ile Asp
            370                 375                 380

ggg ccc ctg gac cgg gag cag atc agt gaa tac caa gtc ctg atc acg    1200
Gly Pro Leu Asp Arg Glu Gln Ile Ser Glu Tyr Gln Val Leu Ile Thr
        385                 390                 395

gcc tca gat agt ggc tca ccg cca ctt agc acc cga agg aca atc act    1248
Ala Ser Asp Ser Gly Ser Pro Pro Leu Ser Thr Arg Arg Thr Ile Thr
    400                 405                 410

gtg tca gtt gct gat gtg aat gac aat aca cca aac ttt cct caa ccc    1296
Val Ser Val Ala Asp Val Asn Asp Asn Thr Pro Asn Phe Pro Gln Pro
415                 420                 425                 430

cag cag gaa ctt ttc gtt gct gaa aac aat ggc cct ggg gcc tct cta    1344
Gln Gln Glu Leu Phe Val Ala Glu Asn Asn Gly Pro Gly Ala Ser Leu
                435                 440                 445

ggc cga gtg ttt gcc cag gac ccc gac ctg ggg aag aat ggc ctt gtc    1392
Gly Arg Val Phe Ala Gln Asp Pro Asp Leu Gly Lys Asn Gly Leu Val
            450                 455                 460

tct tat gag ctg ttg gat gtt atc tct gaa ggg cca tca gcc tct agc    1440
Ser Tyr Glu Leu Leu Asp Val Ile Ser Glu Gly Pro Ser Ala Ser Ser
        465                 470                 475

ttg gtg gca gtg gaa tca tcc agt ggg gcc atc act gcc aaa act tcc    1488
Leu Val Ala Val Glu Ser Ser Ser Gly Ala Ile Thr Ala Lys Thr Ser
    480                 485                 490

ttt gac ttt gag cag ctc agg ggg ttt cat ttc caa gta gaa ggc cgg    1536
Phe Asp Phe Glu Gln Leu Arg Gly Phe His Phe Gln Val Glu Gly Arg
495                 500                 505                 510

gat ggt ggc att cct ccc aga agt gca aca gtg act ata aac ttg ttt    1584
Asp Gly Gly Ile Pro Pro Arg Ser Ala Thr Val Thr Ile Asn Leu Phe
                515                 520                 525

gtg gta gat agg aat gac aat tat ccg gtt atc ttg ttt ccc ttg ccc    1632
Val Val Asp Arg Asn Asp Asn Tyr Pro Val Ile Leu Phe Pro Leu Pro

            530                 535                 540

aga aat ggt tct gtc cca gtg gaa att gtg ccc cgc tct gcc agg act 1680
Arg Asn Gly Ser Val Pro Val Glu Ile Val Pro Arg Ser Ala Arg Thr
        545                 550                 555

gga cac ttg gtc aca aaa gtg gta gca gag gat gct gac agt ggt tct 1728
Gly His Leu Val Thr Lys Val Val Ala Glu Asp Ala Asp Ser Gly Ser
    560                 565                 570

aat gcc tgg ctt tcc tac cac atc tcc cgg gcg tct gac tct agt ctc 1776
Asn Ala Trp Leu Ser Tyr His Ile Ser Arg Ala Ser Asp Ser Ser Leu
575                 580                 585                 590

ttt aga att tca gcc aat ata ggt gag ctc cgt act gct cgc tta gtt 1824
Phe Arg Ile Ser Ala Asn Ile Gly Glu Leu Arg Thr Ala Arg Leu Val
                595                 600                 605

ctt ccc act gat gca gtt aag cag agg gtg gtg gta gtg gtt cgg gac 1872
Leu Pro Thr Asp Ala Val Lys Gln Arg Val Val Val Val Val Arg Asp
            610                 615                 620

cat gga gac cca cca ctt tcc tcc tct gtc act ctg ggt gtg ctg ttg 1920
His Gly Asp Pro Pro Leu Ser Ser Ser Val Thr Leu Gly Val Leu Leu
        625                 630                 635

agc aac tct gtc cct cag tta ctt cca gac ttt gaa gat gtc tgg gaa 1968
Ser Asn Ser Val Pro Gln Leu Leu Pro Asp Phe Glu Asp Val Trp Glu
    640                 645                 650

cca gga ggg cag ctt tct gcc cag aac ttg tat tta gtc gac 2010
Pro Gly Gly Gln Leu Ser Ala Gln Asn Leu Tyr Leu Val Asp
655                 660                 665

<210>122
<211>668
<212>PRT
<213>Homo sapiens

<400>122

Ala Gly Gln Leu Glu Tyr Ser Val Arg Glu Glu Thr Glu Arg Gly Val
1               5                   10                  15

Ala Val Gly Asn Leu Ser Ala Asp Leu Arg Leu Pro Ala Ala Ala Met
            20                  25                  30

Ser Ser Arg Asn Phe Arg Phe Leu Ser Ser His Arg Glu Leu Tyr Phe
        35                  40                  45

Gly Val Asp Leu Pro Ser Gly Asn Leu Val Val Arg Glu Pro Ala Asp
    50                  55                  60

Arg Glu Gln Leu Cys Arg Ala Lys Ala Ala Cys Val Leu Thr Tyr Asp
65                  70                  75                  80

Leu Val Leu Glu Asp Pro Leu Glu Leu His Lys Ile Arg Ile His Val

85                  90                  95

Leu Asp Thr Asn Asp Asn Ser Pro Leu Phe Pro Ala Gly Asp Val Gln
            100                 105                 110

Leu His Ile Pro Glu Phe Leu Thr Pro Gly Ala Arg Phe Thr Leu Pro
        115                 120                 125

Asn Ala Gln Asp Asp Asp Glu Gly Ser Asn Gly Ile Leu Ser Tyr Ser
    130                 135                 140

Leu Ser Pro Ser Gln His Phe Arg Leu Asp Met Gly Ser Arg Val Asp
145                 150                 155                 160

Gly Ser Glu Tyr Pro Glu Leu Val Leu Glu Lys Ala Leu Asp Arg Glu
                165                 170                 175

Gln Arg Ala Thr His Leu Leu Val Leu Thr Ala Arg Asp Gly Gly Leu
            180                 185                 190

Pro Ala Arg Ser Gly Asp Ala Gln Val Thr Ile Ile Val Val Asp Thr
        195                 200                 205

Asn Asp Asn Ala Pro Val Phe Glu Arg Ser Val Tyr Arg Thr Lys Val
    210                 215                 220

Pro Glu Thr Ala Pro Asn Gly Thr Val Leu Phe Arg Val Gln Ala Leu
225                 230                 235                 240

Asp Pro Asp Glu Gly Ser Asn Gly Glu Val Gln Tyr Ser Leu Ser Asn
                245                 250                 255

Ser Thr Gln Ala Glu Leu Arg His Arg Phe His Val His Pro Lys Ser
            260                 265                 270

Gly Glu Val Gln Val Ala Ala Ser Leu Gly Pro Pro Glu Thr Leu Leu
        275                 280                 285

Glu Ala Tyr Ile Glu Ala Arg Asp Glu Gly Val Phe Gly Leu Ala Ser
    290                 295                 300

Thr Ala Lys Leu Leu Val Glu Val Thr Asp Val Asn Asp His Ala Pro
305                 310                 315                 320

Glu Leu Asp Phe Leu Thr Leu Ser Asn Pro Val Pro Glu Asp Ala Ala
                325                 330                 335

Pro Gly Thr Val Ile Ala Leu Phe Ser Val Lys Asp Glu Asp Leu Asp
            340                 345                 350

Ser Asn Gly Arg Val Ile Cys Gly Met Ser Ser Ala Gly Pro Phe Gln
        355                 360                 365

Leu Thr Ala Ser Phe Asp Asn Tyr Tyr Ser Leu Leu Ile Asp Gly Pro
    370                 375                 380

Leu Asp Arg Glu Gln Ile Ser Glu Tyr Gln Val Leu Ile Thr Ala Ser

385                 390                 395                 400

Asp Ser Gly Ser Pro Pro Leu Ser Thr Arg Arg Thr Ile Thr Val Ser
                405                 410                 415

Val Ala Asp Val Asn Asp Asn Thr Pro Asn Phe Pro Gln Pro Gln Gln
            420                 425                 430

Glu Leu Phe Val Ala Glu Asn Asn Gly Pro Gly Ala Ser Leu Gly Arg
        435                 440                 445

Val Phe Ala Gln Asp Pro Asp Leu Gly Lys Asn Gly Leu Val Ser Tyr
    450                 455                 460

Glu Leu Leu Asp Val Ile Ser Glu Gly Pro Ser Ala Ser Ser Leu Val
465                 470                 475                 480

Ala Val Glu Ser Ser Ser Gly Ala Ile Thr Ala Lys Thr Ser Phe Asp
                485                 490                 495

Phe Glu Gln Leu Arg Gly Phe His Phe Gln Val Glu Gly Arg Asp Gly
            500                 505                 510

Gly Ile Pro Pro Arg Ser Ala Thr Val Thr Ile Asn Leu Phe Val Val
        515                 520                 525

Asp Arg Asn Asp Asn Tyr Pro Val Ile Leu Phe Pro Leu Pro Arg Asn
    530                 535                 540

Gly Ser Val Pro Val Glu Ile Val Pro Arg Ser Ala Arg Thr Gly His
545                 550                 555                 560

Leu Val Thr Lys Val Val Ala Glu Asp Ala Asp Ser Gly Ser Asn Ala
                565                 570                 575

Trp Leu Ser Tyr His Ile Ser Arg Ala Ser Asp Ser Ser Leu Phe Arg
            580                 585                 590

Ile Ser Ala Asn Ile Gly Glu Leu Arg Thr Ala Arg Leu Val Leu Pro
        595                 600                 605

Thr Asp Ala Val Lys Gln Arg Val Val Val Val Val Arg Asp His Gly
    610                 615                 620

Asp Pro Pro Leu Ser Ser Ser Val Thr Leu Gly Val Leu Leu Ser Asn
625                 630                 635                 640

Ser Val Pro Gln Leu Leu Pro Asp Phe Glu Asp Val Trp Glu Pro Gly
                645                 650                 655

Gly Gln Leu Ser Ala Gln Asn Leu Tyr Leu Val Asp
            660                 665

<210>123
<211>3630
<212>DNA
<213>Homo sapiens

<220>
<221> CDS
<222>(85)..(3571)

<400>123
   tgcggccgcg gaaagaatgc gcgccgcccg tgcgctccgc ctgccgcgtc 50
   tggccacccg cagccgccgc gtccgcacct gacc atg gag tgc gcc ctc ctg 102
   Met Glu Cys Ala Leu Leu
   1 5
   ctc gcg tgt gcc ttc ccg gct gcg ggt tcg ggc ccg ccg agg ggc ctg 150
   Leu Ala Cys Ala Phe Pro Ala Ala Gly Ser Gly Pro Pro Arg Gly Leu
   10 15 20
   gcg gga ctg ggg cgc gtg gcc aag gcg ctc cag ctg tgc tgc ctc tgc 198
   Ala Gly Leu Gly Arg Val Ala Lys Ala Leu Gln Leu Cys Cys Leu Cys
   25 30 35
   tgt gcg tcg gtc gcc gcg gcc tta gcc agt gac agc agc agc ggc gcc 246
   Cys Ala Ser Val Ala Ala Ala Leu Ala Ser Asp Ser Ser Ser Gly Ala
   40 45 50
   agc gga tta aat gat gat tac gtc ttt gtc acg cca gta gaa gta gac 294
   Ser Gly Leu Asn Asp Asp Tyr Val Phe Val Thr Pro Val Glu Val Asp
   55 60 65 70
   tca gcc ggg tca tat att tca cac gac att ttg cac aac ggc agg aaa 342
   Ser Ala Gly Ser Tyr Ile Ser His Asp Ile Leu His Asn Gly Arg Lys
   75 80 85
   aag cga tcg gcg cag aat gcc aga agc tcc ctg cac tac cga ttt tca 390
   Lys Arg Ser Ala Gln Asn Ala Arg Ser Ser Leu His Tyr Arg Phe Ser
   90 95 100
   gca ttt gga cag gaa ctg cac tta gaa ctt aag ccc tcg gcg att ttg 438
   Ala Phe Gly Gln Glu Leu His Leu Glu Leu Lys Pro Ser Ala Ile Leu
   105 110 115
   agc agt cac ttt att gtc cag gta ctt gga aaa gat ggt gct tca gag 486
   Ser Ser His Phe Ile Val Gln Val Leu Gly Lys Asp Gly Ala Ser Glu
   120 125 130
   act cag aaa ccc gag gtg cag caa tgc ttc tat cag gga ttt atc aga 534
   Thr Gln Lys Pro Glu Val Gln Gln Cys Phe Tyr Gln Gly Phe Ile Arg
   135 140 145 150
   aat gac agc tcc tcc tct gtc gct gtg tct acg tgt gct ggc ttg tca 582
   Asn Asp Ser Ser Ser Ser Val Ala Val Ser Thr Cys Ala Gly Leu Ser
   155 160 165
   ggt tta ata agg aca cga aaa aat gaa ttc ctc atc tcg cca tta cct 630
   Gly Leu Ile Arg Thr Arg Lys Asn Glu Phe Leu Ile Ser Pro Leu Pro
   170 175 180
   cag ctt ctg gcc cag gaa cac aac tac agc tcc cct gcg ggt cac cat 678
   Gln Leu Leu Ala Gln Glu His Asn Tyr Ser Ser Pro Ala Gly His His
   185 190 195
   cct cac gta ctg tac aaa agg aca gca gag gag aag atc cag cgg tac 726
   Pro His Val Leu Tyr Lys Arg Thr Ala Glu Glu Lys Ile Gln Arg Tyr
   200 205 210
   cgt ggc tac ccc ggc tct ggc cgg aat tat cct ggt tac tcc cca agt 774
   Arg Gly Tyr Pro Gly Ser Gly Arg Asn Tyr Pro Gly Tyr Ser Pro Ser
   215 220 225 230
   cac att ccc cat gca tct cag agt cga gag aca gag tat cac cat cga 822
   His Ile Pro His Ala Ser Gln Ser Arg Glu Thr Glu Tyr His His Arg
   235 240 245
   agg ttg caa aag cag cat ttt tgt gga cga cgc aag aaa tat gct ccc 870
   Arg Leu Gln Lys Gln His Phe Cys Gly Arg Arg Lys Lys Tyr Ala Pro
   250 255 260
   aag cct ccc aca gag gac acc tat cta agg ttt gat gaa tat ggg agc 918
   Lys Pro Pro Thr Glu Asp Thr Tyr Leu Arg Phe Asp Glu Tyr Gly Ser
   265 270 275
   tct ggg cga ccc aga aga tca gct gga aaa tca caa aag ggc ctc aat 966
   Ser Gly Arg Pro Arg Arg Ser Ala Gly Lys Ser Gln Lys Gly Leu Asn
   280 285 290
   gtg gaa acc ctc gtg gtg gca gac aag aaa atg gtg gaa aag cat ggc 1014
   Val Glu Thr Leu Val Val Ala Asp Lys Lys Met Val Glu Lys His Gly
   295 300 305 310
   aag gga aat gtc acc aca tac att ctc aca gta atg aac atg gtt tct 1062
   Lys Gly Asn Val Thr Thr Tyr Ile Leu Thr Val Met Asn Met Val Ser
   315 320 325
   ggc cta ttt aaa gat ggg act att gga agt gac ata aac gtg gtt gtg 1110
   Gly Leu Phe Lys Asp Gly Thr Ile Gly Ser Asp Ile Asn Val Val Val
   330 335 340
   gtg agc cta att ctt ctg gaa caa gaa cct gga gga tta ttg atc aac 1158
   Val Ser Leu Ile Leu Leu Glu Gln Glu Pro Gly Gly Leu Leu Ile Asn
   345 350 355
   cat cat gca gac cag tct ctg aat agt ttt tgt caa tgg cag tct gcc 1206
   His His Ala Asp Gln Ser Leu Asn Ser Phe Cys Gln Trp Gln Ser Ala
   360 365 370
   ctc att gga aag aat ggc aag aga cat gat cat gcc atc tta cta aca 1254
   Leu Ile Gly Lys Asn Gly Lys Arg His Asp His Ala Ile Leu Leu Thr
   375 380 385 390
   gga ttt gat att tgt tct tgg aag aat gaa cca tgt gac act cta ggg 1302
   Gly Phe Asp Ile Cys Ser Trp Lys Asn Glu Pro Cys Asp Thr Leu Gly
   395 400 405
   ttt gcc ccc atc agt gga atg tgc tct aag tac cga agt tgt acc atc 1350
   Phe Ala Pro Ile Ser Gly Met Cys Ser Lys Tyr Arg Ser Cys Thr Ile
   410 415 420
   aat gag gac aca gga ctt ggc ctt gcc ttc acc atc gct cat gag tca     1398
   Asn Glu Asp Thr Gly Leu Gly Leu Ala Phe Thr Ile Ala His Glu Ser
           425                 430                 435
   ggg cac aac ttt ggt atg att cac gat gga gaa ggg aat ccc tgc aga     1446
   Gly His Asn Phe Gly Met Ile His Asp Gly Glu Gly Asn Pro Cys Arg
       440                 445                 450
   aag gct gaa ggc aat atc atg tct ccc aca ctg acc gga aac aat gga     1494
   Lys Ala Glu Gly Asn Ile Met Ser Pro Thr Leu Thr Gly Asn Asn Gly
   455                 460                 465                 470
   gtg ttt tca tgg tct tcc tgc agc cgc cag tat ctc aag aaa ttc ctc     1542
   Val Phe Ser Trp Ser Ser Cys Ser Arg Gln Tyr Leu Lys Lys Phe Leu
                   475                 480                 485
   agc aca cct cag gcg ggg tgt cta gtg gat gag ccc aag caa gca gga     1590
   Ser Thr Pro Gln Ala Gly Cys Leu Val Asp Glu Pro Lys Gln Ala Gly
               490                 495                 500
   cag tat aaa tat ccg gac aaa cta cca gga cag att tat gat gct gac     1638
   Gln Tyr Lys Tyr Pro Asp Lys Leu Pro Gly Gln Ile Tyr Asp Ala Asp
           505                 510                 515
   aca cag tgt aaa tgg caa ttt gga gca aaa gcc aag tta tgc agc ctt     1686
   Thr Gln Cys Lys Trp Gln Phe Gly Ala Lys Ala Lys Leu Cys Ser Leu
       520                 525                 530
   ggt ttt gtg aag gat att tgc aaa tca ctt tgg tgc cac cga gta ggc     1734
   Gly Phe Val Lys Asp Ile Cys Lys Ser Leu Trp Cys His Arg Val Gly
   535                 540                 545                 550
   cac agg tgt gag acc aag ttt atg ccc gca gca gaa ggg acc gtt tgt     1782
   His Arg Cys Glu Thr Lys Phe Met Pro Ala Ala Glu Gly Thr Val Cys
                   555                 560                 565
   ggc ttg agt atg tgg tgt cgg caa ggc cag tgc gta aag ttt ggg gag     1830
   Gly Leu Ser Met Trp Cys Arg Gln Gly Gln Cys Val Lys Phe Gly Glu
               570                 575                 580
   ctc ggg ccc cgg ccc atc cac ggc cag tgg tcc gcc tgg tcg aag tgg     1878
   Leu Gly Pro Arg Pro Ile His Gly Gln Trp Ser Ala Trp Ser Lys Trp
           585                 590                 595
   tca gaa tgt tcc cgg aca tgt ggt gga gga gtc aag ttc cag gag aga     1926
   Ser Glu Cys Ser Arg Thr Cys Gly Gly Gly Val Lys Phe Gln Glu Arg
       600                 605                 610
   cac tgc aat aac ccc aag cct cag tat ggt ggc tta ttc tgt cca ggt     1974
   His Cys Asn Asn Pro Lys Pro Gln Tyr Gly Gly Leu Phe Cys Pro Gly
   615                 620                 625                 630
   tct agc cgt att tat cag ctg tgc aat att aac cct tgc aat gaa aat 2022
   Ser Ser Arg Ile Tyr Gln Leu Cys Asn Ile Asn Pro Cys Asn Glu Asn
   635 640 645
   agc ttg gat ttt cgg gct caa cag tgt gca gaa tat aac agc aaa cct 2070
   Ser Leu Asp Phe Arg Ala Gln Gln Cys Ala Glu Tyr Asn Ser Lys Pro
   650 655 660
   ttc cgt gga tgg ttc tac cag tgg aaa ccc tat aca aaa gtg gaa gag 2118
   Phe Arg Gly Trp Phe Tyr Gln Trp Lys Pro Tyr Thr Lys Val Glu Glu
   665 670 675
   gaa gat cga tgc aaa ctg tac tgc aag gct gag aac ttt gaa ttt ttt 2166
   Glu Asp Arg Cys Lys Leu Tyr Cys Lys Ala Glu Asn Phe Glu Phe Phe
   680 685 690
   ttt gca atg tcc ggc aaa gtg aaa gat gga act ccc tgc tcc cca aac 2214
   Phe Ala Met Ser Gly Lys Val Lys Asp Gly Thr Pro Cys Ser Pro Asn
   695 700 705 710
   aaa aat gat gtt tgt att gac ggg gtt tgt gaa cta gtg gga tgt gat 2262
   Lys Asn Asp Val Cys Ile Asp Gly Val Cys Glu Leu Val Gly Cys Asp
   715 720 725
   cat gaa cta ggc tct aaa gca gtt tca gat gct tgt ggc gtt tgc aaa 2310
   His Glu Leu Gly Ser Lys Ala Val Ser Asp Ala Cys Gly Val Cys Lys
   730 735 740
   ggt gat aat tca act tgc aag ttt tat aaa ggc ctg tac ctc aac cag 2358
   Gly Asp Asn Ser Thr Cys Lys Phe Tyr Lys Gly Leu Tyr Leu Asn Gln
   745 750 755
   cat aaa gca aat gaa tat tat ccg gtg gtc ctc att cca gct ggc gcc 2406
   His Lys Ala Asn Glu Tyr Tyr Pro Val Val Leu Ile Pro Ala Gly Ala
   760 765 770
   cga agc atc gaa atc cag gag ctg cag gtt tcc tcc agt tac ctc gca 2454
   Arg Ser Ile Glu Ile Gln Glu Leu Gln Val Ser Ser Ser Tyr Leu Ala
   775 780 785 790
   gtt cga agc ctc agt caa aag tat tac ctc acc ggg ggc tgg agc atc 2502
   Val Arg Ser Leu Ser Gln Lys Tyr Tyr Leu Thr Gly Gly Trp Ser Ile
   795 800 805
   gac tgg cct ggg gag ttc ccc ttc gct ggg acc acg ttt gaa tac cag 2550
   Asp Trp Pro Gly Glu Phe Pro Phe Ala Gly Thr Thr Phe Glu Tyr Gln
   810 815 820
   cgc tct ttc aac cgc ccg gaa cgt ctg tac gcg cca ggg ccc aca aat 2598
   Arg Ser Phe Asn Arg Pro Glu Arg Leu Tyr Ala Pro Gly Pro Thr Asn
   825 830 835
   gag acg ctg att ctg atg caa ggc aaa aat cca ggg ata gct tgg aag 2646
   Glu Thr Leu Ile Leu Met Gln Gly Lys Asn Pro Gly Ile Ala Trp Lys
   840 845 850
   tat gca ctt ccc aag gtc atg aat gga act cca cca gcc aca aaa aga 2694
   Tyr Ala Leu Pro Lys Val Met Asn Gly Thr Pro Pro Ala Thr Lys Arg
   855                 860                 865                 870
   cct gcc tat acc tgc tgg atg cca ggt gaa tgg agt aca tgc agc aag     2742
   Pro Ala Tyr Thr Cys Trp Met Pro Gly Glu Trp Ser Thr Cys Ser Lys
                   875                 880                 885
   gcc tgt gct gga ggc cag cag agc cga aag atc cag tgt gtg caa aag     2790
   Ala Cys Ala Gly Gly Gln Gln Ser Arg Lys Ile Gln Cys Val Gln Lys
               890                 895                 900
   aag ccc ttc caa aag gag gaa gca gtg ttg cat tct ctc tgt cca gtg     2838
   Lys Pro Phe Gln Lys Glu Glu Ala Val Leu His Ser Leu Cys Pro Val
           905                 910                 915
   agc aca ccc act cag gtc caa gcc tgc aac agc cat gcc tgc cct cca     2886
   Ser Thr Pro Thr Gln Val Gln Ala Cys Asn Ser His Ala Cys Pro Pro
       920                 925                 930
   caa tgg agc ctt gga ccc tgg tct cag tgt tcc aag acc tgt gga cga     2934
   Gln Trp Ser Leu Gly Pro Trp Ser Gln Cys Ser Lys Thr Cys Gly Arg
   935                 940                 945                 950
   ggg gtg agg aag cgt gaa ctc ctc tgc aag ggc tct gcc gca gaa acc     2982
   Gly Val Arg Lys Arg Glu Leu Leu Cys Lys Gly Ser Ala Ala Glu Thr
                   955                 960                 965
   ctc ccc gag agc cag tgt acc agt ctc ccc aga cct gag ctg cag gag     3030
   Leu Pro Glu Ser Gln Cys Thr Ser Leu Pro Arg Pro Glu Leu Gln Glu
               970                 975                 980
   ggc tgt gtg ctt gga cga tgc ccc aag aac agc cgg cta cag tgg gtc     3078
   Gly Cys Val Leu Gly Arg Cys Pro Lys Asn Ser Arg Leu Gln Trp Val
           985                 990                 995
   gct tct tcg tgg agc gag tgt tct gca acc tgt ggt ttg ggt gtg agg     3126
   Ala Ser Ser Trp Ser Glu Cys Ser Ala Thr Cys Gly Leu Gly Val Arg
       1000                1005                1010
   aag agg gag atg aag tgc agc gag aag ggc ttc cag gga aag ctg ata     3174
   Lys Arg Glu Met Lys Cys Ser Glu Lys Gly Phe Gln Gly Lys Leu Ile
   1015                1020                1025                1030
   act ttc cca gag cga aga tgc cgt aat att aag aaa cca aat ctg gac     3222
   Thr Phe Pro Glu Arg Arg Cys Arg Asn Ile Lys Lys Pro Asn Leu Asp
                   1035                1040                1045
   ttg gaa gag acc tgc aac cga cgg gct tgc cca gcc cat cca gtg tac     3270
   Leu Glu Glu Thr Cys Asn Arg Arg Ala Cys Pro Ala His Pro Val Tyr
               1050                1055                1060
   aac atg gta gct gga tgg tat tca ttg ccg tgg cag cag tgc aca gtc     3318
   Asn Met Val Ala Gly Trp Tyr Ser Leu Pro Trp Gln Gln Cys Thr Val
           1065                1070                1075
   acc tgt ggg gga ggg gtc cag acc cgg tca gtc cac tgt gtt cag caa     3366
   Thr Cys Gly Gly Gly Val Gln Thr Arg Ser Val His Cys Val Gln Gln
   1080                1085                1090
   ggc cgg cct tcc tca agt tgt ctg ctc cat cag aaa cct ccg gtg cta    3414
   Gly Arg Pro Ser Ser Ser Cys Leu Leu His Gln Lys Pro Pro Val Leu
   1095                1100                1105                1110
   cga gcc tgt aat aca aac ttc tgt cca gct cct gaa aag aga gag gat    3462
   Arg Ala Cys Asn Thr Asn Phe Cys Pro Ala Pro Glu Lys Arg Glu Asp
                   1115                1120                1125
   cca tcc tgc gta gat ttc ttc aac tgg tgt cac cta gtt cct cag cat    3510
   Pro Ser Cys Val Asp Phe Phe Asn Trp Cys His Leu Val Pro Gln His
               1130                1135                1140
   ggt gtc tgc aac cac aag ttt tac gga aaa caa tgc tgc aag tca tgc    3558
   Gly Val Cys Asn His Lys Phe Tyr Gly Lys Gln Cys Cys Lys Ser Cys
           1145                1150                1155
   aca agg aag atc tgatcttggt gtcctcccca gcaccttatg gccaggggct        3610
   Thr Arg Lys Ile
       1160
   tacctttcaa cctctagaga                                              3630

<210>124
<211>1162
<212>PRT
<213>Homo sapiens

<400>124

Met Glu Cys Ala Leu Leu Leu Ala Cys Ala Phe Pro Ala Ala Gly Ser
1               5                   10                  15

Gly Pro Pro Arg Gly Leu Ala Gly Leu Gly Arg Val Ala Lys Ala Leu
            20                  25                  30

Gln Leu Cys Cys Leu Cys Cys Ala Ser Val Ala Ala Ala Leu Ala Ser
        35                  40                  45

Asp Ser Ser Ser Gly Ala Ser Gly Leu Asn Asp Asp Tyr Val Phe Val
    50                  55                  60

Thr Pro Val Glu Val Asp Ser Ala Gly Ser Tyr Ile Ser His Asp Ile
65                  70                  75                  80

Leu His Asn Gly Arg Lys Lys Arg Ser Ala Gln Asn Ala Arg Ser Ser
                85                  90                  95

Leu His Tyr Arg Phe Ser Ala Phe Gly Gln Glu Leu His Leu Glu Leu
            100                 105                 110

Lys Pro Ser Ala Ile Leu Ser Ser His Phe Ile Val Gln Val Leu Gly
        115                 120                 125

Lys Asp Gly Ala Ser Glu Thr Gln Lys Pro Glu Val Gln Gln Cys Phe
    130                 135                 140

Tyr Gln Gly Phe Ile Arg Asn Asp Ser Ser Ser Ser Val Ala Val Ser
145                 150                 155                 160

Thr Cys Ala Gly Leu Ser Gly Leu Ile Arg Thr Arg Lys Asn Glu Phe
                165                 170                 175

Leu Ile Ser Pro Leu Pro Gln Leu Leu Ala Gln Glu His Asn Tyr Ser
            180                 185                 190

Ser Pro Ala Gly His His Pro His Val Leu Tyr Lys Arg Thr Ala Glu
        195                 200                 205

Glu Lys Ile Gln Arg Tyr Arg Gly Tyr Pro Gly Ser Gly Arg Asn Tyr
    210                 215                 220

Pro Gly Tyr Ser Pro Ser His Ile Pro His Ala Ser Gln Ser Arg Glu
225                 230                 235                 240

Thr Glu Tyr His His Arg Arg Leu Gln Lys Gln His Phe Cys Gly Arg
                245                 250                 255

Arg Lys Lys Tyr Ala Pro Lys Pro Pro Thr Glu Asp Thr Tyr Leu Arg
            260                 265                 270

Phe Asp Glu Tyr Gly Ser Ser Gly Arg Pro Arg Arg Ser Ala Gly Lys
        275                 280                 285

Ser Gln Lys Gly Leu Asn Val Glu Thr Leu Val Val Ala Asp Lys Lys
    290                 295                 300

Met Val Glu Lys His Gly Lys Gly Asn Val Thr Thr Tyr Ile Leu Thr
305                 310                 315                 320

Val Met Asn Met Val Ser Gly Leu Phe Lys Asp Gly Thr Ile Gly Ser
                325                 330                 335

Asp Ile Asn Val Val Val Val Ser Leu Ile Leu Leu Glu Gln Glu Pro
            340                 345                 350

Gly Gly Leu Leu Ile Asn His His Ala Asp Gln Ser Leu Asn Ser Phe
        355                 360                 365

Cys Gln Trp Gln Ser Ala Leu Ile Gly Lys Asn Gly Lys Arg His Asp
    370                 375                 380

His Ala Ile Leu Leu Thr Gly Phe Asp Ile Cys Ser Trp Lys Asn Glu
385                 390                 395                 400

Pro Cys Asp Thr Leu Gly Phe Ala Pro Ile Ser Gly Met Cys Ser Lys
                405                 410                 415

Tyr Arg Ser Cys Thr Ile Asn Glu Asp Thr Gly Leu Gly Leu Ala Phe
            420                 425                 430

Thr Ile Ala His Glu Ser Gly His Asn Phe Gly Met Ile His Asp Gly
        435                 440                 445

Glu Gly Asn Pro Cys Arg Lys Ala Glu Gly Asn Ile Met Ser Pro Thr
    450                 455                 460

Leu Thr Gly Asn Asn Gly Val Phe Ser Trp Ser Ser Cys Ser Arg Gln
465                 470                 475                 480

Tyr Leu Lys Lys Phe Leu Ser Thr Pro Gln Ala Gly Cys Leu Val Asp
                485                 490                 495

Glu Pro Lys Gln Ala Gly Gln Tyr Lys Tyr Pro Asp Lys Leu Pro Gly
            500                 505                 510

Gln Ile Tyr Asp Ala Asp Thr Gln Cys Lys Trp Gln Phe Gly Ala Lys
        515                 520                 525

Ala Lys Leu Cys Ser Leu Gly Phe Val Lys Asp Ile Cys Lys Ser Leu
    530                 535                 540

Trp Cys His Arg Val Gly His Arg Cys Glu Thr Lys Phe Met Pro Ala
545                 550                 555                 560

Ala Glu Gly Thr Val Cys Gly Leu Ser Met Trp Cys Arg Gln Gly Gln
                565                 570                 575

Cys Val Lys Phe Gly Glu Leu Gly Pro Arg Pro Ile His Gly Gln Trp
            580                 585                 590

Ser Ala Trp Ser Lys Trp Ser Glu Cys Ser Arg Thr Cys Gly Gly Gly
        595                 600                 605

Val Lys Phe Gln Glu Arg His Cys Asn Asn Pro Lys Pro Gln Tyr Gly
    610                 615                 620

Gly Leu Phe Cys Pro Gly Ser Ser Arg Ile Tyr Gln Leu Cys Asn Ile
625                 630                 635                 640

Asn Pro Cys Asn Glu Asn Ser Leu Asp Phe Arg Ala Gln Gln Cys Ala
                645                 650                 655

Glu Tyr Asn Ser Lys Pro Phe Arg Gly Trp Phe Tyr Gln Trp Lys Pro
            660                 665                 670

Tyr Thr Lys Val Glu Glu Glu Asp Arg Cys Lys Leu Tyr Cys Lys Ala
        675                 680                 685

Glu Asn Phe Glu Phe Phe Phe Ala Met Ser Gly Lys Val Lys Asp Gly
    690                 695                 700

Thr Pro Cys Ser Pro Asn Lys Asn Asp Val Cys Ile Asp Gly Val Cys
705                 710                 715                 720

Glu Leu Val Gly Cys Asp His Glu Leu Gly Ser Lys Ala Val Ser Asp
                725                 730                 735

Ala Cys Gly Val Cys Lys Gly Asp Asn Ser Thr Cys Lys Phe Tyr Lys
            740                 745                 750

Gly Leu Tyr Leu Asn Gln His Lys Ala Asn Glu Tyr Tyr Pro Val Val
        755                 760                 765

Leu Ile Pro Ala Gly Ala Arg Ser Ile Glu Ile Gln Glu Leu Gln Val
    770                 775                 780

Ser Ser Ser Tyr Leu Ala Val Arg Ser Leu Ser Gln Lys Tyr Tyr Leu
785                 790                 795                 800

Thr Gly Gly Trp Ser Ile Asp Trp Pro Gly Glu Phe Pro Phe Ala Gly
                805                 810                 815

Thr Thr Phe Glu Tyr Gln Arg Ser Phe Asn Arg Pro Glu Arg Leu Tyr
            820                 825                 830

Ala Pro Gly Pro Thr Asn Glu Thr Leu Ile Leu Met Gln Gly Lys Asn
        835                 840                 845

Pro Gly Ile Ala Trp Lys Tyr Ala Leu Pro Lys Val Met Asn Gly Thr
    850                 855                 860

Pro Pro Ala Thr Lys Arg Pro Ala Tyr Thr Cys Trp Met Pro Gly Glu
865                 870                 875                 880

Trp Ser Thr Cys Ser Lys Ala Cys Ala Gly Gly Gln Gln Ser Arg Lys
                885                 890                 895

Ile Gln Cys Val Gln Lys Lys Pro Phe Gln Lys Glu Glu Ala Val Leu
            900                 905                 910

His Ser Leu Cys Pro Val Ser Thr Pro Thr Gln Val Gln Ala Cys Asn
        915                 920                 925

Ser His Ala Cys Pro Pro Gln Trp Ser Leu Gly Pro Trp Ser Gln Cys
    930                 935                 940

Ser Lys Thr Cys Gly Arg Gly Val Arg Lys Arg Glu Leu Leu Cys Lys
945                 950                 955                 960

Gly Ser Ala Ala Glu Thr Leu Pro Glu Ser Gln Cys Thr Ser Leu Pro
                965                 970                 975

Arg Pro Glu Leu Gln Glu Gly Cys Val Leu Gly Arg Cys Pro Lys Asn
            980                 985                 990

Ser Arg Leu Gln Trp Val Ala Ser Ser Trp Ser Glu Cys Ser Ala Thr
        995                 1000                1005

Cys Gly Leu Gly Val Arg Lys Arg Glu Met Lys Cys Ser Glu Lys Gly
    1010                1015                1020

Phe Gln Gly Lys Leu Ile Thr Phe Pro Glu Arg Arg Cys Arg Asn Ile
1025                1030                1035                1040

Lys Lys Pro Asn Leu Asp Leu Glu Glu Thr Cys Asn Arg Arg Ala Cys
                1045                1050                1055

Pro Ala His Pro Val Tyr Asn Met Val Ala Gly Trp Tyr Ser Leu Pro
            1060                1065                1070

Trp Gln Gln Cys Thr Val Thr Cys Gly Gly Gly Val Gln Thr Arg Ser
        1075                1080                1085

Val His Cys Val Gln Gln Gly Arg Pro Ser Ser Ser Cys Leu Leu His
    1090                1095                1100

Gln Lys Pro Pro Val Leu Arg Ala Cys Asn Thr Asn Phe Cys Pro Ala
1105                1110                1115                1120

Pro Glu Lys Arg Glu Asp Pro Ser Cys Val Asp Phe Phe Asn Trp Cys
                1125                1130                1135

His Leu Val Pro Gln His Gly Val Cys Asn His Lys Phe Tyr Gly Lys
            1140                1145                1150

Gln Cys Cys Lys Ser Cys Thr Arg Lys Ile
        1155                1160

<210>125
<211>1059
<212>DNA
<213>Homo sapiens

<220>
<221> CDS
<222>(7)..(1054)

<400>125

aagctt gtg gaa acc ctc gtg gtg gca gac aag caa atg gtg gaa aag 48
Val Glu Thr Leu Val Val Ala Asp Lys Gln Met Val Glu Lys
1               5                   10

cat ggc aag gga aat gtc acc aca tac att ctc aca gta atg aac atg 96
His Gly Lys Gly Asn Val Thr Thr Tyr Ile Leu Thr Val Met Asn Met
15                  20                  25                  30

gtt tct ggc cta ttt aaa gat ggg act att gga agt gac ata aac gtg 144
Val Ser Gly Leu Phe Lys Asp Gly Thr Ile Gly Ser Asp Ile Asn Val
                35                  40                  45

gtt gtg gtg agc cta att ctt ctg gaa caa gaa cct gga gga tta ttg 192
Val Val Val Ser Leu Ile Leu Leu Glu Gln Glu Pro Gly Gly Leu Leu
            50                  55                  60

atc aac cat cat gca gac cag tct ctg aat agt ttt tgt caa tgg cag 240
Ile Asn His His Ala Asp Gln Ser Leu Asn Ser Phe Cys Gln Trp Gln
        65                  70                  75

tct gcc ctc att gga aag aat ggc aag aga cat gat cat gcc atc tta 288
Ser Ala Leu Ile Gly Lys Asn Gly Lys Arg His Asp His Ala Ile Leu

80                    85                    90

cta aca gga ttt gat att tgt tct tgg aag aat gaa cca tgt gac act    336
Leu Thr Gly Phe Asp Ile Cys Ser Trp Lys Asn Glu Pro Cys Asp Thr
 95                 100                 105                 110

cta ggg ttt gcc ccc atc agt gga atg tgc tct aag tac cga agt tgt    384
Leu Gly Phe Ala Pro Ile Ser Gly Met Cys Ser Lys Tyr Arg Ser Cys
                115                 120                 125

acc atc aat gag gac aca gga ctt ggc ctt gcc ttc acc atc gct cat    432
Thr Ile Asn Glu Asp Thr Gly Leu Gly Leu Ala Phe Thr Ile Ala His
            130                 135                 140

gag tca ggg cac aac ttt ggt atg att cac gac gga gaa ggg aat ccc    480
Glu Ser Gly His Asn Phe Gly Met Ile His Asp Gly Glu Gly Asn Pro
        145                 150                 155

tgc aga aag gct gaa ggc aat atc atg tct ccc aca ctg acc gga aac    528
Cys Arg Lys Ala Glu Gly Asn Ile Met Ser Pro Thr Leu Thr Gly Asn
    160                 165                 170

aat gga gtg ttt tca tgg tct tcc tgc agc cgc cag tat ctc aag aaa    576
Asn Gly Val Phe Ser Trp Ser Ser Cys Ser Arg Gln Tyr Leu Lys Lys
175                 180                 185                 190

ttc ctc agc aca cct cag gcg ggg tgt cta gtg gat gag ccc aag caa    624
Phe Leu Ser Thr Pro Gln Ala Gly Cys Leu Val Asp Glu Pro Lys Gln
                195                 200                 205

gca gga cag tat aaa tat ccg gac aaa cta cca gga cag att tat gat    672
Ala Gly Gln Tyr Lys Tyr Pro Asp Lys Leu Pro Gly Gln Ile Tyr Asp
            210                 215                 220

gct gac aca ctg tgt aaa tgg caa ttt gga gca aaa gcc aag tta tgc    720
Ala Asp Thr Leu Cys Lys Trp Gln Phe Gly Ala Lys Ala Lys Leu Cys
        225                 230                 235

agc ctt ggt ttt gtg aag gat att tgc aaa tca ctt tgg tgc cac cga    768
Ser Leu Gly Phe Val Lys Asp Ile Cys Lys Ser Leu Trp Cys His Arg
    240                 245                 250

gta ggc cac agg tgt gag acc aag ttt atg ccc gca gca gaa ggg acc    816
Val Gly His Arg Cys Glu Thr Lys Phe Met Pro Ala Ala Glu Gly Thr
255                 260                 265                 270

gtt tgt ggc ttg agt atg tgg tgt cgg caa ggc cag tgc gta aag ttt    864
Val Cys Gly Leu Ser Met Trp Cys Arg Gln Gly Gln Cys Val Lys Phe
                275                 280                 285

ggg gag ctc ggg ccc cgg ccc atc cac ggc cag tgg tcc gcc tgg tcg    912
Gly Glu Leu Gly Pro Arg Pro Ile His Gly Gln Trp Ser Ala Trp Ser
            290                 295                 300

aag tgg tca gaa tgt tcc cgg aca tgt ggt gga gga gtc aag ttc cag    960
Lys Trp Ser Glu Cys Ser Arg Thr Cys Gly Gly Gly Val Lys Phe Gln
        305                 310                 315

gag aga cac tgc aat aac ccc aag cct cag tat ggt ggc ttg ttc tgt    1008
Glu Arg His Cys Asn Asn Pro Lys Pro Gln Tyr Gly Gly Leu Phe Cys
    320                 325                 330

cca ggt tct agc cgt att tat cag ctg tgc aat att aac cct tgc        1053
Pro Gly Ser Ser Arg Ile Tyr Gln Leu Cys Asn Ile Asn Pro Cys
335                 340                 345

ctcgag                                                             1059

<210>126
<211>349
<212>PRT
<213>Homo sapiens

<400>126

Val Glu Thr Leu Val Val Ala Asp Lys Gln Met Val Glu Lys His Gly
  1               5                  10                  15

Lys Gly Asn Val Thr Thr Tyr Ile Leu Thr Val Met Asn Met Val Ser
             20                  25                  30

Gly Leu Phe Lys Asp Gly Thr Ile Gly Ser Asp Ile Asn Val Val Val
         35                  40                  45

Val Ser Leu Ile Leu Leu Glu Gln Glu Pro Gly Gly Leu Leu Ile Asn
     50                  55                  60

His His Ala Asp Gln Ser Leu Asn Ser Phe Cys Gln Trp Gln Ser Ala
 65                  70                  75                  80

Leu Ile Gly Lys Asn Gly Lys Arg His Asp His Ala Ile Leu Leu Thr
                 85                  90                  95

Gly Phe Asp Ile Cys Ser Trp Lys Asn Glu Pro Cys Asp Thr Leu Gly
            100                 105                 110

Phe Ala Pro Ile Ser Gly Met Cys Ser Lys Tyr Arg Ser Cys Thr Ile
        115                 120                 125

Asn Glu Asp Thr Gly Leu Gly Leu Ala Phe Thr Ile Ala His Glu Ser
    130                 135                 140

Gly His Asn Phe Gly Met Ile His Asp Gly Glu Gly Asn Pro Cys Arg
145                 150                 155                 160

Lys Ala Glu Gly Asn Ile Met Ser Pro Thr Leu Thr Gly Asn Asn Gly
                165                 170                 175

Val Phe Ser Trp Ser Ser Cys Ser Arg Gln Tyr Leu Lys Lys Phe Leu
            180                 185                 190

Ser Thr Pro Gln Ala Gly Cys Leu Val Asp Glu Pro Lys Gln Ala Gly

195                 200                 205

Gln Tyr Lys Tyr Pro Asp Lys Leu Pro Gly Gln Ile Tyr Asp Ala Asp
    210                 215                 220

Thr Leu Cys Lys Trp Gln Phe Gly Ala Lys Ala Lys Leu Cys Ser Leu
225                 230                 235                 240

Gly Phe Val Lys Asp Ile Cys Lys Ser Leu Trp Cys His Arg Val Gly
                245                 250                 255

His Arg Cys Glu Thr Lys Phe Met Pro Ala Ala Glu Gly Thr Val Cys
            260                 265                 270

Gly Leu Ser Met Trp Cys Arg Gln Gly Gln Cys Val Lys Phe Gly Glu
        275                 280                 285

Leu Gly Pro Arg Pro Ile His Gly Gln Trp Ser Ala Trp Ser Lys Trp
    290                 295                 300

Ser Glu Cys Ser Arg Thr Cys Gly Gly Gly Val Lys Phe Gln Glu Arg
305                 310                 315                 320

His Cys Asn Asn Pro Lys Pro Gln Tyr Gly Gly Leu Phe Cys Pro Gly
                325                 330                 335

Ser Ser Arg Ile Tyr Gln Leu Cys Asn Ile Asn Pro Cys
            340                 345

<210>127
<211>1059
<212>DNA
<213>Homo sapiens

<220>
<221> CDS
<222>(1)..(1059)

<400>127

aag ctt gtg gaa acc ctc gtg gtg gca gac aag caa atg gtg gaa aag     48
Lys Leu Val Glu Thr Leu Val Val Ala Asp Lys Gln Met Val Glu Lys
  1               5                  10                  15

cat ggc aag gga aat gtc acc aca tac att ctc aca gta atg aac atg     96
His Gly Lys Gly Asn Val Thr Thr Tyr Ile Leu Thr Val Met Asn Met
             20                  25                  30

gtt tct ggc cta ttt aaa gat ggg act att gga agt gac ata aac gtg     144
Val Ser Gly Leu Phe Lys Asp Gly Thr Ile Gly Ser Asp Ile Asn Val
         35                  40                  45

gtt gtg gtg agc cta att ctt ctg gaa caa gaa cct gga gga tta ttg     192
Val Val Val Ser Leu Ile Leu Leu Glu Gln Glu Pro Gly Gly Leu Leu
     50                  55                  60

atc aac cat cat gca gac cag tct ctg aat agt ttt tgt caa tgg cag     240

Ile Asn His His Ala Asp Gln Ser Leu Asn Ser Phe Cys Gln Trp Gln
65                  70                  75                  80

tct gcc ctc att gga aag aat ggc aag aga cat gat cat gcc atc tta     288
Ser Ala Leu Ile Gly Lys Asn Gly Lys Arg His Asp His Ala Ile Leu
                85                  90                  95

cta aca gga ttt gat att tgt tct tgg aag aat gaa cca tgt gac act     336
Leu Thr Gly Phe Asp Ile Cys Ser Trp Lys Asn Glu Pro Cys Asp Thr
            100                 105                 110

cta ggg ttt gcc ccc atc agt gga atg tgc tct aag tac cga agt tgt     384
Leu Gly Phe Ala Pro Ile Ser Gly Met Cys Ser Lys Tyr Arg Ser Cys
        115                 120                 125

acc atc aat gag gac aca gga ctt ggc ctt gcc ttc acc atc gct cat     432
Thr Ile Asn Glu Asp Thr Gly Leu Gly Leu Ala Phe Thr Ile Ala His
    130                 135                 140

gag tca ggg cac aac ttt ggt atg att cac gac gga gaa ggg aat ccc     480
Glu Ser Gly His Asn Phe Gly Met Ile His Asp Gly Glu Gly Asn Pro
145                 150                 155                 160

tgc aga aag gct gaa ggc aat atc atg tct ccc aca ctg acc gga aac     528
Cys Arg Lys Ala Glu Gly Asn Ile Met Ser Pro Thr Leu Thr Gly Asn
                165                 170                 175

aat gga gtg ttt tca tgg tct tcc tgc agc cgc cag tat ctc aag aaa     576
Asn Gly Val Phe Ser Trp Ser Ser Cys Ser Arg Gln Tyr Leu Lys Lys
            180                 185                 190

ttc ctc agc aca cct cag gcg ggg tgt cta gtg gat gag ccc aag caa     624
Phe Leu Ser Thr Pro Gln Ala Gly Cys Leu Val Asp Glu Pro Lys Gln
        195                 200                 205

gca gga cag tat aaa tat ccg gac aaa cta cca gga cag att tat gat     672
Ala Gly Gln Tyr Lys Tyr Pro Asp Lys Leu Pro Gly Gln Ile Tyr Asp
    210                 215                 220

gct gac aca cag tgt aaa tgg caa ttt gga gca aaa gcc aag tta tgc     720
Ala Asp Thr Gln Cys Lys Trp Gln Phe Gly Ala Lys Ala Lys Leu Cys
225                 230                 235                 240

agc ctt ggt ttt gtg aag gat att tgc aaa tca ctt tgg tgc cac cga     768
Ser Leu Gly Phe Val Lys Asp Ile Cys Lys Ser Leu Trp Cys His Arg
                245                 250                 255

gta ggc cac agg tgt gag acc aag ttt atg ccc gca gca gaa ggg acc     816
Val Gly His Arg Cys Glu Thr Lys Phe Met Pro Ala Ala Glu Gly Thr
            260                 265                 270

gtt tgt ggc ttg agt atg tgg tgt cgg caa ggc cag tgc gta aag ttt     864
Val Cys Gly Leu Ser Met Trp Cys Arg Gln Gly Gln Cys Val Lys Phe
        275                 280                 285

ggg gag ctc ggg ccc cgg ccc atc cac ggc cag tgg tcc gcc tgg tcg     912
Gly Glu Leu Gly Pro Arg Pro Ile His Gly Gln Trp Ser Ala Trp Ser

290                 295                 300

aag tgg tca gaa tgt tcc cgg aca tgt ggt gga gga gtc aag ttc cag     960
Lys Trp Ser Glu Cys Ser Arg Thr Cys Gly Gly Gly Val Lys Phe Gln
305                 310                 315                 320

gag aga cac tgc aat aac ccc aag cct cag tat ggt ggc tta ttc tgt     1008
Glu Arg His Cys Asn Asn Pro Lys Pro Gln Tyr Gly Gly Leu Phe Cys
                325                 330                 335

cca ggt tct agc cgt att tat cag ctg tgc aat att aac cct tgc ctc     1056
Pro Gly Ser Ser Arg Ile Tyr Gln Leu Cys Asn Ile Asn Pro Cys Leu
            340                 345                 350

gag                                                                 1059
Glu

<210>128
<211>353
<212>PRT
<213>Homo sapiens

<400>128

Lys Leu Val Glu Thr Leu Val Val Ala Asp Lys Gln Met Val Glu Lys
1               5                   10                  15

His Gly Lys Gly Asn Val Thr Thr Tyr Ile Leu Thr Val Met Asn Met
            20                  25                  30

Val Ser Gly Leu Phe Lys Asp Gly Thr Ile Gly Ser Asp Ile Asn Val
        35                  40                  45

Val Val Val Ser Leu Ile Leu Leu Glu Gln Glu Pro Gly Gly Leu Leu
    50                  55                  60

Ile Asn His His Ala Asp Gln Ser Leu Asn Ser Phe Cys Gln Trp Gln
65                  70                  75                  80

Ser Ala Leu Ile Gly Lys Asn Gly Lys Arg His Asp His Ala Ile Leu
                85                  90                  95

Leu Thr Gly Phe Asp Ile Cys Ser Trp Lys Asn Glu Pro Cys Asp Thr
            100                 105                 110

Leu Gly Phe Ala Pro Ile Ser Gly Met Cys Ser Lys Tyr Arg Ser Cys
        115                 120                 125

Thr Ile Asn Glu Asp Thr Gly Leu Gly Leu Ala Phe Thr Ile Ala His
    130                 135                 140

Glu Ser Gly His Asn Phe Gly Met Ile His Asp Gly Glu Gly Asn Pro
145                 150                 155                 160

Cys Arg Lys Ala Glu Gly Asn Ile Met Ser Pro Thr Leu Thr Gly Asn
                165                 170                 175

Asn Gly Val Phe Ser Trp Ser Ser Cys Ser Arg Gln Tyr Leu Lys Lys
            180                 185                 190

Phe Leu Ser Thr Pro Gln Ala Gly Cys Leu Val Asp Glu Pro Lys Gln
        195                 200                 205

Ala Gly Gln Tyr Lys Tyr Pro Asp Lys Leu Pro Gly Gln Ile Tyr Asp
    210                 215                 220

Ala Asp Thr Gln Cys Lys Trp Gln Phe Gly Ala Lys Ala Lys Leu Cys
225                 230                 235                 240

Ser Leu Gly Phe Val Lys Asp Ile Cys Lys Ser Leu Trp Cys His Arg
                245                 250                 255

Val Gly His Arg Cys Glu Thr Lys Phe Met Pro Ala Ala Glu Gly Thr
            260                 265                 270

Val Cys Gly Leu Ser Met Trp Cys Arg Gln Gly Gln Cys Val Lys Phe
        275                 280                 285

Gly Glu Leu Gly Pro Arg Pro Ile His Gly Gln Trp Ser Ala Trp Ser
    290                 295                 300

Lys Trp Ser Glu Cys Ser Arg Thr Cys Gly Gly Gly Val Lys Phe Gln
305                 310                 315                 320

Glu Arg His Cys Asn Asn Pro Lys Pro Gln Tyr Gly Gly Leu Phe Cys
                325                 330                 335

Pro Gly Ser Ser Arg Ile Tyr Gln Leu Cys Asn Ile Asn Pro Cys Leu
            340                 345                 350

Glu

<210>129
<211>1059
<212>DNA
<213>Homo sapiens

<220>
<221> CDS
<222>(1)..(1059)

<400>129

aag ctt gtg gaa acc ctc gtg gtg gca gac aag aaa atg gtg gaa aag 48
Lys Leu Val Glu Thr Leu Val Val Ala Asp Lys Lys Met Val Glu Lys
1               5                   10                  15

cat ggc aag gga aat gtc acc aca tac att ctc aca gta atg aac atg 96
His Gly Lys Gly Asn Val Thr Thr Tyr Ile Leu Thr Val Met Asn Met
            20                  25                  30

gtt tct ggc cta ttt aaa gat ggg act att gga agt gac ata aac gtg 144

Val Ser Gly Leu Phe Lys Asp Gly Thr Ile Gly Ser Asp Ile Asn Val
        35                  40                  45

gtt gtg gtg agc cta att ctt ctg gaa caa gaa cct gga gga tta ttg     192
Val Val Val Ser Leu Ile Leu Leu Glu Gln Glu Pro Gly Gly Leu Leu
    50                  55                  60

atc aac cat cat gca gac cag tct ctg aat agt ttt tgt caa tgg cag     240
Ile Asn His His Ala Asp Gln Ser Leu Asn Ser Phe Cys Gln Trp Gln
65                  70                  75                  80

tct gcc ctc att gga aag aat ggc aag aga cat gat cat gcc atc tta     288
Ser Ala Leu Ile Gly Lys Asn Gly Lys Arg His Asp His Ala Ile Leu
                85                  90                  95

cta aca gga ttt gat att tgt tct tgg aag aat gaa cca tgt gac act     336
Leu Thr Gly Phe Asp Ile Cys Ser Trp Lys Asn Glu Pro Cys Asp Thr
            100                 105                 110

cta ggg ttt gcc ccc atc agt gga atg tgc tct aag tac cga agt tgt     384
Leu Gly Phe Ala Pro Ile Ser Gly Met Cys Ser Lys Tyr Arg Ser Cys
        115                 120                 125

acc atc aat gag gac aca gga ctt ggc ctt gcc ttc acc atc gct cat     432
Thr Ile Asn Glu Asp Thr Gly Leu Gly Leu Ala Phe Thr Ile Ala His
    130                 135                 140

gag tca ggg cac aac ttt ggt atg att cac gac gga gaa ggg aat ccc     480
Glu Ser Gly His Asn Phe Gly Met Ile His Asp Gly Glu Gly Asn Pro
145                 150                 155                 160

tgc aga aag gct gaa ggc aat atc atg tct ccc aca ctg acc gga aac     528
Cys Arg Lys Ala Glu Gly Asn Ile Met Ser Pro Thr Leu Thr Gly Asn
                165                 170                 175

aat gga gtg ttt tca tgg tct tcc tgc agc cgc cag tat ctc aag aaa     576
Asn Gly Val Phe Ser Trp Ser Ser Cys Ser Arg Gln Tyr Leu Lys Lys
            180                 185                 190

ttc ctc agc aca cct cag gcg ggg tgt cta gtg gat gag ccc aag caa     624
Phe Leu Ser Thr Pro Gln Ala Gly Cys Leu Val Asp Glu Pro Lys Gln
        195                 200                 205

gca gga cag tat aaa tat ccg gac aaa cta cca gga cag att tat gat     672
Ala Gly Gln Tyr Lys Tyr Pro Asp Lys Leu Pro Gly Gln Ile Tyr Asp
    210                 215                 220

gct gac aca cag tgt aaa tgg caa ttt gga gca aaa gcc aag tta tgc     720
Ala Asp Thr Gln Cys Lys Trp Gln Phe Gly Ala Lys Ala Lys Leu Cys
225                 230                 235                 240

agc ctt ggt ttt gtg aag gat att tgc aaa tca ctt tgg tgc cac cgg     768
Ser Leu Gly Phe Val Lys Asp Ile Cys Lys Ser Leu Trp Cys His Arg
                245                 250                 255

gta ggc cac agg tgt gag acc aag ttt atg ccc gca gca gaa ggg acc     816
Val Gly His Arg Cys Glu Thr Lys Phe Met Pro Ala Ala Glu Gly Thr

260                    265                    270

gtt tgt ggc ttg agt atg tgg tgt cgg caa ggc cag tgc gta aag ttt     864
Val Cys Gly Leu Ser Met Trp Cys Arg Gln Gly Gln Cys Val Lys Phe
        275                    280                    285

ggg gag ctc ggg ccc cgg ccc atc cac ggc cag tgg tcc gcc tgg tcg     912
Gly Glu Leu Gly Pro Arg Pro Ile His Gly Gln Trp Ser Ala Trp Ser
    290                    295                    300

aag tgg tca gaa tgt tcc cgg aca tgt ggt gga gga gtc aag ttc cag     960
Lys Trp Ser Glu Cys Ser Arg Thr Cys Gly Gly Gly Val Lys Phe Gln
305                    310                    315                    320

gag aga cac tgc aat aac ccc aag cct cag tat ggt ggc ata ttc tgt     1008
Glu Arg His Cys Asn Asn Pro Lys Pro Gln Tyr Gly Gly Ile Phe Cys
                325                    330                    335

cca ggt tct agc cgt att tat cag ctg tgc aat att aac cct tgc ctc     1056
Pro Gly Ser Ser Arg Ile Tyr Gln Leu Cys Asn Ile Asn Pro Cys Leu
            340                    345                    350

gag                                                                 1059
Glu

<210>130
<211>353
<212>PRT
<213>Homo sapiens

<400>130

Lys Leu Val Glu Thr Leu Val Val Ala Asp Lys Lys Met Val Glu Lys
 1               5                  10                  15

His Gly Lys Gly Asn Val Thr Thr Tyr Ile Leu Thr Val Met Asn Met
            20                  25                  30

Val Ser Gly Leu Phe Lys Asp Gly Thr Ile Gly Ser Asp Ile Asn Val
        35                  40                  45

Val Val Val Ser Leu Ile Leu Leu Glu Gln Glu Pro Gly Gly Leu Leu
    50                  55                  60

Ile Asn His His Ala Asp Gln Ser Leu Asn Ser Phe Cys Gln Trp Gln
65                  70                  75                  80

Ser Ala Leu Ile Gly Lys Asn Gly Lys Arg His Asp His Ala Ile Leu
                85                  90                  95

Leu Thr Gly Phe Asp Ile Cys Ser Trp Lys Asn Glu Pro Cys Asp Thr
            100                 105                 110

Leu Gly Phe Ala Pro Ile Ser Gly Met Cys Ser Lys Tyr Arg Ser Cys
        115                 120                 125

Thr Ile Asn Glu Asp Thr Gly Leu Gly Leu Ala Phe Thr Ile Ala His
    130                 135                 140

Glu Ser Gly His Asn Phé Gly Met Ile His Asp Gly Glu Gly Asn Pro
145                 150                 155                 160

Cys Arg Lys Ala Glu Gly Asn Ile Met Ser Pro Thr Leu Thr Gly Asn
                165                 170                 175

Asn Gly Val Phe Ser Trp Ser Ser Cys Ser Arg Gln Tyr Leu Lys Lys
            180                 185                 190

Phe Leu Ser Thr Pro Gln Ala Gly Cys Leu Val Asp Glu Pro Lys Gln
        195                 200                 205

Ala Gly Gln Tyr Lys Tyr Pro Asp Lys Leu Pro Gly Gln Ile Tyr Asp
    210                 215                 220

Ala Asp Thr Gln Cys Lys Trp Gln Phe Gly Ala Lys Ala Lys Leu Cys
225                 230                 235                 240

Ser Leu Gly Phe Val Lys Asp Ile Cys Lys Ser Leu Trp Cys His Arg
                245                 250                 255

Val Gly His Arg Cys Glu Thr Lys Phe Met Pro Ala Ala Glu Gly Thr
            260                 265                 270

Val Cys Gly Leu Ser Met Trp Cys Arg Gln Gly Gln Cys Val Lys Phe
        275                 280                 285

Gly Glu Leu Gly Pro Arg Pro Ile His Gly Gln Trp Ser Ala Trp Ser
    290                 295                 300

Lys Trp Ser Glu Cys Ser Arg Thr Cys Gly Gly Gly Val Lys Phe Gln
305                 310                 315                 320

Glu Arg His Cys Asn Asn Pro Lys Pro Gln Tyr Gly Gly Ile Phe Cys
                325                 330                 335

Pro Gly Ser Ser Arg Ile Tyr Gln Leu Cys Asn Ile Asn Pro Cys Leu
            340                 345                 350

Glu

<210>131
<211>1059
<212>DNA
<213>Homo sapiens

<220>
<221> CDS
<222>(1)..(1059)

<400>131

aag ctt gtg gaa acc ctc gtg gtg gca gac aag aaa atg gtg gaa aag    48

Lys Leu Val Glu Thr Leu Val Val Ala Asp Lys Lys Met Val Glu Lys
1               5                   10                  15

cat ggc aag gga aat gtc acc aca tac att ctc aca gta atg aac atg     96
His Gly Lys Gly Asn Val Thr Thr Tyr Ile Leu Thr Val Met Asn Met
            20                  25                  30

gtt tct ggc cta ttt aaa gat ggg act att gga agt gac ata aac gtg     144
Val Ser Gly Leu Phe Lys Asp Gly Thr Ile Gly Ser Asp Ile Asn Val
        35                  40                  45

gtt gtg gtg agc cta att ctt ctg gaa caa gaa cct gga gga tta ttg     192
Val Val Val Ser Leu Ile Leu Leu Glu Gln Glu Pro Gly Gly Leu Leu
    50                  55                  60

atc aac cat cat gca gac cag tct ctg aat agt ttt tgt caa tgg cag     240
Ile Asn His His Ala Asp Gln Ser Leu Asn Ser Phe Cys Gln Trp Gln
65                  70                  75                  80

tct gcc ctc att gga aag aat ggc aag aga cat gat cat gcc atc tta     288
Ser Ala Leu Ile Gly Lys Asn Gly Lys Arg His Asp His Ala Ile Leu
                85                  90                  95

cta aca gga ttt gat att tgt tct tgg aag aat gaa cca tgt ggc act     336
Leu Thr Gly Phe Asp Ile Cys Ser Trp Lys Asn Glu Pro Cys Gly Thr
            100                 105                 110

cta ggg ttt gcc ccc atc agt gga atg tgc tct aag tac cga agt tgt     384
Leu Gly Phe Ala Pro Ile Ser Gly Met Cys Ser Lys Tyr Arg Ser Cys
        115                 120                 125

acc atc aat gag gac aca gga ctt ggc ctt gcc ttc acc atc gct cat     432
Thr Ile Asn Glu Asp Thr Gly Leu Gly Leu Ala Phe Thr Ile Ala His
    130                 135                 140

gag tca ggg cac aac ttt ggt atg att cac gac gga gaa ggg aat ccc     480
Glu Ser Gly His Asn Phe Gly Met Ile His Asp Gly Glu Gly Asn Pro
145                 150                 155                 160

tgc aga aag gct gaa ggc aat atc atg tct ccc aca ctg acc gga aac     528
Cys Arg Lys Ala Glu Gly Asn Ile Met Ser Pro Thr Leu Thr Gly Asn
                165                 170                 175

aat gga gtg ttt tca tgg tct tcc tgc agc cgc cag tat ctc aag aaa     576
Asn Gly Val Phe Ser Trp Ser Ser Cys Ser Arg Gln Tyr Leu Lys Lys
            180                 185                 190

ttc ctc agc aca cct cag gcg ggg tgt cta gtg gat gag ccc aag caa     624
Phe Leu Ser Thr Pro Gln Ala Gly Cys Leu Val Asp Glu Pro Lys Gln
        195                 200                 205

gca gga cag tat aaa tat ccg gac aaa cta cca gga cag att tat gat     672
Ala Gly Gln Tyr Lys Tyr Pro Asp Lys Leu Pro Gly Gln Ile Tyr Asp
    210                 215                 220

gct gac aca cag tgt aaa tgg caa ttt gga gca aaa gcc aag tta tgc     720
Ala Asp Thr Gln Cys Lys Trp Gln Phe Gly Ala Lys Ala Lys Leu Cys

225                 230                 235                 240

agc ctt ggt ttt gtg gag gat att tgc aaa tca ctt tgg tgc cac cga     768
Ser Leu Gly Phe Val Glu Asp Ile Cys Lys Ser Leu Trp Cys His Arg
                245                 250                 255

gta ggc cac agg tgt gag acc aag ttt atg ccc gca gca gaa ggg acc     816
Val Gly His Arg Cys Glu Thr Lys Phe Met Pro Ala Ala Glu Gly Thr
            260                 265                 270

gtt tgt ggc ttg agt atg tgg tgt cgg caa ggc cag tgc gta aag ttt     864
Val Cys Gly Leu Ser Met Trp Cys Arg Gln Gly Gln Cys Val Lys Phe
        275                 280                 285

ggg gag ctc ggg ccc cgg ccc atc cac ggc cag tgg tcc gcc tgg tcg     912
Gly Glu Leu Gly Pro Arg Pro Ile His Gly Gln Trp Ser Ala Trp Ser
    290                 295                 300

aat tgg tca gaa tgt tcc cgg aca tgt ggt gga gga gtc aag ttc cag     960
Asn Trp Ser Glu Cys Ser Arg Thr Cys Gly Gly Gly Val Lys Phe Gln
305                 310                 315                 320

gag aga cac tgc aat aac ccc aag cct cag tat ggt ggc ata ttc tgt     1008
Glu Arg His Cys Asn Asn Pro Lys Pro Gln Tyr Gly Gly Ile Phe Cys
                325                 330                 335

cca ggt tct agc cgt att tat cag ctg tgc aat att aac cct tgc ctc     1056
Pro Gly Ser Ser Arg Ile Tyr Gln Leu Cys Asn Ile Asn Pro Cys Leu
            340                 345                 350

gag                                                                 1059
Glu

<210>132
<211>353
<212>PRT
<213>Homo sapiens

<400>132

Lys Leu Val Glu Thr Leu Val Val Ala Asp Lys Lys Met Val Glu Lys
 1               5                  10                  15

His Gly Lys Gly Asn Val Thr Thr Tyr Ile Leu Thr Val Met Asn Met
            20                  25                  30

Val Ser Gly Leu Phe Lys Asp Gly Thr Ile Gly Ser Asp Ile Asn Val
        35                  40                  45

Val Val Val Ser Leu Ile Leu Leu Glu Gln Glu Pro Gly Gly Leu Leu
    50                  55                  60

Ile Asn His His Ala Asp Gln Ser Leu Asn Ser Phe Cys Gln Trp Gln
65                  70                  75                  80

Ser Ala Leu Ile Gly Lys Asn Gly Lys Arg His Asp His Ala Ile Leu

85                  90                  95

Leu Thr Gly Phe Asp Ile Cys Ser Trp Lys Asn Glu Pro Cys Gly Thr
            100                 105                 110

Leu Gly Phe Ala Pro Ile Ser Gly Met Cys Ser Lys Tyr Arg Ser Cys
        115                 120                 125

Thr Ile Asn Glu Asp Thr Gly Leu Gly Leu Ala Phe Thr Ile Ala His
    130                 135                 140

Glu Ser Gly His Asn Phe Gly Met Ile His Asp Gly Glu Gly Asn Pro
145                 150                 155                 160

Cys Arg Lys Ala Glu Gly Asn Ile Met Ser Pro Thr Leu Thr Gly Asn
                165                 170                 175

Asn Gly Val Phe Ser Trp Ser Ser Cys Ser Arg Gln Tyr Leu Lys Lys
            180                 185                 190

Phe Leu Ser Thr Pro Gln Ala Gly Cys Leu Val Asp Glu Pro Lys Gln
        195                 200                 205

Ala Gly Gln Tyr Lys Tyr Pro Asp Lys Leu Pro Gly Gln Ile Tyr Asp
    210                 215                 220

Ala Asp Thr Gln Cys Lys Trp Gln Phe Gly Ala Lys Ala Lys Leu Cys
225                 230                 235                 240

Ser Leu Gly Phe Val Glu Asp Ile Cys Lys Ser Leu Trp Cys His Arg
                245                 250                 255

Val Gly His Arg Cys Glu Thr Lys Phe Met Pro Ala Ala Glu Gly Thr
            260                 265                 270

Val Cys Gly Leu Ser Met Trp Cys Arg Gln Gly Gln Cys Val Lys Phe
        275                 280                 285

Gly Glu Leu Gly Pro Arg Pro Ile His Gly Gln Trp Ser Ala Trp Ser
    290                 295                 300

Asn Trp Ser Glu Cys Ser Arg Thr Cys Gly Gly Gly Val Lys Phe Gln
305                 310                 315                 320

Glu Arg His Cys Asn Asn Pro Lys Pro Gln Tyr Gly Gly Ile Phe Cys
                325                 330                 335

Pro Gly Ser Ser Arg Ile Tyr Gln Leu Cys Asn Ile Asn Pro Cys Leu
            340                 345                 350

Glu

<210>133
<211>1059
<212>DNA
<213>Homo sapiens

<220>
<221> CDS
<222>(1)..(1059)

<400>133

aag ctt gtg gaa acc ctc gtg gtg gca gac aag aaa atg gtg gaa aag 48
Lys Leu Val Glu Thr Leu Val Val Ala Asp Lys Lys Met Val Glu Lys
1               5                   10                  15

cat ggc aag gga aat gtc acc aca tac att ctc aca gta atg aac atg 96
His Gly Lys Gly Asn Val Thr Thr Tyr Ile Leu Thr Val Met Asn Met
            20                  25                  30

gtt tct ggc cta ttt aaa gat ggg act att gga agt gac ata aac gtg 144
Val Ser Gly Leu Phe Lys Asp Gly Thr Ile Gly Ser Asp Ile Asn Val
        35                  40                  45

gtt gtg gtg agc cta atg ctt ctg gaa caa gaa cct gga gga tta ttg 192
Val Val Val Ser Leu Met Leu Leu Glu Gln Glu Pro Gly Gly Leu Leu
    50                  55                  60

atc aac cat cat gca gac cag tct ctg aat agt ttt tgt caa tgg cag 240
Ile Asn His His Ala Asp Gln Ser Leu Asn Ser Phe Cys Gln Trp Gln
65                  70                  75                  80

tct gcc ctc att gga aag aat ggc aag aga cat gat cat gcc atc tta 288
Ser Ala Leu Ile Gly Lys Asn Gly Lys Arg His Asp His Ala Ile Leu
                85                  90                  95

cta aca gga ttt gat att tgt tct tgg aag aat gaa cca tgt gac act 336
Leu Thr Gly Phe Asp Ile Cys Ser Trp Lys Asn Glu Pro Cys Asp Thr
            100                 105                 110

cta ggg ttt gcc ccc atc agt gga atg tgc tct aag tac cga agt tgt 384
Leu Gly Phe Ala Pro Ile Ser Gly Met Cys Ser Lys Tyr Arg Ser Cys
        115                 120                 125

acc atc aat gag gac aca gga ctt ggc ctt gcc ttc acc atc gct cat 432
Thr Ile Asn Glu Asp Thr Gly Leu Gly Leu Ala Phe Thr Ile Ala His
    130                 135                 140

gag tca ggg cac aac ttt ggt atg att cac gac gga gaa ggg aat ccc 480
Glu Ser Gly His Asn Phe Gly Met Ile His Asp Gly Glu Gly Asn Pro
145                 150                 155                 160

tgc aga aag gct gaa ggc aat atc atg tct ccc aca ctg acc gga aac 528
Cys Arg Lys Ala Glu Gly Asn Ile Met Ser Pro Thr Leu Thr Gly Asn
                165                 170                 175

aat gga gtg ttt tca tgg tct tcc tgc agc cgc cag tat ctc aag aaa 576
Asn Gly Val Phe Ser Trp Ser Ser Cys Ser Arg Gln Tyr Leu Lys Lys
            180                 185                 190

ttc ctc agc aca cct cag gcg ggg tgt cta gtg gat gag ccc aag caa 624
Phe Leu Ser Thr Pro Gln Ala Gly Cys Leu Val Asp Glu Pro Lys Gln

195                    200                    205

gca gga cag tat aaa tat ccg gac aaa cta cca gga cag att tat gat    672
Ala Gly Gln Tyr Lys Tyr Pro Asp Lys Leu Pro Gly Gln Ile Tyr Asp
    210                    215                    220

gct gac aca cag tgt aaa tgg caa ttt gga gca aaa gcc aag tta tgc    720
Ala Asp Thr Gln Cys Lys Trp Gln Phe Gly Ala Lys Ala Lys Leu Cys
225                    230                    235                    240

agc ctt ggt ttt gtg aag gat att tgc aaa tca ctt tgg tgc cac cga    768
Ser Leu Gly Phe Val Lys Asp Ile Cys Lys Ser Leu Trp Cys His Arg
                245                    250                    255

gta ggc cac agg tgt gag acc aag ttt atg ccc gca gca gaa ggg acc    816
Val Gly His Arg Cys Glu Thr Lys Phe Met Pro Ala Ala Glu Gly Thr
            260                    265                    270

gtt tgt ggc ttg agt atg tgg tgt cgg caa ggc cag tgc gta aag ttt    864
Val Cys Gly Leu Ser Met Trp Cys Arg Gln Gly Gln Cys Val Lys Phe
        275                    280                    285

ggg gag ctc ggg ccc cgg ccc atc cac ggc cag tgg tcc gcc tgg tcg    912
Gly Glu Leu Gly Pro Arg Pro Ile His Gly Gln Trp Ser Ala Trp Ser
    290                    295                    300

aag tgg tca gaa tgt tcc cgg aca tgt ggt gga gga gtc aag ttc cag    960
Lys Trp Ser Glu Cys Ser Arg Thr Cys Gly Gly Gly Val Lys Phe Gln
305                    310                    315                    320

gag aga cac tgc aat aac ccc aag cct cag tat ggt ggc ata ttc tgt    1008
Glu Arg His Cys Asn Asn Pro Lys Pro Gln Tyr Gly Gly Ile Phe Cys
                325                    330                    335

cca ggt tct agc cgt att tat cag ctg tgc aat att aac cct tgc ctc    1056
Pro Gly Ser Ser Arg Ile Tyr Gln Leu Cys Asn Ile Asn Pro Cys Leu
            340                    345                    350

gag                                                                1059
Glu

<210>134
<211>353
<212>PRT
<213>Homo sapiens

<400>134

Lys Leu Val Glu Thr Leu Val Val Ala Asp Lys Lys Met Val Glu Lys
  1               5                    10                    15

His Gly Lys Gly Asn Val Thr Thr Tyr Ile Leu Thr Val Met Asn Met
            20                    25                    30

Val Ser Gly Leu Phe Lys Asp Gly Thr Ile Gly Ser Asp Ile Asn Val
        35                    40                    45

Val Val Val Ser Leu Met Leu Leu Glu Gln Glu Pro Gly Gly Leu Leu
    50                  55                  60

Ile Asn His His Ala Asp Gln Ser Leu Asn Ser Phe Cys Gln Trp Gln
65                  70                  75                  80

Ser Ala Leu Ile Gly Lys Asn Gly Lys Arg His Asp His Ala Ile Leu
                85                  90                  95

Leu Thr Gly Phe Asp Ile Cys Ser Trp Lys Asn Glu Pro Cys Asp Thr
            100                 105                 110

Leu Gly Phe Ala Pro Ile Ser Gly Met Cys Ser Lys Tyr Arg Ser Cys
        115                 120                 125

Thr Ile Asn Glu Asp Thr Gly Leu Gly Leu Ala Phe Thr Ile Ala His
    130                 135                 140

Glu Ser Gly His Asn Phe Gly Met Ile His Asp Gly Glu Gly Asn Pro
145                 150                 155                 160

Cys Arg Lys Ala Glu Gly Asn Ile Met Ser Pro Thr Leu Thr Gly Asn
                165                 170                 175

Asn Gly Val Phe Ser Trp Ser Ser Cys Ser Arg Gln Tyr Leu Lys Lys
            180                 185                 190

Phe Leu Ser Thr Pro Gln Ala Gly Cys Leu Val Asp Glu Pro Lys Gln
        195                 200                 205

Ala Gly Gln Tyr Lys Tyr Pro Asp Lys Leu Pro Gly Gln Ile Tyr Asp
    210                 215                 220

Ala Asp Thr Gln Cys Lys Trp Gln Phe Gly Ala Lys Ala Lys Leu Cys
225                 230                 235                 240

Ser Leu Gly Phe Val Lys Asp Ile Cys Lys Ser Leu Trp Cys His Arg
                245                 250                 255

Val Gly His Arg Cys Glu Thr Lys Phe Met Pro Ala Ala Glu Gly Thr
            260                 265                 270

Val Cys Gly Leu Ser Met Trp Cys Arg Gln Gly Gln Cys Val Lys Phe
        275                 280                 285

Gly Glu Leu Gly Pro Arg Pro Ile His Gly Gln Trp Ser Ala Trp Ser
    290                 295                 300

Lys Trp Ser Glu Cys Ser Arg Thr Cys Gly Gly Gly Val Lys Phe Gln
305                 310                 315                 320

Glu Arg His Cys Asn Asn Pro Lys Pro Gln Tyr Gly Gly Ile Phe Cys
                325                 330                 335

Pro Gly Ser Ser Arg Ile Tyr Gln Leu Cys Asn Ile Asn Pro Cys Leu
            340                 345                 350

Glu

<210>135
<211>3107
<212>DNA
<213>Homo sapiens

<220>
<221> CDS
<222>(25)..(3058)

<400>135
cgtggaggtt cttgtccagg gcac atg gag gac cgt gtg ttg cgt gac gac 51
ctc tgg aat ctg gcc aag gcc act gtc gtg tgc cgc cag cta cag tgt 99
gga cgg gct gtg gca gcc cca aca ggg gct cac ttc ggg gca ggc tct 147
ggg aaa atc ctg ctg gat gac gtg cag tgt gtg ggg agc gag agc cac 195
ctg ggg cag tgc gtg cat ggg ggc cgg gcc agg cac aac tgt ggg cac 243
ctg gag gat gcc agt gtc atc tgt tca agg cca ctc ccc agt gtc cct 291
act tct tgt gcc tcc cca gga gcc tgg atg gag gtg agg ctg ctg aac 339
ggc aca gga agg tgc tca ggc cgt gtg gaa gtt ctc gtc cag ggc acg 387
tgg ggg acc gtg tgt gat gat ctc tgg gac ctg gct gag gcc act gtc 435
gtg tgc cgc cag ctg cag tgt ggc cag gct gtg gca gcc ccc aca ggg 483
gcc cac ttt cgg gca ggc tct ggg aag atc tta ctg gat gac atg cag 531
tgt gtg ggc agt gag agc cat ctg ggt caa tgc atg cgt ggg gac cag 579
gcc agg cac aac tgt ggg cac ctg gag gat gcc agt gtc atc tgc aca 627
cac cat cag tta cca gct gca gga gcc tgc atg gag gtg agg ctg ctg 675
aat ggc aca ggg agg tgc tta ggc cgc gtg gag gtt ctc atc cag ggc 723
acg tgg ggg acg gtg tgt gac cac ttc tgg aac ctg gcc gag gcc gcc 771
gtt gtg tgc cgc cag ctg caa tgt ggc cag gcc atg gca gcc cac ttc 819
ggg gca agc tct ggg aaa gtc ctg ctg gat gac atg cag tgt gtg ggc 867
agc aag agc cac ctg ggg cgg tgc gtg cac agg ggc tgg gcc agg cac 915
aac tgt ggg cac ctg gag gat gcc agt gtc atc tgt gca gaa aaa tct 963
cgg tgt ggt ggc att att acc aac tca tct gga gcg att agg aat ccc 1011
cca cag aat gaa atg cat gac aac atc act tgt gtg tgg gaa atc aag 1059
gca aat gca tct gat cat ata ctg ctg gca ttt cca cat ctt ctt gac 1107
tgc acc aat gaa tat ttt gaa atc ctg gac ggt cca cca tcc tca gta 1155
aag tca ttg ggg agg acc tgc tct ggc ttc caa cac acc tac atc tcc 1203
tcc tcc agc tca atg acc ctc gtg tat ttc cga agc ttc aac aac cta 1251
gga aaa tgg cta ttg acg atg tat ttc ctt gta ctt gca gag gca gtg 1299
ttg cag acc cca cat cta atc agt cag tgc ccc cag agg aca aca gtc 1347
acc aga tct ctc tct gca ggg gac tgg ccg gag ctg cag ctg gtg ggt 1395
ggc tct ggc cgg tgc tca gga cgc gtg gag att ctc cac cag ggc gcc 1443
tgg ggc acc gtg tgt gat gac ctg tgg gac ctg aac gaa gct gag gtt 1491
gtg tgc cgg cag ctt ggg tgt ggt cga gcc atg tct gcc ctt gga aag 1539
gcc cac ttt ggc ccc ggc tca gga gac atc ttc ctg gac aac ctc cag 1587
tgc gct ggt gtg gag cgc tac ctg ggc cag tgc acc cac tcg ggc tgg 1635
tca gag cac aac cat gtt tca tat tca ggc ata att tcc act tca gaa 1683
gaa gta act cct tca cct ggt tgt ggc ggt tac ctg gat acc ttg gaa 1731
gga tcc ttc acc agc ccc aat tac cca aag ccg cat cct gag ctg gct 1779
tat tgt gtg tgg cac ata caa gtg gag aaa gat tac aag ata aaa cta 1827
aac ttc aaa gag att gaa ata gac aaa cag tgc aaa ttt gat ttt ctt 1875
gcc atc tat gat ggc ccc tcc acc aac tct ggc ctg att gga caa gtc 1923
tgt ggc cgt gtg act ccc acc ttc gaa tcg tca tca aac tct ctg act 1971
gtc gtg ttg tct aca gat tat gcc aat tct tac cgg gga ttt tct gct 2019
tcc tac acc tca att tat gca gaa aac atc aca gca tct tta act tgc 2067
tct tct gac agg atg aga gtt att ata agc aaa tcc tac cta gag gct 2115
ttt aac tct aat ggg aat aac ttg caa cta aaa gac cca act tgc aga 2163
cca aaa tta tca aat gtt gtg gaa ttt tct gtc cct ctt aat gga tgt 2211
ggt aca atc aga cag gta gaa gat cag tca att act tac acc aat ata 2259
atc acc ttt tct gca tcc tca act tct gaa gtg atc acc cgt cag aaa 2307
caa ctc cag att att gtg aag tgt gaa atg gga cat aat tct aca gtg 2355
gag ata ata tac ata aca gaa gat gat gta ata caa agt caa aat gca 2403
ctg ggc aaa tat aac acc agc atg gct ctt ttt gaa tcc aat tca ttt 2451
gaa aag act ata ctt gaa tca cca tat tat gtg gat ttg aac caa act 2499
ctt ttt gtt caa gtt agt ctg cac acc tca gat cca aat ttg gtg gtg 2547
ttt ctt gat acc tgt aga gcc tct ccc acc tct gac ttt gca tct cca 2595
acc tac gac cta atc aag agt ggg tgt agt cga gat gaa act tgt aag 2643
gtg tat ccc tta ttt gga cac tat ggg aga ttc cag ttt aat gcc ttt 2691
aaa ttc ttg aga agt atg agc tct gtg tat ctg cag tgt aaa gtt ttg 2739
ata tgt gat agc agt gac cac cag tct cgc tgc aat caa ggt tgt gtc 2787
tcc aga agc aaa cga gac att tct tca tat aaa tgg aaa aca gat tcc 2835
atc ata gga ccc att cgt ctg aaa agg gat cga agt gca agt ggc aat 2883
tca gga tct cag cat gaa aca cat gcg gaa gaa act cca aac cag cct 2931
ttc aac agt gtg cat ctg ttt tcc ttc atg gtt cta gct ctg aat gtg 2979
gtg act gta gcg aca atc aca gtg agg cat ttt gta aat caa cgg gca 3027
gac tac aaa tac cag aag ctg cag aac tat taactaacag gtccaaccct 3077
aagtgagaca tgtttctcca ggatgccaaa 3107

<210>136
<211>1011
<212>PRT
<213>Homo sapiens

<400>136

<210>137
<211>744
<212>DNA
<213>Homo sapiens

<220>
<221> CDS
<222>(1)..(744)

<400>137

ggt acc act tgc tct tct gac agg atg aga gtt att ata agc aaa tcc    48
Gly Thr Thr Cys Ser Ser Asp Arg Met Arg Val Ile Ile Ser Lys Ser
  1               5                  10                  15

tac cta gag gct ttt aac tct aat ggg aat aac ttg caa cta aaa gac    96
Tyr Leu Glu Ala Phe Asn Ser Asn Gly Asn Asn Leu Gln Leu Lys Asp
             20                  25                  30

cca act tgc aga cca aaa tta tca aat gtt gtg gaa ttt tct gtc cct    144
Pro Thr Cys Arg Pro Lys Leu Ser Asn Val Val Glu Phe Ser Val Pro
         35                  40                  45

ctt aat gga tgt ggt aca atc aga aag gta gaa gat cag tca att act    192
Leu Asn Gly Cys Gly Thr Ile Arg Lys Val Glu Asp Gln Ser Ile Thr
     50                  55                  60

tac acc aat ata atc gcc ttt tct gca tcc tca act tct gaa gtg atc    240
Tyr Thr Asn Ile Ile Ala Phe Ser Ala Ser Ser Thr Ser Glu Val Ile
 65                  70                  75                  80

acc cgt cag aaa caa ctc cag att att gtg aag tgt gaa atg gga cat    288
Thr Arg Gln Lys Gln Leu Gln Ile Ile Val Lys Cys Glu Met Gly His
                 85                  90                  95

aat tct aca gtg gag ata ata tac ata aca gaa gat gat gta ata caa    336
Asn Ser Thr Val Glu Ile Ile Tyr Ile Thr Glu Asp Asp Val Ile Gln
            100                 105                 110

agt caa aat gca ctg ggc aaa tat aac acc agc atg gct ctt ttt gaa    384
Ser Gln Asn Ala Leu Gly Lys Tyr Asn Thr Ser Met Ala Leu Phe Glu
        115                 120                 125

tcc aat tca ttt gaa aag act ata ctt gaa tca cca tat tat gtg gat    432
Ser Asn Ser Phe Glu Lys Thr Ile Leu Glu Ser Pro Tyr Tyr Val Asp
    130                 135                 140

ttg aac caa act ctt ttt gtt caa gtt agt ctg cac acc tca gat cca    480
Leu Asn Gln Thr Leu Phe Val Gln Val Ser Leu His Thr Ser Asp Pro
145                 150                 155                 160

aat ttg gtg gtg ttt ctt gat acc tgt aga gcc tct ccc acc tct gac    528
Asn Leu Val Val Phe Leu Asp Thr Cys Arg Ala Ser Pro Thr Ser Asp
                165                 170                 175

ttt gca tct cca acc tac gac cta atc aag agt gga tgt agt cga gat    576
Phe Ala Ser Pro Thr Tyr Asp Leu Ile Lys Ser Gly Cys Ser Arg Asp
            180                 185                 190

gaa act tgt aag gtg tat ccc tta ttt gga cac tat ggg aga ttc cag    624
Glu Thr Cys Lys Val Tyr Pro Leu Phe Gly His Tyr Gly Arg Phe Gln
        195                 200                 205

ttt aat gcc ttt aaa ttc ttg aga agt atg agc tct gtg tat ctg cag    672
Phe Asn Ala Phe Lys Phe Leu Arg Ser Met Ser Ser Val Tyr Leu Gln
    210                 215                 220

tgt aaa gtt ttg ata tgt gat agc agt gac cac cag tct cgc tgc aat    720
Cys Lys Val Leu Ile Cys Asp Ser Ser Asp His Gln Ser Arg Cys Asn
225                 230                 235                 240

caa ggt tgt gtc tcc aga ctc gag                                    744
Gln Gly Cys Val Ser Arg Leu Glu
                245

<210>138
<211>248
<212>PRT
<213>Homo sapiens

<400>138

Gly Thr Thr Cys Ser Ser Asp Arg Met Arg Val Ile Ile Ser Lys Ser
1               5                   10                  15

Tyr Leu Glu Ala Phe Asn Ser Asn Gly Asn Asn Leu Gln Leu Lys Asp
            20                  25                  30

Pro Thr Cys Arg Pro Lys Leu Ser Asn Val Val Glu Phe Ser Val Pro
        35                  40                  45

Leu Asn Gly Cys Gly Thr Ile Arg Lys Val Glu Asp Gln Ser Ile Thr
    50                  55                  60

Tyr Thr Asn Ile Ile Ala Phe Ser Ala Ser Ser Thr Ser Glu Val Ile
65                  70                  75                  80

Thr Arg Gln Lys Gln Leu Gln Ile Ile Val Lys Cys Glu Met Gly His
                85                  90                  95

Asn Ser Thr Val Glu Ile Ile Tyr Ile Thr Glu Asp Asp Val Ile Gln
            100                 105                 110

Ser Gln Asn Ala Leu Gly Lys Tyr Asn Thr Ser Met Ala Leu Phe Glu
        115                 120                 125

Ser Asn Ser Phe Glu Lys Thr Ile Leu Glu Ser Pro Tyr Tyr Val Asp
    130                 135                 140

Leu Asn Gln Thr Leu Phe Val Gln Val Ser Leu His Thr Ser Asp Pro
145                 150                 155                 160

Asn Leu Val Val Phe Leu Asp Thr Cys Arg Ala Ser Pro Thr Ser Asp
                165                 170                 175

Phe Ala Ser Pro Thr Tyr Asp Leu Ile Lys Ser Gly Cys Ser Arg Asp
            180                 185                 190

Glu Thr Cys Lys Val Tyr Pro Leu Phe Gly His Tyr Gly Arg Phe Gln
        195                 200                 205

Phe Asn Ala Phe Lys Phe Leu Arg Ser Met Ser Ser Val Tyr Leu Gln
    210                 215                 220

Cys Lys Val Leu Ile Cys Asp Ser Ser Asp His Gln Ser Arg Cys Asn
225                 230                 235                 240

Gln Gly Cys Val Ser Arg Leu Glu
                245

<210>139
<211>744
<212>DNA
<213>Homo sapiens

<220>
<221> CDS
<222>(1)..(744)

<400>139
   ggt acc act tgc tct tct gac agg atg aga gtt att ata agc aaa tcc      48
   Gly Thr Thr Cys Ser Ser Asp Arg Met Arg Val Ile Ile Ser Lys Ser
     1               5                  10                  15
   tac cta gag gct ttt aac tct aat ggg aat aac ttg caa cta aaa gac      96
   Tyr Leu Glu Ala Phe Asn Ser Asn Gly Asn Asn Leu Gln Leu Lys Asp
                20                  25                  30
   cca act tgc aga cca aaa tta tca aat gtt gtg gaa ttt tct gtc cct     144
   Pro Thr Cys Arg Pro Lys Leu Ser Asn Val Val Glu Phe Ser Val Pro
            35                  40                  45
   ctt aat gga tgt ggt aca atc aga aag gta gaa gat cag tca att act     192
   Leu Asn Gly Cys Gly Thr Ile Arg Lys Val Glu Asp Gln Ser Ile Thr
        50                  55                  60
   tac acc aat ata atc acc ttt tct gca tcc tca act tct gaa gtg atc     240
   Tyr Thr Asn Ile Ile Thr Phe Ser Ala Ser Ser Thr Ser Glu Val Ile
    65                  70                  75                  80
   acc cgt cag aaa caa ctc cag att att ctg aag tgt gaa atg gga cat     288
   Thr Arg Gln Lys Gln Leu Gln Ile Ile Leu Lys Cys Glu Met Gly His
                    85                  90                  95
   aat tct aca gtg gag ata ata tac ata aca gaa gat gat gta ata caa     336
   Asn Ser Thr Val Glu Ile Ile Tyr Ile Thr Glu Asp Asp Val Ile Gln
               100                 105                 110
   agt caa aat gca ctg ggc aaa tat aac acc agc atg gct ctt ttt gaa     384
   Ser Gln Asn Ala Leu Gly Lys Tyr Asn Thr Ser Met Ala Leu Phe Glu
           115                 120                 125
   tcc aat tca ttt gaa aag act ata ctt gaa aca cca tat tat gtg gat     432
   Ser Asn Ser Phe Glu Lys Thr Ile Leu Glu Thr Pro Tyr Tyr Val Asp
       130                 135                 140
   ttg aac caa act ctt ttt gtt caa gtt agt ctg cac acc tca gat cca 480
   Leu Asn Gln Thr Leu Phe Val Gln Val Ser Leu His Thr Ser Asp Pro
   145 150 155 160
   aat ttg gtg gtg ttt ctt gat acc tgt aga gcc tct ccc acc tct gac 528
   Asn Leu Val Val Phe Leu Asp Thr Cys Arg Ala Ser Pro Thr Ser Asp
   165 170 175
   ttt gca tct cca acc tac gac cta atc aag agt gga tgt agt cga gat     576
   Phe Ala Ser Pro Thr Tyr Asp Leu Ile Lys Ser Gly Cys Ser Arg Asp
               180                 185                 190
   gaa act tgt aag gtg tat ccc tta ttt gga cac tat ggg aga ttc cag     624
   Glu Thr Cys Lys Val Tyr Pro Leu Phe Gly His Tyr Gly Arg Phe Gln
           195                 200                 205
   ttt aat gcc ttt aaa ttc ttg aga agt atg agc tct gtg tat ctg cag     672
   Phe Asn Ala Phe Lys Phe Leu Arg Ser Met Ser Ser Val Tyr Leu Gln
       210                 215                 220
   tgt aaa gtt ttg ata tgt gat agc agt gac cac cag tct cgc tgc aat     720
   Cys Lys Val Leu Ile Cys Asp Ser Ser Asp His Gln Ser Arg Cys Asn
   225                 230                 235                 240
   caa ggt tgt gtc tcc aga ctc gag                                     744
   Gln Gly Cys Val Ser Arg Leu Glu
                   245

<210>140
<211>248
<212>PRT
<213>Homo sapiens

<400>140

Gly Thr Thr Cys Ser Ser Asp Arg Met Arg Val Ile Ile Ser Lys Ser
1               5                   10                  15

Tyr Leu Glu Ala Phe Asn Ser Asn Gly Asn Asn Leu Gln Leu Lys Asp
            20                  25                  30

Pro Thr Cys Arg Pro Lys Leu Ser Asn Val Val Glu Phe Ser Val Pro
        35                  40                  45

Leu Asn Gly Cys Gly Thr Ile Arg Lys Val Glu Asp Gln Ser Ile Thr
    50                  55                  60

Tyr Thr Asn Ile Ile Thr Phe Ser Ala Ser Ser Thr Ser Glu Val Ile
65                  70                  75                  80

Thr Arg Gln Lys Gln Leu Gln Ile Ile Leu Lys Cys Glu Met Gly His
                85                  90                  95

Asn Ser Thr Val Glu Ile Ile Tyr Ile Thr Glu Asp Asp Val Ile Gln
            100                 105                 110

Ser Gln Asn Ala Leu Gly Lys Tyr Asn Thr Ser Met Ala Leu Phe Glu
        115                 120                 125

Ser Asn Ser Phe Glu Lys Thr Ile Leu Glu Thr Pro Tyr Tyr Val Asp
    130                 135                 140

Leu Asn Gln Thr Leu Phe Val Gln Val Ser Leu His Thr Ser Asp Pro
145                 150                 155                 160

Asn Leu Val Val Phe Leu Asp Thr Cys Arg Ala Ser Pro Thr Ser Asp
                165                 170                 175

Phe Ala Ser Pro Thr Tyr Asp Leu Ile Lys Ser Gly Cys Ser Arg Asp
            180                 185                 190

Glu Thr Cys Lys Val Tyr Pro Leu Phe Gly His Tyr Gly Arg Phe Gln
        195                 200                 205

Phe Asn Ala Phe Lys Phe Leu Arg Ser Met Ser Ser Val Tyr Leu Gln
    210                 215                 220

Cys Lys Val Leu Ile Cys Asp Ser Ser Asp His Gln Ser Arg Cys Asn
225                 230                 235                 240

Gln Gly Cys Val Ser Arg Leu Glu
                245

<210>141
<211>744
<212>DNA
<213>Homo sapiens

<220>
<221> CDS

<222>(1)..(744)

<400>141

ggt acc act tgc tct tct gac agg atg aga gtt att ata agc aaa tcc 48
Gly Thr Thr Cys Ser Ser Asp Arg Met Arg Val Ile Ile Ser Lys Ser
1               5                   10                  15

tac cta gag gct ttt aac tct aat ggg aat aac ttg caa cta aaa gac 96
Tyr Leu Glu Ala Phe Asn Ser Asn Gly Asn Asn Leu Gln Leu Lys Asp
            20                  25                  30

cca act tgc aga cca aaa tta tca aat gtt gtg gaa ttt tct gtc cct 144
Pro Thr Cys Arg Pro Lys Leu Ser Asn Val Val Glu Phe Ser Val Pro
        35                  40                  45

ctt aat gga tgt ggt aca atc aga aag gta gaa gat cag tca att act 192
Leu Asn Gly Cys Gly Thr Ile Arg Lys Val Glu Asp Gln Ser Ile Thr
    50                  55                  60

tac acc aat ata atc acc ttt tct gca tcc tca act tct gaa gtg atc 240
Tyr Thr Asn Ile Ile Thr Phe Ser Ala Ser Ser Thr Ser Glu Val Ile
65                  70                  75                  80

acc cgt cag aaa caa ctc cag att att gtg aag tgt gaa atg gga cat 288
Thr Arg Gln Lys Gln Leu Gln Ile Ile Val Lys Cys Glu Met Gly His
                85                  90                  95

aat tct aca gtg gag ata ata tac ata aca gaa gat gat gta ata caa 336
Asn Ser Thr Val Glu Ile Ile Tyr Ile Thr Glu Asp Asp Val Ile Gln
            100                 105                 110

agt caa aat gca ctg ggc aaa tat aac acc agc atg gct ctt ttt gaa 384
Ser Gln Asn Ala Leu Gly Lys Tyr Asn Thr Ser Met Ala Leu Phe Glu
        115                 120                 125

tcc aat tca ttt gaa aag act ata ctt gaa tca cca tat tat gtg gat 432
Ser Asn Ser Phe Glu Lys Thr Ile Leu Glu Ser Pro Tyr Tyr Val Asp
    130                 135                 140

ttg aac caa act ctt ttt gtt caa gtt agt ctg cac acc tca gat cca 480
Leu Asn Gln Thr Leu Phe Val Gln Val Ser Leu His Thr Ser Asp Pro
145                 150                 155                 160

aat ttg gtg gtg ttt ctt gat acc tgt aga gcc tct ccc acc tct gac 528
Asn Leu Val Val Phe Leu Asp Thr Cys Arg Ala Ser Pro Thr Ser Asp
                165                 170                 175

ttt gca tct cca acc tac gac cta atc aag agt gga tgt agt cga gat 576
Phe Ala Ser Pro Thr Tyr Asp Leu Ile Lys Ser Gly Cys Ser Arg Asp
            180                 185                 190

gaa act tgt aag gtg tat ccc tta ttt gga cac tat ggg aga ttc cag 624
Glu Thr Cys Lys Val Tyr Pro Leu Phe Gly His Tyr Gly Arg Phe Gln
        195                 200                 205

ttt aat gcc ttt aaa ttc ttg aga agt atg agc tct gtg tat ctg cag 672

Phe Asn Ala Phe Lys Phe Leu Arg Ser Met Ser Ser Val Tyr Leu Gln
    210                 215                 220

tgt aaa gtt ttg ata tgt gat agc agt gac cac cag tct cgc tgc aat 720
Cys Lys Val Leu Ile Cys Asp Ser Ser Asp His Gln Ser Arg Cys Asn
225                 230                 235                 240

caa ggt tgt gtc tcc aga ctc gag 744
Gln Gly Cys Val Ser Arg Leu Glu
                245

<210>142
<211>248
<212>PRT
<213>Homo sapiens

<400>142

Gly Thr Thr Cys Ser Ser Asp Arg Met Arg Val Ile Ile Ser Lys Ser
1               5                   10                  15

Tyr Leu Glu Ala Phe Asn Ser Asn Gly Asn Asn Leu Gln Leu Lys Asp
            20                  25                  30

Pro Thr Cys Arg Pro Lys Leu Ser Asn Val Val Glu Phe Ser Val Pro
        35                  40                  45

Leu Asn Gly Cys Gly Thr Ile Arg Lys Val Glu Asp Gln Ser Ile Thr
    50                  55                  60

Tyr Thr Asn Ile Ile Thr Phe Ser Ala Ser Ser Thr Ser Glu Val Ile
65                  70                  75                  80

Thr Arg Gln Lys Gln Leu Gln Ile Ile Val Lys Cys Glu Met Gly His
                85                  90                  95

Asn Ser Thr Val Glu Ile Ile Tyr Ile Thr Glu Asp Asp Val Ile Gln
            100                 105                 110

Ser Gln Asn Ala Leu Gly Lys Tyr Asn Thr Ser Met Ala Leu Phe Glu
        115                 120                 125

Ser Asn Ser Phe Glu Lys Thr Ile Leu Glu Ser Pro Tyr Tyr Val Asp
    130                 135                 140

Leu Asn Gln Thr Leu Phe Val Gln Val Ser Leu His Thr Ser Asp Pro
145                 150                 155                 160

Asn Leu Val Val Phe Leu Asp Thr Cys Arg Ala Ser Pro Thr Ser Asp
                165                 170                 175

Phe Ala Ser Pro Thr Tyr Asp Leu Ile Lys Ser Gly Cys Ser Arg Asp
            180                 185                 190

Glu Thr Cys Lys Val Tyr Pro Leu Phe Gly His Tyr Gly Arg Phe Gln
        195                 200                 205

Phe Asn Ala Phe Lys Phe Leu Arg Ser Met Ser Ser Val Tyr Leu Gln
    210                 215                 220

Cys Lys Val Leu Ile Cys Asp Ser Ser Asp His Gln Ser Arg Cys Asn
225                 230                 235                 240

Gln Gly Cys Val Ser Arg Leu Glu
                245

<210>143
<211>744
<212>DNA
<213>Homo sapiens

<220>
<221> CDS
<222>(1)..(744)

<400>143

ggt acc act tgc tct tct gac agg atg aga gtt att ata agc aaa tcc    48
Gly Thr Thr Cys Ser Ser Asp Arg Met Arg Val Ile Ile Ser Lys Ser
  1               5                  10                  15

tac cta gag gct ttt aac tct aat ggg aat aac ttg caa cta aaa gac    96
Tyr Leu Glu Ala Phe Asn Ser Asn Gly Asn Asn Leu Gln Leu Lys Asp
             20                  25                  30

cca act tgc aga cca aaa tta tca aat gtt gtg gaa ttt tct gtc cct    144
Pro Thr Cys Arg Pro Lys Leu Ser Asn Val Val Glu Phe Ser Val Pro
         35                  40                  45

ctt aat gga tgt ggt aca atc aga aag gta gaa gat cag tca att act    192
Leu Asn Gly Cys Gly Thr Ile Arg Lys Val Glu Asp Gln Ser Ile Thr
     50                  55                  60

tac acc aat ata atc acc ctt tct gca tcc tca act tct gaa gtg atc    240
Tyr Thr Asn Ile Ile Thr Leu Ser Ala Ser Ser Thr Ser Glu Val Ile
 65                  70                  75                  80

acc cgt cag aaa caa ctc cag att att gtg aag tgt gaa atg gga cat    288
Thr Arg Gln Lys Gln Leu Gln Ile Ile Val Lys Cys Glu Met Gly His
                 85                  90                  95

aat tct aca gtg gag ata ata tac ata aca gaa gat gat gta ata caa    336
Asn Ser Thr Val Glu Ile Ile Tyr Ile Thr Glu Asp Asp Val Ile Gln
            100                 105                 110

agt caa aat gca ctg ggc aaa tat aac acc agc atg gct ctt ttt gaa    384
Ser Gln Asn Ala Leu Gly Lys Tyr Asn Thr Ser Met Ala Leu Phe Glu
        115                 120                 125

tcc aat tca ttt gaa aag act ata ctt gaa tca cca tat tat gtg gat    432
Ser Asn Ser Phe Glu Lys Thr Ile Leu Glu Ser Pro Tyr Tyr Val Asp
    130                 135                 140

ttg aac caa act ctt ttt gtt caa gtt agt ctg cac acc tca gat cca    480

Leu Asn Gln Thr Leu Phe Val Gln Val Ser Leu His Thr Ser Asp Pro
145                 150                 155                 160

aat ttg gtg gtg ttt ctt gat acc tgt aga gcc tct ccc acc tct gac     528
Asn Leu Val Val Phe Leu Asp Thr Cys Arg Ala Ser Pro Thr Ser Asp
                165                 170                 175

ttt gca tct cca acc tac gac cta atc aag agt gga tgt agt cga gat     576
Phe Ala Ser Pro Thr Tyr Asp Leu Ile Lys Ser Gly Cys Ser Arg Asp
            180                 185                 190

gaa act tgt aag gtg tat ccc tta ttt gga cac tat ggg aga ttc cag     624
Glu Thr Cys Lys Val Tyr Pro Leu Phe Gly His Tyr Gly Arg Phe Gln
        195                 200                 205

ttt aat gcc ttt aaa ttc ttg aga agt atg agc tct gtg tat ctg cag     672
Phe Asn Ala Phe Lys Phe Leu Arg Ser Met Ser Ser Val Tyr Leu Gln
    210                 215                 220

tgt aaa gtt ttg ata tgt gat agc agt gac cac cag tct cgc tgc aat     720
Cys Lys Val Leu Ile Cys Asp Ser Ser Asp His Gln Ser Arg Cys Asn
225                 230                 235                 240

caa ggt tgt gtc tcc aga ctc gag                                     744
Gln Gly Cys Val Ser Arg Leu Glu
                245

<210>144
<211>248
<212>PRT
<213>Homo sapiens

<400>144

Gly Thr Thr Cys Ser Ser Asp Arg Met Arg Val Ile Ile Ser Lys Ser
  1               5                  10                  15

Tyr Leu Glu Ala Phe Asn Ser Asn Gly Asn Asn Leu Gln Leu Lys Asp
             20                  25                  30

Pro Thr Cys Arg Pro Lys Leu Ser Asn Val Val Glu Phe Ser Val Pro
         35                  40                  45

Leu Asn Gly Cys Gly Thr Ile Arg Lys Val Glu Asp Gln Ser Ile Thr
     50                  55                  60

Tyr Thr Asn Ile Ile Thr Leu Ser Ala Ser Ser Thr Ser Glu Val Ile
 65                  70                  75                  80

Thr Arg Gln Lys Gln Leu Gln Ile Ile Val Lys Cys Glu Met Gly His
                 85                  90                  95

Asn Ser Thr Val Glu Ile Ile Tyr Ile Thr Glu Asp Asp Val Ile Gln
            100                 105                 110

Ser Gln Asn Ala Leu Gly Lys Tyr Asn Thr Ser Met Ala Leu Phe Glu
        115                 120                 125

Ser Asn Ser Phe Glu Lys Thr Ile Leu Glu Ser Pro Tyr Tyr Val Asp
    130                 135                 140

Leu Asn Gln Thr Leu Phe Val Gln Val Ser Leu His Thr Ser Asp Pro
145                 150                 155                 160

Asn Leu Val Val Phe Leu Asp Thr Cys Arg Ala Ser Pro Thr Ser Asp
                165                 170                 175

Phe Ala Ser Pro Thr Tyr Asp Leu Ile Lys Ser Gly Cys Ser Arg Asp
            180                 185                 190

Glu Thr Cys Lys Val Tyr Pro Leu Phe Gly His Tyr Gly Arg Phe Gln
        195                 200                 205

Phe Asn Ala Phe Lys Phe Leu Arg Ser Met Ser Ser Val Tyr Leu Gln
    210                 215                 220

Cys Lys Val Leu Ile Cys Asp Ser Ser Asp His Gln Ser Arg Cys Asn
225                 230                 235                 240

Gln Gly Cys Val Ser Arg Leu Glu
                245

<210>145
<211>4011
<212>DNA
<213>Homo sapiens

<220>
<221> CDS
<222>(1)..(4009)

<400>145

atg cct ctg tcc agc cac ctg ctg ccc gcc ttg gtc ctg ttc ctg ggc      48
Met Pro Leu Ser Ser His Leu Leu Pro Ala Leu Val Leu Phe Leu Gly
  1               5                  10                  15

cac ctg gct gtc aac ctc cca gca gca ggg tcc tca ggc tgg gcc tgg      96
His Leu Ala Val Asn Leu Pro Ala Ala Gly Ser Ser Gly Trp Ala Trp
             20                  25                  30

gtc ccc aac cac tgc agg agc cct ggc cag gcc gtg tgc aac ttc gtg     144
Val Pro Asn His Cys Arg Ser Pro Gly Gln Ala Val Cys Asn Phe Val
         35                  40                  45

tgt gac tgc agg gac tgc tca gat gag gcc cag tgt ggt tac cac ggg     192
Cys Asp Cys Arg Asp Cys Ser Asp Glu Ala Gln Cys Gly Tyr His Gly
     50                  55                  60

gcc tcg ccc acc ctg ggc gcc ccc ttc gcc tgt gac ttc gag cag gac     240
Ala Ser Pro Thr Leu Gly Ala Pro Phe Ala Cys Asp Phe Glu Gln Asp
 65                  70                  75                  80

ccc tgc ggc tgg cgg gac att agt acc tca ggc tac agc tgg ctc cga     288

Pro Cys Gly Trp Arg Asp Ile Ser Thr Ser Gly Tyr Ser Trp Leu Arg
                85                  90                  95

gac agg gca ggg gcc gca ctg gag ggt cct ggg cct cac tca gac cac 336
Asp Arg Ala Gly Ala Ala Leu Glu Gly Pro Gly Pro His Ser Asp His
            100                 105                 110

aca ctg ggc acc gac ttg ggc tgg tac atg gcc gtt gga acc cac cga 384
Thr Leu Gly Thr Asp Leu Gly Trp Tyr Met Ala Val Gly Thr His Arg
        115                 120                 125

ggg aaa gag gca tcc acc gca gcc ctg cgc tcg cca acc ctg cga gag 432
Gly Lys Glu Ala Ser Thr Ala Ala Leu Arg Ser Pro Thr Leu Arg Glu
    130                 135                 140

gca gcc tcc tct tgc aag ctg agg ctc tgg tac cac gcg gcc tct gga 480
Ala Ala Ser Ser Cys Lys Leu Arg Leu Trp Tyr His Ala Ala Ser Gly
145                 150                 155                 160

gat gtg gct gaa ctg cgg gtg gag ctg acc cat ggc gca gag acc ctg 528
Asp Val Ala Glu Leu Arg Val Glu Leu Thr His Gly Ala Glu Thr Leu
                165                 170                 175

acc ctg tgg cag agc aca ggg ccc tgg ggc cct ggc tgg cag gag ttg 576
Thr Leu Trp Gln Ser Thr Gly Pro Trp Gly Pro Gly Trp Gln Glu Leu
            180                 185                 190

gca gtg acc aca ggc cgc atc cgg ggt gac ttc cga gtg acc ttc tct 624
Ala Val Thr Thr Gly Arg Ile Arg Gly Asp Phe Arg Val Thr Phe Ser
        195                 200                 205

gcc acc cga aat gcc acc cac agg ggc gct gtg gct cta gat gac cta 672
Ala Thr Arg Asn Ala Thr His Arg Gly Ala Val Ala Leu Asp Asp Leu
    210                 215                 220

gag ttc tgg gac tgt ggt ctg ccc acc ccc cag gcc aac tgt ccc ccg 720
Glu Phe Trp Asp Cys Gly Leu Pro Thr Pro Gln Ala Asn Cys Pro Pro
225                 230                 235                 240

gga cac cac cac tgc cag aac aag gtc tgc gtg gag ccc cag cag ctg 768
Gly His His His Cys Gln Asn Lys Val Cys Val Glu Pro Gln Gln Leu
                245                 250                 255

tgc gac ggg gaa gac aac tgc ggg gac ctg tct gat gag aac cca ctc 816
Cys Asp Gly Glu Asp Asn Cys Gly Asp Leu Ser Asp Glu Asn Pro Leu
            260                 265                 270

acc tgt ggc cgc cac ata gcc acc gac ttt gag aca ggc ctg ggc cca 864
Thr Cys Gly Arg His Ile Ala Thr Asp Phe Glu Thr Gly Leu Gly Pro
        275                 280                 285

tgg aac cgc tcg gaa ggc tgg tcc cgg aac cac cgc gct ggt ggt cct 912
Trp Asn Arg Ser Glu Gly Trp Ser Arg Asn His Arg Ala Gly Gly Pro
    290                 295                 300

gag cgc ccc tcc tgg cca cgc cgt gac cac agc cgg aac agt gca cag 960
Glu Arg Pro Ser Trp Pro Arg Arg Asp His Ser Arg Asn Ser Ala Gln

305                 310                 315                 320

ggc tcc ttc ctg gtc tcc gtg gcc gag cct ggc acc cct gct ata ctc     1008
Gly Ser Phe Leu Val Ser Val Ala Glu Pro Gly Thr Pro Ala Ile Leu
                325                 330                 335

tcc agc ccc gaa ttc caa gcc tca ggc acc tcc aac tgc tcg gcc ccc     1056
Ser Ser Pro Glu Phe Gln Ala Ser Gly Thr Ser Asn Cys Ser Ala Pro
            340                 345                 350

agc cag ctc ttg gtt cca cag ctg gtc ttc tat cag tac ctg agt ggg     1104
Ser Gln Leu Leu Val Pro Gln Leu Val Phe Tyr Gln Tyr Leu Ser Gly
        355                 360                 365

tct gag gct ggc tgc ctc cag ctg ttc ctg cag act ctg ggg ccc ggc     1152
Ser Glu Ala Gly Cys Leu Gln Leu Phe Leu Gln Thr Leu Gly Pro Gly
    370                 375                 380

gcc ccc cgg gcc ccc gtc ctg ctg cgg agg cgc cga ggg gag ctg ggg     1200
Ala Pro Arg Ala Pro Val Leu Leu Arg Arg Arg Arg Gly Glu Leu Gly
385                 390                 395                 400

acc gcc tgg gtc cga gac cgt gtt gac atc cag agc gcc tac ccc ttc     1248
Thr Ala Trp Val Arg Asp Arg Val Asp Ile Gln Ser Ala Tyr Pro Phe
                405                 410                 415

cag atc ctc ctg gcc ggg cag aca ggc ccg ggg ggc gtc gtg ggt ctg     1296
Gln Ile Leu Leu Ala Gly Gln Thr Gly Pro Gly Gly Val Val Gly Leu
            420                 425                 430

gac gac ctc atc ctg tct gac cac tgc aga cca gtc tcg gag gtg tcc     1344
Asp Asp Leu Ile Leu Ser Asp His Cys Arg Pro Val Ser Glu Val Ser
        435                 440                 445

acc ctg cag ccg ctg cct cct ggg ccc cgg gcc cca gcc ccc cag ccc     1392
Thr Leu Gln Pro Leu Pro Pro Gly Pro Arg Ala Pro Ala Pro Gln Pro
    450                 455                 460

ctg ccg ccc agc tcg cgg ctc cag gat tcc tgc aag cag ggg cat ctt     1440
Leu Pro Pro Ser Ser Arg Leu Gln Asp Ser Cys Lys Gln Gly His Leu
465                 470                 475                 480

gcc tgc ggg gac ctg tgt gtg ccc ccg gaa caa ctg tgt gac ttc gag     1488
Ala Cys Gly Asp Leu Cys Val Pro Pro Glu Gln Leu Cys Asp Phe Glu
                485                 490                 495

gag cag tgc gca ggg ggc gag gac gag cag gcc tgt ggt aaa ggg gct     1536
Glu Gln Cys Ala Gly Gly Glu Asp Glu Gln Ala Cys Gly Lys Gly Ala
            500                 505                 510

caa cct cct gca gac ctc ctg ctg cgg agc caa ggg ggc acc aca gac     1584
Gln Pro Pro Ala Asp Leu Leu Leu Arg Ser Gln Gly Gly Thr Thr Asp
        515                 520                 525

ttt gag tcc ccc gag gct ggg ggc tgg gag gac gcc agc gtg ggg cgg     1632
Phe Glu Ser Pro Glu Ala Gly Gly Trp Glu Asp Ala Ser Val Gly Arg
    530                 535                 540

ctg cag tgg cgg cgt gtc tca gcc cag gag agc cag ggg tcc agt gca 1680
Leu Gln Trp Arg Arg Val Ser Ala Gln Glu Ser Gln Gly Ser Ser Ala
545                 550                 555                 560

gct gct gct ggc ttc ctg gca cta gtt gtg gtg gac aac ggc tcc cgg 1728
Ala Ala Ala Gly Phe Leu Ala Leu Val Val Val Asp Asn Gly Ser Arg
                565                 570                 575

gag ctg gca tgg cag gcc ctg agc agc agt gca ggc atc tgg aag gtg 1776
Glu Leu Ala Trp Gln Ala Leu Ser Ser Ser Ala Gly Ile Trp Lys Val
            580                 585                 590

gac aag gtc ctt cta ggg gcc cgc cgc cgg ccc ttc cgg ctg gag ttt 1824
Asp Lys Val Leu Leu Gly Ala Arg Arg Arg Pro Phe Arg Leu Glu Phe
        595                 600                 605

gtc ggt ttg gtg gac ttg gat ggc cct gac cag cag gga gct ggg gtg 1872
Val Gly Leu Val Asp Leu Asp Gly Pro Asp Gln Gln Gly Ala Gly Val
    610                 615                 620

gac aac gtg acc ctg agg gac tgt agc ccc aca gtg acc acc gag aga 1920
Asp Asn Val Thr Leu Arg Asp Cys Ser Pro Thr Val Thr Thr Glu Arg
625                 630                 635                 640

gac aga gag gtc tcc tgt aac ttt gag cgg gac aca tgc agc tgg tac 1968
Asp Arg Glu Val Ser Cys Asn Phe Glu Arg Asp Thr Cys Ser Trp Tyr
                645                 650                 655

cca ggc cac ctc tca gac aca cac tgg cgc tgg gtg gag agc cgc ggc 2016
Pro Gly His Leu Ser Asp Thr His Trp Arg Trp Val Glu Ser Arg Gly
            660                 665                 670

cct gac cac gac cac acc aca ggc caa ggc cac ttt gtg ctc ctg gac 2064
Pro Asp His Asp His Thr Thr Gly Gln Gly His Phe Val Leu Leu Asp
        675                 680                 685

ccc aca gac ccc ctg gcc tgg ggc cac agt gcc cac ctg ctc tcc agg 2112
Pro Thr Asp Pro Leu Ala Trp Gly His Ser Ala His Leu Leu Ser Arg
    690                 695                 700

ccc cag gtg cca gca gca ccc acg gag tgt ctc agc ttc tgg tac cac 2160
Pro Gln Val Pro Ala Ala Pro Thr Glu Cys Leu Ser Phe Trp Tyr His
705                 710                 715                 720

ctc cat ggg ccc cag att ggg act ctg cgc cta gcc atg aga cgg gaa 2208
Leu His Gly Pro Gln Ile Gly Thr Leu Arg Leu Ala Met Arg Arg Glu
                725                 730                 735

ggg gag gag aca cac ctg tgg tcg cgg tca ggc acc cag ggc aac cgc 2256
Gly Glu Glu Thr His Leu Trp Ser Arg Ser Gly Thr Gln Gly Asn Arg
            740                 745                 750

tgg cac gag gcc tgg gcc acc ctt tcc cac cag cct ggc tcc cat gcc 2304
Trp His Glu Ala Trp Ala Thr Leu Ser His Gln Pro Gly Ser His Ala
        755                 760                 765

cag tac cag ctg ctg ttc gag ggc ctc cgg gac gga tac cac ggc acc 2352
Gln Tyr Gln Leu Leu Phe Glu Gly Leu Arg Asp Gly Tyr His Gly Thr
    770                 775                 780

atg gcg ctg gac gat gtg gcc gtg cgg ccg ggc ccc tgc tgg gcc cct 2400
Met Ala Leu Asp Asp Val Ala Val Arg Pro Gly Pro Cys Trp Ala Pro
785                 790                 795                 800

aat tac tgc tcc ttt gag gac tca gac tgc ggc ttc tcc cct gga ggc 2448
Asn Tyr Cys Ser Phe Glu Asp Ser Asp Cys Gly Phe Ser Pro Gly Gly
                805                 810                 815

caa ggt ctc tgg agg cgg cag gcc aat gcc tcg ggc cat gct gcc tgg 2496
Gln Gly Leu Trp Arg Arg Gln Ala Asn Ala Ser Gly His Ala Ala Trp
            820                 825                 830

ggc ccc cca aca gac cat acc act gag aca gcc caa ggg cac tac atg 2544
Gly Pro Pro Thr Asp His Thr Thr Glu Thr Ala Gln Gly His Tyr Met
        835                 840                 845

gtg gtg gac aca agc cca gac gca cta ccc cgg ggc cag acg gcc tcc 2592
Val Val Asp Thr Ser Pro Asp Ala Leu Pro Arg Gly Gln Thr Ala Ser
    850                 855                 860

ctg acc tcc aag gag cac agg ccc ctg gcc cag cct gct tgt ctg acc 2640
Leu Thr Ser Lys Glu His Arg Pro Leu Ala Gln Pro Ala Cys Leu Thr
865                 870                 875                 880

ttc tgg tac cac ggg agc ctc cgc agc cca ggc acc ctg cgg gtc tac 2688
Phe Trp Tyr His Gly Ser Leu Arg Ser Pro Gly Thr Leu Arg Val Tyr
                885                 890                 895

ctg gag gag cgc ggg agg cac cag gtg ctc agc ctc agt gcc cac ggc 2736
Leu Glu Glu Arg Gly Arg His Gln Val Leu Ser Leu Ser Ala His Gly
            900                 905                 910

ggg ctt gcc tgg cgc ctg ggc agc atg gac gtg cag gcc gag cga gcc 2784
Gly Leu Ala Trp Arg Leu Gly Ser Met Asp Val Gln Ala Glu Arg Ala
        915                 920                 925

tgg agg gtg gtg ttt gag gca gtg gcc gca ggc gtg gca cac tcc tac 2832
Trp Arg Val Val Phe Glu Ala Val Ala Ala Gly Val Ala His Ser Tyr
    930                 935                 940

gtg gct ctg gat gat ctg ctc ctc cag gac ggg ccc tgc cct cag cca 2880
Val Ala Leu Asp Asp Leu Leu Leu Gln Asp Gly Pro Cys Pro Gln Pro
945                 950                 955                 960

ggt tcc tgt gat ttt gag tct ggc ctg tgt ggc tgg agc cac ctg gcc 2928
Gly Ser Cys Asp Phe Glu Ser Gly Leu Cys Gly Trp Ser His Leu Ala
                965                 970                 975

tgg ccc ggc ctg ggc gga tac agc tgg gac tgg ggc ggg gga gcc acc 2976
Trp Pro Gly Leu Gly Gly Tyr Ser Trp Asp Trp Gly Gly Gly Ala Thr
            980                 985                 990

ccc tct cgt tac ccc cag ccc cct gtg gac cac acc ctg ggc aca gag 3024

Pro Ser Arg Tyr Pro Gln Pro Pro Val Asp His Thr Leu Gly Thr Glu
        995                 1000                1005

gca ggc cac ttt gcc ttc ttt gaa act ggc gtg ctg ggc ccc ggg ggc 3072
Ala Gly His Phe Ala Phe Phe Glu Thr Gly Val Leu Gly Pro Gly Gly
    1010                1015                1020

cgg gcc gcc tgg ctg cgc agc gag cct ctg ccg gcc acc cca gcc tcc 3120
Arg Ala Ala Trp Leu Arg Ser Glu Pro Leu Pro Ala Thr Pro Ala Ser
1025                1030                1035                1040

tgc ctc cgc ttc tgg tac cac atg ggt ttt cct gag cac ttc tac aag 3168
Cys Leu Arg Phe Trp Tyr His Met Gly Phe Pro Glu His Phe Tyr Lys
                1045                1050                1055

ggg gag ctg aag gta ctg ctg cac agt gct cag ggc cag ctg gct gtg 3216
Gly Glu Leu Lys Val Leu Leu His Ser Ala Gln Gly Gln Leu Ala Val
            1060                1065                1070

tgg ggc gca ggc ggg cat cgg cgg cac cag tgg ctg gag gcc cag gtg 3264
Trp Gly Ala Gly Gly His Arg Arg His Gln Trp Leu Glu Ala Gln Val
        1075                1080                1085

gag gta gcc agt gcc aag gag ttc cag atc gtg ttt gaa gcc act ctg 3312
Glu Val Ala Ser Ala Lys Glu Phe Gln Ile Val Phe Glu Ala Thr Leu
    1090                1095                1100

ggc ggc cag cca gcc ctg ggg ccc att gcc ctg gat gac gtg gag tat 3360
Gly Gly Gln Pro Ala Leu Gly Pro Ile Ala Leu Asp Asp Val Glu Tyr
1105                1110                1115                1120

ctg gct ggg cag cat tgc cag cag cct gcc ccc agc ccg ggg aac aca 3408
Leu Ala Gly Gln His Cys Gln Gln Pro Ala Pro Ser Pro Gly Asn Thr
                1125                1130                1135

gcc gca ccc ggg tct gtg cca gct gtg gtt ggc agt gcc ctc cta ttg 3456
Ala Ala Pro Gly Ser Val Pro Ala Val Val Gly Ser Ala Leu Leu Leu
            1140                1145                1150

ctc atg ctc ctg gtg ctg ctg gga ctt ggg gga cgg cgc tgg ctg cag 3504
Leu Met Leu Leu Val Leu Leu Gly Leu Gly Gly Arg Arg Trp Leu Gln
        1155                1160                1165

aag aag ggg agc tgc ccc ttc cag agc aac aca gag gcc aca gcc cct 3552
Lys Lys Gly Ser Cys Pro Phe Gln Ser Asn Thr Glu Ala Thr Ala Pro
    1170                1175                1180

ggc ttt gac aac atc ctt ttc aat gcg gag cca tgc ggt gtt gga ggg 3600
Gly Phe Asp Asn Ile Leu Phe Asn Ala Glu Pro Cys Gly Val Gly Gly
1185                1190                1195                1200

cac agc aca gca ccc ttg cca gcc gct agg ctc cca tcc gcc ctg ggg 3648
His Ser Thr Ala Pro Leu Pro Ala Ala Arg Leu Pro Ser Ala Leu Gly
                1205                1210                1215

act aag tcc cag cgg agg gcg gca gtt ggg cac ggc tac cgc cgt ccc 3696
Thr Lys Ser Gln Arg Arg Ala Ala Val Gly His Gly Tyr Arg Arg Pro

1220                    1225                    1230

tcg tct tca ggt gcc gtg ggg ctg acc agt gcc cac caa ctg tcc acg    3744
Ser Ser Ser Gly Ala Val Gly Leu Thr Ser Ala His Gln Leu Ser Thr
        1235                    1240                    1245

cag atg ggc gaa gag gaa atg gcc ctg cag aga cct tca gag ctc ccc    3792
Gln Met Gly Glu Glu Glu Met Ala Leu Gln Arg Pro Ser Glu Leu Pro
    1250                    1255                    1260

ccg gca gcc cac tcc cgg gca tca gtc atg aaa att cac cag ctt tcc    3840
Pro Ala Ala His Ser Arg Ala Ser Val Met Lys Ile His Gln Leu Ser
1265                    1270                    1275                    1280

cca caa cta ggg gcc tgg gag ctg aga gca gga ccg gac aat ctg gcc    3888
Pro Gln Leu Gly Ala Trp Glu Leu Arg Ala Gly Pro Asp Asn Leu Ala
                1285                    1290                    1295

cca gcg cca agg gca gga act ttt ccc agc ttc tct tca gag ctg cat    3936
Pro Ala Pro Arg Ala Gly Thr Phe Pro Ser Phe Ser Ser Glu Leu His
            1300                    1305                    1310

caa aga aag cag cgc cca gtg aca ccg ctc ctc ttc ctt cca cgc ctc    3984
Gln Arg Lys Gln Arg Pro Val Thr Pro Leu Leu Phe Leu Pro Arg Leu
        1315                    1320                    1325

agg cct cca ccc ctc acc ctt gta taa                                4011
Arg Pro Pro Pro Leu Thr Leu Val
    1330                    1335

<210>146
<211>1336
<212>PRT
<213>Homo sapiens

<400>146

Met Pro Leu Ser Ser His Leu Leu Pro Ala Leu Val Leu Phe Leu Gly
 1               5                   10                  15

His Leu Ala Val Asn Leu Pro Ala Ala Gly Ser Ser Gly Trp Ala Trp
            20                  25                  30

Val Pro Asn His Cys Arg Ser Pro Gly Gln Ala Val Cys Asn Phe Val
        35                  40                  45

Cys Asp Cys Arg Asp Cys Ser Asp Glu Ala Gln Cys Gly Tyr His Gly
    50                  55                  60

Ala Ser Pro Thr Leu Gly Ala Pro Phe Ala Cys Asp Phe Glu Gln Asp
65                  70                  75                  80

Pro Cys Gly Trp Arg Asp Ile Ser Thr Ser Gly Tyr Ser Trp Leu Arg
                85                  90                  95

Asp Arg Ala Gly Ala Ala Leu Glu Gly Pro Gly Pro His Ser Asp His
            100                 105                 110

Thr Leu Gly Thr Asp Leu Gly Trp Tyr Met Ala Val Gly Thr His Arg
        115                 120                 125

Gly Lys Glu Ala Ser Thr Ala Ala Leu Arg Ser Pro Thr Leu Arg Glu
    130                 135                 140

Ala Ala Ser Ser Cys Lys Leu Arg Leu Trp Tyr His Ala Ala Ser Gly
145                 150                 155                 160

Asp Val Ala Glu Leu Arg Val Glu Leu Thr His Gly Ala Glu Thr Leu
                165                 170                 175

Thr Leu Trp Gln Ser Thr Gly Pro Trp Gly Pro Gly Trp Gln Glu Leu
            180                 185                 190

Ala Val Thr Thr Gly Arg Ile Arg Gly Asp Phe Arg Val Thr Phe Ser
        195                 200                 205

Ala Thr Arg Asn Ala Thr His Arg Gly Ala Val Ala Leu Asp Asp Leu
    210                 215                 220

Glu Phe Trp Asp Cys Gly Leu Pro Thr Pro Gln Ala Asn Cys Pro Pro
225                 230                 235                 240

Gly His His His Cys Gln Asn Lys Val Cys Val Glu Pro Gln Gln Leu
                245                 250                 255

Cys Asp Gly Glu Asp Asn Cys Gly Asp Leu Ser Asp Glu Asn Pro Leu
            260                 265                 270

Thr Cys Gly Arg His Ile Ala Thr Asp Phe Glu Thr Gly Leu Gly Pro
        275                 280                 285

Trp Asn Arg Ser Glu Gly Trp Ser Arg Asn His Arg Ala Gly Gly Pro
    290                 295                 300

Glu Arg Pro Ser Trp Pro Arg Arg Asp His Ser Arg Asn Ser Ala Gln
305                 310                 315                 320

Gly Ser Phe Leu Val Ser Val Ala Glu Pro Gly Thr Pro Ala Ile Leu
                325                 330                 335

Ser Ser Pro Glu Phe Gln Ala Ser Gly Thr Ser Asn Cys Ser Ala Pro
            340                 345                 350

Ser Gln Leu Leu Val Pro Gln Leu Val Phe Tyr Gln Tyr Leu Ser Gly
        355                 360                 365

Ser Glu Ala Gly Cys Leu Gln Leu Phe Leu Gln Thr Leu Gly Pro Gly
    370                 375                 380

Ala Pro Arg Ala Pro Val Leu Leu Arg Arg Arg Arg Gly Glu Leu Gly
385                 390                 395                 400

Thr Ala Trp Val Arg Asp Arg Val Asp Ile Gln Ser Ala Tyr Pro Phe
                405                 410                 415

Gln Ile Leu Leu Ala Gly Gln Thr Gly Pro Gly Gly Val Val Gly Leu
            420                 425                 430

Asp Asp Leu Ile Leu Ser Asp His Cys Arg Pro Val Ser Glu Val Ser
        435                 440                 445

Thr Leu Gln Pro Leu Pro Pro Gly Pro Arg Ala Pro Ala Pro Gln Pro
    450                 455                 460

Leu Pro Pro Ser Ser Arg Leu Gln Asp Ser Cys Lys Gln Gly His Leu
465                 470                 475                 480

Ala Cys Gly Asp Leu Cys Val Pro Pro Glu Gln Leu Cys Asp Phe Glu
                485                 490                 495

Glu Gln Cys Ala Gly Gly Glu Asp Glu Gln Ala Cys Gly Lys Gly Ala
            500                 505                 510

Gln Pro Pro Ala Asp Leu Leu Leu Arg Ser Gln Gly Gly Thr Thr Asp
        515                 520                 525

Phe Glu Ser Pro Glu Ala Gly Gly Trp Glu Asp Ala Ser Val Gly Arg
    530                 535                 540

Leu Gln Trp Arg Arg Val Ser Ala Gln Glu Ser Gln Gly Ser Ser Ala
545                 550                 555                 560

Ala Ala Ala Gly Phe Leu Ala Leu Val Val Val Asp Asn Gly Ser Arg
                565                 570                 575

Glu Leu Ala Trp Gln Ala Leu Ser Ser Ser Ala Gly Ile Trp Lys Val
            580                 585                 590

Asp Lys Val Leu Leu Gly Ala Arg Arg Arg Pro Phe Arg Leu Glu Phe
        595                 600                 605

Val Gly Leu Val Asp Leu Asp Gly Pro Asp Gln Gln Gly Ala Gly Val
    610                 615                 620

Asp Asn Val Thr Leu Arg Asp Cys Ser Pro Thr Val Thr Thr Glu Arg
625                 630                 635                 640

Asp Arg Glu Val Ser Cys Asn Phe Glu Arg Asp Thr Cys Ser Trp Tyr
                645                 650                 655

Pro Gly His Leu Ser Asp Thr His Trp Arg Trp Val Glu Ser Arg Gly
            660                 665                 670

Pro Asp His Asp His Thr Thr Gly Gln Gly His Phe Val Leu Leu Asp
        675                 680                 685

Pro Thr Asp Pro Leu Ala Trp Gly His Ser Ala His Leu Leu Ser Arg
    690                 695                 700

Pro Gln Val Pro Ala Ala Pro Thr Glu Cys Leu Ser Phe Trp Tyr His
705                 710                 715                 720

Leu His Gly Pro Gln Ile Gly Thr Leu Arg Leu Ala Met Arg Arg Glu
                725                 730                 735

Gly Glu Glu Thr His Leu Trp Ser Arg Ser Gly Thr Gln Gly Asn Arg
            740                 745                 750

Trp His Glu Ala Trp Ala Thr Leu Ser His Gln Pro Gly Ser His Ala
        755                 760                 765

Gln Tyr Gln Leu Leu Phe Glu Gly Leu Arg Asp Gly Tyr His Gly Thr
    770                 775                 780

Met Ala Leu Asp Asp Val Ala Val Arg Pro Gly Pro Cys Trp Ala Pro
785                 790                 795                 800

Asn Tyr Cys Ser Phe Glu Asp Ser Asp Cys Gly Phe Ser Pro Gly Gly
                805                 810                 815

Gln Gly Leu Trp Arg Arg Gln Ala Asn Ala Ser Gly His Ala Ala Trp
            820                 825                 830

Gly Pro Pro Thr Asp His Thr Thr Glu Thr Ala Gln Gly His Tyr Met
        835                 840                 845

Val Val Asp Thr Ser Pro Asp Ala Leu Pro Arg Gly Gln Thr Ala Ser
    850                 855                 860

Leu Thr Ser Lys Glu His Arg Pro Leu Ala Gln Pro Ala Cys Leu Thr
865                 870                 875                 880

Phe Trp Tyr His Gly Ser Leu Arg Ser Pro Gly Thr Leu Arg Val Tyr
                885                 890                 895

Leu Glu Glu Arg Gly Arg His Gln Val Leu Ser Leu Ser Ala His Gly
            900                 905                 910

Gly Leu Ala Trp Arg Leu Gly Ser Met Asp Val Gln Ala Glu Arg Ala
        915                 920                 925

Trp Arg Val Val Phe Glu Ala Val Ala Ala Gly Val Ala His Ser Tyr
    930                 935                 940

Val Ala Leu Asp Asp Leu Leu Leu Gln Asp Gly Pro Cys Pro Gln Pro
945                 950                 955                 960

Gly Ser Cys Asp Phe Glu Ser Gly Leu Cys Gly Trp Ser His Leu Ala
                965                 970                 975

Trp Pro Gly Leu Gly Gly Tyr Ser Trp Asp Trp Gly Gly Gly Ala Thr
            980                 985                 990

Pro Ser Arg Tyr Pro Gln Pro Pro Val Asp His Thr Leu Gly Thr Glu
        995                 1000                1005

Ala Gly His Phe Ala Phe Phe Glu Thr Gly Val Leu Gly Pro Gly Gly
    1010                1015                1020

Arg Ala Ala Trp Leu Arg Ser Glu Pro Leu Pro Ala Thr Pro Ala Ser
1025                1030                1035                1040

Cys Leu Arg Phe Trp Tyr His Met Gly Phe Pro Glu His Phe Tyr Lys
                1045                1050                1055

Gly Glu Leu Lys Val Leu Leu His Ser Ala Gln Gly Gln Leu Ala Val
            1060                1065                1070

Trp Gly Ala Gly Gly His Arg Arg His Gln Trp Leu Glu Ala Gln Val
        1075                1080                1085

Glu Val Ala Ser Ala Lys Glu Phe Gln Ile Val Phe Glu Ala Thr Leu
    1090                1095                1100

Gly Gly Gln Pro Ala Leu Gly Pro Ile Ala Leu Asp Asp Val Glu Tyr
1105                1110                1115                1120

Leu Ala Gly Gln His Cys Gln Gln Pro Ala Pro Ser Pro Gly Asn Thr
                1125                1130                1135

Ala Ala Pro Gly Ser Val Pro Ala Val Val Gly Ser Ala Leu Leu Leu
            1140                1145                1150

Leu Met Leu Leu Val Leu Leu Gly Leu Gly Gly Arg Arg Trp Leu Gln
        1155                1160                1165

Lys Lys Gly Ser Cys Pro Phe Gln Ser Asn Thr Glu Ala Thr Ala Pro
    1170                1175                1180

Gly Phe Asp Asn Ile Leu Phe Asn Ala Glu Pro Cys Gly Val Gly Gly
1185                1190                1195                1200

His Ser Thr Ala Pro Leu Pro Ala Ala Arg Leu Pro Ser Ala Leu Gly
                1205                1210                1215

Thr Lys Ser Gln Arg Arg Ala Ala Val Gly His Gly Tyr Arg Arg Pro
            1220                1225                1230

Ser Ser Ser Gly Ala Val Gly Leu Thr Ser Ala His Gln Leu Ser Thr
        1235                1240                1245

Gln Met Gly Glu Glu Glu Met Ala Leu Gln Arg Pro Ser Glu Leu Pro
    1250                1255                1260

Pro Ala Ala His Ser Arg Ala Ser Val Met Lys Ile His Gln Leu Ser
1265                1270                1275                1280

Pro Gln Leu Gly Ala Trp Glu Leu Arg Ala Gly Pro Asp Asn Leu Ala
                1285                1290                1295

Pro Ala Pro Arg Ala Gly Thr Phe Pro Ser Phe Ser Ser Glu Leu His
            1300                1305                1310

Gln Arg Lys Gln Arg Pro Val Thr Pro Leu Leu Phe Leu Pro Arg Leu
        1315                1320                1325

Arg Pro Pro Pro Leu Thr Leu Val
    1330                1335

<210>147
<211>22

<212> DNA
<213> Artificial Sequence

<220>
<223> Description of Artificial Sequence: Forward Primer

<400>147
ccagagtgtt ctgctctttg ag                                                22

<210>148
<211>26

<212> DNA
<213> Artificial Sequence

<220>
<223> Description of Artificial Sequence: probe

<400>148
tgctctttta tcagccagac ttgaaa                                            26

<210>149
<211>21

<212> DNA
<213> Artificial Sequence

<220>
<223> Description of Artificial Sequence: Reverse Primer

<400>149
gagagtttcg tgagggtgaa g                                                 21

<210>150
<211>20

<212> DNA
<213> Artificial Sequence

<220>
<223> Description of Artificial Sequence: Forward Primer

<400>150
tattcttttg ccgagcaaca                                                   20

<210>151
<211>26

<212> DNA
<213> Artificial Sequence

<220>
<223> Description of Artificial Sequence: probe

<400>151
caagttcata cacttgaacg tccagg                                26

<210>152
<211>22

<212> DNA
<213> Artificial Sequence

<220>
<223> Description of Artificial Sequence: Reverse Primer

<400>152
aatgcaatgg ctgtacaaaa ct                                    22

<210>153
<211>22

<212> DNA
<213> Artificial Sequence

<220>
<223> Description of Artificial Sequence: Forward Primer

<400>153
tataactcct tgccatgcaa ac                                    22

<210>154
<211>26

<212> DNA
<213> Artificial Sequence

<220>
<223> Description of Artificial Sequence: probe

<400>154
tcaagaggcc aatatattcc tggcca                                26

<210>155
<211>21

<212> DNA
<213> Artificial Sequence

<220>
<223> Description of Artificial Sequence: Reverse Primer

<400>155
gcatttgcat ggctctaagt t                                     21

<210>156
<211>22

<212> DNA
<213> Artificial Sequence

<220>
<223> Description of Artificial Sequence: Forward Primer

<400>156
cccactttgc tcttacaaag ac                                                22

<210>157
<211>26

<212> DNA
<213> Artificial Sequence

<220>
<223> Description of Artificial Sequence: probe

<400>157
actttcattg aaggcagatc atacca                                            26

<210>158
<211>22

<212> DNA
<213> Artificial Sequence

<220>
<223> Description of Artificial Sequence: Reverse Primer

<400>158
ttttccattt tcaccagatt gt                                                22

<210>159
<211>22

<212> DNA
<213> Artificial Sequence

<220>
<223> Description of Artificial Sequence: Forward Primer

<400>159
ggagaaaatt ctttgggaca ga                                                22

<210>160
<211>28

<212> DNA
<213> Artificial Sequence

<220>
<223> Description of Artificial Sequence: probe

<400>160
caacaaacaa tgtttgcaat cagaatca                                          28

<210>161
<211>22

<212> DNA
<213> Artificial Sequence

<220>
<223> Description of Artificial Sequence: Reverse Primer

<400>161
tgatgaatgt ctcgaggcta tt                                                22

<210>162
<211>20

<212> DNA
<213> Artificial Sequence

<220>
<223> Description of Artificial Sequence: Forward Primer

<400>162
gaacactggg agcagatgtg                                                   20

<210>163
<211>29

<212> DNA
<213> Artificial Sequence

<220>
<223> Description of Artificial Sequence: probe

<400>163
aggaccatgc tcgatcctct ctggtaata                                         29

<210>164
<211>20

<212> DNA
<213> Artificial Sequence

<220>
<223> Description of Artificial Sequence: Reverse Primer

<400>164
aggaggatca caggtggaga                                                   20

<210>165
<211>20

<212> DNA
<213> Artificial Sequence

<220>
<223> Description of Artificial Sequence: Forward Primer

<400>165
ccaacaagca cacctttgac                                                    20

<210>166
<211>25

<212> DNA
<213> Artificial Sequence

<220>
<223> Description of Artificial Sequence: probe

<400>166
ctgtggagat cctcatccac cccag                                              25

<210>167
<211>20

<212> DNA
<213> Artificial Sequence

<220>
<223> Description of Artificial Sequence: Reverse Primer

<400>167
ctatcaggac atggggcata                                                    20

<210>168
<211>22

<212> DNA
<213> Artificial Sequence

<220>
<223> Description of Artificial Sequence: Forward Primer

<400>168
gggatgatgt gtttgcagat at                                                 22

<210>169
<211>30

<212> DNA
<213> Artificial Sequence

<220>
<223> Description of Artificial Sequence: probe

<400>169
cagagaaatt gagtcaactt cagaaaacca                                         30

<210>170
<211>21

<212> DNA
<213> Artificial Sequence

<220>
<223> Description of Artificial Sequence: Reverse Primer

<400>170
agttatcctg ctgctgttgg a                                                21

<210>171
<211>19

<212> DNA
<213> Artificial Sequence

<220>
<223> Description of Artificial Sequence: Forward Primer

<400>171
gatggctctg ttccctctc                                                   19

<210>172
<211>30

<212> DNA
<213> Artificial Sequence

<220>
<223> Description of Artificial Sequence: probe

<400>172
agtactaaag aacttgacca gatcaatgga                                       30

<210>173
<211>24

<212> DNA
<213> Artificial Sequence

<220>
<223> Description of Artificial Sequence: Reverse Primer

<400>173
cagcgagaaa tataaatatt tcct                                             24

<210>174
<211>22

<212> DNA
<213> Artificial Sequence

<220>
<223> Description of Artificial Sequence: Forward Primer

<400>174
agatctggag gatacccaga tc                                               22

<210>175
<211>30

<212> DNA
<213> Artificial Sequence

<220>
<223> Description of Artificial Sequence: probe

<400>175
ccaacatcaa gaagtactcc ttataaacca 30

<210>176
<211>22

<212> DNA
<213> Artificial Sequence

<220>
<223> Description of Artificial Sequence: Reverse Primer

<400>176
gcaaaacatc actggcttta tt 22

<210>177

<211>22

<212> DNA
<213> Artificial Sequence

<220>
<223> Description of Artificial Sequence: Forward Primer

<400>177

acttgaactc caggatggaa tt 22

<210>178
<211>26

<212> DNA
<213> Artificial Sequence

<220>
<223> Description of Artificial Sequence: probe

<400>178
cttcctcctc tgctgggact cctttg 26

<210>179
<211>20

<212> DNA
<213> Artificial Sequence

<220>
<223> Description of Artificial Sequence: Reverse Primer

<400>179
cttgcgagag gtgagatgag                                                    20

<210>180
<211>22

<212> DNA
<213> Artificial Sequence

<220>
<223> Description of Artificial Sequence: Forward Primer

<400>180
ggcagcaatt atgagtacga tt                                                 22

<210>181
<211>27

<212> DNA
<213> Artificial Sequence

<220>
<223> Description of Artificial Sequence: probe

<400>181
tcccaattac ttgtgacttc aagttca                                            27

<210>182
<211>21

<212> DNA
<213> Artificial Sequence

<220>
<223> Description of Artificial Sequence: Reverse Primer

<400>182
tgcttcttca ccacgaatta a                                                  21

<210>183
<211>20

<212> DNA
<213> Artificial Sequence

<220>
<223> Description of Artificial Sequence: Forward Primer

<400>183
cctccctggt agaggtcaac                                                    20

<210>184
<211>25

<212> DNA
<213> Artificial Sequence

<220>
<223> Description of Artificial Sequence: probe

<400>184
ctactcagtg cccagcagcc aggag                                          25

<210>185
<211>19

<212> DNA
<213> Artificial Sequence

<220>
<223> Description of Artificial Sequence: Reverse Primer

<400>185
tgtctgcatg cagcctatg                                                 19

<210>186
<211>22

<212> DNA
<213> Artificial Sequence

<220>
<223> Description of Artificial Sequence: Forward Primer

<400>186
cagagtaatg gtgaaggcat tg                                             22

<210>187
<211>30

<212> DNA
<213> Artificial Sequence

<220>
<223> Description of Artificial Sequence: probe

<400>187
tcagcaatct tctaaacaaa gtctgtttga                                     30

<210>188
<211>22

<212> DNA
<213> Artificial Sequence

<220>
<223> Description of Artificial Sequence: Reverse Primer

<400>188
cccaccatga gacatcttac at                                             22

<210>189
<211>20

<212> DNA
<213> Artificial Sequence

<220>
<223> Description of Artificial Sequence: Forward Primer

<400>189
acctgcagct gagaaaatcc                                                    20

<210>190
<211>25

<212> DNA
<213> Artificial Sequence

<220>
<223> Description of Artificial Sequence: probe

<400>190
ctcaaccacc tcgtctcctc catcg                                              25

<210>191
<211>20

<212> DNA
<213> Artificial Sequence

<220>
<223> Description of Artificial Sequence: Reverse Primer

<400>191
aggcatacaa cccactgtca                                                    20

<210>192
<211>20

<212> DNA
<213> Artificial Sequence

<220>
<223> Description of Artificial Sequence: Forward Primer

<400>192
cctttatgca accaacatgg                                                    20

<210>193
<211>30

<212> DNA
<213> Artificial Sequence

<220>
<223> Description of Artificial Sequence: probe

<400>193
ccatgtcctg ttcttagtgc ttgaatgtcc                                         30

<210>194
<211>20

<212> DNA
<213> Artificial Sequence

<220>
<223> Description of Artificial Sequence: Reverse Primer

<400>194
ggcttcttca gcttcaggtt                                                    20

<210>195
<211>21

<212> DNA
<213> Artificial Sequence

<220>
<223> Description of Artificial Sequence: Forward Primer

<400>195
tgcttcatct tccctcagtt t                                                  21

<210>196
<211>26

<212> DNA
<213> Artificial Sequence

<220>
<223> Description of Artificial Sequence: probe

<400>196
acaacagact ttgtacgaca ccgcgt                                             26

<210>197
<211>22

<212> DNA
<213> Artificial Sequence

<220>
<223> Description of Artificial Sequence: Reverse Primer

<400>197
gctgatgttg tagagggtca ga                                                 22

<210>198
<211>22

<212> DNA
<213> Artificial Sequence

<220>
<223> Description of Artificial Sequence: Forward Primer

<400>198
agactccaaa gtacacgcag aa                                                22

<210>199
<211>26

<212> DNA
<213> Artificial Sequence

<220>
<223> Description of Artificial Sequence: probe

<400>199
tcacatcgag actctgactg aacgct                                            26

<210>200
<211>22

<212> DNA
<213> Artificial Sequence

<220>
<223> Description of Artificial Sequence: Reverse Primer

<400>200
gcctgatctt tgatctttag ca                                                22

<210>201
<211>21

<212> DNA
<213> Artificial Sequence

<220>
<223> Description of Artificial Sequence: Forward Primer

<400>201
acctgctcta cggacacatg t                                                 21

<210>202
<211>25

<212> DNA
<213> Artificial Sequence

<220>
<223> Description of Artificial Sequence: probe

<400>202
ctacatcatg cccgacatgc tgtgt                                             25

<210>203
<211>22

<212> DNA
<213> Artificial Sequence

<220>
<223> Description of Artificial Sequence: Reverse Primer

<400>203
ctcacacacg gtcttagcat tc                                                22

<210>204
<211>22

<212> DNA
<213> Artificial Sequence

<220>
<223> Description of Artificial Sequence: Forward Primer

<400>204
cctctgtacc ctggagtgta tg                                                22

<210>205
<211>27

<212> DNA
<213> Artificial Sequence

<220>
<223> Description of Artificial Sequence: probe

<400>205
ccagtgtttc ctatttctca aaatgga                                           27

<210>206
<211>22

<212> DNA
<213> Artificial Sequence

<220>
<223> Description of Artificial Sequence: Reverse Primer

<400>206
tgggcgtgat ttctatgtta tc                                                22

<210>207
<211>23

<212> DNA
<213> Artificial Sequence

<220>
<223> Description of Artificial Sequence: Forward Primer

<400>207
gaaataggaa acactggcat aca                                               23

<210>208
<211>26

<212> DNA
<213> Artificial Sequence

<220>
<223> Description of Artificial Sequence: probe

<400>208
ctgctccaac cctctgtacc ctggag 26

<210>209
<211>20

<212> DNA
<213> Artificial Sequence

<220>
<223> Description of Artificial Sequence: Reverse Primer

<400>209
ggttgcagat tggaattgtg 20

<210>210
<211>22

<212> DNA
<213> Artificial Sequence

<220>
<223> Description of Artificial Sequence: Forward Primer

<400>210
aatgggatca tgattggata ca 22

<210>211
<211>26

<212> DNA
<213> Artificial Sequence

<220>
<223> Description of Artificial Sequence: probe

<400>211
aatatgtggc ctgtacgttc tcccca 26

<210>212
<211>22

<212> DNA
<213> Artificial Sequence

<220>
<223> Description of Artificial Sequence: Reverse Primer

<400>212
ttcctacttt ggtcccgtta ac 22

<210>213
<211>22

<212> DNA
<213> Artificial Sequence

<220>
<223> Description of Artificial Sequence: Forward Primer

<400>213
cagccctcat ctatgactac ga                                                22

<210>214
<211>23

<212> DNA
<213> Artificial Sequence

<220>
<223> Description of Artificial Sequence: probe

<400>214
acgctgagct ccatcctgtc cag                                               23

<210>215
<211>22

<212> DNA
<213> Artificial Sequence

<220>
<223> Description of Artificial Sequence: Reverse Primer

<400>215
agtcgtagtc ctggtcctca tc                                                22

<210>216
<211>22

<212> DNA
<213> Artificial Sequence

<220>
<223> Description of Artificial Sequence: Forward Primer

<400>216
agttccacgt cttgagaaaa ca                                                22

<210>217
<211>27

<212> DNA
<213> Artificial Sequence

<220>
<223> Description of Artificial Sequence: probe

<400>217
tgagcattaa cttcgatctc caaatca                                27

<210>218
<211>21

<212> DNA
<213> Artificial Sequence

<220>
<223> Description of Artificial Sequence: Reverse Primer

<400>218
gctgtctgga ttgtccttgt t                                      21

<210>219
<211>22

<212> DNA
<213> Artificial Sequence

<220>
<223> Description of Artificial Sequence: Forward Primer

<400>219
agttccacgt cttgagaaaa ca                                     22

<210>220
<211>27

<212> DNA
<213> Artificial Sequence

<220>
<223> Description of Artificial Sequence: probe

<400>220
tgagcattaa cttcgatctc caaatca                                27

<210>221
<211>21

<212> DNA
<213> Artificial Sequence

<220>
<223> Description of Artificial Sequence: Reverse Primer

<400>221
gctgtctgga ttgtccttgt t                                      21

<210>222
<211>20

<212> DNA
<213> Artificial Sequence

<220>
<223> Description of Artificial Sequence: Forward Primer

<400>222
cctatgacca cgagggtttt                                                    20

<210>223
<211>26

<212> DNA
<213> Artificial Sequence

<220>
<223> Description of Artificial Sequence: probe

<400>223
tcatcaccga tggatatctc tcctct                                             26

<210>224
<211>20

<212> DNA
<213> Artificial Sequence

<220>
<223> Description of Artificial Sequence: Reverse Primer

<400>224
ggagagcagg gcaagattaa                                                    20

<210>225
<211>21

<212> DNA
<213> Artificial Sequence

<220>
<223> Description of Artificial Sequence: Forward Primer

<400>225
gcttgatgaa agcaagacag a                                                  21

<210>226
<211>28

<212> DNA
<213> Artificial Sequence

<220>
<223> Description of Artificial Sequence: probe

<400>226
ctcagatctc acaaatgacc ttaaaagg                                           28

<210>227
<211>20

<212> DNA
<213> Artificial Sequence

<220>
<223> Description of Artificial Sequence: Reverse Primer

<400>227
gctgcctttc ttttgtgatg 20

<210>228
<211>20

<212> DNA
<213> Artificial Sequence

<220>
<223> Description of Artificial Sequence: Forward Primer

<400>228
gctgcacttg tgatctccat 20

<210>229
<211>26

<212> DNA
<213> Artificial Sequence

<220>
<223> Description of Artificial Sequence: probe

<400>229
acattaagtc ttctgctcac acgctc 26

<210>230
<211>21

<212> DNA
<213> Artificial Sequence

<220>
<223> Description of Artificial Sequence: Reverse Primer

<400>230
atttaggtcc ttggcattcc t 21

<210>231
<211>22

<212> DNA
<213> Artificial Sequence

<220>
<223> Description of Artificial Sequence: Forward Primer

<400>231
gctgtttgtt tggagtcgtc ta 22

<210>232
<211>26

<212> DNA
<213> Artificial Sequence

<220>
<223> Description of Artificial Sequence: probe

<400>232
cttcttcggt taccgctgct tcaagg                                26

<210>233
<211>21

<212> DNA
<213> Artificial Sequence

<220>
<223> Description of Artificial Sequence: Reverse Primer

<400>233
aacagcaacc cagtgagaaa g                                     21

<210>234
<211>22

<212> DNA
<213> Artificial Sequence

<220>
<223> Description of Artificial Sequence: Forward Primer

<400>234
gacgacaatg tggcagagta tc                                    22

<210>235
<211>24

<212> DNA
<213> Artificial Sequence

<220>
<223> Description of Artificial Sequence: probe

<400>235
aaactggcca acaacgcgga caag                                  24

<210>236
<211>22

<212> DNA
<213> Artificial Sequence

<220>
<223> Description of Artificial Sequence: Reverse Primer

<400>236
tcttctcgaa cacttgcttg at                                                22

<210>237
<211>19

<212> DNA
<213> Artificial Sequence

<220>
<223> Description of Artificial Sequence: Forward Primer

<400>237
agtcacttgg ggacctcag                                                    19

<210>238
<211>25

<212> DNA
<213> Artificial Sequence

<220>
<223> Description of Artificial Sequence: probe

<400>238
ctccttcctg catggaccag agctg                                             25

<210>239
<211>21

<212> DNA
<213> Artificial Sequence

<220>
<223> Description of Artificial Sequence: Reverse Primer

<400>239
catcacgatg gatacgtagc t                                                 21

<210>240
<211>22

<212> DNA
<213> Artificial Sequence

<220>
<223> Description of Artificial Sequence: Forward Primer

<400>240
agcttggtgg acctgactct at                                                22

<210>241
<211>26

<212> DNA
<213> Artificial Sequence

<220>
<223> Description of Artificial Sequence: probe

<400>241
ttttattaca cctcatgctt tcgctg                                          26

<210>242
<211>21

<212> DNA
<213> Artificial Sequence

<220>
<223> Description of Artificial Sequence: Reverse Primer

<400>242
aagccctcaa atttcgtagg t                                               21

<210>243
<211>22

<212> DNA
<213> Artificial Sequence

<220>
<223> Description of Artificial Sequence: Forward Primer

<400>243
ggttgtctgg cttttacctt ct                                              22

<210>244
<211>25

<212> DNA
<213> Artificial Sequence

<220>
<223> Description of Artificial Sequence: probe

<400>244
   ttttccacag gatgcaccct ggact                                          25

<210>245
<211>22

<212> DNA
<213> Artificial Sequence

<220>
<223> Description of Artificial Sequence: Reverse Primer

<400>245
ctgtcttggg taggaactga tg                                              22

<210>246
<211>19

<212> DNA
<213> Artificial Sequence

<220>
<223> Description of Artificial Sequence: Forward Primer

<400>246
ggacccctga ctgaatcct 19

<210>247
<211>24

<212> DNA
<213> Artificial Sequence

<220>
<223> Description of Artificial Sequence: probe

<400>247
ctcccatggc tgcctcctcc ttag 24

<210>248
<211>20

<212> DNA
<213> Artificial Sequence

<220>
<223> Description of Artificial Sequence: Reverse Primer

<400>248
atgctggagc tctccttctc 20

<210>249
<211>22

<212> DNA
<213> Artificial Sequence

<220>
<223> Description of Artificial Sequence: Forward Primer

<400>249
tgtatccaca gactgccaga ct 22

<210>250
<211>27

<212> DNA
<213> Artificial Sequence

<220>
<223> Description of Artificial Sequence: probe

<400>250
tcgtccgaaa catacagtcc tttcaca 27

<210>251
<211>22

<212> DNA
<213> Artificial Sequence

<220>
<223> Description of Artificial Sequence: Reverse Primer

<400>251
atgtcacaaa agttccgtgt gt                                                22

<210>252
<211>20

<212> DNA
<213> Artificial Sequence

<220>
<223> Description of Artificial Sequence: Forward Primer

<400>252
gtctgtgctg tggccgttct                                                   20

<210>253
<211>23

<212> DNA
<213> Artificial Sequence

<220>
<223> Description of Artificial Sequence: probe

<400>253
cagcagggct ccaacatgac gct                                               23

<210>254
<211>21

<212> DNA
<213> Artificial Sequence

<220>
<223> Description of Artificial Sequence: Reverse Primer

<400>254
acctgtctca agggccagtg t                                                 21

<210>255
<211>22

<212> DNA
<213> Artificial Sequence

<220>
<223> Description of Artificial Sequence: Forward Primer

<400>255
tatcctcaga ggcagcagtt ta                                                22

<210>256
<211>26

<212> DNA
<213> Artificial Sequence

<220>
<223> Description of Artificial Sequence: probe

<400>256
tggtatcctt ctgttccctc ttgcag                                            26

<210>257
<211>22

<212> DNA
<213> Artificial Sequence

<220>
<223> Description of Artificial Sequence: Reverse Primer

<400>257
taggacagtg caaccattca ct                                                22

<210>258
<211>22

<212> DNA
<213> Artificial Sequence

<220>
<223> Description of Artificial Sequence: Forward Primer

<400>258
tatcctcaga ggcagcagtt ta                                                22

<210>259
<211>26

<212> DNA
<213> Artificial Sequence

<220>
<223> Description of Artificial Sequence: probe

<400>259
tggtatcctt ctgttccctc ttgcag                                            26

<210>260
<211>22

<212> DNA
<213> Artificial Sequence

<220>
<223> Description of Artificial Sequence: Reverse Primer

<400>260
taggacagtg caaccattca ct                                                22

<210>261
<211>18

<212> DNA
<213> Artificial Sequence

<220>
<223> Description of Artificial Sequence: Forward Primer

<400>261
gcaccctcga tgaaagct                                                     18

<210>262
<211>26

<212> DNA
<213> Artificial Sequence

<220>
<223> Description of Artificial Sequence: probe

<400>262
atacctcgct actaccacgt cctggg                                            26

<210>263
<211>20

<212> DNA
<213> Artificial Sequence

<220>
<223> Description of Artificial Sequence: Reverse Primer

<400>263
agaactggca atggaacatg                                                   20

<210>264
<211>19

<212> DNA
<213> Artificial Sequence

<220>
<223> Description of Artificial Sequence: Forward Primer

<400>264
cacggttcga ggtgttcct                                                    19

<210>265
<211>29

<212> DNA
<213> Artificial Sequence

<220>
<223> Description of Artificial Sequence: probe

<400>265
tacttgtgaa gagcctgcag agcaaggat 29

<210>266
<211>21

<212> DNA
<213> Artificial Sequence

<220>
<223> Description of Artificial Sequence: Reverse Primer

<400>266
tccacatatt ccagcaccat g 21

<210>267
<211>22

<212> DNA
<213> Artificial Sequence

<220>
<223> Description of Artificial Sequence: Forward Primer

<400>267
gctttctgcc cagaacttgt at 22

<210>268
<211>26

<212> DNA
<213> Artificial Sequence

<220>
<223> Description of Artificial Sequence: probe

<400>268
aattgccttg gcttgtattt cctttt 26

<210>269
<211>22

<212> DNA
<213> Artificial Sequence

<220>
<223> Description of Artificial Sequence: Reverse Primer

<400>269
aagaaaagta agcaccccag aa 22

<210>270
<211>20

<212> DNA
<213> Artificial Sequence

<220>
<223> Description of Artificial Sequence: Forward Primer

<400>270
cattggaaag aatggcaaga                                                    20

<210>271
<211>27

<212> DNA
<213> Artificial Sequence

<220>
<223> Description of Artificial Sequence: probe

<400>271
catgatcatg ccatcttact aacagga                                            27

<210>272
<211>21

<212> DNA
<213> Artificial Sequence

<220>
<223> Description of Artificial Sequence: Reverse Primer

<400>272
tcacatggtt cattcttcca a                                                  21

<210>273
<211>21

<212> DNA
<213> Artificial Sequence

<220>
<223> Description of Artificial Sequence: Forward Primer

<400>273
ttggaagaat gaaccatgtg a                                                  21

<210>274
<211>26

<212> DNA
<213> Artificial Sequence

<220>
<223> Description of Artificial Sequence: probe

<400>274
ccccatcagt ggaatgtgct ctaagt 26

<210>275
<211>22

<212> DNA
<213> Artificial Sequence

<220>
<223> Description of Artificial Sequence: Reverse Primer

<400>275
caagtcctgt gtcctcattg at 22

<210>276
<211>23

<212> DNA
<213> Artificial Sequence

<220>
<223> Description of Artificial Sequence: Forward Primer

<400>276
ggatagcttg gaagtatgca ctt 23

<210>277
<211>26

<212> DNA
<213> Artificial Sequence

<220>
<223> Description of Artificial Sequence: probe

<400>277
caaggtcatg aatggaactc caccag 26

<210>278
<211>20

<212> DNA
<213> Artificial Sequence

<220>
<223> Description of Artificial Sequence: Reverse Primer

<400>278
ctggcatcca gcaggtatag 20

<210>279
<211>23

<212> DNA
<213> Artificial Sequence

<220>
<223> Description of Artificial Sequence: Forward Primer

<400>279
ggatagcttg gaagtatgca ctt                                                23

<210>280
<211>26

<212> DNA
<213> Artificial Sequence

<220>
<223> Description of Artificial Sequence: probe

<400>280
caaggtcatg aatggaactc caccag                                             26

<210>281
<211>20

<212> DNA
<213> Artificial Sequence

<220>
<223> Description of Artificial Sequence: Reverse Primer

<400>281
ctggcatcca gcaggtatag                                                    20

<210>282
<211>17

<212> DNA
<213> Artificial Sequence

<220>
<223> Description of Artificial Sequence: Forward Primer

<400>282
ctgccctcca caatgga                                                       17

<210>283
<211>26

<212> DNA
<213> Artificial Sequence

<220>
<223> Description of Artificial Sequence: probe

<400>283
ccttggaccc tggtctcagt gttcca                                             26

<210>284
<211>20

<212> DNA
<213> Artificial Sequence

<220>
<223> Description of Artificial Sequence: Reverse Primer

<400>284
cagaggagtt cacgcttcct                                                    20

<210>285
<211>22

<212> DNA
<213> Artificial Sequence

<220>
<223> Description of Artificial Sequence: Forward Primer

<400>285
caagttgtct gctccatcag aa                                                 22

<210>286
<211>30

<212> DNA
<213> Artificial Sequence

<220>
<223> Description of Artificial Sequence: probe

<400>286
ccggtgctac gagcctgtaa tacaaacttc                                         30

<210>287
<211>23

<212> DNA
<213> Artificial Sequence

<220>
<223> Description of Artificial Sequence: Reverse Primer

<400>287
gatcctctct cttttcagga gct                                                23

<210>288
<211>22

<212> DNA
<213> Artificial Sequence

<220>
<223> Description of Artificial Sequence: Forward Primer

<400>288
tgtggtggca ttattaccaa ct                                                 22

<210>289
<211>24

<212> DNA
<213> Artificial Sequence

<220>
<223> Description of Artificial Sequence: probe

<400>289
ccccacagaa tgaaatgcat gaca                                                24

<210>290
<211>22

<212> DNA
<213> Artificial Sequence

<220>
<223> Description of Artificial Sequence: Reverse Primer

<400>290
atttcccaca cacaagtgat gt                                                  22

<210>291
<211>22

<212> DNA
<213> Artificial Sequence

<220>
<223> Description of Artificial Sequence: Forward Primer

<400>291
ggaaatgcca gcagtatatg at                                                  22

<210>292
<211>26

<212> DNA
<213> Artificial Sequence

<220>
<223> Description of Artificial Sequence: probe

<400>292
catttgcctt gatttcccac acacaa                                              26

<210>293
<211>22

<212> DNA
<213> Artificial Sequence

<220>
<223> Description of Artificial Sequence: Reverse Primer

<400>293
aatgaaatgc atgacaacat ca                                                22

<210>294
<211>21

<212> DNA
<213> Artificial Sequence

<220>
<223> Description of Artificial Sequence: Forward Primer

<400>294
ctatactctc cagccccgaa t                                                 21

<210>295
<211>24

<212> DNA
<213> Artificial Sequence

<220>
<223> Description of Artificial Sequence: probe

<400>295
aagcctcagg cacctccaac tgct                                              24

<210>296
<211>22

<212> DNA
<213> Artificial Sequence

<220>
<223> Description of Artificial Sequence: Reverse Primer

<400>296
tgatagaaga ccagctgtgg aa                                                22

<210>297
<211>21

<212> DNA
<213> Artificial Sequence

<220>
<223> Description of Artificial Sequence: Forward Primer

<400>297
ctacgtggct ctggatgatc t                                                 21

<210>298
<211>23

<212> DNA
<213> Artificial Sequence

<220>
<223> Description of Artificial Sequence: probe

<400>298
cctgccctca gccaggttcc tgt                                    23

<210>299
<211>21

<212> DNA
<213> Artificial Sequence

<220>
<223> Description of Artificial Sequence: Reverse Primer

<400>299
acacaggcca gactcaaaat c                                      21

1

## Description

### FIELD OF THE INVENTION

The present invention relates to novel antibodies that bind immunospecifically to antigenic polypeptides, wherein the polypeptides have characteristic properties related to biochemical or physiological responses in a cell, a tissue, an organ or an organism. The novel polypeptides are gene products of novel genes, or are specified biologically active fragments or derivatives thereof. Methods of use of the antibodies encompass procedures for diagnostic and prognostic assay of the polypeptides, as well as methods of treating diverse pathological conditions.

### BACKGROUND OF THE INVENTION

Eukaryotic cells are characterized by biochemical and physiological processes which under normal conditions are exquisitely balanced to achieve the preservation and propagation of the cells. When such cells are components of multicellular organisms such as vertebrates, or more particularly organisms such as mammals, the regulation of the biochemical and physiological processes involves intricate signaling pathways. Frequently, such signaling pathways involve extracellular signaling proteins, cellular receptors that bind the signaling proteins, and signal transducing components located within the cells.

Signaling proteins may be classified as endocrine effectors, paracrine effectors or autocrine effectors. Endocrine effectors are signaling molecules secreted by a given organ into the circulatory system, which are then transported to a distant target organ or tissue. The target cells include the receptors for the endocrine effector, and when the endocrine effector binds, a signaling cascade is induced. Paracrine effectors involve secreting cells and receptor cells in close proximity to each other, for example two different classes of cells in the same tissue or organ. One class of cells secretes the paracrine effector, which then reaches the second class of cells, for example by diffusion through the extracellular fluid. The second class of cells contains the receptors for the paracrine effector; binding of the effector results in induction of the signaling cascade that elicits the corresponding biochemical or physiological effect. Autocrine effectors are highly analogous to paracrine effectors, except that the same cell type that secretes the autocrine effector also contains the receptor. Thus the autocrine effector binds to receptors on the same cell, or on identical neighboring cells. The binding process then elicits the characteristic biochemical or physiological effect.

Signaling processes may elicit a variety of effects on cells and tissues including by way of nonlimiting example induction of cell or tissue proliferation, suppression of growth or proliferation, induction of differentiation or maturation of a cell or tissue, and suppression of differentiation or maturation of a cell or tissue.

Many pathological conditions involve dysregulation of expression of important effector proteins. In certain classes of pathologies the dysregulation is manifested as elevated or excessive synthesis and secretion of protein effectors. In a clinical setting a subject may be suspected of suffering from a condition brought on by elevated or excessive levels of a protein effector of interest.

Antibodies are multichain proteins that bind specifically to a given antigen, and bind poorly, or not at all, to substances deemed not to be cognate antigens. Antibodies are comprised of two short chains termed light chains and two long chains termed heavy chains. These chains are constituted of immunoglobulin domains, of which generally there are two classes: one variable domain per chain, one constant domain in light chains, and three or more constant domains in heavy chains. The antigen-specific portion of the immunoglobulin molecules resides in the variable domains; the variable domains of one light chain and one heavy chain associate with each other to generate the antigen-binding moiety. Antibodies that bind immunospecifically to a cognate or target antigen bind with high affinities. Accordingly, they are useful in assaying specifically for the presence of the antigen in a sample. In addition, they have the potential of inactivating the activity of the antigen.

Therefore there is a need to assay for the level of a protein effector of interest in a biological sample from such a subject, and to compare this level with that characteristic of a nonpathological condition. In particular, there is a need for such an assay based on the use of an antibody that binds immunospecifically to the antigen. There further is a need to inhibit the activity of the protein effector in cases where a pathological condition arises from elevated or excessive levels of the effector based on the use of an antibody that binds immunospecifically to the effector. Thus, there is a need for the antibody as a product of manufacture. There further is a need for a method of treatment of a pathological condition brought on by an elevated or excessive level of the protein effector of interest based on administering the antibody to the subject.

### SUMMARY OF THE INVENTION

The invention is based in part upon the discovery of nucleic acid sequences encoding novel polypeptides. The novel nucleic acids and polypeptides are referred to herein as NOVX, or NOV1, NOV2, NOV3, *etc*., nucleic acids and polypeptides. These nucleic acids and polypeptides, as well as derivatives, homologs, analogs and fragments thereof, will hereinafter be collectively designated as "NOVX" nucleic acid or polypeptide sequences.

In one aspect, the invention provides an isolated polypeptide comprising a mature form of a NOVX amino acid. The polypeptide can be, for example, a NOVX amino acid sequence or a variant of a NOVX amino acid sequence, wherein any amino acid specified in the chosen sequence is changed to a different amino acid, provided that no more than 15% of the amino acid residues in the sequence are so changed. The invention also includes fragments of any of NOVX polypeptides. In another aspect, the invention also includes an isolated nucleic acid that encodes a NOVX polypeptide, or a fragment, homolog, analog or derivative thereof.

Also included in the invention is a NOVX polypeptide that is a naturally occurring variant of a NOVX sequence. In one embodiment, the variant includes an amino acid sequence that is the translation of a nucleic acid sequence differing by a single nucleotide from a NOVX nucleic acid sequence. In another embodiment, the NOVX polypeptide is a variant polypeptide described therein, wherein any amino acid specified in the chosen sequence is changed to provide a conservative substitution.

In another aspect, invention provides a method for determining the presence or amount of the NOVX polypeptide in a sample by providing a sample; introducing the sample to an antibody that binds immunospecifically to the polypeptide; and determining the presence or amount of antibody bound to the NOVX polypeptide, thereby determining the presence or amount of the NOVX polypeptide in the sample.

In yet another aspect, the invention includes a method for determining the presence of or predisposition to a disease associated with altered levels of a NOVX polypeptide in a mammalian subject by measuring the level of expression of the polypeptide in a sample from the first mammalian subject; and comparing the amount of the polypeptide in the sample of the first step to the amount of the polypeptide present in a control sample from a second mammalian subject known not to have, or not to be predisposed to, the disease. An alteration in the expression level of the polypeptide in the first subject as compared to the control sample indicates the presence of or predisposition to the disease.

In another aspect, the invention includes pharmaceutical compositions that include therapeutically- or prophylactically-effective amounts of a therapeutic and a pharmaceutically-acceptable carrier. The therapeutic can be, e.g., a NOVX nucleic acid, a NOVX polypeptide, or an antibody specific for a NOVX polypeptide. In a further aspect, the invention includes, in one or more containers, a therapeutically- or prophylactically-effective amount of this pharmaceutical composition.

In still another aspect, the invention provides the use of a therapeutic in the manufacture of a medicament for treating a syndrome associated with a human disease that is associated with a NOVX polypeptide.

In a further aspect, the invention provides a method for modulating the activity of a NOVX polypeptide by contacting a cell sample expressing the NOVX polypeptide with antibody that binds the NOVX polypeptide in an amount sufficient to modulate the activity of the polypeptide.

The invention also includes an isolated nucleic acid that encodes a NOVX polypeptide, or a fragment, homolog, analog or derivative thereof. In a preferred embodiment, the nucleic acid molecule comprises the nucleotide sequence of a naturally occurring allelic nucleic acid variant. In another embodiment, the nucleic acid encodes a variant polypeptide, wherein the variant polypeptide has the polypeptide sequence of a naturally occurring polypeptide variant. In another embodiment, the nucleic acid molecule differs by a single nucleotide from a NOVX nucleic acid sequence. In one embodiment, the NOVX nucleic acid molecule hybridizes under stringent conditions to the nucleotide sequence selected from the group consisting of SEQ ID NO: 2n-1, wherein n is an integer between 1 and 73, or a complement of the nucleotide sequence. In one embodiment, the invention provides a nucleic acid molecule wherein the nucleic acid includes the nucleotide sequence of a naturally occurring allelic nucleic acid variant.

Also included in the invention is a vector containing one or more of the nucleic acids described herein, and a cell containing the vectors or nucleic acids described herein. The invention is also directed to host cells transformed with a vector comprising any of the nucleic acid molecules described above.

In yet another aspect, the invention provides for a method for determining the presence or amount of a nucleic acid molecule in a sample by contacting a sample with a probe that binds a NOVX nucleic acid and determining the amount of the probe that is bound to the NOVX nucleic acid. For example the NOVX nucleic may be a marker for cell or tissue type such as a cell or tissue type that is cancerous.

In yet a further aspect, the invention provides a method for determining the presence of or predisposition to a disease associated with altered levels of a nucleic acid molecule in a first mammalian subject, wherein an alteration in the level of the nucleic acid in the first subject as compared to the control sample indicates the presence of or predisposition to the disease.

The invention further provides an antibody that binds immunospecifically to a NOVX polypeptide. The NOVX antibody may be monoclonal, humanized, or a fully human antibody. Preferably, the antibody has a dissociation constant for the binding of the NOVX polypeptide to the antibody less than 1 x 10⁻⁹ M. More preferably, the NOVX antibody neutralizes the activity of the NOVX polypeptide.

In a further aspect, the invention provides for the use of a therapeutic in the manufacture of a medicament for treating a syndrome associated with a human disease, associated with a NOVX polypeptide. Preferably the therapeutic is a NOVX antibody.

In yet a further aspect, the invention provides a method of treating or preventing a NOVX-associated disorder, a method of treating a pathological state in a mammal, and a method of treating or preventing a pathology associated with a polypeptide by administering a NOVX antibody to a subject in an amount sufficient to treat or prevent the disorder.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and are not intended to be limiting.

Other features and advantages of the invention will be apparent from the following detailed description and claims.

### DETAILED DESCRIPTION OF THE INVENTION

The present invention provides novel nucleotides and polypeptides encoded thereby. Included in the invention are the novel nucleic acid sequences, their encoded polypeptides, antibodies, and other related compunds. The sequences are collectively referred to herein as "NOVX nucleic acids" or "NOVX polynucleotides" and the corresponding encoded polypeptides are referred to as "NOVX polypeptides" or "NOVX proteins." Unless indicated otherwise, "NOVX" is meant to refer to any of the novel sequences disclosed herein. Table 1 provides a summary of the NOVX nucleic acids and their encoded polypeptides.

**TABLE 1. NOVX Polynucleotide and Polypeptide Sequences and Corresponding SEQ ID Numbers**

| | **Internal Identification** | **SEQ ID NO (nucleic acid)** | **SEQ ID NO (polypeptide)** | **Homology** |
|---|---|---|---|---|
| 1a | CG100653-01 | 1 | 2 | Cadherin Associated Protein-like |
| 2a | CG100689-01 | 3 | 4 | Leucine Rich Repeat-like |
| 3a | CG100760-01 | 5 | 6 | Leucine Rich Repeat-like |
| 4a | CG100851-02 | 7 | 8 | Leukocyte Surface Antigen CD53-like |
| 5a | CG101068-01 | 9 | 10 | Claudin-9-like |
| 6a | CG101231-01 | 11 | 12 | Integral Membrane Protein Isoform 2-like |
| 6b | CG101231-02 | 13 | 14 | Integral Membrane Protein Isoform 2-like |
| 7a | CG101362-01 | 15 | 16 | Prion Protein-like |
| 8a | CG101458-01 | 17 | 18 | Von Willebrand Domain Containing Protein-like |
| 9a | CG101475-01 | 19 | 20 | Plasma Membrane Protein-like |
| 9b | CG101475-02 | 21 | 22 | Plasma Membrane Protein-like |
| 10a | CG101772-01 | 23 | 24 | XAGE-like |
| 11a | CG102532-01 | 25 | 26 | Emerin-like |
| 12a | CG102575-01 | 27 | 28 | ATPase-like |
| 12b | CG102575-02 | 29 | 30 | ATPase-like |
| 13a | CG102615-01 | 31 | 32 | Mat8 (Mammary Tumor 8 kDa) Protein-like |
| 13b | CG102615-04 | 33 | 34 | Mat8 (Mammary Tumor 8 kDa) Protein-like |
| 14a | CG102646-01 | 35 | 36 | High Affinity Proline Permease-like |
| 15a | CG102878-01 | 37 | 38 | Transmembrane-like |
| 15b | CG102878-02 | 39 | 40 | Transmembrane-like |
| 16a | CG103459-01 | 41 | 42 | Peptide/Histidine Transporter-like |
| 17a | CG104210-01 | 43 | 44 | Type III Membrane Protein-like |
| 17b | CG104210-02 | 45 | 46 | Type III Membrane Protein-like |
| 17c | 272249075 | 47 | 48 | Type III Membrane Protein-like |
| 18a | CG104251-01 | 49 | 50 | Type III Membrane Protein-like |
| 19a | CG104934-01 | 51 | 52 | Phospholipid-Transporting ATPase IH-like |
| 20a | CG105463-01 | 53 | 54 | Meningioma-Expressed Antigen 6/11 (MEA6) (MEA11)-like |
| 20b | CG105463-02 | 55 | 56 | Meningioma-Expressed Antigen 6/11 (MEA6) (MEA 11)-like |
| 21a | CG105491-01 | 57 | 58 | Serine Protease-like |
| 22a | CG105954-01 | 59 | 60 | Neurofascin Precursor-like |
| 23a | CG105963-01 | 61 | 62 | Cadherin-like |
| 24a | CG105973-01 | 63 | 64 | Integrin Alpha 8-like |
| 24b | CG105973-02 | 65 | 66 | Integrin Alpha 8-like |
| 25a | CG106915-01 | 67 | 68 | Nogo Receptor Isoform-1-like |
| 26a | CG106924-01 | 69 | 70 | Nogo Receptor Isoform-2-like |
| 26b | 21062144 | 71 | 72 | Nogo Receptor Isoform-2-like |
| 27a | CG106942-01 | 73 | 74 | NRAMP-like Membrane Protein |
| 28a | CG107513-01 | 75 | 76 | Syntaxin Domain Containing Protein-like |
| 29a | CG107533-02 | 77 | 78 | Tumor Necrosis Factor-like |
| 30a | CG107562-01 | 79 | 80 | Leucine-Rich Repeat Type III |
| | | | | Transmembrane-like |
| 30b | CG107562-02 | 81 | 82 | Leucine-Rich Repeat Type III Transmembrane-like |
| 30c | 210086373 | 83 | 84 | Leucine-Rich Repeat Type III Transmembrane-like |
| 30d | 210086403 | 85 | 86 | Leucine-Rich Repeat Type III Transmembrane-like |
| 30e | 210086422 | 87 | 88 | Leucine-Rich Repeat Type III Transmembrane-like |
| 31a | CG108184-01 | 89 | 90 | Transmembrane Protein Tm7-like |
| 31b | CG108184-02 | 91 | 92 | Transmembrane Protein Tm7-like |
| 31c | CG108184-03 | 93 | 94 | Transmembrane Protein Tm7-like |
| 32a | CG108238-01 | 95 | 96 | Sialic Acid Binding Immunoglobulin-like |
| 33a | CG108695-01 | 97 | 98 | OB binding protein (SIGLEC)-like |
| 34a | CG109505-01 | 99 | 100 | Aldehyde Dehydrogenase-like |
| 35a | CG109742-01 | 101 | 102 | Latent Transforming Growth Factor Beta Binding Protein 3-like |
| 35b | 207639410 | 103 | 104 | Latent Transforming Growth Factor Beta Binding Protein 3-like |
| 35c | 207639427 | 105 | 106 | Latent Transforming Growth Factor Beta Binding Protein 3-like |
| 35d | 207639438 | 107 | 108 | Latent Transforming Growth Factor Beta Binding Protein 3-like |
| 35e | 207639448 | 109 | 110 | Latent Transforming Growth Factor Beta Binding Protein 3-like |
| 36a | CG109844-01 | 111 | 112 | C4B-Binding Protein-like |
| 37a | CG110014-02 | 113 | 114 | Colon Carcinoma kinase 4-like |
| 37b | CG110014-03 | 115 | 116 | Colon Carcinoma kinase 4-like |
| 37c | CG110014-04 | 117 | 118 | Colon Carcinoma kinase 4-like |
| 38a | CG110187-01 | 119 | 120 | Alpha C1-like Protocadherin |
| 38b | CG110187-03 | 121 | 122 | Alpha C1-like Protocadherin |
| 39a | CG110205-01 | 123 | 124 | Disintegrin-like / Metalloprotease (Reprolysin Type) with Thrombospondin Type I Motif-like |
| 39b | CG110205-02 | 125 | 126 | Disintegrin-like / Metalloprotease (Reprolysin Type) with Thrombospondin Type I Motif-like |
| 39c | 207756942 | 127 | 128 | Disintegrin-like / Metalloprotease (Reprolysin Type) with Thrombospondin Type I Motif-like |
| 39d | 207756946 | 129 | 130 | Disintegrin-like / Metalloprotease (Reprolysin Type) with Thrombospondin Type I Motif-like |
| 39e | 207756950 | 131 | 132 | Disintegrin-like / Metalloprotease (Reprolysin Type) with Thrombospondin Type I Motif-like |
| 39f | 207756966 | 133 | 134 | Disintegrin-like / Metalloprotease (Reprolysin Type) with Thrombospondin Type I Motif-like |
| 40a | CG110242-01 | 135 | 136 | Ebnerin-like |
| 40b | 207728344 | 137 | 138 | Ebnerin-like |
| 40c | 207728348 | 139 | 140 | Ebnerin-like |
| 40d | 207728354 | 141 | 142 | Ebnerin-like |
| 40e | 207728365 | 143 | 144 | Ebnerin-like |
| 41a | CG99598-01 | 145 | 146 | Endosomal Glycoprotein Precursor-like |

Table 1 indicates the homology of NOVX polypeptides to known protein families. Thus, the nucleic acids and polypeptides, antibodies and related compounds according to the invention corresponding to a NOVX as identified in column 1 of Table 1 will be useful in therapeutic and diagnostic applications implicated in, for example, pathologies and disorders associated with the known protein families identified in column 5 of Table 1.

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

Consistent with other known members of the family of proteins, identified in column 5 of Table 1, the NOVX polypeptides of the present invention show homology to, and contain domains that are characteristic of, other members of such protein families. Details of the sequence relatedness and domain analysis for each NOVX are presented in Example A.

The NOVX nucleic acids and polypeptides can also be used to screen for molecules, which inhibit or enhance NOVX activity or function. Specifically, the nucleic acids and polypeptides according to the invention may be used as targets for the identification of small molecules that modulate or inhibit diseases associated with the protein families listed in Table 1.

The NOVX nucleic acids and polypeptides are also useful for detecting specific cell types. Details of the expression analysis for each NOVX are presented in Example C. Accordingly, the NOVX nucleic acids, polypeptides, antibodies and related compounds according to the invention will have diagnostic and therapeutic applications in the detection of a variety of diseases with differential expression in normal vs. diseased tissues, e.g. detection of a variety of cancers.

Additional utilities for NOVX nucleic acids and polypeptides according to the invention are disclosed herein.

### NOVX clones

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

The NOVX genes and their corresponding encoded proteins are useful for preventing, treating or ameliorating medical conditions, e.g., by protein or gene therapy. Pathological conditions can be diagnosed by determining the amount of the new protein in a sample or by determining the presence of mutations in the new genes. Specific uses are described for each of the NOVX genes, based on the tissues in which they are most highly expressed. Uses include developing products for the diagnosis or treatment of a variety of diseases and disorders.

The NOVX nucleic acids and proteins of the invention are useful in potential diagnostic and therapeutic applications and as research tools. These include serving as a specific or selective nucleic acid or protein diagnostic and/or prognostic marker, wherein the presence or amount of the nucleic acid or the protein are to be assessed, as well as potential therapeutic applications such as the following: (i) a protein therapeutic, (ii) a small molecule drug target, (iii) an antibody target (therapeutic, diagnostic, drug targeting/cytotoxic antibody), (iv) a nucleic acid useful in gene therapy (gene delivery/gene ablation), and (v) a composition promoting tissue regeneration *in vitro* and *in vivo* (vi) a biological defense weapon.

In one specific embodiment, the invention includes an isolated polypeptide comprising an amino acid sequence selected from the group consisting of: (a) a mature form of the amino acid sequence selected from the group consisting of SEQ ID NO: 2n, wherein n is an integer between 1 and 73; (b) a variant of a mature form of the amino acid sequence selected from the group consisting of SEQ ID NO: 2n, wherein n is an integer between 1 and 73, wherein any amino acid in the mature form is changed to a different amino acid, provided that no more than 15% of the amino acid residues in the sequence of the mature form are so changed; (c) an amino acid sequence selected from the group consisting of SEQ ID NO: 2n, wherein n is an integer between 1 and 73; (d) a variant of the amino acid sequence selected from the group consisting of SEQ ID NO:2n, wherein n is an integer between 1 and 73 wherein any amino acid specified in the chosen sequence is changed to a different amino acid, provided that no more than 15% of the amino acid residues in the sequence are so changed; and (e) a fragment of any of (a) through (d).

In another specific embodiment, the invention includes an isolated nucleic acid molecule comprising a nucleic acid sequence encoding a polypeptide comprising an amino acid sequence selected from the group consisting of: (a) a mature form of the amino acid sequence given SEQ ID NO: 2n, wherein n is an integer between 1 and 73; (b) a variant of a mature form of the amino acid sequence selected from the group consisting of SEQ ID NO: 2n, wherein n is an integer between 1 and 73 wherein any amino acid in the mature form of the chosen sequence is changed to a different amino acid, provided that no more than 15% of the amino acid residues in the sequence of the mature form are so changed; (c) the amino acid sequence selected from the group consisting of SEQ ID NO: 2n, wherein n is an integer between 1 and 73; (d) a variant of the amino acid sequence selected from the group consisting of SEQ ID NO: 2n, wherein n is an integer between 1 and 73, in which any amino acid specified in the chosen sequence is changed to a different amino acid, provided that no more than 15% of the amino acid residues in the sequence are so changed; (e) a nucleic acid fragment encoding at least a portion of a polypeptide comprising the amino acid sequence selected from the group consisting of SEQ ID NO: 2n, wherein n is an integer between 1 and 73 or any variant of said polypeptide wherein any amino acid of the chosen sequence is changed to a different amino acid, provided that no more than 10% of the amino acid residues in the sequence are so changed; and (f) the complement of any of said nucleic acid molecules.

In yet another specific embodiment, the invention includes an isolated nucleic acid molecule, wherein said nucleic acid molecule comprises a nucleotide sequence selected from the group consisting of: (a) the nucleotide sequence selected from the group consisting of SEQ ID NO: 2n-1, wherein n is an integer between 1 and 73; (b) a nucleotide sequence wherein one or more nucleotides in the nucleotide sequence selected from the group consisting of SEQ ID NO: 2n-1, wherein n is an integer between 1 and 73 is changed from that selected from the group consisting of the chosen sequence to a different nucleotide provided that no more than 15% of the nucleotides are so changed; (c) a nucleic acid fragment of the sequence selected from the group consisting of SEQ ID NO: 2n-1, wherein n is an integer between 1 and 73; and (d) a nucleic acid fragment wherein one or more nucleotides in the nucleotide sequence selected from the group consisting of SEQ ID NO: 2n-1, wherein n is an integer between 1 and 73 is changed from that selected from the group consisting of the chosen sequence to a different nucleotide provided that no more than 15% of the nucleotides are so changed.

### NOVX Nucleic Acids and Polypeptides

One aspect of the invention pertains to isolated nucleic acid molecules that encode NOVX polypeptides or biologically active portions thereof. Also included in the invention are nucleic acid fragments sufficient for use as hybridization probes to identify NOVX-encoding nucleic acids (*e*.*g*., NOVX mRNA's) and fragments for use as PCR primers for the amplification and/or mutation of NOVX nucleic acid molecules. As used herein, the term "nucleic acid molecule" is intended to include DNA molecules (e.g., cDNA or genomic DNA), RNA molecules (e.g., mRNA), analogs of the DNA or RNA generated using nucleotide analogs, and derivatives, fragments and homologs thereof. The nucleic acid molecule may be single-stranded or double-stranded, but preferably is comprised double-stranded DNA.

A NOVX nucleic acid can encode a mature NOVX polypeptide. As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full-length gene product encoded by the corresponding gene. Alternatively, it may be defined as the polypeptide, precursor or proprotein encoded by an ORF described herein. The product "mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an ORF, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristylation or phosphorylation. In general, a mature polypeptide or protein may result from the operation of only one of these processes, or a combination of any of them.

The term "probes", as utilized herein, refers to nucleic acid sequences of variable length, preferably between at least about 10 nucleotides (nt), 100 nt, or as many as approximately, e.g., 6,000 nt, depending upon the specific use. Probes are used in the detection of identical, similar, or complementary nucleic acid sequences. Longer length probes are generally obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies.

The term "isolated" nucleic acid molecule, as utilized herein, is one, which is separated from other nucleic acid molecules which are present in the natural source of the nucleic acid. Preferably, an "isolated" nucleic acid is free of sequences which naturally flank the nucleic acid (i.e., sequences located at the 5'- and 3'-termini of the nucleic acid) in the genomic DNA of the organism from which the nucleic acid is derived. For example, in various embodiments, the isolated NOVX nucleic acid molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (e.g., brain, heart, liver, spleen, etc.). Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the invention, e.g., a nucleic acid molecule having the nucleotide sequence of SEQ ID NO:2*n*-1, wherein n is an integer between 1-73, or a complement of this aforementioned nucleotide sequence, can be isolated using standard molecular biology techniques and the sequence information provided herein. Using all or a portion of the nucleic acid sequence of SEQ ID NO:2*n*-1, wherein n is an integer between 1-73, as a hybridization probe, NOVX molecules can be isolated using standard hybridization and cloning techniques (e.g., as described in Sambrook, *et al*., (eds.), MOLECULAR CLONING: A LABORATORY MANUAL 2^{nd} Ed., Cold Spring Harbor Laboratory Press, Cold Spring Harbor, NY, 1989; and Ausubel, *et al*., (eds.), CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, e.g., using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides of SEQ ID NO:2*n*-1, wherein n is an integer between 1-73, or a complement thereof. Oligonucleotides may be chemically synthesized and may also be used as probes.

In another embodiment, an isolated nucleic acid molecule of the invention comprises a nucleic acid molecule that is a complement of the nucleotide sequence SEQ ID NO:2*n*-1, wherein n is an integer between 1-73, or a portion of this nucleotide sequence (e.g., a fragment that can be used as a probe or primer or a fragment encoding a biologically-active portion of a NOVX polypeptide). A nucleic acid molecule that is complementary to the nucleotide sequence of SEQ ID NO:2*n*-1, wherein n is an integer between 1-73, is one that is sufficiently complementary to the nucleotide sequence of SEQ ID NO:2*n*-1, wherein *n* is an integer between 1-73, that it can hydrogen bond with little or no mismatches to the nucleotide sequence of SEQ ID NO:2*n*-1, wherein n is an integer between 1-73, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

A full-length NOVX clone is identified as containing an ATG translation start codon and an in-frame stop codon. Any disclosed NOVX nucleotide sequence lacking an ATG start codon therefore encodes a truncated C-terminal fragment of the respective NOVX polypeptide, and requires that the corresponding full-length cDNA extend in the 5' direction of the disclosed sequence. Any disclosed NOVX nucleotide sequence lacking an in-frame stop codon similarly encodes a truncated N-terminal fragment of the respective NOVX polypeptide, and requires that the corresponding full-length cDNA extend in the 3' direction of the disclosed sequence.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. *See e.g.* Ausubel, *et al*., CURRENT PROTOCOLS IN MOLECULAR BIOLOGY, John Wiley & Sons, New York, NY, 1993, and below.

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of NOVX polypeptides. Isoforms can be expressed in different tissues of the same organism as a result of, for example, alternative splicing of RNA. Alternatively, isoforms can be encoded by different genes. In the invention, homologous nucleotide sequences include nucleotide sequences encoding for a NOVX polypeptide of species other than humans, including, but not limited to: vertebrates, and thus can include, e.g., frog, mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human NOVX protein. Homologous nucleic acid sequences include those nucleic acid sequences that encode conservative amino acid substitutions (see below) in SEQ ID NO:2*n*-1, wherein n is an integer between 1-73, as well as a polypeptide possessing NOVX biological activity. Various biological activities of the NOVX proteins are described below.

A NOVX polypeptide is encoded by the open reading frame ("ORF") of a NOVX nucleic acid. An ORF corresponds to a nucleotide sequence that could potentially be translated into a polypeptide. A stretch of nucleic acids comprising an ORF is uninterrupted by a stop codon. An ORF that represents the coding sequence for a full protein begins with an ATG "start" codon and terminates with one of the three "stop" codons, namely, TAA, TAG, or TGA. For the purposes of this invention, an ORF may be any part of a coding sequence, with or without a start codon, a stop codon, or both. For an ORF to be considered as a good candidate for coding for a *bona fide* cellular protein, a minimum size requirement is often set, e.g., a stretch of DNA that would encode a protein of 50 amino acids or more.

The nucleotide sequences determined from the cloning of the human NOVX genes allows for the generation of probes and primers designed for use in identifying and/or cloning NOVX homologues in other cell types, e.g. from other tissues, as well as NOVX homologues from other vertebrates. The probe/primer typically comprises substantially purified oligonucleotide. The oligonucleotide typically comprises a region of nucleotide sequence that hybridizes under stringent conditions to at least about 12, 25, 50, 100, 150, 200, 250, 300, 350 or 400 consecutive sense strand nucleotide sequence of SEQ ID NO:2*n-*1, wherein *n* is an integer between 1-73; or an anti-sense strand nucleotide sequence of SEQ ID NO:2*n*-1, wherein n is an integer between 1-73; or of a naturally occurring mutant of SEQ ID NO:2*n*-1, wherein n is an integer between 1-73.

Probes based on the human NOVX nucleotide sequences can be used to detect transcripts or genomic sequences encoding the same or homologous proteins. In various embodiments, the probe further comprises a label group attached thereto, e.g. the label group can be a radioisotope, a fluorescent compound, an enzyme, or an enzyme co-factor. Such probes can be used as a part of a diagnostic test kit for identifying cells or tissues which mis-express a NOVX protein, such as by measuring a level of a NOVX-encoding nucleic acid in a sample of cells from a subject e.g., detecting NOVX mRNA levels or determining whether a genomic NOVX gene has been mutated or deleted.

"A polypeptide having a biologically-active portion of a NOVX polypeptide" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of NOVX" can be prepared by isolating a portion of SEQ ID NO:2*n*-1, wherein n is an integer between 1-73, that encodes a polypeptide having a NOVX biological activity (the biological activities of the NOVX proteins are described below), expressing the encoded portion of NOVX protein (e.g., by recombinant expression *in vitro)* and assessing the activity of the encoded portion of NOVX.

### NOVX Nucleic Acid and Polypeptide Variants

The invention further encompasses nucleic acid molecules that differ from the nucleotide sequences of SEQ ID NO:2*n*-1, wherein n is an integer between 1-73, due to degeneracy of the genetic code and thus encode the same NOVX proteins as that encoded by the nucleotide sequences of SEQ ID NO:2*n*-1, wherein n is an integer between 1-73. In another embodiment, an isolated nucleic acid molecule of the invention has a nucleotide sequence encoding a protein having an amino acid sequence of SEQ ID NO:2*n*, wherein n is an integer between 1-73.

In addition to the human NOVX nucleotide sequences of SEQ ID NO:2*n*-1, wherein n is an integer between 1-73, it will be appreciated by those skilled in the art that DNA sequence polymorphisms that lead to changes in the amino acid sequences of the NOVX polypeptides may exist within a population (e.g., the human population). Such genetic polymorphism in the NOVX genes may exist among individuals within a population due to natural allelic variation. As used herein, the terms "gene" and "recombinant gene" refer to nucleic acid molecules comprising an open reading frame (ORF) encoding a NOVX protein, preferably a vertebrate NOVX protein. Such natural allelic variations can typically result in 1-5% variance in the nucleotide sequence of the NOVX genes. Any and all such nucleotide variations and resulting amino acid polymorphisms in the NOVX polypeptides, which are the result of natural allelic variation and that do not alter the functional activity of the NOVX polypeptides, are intended to be within the scope of the invention.

Moreover, nucleic acid molecules encoding NOVX proteins from other species, and thus that have a nucleotide sequence that differs from any one of the human SEQ ID NO:2*n*-1, wherein n is an integer between 1-73, are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions.

Accordingly, in another embodiment, an isolated nucleic acid molecule of the invention is at least 6 nucleotides in length and hybridizes under stringent conditions to the nucleic acid molecule comprising the nucleotide sequence of SEQ ID NO:2*n*-1, wherein *n* is an integer between 1-73. In another embodiment, the nucleic acid is at least 10, 25, 50, 100, 250, 500, 750, 1000, 1500, or 2000 or more nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (i.e., nucleic acids encoding NOVX proteins derived from species other than human) or other related sequences (e.g., paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5 °C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30 °C for short probes, primers or oligonucleotides (e.g., 10 nt to 50 nt) and at least about 60 °C for longer probes, primers and oligonucleotides. Stringent conditions may also be achieved with the addition of destabilizing agents, such as formamide.

Stringent conditions are known to those skilled in the art and can be found in Ausubel, *et al*., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65 °C, followed by one or more washes in 0.2X SSC, 0.01% BSA at 50 °C. An isolated nucleic acid molecule of the invention that hybridizes under stringent conditions to any one of the sequences of SEQ ID NO:2*n*-1, wherein n is an integer between 1-73, corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

In a second embodiment, a nucleic acid sequence that is hybridizable to the nucleic acid molecule comprising the nucleotide sequence of SEQ ID NO:2*n*-1, wherein n is an integer between 1-73, or fragments, analogs or derivatives thereof, under conditions of moderate stringency is provided. A non-limiting example of moderate stringency hybridization conditions are hybridization in 6X SSC, 5X Reinhardt's solution, 0.5% SDS and 100 mg/ml denatured salmon sperm DNA at 55 °C, followed by one or more washes in 1X SSC, 0.1% SDS at 37 °C. Other conditions of moderate stringency that may be used are well-known within the art. *See, e.g.,* Ausubel, *et al*. (eds.), 1993, CURRENT PROTOCOLS IN MOLECULAR BIOLOGY, John Wiley & Sons, NY, and Krieger, 1990; GENE TRANSFER AND EXPRESSION, A LABORATORY MANUAL, Stockton Press, NY.

In a third embodiment, a nucleic acid that is hybridizable to the nucleic acid molecule comprising the nucleotide sequences of SEQ ID NO:2*n*-1, wherein *n* is an integer between 1-73, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/volt) dextran sulfate at 40 °C, followed by one or more washes in 2X SSC, 25 mM Tris-HCl (pH 7.4), 5 mM EDTA, and 0.1% SDS at 50 °C. Other conditions of low stringency that may be used are well known in the art (e.g., as employed for cross-species hybridizations). *See, e.g.,* Ausubel, *et al*. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. *Proc Natl Acad Sci USA* **78:** 6789-6792.

### Conservative Mutations

In addition to naturally-occurring allelic variants of NOVX sequences that may exist in the population, the skilled artisan will further appreciate that changes can be introduced by mutation into the nucleotide sequences of SEQ ID NO:2*n*-1, wherein n is an integer between 1-73, thereby leading to changes in the amino acid sequences of the encoded NOVX proteins, without altering the functional ability of said NOVX proteins. For example, nucleotide substitutions leading to amino acid substitutions at "non-essential" amino acid residues can be made in the sequence of SEQ ID NO:2*n*, wherein n is an integer between 1-73. A "non-essential" amino acid residue is a residue that can be altered from the wild-type sequences of the NOVX proteins without altering their biological activity, whereas an "essential" amino acid residue is required for such biological activity. For example, amino acid residues that are conserved among the NOVX proteins of the invention are particularly non-amenable to alteration. Amino acids for which conservative substitutions can be made are well-known within the art.

Another aspect of the invention pertains to nucleic acid molecules encoding NOVX proteins that contain changes in amino acid residues that are not essential for activity. Such NOVX proteins differ in amino acid sequence from any one of SEQ ID NO:2*n*-1, wherein n is an integer between 1-73, yet retain biological activity. In one embodiment, the isolated nucleic acid molecule comprises a nucleotide sequence encoding a protein, wherein the protein comprises an amino acid sequence at least about 45% homologous to the amino acid sequences of SEQ ID NO:2*n*, wherein n is an integer between 1-73. Preferably, the protein encoded by the nucleic acid molecule is at least about 60% homologous to SEQ ID NO:2*n*, wherein n is an integer between 1-73; more preferably at least about 70% homologous to SEQ ID NO:2*n*, wherein n is an integer between 1-73; still more preferably at least about 80% homologous to SEQ ID NO:2*n*, wherein n is an integer between 1-73; even more preferably at least about 90% homologous to SEQ ID NO:2*n*, wherein n is an integer between 1-73; and most preferably at least about 95% homologous to SEQ ID NO:2*n*, wherein n is an integer between 1-73.

An isolated nucleic acid molecule encoding a NOVX protein homologous to the protein of SEQ ID NO:2*n*, wherein n is an integer between 1-73, can be created by introducing one or more nucleotide substitutions, additions or deletions into the nucleotide sequence of SEQ ID NO:2*n*-1, wherein n is an integer between 1-73, such that one or more amino acid substitutions, additions or deletions are introduced into the encoded protein.

Mutations can be introduced into any of SEQ ID NO:2*n*-1, wherein n is an integer between 1-73, by standard techniques, such as site-directed mutagenesis and PCR-mediated mutagenesis. Preferably, conservative amino acid substitutions are made at one or more predicted, non-essential amino acid residues. A "conservative amino acid substitution" is one in which the amino acid residue is replaced with an amino acid residue having a similar side chain. Families of amino acid residues having similar side chains have been defined within the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted non-essential amino acid residue in the NOVX protein is replaced with another amino acid residue from the same side chain family. Alternatively, in another embodiment, mutations can be introduced randomly along all or part of a NOVX coding sequence, such as by saturation mutagenesis, and the resultant mutants can be screened for NOVX biological activity to identify mutants that retain activity. Following mutagenesis of any one of SEQ ID NO:2*n*-1, wherein n is an integer between 1-73, the encoded protein can be expressed by any recombinant technology known in the art and the activity of the protein can be determined.

The relatedness of amino acid families may also be determined based on side chain interactions. Substituted amino acids may be fully conserved "strong" residues or fully conserved "weak" residues. The "strong" group of conserved amino acid residues may be any one of the following groups: STA, NEQK, NHQK, NDEQ, QHRK, MILV, MILF, HY, FYW, wherein the single letter amino acid codes are grouped by those amino acids that may be substituted for each other. Likewise, the "weak" group of conserved residues may be any one of the following: CSA, ATV, SAG, STNK, STPA, SGND, SNDEQK, NDEQHK, NEQHRK, HFY, wherein the letters within each group represent the single letter amino acid code.

In one embodiment, a mutant NOVX protein can be assayed for (*i*) the ability to form protein:protein interactions with other NOVX proteins, other cell-surface proteins, or biologically-active portions thereof, (*ii*) complex formation between a mutant NOVX protein and a NOVX ligand; or *(iii)* the ability of a mutant NOVX protein to bind to an intracellular target protein or biologically-active portion thereof; (e.g. avidin proteins).

In yet another embodiment, a mutant NOVX protein can be assayed for the ability to regulate a specific biological function (e.g., regulation of insulin release).

### Antisense Nucleic Acids

Another aspect of the invention pertains to isolated antisense nucleic acid molecules that are hybridizable to or complementary to the nucleic acid molecule comprising the nucleotide sequence of SEQ ID NO:2*n*-1, wherein n is an integer between 1-73, or fragments, analogs or derivatives thereof. An "antisense" nucleic acid comprises a nucleotide sequence that is complementary to a "sense" nucleic acid encoding a protein (e.g., complementary to the coding strand of a double-stranded cDNA molecule or complementary to an mRNA sequence). In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of a NOVX protein of SEQ ID NO:2*n*, wherein *n* is an integer between 1-73, or antisense nucleic acids complementary to a NOVX nucleic acid sequence of SEQ ID NO:2*n*-1, wherein n is an integer between 1-73, are additionally provided.

In one embodiment, an antisense nucleic acid molecule is antisense to a "coding region" of the coding strand of a nucleotide sequence encoding a NOVX protein. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding the NOVX protein. The term "noncoding region" refers to 5' and 3' sequences which flank the coding region that are not translated into amino acids (i.e., also referred to as 5' and 3' untranslated regions).

Given the coding strand sequences encoding the NOVX protein disclosed herein, antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site of NOVX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (e.g., an antisense oligonucleotide) can be chemically synthesized using naturally-occurring nucleotides or variously modified nucleotides designed to increase the biological stability of the molecules or to increase the physical stability of the duplex formed between the antisense and sense nucleic acids (*e*.*g*., phosphorothioate derivatives and acridine substituted nucleotides can be used).

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i*.*e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a NOVX protein to thereby inhibit expression of the protein (e.g., by inhibiting transcription and/or translation). The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (e.g., by linking the antisense nucleic acid molecules to peptides or antibodies that bind to cell surface receptors or antigens). The antisense nucleic acid molecules can also be delivered to cells using the vectors described herein. To achieve sufficient nucleic acid molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other. *See, e.g.,* Gaultier, *et al*., 1987. *Nucl. Acids Res.* **15**: 6625-6641. The antisense nucleic acid molecule can also comprise a 2'-o-methylribonucleotide (*See, e.g.,* Inoue, *et al*. 1987. *Nucl. Acids Res.* **15:** 6131-6148) or a chimeric RNA-DNA analogue (*See, e.g.,* Inoue, *et al*., 1987. *FEBS Lett.* **215:** 327-330.

### Ribozymes and PNA Moieties

Nucleic acid modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In one embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (e.g., hammerhead ribozymes as described in Haselhoff and Gerlach 1988. *Nature* 334: 585-591) can be used to catalytically cleave NOVX mRNA transcripts to thereby inhibit translation of NOVX mRNA. A ribozyme having specificity for a NOVX-encoding nucleic acid can be designed based upon the nucleotide sequence of a NOVX cDNA disclosed herein *(i.e.,* any one of SEQ ID NO:2*n*-1, wherein *n* is an integer between 1-73). For example, a derivative of a *Tetrahymena* L-19 IVS RNA can be constructed in which the nucleotide sequence of the active site is complementary to the nucleotide sequence to be cleaved in a NOVX-encoding mRNA. *See, e.g.,* U.S. Patent 4,987,071 to Cech, *et al*. and U.S. Patent 5,116,742 to *Cech, et al.* NOVX mRNA can also be used to select a catalytic RNA having a specific ribonuclease activity from a pool of RNA molecules. *See, e.g.,* Bartel *et al.,* (1993) *Science* 261:1411-1418.

Alternatively, NOVX gene expression can be inhibited by targeting nucleotide sequences complementary to the regulatory region of the NOVX nucleic acid (e.g., the NOVX promoter and/or enhancers) to form triple helical structures that prevent transcription of the NOVX gene in target cells. *See, e.g.,* Helene, 1991. *Anticancer Drug Des.* 6: 569-84; Helene, *et al*. 1992. *Ann. N. Y. Acad. Sci.* 660: 27-36; Maher, 1992. *Bioassays* 14: 807-15.

In various embodiments, the NOVX nucleic acids can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids. *See, e.g.,* Hyrup, *et al*., 1996. *Bioorg Med Chem* 4: 5-23. As used herein, the terms "peptide nucleic acids" or "PNAs" refer to nucleic acid mimics (*e*.*g*., DNA mimics) in which the deoxyribose phosphate backbone is replaced by a pseudopeptide backbone and only the four natural nucleotide bases are retained. The neutral backbone of PNAs has been shown to allow for specific hybridization to DNA and RNA under conditions of low ionic strength. The synthesis of PNA oligomer can be performed using standard solid phase peptide synthesis protocols as described in Hyrup, *et al*., 1996. *supra*; Perry-O'Keefe, *et al*., 1996. *Proc. Natl. Acad. Sci. USA* 93: 14670-14675.

PNAs of NOVX can be used in therapeutic and diagnostic applications. For example, PNAs can be used as antisense or antigene agents for sequence-specific modulation of gene expression by, e.g., inducing transcription or translation arrest or inhibiting replication. PNAs of NOVX can also be used, for example, in the analysis of single base pair mutations in a gene (*e.g.*, PNA directed PCR clamping; as artificial restriction enzymes when used in combination with other enzymes, *e*.*g*., S₁ nucleases *(See,* Hyrup, *et al*., 1996.*supra*); or as probes or primers for DNA sequence and hybridization *(See,* Hyrup, *et al*., 1996, *supra*; Perry-O'Keefe, *et al*., 1996. *supra*).

In another embodiment, PNAs of NOVX can be modified, *e.g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes (e.g., RNase H and DNA polymerases) to interact with the DNA portion while the PNA portion would provide high binding affinity and specificity. PNA-DNA chimeras can be linked using linkers of appropriate lengths selected in terms of base stacking, number of bonds between the nucleotide bases, and orientation *(see,* Hyrup, et al., 1996. *supra*). The synthesis of PNA-DNA chimeras can be performed as described in Hyrup, *et al*., 1996. *supra* and Finn, *et al*., 1996. *Nucl Acids Res* 24: 3357-3363. For example, a DNA chain can be synthesized on a solid support using standard phosphoramidite coupling chemistry, and modified nucleoside analogs, *e.g.*, 5'-(4-methoxytrityl)amino-5'-deoxy-thymidine phosphoramidite, can be used between the PNA and the 5' end of DNA. *See, e.g.,* Mag, *et al*., 1989. *Nucl Acid Res* 17: 5973-5988. PNA monomers are then coupled in a stepwise manner to produce a chimeric molecule with a 5' PNA segment and a 3' DNA segment. *See, e.g.,* Finn, *et al*., 1996. *supra*. Alternatively, chimeric molecules can be synthesized with a 5' DNA segment and a 3' PNA segment. *See, e.g.,* Petersen, *et al*., 1975. *Bioorg. Med. Chem. Lett.* 5: 1119-11124.

In other embodiments, the oligonucleotide may include other appended groups such as peptides (e.g., for targeting host cell receptors *in vivo),* or agents facilitating transport across the cell membrane *(see, e.g.,* Letsinger, *et al*., 1989. *Proc. Natl. Acad. Sci. U.S.A*. 86: 6553-6556; Lemaitre, *et al*., 1987. *Proc. Natl. Acad. Sci.* 84: 648-652; PCT Publication No. WO88/09810) or the blood-brain barrier *(see, e.g.,* PCT Publication No. WO 89/10134). In addition, oligonucleotides can be modified with hybridization triggered cleavage agents *(see, e.g.,* Krol, *et al*., 1988. *BioTechniques* 6:958-976) or intercalating agents (*see, e.g.,* Zon, 1988. *Pharm. Res.* 5: 539-549). To this end, the oligonucleotide may be conjugated to another molecule, *e.g*., a peptide, a hybridization triggered cross-linking agent, a transport agent, a hybridization-triggered cleavage agent, and the like.

### NOVX Polypeptides

A polypeptide according to the invention includes a polypeptide including the amino acid sequence of NOVX polypeptides whose sequences are provided in any one of SEQ ID NO:2*n*, wherein n is an integer between 1-73. The invention also includes a mutant or variant protein any of whose residues may be changed from the corresponding residues shown in any one of SEQ ID NO:2*n*, wherein n is an integer between 1-73, while still encoding a protein that maintains its NOVX activities and physiological functions, or a functional fragment thereof.

In general, a NOVX variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated NOVX proteins, and biologically-active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, a NOVX protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX proteins in which the protein is separated from cellular components of the cells from which it is isolated or recombinantly-produced. In one embodiment, the language "substantially free of cellular material" includes preparations of NOVX proteins having less than about 30% (by dry weight) of non-NOVX proteins (also referred to herein as a "contaminating protein"), more preferably less than about 20% of non-NOVX proteins, still more preferably less than about 10% of non-NOVX proteins, and most preferably less than about 5% of non-NOVX proteins. When the NOVX protein or biologically-active portion thereof is recombinantly-produced, it is also preferably substantially free of culture medium, i.e., culture medium represents less than about 20%, more preferably less than about 10%, and most preferably less than about 5% of the volume of the NOVX protein preparation.

The language "substantially free of chemical precursors or other chemicals" includes preparations of NOVX proteins in which the protein is separated from chemical precursors or other chemicals that are involved in the synthesis of the protein. In one embodiment, the language "substantially free of chemical precursors or other chemicals" includes preparations of NOVX proteins having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically-active portions of NOVX proteins include peptides comprising amino acid sequences sufficiently homologous to or derived from the amino acid sequences of the NOVX proteins (*e*.*g*., the amino acid sequence of SEQ ID NO:2*n*, wherein *n* is an integer between 1-73) that include fewer amino acids than the full-length NOVX proteins, and exhibit at least one activity of a NOVX protein. Typically, biologically-active portions comprise a domain or motif with at least one activity of the NOVX protein. A biologically-active portion of a NOVX protein can be a polypeptide which is, for example, 10, 25, 50, 100 or more amino acid residues in length.

Moreover, other biologically-active portions, in which other regions of the protein are deleted, can be prepared by recombinant techniques and evaluated for one or more of the functional activities of a native NOVX protein.

In an embodiment, the NOVX protein has an amino acid sequence of SEQ ID NO:2*n*, wherein n is an integer between 1-73. In other embodiments, the NOVX protein is substantially homologous to SEQ ID NO:2*n*, wherein n is an integer between 1-73, and retains the functional activity of the protein of SEQ ID NO:2*n*, wherein n is an integer between 1-73, yet differs in amino acid sequence due to natural allelic variation or mutagenesis, as described in detail, below. Accordingly, in another embodiment, the NOVX protein is a protein that comprises an amino acid sequence at least about 45% homologous to the amino acid sequence of SEQ ID NO:2*n*, wherein n is an integer between 1-73, and retains the functional activity of the NOVX proteins of SEQ ID NO:2*n*, wherein n is an integer between 1-73.

### Determining Homology Between Two or More Sequences

To determine the percent homology of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i*.*e*., as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See*, Needleman and Wunsch, 1970. *J Mol Biol* 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence of SEQ ID NO:2*n*-1, wherein n is an integer between 1-73.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (e.g., A, T, C, G, U, or I, in the case of nucleic acids) occurs in both sequences to yield the number of matched positions, dividing the number of matched positions by the total number of positions in the region of comparison (i.e., the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides NOVX chimeric or fusion proteins. As used herein, a NOVX "chimeric protein" or "fusion protein" comprises a NOVX polypeptide operatively-linked to a non-NOVX polypeptide. An "NOVX polypeptide" refers to a polypeptide having an amino acid sequence corresponding to a NOVX protein of SEQ ID NO:2*n*, wherein n is an integer between 1-73, whereas a "non-NOVX polypeptide" refers to a polypeptide having an amino acid sequence corresponding to a protein that is not substantially homologous to the NOVX protein, *e.g*., a protein that is different from the NOVX protein and that is derived from the same or a different organism. Within a NOVX fusion protein the NOVX polypeptide can correspond to all or a portion of a NOVX protein. In one embodiment, a NOVX fusion protein comprises at least one biologically-active portion of a NOVX protein. In another embodiment, a NOVX fusion protein comprises at least two biologically-active portions of a NOVX protein. In yet another embodiment, a NOVX fusion protein comprises at least three biologically-active portions of a NOVX protein. Within the fusion protein, the term "operatively-linked" is intended to indicate that the NOVX polypeptide and the non-NOVX polypeptide are fused in-frame with one another. The non-NOVX polypeptide can be fused to the N-terminus or C-terminus of the NOVX polypeptide.

In one embodiment, the fusion protein is a GST-NOVX fusion protein in which the NOVX sequences are fused to the C-terminus of the GST (glutathione S-transferase) sequences. Such fusion proteins can facilitate the purification of recombinant NOVX polypeptides.

In another embodiment, the fusion protein is a NOVX protein containing a heterologous signal sequence at its N-terminus. In certain host cells (e.g., mammalian host cells), expression and/or secretion of NOVX can be increased through use of a heterologous signal sequence.

In yet another embodiment, the fusion protein is a NOVX-immunoglobulin fusion protein in which the NOVX sequences are fused to sequences derived from a member of the immunoglobulin protein family. The NOVX-immunoglobulin fusion proteins of the invention can be incorporated into pharmaceutical compositions and administered to a subject to inhibit an interaction between a NOVX ligand and a NOVX protein on the surface of a cell, to thereby suppress NOVX-mediated signal transduction *in vivo.* The NOVX-immunoglobulin fusion proteins can be used to affect the bioavailability of a NOVX cognate ligand. Inhibition of the NOVX ligand/NOVX interaction may be useful therapeutically for both the treatment of proliferative and differentiative disorders, as well as modulating (*e*.*g*. promoting or inhibiting) cell survival. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with a NOVX ligand.

A NOVX chimeric or fusion protein of the invention can be produced by standard recombinant DNA techniques. For example, DNA fragments coding for the different polypeptide sequences are ligated together in-frame in accordance with conventional techniques, e.g., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (*see*, *e.g.,* Ausubel, *et al*. (eds.) CURRENT PROTOCOLS IN MOLECULAR BIOLOGY, John Wiley & Sons, 1992). Moreover, many expression vectors are commercially available that already encode a fusion moiety (*e*.*g*., a GST polypeptide). A NOVX-encoding nucleic acid can be cloned into such an expression vector such that the fusion moiety is linked in-frame to the NOVX protein.

### NOVX Agonists and Antagonists

The invention also pertains to variants of the NOVX proteins that function as either NOVX agonists (i.e., mimetics) or as NOVX antagonists. Variants of the NOVX protein can be generated by mutagenesis (e.g., discrete point mutation or truncation of the NOVX protein). An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX proteins that function as either NOVX agonists (i.e., mimetics) or as NOVX antagonists can be identified by screening combinatorial libraries of mutants (*e.g*., truncation mutants) of the NOVX proteins for NOVX protein agonist or antagonist activity. In one embodiment, a variegated library of NOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (e.g., for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are well-known within the art. *See, e.g*., Narang, 1983. *Tetrahedron* 39: 3; Itakura, *et al.,* 1984. *Annu. Rev. Biochem.* 53: 323; Itakura, *et al.,* 1984. *Science* 198: 1056; Ike, *et al.,* 1983. *Nucl. Acids Res.* 11: 477.

### Polypeptide Libraries

In addition, libraries of fragments of the NOVX protein coding sequences can be used to generate a variegated population of NOVX fragments for screening and subsequent selection of variants of a NOVX protein. In one embodiment, a library of coding sequence fragments can be generated by treating a double stranded PCR fragment of a NOVX coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with S₁ nuclease, and ligating the resulting fragment library into an expression vector. By this method, expression libraries can be derived which encodes N-terminal and internal fragments of various sizes of the NOVX proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify NOVX variants. *See, e*.*g*., Arkin and Yourvan, 1992. *Proc. Natl. Acad. Sci. USA* 89: 7811-7815; Delgrave, *et al*., 1993. *Protein Engineering* 6:327-331.

### NOVX Antibodies

The term "antibody" as used herein refers to immunoglobulin molecules and immunologically active portions of immunoglobulin (Ig) molecules, *i*.*e*., molecules that contain an antigen binding site that specifically binds (immunoreacts with) an antigen. Such antibodies include, but are not limited to, polyclonal, monoclonal, chimeric, single chain, F_{ab}, F_{ab}, and F_{(ab')2} fragments, and an F_{ab} expression library. In general, antibody molecules obtained from humans relates to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain. Reference herein to antibodies includes a reference to all such classes, subclasses and types of human antibody species.

An isolated protein of the invention intended to serve as an antigen, or a portion or fragment thereof, can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein, such as an amino acid sequence of SEQ ID NO:2*n*, wherein n is an integer between 1-73, and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of NOVX that is located on the surface of the protein, *e.g*., a hydrophilic region. A hydrophobicity analysis of the human NOVX protein sequence will indicate which regions of a NOVX polypeptide are particularly hydrophilic and, therefore, encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e.g.,* Hopp and Woods, 1981, *Proc. Nat. Acad. Sci. USA* 78: 3824-3828; Kyte and Doolittle 1982, *J. Mol. Biol*. 157: 105-142, each incorporated herein by reference in their entirety. Antibodies that are specific for one or more domains within an antigenic protein, or derivatives, fragments, analogs or homologs thereof, are also provided herein.

The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. A NOVX polyppeptide or a fragment thereof comprises at least one antigenic epitope. An anti-NOVX antibody of the present invention is said to specifically bind to antigen NOVX when the equilibrium binding constant (K_{D}) is ≤1 µM, preferably ≤ 100 nM, more preferably ≤ 10 nM, and most preferably ≤ 100 pM to about 1 pM, as measured by assays such as radioligand binding assays or similar assays known to those skilled in the art.

A protein of the invention, or a derivative, fragment, analog, homolog or ortholog thereof, may be utilized as an immunogen in the generation of antibodies that immunospecifically bind these protein components. Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow E, and Lane D, 1988, Cold Spring Harbor Laboratory Press, Cold Spring Harbor, NY, incorporated herein by reference). Some of these antibodies are discussed below.

### Polyclonal Antibodies

For the production of polyclonal antibodies, various suitable host animals (e.g., rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant. Various adjuvants used to increase the immunological response include, but are not limited to, Freund's (complete and incomplete), mineral gels (e.g., aluminum hydroxide), surface active substances (e.g., lysolecithin, pluronic polyols, polyanions, peptides, oil emulsions, dinitrophenol, etc.), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (e.g., from the blood) and further purified by well known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### Monoclonal Antibodies

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro.

The immunizing agent will typically include the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, Marcel Dekker, Inc., New York, (1987) pp. 51-63).

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). It is an objective, especially important in therapeutic applications of monoclonal antibodies, to identify antibodies having a high degree of specificity and a high binding affinity for the target antigen.

After the desired hybridoma cells are identified, the clones can be subcloned by limiting dilution procedures and grown by standard methods (Goding,1986). Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Patent No. 4,816,567; Morrison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### Humanized Antibodies

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, Curr. Op. Struct. Biol., 2:593-596 (1992)).

### Human Antibodies

Fully human antibodies essentially relate to antibody molecules in which the entire sequence of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R. Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R. Liss, Inc., pp. 77-96).

In addition, human antibodies can also be produced using additional techniques, including phage display libraries (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals, *e.g*., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. (Bio/Technology 10, 779-783 (1992)); Lonberg et al. (Nature 368 856-859 (1994)); Morrison ( Nature 368, 812-13 (1994)); Fishwild et al,( Nature Biotechnology 14, 845-51 (1996)); Neuberger (Nature Biotechnology 14, 826 (1996)); and Lonberg and Huszar (Intern. Rev. Immunol. 13 65-93 (1995)).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### F_{ab} Fragments and Single Chain Antibodies

According to the invention, techniques can be adapted for the production of single-chain antibodies specific to an antigenic protein of the invention (see *e*.*g*., U.S. Patent No. 4,946,778). In addition, methods can be adapted for the construction of F_{ab} expression libraries (see *e.g.*, Huse, et al., 1989 Science 246: 1275-1281) to allow rapid and effective identification of monoclonal F_{ab} fragments with the desired specificity for a protein or derivatives, fragments, analogs or homologs thereof. Antibody fragments that contain the idiotypes to a protein antigen may be produced by techniques known in the art including, but not limited to: (i) an F_{(ab')2} fragment produced by pepsin digestion of an antibody molecule; (ii) an F_{ab} fragment generated by reducing the disulfide bridges of an F_{(ab')2} fragment; (iii) an F_{ab} fragment generated by the treatment of the antibody molecule with papain and a reducing agent and (iv) Fᵥ fragments.

### Bispecific Antibodies

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Additionally, Fab' fragments can be directly recovered from E. coli and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

### Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### Effector Function Engineering

It can be desirable to modify the antibody of the invention with respect to effector function, so as to enhance, *e.g*., the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176: 1191-1195 (1992) and Shopes, J. humunol., 148: 2918-2922 (1992). Homodimeric antibodies with enhanced anti-tumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

### Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (*i*.*e*., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e*.*g*., avidin) that is in turn conjugated to a cytotoxic agent.

### Immunoliposomes

The antibodies disclosed herein can also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al .,_J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

### Diagnostic Applications of Antibodies Directed Against the Proteins of the Invention

Antibodies directed against a protein of the invention may be used in methods known within the art relating to the localization and/or quantitation of the protein (e.g., for use in measuring levels of the protein within appropriate physiological samples, for use in diagnostic methods, for use in imaging the protein, and the like). In a given embodiment, antibodies against the proteins, or derivatives, fragments, analogs or homologs thereof, that contain the antigen binding domain, are utilized as pharmacologically-active compounds (see below).

An antibody specific for a protein of the invention can be used to isolate the protein by standard techniques, such as immunoaffinity chromatography or immunoprecipitation. Such an antibody can facilitate the purification of the natural protein antigen from cells and of recombinantly produced antigen expressed in host cells. Moreover, such an antibody can be used to detect the antigenic protein (e.g., in a cellular lysate or cell supernatant) in order to evaluate the abundance and pattern of expression of the antigenic protein. Antibodies directed against the protein can be used diagnostically to monitor protein levels in tissue as part of a clinical testing procedure, *e.g*., to, for example, determine the efficacy of a given treatment regimen. Detection can be facilitated by coupling *(i.e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### Antibody Therapeutics

Antibodies of the invention, including polyclonal, monoclonal, humanized and fully human antibodies, may used as therapeutic agents. Such agents will generally be employed to treat or prevent a disease or pathology in a subject. An antibody preparation, preferably one having high specificity and high affinity for its target antigen, is administered to the subject and will generally have an effect due to its binding with the target. Such an effect may be one of two kinds, depending on the specific nature of the interaction between the given antibody molecule and the target antigen in question. In the first instance, administration of the antibody may abrogate or inhibit the binding of the target with an endogenous ligand to which it naturally binds. In this case, the antibody binds to the target and masks a binding site of the naturally occurring ligand, wherein the ligand serves as an effector molecule. Thus the receptor mediates a signal transduction pathway for which ligand is responsible.

Alternatively, the effect may be one in which the antibody elicits a physiological result by virtue of binding to an effector binding site on the target molecule. In this case the target, a receptor having an endogenous ligand which may be absent or defective in the disease or pathology, binds the antibody as a surrogate effector ligand, initiating a receptor-based signal transduction event by the receptor.

A therapeutically effective amount of an antibody of the invention relates generally to the amount needed to achieve a therapeutic objective. As noted above, this may be a binding interaction between the antibody and its target antigen that, in certain cases, interferes with the functioning of the target, and in other cases, promotes a physiological response. The amount required to be administered will furthermore depend on the binding affinity of the antibody for its specific antigen, and will also depend on the rate at which an administered antibody is depleted from the free volume other subject to which it is administered. Common ranges for therapeutically effective dosing of an antibody or antibody fragment of the invention may be, by way of nonlimiting example, from about 0.1 mg/kg body weight to about 50 mg/kg body weight. Common dosing frequencies may range, for example, from twice daily to once a week.

### Pharmaceutical Compositions of Antibodies

Antibodies specifically binding a protein of the invention, as well as other molecules identified by the screening assays disclosed herein, can be administered for the treatment of various disorders in the form of pharmaceutical compositions. Principles and considerations involved in preparing such compositions, as well as guidance in the choice of components are provided, for example, in Remington : The Science And Practice Of Pharmacy 19th ed. (Alfonso R. Gennaro, et al., editors) Mack Pub. Co., Easton, Pa. : 1995; Drug Absorption Enhancement: Concepts, Possibilities, Limitations, And Trends, Harwood Academic Publishers, Langhorne, Pa., 1994; and Peptide And Protein Drug Delivery (Advances In Parenteral Sciences, Vol. 4), 1991, M. Dekker, New York.

If the antigenic protein is intracellular and whole antibodies are used as inhibitors, internalizing antibodies are preferred. However, liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g*., Marasco et al., Proc. Natl. Acad. Sci. USA, 90: 7889-7893 (1993). The formulation herein can also contain more than one active compound as necessary for the particular indication being treated, preferably those with complementary activities that do not adversely affect each other. Alternatively, or in addition, the composition can comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients can also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions.

The formulations to be used for in vivo administration must be sterile. This is readily accomplished by filtration through sterile filtration membranes.

Sustained-release preparations can be prepared. Suitable examples of sustained-release preparations include semipermeable matrices of solid hydrophobic polymers containing the antibody, which matrices are in the form of shaped articles, *e*.*g*., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, nondegradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

### ELISA Assay

An agent for detecting an analyte protein is an antibody capable of binding to an analyte protein, preferably an antibody with a detectable label. Antibodies can be polyclonal, or more preferably, monoclonal. An intact antibody, or a fragment thereof (e.g., F_{ab} or F_{(ab)2}) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. Included within the usage of the term "biological sample", therefore, is blood and a fraction or component of blood including blood serum, blood plasma, or lymph. That is, the detection method of the invention can be used to detect an analyte mRNA, protein, or genomic DNA in a biological sample *in vitro* as well as *in vivo.* For example, *in vitro* techniques for detection of an analyte mRNA include Northern hybridizations and *in situ* hybridizations. *In vitro* techniques for detection of an analyte protein include enzyme linked immunosorbent assays (ELISAs), Western blots, immunoprecipitations, and immunofluorescence. *In vitro* techniques for detection of an analyte genomic DNA include Southern hybridizations. Procedures for conducting immunoassays are described, for example in "ELISA: Theory and Practice: Methods in Molecular Biology", Vol. 42, J. R. Crowther (Ed.) Human Press, Totowa, NJ, 1995; "Immunoassay", E. Diamandis and T. Christopoulus, Academic Press, Inc., San Diego, CA, 1996; and "Practice and Thory of Enzyme Immunoassays", P. Tijssen, Elsevier Science Publishers, Amsterdam, 1985. Furthermore, *in vivo* techniques for detection of an analyte protein include introducing into a subject a labeled anti-an analyte protein antibody. For example, the antibody can be labeled with a radioactive marker whose presence and location in a subject can be detected by standard imaging techniques.

### NOVX Recombinant Expression Vectors and Host Cells

Another aspect of the invention pertains to vectors, preferably expression vectors, containing a nucleic acid encoding a NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e*.*g*., bacterial vectors having a bacterial origin of replication and episomal mammalian vectors). Other vectors (*e*.*g*., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence *(e.g.,* in an *in vitro* transcription/translation system or in a host cell when the vector is introduced into the host cell).

The term "regulatory sequence" is intended to includes promoters, enhancers and other expression control elements (e.g., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, *etc.* The expression vectors of the invention can be introduced into host cells to thereby produce proteins or peptides, including fusion proteins or peptides, encoded by nucleic acids as described herein (e.g., NOVX proteins, mutant forms of NOVX proteins, fusion proteins, *etc. ).*

The recombinant expression vectors of the invention can be designed for expression of NOVX proteins in prokaryotic or eukaryotic cells. For example, NOVX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro*, for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (i) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. *Gene* 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann *et al*., (1988) *Gene* 69:301-315) and pET 11d (Studier *et al*., GENE EXPRESSION TECHNOLOGY: METHODS IN ENZYMOLOGY 185, Academic Press, San Diego, Calif. (1990) 60-89).

One strategy to maximize recombinant protein expression in *E*. *coli* is to express the protein in a host bacteria with an impaired capacity to proteolytically cleave the recombinant protein. *See, e*.*g*., Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E*. *coli* (*see, e.g.,* Wada, *et al*., 1992. *Nucl. Acids Res.* 20: 2111-2118). Such alteration of nucleic acid sequences of the invention can be carried out by standard DNA synthesis techniques.

In another embodiment, the NOVX expression vector is a yeast expression vector. Examples of vectors for expression in yeast *Saccharomyces cerivisae* include pYepSec1 (Baldari, *et al*., 1987. *EMBO J.* 6: 229-234), pMFa (Kurjan and Herskowitz, 1982. *Cell* 30: 933-943), pJRY88 (Schultz *et al*., 1987. *Gene* 54: 113-123), pYES2 (Invitrogen Corporation, San Diego, Calif.), and picZ (InVitrogen Corp, San Diego, Calif.).

Alternatively, NOVX can be expressed in insect cells using baculovirus expression vectors. Baculovirus vectors available for expression of proteins in cultured insect cells *(e.g.,* SF9 cells) include the pAc series (Smith, *et al*., 1983. *Mol. Cell. Biol*. 3: 2156-2165) and the pVL series (Lucklow and Summers, 1989. *Virology* 170: 31-39).

In yet another embodiment, a nucleic acid of the invention is expressed in mammalian cells using a mammalian expression vector. Examples of mammalian expression vectors include pCDM8 (Seed, 1987. *Nature* 329: 840) and pMT2PC (Kaufman, *et al*., 1987. *EMBO J.* 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e*.*g*., Chapters 16 and 17 of Sambrook, *et al*., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, *et al*., 1987. *Genes Dev.* 1: 268-277), lymphoid-specific promoters (Calame and Eaton, 1988. *Adv. Immunol*. 43: 235-275), in particular promoters of T cell receptors (Winoto and Baltimore, 1989. *EMBO J.* 8: 729-733) and immunoglobulins (Banerji, *et al*., 1983. *Cell* 33: 729-740; Queen and Baltimore, 1983. *Cell* 33: 741-748), neuron-specific promoters (e.g., the neurofilament promoter; Byrne and Ruddle, 1989. *Proc. Natl. Acad. Sci. USA* 86: 5473-5477), pancreas-specific promoters (Edlund, *et al.,* 1985. *Science* 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. *Science 249:* 374-379) and the α-fetoprotein promoter (Campes and Tilghman, 1989. *Genes Dev. 3:* 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, *et al*., "Antisense RNA as a molecular tool for genetic analysis," *Reviews-Trends in Genetics,* Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E. coli*, insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al.* (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce (*i*.*e*., express) NOVX protein. Accordingly, the invention further provides methods for producing NOVX protein using the host cells of the invention. In one embodiment, the method comprises culturing the host cell of invention (into which a recombinant expression vector encoding NOVX protein has been introduced) in a suitable medium such that NOVX protein is produced. In another embodiment, the method further comprises isolating NOVX protein from the medium or the host cell.

### Transgenic NOVX Animals

The host cells of the invention can also be used to produce non-human transgenic animals. For example, in one embodiment, a host cell of the invention is a fertilized oocyte or an embryonic stem cell into which NOVX protein-coding sequences have been introduced. Such host cells can then be used to create non-human transgenic animals in which exogenous NOVX sequences have been introduced into their genome or homologous recombinant animals in which endogenous NOVX sequences have been altered. Such animals are useful for studying the function and/or activity of NOVX protein and for identifying and/or evaluating modulators of NOVX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, *etc*. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g*., an embryonic cell of the animal, prior to development of the animal.

A transgenic animal of the invention can be created by introducing NOVX-encoding nucleic acid into the male pronuclei of a fertilized oocyte (e.g., by microinjection, retroviral infection) and allowing the oocyte to develop in a pseudopregnant female foster animal. The human NOVX cDNA sequences, *i*.*e*., any one of SEQ ID NO:2*n*-1, wherein n is an integer between 1-73, can be introduced as a transgene into the genome of a non-human animal. Alternatively, a non-human homologue of the human NOVX gene, such as a mouse NOVX gene, can be isolated based on hybridization to the human NOVX cDNA (described further *supra*) and used as a transgene. Intronic sequences and polyadenylation signals can also be included in the transgene to increase the efficiency of expression of the transgene. A tissue-specific regulatory sequence(s) can be operably-linked to the NOVX transgene to direct expression of NOVX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX transgene in its genome and/or expression of NOVX mRNA in tissues or cells of the animals. A transgenic founder animal can then be used to breed additional animals carrying the transgene. Moreover, transgenic animals carrying a transgene- encoding NOVX protein can further be bred to other transgenic animals carrying other transgenes.

To create a homologous recombinant animal, a vector is prepared which contains at least a portion of a NOVX gene into which a deletion, addition or substitution has been introduced to thereby alter, *e.g*., functionally disrupt, the NOVX gene. The NOVX gene can be a human gene *(e.g.,* the cDNA of any one of SEQ ID NO:2*n*-1, wherein n is an integer between 1-73), but more preferably, is a non-human homologue of a human NOVX gene. For example, a mouse homologue of human NOVX gene of SEQ ID NO:2*n*-1, wherein n is an integer between 1-73, can be used to construct a homologous recombination vector suitable for altering an endogenous NOVX gene in the mouse genome. In one embodiment, the vector is designed such that, upon homologous recombination, the endogenous NOVX gene is functionally disrupted (i.e., no longer encodes a functional protein; also referred to as a "knock out" vector).

Alternatively, the vector can be designed such that, upon homologous recombination, the endogenous NOVX gene is mutated or otherwise altered but still encodes functional protein (e.g., the upstream regulatory region can be altered to thereby alter the expression of the endogenous NOVX protein). In the homologous recombination vector, the altered portion of the NOVX gene is flanked at its 5'- and 3'-termini by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5'- and 3'-termini) are included in the vector. *See, e.g.,* Thomas, *et al*., 1987. *Cell* 51: 503 for a description of homologous recombination vectors. The vector is ten introduced into an embryonic stem cell line *(e.g.,* by electroporation) and cells in which the introduced NOVX gene has homologously-recombined with the endogenous NOVX gene are selected. *See, e.g.,* Li, *et al*., 1992. *Cell* 69: 915.

The selected cells are then injected into a blastocyst of an animal (e.g., a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. *Curr. Opin. Biotechnol*. 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e.g.,* Lakso, *et al*., 1992. *Proc. Natl. Acad. Sci. USA* 89: 6232-6236. Another example of a recombinase system is the FLP recombinase system of *Saccharomyces cerevisiae. See,* O'Gorman, *et al*., 1991. *Science* 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g*., by mating two transgenic animals, one containing a transgene encoding a selected protein and the other containing a transgene encoding a recombinase.

Clones of the non-human transgenic animals described herein can also be produced according to the methods described in Wilmut, *et al*., 1997. *Nature* 385: 810-813. In brief, a cell (e.g., a somatic cell) from the transgenic animal can be isolated and induced to exit the growth cycle and enter Go phase. The quiescent cell can then be fused, *e.g*., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell (e.g., the somatic cell) is isolated.

### Pharmaceutical Compositions

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g*., intravenous, intradermal, subcutaneous, oral (*e*.*g*., inhalation), transdermal *(i.e.,* topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (*see, e*.*g*., U.S. Patent No. 5,328,470) or by stereotactic injection *(see, e*.*g*., Chen, *et al*., 1994. *Proc. Natl. Acad. Sci. USA* 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g*., retroviral vectors, the pharmaceutical preparation can include one or more cells that produce the gene delivery system.

The pharmaceutical compositions can be included in a container, pack, or dispenser together with instructions for administration.

### Screening and Detection Methods

The isolated nucleic acid molecules of the invention can be used to express NOVX protein (e.g., via a recombinant expression vector in a host cell in gene therapy applications), to detect NOVX mRNA (e.g., in a biological sample) or a genetic lesion in a NOVX gene, and to modulate NOVX activity, as described further, below. In addition, the NOVX proteins can be used to screen drugs or compounds that modulate the NOVX protein activity or expression as well as to treat disorders characterized by insufficient or excessive production of NOVX protein or production of NOVX protein forms that have decreased or aberrant activity compared to NOVX wild-type protein *(e.g*.; diabetes (regulates insulin release); obesity (binds and transport lipids); metabolic disturbances associated with obesity, the metabolic syndrome X as well as anorexia and wasting disorders associated with chronic diseases and various cancers, and infectious disease(possesses anti-microbial activity) and the various dyslipidemias. In addition, the anti-NOVX antibodies of the invention can be used to detect and isolate NOVX proteins and modulate NOVX activity. In yet a further aspect, the invention can be used in methods to influence appetite, absorption of nutrients and the disposition of metabolic substrates in both a positive and negative fashion.

The invention further pertains to novel agents identified by the screening assays described herein and uses thereof for treatments as described, *supra*.

### Screening Assays

The invention provides a method (also referred to herein as a "screening assay") for identifying modulators, i.e., candidate or test compounds or agents (e.g., peptides, peptidomimetics, small molecules or other drugs) that bind to NOVX proteins or have a stimulatory or inhibitory effect on, *e.g*., NOVX protein expression or NOVX protein activity. The invention also includes compounds identified in the screening assays described herein.

In one embodiment, the invention provides assays for screening candidate or test compounds which bind to or modulate the activity of the membrane-bound form of a NOVX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. *Anticancer Drug Design* 12: 145.

A "small molecule" as used herein, is meant to refer to a composition that has a molecular weight of less than about 5 kD and most preferably less than about 4 kD. Small molecules can be, *e.g*., nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, *et al*., 1993. *Proc. Natl. Acad. Sci. U.S.A*. 90: 6909; Erb, *et al*., 1994. *Proc. Natl. Acad. Sci. U.S.A.* 91: 11422; Zuckermann, *et al*., 1994. *J. Med. Chem. 37:* 2678; Cho, *et al*., 1993. *Science* 261: 1303; Carrell, *et al*., 1994. *Angew. Chem. Int. Ed. Engl.* 33: 2059; Carell, *et al.,* 1994. *Angew. Chem. Int. Ed. Engl.* 33: 2061; and Gallop, *et al*., 1994. *J. Med. Chem.* 37: 1233.

Libraries of compounds may be presented in solution (*e*.*g*., Houghten, 1992. *Biotechniques* 13: 412-421), or on beads (Lam, 1991. *Nature* 354: 82-84), on chips (Fodor, 1993. *Nature* 364: 555-556), bacteria (Ladner, U.S. Patent No. 5,223,409), spores (Ladner, U.S. Patent 5,233,409), plasmids (Cull, *et al*., 1992. *Proc. Natl. Acad. Sci. USA* 89: 1865-1869) or on phage (Scott and Smith, 1990. *Science* 249: 386-390; Devlin, 1990. *Science* 249: 404-406; Cwirla, *et al*., 1990. *Proc. Natl. Acad. Sci. U.S.A.* 87: 6378-6382; Felici, 1991. *J. Mol. Biol*. 222: 301-310; Ladner, U.S. Patent No. 5,233,409.).

In one embodiment, an assay is a cell-based assay in which a cell which expresses a membrane-bound form of NOVX protein, or a biologically-active portion thereof, on the cell surface is contacted with a test compound and the ability of the test compound to bind to a NOVX protein determined. The cell, for example, can of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of NOVX protein, or a biologically-active portion thereof, on the cell surface with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX protein or a biologically-active portion thereof as compared to the known compound.

In another embodiment, an assay is a cell-based assay comprising contacting a cell expressing a membrane-bound form of NOVX protein, or a biologically-active portion thereof, on the cell surface with a test compound and determining the ability of the test compound to modulate (e.g., stimulate or inhibit) the activity of the NOVX protein or biologically-active portion thereof. Determining the ability of the test compound to modulate the activity of NOVX or a biologically-active portion thereof can be accomplished, for example, by determining the ability of the NOVX protein to bind to or interact with a NOVX target molecule. As used herein, a "target molecule" is a molecule with which a NOVX protein binds or interacts in nature, for example, a molecule on the surface of a cell which expresses a NOVX interacting protein, a molecule on the surface of a second cell, a molecule in the extracellular milieu, a molecule associated with the internal surface of a cell membrane or a cytoplasmic molecule. A NOVX target molecule can be a non-NOVX molecule or a NOVX protein or polypeptide of the invention. In one embodiment, a NOVX target molecule is a component of a signal transduction pathway that facilitates transduction of an extracellular signal (e.g. a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with a NOVX target molecule can be accomplished by one of the methods described above for determining direct binding. In one embodiment, determining the ability of the NOVX protein to bind to or interact with a NOVX target molecule can be accomplished by determining the activity of the target molecule. For example, the activity of the target molecule can be determined by detecting induction of a cellular second messenger of the target (*i*.*e*. intracellular Ca²⁺, diacylglycerol, IP₃, *etc*.), detecting catalytic/enzymatic activity of the target an appropriate substrate, detecting the induction of a reporter gene (comprising a NOVX-responsive regulatory element operatively linked to a nucleic acid encoding a detectable marker, *e.g.*, luciferase), or detecting a cellular response, for example, cell survival, cellular differentiation, or cell proliferation.

In yet another embodiment, an assay of the invention is a cell-free assay comprising contacting a NOVX protein or biologically-active portion thereof with a test compound and determining the ability of the test compound to bind to the NOVX protein or biologically-active portion thereof. Binding of the test compound to the NOVX protein can be determined either directly or indirectly as described above. In one such embodiment, the assay comprises contacting the NOVX protein or biologically-active portion thereof with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX or biologically-active portion thereof as compared to the known compound.

In still another embodiment, an assay is a cell-free assay comprising contacting NOVX protein or biologically-active portion thereof with a test compound and determining the ability of the test compound to modulate (e.g. stimulate or inhibit) the activity of the NOVX protein or biologically-active portion thereof. Determining the ability of the test compound to modulate the activity of NOVX can be accomplished, for example, by determining the ability of the NOVX protein to bind to a NOVX target molecule by one of the methods described above for determining direct binding. In an alternative embodiment, determining the ability of the test compound to modulate the activity of NOVX protein can be accomplished by determining the ability of the NOVX protein further modulate a NOVX target molecule. For example, the catalytic/enzymatic activity of the target molecule on an appropriate substrate can be determined as described, *supra.*

In yet another embodiment, the cell-free assay comprises contacting the NOVX protein or biologically-active portion thereof with a known compound which binds NOVX protein to form an assay mixture, contacting the assay mixture with a test compound, and determining the ability of the test compound to interact with a NOVX protein, wherein determining the ability of the test compound to interact with a NOVX protein comprises determining the ability of the NOVX protein to preferentially bind to or modulate the activity of a NOVX target molecule.

The cell-free assays of the invention are amenable to use of both the soluble form or the membrane-bound form of NOVX protein. In the case of cell-free assays comprising the membrane-bound form of NOVX protein, it may be desirable to utilize a solubilizing agent such that the membrane-bound form of NOVX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, N-dodecyl--N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either NOVX protein or its target molecule to facilitate separation of complexed from uncomplexed forms of one or both of the proteins, as well as to accommodate automation of the assay. Binding of a test compound to NOVX protein, or interaction of NOVX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described, *supra*. Alternatively, the complexes can be dissociated from the matrix, and the level of NOVX protein binding or activity determined using standard techniques.

Other techniques for immobilizing proteins on matrices can also be used in the screening assays of the invention. For example, either the NOVX protein or its target molecule can be immobilized utilizing conjugation of biotin and streptavidin. Biotinylated NOVX protein or target molecules can be prepared from biotin-NHS (N-hydroxy-succinimide) using techniques well-known within the art (e.g., biotinylation kit, Pierce Chemicals, Rockford, Ill.), and immobilized in the wells of streptavidin-coated 96 well plates (Pierce Chemical). Alternatively, antibodies reactive with NOVX protein or target molecules, but which do not interfere with binding of the NOVX protein to its target molecule, can be derivatized to the wells of the plate, and unbound target or NOVX protein trapped in the wells by antibody conjugation. Methods for detecting such complexes, in addition to those described above for the GST-immobilized complexes, include immunodetection of complexes using antibodies reactive with the NOVX protein or target molecule, as well as enzyme-linked assays that rely on detecting an enzymatic activity associated with the NOVX protein or target molecule.

In another embodiment, modulators of NOVX protein expression are identified in a method wherein a cell is contacted with a candidate compound and the expression of NOVX mRNA or protein in the cell is determined. The level of expression of NOVX mRNA or protein in the presence of the candidate compound is compared to the level of expression of NOVX mRNA or protein in the absence of the candidate compound. The candidate compound can then be identified as a modulator of NOVX mRNA or protein expression based upon this comparison. For example, when expression of NOVX mRNA or protein is greater (i.e., statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay (*see, e.g.,* U.S. Patent No. 5,283,317; Zervos, *et al*., 1993. *Cell* 72: 223-232; Madura, *et al*., 1993. *J. Biol. Chem*. 268: 12046-12054; Bartel, *et al*., 1993. *Biotechniques* 14: 920-924; Iwabuchi, *et al*., 1993. *Oncogene* 8: 1693-1696; and Brent WO 94/10300), to identify other proteins that bind to or interact with NOVX ("NOVX-binding proteins" or "NOVX-bp") and modulate NOVX activity. Such NOVX-binding proteins are also involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a NOVX-dependent complex, the DNA-binding and activation domains of the transcription factor are brought into close proximity. This proximity allows transcription of a reporter gene (e.g., LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with NOVX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) map their respective genes on a chromosome; and, thus, locate gene regions associated with genetic disease; (*ii*) identify an individual from a minute biological sample (tissue typing); and *(iii)* aid in forensic identification of a biological sample. Some of these applications are described in the subsections, below.

### Chromosome Mapping

Once the sequence (or a portion of the sequence) of a gene has been isolated, this sequence can be used to map the location of the gene on a chromosome. This process is called chromosome mapping. Accordingly, portions or fragments of the NOVX sequences of SEQ ID NO:2*n*-1, wherein n is an integer between 1-73, or fragments or derivatives thereof, can be used to map the location of the NOVX genes, respectively, on a chromosome. The mapping of the NOVX sequences to chromosomes is an important first step in correlating these sequences with genes associated with disease.

Briefly, NOVX genes can be mapped to chromosomes by preparing PCR primers (preferably 15-25 bp in length) from the NOVX sequences. Computer analysis of the NOVX, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the NOVX sequences will yield an amplified fragment.

Somatic cell hybrids are prepared by fusing somatic cells from different mammals (e.g., human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. *See, e.g.,* D'Eustachio, *et al*., 1983. *Science* 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the NOVX sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, *see,* Verma, *et al*., HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, *e.g*., in McKusick, MENDELIAN INHERITANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e.g*., Egeland, *et al*., 1987. *Nature,* 325: 783-787.

Moreover, differences in the DNA sequences between individuals affected and unaffected with a disease associated with the NOVX gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

The NOVX sequences of the invention can also be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the NOVX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If coding sequences, such as those of SEQ ID NO:2*n*-1, wherein n is an integer between 1-73, are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining NOVX protein and/or nucleic acid expression as well as NOVX activity, in the context of a biological sample (e.g., blood, serum, cells, tissue) to thereby determine whether an individual is afflicted with a disease or disorder, or is at risk of developing a disorder, associated with aberrant NOVX expression or activity. The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers. The invention also provides for prognostic (or predictive) assays for determining whether an individual is at risk of developing a disorder associated with NOVX protein, nucleic acid expression or activity. For example, mutations in a NOVX gene can be assayed in a biological sample. Such assays can be used for prognostic or predictive purpose to thereby prophylactically treat an individual prior to the onset of a disorder characterized by or associated with NOVX protein, nucleic acid expression, or biological activity.

Another aspect of the invention provides methods for determining NOVX protein, nucleic acid expression or activity in an individual to thereby select appropriate therapeutic or prophylactic agents for that individual (referred to herein as "pharmacogenomics"). Pharmacogenomics allows for the selection of agents (e.g., drugs) for therapeutic or prophylactic treatment of an individual based on the genotype of the individual (e.g., the genotype of the individual examined to determine the ability of the individual to respond to a particular agent.)

Yet another aspect of the invention pertains to monitoring the influence of agents (e.g., drugs, compounds) on the expression or activity of NOVX in clinical trials.

These and other agents are described in further detail in the following sections.

### Diagnostic Assays

An exemplary method for detecting the presence or absence of NOVX in a biological sample involves obtaining a biological sample from a test subject and contacting the biological sample with a compound or an agent capable of detecting NOVX protein or nucleic acid (e.g., mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NO:2*n*-1, wherein n is an integer between 1-73, or a portion thereof, such as an oligonucleotide of at least 15, 30, 50, 100, 250 or 500 nucleotides in length and sufficient to specifically hybridize under stringent conditions to NOVX mRNA or genomic DNA. Other suitable probes for use in the diagnostic assays of the invention are described herein.

An agent for detecting NOVX protein is an antibody capable of binding to NOVX protein, preferably an antibody with a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (*i*.*e*., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo*. For example*, in vitro* techniques for detection of NOVX mRNA include Northern hybridizations and *in situ* hybridizations. *In vitro* techniques for detection of NOVX protein include enzyme linked immunosorbent assays (ELISAs), Western blots, immunoprecipitations, and immunofluorescence. *In vitro* techniques for detection of NOVX genomic DNA include Southern hybridizations. Furthermore*, in vivo* techniques for detection of NOVX protein include introducing into a subject a labeled anti-NOVX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence of NOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

### Prognostic Assays

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity. Alternatively, the prognostic assays can be utilized to identify a subject having or at risk for developing a disease or disorder. Thus, the invention provides a method for identifying a disease or disorder associated with aberrant NOVX expression or activity in which a test sample is obtained from a subject and NOVX protein or nucleic acid (e.g., mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (e.g., serum), cell sample, or tissue.

Furthermore, the prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder. Thus, the invention provides methods for determining whether a subject can be effectively treated with an agent for a disorder associated with aberrant NOVX expression or activity in which a test sample is obtained and NOVX protein or nucleic acid is detected (e.g., wherein the presence of NOVX protein or nucleic acid is diagnostic for a subject that can be administered the agent to treat a disorder associated with aberrant NOVX expression or activity).

The methods of the invention can also be used to detect genetic lesions in a NOVX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding a NOVX-protein, or the misexpression of the NOVX gene. For example, such genetic lesions can be detected by ascertaining the existence of at least one of: *(i)* a deletion of one or more nucleotides from a NOVX gene; (*ii*) an addition of one or more nucleotides to a NOVX gene; *(iii)* a substitution of one or more nucleotides of a NOVX gene, (iv) a chromosomal rearrangement of a NOVX gene; (v) an alteration in the level of a messenger RNA transcript of a NOVX gene, (*vi*) aberrant modification of a NOVX gene, such as of the methylation pattern of the genomic DNA, *(vii)* the presence of a non-wild-type splicing pattern of a messenger RNA transcript of a NOVX gene, *(viii)* a non-wild-type level of a NOVX protein, (*ix*) allelic loss of a NOVX gene, and (*x*) inappropriate post-translational modification of a NOVX protein. As described herein, there are a large number of assay techniques known in the art which can be used for detecting lesions in a NOVX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) (*see, e.g.,* U.S. Patent Nos. 4,683,195 and 4,683,202), such as anchor PCR or RACE PCR, or, alternatively, in a ligation chain reaction (LCR) (*see, e*.*g*., Landegran, *et al*., 1988. *Science* 241: 1077-1080; and Nakazawa, *et al*., 1994. *Proc. Natl. Acad. Sci. USA* 91: 360-364), the latter of which can be particularly useful for detecting point mutations in the NOVX-gene (*see*, Abravaya, *et al*., 1995. *Nucl. Acids Res.* 23: 675-682). This method can include the steps of collecting a sample of cells from a patient, isolating nucleic acid (e.g., genomic, mRNA or both) from the cells of the sample, contacting the nucleic acid sample with one or more primers that specifically hybridize to a NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication *(see,* Guatelli, *et al*., 1990. *Proc. Natl. Acad. Sci. USA* 87: 1874-1878), transcriptional amplification system (*see*, Kwoh, *et al*., 1989. *Proc. Natl. Acad. Sci. USA* 86: 1173-1177); Qβ Replicase (*see*, Lizardi, *et al*, 1988. *BioTechnology* 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (*see, e.g.,* U.S. Patent No. 5,493,531) can be used to score for the presence of specific mutations by development or loss of a ribozyme cleavage site.

In other embodiments, genetic mutations in NOVX can be identified by hybridizing a sample and control nucleic acids, *e.g*., DNA or RNA, to high-density arrays containing hundreds or thousands of oligonucleotides probes. *See, e.g.,* Cronin, *et al*., 1996. *Human Mutation* 7: 244-255; Kozal, *et al*., 1996. *Nat. Med*. 2: 753-759. For example, genetic mutations in NOVX can be identified in two dimensional arrays containing light-generated DNA probes as described in Cronin, *et al., supra*. Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert, 1977. *Proc. Natl. Acad. Sci. USA* 74: 560 or Sanger, 1977. *Proc. Natl. Acad. Sci. USA* 74: 5463. It is also contemplated that any of a variety of automated sequencing procedures can be utilized when performing the diagnostic assays (*see, e.g.,* Naeve, *et al*., 1995. *Biotechniques* 19: 448), including sequencing by mass spectrometry (see, *e.g.,* PCT International Publication No. WO 94/16101; Cohen, *et al*., 1996. *Adv. Chromatography* 36: 127-162; and Griffin, *et al*., 1993. *Appl. Biochem. Biotechnol.* 38: 147-159).

Other methods for detecting mutations in the NOVX gene include methods in which protection from cleavage agents is used to detect mismatched bases in RNA/RNA or RNA/DNA heteroduplexes. *See, e.g.,* Myers, *et al*., 1985. *Science* 230: 1242. In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g.,* Cotton, *et al*., 1988. *Proc. Natl. Acad. Sci. USA* 85: 4397; Saleeba, *et al*., 1992. *Methods Enzymol*. 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E*. *coli* cleaves A at G/A mismatches and the thymidine DNA glycosylase from HeLa cells cleaves T at G/T mismatches. *See, e.g.,* Hsu, *et al*., 1994. *Carcinogenesis* 15: 1657-1662. According to an exemplary embodiment, a probe based on a NOVX sequence, *e.g*., a wild-type NOVX sequence, is hybridized to a cDNA or other DNA product from a test cell(s). The duplex is treated with a DNA mismatch repair enzyme, and the cleavage products, if any, can be detected from electrophoresis protocols or the like. *See, e.g.,* U.S. Patent No. 5,459,039.

In other embodiments, alterations in electrophoretic mobility will be used to identify mutations in NOVX genes. For example, single strand conformation polymorphism (SSCP) may be used to detect differences in electrophoretic mobility between mutant and wild type nucleic acids. *See, e.g.,* Orita, *et al*., 1989. *Proc. Natl. Acad. Sci. USA*: 86: 2766; Cotton, 1993. *Mutat. Res.* 285: 125-144; Hayashi, 1992. *Genet. Anal. Tech. Appl*. 9: 73-79. Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e*.*g*., Keen, *et al*., 1991. *Trends Genet*. 7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, *et al*., 1985. *Nature* 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e.g.,* Rosenbaum and Reissner, 1987. *Biophys. Chem.* 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e.g.,* Saiki, *et al*., 1986. *Nature* 324: 163; Saiki, *et al*., 1989. *Proc. Natl. Acad. Sci. USA* 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e.g.,* Gibbs, *et al.,* 1989. *Nucl. Acids Res.* 17: 2437-2448) or at the extreme 3'-terminus of one primer where, under appropriate conditions, mismatch can prevent, or reduce polymerase extension (*see, e.g.,* Prossner, 1993. *Tibtech.* 11: 238). In addition it may be desirable to introduce a novel restriction site in the region of the mutation to create cleavage-based detection. *See, e.g.,* Gasparini, *et al*., 1992. *Mol. Cell Probes* 6: 1. It is anticipated that in certain embodiments amplification may also be performed using *Taq* ligase for amplification. *See, e.g.,* Barany, 1991. *Proc. Natl. Acad. Sci. USA* 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e.g*., in clinical settings to diagnose patients exhibiting symptoms or family history of a disease or illness involving a NOVX gene.

Furthermore, any cell type or tissue, preferably peripheral blood leukocytes, in which NOVX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on NOVX activity (e.g., NOVX gene expression), as identified by a screening assay described herein can be administered to individuals to treat (prophylactically or therapeutically) disorders (The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers.) In conjunction with such treatment, the pharmacogenomics (i.e., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content of NOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g*., Eichelbaum, 1996. *Clin. Exp. Pharmacol. Physiol*., 23: 983-985; Linder, 1997. *Clin. Chem.,* 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome pregnancy zone protein precursor enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a NOVX modulator, such as a modulator identified by one of the exemplary screening assays described herein.

### Monitoring of Effects During Clinical Trials

Monitoring the influence of agents (e.g., drugs, compounds) on the expression or activity of NOVX (e.g., the ability to modulate aberrant cell proliferation and/or differentiation) can be applied not only in basic drug screening, but also in clinical trials. For example, the effectiveness of an agent determined by a screening assay as described herein to increase NOVX gene expression, protein levels, or upregulate NOVX activity, can be monitored in clinical trails of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trails of subjects exhibiting increased NOVX gene expression, protein levels, or upregulated NOVX activity. In such clinical trials, the expression or activity of NOVX and, preferably, other genes that have been implicated in, for example, a cellular proliferation or immune disorder can be used as a "read out" or markers of the immune responsiveness of a particular cell.

By way of example, and not of limitation, genes, including NOVX, that are modulated in cells by treatment with an agent (e.g., compound, drug or small molecule) that modulates NOVX activity (e.g., identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression (*i*.*e*., a gene expression pattern) can be quantified by Northern blot analysis or RT-PCR, as described herein, or alternatively by measuring the amount of protein produced, by one of the methods as described herein, or by measuring the levels of activity of NOVX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (e.g., an agonist, antagonist, protein, peptide, peptidomimetic, nucleic acid, small molecule, or other drug candidate identified by the screening assays described herein) comprising the steps of (*i*) obtaining a pre-administration sample from a subject prior to administration of the agent; (*ii*) detecting the level of expression of a NOVX protein, mRNA, or genomic DNA in the preadministration sample; (iii) obtaining one or more post-administration samples from the subject; (iv) detecting the level of expression or activity of the NOVX protein, mRNA, or genomic DNA in the post-administration samples; (v) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity of NOVX to higher levels than detected, i.e., to increase the effectiveness of the agent. Alternatively, decreased administration of the agent may be desirable to decrease expression or activity of NOVX to lower levels than detected, *i*.*e*., to decrease the effectiveness of the agent.

### Methods of Treatment

The invention provides for both prophylactic and therapeutic methods of treating a subject at risk of (or susceptible to) a disorder or having a disorder associated with aberrant NOVX expression or activity. The disorders include cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, adrenoleukodystrophy, congenital adrenal hyperplasia, prostate cancer, neoplasm; adenocarcinoma, lymphoma, uterus cancer, fertility, hemophilia, hypercoagulation, idiopathic thrombocytopenic purpura, immunodeficiencies, graft versus host disease, AIDS, bronchial asthma, Crohn's disease; multiple sclerosis, treatment of Albright Hereditary Ostoeodystrophy, and other diseases, disorders and conditions of the like.

These methods of treatment will be discussed more fully, below.

### Disease and Disorders

Diseases and disorders that are characterized by increased (relative to a subject not suffering from the disease or disorder) levels or biological activity may be treated with Therapeutics that antagonize (*i*.*e*., reduce or inhibit) activity. Therapeutics that antagonize activity may be administered in a therapeutic or prophylactic manner. Therapeutics that may be utilized include, but are not limited to: (i) an aforementioned peptide, or analogs, derivatives, fragments or homologs thereof; (*ii*) antibodies to an aforementioned peptide; (*iii*) nucleic acids encoding an aforementioned peptide; (*iv*) administration of antisense nucleic acid and nucleic acids that are "dysfunctional" (*i*.*e*., due to a heterologous insertion within the coding sequences of coding sequences to an aforementioned peptide) that are utilized to "knockout" endogenous function of an aforementioned peptide by homologous recombination *(see, e.g.,* Capecchi, 1989. *Science* 244: 1288-1292); or (*v*) modulators (*i*.*e*., inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase (i.e., are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (e.g., from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (e.g., by Western blot analysis, immunoprecipitation followed by sodium dodecyl sulfate (SDS) polyacrylamide gel electrophoresis, immunocytochemistry, *etc.)* and/or hybridization assays to detect expression of mRNAs (e.g., Northern assays, dot blots, *in situ* hybridization, and the like).

### Prophylactic Methods

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending upon the type of NOVX aberrancy, for example, a NOVX agonist or NOVX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of a NOVX protein, a peptide, a NOVX peptidomimetic, or other small molecule. In one embodiment, the agent stimulates one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and anti-NOVX antibodies. These modulatory methods can be performed *in vitro (e.g.,* by culturing the cell with the agent) or, alternatively, *in vivo (e.g.,* by administering the agent to a subject). As such, the invention provides methods of treating an individual afflicted with a disease or disorder characterized by aberrant expression or activity of a NOVX protein or nucleic acid molecule. In one embodiment, the method involves administering an agent (e.g., an agent identified by a screening assay described herein), or combination of agents that modulates (e.g., up-regulates or down-regulates) NOVX expression or activity. In another embodiment, the method involves administering a NOVX protein or nucleic acid molecule as therapy to compensate for reduced or aberrant NOVX expression or activity.

Stimulation of NOVX activity is desirable in situations in which NOVX is abnormally downregulated and/or in which increased NOVX activity has a beneficial effect. One example of such a situation is where a subject has a disorder characterized by aberrant cell proliferation and/or differentiation (e.g., cancer or immune associated disorders). Another example of such a situation is where the subject has a gestational disease (e.g., preclampsia).

### Determination of the Biological Effect of the Therapeutic

In various embodiments of the invention, suitable *in vitro* or *in vivo* assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Prophylactic and Therapeutic Uses of the Compositions of the Invention

The NOVX nucleic acids and proteins of the invention are useful in potential prophylactic and therapeutic applications implicated in a variety of disorders including, but not limited to: metabolic disorders, diabetes, obesity, infectious disease, anorexia, cancer-associated cancer, neurodegenerative disorders, Alzheimer's Disease, Parkinson's Disorder, immune disorders, hematopoietic disorders, and the various dyslipidemias, metabolic disturbances associated with obesity, the metabolic syndrome X and wasting disorders associated with chronic diseases and various cancers.

As an example, a cDNA encoding the NOVX protein of the invention may be useful in gene therapy, and the protein may be useful when administered to a subject in need thereof. By way of non-limiting example, the compositions of the invention will have efficacy for treatment of patients suffering from: metabolic disorders, diabetes, obesity, infectious disease, anorexia, cancer-associated cachexia, cancer, neurodegenerative disorders, Alzheimer's Disease, Parkinson's Disorder, immune disorders, hematopoietic disorders, and the various dyslipidemias.

Both the novel nucleic acid encoding the NOVX protein, and the NOVX protein of the invention, or fragments thereof, may also be useful in diagnostic applications, wherein the presence or amount of the nucleic acid or the protein are to be assessed. A further use could be as an anti-bacterial molecule (i.e., some peptides have been found to possess anti-bacterial properties). These materials are further useful in the generation of antibodies, which immunospecifically-bind to the novel substances of the invention for use in therapeutic or diagnostic methods.

### EXAMPLES

### Example A: Polynucleotide and Polypeptide Sequences, and Homology Data

### Example 1.

The NOV1 clone was analyzed, and the nucleotide and encoded polypeptide sequences are shown in Table 1A.

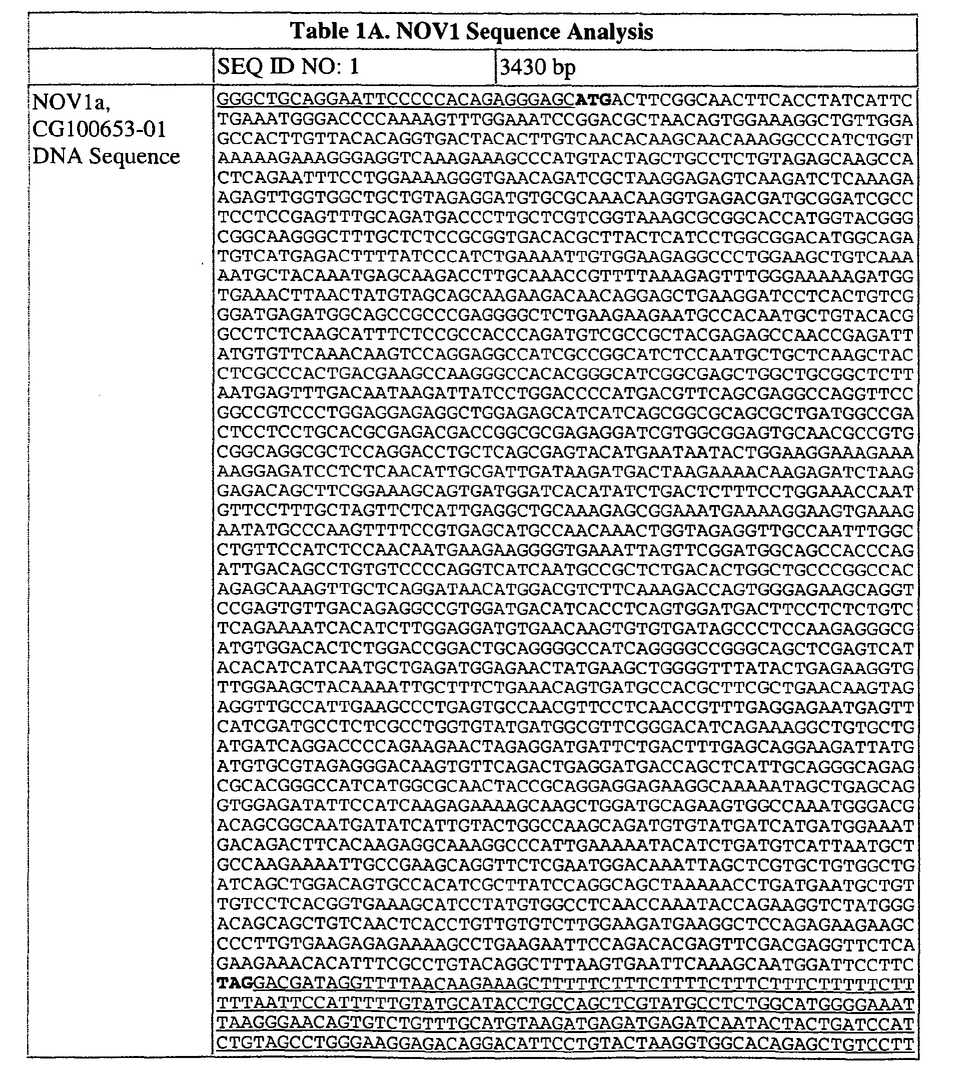

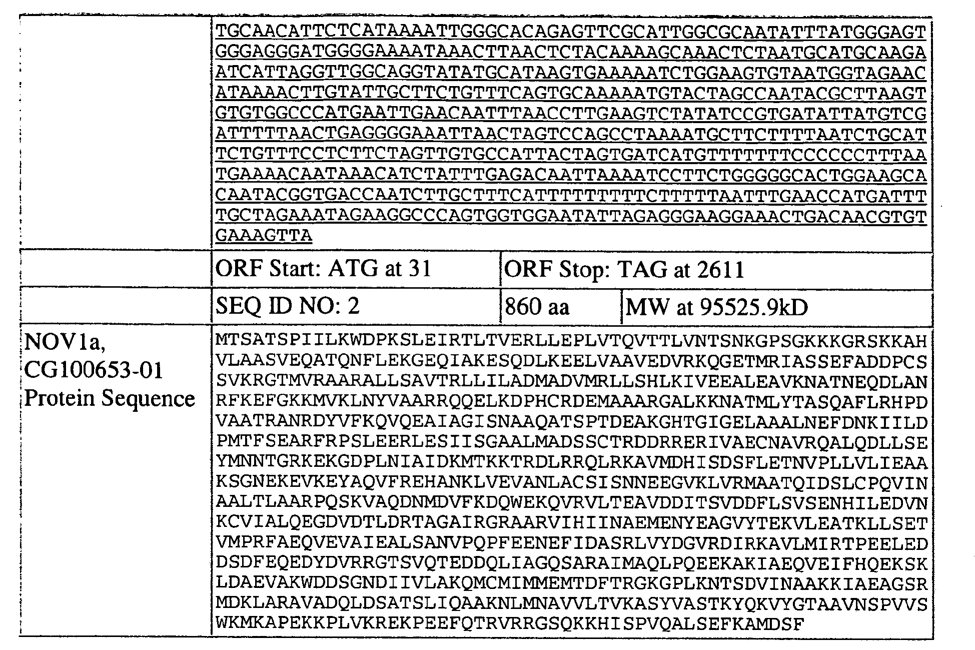

Further analysis of the NOV1a protein yielded the following properties shown in Table 1B.

| **Table 1B. Protein Sequence Properties NOV1a** | |
|---|---|
| PSort analysis: | 0.3600 probability located in mitochondrial matrix space; 0.3000 probability located in microbody (peroxisome); 0.1000 probability located in lysosome (lumen); 0.0000 probability located in endoplasmic reticulum (membrane) |
| SignalP analysis: | No Known Signal Sequence Predicted |

A search of the NOVIa protein against the Geneseq database, a proprietary database that contains sequences published in patents and patent publication, yielded several homologous proteins shown in Table 1C.

| **Table 1C. Geneseq Results for NOV1a** | | | | |
|---|---|---|---|---|
| **Geneseq Identifier** | **Protein/Organism/Length [Patent #, Date]** | **NOV1a Residues/ Match Residues** | **Identities/ Similarities for the Matched Region** | **Expect Value** |
| AAR58778 | Neural alpha-catenin protein - Homo sapiens, 906 aa. [JP06211898-A, 02-AUG-1994] | 1..860 | 851/906 (93%) | 0.0 |
| | | 1..906 | 855/906 (93%) | |
| AAY07060 | Renal cancer associated antigen precursor sequence - Homo sapiens, 906 aa. [WO9904265-A2, 28-JAN-1999] | 8..859 | 694/899 (77%) | 0.0 |
| | | 9..905 | 773/899 (85%) | |
| AAU32945 | Novel human secreted protein #3436 - Homo sapiens, 932 aa. [WO200179449-A2, 25-OCT-2001] | 8..769 | 611/766 (79%) | 0.0 |
| | | 10..773 | 683/766 (88%) | |
| ABG10622 | Novel human diagnostic protein #10613 - Homo sapiens, 932 aa. [W0200175067-A2, 11-OCT-2001] | 8..769 | 610/766 (79%) | 0.0 |
| | | 10..773 | 682/766 (88%) | |
| ABG10622 | Novel human diagnostic protein #10613 - Homo sapiens, 932 aa. [WO200175067-A2, 11-OCT-2001] | 8..769 | 610/766 (79%) | 0.0 |
| | | 10..773 | 682/766 (88%) | |

In a BLAST search of public sequence databases, the NOV1a protein was found to have homology to the proteins shown in the BLASTP data in Table 1D.

| **Table 1D. Public BLASTP Results for NOV1a** | | | | |
|---|---|---|---|---|
| **Protein Accession Number** | **Protein/Organism/Length** | **NOV1a Residues/ Match Residues** | **Identities/ Similarities for the Matched Portion** | **Expect Value** |
| P30997 | Alpha-2 catenin (Alpha N-catenin) (Neural alpha-catenin) - Gallus gallus (Chicken), 906 aa. | 1..860 | 851/906 (93%) | 0.0 |
| | | 1..906 | 855/906 (93%) | |
| I49499 | alpha N-catenin I - mouse, 905 aa. | 1..860 | 850/905 (93%) | 0.0 |
| | | 1..905 | 854/905 (93%) | |
| A45011 | alpha-catenin 2 - human, 945 aa. | 1..769 | 768/770 (99%) | 0.0 |
| | | 1..770 | 768/770 (99%) | |
| P26232 | Alpha-2 catenin (Alpha-catenin related protein) (Alpha N-catenin) - Homo sapiens (Human), 953 aa. | 1..769 | 768/770 (99%) | 0.0 |
| | | 1..770 | 768/770 (99%) | |
| Q61301 | Alpha-2 catenin (Alpha-catenin related protein) (Alpha N-catenin) - Mus musculus (Mouse), 953 aa. | 1..769 | 759/770 (98%) | 0.0 |
| | | 1..770 | 762/770 (98%) | |

PFam analysis predicts that the NOV1a protein contains the domains shown in the Table 1E.

| **Table 1E. Domain Analysis of NOV1a** | | | |
|---|---|---|---|
| **Pfam Domain** | **NOVIa Match Region** | **Identities/ Similarities for the Matched Region** | **Expect Value** |
| Vinculin | 18..765 | 424/948 (45%) | 0 |
| | | 736/948 (78%) | |
| Vinculin | 766..821 | 32/57 (56%) | 5.4e-30 |
| | | 56/57 (98%) | |

### Example 2.

The NOV2 clone was analyzed, and the nucleotide and encoded polypeptide sequences are shown in Table 2A.

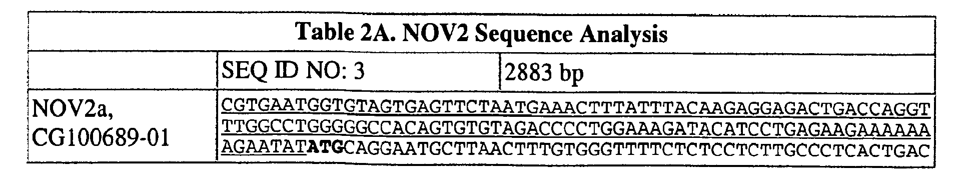

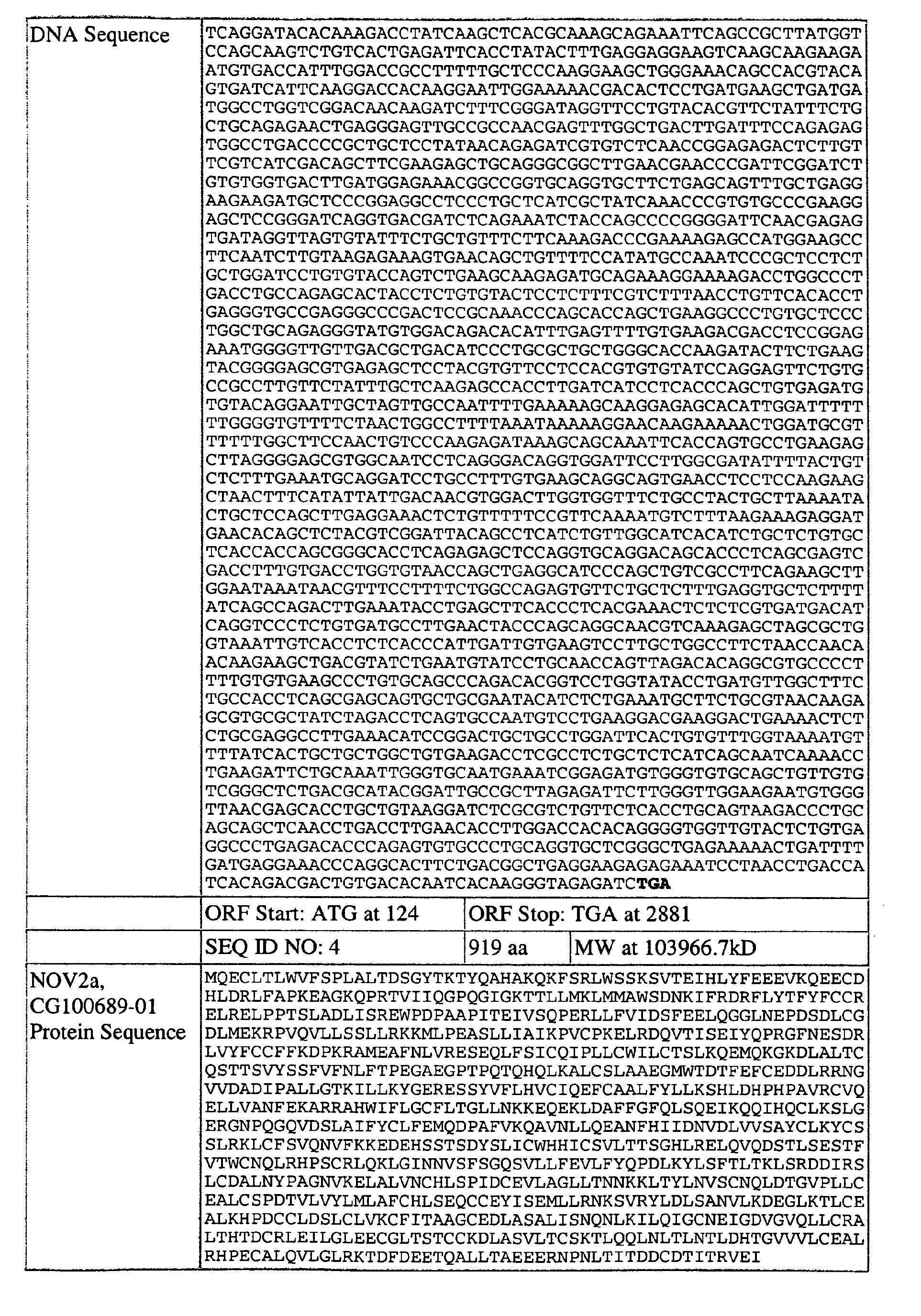

Further analysis of the NOV2a protein yielded the following properties shown in Table 2B.

| **Table 2B. Protein Sequence Properties NOV2a** | |
|---|---|
| PSort analysis: | 0.6000 probability located in nucleus; 0.3000 probability located in microbody (peroxisome); 0.2000 probability located in endoplasmic reticulum (membrane); 0.1000 probability located in mitochondrial inner membrane |
| SignalP analysis: | Cleavage site between residues 17 and 18 |

A search of the NOV2a protein against the Geneseq database, a proprietary database that contains sequences published in patents and patent publication, yielded several homologous proteins shown in Table 2C.

| **Table 2C. Geneseq Results for NOV2a** | | | | |
|---|---|---|---|---|
| **Geneseq Identifier** | **Protein/Organism/Length [Patent #, Date]** | **NOV2a Residues/ Match Residues** | **Identities/ Similarities for the Matched Region** | **Expect Value** |
| AAM50328 | Human nucleotide binding site protein NBS-5 - Homo sapiens, 858 aa. [W0200183753-A2, 08-NOV-2001] | 33..882 | 849/850 (99%) | 0.0 |
| | | 1..850 | 850/850 (99%) | |
| AAU07878 | Polypeptide sequence for mammalian Spg65 - Mammalia, 748 aa. [WO200166752-A2, 13-SEP-2001] | 165..907 | 375/743 (50%) | 0.0 |
| | | 5..744 | 528/743 (70%) | |
| AAE07514 | Human PYRIN-1 protein - Homo sapiens, 1034 aa. [WO200161005-A2, 23-AUG-2001] | 20..907 | 320/926 (34%) | e-146 |
| | | 134..1028 | 491/926 (52%) | |
| AAG65895 | Amino acid sequence of GSK gene Id 97078 - Homo sapiens, 1062 aa. [WO200172961-A2, 04-OCT-2001] | 75..907 | 301/849 (35%) | e-137 |
| | | 208..1043 | 460/849 (53%) | |
| AAE07513 | Human nucleotide binding site 1 (NBS-1) protein - Homo sapiens, 1033 aa. [W0200161005-A2, 23-AUG-2001] | 75..907 | 299/849 (35%) | e-134 |
| | | 180..1014 | 459/849 (53%) | |

In a BLAST search of public sequence databases, the NOV2a protein was found to have homology to the proteins shown in the BLASTP data in Table 2D.

| **Table 2D. Public BLASTP Results for NOV2a** | | | | |
|---|---|---|---|---|
| **Protein Accession Number** | **Protein/Organism/Length** | **NOV2a Residues/ Match Residues** | **Identities/ Similarities for the Matched Portion** | **Expect Value** |
| Q96MN2 | CDNA FLJ32126 FIS, CLONE PEBLM2000112, WEAKLY SIMILAR TO HOMO SAPIENS NUCLEOTIDE-BINDING SITE PROTEIN 1 MRNA - Homo sapiens (Human), 919 aa. | 1..919 | 918/919 (99%) | 0.0 |
| | | 1..919 | 918/919 (99%) | |
| Q96MN2 | NACHT-, LRR- and PYD-containing protein 4 (PAAD and NACHT-containing protein 2) (PYRIN-containing APAF1-like protein 4) (Ribonuclease inhibitor 2) - Homo sapiens (Human), 994 aa. | 18..919 | 900/902 (99%) | 0.0 |
| | | 93..994 | 901/902 (99%) | |
| AAL88672 | RIBONUCLEASE INHIBITOR 2 - Homo sapiens (Human), 916 aa. | 18..919 | 894/902 (99%) | 0.0 |
| | | 15..916 | 897/902 (99%) | |
| CAD19386 | SEQUENCE 7 FROM PATENT WO0183753 - Homo sapiens (Human), 858 aa (fragment). | 33..882 | 849/850 (99%) | 0.0 |
| | | 1..850 | 850/850 (99%) | |
| Q99MW0 | RIBONUCLEASE/ANGIOGENIN INHIBITOR 2 - Mus musculus (Mouse), 748 aa. | 165..907 | 374/743 (50%) | 0.0 |
| | | 5..744 | 528/743 (70%) | |

PFam analysis predicts that the NOV2a protein contains the domains shown in the Table 2E.

| **Table 2E. Domain Analysis of NOV2a** | | | |
|---|---|---|---|
| **Pfam Domain** | **NOV2a Match Region** | **Identities/ Similarities for the Matched Region** | **Expect Value** |
| SRP54 | 71..93 | 11/23 (48%) | 0.18 |
| | | 17/23 (74%) | |

### Example 3.

The NOV3 clone was analyzed, and the nucleotide and encoded polypeptide sequences are shown in Table 3A.

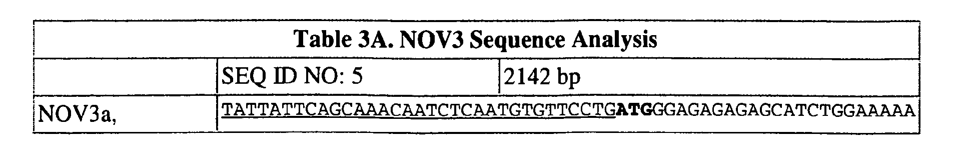

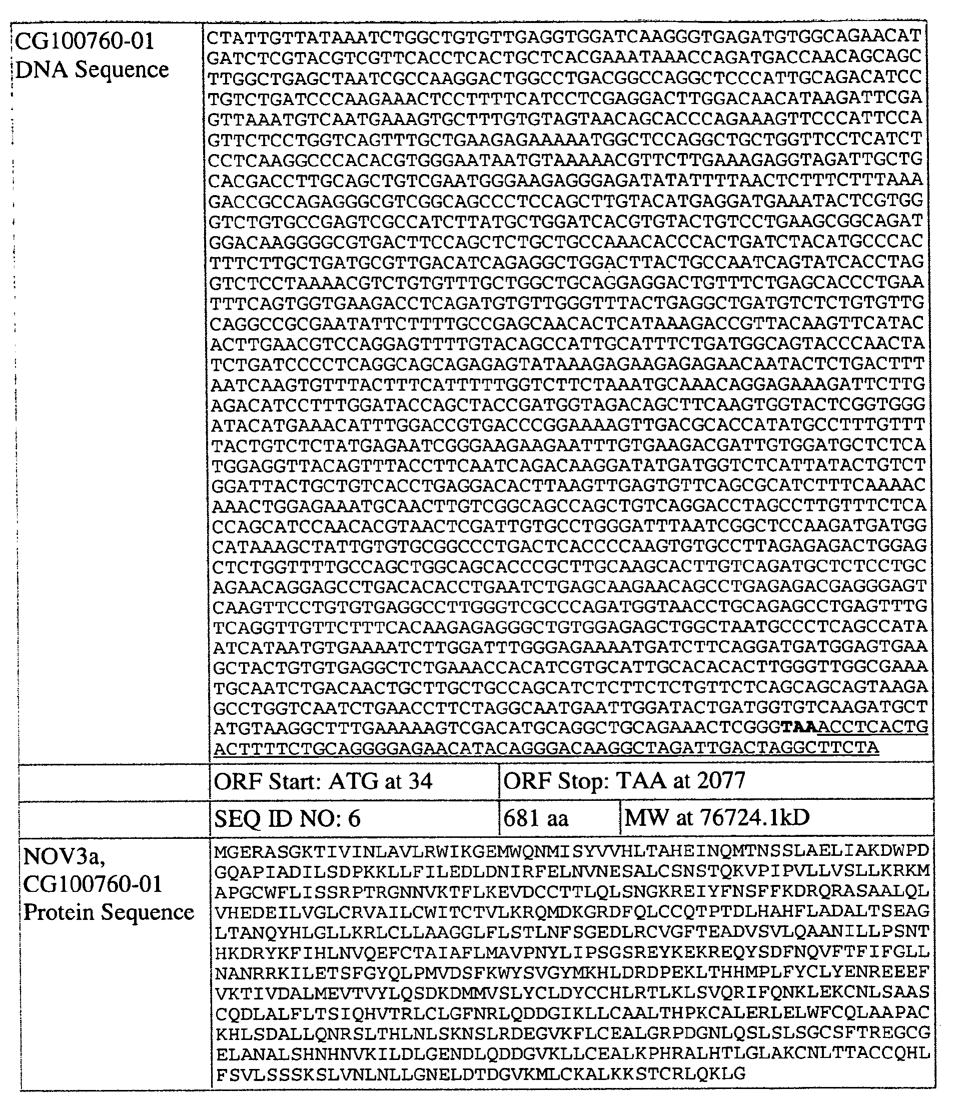

Further analysis of the NOV3a protein yielded the following properties shown in Table 3B.

| **Table 3B. Protein Sequence Properties NOV3a** | |
|---|---|
| PSort analysis: | 0.8200 probability located in endoplasmic reticulum (membrane); 0.1900 probability located in plasma membrane; 0.1000 probability located in endoplasmic reticulum (lumen); 0.1000 probability located in outside |
| SignalP analysis: | Cleavage site between residues 23 and 24 |

A search of the NOV3a protein against the Geneseq database, a proprietary database that contains sequences published in patents and patent publication, yielded several homologous proteins shown in Table 3C.

| **Table 3C. Geneseq Results for NOV3a** | | | | |
|---|---|---|---|---|
| **Geneseq Identifier** | **Protein/Organism/Length [Patent #, Date]** | **NOV3a Residues/ Match Residues** | **Identities/ Similarities for the Matched Region** | **Expect Value** |
| AAM50330 | Human nucleotide binding site protein NBS-3 - Homo sapiens, 875 aa. [W0200183753-A2, 08-NOV-2001] | 1..681 | 539/745 (72%) | 0.0 |
| | | 116..859 | 587/745 (78%) | |
| AAM50326 | Human nucleotide binding site protein NBS-3-Homo sapiens, 631 aa. [WO200183753-A2, 08-NOV-2001] | 1..510 | 469/517 (90%) | 0.0 |
| | | 116..631 | 479/517 (91%) | |
| AAM50328 | Human nucleotide binding site protein NBS-5 - Homo sapiens, 858 aa. [W0200183753-A2, 08-NOV-2001] | 2..681 | 247/750 (32%) | e-105 |
| | | 48..792 | 381/750 (49%) | |
| AAE07514 | Human PYRIN-1 protein - Homo sapiens, 1034 aa. [WO200161005- A2, 23-AUG-2001] | 2..680 | 238/729 (32%) | e-100 |
| | | 224..944 | 362/729 (49%) | |
| ABG03924 | Novel human diagnostic protein #3915 - Homo sapiens, 952 aa. [W0200175067-A2, 11-OCT-2001] | 2..680 | 228/741 (30%) | 7e-78 |
| | | 178..908 | 334/741 (44%) | |

In a BLAST search of public sequence databases, the NOV3a protein was found to have homology to the proteins shown in the BLASTP data in Table 3D.

| **Table 3D. Public BLASTP Results for NOV3a** | | | | |
|---|---|---|---|---|
| **Protein Accession Number** | **Protein/Organism/Length** | **NOV3a Residues/ Match Residues** | **Identities/ Similarities for the Matched Portion** | **Expect Value** |
| CAD19388 | SEQUENCE 15 FROM PATENT WO0183753 - Homo sapiens (Human), 875 aa. | 1..681 | 539/745 (72%) | 0.0 |
| | | 116..859 | 587/745 (78%) | |
| CAD19384 | SEQUENCE 3 FROM PATENT WO0183753 - Homo sapiens (Human), 631 aa (fragment). | 1..510 | 469/517 (90%) | 0.0 |
| | | 116..631 | 479/517 (91%) | |
| CAD19386 | SEQUENCE 7 FROM PATENT WO0183753 - Homo sapiens (Human), 858 aa (fragment). | 2..681 | 247/750 (32%) | e-104 |
| | | 48..792 | 381/750 (49%) | |
| Q96MN2 | CDNA FLJ32126 FIS, CLONE PEBLM2000112, WEAKLY SIMILAR TO HOMO SAPIENS NUCLEOTIDE-BINDING SITE PROTEIN 1 MRNA - Homo sapiens (Human), 919 aa. | 2..681 | 247/750 (32%) | e-104 |
| | | 80..824 | 381/750 (49%) | |
| Q96MN2 | NACHT-, LRR- and PYD-containing protein 4 (PAAD and NACHT- containing protein 2) (PYRIN-containing APAF1-like protein 4) (Ribonuclease inhibitor 2) - Homo sapiens (Human), 994 aa. | 2..681 | 247/750 (32%) | e-104 |
| | | 155..899 | 381/750 (49%) | |

### Example 4.

The NOV4 clone was analyzed, and the nucleotide and encoded polypeptide sequences are shown in Table 4A.

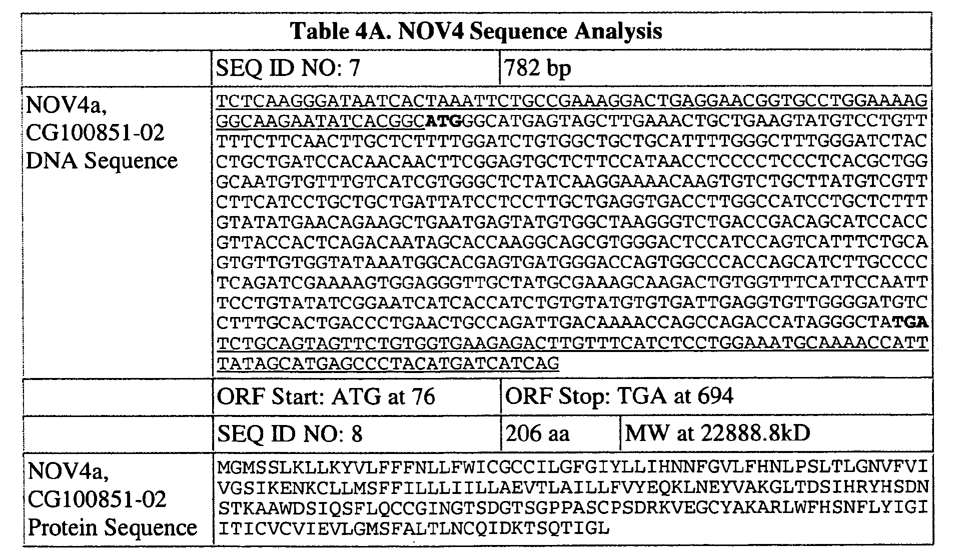

Further analysis of the NOV4a protein yielded the following properties shown in Table 4B.

| **Table 4B. Protein Sequence Properties NOV4a** | |
|---|---|
| PSort analysis: | 0.6400 probability located in plasma membrane; 0.4600 probability located in Golgi body; 0.3700 probability located in endoplasmic reticulum (membrane); |
| | 0.1000 probability located in endoplasmic reticulum (lumen) |
| SignalP analysis: | Cleavage site between residues 32 and 33 |

A search of the NOV4a protein against the Geneseq database, a proprietary database that contains sequences published in patents and patent publication, yielded several homologous proteins shown in Table 4C.

| **Table 4C. Geneseq Results for NOV4a** | | | | |
|---|---|---|---|---|
| **Geneseq Identifier** | **Protein/Organism/Length [Patent #, Date]** | **NOV4a Residues/ Match Residues** | **Identities/ Similarities for the Matched Region** | **Expect Value** |
| AAY96141 | Human haematopoietic CD53 - Homo sapiens, 219 aa. [US6111093-A, 29-AUG-2000] | 1..206 | 205/219 (93%) | e-115 |
| | | 1..219 | 205/219 (93%) | |
| AAB58136 | Lung cancer associated polypeptide sequence SEQ ID 474 - Homo sapiens, 231 aa. [WO200055180-A2, 21-SEP-2000] | 1..206 | 205/219 (93%) | e-115 |
| | | 13..231 | 205/219 (93%) | |
| AAW89152 | Human CD53 antigen - Homo sapiens, 219 aa. [US5849898-A, 15- DEC-1998] | 1..206 | 205/219 (93%) | e-115 |
| | | 1..219 | 205/219 (93%) | |
| AAW80455 | Human CD53 antigen - Homo sapiens, 219 aa. [US5830731-A, 03- NOV-1998] | 1..206 | 205/219 (93%) | e-115 |
| | | 1..219 | 205/219 (93%) | |
| AAR91446 | Human CD53 antigen - Homo sapiens, 219 aa. [US5506126-A, 09- APR-1996] | 1..206 | 205/219 (93%) | e-115 |
| | | 1..219 | 205/219 (93%) | |

In a BLAST search of public sequence databases, the NOV4a protein was found to have homology to the proteins shown in the BLASTP data in Table 4D.

| **Table 4D. Public BLASTP Results for NOV4a** | | | | |
|---|---|---|---|---|
| **Protein Accession Number** | **Protein/Organism/Length** | **NOV4a Residues/ Match Residues** | **Identities/ Similarities for the Matched Portion** | **Expect Value** |
| P19397 | Leukocyte surface antigen CD53 (Cell surface glycoprotein CD53) - Homo sapiens (Human), 219 aa. | 1..206 | 205/219 (93%) | e-115 |
| | | 1..219 | 205/219 (93%) | |
| AAH21310 | CD53 ANTIGEN - Mus musculus (Mouse), 219 aa. | 1..206 | 168/219 (76%) | 6e-95 |
| | | 1..219 | 183/219 (82%) | |
| Q61451 | Leukocyte surface antigen CD53 (Cell surface glycoprotein CD53) - Mus musculus (Mouse), 218 aa. | 2..206 | 167/218 (76%) | 2e-94 |
| | | 1..218 | 182/218 (82%) | |
| A39574 | leukocyte antigen OX-44 - rat, 219 aa. | 1..206 | 164/219 (74%) | 7e-94 |
| | | 1..219 | 183/219 (82%) | |
| P24485 | Leukocyte surface antigen CD53 (Cell surface glycoprotein CD53) (Leukocyte antigen MRC OX-44)-Rattus norvegicus (Rat), 218 aa. | 2..206 | 163/218 (74%) | 3e-93 |
| | | 1..218 | 182/218 (82%) | |

PFam analysis predicts that the NOV4a protein contains the domains shown in the Table 4E.

| **Table 4E. Domain Analysis of NOV4a** | | | |
|---|---|---|---|
| **Pfam Domain** | **NOV4a Match Region** | **Identities/ Similarities for the Matched Region** | **Expect Value** |
| transmembrane4 | 10..36 | 17/27 (63%) | 4.4e-08 |
| | | 27/27 (100%) | |
| transmembrane4 | 58..197 | 52/202 (26%) | 1.2e-44 |
| | | 120/202 (59%) | |

### Example 5.

The NOV5 clone was analyzed, and the nucleotide and encoded polypeptide sequences are shown in Table 5A.

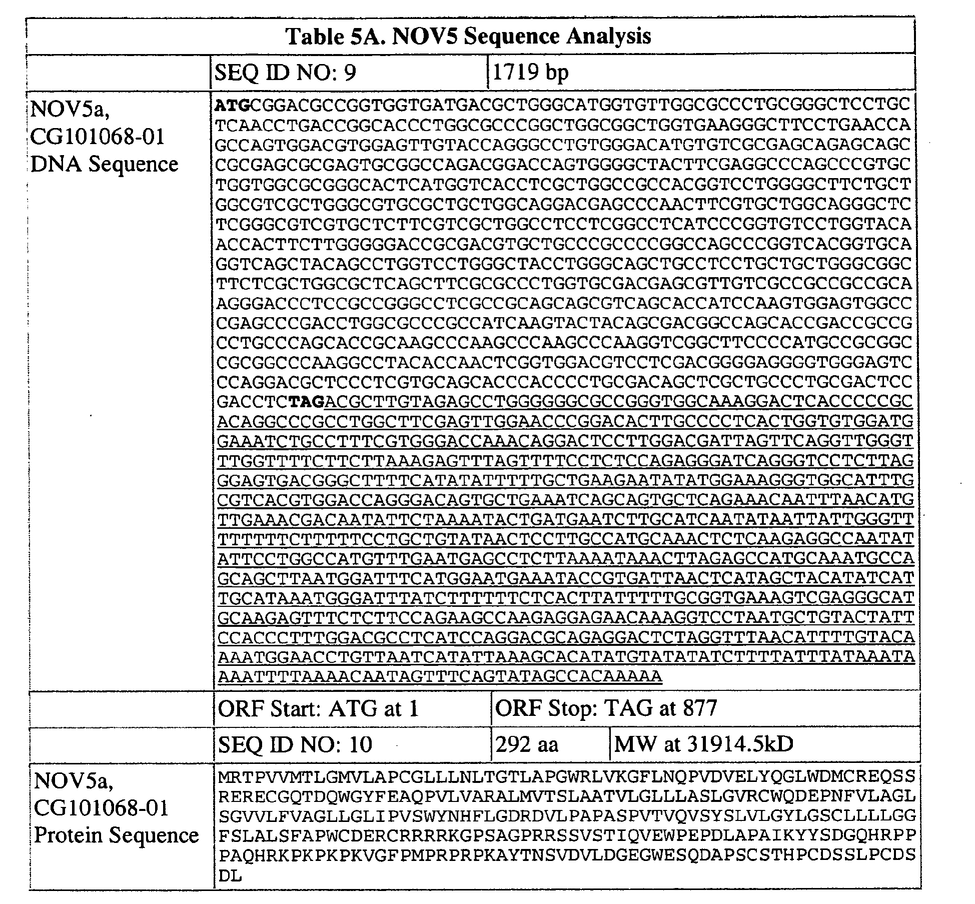

Further analysis of the NOV5a protein yielded the following properties shown in Table 5B.

| **Table 5B. Protein Sequence Properties NOV5a** | |
|---|---|
| PSort analysis: | 0.6400 probability located in plasma membrane; 0.4600 probability located in Golgi body; 0.3700 probability located in endoplasmic reticulum (membrane); 0.1000 probability located in endoplasmic reticulum (lumen) |
| SignalP analysis: | Cleavage site between residues 28 and 29 |

A search of the NOV5a protein against the Geneseq database, a proprietary database that contains sequences published in patents and patent publication, yielded several homologous proteins shown in Table 5C.

| **Table 5C. Geneseq Results for NOV5a** | | | | |
|---|---|---|---|---|
| **Geneseq Identifier** | **Protein/Organism/Length [Patent #, Date]** | **NOV5a Residues/ Match Residues** | **Identities/ Similarities for the Matched Region** | **Expect Value** |
| AAB64401 | Amino acid sequence of human intracellular signalling molecule INTRA33 - Homo sapiens, 217 aa. [W0200077040-A2,21-DEC-2000] | 9..206 | 74/206 (35%) | 1e-16 |
| | | 9..209 | 101/206 (48%) | |
| AAG75467 | Human colon cancer antigen protein SEQ ID NO:6231 - Homo sapiens, 210 aa. [W0200122920-A2,05-APR-2001] | 6..187 | 59/188 (31%) | 2e-13 |
| | | 7..192 | 92/188 (48%) | |
| ABB50278 | Claudin 4 ovarian tumour marker protein, SEQ ID NO:45 - Homo sapiens, 209 aa. [WO200175177-A2, 11-OCT-2001] | 6..187 | 59/188 (31%) | 2e-13 |
| | | 6..191 | 92/188 (48%) | |
| AAB43133 | Human ORFX ORF2897 polypeptide sequence SEQ ID NO:5794 - Homo sapiens, 209 aa. [WO200058473-A2, 05-OCT-2000] | 6..187 | 59/188 (31%) | 2e-13 |
| | | 6..191 | 92/188 (48%) | |
| ABB50396 | Human secreted protein encoded by gene 96 SEQ ID NO:344 - Homo sapiens, 202 aa. [WO200162891-A2, 30-AUG-2001] | 9..187 | 59/185 (31%) | 2e-13 |
| | | 1..183 | 91/185 (48%) | |

In a BLAST search of public sequence databases, the NOV5a protein was found to have homology to the proteins shown in the BLASTP data in Table 5D.

| **Table 5D. Public BLASTP Results for NOV5a** | | | | |
|---|---|---|---|---|
| **Protein Accession Number** | **Protein/Organism/Length** | **NOV5a Residues/ Match Residues** | **Identities/ Similarities for the Matched Portion** | **Expect Value** |
| Q96B33 | SIMILAR TO RIKEN NA 2310014B08 GENE - Homo sapiens (Human), 268 aa (fragment). | 28..292 | 252/267 (94%) | e-147 |
| | | 2..268 | 254/267 (94%) | |
| Q9D7D7 | 2310014B08RIK PROTEIN (RIKEN CDNA 2310014B08 GENE) - Mus musculus (Mouse), 296 aa. | 1..292 | 230/296 (77%) | e-135 |
| | | 1..296 | 248/296 (83%) | |
| 095484 | Claudin-9 - Homo sapiens (Human), 217 aa. | 9..206 | 74/206 (35%) | 4e-16 |
| | | 9..209 | 101/206 (48%) | |
| Q9Z0S7 | Claudin-9 - Mus musculus (Mouse), 217 aa. | 9..206 | 71/206 (34%) | 1e-14 |
| | | 9..209 | 99/206 (47%) | |
| Q98SR2 | CLAUDIN-3 - Gallus gallus (Chicken), 214 aa. | 10..206 | 64/202 (31%) | 1e-13 |
| | | 9..207 | 99/202 (48%) | |

PFam analysis predicts that the NOV5a protein contains the domains shown in the Table 5E.

| **Table 5E. Domain Analysis of NOV5a** | | | |
|---|---|---|---|
| **Pfam Domain** | **NOV5a Match Region** | **Identities/ Similarities for the Matched Region** | **Expect Value** |
| PMP22_Claudin | 3..177 | 40/194 (21%) | 0.00018 |
| | | 108/194 (56%) | |

### Example 6.

The NOV6 clone was analyzed, and the nucleotide and encoded polypeptide sequences are shown in Table 6A.

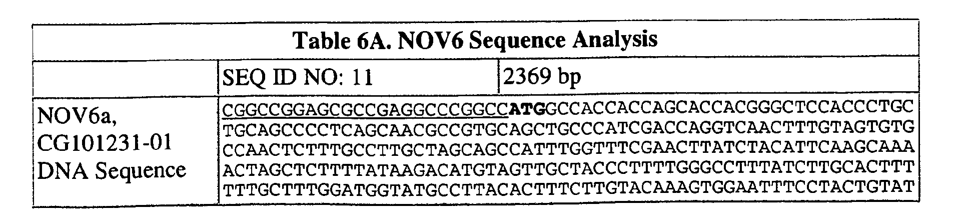

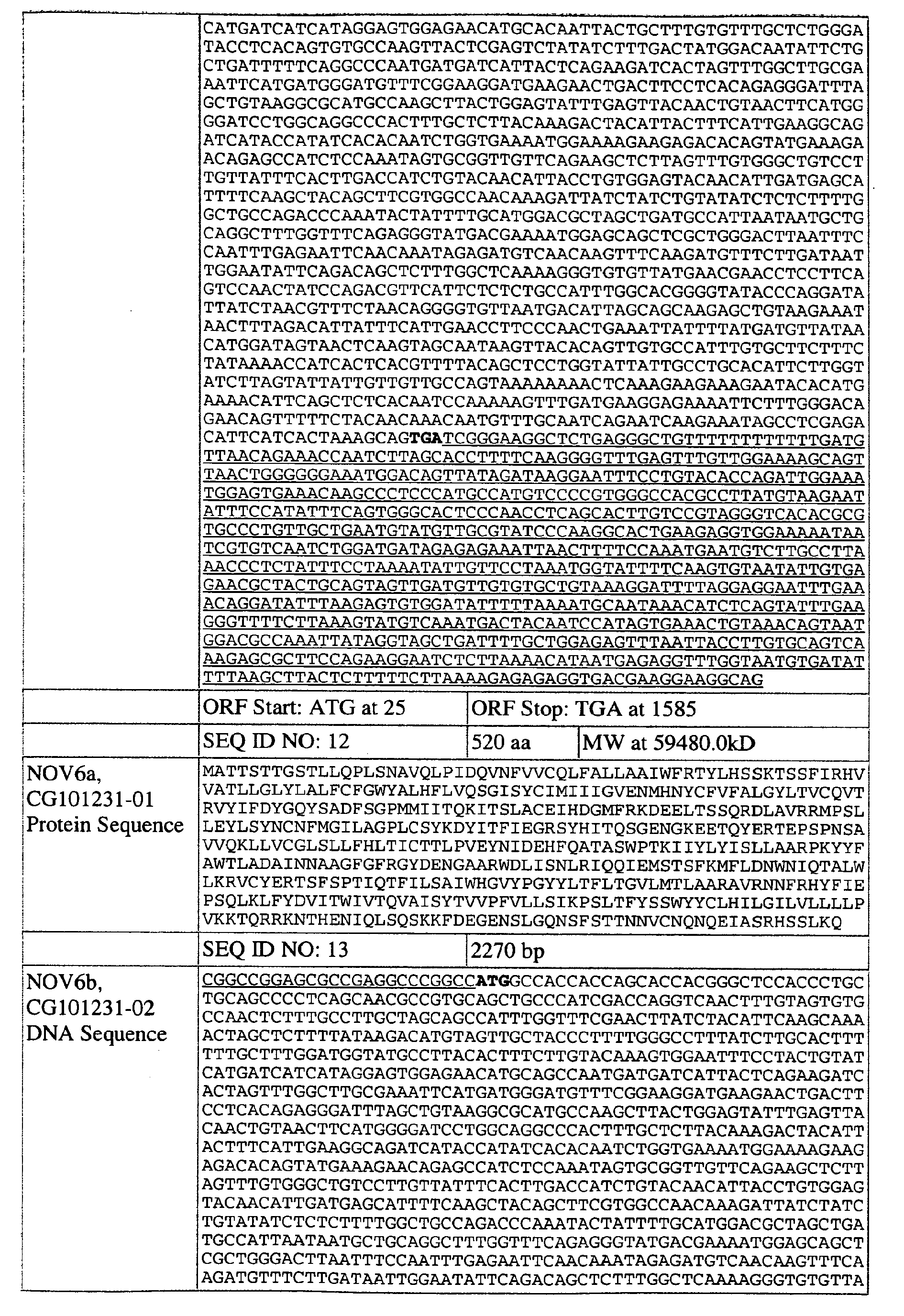

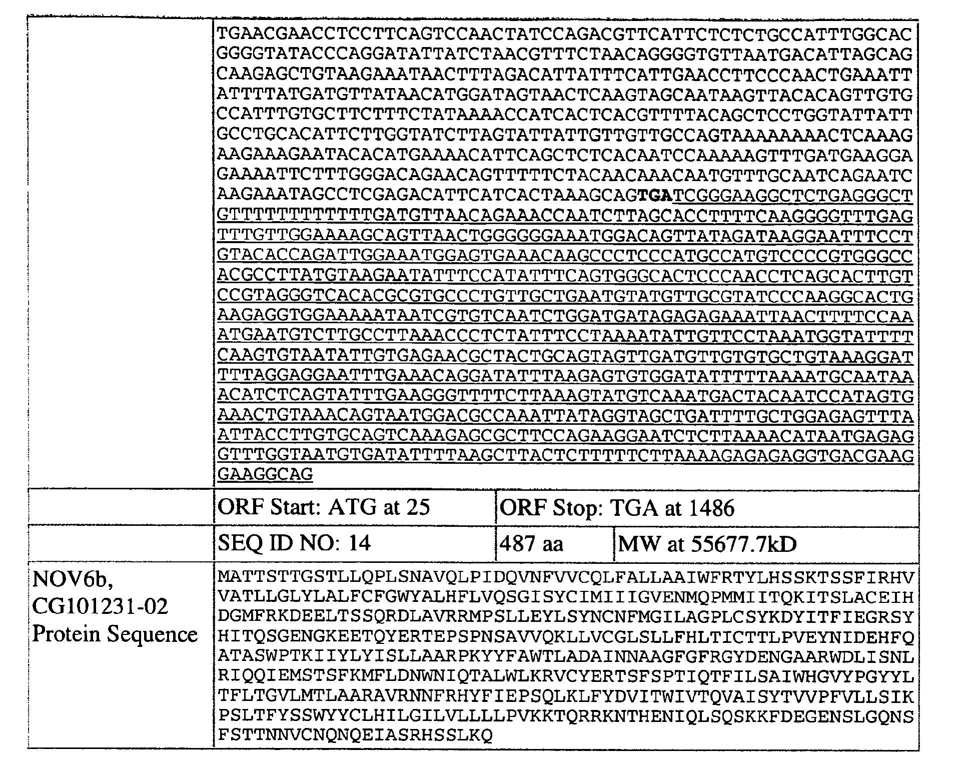

Sequence comparison of the above protein sequences yields the following sequence relationships shown in Table 6B.

| **Table 6B. Comparison of NOV6a against NOV6b.** | | |
|---|---|---|
| **Protein Sequence** | **NOV6a Residues/ Match Residues** | **Identities/ Similarities for the Matched Region** |
| NOV6b | 1..520 | 474/520 (91%) |
| | 1..487 | 474/520 (91%) |

Further analysis of the NOV6a protein yielded the following properties shown in Table 6C.

| **Table 6C. Protein Sequence Properties NOV6a** | |
|---|---|
| PSort analysis: | 0.6000 probability located in plasma membrane; 0.4000 probability located in Golgi body; 0.3406 probability located in mitochondrial intermembrane space; 0.3384 probability located in mitochondrial inner membrane |
| SignalP analysis: | Cleavage site between residues 44 and 45 |

A search of the NOV6a protein against the Geneseq database, a proprietary database that contains sequences published in patents and patent publication, yielded several homologous proteins shown in Table 6D.

| **Table 6D. Geneseq Results for NOV6a** | | | | |
|---|---|---|---|---|
| **Geneseq Identifier** | **Protein/Organism/Length [Patent #, Date]** | **NOV6a Residues/ Match Residues** | **Identities/ Similarities for the Matched Region** | **Expect Value** |
| AAG81345 | Human AFP protein sequence SEQ ID NO:208 - Homo sapiens, 423 aa. [W0200129221-A2, 26-APR-2001] | 98..520 | 419/423 (99%) | 0.0 |
| | | 1..423 | 421/423 (99%) | |
| AAB93797 | Human protein sequence SEQ ID NO:13560 - Homo sapiens, 432 aa. [EP1074617-A2, 07-FEB-2001] | 102..520 | 416/419 (99%) | 0.0 |
| | | 14..432 | 419/419 (99%) | |
| AAM93974 | Human stomach cancer expressed polypeptide SEQ ID NO 17 - Homo sapiens, 432 aa. [WO200109317-A1, 08-FEB-2001] | 102..520 | 416/419 (99%) | 0.0 |
| | | 14..432 | 419/419 (99%) | |
| ABG04835 | Novel human diagnostic protein #4826 - Homo sapiens, 371 aa. [WO200175067-A2, 11-OCT-2001] | 50..297 | 243/248 (97%) | e-143 |
| | | 23..270 | 246/248 (98%) | |
| ABG04835 | Novel human diagnostic protein #4826 - Homo sapiens, 371 aa. [WO200175067-A2, 11-OCT-2001] | 50..297 | 243/248 (97%) | e-143 |
| | | 23..270 | 246/248 (98%) | |

In a BLAST search of public sequence databases, the NOV6a protein was found to have homology to the proteins shown in the BLASTP data in Table 6E.

| **Table 6E. Public BLASTP Results for NOV6a** | | | | |
|---|---|---|---|---|
| **Protein Accession Number** | **Protein/Organism/Length** | **NOV6a Residues/ Match Residues** | **Identities/ Similarities for the Matched Portion** | **Expect Value** |
| AAH25429 | SIMILAR TO RIKEN CDNA 2810049G06 GENE - Mus musculus (Mouse), 519 aa. | 1..520 | 451/520 (86%) | 0.0 |
| | | 1..519 | 479/520 (91%) | |
| CAC38595 | SEQUENCE 207 FROM PATENT WO0129221 - Homo sapiens (Human), 423 aa. | 98..520 | 419/423 (99%) | 0.0 |
| | | 1..423 | 421/423 (99%) | |
| AAH25020 | RIKEN CDNA 2810049G06 GENE - Mus musculus (Mouse), 487 aa. | 1..520 | 422/520 (81%) | 0.0 |
| | | 1..487 | 449/520 (86%) | |
| Q9CZ73 | 2810049G06RIK PROTEIN - Mus musculus (Mouse), 487 aa. | 1..520 | 421/520 (80%) | 0.0 |
| | | 1..487 | 448/520 (85%) | |
| Q96KY4 | SIMILAR TO RIKEN CDNA 2810049G06 GENE - Homo sapiens (Human), 350 aa. | 171..520 | 348/350 (99%) | 0.0 |
| | | 1..350 | 350/350 (99%) | |

PFam analysis predicts that the NOV6a protein contains the domains shown in the Table 6F.

| **Table 6F. Domain Analysis of NOV6a** | | | |
|---|---|---|---|
| **Pfam Domain** | **NOV6a Match Region** | **Identities/ Similarities for the Matched Region** | **Expect Value** |
| Adeno_Penton_B | 204..222 | 8/20 (40%) | 0.54 |
| | | 17/20 (85%) | |
| MBOAT | 148..442 | 108/334 (32%) | 4.1e-89 |
| | | 225/334 (67%) | |

### Example 7.

The NOV7 clone was analyzed, and the nucleotide and encoded polypeptide sequences are shown in Table 7A.

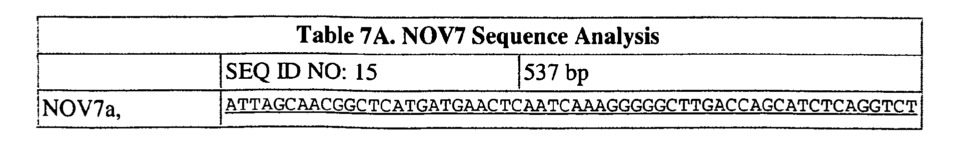

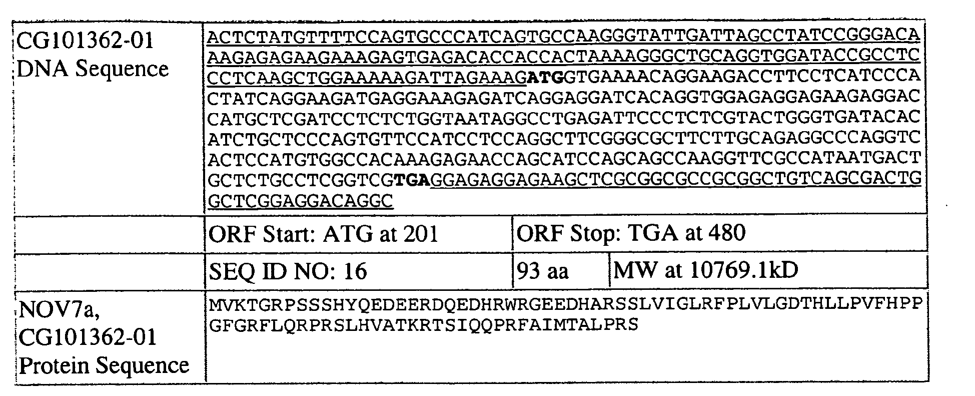

Further analysis of the NOV7a protein yielded the following properties shown in Table 7B.

| **Table 7B. Protein Sequence Properties NOV7a** | |
|---|---|
| PSort analysis: | 0.6400 probability located in microbody (peroxisome); 0.4500 probability located in cytoplasm; 0.2288 probability located in lysosome (lumen); 0.1000 probability located in mitochondrial matrix space |
| SignalP analysis: | No Known Signal Sequence Predicted |

A search of the NOV7a protein against the Geneseq database, a proprietary database that contains sequences published in patents and patent publication, yielded several homologous proteins shown in Table 7C.

| **Table 7C. Geneseq Results for NOV7a** | | | | |
|---|---|---|---|---|
| **Geneseq Identifier** | **Protein/Organism/Length [Patent #, Date]** | **NOV7a Residues/ Match Residues** | **Identities/ Similarities for the Matched Region** | **Expect Value** |
| AAY19678 | SEQ ID NO 396 from WO9922243 - Homo sapiens, 133 aa. [WO9922243- A1, 06-MAY-1999] | 1..30 | 14/30 (46%) | 0.94 |
| | | 63..91 | 19/30 (62%) | |
| AAB92467 | Human protein sequence SEQ ID NO:10527 - Homo sapiens, 563 aa. [EP1074617-A2, 07-FEB-2001] | 2..90 | 24/90 (26%) | 1.2 |
| | | 318..398 | 41/90 (44%) | |
| AAU16292 | Human novel secreted protein, Seq ID 1245 - Homo sapiens, 564 aa. [WO200155322-A2, 02-AUG-2001] | 2..90 | 24/90 (26%) | 1.2 |
| | | 319..399 | 41/90 (44%) | |
| ABB50224 | Human transcription factor TRFX-75 - Homo sapiens, 596 aa. [WO200172777-A2, 04-OCT-2001] | 2..90 | 24/90 (26%) | 1.2 |
| | | 351..431 | 41/90 (44%) | |
| AAM33060 | Peptide #7097 encoded by probe for measuring placental gene expression - Homo sapiens, 49 aa. [WO200157272-A2, 09-AUG-2001] | 5..30 | 13/26 (50%) | 1.6 |
| | | 1..25 | 18/26 (69%) | |

In a BLAST search of public sequence databases, the NOV7a protein was found to have homology to the proteins shown in the BLASTP data in Table 7D.

| **Table 7D. Public BLASTP Results for NOV7a** | | | | |
|---|---|---|---|---|
| **Protein Accession Number** | **Protein/Organism/Length** | **NOV7a Residues/ Match Residues** | **Identities/ Similarities for the Matched Portion** | **Expect Value** |
| Q9D3A0 | 6330414C15RIK PROTEIN - Mus musculus (Mouse), 150 aa. | 1..30 | 14/30 (46%) | 2.2 |
| | | 51..79 | 19/30 (62%) | |
| Q9Y269 | Protein HSPC020 - Homo sapiens (Human), and, 121 aa. | 1..30 | 14/30 (46%) | 2.2 |
| | | 51..79 | 19/30 (62%) | |
| Q9UKD0 | DNA BINDING PROTEIN P96PIF (GLUCOCORTICOID MODULATORY ELEMENT BINDING PROTEIN 1) - Homo sapiens (Human), 563 aa. | 2..90 | 24/90 (26%) | 2.9 |
| | | 318..398 | 41/90 (44%) | |
| Q9NWH1 | HYPOTHETICAL 61.4 KDA PROTEIN - Homo sapiens (Human), 563 aa. | 2..90 | 24/90 (26%) | 2.9 |
| | | 318..398 | 41/90 (44%) | |
| Q9Y692 | GLUCOCORTICOID MODULATORY ELEMENT BINDING PROTEIN-1- Homo sapiens (Human), 573 aa. | 2..90 | 24/90 (26%) | 3.8 |
| | | 328..408 | 40/90 (43%) | |

### Example 8.

The NOV8 clone was analyzed, and the nucleotide and encoded polypeptide sequences are shown in Table 8A.

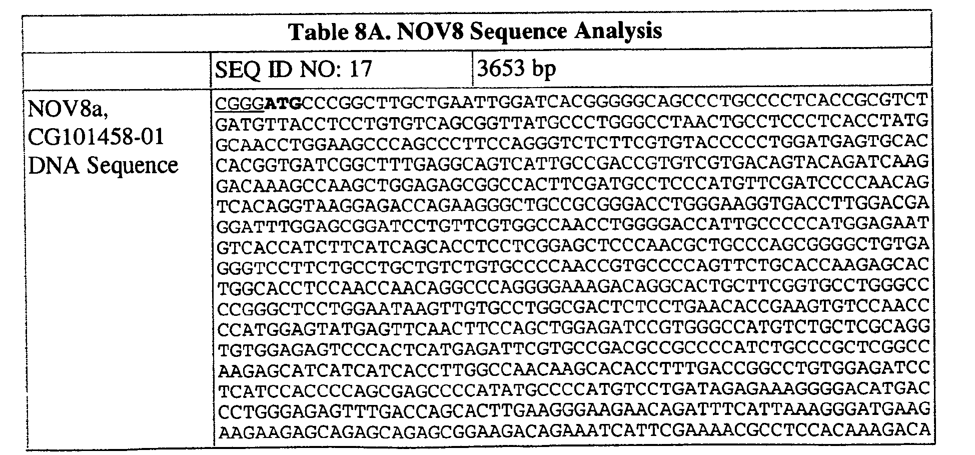

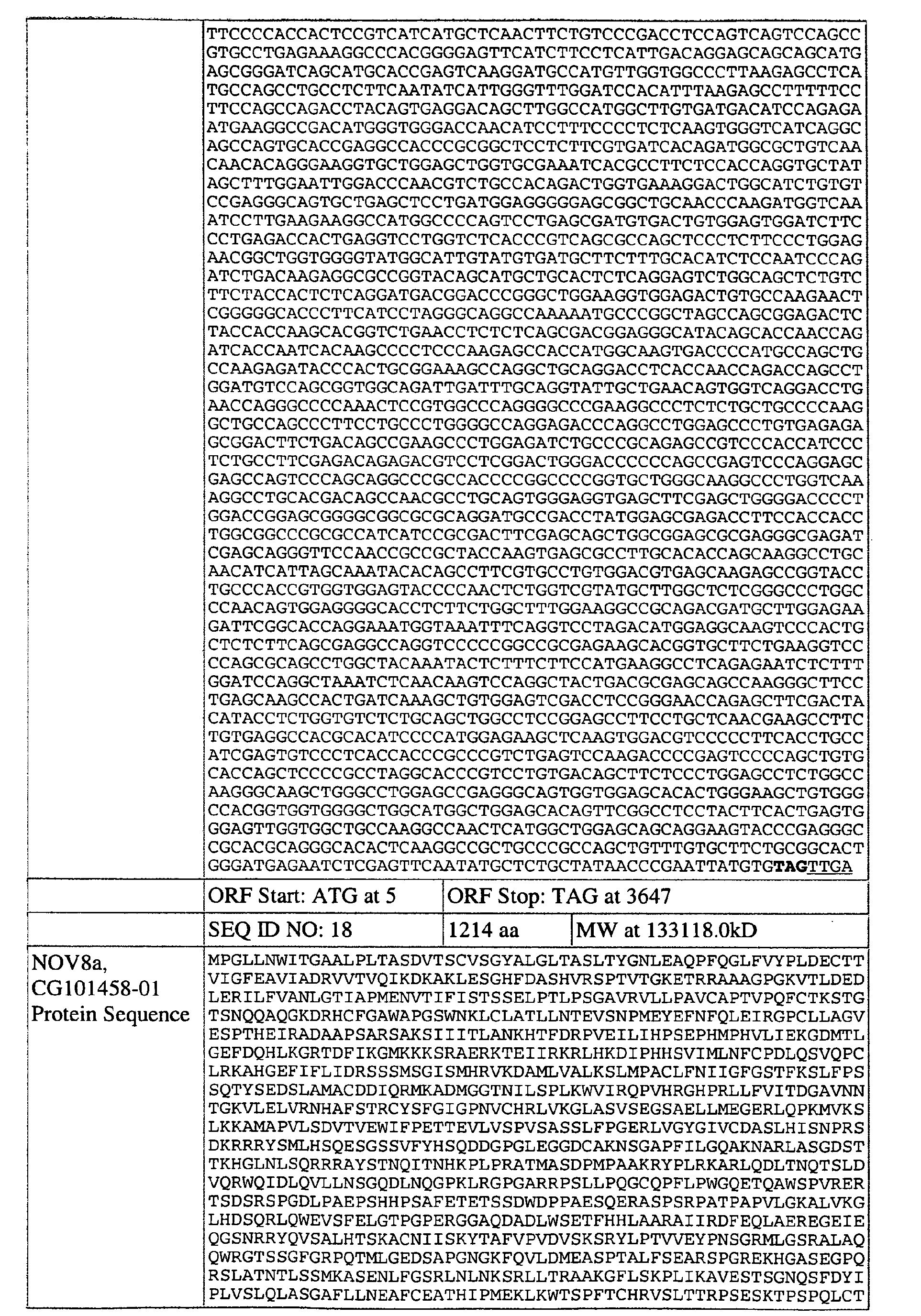

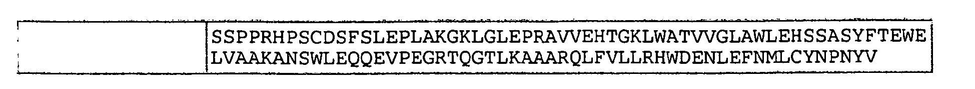

Further analysis of the NOV8a protein yielded the following properties shown in Table 8B.

| **Table 8B. Protein Sequence Properties NOV8a** | |
|---|---|
| PSort analysis: | 0.8700 probability located in nucleus; 0.8500 probability located in endoplasmic reticulum (membrane); 0.7900 probability located in plasma membrane; 0.3325 probability located in microbody (peroxisome) |
| SignalP analysis: | Cleavage site between residues 19 and 20 |

A search of the NOV8a protein against the Geneseq database, a proprietary database that contains sequences published in patents and patent publication, yielded several homologous proteins shown in Table 8C.

| **Table 8C. Geneseq Results for NOV8a** | | | | |
|---|---|---|---|---|
| **Geneseq Identifier** | **Protein/Organism/Length [Patent #, Date]** | **NOV8a Residues/ Match Residues** | **Identities/ Similarities for Expect the Matched Value Region** | |
| AAB82047 | Human mast cell surface antigen - Homo sapiens, 786 aa. [JP2001025388-A, 30-JAN-2001] | 13..565 | 168/565 (29%) | 2e-59 |
| | | 15..500 | 269/565 (46%) | |
| AAY82530 | Human neurotransmitter associated protein sequence SEQ ID NO:6 - Homo sapiens, 210 aa. [W0200012685-A2,09-MAR-2000] | 1034..1211 | 82/194 (42%) | 1e-32 |
| | | 16..207 | 105/194 (53%) | |
| AAU33242 | Novel human secreted protein #3733 - Homo sapiens, 1730 aa. [WO200179449-A2, 25-OCT-2001] | 36..568 | 120/537 (22%) | 4e-23 |
| | | 650..1096 | 214/537 (39%) | |
| AAB51022 | Human minor vault protein p193 - Homo sapiens, 1724 aa. [US6156879-A, 05-DEC-2000] | 36..568 | 120/537 (22%) | 6e-23 |
| | | 644..1090 | 214/537 (39%) | |
| AAY54373 | cDNA sequence encoding the human minor vault protein p193 - Homo sapiens, 1724 aa. [WO9962547-A1, 09-DEC-1999] | 36..568 | 120/537 (22%) | 6e-23 |
| | | 644..1090 | 214/537 (39%) | |

In a BLAST search of public sequence databases, the NOV8a protein was found to have homology to the proteins shown in the BLASTP data in Table 8D.

| **Table 8D. Public BLASTP Results for NOV8a** | | | | |
|---|---|---|---|---|
| **Protein Accession Number** | **Protein/Organism/Length** | **NOV8a Residues/ Match Residues** | **Identities/ Similarities for the Matched Portion** | **Expect Value** |
| Q9CUE8 | 4931403E03RIK PROTEIN - Mus musculus (Mouse), 1209 aa (fragment). | 1..1208 | 883/1218 (72%) | 0.0 |
| | | 1..1209 | 1012/1218 (82%) | |
| Q96M71 | CDNA FLJ32784 FIS, CLONE TESTI2002245 - Homo sapiens (Human), 424 aa. | 588..953 | 362/369 (98%) | 0.0 |
| | | 1..367 | 362/369 (98%) | |
| Q9BVH8 | HYPOTHETICAL 106.2 KDA PROTEIN - Homo sapiens (Human), 1001 aa (fragment). | 274..1211 | 311/1047 (29%) | e-106 |
| | | 32..998 | 467/1047 (43%) | |
| 075668 | DJ745E8.1 (BREAST CANCER SUPPRESSOR CANDIDATE 1 (BCSC-1) LIKE) - Homo sapiens (Human), 148 aa (fragment). | 417..564 | 148/148 (100%) | 4e-80 |
| | | 1..148 | 148/148 (100%) | |
| Q9CTV9 | 5830475I06RIK PROTEIN - Mus musculus (Mouse), 565 aa (fragment). | 13..565 | 165/567 (29%) | 5e-57 |
| | | 15..500 | 259/567 (45%) | |

PFam analysis predicts that the NOV8a protein contains the domains shown in the Table 8E.

| **Table 8E. Domain Analysis of NOV8a** | | | |
|---|---|---|---|
| **Pfam Domain** | **NOV8a Match Region** | **Identities/ Similarities for the Matched Region** | **Expect Value** |
| vwa | 355..523 | 37/203 (18%) | 0.021 |
| | | 107/203 (53%) | |

### Example 9.

The NOV9 clone was analyzed, and the nucleotide and encoded polypeptide sequences are shown in Table 9A.

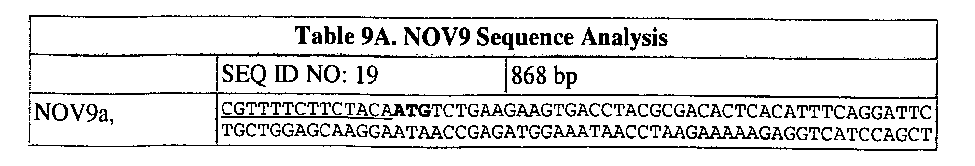

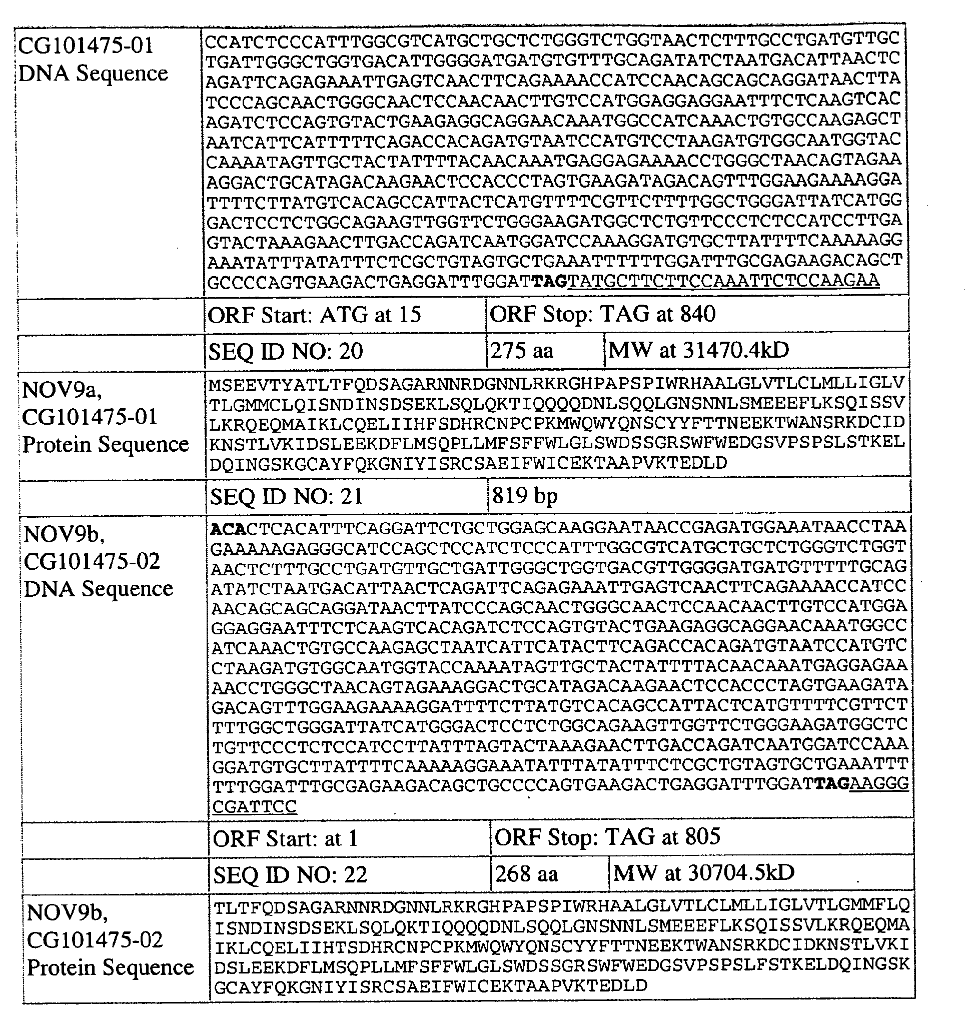

Sequence comparison of the above protein sequences yields the following sequence relationships shown in Table 9B.

| **Table 9B. Comparison of NOV9a against NOV9b.** | | |
|---|---|---|
| **Protein Sequence** | **NOV9a Residues/ Match Residues** | **Identities/ Similarities for the Matched Region** |
| NOV9b | 9..275 | 241/268 (89%) |
| | 1..268 | 241/268 (89%) |

Further analysis of the NOV9a protein yielded the following properties shown in Table 9C.

| **Table 9C. Protein Sequence Properties NOV9a** | |
|---|---|
| PSort analysis: | 0.7900 probability located in plasma membrane; 0.3000 probability located in Golgi body; 0.2000 probability located in endoplasmic reticulum (membrane); 0.1000 probability located in mitochondrial inner membrane |
| SignalP analysis: | Cleavage site between residues 62 and 63 |

A search of the NOV9a protein against the Geneseq database, a proprietary database that contains sequences published in patents and patent publication, yielded several homologous proteins shown in Table 9D.

| **Table 9D. Geneseq Results for NOV9a** | | | | |
|---|---|---|---|---|
| **Geneseq Identifier** | **Protein/Organism/Length [Patent #, Date]** | **NOV9a Residues/ Match Residues** | **Identities/ Similarities for the Matched Region** | **Expect Value** |
| AAU29320 | Human PRO polypeptide sequence #297 - Homo sapiens, 232 aa. [WO200168848-A2, 20-SEP-2001] | 1..227 | 224/227 (98%) | e-131 |
| | | 1..227 | 225/227 (98%) | |
| AAM79324 | Human protein SEQ ID NO 2970 - Homo sapiens, 289 aa. [WO200157190-A2, 09-AUG-2001] | 1..270 | 91/270 (33%) | 3e-37 |
| | | 25..280 | 147/270 (53%) | |
| ABB11776 | Human macrophage Ag homologue, SEQ ID NO:2146 - Homo sapiens, 289 aa. [W0200157188-A2,09-AUG-2001] | 1..270 | 91/270 (33%) | 3e-37 |
| | | 25..280 | 147/270 (53%) | |
| AAM78340 | Human protein SEQ ID NO 1002 - Homo sapiens, 265 aa. [W0200157190-A2,09-AUG-2001] | 1..270 | 88/270 (32%) | 1e-35 |
| | | 1..256 | 147/270 (53%) | |
| AAY02283 | Secreted protein clone br342_11 polypeptide sequence - Homo sapiens, 265 aa. [WO9918127-A1, 15-APR-1999] | 1..270 | 88/270 (32%) | 1e-35 |
| | | 1..256 | 147/270 (53%) | |

In a BLAST search of public sequence databases, the NOV9a protein was found to have homology to the proteins shown in the BLASTP data in Table 9E.

| **Table 9E. Public BLASTP Results for NOV9a** | | | | |
|---|---|---|---|---|
| **Protein Accession Number** | **Protein/Organism/Length** | **NOV9a Residues/ Match Residues** | **Identities/ Similarities for the Matched Portion** | **Expect Value** |
| Q9D403 | 4933425B16RIK PROTEIN - Mus musculus (Mouse), 275 aa. | 1..275 | 197/276 (71%) | e-113 |
| | | 1..275 | 227/276 (81%) | |
| AAL95693 | C-TYPE LECTIN PROTEIN CLL-1 - Homo sapiens (Human), 265 aa. | 1..270 | 88/270 (32%) | 2e-35 |
| | | 1..256 | 147/270 (53%) | |
| Q9NZH3 | C-TYPE LECTIN-LIKE RECEPTOR-1-Homo sapiens (Human), 280 aa. | 28..274 | 83/249 (33%) | 5e-33 |
| | | 36..269 | 131/249 (52%) | |
| Q9XTA8 | LECTIN-LIKE OXIDIZED LDL RECEPTOR - Oryctolagus cuniculus (Rabbit), 278 aa. | 36..272 | 79/247 (31%) | 5e-27 |
| | | 36..278 | 124/247 (49%) | |
| P78380 | LECTIN-LIKE OXIDIZED LDL RECEPTOR - Homo sapiens (Human), 273 aa. | 36..266 | 79/245 (32%) | 3e-24 |
| | | 32..268 | 124/245 (50%) | |

PFam analysis predicts that the NOV9a protein contains the domains shown in the Table 9F.

| **Table 9F. Domain Analysis of NOV9a** | | | |
|---|---|---|---|
| **Pfam Domain** | **NOV9a Match Region** | **Identities/ Similarities for the Matched Region** | **Expect Value** |
| lectin_c | 161..264 | 29/125 (23%) | 7.4e-06 |
| | | 61/125 (49%) | |

### Example 10.

The NOV10 clone was analyzed, and the nucleotide and encoded polypeptide sequences are shown in Table 10A.

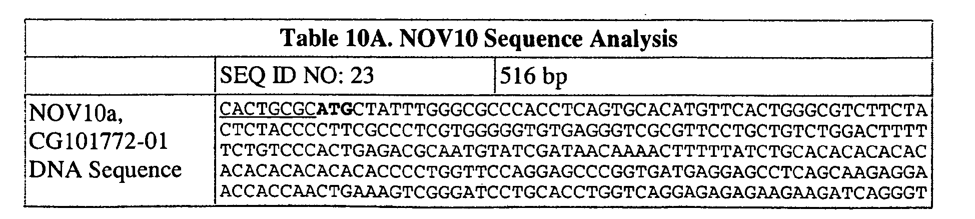

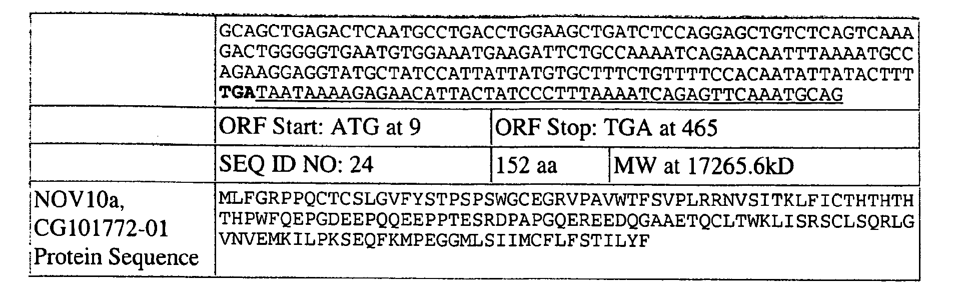

Further analysis of the NOV10a protein yielded the following properties shown in Table 10B.

| **Table 10B. Protein Sequence Properties NOV10a** | |
|---|---|
| PSort analysis: | 0.9190 probability located in plasma membrane; 0.2000 probability located in lysosome (membrane); 0.1021 probability located in microbody (peroxisome); 0.1000 probability located in endoplasmic reticulum (membrane) |
| SignalP analysis: | No Known Signal Sequence Predicted |

A search of the NOV10a protein against the Geneseq database, a proprietary database that contains sequences published in patents and patent publication, yielded several homologous proteins shown in Table 10C.

| **Table 10C. Geneseq Results for NOV10a** | | | | |
|---|---|---|---|---|
| **Geneseq Identifier** | **Protein/Organism/Length [Patent #, Date]** | **NOV10a Residues/ Match Residues** | **Identities/ Similarities for the Matched Region** | **Expect Value** |
| AAM39588 | Human polypeptide SEQ ID NO 2733 - Homo sapiens, 111 aa. [WO200153312-A1, 26-JUL-2001] | 65..136 | 51/78 (65%) | 4e-20 |
| | | 28..105 | 56/78 (71%) | |
| AAM41374 | Human polypeptide SEQ ID NO 6305 - Homo sapiens, 106 aa. [WO200153312-A1, 26-JUL-2001] | 65..135 | 52/77 (67%) | 2e-19 |
| | | 29..105 | 57/77 (73%) | |
| ABG05297 | Novel human diagnostic protein #5288 - Homo sapiens, 112 aa. [WO200175067-A2, 11-OCT-2001] | 65..136 | 48/78 (61%) | 8e-19 |
| | | 29..106 | 56/78 (71%) | |
| ABG05297 | Novel human diagnostic protein #5288 - Homo sapiens, 112 aa. [WO200175067-A2, 11-OCT-2001] | 65..136 | 48/78 (61%) | 8e-19 |
| | | 29..106 | 56/78 (71%) | |
| ABG27048 | Novel human diagnostic protein #27039 - Homo sapiens, 249 aa. [WO200175067-A2, 11-OCT-2001] | 64..135 | 45/78 (57%) | 5e-15 |
| | | 70..147 | 53/78 (67%) | |

In a BLAST search of public sequence databases, the NOV10a protein was found to have homology to the proteins shown in the BLASTP data in Table 10D.

| **Table 10D. Public BLASTP Results for NOV10a** | | | | |
|---|---|---|---|---|
| **Protein Accession Number** | **Protein/Organism/Length** | **NOV10a Residues/ Match Residues Portion** | **Identities/ Similarities for the Matched** | **Expect Value** |
| Q8WTP9 | XAGE-3 PROTEIN - Homo sapiens (Human), 111 aa. | 65..136 | 51/78 (65%) | le-19 |
| | | 28..105 | 56/78 (71%) | |
| Q8WYS9 | HYPOTHETICAL 12.3 KDA PROTEIN - Homo sapiens (Human), 111 aa. | 65..136 | 51/78 (65%) | 1e-19 |
| | | 28..105 | 56/78 (71%) | |
| Q9HD64 | G antigen family D 2 protein (XAGE-1) - Homo sapiens (Human), 146 aa. | 1..136 | 59/149 (39%) | 3e-18 |
| | | 1..140 | 76/149 (50%) | |
| Q8WWM1 | XAGE-5 PROTEIN - Homo sapiens (Human), 108 aa. | 65..136 | 45/78 (57%) | 9e-15 |
| | | 25..102 | 53/78 (67%) | |
| Q96GT9 | SIMILAR TO G ANTIGEN 8 (XAGE-2 PROTEIN) - Homo sapiens (Human), 111 aa. | 65..136 | 39/78 (50%) | 7e-13 |
| | | 28..105 | 53/78 (67%) | |

### Example 11.

The NOV11 clone was analyzed, and the nucleotide and encoded polypeptide sequences are shown in Table 11A.

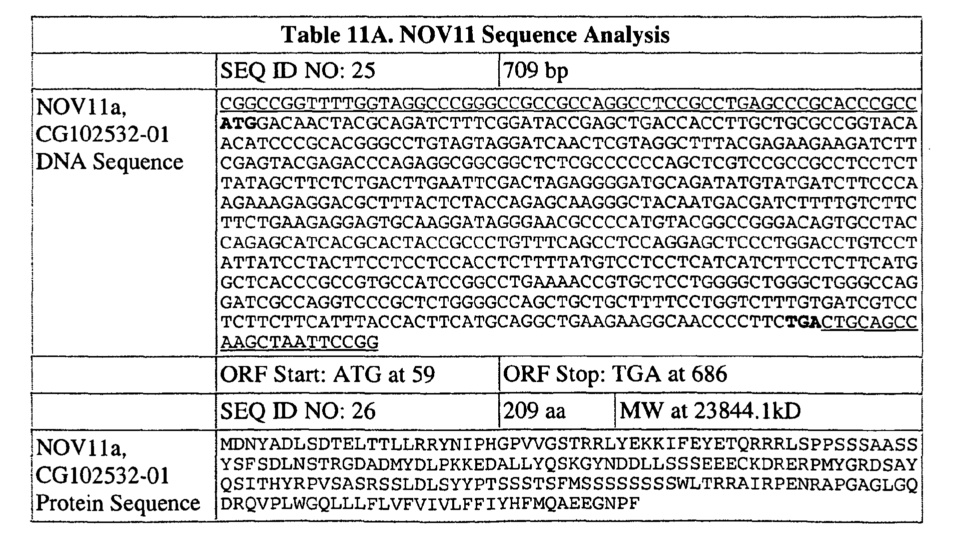

Further analysis of the NOV11a protein yielded the following properties shown in Table 11B.

| **Table 11B. Protein Sequence Properties NOV11a** | |
|---|---|
| PSort analysis: | 0.8500 probability located in endoplasmic reticulum (membrane); 0.6000 probability located in nucleus; 0.4400 probability located in plasma membrane; 0.2323 probability located in microbody (peroxisome) |
| SignalP analysis: | No Known Signal Sequence Predicted |

A search of the NOV11a protein against the Geneseq database, a proprietary database that contains sequences published in patents and patent publication, yielded several homologous proteins shown in Table 11C.

| **Table 11C. Geneseq Results for NOV11a** | | | | |
|---|---|---|---|---|
| **Geneseq Identifier** | **Protein/Organism/Length [Patent #, Date]** | **NOV11a Residues/ Match Residues** | **Identities/ Similarities for the Matched Region** | **Expect Value** |
| AAY41294 | Human emerin sequence (EMD_HU) - Homo sapiens, 254 aa. [WO9954468-A1, 28-OCT-1999] | 1..209 | 209/254 (82%) | e-112 |
| | | 1..254 | 209/254 (82%) | |
| AAG02346 | Human secreted protein, SEQ ID NO: 6427 - Homo sapiens, 51 aa. [EP1033401-A2, 06-SEP-2000] | 1..51 | 51/51 (100%) | 2e-23 |
| | | 1..51 | 51/51 (100%) | |
| AAY41297 | Human thymopoietin gamma sequence - Homo sapiens, 345 aa. [WO9954468-A1, 28-OCT-1999] | 6..209 | 60/231 (25%) | 7e-10 |
| | | 114..333 | 107/231 (45%) | |
| AAR93188 | Thymopoietin-gamma - Homo sapiens, 345 aa. [WO9609526-A1, 28-MAR-1996] | 6..209 | 60/231 (25%) | 7e-10 |
| | | 114..333 | 107/231 (45%) | |
| AAR76499 | Human thymopoietin-gamma - Homo sapiens, 345 aa. [WO9517205-A1, 29-JUN-1995] | 6..209 | 60/231 (25%) | 7e-10 |
| | | 114..333 | 107/231 (45%) | |

In a BLAST search of public sequence databases, the NOV11a protein was found to have homology to the proteins shown in the BLASTP data in Table 11D.

| **Table 11D. Public BLASTP Results for NOV11a** | | | | |
|---|---|---|---|---|
| **Protein Accession Number** | **Protein/Organism/Length** | **NOV11a Residues/ Match Residues** | **Identities/ Similarities for the Matched Portion** | **Expect Value** |
| P50402 | Emerin - Homo sapiens (Human), 254 aa. | 1..209 | 209/254 (82%) | e-111 |
| | | 1..254 | 209/254 (82%) | |
| Q63190 | Emerin - Rattus norvegicus (Rat), 260 aa. | 1..209 | 162/256 (63%) | 1e-81 |
| | | 1..256 | 182/256 (70%) | |
| 008579 | Emerin - Mus musculus (Mouse), 259 aa. | 1..209 | 162/255 (63%) | 1e-81 |
| | | 1..255 | 182/255 (70%) | |
| Q61032 | THYMOPOIETIN GAMMA - Mus musculus (Mouse), 342 aa. | 6..209 | 66/231 (28%) | 2e-11 |
| | | 112..331 | 106/231 (45%) | |
| AAC25390 | THYMOPOIETIN GAMMA - Homo sapiens (Human), 345 aa. | 6..209 | 60/231 (25%) | 2e-09 |
| | | 114..333 | 107/231 (45%) | |

PFam analysis predicts that the NOV11a protein contains the domains shown in the Table 11E.

| **Table 11E. Domain Analysis of NOV11a** | | | |
|---|---|---|---|
| **Pfam Domain** | **NOV11a Match Region** | **Identities/ Similarities for the Matched Region** | **Expect Value** |
| LEM | 1..44 | 22/47 (47%) | 4.4e-24 |
| | | 43/47 (91 %) | |

### Example 12.

The NOV12 clone was analyzed, and the nucleotide and encoded polypeptide sequences are shown in Table 12A.

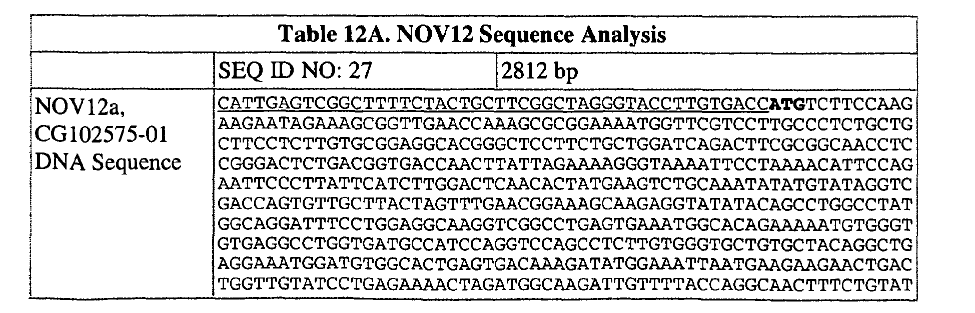

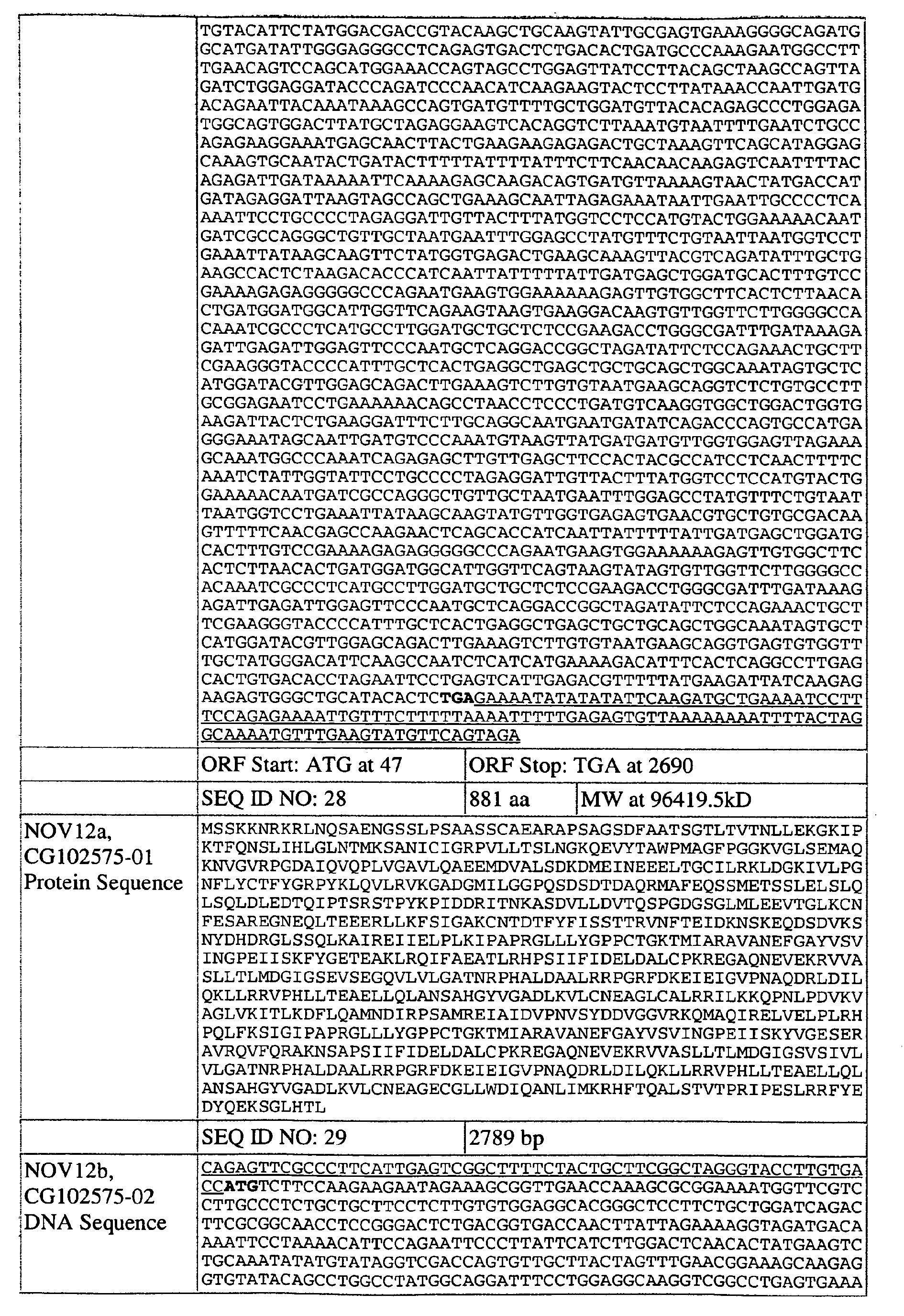

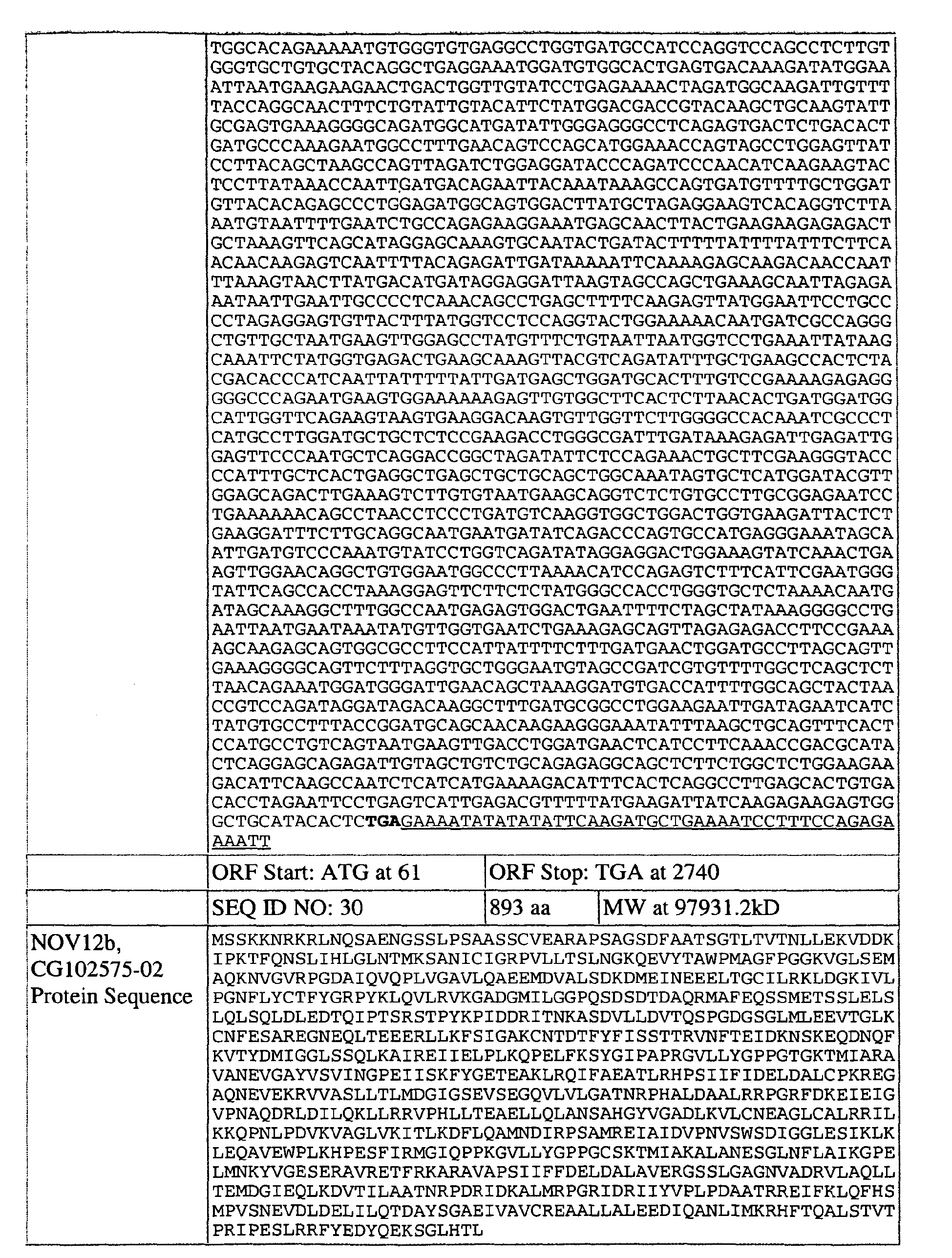

Sequence comparison of the above protein sequences yields the following sequence relationships shown in Table 12B.

| **Table 12B. Comparison of NOV12a against NOV12b.** | | |
|---|---|---|
| **Protein Sequence** | **NOV12a Residues/ Match Residues** | **Identities/ Similarities for the Matched Region** |
| NOV12b | 1..881 | 724/895(80%) |
| | 1..893 | 764/895(84%) |

Further analysis of the NOV12a protein yielded the following properties shown in Table 12C.

| **Table 12C. Protein Sequence Properties NOV12a** | |
|---|---|
| PSort analysis: | 0.7000 probability located in plasma membrane; 0.3000 probability located in microbody (peroxisome); 0.2000 probability located in endoplasmic reticulum (membrane); 0.1000 probability located in mitochondrial inner membrane |
| SignalP analysis: | No Known Signal Sequence Predicted |

A search of the NOV12a protein against the Geneseq database, a proprietary database that contains sequences published in patents and patent publication, yielded several homologous proteins shown in Table 12D.

| **Table 12D. Geneseq Results for NOV12a** | | | | |
|---|---|---|---|---|
| **Geneseq Identifier** | **Protein/Organism/Length [Patent #, Date]** | **NOV12a Residues/ Match Residues** | **Identities/ Similarities for the Matched Region** | **Expect Value** |
| AAU17209 | Novel signal transduction pathway protein, Seq ID 774 - Homo sapiens, 574 aa. [WO200154733-A1, 02-AUG-2001] | 261..823 | 442/575 (76%) | 0.0 |
| | | 2..574 | 481/575 (82%) | |
| AAB59399 | Protein tyrosine phosphatase related sequence - Unidentified, 806 aa. [WO200075339-A1, 14-DEC-2000] | 337..848 | 229/527 (43%) | e-120 |
| | | 190..711 | 340/527 (64%) | |
| AAE09327 | Human intracellular regulatory molecule, VCP - Homo sapiens, 806 aa. [US6274312-B1, 14-AUG-2001] | 337..848 | 229/527 (43%) | e-120 |
| | | 190..711 | 340/527 (64%) | |
| AAB05879 | Human transitional endoplasmic reticulum ATPase protein sequence - Homo sapiens, 806 aa. [WO200034470-A1, 15-JUN-2000] | 337..848 | 229/527 (43%) | e-120 |
| | | 190..711 | 340/527 (64%) | |
| ABB59038 | Drosophila melanogaster polypeptide SEQ ID NO 3906 - Drosophila melanogaster, 801 aa. [WO200171042-A2, 27-SEP-2001] | 322..844 | 228/540 (42%) | e-117 |
| | | 170..704 | 342/540 (63%) | |

In a BLAST search of public sequence databases, the NOV12a protein was found to have homology to the proteins shown in the BLASTP data in Table 12E.

| **Table 12E. Public BLASTP Results for NOV12a** | | | | |
|---|---|---|---|---|
| **Protein Accession Number** | **Protein/Organism/Length** | **NOV12a Residues/ Match Residues** | **Identities/ Similarities for the Matched Portion** | **Expect Value** |
| AAM00262 | SPERMATOGENESIS ASSOCIATED FACTOR - Homo sapiens (Human), 893 aa. | 1..881 | 745/895 (83%) | 0.0 |
| | | 1..893 | 785/895 (87%) | |
| Q9Z2K7 | SPAF - Mus musculus (Mouse), 892 aa. | 1..881 | 640/895 (71%) | 0.0 |
| | | 1..892 | 721/895 (80%) | |
| Q9CXZ7 | 2510048F20RIK PROTEIN - Mus musculus (Mouse), 893 aa. | 1..881 | 640/896 (71%) | 0.0 |
| | | 1..893 | 721/896 (80%) | |
| Q8ZYN4 | AAA FAMILY ATPASE, POSSIBLE CELL DIVISION CONTROL PROTEIN CDC48 -Pyrobaculum aerophilum, 731 aa. | 356..876 | 265/537 (49%) | e-136 |
| | | 184..714 | 358/537 (66%) | |
| Q58556 | Cell division cycle protein 48 homolog MJ1156 - Methanococcus jannaschii, 903 aa. | 309..855 | 271/578 (46%) | e-136 |
| | | 127..697 | 378/578 (64%) | |

PFam analysis predicts that the NOV12a protein contains the domains shown in the Table 12F.

| **Table 12F. Domain Analysis of NOV12a** | | | |
|---|---|---|---|
| **Pfam Domain** | **NOV12a Match Region** | **Identities/ Similarities for the Matched Region** | **Expect Value** |
| AAA | 378..566 | 95/217(44%) | 3.3e-75 |
| | | 165/217 (76%) | |
| AAA | 652..837 | 98/217(45%) | 2e-77 |
| | | 165/217 (76%) | |

### Example 13.

The NOV13 clone was analyzed, and the nucleotide and encoded polypeptide sequences are shown in Table 13A.

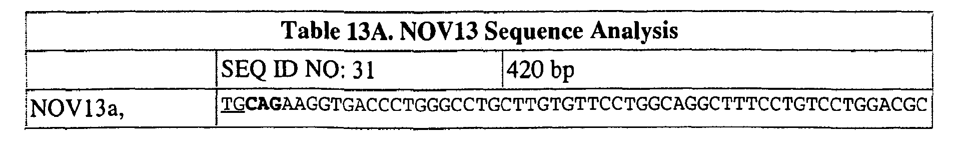

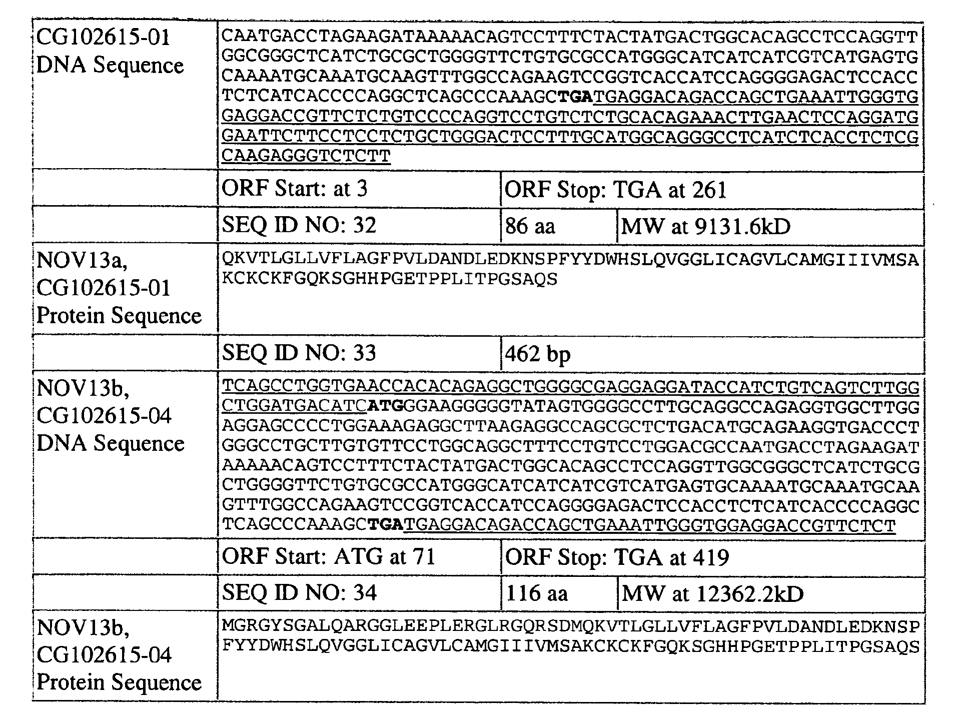

Sequence comparison of the above protein sequences yields the following sequence relationships shown in Table 13B.

| **Table 13B. Comparison of NOV13a against NOV13b.** | | |
|---|---|---|
| **Protein Sequence** | **NOV13a Residues/ Match Residues** | **Identities/ Similarities for the Matched Region** |
| NOV13b | 1..86 | 86/86 (100%) |
| | 31..116 | 86/86 (100%) |

Further analysis of the NOV13a protein yielded the following properties shown in Table 13C.

| **Table 13C. Protein Sequence Properties NOV13a** | |
|---|---|
| PSort analysis: | 0.4600 probability located in plasma membrane; 0.2000 probability located in lysosome (membrane); 0.1000 probability located in endoplasmic reticulum (membrane); 0.1000 probability located in endoplasmic reticulum (lumen) |
| SignalP analysis: | Cleavage site between residues 20 and 21 |

A search of the NOV13a protein against the Geneseq database, a proprietary database that contains sequences published in patents and patent publication, yielded several homologous proteins shown in Table 13D.

| **Table 13D. Geneseq Results for NOV13a** | | | | |
|---|---|---|---|---|
| **Geneseq Identifier** | **Protein/Organism/Length [Patent #, Date]** | **NOV13a Residues/ Match Residues** | **Identities/ Similarities for the Matched Region** | **Expect Value** |
| AAM23962 | Human EST encoded protein SEQ ID NO: 1487 - Homo sapiens, 87 aa. [WO200154477-A2, 02-AUG-2001] | 1..86 | 86/86 (100%) | 7e-47 |
| | | 2..87 | 86/86 (100%) | |
| AAW92959 | Human MAT-8 protein - Homo sapiens, 87 aa. [W09905276-A1, 04-FEB-1999] | 1..86 | 86/86 (100%) | 7e-47 |
| | | 2..87 | 86/86 (100%) | |
| AAY48304 | Human prostate cancer-associated protein 1 - Homo sapiens, 87 aa. [DE19811194-A1, 16-SEP-1999] | 1..86 | 86/86 (100%) | 7e-47 |
| | | 2..87 | 86/86 (100%) | |
| AAR90990 | Human Mat-8 polypeptide - Homo sapiens, 87 aa. [WO9605322-A1, 22-FEB-1996] | 1..86 | 86/86 (100%) | 7e-47 |
| | | 2..87 | 86/86 (100%) | |
| AAB53415 | Human colon cancer antigen protein sequence SEQ ID NO:955 - Homo sapiens, 150 aa. [WO200055351-A1, 21-SEP-2000] | 1..86 | 86/112 (76%) | 2e-42 |
| | | 39..150 | 86/112 (76%) | |

In a BLAST search of public sequence databases, the NOV13a protein was found to have homology to the proteins shown in the BLASTP data in Table 13E.

| **Table 13E. Public BLASTP Results for NOV13a** | | | | |
|---|---|---|---|---|
| **Accession Number** | **Protein/Organism/Length** | **NOV13a Residues/ Match Residues** | **Identities/ Similarities for the Matched Portion** | **Expect Value** |
| Q14802 | FXYD domain-containing ion transport regulator 3 precursor (Chloride conductance inducer protein Mat-8) (Mammary tumor 8 kDa protein) (Phospholemman-like) - Homo sapiens (Human), 87 aa. | 1..86 | 86/86 (100%) | 2e-46 |
| | | 2..87 | 86/86 (100%) | |
| Q61835 | FXYD domain-containing ion transport regulator 3 precursor (Chloride conductance inducer protein Mat-8) (Mammary tumor 8 kDa protein) (Phospholemman-like) - Mus musculus (Mouse), 88 aa. | 1..86 | 63/86 (73%) | 2e-33 |
| | | 2..87 | 72/86 (83%) | |
| 097797 | FXYD domain-containing ion transport regulator 3 precursor (Chloride conductance inducer protein Mat-8) (Mammary tumor 8 kDa protein) - Sus scrofa (Pig), 88 aa. | 2..84 | 60/83 (72%) | 8e-32 |
| | | 3..85 | 68/83 (81%) | |
| Q9D2W0 | FXYD domain-containing ion transport regulator 4 precursor (Channel inducing factor) (CHIF) - Mus musculus (Mouse), 88 aa. | 1..86 | 45/86 (52%) | 4e-21 |
| | | 2..87 | 59/86 (68%) | |
| Q63113 | FXYD domain-containing ion transport regulator 4 precursor (Channel inducing factor) (CHIF) (Corticosteroid-induced protein) -Rattus norvegicus (Rat), 87 aa. | 3..86 | 44/84 (52%) | 7e-20 |
| | | 4..87 | 55/84 (65%) | |

PFam analysis predicts that the NOV13a protein contains the domains shown in the Table 13F.

| **Table 13F. Domain Analysis of NOV13a** | | | |
|---|---|---|---|
| **Pfam Domain** | **NOV13a Match Region** | **Identities/ Similarities for the Matched Region** | **Expect Value** |
| ATP1G1_PLM_MAT8 | 19..74 | 27/57 (47%) | 2.7e-35 |
| | | 55/57 (96%) | |

### Example 14.

The NOV14 clone was analyzed, and the nucleotide and encoded polypeptide sequences are shown in Table 14A.

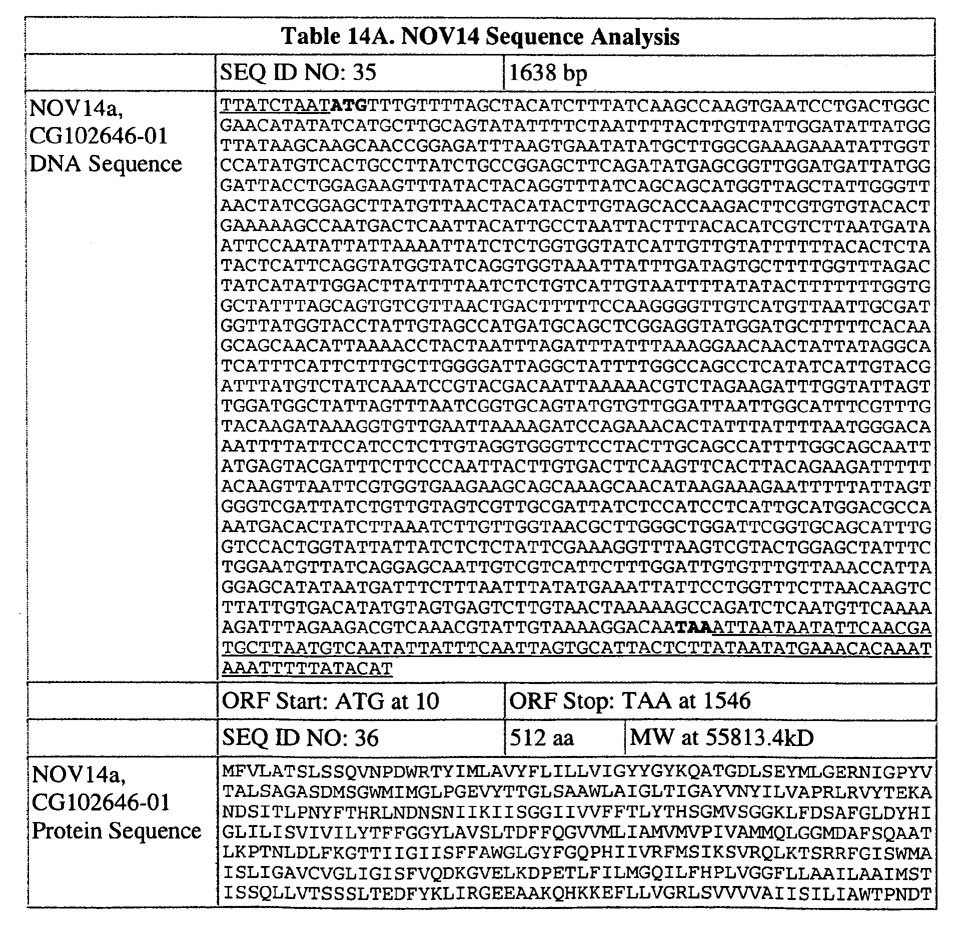

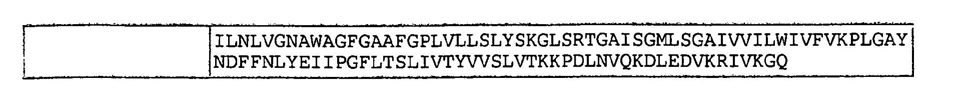

Further analysis of the NOV14a protein yielded the following properties shown in Table 14B.

| **Table 14B. Protein Sequence Properties NOV14a** | |
|---|---|
| PSort analysis: | 0.8200 probability located in plasma membrane; 0.4600 probability located in Golgi body; 0.3700 probability located in endoplasmic reticulum (membrane); 0.1000 probability located in endoplasmic reticulum (lumen) |
| SignalP analysis: | Cleavage site between residues 37 and 38 |

A search of the NOV14a protein against the Geneseq database, a proprietary database that contains sequences published in patents and patent publication, yielded several homologous proteins shown in Table 14C.

| **Table 14C. Geneseq Results for NOV14a** | | | | |
|---|---|---|---|---|
| **Geneseq Identifier** | **Protein/Organism/Length [Patent #, Date]** | **NOV14a Residues/ Match Residues** | **Identities/ Similarities for the Matched Region** | **Expect Value** |
| AAB76757 | Corynebacterium glutamicum MCT protein SEQ ID NO:496 - Corynebacterium glutamicum, 524 aa. [WO200100805-A2, 04-JAN-2001] | 20..506 | 233/502 (46%) | e-127 |
| | | 9..499 | 339/502 (67%) | |
| AAG93195 | C glutamicum protein fragment SEQ ID NO: 6949 - Corynebacterium glutamicum, 524 aa. [EP1108790-A2, 20-JUN-2001] | 20..506 | 233/502 (46%) | e-127 |
| | | 9..499 | 339/502 (67%) | |
| AAW20806 | H. pylori transporter protein, 09ap20802orf27 - Helicobacter pylori, 446 aa. [WO9640893-A1, 19-DEC-1996] | 64..506 | 208/450 (46%) | e-112 |
| | | 5..445 | 306/450 (67%) | |
| AAG82596 | S. epidermidis open reading frame protein sequence SEQ ID NO:2286 - Staphylococcus epidermidis, 408 aa. [WO200134809-A2, 17-MAY-2001] | 266..510 | 171/245 (69%) | 1e-94 |
| | | 163..407 | 208/245 (84%) | |
| AAB96626 | Putative P. abyssi permease #22 - Pyrococcus abyssi, 537 aa. [FR2792651-A1, 27-OCT-2000] | 24..508 | 174/503 (34%) | 4e-83 |
| | | 11..507 | 275/503 (54%) | |

In a BLAST search of public sequence databases, the NOV14a protein was found to have homology to the proteins shown in the BLASTP data in Table 14D.

| **Table 14D. Public BLASTP Results for NOV14a** | | | | |
|---|---|---|---|---|
| **Protein Accession Number** | **Protein/Organism/Length** | **NOV14a Residues/ Match Residues** | **Identities/ Similarities for the Matched Portion** | **Expect Value** |
| Q99SY5 | HIGH AFFINITY PROLINE PERMEASE - Staphylococcus aureus (strain Mu50 / ATCC 700699), and, 512 aa. | 1..510 | 378/510 (74%) | 0.0 |
| | | 1..510 | 443/510 (86%) | |
| 030986 | HIGH AFFINITY PROLINE PERMEASE - Staphylococcus aureus, 497 aa. | 1..494 | 366/494 (74%) | 0.0 |
| | | 1..493 | 431/494 (87%) | |
| Q53584 | PROLINE PERMEASE HOMOLOG - Staphylococcus aureus, 497 aa. | 1..494 | 366/494 (74%) | 0.0 |
| | | 1..493 | 430/494 (86%) | |
| 006493 | Osmoregulated proline transporter (Sodium/proline symporter) - Bacillus subtilis, 492 aa. | 20..494 | 268/478 (56%) | e-158 |
| | | 7..473 | 371/478 (77%) | |
| P94392 | HOMOLOGUE OF PROLINE PERMEASE OF E. COLI - Bacillus subtilis, 449 aa. | 54..504 | 243/452 (53%) | e-142 |
| | | 1..442 | 336/452 (73%) | |

PFam analysis predicts that the NOV 14a protein contains the domains shown in the Table 14E.

| **Table 14E. Domain Analysis of NOV14a** | | | |
|---|---|---|---|
| **Pfam Domain** | **NOV14a Match Region** | **Identities/ Similarities for the Matched Region** | **Expect Value** |
| SSF | 47..447 | 134/449 (30%) | 5.7e-121 |
| | | 318/449 (71%) | |

### Example 15.

The NOV15 clone was analyzed, and the nucleotide and encoded polypeptide sequences are shown in Table 15A.

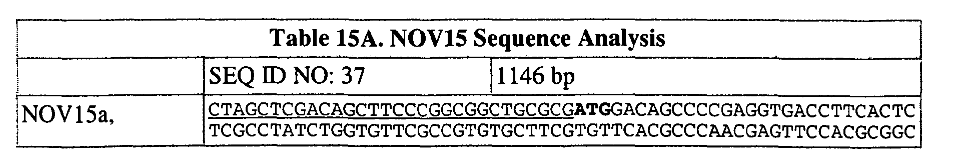

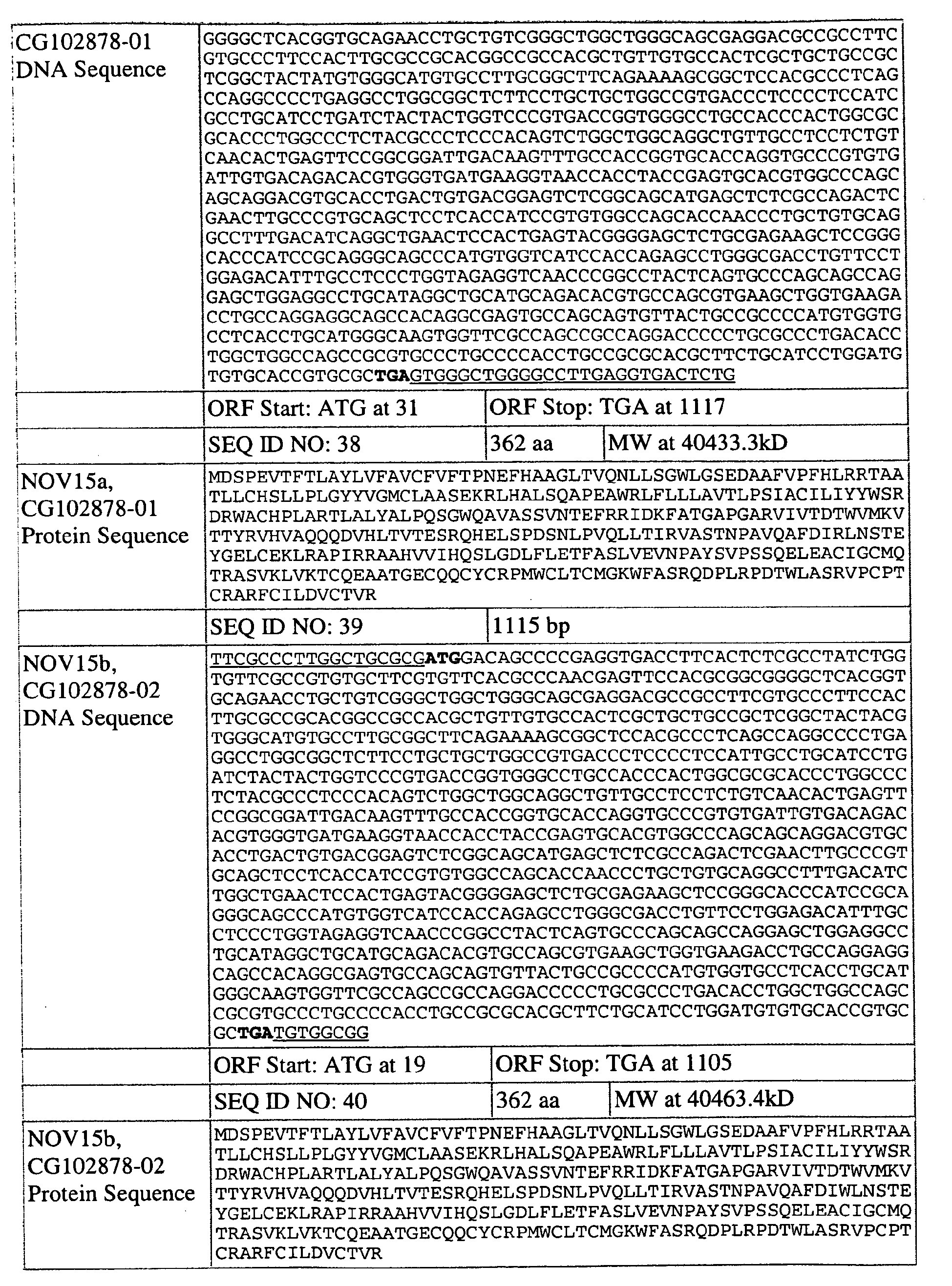

Sequence comparison of the above protein sequences yields the following sequence relationships shown in Table 15B.

| **Table 15B. Comparison of NOV15a against NOV15b.** | | |
|---|---|---|
| **Protein Sequence** | **NOV15a Residues/ Match Residues** | **Identities/ Similarities for the Matched Region** |
| NOV15b | 1..362 | 361/362 (99%) |
| | 1..362 | 361/362 (99%) |

Further analysis of the NOV15a protein yielded the following properties shown in Table 15C.

| **Table 15C. Protein Sequence Properties NOV15a** | |
|---|---|
| PSort analysis: | 0.6760 probability located in plasma membrane; 0.1000 probability located in endoplasmic reticulum (membrane); 0.1000 probability located in endoplasmic reticulum (lumen); 0.1000 probability located in outside |
| SignalP analysis: | Cleavage site between residues 29 and 30 |

A search of the NOV15a protein against the Geneseq database, a proprietary database that contains sequences published in patents and patent publication, yielded several homologous proteins shown in Table 15D.

| **Table 15D. Geneseq Results for NOV15a** | | | | |
|---|---|---|---|---|
| **Geneseq Identifier** | **Protein/Organism/Length [Patent #, Date]** | **NOV15a Residues/ Match Residues** | **Identities/ Similarities for the Matched Region** | **Expect Value** |
| AAG81377 | Human AFP protein sequence SEQ ID NO:272 - Homo sapiens, 362 aa. [W0200129221-A2, 26-APR-2001] | 1..362 | 360/362 (99%) | 0.0 |
| | | 1..362 | 360/362 (99%) | |
| ABB69639 | Drosophila melanogaster polypeptide SEQ ID NO 35709 - Drosophila melanogaster, 409 aa. [WO200171042-A2, 27-SEP-2001] | 1..358 | 122/389 (31%) | 7e-60 |
| | | 1..383 | 200/389 (51%) | |
| AAG23427 | Arabidopsis thaliana protein fragment SEQ ID NO: 26729 - Arabidopsis thaliana, 284 aa. [EP1033405-A2, 06-SEP-2000] | 337..362 | 13/26 (50%) | 2.8 |
| | | 77..102 | 16/26 (61%) | |
| AAG23426 | Arabidopsis thaliana protein fragment SEQ ID NO: 26728 - Arabidopsis thaliana, 413 aa. [EP1033405-A2, 06-SEP-2000] | 337..362 | 13/26 (50%) | 2.8 |
| | | 206..231 | 16/26 (61%) | |
| ABG11786 | Novel human diagnostic protein #11777 - Homo sapiens, 198 aa. [WO200175067-A2, 11-OCT-2001] | 285..354 | 23/89 (25%) | 3.6 |
| | | 54..141 | 37/89 (40%) | |

In a BLAST search of public sequence databases, the NOV15a protein was found to have homology to the proteins shown in the BLASTP data in Table 15E.

| **Table 15E. Public BLASTP Results for NOV15a** | | | | |
|---|---|---|---|---|
| **Protein Accession Number** | **Protein/Organism/Length** | **NOV15a Residues/ Match Residues** | **Identities/ Similarities for the Matched Portion** | **Expect Value** |
| CAC38627 | SEQUENCE 271 FROM PATENT WO0129221 - Homo sapiens (Human), 362 aa. | 1..362 | 361/362 (99%) | 0.0 |
| | | 1..362 | 361/362 (99%) | |
| Q9DCF3 | 0610039G24RIK PROTEIN - Mus musculus (Mouse), 362 aa. | 1..362 | 323/362 (89%) | 0.0 |
| | | 1..362 | 341/362 (93%) | |
| Q96GP5 | SIMILAR TO RIKEN CDNA 0610039G24 GENE - Homo sapiens (Human), 232 aa. | 1..226 | 226/226 (100%) | e-129 |
| | | 1..226 | 226/226 (100%) | |
| Q9VN16 | CG14646 PROTEIN - Drosophila melanogaster (Fruit fly), 409 aa. | 1..358 | 122/389 (31%) | 2e-59 |
| | | 1..383 | 200/389 (51%) | |
| Q95TM4 | LD39811P - Drosophila melanogaster (Fruit fly), 393 aa. | 20..358 | 116/370 (31%) | 1e-55 |
| | | 4..367 | 190/370 (51%) | |

### Example 16.

The NOV16 clone was analyzed, and the nucleotide and encoded polypeptide sequences are shown in Table 16A.

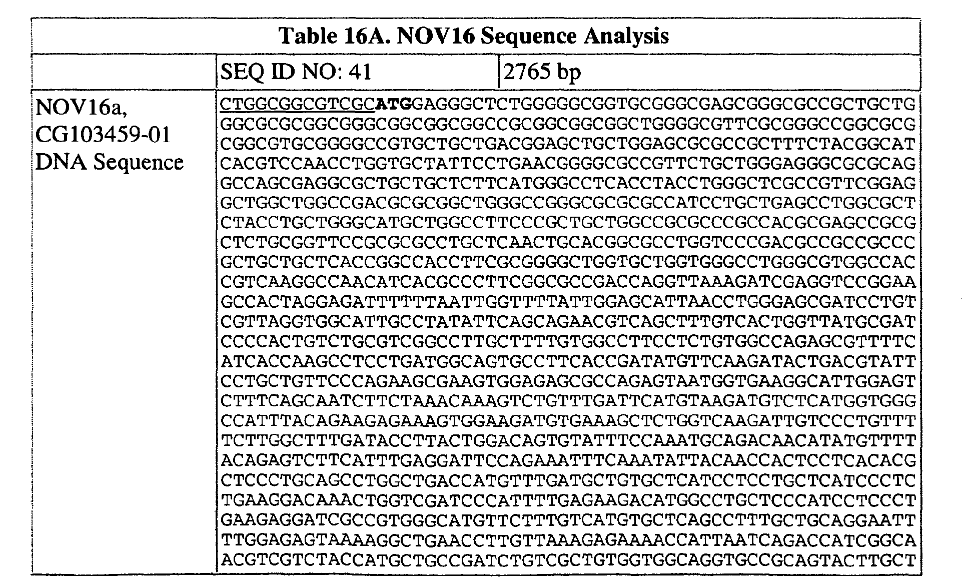

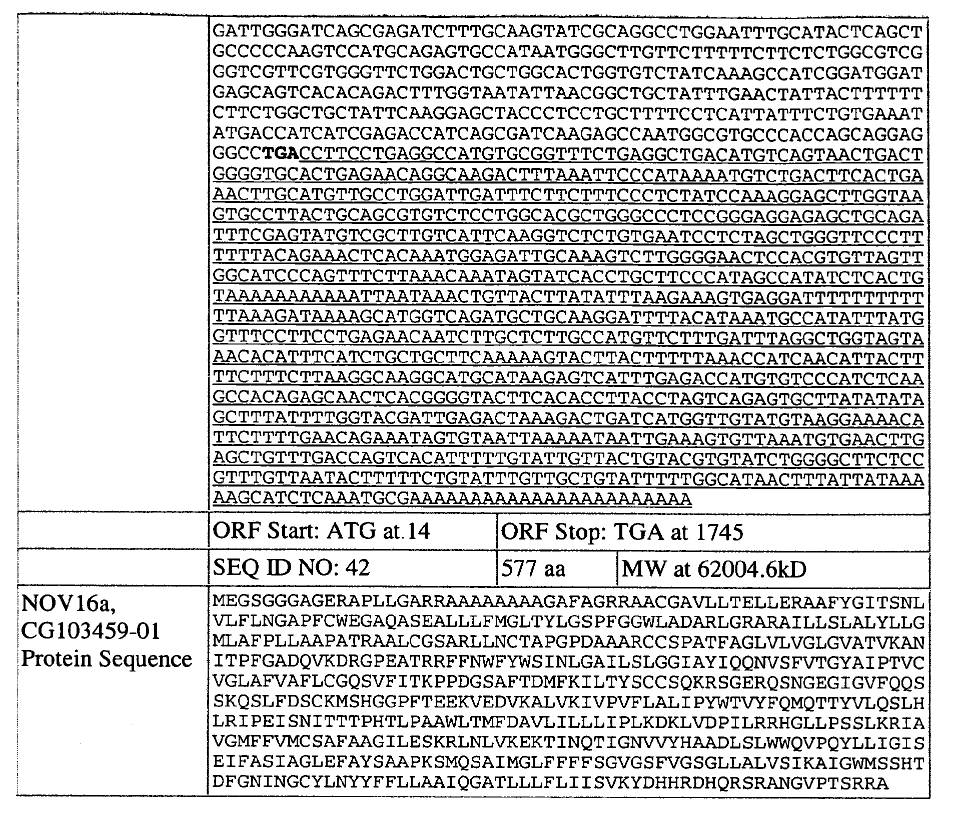

Further analysis of the NOV16a protein yielded the following properties shown in Table 16B.

| **Table 16B. Protein Sequence Properties NOV16a** | |
|---|---|
| PSort analysis: | 0.6000 probability located in plasma membrane; 0.4000 probability located in Golgi body; 0.3000 probability located in endoplasmic reticulum (membrane); 0.3000 probability located in microbody (peroxisome) |
| SignalP analysis: | No Known Signal Sequence Predicted |

A search of the NOV16a protein against the Geneseq database, a proprietary database that contains sequences published in patents and patent publication, yielded several homologous proteins shown in Table 16C.

| **Table 16C. Geneseq Results for NOV16a** | | | | |
|---|---|---|---|---|
| **Geneseq Identifier** | **Protein/Organism/Length [Patent #, Date]** | **NOV16a Residues/ Match Residues** | **Identities/ Similarities for the Matched Region** | **Expect Value** |
| AAU12071 | Human PHT1 variant protein from Caco-2 cells - Homo sapiens, 577 aa. [WO200192468-A2, 06-DEC-2001] | 1..577 | 577/577 (100%) | 0.0 |
| | | 1..577 | 577/577 (100%) | |
| AAU12068 | Human PHT1 protein isolated from Caco-2 cells - Homo sapiens, 577 aa. [W0200192468-A2, 06-DEC-2001] | 1..577 | 577/577 (100%) | 0.0 |
| | | 1..577 | 577/577 (100%) | |
| AAU12070 | Human PHT1 variant protein from BeWo cells - Homo sapiens, 577 aa. [WO200192468-A2, 06-DEC-2001] | 1..577 | 575/577 (99%) | 0.0 |
| | | 1..577 | 576/577 (99%) | |
| AAE16771 | Human transporter and ion channel-8 (TRICH-8) protein - Homo sapiens, 576 aa. [WO200192304-A2, 06-DEC-2001] | 1..577 | 576/577 (99%) | 0.0 |
| | | 1..576 | 576/577 (99%) | |
| AAB82821 | Human proton/oligonucleotide transporter hPHT1 polypeptide - Homo sapiens, 556 aa. [WO200160854-A1, 23-AUG-2001] | 22..577 | 555/556 (99%) | 0.0 |
| | | 1..556 | 555/556 (99%) | |

In a BLAST search of public sequence databases, the NOV16a protein was found to have homology to the proteins shown in the BLASTP data in Table 16D.

| **Table 16D. Public BLASTP Results for NOV16a** | | | | |
|---|---|---|---|---|
| **Protein Accession Number** | **Protein/Organism/Length** | **NOV16a Residues/ Match Residues** | **Identities/ Similarities for the Matched Portion** | **Expect Value** |
| 009014 | PEPTIDE/HISTIDINE TRANSPORTER - Rattus norvegicus (Rat), 572 aa. | 9..576 | 500/578 (86%) | 0.0 |
| | | 3..571 | 531/578 (91%) | |
| Q91W98 | SIMILAR TO PEPTIDE TRANSPORTER 3 - Mus musculus (Mouse), 574 aa. | 9..576 | 496/578 (85%) | 0.0 |
| | | 3..573 | 531/578 (91%) | |
| AAH28394 | SIMILAR TO PEPTIDE TRANSPORTER 3 - Homo sapiens (Human), 461 aa. | 117..577 | 460/461 (99%) | 0.0 |
| | | 1..461 | 460/461 (99%) | |
| Q9P2X9 | PEPTIDE TRANSPORTER 3 - Homo sapiens (Human), 581 aa. | 9..558 | 289/570 (50%) | e-152 |
| | | 14..564 | 379/570 (65%) | |
| Q9WU80 | CAMP INDUCIBLE 1 PROTEIN - Mus musculus (Mouse), 578 aa. | 8..567 | 279/577 (48%) | e-144 |
| | | 6..570 | 366/577 (63%) | |

PFam analysis predicts that the NOV16a protein contains the domains shown in the Table 16E.

| **Table 16E. Domain Analysis of NOV16a** | | | |
|---|---|---|---|
| **Pfam Domain** | **NOV16a Match Region** | **Identities/ Similarities for the Matched Region** | **Expect Value** |
| PTR2 | 103..496 | 109/448 (24%) | 6.7e-103 |
| | | 310/448 (69%) | |

### Example 17.

The NOV17 clone was analyzed, and the nucleotide and encoded polypeptide sequences are shown in Table 17A.

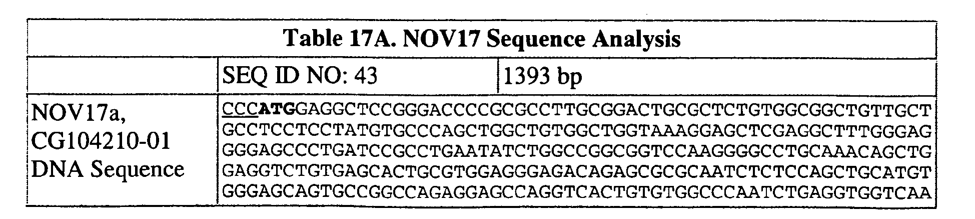

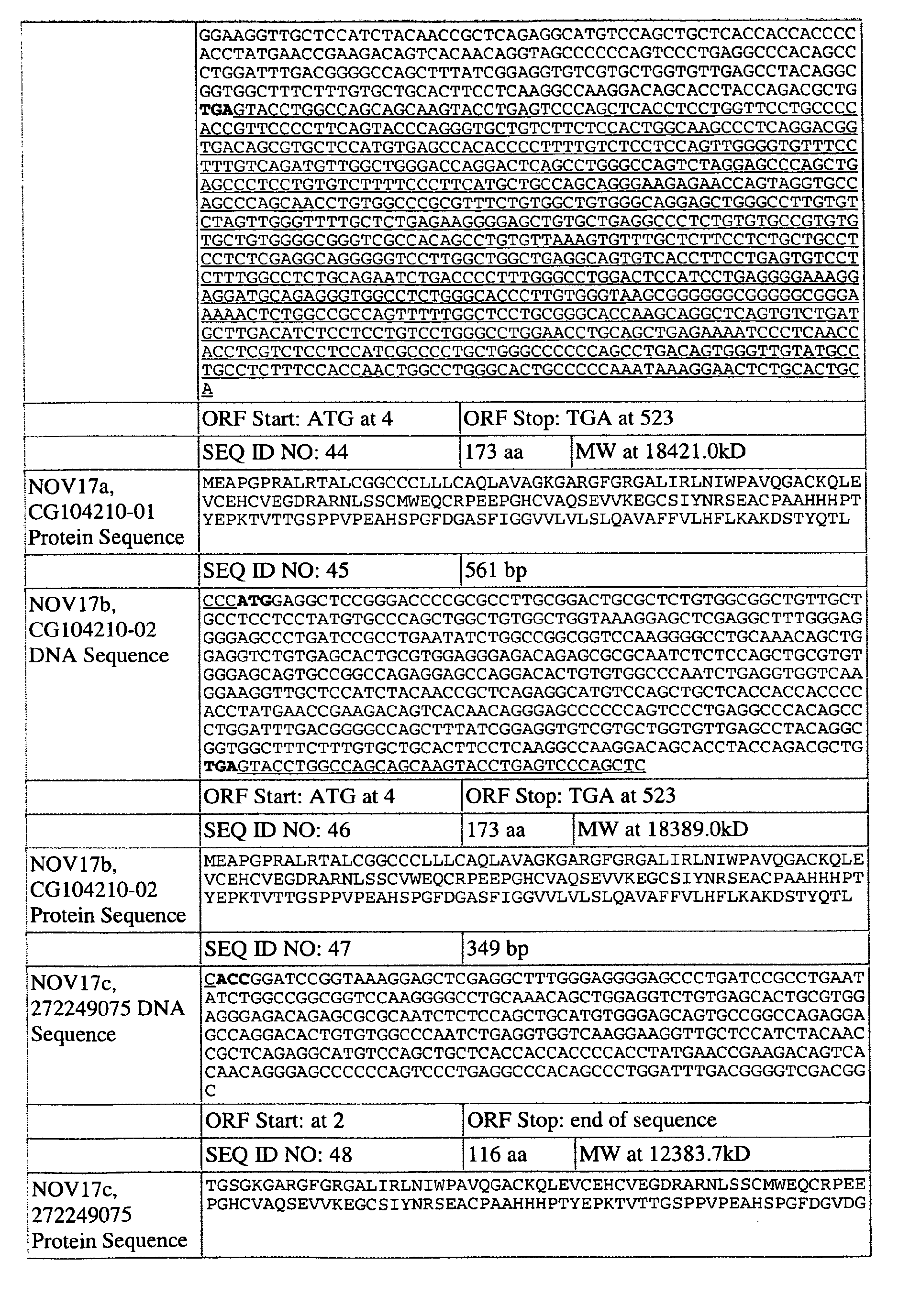

Sequence comparison of the above protein sequences yields the following sequence relationships shown in Table 17B.

| **Table 17B. Comparison of NOV17a against NOV17b and NOV17c.** | | |
|---|---|---|
| **Protein Sequence** | **NOV17a Residues/ Match Residues** | **Identities/ Similarities for the Matched Region** |
| NOV17b | 1..173 | 139/173 (80%) |
| | 1..173 | 140/173 (80%) |
| NOV17c | 41..139 | 99/99 (100%) |
| | 15..113 | 99/99 (100%) |

Further analysis of the NOV17a protein yielded the following properties shown in Table 17C.

| **Table 17C. Protein Sequence Properties NOV17a** | |
|---|---|
| PSort analysis: | 0.6850 probability located in endoplasmic reticulum (membrane); 0.6400 probability located in plasma membrane; 0.4600 probability located in Golgi body; 0.1000 probability located in endoplasmic reticulum (lumen) |
| SignalP analysis: | Cleavage site between residues 30 and 31 |

A search of the NOV17a protein against the Geneseq database, a proprietary database that contains sequences published in patents and patent publication, yielded several homologous proteins shown in Table 17D.

| **Table 17D. Geneseq Results for NOV17a** | | | | |
|---|---|---|---|---|
| **Geneseq Identifier** | **Protein/Organism/Length [Patent #, Date]** | **NOV17a Residues/ Match Residues** | **Identities/ Similarities for the Matched Region** | **Expect Value** |
| AAE03827 | Human gene 10 encoded secreted protein HBINS58, SEQ ID NO: 73 - Homo sapiens, 173 aa. [WO200136440-A1, 25-MAY-2001] | 1..173 | 173/173 (100%) | e-103 |
| | | 1..173 | 173/173 (100%) | |
| AAE03852 | Human gene 10 encoded secreted protein HBINS58, SEQ ID NO:98 - Homo sapiens, 210 aa. [WO200136440-A1, 25-MAY-2001] | 1..160 | 159/160 (99%) | 5e-94 |
| | | 1..160 | 159/160 (99%) | |
| AAB58415 | Lung cancer associated polypeptide sequence SEQ ID 753 - Homo sapiens, 214 aa. [WO200055180-A2, 21-SEP-2000] | 73..173 | 41/124 (33%) | 8e-10 |
| | | 95..214 | 56/124 (45%) | |
| AAG03771 | Human secreted protein, SEQ ID NO: 7852 - Homo sapiens, 197 aa. [EP1033401-A2, 06-SEP-2000] | 73..173 | 38/124 (30%) | 1e-07 |
| | | 78..197 | 52/124 (41%) | |
| ABB65987 | Drosophila melanogaster polypeptide SEQ ID NO 24753 - Drosophila melanogaster, 183 aa. [WO200171042-A2, 27-SEP-2001] | 116..173 | 29/60 (48%) | 9e-05 |
| | | 127..183 | 35/60 (58%) | |

In a BLAST search of public sequence databases, the NOV17a protein was found to have homology to the proteins shown in the BLASTP data in Table 17E.

| **Table 17E. Public BLASTP Results for NOV17a** | | | | |
|---|---|---|---|---|
| **Protein Accession Number** | **Protein/Organism/Length** | **NOV17a Residues/ Match Residues** | **Identities/ Similarities for the Matched Portion** | **Expect Value** |
| Q9D6W7 | 2310047N01RIK PROTEIN - Mus musculus (Mouse), 172 aa. | 1..173 | 140/173 (80%) | 1e-82 |
| | | 1..171 | 150/173 (85%) | |
| Q9BPV0 | CD164 ISOFORM DELTA 4 - Homo sapiens (Human), 184 aa. | 73..173 | 41/111 (36%) | 6e-11 |
| | | 78..184 | 56/111 (49%) | |
| Q9CVT7 | CD164 ANTIGEN - Mus musculus (Mouse), 161 aa (fragment). | 25..173 | 51/173 (29%) | 2e-10 |
| | | 5..161 | 67/173 (38%) | |
| Q9QX82 | ENDOLYN PRECURSOR - Rattus norvegicus (Rat), 195 aa. | 54..173 | 41/140 (29%) | 4e-10 |
| | | 57..195 | 59/140 (41%) | |
| Q9Z317 | MGC-24V - Mus musculus (Mouse), 197 aa. | 54..173 | 44/144 (30%) | 7e-10 |
| | | 58..197 | 58/144 (39%) | |

### Example 18.

The NOV18 clone was analyzed, and the nucleotide and encoded polypeptide sequences are shown in Table 18A.

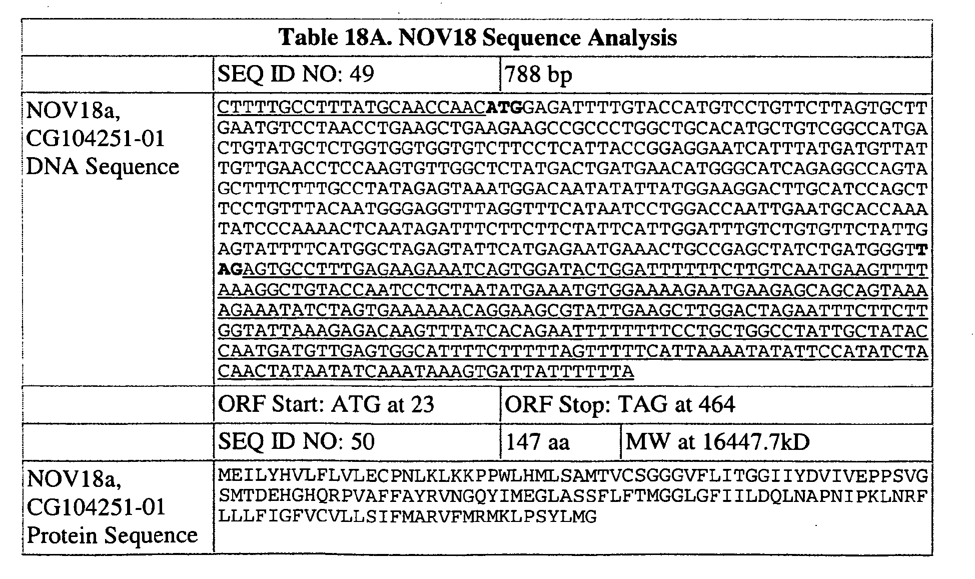

Further analysis of the NOV18a protein yielded the following properties shown in Table 18B.

| **Table 18B. Protein Sequence Properties NOV18a** | |
|---|---|
| PSort analysis: | 0.6400 probability located in plasma membrane; 0.4600 probability located in Golgi body; 0.3700 probability located in endoplasmic reticulum (membrane); 0.1000 probability located in endoplasmic reticulum (lumen) |
| SignalP analysis: | Cleavage site between residues 42 and 43 |

A search of the NOV18a protein against the Geneseq database, a proprietary database that contains sequences published in patents and patent publication, yielded several homologous proteins shown in Table 18C.

| **Table 18C. Geneseq Results for NOV18a** | | | | |
|---|---|---|---|---|
| **Geneseq Identifier** | **Protein/Organism/Length [Patent #, Date]** | **NOV18a Residues/ Match Residues** | **Identities/ Similarities for the Matched Region** | **Expect Value** |
| AAY53631 | A bone marrow secreted protein designated BMS155 - Homo sapiens, 149 aa. [WO9933979-A2, 08-JUL-1999] | 1..147 | 133/149 (89%) | 1e-69 |
| | | 1..149 | 135/149 (90%) | |
| AAY53042 | Human secreted protein clone pu282_10 protein sequence SEQ ID NO:90 - Homo sapiens, 149 aa. [WO9957132-A1, 11-NOV-1999] | 1..147 | 133/149 (89%) | 1e-69 |
| | | 1..149 | 135/149 (90%) | |
| AAB12143 | Hydrophobic domain protein isolated from WERI-RB cells - Homo sapiens, 149 aa. [WO200029448-A2, 25-MAY-2000] | 1..147 | 133/149 (89%) | 1e-69 |
| | | 1..149 | 135/149 (90%) | |
| AAY59670 | Secreted protein 108-005-5-0-F6-FL - Homo sapiens, 149 aa. [WO9940189-A2, 12-AUG-1999] | 1..147 | 133/149 (89%) | 1e-69 |
| | | 1..149 | 135/149 (90%) | |
| AAY60146 | Human endometrium tumour EST encoded protein 206 - Homo sapiens, 171 aa. [DE19817948-A1, 21-OCT-1999] | 1..147 | 133/149 (89%) | 1e-69 |
| | | 23..171 | 135/149 (90%) | |

In a BLAST search of public sequence databases, the NOV18a protein was found to have homology to the proteins shown in the BLASTP data in Table 18D.

| **Table 18D. Public BLASTP Results for NOV18a** | | | | |
|---|---|---|---|---|
| **Protein Accession Number** | **Protein/Organism/Length** | **NOV18a Residues/ Match Residues** | **Identities/ Similarities for the Matched Portion** | **Expect Value** |
| Q9NRP0 | DC2 (HYDROPHOBIC PROTEIN HSF-28) (HYPOTHETICAL 16.8 KDA PROTEIN) - Homo sapiens (Human), 149 aa. | 1..147 | 133/149 (89%) | 3e-69 |
| | | 1..149 | 135/149 (90%) | |
| Q9P075 | HSPC307 - Homo sapiens (Human), 167 aa (fragment). | 1..147 | 133/149 (89%) | 3e-69 |
| | | 19..167 | 135/149 (90%) | |
| Q9CPZ2 | 2310008M10RIK PROTEIN (RIKEN CDNA 2310008M10 GENE) - Mus musculus (Mouse), 149 aa. | 1..147 | 132/149 (88%) | 6e-69 |
| | | 1..149 | 135/149 (90%) | |
| Q9P1R4 | HDCMD45P - Homo sapiens (Human), 160 aa (fragment). | 1..147 | 132/149 (88%) | 2e-68 |
| | | 12..160 | 134/149 (89%) | |
| AAH24224 | SIMILAR TO DC2 PROTEIN - Homo sapiens (Human), 119 aa. | 40..147 | 96/108 (88%) | 7e-50 |
| | | 12..119 | 100/108 (91%) | |

### Example 19.

The NOV19 clone was analyzed, and the nucleotide and encoded polypeptide sequences are shown in Table 19A.

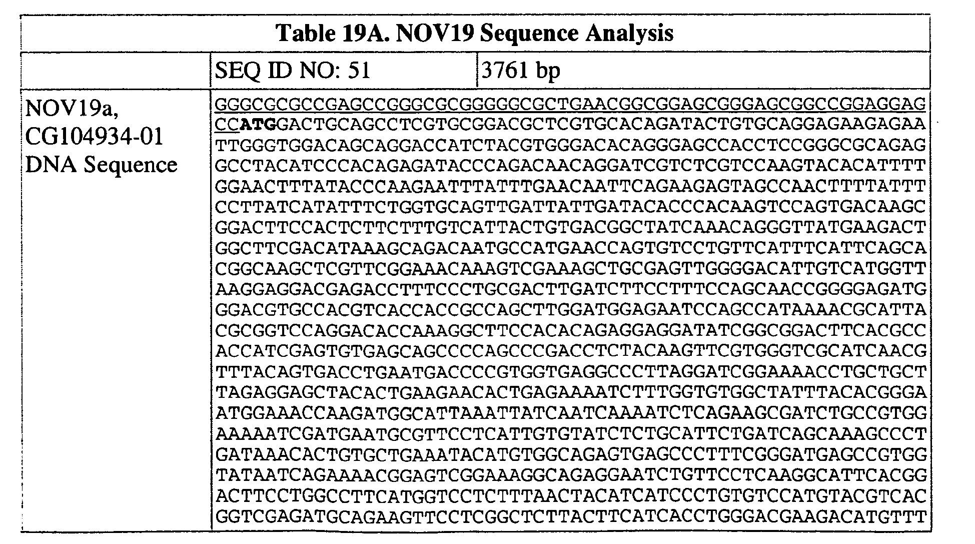

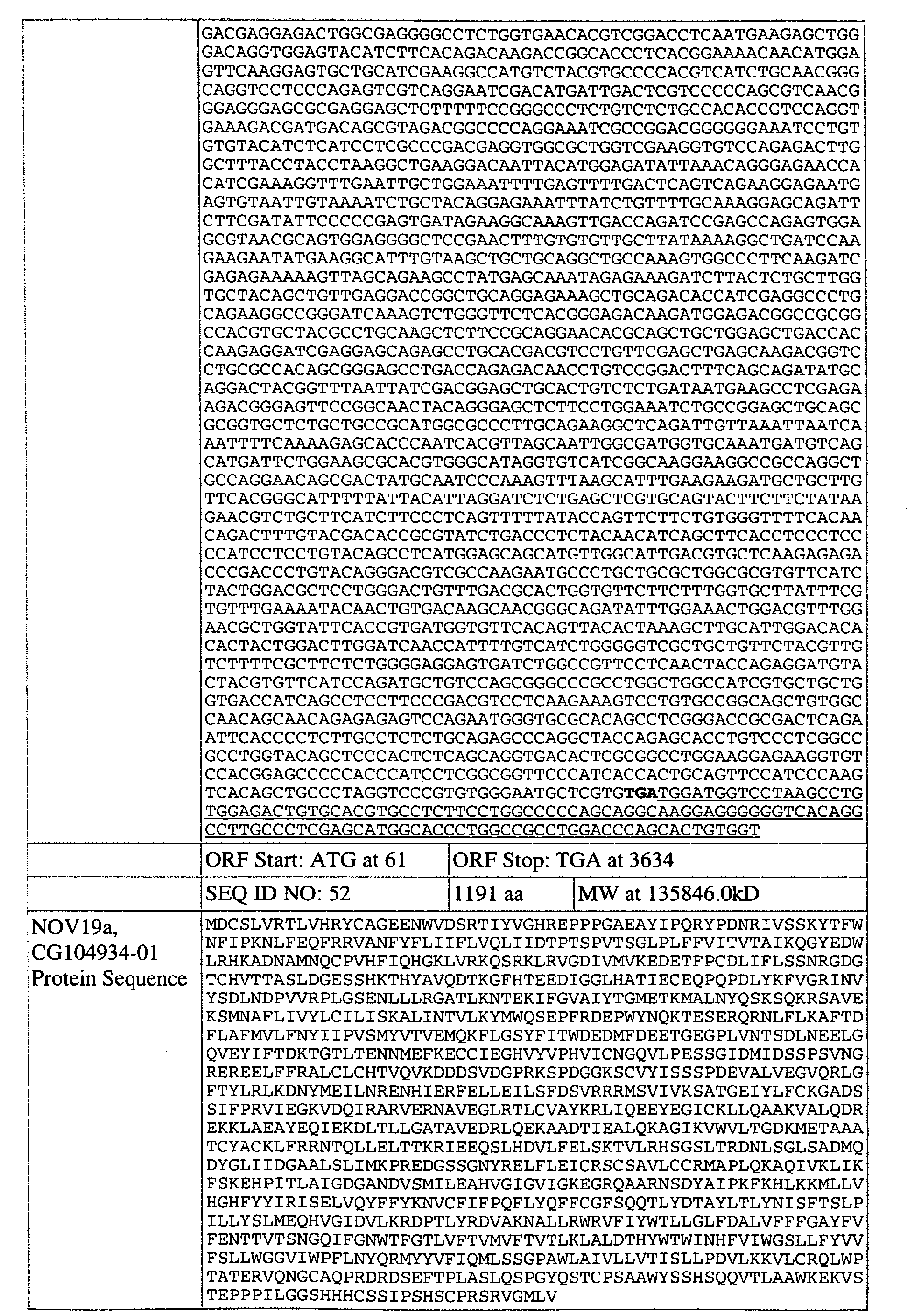

Further analysis of the NOV19a protein yielded the following properties shown in Table 19B.

| **Table 19B. Protein Sequence Properties NOV19a** | |
|---|---|
| PSort analysis: | 0.6000 probability located in plasma membrane; 0.4000 probability located in Golgi body; 0.3000 probability located in endoplasmic reticulum (membrane); 0.0300 probability located in mitochondrial inner membrane |
| SignalP analysis: | No Known Signal Sequence Predicted |

A search of the NOV19a protein against the Geneseq database, a proprietary database that contains sequences published in patents and patent publication, yielded several homologous proteins shown in Table 19C.

| **Table 19C. Geneseq Results for NOV19a** | | | | |
|---|---|---|---|---|
| **Geneseq Identifier** | **Protein/Organism/Length [Patent #, Date]** | **NOV19a Residues/ Match Residues** | **Identities/ Similarities for the Matched Region** | **Expect Value** |
| AAO14200 | Human transporter and ion channel TRICH-17 - Homo sapiens, 1192 aa. [WO200204520-A2, 17-JAN-2002] | 1..1191 | 1190/1192 (99%) | 0.0 |
| | | 1..1192 | 1191/1192 (99%) | |
| AAB42368 | Human ORFX ORF2132 polypeptide sequence SEQ ID NO:4264 - Homo sapiens, 797 aa. [WO200058473-A2, 05-OCT-2000] | 338..1109 | 770/772 (99%) | 0.0 |
| | | 1..772 | 772/772 (99%) | |
| AAG67546 | Amino acid sequence of a human transporter protein - Homo sapiens, 1177 aa. [WO200164878-A2, 07-SEP-2001] | 22..1109 | 657/1119 (58%) | 0.0 |
| | | 18..1106 | 833/1119 (73%) | |
| AAO14203 | Human transporter and ion channel TRICH-20 - Homo sapiens, 1096 aa. [WO200204520-A2, 17-JAN-2002] | 22..1109 | 583/1119 (52%) | 0.0 |
| | | 18..1040 | 755/1119 (67%) | |
| AAM39290 | Human polypeptide SEQ ID NO 2435 - Homo sapiens, 815 aa. [WO200153312-A1, 26-JUL-2001] | 370..1109 | 424/771 (54%) | 0.0 |
| | | 1..744 | 544/771 (69%) | |

In a BLAST search of public sequence databases, the NOV19a protein was found to have homology to the proteins shown in the BLASTP data in Table 19D.

| **Table 19D. Public BLASTP Results for NOV19a** | | | | |
|---|---|---|---|---|
| **Protein Accession Number** | **Protein/Organism/Length** | **NOV19a Residues/ Match Residues** | **Identities/ Similarities for the Matched Portion** | **Expect Value** |
| P98197 | Potential phospholipid-transporting ATPase IH (EC 3.6.3.1) - Mus musculus (Mouse), 1187 aa. | 1..1189 | 1074/1195 (89%) | 0.0 |
| | | 1..1185 | 1117/1195 (92%) | |
| P98196 | Potential phospholipid-transporting ATPase IS (EC 3.6.3.1) - Homo sapiens (Human), 797 aa (fragment). | 338..1109 | 772/772 (100%) | 0.0 |
| | | 1..772 | 772/772 (100%) | |
| Q8WX24 | BB206I21.1 (ATPASE, CLASS VI, TYPE 11C) - Homo sapiens (Human), 962 aa (fragment). | 14..997 | 633/992 (63%) | 0.0 |
| | | 1..962 | 770/992 (76%) | |
| Q9N0Z4 | RING-FINGER BINDING PROTEIN - Oryctolagus cuniculus (Rabbit), 1107 aa (fragment). | 22..1109 | 574/1123 (51%) | 0.0 |
| | | 10..1036 | 752/1123 (66%) | |
| Q9Y2G3 | Potential phospholipid-transporting ATPase IR (EC 3.6.3.1) - Homo sapiens (Human), 672 aa (fragment). | 486..1109 | 358/625 (57%) | 0.0 |
| | | 1..601 | 462/625 (73%) | |

PFam analysis predicts that the NOV19a protein contains the domains shown in the Table 19E.

| **Table 19E. Domain Analysis of NOV19a** | | | |
|---|---|---|---|
| **Pfam Domain** | **NOV19a Match Region** | **Identities/ Similarities for the Matched Region** | **Expect Value** |
| Hydrolase | 408..846 | 46/448 (10%) | 0.0058 |
| | | 258/448 (58%) | |

### Example 20.

The NOV20 clone was analyzed, and the nucleotide and encoded polypeptide sequences are shown in Table 20A.

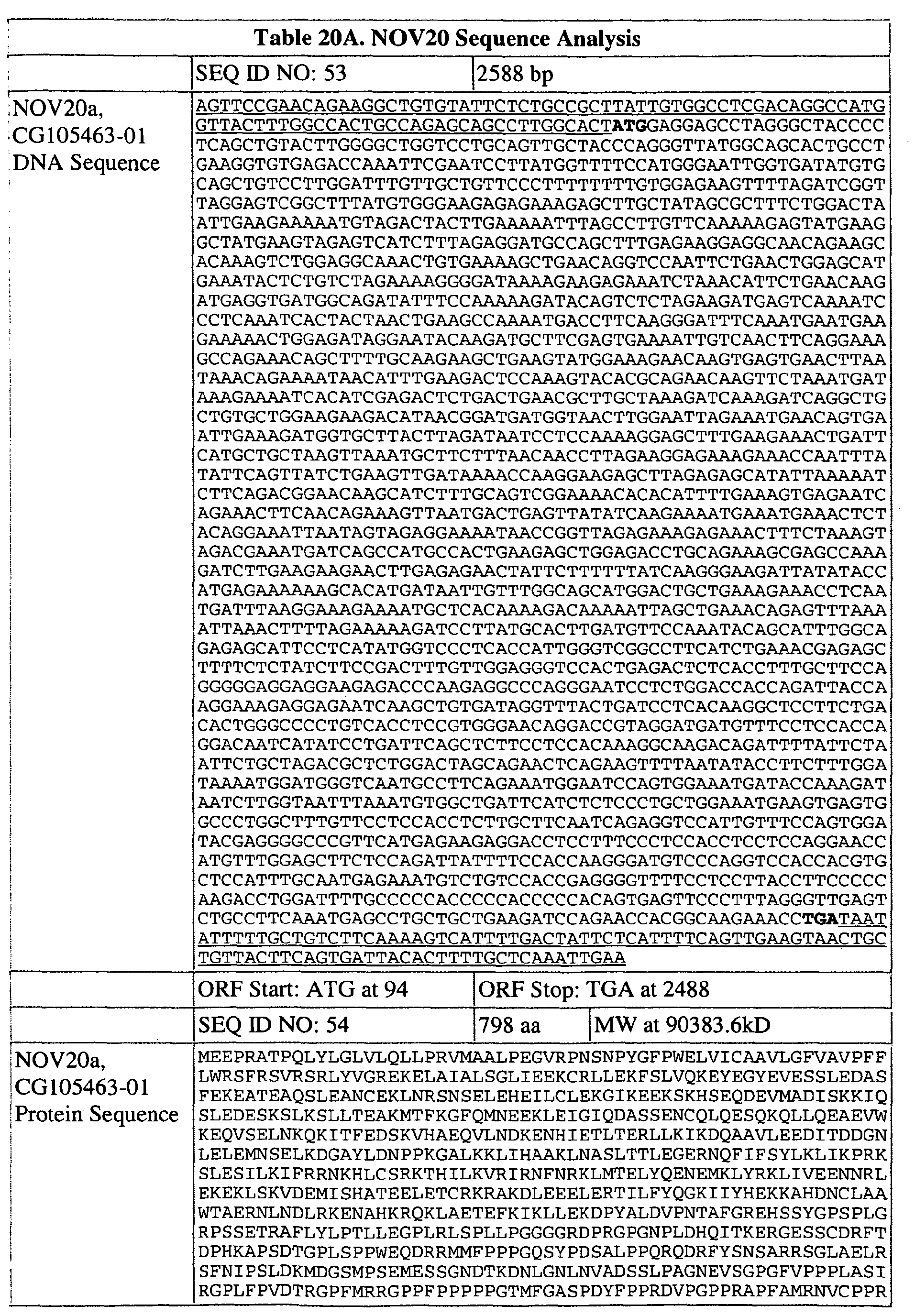

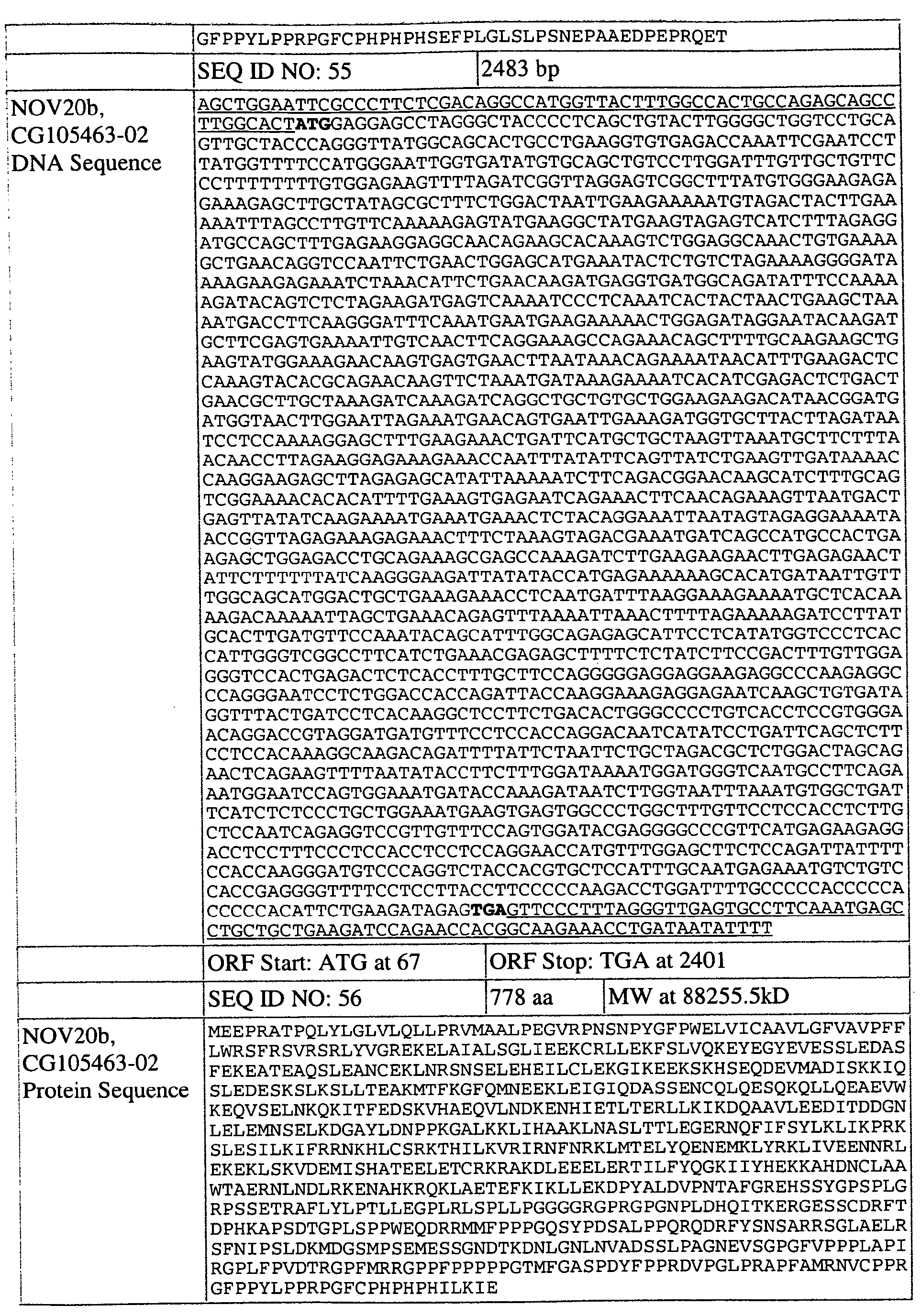

Sequence comparison of the above protein sequences yields the following sequence relationships shown in Table 20B.

| **Table 20B. Comparison of NOV20a against NOV20b.** | | |
|---|---|---|
| **Protein Sequence** | **NOV20a Residues/ Match Residues** | **Identities/ Similarities for the Matched Region** |
| NOV20b | 1..750 | 662/750 (88%) |
| | 1..750 | 662/750 (88%) |

Further analysis of the NOV20a protein yielded the following properties shown in Table 20C.

| **Table 20C. Protein Sequence Properties NOV20a** | |
|---|---|
| PSort analysis: | 0.4600 probability located in plasma membrane; 0.1000 probability located in endoplasmic reticulum (membrane); 0.1000 probability located in endoplasmic reticulum (lumen); 0.1000 probability located in outside |
| SignalP analysis: | Cleavage site between residues 25 and 26 |

A search of the NOV20a protein against the Geneseq database, a proprietary database that contains sequences published in patents and patent publication, yielded several homologous proteins shown in Table 20D.

| **Table 20D. Geneseq Results for NOV20a** | | | | |
|---|---|---|---|---|
| **Geneseq Identifier** | **Protein/Organism/Length [Patent #, Date]** | **NOV20a Residues/ Match Residues** | **Identities/ Similarities for the Matched Region** | **Expect Value** |
| AAY77574 | Human cytoskeletal protein (HCYT) (clone 3768043) - Homo sapiens, 806 aa. [WO200006730-A2, 10-FEB-2000] | 1..798 | 638/812 (78%) | 0.0 |
| | | 1..806 | 683/812 (83%) | |
| ABG05280 | Novel human diagnostic protein #5271 - Homo sapiens, 881 aa. [W0200175067-A2, 11-OCT-2001] | 1..797 | 639/814 (78%) | 0.0 |
| | | 59..867 | 684/814 (83%) | |
| ABG05280 | Novel human diagnostic protein #5271 - Homo sapiens, 881 aa. [WO200175067-A2, 11-OCT-2001] | 1..797 | 639/814 (78%) | 0.0 |
| | | 59..867 | 684/814 (83%) | |
| ABG20258 | Novel human diagnostic protein #20249 - Homo sapiens, 881 aa. [WO200175067-A2, 11-OCT-2001] | 1..797 | 634/814 (77%) | 0.0 |
| | | 59..867 | 681/814 (82%) | |
| ABG20258 | Novel human diagnostic protein #20249 - Homo sapiens, 881 aa. [WO200175067-A2, 11-OCT-2001] | 1..797 | 634/814 (77%) | 0.0 |
| | | 59..867 | 681/814 (82%) | |

In a BLAST search of public sequence databases, the NOV20a protein was found to have homology to the proteins shown in the BLASTP data in Table 20E.

| **Table 20E. Public BLASTP Results for NOV20a** | | | | |
|---|---|---|---|---|
| **Protein Accession Number** | **Protein/Organism/Length** | **NOV20a Residues/ Match the Residues** | **Identities/ Similarities for Matched Portion** | **Expect Value** |
| 015320 | Meningioma-expressed antigen 6/11 (MEA6) (MEA11) - Homo sapiens (Human), 804 aa. | 1..798 | 653/810 (80%) | 0.0 |
| | | 1..804 | 696/810 (85%) | |
| Q96SG9 | BA500G10.2 (NOVEL PROTEIN SIMILAR TO MENINGIOMA EXPRESSED ANTIGEN 6 (MEA6) AND 11 (MEA11)) - Homo sapiens (Human), 825 aa (fragment). | 1..798 | 616/805 (76%) | 0.0 |
| | | 15..816 | 670/805 (82%) | |
| Q96RT6 | CTAGE-2 - Homo sapiens (Human), 754 aa. | 30..775 | 605/749 (80%) | 0.0 |
| | | 1..746 | 642/749 (84%) | |
| 095046 | WUGSC:H_DJ0988G15.3 PROTEIN (DJ1005H11.2) (WUGSC:H_DJ0988G15.3 PROTEIN) - Homo sapiens (Human), 777 aa. | 1..770 | 570/775 (73%) | 0.0 |
| | | 1..775 | 633/775 (81%) | |
| AAH26864 | SIMILAR TO MENINGIOMA EXPRESSED ANTIGEN 6 (COILED-COIL PROLINE-RICH) -Mus musculus (Mouse), 779 aa. | 30..796 | 520/783 (66%) | 0.0 |
| | | 1..778 | 600/783 (76%) | |

PFam analysis predicts that the NOV20a protein contains the domains shown in the Table 20F.

| **Table 20F. Domain Analysis of NOV20a** | | | |
|---|---|---|---|
| **Pfam Domain** | **NOV20a Match Region** | **Identities/ Similarities for the Matched Region** | **Expect Value** |
| No Significant Matches Found | | | |

### Example 21.

The NOV21 clone was analyzed, and the nucleotide and encoded polypeptide sequences are shown in Table 21A.

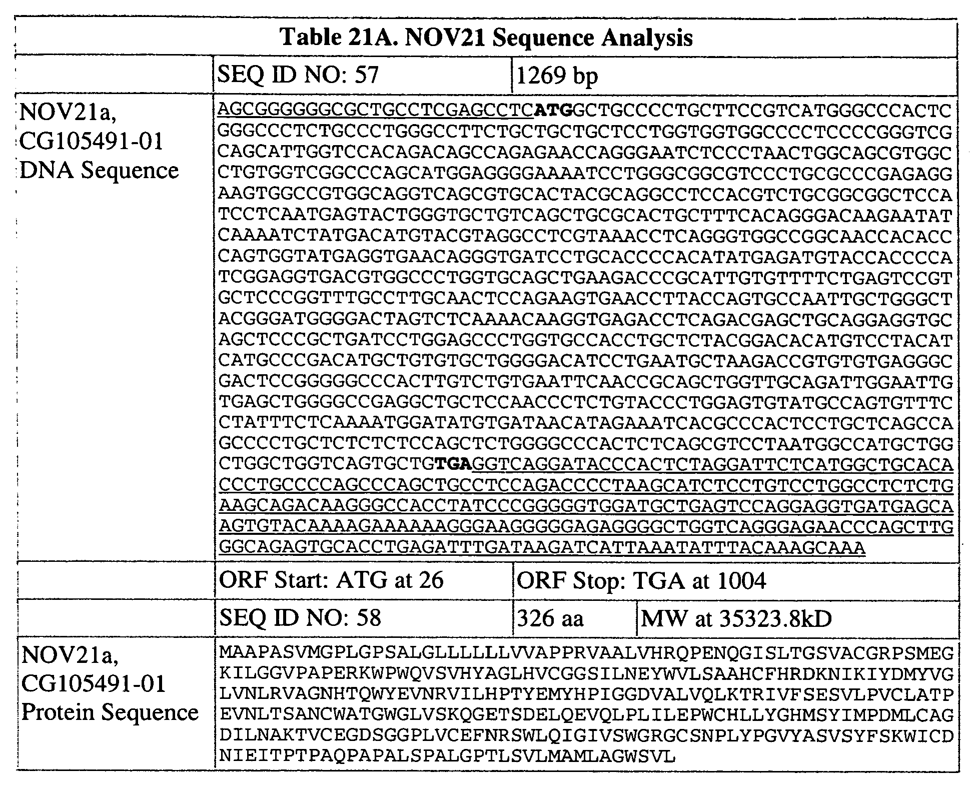

Further analysis of the NOV21a protein yielded the following properties shown in Table 21B.

| **Table 21B. Protein Sequence Properties NOV21a** | |
|---|---|
| PSort analysis: | 0.7900 probability located in plasma membrane; 0.3000 probability located in Golgi body; 0.2000 probability located in endoplasmic reticulum (membrane); 0.1007 probability located in microbody (peroxisome) |
| SignalP analysis: | Cleavage site between residues 33 and 34 |

A search of the NOV21a protein against the Geneseq database, a proprietary database that contains sequences published in patents and patent publication, yielded several homologous proteins shown in Table 21C.

| **Table 21C. Geneseq Results for NOV21a** | | | | |
|---|---|---|---|---|
| **Geneseq Identifier** | **Protein/Organism/Length [Patent #, Date]** | **NOV21a Residues/ Match Residues** | **Identities/ Similarities for the Matched Region** | **Expect Value** |
| AAU82747 | Amino acid sequence of novel human protease #46 - Homo sapiens, 326 aa. [WO200200860-A2,03-JAN-2002] | 1..326 | 326/326 (100%) | 0.0 |
| | | 1..326 | 326/326 (100%) | |
| AAB73945 | Human protease T - Homo sapiens, 290 aa. [W0200116293-A2, 08- MAR-2001] | 13..288 | 115/286 (40%) | 6e-53 |
| | | 4..272 | 152/286 (52%) | |
| AAE03821 | Human gene 4 encoded secreted protein HWHIH10, SEQ ID NO: 67 - Homo sapiens, 290 aa. [WO200136440-A1, 25-MAY-2001] | 13..288 | 115/286 (40%) | 6e-53 |
| | | 4..272 | 152/286 (52%) | |
| AAU12282 | Human PRO4327 polypeptide sequence - Homo sapiens, 290 aa. [WO200140466-A2, 07-JUN-2001] | 13..288 | 115/286 (40%) | 6e-53 |
| | | 4..272 | 152/286 (52%) | |
| AAY73388 | HTRM clone 3376404 protein sequence - Homo sapiens, 290 aa. [WO9957144-A2, 11-NOV-1999] | 13..288 | 115/286 (40%) | 6e-53 |
| | | 4..272 | 152/286 (52%) | |

In a BLAST search of public sequence databases, the NOV21a protein was found to have homology to the proteins shown in the BLASTP data in Table 21D.

| **Table 21D. Public BLASTP Results for NOV21a** | | | | |
|---|---|---|---|---|
| **Protein Accession Number** | **Protein/Organism/Length** | **NOV21a Residues/ Match Residues** | **Identities/ Similarities for the Matched Portion** | **Expect Value Value** |
| Q9BQR3 | Marapsin precursor (EC 3.4.21.-)- Homo sapiens (Human), 290 aa. | 13..288 | 115/286 (40%) | 1e-52 |
| | | 4..272 | 152/286 (52%) | |
| Q9PVX7 | EPIDERMIS SPECIFIC SERINE PROTEASE - Xenopus laevis (African clawed frog), 389 aa. | 50..314 | 98/275 (35%) | 5e-52 |
| | | 16..287 | 159/275 (57%) | |
| AAH24903 | RIKEN CDNA 2010001P08 GENE - Mus musculus (Mouse), 331 aa. | 51..326 | 114/288 (39%) | 2e-51 |
| | | 45..329 | 158/288 (54%) | |
| Q91XC4 | SIMILAR TO DISTAL INTESTINAL SERINE PROTEASE - Mus musculus (Mouse), 310 aa. | 51..288 | 106/247 (42%) | 6e-51 |
| | | 28..272 | 138/247 (54%) | |
| Q9QYZ9 | DISTAL INTESTINAL SERINE PROTEASE - Mus musculus (Mouse), 310 aa. | 51..288 | 105/247 (42%) | 7e-50 |
| | | 28..272 | 137/247 (54%) | |

PFam analysis predicts that the NOV21a protein contains the domains shown in the Table 21E.

| **Table 21E. Domain Analysis of NOV21a** | | | |
|---|---|---|---|
| **Pfam Domain** | **NOV21a Match Region** | **Identities/ Similarities for the Matched Region** | **Expect Value** |
| trypsin | 60..288 | 87/265 (33%) | 5.3e-72 |
| | | 172/265 (65%) | |

### Example 22.

The NOV22 clone was analyzed, and the nucleotide and encoded polypeptide sequences are shown in Table 22A.

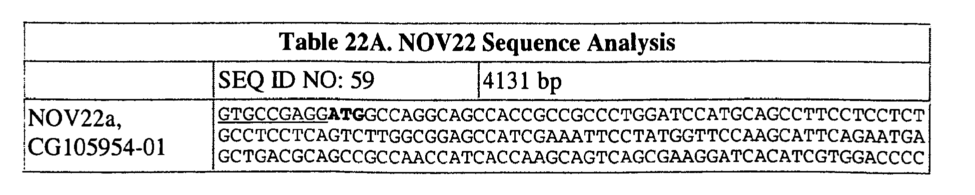

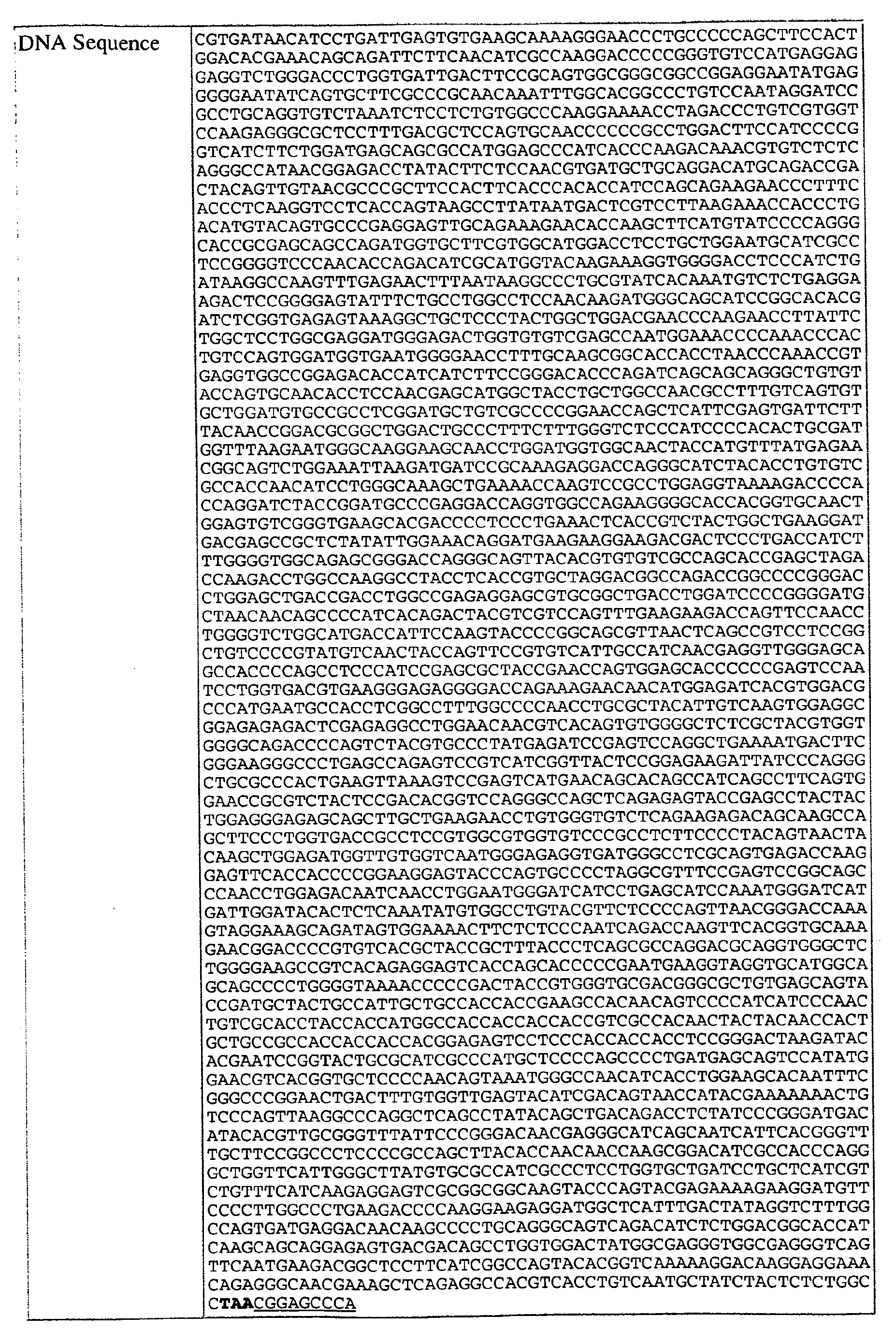

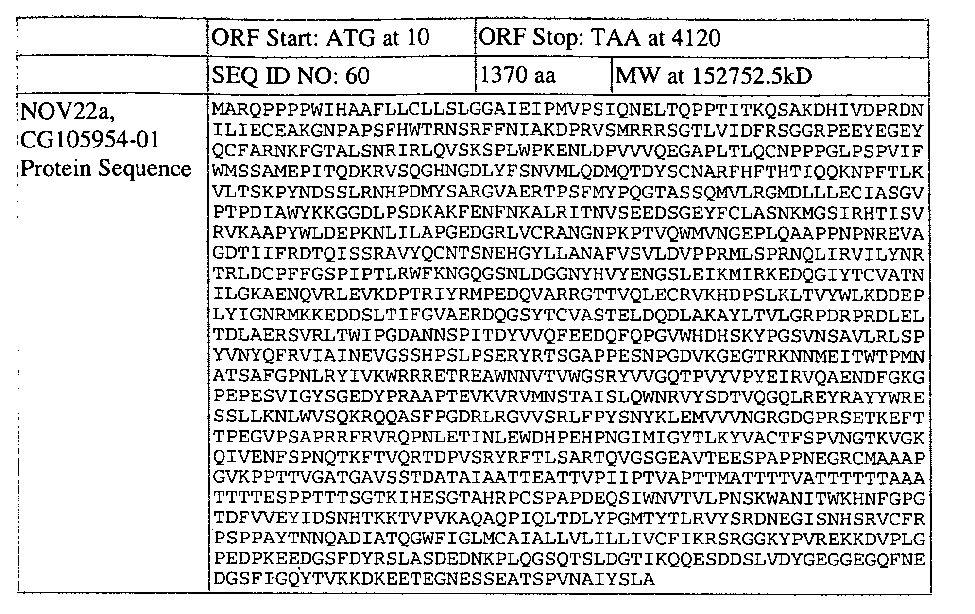

Further analysis of the NOV22a protein yielded the following properties shown in Table 22B.

| **Table 22B. Protein Sequence Properties NOV22a** | |
|---|---|
| PSort analysis: | 0.4600 probability located in plasma membrane; 0.1000 probability located in endoplasmic reticulum (membrane); 0.1000 probability located in endoplasmic reticulum (lumen); 0.1000 probability located in outside |
| SignalP analysis: | Cleavage site between residues 25 and 26 |

A search of the NOV22a protein against the Geneseq database, a proprietary database that contains sequences published in patents and patent publication, yielded several homologous proteins shown in Table 22C.

| **Table 22C. Geneseq Results for NOV22a** | | | | |
|---|---|---|---|---|
| **Geneseq Identifier** | **Protein/Organism/Length [Patent #, Date]** | **NOV22a Residues/ Match Residues** | **Identities/ Similarities for the Matched Region** | **Expect Value** |
| AAM78714 | Human protein SEQ ID NO 1376 - Homo sapiens, 937 aa. [W0200157190-A2,09-AUG-2001] | 92..1050 | 928/959 (96%) | 0.0 |
| | | 1..937 | 933/959 (96%) | |
| AAM78715 | Human protein SEQ ID NO 1377 - Homo sapiens, 952 aa. [WO200157190-A2, 09-AUG-2001] | 92..1050 | 928/974 (95%) | 0.0 |
| | | 1..952 | 933/974 (95%) | |
| AAW59994 | Human neural cell adhesion molecule splice variant NrCAMvar - Homo sapiens, 1304 aa. [WO9836062-A1, 20-AUG-1998] | 15..1072 | 517/1075 (48%) | 0.0 |
| | | 20..1082 | 708/1075 (65%) | |
| AAU10650 | Chicken Nr-CAM protein sequence - Gallus sp, 1268 aa. [US6313265- B1, 06-NOV-2001] | 12..1054 | 508/1055 (48%) | 0.0 |
| | | 12..1042 | 696/1055 (65%) | |
| AAB90717 | Human C0722_1 protein sequence SEQ ID 130 - Homo sapiens, 1192 aa. [WO200119988-A1, 22-MAR-2001] | 15..1060 | 509/1058 (48%) | 0.0 |
| | | 20..1047 | 701/1058 (66%) | |

In a BLAST search of public sequence databases, the NOV22a protein was found to have homology to the proteins shown in the BLASTP data in Table 22D.

| **Table 22D. Public BLASTP Results for NOV22a** | | | | |
|---|---|---|---|---|
| **Protein Accession Number** | **Protein/Organism/Length** | **NOV22a Residues/ Residues/ Match Residues** | **Identities/ Similarities for the Matched Portion** | **Expect Expect Value** |
| 042414 | NEUROFASCIN PRECURSOR - Gallus gallus (Chicken), 1369 aa. | 13..1370 | 1026/1372 (74%) | 0.0 |
| | | 14..1369 | 1148/1372 (82%) | |
| Q91Z60 | NEUROFASCIN 155 KDA ISOFORM - Rattus norvegicus (Rat), 1174 aa. | 1..1042 | 985/1042 (94%) | 0.0 |
| | | 1..1031 | 1008/1042 (96%) | |
| Q9QVN5 | NEUROFASCIN ISOFORM - Rattus sp, 1151 aa. | 25..1042 | 965/1019 (94%) | 0.0 |
| | | 1..1008 | 987/1019 (96%) | |
| Q90924 | NEUROFASCIN PRECURSOR - Gallus gallus (Chicken), 1272 aa. | 13..1114 | 844/1114 (75%) | 0.0 |
| | | 14..1120 | 949/1114 (84%) | |
| 094856 | KIAA0756 PROTEIN - Homo sapiens (Human), 836 aa (fragment). | 193..1050 | 830/858 (96%) | 0.0 |
| | | 1..836 | 833/858 (96%) | |

PFam analysis predicts that the NOV22a protein contains the domains shown in the Table 22E.

| **Table 22E. Domain Analysis of NOV22a** | | | |
|---|---|---|---|
| **Pfam Domain** | **NOV22a Match Region** | **Identities/ Similarities for the Matched Region** | **Expect Value** |
| ig | 56..120 | 11/68 (16%) | 0.032 |
| | | 43/68 (63%) | |
| ig | 155..215 | 13/62 (21%) | 0.01 |
| | | 39/62 (63%) | |
| ig | 278..335 | 18/61 (30%) | 4.3e-11 |
| | | 44/61 (72%) | |
| ig | 368..427 | 13/63 (21%) | 2.1e-05 |
| | | 44/63 (70%) | |
| ig | 462..520 | 18/62 (29%) | 1.1e-07 |
| | | 45/62 (73%) | |
| ig | 553..611 | 19/60 (32%) | 5.9e-09 |
| | | 40/60 (67%) | |
| fn3 | 630..716 | 28/88 (32%) | 6.6e-14 |
| | | 64/88 (73%) | |
| fn3 | 729..815 | 27/92 (29%) | 5.9e-07 |
| | | 62/92 (67%) | |
| fn3 | 827..922 | 24/97 (25%) | 3.7e-07 |
| | | 66/97 (68%) | |
| fn3 | 934..1026 | 20/97 (21%) | 2.1e-08 |
| | | 66/97 (68%) | |
| fn3 | 1134..1213 | 22/85 (26%) | 1.5e-08 |
| | | 56/85 (66%) | |

### Example 23.

The NOV23 clone was analyzed, and the nucleotide and encoded polypeptide sequences are shown in Table 23A.

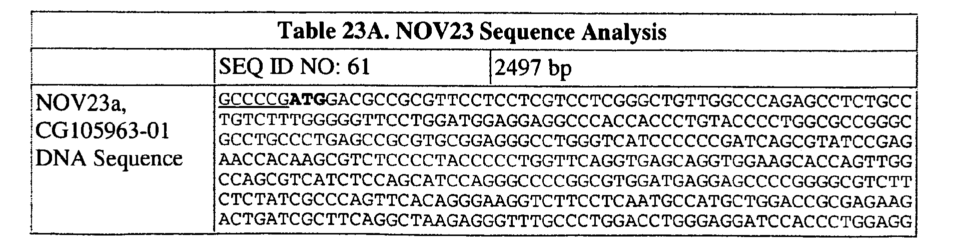

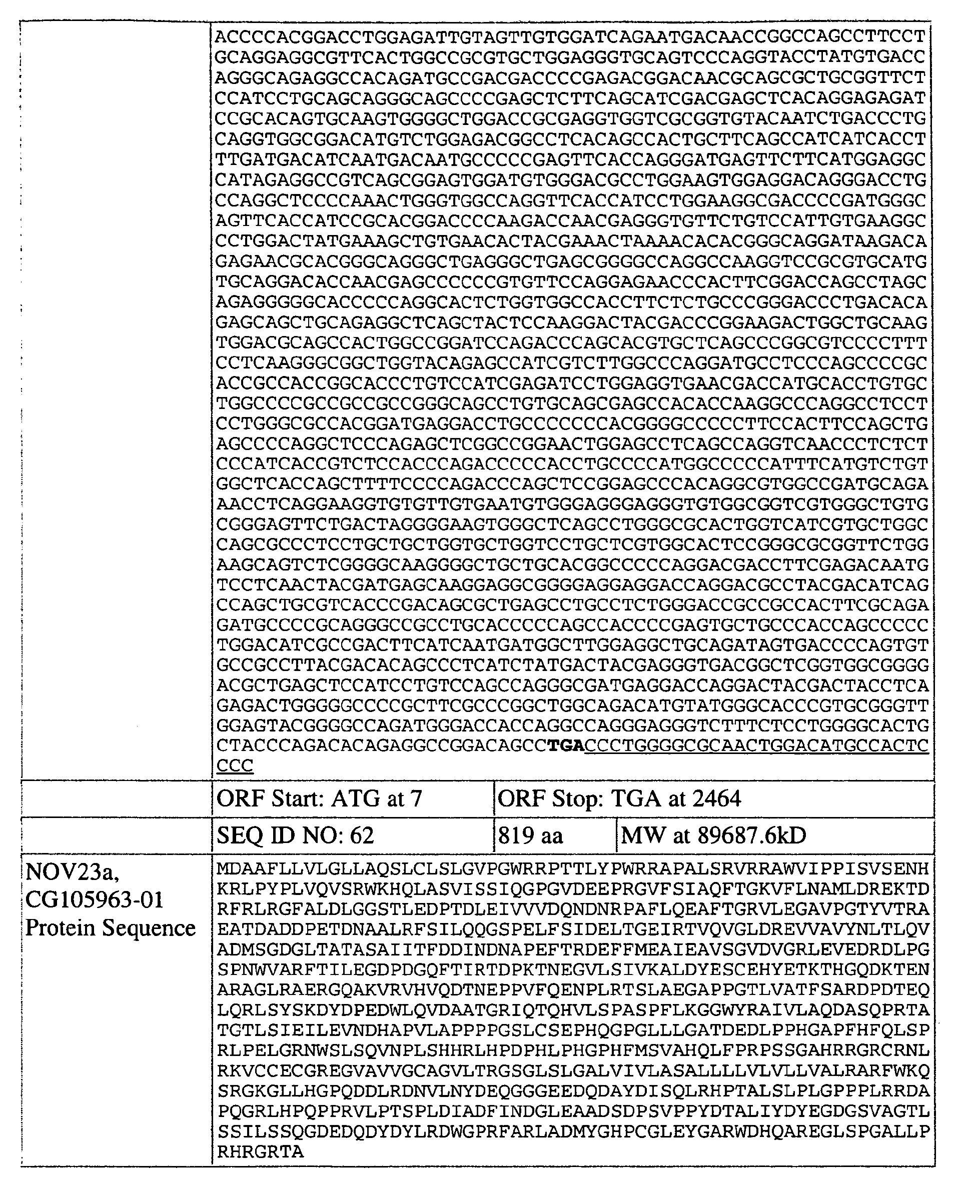

Further analysis of the NOV23a protein yielded the following properties shown in Table 23B.

| | **Table 23B. Protein Sequence Properties NOV23a** |
|---|---|
| PSort analysis: | 0.6850 probability located in endoplasmic reticulum (membrane); 0.6400 probability located in plasma membrane; 0.4600 probability located in Golgi body; 0.1000 probability located in endoplasmic reticulum (lumen) |
| SignalP analysis: | Cleavage site between residues 22 and 23 |

A search of the NOV23a protein against the Geneseq database, a proprietary database that contains sequences published in patents and patent publication, yielded several homologous proteins shown in Table 23C.

| **Table 23C. Geneseq Results for NOV23a** | | | | |
|---|---|---|---|---|
| **Geneseq Identifier** | **Protein/Organism/Length [Patent #, Date]** | **NOV23a Residues/ Match Residues** | **Identities/ Similarities for the Matched Region** | **Expect Value** |
| ABG30224 | Novel human diagnostic protein #30215 - Homo sapiens, 814 aa. [WO200175067-A2, 11-OCT-2001] | 1..819 | 750/820 (91%) | 0.0 |
| | | 1..814 | 766/820 (92%) | |
| ABG30224 | Novel human diagnostic protein #30215 - Homo sapiens, 814 aa. [WO200175067-A2, 11-OCT-2001] | 1..819 | 750/820 (91%) | 0.0 |
| | | 1..814 | 766/820 (92%) | |
| AAB24089 | Human PRO2198 protein sequence SEQ ID NO:79 - Homo sapiens, 814 aa. [WO200053755-A2, 14-SEP-2000] | 1..819 | 750/820 (91%) | 0.0 |
| | | 1..814 | 766/820 (92%) | |
| ABB57233 | Mouse ischaemic condition related protein sequence SEQ ID NO:606 - Mus musculus, 906 aa. [WO200188188-A2, 22-NOV-2001] | 35..786 | 313/767 (40%) | e-152 |
| | | 149..902 | 436/767 (56%) | |
| AAY70741 | Human N-cadherin - Homo sapiens, 906 aa. [WO200021555-A1, 20- APR-2000] | 40..786 | 311/762 (40%) | e-151 |
| | | 154..902 | 435/762 (56%) | |

In a BLAST search of public sequence databases, the NOV23a protein was found to have homology to the proteins shown in the BLASTP data in Table 23D.

| **Table 23D. Public BLASTP Results for NOV23a** | | | | |
|---|---|---|---|---|
| **Protein Accession Number** | **Protein/Organism/Length** | **NOV23a Residues/ Match Residues** | **Identities/ Similarities for the Matched Portion** | **Expect Value** |
| P55291 | Muscle-cadherin precursor (M-cadherin) (Cadherin-15) (Cadherin-14) - Homo sapiens (Human), 814 aa. | 1..819 | 750/820 (91%) | 0.0 |
| | | 1..814 | 766/820 (92%) | |
| P33146 | Muscle-cadherin precursor (M-cadherin) (Cadherin-15) (Cadherin-14) - Mus musculus (Mouse), 784 aa. | 1..787 | 616/791 (77%) | 0.0 |
| | | 1..783 | 662/791 (82%) | |
| IJMSCM | M-cadherin - mouse, 730 aa (fragment). | 56..787 | 576/736 (78%) | 0.0 |
| | | 1..729 | 620/736 (83%) | |
| Q8UVQ7 | N-CADHERIN - Brachydanio rerio (Zebrafish) (Zebra danio), 893 aa. | 39..786 | 316/762 (41%) | e-157 |
| | | 140..889 | 443/762 (57%) | |
| Q90275 | NEURAL-CADHERIN PRECURSOR (N-CADHERIN) - Brachydanio rerio (Zebrafish) (Zebra danio), 783 aa. | 39..786 | 315/763 (41%) | e-154 |
| | | 29..779 | 442/763 (57%) | |

PFam analysis predicts that the NOV23a protein contains the domains shown in the Table 23E.

| **Table 23E. Domain Analysis of NOV23a** | | | |
|---|---|---|---|
| **Pfam Domain** | **NOV23a Match Region** | **Identities/ Similarities for the Matched Region** | **Expect Value** |
| cadherin | 50..143 | 23/111 (21%) | 0.011 |
| | | 61/111 (55%) | |
| cadherin | 157..251 | 38/108 (35%) | 8.7e-25 |
| | | 74/108 (69%) | |
| cadherin | 265..367 | 34/107 (32%) | 6.2e-18 |
| | | 74/107 (69%) | |
| cadherin | 380..473 | 34/109 (31%) | 7.7e-20 |
| | | 71/109 (65%) | |
| Cadherin_C_term | 634..788 | 83/158 (53%) | 5.3e-90 |
| | | 146/158 (92%) | |

### Example 24.

The NOV24 clone was analyzed, and the nucleotide and encoded polypeptide sequences are shown in Table 24A.

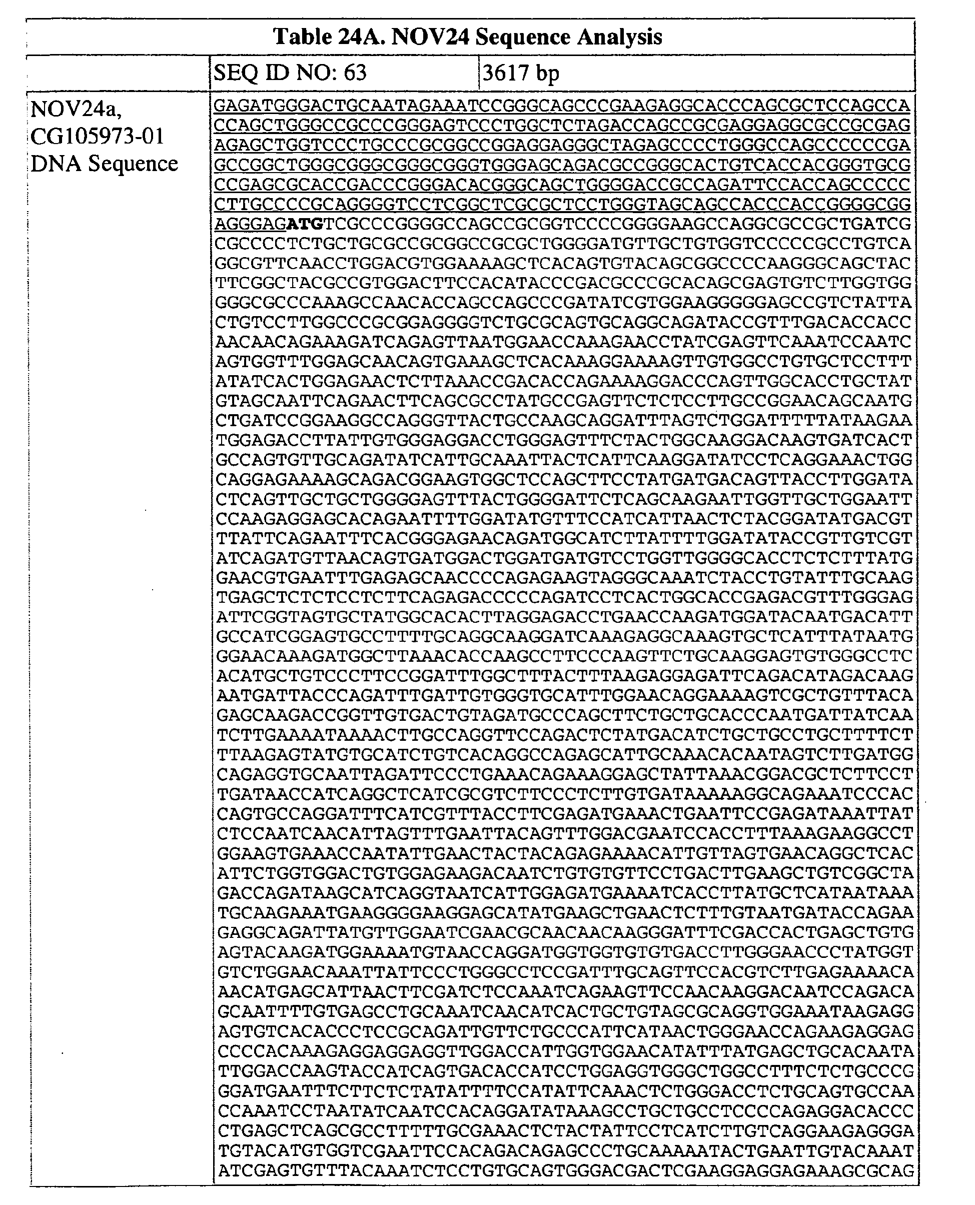

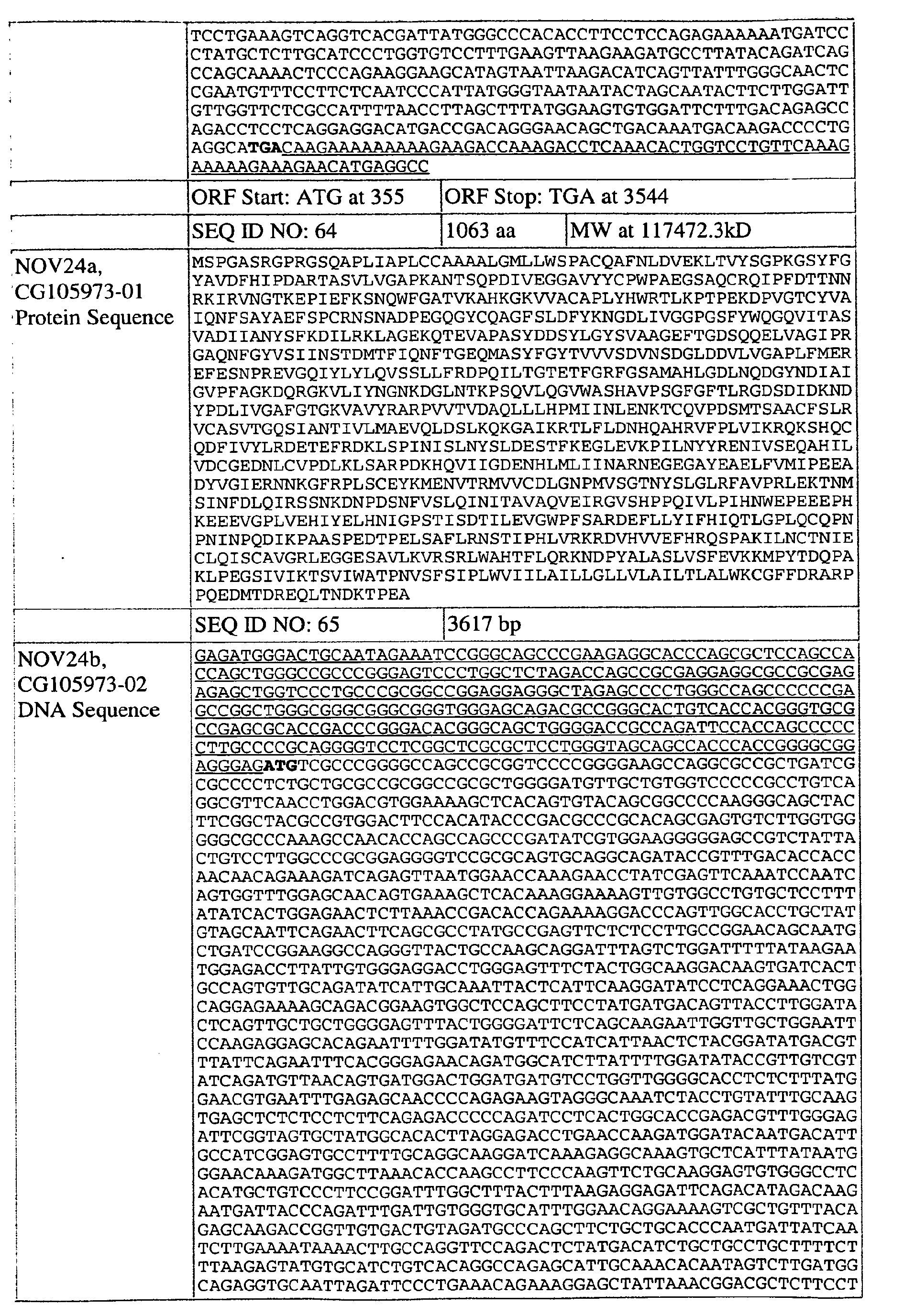

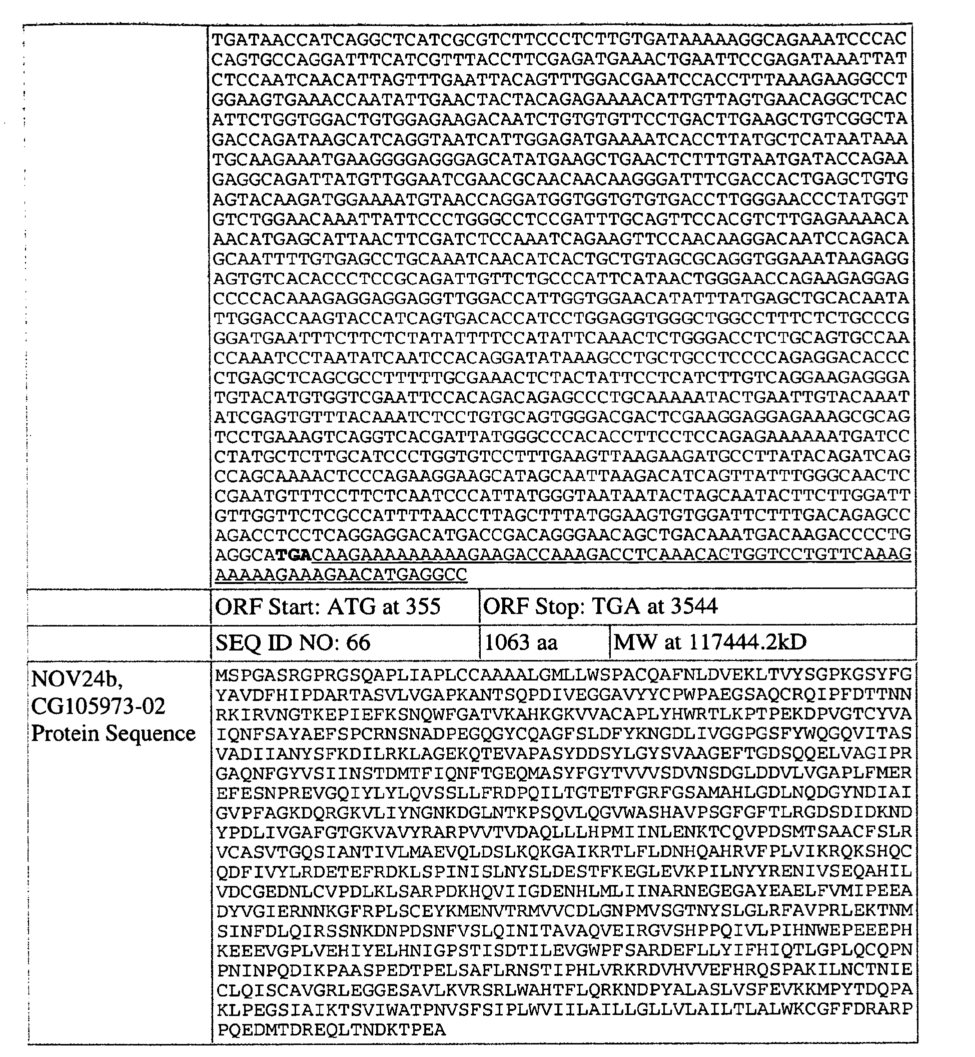

Sequence comparison of the above protein sequences yields the following sequence relationships shown in Table 24B.

| **Table 24B. Comparison of NOV24a against NOV24b.** | | |
|---|---|---|
| **Protein Sequence** | **NOV24a Residues/ Match Residues** | **Identities/ Similarities for the Matched Region** |
| NOV24b | 1..1063 | 1010/1063 (95%) |
| | 1..1063 | 1010/1063 (95%) |

Further analysis of the NOV24a protein yielded the following properties shown in Table 24C.

| **Table 24C. Protein Sequence Properties NOV24a** | |
|---|---|
| PSort analysis: | 0.4600 probability located in plasma membrane; 0.1125 probability located in microbody (peroxisome); 0.1000 probability located in endoplasmic reticulum (membrane); 0.1000 probability located in endoplasmic reticulum (lumen) |
| SignalP analysis: | Cleavage site between residues 39 and 40 |

A search of the NOV24a protein against the Geneseq database, a proprietary database that contains sequences published in patents and patent publication, yielded several homologous proteins shown in Table 24D.

| **Table 24D. Geneseq Results for NOV24a** | | | | |
|---|---|---|---|---|
| **Geneseq Identifier** | **Protein/Organism/Length [Patent #, Date]** | **NOV24a Residues/ Match Residues** | **Identities/ Similarities for the Matched Region** | **Expect Value** |
| AAM39241 | Human polypeptide SEQ ID NO 2386 - Homo sapiens, 1035 aa. [WO200153312-A1, 26-JUL-2001] | 29..1063 | 1031/1035 (99%) | 0.0 |
| | | 1..1035 | 1031/1035 (99%) | |
| AAM41027 | Human polypeptide SEQ ID NO 5958 - Homo sapiens, 1044 aa. [WO200153312-A1, 26-JUL-2001] | 24..1063 | 1024/1046 (97%) | 0.0 |
| | | 1..1044 | 1027/1046 (97%) | |
| ABG18895 | Novel human diagnostic protein #18886 - Homo sapiens, 1061 aa. [WO200175067-A2, 11-OCT-2001] | 8..1055 | 500/1052 (47%) | 0.0 |
| | | 25..1049 | 692/1052 (65%) | |
| ABG18895 | Novel human diagnostic protein #18886 - Homo sapiens, 1061 aa. [W0200175067-A2, 11-OCT-2001] | 8..1055 | 500/1052 (47%) | 0.0 |
| | | 25..1049 | 692/1052 (65%) | |
| AAB70508 | Tissue remodeling protein alpha 5 beta 1 integrin (VLA-5) protein - Mammalian, 1049 aa. [W0200111086-A2, 15-FEB-2001] | 34..1063 | 474/1036 (45%) | 0.0 |
| | | 37..1049 | 667/1036 (63%) | |

In a BLAST search of public sequence databases, the NOV24a protein was found to have homology to the proteins shown in the BLASTP data in Table 24E.

| **Table 24E. Public BLASTP Results for NOV24a** | | | | |
|---|---|---|---|---|
| **Protein Accession Number** | **Protein/Organism/Length** | **NOV24a Residues/ Match Residues** | **Identities/ Similarities for the Matched Portion** | **Expect Value** |
| P53708 | Integrin alpha-8 - Homo sapiens (Human), 1025 aa. | 39..1063 | 1020/1025 (99%) | 0.0 |
| | | 1..1025 | 1020/1025 (99%) | |
| 070304 | INTEGRIN ALPHA8 - Mus musculus (Mouse), 1012 aa (fragment). | 46..1057 | 910/1012 (89%) | 0.0 |
| | | 1..1012 | 972/1012 (95%) | |
| P26009 | Integrin alpha-8 precursor - Gallus gallus (Chicken), 1044 aa. | 27..1063 | 797/1037 (76%) | 0.0 |
| | | 13..1044 | 907/1037 (86%) | |
| P26008 | Integrin alpha-V precursor (Vitronectin receptor alpha subunit) (CD51) - Gallus gallus | 35..1055 | 493/1024 (48%) | 0.0 |
| | (Chicken), 1034 aa. | 16..1022 | 678/1024 (66%) | |
| Q9MZD6 | INTEGRIN ALPHA V SUBUNIT PRECURSOR - Bos taurus (Bovine), 1047 aa. | 8..1055 | 508/1057 (48%) | 0.0 |
| | | 12..1035 | 692/1057 (65%) | |

PFam analysis predicts that the NOV24a protein contains the domains shown in the Table 24F.

| **Table 24F. Domain Analysis of NOV24a** | | | |
|---|---|---|---|
| **Pfam Domain** | **NOV24a Match Region** | **Identities/ Similarities for the Matched Region** | **Expect Value** |
| FG-GAP | 54..117 | 22/67 (33%) | 1.4e-15 |
| | | 53/67 (79%) | |
| FG-GAP | 264..317 | 19/63 (30%) | 2.1e-06 |
| | | 42/63 (67%) | |
| FG-GAP | 318..383 | 23/66 (35%) | 1.7e-15 |
| | | 49/66 (74%) | |
| FG-GAP | 384..443 | 29/67 (43%) | 2e-16 |
| | | 53/67 (79%) | |
| FG-GAP | 447..501 | 22/66 (33%) | 7.8e-10 |
| | | 41/66 (62%) | |
| integrin_A | 1035..1049 | 10/15 (67%) | 0.00011 |
| | | 14/15 (93%) | |

### Example 25.

The NOV25 clone was analyzed, and the nucleotide and encoded polypeptide sequences are shown in Table 25A.

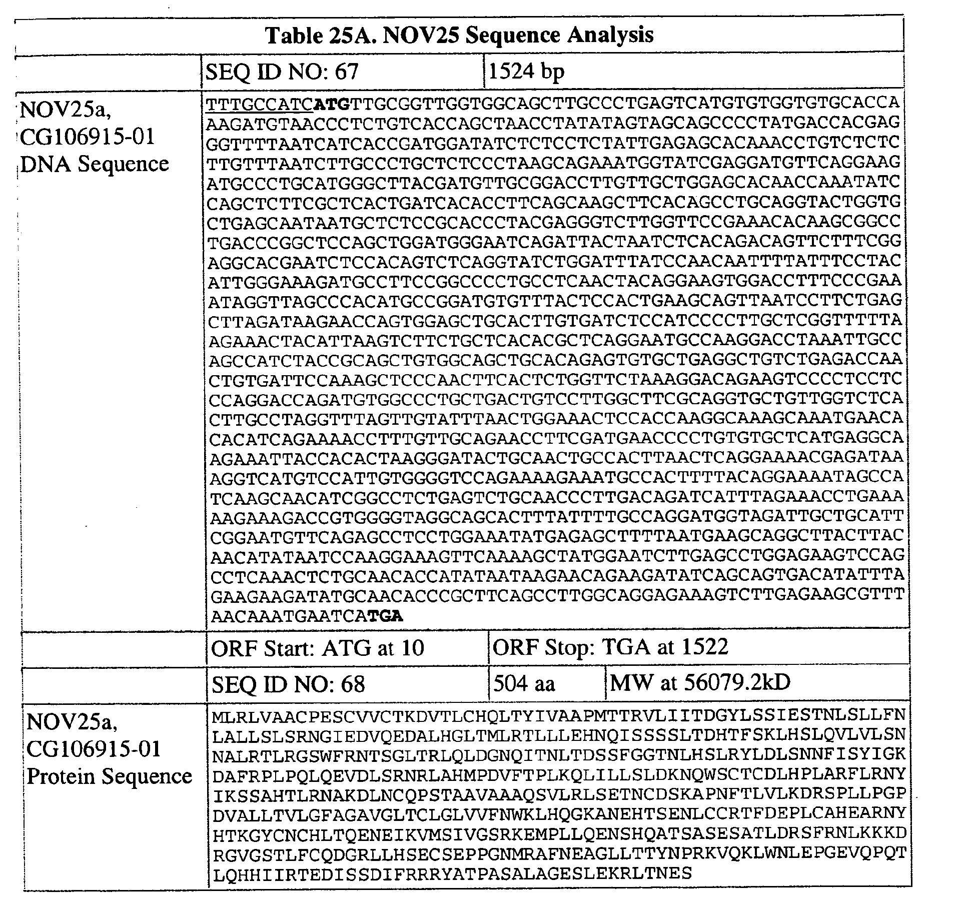

Further analysis of the NOV25a protein yielded the following properties shown in Table 25B.

| **Table 25B. Protein Sequence Properties NOV25a** | |
|---|---|
| PSort analysis: | 0.8524 probability located in mitochondrial inner membrane; 0.7000 probability located in plasma membrane; 0.3000 probability located in microbody (peroxisome); 0.2622 probability located in mitochondrial matrix space |
| SignalP analysis: | No Known Signal Sequence Predicted |

A search of the NOV25a protein against the Geneseq database, a proprietary database that contains sequences published in patents and patent publication, yielded several homologous proteins shown in Table 25C.

| **Table 25C. Geneseq Results for NOV25a** | | | | |
|---|---|---|---|---|
| **Geneseq Identifier** | **Protein/Organism/Length [Patent #, Date]** | **NOV25a Residues/ Match Residues** | **Identities/ Similarities for the Matched Region** | **Expect Value** |
| AAU83655 | Human PRO protein, Seq ID No 128 - Homo sapiens, 473 aa. [WO200208288-A2, 31-JAN-2002] | 3..237 | 79/264 (29%) | 5e-19 |
| | | 22..283 | 121/264 (44%) | |
| AAB49891 | Human PRO526 protein sequence - Homo sapiens, 473 aa. [WO200070050-A1, 23-NOV-2000] | 3..237 | 79/264 (29%) | 5e-19 |
| | | 22..283 | 121/264 (44%) | |
| AAB50908 | Human PRO526 protein - Homo sapiens, 473 aa. [WO200073452-A2, 07-DEC-2000] | 3..237 | 79/264 (29%) | 5e-19 |
| | | 22..283 | 121/264 (44%) | |
| AAU04589 | Human Nogo receptor - Homo sapiens, 473 aa. [WO200151520-A2, 19-JUL-2001] | 3..237 | 79/264 (29%) | 5e-19 |
| | | 22..283 | 121/264 (44%) | |
| AAU12362 | Human PRO526 polypeptide sequence - Homo sapiens, 473 aa. [W0200140466-A2,07-JUN-2001] | 3..237 | 79/264 (29%) | 5e-19 |
| | | 22..283 | 121/264 (44%) | |

In a BLAST search of public sequence databases, the NOV25a protein was found to have homology to the proteins shown in the BLASTP data in Table 25D.

| **Table 25D. Public BLASTP Results for NOV25a** | | | | |
|---|---|---|---|---|
| **Protein Accession Number** | **Protein/Organism/Length** | **NOV25a Residues/ Match Residues** | **Identities/ Similarities for the Matched Portion** | **Expect Value** |
| Q9BGY6 | HYPOTHETICAL 56.5 KDA PROTEIN - Macaca fascicularis (Crab eating macaque) (Cynomolgus monkey), 510 aa. | 1..504 | 478/504 (94%) | 0.0 |
| | | 1..504 | 481/504 (94%) | |
| Q961X3 | GH01279P - Drosophila melanogaster (Fruit fly), 615 aa. | 45..233 | 68/212 (32%) | 3e-21 |
| | | 313..522 | 103/212 (48%) | |
| Q9N0E3 | UNNAMED PROTEIN PRODUCT - Macaca fascicularis (Crab eating macaque) (Cynomolgus monkey), 473 aa. | 3..237 | 82/264 (31%) | 1e-19 |
| | | 22..283 | 125/264 (47%) | |
| Q9VZ84 | CG7509 PROTEIN - Drosophila melanogaster (Fruit fly), 633 aa. | 45..233 | 69/230 (30%) | 6e-19 |
| | | 313..540 | 103/230 (44%) | |
| Q9BZR6 | NOGO RECEPTOR - Homo sapiens (Human), 473 aa. | 3..237 | 79/264 (29%) | 1e-18 |
| | | 22..283 | 121/264 (44%) | |

PFam analysis predicts that the NOV25a protein contains the domains shown in the Table 25E.

| **Table 25E. Domain Analysis of NOV25a** | | | |
|---|---|---|---|
| **Pfam Domain** | **NOV25a Match Region** | **Identities/ Similarities for the Matched Region** | **Expect Value** |
| LRR | 58..81 | 7/25 (28%) | 0.3 |
| | | 19/25 (76%) | |
| LRR | 108..131 | 10/25 (40%) | 0.11 |
| | | 19/25 (76%) | |
| LRR | 132..155 | 8/25 (32%) | 0.7 |
| | | 18/25 (72%) | |
| LRR | 158..181 | 11/25 (44%) | 0.00021 |
| | | 19/25 (76%) | |
| LRR | 182..204 | 10/25 (40%) | 0.093 |
| | | 18/25 (72%) | |
| LRRCT | 214..270 | 15/63 (24%) | 0.046 |
| | | 42/63 (67%) | |

### Example 26.

The NOV26 clone was analyzed, and the nucleotide and encoded polypeptide sequences are shown in Table 26A.

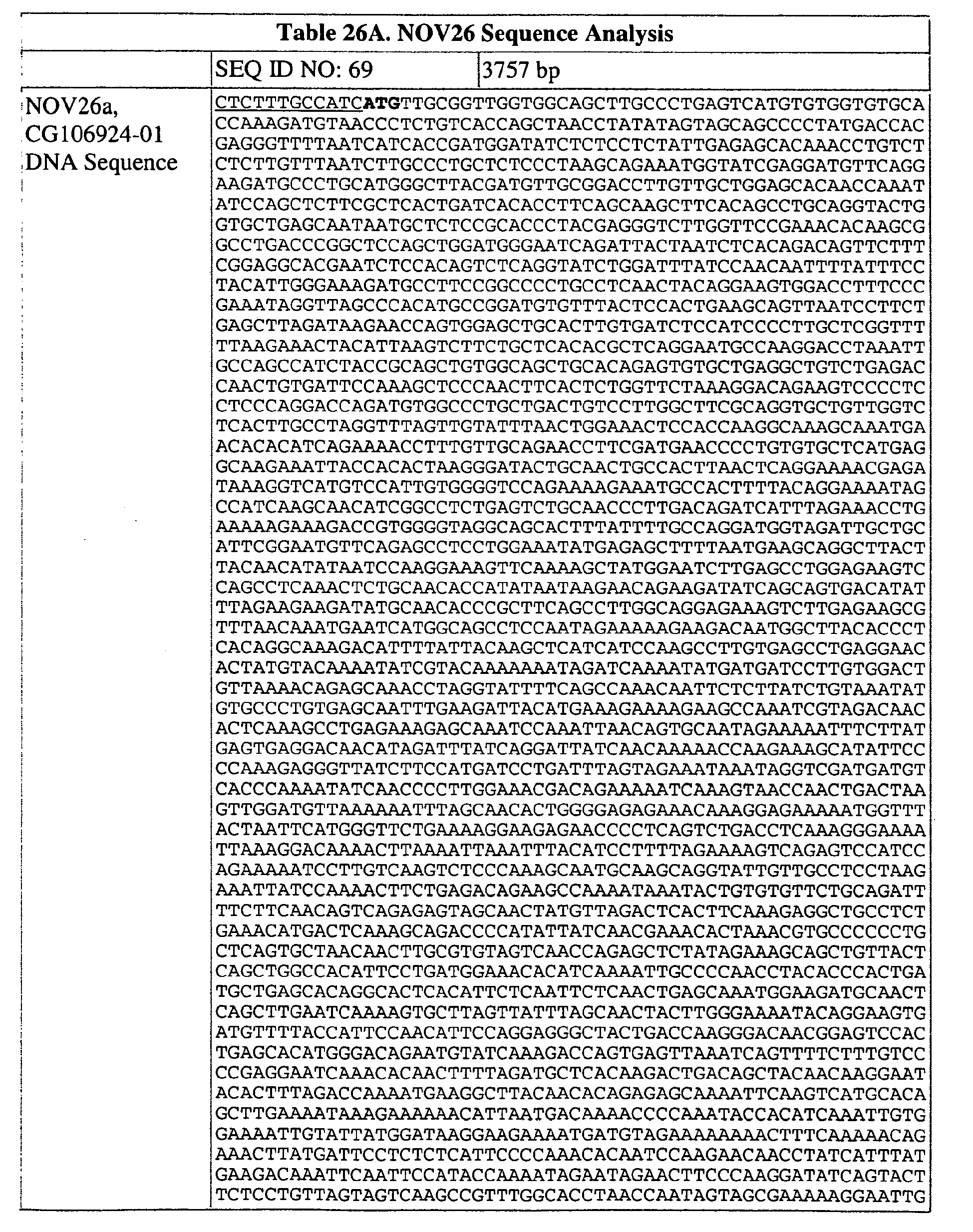

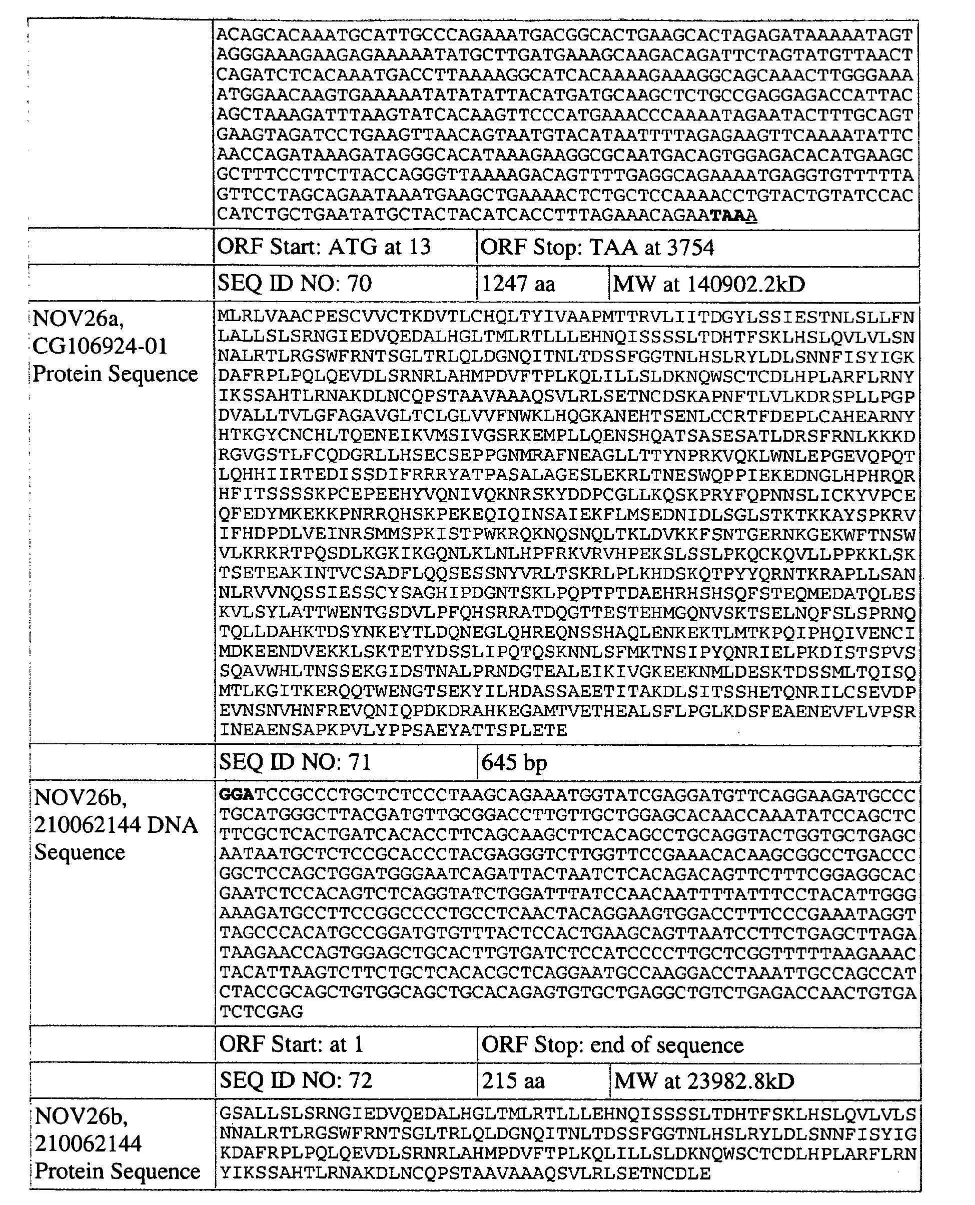

Sequence comparison of the above protein sequences yields the following sequence relationships shown in Table 26B.

| **Table 26B. Comparison of NOV26a against NOV26b.** | | |
|---|---|---|
| **Protein Sequence** | **NOV26a Residues/Match Residues** | **Identities/Similarities for the Matched Region** |
| NOV26b | 60..270 | 199/211 (94%) |
| | 3..213 | 199/211 (94%) |

Further analysis of the NOV26a protein yielded the following properties shown in Table 26C.

| **Table 26C. Protein Sequence Properties NOV26a** | |
|---|---|
| PSort analysis: | 0.8524 probability located in mitochondrial inner membrane; 0.6000 probability located in endoplasmic reticulum (membrane); 0.3000 probability located in microbody (peroxisome); 0.2622 probability located in mitochondrial matrix space |
| SignalP analysis: | No Known Signal Sequence Predicted |

A search of the NOV26a protein against the Geneseq database, a proprietary database that contains sequences published in patents and patent publication, yielded several homologous proteins shown in Table 26D.

| **Table 26D. Geneseq Results for NOV26a** | | | | |
|---|---|---|---|---|
| **Geneseq Identifier** | **Protein/Organism/Length [Patent #, Date]** | **NOV26a Residues/ Match Residues** | **Identities/ Similarities for the Matched Region** | **Expect Value** |
| AAU83655 | Human PRO protein, Seq ID No 128 - Homo sapiens, 473 aa. [WO200208288-A2, 31-JAN-2002] | 3..237 | 79/264 (29%) | 1e-18 |
| | | 22..283 | 121/264 (44%) | |
| AAB49891 | Human PRO526 protein sequence - Homo sapiens, 473 aa. [WO200070050-A1, 23-NOV-2000] | 3..237 | 79/264 (29%) | 1e-18 |
| | | 22..283 | 121/264 (44%) | |
| AAB50908 | Human PRO526 protein - Homo sapiens, 473 aa. [WO200073452-A2, 07-DEC-2000] | 3..237 | 79/264 (29%) | 1e-18 |
| | | 22..283 | 121/264 (44%) | |
| AAU04589 | Human Nogo receptor - Homo sapiens, 473 aa. [W0200151520-A2, 19-JUL-2001] | 3..237 | 79/264 (29%) | 1e-18 |
| | | 22..283 | 121/264 (44%) | |
| AAU12362 | Human PRO526 polypeptide sequence - Homo sapiens, 473 aa. [WO200140466-A2, 07-JUN-2001] | 3..237 | 79/264 (29%) | 1e-18 |
| | | 22..283 | 121/264 (44%) | |

In a BLAST search of public sequence databases, the NOV26a protein was found to have homology to the proteins shown in the BLASTP data in Table 26E.

| **Table 26E. Public BLASTP Results for NOV26a** | | | | |
|---|---|---|---|---|
| **Protein Accession Number** | **Protein/Organism/Length** | **NOV26a Residues/ Match Residues** | **Identities/ Similarities for the Matched Portion** | **Expect Value** |
| Q9BGY6 | HYPOTHETICAL 56.5 KDA PROTEIN - Macaca fascicularis (Crab eating macaque) (Cynomolgus monkey), 510 aa. | 1..504 | 478/504 (94%) | 0.0 |
| | | 1..504 | 481/504 (94%) | |
| Q961X3 | GH01279P - Drosophila melanogaster (Fruit fly), 615 aa. | 45..233 | 68/212 (32%) | 9e-21 |
| | | 313..522 | 103/212 (48%) | |
| Q9N0E3 | UNNAMED PROTEIN PRODUCT - Macaca fascicularis (Crab eating macaque) (Cynomolgus monkey), 473 aa. | 3..237 | 82/264 (31%) | 3e-19 |
| | | 22..283 | 125/264 (47%) | |
| Q9VZ84 | CG7509 PROTEIN - Drosophila melanogaster (Fruit fly), 633 aa. | 45..233 | 69/230 (30%) | le-18 |
| | | 313..540 | 103/230 (44%) | |
| Q9BZR6 | NOGO RECEPTOR - Homo sapiens (Human), 473 aa. | 3..237 | 79/264 (29%) | 3e-18 |
| | | 22..283 | 121/264 (44%) | |

PFam analysis predicts that the NOV26a protein contains the domains shown in the Table 26F.

| **Table 26F. Domain Analysis of NOV26a** | | | |
|---|---|---|---|
| **Pfam Domain** | **NOV26a Match Region** | **Identities/ Similarities for the Matched Region** | **Expect Value** |
| LRR | 58..81 | 7/25 (28%) | 0.3 |
| | | 19/25 (76%) | |
| LRR | 108..131 | 10/25 (40%) | 0.11 |
| | | 19/25 (76%) | |
| LRR | 132..155 | 8/25 (32%) | 0.7 |
| | | 18/25 (72%) | |
| LRR | 158..181 | 11/25 (44%) | 0.00021 |
| | | 19/25 (76%) | |
| LRR | 182..204 | 10/25 (40%) | 0.093 |
| | | 18/25 (72%) | |
| LRRCT | 214..270 | 15/63 (24%) | 0.046 |
| | | 42/63 (67%) | |

### Example 27.

The NOV27 clone was analyzed, and the nucleotide and encoded polypeptide sequences are shown in Table 27A.

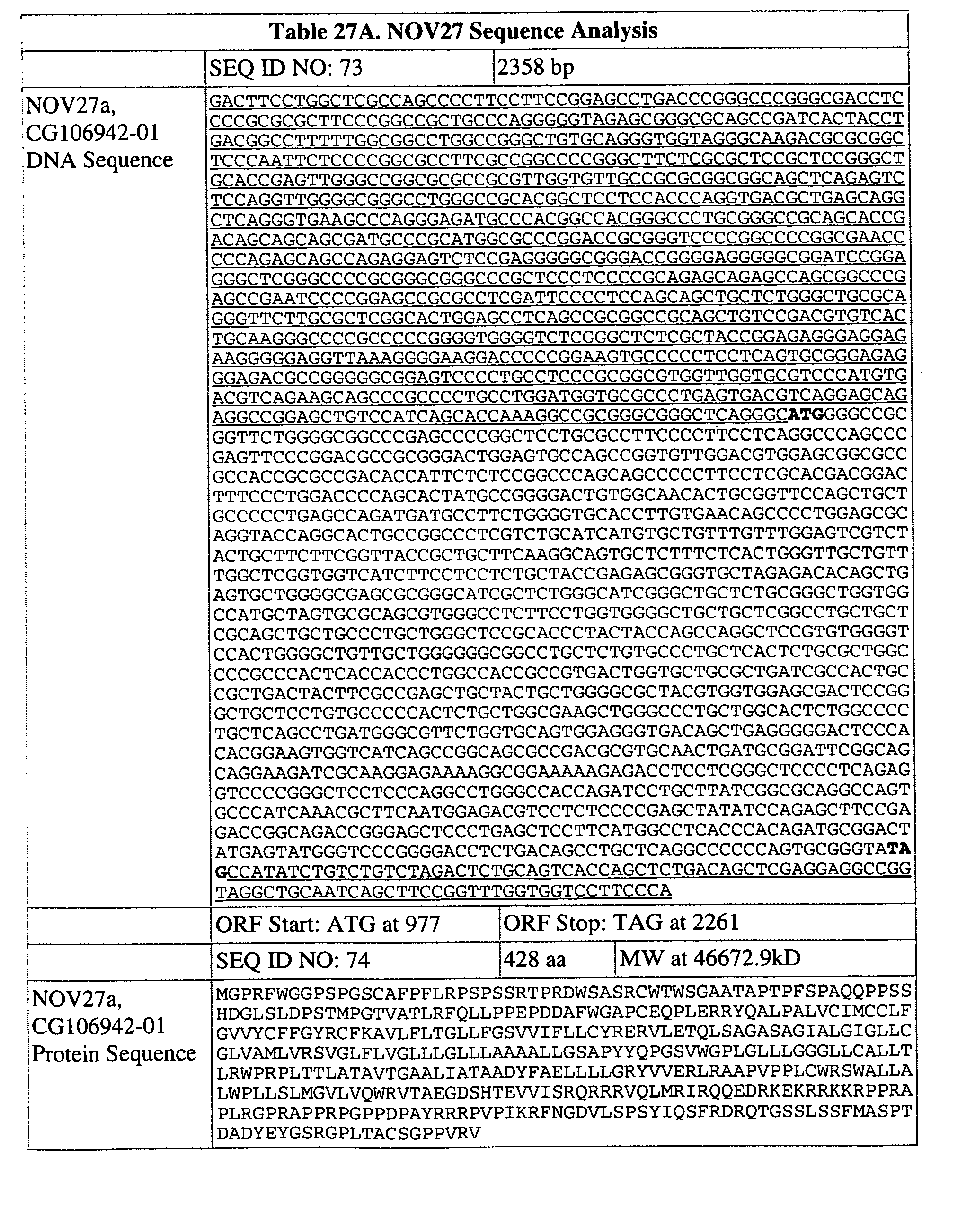

Further analysis of the NOV27a protein yielded the following properties shown in Table 27B.

| **Table 27B. Protein Sequence Properties NOV27a** | |
|---|---|
| PSort analysis: | 0.6000 probability located in plasma membrane; 0.4000 probability located in Golgi body; 0.3000 probability located in endoplasmic reticulum (membrane); 0.2400 probability located in nucleus |
| SignalP analysis: | No Known Signal Sequence Predicted |

A search of the NOV27a protein against the Geneseq database, a proprietary database that contains sequences published in patents and patent publication, yielded several homologous proteins shown in Table 27C.

| **Table 27C. Geneseq Results for NOV27a** | | | | |
|---|---|---|---|---|
| **Geneseq Identifier** | **Protein/Organism/Length [Patent #, Date]** | **NOV27a Residues/ Match Residues** | **Identities/ Similarities for the Matched Region** | **Expect Value** |
| AAM34044 | Peptide #8081 encoded by probe for measuring placental gene expression - Homo sapiens, 124 aa. [WO200157272-A2, 09-AUG-2001] | 79..188 | 60/111 (54%) | 2e-25 |
| | | 19..124 | 76/111 (68%) | |
| AAM20130 | Peptide #6564 encoded by probe for measuring cervical gene expression - Homo sapiens, 124 aa. [WO200157278-A2, 09-AUG-2001] | 79..188 | 60/111 (54%) | 2e-25 |
| | | 19..124 | 76/111 (68%) | |
| AAM73861 | Human bone marrow expressed probe encoded protein SEQ ID NO: 34167 - Homo sapiens, 124 aa. [WO200157276-A2, 09-AUG-2001] | 79..188 | 60/111 (54%) | 2e-25 |
| | | 19..124 | 76/111 (68%) | |
| AAM61147 | Human brain expressed single exon probe encoded protein SEQ ID NO: 33252 - Homo sapiens, 124 aa. [WO200157275-A2, 09-AUG-2001] | 79..188 | 60/111 (54%) | 2e-25 |
| | | 19..124 | 76/111 (68%) | |
| ABB24734 | Protein #6733 encoded by probe for measuring heart cell gene expression - Homo sapiens, 124 aa. [WO200157274-A2, 09-AUG-2001] | 79..188 | 60/111 (54%) | 2e-25 |
| | | 19..124 | 76/111 (68%) | |

In a BLAST search of public sequence databases, the NOV27a protein was found to have homology to the proteins shown in the BLASTP data in Table 27D.

| **Table 27D. Public BLASTP Results for NOV27a** | | | | |
|---|---|---|---|---|
| **Protein Accession Number** | **Protein/Organism/Length** | **NOV27a Residues/ Match Residues** | **Identities/ Similarities for the Matched Portion** | **Expect Value** |
| Q9VWD2 | CG14234 PROTEIN - Drosophila melanogaster (Fruit fly), 381 aa. | 103..392 | 82/299 (27%) | 3e-20 |
| | | 43..329 | 143/299 (47%) | |
| Q9CRG1 | 2010003B14RIK PROTEIN - Mus musculus (Mouse), 556 aa (fragment). | 112..305 | 53/208 (25%) | 6e-07 |
| | | 286..490 | 95/208 (45%) | |
| Q9NS93 | SEVEN TRANSMEMBRANE PROTEIN TM7SF3 - Homo sapiens (Human), 570 aa. | 115..305 | 50/205 (24%) | 4e-05 |
| | | 303..504 | 88/205 (42%) | |
| Q9NUS4 | CDNA FLJ11169 FIS, CLONE PLACE1007282 - Homo sapiens (Human), 570 aa. | 115..305 | 50/205 (24%) | 4e-05 |
| | | 303..504 | 88/205 (42%) | |
| 028838 | Hypothetical protein AF1434 - Archaeoglobus fulgidus, 199 aa. | 107..304 | 51/201 (25%) | 7e-04 |
| | | 14..188 | 82/201 (40%) | |

### Example 28.

The NOV28 clone was analyzed, and the nucleotide and encoded polypeptide sequences are shown in Table 28A.

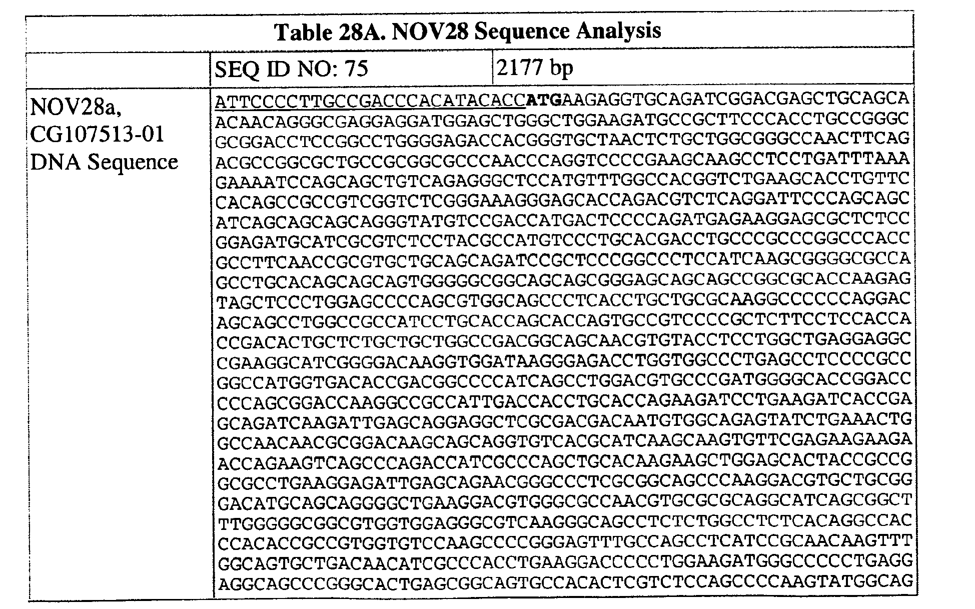

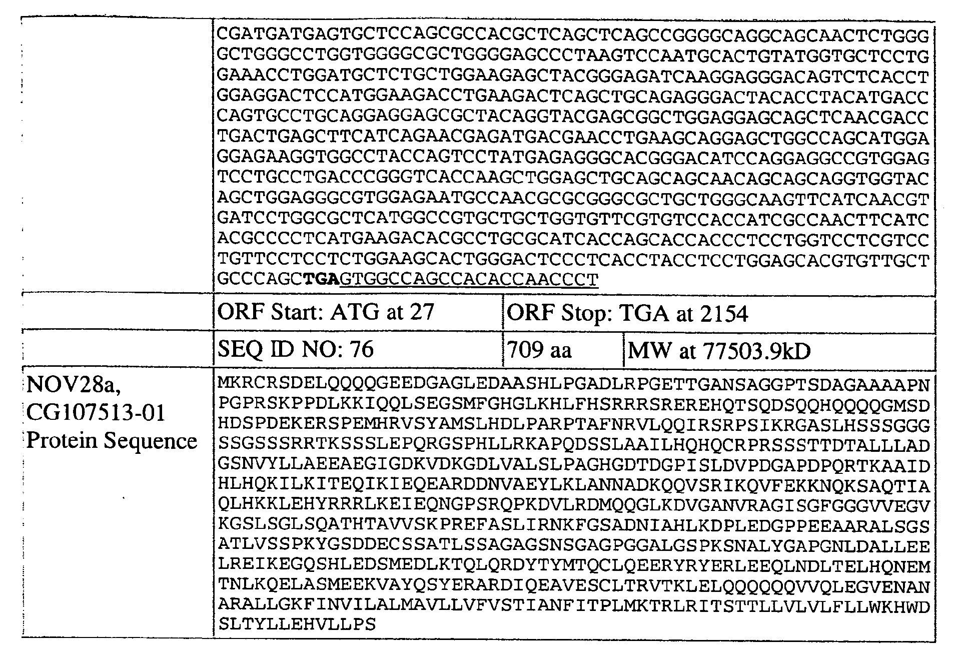

Further analysis of the NOV28a protein yielded the following properties shown in Table 28B.

| **Table 28B. Protein Sequence Properties NOV28a** | |
|---|---|
| PSort analysis: | 0.6000 probability located in plasma membrane; 0.4000 probability located in Golgi body; 0.3000 probability located in endoplasmic reticulum (membrane); 0.3000 probability located in microbody (peroxisome) |
| SignalP analysis: | No Known Signal Sequence Predicted |

A search of the NOV28a protein against the Geneseq database, a proprietary database that contains sequences published in patents and patent publication, yielded several homologous proteins shown in Table 28C.

| **Table 28C. Geneseq Results for NOV28a** | | | | |
|---|---|---|---|---|
| **Geneseq Identifier** | **Protein/Organism/Length [Patent #, Date]** | **NOV28a Residues/ Match Residues** | **Identities/ Similarities for the Matched Region** | **Expect Value** |
| AAY94907 | Human secreted protein clone ca106_19x protein sequence SEQ ID NO:20 - Homo sapiens, 653 aa. [WO200009552-A1, 24-FEB-2000] | 57..703 | 359/672 (53%) | e-178 |
| | | 13..646 | 438/672 (64%) | |
| AAM78708 | Human protein SEQ ID NO 1370 - Homo sapiens, 477 aa. [WO200157190-A2, 09-AUG-2001] | 249..708 | 258/466 (55%) | e-134 |
| | | 26..476 | 326/466 (69%) | |
| AAU28090 | Novel human secretory protein, Seq ID No 259 - Homo sapiens, 446 aa. [WO200166689-A2, 13-SEP-2001] | 258..708 | 254/457 (55%) | e-132 |
| | | 4..445 | 320/457 (69%) | |
| AAM40705 | Human polypeptide SEQ ID NO 5636 - Homo sapiens, 369 aa. [WO200153312-A1, 26-JUL-2001] | 352..703 | 224/355 (63%) | e-117 |
| | | 13..362 | 267/355 (75%) | |
| AAM38919 | Human polypeptide SEQ ID NO 2064 - Homo sapiens, 331 aa. [WO200153312-A1, 26-JUL-2001] | 379..703 | 209/328 (63%) | e-108 |
| | | 2..324 | 248/328 (74%) | |

In a BLAST search of public sequence databases, the NOV28a protein was found to have homology to the proteins shown in the BLASTP data in Table 28D.

| **Table 28D. Public BLASTP Results for NOV28a** | | | | |
|---|---|---|---|---|
| **Protein Accession Number** | **Protein/Organism/Length** | **NOV28a Residues/ Match Residues** | **Identities/ Similarities for the Matched Portion** | **Expect Value** |
| 075069 | Hypothetical protein KIAA0481 (Cerebral protein-11) (hucep-11) - Homo sapiens (Human), 650 aa | 69..709 | 638/641 (99%) | 0.0 |
| | (fragment). | 10..650 | 640/641 (99%) | |
| Q9ULS5 | Hypothetical protein KIAA1145 - Homo sapiens (Human), 467 aa (fragment). | 250..708 | 258/465 (55%) | e-134 |
| | | 17..466 | 325/465 (69%) | |
| AAH26867 | SIMILAR TO KIAA1145 PROTEIN - Mus musculus (Mouse), 446 aa. | 258..708 | 249/457 (54%) | e-129 |
| | | 4..445 | 316/457 (68%) | |
| 094876 | Hypothetical protein KIAA0779 - Homo sapiens (Human), 320 aa (fragment). | 393..703 | 202/314 (64%) | e-105 |
| | | 1..313 | 241/314 (76%) | |
| Q9VI21 | CG1021 PROTEIN - Drosophila melanogaster (Fruit fly), 638 aa. | 288..675 | 188/409 (45%) | 1e-84 |
| | | 252..638 | 248/409 (59%) | |

### Example 29.

The NOV29 clone was analyzed, and the nucleotide and encoded polypeptide sequences are shown in Table 29A.

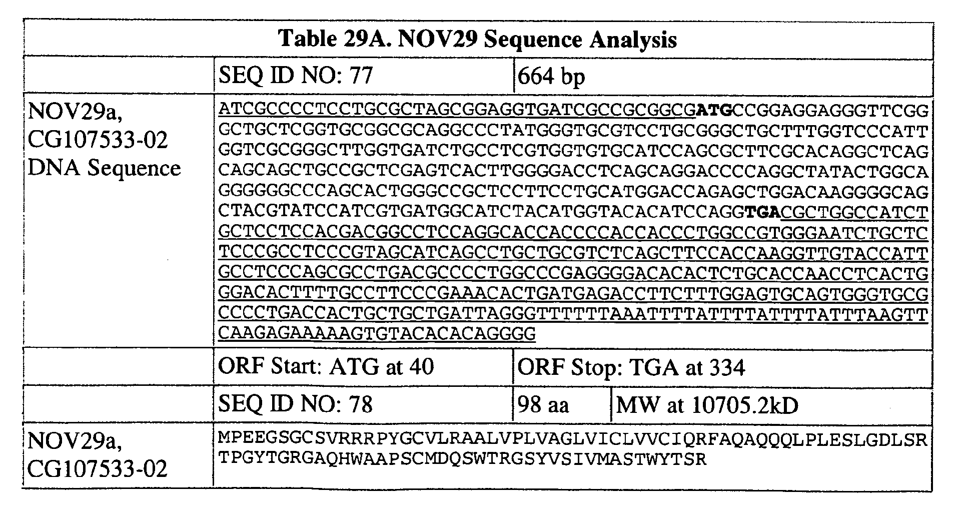

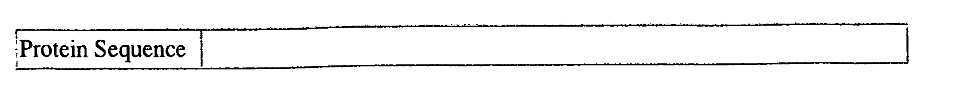

Further analysis of the NOV29a protein yielded the following properties shown in Table 29B.

| **Table 29B. Protein Sequence Properties NOV29a** | |
|---|---|
| PSort analysis: | 0.7900 probability located in plasma membrane; 0.3000 probability located in Golgi body; 0.2000 probability located in endoplasmic reticulum (membrane); 0.1000 probability located in mitochondrial inner membrane |
| SignalP analysis: | Cleavage site between residues 45 and 46 |

A search of the NOV29a protein against the Geneseq database, a proprietary database that contains sequences published in patents and patent publication, yielded several homologous proteins shown in Table 29C.

| **Table 29C. Geneseq Results for NOV29a** | | | | |
|---|---|---|---|---|
| **Geneseq Identifier** | **Protein/Organism/Length [Patent #, Date]** | **NOV29a Residues/ Match Residues** | **Identities/ Similarities for the Matched Region** | **Expect Value** |
| AAR50121 | CD27L - Homo sapiens, 193 aa. [WO9405691-A, 17-MAR-1994] | 1..54 | 54/54 (100%) | 7e-25 |
| | | 1..54 | 54/54 (100%) | |
| AAW41180 | CD27 ligand - Homo sapiens, 216 aa. [US5716805-A, 10-FEB-1998] | 39..54 | 16/16 (100%) | 0.16 |
| | | 62..77 | 16/16 (100%) | |
| AAR50122 | sCD27L-3 - Homo sapiens, 216 aa. [WO9405691-A, 17-MAR-1994] | 39..54 | 16/16 (100%) | 0.16 |
| | | 62..77 | 16/16 (100%) | |
| AAR53971 | CD27-L type II transmembrane protein - Mammalia, 216 aa. [WO9410308-A, 11-MAY-1994] | 39..54 | 16/16 (100%) | 0.16 |
| | | 62..77 | 16/16 (100%) | |
| AAG26041 | Zea mays protein fragment SEQ ID NO: 30347 - Zea mays subsp. mays, 172 aa. [EP1033405-A2, 06-SEP-2000] | 24..72 | 19/55 (34%) | 2.4 |
| | | 6..59 | 26/55 (46%) | |

In a BLAST search of public sequence databases, the NOV29a protein was found to have homology to the proteins shown in the BLASTP data in Table 29D.

| **Table 29D. Public BLASTP Results for NOV29a** | | | | |
|---|---|---|---|---|
| **Protein Accession Number** | **Protein/Organism/Length** | **NOV29a Residues/ Match Residues** | **Identities/ Similarities for the Matched Portion** | **Expect Value** |
| Q96J57 | TUMOR NECROSIS FACTOR (LIGAND) SUPERFAMILY, MEMBER 7 - Homo sapiens (Human), 193 aa. | 1..54 | 54/54 (100%) | 2e-24 |
| | | 1..54 | 54/54 (100%) | |
| P32970 | CD27 ligand (CD27-L) (CD70 antigen) - Homo sapiens (Human), 193 aa. | 1..54 | 54/54 (100%) | 2e-24 |
| | | 1..54 | 54/54 (100%) | |
| Q9KFY7 | HYPOTHETICAL PROTEIN BH0329 - Bacillus halodurans, 423 aa. | 39..98 | 18/61 (29%) | 7.4 |
| | | 240..300 | 28/61 (45%) | |
| Q9RC64 | UNKNOWN - Bacillus halodurans, 262 aa. | 39..98 | 18/61 (29%) | 7.4 |
| | | 79..139 | 28/61 (45%) | |
| 034255 | PURL PROTEIN - Wolinella succinogenes, 331 aa (fragment). | 70..93 | 11/24 (45%) | 9.7 |
| | | 8..31 | 15/24 (61%) | |

### Example 30.

The NOV30 clone was analyzed, and the nucleotide and encoded polypeptide sequences are shown in Table 30A.

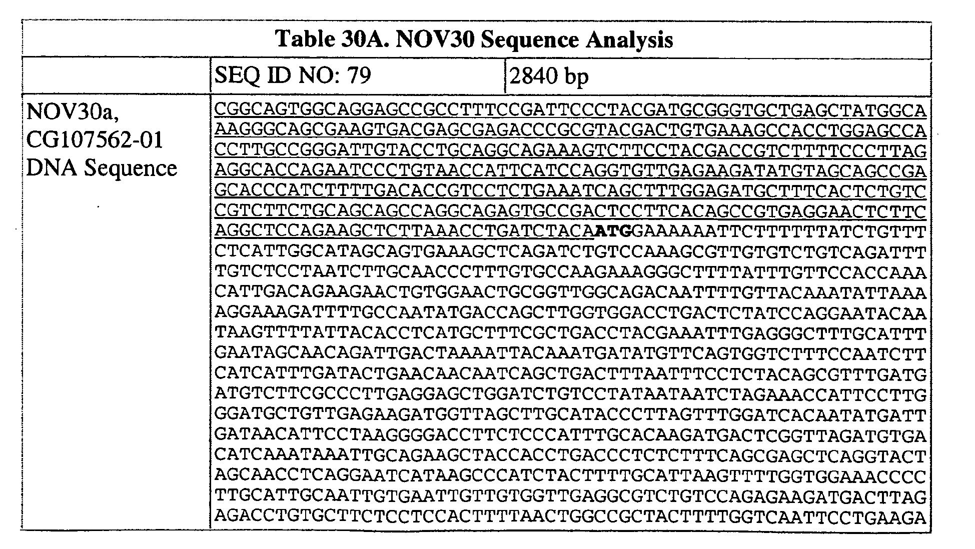

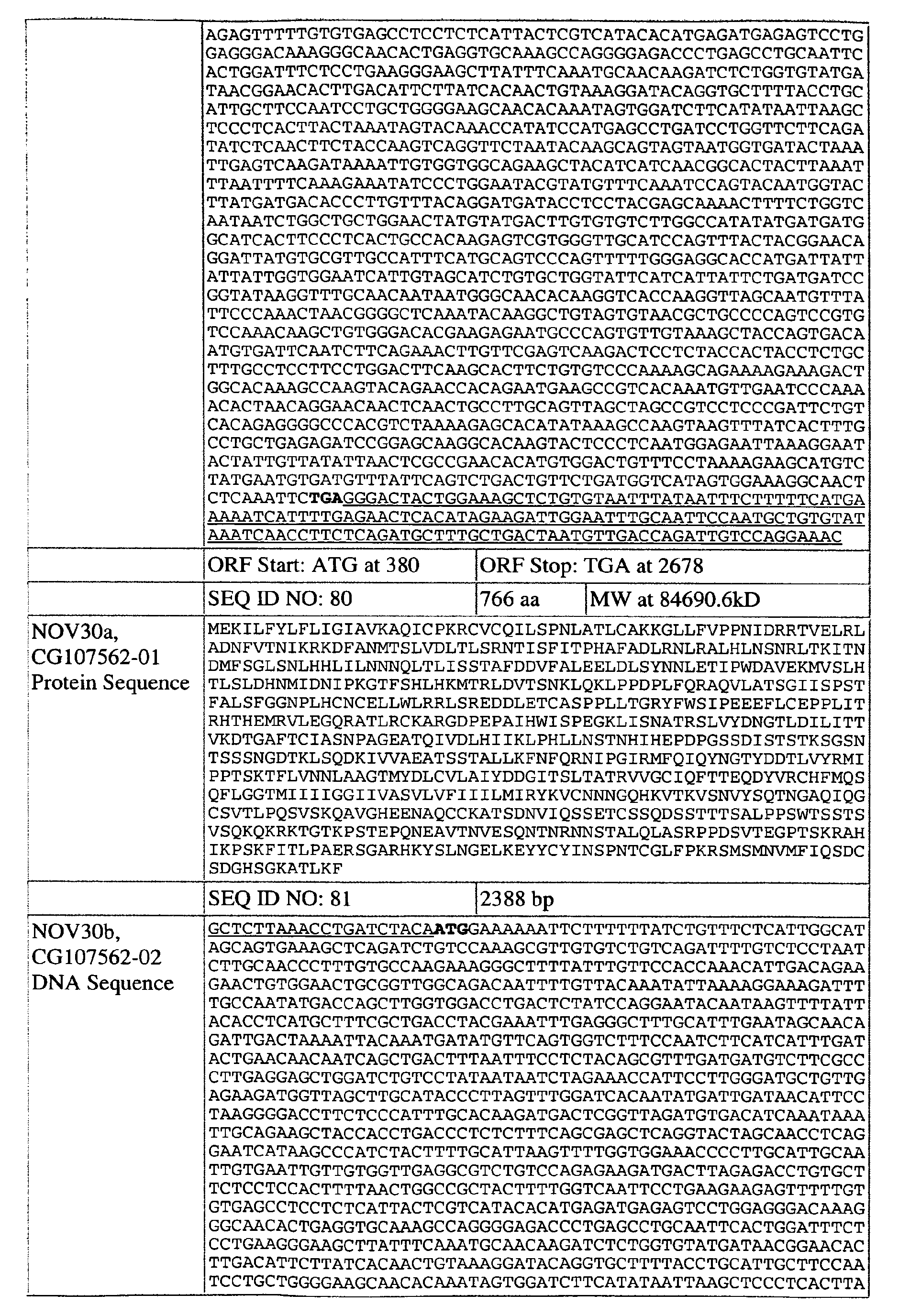

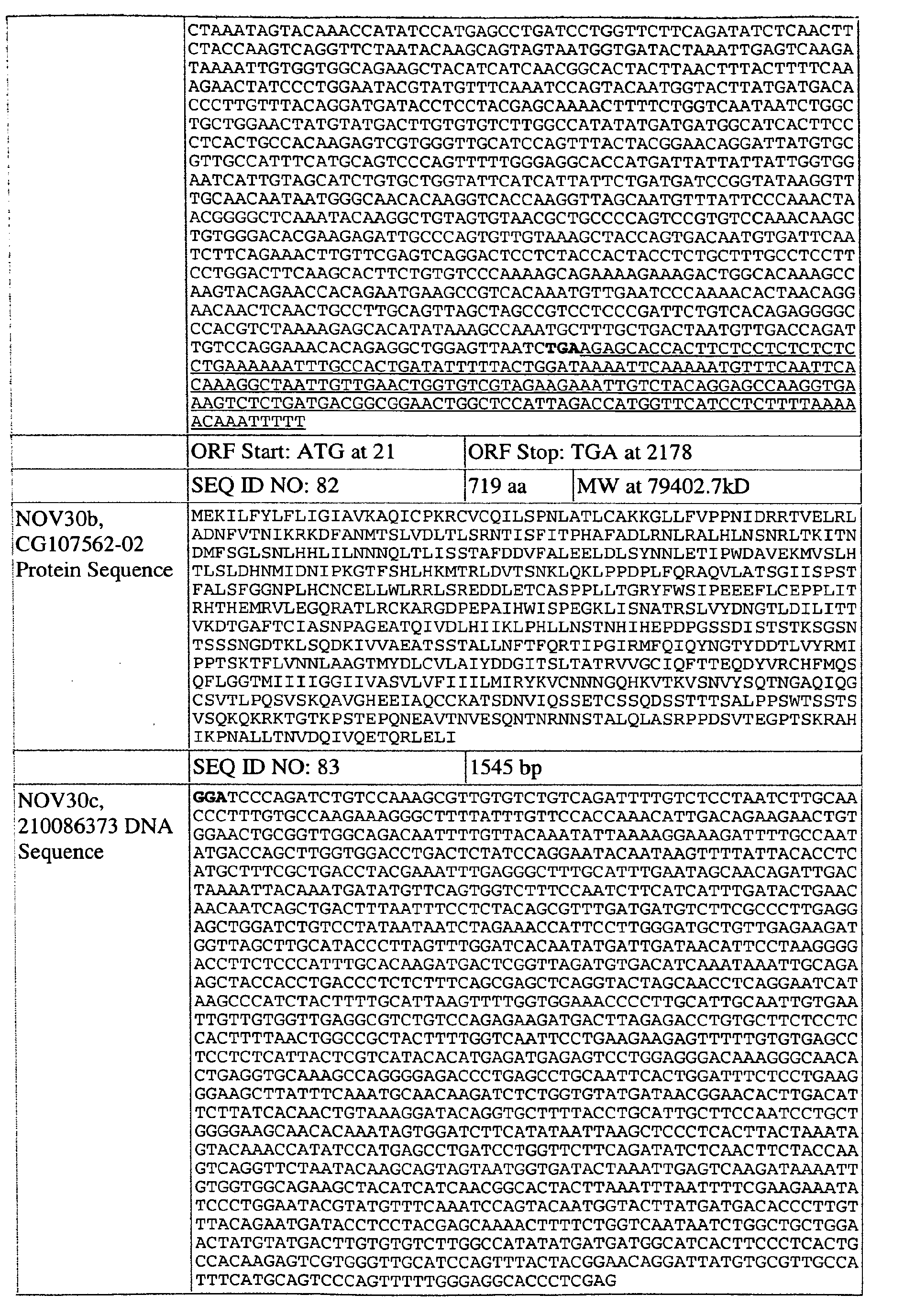

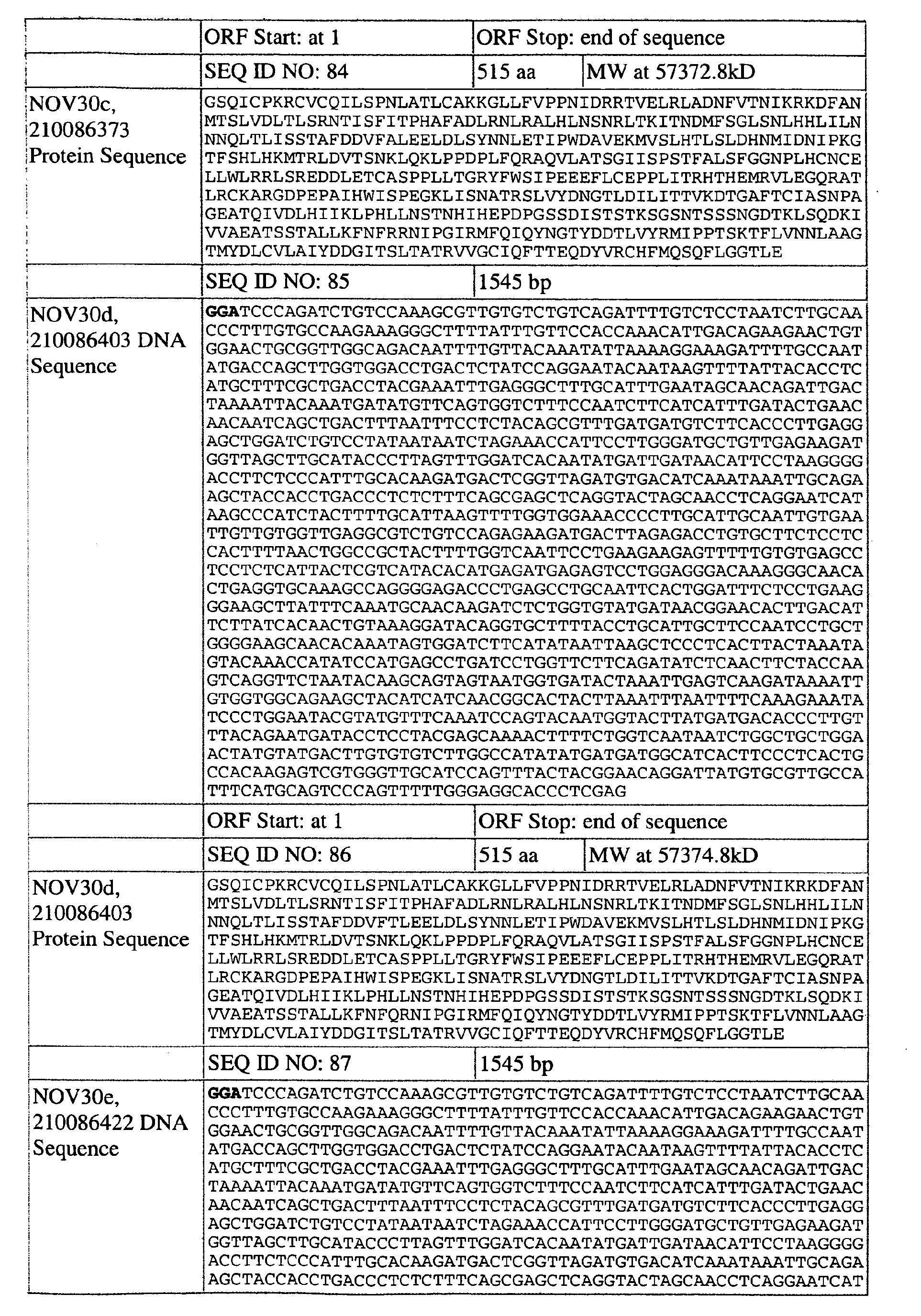

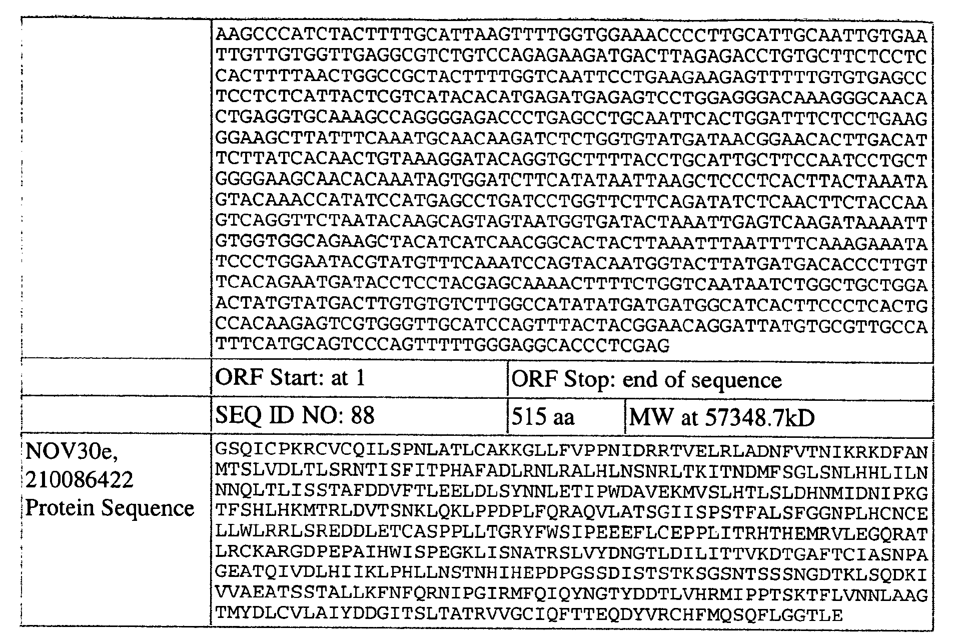

Sequence comparison of the above protein sequences yields the following sequence relationships shown in Table 30B.

| **Table 30B. Comparison of NOV30a against NOV30b through NOV30e.** | | |
|---|---|---|
| **Protein Sequence** | **NOV30a Residues/ Match Residues** | **Identities/ Similarities for the Matched Region** |
| NOV30b | 1..704 | 624/704 (88%) |
| | 1..704 | 626/704 (88%) |
| NOV30c | 17..529 | 465/513 (90%) |
| | 2..514 | 468/513 (90%) |
| NOV30d | 17..529 | 465/513 (90%) |
| | 2..514 | 467/513 (90%) |
| NOV30e | 17..529 | 464/513 (90%) |
| | 2..514 | 467/513 (90%) |

Further analysis of the NOV30a protein yielded the following properties shown in Table 30C.

| **Table 30C. Protein Sequence Properties NOV30a** | |
|---|---|
| PSort analysis: | 0.6850 probability located in endoplasmic reticulum (membrane); 0.6400 probability located in plasma membrane; 0.4600 probability located in Golgi body; 0.1000 probability located in endoplasmic reticulum (lumen) |
| SignalP analysis: | Cleavage site between residues 18 and 19 |

A search of the NOV30a protein against the Geneseq database, a proprietary database that contains sequences published in patents and patent publication, yielded several homologous proteins shown in Table 30D.

| **Table 30D. Geneseq Results for NOV30a** | | | | |
|---|---|---|---|---|
| **Geneseq Identifier** | **Protein/Organism/Length [Patent #, Date]** | **NOV30a Residues/ Match Residues** | **Identities/ Similarities for the Matched Region** | **Expect Value** |
| AAG67505 | Amino acid sequence of a human secreted polypeptide - Homo sapiens, 766 aa. [WO200166690-A2, 13-SEP-2001] | 1..766 | 765/766 (99%) | 0.0 |
| | | 1..766 | 766/766 (99%) | |
| AAB09968 | Human brain-specific transmembrane glycoprotein - Homo sapiens, 789 aa. [WO200031256-A1, 02-JUN-2000] | 1..756 | 374/780 (47%) | 0.0 |
| | | 1..770 | 505/780 (63%) | |
| AAM39059 | Human polypeptide SEQ ID NO 2204 - Homo sapiens, 789 aa. [WO200153312-A1, 26-JUL-2001] | 1..756 | 373/780 (47%) | 0.0 |
| | | 1..770 | 504/780 (63%) | |
| AAU28092 | Novel human secretory protein, Seq ID No 261 - Homo sapiens, 789 aa. [WO200166689-A2, 13-SEP-2001] | 1..756 | 373/780 (47%) | 0.0 |
| | | 1..770 | 504/780 (63%) | |
| AAB12448 | Human hh00149 protein SEQ ID NO:4 - Homo sapiens, 785 aa. [WO200031255-A1, 02-JUN-2000] | 20..756 | 368/760 (48%) | 0.0 |
| | | 17..766 | 497/760 (64%) | |

In a BLAST search of public sequence databases, the NOV30a protein was found to have homology to the proteins shown in the BLASTP data in Table 30E.

| **Table 30E. Public BLASTP Results for NOV30a** | | | | |
|---|---|---|---|---|
| **Protein Accession Number** | **Protein/Organism/Length** | **NOV30a Residues/ Match Residues** | **Identities/ Similarities for the Matched Portion** | **Expect Value** |
| Q96NI6 | CDNA FLJ30803 FIS, CLONE FEBRA2001245, WEAKLY SIMILAR TO NAG14 - Homo sapiens (Human), 719 aa. | 1..704 | 699/704 (99%) | 0.0 |
| | | 1..704 | 701/704 (99%) | |
| Q9ULH4 | KIAA1246 PROTEIN - Homo sapiens (Human), 832 aa (fragment). | 1..756 | 374/780 (47%) | 0.0 |
| | | 44..813 | 505/780 (63%) | |
| Q9BE71 | HYPOTHETICAL 84.7 KDA PROTEIN - Macaca fascicularis (Crab eating macaque) (Cynomolgus monkey), 789 aa. | 1..756 | 374/780 (47%) | 0.0 |
| | | 1..770 | 503/780 (63%) | |
| Q9CYK3 | 5730420O05RIK PROTEIN - Mus musculus (Mouse), 788 aa. | 1..756 | 378/783 (48%) | 0.0 |
| | | 1..769 | 506/783 (64%) | |
| Q9P244 | KIAA1484 PROTEIN - Homo sapiens (Human), 700 aa (fragment). | 58..701 | 332/655 (50%) | e-177 |
| | | 1..631 | 441/655 (66%) | |

PFam analysis predicts that the NOV30a protein contains the domains shown in the Table 30F.

| **Table 30F. Domain Analysis of NOV30a** | | | |
|---|---|---|---|
| **Pfam Domain** | **NOV30a Match Region** | **Identities/ Similarities for the Matched Region** | **Expect Value** |
| LRR | 76..99 | 9/25 (36%) | 0.49 |
| | | 18/25 (72%) | |
| LRR | 100..123 | 8/25 (32%) | 0.0031 |
| | | 21/25 (84%) | |
| LRR | 148..171 | 11/25 (44%) | 0.0021 |
| | | 20/25 (80%) | |
| LRR | 172..195 | 7/25 (28%) | 0.0035 |
| | | 19/25 (76%) | |
| LRRCT | 240..285 | 21/54 (39%) | 0.00023 |
| | | 37/54 (69%) | |
| ig | 301..359 | 15/62 (24%) | 1.4e-08 |
| | | 41/62 (66%) | |
| fn3 | 416..496 | 16/86 (19%) | 0.066 |
| | | 57/86 (66%) | |

### Example 31.

The NOV31 clone was analyzed, and the nucleotide and encoded polypeptide sequences are shown in Table 31A.

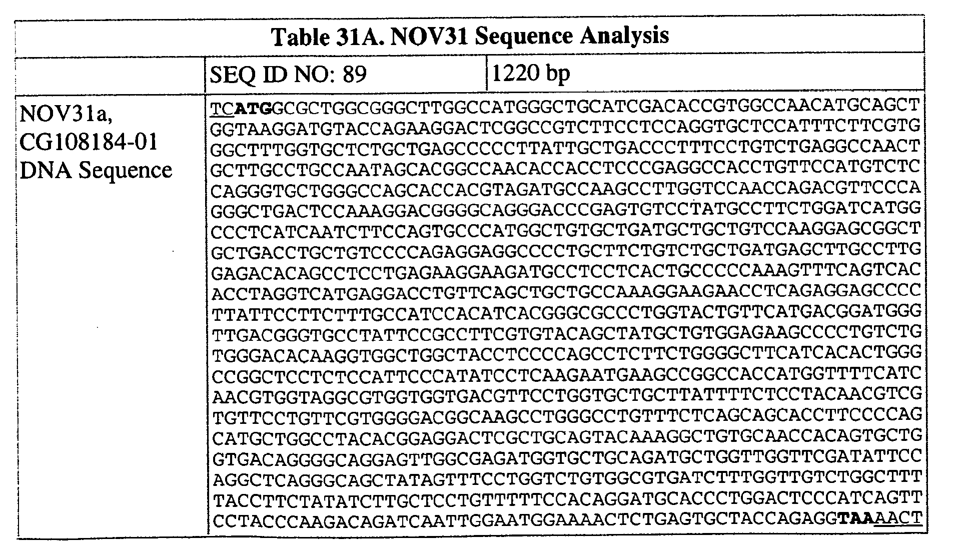

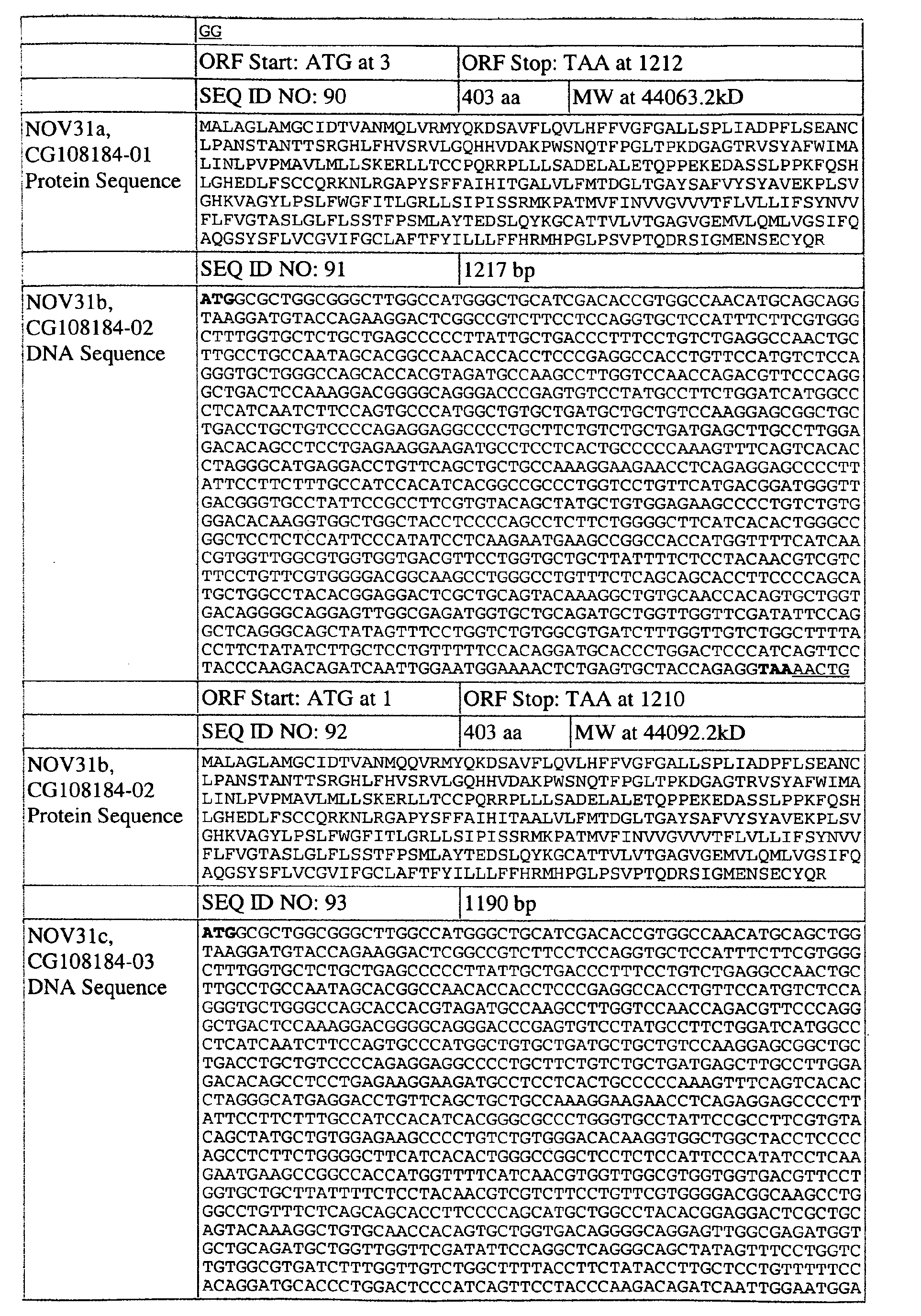

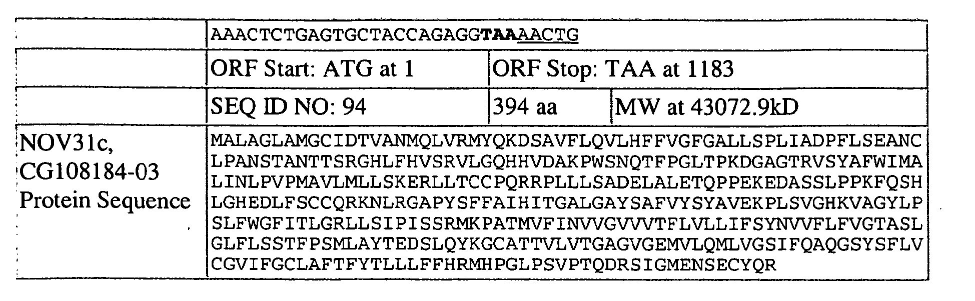

Sequence comparison of the above protein sequences yields the following sequence relationships shown in Table 31B.

| **Table 31B. Comparison of NOV31a against NOV31b and NOV31c.** | | |
|---|---|---|
| **Protein Sequence** | **NOV31a Residues/ Match Residues** | **Identities/ Similarities for the Matched Region** |
| NOV31b | 1..403 | 383/403 (95%) |
| | 1..403 | 383/403 (95%) |
| NOV31c | 1..403 | 364/403 (90%) |
| | 1..394 | 364/403 (90%) |

Further analysis of the NOV31a protein yielded the following properties shown in Table 31C.

| **Table 31C. Protein Sequence Properties NOV31a** | |
|---|---|
| PSort analysis: | 0.6000 probability located in plasma membrane; 0.4445 probability located in mitochondrial inner membrane; 0.4000 probability located in Golgi body; 0.3000 probability located in endoplasmic reticulum (membrane) |
| SignalP analysis: | Cleavage site between residues 50 and 51 |

A search of the NOV31a protein against the Geneseq database, a proprietary database that contains sequences published in patents and patent publication, yielded several homologous proteins shown in Table 31D.

| **Table 31D. Geneseq Results for NOV31a** | | | | |
|---|---|---|---|---|
| **Geneseq Identifier** | **Protein/Organism/Length [Patent #, Date]** | **NOV31a Residues/ Match Residues** | **Identities/ Similarities for the Matched Region** | **Expect Value** |
| AAM24206 | Human EST encoded protein SEQ ID NO: 1731 - Homo sapiens, 226 aa. [WO200154477-A2, 02-AUG-2001] | 1..101 | 76/109 (69%) | 9e-32 |
| | | 112..220 | 81/109 (73%) | |
| AAY11034 | H. pylori ORF 04ep41903_19689182_c1_43 inner membrane protein - Helicobacter pylori, 407 aa. [WO9824475-A1, 11-JUN-1998] | 201..346 | 32/146 (21%) | 0.35 |
| | | 226..368 | 68/146 (45%) | |
| AAY11033 | H. pylori ORF 04ep41903_16667055_c1_37 inner membrane protein - Helicobacter pylori, 312 aa. [W09824475-A1, 11-JUN-1998] | 201..346 | 32/146 (21%) | 0.35 |
| | | 131..273 | 68/146 (45%) | |
| ABB68766 | Drosophila melanogaster polypeptide SEQ ID NO 33090 - Drosophila melanogaster, 816 aa. [WO200171042-A2, 27-SEP-2001] | 212..371 | 39/168 (23%) | 0.46 |
| | | 582..746 | 71/168 (42%) | |
| ABB48281 | Listeria monocytogenes protein #985 - Listeria monocytogenes, 402 aa. [W0200177335-A2, 18-OCT-2001] | 250..356 | 32/117 (27%) | 1.0 |
| | | 62..172 | 52/117 (44%) | |

In a BLAST search of public sequence databases, the NOV31a protein was found to have homology to the proteins shown in the BLASTP data in Table 31E.

| **Table 31E. Public BLASTP Results for NOV31a** | | | | |
|---|---|---|---|---|
| **Protein Accession Number** | **Protein/Organism/Length** | **NOV31a Residues/ Match Residues** | **Identities/ Similarities for the Matched Portion** | **Expect Value** |
| Q9GM43 | HYPOTHETICAL 44.1 KDA PROTEIN - Macaca fascicularis (Crab eating macaque) (Cynomolgus monkey), 403 aa. | 1..403 | 397/403 (98%) | 0.0 |
| | | 1..403 | 399/403 (98%) | |
| CAD28481 | HYPOTHETICAL 34.1 KDA PROTEIN - Homo sapiens (Human), 311 aa (fragment). | 93..403 | 311/311 (100%) | e-180 |
| | | 1..311 | 311/311 (100%) | |
| Q9D8I5 | 2010001E11RIK PROTEIN - Mus musculus (Mouse), 366 aa. | 205..375 | 49/171 (28%) | 1e-13 |
| | | 188..353 | 89/171 (51%) | |
| Q8VCV9 | SIMILAR TO RIKEN CDNA 2010001E11 GENE - Mus musculus (Mouse), 377 aa. | 205..375 | 46/171 (26%) | 1e-12 |
| | | 187..352 | 89/171 (51%) | |
| Q96PW9 | KIAA1919 PROTEIN - Homo sapiens (Human), 403 aa (fragment). | 205..353 | 44/149 (29%) | 1e-12 |
| | | 121..265 | 79/149 (52%) | |

### Example 32.

The NOV32 clone was analyzed, and the nucleotide and encoded polypeptide sequences are shown in Table 32A.

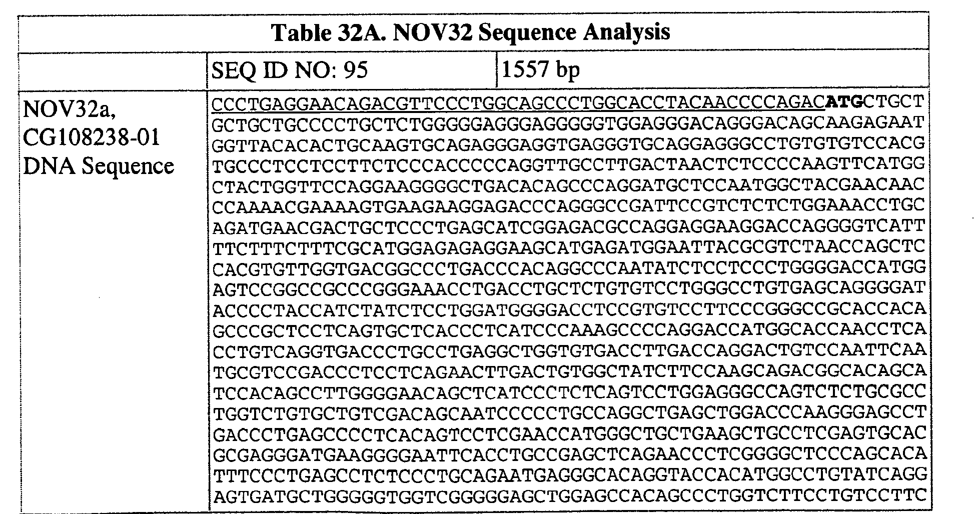

| **Table 32C. Geneseq Results for NOV32a** | | | | |
|---|---|---|---|---|
| **Geneseq Identifier** | **Protein/Organism/Length [Patent #, Date]** | **NOV32a Residues/ Match Residues** | **Identities/ Similarities for the Matched Region** | **Expect Value** |
| ABB53288 | Human polypeptide #28 - Homo sapiens, 490 aa. [W0200181363-A1, 01-NOV-2001] | 1..477 | 326/479 (68%) | e-180 |
| | | 5..476 | 374/479 (78%) | |
| AAE15809 | Human sialoadhesin factor-3 (SAF-3) - Homo sapiens, 467 aa. [WO200190193-A1, 29-NOV-2001] | 1..469 | 317/469 (67%) | e-172 |
| | | 3..467 | 362/469 (76%) | |
| AAW59992 | Sialoadhesin family member-3 (SAF-3) - Homo sapiens, 467 aa. [EP869178-A1, 07-OCT-1998] | 1..469 | 317/469 (67%) | e-172 |
| | | 3..467 | 362/469 (76%) | |
| AAB29188 | Siglec 5 protein - Homo sapiens, 467 aa. [WO200053747-A1, 14-SEP-2000] | 1..469 | 316/469 (67%) | e-171 |
| | | 3..467 | 361/469 (76%) | |
| AAU14582 | Human novel protein #453 - Homo sapiens, 467 aa. [WO200155437-A2, 02-AUG-2001] | 1..469 | 316/469 (67%) | e-171 |
| | | 3..467 | 361/469 (76%) | |

In a BLAST search of public sequence databases, the NOV32a protein was found to have homology to the proteins shown in the BLASTP data in Table 32D.

| **Table 32D. Public BLASTP Results for NOV32a** | | | | |
|---|---|---|---|---|
| **Protein Accession Number** | **Protein/Organism/Length** | **NOV32a Residues/ Match Residues** | **Identities/ Similarities for the Matched Portion** | **Expect Value** |
| AAG00573 | SIALIC ACID BINDING IMMUNOGLOBULIN-LIKE LECTIN 8 LONG SPLICE VARIANT - Homo sapiens (Human), 499 aa. | 1..477 | 351/485 (72%) | 0.0 |
| | | 4..485 | 396/485 (81%) | |
| Q9Y286 | QA79 MEMBRANE PROTEIN, ALLELIC VARIANT AIRM-1B PRECURSOR - Homo sapiens (Human), 467 aa. | 1..469 | 316/469 (67%) | e-170 |
| | | 3..467 | 361/469 (76%) | |
| Q9NYZ4 | SIGLEC SAF2 - Homo sapiens (Human), 431 aa. | 1..408 | 299/414 (72%) | e-168 |
| | | 4..415 | 338/414 (81%) | |
| AAK51233 | SIALIC ACID-BINDING IMMUNOGLOBULIN-LIKE LECTIN-LIKE SHORT SPLICE VARIANT - Homo sapiens (Human), 477 aa. | 3..469 | 309/480 (64%) | e-167 |
| | | 1..477 | 355/480 (73%) | |
| Q9BYI9 | FOAP-9 - Homo sapiens (Human), 463 aa. | 1..469 | 312/475 (65%) | e-167 |
| | | 2..463 | 362/475 (75%) | |

PFam analysis predicts that the NOV32a protein contains the domains shown in the Table 32E.

| **Table 32E. Domain Analysis of NOV32a** | | | |
|---|---|---|---|
| **Pfam Domain** | **NOV32a Match Region** | **Identities/ Similarities for the Matched Region** | **Expect Value** |
| ig | 160..219 | 18/60 (30%) | 0.023 |
| | | 40/60 (67%) | |
| ig | 269..323 | 13/58 (22%) | 1.7e-08 |
| | | 45/58 (78%) | |

### Example 33.

The NOV33 clone was analyzed, and the nucleotide and encoded polypeptide sequences are shown in Table 33A.

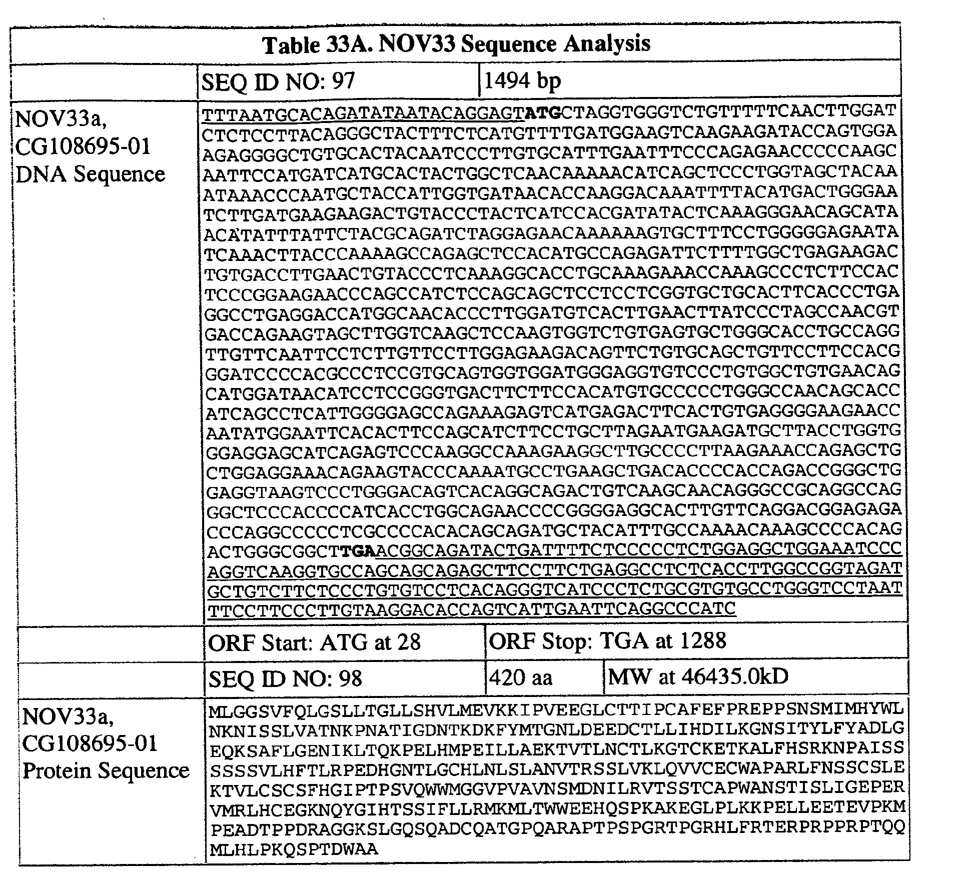

Further analysis of the NOV33a protein yielded the following properties shown in Table 33B.

| **Table 33B. Protein Sequence Properties NOV33a** | |
|---|---|
| PSort analysis: | 0.4500 probability located in cytoplasm; 0.3000 probability located in microbody (peroxisome); 0.1000 probability located in mitochondrial matrix space; 0.1000 probability located in lysosome (lumen) |
| SignalP analysis: | Cleavage site between residues 23 and 24 |

A search of the NOV33a protein against the Geneseq database, a proprietary database that contains sequences published in patents and patent publication, yielded several homologous proteins shown in Table 33C.

| **Table 33C. Geneseq Results for NOV33a** | | | | |
|---|---|---|---|---|
| **Geneseq Identifier** | **Protein/Organism/Length [Patent #, Date]** | **NOV33a Residues/ Match Residues** | **Identities/ Similarities for the Matched Region** | **Expect Value** |
| AAM00948 | Human bone marrow protein, SEQ | 25..232 | 69/226 (30%) | 6e-21 |
| | ID NO: 424 - Homo sapiens, 556 aa. [W0200153453-A2, 26-JUL-2001] | 32..254 | 113/226 (49%) | |
| AAU14239 | Human novel protein #110 - Homo | 25..232 | 69/226 (30%) | 6e-21 |
| | sapiens, 551 aa. [WO200155437-A2, 02-AUG-2001] | 27..249 | 113/226 (49%) | |
| AAW55884 | Human CD33-like protein - Homo | 25..232 | 69/226 (30%) | 6e-21 |
| | sapiens, 551 aa. [WO9806733-A1, 19-FEB-1998] | 27..249 | 113/226 (49%) | |
| AAB50907 | Human PRO333 protein - Homo | 25..224 | 67/216 (31%) | 7e-21 |
| | sapiens, 394 aa. [WO200073452-A2,07-DEC-2000] | 25..237 | 108/216 (49%) | |
| AAB33462 | Human PRO333 protein UNQ294 | 25..224 | 67/216 (31%) | 7e-21 |
| | SEQ ID NO:249 - Homo sapiens, 394 aa. [WO200053758-A2, 14-SEP-2000] | 25..237 | 108/216 (49%) | |

In a BLAST search of public sequence databases, the NOV33a protein was found to have homology to the proteins shown in the BLASTP data in Table 33D.

| **Table 33D. Public BLASTP Results for NOV33a** | | | | |
|---|---|---|---|---|
| **Protein Accession Number** | **Protein/Organism/Length** | **NOV33a Residues/ Match Residues** | **Identities/ Similarities for the Matched Portion** | **Expect Value** |
| Q95JN1 | HYPOTHETICAL 34.6 KDA PROTEIN - Macaca fascicularis (Crab eating macaque) (Cynomolgus monkey), 312 aa. | 85..359 | 244/315 (77%) | e-132 |
| | | 1..311 | 248/315 (78%) | |
| Q9D4M0 | 4931406B18RIK PROTEIN - Mus musculus (Mouse), 367 aa. | 12..339 | 166/369 (44%) | 1e-79 |
| | | 11..359 | 216/369 (57%) | |
| Q9D4Y7 | 4930538L19RIK PROTEIN - Mus musculus (Mouse), 283 aa. | 52..312 | 133/267 (49%) | 1e-65 |
| | | 1..247 | 173/267 (63%) | |
| AAD50978 | SIALIC ACID BINDING IG-LIKE LECTIN-5 - Homo sapiens (Human), 551 aa. | 25..232 | 69/226 (30%) | 1e-20 |
| | | 27..249 | 113/226 (49%) | |
| 015389 | OB BINDING PROTEIN-2 (SIGLEC5) - Homo sapiens (Human), 551 aa. | 25..232 | 69/226 (30%) | 1e-20 |
| | | 27..249 | 113/226 (49%) | |

### Example 34.

The NOV34 clone was analyzed, and the nucleotide and encoded polypeptide sequences are shown in Table 34A.

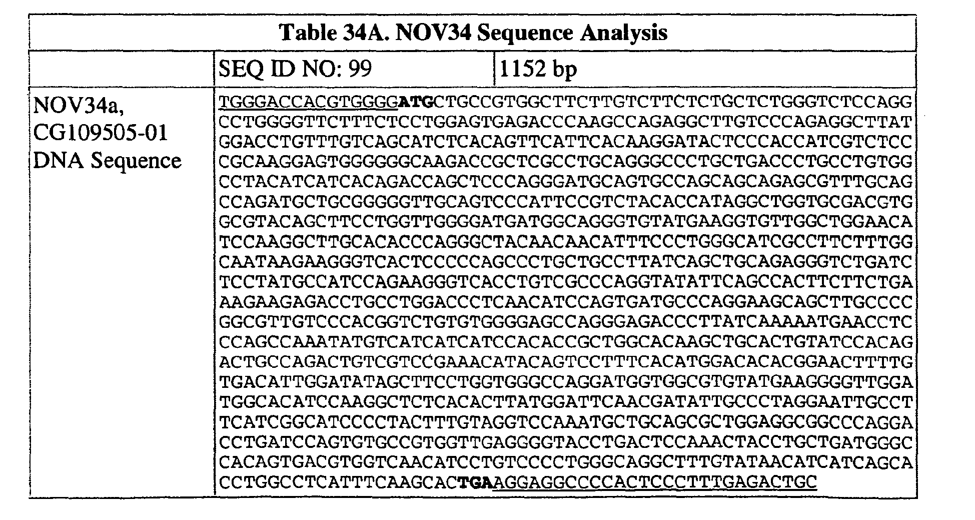

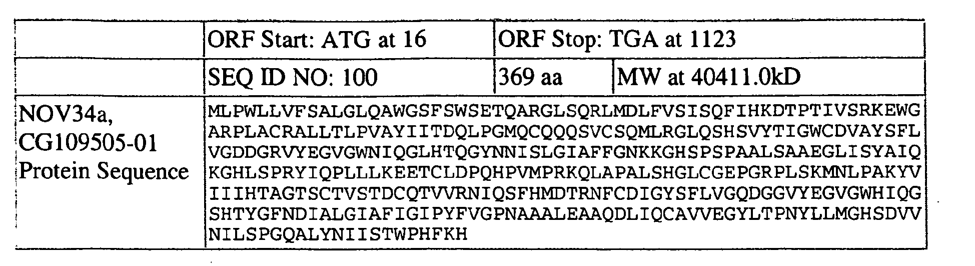

Further analysis of the NOV34a protein yielded the following properties shown in Table 34B.

| **Table 34B. Protein Sequence Properties NOV34a** | |
|---|---|
| PSort analysis: | 0.3894 probability located in outside; 0.1213 probability located in microbody (peroxisome); 0.1000 probability located in endoplasmic reticulum (membrane); 0.1000 probability located in endoplasmic reticulum (lumen) |
| SignalP analysis: | Cleavage site between residues 18 and 19 |

A search of the NOV34a protein against the Geneseq database, a proprietary database that contains sequences published in patents and patent publication, yielded several homologous proteins shown in Table 34C.

| **Table 34C. Geneseq Results for NOV34a** | | | | |
|---|---|---|---|---|
| **Geneseq Identifier** | **Protein/Organism/Length [Patent #, Date]** | **NOV34a Residues/ Match Residues** | **Identities/ Similarities for the Matched Region** | **Expect Value** |
| AAY96963 | Wound healing tissue peptidoglycan recognition protein-like protein - Homo sapiens, 368 aa. [WO200039327-A1, 06-JUL-2000] | 1..369 | 252/369 (68%) | e-150 |
| | | 1..368 | 295/369 (79%) | |
| ABB53272 | Human polypeptide #12 - Homo sapiens, 369 aa. [W0200181363-A1, 01-NOV-2001] | 1..369 | 236/369 (63%) | e-140 |
| | | 1..369 | 282/369 (75%) | |
| AAE00693 | Human full length granulocyte peptide homolog Zgpal protein #2 - Homo sapiens, 369 aa. [WO200129224-A2, 26-APR-2001] | 1..369 | 236/369 (63%) | e-140 |
| | | 1..369 | 282/369 (75%) | |
| AAE00692 | Human full length granulocyte peptide homolog Zgpal protein #1 - Homo sapiens, 375 aa. [WO200129224-A2, 26-APR-2001] | 1..369 | 235/375 (62%) | e-137 |
| | | 1..375 | 282/375 (74%) | |
| AAY76124 | Human secreted protein encoded by gene 1 - Homo sapiens, 244 aa. [WO9958660-A1, 18-NOV-1999] | 2..270 | 213/269 (79%) | e-118 |
| | | 4..242 | 215/269 (79%) | |

In a BLAST search of public sequence databases, the NOV34a protein was found to have homology to the proteins shown in the BLASTP data in Table 34D.

| **Table 34D. Public BLASTP Results for NOV34a** | | | | |
|---|---|---|---|---|
| **Protein Accession Number** | **Protein/Organism/Length** | **NOV34a Residues/ Match Residues** | **Identities/ Similarities for the Matched Portion** | **Expect Value** |
| Q96LB9 | PEPTIDOGLYCAN RECOGNITION PROTEIN-I-ALPHA PRECURSOR - Homo sapiens (Human), 341 aa. | 2..369 | 309/370 (83%) | e-175 |
| | | 4..341 | 311/370 (83%) | |
| Q9HD75 | HYPOTHETICAL 40.0 KDA PROTEIN - Homo sapiens (Human), 368 aa. | 1..369 | 252/369 (68%) | e-149 |
| | | 1..368 | 295/369 (79%) | |
| CAC38715 | SEQUENCE 7 FROM PATENT WO0129224 - Homo sapiens (Human), 369 aa. | 1..369 | 236/369 (63%) | e-140 |
| | | 1..369 | 282/369 (75%) | |
| Q96LB8 | PEPTIDOGLYCAN RECOGNITION PROTEIN-I-BETA PRECURSOR - Homo sapiens (Human), 373 aa. | 1..369 | 237/373 (63%) | e-138 |
| | | 1..373 | 282/373 (75%) | |
| CAC38714 | SEQUENCE 4 FROM PATENT WO0129224 - Homo sapiens (Human), 375 aa. | 1..369 | 235/375 (62%) | e-137 |
| | | 1..375 | 282/375 (74%) | |

### Example 35.

The NOV35 clone was analyzed, and the nucleotide and encoded polypeptide sequences are shown in Table 35A.

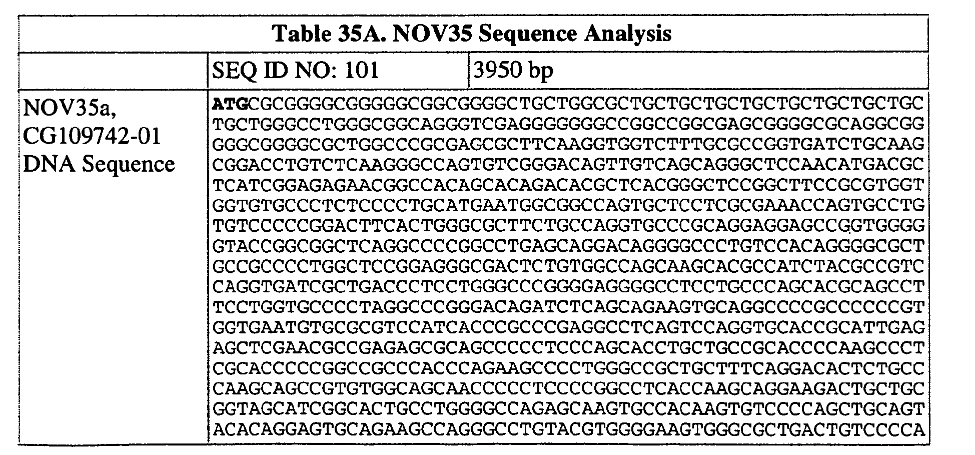

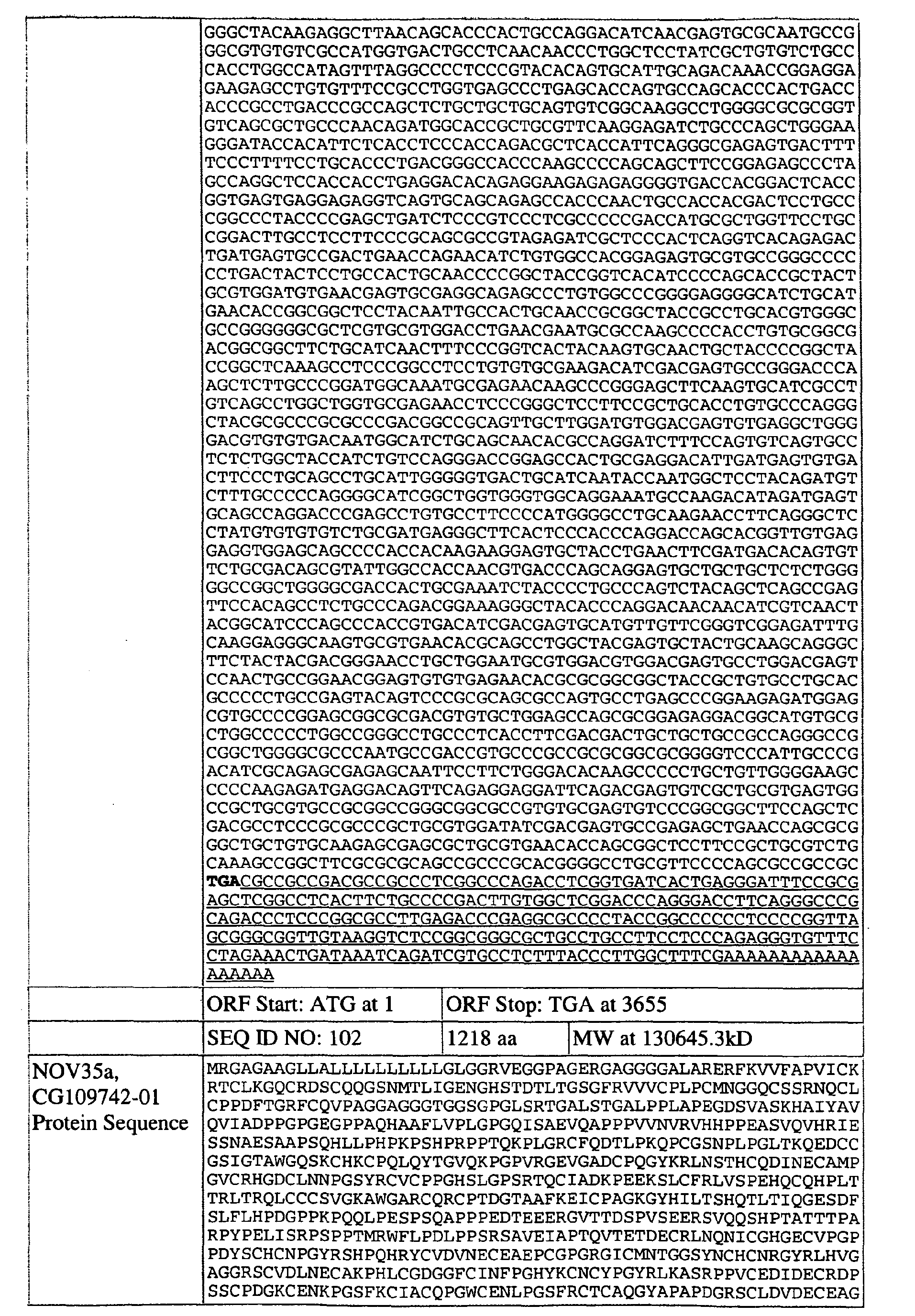

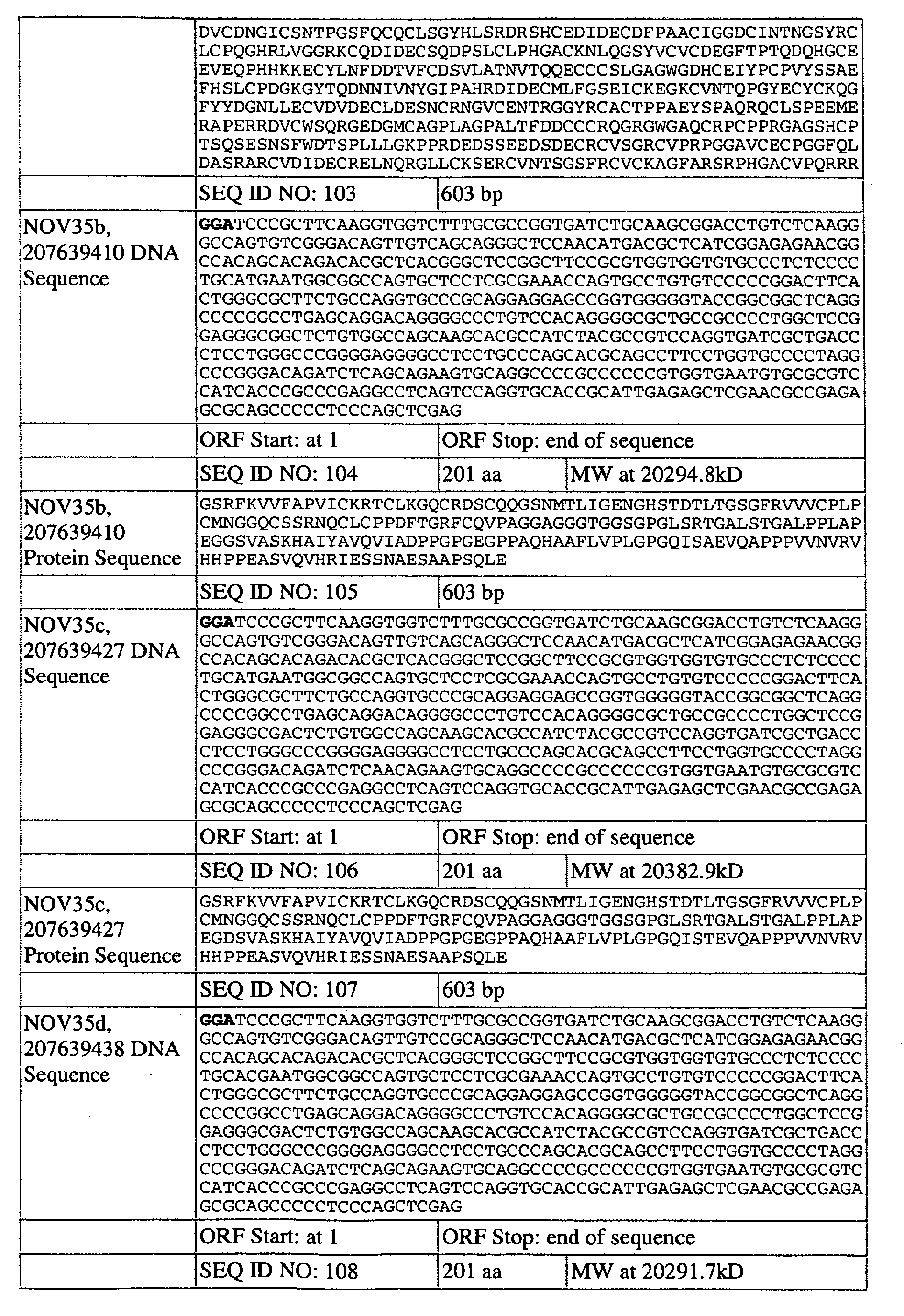

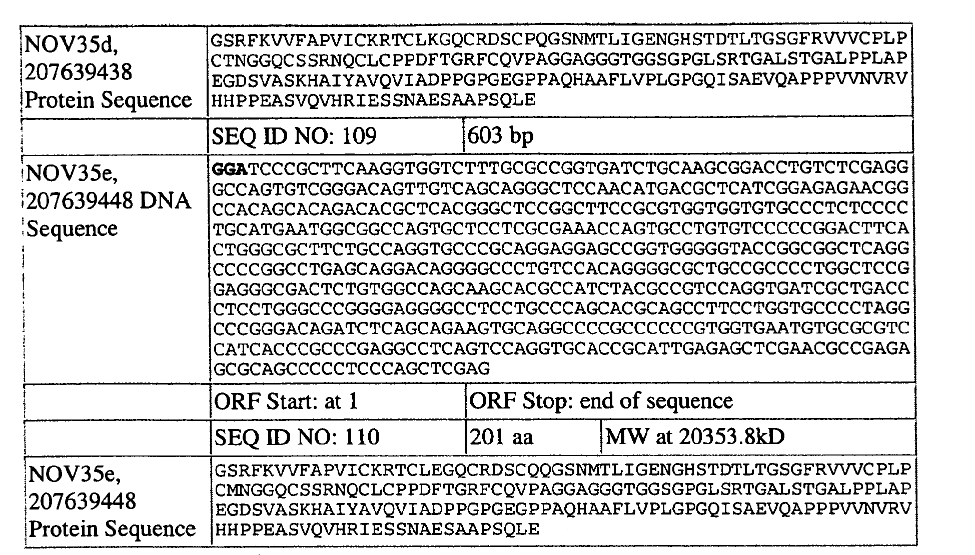

Sequence comparison of the above protein sequences yields the following sequence relationships shown in Table 35B.

| **Table 35B. Comparison of NOV35a against NOV35b through NOV35e.** | | |
|---|---|---|
| **Protein Sequence** | **NOV35a Residues/ Match Residues** | **Identities/ Similarities for the Matched Region** |
| NOV35b | 47..243 | 154/197 (78%) |
| | 3..199 | 154/197 (78%) |
| NOV35c | 47..243 | 154/197 (78%) |
| | 3..199 | 154/197 (78%) |
| NOV35d | 47..243 | 153/197 (77%) |
| | 3..199 | 153/197 (77%) |
| NOV35e | 47..243 | 154/197 (78%) |
| | 3..199 | 155/197 (78%) |

Further analysis of the NOV35a protein yielded the following properties shown in Table 35C.

| **Table 35C. Protein Sequence Properties NOV35a** | |
|---|---|
| PSort analysis: | 0.8200 probability located in outside; 0.1900 probability located in lysosome (lumen); 0.1000 probability located in endoplasmic reticulum (membrane); 0.1000 probability located in endoplasmic reticulum (lumen) |
| SignalP analysis: | Cleavage site between residues 30 and 31 |

A search of the NOV35a protein against the Geneseq database, a proprietary database that contains sequences published in patents and patent publication, yielded several homologous proteins shown in Table 35D.

| **Table 35D. Geneseq Results for NOV35a** | | | | |
|---|---|---|---|---|
| **Geneseq Identifier** | **Protein/Organism/Length [Patent #, Date]** | **NOV35a Residues/ Match Residues** | **Identities/ Similarities for the Matched Region** | **Expect Value** |
| AAB61419 | Human TANGO 275 protein - Homo sapiens, 1289 aa. [WO200100672-A1, 04-JAN-2001] | 1..1218 | 1217/1289 (94%) | 0.0 |
| | | 1..1289 | 1217/1289 (94%) | |
| AAY70551 | Human latent transforming growth factor-beta binding protein 3 (I) - Homo sapiens, 1208 aa. [WO200012551-A1, 09-MAR-2000] | 35..1218 | 1183/1208 (97%) | 0.0 |
| | | 1..1208 | 1183/1208 (97%) | |
| AAY70554 | Human latent transforming growth factor-beta binding protein 3 (III) - Homo sapiens, 1257 aa. [WO200012551-A1, 09-MAR-2000] | 35..1218 | 1183/1257 (94%) | 0.0 |
| | | 1..1257 | 1183/1257 (94%) | |
| AAB61483 | Human TANGO 300 extracellular domain - Homo sapiens, 1251 aa. [WO200100672-A1, 04-JAN-2001] | 10..1213 | 1056/1230 (85%) | 0.0 |
| | | 6..1229 | 1078/1230 (86%) | |
| AAR79475 | Mouse LTBP-3 - Mus sp, 1251 aa. [W09522611-A2, 24-AUG-1995] | 10..1213 | 1056/1230 (85%) | 0.0 |
| | | 6..1229 | 1078/1230 (86%) | |

In a BLAST search of public sequence databases, the NOV35a protein was found to have homology to the proteins shown in the BLASTP data in Table 35E.

| **Table 35E. Public BLASTP Results for NOV35a** | | | | |
|---|---|---|---|---|
| **Protein Accession Number** | **Protein/Organism/Length** | **NOV35a Residues/ Match Residues** | **Identities/ Similarities for the Matched Portion** | **Expect Value** |
| Q9NS15 | LATENT TRANSFORMING GROWTH FACTOR BETA BINDING PROTEIN 3 - Homo sapiens (Human), 1256 aa. | 1..1218 | 1218/1242 (98%) | 0.0 |
| | | 15..1256 | 1218/1242 (98%) | |
| Q9H7K2 | FLJ00070 PROTEIN - Homo sapiens (Human), 1382 aa (fragment). | 1..1218 | 1217/1289 (94%) | 0.0 |
| | | 95..1382 | 1217/1289 (94%) | |
| A57293 | latent transforming growth factor beta-binding protein 3 precursor - mouse, 1251 aa. | 10..1213 | 1056/1230 (85%) | 0.0 |
| | | 6..1229 | 1078/1230 (86%) | |
| Q61810 | LATENT TRANSFORMING GROWTH FACTOR-BETA BINDING PROTEIN - Mus musculus (Mouse), 1253 aa. | 1..1213 | 1054/1240 (85%) | 0.0 |
| | | 1..1231 | 1074/1240 (86%) | |
| Q96HB9 | SIMILAR TO LATENT TRANSFORMING GROWTH FACTOR BETA BINDING PROTEIN 3 - Homo sapiens (Human), 746 aa (fragment). | 500..1218 | 719/743 (96%) | 0.0 |
| | | 4..746 | 719/743 (96%) | |

PFam analysis predicts that the NOV35a protein contains the domains shown in the Table 35F.

| **Table 35F. Domain Analysis of NOV35a** | | | |
|---|---|---|---|
| **Pfam Domain** | **NOV35a Match Region** | **Identities/ Similarities for the Matched Region** | **Expect Value** |
| EGF | 99..126 | 14/47 (30%) | 0.00034 |
| | | 23/47 (49%) | |
| TB | 273..316 | 20/48 (42%) | 0.013 |
| | | 32/48 (67%) | |
| EGF | 345..380 | 16/47 (34%) | 0.00073 |
| | | 28/47 (60%) | |
| TB | 399..440 | 23/47 (49%) | 3.5e-17 |
| | | 35/47 (74%) | |
| EGF | 564..600 | 14/47 (30%) | 0.0033 |
| | | 28/47 (60%) | |
| EGF | 606..644 | 17/48 (35%) | 0.66 |
| | | 28/48 (58%) | |
| EGF | 716..745 | 12/47 (26%) | 0.0073 |
| | | 26/47 (55%) | |
| EGF | 751..786 | 16/47 (34%) | 3e-07 |
| | | 28/47 (60%) | |
| EGF | 792..826 | 14/47 (30%) | 0.069 |
| | | 26/47 (55%) | |
| EGF | 832..869 | 11/47 (23%) | 8.6e-05 |
| | | 26/47 (55%) | |
| TB | 889..932 | 21/47 (45%) | 6.7e-14 |
| | | 33/47 (70%) | |
| EGF | 959..996 | 14/47 (30%) | 0.0034 |
| | | 28/47 (60%) | |
| EGF | 1002..1037 | 15/47 (32%) | 2.2e-05 |
| | | 29/47 (62%) | |
| TB | 1061..1106 | 18/48 (38%) | 0.0014 |
| | | 33/48 (69%) | |
| EGF | 1173..1208 | 12/49 (24%) | 0.32 |
| | | 26/49 (53%) | |

### Example 36.

The NOV36 clone was analyzed, and the nucleotide and encoded polypeptide sequences are shown in Table 36A.

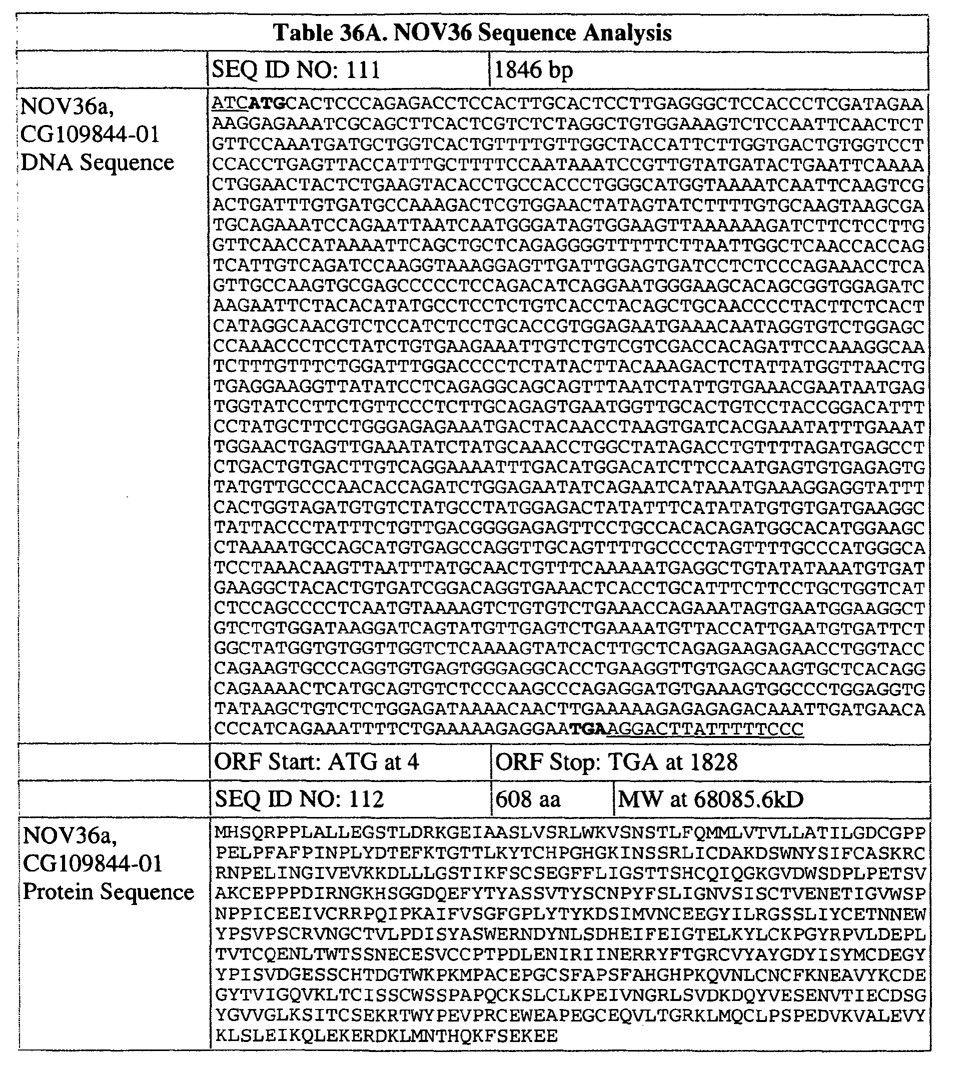

Further analysis of the NOV36a protein yielded the following properties shown in Table 36B.

| **Table 36B. Protein Sequence Properties NOV36a** | |
|---|---|
| PSort analysis: | 0.7900 probability located in plasma membrane; 0.3000 probability located in microbody (peroxisome); 0.3000 probability located in Golgi body; 0.2000 probability located in endoplasmic reticulum (membrane) |
| SignalP analysis: | Cleavage site between residues 54 and 55 |

A search of the NOV36a protein against the Geneseq database, a proprietary database that contains sequences published in patents and patent publication, yielded several homologous proteins shown in Table 36C.

| **Table 36C. Geneseq Results for NOV36a** | | | | |
|---|---|---|---|---|
| **Geneseq Identifier** | **Protein/Organism/Length [Patent #, Date]** | **NOV36a Residues/ Match Residues** | **Identities/ Similarities for the Matched Region** | **Expect Value** |
| AAR13490 | Human C4 binding protein - Homo sapiens, 581 aa. [WO9111461-A, 08-AUG-1991] | 22..594 | 341/577 (59%) | 0.0 |
| | | 1..570 | 428/577 (74%) | |
| AAW39924 | Amino acid sequence of a mouse sperm protein designated sp56 - Mus sp, 579 aa. [WO9800440-A1, 08-JAN-1998] | 30..602 | 315/575 (54%) | 0.0 |
| | | 9..565 | 403/575 (69%) | |
| AAB43640 | Human cancer associated protein sequence SEQ ID NO: 1085 - Homo sapiens, 220 aa. [WO200055350-A1, 21-SEP-2000] | 390..594 | 136/205 (66%) | 3e-80 |
| | | 10..209 | 162/205 (78%) | |
| AAM50797 | Human C3B/C4B receptor CR1 (complement receptor type 1) - Homo sapiens, 2039 aa. [US6316604-B1, 13-NOV-2001] | 48..539 | 158/532 (29%) | 2e-59 |
| | | 1389..1897 | 238/532 (44%) | |
| ABG00287 | Novel human diagnostic protein #278 - Homo sapiens, 2039 aa. [WO200175067-A2, 11-OCT-2001] | 48..539 | 158/532 (29%) | 2e-59 |
| | | 1389..1897 | 238/532 (44%) | |

In a BLAST search of public sequence databases, the NOV36a protein was found to have homology to the proteins shown in the BLASTP data in Table 36D.

| **Table 36D. Public BLASTP Results for NOV36a** | | | | |
|---|---|---|---|---|
| **Protein Accession Number** | **Protein/Organism/Length** | **NOV36a Residues/ Match the Residues** | **Identities/ Similarities for Matched Portion** | **Expect Value** |
| P04003 | C4b-binding protein alpha chain precursor (C4bp) (Proline-rich protein) (PRP) - Homo sapiens (Human), 597 aa. | 1..594 | 347/598 (58%) | 0.0 |
| | | 1..586 | 436/598 (72%) | |
| S53711 | C4BP alpha chain precursor - rabbit, 597 aa. | 1..595 | 330/599 (55%) | 0.0 |
| | | 1..587 | 414/599 (69%) | |
| Q60736 | SPERM FERTILIZATION PROTEIN SP56 PRECURSOR - Mus musculus (Mouse), 579 aa. | 30..602 | 315/575 (54%) | 0.0 |
| | | 9..565 | 403/575 (69%) | |
| Q28065 | C4b-binding protein alpha chain precursor (C4bp) - Bos taurus (Bovine), 610 aa. | 1..595 | 327/601 (54%) | 0.0 |
| | | 1..590 | 416/601 (68%) | |
| Q63514 | C4b-binding protein alpha chain precursor (C4bp) - Rattus norvegicus (Rat), 558 aa. | 41..595 | 278/558 (49%) | e-174 |
| | | 1..550 | 385/558 (68%) | |

PFam analysis predicts that the NOV36a protein contains the domains shown in the Table 36E.

| **Table 36E. Domain Analysis of NOV36a** | | | |
|---|---|---|---|
| **Pfam Domain** | **NOV36a Match Region** | **Identities/ Similarities for the Matched Region** | **Expect Value** |
| sushi | 55..111 | 14/66 (21%) | 7.2e-10 |
| | | 41/66 (62%) | |
| sushi | 116..172 | 21/63 (33%) | 2.2e-08 |
| | | 41/63 (65%) | |
| sushi | 177..237 | 27/67 (40%) | 9.2e-17 |
| | | 49/67 (73%) | |
| sushi | 242..297 | 21/64 (33%) | 6.6e-12 |
| | | 40/64 (62%) | |
| sushi | 302..364 | 17/72 (24%) | 9.9e-05 |
| | | 46/72 (64%) | |
| sushi | 369..430 | 23/70 (33%) | 4.1e-11 |
| | | 46/70 (66%) | |
| sushi | 434..488 | 16/64 (25%) | 2.3e-06 |
| | | 35/64 (55%) | |
| sushi | 492..546 | 24/63 (38%) | 3.7e-11 |
| | | 37/63 (59%) | |

### Example 37.

The NOV37 clone was analyzed, and the nucleotide and encoded polypeptide sequences are shown in Table 37A.

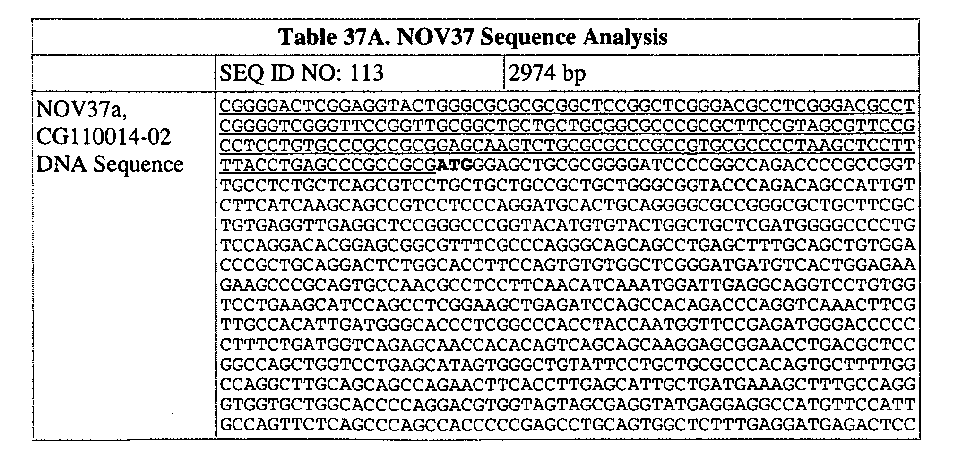

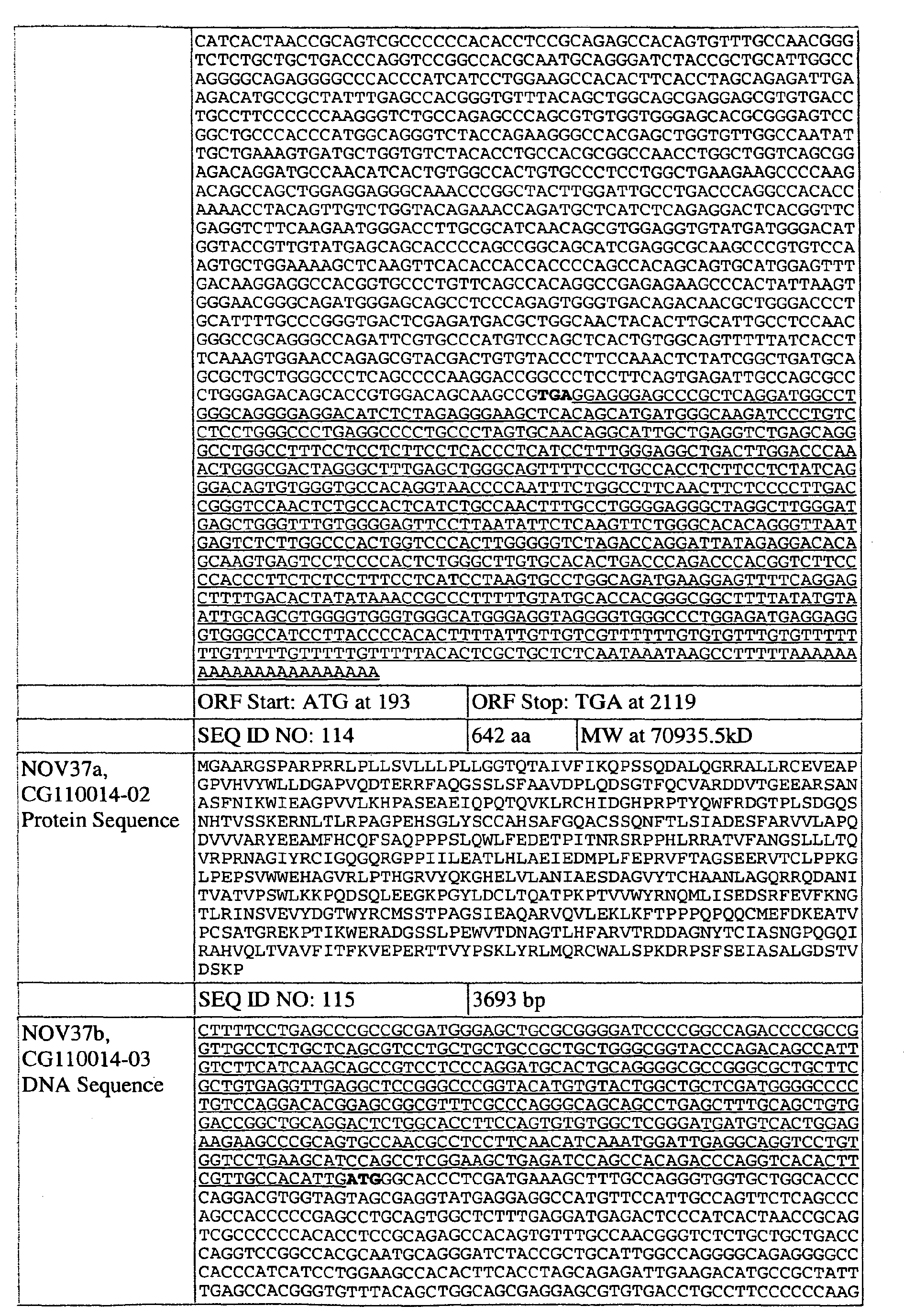

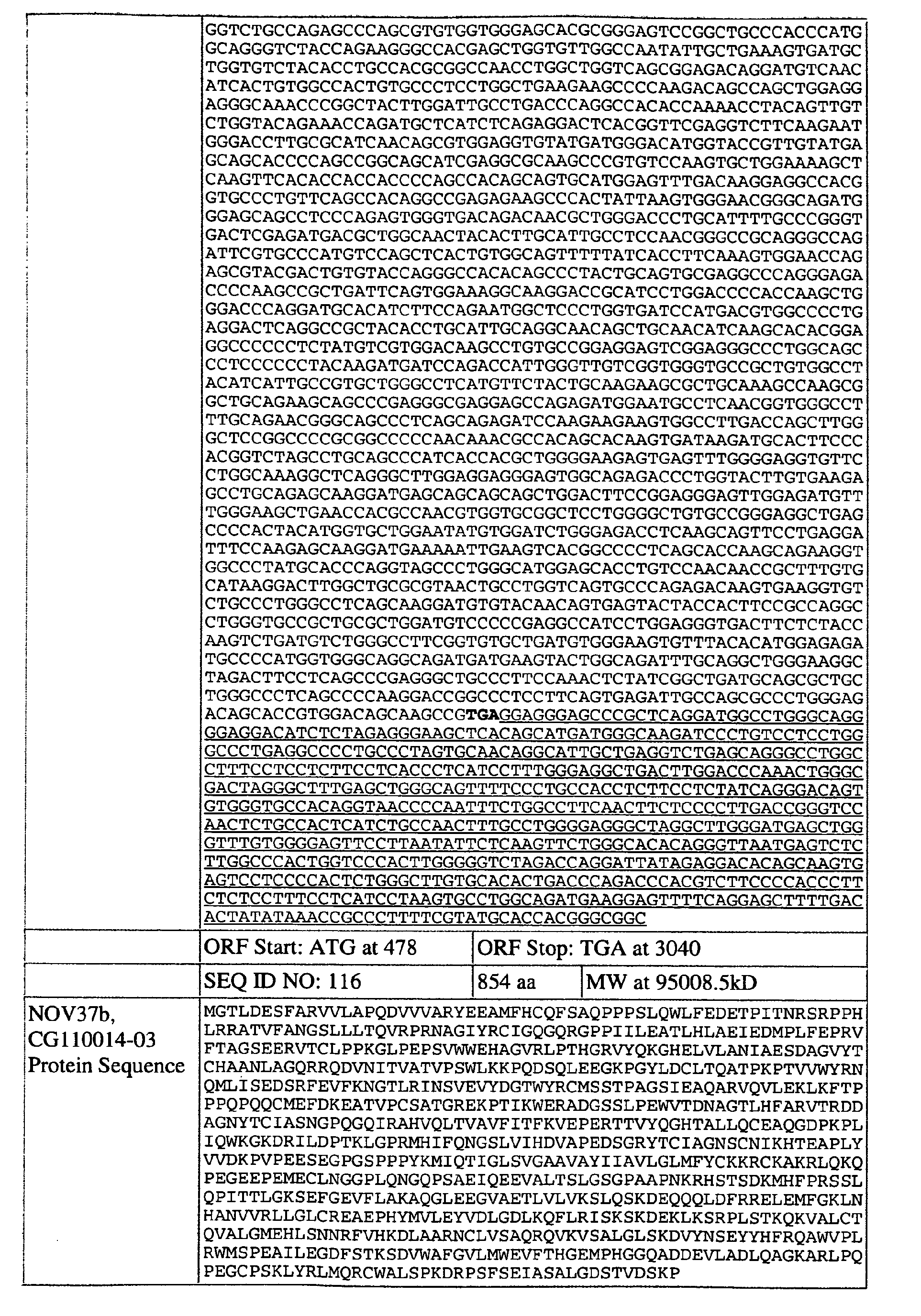

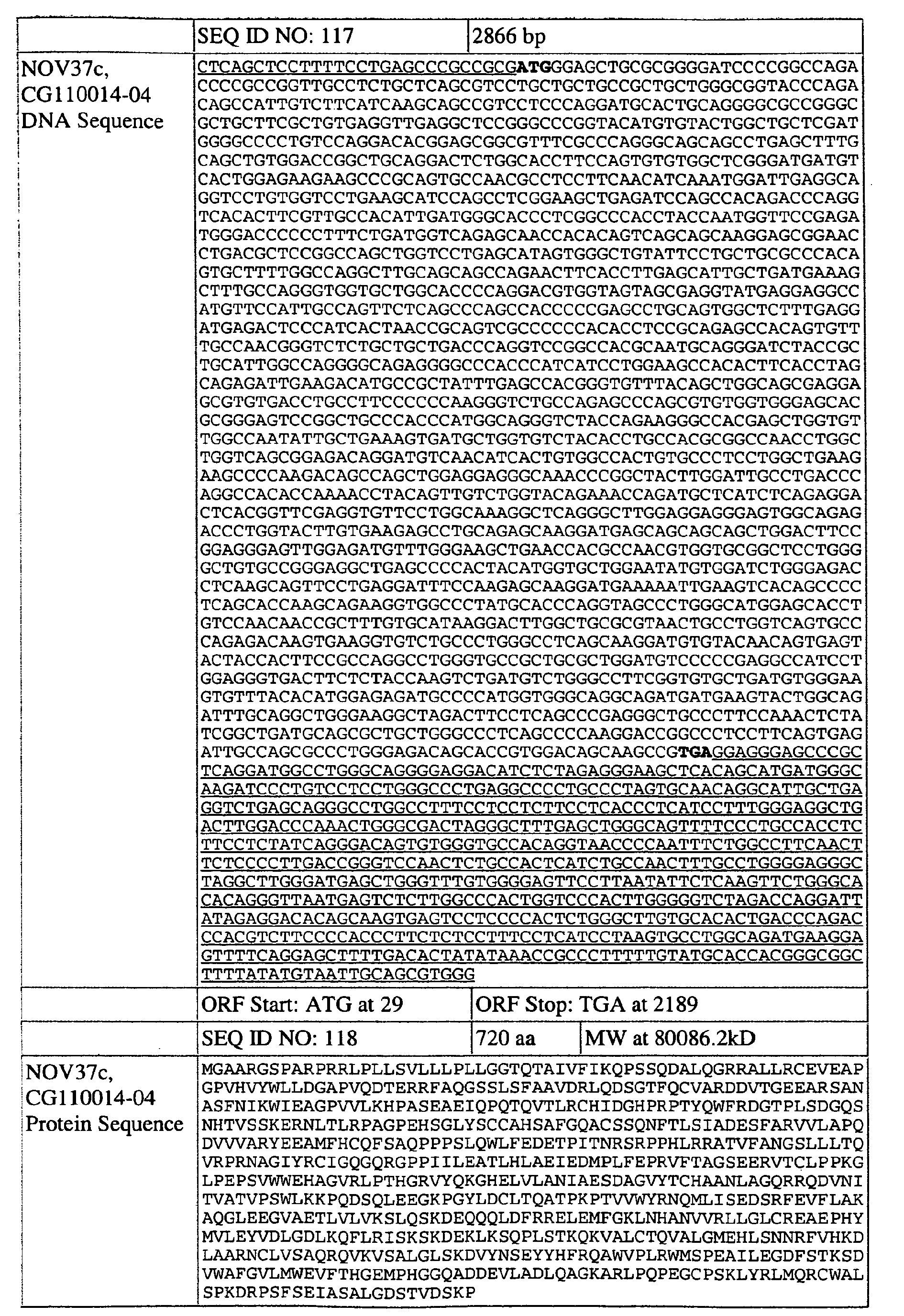

Sequence comparison of the above protein sequences yields the following sequence relationships shown in Table 37B.

| **Table 37B. Comparison of NOV37a against NOV37b and NOV37c.** | | |
|---|---|---|
| **Protein Sequence** | **NOV37a Residues/ Match Residues** | **Identities/ Similarities for the Matched Region** |
| NOV37b | 221..604 | 383/384 (99%) |
| | 5..388 | 383/384 (99%) |
| NOV37c | 28..461 | 431/434 (99%) |
| | 28..461 | 431/434 (99%) |

Further analysis of the NOV37a protein yielded the following properties shown in Table 37C.

| **Table 37C. Protein Sequence Properties NOV37a** | |
|---|---|
| PSort analysis: | 0.6950 probability located in outside; 0.1900 probability located in lysosome (lumen); 0.1900 probability located in plasma membrane; 0.1363 probability located in microbody (peroxisome) |
| SignalP analysis: | Cleavage site between residues 31 and 32 |

A search of the NOV37a protein against the Geneseq database, a proprietary database that contains sequences published in patents and patent publication, yielded several homologous proteins shown in Table 37D.

| **Table 37D. Geneseq Results for NOV37a** | | | | |
|---|---|---|---|---|
| **Geneseq Identifier** | **Protein/Organism/Length [Patent #, Date]** | **NOV37a Residues/ Match Residues** | **Identities/ Similarities for the Matched Region** | **Expect Value** |
| AAW08747 | Human colon carcinoma kinase 4 (CCK-4) - Homo sapiens, 1070 aa. [WO9637610-A2, 28-NOV-1996] | 1..604 | 599/604 (99%) | 0.0 |
| | | 1..604 | 600/604 (99%) | |
| ABB68257 | Drosophila melanogaster polypeptide SEQ ID NO 31563 - Drosophila melanogaster, 1395 aa. [WO200171042-A2, 27-SEP-2001] | 128..588 | 136/486 (27%) | 5e-40 |
| | | 56..531 | 217/486 (43%) | |
| AAY08404 | Human ROBO1 protein - Homo sapiens, 1649 aa. [WO9920764-A1, 29-APR-1999] | 128..602 | 144/508 (28%) | 1e-39 |
| | | 68..555 | 216/508 (42%) | |
| AAY13566 | Human Robo 1 polypeptde - Homo sapiens, 1651 aa. [WO9925833-A1, 27-MAY-1999] | 128..602 | 144/508 (28%) | 1e-39 |
| | | 68..555 | 216/508 (42%) | |
| AAY08401 | Drosophila sp. ROBO1 protein - Drosophila sp, 1395 aa. [WO9920764-A1, 29-APR-1999] | 128..588 | 135/486 (27%) | 2e-39 |
| | | 56..531 | 217/486 (43%) | |

In a BLAST search of public sequence databases, the NOV37a protein was found to have homology to the proteins shown in the BLASTP data in Table 37E.

| **Table 37E. Public BLASTP Results for NOV37a** | | | | |
|---|---|---|---|---|
| **Protein Accession Number** | **Protein/Organism/Length** | **NOV37a Residues/ Match Residues** | **Identities/ Similarities for the Matched Portion** | **Expect Value** |
| JC4593 | protein-tyrosine kinase-related receptor PTK7 precursor - human, 1070 aa. | 1..604 | 601/604 (99%) | 0.0 |
| | | 1..604 | 601/604 (99%) | |
| AAC50484 | TRANSMEMBRANE RECEPTOR PRECURSOR - Homo sapiens (Human), 1070 aa. | 1..604 | 601/604 (99%) | 0.0 |
| | | 1..604 | 601/604 (99%) | |
| Q13308 | Tyrosine-protein kinase-like 7 precursor (Colon carcinoma kinase-4) (CCK-4) - Homo sapiens (Human), 1070 aa. | 1..604 | 599/604 (99%) | 0.0 |
| | | 1..604 | 600/604 (99%) | |
| Q91048 | Tyrosine-protein kinase-like 7 precursor (Kinase like protein) - Gallus gallus (Chicken), 1051 aa. | 17..604 | 382/589 (64%) | 0.0 |
| | | 4..585 | 462/589 (77%) | |
| Q9NSQ6 | HYPOTHETICAL 40.9 KDA PROTEIN - Homo sapiens (Human), 364 aa (fragment). | 357..461 | 104/105 (99%) | 5e-56 |
| | | 1..105 | 104/105 (99%) | |

PFam analysis predicts that the NOV37a protein contains the domains shown in the Table 37F.

| **Table 37F. Domain Analysis of NOV37a** | | | |
|---|---|---|---|
| **Pfam Domain** | **NOV37a Match Region** | **Identities/ Similarities for the Matched Region** | **Expect Value** |
| ig | 46..103 | 17/60 (28%) | 9.7e-07 |
| | | 42/60 (70%) | |
| ig | 143..202 | 17/63 (27%) | 1.4e-09 |
| | | 48/63 (76%) | |
| ig | 239..303 | 14/68 (21%) | 0.00022 |
| | | 47/68 (69%) | |
| ig | 336..393 | 15/60 (25%) | 3e-05 |
| | | 38/60 (63%) | |
| ig | 426..483 | 16/61 (26%) | 0.019 |
| | | 37/61 (61%) | |
| ig | 517..572 | 15/59 (25%) | 3.1e-09 |
| | | 41/59 (69%) | |
| pkinase | 605..629 | 8/28 (29%) | 0.024 |
| | | 20/28 (71%) | |

### Example 38.

The NOV38 clone was analyzed, and the nucleotide and encoded polypeptide sequences are shown in Table 38A.

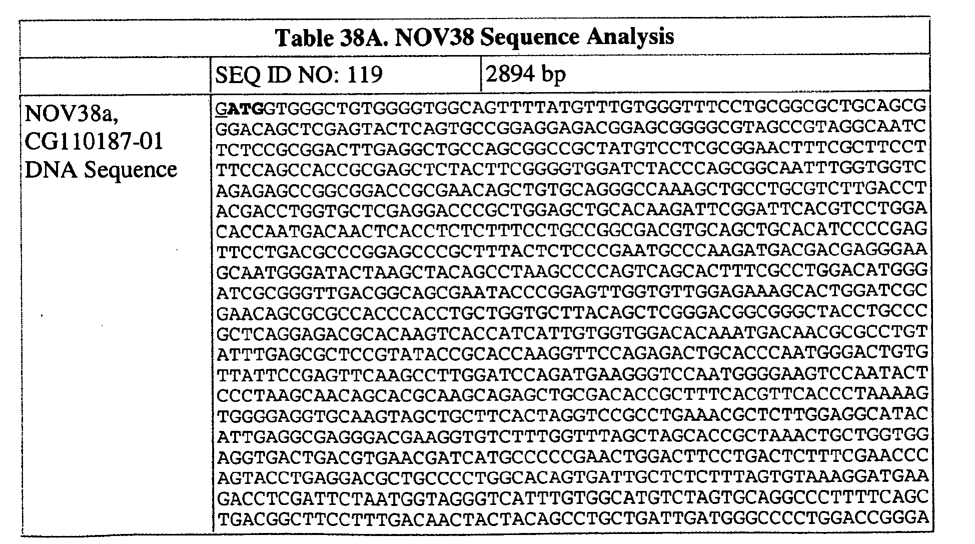

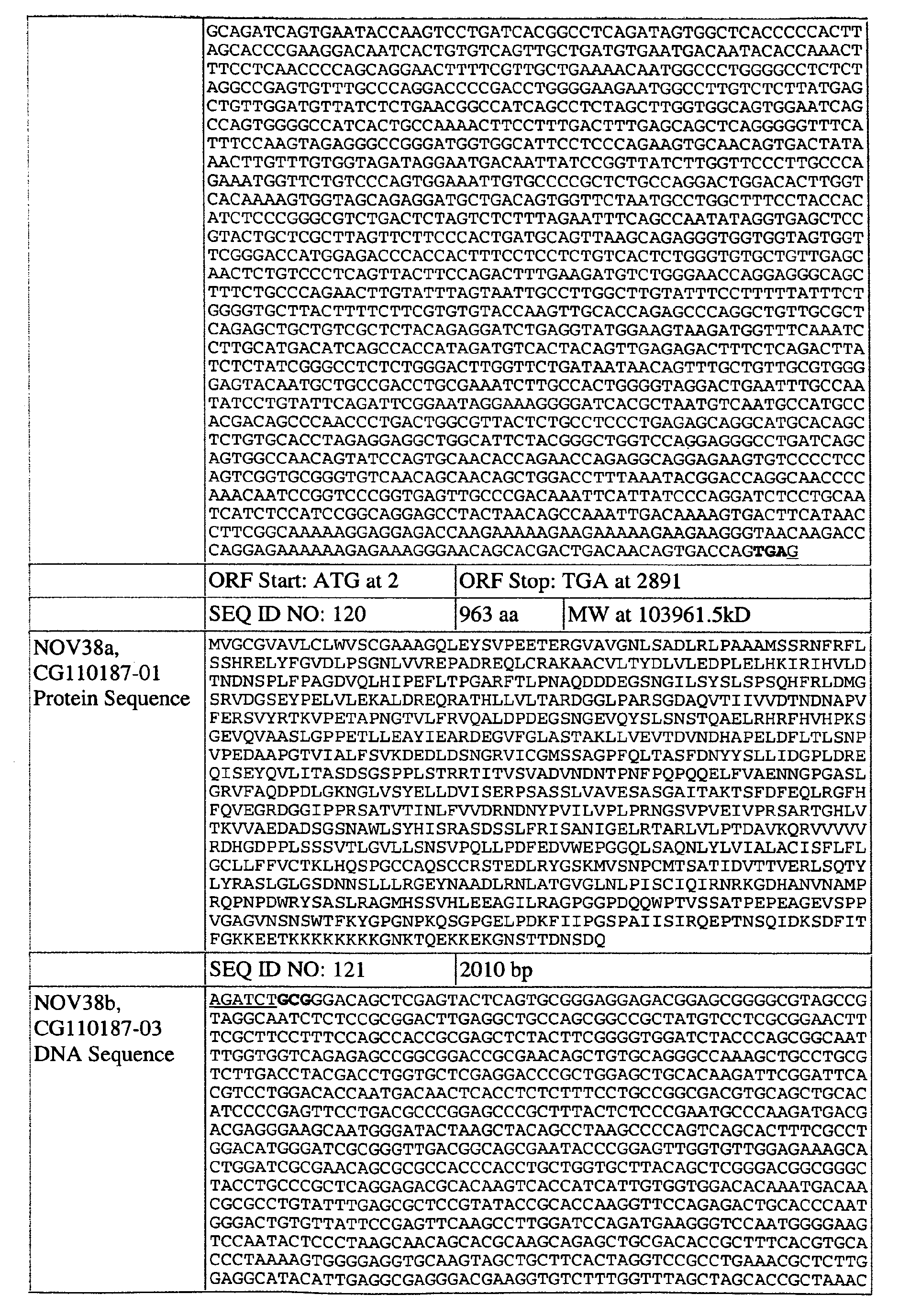

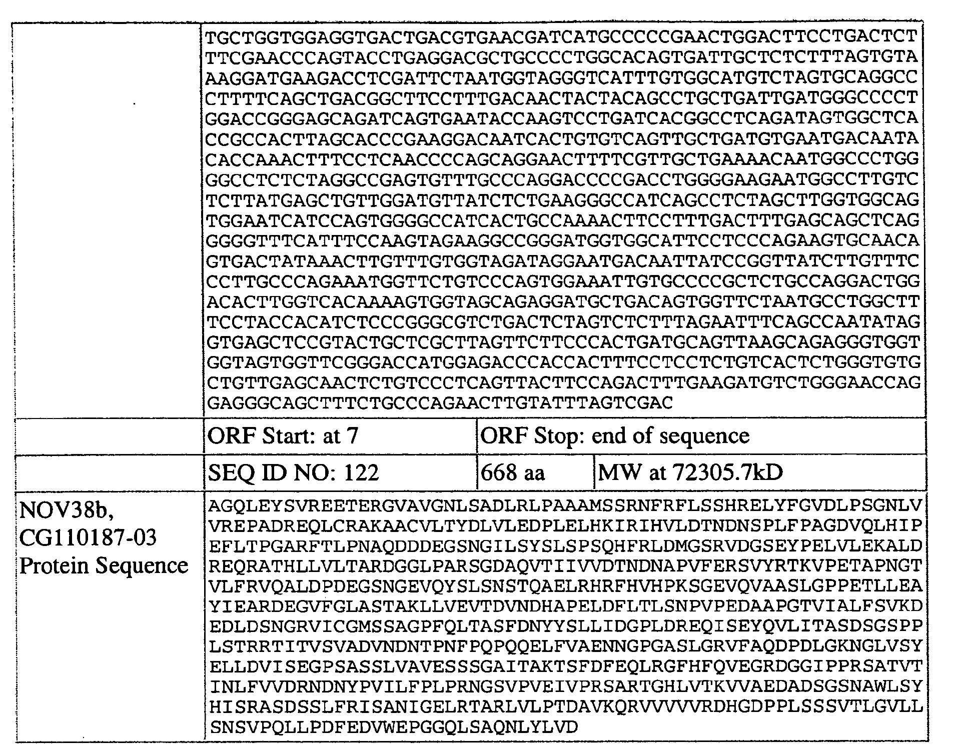

Sequence comparison of the above protein sequences yields the following sequence relationships shown in Table 38B.

| **Table 38B. Comparison of NOV38a against NOV38b.** | | |
|---|---|---|
| **Protein Sequence** | **NOV38a Residues/ Match Residues** | **Identities/ Similarities for the Matched Region** |
| NOV38b | 19..685 | 630/667 (94%) |
| | 1..667 | 630/667 (94%) |

Further analysis of the NOV38a protein yielded the following properties shown in Table 38C.

| **Table 38C. Protein Sequence Properties NOV38a** | |
|---|---|
| PSort analysis: | 0.4600 probability located in plasma membrane; 0.2400 probability located in nucleus; 0.1000 probability located in endoplasmic reticulum (membrane); 0.1000 probability located in endoplasmic reticulum (lumen) |
| SignalP analysis: | Cleavage site between residues 19 and 20 |

A search of the NOV38a protein against the Geneseq database, a proprietary database that contains sequences published in patents and patent publication, yielded several homologous proteins shown in Table 38D.

| **Table 38D. Geneseq Results for NOV38a** | | | | |
|---|---|---|---|---|
| **Geneseq Identifier** | **Protein/Organism/Length [Patent #, Date]** | **NOV38a Residues/ Match Residues** | **Identities/ Similarities for the Matched Region** | **Expect Value** |
| ABG15234 | Novel human diagnostic protein #15225 - Homo sapiens, 1008 aa. [WO200175067-A2, 11-OCT-2001] | 8..963 | 480/984 (48%) | 0.0 |
| | | 31..1008 | 637/984 (63%) | |
| ABG15234 | Novel human diagnostic protein #15225 - Homo sapiens, 1008 aa. [WO200175067-A2, 11-OCT-2001] | 8..963 | 480/984 (48%) | 0.0 |
| | | 31..1008 | 637/984 (63%) | |
| AAM78649 | Human protein SEQ ID NO 1311 - Homo sapiens, 931 aa. [W0200157190-A2,09-AUG-2001] | 3..941 | 368/957 (38%) | e-170 |
| | | 9..931 | 526/957 (54%) | |
| AAM79633 | Human protein SEQ ID NO 3279 - Homo sapiens, 949 aa. [W0200157190-A2,09-AUG-2001] | 3..941 | 370/962 (38%) | e-169 |
| | | 21..949 | 528/962 (54%) | |
| ABB12315 | Human protocadherin homologue, SEQ ID NO:2685 - Homo sapiens, 949 aa. [W0200157188-A2,09-AUG-2001] | 3..941 | 370/962 (38%) | e-169 |
| | | 21..949 | 528/962 (54%) | |

In a BLAST search of public sequence databases, the NOV38a protein was found to have homology to the proteins shown in the BLASTP data in Table 38E.

| **Table 38E. Public BLASTP Results for NOV38a** | | | | |
|---|---|---|---|---|
| **Protein Accession Number** | **Protein/Organism/Length** | **NOV38a Residues/ Match Residues** | **Identities/ Similarities for the Matched Portion** | **Expect Value** |
| Q9H158 | Protocadherin alpha-C1 precursor (PCDH-alpha-C1) - Homo sapiens (Human), 963 aa. | 1..963 | 956/963 (99%) | 0.0 |
| | | 1..963 | 959/963 (99%) | |
| Q91Y10 | PROTOCADHERIN ALPHA C1 - Mus musculus (Mouse), 964 aa. | 1..963 | 813/964 (84%) | 0.0 |
| | | 1..964 | 873/964 (90%) | |
| Q91Y09 | PROTOCADHERIN ALPHA C2 - Mus musculus (Mouse), 1006 aa. | 16..963 | 484/980 (49%) | 0.0 |
| | | 37..1006 | 636/980 (64%) | |
| Q9Y5I4 | Protocadherin alpha C2 precursor (PCDH-alpha-C2) - Homo sapiens (Human), 1007 aa. | 8..963 | 482/984 (48%) | 0.0 |
| | | 30..1007 | 639/984 (63%) | |
| Q9Y5H6 | Protocadherin alpha 8 precursor (PCDH-alpha8) - Homo sapiens (Human), 950 aa. | 8..963 | 455/963 (47%) | 0.0 |
| | | 17..950 | 590/963 (61%) | |

PFam analysis predicts that the NOV38a protein contains the domains shown in the Table 38F.

| **Table 38F. Domain Analysis of NOV38a** | | | |
|---|---|---|---|
| **Pfam Domain** | **NOV38a Match Region** | **Identities/ Similarities for the Matched Region** | **Expect Value** |
| cadherin | 129..224 | 31/110 (28%) | 1.6e-10 |
| | | 65/110 (59%) | |
| cadherin | 238..331 | 31/110 (28%) | 6e-16 |
| | | 66/110 (60%) | |
| cadherin | 345..436 | 36/107 (34%) | 6.2e-10 |
| | | 64/107 (60%) | |
| cadherin | 450..546 | 28/112 (25%) | 5e-12 |
| | | 67/112 (60%) | |
| cadherin | 566..657 | 28/108 (26%) | 0.00086 |
| | | 60/108 (56%) | |

### Example 39.

The NOV39 clone was analyzed, and the nucleotide and encoded polypeptide sequences are shown in Table 39A.

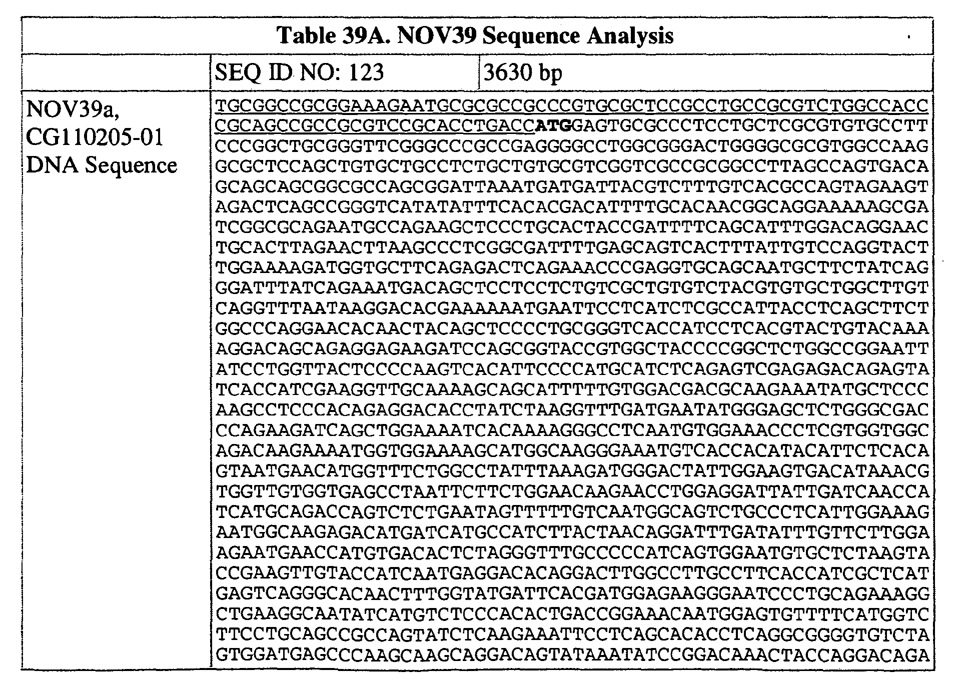

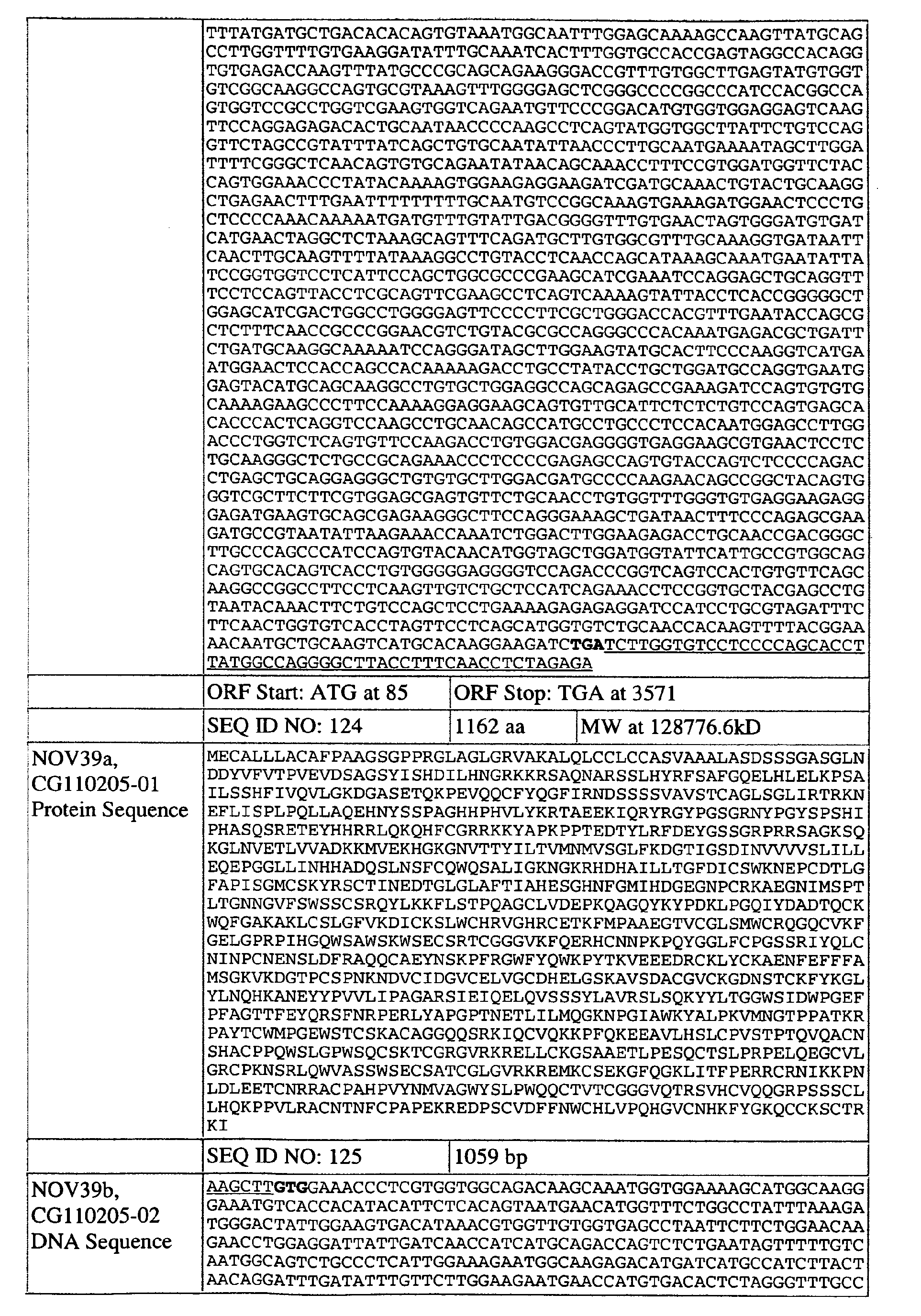

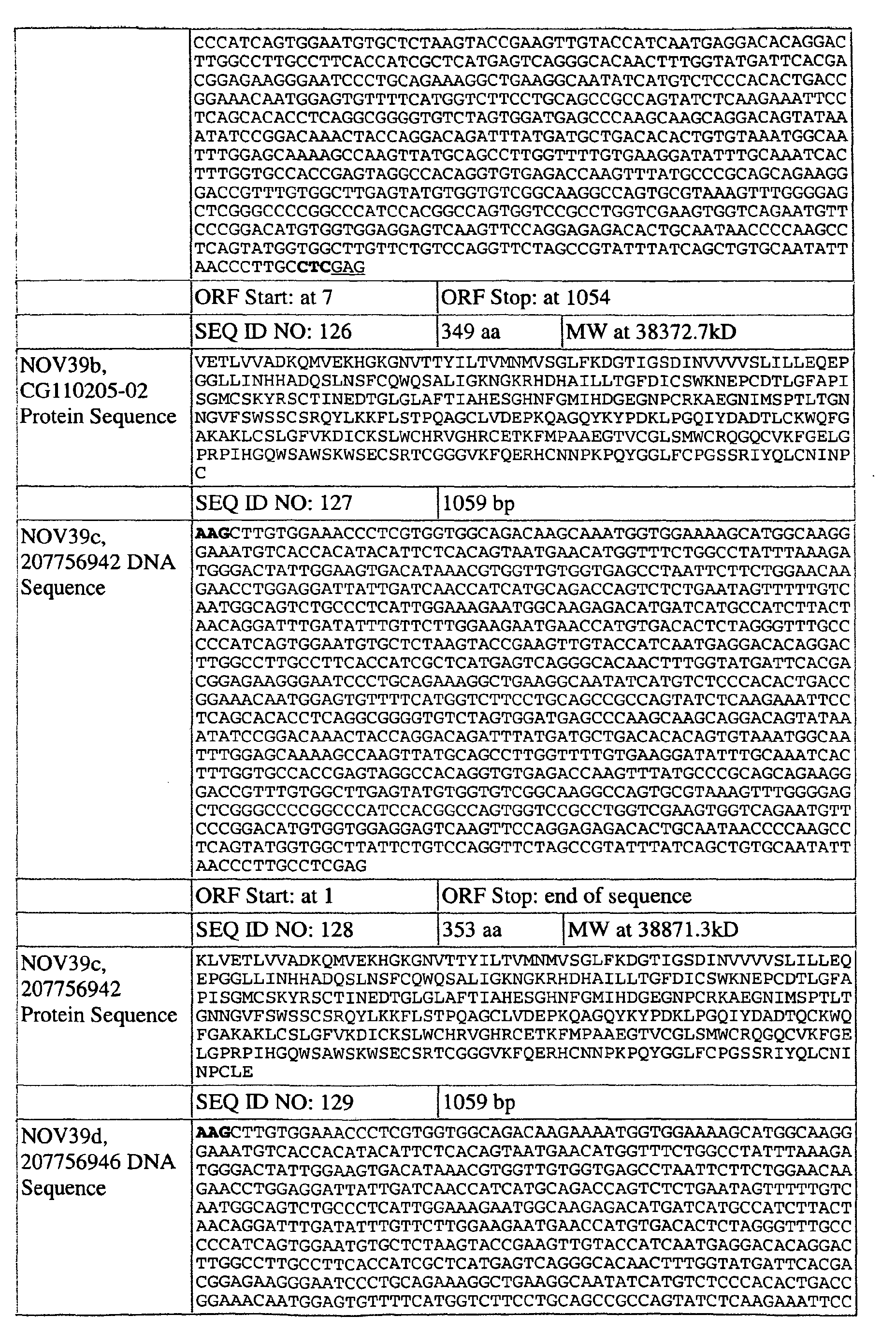

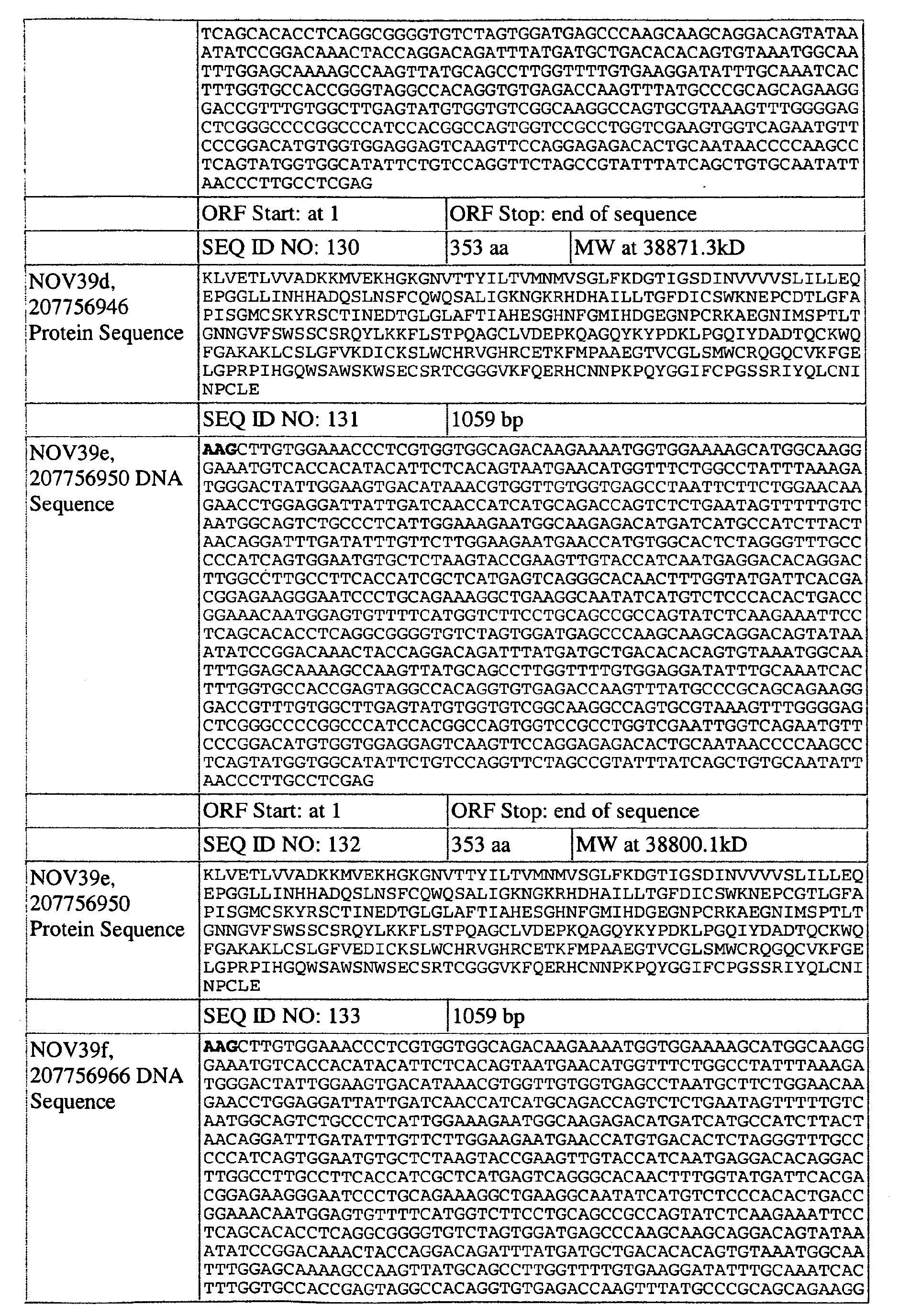

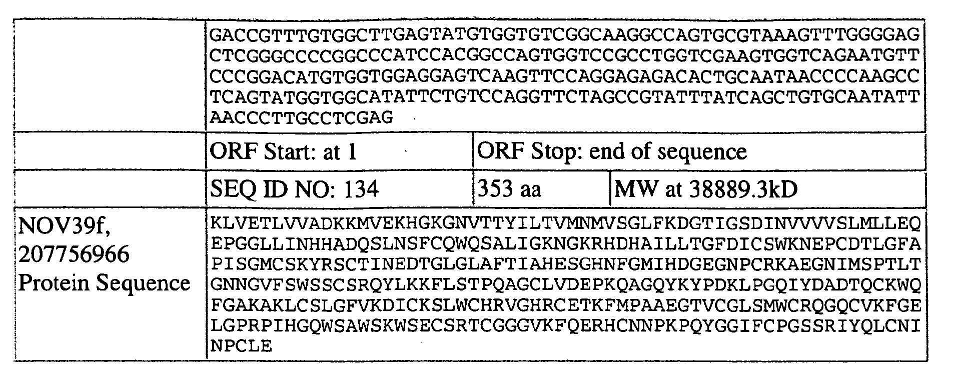

Sequence comparison of the above protein sequences yields the following sequence relationships shown in Table 39B.

| **Table 39B. Comparison of NOV39a against NOV39b through NOV39f.** | | |
|---|---|---|
| **Protein Sequence** | **NOV39a Residues/ Match Residues** | **Identities/ Similarities for the Matched Region** |
| NOV39b | 295..643 | 347/349 (99%) |
| | 1..349 | 348/349 (99%) |
| NOV39c | 295..645 | 349/351 (99%) |
| | 3..353 | 350/351 (99%) |
| NOV39d | 295..645 | 349/351 (99%) |
| | 3..353 | 350/351 (99%) |
| NOV39e | 295..645 | 346/351 (98%) |
| | 3..353 | 348/351 (98%) |
| NOV39f | 295..645 | 348/351 (99%) |
| | 3..353 | 350/351 (99%) |

Further analysis of the NOV39a protein yielded the following properties shown in Table 39C.

| **Table 39C. Protein Sequence Properties NOV39a** | |
|---|---|
| PSort analysis: | 0.6400 probability located in plasma membrane; 0.4600 probability located in Golgi body; 0.3700 probability located in endoplasmic reticulum (membrane); 0.1000 probability located in endoplasmic reticulum (lumen) |
| SignalP analysis: | Cleavage site between residues 48 and 49 |

A search of the NOV39a protein against the Geneseq database, a proprietary database that contains sequences published in patents and patent publication, yielded several homologous proteins shown in Table 39D.

| **Table 39D. Geneseq Results for NOV39a** | | | | |
|---|---|---|---|---|
| **Geneseq Identifier** | **Protein/Organism/Length [Patent #, Date]** | **NOV39a Residues/ Match Residues** | **Identities/ Similarities for the Matched Region** | **Expect Value** |
| AAU72893 | Human metalloprotease partial protein sequence #5 - Homo sapiens, 934 aa. [WO200183782-A2, 08-NOV-2001] | 305..1162 | 856/934 (91 %) | 0.0 |
| | | 1..934 | 858/934 (91 %) | |
| AAU72891 | Human metalloprotease partial protein sequence #3 - Homo sapiens, 1224 aa. [WO200183782-A2, 08-NOV-2001] | 20..1160 | 660/1238 (53%) | 0.0 |
| | | 9..1221 | 828/1238 (66%) | |
| AAU72890 | Human metalloprotease partial protein sequence #2 - Homo sapiens, 1103 aa. [WO200183782-A2, 08-NOV-2001] | 59..1158 | 415/1134 (36%) | 0.0 |
| | | 37..1100 | 585/1134 (50%) | |
| AAB74945 | Human ADAM type metal protease MDTS2 protein SEQ ID NO:10 - Homo sapiens, 1103 aa. [JP2001008687-A, 16-JAN-2001] | 59..1158 | 413/1134 (36%) | 0.0 |
| | | 37..1100 | 585/1134 (51%) | |
| AAB47719 | ADAMTS-E - Homo sapiens, 1104 aa. [EP1149903-A1, 31-OCT-2001] | 59..1158 | 412/1134 (36%) | 0.0 |
| | | 37..1101 | 583/1134 (51%) | |

In a BLAST search of public sequence databases, the NOV39a protein was found to have homology to the proteins shown in the BLASTP data in Table 39E.

| **Table 39E. Public BLASTP Results for NOV39a** | | | | |
|---|---|---|---|---|
| **Protein Accession Number** | **Protein/Organism/Length** | **NOV39a Residues/ Match Residues** | **Identities/ Similarities for the Matched Portion** | **Expect Value** |
| CAC83612 | ADAMTS18 PROTEIN - Homo sapiens (Human), 1081 aa. | 1..1073 | 937/1081 (86%) | 0.0 |
| | | 1..1064 | 965/1081 (88%) | |
| CAC86015 | METALLOPROTEASE DISINTEGRIN 16 WITH THROMBOSPONDIN TYPE I MOTIF - Homo sapiens (Human), 1072 aa. | 20..1007 | 576/1081 (53%) | 0.0 |
| | | 9..1065 | 719/1081 (66%) | |
| Q9H324 | ADAMTS-10 precursor (EC 3.4.24.-) (A disintegrin and metalloproteinase with thrombospondin motifs 10) (ADAM-TS 10) (ADAM-TS10) - Homo sapiens (Human), 1077 aa (fragment). | 59..1158 | 412/1134 (36%) | 0.0 |
| | | 11..1074 | 584/1134 (51%) | |
| CAD20434 | SEQUENCE 8 FROM PATENT WO0188156 - Homo sapiens (Human), 1044 aa (fragment). | 59..1101 | 398/1074(37%) | 0.0 |
| | | 37..1041 | 558/1074 (51%) | |
| P58397 | ADAMTS-12 precursor (EC 3.4.24.-) (A disintegrin and metalloproteinase with thrombospondin motifs 12) (ADAM-TS 12) (ADAM- TS12) - Homo sapiens (Human), 1593 aa. | 60..1058 | 375/1026 (36%) | 0.0 |
| | | 51..997 | 548/1026 (52%) | |

PFam analysis predicts that the NOV39a protein contains the domains shown in the Table 39F.

| **Table 39F. Domain Analysis of NOV39a** | | | |
|---|---|---|---|
| **Pfam Domain** | **NOV39a Match Region** | **Identities/ Similarities for the Matched Region** | **Expect Value** |
| Pep_M12B_propep | 111..222 | 27/119 (23%) | 1.6e-13 |
| | | 72/119 (61%) | |
| Reprolysin | 295..498 | 66/221 (30%) | 1.1e-21 |
| | | 158/221 (71 %) | |
| tsp_1 | 593..643 | 23/54 (43%) | 1.2e-12 |
| | | 36/54 (67%) | |
| tsp_1 | 879..932 | 13/60 (22%) | 0.0042 |
| | | 39/60 (65%) | |
| tsp_1 | 934..989 | 18/64 (28%) | 0.022 |
| | | 36/64 (56%) | |
| tsp_1 | 997..1056 | 18/64 (28%) | 0.015 |
| | | 39/64 (61%) | |
| tsp_1 | 1072..1118 | 14/55 (25%) | 0.0041 |
| | | 34/55 (62%) | |

### Example 40.

The NOV40 clone was analyzed, and the nucleotide and encoded polypeptide sequences are shown in Table 40A.

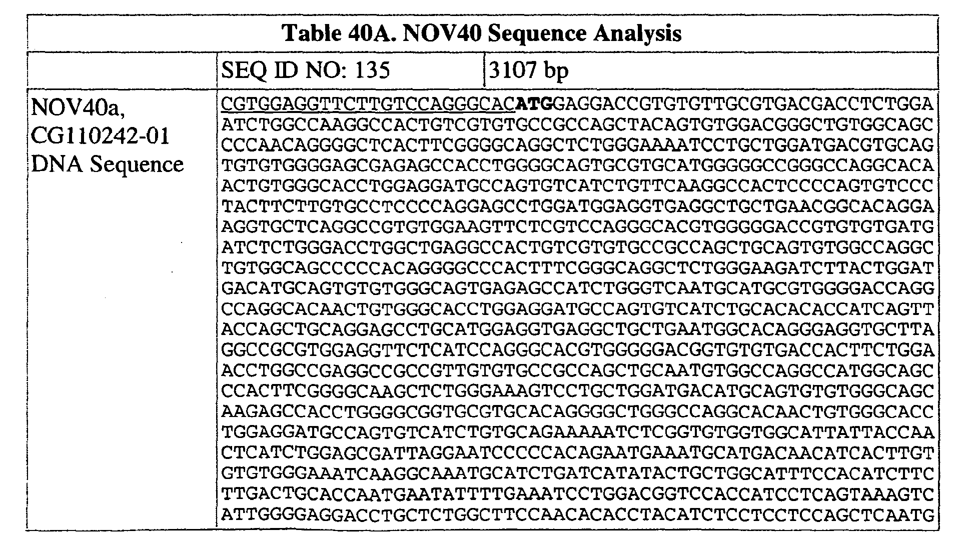

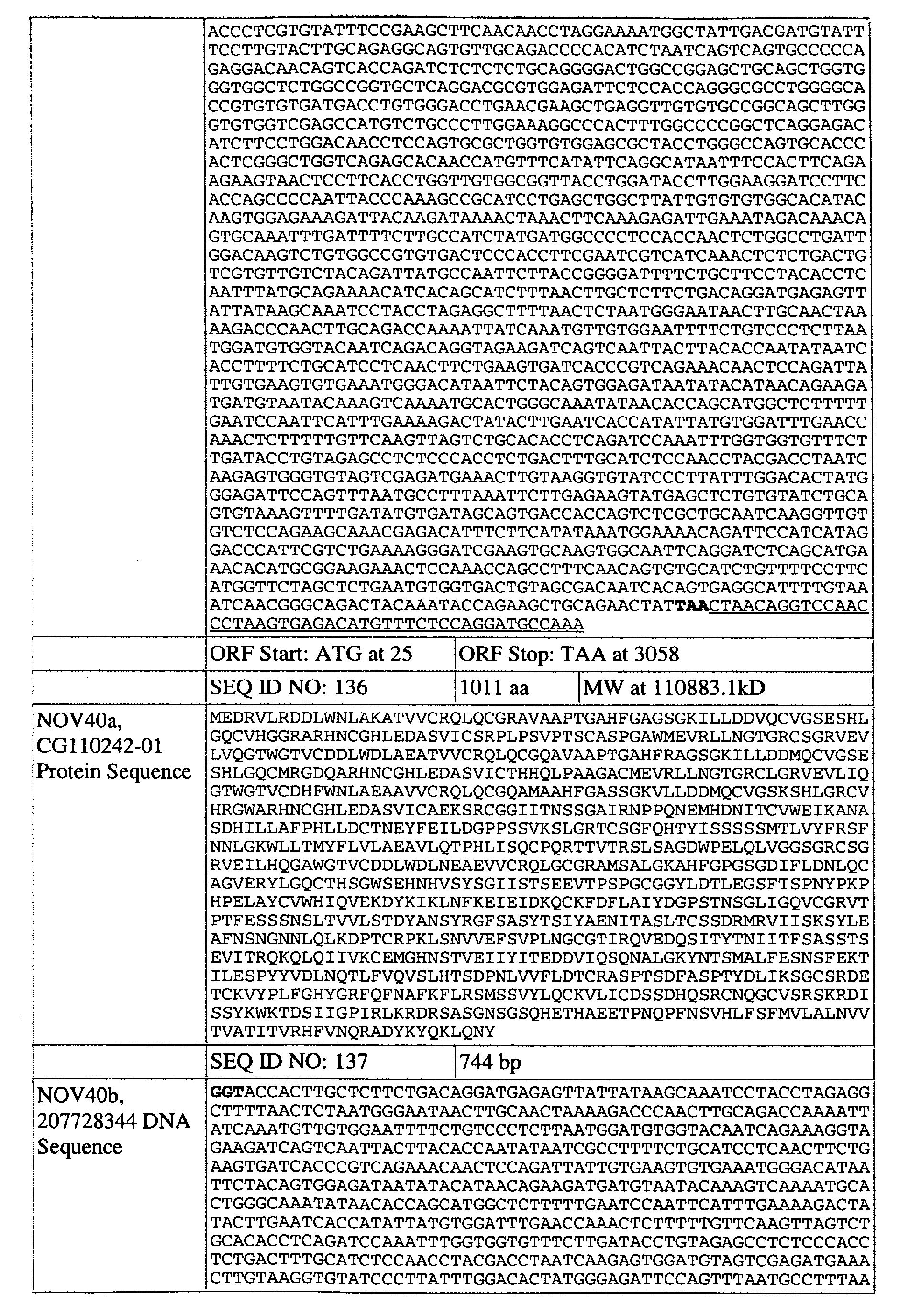

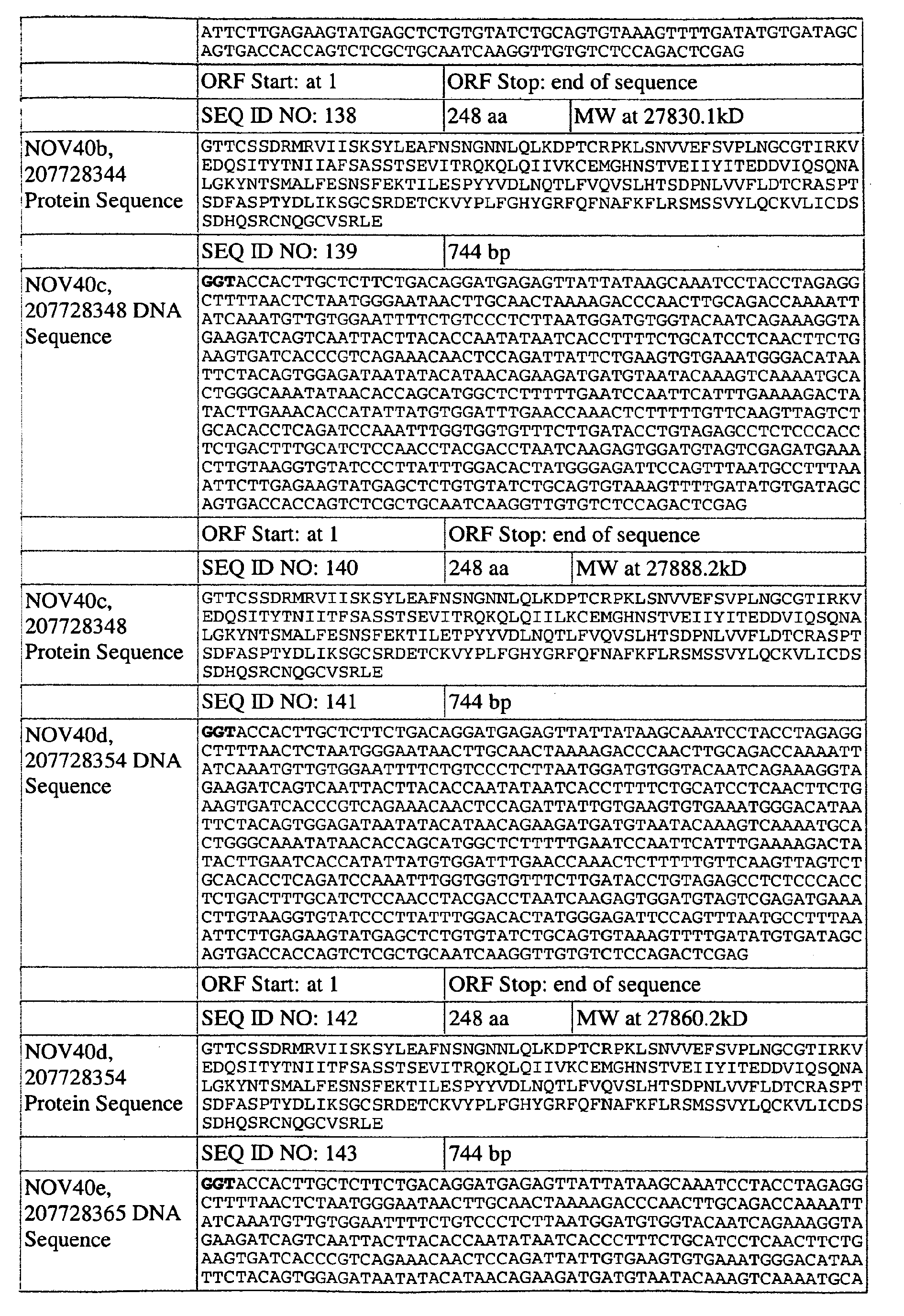

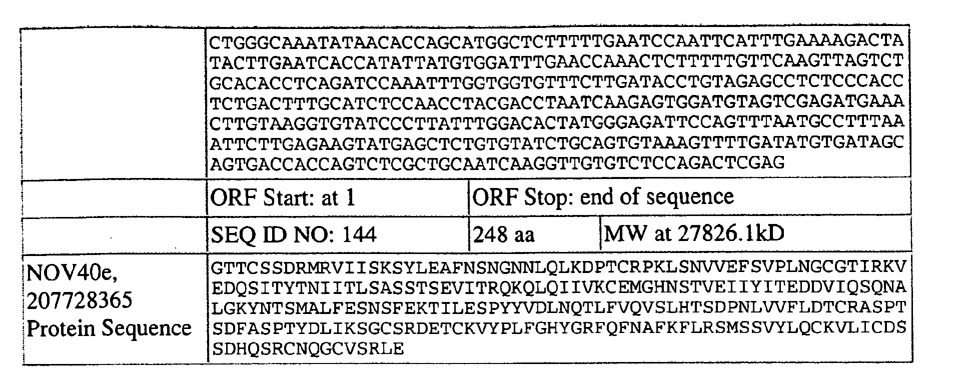

Sequence comparison of the above protein sequences yields the following sequence relationships shown in Table 40B.

| **Table 40B. Comparison of NOV40a against NOV40b through NOV40e.** | | |
|---|---|---|
| **Protein Sequence** | **NOV40a Residues/ Match Residues** | **Identities/ Similarities for the Matched Region** |
| NOV40b | 680..923 | 242/244 (99%) |
| | 3..246 | 243/244 (99%) |
| NOV40c | 680..923 | 241/244 (98%) |
| | 3..246 | 244/244 (99%) |
| NOV40d | 680..923 | 243/244 (99%) |
| | 3..246 | 244/244 (99%) |
| NOV40e | 680..923 | 242/244 (99%) |
| | 3..246 | 243/244 (99%) |

Further analysis of the NOV40a protein yielded the following properties shown in Table 40C.

| **Table 40C. Protein Sequence Properties NOV40a** | |
|---|---|
| PSort analysis: | 0.7000 probability located in plasma membrane; 0.5843 probability located in mitochondrial inner membrane; 0.3000 probability located in microbody (peroxisome); 0.2000 probability located in endoplasmic reticulum (membrane) |
| SignalP analysis: | No Known Signal Sequence Predicted |

A search of the NOV40a protein against the Geneseq database, a proprietary database that contains sequences published in patents and patent publication, yielded several homologous proteins shown in Table 40D.

| **Table 40D. Geneseq Results for NOV40a** | | | | |
|---|---|---|---|---|
| **Geneseq Identifier** | **Protein/Organism/Length [Patent #, Date]** | **NOV40a Residues/ Match Residues** | **Identities/ Similarities for the Matched Region** | **Expect Value** |
| AAB80245 | Human PRO257 protein - Homo sapiens, 607 aa. [W0200104311-A1, 18-JAN-2001] | 559..1011 | 449/456 (98%) | 0.0 |
| | | 152..607 | 450/456 (98%) | |
| AAU12343 | Human PRO257 polypeptide sequence - Homo sapiens, 607 aa. [WO200140466-A2, 07-JUN-2001] | 559..1011 | 449/456 (98%) | 0.0 |
| | | 152..607 | 450/456 (98%) | |
| AAB07456 | Protein encoded by a novel gene associated with insulin synthesis - Homo sapiens, 585 aa. [WO200040722-A2, 13-JUL-2000] | 559..1011 | 449/456 (98%) | 0.0 |
| | | 130..585 | 450/456 (98%) | |
| AAY13377 | Amino acid sequence of protein PRO257 - Homo sapiens, 607 aa. [WO9914328-A2, 25-MAR-1999] | 559..1011 | 449/456 (98%) | 0.0 |
| | | 152..607 | 450/456 (98%) | |
| AAY25323 | Human pancreatic PA153 consensus protein - Homo sapiens, 607 aa. [WO9931274-A2, 24-JUN-1999] | 559..1011 | 449/456 (98%) | 0.0 |
| | | 152..607 | 450/456 (98%) | |

In a BLAST search of public sequence databases, the NOV40a protein was found to have homology to the proteins shown in the BLASTP data in Table 40E.

| **Table 40E. Public BLASTP Results for NOV40a** | | | | |
|---|---|---|---|---|
| **Protein Accession Number** | **Protein/Organism/Length** | **NOV40a Residues/ Match Residues** | **Identities/ Similarities for the Matched Portion** | **Expect Value** |
| Q96DU4 | DMBT1/8KB.2 PROTEIN PRECURSOR - Homo sapiens (Human), 2413 aa. | 8..945 | 435/1028 (42%) | 0.0 |
| | | 1406..2403 | 605/1028 (58%) | |
| Q9UGM3 | DMBT1 PROTOTYPE PRECURSOR - Homo sapiens (Human), 2426 aa. | 8..945 | 435/1028 (42%) | 0.0 |
| | | 1419..2416 | 605/1028 (58%) | |
| Q9Y4V9 | DMBT1/6KB.1 PROTEIN PRECURSOR - Homo sapiens (Human), 1785 aa. | 8..945 | 435/1028 (42%) | 0.0 |
| | | 778..1775 | 605/1028 (58%) | |
| Q9UKJ4 | GP-340 VARIANT PROTEIN - Homo sapiens (Human), 2413 aa. | 8..945 | 435/1028 (42%) | 0.0 |
| | | 1406..2403 | 605/1028 (58%) | |
| Q9Y211 | DMBT1 - Homo sapiens (Human), 1785 aa. | 8..945 | 435/1028 (42%) | 0.0 |
| | | 778..1775 | 605/1028 (58%) | |

PFam analysis predicts that the NOV40a protein contains the domains shown in the Table 40F.

| **Table 40F. Domain Analysis of NOV40a** | | | |
|---|---|---|---|
| **Pfam Domain** | **NOV40a Match Region** | **Identities/ Similarities for the Matched Region** | **Expect Value** |
| SRCR | 1..82 | 37/114 (32%) | 2.2e-10 |
| | | 62/114 (54%) | |
| SRCR | 104..201 | 48/114 (42%) | 2.4e-34 |
| | | 78/114 (68%) | |
| SRCR | 217..310 | 43/113 (38%) | 3.2e-24 |
| | | 72/113 (64%) | |
| CUB | 315..416 | 38/118 (32%) | 2.6e-10 |
| | | 72/118 (61%) | |
| SRCR | 456..551 | 47/114 (41%) | 1.9e-28 |
| | | 78/114 (68%) | |
| CUB | 561..667 | 38/117 (32%) | 4.9e-35 |
| | | 83/117 (71%) | |
| zona_pellucida | 680..923 | 78/286 (27%) | 4.1e-40 |
| | | 184/286 (64%) | |

### Example 41.

The NOV41 clone was analyzed, and the nucleotide and encoded polypeptide sequences are shown in Table 41A.

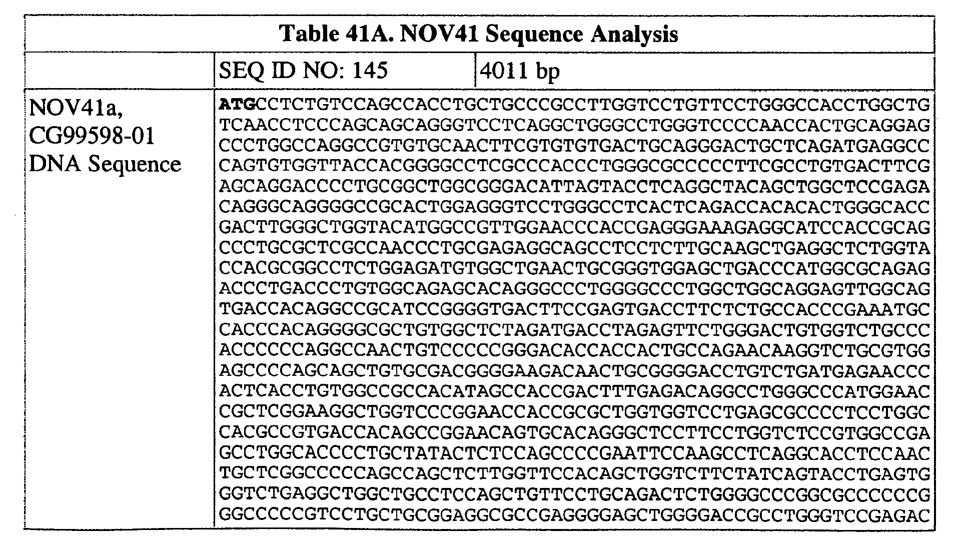

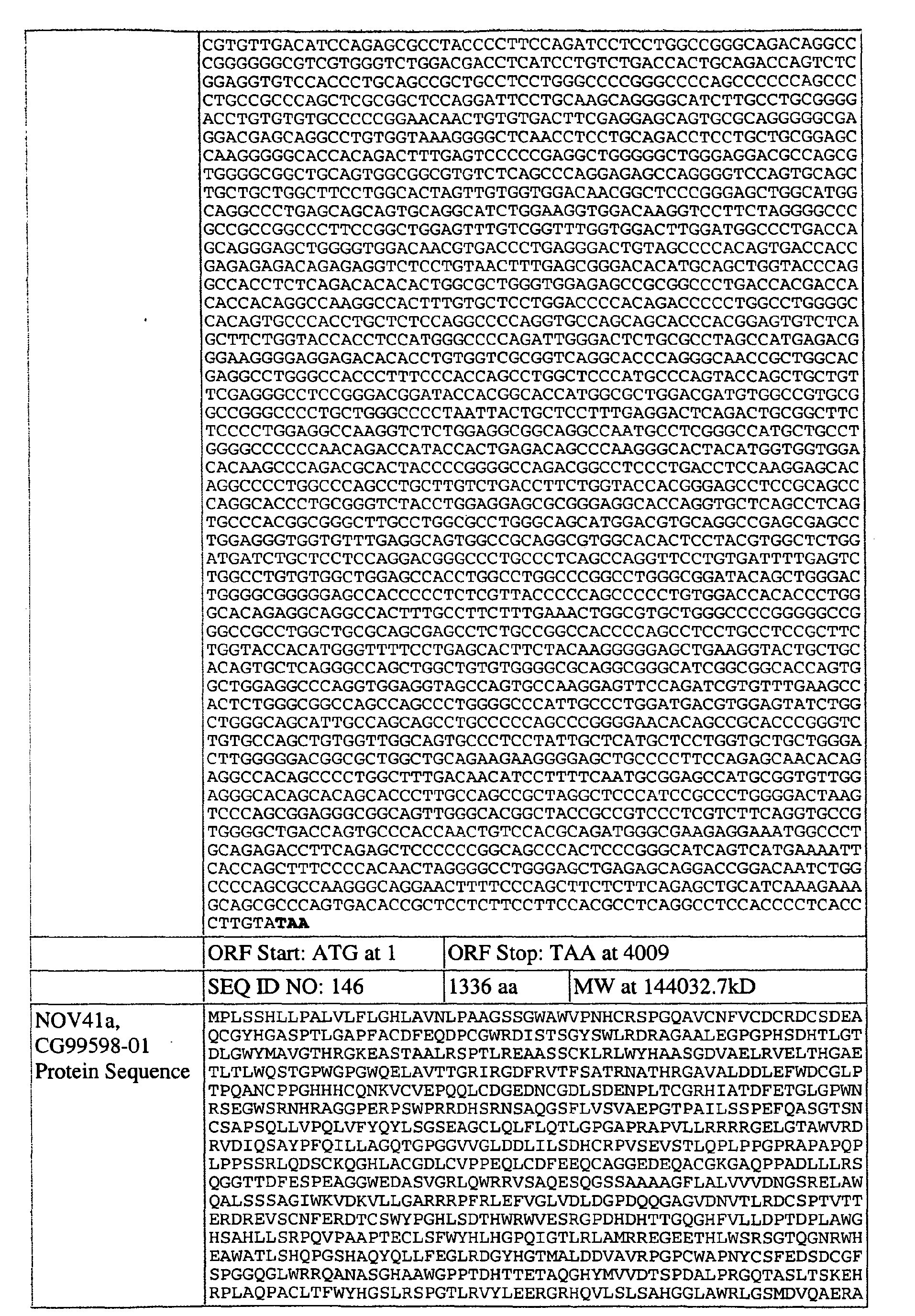

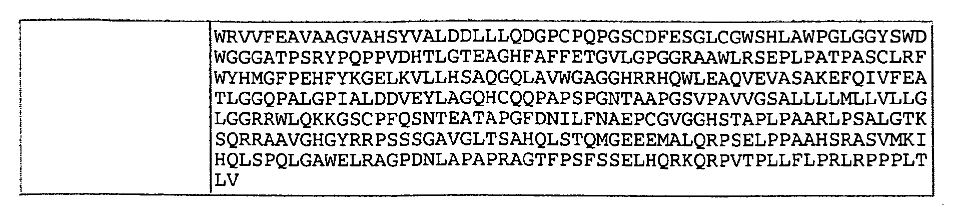

Further analysis of the NOV41a protein yielded the following properties shown in Table 41B.

| **Table 41B. Protein Sequence Properties NOV41a** | |
|---|---|
| PSort analysis: | 0.4600 probability located in plasma membrane; 0.2464 probability located in microbody (peroxisome); 0.1000 probability located in endoplasmic reticulum (membrane); 0.1000 probability located in endoplasmic reticulum (lumen) |
| SignalP analysis: | Cleavage site between residues 20 and 21 |

A search of the NOV41a protein against the Geneseq database, a proprietary database that contains sequences published in patents and patent publication, yielded several homologous proteins shown in Table 41C.

| **Table 41C. Geneseq Results for NOV41a** | | | | |
|---|---|---|---|---|
| **Geneseq Identifier** | **Protein/Organism/Length [Patent #, Date]** | **NOV41a Residues/ Match Residues** | **Identities/ Similarities for the Matched Region** | **Expect Value** |
| AAE17494 | Human secretion and trafficking protein-3 (SAT-3) - Homo sapiens, 1217 aa. [WO200202610-A2, 10-JAN-2002] | 1..1194 | 1159/1238 (93%) | 0.0 |
| | | 1..1205 | 1160/1238 (93%) | |
| AAU29282 | Human PRO polypeptide sequence #259 - Homo sapiens, 1137 aa. [WO200168848-A2, 20-SEP-2001] | 1..1194 | 1093/1195 (91%) | 0.0 |
| | | 1..1125 | 1099/1195 (91%) | |
| AAB42780 | Human ORFX ORF2544 polypeptide sequence SEQ ID NO:5088 - Homo sapiens, 465 aa. [WO200058473-A2, 05-OCT-2000] | 161..620 | 372/475 (78%) | 0.0 |
| | | 45..453 | 379/475 (79%) | |
| AAB01432 | Human TANGO 239 (form 2) - Homo sapiens, 686 aa. [WO200039284-A1, 06-JUL-2000] | 574..957 | 110/406 (27%) | 4e-29 |
| | | 270..664 | 178/406 (43%) | |
| ABB53298 | Human polypeptide #38 - Homo sapiens, 686 aa. [W0200181363-A1, 01-NOV-2001] | 574..957 | 109/406 (26%) | 8e-28 |
| | | 270..664 | 176/406 (42%) | |

In a BLAST search of public sequence databases, the NOV41a protein was found to have homology to the proteins shown in the BLASTP data in Table 41D.

| **Table 41D. Public BLASTP Results for NOV41a** | | | | |
|---|---|---|---|---|
| **Protein Accession Number** | **Protein/Organism/Length** | **NOV41a Residues/ Match Residues** | **Identities/ Similarities for the Matched Portion** | **Expect Value** |
| Q63191 | Apical endosomal glycoprotein precursor - Rattus norvegicus (Rat), 1216 aa. | 1..1194 | 848/1245 (68%) | 0.0 |
| | | 1..1204 | 944/1245 (75%) | |
| Q91641 | Thyroid hormone-induced protein B precursor - Xenopus laevis (African clawed frog), 688 aa. | 573..957 | 118/415 (28%) | 1e-28 |
| | | 270..667 | 187/415 (44%) | |
| 088799 | Zonadhesin precursor - Mus musculus (Mouse), 5376 aa. | 631..1109 | 141/518 (27%) | 3e-27 |
| | | 30..525 | 221/518 (42%) | |
| Q99ND0 | ZAN (ZONADHESIN) - Mus musculus (Mouse), 5374 aa. | 631..1109 | 141/518 (27%) | 6e-27 |
| | | 30..525 | 221/518 (42%) | |
| Q9BZ83 | ZONADHESIN VARIANT 6 - Homo sapiens (Human), 2721 aa. | 636..1109 | 143/522 (27%) | 8e-20 |
| | | 29..519 | 209/522 (39%) | |

PFam analysis predicts that the NOV41a protein contains the domains shown in the Table 41E.

| **Table 41E. Domain Analysis of NOV41a** | | | |
|---|---|---|---|
| **Pfam Domain** | **NOV41a Match Region** | **Identities/ Similarities for the Matched Region** | **Expect Value** |
| MAM | 75..231 | 57/174 (33%) | 1.4e-44 |
| | | 122/174 (70%) | |
| ldl_recept_a | 236..276 | 13/43 (30%) | 3.3e-09 |
| | | 29/43 (67%) | |
| MAM | 280..443 | 48/188 (26%) | 7.2e-22 |
| | | 122/188 (65%) | |
| ldl_recept_a | 473..510 | 12/43 (28%) | 0.2 |
| | | 26/43 (60%) | |
| MAM | 493..634 | 33/174 (19%) | 0.0004 |
| | | 86/174 (49%) | |
| MAM | 646..799 | 68/173 (39%) | 6.7e-54 |
| | | 132/173 (76%) | |
| MAM | 803..959 | 67/173 (39%) | 2.5e-47 |
| | | 119/173 (69%) | |
| MAM | 963..1128 | 62/176 (35%) | 6.4e-56 |
| | | 137/176 (78%) | |

### Example B: Sequencing Methodology and Identification of NOVX Clones

**1. GeneCalling™ Technology:** This is a proprietary method of performing differential gene expression profiling between two or more samples developed at CuraGen and described by Shimkets, et al., "Gene expression analysis by transcript profiling coupled to a gene database query" Nature Biotechnology 17:198-803 (1999). cDNA was derived from various human samples representing multiple tissue types, normal and diseased states, physiological states, and developmental states from different donors. Samples were obtained as whole tissue, primary cells or tissue cultured primary cells or cell lines. Cells and cell lines may have been treated with biological or chemical agents that regulate gene expression, for example, growth factors, chemokines or steroids. The cDNA thus derived was then digested with up to as many as 120 pairs of restriction enzymes and pairs of linker-adaptors specific for each pair of restriction enzymes were ligated to the appropriate end. The restriction digestion generates a mixture of unique cDNA gene fragments. Limited PCR amplification is performed with primers homologous to the linker adapter sequence where one primer is biotinylated and the other is fluorescently labeled. The doubly labeled material is isolated and the fluorescently labeled single strand is resolved by capillary gel electrophoresis. A computer algorithm compares the electropherograms from an experimental and control group for each of the restriction digestions. This and additional sequence-derived information is used to predict the identity of each differentially expressed gene fragment using a variety of genetic databases. The identity of the gene fragment is confirmed by additional, gene-specific competitive PCR or by isolation and sequencing of the gene fragment.
**2. SeqCalling™ Technology:** cDNA was derived from various human samples representing multiple tissue types, normal and diseased states, physiological states, and developmental states from different donors. Samples were obtained as whole tissue, primary cells or tissue cultured primary cells or cell lines. Cells and cell lines may have been treated with biological or chemical agents that regulate gene expression, for example, growth factors, chemokines or steroids. The cDNA thus derived was then sequenced using CuraGen's proprietary SeqCalling technology. Sequence traces were evaluated manually and edited for corrections if appropriate. cDNA sequences from all samples were assembled together, sometimes including public human sequences, using bioinformatic programs to produce a consensus sequence for each assembly. Each assembly is included in CuraGen Corporation's database. Sequences were included as components for assembly when the extent of identity with another component was at least 95% over 50 bp. Each assembly represents a gene or portion thereof and includes information on variants, such as splice forms single nucleotide polymorphisms (SNPs), insertions, deletions and other sequence variations.
**3. PathCalling™ Technology:**
   The NOVX nucleic acid sequences are derived by laboratory screening of cDNA library by the two-hybrid approach. cDNA fragments covering either the full length of the DNA sequence, or part of the sequence, or both, are sequenced. In silico prediction was based on sequences available in CuraGen Corporation's proprietary sequence databases or in the public human sequence databases, and provided either the full length DNA sequence, or some portion thereof.
   The laboratory screening was performed using the methods summarized below:
   cDNA libraries were derived from various human samples representing multiple tissue types, normal and diseased states, physiological states, and developmental states from different donors. Samples were obtained as whole tissue, primary cells or tissue cultured primary cells or cell lines. Cells and cell lines may have been treated with biological or chemical agents that regulate gene expression, for example, growth factors, chemokines or steroids. The cDNA thus derived was then directionally cloned into the appropriate two-hybrid vector (Gal4-activation domain (Gal4-AD) fusion). Such cDNA libraries as well as commercially available cDNA libraries from Clontech (Palo Alto, CA) were then transferred from E.coli into a CuraGen Corporation proprietary yeast strain (disclosed in U. S. Patents 6,057,101 and 6,083,693, incorporated herein by reference in their entireties).
   Gal4-binding domain (Gal4-BD) fusions of a CuraGen Corportion proprietary library of human sequences was used to screen multiple Gal4-AD fusion cDNA libraries resulting in the selection of yeast hybrid diploids in each of which the Gal4-AD fusion contains an individual cDNA. Each sample was amplified using the polymerase chain reaction (PCR) using non-specific primers at the cDNA insert boundaries. Such PCR product was sequenced; sequence traces were evaluated manually and edited for corrections if appropriate. cDNA sequences from all samples were assembled together, sometimes including public human sequences, using bioinformatic programs to produce a consensus sequence for each assembly. Each assembly is included in CuraGen Corporation's database. Sequences were included as components for assembly when the extent of identity with another component was at least 95% over 50 bp. Each assembly represents a gene or portion thereof and includes information on variants, such as splice forms single nucleotide polymorphisms (SNPs), insertions, deletions and other sequence variations.
   Physical clone: the cDNA fragment derived by the screening procedure, covering the entire open reading frame is, as a recombinant DNA, cloned into pACT2 plasmid (Clontech) used to make the cDNA library. The recombinant plasmid is inserted into the host and selected by the yeast hybrid diploid generated during the screening procedure by the mating of both CuraGen Corporation proprietary yeast strains N106' and YULH (U. S. Patents 6,057,101 and 6,083,693).
**4. RACE:** Techniques based on the polymerase chain reaction such as rapid amplification of cDNA ends (RACE), were used to isolate or complete the predicted sequence of the cDNA of the invention. Usually multiple clones were sequenced from one or more human samples to derive the sequences for fragments. Various human tissue samples from different donors were used for the RACE reaction. The sequences derived from these procedures were included in the SeqCalling Assembly process described in preceding paragraphs.
**5. Exon Linking:** The NOVX target sequences identified in the present invention were subjected to the exon linking process to confirm the sequence. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such primers were designed based on in silico predictions for the full length cDNA, part (one or more exons) of the DNA or protein sequence of the target sequence, or by translated homology of the predicted exons to closely related human sequences from other species. These primers were then employed in PCR amplification based on the following pool of human cDNAs: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. Usually the resulting amplicons were gel purified, cloned and sequenced to high redundancy. The PCR product derived from exon linking was cloned into the pCR2.1 vector from Invitrogen. The resulting bacterial clone has an insert covering the entire open reading frame cloned into the pCR2.1 vector. The resulting sequences from all clones were assembled with themselves, with other fragments in CuraGen Corporation's database and with public ESTs. Fragments and ESTs were included as components for an assembly when the extent of their identity with another component of the assembly was at least 95% over 50 bp. In addition, sequence traces were evaluated manually and edited for corrections if appropriate. These procedures provide the sequence reported herein.
**6. Physical Clone:** Exons were predicted by homology and the intron/exon boundaries were determined using standard genetic rules. Exons were further selected and refined by means of similarity determination using multiple BLAST (for example, tBlastN, BlastX, and BlastN) searches, and, in some instances, GeneScan and Grail. Expressed sequences from both public and proprietary databases were also added when available to further define and complete the gene sequence. The DNA sequence was then manually corrected for apparent inconsistencies thereby obtaining the sequences encoding the full-length protein.
   The PCR product derived by exon linking, covering the entire open reading frame, was cloned into the pCR2.1 vector from Invitrogen to provide clones used for expression and screening purposes.

### Example C: Quantitative expression analysis of clones in various cells and tissues

The quantitative expression of various clones was assessed using microtiter plates containing RNA samples from a variety of normal and pathology-derived cells, cell lines and tissues using real time quantitative PCR (RTQ PCR). RTQ PCR was performed on an Applied Biosystems ABI PRISM® 7700 or an ABI PRISM® 7900 HT Sequence Detection System. Various collections of samples are assembled on the plates, and referred to as Panel 1 (containing normal tissues and cancer cell lines), Panel 2 (containing samples derived from tissues from normal and cancer sources), Panel 3 (containing cancer cell lines), Panel 4 (containing cells and cell lines from normal tissues and cells related to inflammatory conditions), Panel 5D/5I (containing human tissues and cell lines with an emphasis on metabolic diseases), AI_comprehensive_panel (containing normal tissue and samples from autoimmune diseases), Panel CNSD.01 (containing central nervous system samples from normal and diseased brains) and CNS_neurodegeneration_panel (containing samples from normal and Alzheimer's diseased brains).

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

First, the RNA samples were normalized to reference nucleic acids such as constitutively expressed genes (for example, β-actin and GAPDH). Normalized RNA (5 ul) was converted to cDNA and analyzed by RTQ-PCR using One Step RT-PCR Master Mix Reagents (Applied Biosystems; Catalog No. 4309169) and gene-specific primers according to the manufacturer's instructions.

In other cases, non-normalized RNA samples were converted to single strand cDNA (sscDNA) using Superscript II (Invitrogen Corporation; Catalog No. 18064-147) and random hexamers according to the manufacturer's instructions. Reactions containing up to 10 µg of total RNA were performed in a volume of 20 µl and incubated for 60 minutes at 42 °C. This reaction can be scaled up to 50 µg of total RNA in a final volume of 100 µl. sscDNA samples are then normalized to reference nucleic acids as described previously, using 1X TaqMan® Universal Master mix (Applied Biosystems; catalog No. 4324020), following the manufacturer's instructions.

Probes and primers were designed for each assay according to Applied Biosystems Primer Express Software package (version I for Apple Computer's Macintosh Power PC) or a similar algorithm using the target sequence as input. Default settings were used for reaction conditions and the following parameters were set before selecting primers: primer concentration = 250 nM, primer melting temperature (Tm) range = 58 °-60 °C, primer optimal Tm = 59 °C, maximum primer difference = 2 °C, probe does not have 5'G, probe Tm must be 10 °C greater than primer Tm, amplicon size 75bp to 100bp. The probes and primers selected (see below) were synthesized by Synthegen (Houston, TX, USA). Probes were double purified by HPLC to remove uncoupled dye and evaluated by mass spectroscopy to verify coupling of reporter and quencher dyes to the 5' and 3' ends of the probe, respectively. Their final concentrations were: forward and reverse primers, 900nM each, and probe, 200nM.

PCR conditions: When working with RNA samples, normalized RNA from each tissue and each cell line was spotted in each well of either a 96 well or a 384-well PCR plate (Applied Biosystems). PCR cocktails included either a single gene specific probe and primers set, or two multiplexed probe and primers sets (a set specific for the target clone and another gene-specific set multiplexed with the target probe). PCR reactions were set up using TaqMan® One-Step RT-PCR Master Mix (Applied Biosystems, Catalog No. 4313803) following manufacturer's instructions. Reverse transcription was performed at 48 °C for 30 minutes followed by amplification/PCR cycles as follows: 95°C 10 min, then 40 cycles of 95 °C for 15 seconds, 60 °C for 1 minute. Results were recorded as CT values (cycle at which a given sample crosses a threshold level of fluorescence) using a log scale, with the difference in RNA concentration between a given sample and the sample with the lowest CT value being represented as 2 to the power of delta CT. The percent relative expression is then obtained by taking the reciprocal of this RNA difference and multiplying by 100.

When working with sscDNA samples, normalized sscDNA was used as described previously for RNA samples. PCR reactions containing one or two sets of probe and primers were set up as described previously, using 1X TaqMan® Universal Master mix (Applied Biosystems; catalog No. 4324020), following the manufacturer's instructions. PCR amplification was performed as follows: 95 °C 10 min, then 40 cycles of 95 °C for 15 seconds, 60 °C for 1 minute. Results were analyzed and processed as described previously.

### Panels 1, 1.1, 1.2, and 1.3D

The plates for Panels 1, 1.1, 1.2 and 1.3D include 2 control wells (genomic DNA control and chemistry control) and 94 wells containing cDNA from various samples. The samples in these panels are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in these panels are widely available through the American Type Culture Collection (ATCC), a repository for cultured cell lines, and were cultured using the conditions recommended by the ATCC. The normal tissues found on these panels are comprised of samples derived from all major organ systems from single adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose.
In the results for Panels 1, 1.1, 1.2 and 1.3D, the following abbreviations are used:
ca. = carcinoma,
* = established from metastasis,
met = metastasis,
s cell var = small cell variant,
non-s = non-sm = non-small,
squam = squamous,
pl. eff = pl effusion = pleural effusion,
glio = glioma,
astro = astrocytoma, and
neuro = neuroblastoma.

### General_screening_panel_v1.4

The plates for Panel 1.4 include 2 control wells (genomic DNA control and chemistry control) and 94 wells containing cDNA from various samples. The samples in Panel 1.4 are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in Panel 1.4 are widely available through the American Type Culture Collection (ATCC), a repository for cultured cell lines, and were cultured using the conditions recommended by the ATCC. The normal tissues found on Panel 1.4 are comprised of pools of samples derived from all major organ systems from 2 to 5 different adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose. Abbreviations are as described for Panels 1, 1.1, 1.2, and 1.3D.

### Panels 2D and 2.2

The plates for Panels 2D and 2.2 generally include 2 control wells and 94 test samples composed of RNA or cDNA isolated from human tissue procured by surgeons working in close cooperation with the National Cancer Institute's Cooperative Human Tissue Network (CHTN) or the National Disease Research Initiative (NDRI). The tissues are derived from human malignancies and in cases where indicated many malignant tissues have "matched margins" obtained from noncancerous tissue just adjacent to the tumor. These are termed normal adjacent tissues and are denoted "NAT" in the results below. The tumor tissue and the "matched margins" are evaluated by two independent pathologists (the surgical pathologists and again by a pathologist at NDRI or CHTN). This analysis provides a gross histopathological assessment of tumor differentiation grade. Moreover, most samples include the original surgical pathology report that provides information regarding the clinical stage of the patient. These matched margins are taken from the tissue surrounding (*i*.*e*. immediately proximal) to the zone of surgery (designated "NAT", for normal adjacent tissue, in Table RR). In addition, RNA and cDNA samples were obtained from various human tissues derived from autopsies performed on elderly people or sudden death victims (accidents, etc.). These tissues were ascertained to be free of disease and were purchased from various commercial sources such as Clontech (Palo Alto, CA), Research Genetics, and Invitrogen. General oncology screening panel_v_2.4 is an updated version of Panel 2D.

### Panel 3D

The plates of Panel 3D are comprised of 94 cDNA samples and two control samples. Specifically, 92 of these samples are derived from cultured human cancer cell lines, 2 samples of human primary cerebellar tissue and 2 controls. The human cell lines are generally obtained from ATCC (American Type Culture Collection), NCI or the German tumor cell bank and fall into the following tissue groups: Squamous cell carcinoma of the tongue, breast cancer, prostate cancer, melanoma, epidermoid carcinoma, sarcomas, bladder carcinomas, pancreatic cancers, kidney cancers, leukemias/lymphomas, ovarian/uterine/cervical, gastric, colon, lung and CNS cancer cell lines. In addition, there are two independent samples of cerebellum. These cells are all cultured under standard recommended conditions and RNA extracted using the standard procedures. The cell lines in panel 3D and 1.3D are of the most common cell lines used in the scientific literature. Oncology_cell_line_screening_panel_v3.2 is an updated version of Panel 3. The cell lines in panel 3D, 1.3D and oncology_cell_line_screening_panel_v3.2 are of the most common cell lines used in the scientific literature.

### Panels 4D, 4R, and 4.1D

Panel 4 includes samples on a 96 well plate (2 control wells, 94 test samples) composed of RNA (Panel 4R) or cDNA (Panels 4D/4.1D) isolated from various human cell lines or tissues related to inflammatory conditions. Total RNA from control normal tissues such as colon and lung (Stratagene, La Jolla, CA) and thymus and kidney (Clontech) was employed. Total RNA from liver tissue from cirrhosis patients and kidney from lupus patients was obtained from BioChain (Biochain Institute, Inc., Hayward, CA). Intestinal tissue for RNA preparation from patients diagnosed as having Crohn's disease and ulcerative colitis was obtained from the National Disease Research Interchange (NDRI) (Philadelphia, PA).

Astrocytes, lung fibroblasts, dermal fibroblasts, coronary artery smooth muscle cells, small airway epithelium, bronchial epithelium, microvascular dermal endothelial cells, microvascular lung endothelial cells, human pulmonary aortic endothelial cells, human umbilical vein endothelial cells were all purchased from Clonetics (Walkersville, MD) and grown in the media supplied for these cell types by Clonetics. These primary cell types were activated with various cytokines or combinations of cytokines for 6 and/or 12-14 hours, as indicated. The following cytokines were used; IL-1 beta at approximately 1-5ng/ml, TNF alpha at approximately 5-10ng/ml, IFN gamma at approximately 20-50ng/ml, IL-4 at approximately 5-10ng/ml, IL-9 at approximately 5-10ng/ml, IL-13 at approximately 5-10ng/ml. Endothelial cells were sometimes starved for various times by culture in the basal media from Clonetics with 0.1% serum.

Mononuclear cells were prepared from blood of employees at CuraGen Corporation, using Ficoll. LAK cells were prepared from these cells by culture in DMEM 5% FCS (Hyclone), 100µM non essential amino acids (Gibco/Life Technologies, Rockville, MD), 1mM sodium pyruvate (Gibco), mercaptoethanol 5.5x10⁻⁵M (Gibco), and 10mM Hepes (Gibco) and Interleukin 2 for 4-6 days. Cells were then either activated with 10-20ng/ml PMA and 1-2µg/ml ionomycin, IL-12 at 5-10ng/ml, IFN gamma at 20-50ng/ml and IL-18 at 5-10ng/ml for 6 hours. In some cases, mononuclear cells were cultured for 4-5 days in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) with PHA (phytohemagglutinin) or PWM (pokeweed mitogen) at approximately 5µg/ml. Samples were taken at 24, 48 and 72 hours for RNA preparation. MLR (mixed lymphocyte reaction) samples were obtained by taking blood from two donors, isolating the mononuclear cells using Ficoll and mixing the isolated mononuclear cells 1:1 at a final concentration of approximately 2x10⁶cells/ml in DMEM 5% FCS (Hyclone), 100µM non essential amino acids (Gibco), 1mM sodium pyruvate (Gibco), mercaptoethanol (5.5x10⁻⁵M) (Gibco), and 10mM Hepes (Gibco). The MLR was cultured and samples taken at various time points ranging from 1- 7 days for RNA preparation.

Monocytes were isolated from mononuclear cells using CD14 Miltenyi Beads, +ve VS selection columns and a Vario Magnet according to the manufacturer's instructions. Monocytes were differentiated into dendritic cells by culture in DMEM 5% fetal calf serum (FCS) (Hyclone, Logan, UT), 100µM non essential amino acids (Gibco), 1mM sodium pyruvate (Gibco), mercaptoethanol 5.5x10⁻⁵M (Gibco), and 10mM Hepes (Gibco), 50ng/ml GMCSF and 5ng/ml IL-4 for 5-7 days. Macrophages were prepared by culture of monocytes for 5-7 days in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and 10% AB Human Serum or MCSF at approximately 50ng/ml. Monocytes, macrophages and dendritic cells were stimulated for 6 and 12-14 hours with lipopolysaccharide (LPS) at 100ng/ml. Dendritic cells were also stimulated with anti-CD40 monoclonal antibody (Pharmingen) at 10µg/ml for 6 and 12-14 hours.

CD4 lymphocytes, CD8 lymphocytes and NK cells were also isolated from mononuclear cells using CD4, CD8 and CD56 Miltenyi beads, positive VS selection columns and a Vario Magnet according to the manufacturer's instructions. CD45RA and CD45RO CD4 lymphocytes were isolated by depleting mononuclear cells of CD8, CD56, CD14 and CD19 cells using CD8, CD56, CD14 and CD19 Miltenyi beads and positive selection. CD45RO beads were then used to isolate the CD45RO CD4 lymphocytes with the remaining cells being CD45RA CD4 lymphocytes. CD45RA CD4, CD45RO CD4 and CD8 lymphocytes were placed in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and plated at 10⁶cells/ml onto Falcon 6 well tissue culture plates that had been coated overnight with 0.5µg/ml anti-CD28 (Pharmingen) and 3ug/ml anti-CD3 (OKT3, ATCC) in PBS. After 6 and 24 hours, the cells were harvested for RNA preparation. To prepare chronically activated CD8 lymphocytes, we activated the isolated CD8 lymphocytes for 4 days on anti-CD28 and anti-CD3 coated plates and then harvested the cells and expanded them in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and IL-2. The expanded CD8 cells were then activated again with plate bound anti-CD3 and anti-CD28 for 4 days and expanded as before. RNA was isolated 6 and 24 hours after the second activation and after 4 days of the second expansion culture. The isolated NK cells were cultured in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and IL-2 for 4-6 days before RNA was prepared.

To obtain B cells, tonsils were procured from NDRI. The tonsil was cut up with sterile dissecting scissors and then passed through a sieve. Tonsil cells were then spun down and resupended at 10⁶ cells/ml in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco). To activate the cells, we used PWM at 5µg/ml or anti-CD40 (Pharmingen) at approximately 10µg/ml and IL-4 at 5-10ng/ml. Cells were harvested for RNA preparation at 24, 48 and 72 hours.

To prepare the primary and secondary Thl/Th2 and Tr1 cells, six-well Falcon plates were coated overnight with 10µg/ml anti-CD28 (Pharmingen) and 2µg/ml OKT3 (ATCC), and then washed twice with PBS. Umbilical cord blood CD4 lymphocytes (Poietic Systems, German Town, MD) were cultured at 10⁵-10⁶ cells/ml in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and IL-2 (4ng/ml). IL-12 (5ng/ml) and anti-IL4 (1µg/ml) were used to direct to Th1, while IL-4 (5ng/ml) and anti-IFN gamma (1µg/ml) were used to direct to Th2 and IL-10 at 5ng/ml was used to direct to Tr1. After 4-5 days, the activated Th1, Th2 and Tr1 lymphocytes were washed once in DMEM and expanded for 4-7 days in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and IL-2 (1ng/ml). Following this, the activated Th1, Th2 and Tr1 lymphocytes were re-stimulated for 5 days with anti-CD28/OKT3 and cytokines as described above, but with the addition of anti-CD95L (1µg/ml) to prevent apoptosis. After 4-5 days, the Th1, Th2 and Tr1 lymphocytes were washed and then expanded again with IL-2 for 4-7 days. Activated Th1 and Th2 lymphocytes were maintained in this way for a maximum of three cycles. RNA was prepared from primary and secondary Th1, Th2 and Tr1 after 6 and 24 hours following the second and third activations with plate bound anti-CD3 and anti-CD28 mAbs and 4 days into the second and third expansion cultures in Interleukin 2.

The following leukocyte cells lines were obtained from the ATCC: Ramos, EOL-1, KU-812. EOL cells were further differentiated by culture in 0.1mM dbcAMP at 5x10⁵ cells/ml for 8 days, changing the media every 3 days and adjusting the cell concentration to 5x10⁵cells/ml. For the culture of these cells, we used DMEM or RPMI (as recommended by the ATCC), with the addition of 5% FCS (Hyclone), 100µM non essential amino acids (Gibco), 1mM sodium pyruvate (Gibco), mercaptoethanol 5.5x10⁻⁵M (Gibco), 10mM Hepes (Gibco). RNA was either prepared from resting cells or cells activated with PMA at 10ng/ml and ionomycin at 1µg/ml for 6 and 14 hours. Keratinocyte line CCD106 and an airway epithelial tumor line NCI-H292 were also obtained from the ATCC. Both were cultured in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco). CCD1106 cells were activated for 6 and 14 hours with approximately 5 ng/ml TNF alpha and 1ng/ml IL-1 beta, while NCI-H292 cells were activated for 6 and 14 hours with the following cytokines: 5ng/ml IL-4, 5ng/ml IL-9, 5ng/ml IL-13 and 25ng/ml IFN gamma.

For these cell lines and blood cells, RNA was prepared by lysing approximately 10⁷ cells/ml using Trizol (Gibco BRL). Briefly, 1/10 volume of bromochloropropane (Molecular Research Corporation) was added to the RNA sample, vortexed and after 10 minutes at room temperature, the tubes were spun at 14,000 rpm in a Sorvall SS34 rotor. The aqueous phase was removed and placed in a 15ml Falcon Tube. An equal volume of isopropanol was added and left at -20 °C overnight. The precipitated RNA was spun down at 9,000 rpm for 15 min in a Sorvall SS34 rotor and washed in 70% ethanol. The pellet was redissolved in 300µl of RNAse-free water and 35µl buffer (Promega) 5µl DTT, 7µl RNAsin and 8µl DNAse were added. The tube was incubated at 37 °C for 30 minutes to remove contaminating genomic DNA, extracted once with phenol chloroform and reprecipitated with 1/10 volume of 3M sodium acetate and 2 volumes of 100% ethanol. The RNA was spun down and placed in RNAse free water. RNA was stored at -80 °C.

### AI_comprehensive panel_v1.0

The plates for AI_comprehensive panel_v1.0 include two control wells and 89 test samples comprised of cDNA isolated from surgical and postmortem human tissues obtained from the Backus Hospital and Clinomics (Frederick, MD). Total RNA was extracted from tissue samples from the Backus Hospital in the Facility at CuraGen. Total RNA from other tissues was obtained from Clinomics.

Joint tissues including synovial fluid, synovium, bone and cartilage were obtained from patients undergoing total knee or hip replacement surgery at the Backus Hospital. Tissue samples were immediately snap frozen in liquid nitrogen to ensure that isolated RNA was of optimal quality and not degraded. Additional samples of osteoarthritis and rheumatoid arthritis joint tissues were obtained from Clinomics. Normal control tissues were supplied by Clinomics and were obtained during autopsy of trauma victims.

Surgical specimens of psoriatic tissues and adjacent matched tissues were provided as total RNA by Clinomics. Two male and two female patients were selected between the ages of 25 and 47. None of the patients were taking prescription drugs at the time samples were isolated.

Surgical specimens of diseased colon from patients with ulcerative colitis and Crohns disease and adjacent matched tissues were obtained from Clinomics. Bowel tissue from three female and three male Crohn's patients between the ages of 41-69 were used. Two patients were not on prescription medication while the others were taking dexamethasone, phenobarbital, or tylenol. Ulcerative colitis tissue was from three male and four female patients. Four of the patients were taking lebvid and two were on phenobarbital.

Total RNA from post mortem lung tissue from trauma victims with no disease or with emphysema, asthma or COPD was purchased from Clinomics. Emphysema patients ranged in age from 40-70 and all were smokers, this age range was chosen to focus on patients with cigarette-linked emphysema and to avoid those patients with alpha-1antitrypsin deficiencies. Asthma patients ranged in age from 36-75, and excluded smokers to prevent those patients that could also have COPD. COPD patients ranged in age from 35-80 and included both smokers and non-smokers. Most patients were taking corticosteroids, and bronchodilators.

In the labels employed to identify tissues in the AI_comprehensive panel_v1.0 panel, the following abbreviations are used:
AI = Autoimmunity
Syn = Synovial
Normal = No apparent disease
Rep22 /Rep20 = individual patients
RA = Rheumatoid arthritis
Backus = From Backus Hospital
OA = Osteoarthritis
(SS) (BA) (MF) = Individual patients
Adj = Adjacent tissue
Match control = adjacent tissues
-M = Male
-F = Female
COPD = Chronic obstructive pulmonary disease

### Panels 5D and 5I

The plates for Panel 5D and 5I include two control wells and a variety of cDNAs isolated from human tissues and cell lines with an emphasis on metabolic diseases. Metabolic tissues were obtained from patients enrolled in the Gestational Diabetes study. Cells were obtained during different stages in the differentiation of adipocytes from human mesenchymal stem cells. Human pancreatic islets were also obtained.

In the Gestational Diabetes study subjects are young (18 - 40 years), otherwise healthy women with and without gestational diabetes undergoing routine (elective) Caesarean section. After delivery of the infant, when the surgical incisions were being repaired/closed, the obstetrician removed a small sample (<1 cc) of the exposed metabolic tissues during the closure of each surgical level. The biopsy material was rinsed in sterile saline, blotted and fast frozen within 5 minutes from the time of removal. The tissue was then flash frozen in liquid nitrogen and stored, individually, in sterile screw-top tubes and kept on dry ice for shipment to or to be picked up by CuraGen. The metabolic tissues of interest include uterine wall (smooth muscle), visceral adipose, skeletal muscle (rectus) and subcutaneous adipose. Patient descriptions are as follows:
Patient 2 Diabetic Hispanic, overweight, not on insulin
Patient 7-9 Nondiabetic Caucasian and obese (BMI>30)
Patient 10 Diabetic Hispanic, overweight, on insulin
Patient 11 Nondiabetic African American and overweight
Patient 12 Diabetic Hispanic on insulin

Adipocyte differentiation was induced in donor progenitor cells obtained from Osirus (a division of Clonetics/BioWhittaker) in triplicate, except for Donor 3U which had only two replicates. Scientists at Clonetics isolated, grew and differentiated human mesenchymal stem cells (HuMSCs) for CuraGen based on the published protocol found in Mark F. Pittenger, et al., Multilineage Potential of Adult Human Mesenchymal Stem Cells Science Apr 2 1999: 143-147. Clonetics provided Trizol lysates or frozen pellets suitable for mRNA isolation and ds cDNA production. A general description of each donor is as follows:
Donor 2 and 3 U: Mesenchymal Stem cells, Undifferentiated Adipose
Donor 2 and 3 AM: Adipose, AdiposeMidway Differentiated
Donor 2 and 3 AD: Adipose, Adipose Differentiated

Human cell lines were generally obtained from ATCC (American Type Culture Collection), NCI or the German tumor cell bank and fall into the following tissue groups: kidney proximal convoluted tubule, uterine smooth muscle cells, small intestine, liver HepG2 cancer cells, heart primary stromal cells, and adrenal cortical adenoma cells. These cells are all cultured under standard recommended conditions and RNA extracted using the standard procedures. All samples were processed at CuraGen to produce single stranded cDNA.

Panel 5I contains all samples previously described with the addition of pancreatic islets from a 58 year old female patient obtained from the Diabetes Research Institute at the University of Miami School of Medicine. Islet tissue was processed to total RNA at an outside source and delivered to CuraGen for addition to panel 5I.
In the labels employed to identify tissues in the 5D and 5I panels, the following abbreviations are used:
GO Adipose = Greater Omentum Adipose
SK = Skeletal Muscle
UT = Uterus
PL = Placenta
AD = Adipose Differentiated
AM = Adipose Midway Differentiated
U = Undifferentiated Stem Cells

### Panel CNSD.01

The plates for Panel CNSD.01 include two control wells and 94 test samples comprised of cDNA isolated from postmortem human brain tissue obtained from the Harvard Brain Tissue Resource Center. Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains two brains from each of the following diagnoses: Alzheimer's disease, Parkinson's disease, Huntington's disease, Progressive Supernuclear Palsy, Depression, and "Normal controls". Within each of these brains, the following regions are represented: cingulate gyrus, temporal pole, globus palladus, substantia nigra, Brodman Area 4 (primary motor strip), Brodman Area 7 (parietal cortex), Brodman Area 9 (prefrontal cortex), and Brodman area 17 (occipital cortex). Not all brain regions are represented in all cases; *e*.*g*., Huntington's disease is characterized in part by neurodegeneration in the globus palladus, thus this region is impossible to obtain from confirmed Huntington's cases. Likewise Parkinson's disease is characterized by degeneration of the substantia nigra making this region more difficult to obtain. Normal control brains were examined for neuropathology and found to be free of any pathology consistent with neurodegeneration.
In the labels employed to identify tissues in the CNS panel, the following abbreviations are used:
PSP = Progressive supranuclear palsy
Sub Nigra = Substantia nigra
Glob Palladus= Globus palladus
Temp Pole = Temporal pole
Cing Gyr = Cingulate gyrus
BA 4 = Brodman Area 4

### Panel CNS_Neurodegeneration_V1.0

The plates for Panel CNS_Neurodegeneration_V1.0 include two control wells and 47 test samples comprised of cDNA isolated from postmortem human brain tissue obtained from the Harvard Brain Tissue Resource Center (McLean Hospital) and the Human Brain and Spinal Fluid Resource Center (VA Greater Los Angeles Healthcare System). Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains six brains from Alzheimer's disease (AD) patients, and eight brains from "Normal controls" who showed no evidence of dementia prior to death. The eight normal control brains are divided into two categories: Controls with no dementia and no Alzheimer's like pathology (Controls) and controls with no dementia but evidence of severe Alzheimer's like pathology, (specifically senile plaque load rated as level 3 on a scale of 0-3; 0 = no evidence of plaques, 3 = severe AD senile plaque load). Within each of these brains, the following regions are represented: hippocampus, temporal cortex (Brodman Area 21), parietal cortex (Brodman area 7), and occipital cortex (Brodman area 17). These regions were chosen to encompass all levels of neurodegeneration in AD. The hippocampus is a region of early and severe neuronal loss in AD; the temporal cortex is known to show neurodegeneration in AD after the hippocampus; the parietal cortex shows moderate neuronal death in the late stages of the disease; the occipital cortex is spared in AD and therefore acts as a "control" region within AD patients. Not all brain regions are represented in all cases.
In the labels employed to identify tissues in the CNS_Neurodegeneration_V1.0 panel, the following abbreviations are used:
AD = Alzheimer's disease brain; patient was demented and showed AD-like pathology upon autopsy
Control = Control brains; patient not demented, showing no neuropathology
Control (Path) = Control brains; pateint not demented but showing sever AD-like pathology
SupTemporal Ctx = Superior Temporal Cortex
Inf Temporal Ctx = Inferior Temporal Cortex

### A. CG100689-01: LRR Protein

Expression of gene CG100689-01 was assessed using the primer-probe set Ag4186, described in Table AA. Results of the RTQ-PCR runs are shown in Tables AB and AC.

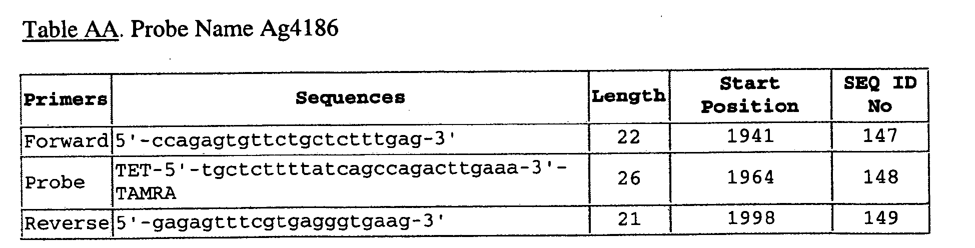

**Table AB. General_screening_panel_v1.4**

| **Tissue Name** | **Rel. Exp.(%) Ag4186, Run 221154078** | **Tissue Name** | **Rel. Exp.(%) Ag4186, Run 221154078** |
|---|---|---|---|
| Adipose | 0.0 | Renal ca. TK-10 | 0.0 |
| Melanoma* Hs688(A).T | 0.0 | Bladder | 0.0 |
| Melanoma* Hs688(B).T | 0.0 | Gastric ca. (liver met.) NCI-N87 | 0.0 |
| Melanoma* M14 | 2.3 | Gastric ca. KATO III | 15.9 |
| Melanoma* LOXIMVI | 0.0 | Colon ca. SW-948 | 0.0 |
| Melanoma* SK-MEL-5 | 0.0 | Colon ca. SW480 | 12.3 |
| Squamous cell carcinoma SCC-4 | 2.2 | Colon ca.* (SW480 met) SW620 | 0.0 |
| Testis Pool | **100.0** | Colon ca. HT29 | 0.0 |
| Prostate ca.* (bone met) PC-3 | 0.0 | Colon ca. HCT-116 | 28.3 |
| Prostate Pool | 0.0 | Colon ca. CaCo-2 | 3.0 |
| Placenta | 0.0 | Colon cancer tissue | 0.0 |
| Uterus Pool | 0.0 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 30.1 | Colon Pool | 1.7 |
| Ovarian ca. OVCAR-5 | 0.0 | Small Intestine Pool | 1.7 |
| Ovarian ca. IGROV-1 | 0.0 | Stomach Pool | 0.0 |
| Ovarian ca. OVCAR-8 | 0.0 | Bone Marrow Pool | 0.0 |
| Ovary | 0.0 | Fetal Heart | 0.0 |
| Breast ca. MCF-7 | 1.7 | Heart Pool | 1.3 |
| Breast ca. MDA-MB-231 | 0.0 | Lymph Node Pool | 3.2 |
| Breast ca. BT 549 | 0.0 | Fetal Skeletal Muscle | 0.0 |
| Breast ca. T47D | 0.0 | Skeletal Muscle Pool | 0.0 |
| Breast ca. MDA-N | 0.0 | Spleen Pool | 12.2 |
| Breast Pool | 0.0 | Thymus Pool | 0.0 |
| Trachea | 1.7 | CNS cancer (glio/astro) U87-MG | 0.0 |
| Lung | 0.0 | CNS cancer (glio/astro) U-118-MG | 0.0 |
| Fetal Lung | 2.6 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 34.6 | CNS cancer (glio) SNB-19 | 1.6 |
| Lung ca. SHP-77 | 10.5 | CNS cancer (glio) SF-295 | 0.0 |
| Lung ca. A549 | 0.0 | Brain (Amygdala) Pool | 5.5 |
| Lung ca. NCI-H526 | 0.0 | Brain (cerebellum) | 4.8 |
| Lung ca. NCI-H23 | 2.4 | Brain (fetal) | 0.0 |
| Lung ca. NCI-H460 | 0.0 | Brain (Hippocampus) Pool | 9.4 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 4.4 |
| Fetal Liver | 1.7 | Brain (whole) | 0.0 |
| Liver ca. HepG2 | 0.0 | Spinal Cord Pool | 2.7 |
| Kidney Pool | 2.6 | Adrenal Gland | 0.0 |
| Fetal Kidney | 0.0 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 2.4 |

**Table AC. Panel 4.1D**

| **Tissue Name** | **Rel. Exp.(%) Ag4186, Run 182086756** | **Tissue Name** | **Rel. Exp.(%) Ag4186, Run 182086756** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.5 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha + IL-1beta | 0.0 |
| CD45RA CD4 lymphocyte act | 0.0 | Coronery artery SMC rest | 0.0 |
| CD45RO CD4 lymphocyte act | 0.5 | Coronery artery SMC TNFalpha+IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 0.4 |
| LAK cells IL-2+IL-12 | 0.0 | NCI-H292 none | 0.0 |
| LAK cells IL-2+IFN gamma | 0.0 | NCI-H292 IL-4 | 0.0 |
| LAK cells IL-2+ IL-18 | 0.0 | NCI-H292 IL-9 | 0.0 |
| LAK cells PMA/ionomycin | 0.0 | NCI-H292 IL-13 | 0.0 |
| NK Cells IL-2 rest | 0.0 | NCI-H292 IFN gamma | 0.0 |
| Two Way MLR 3 day | 0.0 | HPAEC none | 0.0 |
| Two Way MLR 5 day | 0.0 | HPAEC TNF alpha + IL-1 beta | 0.0 |
| Two Way MLR 7 day | 0.0 | Lung fibroblast none | 0.0 |
| PBMC rest | 0.0 | Lung fibroblast TNF alpha + IL-1 beta | 0.4 |
| PBMC PWM | 0.0 | Lung fibroblast IL-4 | 0.0 |
| PBMC PHA-L | 0.0 | Lung fibroblast IL-9 | 0.0 |
| Ramos (B cell) none | 0.4 | Lung fibroblast IL-13 | 0.0 |
| Ramos (B cell) ionomycin | 0.5 | Lung fibroblast IFN gamma | 1.1 |
| B lymphocytes PWM | 0.0 | Dermal fibroblast CCD1070 rest | 0.0 |
| B lymphocytes CD40L and IL-4 | 16.5 | Dermal fibroblast CCD 1070 TNF alpha | 0.0 |
| EOL-1 dbcAMP | 1.0 | Dermal fibroblast CCD1070 IL-1 beta | 0.0 |
| EOL-1 dbcAMP PMA/ionomycin | 3.8 | Dermal fibroblast IFN gamma | 3.2 |
| Dendritic cells none | 0.0 | Dermal fibroblast IL-4 | 0.0 |
| Dendritic cells LPS | 0.0 | Dermal Fibroblasts rest | 1.0 |
| Dendritic cells anti-CD40 | 0.0 | Neutrophils TNFa+LPS | 0.0 |
| Monocytes rest | 0.0 | Neutrophils rest | 2.3 |
| Monocytes LPS | 0.0 | Colon | 1.4 |
| Macrophages rest | 0.4 | Lung | 1.6 |
| Macrophages LPS | 0.0 | Thymus | 19.6 |
| HUVEC none | 0.6 | Kidney | **100.0** |
| HUVEC starved | 0.0 | | |

**General_screening_panel_v1.4 Summary:** Ag4186 Expression of this gene is restricted to the testis (CT=33.7). Thus, expression of this gene could be used to differentiate between this sample and other samples on this panel and as a marker of testicular tissue. Therapeutic modulation of the expression or function of this gene may be useful in the treatment of male infertility and hypogonadism.

**Panel 4.1D Summary:** Ag4186 Expression of this gene is restricted to the kidney, thymus, and activated B lymphocytes (CTs=30-33). Thus, expression of this gene could be used to differentiate between the kidney derived sample and other samples on this panel and as a marker of kidney tissue. Therapeutic modulation of the expression or function of this gene could modulate kidney function and be important in the treatment of inflammatory or autoimmune diseases that affect the kidney, including lupus and glomerulonephritis.

### B. CG100760-01: LRR Protein

Expression of gene CG100760-01 was assessed using the primer-probe set Ag4192, described in Table BA. Results of the RTQ-PCR runs are shown in Tables BB, BC and BD.

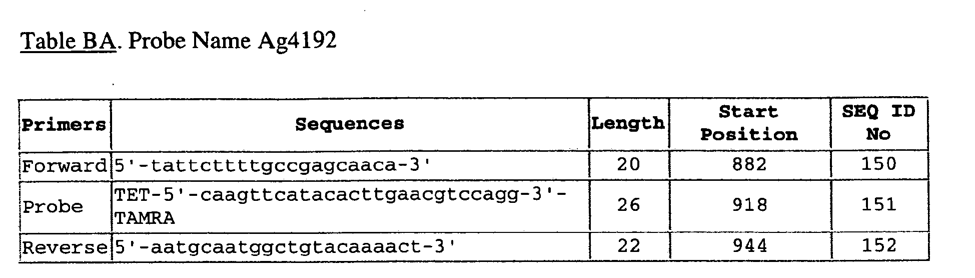

**Table BB. General_screening_panel_v1.4**

| **Tissue Name** | **Rel. Exp.(%) Ag4192, Run 221157609** | **Tissue Name** | **Rel. Exp.(%) Ag4192, Run 221157609** |
|---|---|---|---|
| Adipose | 0.0 | Renal ca. TK-10 | 0.0 |
| Melanoma* Hs688(A).T | 0.2 | Bladder | 0.0 |
| Melanoma* Hs688(B).T | 0.0 | Gastric ca. (liver met.) NCI-N87 | 0.0 |
| Melanoma* M 14 | 3.3 | Gastric ca. KATO III | 4.8 |
| Melanoma* LOXIMVI | 0.0 | Colon ca. SW-948 | 1.0 |
| Melanoma* SK-MEL-5 | 2.8 | Colon ca. SW480 | 47.3 |
| Squamous cell carcinoma SCC-4 | 0.0 | Colon ca.* (SW480 met) SW620 | 5.7 |
| Testis Pool | 4.7 | Colon ca. HT29 | 1.0 |
| Prostate ca.* (bone met) PC-3 | 7.8 | Colon ca. HCT-116 | 5.3 |
| Prostate Pool | 0.0 | Colon ca. CaCo-2 | 3.7 |
| Placenta | 0.0 | Colon cancer tissue | 0.1 |
| Uterus Pool | 0.0 | Colon ca. SW1116 | 0.0 |
| Ovarian ca. OVCAR-3 | 0.3 | Colon ca. Colo-205 | 0.0 |
| Ovarian ca. SK-OV-3 | 0.0 | Colon ca. SW-48 | 0.0 |
| Ovarian ca. OVCAR-4 | 1.4 | Colon Pool | 0.1 |
| Ovarian ca. OVCAR-5 | 0.2 | Small Intestine Pool | 0.8 |
| Ovarian ca. IGROV-1 | 0.0 | Stomach Pool | 0.4 |
| Ovarian ca. OVCAR-8 | 1.3 | Bone Marrow Pool | 0.0 |
| Ovary | 3.1 | Fetal Heart | 0.0 |
| Breast ca. MCF-7 | 0.5 | Heart Pool | 0.3 |
| Breast ca. MDA-MB-231 | 1.2 | Lymph Node Pool | 0.7 |
| Breast ca. BT 549 | 0.1 | Fetal Skeletal Muscle | 0.0 |
| Breast ca. T47D | 0.0 | Skeletal Muscle Pool | 0.0 |
| Breast ca. MDA-N | 7.5 | Spleen Pool | 0.6 |
| Breast Pool | 0.0 | Thymus Pool | 0.8 |
| Trachea | 0.6 | CNS cancer (glio/astro) U87-MG | 12.8 |
| Lung | 0.2 | CNS cancer (glio/astro) U-118-MG | 15.2 |
| Fetal Lung | 0.0 | CNS cancer (neuro;met) SK-N-AS | 0.5 |
| Lung ca. NCI-N417 | 0.7 | CNS cancer (astro) SF-539 | 0.3 |
| Lung ca. LX-1 | 0.6 | CNS cancer (astro) SNB-75 | 0.9 |
| Lung ca. NCI-H146 | 7.7 | CNS cancer (glio) SNB-19 | 0.3 |
| Lung ca. SHP-77 | **100.0** | CNS cancer (glio) SF-295 | 14.4 |
| Lung ca. A549 | 0.0 | Brain (Amygdala) Pool | 0.0 |
| Lung ca. NCI-H526 | 33.0 | Brain (cerebellum) | 0.3 |
| Lung ca. NCI-H23 | 1.5 | Brain (fetal) | 0.0 |
| Lung ca. NCI-H460 | 0.0 | Brain (Hippocampus) Pool | 0.1 |
| Lung ca. HOP-62 | 0.0 | Cerebral Cortex Pool | 0.0 |
| Lung ca. NCI-H522 | 4.8 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.8 | Spinal Cord Pool | 0.0 |
| Kidney Pool | 0.3 | Adrenal Gland | 0.0 |
| Fetal Kidney | 2.9 | Pituitary gland Pool | 0.1 |
| Renal ca. 786-0 | 0.0 | Salivary Gland | 0.2 |
| Renal ca. A498 | 0.6 | Thyroid (female) | 0.6 |
| Renal ca. ACHN | 0.0 | Pancreatic ca. CAPAN2 | 0.3 |
| Renal ca. UO-31 | 0.0 | Pancreas Pool | 0.0 |

**Table BC. Panel 4.1D**

| **Tissue Name** | **Rel. Exp.(%) Ag4192, Run 175226746** | **Tissue Name** | **Rel. Exp.(%) Ag4192, Run 175226746** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha + IL-1 beta | 0.0 |
| CD45RA CD4 lymphocyte act | 0.9 | Coronery artery SMC rest | 0.0 |
| CD45RO CD4 lymphocyte act | 2.9 | Coronery artery SMC TNFalpha + IL-1beta | 0.0 |
| CD8 lymphocyte act | 0.0 | Astrocytes rest | 0.0 |
| Secondary CD8 lymphocyte rest | 2.1 | Astrocytes TNFalpha + IL-beta | 0.0 |
| Secondary CD8 lymphocyte act | 0.4 | KU-812 (Basophil) rest | 0.0 |
| CD4 lymphocyte none | 0.0 | KU-812 (Basophil) PMA/ionomycin | 1.0 |
| 2ry Th1/Th2/Tr1_anti-CD95 CH 11 | 0.0 | CCD 1106 (Keratinocytes) none | 0.0 |
| LAK cells rest | 0.0 | CCD1106 (Keratinocytes) TNFalpha + IL-1beta | 0.2 |
| LAK cells IL-2 | 0.0 | Liver cirrhosis | 0.0 |
| LAK cells IL-2+IL-12 | 0.0 | NCI-H292 none | 0.0 |
| LAK cells IL-2+IFN gamma | 0.9 | NCI-H292 IL-4 | 0.0 |
| LAK cells IL-2+IL-18 | 0.9 | NCI-H292 IL-9 | 0.9 |
| LAK cells PMA/ionomycin | 0.0 | NCI-H292 IL-13 | 0.0 |
| NK Cells IL-2 rest | 0.0 | NCI-H292 IFN gamma | 0.0 |
| Two Way MLR 3 day | 0.0 | HPAEC none | 0.0 |
| Two Way MLR 5 day | 0.0 | HPAEC TNF alpha + IL-1 beta | 0.0 |
| Two Way MLR 7 day | 0.0 | Lung fibroblast none | 0.0 |
| PBMC rest | 0.0 | Lung fibroblast TNF alpha + IL-1 beta | 0.6 |
| PBMC PWM | 0.0 | Lung fibroblast IL-4 | 0.0 |
| PBMC PHA-L | 2.1 | Lung fibroblast IL-9 | 0.0 |
| Ramos (B cell) none | 58.6 | Lung fibroblast IL-13 | 0.0 |
| Ramos (B cell) ionomycin | **100.0** | Lung fibroblast IFN gamma | 0.0 |
| B lymphocytes PWM | 2.6 | Dermal fibroblast CCD 1070 rest | 0.0 |
| B lymphocytes CD40L and IL-4 | 4.7 | Dermal fibroblast CCD1070 TNF alpha | 0.0 |
| EOL-1 dbcAMP | 0.3 | Dermal fibroblast CCD1070 IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 0.0 |
| Dendritic cells anti-CD40 | 0.0 | Neutrophils TNFa+LPS | 0.0 |
| Monocytes rest | 0.0 | Neutrophils rest | 0.0 |
| Monocytes LPS | 0.0 | Colon | 0.9 |
| Macrophages rest | 0.0 | Lung | 0.8 |
| Macrophages LPS | 0.0 | Thymus | 7.5 |
| HUVEC none | 0.0 | Kidney | 36.1 |
| HUVEC starved | 0.0 | | |

**Table BD. general oncology screening panel_v_2.4**

| **Tissue Name** | **Rel.Exp.(%) Ag4192, Run 268689533** | **Tissue Name** | **Rel.Exp.(%)Ag4192,Run 268689533** |
|---|---|---|---|
| Colon cancer 1 | 0.0 | Bladder cancer NAT 2 | 0.0 |
| Colon cancer NAT 1 | 0.8 | Bladder cancer NAT 3 | 0.0 |
| Colon cancer 2 | 0.0 | Bladder cancer NAT 4 | 0.0 |
| Colon cancer NAT 2 | 2.0 | Adenocarcinoma of the prostate 1 | 4.7 |
| Colon cancer 3 | 3.3 | Adenocarcinoma of the prostate 2 | 0.0 |
| Colon cancer NAT 3 | 2.4 | Adenocarcinoma of the prostate 3 | 1.3 |
| Colon malignant cancer 4 | 0.0 | Adenocarcinoma of the prostate 4 | 0.0 |
| Colon normal adjacent tissue 4 | 0.0 | Prostate cancer NAT 5 | 0.0 |
| Lung cancer 1 | 0.0 | Adenocarcinoma of the prostate 6 | 1.8 |
| Lung NAT 1 | 0.0 | Adenocarcinoma of the prostate 7 | 0.0 |
| Lung cancer 2 | 6.7 | Adenocarcinoma of the prostate 8 | 1.2 |
| Lung NAT 2 | 0.0 | Adenocarcinoma of the prostate 9 | 0.0 |
| Squamous cell carcinoma 3 | 4.3 | Prostate cancer NAT 10 | 0.0 |
| Lung NAT 3 | 0.0 | Kidney cancer 1 | 1.8 |
| metastatic melanoma | 5.0 | KidneyNAT 1 | 3.2 |
| Melanoma 2 | 0.0 | Kidney cancer 2 | **100.0** |
| Melanoma 3 | 0.0 | Kidney NAT 2 | 10.7 |
| metastatic melanoma 4 | 2.4 | Kidney cancer 3 | 0.9 |
| metastatic melanoma 5 | 20.0 | Kidney NAT 3 | 2.1 |
| Bladder cancer 1 | 0.0 | Kidney cancer 4 | 12.2 |
| Bladder cancer NAT 1 | 0.0 | Kidney NAT 4 | 5.4 |
| Bladder cancer 2 | 0.0 | | |

**General_screening_panel_v1.4 Summary:** Ag4192 Highest expression of the CG100760-01 is seen in a lung cancer cell line (CT=29.6). Moderate levels of expression are also seen in a cluster of cell lines derived from lung, colon, and brain cancers. Thus, expression of this gene could be used to differentiate the lung cancer cell line sample from other samples on this panel and as a marker of lung, colon and brain cancer. Furthermore, this restricted pattern of expression suggests that therapeutic modulation of the expression or function of this gene may be useful in the treatment of these cancers.

**Panel 4.1D Summary:** Ag4192 Expression of this gene is limited to a few samples on this panel, with highest expression of the CG100760-01 gene in Ramos B cells stimulated with ionomycin (CT=30.4). Lower but still significant levels of expression are seen in untreated Ramos B cells, activated B lymphocytes, kidney and thymus. B cells represent a principle component of immunity and contribute to the immune response in a number of important functional roles, including antibody production. Production of antibodies against self-antigens is a major component in autoimmune disorders. Since B cells play an important role in autoimmunity, inflammatory processes and inflammatory cascades, therapeutic modulation of this gene product may reduce or eliminate the symptoms of patients suffering from asthma, allergies, chronic obstructive pulmonary disease, emphysema, Crohn's disease, ulcerative colitis, rheumatoid arthritis, psoriasis, osteoarthritis, systemic lupus erythematosus and other autoimmune disorders.

**general oncology screening panel_v_2.4 Summary:** Ag4192 Expression of the CG100760-01 gene is limited in kidney cancer (CT=32) and melanoma on this panel. This expression in cancer derived samples is consistent with expression seen in Panel 1.4. Thus, expression of this gene could be used to differentiate the kidney cancer sample from other samples on this panel and as a marker of kidney cancer. Furthermore, therapeutic modulation of the expression or function of this gene may be useful in the treatment of kidney cancer.

### C. CG101068-01: Claudin-9

Expression of gene CG101068-01 was assessed using the primer-probe set Ag4202, described in Table CA. Results of the RTQ-PCR runs are shown in Table CB.

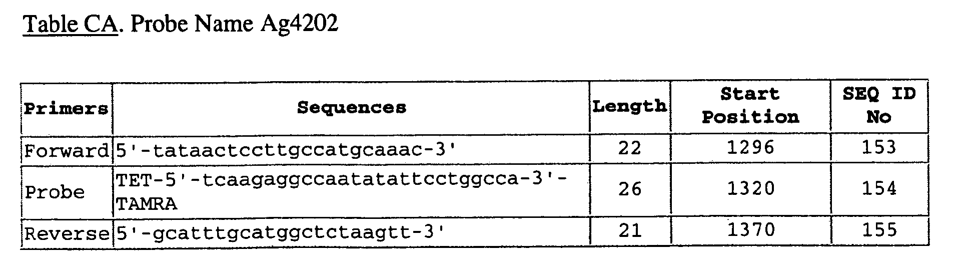

**Table CB. General_screening_panel_v1.4**

| **Tissue Name** | **Rel. Exp.(%) Ag4202, Run 221178754** | **Tissue Name** | **Rel. Exp.(%) Ag4202, Run 221178754** |
|---|---|---|---|
| Adipose | 0.4 | Renal ca. TK-10 | 0.3 |
| Melanoma* Hs688(A).T | 0.0 | Bladder | 9.2 |
| Melanoma* Hs688(B).T | 0.0 | Gastric ca. (liver met.) NCI-N87 | 47.6 |
| Melanoma* M14 | 0.0 | Gastric ca. KATO III | **100.0** |
| Melanoma* LOXIMVI | 0.0 | Colon ca. SW-948 | 0.9 |
| Melanoma* SK-MEL-5 | 0.7 | Colon ca. SW480 | 7.4 |
| Squamous cell carcinoma SCC-4 | 0.0 | Colon ca.* (SW480 met) SW620 | 0.0 |
| Testis Pool | 0.0 | Colon ca. HT29 | 0.0 |
| Prostate ca.* (bone met) PC-3 | 0.0 | Colon ca. HCT-116 | 2.1 |
| Prostate Pool | 0.0 | Colon ca. CaCo-2 | 14.6 |
| Placenta | 3.6 | Colon cancer tissue | 7.5 |
| Uterus Pool | 0.0 | Colon ca. SW1116 | 0.0 |
| Ovarian ca. OVCAR-3 | 0.7 | Colon ca. Colo-205 | 0.0 |
| Ovarian ca. SK-OV-3 | 6.7 | Colon ca. SW-48 | 0.5 |
| Ovarian ca. OVCAR-4 | 0.0 | Colon Pool | 0.0 |
| Ovarian ca. OVCAR-5 | 12.9 | Small Intestine Pool | 0.0 |
| Ovarian ca. IGROV-1 | 2.3 | Stomach Pool | 0.0 |
| Ovarian ca. OVCAR-8 | 0.3 | Bone Marrow Pool | 0.0 |
| Ovary | 1.8 | Fetal Heart | 0.0 |
| Breast ca. MCF-7 | 5.9 | Heart Pool | 0.0 |
| Breast ca. MDA-MB-231 | 12.3 | Lymph Node Pool | 0.0 |
| Breast ca. BT 549 | 0.0 | Fetal Skeletal Muscle | 0.0 |
| Breast ca. T47D | 30.1 | Skeletal Muscle Pool | 0.0 |
| Breast ca. MDA-N | 0.0 | Spleen Pool | 0.0 |
| Breast Pool | 0.0 | Thymus Pool | 0.0 |
| Trachea | 3.1 | CNS cancer (glio/astro) U87-MG | 0.0 |
| Lung | 0.0 | CNS cancer (glio/astro) U-118-MG | 0.0 |
| Fetal Lung | 4.2 | CNS cancer (neuro;met) SK-N-AS | 0.0 |
| Lung ca. NCI-N417 | 0.0 | CNS cancer (astro) SF-539 | 0.5 |
| Lung ca. LX-1 | 1.9 | CNS cancer (astro) SNB-75 | 11.3 |
| Lung ca. NCI-H146 | 0.0 | CNS cancer (glio) SNB-19 | 2.6 |
| Lung ca. SHP-77 | 0.1 | CNS cancer (glio) SF-295 | 0.0 |
| Lung ca. A549 | 8.1 | Brain (Amygdala) Pool | 0.0 |
| Lung ca. NCI-H526 | 0.0 | Brain (cerebellum) | 0.0 |
| Lung ca. NCI-H23 | 4.0 | Brain (fetal) | 0.0 |
| Lung ca. NCI-H460 | 0.0 | Brain (Hippocampus) Pool | 0.0 |
| Lung ca. HOP-62 | 0.5 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 1.0 | Brain (whole) | 0.0 |
| Liver ca. HepG2 | 0.0 | Spinal Cord Pool | 0.0 |
| Kidney Pool | 0.0 | Adrenal Gland | 0.0 |
| Fetal Kidney | 0.0 | Pituitary gland Pool | 0.0 |
| Renal ca. 786-0 | 0.0 | Salivary Gland | 0.1 |
| Renal ca. A498 | 6.8 | Thyroid (female) | 3.5 |
| Renal ca. ACHN | 3.9 | Pancreatic ca. CAPAN2 | 6.7 |
| Renal ca. UO-31 | 0.0 | Pancreas Pool | 0.1 |

**General_screening_panel_v1.4 Summary:** Ag4202 Highest expression of the CG101068-01 gene is seen in a gastric cancer cell line (CT=26.5). Moderate levels of expression are seen in cell lines derived from pancreatic, brain, renal, lung, colon, breast and ovarian cancers. Thus, expression of this gene may be used to differentiate the gastric cancer cell line from other samples on this panel and as a marker of these cancers. This gene encodes a protein with homology to claudin, a family of proteins that are integral components of the tight junction. Members of this family have been shown to be upregulated in pancreatic cancer and colon cancer and in the former case proposed as novel targets for the treatment of this disease (Michl P. Gastroenterology 2001 Sep;121(3):678-84; Miwa, N. Oncol Res 2001;12(11-12):469-76) Therefore, therapeutic modulation of the expression or function of this protein may be of use in the treatment of these cancers.

Claudin 11 has been shown to be a component of the CNS myelin and has been implicated in the regulation of growth and differentiation via signal transduction pathways.

Furthermore, evidence has been presented that shows that claudin 11 may be involved in the autoantigen that is responsible for the development of autoimmune demyelinating disease.(Bronstein JM. J Neurosci Res 2000 Mar 15;59(6):706-11). Therefore, therapeutic modulation of the expression or function of this putative claudin may be of use in the treatment of demyelinating diseases such as multiple sclerosis and in restoring normal function to the CNS.

### D. CG101231-01 and CG101231-02: Integral membrane protein

Expression of gene CG101231-01 and CG101231-02 was assessed using the primer-probe sets Ag4208 and Ag4997, described in Tables DA and DB. Results of the RTQ-PCR runs are shown in Tables DC and DD.

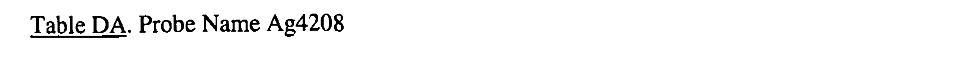

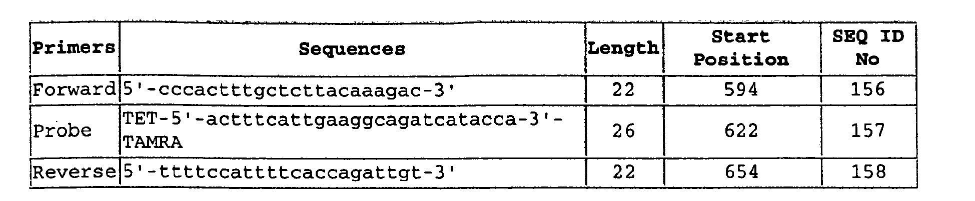

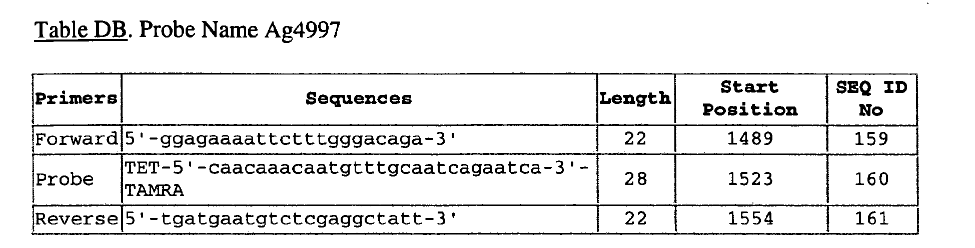

**Table DC. General_screening_panel_v1.4**

| **Tissue Name** | **Rel. Exp.(%) Ag4997, Run 222456716** | **Tissue Name** | **Rel. Exp.(%) Ag4997, Run 222456716** |
|---|---|---|---|
| Adipose | 1.2 | Renal ca. TK-10 | 13.1 |
| Melanoma* Hs688(A).T | 26.4 | Bladder | 3.2 |
| Melanoma* Hs688(B).T | 30.6 | Gastric ca. (liver met.) NCI-N87 | 36.3 |
| Melanoma* M14 | 8.2 | Gastric ca. KATO III | 29.3 |
| Melanoma* LOXIMVI | 9.0 | Colon ca. SW-948 | 4.0 |
| Melanoma* SK-MEL-5 | 4.5 | Colon ca. SW480 | 33.4 |
| Squamous cell carcinoma SCC-4 | 11.2 | Colon ca.* (SW480 met) SW620 | 13.5 |
| Testis Pool | 2.8 | Colon ca. HT29 | 0.6 |
| Prostate ca.* (bone met) PC-3 | 20.3 | Colon ca. HCT-116 | 10.8 |
| Prostate Pool | 12.4 | Colon ca. CaCo-2 | 2.7 |
| Placenta | 0.3 | Colon cancer tissue | 5.8 |
| Uterus Pool | 1.4 | Colon ca. SW1116 | 8.9 |
| Ovarian ca. OVCAR-3 | 10.4 | Colon ca. Colo-205 | 5.9 |
| Ovarian ca. SK-OV-3 | **100.0** | Colon ca. SW-48 | 1.4 |
| Ovarian ca. OVCAR-4 | 4.7 | Colon Pool | 5.6 |
| Ovarian ca. OVCAR-5 | 44.8 | Small Intestine Pool | 4.0 |
| Ovarian ca. IGROV-1 | 6.3 | Stomach Pool | 3.3 |
| Ovarian ca. OVCAR-8 | 10.4 | Bone Marrow Pool | 2.0 |
| Ovary | 3.0 | Fetal Heart | 17.2 |
| Breast ca. MCF-7 | 11.0 | Heart Pool | 2.1 |
| Breast ca. MDA-MB-231 | 22.4 | Lymph Node Pool | 6.9 |
| Breast ca. BT 549 | 67.8 | Fetal Skeletal Muscle | 2.0 |
| Breast ca. T47D | 66.4 | Skeletal Muscle Pool | 0.5 |
| Breast ca. MDA-N | 6.9 | Spleen Pool | 1.3 |
| Breast Pool | 5.5 | Thymus Pool | 5.3 |
| Trachea | 5.3 | CNS cancer (glio/astro) U87-MG | 21.3 |
| Lung | 1.4 | CNS cancer (glio/astro) U-118-MG | 17.7 |
| Fetal Lung | 29.1 | CNS cancer (neuro;met) SK-N-AS | 12.3 |
| Lung ca. NCI-N417 | 2.4 | CNS cancer (astro) SF-539 | 18.0 |
| Lung ca. LX-1 | 29.5 | CNS cancer (astro) SNB-75 | 49.7 |
| Lung ca. NCI-H146 | 12.0 | CNS cancer (glio) SNB-191 | 6.1 |
| Lung ca. SHP-77 | 17.8 | CNS cancer (glio) SF-295 | 20.6 |
| Lung ca. A549 | 11.9 | Brain (Amygdala) Pool | 9.3 |
| Lung ca. NCI-H526 | 5.0 | Brain (cerebellum) | 6.9 |
| Lung ca. NCI-H23 | 11.3 | Brain (fetal) | 19.6 |
| Lung ca. NCI-H460 | 1.1 | Brain (Hippocampus) Pool | 11.7 |
| Lung ca. HOP-62 | 8.5 | Cerebral Cortex Pool | 14.9 |
| Lung ca. NCI-H522 | 19.6 | Brain (Substantia nigra) Pool | 12.2 |
| Liver | 0.0 | Brain (Thalamus) Pool | 17.2 |
| Fetal Liver | 21.5 | Brain (whole) | 10.4 |
| Liver ca. HepG2 | 0.0 | Spinal Cord Pool | 18.9 |
| Kidney Pool | 4.8 | Adrenal Gland | 0.7 |
| Fetal Kidney | 12.9 | Pituitary gland Pool | 3.3 |
| Renal ca. 786-0 | 11.6 | Salivary Gland | 1.0 |
| Renal ca. A498 | 5.4 | Thyroid (female) | 4.0 |
| Renal ca. ACHN | 4.9 | Pancreatic ca. CAPAN2 | 52.9 |
| Renal ca. UO-31 | 7.8 | Pancreas Pool | 5.3 |

**Table DD. Panel 4.1D**

| **Tissue Name** | **Rel. Exp.(%) Ag4997, Run 225428030** | **Tissue Name** | **Rel. Exp.(%) Ag4997, Run 225428030** |
|---|---|---|---|
| Secondary Th1 act | 2.7 | HUVEC IL-1beta | 3.2 |
| Secondary Th2 act | 3.8 | HUVEC IFN gamma | 4.0 |
| Secondary Tr act | 4.5 | HUVEC TNF alpha + IFN gamma | 1.9 |
| Secondary Th1 rest | 1.8 | HUVEC TNF alpha + IL4 | 3.0 |
| Secondary Th2 rest | 2.0 | HUVEC IL-11 | 11.9 |
| SecondaryTrl rest | 1.4 | Lung Microvascular EC none | 8.8 |
| Primary Th1 act Primary Th1 act | 1.9 | Lung Microvascular EC TNFalpha + IL-1 beta | 3.9 |
| Primary Th2 act | 3.9 | Microvascular Dermal EC none | 7.5 |
| Primary Tr1 act | 3.5 | Microsvasular Dermal EC TNFalpha + IL-1 beta | 3.1 |
| Primary Th1 rest | 1.7 | Bronchial epithelium TNFalpha + IL 1 beta | 38.7 |
| Primary Th2 rest | 3.9 | Small airway epithelium none | 14.8 |
| Primary Tr1 rest | 8.5 | Small airway epithelium TNFalpha + IL-1beta | 24.0 |
| CD45RA CD4 lymphocyte act | 7.9 | Coronery artery SMC rest | 13.3 |
| CD45RO CD4 lymphocyte act | 1.6 | Coronery artery SMC TNFalpha + IL-1beta | 12.9 |
| CD8 lymphocyte act | 1.4 | Astrocytes rest | 10.4 |
| Secondary CD8 lymphocyte rest | 1.7 | Astrocytes TNFalpha + IL-I beta | 12.0 |
| Secondary CD8 lymphocyte act | 0.4 | KU-812 (Basophil) rest | 78.5 |
| CD4 lymphocyte none | 0.2 | KU-812 (Basophil) PMA/ionomycin | **100.0** |
| 2ry Th1/Th2/Tr1_anti-CD95 CH11 | 2.0 | CCD1106 (Keratinocytes) none | 31.2 |
| LAK cells rest | 1.6 | CCD1106 (Keratinocytes) TNFalpha + IL-1beta | 17.0 |
| LAK cells IL-2 | 3.0 | Liver cirrhosis | 1.9 |
| LAK cells IL-2+IL-12 | 1.0 | NCI-H292 none | 9.5 |
| LAK cells IL-2+IFN gamma | 1.6 | NCI-H292 IL-4 | 12.1 |
| LAK cells IL-2+ IL-18 | 1.3 | NCI-H292IL-9 | 14.8 |
| LAK cells PMA/ionomycin | 1.3 | NCI-H292 IL-13 | 11.7 |
| NK Cells IL-2 rest | 3.2 | NCI-H292 IFN gamma | 8.5 |
| Two Way MLR 3 day | 0.5 | HPAEC none | 4.9 |
| Two Way MLR 5 day | 2.2 | HPAEC TNF alpha + IL-1 beta | 3.8 |
| Two Way MLR 7 day | 0.4 | Lung fibroblast none | 13.4 |
| PBMC rest | 1.8 | Lung fibroblast TNF alpha + IL-1 beta | 17.2 |
| PBMC PWM | 0.5 | Lung fibroblast IL-4 | 11.3 |
| PBMC PHA-L | 1.4 | Lung fibroblast IL-9 | 22.7 |
| Ramos (B cell) none | 0.0 | Lung fibroblast IL-13 | 8.9 |
| Ramos (B cell) ionomycin | 0.0 | Lung fibroblast IFN gamma | 8.7 |
| B lymphocytes PWM | 1.9 | Dermal fibroblast CCD 1070 rest | 24.8 |
| B lymphocytes CD40L and IL-4 | 3.0 | Dermal fibroblast CCD1070 TNF alpha | 13.8 |
| EOL-1 dbcAMP | 0.1 | Dermal fibroblast CCD 1070 IL-1 beta | 8.2 |
| EOL-1 dbcAMP PMA/ionomycin | 0.0 | Dermal fibroblast IFN gamma | 7.9 |
| Dendritic cells none | 3.1 | Dermal fibroblast IL-4 | 15.7 |
| Dendritic cells LPS | 5.2 | Dermal Fibroblasts rest | 12.9 |
| Dendritic cells anti-CD40 | 3.7 | Neutrophils TNFa+LPS | 3.3 |
| Monocytes rest | 3.8 | Neutrophils rest | 20.2 |
| Monocytes LPS | 1.6 | Colon | 2.9 |
| Macrophages rest | 1.3 | Lung | 5.7 |
| Macrophages LPS | 0.1 | Thymus | 6.4 |
| HUVEC none | 3.6 | Kidney | 22.4 |
| HUVEC starved | 5.6 | | |

**General_screening_panel_v1.4 Summary:** Ag4997 Highest expression of the CG101231-01 gene is detected in an ovarian cancer SK-OV-3 cell line (CT=27). In addition, expression of this gene is also seen in cluster of cancer cell lines including pancreatic, CNS, colon, gastric, renal, lung, breast, ovarian, prostate, squamous cell carcinoma, and melanoma cancer cell lines. Overall, expression of this gene appears to be higher in samples derived from cancer cell lines than in normal tissues. Thus, expression of this gene could be used as a marker to detect the presence of cancer. Furthermore, therapeutic modulation of the expression or function of this gene product may be useful in the treatment of these cancers.

Among tissues with metabolic or endocrine function, this gene is expressed at low to moderate levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

Interestingly, this gene is expressed at much higher levels in fetal (CTs=29) when compared to adult lung and liver(CTs=33-38). This observation suggests that expression of this gene can be used to distinguish fetal from adult lung and liver. In addition, the relative overexpression of this gene in fetal tissue suggests that the protein product may enhance growth or development of liver and lung in the fetus and thus may also act in a regenerative capacity in the adult. Therefore, therapeutic modulation of the protein encoded by this gene could be useful in treatment of liver and lung related diseases.

In addition, this gene is expressed at moderate levels in all regions of the central nervous system examined, including amygdala, hippocampus, substantia nigra, thalamus, cerebellum, cerebral cortex, and spinal cord. Therefore, this gene may play an important role in central nervous system and therapeutic modulation of this gene product may be useful in the treatment of neurological disorders such as Alzheimer's disease, Parkinson's disease, epilepsy, multiple sclerosis, schizophrenia and depression.

**Panel 4.1D Summary:** Ag4997 Highest expression of the CG101231-01 gene is detected in PMA/ionomycin treated basophils (CT=29.4). This gene is expressed at low to moderate levels in a wide range of cell types of significance in the immune response in health and disease. These cells include members of the T-cell, lymphocytes, endothelial cell, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. This pattern is in agreement with the expression profile in General_screening_panel_v1.4 and also suggests a role for the gene product in cell survival and proliferation. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### E. CG101362-01: Prion Protein

Expression of gene CG101362-01 was assessed using the primer-probe set Ag6902, described in Table EA. Results of the RTQ-PCR runs are shown in Table EB.

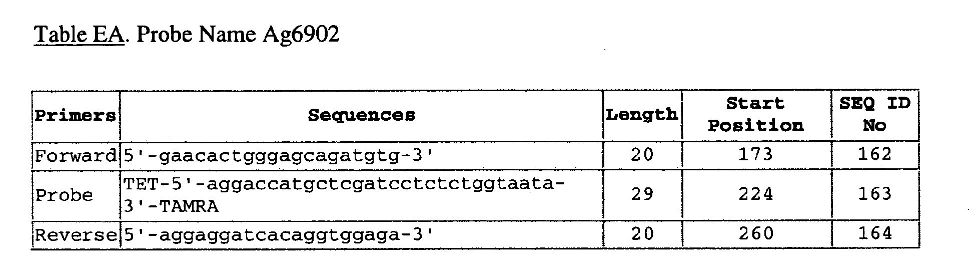

**Table EB. General_screening_panel_v1.6**

| **Tissue Name** | **Rel. Exp.(%) Ag6902, Run 278388399** | **Tissue Name** | **Rel. Exp.(%) Ag6902, Run 278388399** |
|---|---|---|---|
| Adipose | 3.0 | Renal ca. TK-10 | 9.0 |
| Melanoma* Hs688(A).T | 23.5 | Bladder | 3.8 |
| Melanoma* Hs688(B).T | 21.0 | Gastric ca. (liver met.) NCI-N87 | 21.2 |
| Melanoma* M14 | 50.3 | Gastric ca. KATO III | 15.9 |
| Melanoma* LOXIMVI | 38.7 | Colon ca. SW-948 | 2.6 |
| Melanoma* SK-MEL-5 | 14.3 | Colon ca. SW480 | 24.5 |
| Squamous cell carcinoma SCC-4 | 24.0 | Colon ca.* (SW480 met) SW620 | 16.8 |
| Testis Pool | 6.5 | Colon ca. HT29 | 5.3 |
| Prostate ca.* (bone met) PC-3 | **100.0** | Colon ca. HCT-116 | 12.2 |
| Prostate Pool | 6.4 | IColon ca. CaCo-2 | 4.1 |
| Placenta | 1.5 | Colon cancer tissue | 6.6 |
| Uterus Pool | 4.1 | Colon ca. SW 1116 | 1.5 |
| Ovarian ca. OVCAR-3 | 2.5 | Colon ca. Colo-205 | 2.3 |
| Ovarian ca. SK-OV-3 | 6.1 | Colon ca. SW-48 | 0.7 |
| Ovarian ca. OVCAR-4 | 3.3 | Colon Pool | 9.6 |
| Ovarian ca. OVCAR-5 | 16.3 | Small Intestine Pool | 8.5 |
| Ovarian ca. IGROV- 1 | 21.9 | Stomach Pool | 4.0 |
| Ovarian ca. OVCAR-8 | 16.8 | Bone Marrow Pool | 4.9 |
| Ovary | 6.0 | Fetal Heart | 8.2 |
| Breast ca. MCF-7 | 2.2 | Heart Pool | 5.6 |
| Breast ca. MDA-MB-231 | 32.1 | Lymph Node Pool | 11.3 |
| Breast ca. BT 549 | 41.2 | Fetal Skeletal Muscle | 4.0 |
| Breast ca. T47D | 1.7 | Skeletal Muscle Pool | 0.7 |
| Breast ca. MDA-N | 7.1 | Spleen Pool | 1.6 |
| Breast Pool | 12.1 | Thymus Pool | 3.7 |
| Trachea | 5.4 | CNS cancer (glio/astro) U87-MG | 31.4 |
| Lung | 2.1 | CNS cancer (glio/astro) U-118-MG | 30.4 |
| Fetal Lung | 13.4 | CNS cancer (neuro;met) SK-N-AS | 2.7 |
| Lung ca. NCI-N417 | 1.6 | CNS cancer (astro) SF-539 | 7.5 |
| Lung ca. LX-1 | 20.3 | CNS cancer (astro) SNB-75 | 32.8 |
| Lung ca. NCI-H146 | 1.0 | CNS cancer (glio) SNB-19 | 20.2 |
| Lung ca. SHP-77 | 1.7 | CNS cancer (glio) SF-295 | 66.4 |
| Lung ca. A549 | 17.6 | Brain (Amygdala) Pool | 18.6 |
| Lung ca. NCI-H526 | 1.4 | Brain (cerebellum) | 67.8 |
| Lung ca. NCI-H23 | 23.8 | Brain (fetal) | 10.9 |
| Lung ca. NCI-H460 | 18.7 | Brain (Hippocampus) Pool | 21.9 |
| Lung ca. HOP-62 | 28.5 | Cerebral Cortex Pool | 33.0 |
| Lung ca. NCI-H522 | 4.0 | Brain (Substantia nigra) Pool | 17.7 |
| Liver | 0.5 | Brain (Thalamus) Pool | 30.4 |
| Fetal Liver | 5.5 | Brain (whole) | 29.3 |
| Liver ca. HepG2 | 8.0 | Spinal Cord Pool | 13.2 |
| Kidney Pool | 14.0 | Adrenal Gland | 5.4 |
| Fetal Kidney | 2.4 | Pituitary gland Pool | 3.0 |
| Renal ca. 786-0 | 8.0 | Salivary Gland | 2.2 |
| Renal ca. A498 | 3.8 | Thyroid (female) | 3.5 |
| Renal ca. ACHN | 10.6 | Pancreatic ca. CAPAN2 | 13.0 |
| Renal ca. UO-31 | 26.1 | Pancreas Pool | 3.7 |

**General_screening_panel_v1.6 Summary:** Ag6902 Highest expression of the CG101362-01 gene is detected in prostate cancer cell line (CT=29.5). Moderate to low levels of expression of this gene is also seen in cluster of cancer cell lines derived from gastric, colon, lung, renal, breast, ovarian, prostate, squamous cell carcinoma, melanoma and brain cancers. Thus, expression of this gene could be used as a marker to detect the presence of these cancers. Furthermore, therapeutic modulation of the expression or function of this gene may be effective in the treatment of gastric, colon, lung, renal, breast, ovarian, prostate, squamous cell carcinoma, melanoma and brain cancers.

Among tissues with metabolic or endocrine function, this gene is expressed at moderate to low levels in pancreas, adipose, adrenal gland, thyroid, pituitary gland, fetal skeletal muscle, heart, fetal liver and the gastrointestinal tract. Therefore, therapeutic modulation of the activity of this gene may prove useful in the treatment of endocrine/metabolically related diseases, such as obesity and diabetes.

Interestingly, this gene is expressed at much higher levels in fetal (CT=33.7) when compared to adult liver (CT=37). This observation suggests that expression of this gene can be used to distinguish fetal from adult liver. In addition, the relative overexpression of this gene in fetal liver suggests that the protein product may enhance liver growth or development in the fetus and thus may also act in a regenerative capacity in the adult. Therefore, therapeutic modulation of the protein encoded by this gene could be useful in treatment of liver related diseases.

In addition, this gene is expressed at moderate levels in all regions of the central nervous system examined, including amygdala, hippocampus, substantia nigra, thalamus, cerebellum, cerebral cortex, and spinal cord. Therefore, therapeutic modulation of this gene product may be useful in the treatment of central nervous system disorders such as Alzheimer's disease, Parkinson's disease, epilepsy, multiple sclerosis, schizophrenia and depression.

### F. CG101458-01: von Willebrand domain protein

Expression of gene CG101458-01 was assessed using the primer-probe set Ag4220, described in Table FA. Results of the RTQ-PCR runs are shown in Tables FB, FC, FD and FE.

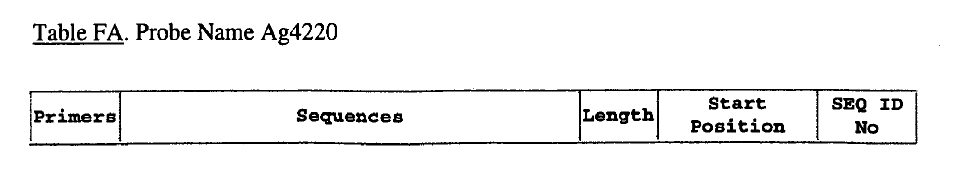

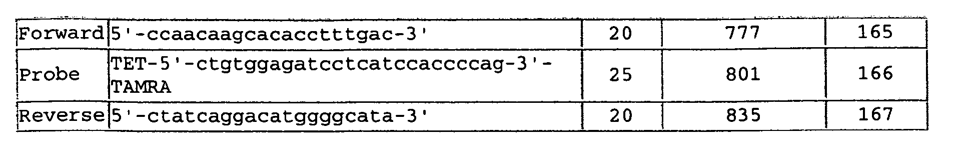

**Table FB. CNS_neurodegeneration_v1.0**

| **Tissue Name** | **Rel. Exp.(%) Ag4220, Run 215620528** | **Tissue Name** | **Rel. Exp.(%) Ag4220, Run 215620528** |
|---|---|---|---|
| AD 1 Hippo | 12.3 | Control (Path) 3 Temporal Ctx | 0.0 |
| AD 2 Hippo | 32.8 | Control (Path) 4 Temporal Ctx | 7.5 |
| AD 3 Hippo | 18.6 | AD 1 Occipital Ctx | 3.1 |
| AD 4 Hippo | 22.2 | AD 2 Occipital Ctx (Missing) | 0.0 |
| AD 5 Hippo | 7.1 | AD 3 Occipital Ctx | 3.6 |
| AD 6 Hippo | 11.6 | AD 4 Occipital Ctx | 11.5 |
| Control 2 Hippo | 19.6 | AD 5 Occipital Ctx | 8.7 |
| Control 4 Hippo | 43.8 | AD 6 Occipital Ctx | 2.3 |
| Control (Path) 3 Hippo | 98.6 | Control 1 Occipital Ctx | 1.8 |
| AD 1 Temporal Ctx | 4.9 | Control 2 Occipital Ctx | 5.5 |
| AD 2 Temporal Ctx | 12.8 | Control 3 Occipital Ctx | 0.7 |
| AD 3 Temporal Ctx | 2.8 | Control 4 Occipital Ctx | 12.2 |
| AD 4 Temporal Ctx | 12.9 | Control (Path) 1 Occipital Ctx | 30.8 |
| AD 5 Inf Temporal Ctx | 7.2 | Control (Path) 2 Occipital Ctx | 2.8 |
| AD 5 Sup Temporal Ctx | **100.0** | Control (Path) 3 Occipital Ctx | 1.5 |
| AD 6 Inf Temporal Ctx | 8.1 | Control (Path) 4 Occipital Ctx | 2.2 |
| AD 6 Sup Temporal Ctx | 15.1 | Control 1 Parietal Ctx | 2.6 |
| Control 1 Temporal Ctx | 3.6 | Control 2 Parietal Ctx | 17.0 |
| Control 2 Temporal Ctx | 2.7 | Control 3 Parietal Ctx | 1.4 |
| Control 3 Temporal Ctx | 6.4 | Control (Path) 1 Parietal Ctx | 14.2 |
| Control 3 Temporal Ctx | 5.8 | Control (Path) 2 Parietal Ctx | 15.6 |
| Control (Path) 1 Temporal Ctx | 11.0 | Control (Path) 3 Parietal Ctx | 0.0 |
| Control (Path) 2 Temporal Ctx | 5.0 | Control (Path) 4 Parietal Ctx | 17.8 |

**Table FC. General_screening_panel_v1.4**

| **Tissue Name** | **Rel. Exp.(%) Ag4220, Run 221153243** | **Tissue Name** | **Rel. Exp.(%) Ag4220, Run 221153243** |
|---|---|---|---|
| Adipose | 0.4 | Renal ca. TK-10 | 0.0 |
| Melanoma* Hs688(A).T | 0.0 | Bladder | 1.5 |
| Melanoma* Hs688(B).T | 0.0 | Gastric ca. (liver met.) NCI-N87 | 0.0 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 35.1 | Colon ca. HT29 | 0.0 |
| Prostate ca.* (bone met) PC-3 | 0.0 | Colon ca. HCT-116 | 0.0 |
| Prostate Pool | 0.1 | Colon ca. CaCo-2 | 0.0 |
| Placenta | 0.0 | Colon cancer tissue | 13.1 |
| Uterus Pool | 0.0 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 2.7 |
| Ovarian ca. OVCAR-4 | 0.5 | Colon Pool | 0.0 |
| Ovarian ca. OVCAR-5 | 0.6 | Small Intestine Pool | 1.9 |
| Ovarian ca. IGROV-1 | 1.2 | Stomach Pool | 0.1 |
| Ovarian ca. OVCAR-8 | 0.0 | Bone Marrow Pool | 0.4 |
| Ovary | 0.0 | Fetal Heart | 0.0 |
| Breast ca. MCF-7 | 0.0 | Heart Pool | 0.2 |
| Breast ca. MDA-MB-231 | 0.0 | Lymph Node Pool | 0.0 |
| Breast ca. BT 549 | 0.0 | Fetal Skeletal Muscle | 0.6 |
| Breast ca. T47D | 0.7 | Skeletal Muscle Pool | 0.9 |
| Breast ca. MDA-N | 0.0 | Spleen Pool | 0.5 |
| Breast Pool | 1.0 | Thymus Pool | 0.5 |
| Trachea | 0.4 | CNS cancer (glio/astro) U87-MG | 0.0 |
| Lung | 0.0 | CNS cancer (glio/astro) U-118-MG | 0.0 |
| Fetal Lung | 3.7 | CNS cancer (neuro;met) SK-N-AS | 0.0 |
| Lung ca. NCI-N417 | 0.5 | CNS cancer (astro) SF-539 | 0.0 |
| Lung ca. LX-1 | 0.3 | CNS cancer (astro) SNB-75 | 0.0 |
| Lung ca. NCI-H146 | 0.0 | CNS cancer (glio) SNB-19 | 2.7 |
| Lung ca. SHP-77 | 0.0 | CNS cancer (glio) SF-295 | 0.7 |
| Lung ca. A549 | 0.3 | Brain (Amygdala) Pool | 1.3 |
| Lung ca. NCI-H526 | 0.0 | Brain (cerebellum) | 0.9 |
| Lung ca. NCI-H23 | 0.0 | Brain (fetal) | 6.8 |
| Lung ca. NCI-H460 | 0.0 | Brain (Hippocampus) Pool | 8.0 |
| Lung ca. HOP-62 | 0.0 | Cerebral Cortex Pool | 2.9 |
| Lung ca. NCI-H522 | 0.0 | Brain (Substantia nigra) Pool | 6.7 |
| Liver | 0.0 | Brain (Thalamus) Pool | 8.8 |
| Fetal Liver | 0.0 | Brain (whole) | 5.2 |
| Liver ca. HepG2 | 0.0 | Spinal Cord Pool | 8.9 |
| Kidney Pool | 0.0 | Adrenal Gland | 7.7 |
| Fetal Kidney | 21.9 | Pituitary gland Pool | **100.0** |
| Renal ca. 786-0 | 0.0 | Salivary Gland | 17.6 |
| Renal ca. A498 | 0.0 | Thyroid (female) | 8.6 |
| Renal ca. ACHN | 0.0 | Pancreatic ca. CAPAN2 | 0.0 |
| Renal ca. UO-31 | 2.4 | Pancreas Pool | 5.1 |

**Table FD. Panel 4.1D**

| **Tissue Name** | **Rel. Exp.(%) Ag4220, Run 174926565** | **Tissue Name** | **Rel. Exp.(%) Ag4220, Run 174926565** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1 beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha + IL-1 beta | 0.0 |
| CD45RA CD4 lymphocyte act | 1.5 | Coronery artery SMC rest | 0.0 |
| CD45R0 CD4 lymphocyte act | 2.4 | Coronery artery SMC TNFalpha + IL-1 beta | 0.0 |
| CD8 lymphocyte act | 2.5 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 I | 0.0 | CCD1106 (Keratinocytes) none | 0.0 |
| LAK cells rest | 0.0 | CCD1106 (Keratinocytes) TNFalpha + IL- 1beta | 0.0 |
| LAK cells IL-2 | 0.0 | Liver cirrhosis | 0.0 |
| LAK cells IL-2+IL-12 | 0.0 | NCI-H292 none | 0.0 |
| LAK cells IL-2+IFN gamma | 0.0 | NCI-H292 IL-4 | 0.0 |
| LAK cells IL-2+ IL-18 | 1.5 | NCI-H292 IL-9 | 0.0 |
| LAK cells | 0.0 | NCI-H292 IL-13 | 0.0 |
| PMA/ionomycin | | | |
| NK Cells IL-2 rest | 0.0 | NCI-H292 IFN gamma | 0.0 |
| Two Way MLR 3 day | 2.5 | HPAEC none | 0.0 |
| Two Way MLR 5 day | 0.0 | HPAEC TNF alpha + IL-1 beta | 0.0 |
| Two Way MLR 7 day | 0.0 | Lung fibroblast none | 0.0 |
| PBMC rest | 0.0 | Lung fibroblast TNF alpha + IL-1 beta | 0.0 |
| PBMC PWM | 0.0 | Lung fibroblast IL-4 | 0.0 |
| PBMC PHA-L | 2.8 | Lung fibroblast IL-9 | 0.0 |
| Ramos (B cell) none | 0.0 | Lung fibroblast IL-13 | 0.0 |
| Ramos (B cell) ionomycin | 0.0 | Lung fibroblast IFN gamma | 0.0 |
| B lymphocytes PWM | 0.0 | Dermal fibroblast CCD 1070 rest | 0.0 |
| B lymphocytes CD40L and IL-4 | 0.0 | Dermal fibroblast CCD1070 TNF alpha | 0.0 |
| EOL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 5.4 |
| Dendritic cells LPS | 0.0 | Dermal Fibroblasts rest | 2.8 |
| Dendritic cells anti-CD40 | 0.0 | Neutrophils TNFa+LPS | 0.0 |
| Monocytes rest | 0.0 | Neutrophils rest | 0.0 |
| Monocytes LPS | 0.0 | Colon | 0.0 |
| Macrophages rest | 0.0 | Lung | 2.9 |
| Macrophages LPS | 0.0 | Thymus | 11.0 |
| HUVEC none | 0.0 | Kidney | **100.0** |
| HUVEC starved | 0.0 | | |

**Table FE. general oncology screening panel_v_2.4**

| **Tissue Name** | **Rel. Exp.(%) Ag4220, Run 268624960** | **Tissue Name** | **Rel. Exp.(%) Ag4220, Run 268624960** |
|---|---|---|---|
| Colon cancer 1 | 0.8 | Bladder cancer NAT 2 | 0.0 |
| Colon cancer NAT 1 | 0.0 | Bladder cancer NAT 3 | 0.0 |
| Colon cancer 2 | 0.0 | Bladder cancer NAT 4 | 0.0 |
| Colon cancer NAT 2 | 3.1 | Adenocarcinoma of the prostate 1 | 0.6 |
| Colon cancer 3 | 1.8 | Adenocarcinoma of the prostate 2 | 0.0 |
| Colon cancer NAT 3 | 5.3 | Adenocarcinoma of the prostate 3 | 3.0 |
| Colon malignant cancer 4 | 0.0 | Adenocarcinoma of the prostate 4 | 3.6 |
| Colon 4 normal adjacent tissue 4 | 0.0 | Prostate cancer NAT 5 | 0.0 |
| Lung cancer 1 | 0.0 | Adenocarcinoma of the prostate 6 | 0.0 |
| Lung NAT 1 | 0.7 | Adenocarcinoma of the prostate 7 | 1.9 |
| Lung cancer 2 | 16.5 | Adenocarcinoma of the prostate 8 | 0.0 |
| Lung NAT 2 | 0.7 | Adenocarcinoma of the prostate 9 | 3.3 |
| Squamous cell carcinoma 3 | 0.0 | Prostate cancer NAT 10 | 0.7 |
| Lung NAT 3 | 0.0 | Kidney cancer 1 | 0.8 |
| metastatic melanoma | 27.4 | KidneyNAT 1 | 89.5 |
| Melanoma 2 | 1.0 | Kidney cancer 2 | 13.9 |
| Melanoma 3 | 0.8 | Kidney NAT 2 | **100.0** |
| metastatic melanoma | 0.9 | Kidney cancer 3 | 3.0 |
| metastatic melanoma | 0.0 | Kidney NAT 3 | 92.7 |
| Bladder cancer 1 | 0.0 | Kidney cancer 4 | 0.0 |
| Bladder cancer NAT 1 | 0.0 | Kidney NAT 4 | 17.2 |
| Bladder cancer 2 | 25.9 | | |

**CNS_neurodegeneration_v1.0 Summary:** Ag4220 This panel confirms the expression of the CG101458-01 gene at low levels in the brains of an independent group of individuals. However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4220 Highest expression of the CG101458-01 gene is detected in pituitary gland (CT=27.9). Furthermore, moderate to low levels of expression of this gene is also seen in other tissues with metabolic or endocrine functions including pancrease, adrenal gland, thyroid, skeletal muscle, and small intestine. Therefore, therapeutic modulation of the activity of this gene may prove useful in the treatment of endocrine/metabolically related diseases, such as obesity and diabetes.

In addition, this gene is expressed at moderate to low levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

Moderate expression of this gene is also seen in testis. Therefore, therapeutic modulation of this gene product may be useful in the treatment of testis related diseases such as fertility and hypogonadism.

Significant expression of this gene is seen in fetal kidney and lung. Interestingly, this gene is expressed at much higher levels in fetal (CTs=30-32) when compared to adult kidney and lung(CTs=40). This observation suggests that expression of this gene can be used to distinguish fetal from adult kidney and lung. In addition, the relative overexpression of this gene in fetal tissue suggests that the protein product may enhance growth or development of kidney and lung in the fetus and thus may also act in a regenerative capacity in the adult. Therefore, therapeutic modulation of the protein encoded by this gene could be useful in treatment of kidney and lung related diseases.

**Panel 4.1D Summary:** Ag4220 Significant expression of the CG101458-01 gene is detected exclusively in kidney (CT=32.3). Therefore, expression of this gene may be used to distinguish kidney sample from other samples in this panel. In addition, therapeutic modulation of this gene product may be beneficial in the treatment of autoimmune and inflammatory diseases that affect kidney, including lupus and golomerulonephritis.

**general oncology screening panel_v_2.4 Summary:** Ag4220 Highest expression of the CG101458-01 gene is detected in control kidney samples (CTs=31). Interestingly, expression of this gene is higher in control samples as compared to kidney cancer samples. Therefore, expression of this gene may be used to distinguish between cancer and normal kidney samples. In addition, therapeutic modulation of this gene product that stimulates the function or expression of the this gene product may be beneficial in the treatment of kidney cancer.

In addition, significant expression of this gene is also seen in a bladder cancer, a lung cancer and a metastatic melanoma samples. Expression of this gene is higher in these cancer samples as compared to the adjacent control samples. Therefore, expression of this gene may be used as diagnostic marker for detection of these cancers and therapeutic modulation of this gene product may be beneficial in the treatment of these cancers.

### G. CG101475-01: Novel plasma membrane protein containing lectin C-

### type domain

Expression of gene CG101475-01 was assessed using the primer-probe set Ag4214, described in Table GA. Results of the RTQ-PCR runs are shown in Tables GB, GC and GD.

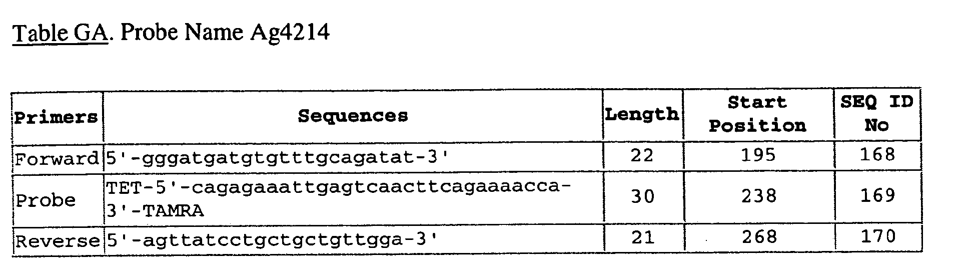

**Table GB. AI_comprehensive panel_v1.0**

| **Tissue Name** | **Rel. Exp.(%) Ag4214, Run 248080020** | **Tissue Name** | **Rel. Exp.(%) Ag4214, Run 248080020** |
|---|---|---|---|
| 110967 COPD-F | 1.3 | 112427 Match Control Psoriasis-F | 3.9 |
| 110980 COPD-F | 0.0 | 112418 Psoriasis-M | 2.7 |
| 110968 COPD-M | 3.1 | 112723 Match Control | 8.8 |
| 110977 COPD-M | 0.3 | 112419 Psoriasis-M | 1.5 |
| 110989 Emphysema-F | 20.3 | 112424 Match Control Psoriasis-M | 2.3 |
| 110992 Emphysema-F | 1.5 | 112420 Psoriasis-M | 3.9 |
| 110993 Emphysema-F | 7.0 | 112425 Match Control Psoriasis-M | 2.7 |
| 110994 Emphysema-F | 3.3 | 104689 (MF) OA Bone-Backus | 11.0 |
| 110995 Emphysema-F | 3.1 | 104690 (MF) Adj "Normal" Bone-Backus | 6.7 |
| 110996 Emphysema-F | 0.0 | 104691 (MF) OA Synovium-Backus | 1.3 |
| 110997 Asthma-M | 2.6 | 104692 (BA) OA Cartilage-Backus | 0.0 |
| 111001 Asthma-F | 21.6 | 104694 (BA) OA Bone-Backus | 1.6 |
| 111002 Asthma-F | 11.5 | 104695 (BA) Adj "Normal" Bone-Backus | 3.8 |
| 111003 Atopic Asthma-F | 49.3 | 104696 (BA) OA Synovium-Backus | 9.8 |
| 111004 Atopic Asthma-F | 19.6 | 104700 (SS) OA Bone-Backus | 1.4 |
| 111005 Atopic Asthma-F | 24.5 | 104701 (SS) Adj "Normal" Bone-Backus | 1.6 |
| 111006 Atopic Asthma-F/ | 7.4 | 104702 (SS) OA Synovium-Backus | 35.4 |
| 111417 Allergy-M | 11.6 | 117093 OA Cartilage Rep7 | 2.6 |
| 112347 Allergy-M | 0.0 | 112672 OA Bone5 | 1.4 |
| 112349 Normal Lung-F | 0.0 | 112673 OA Synovium5 | 1.2 |
| 112357 Normal Lung-F | 10.6 | 112674 OA Synovial Fluid cells5 | 0.4 |
| 112354 Normal Lung-M | 0.8 | 117100 OA Cartilage Rep14 | 0.6 |
| 112374 Crohns-F | 2.9 | 112756 OA Bone9 | 8.9 |
| 112389 Match Control Crohns-F | 0.5 | 112757 OA Synovium9 | 0.0 |
| 112375 Crohns-F | 6.4 | 112758 OA Synovial Fluid Cells9 | 6.0 |
| 112732 Match Control Crohns-F | 0.0 | 117125 RA Cartilage Rep2 | 16.4 |
| 112725 Crohns-M | 0.0 | 113492 Bone2 RA | 0.4 |
| 112387 Match Control Crohns-M | 0.4 | 113493 Synovium2 RA | 0.4 |
| 112378 Crohns-M | 0.0 | 113494 Syn Fluid Cells RA | 0.0 |
| 112390 Match Control Crohns-M | 5.7 | 113499 Cartilage4 RA | 0.8 |
| 112726 Crohns-M | **100.0** | 113500 Bone4 RA | 1.2 |
| 112731 Match Control Crohns-M | 28.9 | 113501 Synovium4 RA | 0.6 |
| 112380 Ulcer Col-F | 30.8 | 113502 Syn Fluid Cells4 RA | 0.8 |
| 112734 Match Control Ulcer Col-F | 0.0 | 113495 Cartilage3 RA | 0.8 |
| 112384 Ulcer Col-F | 0.5 | 113496 Bone3 RA | 0.2 |
| 112737 Match Control Ulcer Col-F | 28.9 | 113497 Synovium3 RA | 0.0 |
| 112386 Ulcer Col-F | 0.5 | 113498 Syn Fluid Cells3 RA | 0.2 |
| 112738 Match Control Ulcer Col-F | 0.0 | 117106 Normal Cartilage Rep20 | 1.3 |
| 112381 Ulcer Col-M | 1.7 | 113663 Bone3 Normal | 0.0 |
| 112735 Match Control Ulcer Col-M | 1.2 | 113664 Synovium3 Normal | 0.0 |
| 112382 Ulcer Col-M | 0.0 | 113665 Syn Fluid Cells3 Normal Normal | 0.0 |
| 112394 Match Control Ulcer Col-M | 0.0 | 117107 Normal Cartilage Rep22 | 1.8 |
| 112383 Ulcer Col-M | 12.0 | 113667 Bone4 Normal | 0.0 |
| 112736 Match Control Ulcer Col-M | 0.0 | 113668 Synovium4 Normal | 0.7 |
| 112423 Psoriasis-F | 11.3 | 113669 Syn Fluid Cells4 Normal | 0.9 |

**Table GC. General_screening_panel_v1.4**

| **Tissue Name** | **Rel. Exp.(%) Ag4214, Run 221254821** | **Tissue Name** | **Rel. Exp.(%) Ag4214, Run 221254821** |
|---|---|---|---|
| Adipose | 0.0 | Renal ca. TK-10 | 0.0 |
| Melanoma* Hs688(A).T | 0.9 | Bladder | 0.4 |
| Melanoma* Hs688(B).T | 0.0 | Gastric ca. (liver met.) NCI-N87 | 0.0 |
| Melanoma* M14 | 0.0 | Gastric ca. KATO III | 0.0 |
| Melanoma* LOXIMVI | 2.9 | Colon ca. SW-948 | 0.0 |
| Melanoma* SK-MEL-5 | 0.8 | Colon ca. SW480 | 0.0 |
| Squamous cell carcinoma SCC-4 | 0.0 | Colon ca.* (SW480 met) SW620 | 0.0 |
| Testis Pool | **100.0** | Colon ca. HT29 | 0.0 |
| Prostate ca.* (bone met) PC-3 | 5.1 | Colon ca. HCT-116 | 0.0 |
| Prostate Pool | 0.3 | Colon ca. CaCo-2 | 0.0 |
| Placenta | 0.0 | Colon cancer tissue | 0.0 |
| Uterus Pool | 0.0 | Colon ca. SW 1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.0 |
| Ovarian ca. OVCAR-5 | 0.0 | Small Intestine Pool | 0.0 |
| Ovarian ca. IGROV-1 | 0.0 | Stomach Pool | 0.3 |
| Ovarian ca. OVCAR-8 | 0.0 | Bone Marrow Pool | 0.0 |
| Ovary | 0.3 | Fetal Heart | 0.0 |
| Breast ca. MCF-7 | 0.0 | Heart Pool | 0.0 |
| Breast ca. MDA-MB-231 | 0.0 | Lymph Node Pool | 0.0 |
| Breast ca. BT 549 | 0.0 | Fetal Skeletal Muscle | 0.0 |
| Breast ca. T47D | 0.0 | Skeletal Muscle Pool | 0.2 |
| Breast ca. MDA-N | 0.0 | Spleen Pool | 1.8 |
| Breast Pool | 0.0 | Thymus Pool | 0.0 |
| Trachea | 0.0 | Cancer (glio/astro) U87-MG | 1.7 |
| Lung | 0.0 | CNS cancer (glio/astro) U-118-MG | 0.0 |
| Fetal Lung Lung | 0.3 | CNS cancer (neuro;met) SK-N-AS | 0.0 |
| Lung ca. NCI-N417 | 0.0 | CNS cancer (astro) SF-539 | 0.4 |
| Lung ca. LX-1 | 0.0 | CNS cancer (astro) SNB-75 | 0.5 |
| Lung ca. NCI-H146 | 0.0 | CNS cancer (glio) SNB-19 | 0.0 |
| Lung ca. SHP-77 | 0.7 | CNS cancer (glio) SF-295 | 11.5 |
| Lung ca. A549 | 0.0 | Brain (Amygdala) Pool | 0.3 |
| Lung ca. NCI-H526 | 0.0 | Brain (cerebellum) | 0.4 |
| Lung ca. NCI-H23 | 0.4 | Brain (fetal) | 0.3 |
| Lung ca. NCI-H460 | 0.0 | Brain (Hippocampus) Pool | 1.6 |
| Lung ca. HOP-62 | 0.4 | Cerebral Cortex Pool | 0.8 |
| Lung ca. NCI-H522 | 0.0 | Brain (Substantia nigra) Pool | 0.3 |
| Liver | 0.2 | Brain (Thalamus) Pool | 1.6 |
| Fetal Liver | 0.3 | Brain (whole) | 0.3 |
| Liver ca. HepG2 | 0.0 | Spinal Cord Pool | 0.9 |
| Kidney Pool | 0.7 | Adrenal Gland | 0.0 |
| Fetal Kidney | 0.0 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 0.4 |

**Table GD. Panel 4.1D**

| **Tissue Name** | **Rel. Exp.(%) Ag4214, Run 174261198** | **Tissue Name** | **Rel. Exp.(%) Ag4214, Run 174261198** |
|---|---|---|---|
| Secondary Th1 act | 0.0 | HUVEC IL-1beta | 22.5 |
| Secondary Th2 act | 0.0 | HUVEC IFN gamma | 13.8 |
| Secondary Tr1 act | 0.0 | HUVEC TNF alpha + IFN gamma | 0.0 |
| Secondary Th1 rest | 0.0 | HUVEC TNF alpha + IL4 | 3.7 |
| Secondary Th2 rest | 0.0 | HUVEC EL-11 | 11.1 |
| Secondary Tr1 rest | 0.0 | Lung Microvascular EC none | 0.0 |
| Primary Th1 act | 0.0 | Lung Microvascular EC TNFalpha + IL-1beta | 1.3 |
| Primary Th2 act | 0.0 | Microvascular Dermal EC none | 9.5 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha + IL-1beta | 0.0 |
| CD45RA CD4 lymphocyte act | 0.0 | Coronery artery SMC rest | 10.3 |
| CD45RO CD4 lymphocyte act | 0.0 | Coronery artery SMC TNFalpha + IL-1beta | 15.4 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest KU-812 (Basophil) rest | 5.2 |
| CD4 lymphocyte none | 4.6 | KU-812 (Basophil) PMA/ionomycin PMA/ionomycin | 0.0 |
| 2ry Th1/Th2/Tr1_anti-CD95 CH11 | 0.0 | CCD1106 (Keratinocytes) none | 0.0 |
| LAK cells rest | 32.5 | CCD1106 (Keratinocytes) TNFalpha + IL-1beta | 0.0 |
| LAK cells IL-2 | 0.0 | Liver cirrhosis | 0.0 |
| LAK cells IL-2+IL-12 | 0.0 | NCI-H292 none | 0.0 |
| LAK cells IL-2+IFN gamma | 0.0 | NCI-H292 IL-4 | 0.0 |
| LAK cells IL-2+ IL-18 | 0.0 | NCI-H292 IL-9 | 0.0 |
| LAK cells PMA/ionomycin | 5.7 | NCI-H292 IL-13 | 0.0 |
| NK Cells IL-2 rest | 0.0 | NCI-H292 IFN gamma | 0.0 |
| Two Way MLR 3 day | 6.0 | HPAEC none | 16.6 |
| Two Way MLR 5 day | 6.2 | HPAEC TNF alpha + IL-1 beta | 3.2 |
| Two Way MLR 7 day | 0.0 | Lung fibroblast none | 0.0 |
| PBMC rest | 18.0 | Lung fibroblast TNF alpha + IL-1 beta | 0.0 |
| PBMC PWM | 0.0 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 0.0 |
| Ramos (B cell) ionomycin | 0.0 | Lung fibroblast IFN gamma | 0.0 |
| B lymphocytes PWM | 0.0 | Dermal fibroblast CCD1070 rest | 0.0 |
| B lymphocytes CD40L and IL-4 | 0.0 | Dermal fibroblast CCD 1070 TNF alpha | 0.0 |
| EOL-1 dbcAMP | 6.7 | Dermal fibroblast CCD1070 IL-1 beta | 0.0 |
| EOL-1 dbcAMP PMA/ionomycin | 0.0 | Dermal fibroblast IFN gamma | 3.3 |
| Dendritic cells none | 27.0 | Dermal fibroblast IL-4 | 3.0 |
| Dendritic cells LPS | 2.3 | Dermal Fibroblasts rest | 0.0 |
| Dendritic cells anti-CD40 | 13.3 | Neutrophils TNFa+LPS | 8.5 |
| Monocytes rest | 54.3 | Neutrophils rest | 20.4 |
| Monocytes LPS | **100.0** | Colon | 0.0 |
| Macrophages rest | 5.8 | Lung | 3.1 |
| Macrophages LPS | 0.0 | Thymus | 0.0 |
| HUVEC none | 22.2 | Kidney | 0.0 |
| HUVEC starved | 12.8 | | |

**AI_comprehensive panel_v 1.0 Summary:** Ag4214 Highest expression of the CG101475-01 gene is detected in Crohn's disease sample (CT=29). In addition, significant expression of this gene is also seen in samples derived from normal lung samples, COPD lung, emphysema, atopic asthma, asthma, allergy, Crohn's disease (normal matched control and diseased), ulcerative colitis(normal matched control and diseased), psoriasis (normal matched control and diseased), bone (Orthoarthritis and matched control), OA synovium and rheumatoid arthritis cartilage Rep2. Therefore, therapeutic modulation of this gene product may ameliorate symptoms/conditions associated with autoimmune and inflammatory disorders including psoriasis, allergy, asthma, inflammatory bowel disease, rheumatoid arthritis and osteoarthritis

**General_screening_panel_v1.4 Summary:** Ag4214 Moderate level of expression of the CG101475-01 gene is detected only in testis (CT=31). Therefore, expression of this gene may be used to distinguish testis from other samples used in this panel. In addition, therapeutic modulation of this gene may be useful in the treatment of testis related diseases, including fertility and hypogonadism.

Low levels of expression of this gene is also detected in one of the CNS cancer cell line. Therefore, therapeutic modulation of this gene product may be beneficial in the treatment of CNS cancer.

**Panel 4.1D Summary:** Ag4214 Highest expression of the CG101475-01 gene is detected in LPS treated monocytes (CT=33). Low levels of expression of this gene is also detected in resting monocytes and LAK cells. Therefore, expression of this gene may be used to distinguish monocytes and LAK cells from other samples used in this panel. The expression of this gene in resting cells suggests that the protein encoded by this gene may be involved in normal immunological processes associated with immune homeostasis. In addition, expression of this gene in activated monocytes suggests that this gene may be involved in their function as antigen-presenting cells and antibodies or small molecule therapeutics that block the function of this membrane protein may be useful as antiinflammatory therapeutics for the treatment of autoimmune and inflammatory diseases.

### H. CG101475-02: Novel plasma membrane protein containing lectin C-type domain

Expression of gene CG101475-02 was assessed using the primer-probe set Ag6376, described in Table HA. Please note that CG101475-02 represents a full-length physical clone

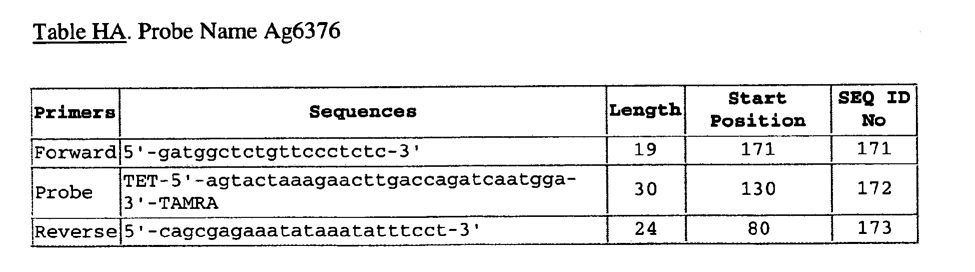

### I. CG102575-01 and CG102575-02: Novel ATPase associated with various cellular activities

Expression of gene CG102575-01 and CG102575-02 was assessed using the primer-probe set Ag4238, described in Table IA. Results of the RTQ-PCR runs are shown in Tables IB, IC, ID and IE. Please note that CG102575-02 represents a full-length physical clone of the CG102575-01 gene, validating the prediction of the gene sequence.

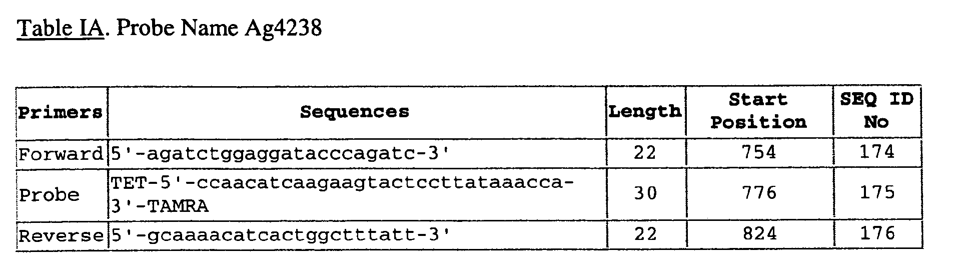

**Table IB. CNS_neurodegeneration_v1.0**

| **Tissue Name** | **Rel. Exp.(%) Ag4238, Run 224065201** | **Tissue Name** | **Rel. Exp.(%) Ag4238, Run 224065201** |
|---|---|---|---|
| AD 1 Hippo | 10.2 | Control (Path) 3 Temporal Ctx | 8.2 |
| AD 2 Hippo | 22.8 | Control (Path) 4 Temporal Ctx | 31.0 |
| AD 3 Hippo | 14.4 | AD I Occipital Ctx | 32.1 |
| AD 4 Hippo | 8.2 | AD 2 Occipital Ctx (Missing) | 0.0 |
| AD 5 hippo | **100.0** | AD 3 Occipital Ctx | 12.9 |
| AD 6 Hippo | 68.3 | AD 4 Occipital Ctx | 20.9 |
| Control 2 Hippo | 47.6 | AD 5 Occipital Ctx | 26.4 |
| Control 4 Hippo | 17.4 | AD 6 Occipital Ctx | 59.5 |
| Control (Path) 3 Hippo | 9.8 | Control 1 Occipital Ctx | 6.2 |
| AD 1 Temporal Ctx | 27.7 | Control 2 Occipital Ctx | 75.8 |
| AD 2 Temporal Ctx | 27.5 | Control 3 Occipital Ctx | 37.4 |
| AD 3 Temporal Ctx | 10.3 | Control 4 Occipital Ctx | 8.5 |
| AD 4 Temporal Ctx | 18.3 | Control (Path) 1 Occipital Ctx | 85.3 |
| AD 5 Inf Temporal Ctx | 86.5 | (Control (Path) 2 Occipital Ctx | 12.1 |
| AD 5 SupTemporal Ctx | 40.1 | Control (Path) 3 Occipital Ctx Occipital Ctx | 11.4 |
| AD 6 Inf Temporal Ctx | 59.5 | Control (Path) 4 Occipital Ctx | 19.6 |
| AD 6 Sup Temporal Ctx | 81.8 | Control 1 Parietal Ctx | 8.0 |
| Control 1 Temporal Ctx | 9.1 | Control 2 Parietal Ctx | 53.2 |
| Control 2 Temporal Ctx | 48.0 | Control 3 Parietal Ctx | 17.7 |
| Control 3 Temporal Ctx | 29.3 | Control (Path) 1 Parietal Ctx | 81.8 |
| Control 4 Temporal Ctx | 9.4 | Control (Path) 2 Parietal Ctx | 30.1 |
| Control (Path) 1 Temporal Ctx | 51.8 | Control (Path) 3 Parietal Ctx | 8.5 |
| Control (Path) 2 Temporal Ctx | 51.8 | Control (Path) 4 Parietal Ctx | 52.5 |

**Table IC. General_screening_panel_v1.4**

| **Tissue Name** | **Rel. Exp.(%) Ag4238, Run 222026508** | **Tissue Name** | **Rel. Exp.(%) Ag4238, Run 222026508** |
|---|---|---|---|
| Adipose | 12.2 | Renal ca. TK-10 | 24.3 |
| Melanoma* Hs688(A).T | 24.5 | Bladder | 25.0 |
| Melanoma* Hs688(B).T | 16.3 | Gastric ca. (liver met.) NCI-N87 | 75.8 |
| Melanoma* M 14 | 35.8 | Gastric ca. KATO III | 60.7 |
| Melanoma* LOXIMVI | 40.6 | Colon ca. SW-948 | 18.9 |
| Melanoma* SK-MEL-5 | 32.1 | Colon ca. SW480 | 26.8 |
| Squamous cell carcinoma SCC-4 | 29.7 | Colon ca.* (SW480 met) SW620 | 12.2 |
| Testis Pool | 20.0 | Colon ca. HT29 | 18.7 |
| Prostate ca.* (bone met) PC-3 | 27.5 | Colon ca. HCT-116 | 95.3 |
| Prostate Pool | 10.7 | Colon ca. CaCo-2 | 25.7 |
| Placenta | 2.6 | Colon cancer tissue | 13.5 |
| Uterus Pool | 8.4 | Colon ca. SW 1116 | 3.5 |
| Ovarian ca. OVCAR-3 | 29.1 | Colon ca. Colo-205 | 3.6 |
| Ovarian ca. SK-OV-3 | 69.7 | Colon ca. SW-48 | 3.0 |
| Ovarian ca. OVCAR-4 | 11.1 | Colon Pool | 37.9 |
| Ovarian ca. OVCAR-5 | 25.5 | Small Intestine Pool | 30.8 |
| Ovarian ca. IGROV-1 | 12.6 | Stomach Pool | 16.6 |
| Ovarian ca. OVCAR-8 | 7.8 | Bone Marrow Pool | 16.8 |
| Ovary | 7.9 | Fetal Heart | 24.3 |
| Breast ca. MCF-7 | 13.4 | Heart Pool | 13.7 |
| Breast ca. MDA-MB-231 | 55.1 | Lymph Node Pool | 38.7 |
| Breast ca. BT 549 | **100.0** | Fetal Skeletal Muscle | 11.1 |
| Breast ca. T47D | 40.1 | Skeletal Muscle Pool | 11.8 |
| Breast ca. MDA-N | 28.1 | Spleen Pool | 19.5 |
| Breast Pool | 38.2 | Thymus Pool | 25.7 |
| Trachea | 9.5 | CNS cancer (glio/astro) U87-MG | 34.2 |
| Lung | 6.1 | CNS cancer (glio/astro) U-118-MG | 47.0 |
| Fetal Lung | 20.0 | CNS cancer (neuro;met) SK-N-AS | 59.9 |
| Lung ca. NCI-N417 | 5.4 | CNS cancer (astro) SF-539 | 11.2 |
| Lung ca. LX-1 | 24.0 | CNS cancer (astro) SNB-75 | 35.1 |
| Lung ca. NCI-H146 | 12.1 | CNS cancer (glio) SNB-19 | 13.2 |
| Lung ca. SHP-77 | 31.0 | CNS cancer (glio) SF-295 | 53.2 |
| Lung ca. A549 | 31.0 | Brain (Amygdala) Pool | 11.7 |
| Lung ca. NCI-H526 | 7.3 | Brain (cerebellum) | 28.5 |
| Lung ca. NCI-H23 | 54.7 | Brain (fetal) | 37.4 |
| Lung ca. NCI-H460 | 42.6 | Brain (Hippocampus) Pool | 11.0 |
| Lung ca. HOP-62 | 17.6 | Cerebral Cortex Pool | 16.7 |
| Lung ca. NCI-H522 | 36.9 | Brain (Substantia nigra) Pool | 8.8 |
| Liver | 0.8 | Brain (Thalamus) Pool | 21.3 |
| Fetal Liver | 28.7 | Brain (whole) | 13.0 |
| Liver ca. HepG2 | 11.3 | Spinal Cord Pool | 9.0 |
| Kidney Pool | 53.6 | Adrenal Gland | 14.2 |
| Fetal Kidney | 37.6 | Pituitary gland Pool | 5.3 |
| Renal ca. 786-0 | 18.2 | Salivary Gland | 5.4 |
| Renal ca. A498 | 12.7 | Thyroid (female) | 4.3 |
| Renal ca. ACHN | 12.2 | Pancreatic ca. CAPAN2 | 0.0 |
| Renal ca. UO-31 | 21.9 | Pancreas Pool | 28.1 |

**Table ID. Panel 4.1D**

| **Tissue Name** | **Rel. Exp.(%) Ag4238, Run 175226771** | **Tissue Name** | **Rel. Exp.(%) Ag4238, Run 175226771** |
|---|---|---|---|
| Secondary Th 1 act | 64.2 | HUVEC IL-1 beta | 21.8 |
| Secondary Th2 act | 52.1 | HUVEC IFN gamma | 19.5 |
| Secondary Tr1 act | 34.9 | HUVEC TNF alpha + IFN gamma | 9.1 |
| Secondary Th 1 rest | 10.2 | HUVEC TNF alpha + IL4 | 20.2 |
| Secondary Th2 rest | 7.8 | HUVEC IL-11 | 22.5 |
| Secondary Tr1 rest | 8.5 | Lung Microvascular EC none | 28.3 |
| Primary Th1 act | **100.0** | Lung Microvascular EC TNFalpha + IL-1beta | 9.5 |
| Primary Th2 act | 62.4 | Microvascular Dermal EC none | 21.0 |
| Primary Tr1 act | 62.4 | Microsvasular Dermal EC TNFalpha + IL-1beta | 11.0 |
| Primary Th1 rest | 12.5 | Bronchial epithelium TNFalpha + IL 1 beta | 10.4 |
| Primary Th2 rest | 11.2 | Small airway epithelium none | 3.0 |
| Primary Tr1 rest | 24.0 | Small airway epithelium TNFalpha + IL-1beta | 6.2 |
| CD45RA CD4 lymphocyte act | 25.2 | Coronery artery SMC rest | 7.3 |
| CD45RO CD4 lymphocyte act | 49.0 | Coronery artery SMC TNFalpha + IL-1 beta | 6.2 |
| CD8 lymphocyte act | 37.6 | Astrocytes rest | 3.3 |
| Secondary CD8 lymphocyte rest | 39.5 | Astrocytes TNFalpha + IL-lbeta | 3.8 |
| Secondary CD8 lymphocyte act | 19.3 | KU-812 (Basophil) rest | 62.9 |
| CD4 lymphocyte none | 12.4 | KU-812 (Basophil) PMA/ionomycin | 79.6 |
| 2ry Th1/Th2/Tr1_anti-CD95 CH11 | 14.3 | CCD 1106 (Keratinocytes) none | 23.3 |
| LAK cells rest | 19.8 | CCD1106 (Keratinocytes) TNFalpha + IL-1beta | 14.1 |
| LAK cells IL-2 | 43.8 | Liver cirrhosis | 4.2 |
| LAK cells IL-2+IL-12 | 21.8 | NCI-H292 none | 9.3 |
| LAK cells IL-2+IFN gamma | 20.3 | NCI-H292 IL-4 | 20.2 |
| LAK cells IL-2+ IL-18 | 28.1 | NCI-H292 IL-9 | 22.1 |
| LAK cells PMA/ionomycin | 9.2 | NCI-H292 IL-13 | 16.3 |
| NK Cells IL-2 rest | 53.2 | NCI-H292 IFN gamma | 15.1 |
| Two Way MLR 3 day | 24.7 | HPAEC none | 13.8 |
| Two Way MLR 5 day | 27.2 | HPAEC TNF alpha + IL-1 beta | 14.2 |
| Two Way MLR 7 day | 24.3 | Lung fibroblast none | 10.4 |
| PBMC rest | 4.8 | Lung fibroblast TNF alpha + IL-1 beta | 5.4 |
| PBMC PWM | 27.7 | Lung fibroblast IL-4 | 6.0 |
| PBMC PHA-L | 24.0 | Lung fibroblast IL-9 | 13.0 |
| Ramos (B cell) none | 53.6 | Lung fibroblast IL-13 | 11.8 |
| Ramos (B cell) ionomycin | 45.7 | Lung fibroblast IFN gamma | 11.3 |
| B lymphocytes PWM | 35.6 | Dermal fibroblast CCD1070 rest | 52.9 |
| B lymphocytes CD40L and IL-4 | 21.0 | Dermal fibroblast CCD1070 TNF alpha | 53.6 |
| EOL-1 dbcAMP | 41.8 | Dermal fibroblast CCD1070 IL-1 beta | 23.0 |
| EOL-1 dbcAMP PMA/ionomycin | 24.5 | Dermal fibroblast IFN gamma | 13.1 |
| Dendritic cells none | 10.4 | Dermal fibroblast IL-4 | 22.1 |
| Dendritic cells LPS | 4.6 | Dermal Fibroblasts rest | 6.3 |
| Dendritic cells anti-CD40 | 10.6 | Neutrophils TNFa+LPS | 0.0 |
| Monocytes rest | 7.3 | Neutrophils rest | 2.6 |
| Monocytes LPS | 6.5 | Colon | 4.0 |
| Macrophages rest | 10.4 | Lung | 7.2 |
| Macrophages LPS | 2.0 | Thymus | 21.5 |
| HUVEC none | 18.9 | Kidney | 24.0 |
| HUVEC starved | 24.7 | | |

**Table IE. general oncology screening panel_v_2.4**

| **Tissue Name** | **Rel. Exp.(%) Ag4238, Run 268664314** | **Tissue Name** | **Rel. Exp.(%) Ag4238, Run 268664314** |
|---|---|---|---|
| Colon cancer 1 | 16.0 | Bladder cancer NAT 2 | 0.0 |
| Colon cancer NAT 1 | 4.0 | Bladder cancer NAT 3 | 0.0 |
| Colon cancer 2 | 13.3 | Bladder cancer NAT 4 | 3.4 |
| Colon cancer NAT 2 | 9.5 | Adenocarcinoma of the prostate 1 | 16.3 |
| Colon cancer 3 | 14.8 | Adenocarcinoma of the prostate 2 | 1.7 |
| Colon cancer NAT 3 | 14.5 | Adenocarcinoma of the prostate 3 | 8.9 |
| Colon malignant cancer 4 | 37.4 | Adenocarcinoma of the prostate 4 | 25.7 |
| Colon normal adjacent tissue 4 | 1.9 | Prostate cancer NAT 5 | 1.6 |
| Lung cancer 1 | 9.2 | Adenocarcinoma of the prostate 6 | 1.1 |
| Lung NAT 1 | 0.0 | Adenocarcinoma of the prostate 7 | 3.0 |
| Lung cancer 2 | **100.0** | Adenocarcinoma of the prostate 8 | 0.8 |
| Lung NAT 2 | 1.6 | Adenocarcinoma of the prostate 9 of the | 10.6 |
| Squamous cell carcinoma 3 | 26.4 | Prostate cancer NAT 10 | 0.0 |
| Lung NAT 3 | 0.0 | Kidney cancer 1 | 18.2 |
| metastatic melanoma 1 | 15.2 | KidneyNAT1 | 13.5 |
| Melanoma 2 | 1.5 | Kidney cancer 2 | 30.1 |
| Melanoma 3 | 1.9 | Kidney NAT 2 | 12.6 |
| metastatic melanoma 4 | 24.0 | Kidney cancer 3 | 27.0 |
| metastatic melanoma 5 | 38.4 | Kidney NAT 3 | 7.6 |
| Bladder cancer 1 | 0.0 | Kidney cancer 4 | 7.3 |
| Bladder cancer NAT 1 | 0.0 | Kidney NAT 4 | 3.0 |
| Bladder cancer 2 | 1.9 | | |

**CNS_neurodegeneration_v1.0 Summary:** Ag4238 This panel does not show differential expression of this gene in Alzheimer's disease. However, this expression profile confirms the presence of this gene in the brain. Please see Panel 1.4 for discussion of this gene in the central nervous system.

**General_screening_panel_v1.4 Summary:** Ag4238 Highest expression of this gene is seen in a breast cancer cell line (CT=30.2). This gene is widely expressed in this panel, with moderate expression seen in brain, colon, gastric, lung, breast, ovarian, and melanoma cancers. This expression profile suggests a role for this gene product in cell survival and proliferation. Modulation of this gene product may be useful in the treatment of cancer.

Among tissues with metabolic function, this gene is expressed at low but significant levels in pituitary, adipose, adrenal gland, pancreas, thyroid, and adult and fetal skeletal muscle, heart, and liver. This widespread expression among these tissues suggests that this gene product may play a role in normal neuroendocrine and metabolic function and that disregulated expression of this gene may contribute to neuroendocrine disorders or metabolic diseases, such as obesity and diabetes.

Interestingly, this gene is expressed at much higher levels in fetal (CT=32) when compared to adult liver (CT=37). This observation suggests that expression of this gene can be used to distinguish fetal from adult liver. In addition, the relative overexpression of this gene in fetal liver suggests that the protein product may enhance liver growth or development in the fetus and thus may also act in a regenerative capacity in the adult. Therefore, therapeutic modulation of the protein encoded by this gene could be useful in treatment of liver related diseases.

This gene is also expressed at low but significant levels in the CNS, including the hippocampus, thalamus, substantia nigra, amygdala, cerebellum and cerebral cortex. Therefore, therapeutic modulation of the expression or function of this gene may be useful in the treatment of neurologic disorders, such as Alzheimer's disease, Parkinson's disease, schizophrenia, multiple sclerosis, stroke and epilepsy.

**Panel 4.1D Summary:** Ag4238 Highest expression of this gene is seen in acutely activated Th1 cells (CT=33.2). In addition, this gene is expressed in a wide range of cell types including activated T cells, LAK cells, dermal fibroblasts, and basophils. This pattern of expression is in agreement with the expression profile in General_screening_panel_v1.4 and also suggests a role for the gene product in cell survival and proliferation. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

**general oncology screening panel_v_2.4 Summary:** Ag4238 This gene is widely expressed in this panel, with highest expression in lung cancer (CT=32.6). In addition, this gene is more highly expressed in lung and kidney cancer than in the corresponding normal adjacent tissue. In addition, significant expression of this gene is also associated with colon, and prostate cancer and also with melanoma. Thus, expression of this gene could be used as a marker of these cancers. Furthemore, therapeutic modulation of the expression or function of this gene product may be useful in the treatment of lung and kidney cancer.

### J. CG102615-01: mat8

Expression of gene CG102615-01 was assessed using the primer-probe set Ag2173, described in Table JA. Results of the RTQ-PCR runs are shown in Tables JB, JC, JD and JE.

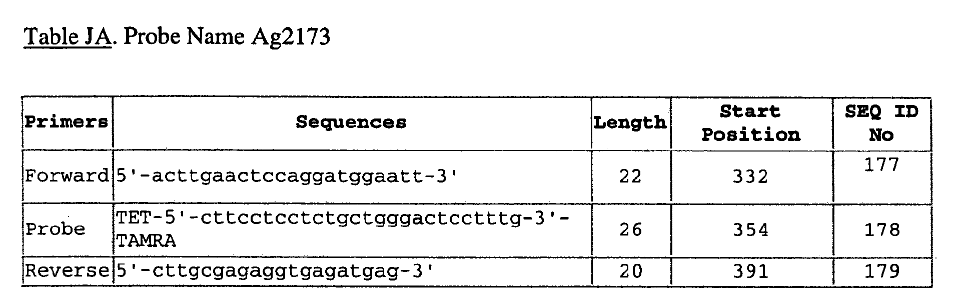

**Table JB. Panel 1.3D**

| **Tissue Name** | **Rel. Exp.(%) Ag2173, Run 162554249** | **Tissue Name** | **Rel. Exp.(%) Ag2173, Run 162554249** |
|---|---|---|---|
| Liver adenocarcinoma | 8.6 | Kidney (fetal) | 0.6 |
| Pancreas | 0.3 | Renal ca. 786-0 | 0.0 |
| Pancreatic ca. CAPAN 2 | 1.8 | Renal ca. A498 | 0.1 |
| Adrenal gland | 0.1 | Renal ca. RXF 393 | 0.0 |
| Thyroid | 0.1 | Renal ca. ACHN | 0.0 |
| Salivary gland | 3.6 | Renal ca. UO-31 | 0.2 |
| Pituitary gland | 0.3 | Renal ca. TK-10 | 0.0 |
| Brain (fetal) | 0.0 | Liver | 0.0 |
| Brain (whole) | 0.0 | Liver (fetal) | 0.0 |
| Brain (amygdala) | 0.1 | Liver ca. (hepatoblast) HepG2 | 0.6 |
| Brain (cerebellum) | 0.0 | Lung | 4.7 |
| Brain (hippocampus) | 0.2 | Lung (fetal) | 3.8 |
| Brain (substantia nigra) | 0.1 | Lung ca. (small cell) LX-1 | 1.3 |
| Brain (thalamus) Brain (thalamus) | 0.1 | Lung ca. (small cell) NCI-H69 | 1.5 |
| Cerebral Cortex | 0.5 | Lung ca. (s.cell var.) SHP-77 | 0.0 |
| Spinal cord | 0.8 | Lung ca. (large cell)NCI-H460 | 0.0 |
| glio/astro U87-MG | 0.0 | Lung ca. (non-sm. cell) A549 | 0.0 |
| glio/astro U-118-MG | 0.1 | Lung ca. (non-s.cell) | 0.0 |
| | | NCI-H23 | |
| astrocytoma SW1783 | 0.0 | Lung ca. (non-s.cell) HOP-62 | 0.0 |
| neuro*; met SK-N-AS | 0.0 | Lung ca. (non-s.cl) NCI-H522 | 0.0 |
| astrocytoma SF-539 | 0.1 | Lung ca. (squam.) SW 900 | 0.3 |
| astrocytoma SNB-75 | 0.2 | Lung ca. (squam.) NCI-H596 | 5.2 |
| glioma SNB-19 | 0.0 | Mammary gland | 3.7 |
| glioma U251 | 0.0 | Breast ca.* (pl.ef) MCF-7 | 7.9 |
| glioma SF-295 | 0.0 | Breast ca.* (pl.ef) MDA-MB-231 | 0.0 |
| Heart (fetal) | 0.0 | Breast ca.* (pl.ef) T47D | 1.0 |
| Heart | 0.1 | Breast ca. BT-549 | 0.0 |
| Skeletal muscle (fetal) | 0.6 | Breast ca. MDA-N | 0.0 |
| Skeletal muscle | 0.1 | Ovary | 1.0 |
| Bone marrow | 0.1 | Ovarian ca. OVCAR-3 | 0.2 |
| Thymus | 1.6 | Ovarian ca. OVCAR-4 | 0.1 |
| Spleen | 0.1 | Ovarian ca. OVCAR-5 | 1.7 |
| Lymph node | 0.0 | Ovarian ca. OVCAR-8 | 0.0 |
| Colorectal | **100.0** | Ovarian ca. IGROV-1 | 0.0 |
| Stomach | 5.2 | Ovarian ca.* (ascites) SK-OV-3 | 0.0 |
| Small intestine | 0.5 | Uterus | 0.1 |
| Colon ca. SW480 | 0.0 | Placenta | 3.1 |
| Colon ca.* SW620(SW480 met) | 0.1 | Prostate | 12.7 |
| Colon ca. HT29 | 2.9 | Prostate ca.* (bone met)PC-3 | 0.0 |
| Colon ca. HCT-116 | 0.2 | Testis | 0.0 |
| Colon ca. CaCo-2 | 3.3 | Melanoma Hs688(A).T | 0.0 |
| Colon ca. tissue(ODO3866) | 6.1 | Melanoma* (met) Hs688(B).T | 0.0 |
| Colon ca. HCC-2998 | 1.3 | Melanoma UACC-62 | 5.1 |
| Gastric ca.* (liver met) NCI-N87 | 15.4 | Melanoma M14 | 5.4 |
| Bladder | 6.1 | Melanoma LOX IMVI | 0.0 |
| Trachea | 21.6 | Melanoma* (met) SK-MEL-5 | 0.0 |
| Kidney | 0.5 | Adipose | 0.5 |

**Table JC. Panel 2D**

| **Tissue Name** | **Rel. Exp.(%) Ag2173, Run 162309316** | **Tissue Name** | **Rel. Exp.(%) Ag2173, Run 162309316** |
|---|---|---|---|
| Normal Colon | 94.6 | Kidney Margin 8120608 | 0.3 |
| CC Well to Mod Diff (ODO3866) | 9.3 | Kidney Cancer 8120613 | 0.0 |
| CC Margin (OD03866) | 33.2 | Kidney Margin 8120614 | 0.6 |
| CC Gr.2 rectosigmoid (OD03868) | 15.7 | Kidney Cancer 9010320 Kidney Cancer 9010320 | 0.8 |
| CC Margin (OD03868) | 2.3 | Kidney Margin 9010321 | 0.3 |
| CC Mod Diff (ODO3920) | 13.7 | Normal Uterus | 0.0 |
| CC Margin (OD03920) | 40.1 | Uterus Cancer 064011 | 4.6 |
| CC Gr.2 ascend colon (ODO3921) | 52.9 | Normal Thyroid | 0.1 |
| CC Margin (ODO3921) | 36.6 | Thyroid Cancer 064010 | 0.2 |
| CC from Partial Hepatectomy (OD04309) Mets | 13.1 | Thyroid Cancer A302152 | 0.4 |
| Liver Margin (OD04309) | 0.1 | Thyroid Margin A302153 | 0.0 |
| Colon mets to lung (OD04451-01) | 11.3 | Normal Breast | 9.9 |
| Lung Margin (OD04451-02) | 8.7 | Breast Cancer (OD04566) | 10.7 |
| Normal Prostate 6546-1 | **100.0** | Breast Cancer (OD04590-01) | 37.9 |
| Prostate Cancer (OD04410) | 31.2 | Breast Cancer Mets (OD04590-03) | 58.2 |
| Prostate Margin (OD04410) | 32.3 | Breast Cancer Metastasis (OD04655-05) | 14.8 |
| Prostate Cancer (OD04720-01) | 11.1 | Breast Cancer 064006 | 11.0 |
| Prostate Margin (OD04720-02) | 22.2 | Breast Cancer 1024 | 20.4 |
| Normal Lung 061010 | 16.8 | Breast Cancer 9100266 | 26.6 |
| Lung Met to Muscle (ODO4286) | 0.1 | Breast Margin 9100265 | 10.6 |
| Muscle Margin (OD04286) | 0.1 | Breast Cancer A209073 | 27.0 |
| Lung Malignant Cancer (OD03126) | 33.0 | Breast Margin A209073 | 9.3 |
| Lung Margin (OD03126) | 13.5 | Normal Liver | 0.0 |
| Lung Cancer (OD04404) | 64.6 | Liver Cancer 064003 | 0.0 |
| Lung Margin (OD04404) | 9.9 | Liver Cancer 1025 | 0.0 |
| Lung Cancer (OD04565) | 34.4 | Liver Cancer 1026 | 0.5 |
| Lung Margin (OD04565) | 5.5 | Liver Cancer 6004-T | 0.0 |
| Lung Cancer (OD04237-01) | 4.0 | Liver Tissue 6004-N | 0.1 |
| Lung Margin (OD04237-02) | 10.7 | Liver Cancer 6005-T | 0.4 |
| Ocular Mel Met to Liver (OD04310) | 0.0 | Liver Tissue 6005-N | 0.0 |
| Liver Margin (OD04310) | 0.0 | (Normal Bladder | 11.0 |
| Melanoma Mets to Lung (OD04321) | 5.1 | Bladder Cancer 1023 | 15.5 |
| Lung Margin (OD04321) | 9.5 | Bladder Cancer A302173 | 20.4 |
| Normal Kidney | 0.6 | Bladder Cancer (OD04718-01) | 84.7 |
| Kidney Ca, Nuclear grade 2 (OD04338) | 0.5 | Bladder Normal Adjacent (OD04718-03) | 0.3 |
| Kidney Margin (OD04338) | 1.4 | Normal Ovary | 0.5 |
| Kidney Ca Nuclear grade 1/2 (OD04339) | 0.0 | Ovarian Cancer 064008 | 5.5 |
| Kidney Margin (OD04339) | 0.5 | Ovarian Cancer (OD04768-07) | 3.0 |
| Kidney Ca, Clear cell type (OD04340) | 0.0 | Ovary Margin (OD04768-08) | 0.0 |
| Kidney Margin (OD04340) | 0.7 | Normal Stomach | 18.3 |
| Kidney Ca, Nuclear grade 3 (OD04348) | 0.1 | Gastric Cancer 9060358 | 3.3 |
| Kidney Margin (OD04348) | 0.4 | Stomach Margin 9060359 | 22.5 |
| Kidney Cancer (OD04622-01) | 0.1 | Gastric Cancer 9060395 | 24.8 |
| Kidney Margin (OD04622-03) | 1.1 | Stomach Margin 9060394 | 20.6 |
| Kidney Cancer (OD04450-01) | 0.0 | Gastric Cancer 9060397 | 27.2 |
| Kidney Margin (OD04450-03) | 0.9 | Stomach Margin 9060396 | 30.4 |
| Kidney Cancer 8120607 | 0.0 | Gastric Cancer 064005 | 20.6 |

**Table JD. Panel 4D**

| **Tissue Name** | **Rel. Exp.(%) Ag2173, Run 162292974** | **Tissue Name** | **Rel. Exp.(%) Ag2173, Run 162292974** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.1 | Bronchial epithelium TNFalpha + IL1beta | 61.6 |
| Primary Th2 rest | 0.0 | Small airway epithelium none | 23.2 |
| Primary Tr1 rest | 0.0 | Small airway epithelium TNFalpha + IL-1beta | 76.8 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 0.3 |
| CD4 lymphocyte none | 0.0 | KU-812 (Basophil) PMA/ionomycin | 0.3 |
| 2ry Th1/Th2/Tr1_anti-CD95 CH11 | 0.0 | CCD1106 (Keratinocytes) none | 21.2 |
| LAK cells rest | 0.0 | CCD 1106 (Keratinocytes) TNFalpha + IL-1beta | 20.4 |
| LAK cells IL-2 | 0.0 | Liver cirrhosis | 0.2 |
| LAK cells IL-2+IL-12 | 0.0 | Lupus kidney | 0.3 |
| LAK cells IL-2+IFN gamma | 0.0 | NCI-H292 none | **100.0** |
| LAK cells IL-2+ IL-18 | 0.0 | NCI-H292 IL-4 | 60.3 |
| LAK cells PMA/ionomycin | 0.0 | NCI-H292 IL-9 | 80.7 |
| NK Cells IL-2 rest | 0.0 | NCI-H292 IL-13 | 42.9 |
| Two Way MLR 3 day | 0.0 | NCI-H292 IFN gamma | 62.9 |
| Two Way MLR 5 day | 0.0 | HPAEC none | 0.0 |
| Two Way MLR 7 day | 0.0 | HPAEC TNF alpha + IL-1 beta | 0.0 |
| PBMC rest | 0.0 | Lung fibroblast none | 0.0 |
| PBMC PWM | 0.0 | Lung fibroblast TNF alpha + IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.1 |
| Dendritic cells LPS | 0.0 | Dermal fibroblast IL-4 | 0.0 |
| Dendritic cells anti-CD40 | 0.1 | IBD Colitis 2 | 0.1 |
| Monocytes rest | 0.0 | IBD Crohn's | 0.1 |
| Monocytes LPS | 0.0 | Colon | 7.4 |
| Macrophages rest | 0.0 | Lung | 3.5 |
| Macrophages LPS | 0.0 | Thymus | 0.5 |
| HUVEC none | 0.0 | Kidney | 0.6 |
| HUVEC starved | 0.0 | | |

**Table JE. Panel 5 Islet**

| **Tissue Name** | **Rel. Exp.(%) Ag2173, Run 279370794** | **Tissue Name** | **Rel. Exp.(%) Ag2173, Run 279370794** |
|---|---|---|---|
| 97457_Patient-02go_adipose | 1.9 | 94709_Donor 2 AM - A_adipose | 0.0 |
| 97476_Patient-07sk_skeletal muscle | 0.0 | 94710_Donor 2 AM - B_adipose | 0.3 |
| 97477_Patient-07ut_uterus | 0.0 | 94711_Donor 2 AM - C_adipose | 0.0 |
| 97478_Patient-07pl_placenta | 60.7 | 94712_Donor 2 AD - A_adipose | 0.0 |
| 99167_Bayer Patient 1 | 47.6 | 94713_Donor 2 AD - B_adipose | 2.1 |
| 97482_Patient-08ut_uterus | 1.2 | 94714_Donor2AD-C_adipose | 0.3 |
| 97413_Patient-08pl_placenta | 69.3 | 94742_Donor 3 U - A_Mesenchymal Stem Cells | 0.0 |
| 97486_Patient-09sk_skeletal muscle | 0.0 | 94743_Donor 3 U - B_Mesenchymal Stem Cells | 0.0 |
| 97487_Patient-09ut_uterus | 0.2 | 94730_Donor 3 AM - A_adipose | 1.1 |
| 97488_Patient-09pl_placenta | 31.6 | 94731_Donor 3 AM - B_adipose | 0.7 |
| 97492_Patient-10ut_uterus | 2.7 | 94732_Donor3AM-C_adipose | 1.0 |
| 97493_Patient-10pl_placenta | 73.7 | 94733_Donor 3 AD - A adipose | 0.8 |
| 97495_Patient-11go_adipose | 0.6 | 94734_Donor 3 AD - B_adipose | 0.2 |
| 97496_Patient-11sk_skeletal muscle | 1.1 | 94735_Donor 3 AD - C_adipose | 1.0 |
| 97497_Patient-11ut_uterus | 1.0 | 77138_Liver_HepG2untreated | **100.0** |
| 97498_Patient-11p1_placenta | 31.4 | 73556_Heart_Cardiac stromal cells (primary) | 0.0 |
| 97500_Patient-12go_adipose | 0.0 | 81735_Small Intestine | 61.6 |
| 97501_Patient-12sk_skeletal muscle | 0.4 | 72409_Kidney_Proximal Convoluted Tubule | 0.0 |
| 97502_Patient-12ut_uterus | 1.3 | 82685_Small intestine_Duodenum | 12.2 |
| 97503_Patient-12pl_placenta | 68.8 | 90650_Adrenal_Adrenocortical adenoma | 0.0 |
| 94721 _Donor 2 U - A_Mesenchymal Stem Cells | 0.5 | 72410_Kidney_HRCE | 1.9 |
| 94722_Donor 2 U - B_MesenchymalStem Cells | 0.8 | 72411_Kidney_HRE | 0.6 |
| 94723_Donor 2 U - C_Mesenchymal Stem Cells | 0.3 | 73139_Uterus_Uterine smooth muscle cells | 0.0 |

**Panel 1.3D Summary:** Ag2173 Highest expression of the CG102615-01 gene is detected in colorectal sample (CT=24). Therefore, expression of this gene may be used to distinguish this sample from other samples used in this panel. In addition, significant expression of this gene is seen in number of cancer cell lines including melanoma, ovarian, breast, lung, colon, pancreatic and liver cancer cell lines. The CG102615-01 gene codes for chloride conductane inducer protein MAT-8 precursor. MAT-8 is known to mediate chloride flow, affecting the membrane potential of the cell (Morrison et al., 1995, J. Biol. Chem. 270:2176-2182, PMID=7836447). Changes in membrane potential can affect tumor cell and associated smooth muscle cells (therefore tumor-induced vasculature) growth and motility. In this respect the expression of this gene in fetal muscle is an indication of a role in muscle growth/development. Therapeutic targeting of the CG102615-01 gene product with a monoclonal antibody is anticipated to limit or block the extent of tumor cell growth and motility and tumor associated angiogenesis, preferably in breast, ovarian bladder, lung tumors.

Among tissues with metabolic or endocrine function, this gene is expressed at high to moderate levels in pancreas, adipose, thyroid, pituitary gland, skeletal muscle, heart, and the gastrointestinal tract. Therefore, therapeutic modulation of the activity of this gene may prove useful in the treatment of endocrine/metabolically related diseases, such as obesity and diabetes.

In addition, this gene is expressed at low levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

**Panel 2D Summary:** Ag2173 Highest expression of the CG102615-01 gene is detected in normal prostate (CT=22.8). High expression of this gene is seen in normal and cancer samples derived from colon, prostate, lung, melanoma, uterus, thyroid, breast, liver, bladder, ovary, and stomach. Interestingly, expression of this gene is higher in ovarian, bladder, breast, uterine, and lung cancer samples as compared to their corresponding adjacent control samples. Therefore, therapeutic targeting of the CG102615-01 gene product with antibodies or small molecule inhibitors is anticipated to limit or block the extent of tumor cell growth and motility as well as tumor associated angiogenesis, preferably in ovarian, bladder, breast, uterine, and lung cancer.

Also the expression of this gene is decreased in kidney cancers compared to the normal adjacent tissues. Hence the protein product or fragments of this protein may be useful in the treatment of kidney cancer.

**Panel 4D Summary:** Ag2173 Highest expression of the CG102615-01 gene is detected in NCI-H292 cells (CT=23.8). High to moderate level of this gene is also found in lung derived cell types: small airway and bronchial epithelium treated with TNF-a and Il-1, lung fibroblast treated with IFN. This pattern of expression suggests a role for this gene in pathology of lung inflammatory dideases. Therefore therapeutic modulation of this gene product may be beneficial in the treatment of asthma, emphysema or lung infection. Interestingly, high to low levels of expression of this gene is also seen in keratinocytes, basophils, IFN gamma treated dermal fibroblasts, liver cirrhosis, IBD colitis and Crohn's disease samples and normal tissues represented by colon, lung, thymus and kidney. Therefore, therapeutic modulation of this gene product may be beneficial in the treatment of autoimmune and inflammatory disease associated with these cell types and tissues including asthma, allergies, inflammatory bowel disease, lupus erythematosus, psoriasis, rheumatoid arthritis, and osteoarthritis.

**Panel 5 Islet Summary:** Ag2173 Highest expression of the CG102615-01 gene is detected in untreated liver HepG2 samples (CT=28.6). In addition, high to low levels of expression of this gene is also seen in islet cells, placenta, uterus, small intestine, kidney and adipose tissues. The CG102615-01 gene codes for chloride conductane inducer protein MAT-8 precursor. MAT-8 is known to mediate chloride flow, affecting the membrane potential of the cell (Morrison et al., 1995, J. Biol. Chem. 270:2176-2182, PMID=7836447). Since membrane potential is critical in the secretory process in the beta cell, therapeutic modulation of the activity of this gene may prove useful in enhancing insulin secretion in Type II diabetes.

### K. CG102646-01: High Affinity Proline Permease Like

Expression of gene CG102646-01 was assessed using the primer-probe set Ag4241, described in Table KA.

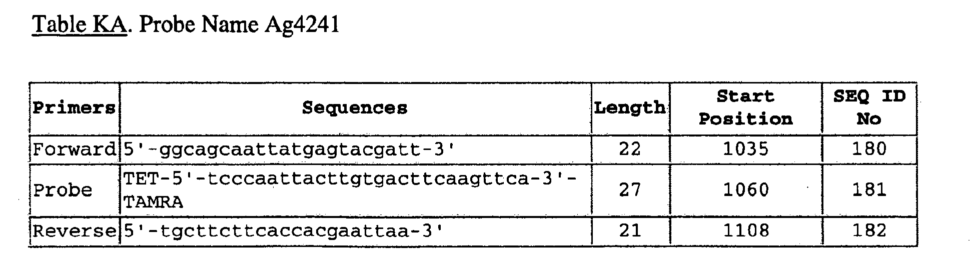

### L. CG102878-01 and CG102878-02: Hypothetical Transmembrane

Expression of gene CG102878-O1 and CG102878-02 was assessed using the primer-probe set Ag4246, described in Table LA. Results of the RTQ-PCR runs are shown in Tables LB, LC, LD and LE. Please note that CG102878-02 represents a full-length physical clone of the CG102878-01 gene, validating the prediction of the gene sequence.

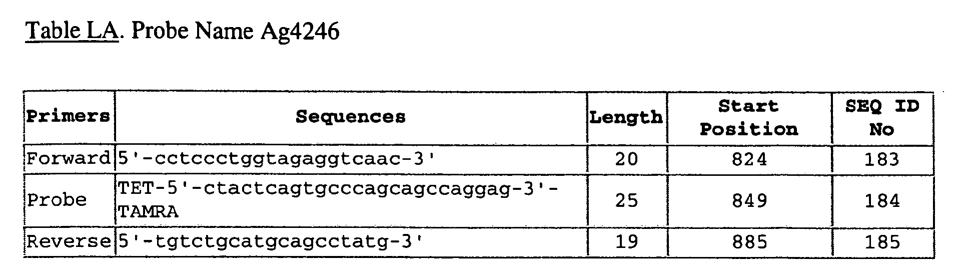

**Table LB. CNS_neurodegeneration_v1.0**

| **Tissue Name** | **Rel. Exp.(%) Ag4246, Run 224077627** | **Tissue Name** | **Rel. Exp.(%) Ag4246, Run 224077627** |
|---|---|---|---|
| AD 1 Hippo | 31.0 | Control (Path) 3 Temporal Ctx | 40.3 |
| AD 2 Hippo | 56.3 | Control (Path) 4 Temporal Ctx | 46.3 |
| AD 3 Hippo | 22.4 | AD I Occipital Ctx | 20.6 |
| AD 4 Hippo | 28.5 | AD 2 Occipital Ctx (Missing) | 0.0 |
| AD 5 Hippo | 79.0 | AD 3 Occipital Ctx | 21.3 |
| AD 6 Hippo | 76.3 | AD 4 Occipital Ctx | 31.6 |
| Control 2 Hippo | 65.5 | AD 5 Occipital Ctx | 83.5 |
| Control 4 Hippo | 49.0 | AD 6 Occipital Ctx | 41.5 |
| Control (Path) 3 Hippo | 35.4 | Control 1 Occipital Ctx | 20.2 |
| AD 1 Temporal Ctx | 30.1 | Control 2 Occipital Ctx | 92.0 |
| AD 2 Temporal Ctx | 36.3 | Control 3 Occipital Ctx | 70.7 |
| AD 3 Temporal Ctx | 14.3 | Control 4 Occipital Ctx | 28.7 |
| AD 4 Temporal Ctx | 28.1 | Control (Path) 1 Occipital Ctx | **100.0** |
| AD 5 Inf Temporal Ctx: | 80.7 | Control (Path) 2 Occipital Ctx | 49.0 |
| AD 5 Sup Temporal Ctx | 66.4 | Control (Path) 3 Occipital Ctx | 36.1 |
| AD 6 Inf Temporal Ctx | 64.2 | Control (Path) 4 Occipital Ctx | 60.3 |
| AD 6 Sup Temporal Ctx | 78.5 | Control 1 Parietal Ctx | 24.8 |
| Control 1 Temporal Ctx | 35.4 | Control 2 Parietal Ctx | 83.5 |
| Control 2 Temporal Ctx | 68.3 | Control 3 Parietal Ctx | 34.4 |
| Control 3 Temporal Ctx | 41.2 | Control (Path) 1 Parietal Ctx | 71.2 |
| Control 3 Temporal Ctx | 32.3 | Control (Path) 2 Parietal Ctx | 52.5 |
| Control (Path) 1 Temporal Ctx | 55.5 | Control (Path) 3 Parietal Ctx | 32.5 |
| Control (Path) 2 Temporal Ctx | 67.8 | Control (Path) 4 Parietal Ctx | 76.8 |

**Table LC. General_screening_panel_v1.4**

| **Tissue Name** | **Rel. Exp.(%) Ag4246, Run 222018714** | **Tissue Name** | **Rel. Exp.(%) Ag4246, Run 222018714** |
|---|---|---|---|
| Adipose | 1.1 | Renal ca. TK-10 | 3.5 |
| Melanoma* Hs688(A).T | 4.8 | Bladder | 5.7 |
| Melanoma* Hs688(B).T | 2.8 | Gastric ca. (liver met.) NCI-N87 | 12.8 |
| Melanoma* M14 | 3.1 | Gastric ca. KATO III | 12.7 |
| Melanoma* LOXIMVI | 3.3 | Colon ca. SW-948 | 6.2 |
| Melanoma* SK-MEL-5 | 3.2 | Colon ca. SW480 | 12.0 |
| Squamous cell carcinoma SCC-4 | 1.6 | Colon ca.* (SW480 met) SW620 | 7.5 |
| Testis Pool | 2.5 | Colon ca. HT29 | 7.1 |
| Prostate ca.* (bone met) PC-3 | 1.6 | Colon ca. HCT-116 | 8.4 |
| Prostate Pool | 2.7 | Colon ca. CaCo-2 | 10.6 |
| Placenta | 2.6 | Colon cancer tissue | 4.8 |
| Uterus Pool | 0.8 | Colon ca. SW1116 | 9.7 |
| Ovarian ca. OVCAR-3 | 18.7 | Colon ca. Colo-205 | 4.0 |
| Ovarian ca. SK-OV-3 | 14.1 | Colon ca. SW-48 | 5.0 |
| Ovarian ca. OVCAR-4 | 3.3 | Colon Pool | 4.9 |
| Ovarian ca. OVCAR-5 | 27.5 | Small Intestine Pool | 3.4 |
| Ovarian ca. IGROV-1 | 14.9 | Stomach Pool | 1.9 |
| Ovarian ca. OVCAR-8 | 14.2 | Bone Marrow Pool | 1.1 |
| Ovary | 3.3 | Fetal Heart | 3.8 |
| Breast ca. MCF-7 | 5.3 | Heart Pool | 2.4 |
| Breast ca. MDA-MB-231 | 9.4 | Lymph Node Pool | 3.2 |
| Breast ca. BT 549 | 13.5 | Fetal Skeletal Muscle | 0.6 |
| Breast ca. T47D | **100.0** | Skeletal Muscle Pool | 3.5 |
| Breast ca. MDA-N | 9.7 | Spleen Pool | 4.5 |
| Breast Pool | 4.2 | Thymus Pool | 2.6 |
| Trachea | 2.6 | CNS cancer (glio/astro) U87-MG | 12.5 |
| Lung | 0.4 | CNS cancer (glio/astro) U-118-MG | 9.1 |
| Fetal Lung | 3.7 | CNS cancer (neuro;met) SK-N-AS | 11.3 |
| Lung ca. NCI-N417 | 1.9 | CNS cancer (astro) SF-539 | 5.1 |
| Lung ca. LX-1 | 6.9 | CNS cancer (astro) SNB-75 | 13.5 |
| Lung ca. NCI-H146 | 4.2 | CNS cancer (glio) SNB-19 | 13.3 |
| Lung ca. SHP-77 | 1.4 | CNS cancer (glio) SF-295 | 21.8 |
| Lung ca. A549 | 3.8 | Brain(Amygdala) Pool | 3.8 |
| Lung ca. NCI-H526 | 5.7 | Brain (cerebellum) | 4.7 |
| Lung ca. NCI-H23 | 4.7 | Brain (fetal) | 1.4 |
| Lung ca. NCI-H460 | 2.8 | Brain (Hippocampus) Pool | 4.7 |
| Lung ca. HOP-62 | 6.7 | Cerebral Cortex Pool | 6.4 |
| Lung ca. NCI-H522 | 4.5 | Brain (Substantia nigra) Pool | 9.3 |
| Liver | 2.0 | Brain (Thalamus) Pool | 6.0 |
| Fetal Liver | 2.4 | Brain (whole) | 2.8 |
| Liver ca. HepG2 | 4.5 | Spinal Cord Pool | 6.4 |
| Kidney Pool | 4.5 | Adrenal Gland | 3.1 |
| Fetal Kidney | 3.2 | Pituitary gland Pool | 4.3 |
| Renal ca. 786-0 | 6.7 | Salivary Gland | 4.6 |
| Renal ca. A498 | 5.4 | Thyroid (female) | 6.9 |
| Renal ca. ACHN | 3.6 | Pancreatic ca. CAPAN2 | 9.5 |
| Renal ca. UO-31 | 6.3 | Pancreas Pool | 6.0 |

**Table LD. Panel 4.1D**

| **Tissue Name** | **Rel. Exp.(%) Ag4246, Run 175165709** | **Tissue Name** | **Rel. Exp.(%) Ag4246, Run 175165709** |
|---|---|---|---|
| Secondary Th1 act | 9.5 | HUVEC IL-1beta | 17.1 |
| Secondary Th2 act | 28.9 | HUVEC IFN gamma | 18.6 |
| Secondary Tr1 act | 13.6 | HUVEC TNF alpha + IFN gamma | 12.2 |
| Secondary Th1 rest | 12.5 | HUVEC TNF alpha + IL4 | 11.2 |
| Secondary Th2 rest | 11.9 | HUVEC IL-11 | 23.0 |
| Secondary Tr1 rest | 21.9 | Lung Microvascular EC none | 40.1 |
| Primary Th1 act | 19.8 | Lung Microvascular EC TNFalpha + IL-1beta | 40.6 |
| Primary Th2 act | 34.4 | Microvascular Dermal EC none | 26.2 |
| Primary Tr1 act | 25.0 | Microsvasular Dermal EC TNFalpha + IL-1beta | 12.9 |
| Primary Th1 rest | 11.8 | Bronchial epithelium TNFalpha + IL1beta | 21.3 |
| Primary Th2 rest | 11.2 | Small airway epithelium none | 13.3 |
| Primary Tr1 rest | 11.5 | Small airway epithelium TNFalpha + IL-1beta | 43.2 |
| CD45RA CD4 lymphocyte act | 9.4 | Coronery artery SMC rest | 25.0 |
| CD45RO CD4 lymphocyte act | 10.4 | Coronery artery SMC TNFalpha + IL-1beta | 16.5 |
| CD8 lymphocyte act | 26.4 | Astrocytes rest | 20.3 |
| Secondary CD8 lymphocyte rest | 24.0 | Astrocytes TNFalpha + IL-1beta | 15.3 |
| Secondary CD8 lymphocyte act | 22.4 | KU-812 (Basophil) rest | 10.4 |
| CD4 lymphocyte none | 13.6 | KU-812 (Basophil) PMA/ionomycin | 8.4 |
| 2ry Th1/Th2/Tr1_anti-CD95 CH11 | 28.9 | CCD1106 (Keratinocytes) none | 28.1 |
| LAK cells rest | 24.1 | CCD1106 (Keratinocytes) TNFalpha + IL- 1beta | 20.3 |
| LAK cells IL-2 | 10.9 | Liver cirrhosis | 36.3 |
| LAK cells IL-2+IL-12 | 14.5 | NCI-H292 none | 67.4 |
| LAK cells IL-2+IFN gamma | 17.4 | NCI-H292 IL-4 | 66.9 |
| LAK cells IL-2+IL-18 | 22.1 | NCI-H292 IL-9 | 74.7 |
| LAK cells PMA/ionomycin | 1.4 | NCI-H292 IL-13 | 38.4 |
| NK Cells IL-2 rest | 19.1 | NCI-H292 IFN gamma | 60.7 |
| Two Way MLR 3 day | 33.4 | IHPAEC none | 15.9 |
| Two Way MLR 5 day | 14.1 | HPAEC TNF alpha + IL-1 beta | 26.6 |
| Two Way MLR 7 day | 22.1 | Lung fibroblast none | 45.7 |
| PBMC rest | 18.0 | Lung fibroblast TNF alpha + BL-1 beta | 55.1 |
| PBMC PWM | 23.8 | Lung fibroblast IL-4 | 54.3 |
| PBMC PHA-L | 21.8 | Lung fibroblast IL-9 | 77.9 |
| Ramos (B cell) none | 67.8 | Lung fibroblast IL-13 | 32.1 |
| Ramos (B cell) ionomycin | 64.6 | Lung fibroblast IFN gamma | 32.1 |
| B lymphocytes PWM | 8.5 | Dermal fibroblast CCD1070 rest | 33.0 |
| B lymphocytes CD40L and IL-4 | 17.0 | Dermal fibroblast CCD1070 TNF alpha | 15.3 |
| EOL-1 dbcAMP | 27.4 | Dermal fibroblast CCD1070 IL-1 beta | 12.2 |
| EOL-1 dbcAMP PMA/ionomycin | 0.0 | Dermal fibroblast IFN gamma | 24.8 |
| Dendritic cells none | 50.0 | Dermal fibroblast IL-4 | 27.4 |
| Dendritic cells LPS | 36.9 | Dermal Fibroblasts rest | 38.2 |
| Dendritic cells anti-CD40 | 40.1 | Neutrophils TNFa+LPS | 0.0 |
| Monocytes rest | 37.1 | Neutrophils rest | 9.2 |
| Monocytes LPS | 4.7 | Colon | 23.7 |
| Macrophages rest | 37.9 | Lung | 16.2 |
| Macrophages LPS | 27.2 | Thymus | 39.5 |
| HUVEC none | 20.2 | Kidney | **100.0** |
| HUVEC starved | 19.2 | | |

**Table LE. general oncology screening panel_v_2.4**

| **Tissue Name** | **Rel. Exp.(%) Ag4246, Run 268664320** | **Tissue Name** | **Rel. Exp.(%) Ag4246, Run 268664320** |
|---|---|---|---|
| Colon cancer 1 | 63.7 | Bladder cancer NAT 2 | 2.4 |
| Colon cancer NAT 1 | 33.4 | Bladder cancer NAT 3 | 1.7 |
| Colon cancer 2 | 17.8 | Bladder cancer NAT 4 | 16.3 |
| Colon cancer NAT 2 | 23.3 | Adenocarcinoma of the prostate 1 | 23.8 |
| Colon cancer 3 | 82.4 | Adenocarcinoma of the prostate 2 | 11.2 |
| Colon cancer NAT 3 | 39.8 | Adenocarcinoma of the prostate 3 | 38.2 |
| Colon malignant cancer 4 | 50.3 | Adenocarcinoma of the prostate 4 | 25.3 |
| Colon normal adjacent tissue 4 | 10.2 | Prostate cancer NAT 5 | 15.8 |
| Lung cancer 1 | 26.1 | Adenocarcinoma of the prostate 6 | 12.9 |
| Lung NAT 1 | 1.9 | Adenocarcinoma of the prostate 7 | 16.4 |
| Lung cancer 2 | 74.7 | Adenocarcinoma of the prostate 8 | 2.9 |
| Lun NAT 2 | 5.1 | Adenocarcinoma of the prostate 9 | 33.7 |
| Squamous cell carcinoma 3 | 25.7 | Prostate cancer NAT 10 | 14.9 |
| Lung NAT 3 | 1.9 | Kidney cancer 1 | 49.7 |
| metastatic melanoma 1 | 22.5 | KidneyNAT 1 | 23.3 |
| Melanoma 2 | 6.6 | Kidney cancer 2 | 87.1 |
| Melanoma 3 | 2.8 | Kidney NAT 2 | 90.1 |
| metastatic melanoma 4 | 45.7 | Kidney cancer 3 | 90.8 |
| metastatic melanoma 5 | 43.8 | Kidney NAT 3 | 20.3 |
| Bladder cancer 1 | 0.4 | Kidney cancer 4 | **100.0** |
| Bladder cancer NAT 1 | 0.0 | Kidney NAT 4 | 63.7 |
| Bladder cancer 2 | 11.6 | | |

**CNS_neurodegeneration_v1.0 Summary:** Ag4246 This panel confirms the expression of the CG102878-01 gene at low levels in the brain in an independent group of individuals. This gene is found to be down-regulated in the temporal cortex of Alzheimer's disease patients. Therefore, up-regulation of this gene or its protein product, or treatment with specific agonists for this receptor may be of use in reversing the dementia/memory loss associated with this disease and neuronal death.

**General_screening_panel_v1.4 Summary:** Ag4246 Highest expression of the CG102878-01 gene is detected in breast cancer T47D cell line (CT=26.2). Significant expression of this gene is also seen in clusters of cancer cell lines derived from melanoma, pancreatic, renal, gastric, colon, lung, breast, ovarian and brain cancers. Thus, expression of this gene could be used as a marker to detect the presence of these cancers. Furthermore, therapeutic modulation of the expression or function of this gene may be effective in the treatment of gastric, colon, lung, breast, ovarian and brain cancer.

Among tissues with metabolic or endocrine function, this gene is expressed at high to moderate levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

In addition, this gene is expressed at moderate levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

**Panel 4.1D Summary:** Ag4246 Highest expression of the CG102878-01 gene is detected in kidney (CT=31.9). This gene is expressed at moderate to low levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. This pattern is in agreement with the expression profile in General_screening_panel_v1.4 and also suggests a role for the gene product in cell survival and proliferation. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

**general oncology screening panel_v_2.4 Summary:** Ag4246 Highest expression of the CG102878-01 gene is detected in kidney cancer sample (CT=31). Moderate to low levels of expression of this gene is seen in normal and cancer samples derived from kidney, colon, lung, and prostate. Significant expression of this gene is also seen in metastatic melanoma. Interestingly, expression of this gene is higher in lung cancer samples as compared to adjacent control samples. Therefore, expression of this gene may be used as diagnostic marker for lung cancer and metastic melanoma. Furtherermore, therapeutic modulation of this gene product may be beneficial in the treatment of melanoma, colon, lung, prostate and some cases of kidney cancer.

### M. CG103459-01: Novel Peptide/Histidine Transporter

Expression of gene CG103459-01 was assessed using the primer-probe set Ag4262, described in Table MA. Results of the RTQ-PCR runs are shown in Tables MB, MC and MD.

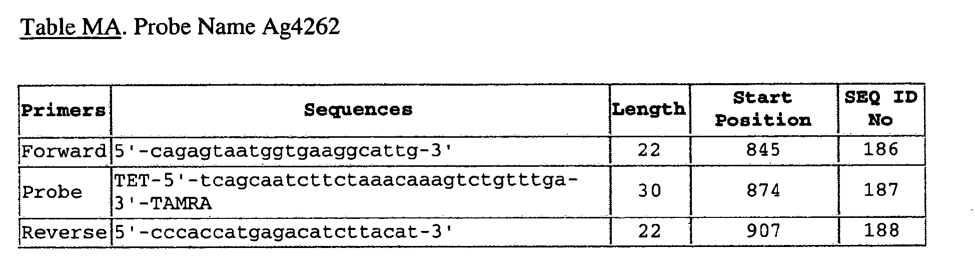

**Table MB. CNS_neurodegeneration_v1.0**

| **Tissue Name** | **Rel. Exp.(%) Ag4262, Run 224076196** | **Tissue Name** | **Rel. Exp.(%) Ag4262, Run 224076196** |
|---|---|---|---|
| AD 1 Hippo | 6.8 | Control (Path) 3 Temporal Ctx | 3.5 |
| AD 2 Hippo | 18.8 | Control (Path) 4 Temporal Ctx | 15.3 |
| AD 3 Hippo | 6.7 | AD 1 Occipital Ctx | 11.0 |
| AD 4 Hippo | 10.2 | AD 2 Occipital Ctx (Missing) | 0.0 |
| AD 5 Hippo | 32.8 | AD 3 Occipital Ctx | 4.9 |
| AD 6 Hippo | **100.0** | AD 4 Occipital Ctx | 12.2 |
| Control 2 Hippo | 17.6 | AD 5 Occipital Ctx | 20.0 |
| Control 4 Hippo | 42.0 | AD 6 Occipital Ctx | 32.3 |
| Control (Path) 3 Hippo | 20.6 | Control 1 Occipital Ctx | 3.6 |
| AD 1 Temporal Ctx | 10.7 | Control 2 Occipital Ctx | 29.1 |
| AD 2 Temporal Ctx | 19.5 | Control 3 Occipital Ctx | 8.2 |
| AD 3 Temporal Ctx | 4.0 | Control 4 Occipital Ctx | 7.4 |
| AD 4 Temporal Ctx | 14.4 | Control (Path) 1 Occipital Ctx | 27.4 |
| AD 5 Inf Temporal Ctx | 42.9 | Control (Path) 2 Occipital Ctx | 6.0 |
| AD 5 Sup Temporal Ctx | 68.8 | Control (Path) 3 Occipital Ctx | 4.1 |
| AD 6 Inf Temporal Ctx | 72.7 | Control (Path) 4 Occipital Ctx | 9.9 |
| AD 6 Sup Temporal Ctx | 68.8 | Control 1 Parietal Ctx | 4.7 |
| Control 1 Temporal Ctx | 7.7 | Control 2 Parietal Ctx | 33.2 |
| Control 2 Temporal Ctx | 16.8 | Control 3 Parietal Ctx | 9.0 |
| Control 3 Temporal Ctx | 9.4 | Control (Path) 1 Parietal Ctx | 27.0 |
| Control 3 Temporal Ctx | 9.5 | Control (Path) 2 Parietal Ctx | 12.6 |
| Control (Path) 1 Temporal Ctx | 25.9 | Control (Path) 3 Parietal Ctx | 4.3 |
| Control (Path) 2 Temporal Ctx | 15.6 | Control (Path) 4 Parietal Ctx | 14.8 |

**Table MC. General_screening_panel_v1.4**

| **Tissue Name** | **Rel. Exp.(%) Ag4262, Run 222046622** | **Tissue Name** | **Rel. Exp.(%) Ag4262, Run 222046622** |
|---|---|---|---|
| Adipose | 8.0 | Renal ca. TK-10 | 34.2 |
| Melanoma* Hs688(A).T | 35.4 | Bladder | 15.8 |
| Melanoma* Hs688(B).T | 41.2 | Gastric ca. (liver met.) NCI-N87 | 20.6 |
| Melanoma* M14 | 97.9 | Gastric ca. KATO III | 36.3 |
| Melanoma* LOXIMVI | 37.9 | Colon ca. SW-948 | 13.4 |
| Melanoma* SK-MEL-5 | 40.9 | Colon ca. SW480 | 40.9 |
| Squamous cell carcinoma SCC-4 | 10.2 | Colon ca.* (SW480 met) SW620 | 19.9 |
| Testis Pool | 16.6 | Colon ca. HT29 | 15.9 |
| Prostate ca.* (bone met) PC-3 | 44.8 | Colon ca. HCT-116 | 47.6 |
| Prostate Pool | 5.3 | Colon ca. CaCo-2 | 20.6 |
| Placenta | 8.2 | Colon cancer tissue | 36.3 |
| Uterus Pool | 7.5 | Colon ca. SW1116 | 2.8 |
| Ovarian ca. OVCAR-3 | 26.2 | Colon ca. Colo-205 | 9.9 |
| Ovarian ca. SK-OV-3 | 55.9 | Colon ca. SW-48 | 8.8 |
| Ovarian ca. OVCAR-4 | 6.5 | Colon Pool | 18.8 |
| Ovarian ca. OVCAR-5 | 34.9 | Small Intestine Pool | 12.2 |
| Ovarian ca. IGROV-1 | 25.3 | Stomach Pool | 9.4 |
| Ovarian ca. OVCAR-8 | 20.0 | Bone Marrow Pool | 8.4 |
| Ovary | 9.0 | Fetal Heart | 5.0 |
| Breast ca. MCF-7 | 27.0 | Heart Pool | 7.5 |
| Breast ca. MDA-MB-231 | 46.3 | Lymph Node Pool | 26.4 |
| Breast ca. BT 549 | 63.7 | Fetal Skeletal Muscle | 7.5 |
| Breast ca. T47D | 78.5 | Skeletal Muscle Pool | 17.0 |
| Breast ca. MDA-N | 23.3 | Spleen Pool | 9.6 |
| Breast Pool | 19.2 | Thymus Pool | 14.7 |
| Trachea | 12.9 | CNS cancer (glio/astro) U87-MG | 57.8 |
| Lung | 2.0 | CNS cancer (glio/astro) U- | 65.5 |
| | | 118-MG | |
| Fetal Lung | 19.8 | CNS cancer (neuro;met) SK-N-AS | 33.4 |
| Lung ca. NCI-N417 | 3.6 | CNS cancer (astro) SF-539 | 30.6 |
| Lung ca. LX-1 | 25.9 | CNS cancer (astro) SNB-75 | **100.0** |
| Lung ca. NCI-H146 | 7.6 | CNS cancer (glio) SNB-19 | 23.2 |
| Lung ca. SHP-77 | 17.7 | CNS cancer (glio) SF-295 | 47.3 |
| Lung ca. A549 | 44.1 | Brain (Amygdala) Pool | 6.2 |
| Lung ca. NCI-H526 | 7.4 | Brain (cerebellum) | 12.1 |
| Lung ca. NCI-H23 | 33.7 | Brain (fetal) | 6.7 |
| Lung ca. NCI-H460 | 22.7 | Brain (Hippocampus) Pool | 8.8 |
| Lung ca. HOP-62 | 25.5 | Cerebral Cortex Pool | 7.5 |
| Lung ca. NCI-H522 | 31.4 | Brain (Substantia nigra) Pool | 7.6 |
| Liver | 4.4 | Brain (Thalamus) Pool | 9.8 |
| Fetal Liver | 17.8 | Brain (whole) | 9.4 |
| Liver ca. HepG2 | 19.3 | Spinal Cord Pool | 13.2 |
| Kidney Pool | 26.2 | Adrenal Gland | 9.7 |
| Fetal Kidney | 9.5 | Pituitary gland Pool | 3.7 |
| Renal ca. 786-0 | 69.3 | Salivary Gland | 6.1 |
| Renal ca. A498 | 20.6 | Thyroid (female) | 8.2 |
| Renal ca. ACHN | 38.4 | Pancreatic ca. CAPAN2 | 31.4 |
| Renal ca. UO-31 | 94.0 | Pancreas Pool | 45.7 |

**Table MD. Panel 4.1D**

| **Tissue Name** | **Rel. Exp.(%) Ag4262, Run 176243568** | **Tissue Name** | **Rel. Exp.(%) Ag4262, Run 176243568** |
|---|---|---|---|
| Secondary Th1 act | 37.1 | HUVEC IL-1beta | 28.9 |
| Secondary Th2 act | 30.4 | HUVEC IFN gamma | 18.0 |
| Secondary Tr1 act | 14.1 | HUVEC TNF alpha + IFN gamma | 34.9 |
| Secondary Th1 rest | 9.0 | HUVEC TNF alpha + IL4 | 28.5 |
| Secondary Th2 rest | 6.4 | HUVEC IL-11 | 8.4 |
| Secondary Tr1 rest | 7.4 | Lung Microvascular EC none | 27.7 |
| Primary Th1 act | 14.7 | Lung Microvascular EC TNFalpha+IL-1beta | 28.3 |
| Primary Th2 act | 17.4 | Microvascular Dermal EC none | 16.0 |
| Primary Tr1 act | 22.5 | Microsvasular Dermal EC TNFalpha + IL-1beta | 18.9 |
| Primary Th1 rest | 5.6 | Bronchial epithelium TNFalpha + IL1beta | 39.8 |
| Primary Th2 rest | 2.5 | Small airway epithelium none | 14.6 |
| Primary Tr1 rest | 13.5 | Small airway epithelium TNFalpha + IL- 1beta | 47.0 |
| CD45RA CD4 lymphocyte act | 40.1 | Coronery artery SMC rest | 20.9 |
| CD45RO CD4 | 33.7 | Coronery artery SMC | 25.9 |
| lymphocyte act | | TNFalpha + IL-1beta | |
| CD8 lymphocyte act | 31.6 | Astrocytes rest | 22.5 |
| Secondary CD8 lymphocyte rest | 20.4 | Astrocytes TNFalpha + IL-1beta | 22.2 |
| Secondary CD8 lymphocyte act | 9.7 | KU-812 (Basophil) rest | 14.0 |
| CD4 lymphocyte none | 7.0 | KU-812 (Basophil) PMA/ionomycin | 16.7 |
| 2ry Th1/Th2/Tr1_anti-CD95 CH11 | 12.3 | CCD1106 (Keratinocytes) none | 44.8 |
| LAK cells rest | 46.0 | CCD1106 (Keratinocytes) TNFalpha + IL-1beta | 27.4 |
| LAK cells IL-2 | 28.9 | Liver cirrhosis | 7.5 |
| LAK cells IL-2+IL-12 | 18.2 | NCI-H292 none | 18.0 |
| LAK cells IL-2+IFN gamma | 21.8 | NCI-H292 IL-4 | 24.0 |
| LAK cells IL-2+ IL-18 | 34.2 | NCI-H292 IL-9 | 33.2 |
| LAK cells PMA/ionomycin | 26.8 | NCI-H292 IL-13 | 27.5 |
| NK Cells IL-2 rest | 71.7 | NCI-H292 IFN gamma | 20.6 |
| Two Way MLR 3 day | 41.5 | HPAEC none | 13.0 |
| Two Way MLR 5 day | 41.5 | HPAEC TNF alpha + IL-1 beta | 41.2 |
| Two Way MLR 7 day | 25.9 | Lung fibroblast none | 27.7 |
| PBMC rest | 15.3 | Lung fibroblast TNF alpha + IL-1 beta | 29.3 |
| PBMC PWM | 27.0 | Lung fibroblast IL-4 | 24.7 |
| PBMC PHA-L | 43.8 | Lung fibroblast IL-9 | 34.9 |
| Ramos (B cell) none | 32.3 | Lung fibroblast IL-13 | 24.5 |
| Ramos (B cell) ionomycin | 51.1 | Lung fibroblast IFN gamma | 52.1 |
| B lymphocytes PWM | 28.3 | Dermal fibroblast CCD 1070 rest | 31.6 |
| B lymphocytes CD40L and IL-4 | 66.4 | Dermal fibroblast CCD1070 TNF alpha | 45.1 |
| EOL-1 dbcAMP | 17.3 | Dermal fibroblast CCD1070 IL-1 beta | 28.1 |
| EOL-1 dbcAMP PMA/ionomycin | 18.2 | Dermal fibroblast IFN gamma | 28.1 |
| Dendritic cells none | 44.8 | Dermal fibroblast IL-4 | 38.7 |
| Dendritic cells LPS | 36.1 | Dermal Fibroblasts rest | 22.5 |
| Dendritic cells anti-CD40 | 42.9 | Neutrophils TNFa+LPS | 56.6 |
| Monocytes rest | 49.0 | Neutrophils rest | **100.0** |
| Monocytes LPS | 47.6 | Colon | 9.4 |
| Macrophages rest | 52.9 | Lung | 16.4 |
| Macrophages LPS | 21.9 | Thymus | 23.8 |
| HUVEC none | 9.9 | Kidney | 15.2 |
| HUVEC starved | 21.2 | | |

**CNS_neurodegeneration_v1.0 Summary:** Ag4262 This panel confirms the expression of the CG103459-01 gene at low levels in the brains of an independent group of individuals. However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4262 Highest expression of the CG103459-01 gene is detected in CNS cancer (astro) SNB-75 cell line (28.7). High to moderate levels of expression of this gene is seen in cluster of cancer cell lines derived from pancreatic, gastric, colon, renal, lung, breast, ovarian, prostate, squamous cell carcinoma and brain cancers. Thus, expression of this gene could be used as a marker to detect the presence of these cancers. Furthermore, therapeutic modulation of the expression or function of this gene may be effective in the treatment of these cancers.

Among tissues with metabolic or endocrine function, this gene is expressed at high to moderate levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

In addition, this gene is expressed at moderate levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

**Panel 4.1D Summary:** Ag4262 Highest expression of the CG103459-01 gene is detected in resting neutrophils (CT=29.3). This gene is expressed at high to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. This pattern is in agreement with the expression profile in General_screening_panel_v1.4 and also suggests a role for the gene product in cell survival and proliferation. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### N. CG104210-01: type III membrane protein

Expression of gene CG104210-01 was assessed using the primer-probe set Ag4270, described in Table NA. Results of the RTQ-PCR runs are shown in Tables NB, NC, ND and NE.

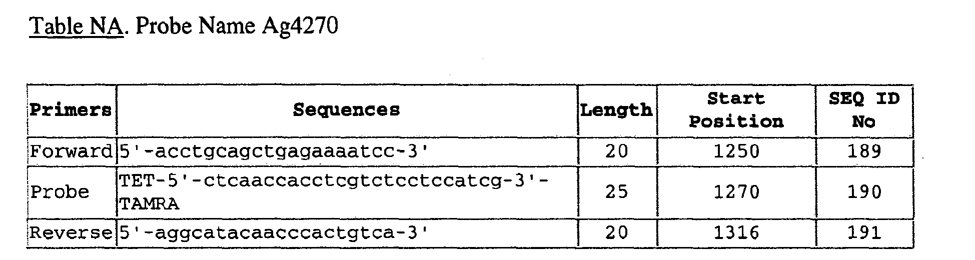

**Table NB. CNS_neurodegeneration_v1.0**

| **Tissue Name** | **Rel. Exp.(%) Ag4270, Run 224075728** | **Tissue Name** | **Rel. Exp.(%) Ag4270, Run 224075728** |
|---|---|---|---|
| AD 1 Hippo | 69.7 | Control (Path) 3 Temporal Ctx | 0.0 |
| AD 2 Hippo | 36.9 | Control (Path) 4 Temporal Ctx | 0.0 |
| AD 3 Hippo | 0.0 | AD 1 Occipital Ctx | 0.0 |
| AD 4 Hippo | 0.0 | AD 2 Occipital Ctx (Missing) | 0.0 |
| AD 5 Hippo | 0.0 | AD 3 Occipital Ctx | 0.0 |
| AD 6 Hippo | 0.0 | AD 4 Occipital Ctx | 27.7 |
| Control 2 Hippo | 20.7 | AD 5 Occipital Ctx | 0.0 |
| Control 4 Hippo | **100.0** | AD 6 Occipital Ctx | 22.2 |
| Control (Path) 3 Hippo | 37.9 | Control 1 Occipital Ctx | 17.3 |
| AD 1Temporal Ctx | 0.0 | Control 2 Occipital Ctx | 21.6 |
| AD 2 Temporal Ctx | 0.0 | Control 3 Occipital Ctx | 36.9 |
| AD 3 Temporal Ctx | 0.0 | Control 4 Occipital Ctx | 0.0 |
| AD 4 Temporal Ctx | 35.6 | Control (Path) 1 Occipital Ctx | 34.4 |
| AD 5 Inf Temporal Ctx | 0.0 | Control (Path) 2 Occipital Ctx | 0.0 |
| AD 5 Sup Temporal Ctx | 30.6 | Control (Path) 3 Occipital Ctx | 0.0 |
| AD 6 Inf Temporal Ctx | 37.1 | Control (Path) 4 | 0.0 |
| | | Occipital Ctx | |
| AD 6 Sup Temporal Ctx | 73.7 | Control 1 Parietal Ctx | 0.0 |
| Control 1 Temporal Ctx | 11.9 | Control 2 Parietal Ctx | 18.2 |
| Control 2 Temporal Ctx | 0.0 | Control 3 Parietal Ctx | 0.0 |
| Control 3 Temporal Ctx | 17.1 | Control (Path) 1 Parietal Ctx | 11.3 |
| Control 3 Temporal Ctx | 0.0 | Control (Path) 2 Parietal Ctx | 14.5 |
| Control (Path) 1 Temporal Ctx | 41.5 | Control (Path) 3 Parietal Ctx | 0.0 |
| Control (Path) 2 Temporal Ctx | 0.0 | Control (Path) 4 Parietal Ctx | 49.3 |

**Table NC. HASS Panel v1.0**

| **Tissue Name** | **Rel. Exp.(%) Ag4270, Run 268623848** | **Tissue Name** | **Rel. Exp.(%) Ag4270, Run 268623848** |
|---|---|---|---|
| MCF-7 C1 | 13.7 | U87-MG F1(B) | 0.0 |
| MCF-7 C2 | 18.4 | U87-MG F2 | 0.0 |
| MCF-7 C3 | 8.7 | U87-MG F3 | 2.4 |
| MCF-7 C4 | 22.5 | U87-MG F4 | 0.0 |
| MCF-7 C5 | 9.9 | U87-MG F5 | 0.0 |
| MCF-7 C6 | 12.6 | U87-MG F6 | 0.0 |
| MCF-7 C7 | 29.9 | U87-MG F7 | 0.0 |
| MCF-7 C9 | 8.7 | U87-MG F8 | 0.0 |
| MCF-7 C10 | 29.9 | U87-MG F9 | 0.0 |
| MCF-7 C11 | 2.7 | U87-MG F10 | 0.0 |
| MCF-7 C12 | 12.7 | U87-MG F11 | 0.0 |
| MCF-7 C13 | 40.3 | U87-MG F12 | 0.0 |
| MCF-7 C15 | 10.1 | U87-MG F13 | 0.0 |
| MCF-7 C16 | 10.1 | U87-MG F14 | 1.3 |
| MCF-7 C17 | 15.0 | U87-MG F15 | 0.0 |
| T24 D1 | 0.0 | U87-MG F16 | 0.0 |
| T24 D2 | 0.0 | U87-MG F17 | 0.0 |
| T24 D3 | 0.0 | LnCAP A1 | 60.3 |
| T24 D4 | 0.0 | LnCAPA2 | 40.1 |
| T24 D5 | 0.0 | LnCAP A3 | 74.2 |
| T24 D6 | 0.0 | LnCAP A4 | 26.1 |
| T24 D7 | 0.0 | LnCAP A5 | 33.0 |
| T24 D9 | 0.0 | LnCAP A6 | 23.0 |
| T24 D10 | 0.0 | LnCAP A7 | 68.3 |
| T24 D11 | 0.0 | LnCAP A8 | 69.7 |
| T24 D12 | 0.0 | LnCAP A9 | 38.4 |
| T24 D13 | 0.0 | LnCAP A10 | 62.0 |
| T24 D15 | 0.0 | LnCAP A11 | 100.0 |
| T24 D16 | 0.0 | LnCAP A12 | 13.2 |
| T24 D17 | 0.0 | LnCAP A13 | 51.4 |
| CAPaN B1 | 39.0 | LnCAP A14 | 30.1 |
| CAPaN B2 | 16.8 | LnCAP A15 | 22.5 |
| CAPaN B3 | 17.7 | LnCAP A16 | 48.0 |
| CAPaN B4 | 16.8 | LnCAP A 17 | 70.2 |
| CAPaN B5 | 26.6 | Primary Astrocytes | 0.0 |
| CAPaN B6 | 5.7 | Primary Renal Proximal Tubule Epithelial cell A2 | 0.0 |
| CAPaN B7 | 24.3 | Primary melanocytes A5 | 0.0 |
| CAPaN B8 | 27.2 | 126443 - 341 medullo | 4.2 |
| CAPaN B9 | 18.3 | 126444 - 487 medullo | 0.0 |
| CAPaN B10 | 67.8 | 126445 - 425 medullo | 0.0 |
| CAPaN B11 | 81.8 | 126446 - 690 medullo | 1.3 |
| CAPaN B12 | 23.0 | 126447 - 54 adult glioma | 0.0 |
| CAPaN B13 | 71.2 | 126448 - 245 adult glioma | 0.0 |
| CAPaN B14 | 27.9 | 126449 - 317 adult glioma | 0.0 |
| CAPaN B15 | 14.8 | 126450 - 212 glioma | 0.0 |
| CAPaN B16 | 17.4 | 126451 - 456 glioma | 0.0 |
| CAPaN B17 | 48.3 | | |

**Table ND. Panel 4.1D**

| **Tissue Name** | **Rel. Exp.(%) Ag4270, Run 181080817** | **Tissue Name** | **Rel. Exp.(%) Ag4270, Run 181080817** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.4 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.5 |
| Secondary Tr1 rest | 0.0 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 1.3 |
| Primary Th2 rest | 0.0 | Small airway epithelium none | 2.0 |
| Primary Tr1 rest | 0.0 | Small airway epithelium TNFalpha + IL-1beta | 6.2 |
| CD45RA CD4 lymphocyte act | **100.0** | Coronery artery SMC rest | 0.0 |
| CD45RO CD4 lymphocyte act | 0.0 | Coronery artery SMC TNFalpha + IL-1 beta | 0.0 |
| CD8 lymphocyte act | 0.0 | Astrocytes rest | 0.0 |
| Secondary CD8 lymphocyte rest | 0.0 | Astrocytes TNFalpha + IL-1 beta | 0.0 |
| Secondary CD8 lymphocyte act | 0.0 | KU-812 (Basophil) rest | 0.0 |
| CD4 lymphocyte none | 0.0 | KU-812 (Basophil) PAM/ionomycin | 0.0 |
| 2ry Th1/Th2/Tr1_anti-CD95 CH11 | 0.0 | CCD1106 (Keratinocytes) none | 1.5 |
| LAK cells rest | 0.0 | CCD1106 (Keratinocytes) TNFalpha + IL-1beta | 0.9 |
| LAK cells IL-2 | 0.4 | Liver cirrhosis | 0.0 |
| LAK cells IL-2+IL-12 | 0.0 | NCI-H292 none | 0.8 |
| LAK cells IL-2+IFN gamma | 0.0 | NCI-H292 IL-4 | 0.5 |
| LAK cells IL-2+ IL-18 | 0.0 | NCI-H292 IL-9 | 1.6 |
| LAK cells PMA/ionomycin | 5.8 | NCI-H292 IL-13 | 0.5 |
| NK Cells IL-2 rest | 0.0 | NCI-H292 IFN gamma | 0.3 |
| Two Way MLR 3 day | 0.0 | HPAEC none | 0.0 |
| Two Way MLR 5 day | 0.5 | HPAEC TNF alpha + IL-1 beta | 0.0 |
| Two Way MLR 7 day | 0.0 | Lung fibroblast none | 0.0 |
| PBMC rest | 0.0 | Lung fibroblast TNF alpha + IL-1 beta | 0.0 |
| PBMC PWM | 0.0 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 0.0 |
| Ramos (B cell) ionomycin | 0.0 | Lung fibroblast IFN gamma | 0.0 |
| B lymphocytes PWM | 0.0 | Dermal fibroblast CCD1070 rest | 0.0 |
| B lymphocytes CD40L and IL-4 | 0.0 | Dermal fibroblast CCD1070 TNF alpha | 0.0 |
| EOL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 0.7 |
| Dendritic cells anti-CD40 | 0.0 | Neutrophils TNFa+LPS | 0.5 |
| Monocytes rest | 0.0 | Neutrophils rest | 0.5 |
| Monocytes LPS | 1.2 | Colon | 0.0 |
| Macrophages rest | 0.0 | Lung | 1.6 |
| Macrophages LPS | 0.0 | Thymus | 4.1 |
| HUVEC none | 0.0 | Kidney | 30.1 |
| HUVEC starved | 0.0 | | |

**Table NE. general oncology screening panel_v_2.4**

| **Tissue Name** | **Rel. Exp.(%) Ag4270, Run 260280401** | **Tissue Name** | **Rel. Exp.(%) Ag4270, Run 260280401** |
|---|---|---|---|
| Colon cancer 1 | 3.8 | Bladder cancer NAT 2 | 0.0 |
| Colon cancer NAT 1 | 0.0 | Bladder cancer NAT 3 | 0.0 |
| Colon cancer 2 | 10.4 | Bladder cancer NAT 4 | 0.0 |
| Colon cancer NAT 2 | 0.0 | Adenocarcinoma of the prostate 1 of the | 4.3 |
| Colon cancer 3 | 3.3 | Adenocarcinoma of the prostate 2 | 11.1 |
| Colon cancer NAT 3 | 0.0 | Adenocarcinoma of the prostate 3 | 36.3 |
| Colon malignant cancer 4 | 2.0 | Adenocarcinoma of the prostate 4 | 9.9 |
| Colon normal adjacent tissue 4 | 0.0 | Prostate cancer NAT 5 | 73.7 |
| Lung cancer 1 | 82.9 | Adenocarcinoma of the prostate 6 | 12.1 |
| Lung NAT 1 | 11.5 | Adenocarcinoma of the prostate 7 | 9.9 |
| Lung cancer 2 | 9.3 | Adenocarcinoma of the prostate 8 | 4.0 |
| Lung NAT 2 | 3.3 | Adenocarcinoma of the prostate 9 | 18.3 |
| Squamous cell carcinoma 3 | 33.7 | Prostate cancer NAT 10 | 1.8 |
| Lung NAT 3 | 15.0 | Kidney cancer 1 | 0.0 |
| metastatic melanoma 1 | **100.0** | KidneyNAT 1 | 1.9 |
| Melanoma 2 | 81.8 | Kidney cancer 2 | 2.1 |
| Melanoma 3 | 81.8 | Kidney NAT 2 | 2.1 |
| metastatic melanoma 4 | 0.0 | Kidney cancer 3 | 0.0 |
| metastatic melanoma 5 | 2.2 | Kidney NAT 3 | 0.0 |
| Bladder cancer 1 | 0.0 | Kidney cancer 4 | 0.0 |
| Bladder cancer NAT 1 | 0.0 | Kidney NAT 4 | 0.0 |
| Bladder cancer 2 | 0.0 | | |

**CNS_neurodegeneration_v1.0 Summary:** Ag4270 This panel confirms the expression of the CG104210-01 gene at low levels in the brains of an independent group of individuals. However, no differential expression of this gene was detected between Alzheimer's diseased postmortem brains and those of non-demented controls in this experiment. Low expression of this gene in brain suggests that this gene may play role in neurological disorders such as Parkinson's disease, epilepsy, multiple sclerosis, schizophrenia and depression. Therefore, therapeutic modulation of this gene may be useful in the treatment of these neurological disorders.

**HASS Panel v1.0 Summary:** Ag4270 Highest expression of the CG104210-01 gene is detected in LnCAP (A11) cell line sample (CT=31.6) that are exposed to an acidic environment. CaPAN cells also show a modest increase in gene expression when exposed to an acidic environment (A10, A11 compared to A4, A5 resp.)

This suggests a possible induction of this gene in acidotic regions of prostate and pancreatic cancer.

**Panel 4.1D Summary:** Ag4270 Highest expression of the CG104210-01 gene is detected in activated CD45RA CD4 lymphocyte (CT=29), which represent activated naive T cells. In activated memory T cells (CD45RO CD4 lymphocyte) or CD4 Th1 or Th2 cells, resting CD4 cells (CTs=40), the expression of CG104210-O1 is strongly down regulated suggesting a role for this putative protein in differentiation or activation of naive T cells. Therefore, expression of this gene may be used to distinguish this sample from other samples used in this panel. In addition, Therefore modulation of the expression and/or activity of this putative protein encoded by this gene might be beneficial for the control of autoimmune diseases and T cell mediated diseases such as arthritis, psoriasis, IBD and asthma.

Furthermore, low expression of this gene is also seen in small airway epithelium, and PMA/ionomycin treated LAK cells. In addition, moderate expression of this gene is also seen in kidney and thymus. Therefore, therapeutic modulation of this gene product may be useful in the treatment of autoimmune and inflammatory diseases involving these cell and tissue types such as asthma, COPD, arthritis, psoriasis, IBD, lupus, viral and bacterial infection.

**general oncology screening panel_v_2.4 Summary:** Ag4270 Highest expression of the CG104210-01 gene is detected in metastatic melanoma (CT=33). Significant expression of this gene is also seen in melanoma and a lung cancer (OD06850-03C) samples. Interestingly, expression of this gene in lung cancer is higher as compared to the adjacent control sample. Therefore, expression of this gene may be used as diagnostic marker for detection of melanoma and lung cancer. Furthermore, therapeutic modulation of this gene may be useful in the treatment of melanoma and lung cancer.

### O. CG104251-01: Type III membrane protein

Expression of gene CG104251-01 was assessed using the primer-probe set Ag4280, described in Table OA. Results of the RTQ-PCR runs are shown in Tables OB, OC and OD.

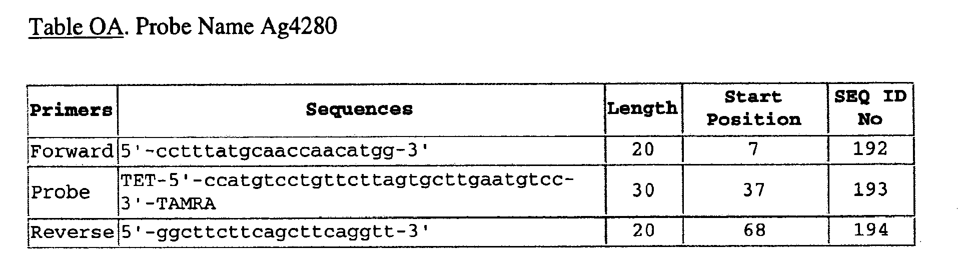

**Table OB. CNS_neurodegeneration_v1.0**

| **Tissue Name** | **Rel. Exp.(%) Ag4280, Run 224075293** | **Tissue Name** | **Rel. Exp.(%) Ag4280, Run 224075293** |
|---|---|---|---|
| AD 1 Hippo | 11.3 | Control (Path) 3 Temporal Ctx | 3.5 |
| AD 2 Hippo | 18.6 | Control (Path) 4 Temporal Ctx | 15.3 |
| AD 3 Hippo | 7.9 | AD 1Occipital Ctx | 6.0 |
| AD 4 Hippo | 6.9 | AD 2 Occipital Ctx (Missing) | 0.0 |
| AD 5 hippo | 54.3 | AD 3 Occipital Ctx | 5.3 |
| AD 6 Hippo | **100.0** | AD 4 Occipital Ctx | 5.9 |
| Control 2 Hippo | 14.8 | AD 5 Occipital Ctx | 7.4 |
| Control 4 Hippo | 13.6 | AD 6 Occipital Ctx | 33.9 |
| Control (Path) 3 Hippo | 9.9 | Control 1 Occipital Ctx | 2.6 |
| AD 1 Temporal Ctx | 10.8 | Control 2 Occipital Ctx | 30.6 |
| AD 2 Temporal Ctx | 7.1 | Control 3 Occipital Ctx | 10.4 |
| AD 3 Temporal Ctx | 5.5 | Control 4 Occipital Ctx | 6.5 |
| AD 4 Temporal Ctx | 7.4 | Control (Path) 1 Occipital Ctx | 51.4 |
| AD 5 Inf Temporal Ctx | 47.0 | Control (Path) 2 Occipital Ctx | 13.8 |
| AD 5 SupTemporal Ctx | 37.1 | Control (Path) 3 Occipital Ctx | 5.2 |
| AD 6 Inf Temporal Ctx | 42.0 | Control (Path) 4 Occipital Ctx | 12.7 |
| AD 6 Sup Temporal Ctx | 50.0 | Control 1 Parietal Ctx | 7.2 |
| Control 1 Temporal Ctx | 4.4 | Control 2 Parietal Ctx | 29.1 |
| Control 2 Temporal Ctx | 27.0 | Control 3 Parietal Ctx | 23.3 |
| Control 3 Temporal Ctx | 8.8 | Control (Path) 1 Parietal Ctx | 33.7 |
| Control 4 Temporal Ctx | 2.9 | Control (Path) 2 Parietal Ctx | 6.6 |
| Control (Path) 1 Temporal Ctx | 44.4 | Control (Path) 3 Parietal Ctx | 14.2 |
| Control (Path) 2 Temporal Ctx | 26.1 | Control (Path) 4 Parietal Ctx | 16.5 |

**Table OC. General_screening_panel_v1.4**

| **Tissue Name** | **Rel. Exp.(%) Ag4280, Run 222183179** | **Tissue Name** | **Rel. Exp.(%) Ag4280, Run 222183179** |
|---|---|---|---|
| Adipose | 3.0 | Renal ca. TK-10 | 60.7 |
| Melanoma* Hs688(A).T | 43.5 | Bladder | 23.5 |
| Melanoma* Hs688(B).T | 45.1 | Gastric ca. (liver met.) NCI-N87 | 56.6 |
| Melanoma* M14 | 23.0 | Gastric ca. KATO III | 52.9 |
| Melanoma* LOXIMVI | 20.0 | Colon ca. SW-948 | 19.3 |
| Melanoma* SK-MEL-5 | 41.5 | Colon ca. SW480 | 48.3 |
| Squamous cell carcinoma SCC-4 | 21.0 | Colon ca.* (SW480 met) SW620 | 34.4 |
| Testis Pool | 6.1 | Colon ca. HT29 | 41.2 |
| Prostate ca.* (bone met) PC-3 | 46.0 | Colon ca. HCT-116 | **100.0** |
| Prostate Pool | 5.2 | Colon ca. CaCo-2 | 35.4 |
| Placenta | 7.5 | Colon cancer tissue | 30.4 |
| Uterus Pool | 1.3 | Colon ca. SW1116 | 13.4 |
| Ovarian ca. OVCAR-3 | 88.3 | Colon ca. Colo-205 | 15.8 |
| Ovarian ca. SK-OV-3 | 52.1 | Colon ca. SW-48 | 11.1 |
| Ovarian ca. OVCAR-4 | 17.7 | Colon Pool | 4.3 |
| Ovarian ca. OVCAR-5 | 80.1 | Small Intestine Pool | 2.6 |
| Ovarian ca. IGROV-1 | 51.8 | Stomach Pool | 8.7 |
| Ovarian ca. OVCAR-8 | 29.3 | Bone Marrow Pool | 2.0 |
| Ovary | 8.4 | Fetal Heart | 6.4 |
| Breast ca. MCF-7 | 31.6 | Heart Pool | 2.1 |
| Breast ca. MDA-MB-231 | 38.4 | Lymph Node Pool | 7.7 |
| Breast ca. BT 549 | 19.8 | Fetal Skeletal Muscle | 3.5 |
| Breast ca. T47D | 79.6 | Skeletal Muscle Pool | 3.0 |
| Breast ca. MDA-N | 23.3 | Spleen Pool | 5.4 |
| Breast Pool | 8.6 | Thymus Pool | 11.5 |
| Trachea | 13.3 | CNS cancer (glio/astro) U87-MG | 55.5 |
| Lung | 3.8 | CNS cancer (glio/astro) U-118-MG | 50.3 |
| Fetal Lung | 11.5 | CNS cancer (neuro;met) SK-N-AS | 80.7 |
| Lung ca. NCI-N417 | 13.1 | CNS cancer (astro) SF-539 | 37.1 |
| Lung ca. LX-1 | 32.1 | CNS cancer (astro) SNB-75 | 61.1 |
| Lung ca. NCI-H146 | 9.9 | CNS cancer (glio) SNB-19 | 38.7 |
| Lung ca. SHP-77 | 23.5 | CNS cancer (glio) SF-295 | 47.0 |
| Lung ca. A549 | 40.1 | Brain (Amygdala) Pool | 2.3 |
| Lung ca. NCI-H526 | 8.7 | Brain (cerebellum) | 2.8 |
| Lung ca. NCI-H23 | 46.0 | Brain (fetal) | 3.1 |
| Lung ca. NCI-H460 | 23.2 | Brain (Hippocampus) Pool | 1.8 |
| Lung ca. HOP-62 | 27.7 | Cerebral Cortex Pool | 1.5 |
| Lung ca. NCI-H522 | 18.6 | Brain (Substantia nigra) Pool | 1.9 |
| Liver | 1.6 | Brain (Thalamus) Pool | 3.4 |
| Fetal Liver | 17.0 | Brain (whole) | 2.6 |
| Liver ca. HepG2 | 51.8 | Spinal Cord Pool | 2.8 |
| Kidney Pool | 8.8 | Adrenal Gland | 6.1 |
| Fetal Kidney | 13.2 | Pituitary gland Pool | 5.2 |
| Renal ca. 786-0 | 70.2 | Salivary Gland | 4.4 |
| Renal ca. A498 | 21.6 | Thyroid (female) | 7.5 |
| Renal ca. ACHN | 15.6 | Pancreatic ca. CAPAN2 | 52.1 |
| Renal ca. UO-31 | 27.4 | Pancreas Pool | 13.7 |

**Table OD. Panel 4.1D**

| **Tissue Name** | **Rel. Exp.(%) Ag4280, Run 176282949** | **Tissue Name** | **Rel. Exp.(%) Ag4280, Run 176282949** |
|---|---|---|---|
| Secondary Th1 act | 20.6 | HUVEC IL-1beta | 33.0 |
| Secondary Th2 act | 28.5 | HUVEC IFN gamma | 27.4 |
| Secondary Tr1 act | 23.7 | HUVEC TNF alpha + IFN gamma | 18.2 |
| Secondary Th1 rest | 2.8 | HUVEC TNF alpha + IL4 | 22.5 |
| Secondary Th2 rest | 6.8 | HUVEC IL-11 | 9.8 |
| Secondary Tr1 rest | 7.9 | Lung Microvascular EC none | 33.4 |
| Primary Th1 act | 10.3 | Lung Microvascular EC TNFalpha + IL-1beta | 22.8 |
| Primary Th2 act | 18.8 | Microvascular Dermal EC none | 13.8 |
| Primary Tr1 act | 15.9 | Microsvasular Dermal EC TNFalpha + IL-1beta | 10.7 |
| Primary Th1 rest Primary Th1 rest | 3.4 | Bronchial epithelium TNFalpha + IL1beta | 17.0 |
| Primary Th2 rest | 3.9 | Small airway epithelium none | 5.8 |
| Primary Tr1 rest | 5.2 | Small airway epithelium TNFalpha + IL-1 beta | 8.4 |
| CD45RA CD4 lymphocyte act | 11.2 | Coronery artery SMC rest | 25.0 |
| CD45RO CD4 lymphocyte act | 16.7 | Coronery artery SMC TNFalpha + IL-1beta | 25.9 |
| CD8 lymphocyte act | 14.6 | Astrocytes rest | 18.3 |
| Secondary CD8 lymphocyte rest | 11.1 | Astrocytes TNFalpha + IL-1beta | 17.1 |
| Secondary CD8 lymphocyte act | 5.5 | KU-812 (Basophil) rest | 19.9 |
| CD4 lymphocyte none | 1.8 | KU-812 (Basophil) PMA/ionomycin | 21.6 |
| 2ry Th1/Th2/Tr1_anti-CD95 CH11 | 4.9 | CCD1106 (Keratinocytes) none | 14.6 |
| LAK cells rest | 10.2 | CCD1106 (Keratinocytes) TNFalpha + IL-1beta | 15.2 |
| LAK cells IL-2 | 6.7 | Liver cirrhosis | 1.1 |
| LAK cells IL-2+IL-12 | 9.1 | NCI-H292 none | 11.8 |
| LAK cells IL-2+IFN gamma | 7.7 | NCI-H292 IL-4 | 15.3 |
| LAK cells IL-2+ IL-18 | 8.3 | NCI-H292 IL-9 | 14.4 |
| LAK cells PMA/ionomycin | 11.2 | NCI-H292 IL-13 | 14.8 |
| NK Cells IL-2 rest | 9.4 | NCI-H292 IFN gamma | 11.7 |
| Two Way MLR 3 day | 8.6 | HPAEC none | 12.5 |
| Two Way MLR 5 day | 7.3 | HPAEC TNF alpha + IL-1 beta | 42.9 |
| Two Way MLR 7 day | 8.0 | Lung fibroblast none | 10.7 |
| PBMC rest | 2.0 | Lung fibroblast TNF alpha + IL-1 beta | 12.3 |
| PBMC PWM | 9.5 | Lung fibroblast IL-4 | 14.4 |
| PBMC PHA-L | 5.0 | Lung fibroblast IL-9 | 18.4 |
| Ramos (B cell) none | 21.6 | Lung fibroblast IL-13 | 15.4 |
| Ramos (B cell) ionomycin | 28.3 | Lung fibroblast IFN gamma | 26.2 |
| B lymphocytes PWM | 6.0 | Dermal fibroblast CCD 1070 rest | 31.4 |
| B lymphocytes CD40L and IL-4 | 8.8 | Dermal fibroblast CCD1070 TNF alpha | 31.9 |
| EOL-1 dbcAMP | 10.6 | Dermal fibroblast CCD1070 IL-1 beta | 26.2 |
| EOL-1 dbcAMP PMA/ionomycin | 12.7 | Dermal fibroblast IFN gamma | 18.2 |
| Dendritic cells none | 8.9 | Dermal fibroblast IL-4 | 20.7 |
| Dendritic cells LPS | 5.9 | Dermal Fibroblasts rest | 10.4 |
| Dendritic cells anti-CD40 | 7.6 | Neutrophils TNFa+LPS | 1.8 |
| Monocytes rest | 5.3 | Neutrophils rest | 2.6 |
| Monocytes LPS | 10.8 | Colon | 7.7 |
| Macrophages rest | 10.2 | Lung | 12.2 |
| Macrophages LPS | 2.7 | Thymus | 21.6 |
| HUVEC none | 16.0 | Kidney | **100.0** |
| HUVEC starved | 26.6 | | |

**CNS_neurodegeneration_v1.0 Summary:** Ag4280 Very low levels of expression of the CG104251-01 gene is seen in the brains of an independent group of individuals, with highest expression in hippocampus of an Alzeimer patient (CT=34.3). However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4280 Highest expression of the CG104251-01 gene is detected in a colon cancer HCT-116 cell line (CT=30). Significant expression of this gene is also seen in clusters of cancer cell lines derived from pancreatic, gastric, colon, renal, lung, breast, ovarian, prostate, squamous cell carcinoma, melanoma and brain cancers. Thus, expression of this gene may be useful as a marker to detect the presence of these cancers. Furthermore, therapeutic modulation of the expression or function of this gene product may be effective in the treatment of these cancers.

Among tissues with metabolic or endocrine function, this gene is expressed at low levels in pancreas, adrenal gland, thyroid, pituitary gland, fetal skeletal muscle, heart, fetal liver and the gastrointestinal tract. Therefore, therapeutic modulation of the activity of this gene may prove useful in the treatment of endocrine/metabolically related diseases, such as obesity and diabetes.

Interestingly, this gene is expressed at much higher levels in fetal (CT =32.7) when compared to adult liver (CT=36). This observation suggests that expression of this gene can be used to distinguish fetal from adult liver. In addition, the relative overexpression of this gene in fetal liver suggests that the protein product may enhance liver growth or development in the fetus and thus may also act in a regenerative capacity in the adult. Therefore, therapeutic modulation of the protein encoded by this gene could be useful in treatment of liver related diseases.

In addition, low expression of this gene is also seen in brain (thalamus). Therefore, therapeutic modulation of this gene product may be beneficial in the treatment of neurological disorders.

**Panel 4.1D Summary:** Ag4280 Highest expression of the CG104251-01 gene is detected in kidney (CT=31.8). This gene is expressed at low levels in a wide range of cell types of significance in the immune response in health and disease. These cells include members of the T-cell, B-cell, and endothelial cell family, as well as epithelial and fibroblast cell types from lung and skin, and normal tissues represented by lung, thymus and kidney. This pattern of expression suggests that this gene product may be involved in homeostatic processes for these and other cell types and tissues. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### P. CG104934-01: POTENTIAL PHOSPHOLIPID-TRANSPORTING ATPASE IH

Expression of gene CG104934-01 was assessed using the primer-probe set Ag4274, described in Table PA. Results of the RTQ-PCR runs are shown in Tables PB, PC and PD.

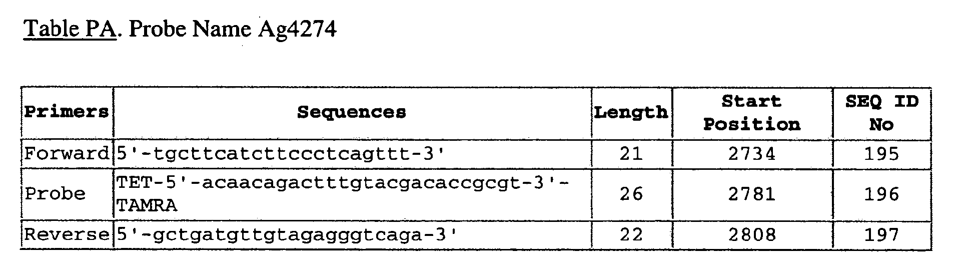

**Table PB. CNS_neurodegeneration_v1.0**

| **Tissue Name** | **Rel. Exp.(%) Ag4274, Run 224075762** | **Tissue Name** | **Rel. Exp.(%) Ag4274, Run 224075762** |
|---|---|---|---|
| AD 1 Hippo | 24.5 | Control (Path) 3 Temporal Ctx | 6.9 |
| AD 2 Hippo | 39.5 | Control (Path) 4 Temporal Ctx | 27.5 |
| AD 3 Hippo | 11.1 | AD 1 Occipital Ctx | 27.5 |
| AD 4 Hippo | 9.3 | AD 2 Occipital Ctx (Missing) | 0.0 |
| AD 5 Hippo | 86.5 | AD 3 Occipital Ctx | 13.2 |
| AD 6 Hippo | 71.2 | AD 4 Occipital Ctx | 16.0 |
| Control 2 Hippo | 27.9 | AD 5 Occipital Ctx | 31.4 |
| Control 4 Hippo | 17.4 | AD 6 Occipital Ctx | 35.1 |
| Control (Path) 3 Hippo | 13.6 | Control 1 Occipital Ctx | 0.0 |
| AD 1 Temporal Ctx | 42.6 | Control 2 Occipital Ctx | 50.3 |
| AD 2 Temporal Ctx | 34.6 | Control 3 Occipital Ctx | 39.2 |
| AD 3 Temporal Ctx | 12.5 | Control 4 Occipital Ctx | 13.2 |
| AD 4 Temporal Ctx | 36.1 | Control (Path) 1 Occipital Ctx | **100.0** |
| AD 5 Inf Temporal Ctx | 77.9 | Control (Path) 2 Occipital Ctx | 21.6 |
| AD 5 Sup Temporal Ctx | 48.0 | Control (Path) 3 Occipital Ctx | 12.3 |
| AD 6 Inf Temporal Ctx | 97.9 | Control (Path) 4 Occipital Ctx | 22.2 |
| AD 6 Sup Temporal Ctx | 66.0 | Control 1 Parietal Ctx | 10.5 |
| Control 1 Temporal Ctx | 7.7 | Control 2 Parietal Ctx | 47.0 |
| Control 2 Temporal Ctx | 25.7 | Control 3 Parietal Ctx | 24.7 |
| Control 3 Temporal Ctx | 21.6 | Control (Path) 1 Parietal Ctx | 53.2 |
| Control 3 Temporal Ctx | 10.6 | Control (Path) 2 Parietal Ctx | 25.0 |
| Control (Path) 1 Temporal Ctx | 42.0 | Control (Path) 3 Parietal Ctx | 12.3 |
| Control (Path) 2 Temporal Ctx | 29.3 | Control (Path) 4 Parietal Ctx | 40.6 |

**Table PC. General_screening_panel_v1.4**

| **Tissue Name** | **Rel. Exp.(%) Ag4274, Run 222182089** | **Tissue Name** | **Rel. Exp.(%) Ag4274, Run 222182089** |
|---|---|---|---|
| Adipose | 4.8 | Renal ca. TK-10 | 44.8 |
| Melanoma* Hs688(A).T | 4.1 | Bladder Bladder | 14.5 |
| Melanoma* Hs688(B).T | 6.0 | Gastric ca. (liver met.) NCI-N87 | 18.9 |
| Melanoma* M14 | 27.5 | Gastric ca. KATO III | 64.6 |
| Melanoma* LOXIMVI | 0.0 | Colon ca. SW-948 | 6.4 |
| Melanoma* SK-MEL-5 | 33.2 | Colon ca. SW480 | 25.3 |
| Squamous cell carcinoma SCC-4 | 9.2 | Colon ca.* (SW480 met) SW620 | 26.6 |
| Testis Pool | 7.4 | Colon ca. HT29 | 10.1 |
| Prostate ca.* (bone met) PC-3 | 20.2 | Colon ca. HCT-116 | 26.2 |
| Prostate Pool | 2.1 | Colon ca. CaCo-2 | 44.1 |
| Placenta | 7.9 | Colon cancer tissue | 20.9 |
| Uterus Pool | 3.6 | Colon ca. SW1116 | 4.0 |
| Ovarian ca. OVCAR-3 | 15.8 | Colon ca. Colo-205 | 20.9 |
| Ovarian ca. SK-OV-3 | 38.7 | Colon ca. SW-48 | 8.1 |
| Ovarian ca. OVCAR-4 | 14.0 | Colon Pool | 14.3 |
| Ovarian ca. OVCAR-5 | 19.3 | Small Intestine Pool | 8.8 |
| Ovarian ca. IGROV-1 | 9.3 | Stomach Pool | 5.9 |
| Ovarian ca. OVCAR-8 | 4.1 | Bone Marrow Pool | 3.5 |
| Ovary | 5.4 | Fetal Heart | 15.8 |
| Breast ca. MCF-7 | 8.0 | Heart Pool | 8.4 |
| Breast ca. MDA-MB-231 | 17.8 | Lymph Node Pool | 14.0 |
| Breast ca. BT 549 | 20.7 | Fetal Skeletal Muscle | 3.1 |
| Breast ca. T47D | 28.5 | Skeletal Muscle Pool | 6.1 |
| Breast ca. MDA-N | 0.0 | Spleen Pool | 7.0 |
| Breast Pool | 15.2 | Thymus Pool | 8.8 |
| Trachea | 6.5 | CNS cancer (glio/astro) U87-MG | 1.0 |
| Lung | 1.5 | CNS cancer (glio/astro) U-118-MG | 0.0 |
| Fetal Lung | **100.0** | CNS cancer (neuro;met) SK-N-AS | 4.6 |
| Lung ca. NCI-N417 | 2.2 | CNS cancer (astro) SF-539 | 3.8 |
| Lung ca. LX-1 | 48.6 | CNS cancer (astro) SNB-75 | 15.1 |
| Lung ca. NCI-H146 | 7.7 | CNS cancer (glio) SNB-19 | 11.1 |
| Lung ca. SHP-77 | 17.4 | CNS cancer (glio) SF-295 | 10.4 |
| Lung ca. A549 | 10.8 | Brain (Amygdala) Pool | 5.6 |
| Lung ca. NCI-H526 | 3.7 | Brain (cerebellum) | 10.4 |
| Lung ca. NCI-H23 | 12.8 | Brain (fetal) | 6.3 |
| Lung ca. NCI-H460 | 12.5 | Brain (Hippocampus) Pool | 5.6 |
| Lung ca. HOP-62 | 4.0 | Cerebral Cortex Pool | 7.1 |
| Lung ca. NCI-H522 | 5.8 | Brain (Substantia nigra) Pool | 6.2 |
| Liver | 2.0 | Brain (Thalamus) Pool | 6.9 |
| Fetal Liver | 10.7 | Brain (whole) | 7.5 |
| Liver ca. HepG2 | 18.6 | Spinal Cord Pool | 10.2 |
| Kidney Pool | 12.8 | Adrenal Gland | 13.2 |
| Fetal Kidney | 11.1 | Pituitary gland Pool | 6.1 |
| Renal ca. 786-0 | 17.0 | Salivary Gland | 2.7 |
| Renal ca. A498 | 11.6 | Thyroid (female) | 3.2 |
| Renal ca. ACHN | 12.2 | Pancreatic ca. CAPAN2 | 26.1 |
| Renal ca. UO-31 | 10.2 | Pancreas Pool | 22.5 |

**Table PD. Panel 4.1D**

| **Tissue Name** | **Rel. Exp.(%) Ag4274, Run 176243763** | **Tissue Name** | **Rel. Exp.(%) Ag4274, Run 176243763** |
|---|---|---|---|
| Secondary Th1 act | 58.2 | HUVEC IL-1beta | 34.9 |
| Secondary Th2 act | 55.9 | HUVEC IFN gamma | 54.3 |
| Secondary Tr1 act | 44.1 | HUVEC TNF alpha + IFN gamma | 15.8 |
| Secondary Th1 rest | 16.3 | HUVEC TNF alpha + IL4 | 31.4 |
| Secondary Th2 rest | 14.2 | HUVEC IL-11 | 30.4 |
| Secondary Tr1 rest | 24.1 | Lung Microvascular EC none | 44.4 |
| Primary Th1 act | 26.4 | Lung Microvascular EC TNFalpha + IL-1beta | 23.8 |
| Primary Th2 act | 47.6 | Microvascular Dermal EC none | 37.1 |
| Primary Tr1 act | 36.3 | Microsvasular Dermal EC TNFalpha + IL-1beta | 33.9 |
| Primary Th1 rest | 15.3 | Bronchial epithelium | 17.0 |
| | | TNFalpha + IL1beta | |
| Primary Th2 rest | 11.3 | Small airway epithelium none | 14.3 |
| Primary Tr1 rest | 25.5 | Small airway epithelium TNFalpha + IL-1beta | 27.4 |
| CD45RA CD4 lymphocyte act | 24.5 | Coronery artery SMC rest | 23.3 |
| CD45R0 CD4 lymphocyte act | 42.0 | Coronery artery SMC TNFalpha + IL-1beta | 20.3 |
| CD8 lymphocyte act | 28.5 | Astrocytes rest | 15.3 |
| Secondary CD8 lymphocyte rest | 36.1 | Astrocytes TNFalpha + IL-1beta | 10.0 |
| Secondary CD8 lymphocyte act | 12.7 | KU-812 (Basophil) rest | 50.7 |
| CD4 lymphocyte none | 14.9 | KU-812 (Basophil) PMA/ionomycin | 56.6 |
| 2ry Th1/Th2/Tr1_anti-CD95 CH11 I | 31.9 | CCD1106 (Keratinocytes) none | 36.1 |
| LAK cells rest | 55.1 | CCD1106 (Keratinocytes) TNFalpha + IL-1beta | 27.7 |
| LAK cells IL-2 | 26.2 | Liver cirrhosis | 15.7 |
| LAK cells IL-2+IL-12 | 22.4 | NCI-H292 none | 25.7 |
| LAK cells IL-2+IFN gamma | 13.3 | NCI-H292 IL-4 | 58.2 |
| LAK cells IL-2+ IL-18 | 19.2 | NCI-H292 IL-9 | 48.0 |
| LAK cells PMA/ionomycin | 26.4 | NCI-H292 IL-13 | 63.3 |
| NK Cells IL-2 rest | 26.1 | NCI-H292 IFN gamma | 29.7 |
| Two Way MLR 3 day | 33.4 | HPAEC none | 29.3 |
| Two Way MLR 5 day | 39.8 | HPAEC TNF alpha + IL-1 beta | 51.8 |
| Two Way MLR 7 day | 21.8 | Lung fibroblast none | 25.7 |
| PBMC rest | 23.2 | Lung fibroblast TNF alpha + IL-1 beta | 20.0 |
| PBMC PWM | 25.9 | Lung fibroblast IL-4 | 42.9 |
| PBMC PHA-L | 30.8 | Lung fibroblast IL-9 | 36.3 |
| Ramos (B cell) none | 0.0 | Lung fibroblast IL-13 | 48.0 |
| Ramos (B cell) ionomycin | 0.0 | Lung fibroblast IFN gamma | 25.5 |
| B lymphocytes PWM | 16.5 | Dermal fibroblast CCD1070 rest | 17.7 |
| B lymphocytes CD40L and IL-4 | 17.4 | Dermal fibroblast CCD1070 TNF alpha | 30.1 |
| EOL- 1 dbcAMP | 65.5 | Dermal fibroblast CCD1070 IL-1 beta | 10.7 |
| EOL-1 dbcAMP PMA/ionomycin | 66.4 | Dermal fibroblast IFN gamma | 20.7 |
| Dendritic cells none | 58.6 | Dermal fibroblast IL-4 | 32.1 |
| Dendritic cells LPS | 46.0 | Dermal Fibroblasts rest | 16.2 |
| Dendritic cells anti-CD40 | 65.1 | Neutrophils TNFa+LPS | 36.1 |
| Monocytes rest | 95.9 | Neutrophils rest | 73.2 |
| Monocytes LPS | **100.0** | Colon | 9.2 |
| Macrophages rest | 65.5 | Lung | 62.0 |
| Macrophages LPS | 45.7 | Thymus | 25.9 |
| HUVEC none | 28.1 | Kidney | 51.1 |
| HUVEC starved | 45.7 | | |

**CNS_neurodegeneration_v1.0 Summary:** Ag4274 This panel confirms the expression of the CG104934-01 gene at low levels in the brain in an independent group of individuals. This gene is found to be upregulated in the temporal cortex of Alzheimer's disease patients. Blockade of this receptor may be of use in the treatment of this disease and decrease neuronal death.

**General_screening_panel_v1.4 Summary:** Ag4274 Highest expression of the CG104934-01 gene is detected in fetal lung (CT=23.5). Interestingly, this gene is expressed at much higher levels in fetal (CT=23.5) when compared to adult lung (CT=29.5). This observation suggests that expression of this gene can be used to distinguish fetal from adult lung. In addition, the relative overexpression of this gene in fetal lung suggests that the protein product may enhance lung growth or development in the fetus and thus may also act in a regenerative capacity in the adult. Therefore, therapeutic modulation of the protein encoded by this gene could be useful in treatment of lung related diseases.

Significant expression of this gene is also seen in clusters of cancer cell lines derived from pancreatic, gastric, colon, renal, lung, breast, ovarian, prostate, squamous cell carcinoma, melanoma and brain cancers. Thus, expression of this gene may be used as a diagnostic marker for detection of these cancers. Furthermore, therapeutic modulation of the expression or function of this gene may be effective in the treatment of pancreatic, gastric, colon, renal, lung, breast, ovarian, prostate, squamous cell carcinoma, melanoma and brain cancers.

Among tissues with metabolic or endocrine function, this gene is expressed at high levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

The CG104934-01 gene codes for a variant of potential phospholipid-transporting ATPase, a P-type ATPase with phospholipid transporting activity. In mice, a P-type ATPase (p-locus fat-associated ATPase) was mapped to locus that deletion of which results in increase in the body fat of the mice (Dhar et al, 2000, Physiol Genomics 4(1):93-100, PMID: 11074018). Therefore, based on functional homology, the CG104934-01 gene may also play a role in modulation of the body fat in human and therapeutic modulation of this protein may be useful in the treatment of obesity and diabetes.

Mutations in the FIC1 gene, a member of phospholipid-transporting ATPase, is shown to constitute the molecular defect in familial intrahepatic cholestasis I (Byler's disease) and benign recurrent intrahepatic cholestasis (Ujhazy et al., 2001, Hepatology 34:768-75, PMID: 11584374). Thus, based on homology, potential phospholipid-transporting ATPase encoded by this gene may also play a role in pathology of Byler's disease and intrahepatic cholestasis and therapeutic modulation of this protein may be useful in the treatment of these diseases.

In addition, this gene is expressed at high levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

**Panel 4.1D Summary:** Ag4274 Highest expression of the CG104934-01 gene is detected in monocytes (CTs=28.4). This gene is expressed at high to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. This pattern is in agreement with the expression profile in General_screening_panel_v1.4 and also suggests a role for the gene product in cell survival and proliferation. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### Q. CG105463-01 and CG105463-02: Meningioma-Expressed Antigen 6/11 (MEA6) (MEA11)

Expression of gene CG105463-01 and CG105463-02 was assessed using the primer-probe set Ag4288, described in Table QA. Results of the RTQ-PCR runs are shown in Tables QB, QC and QD. Please note that CG105463-02 represents a full-length physical clone of the CG105463-01 gene, validating the prediction of the gene sequence.

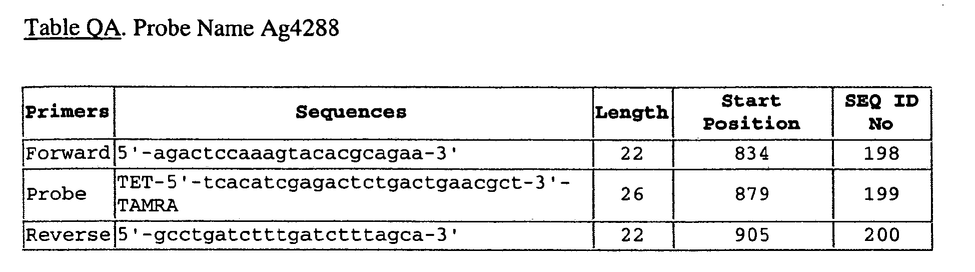

**Table QB. CNS_neurodegeneration_v1.0**

| **Tissue Name** | **Rel. Exp.(%) Ag4288, Run 224064582** | **Tissue Name** | **Rel. Exp.(%) Ag4288, Run 224064582** |
|---|---|---|---|
| AD 1 Hippo | 9.7 | Control (Path) 3 Temporal Ctx | 5.4 |
| AD 2 Hippo | 15.1 | Control (Path) 4 Temporal Ctx | 65.5 |
| AD 3 Hippo | 15.2 | AD 1 Occipital Ctx | 28.3 |
| AD 4 Hippo | 13.9 | AD 2 Occipital Ctx (Missing) | 0.0 |
| AD 5 Hippo | 63.3 | AD 3 Occipital Ctx | 2.9 |
| AD 6 Hippo | 68.3 | AD 4 Occipital Ctx | 21.8 |
| Control 2 Hippo | 13.8 | AD 5 Occipital Ctx | 19.3 |
| Control 4 Hippo | 7.7 | AD 6 Occipital Ctx | 16.5 |
| Control (Path) 3 Hippo | 7.0 | Control 1 Occipital Ctx | 2.7 |
| AD 1 Temporal Ctx | 18.3 | Control 2 Occipital Ctx | 20.3 |
| AD 2 Temporal Ctx | 15.1 | Control 3 Occipital Ctx | 30.6 |
| AD 3 Temporal Ctx | 7.9 | Control 4 Occipital Ctx | 9.5 |
| AD 4 Temporal Ctx | 24.0 | Control (Path) 1 Occipital Ctx | 41.5 |
| AD 5 Inf Temporal Ctx | 62.4 | Control (Path) 2 Occipital Ctx | 23.5 |
| AD 5 Sup Temporal Ctx | 44.1 | Control (Path) 3 Occipital Ctx | 4.6 |
| AD 6 Inf Temporal Ctx | 77.4 | Control (Path) 4 Occipital Ctx | 47.6 |
| AD 6 Sup Temporal Ctx | **100.0** | Control 1 Parietal Ctx | 7.6 |
| Control 1 Temporal Ctx | 13.7 | Control 2 Parietal Ctx | 46.0 |
| Control 2 Temporal Ctx | 5.9 | Control 3 Parietal Ctx | 7.6 |
| Control 3 Temporal | 11.7 | Control (Path) 1 | 33.0 |
| Ctx | | Parietal Ctx | |
| Control 3 Temporal Ctx | 20.3 | Control (Path) 2 Parietal Ctx | 41.5 |
| Control (Path) 1 Temporal Ctx | 36.1 | Control (Path) 3 Parietal Ctx | 6.5 |
| Control (Path) 2 Temporal Ctx | 21.8 | Control (Path) 4 Parietal Ctx | 66.4 |

**Table QC. General_screening_panel_v1.4**

| **Tissue Name** | **Rel. Exp.(%) Ag4288, Run 222182748** | **Tissue Name** | **Rel. Exp.(%) Ag4288, Run 222182748** |
|---|---|---|---|
| Adipose | 11.0 | Renal ca. TK-10 | 40.1 |
| Melanoma* Hs688(A).T | 2.6 | Bladder | 6.7 |
| Melanoma* Hs688(B).T | 1.9 | Gastric ca. (liver met.) NCI-N87 | 3.2 |
| Melanoma* M14 | 1.5 | Gastric ca. KATO III | 1.6 |
| Melanoma* LOXIMVI | 0.0 | Colon ca. SW-948 | 5.8 |
| Melanoma* SK-MEL-5 | 4.8 | Colon ca. SW480 | 15.7 |
| Squamous cell carcinoma SCC-4 | 1.4 | Colon ca.* (SW480 met) SW620 | 8.3 |
| Testis Pool | 95.9 | Colon ca. HT29 | 4.1 |
| Prostate ca.* (bone met) PC-3 | 3.5 | Colon ca. HCT-116 | 16.4 |
| Prostate Pool | 4.5 | Colon ca. CaCo-2 | **100.0** |
| Placenta | 0.6 | Colon cancer tissue | 17.6 |
| Uterus Pool | 2.7 | Colon ca. SW1116 | 1.9 |
| Ovarian ca. OVCAR-3 | 2.1 | Colon ca. Colo-205 | 0.6 |
| Ovarian ca. SK-OV-3 | 18.7 | Colon ca. SW-48 | 5.5 |
| Ovarian ca. OVCAR-4 | 0.5 | Colon Pool | 5.4 |
| Ovarian ca. OVCAR-5 | 6.4 | Small Intestine Pool | 12.4 |
| Ovarian ca. IGROV-1 | 30.1 | Stomach Pool | 5.6 |
| Ovarian ca. OVCAR-8 | 0.0 | Bone Marrow Pool | 2.6 |
| Ovary | 2.8 | Fetal Heart | 18.4 |
| Breast ca. MCF-7 | 5.5 | Heart Pool | 0.8 |
| Breast ca. MDA-MB-231 | 1.4 | Lymph Node Pool | 12.8 |
| Breast ca. BT 549 | 2.2 | Fetal Skeletal Muscle | 1.4 |
| Breast ca. T47D | 6.2 | Skeletal Muscle Pool | 9.9 |
| Breast ca. MDA-N | 1.1 | Spleen Pool | 4.2 |
| Breast Pool | 10.4 | Thymus Pool | 8.8 |
| Trachea | 7.3 | CNS cancer (glio/astro) U87-MG | 0.6 |
| Lung | 4.1 | CNS cancer (glio/astro) U-118-MG | 0.0 |
| Fetal Lung | 10.1 | CNS cancer (neuro;met) SK-N-AS | 12.0 |
| Lung ca. NCI-N417 | 0.0 | CNS cancer (astro) SF-539 | 0.0 |
| Lung ca. LX-1 | 25.5 | CNS cancer (astro) SNB- | 6.1 |
| | | | |
| Lung ca. NCI-H146 | 0.0 | CNS cancer (glio) SNB-19 | 34.6 |
| Lung ca. SHP-77 | 4.0 | CNS cancer (glio) SF-295 | 0.9 |
| Lung ca. A549 | 1.6 | Brain (Amygdala) Pool | 5.7 |
| Lung ca. NCI-H526 | 0.5 | Brain (cerebellum) | 5.8 |
| Lung ca. NCI-H23 | 5.4 | Brain (fetal) | 37.4 |
| Lung ca. NCI-H460 | 5.0 | Brain (Hippocampus) Pool | 12.2 |
| Lung ca. HOP-62 | 1.1 | Cerebral Cortex Pool | 9.4 |
| Lung ca. NCI-H522 | 1.6 | Pool Brain (Substantia nigra) | 6.5 |
| Liver | 0.0 | Brain (Thalamus) Pool | 12.5 |
| Fetal Liver | 41.5 | Brain (whole) | 4.0 |
| Liver ca. HepG2 | 62.0 | Spinal Cord Pool | 7.6 |
| Kidney Pool | 21.0 | Adrenal Gland | 0.0 |
| Fetal Kidney | 25.0 | Pituitary gland Pool | 0.6 |
| Renal ca. 786-0 | 0.5 | Salivary Gland | 0.6 |
| Renal ca. A498 | 5.0 | Thyroid (female) | 0.2 |
| Renal ca. ACHN | 0.5 | Pancreatic ca. CAPAN2 | 0.0 |
| Renal ca. UO-31 | 0.0 | Pancreas Pool | 12.1 |

**Table QD. Panel 4.1D**

| **Tissue Name** | **Rel. Exp.(%) Ag4288, Run 181981927** | **Tissue Name** | **Rel. Exp.(%) Ag4288, Run 181981927** |
|---|---|---|---|
| Secondary Th1 act | 0.4 | HUVEC IL-1beta | 0.5 |
| Secondary Th2 act | 0.6 | HUVEC IFN gamma | 0.0 |
| Secondary Tr1 act | 0.0 | HUVEC TNF alpha + IFN gamma | 0.1 |
| Secondary Th1 rest | 0.6 | HUVEC TNF alpha + IL4 | 0.0 |
| Secondary Th2 rest | 0.3 | HUVEC IL-11 | 0.0 |
| Secondary Tr 1 rest | 0.0 | Lung Microvascular EC none | 1.9 |
| Primary Th1 act | 0.0 | Lung Microvascular EC TNFalpha + IL-1beta | 0.5 |
| Primary Th2 act | 0.1 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 4.1 |
| Primary Th2 rest | 0.0 | Small airway epithelium none | 0.6 |
| Primary Tr1 rest | 0.0 | Small airway epithelium TNFalpha + IL-1beta | 1.4 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.5 |
| CD8 lymphocyte act | 0.0 | Astrocytes rest | 2.3 |
| Secondary CD8 lymphocyte rest | 0.0 | Astrocytes TNFalpha + IL-1beta | 0.8 |
| Secondary CD8 | 0.0 | KU-812 (Basophil) rest | 13.6 |
| lymphocyte act | | | |
| CD4 lymphocyte none | 0.3 | KU-812 (Basophil) PMA/ionomycin | 13.5 |
| 2ry Th1/Th2/Tr1_anti-CD95 CH11 | 0.0 | CCD1106 (Keratinocytes) none | 0.5 |
| LAK cells rest | 0.0 | CCD1106 (Keratinocytes) TNFalpha + IL-1 beta | 0.0 |
| LAK cells IL-2 | 0.0 | Liver cirrhosis | 1.1 |
| LAK cells IL-2+IL-12 | 0.0 | NCI-H292 none | 3.8 |
| LAK cells IL-2+IFN gamma | 0.0 | NCI-H292 IL-4 | 0.9 |
| LAK cells IL-2+ IL-18 | 0.6 | NCI-H292 IL-9 | 1.0 |
| LAK cells PMA/ionomycin | 0.0 | NCI-H292 IL-13 | 1.2 |
| NK Cells IL-2 rest | 0.0 | NCI-H292 IFN gamma | 0.0 |
| Two Way MLR 3 day | 0.0 | HPAEC none | 1.9 |
| Two Way MLR 5 day | 0.0 | HPAEC TNF alpha + IL-1 beta | 0.0 |
| Two Way MLR 7 day | 0.0 | Lung fibroblast none | 0.4 |
| PBMC rest | 0.7 | Lung fibroblast TNF alpha + IL-1 beta | 0.9 |
| PBMC PWM | 0.0 | Lung fibroblast IL-4 | 0.4 |
| PBMC PHA-L | 0.4 | Lung fibroblast IL-9 | 0.9 |
| Ramos (B cell) none | 0.0 | Lung fibroblast IL-13 | 0.5 |
| Ramos (B cell) ionomycin | 0.0 | Lung fibroblast IFN gamma | 0.2 |
| B lymphocytes PWM | 1.5 | Dermal fibroblast CCD1070 rest | 0.0 |
| B lymphocytes CD40L and IL-4 | 0.0 | Dermal fibroblast CCD1070 TNF alpha | 1.5 |
| EOL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 0.7 |
| Dendritic cells none | 0.9 | Dermal fibroblast IL-4 | 0.0 |
| Dendritic cells LPS | 0.0 | Dermal Fibroblasts rest | 0.5 |
| Dendritic cells anti-CD40 | 0.0 | Neutrophils TNFa+LPS | 0.4 |
| Monocytes rest | 0.0 | Neutrophils rest | 2.6 |
| Monocytes LPS | 0.8 | Colon | 6.7 |
| Macrophages rest | 0.0 | Lung | 1.8 |
| Macrophages LPS | 0.0 | Thymus | 8.8 |
| HUVEC none | 0.0 | Kidney | **100.0** |
| HUVEC starved | 0.0 | | |

**CNS_neurodegeneration_v1.0 Summary:** Ag4288 This panel confirms the expression of the CG105463-01 gene at low levels in the brains of an independent group of individuals. However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4288 Highest expression of the CG105463-01 gene is detected in colon cancer CaCo-2 cell line (CT=30). Significant expression is also seen in number of cancer cell lines derived from colon, renal, lung, liver, breast, ovarian, and brain cancers. Thus, expression of this gene as a marker to detect the presence of these cancers. Furthermore, therapeutic modulation of the expression or function of this gene may be effective in the treatment of colon, renal, lung, liver, breast, ovarian, and brain cancers.

The CG105463-01 gene codes for a homolog of meningioma-expressed antigen 6/11 (MEA6). MGEA6 is overexpressed in meningioma and glioma tumor cells. Furthermore, the immune response to MGEA6/11 is frequent in both meningioma and glioma patients (Comtesse et al., 2002, Oncogene 21(2):239-47, PMID: 11803467). Thus, based on the homology, MEA6 like protein encoded by the CG105463-01 gene may play a role in pathology of meningioma and glioma and therapeutic modulation of this gene may be beneficial in the treatment of these tumors.

Among tissues with metabolic or endocrine function, this gene is expressed at moderate to low levels in pancreas, adipose, skeletal muscle, fetal heart, fetal liver and the gastrointestinal tract. Therefore, therapeutic modulation of the activity of this gene may prove useful in the treatment of endocrine/metabolically related diseases, such as obesity and diabetes.

Interestingly, this gene is expressed at much higher levels in fetal (CTs=31-32) when compared to adult liver and heart(CTs>37). This observation suggests that expression of this gene can be used to distinguish fetal heart and liver from corresponding adult tissues. In addition, the relative overexpression of this gene in fetal tissue suggests that the protein product may enhance growth or development of heart and liver in the fetus and thus may also act in a regenerative capacity in the adult. Therefore, therapeutic modulation of MEA6 like protein encoded by this gene could be useful in treatment of heart and liver related diseases.

In addition, this gene is expressed at moderate levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

**Panel 4.1D Summary:** Ag4288 Highest expression of the CG105463-01 gene is detected in kidney (CT=29.3). Therefore, expression of this gene may be used to distinguish kidney from other samples used in this panel. Furthermore, therapeutic modulation of this gene product may be useful in the treatment of autoimmune and inflammatory disease that affect kidney, including lupus and glomerulonephritis.

In addition, moderate to low expression of this gene is also seen in TNFalpha + IL1beta treated bronchial epithelium, basophils, NCI-H292, resting neutrophils and normal tissues represented by colon and thymus. Therefore, therapeutic modulation of this gene product may be beneficial in the treatment of Crohn's disease, ulcerative colitis, multiple sclerosis, chronic obstructive pulmonary disease, asthma, emphysema, rheumatoid arthritis, lupus erythematosus, or psoriasis.

### R. CG105491-01: Serine protease

Expression of gene CG105491-01 was assessed using the primer-probe sets Ag4348, Ag4302 and Ag6953, described in Tables RA, RB and RC. Results of the RTQ-PCR runs are shown in Tables RD, RE and RF.

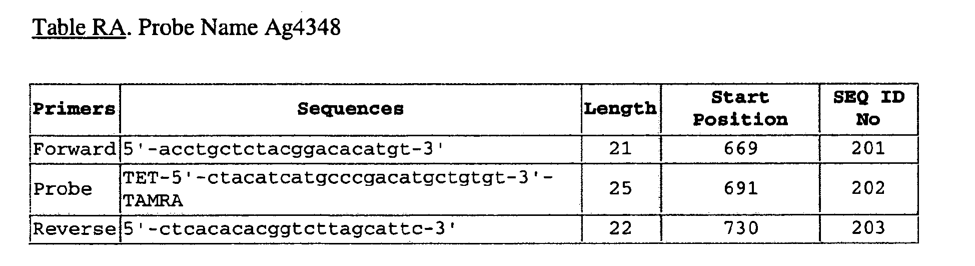

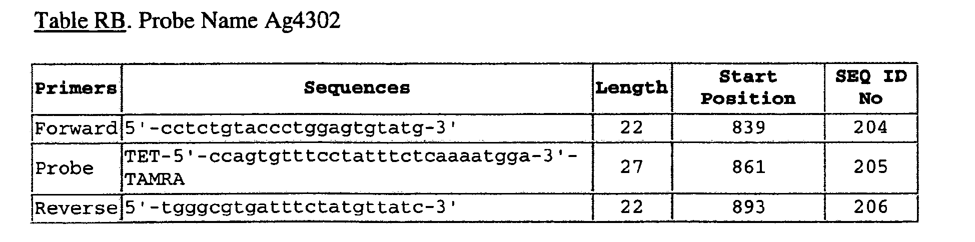

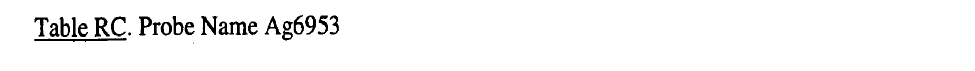

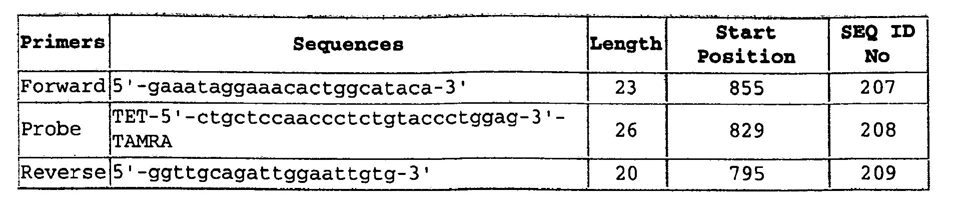

**Table RD. CNS_neurodegeneration_v1.0**

| **Tissue Name** | **Rel. Exp.(%) Ag4348, Run 224364195** | **Tissue Name** | **Rel. Exp.(%) Ag4348, Run 224364195** |
|---|---|---|---|
| AD 1 Hippo | 14.2 | Control (Path) 3 Temporal Ctx | 6.3 |
| AD 2 Hippo | 30.4 | Control (Path) 4 Temporal Ctx | 39.8 |
| AD 3 Hippo | 10.3 | AD 1 Occipital Ctx | 21.9 |
| AD 4 Hippo | 16.7 | AD 2 Occipital Ctx (Missing) | 0.0 |
| AD 5 hippo | **100.0** | AD 3 Occipital Ctx | 9.2 |
| AD 6 Hippo | 49.0 | AD 4 Occipital Ctx | 23.3 |
| Control 2 Hippo | 21.5 | AD 5 Occipital Ctx | 25.3 |
| Control 4 Hippo | 12.1 | AD 6 Occipital Ctx | 29.5 |
| Control (Path) 3 Hippo | 5.1 | Control 1 Occipital Ctx | 3.8 |
| AD 1 Temporal Ctx | 19.8 | Control 2 Occipital Ctx | 36.9 |
| AD 2 Temporal Ctx | 28.1 | Control 3 Occipital Ctx | 29.7 |
| AD 3 Temporal Ctx | 8.3 | Control 4 Ctx Occipital | 6.3 |
| AD 4 Temporal Ctx | 22.8 | Control (Path) 1 Occipital Ctx | 65.5 |
| AD 5 Inf Temporal Ctx | 59.0 | Control (Path) 2 Occipital Ctx | 18.2 |
| AD 5 SupTemporal Ctx | 48.3 | Control (Path) 3 Occipital Ctx | 2.1 |
| AD 6 Inf Temporal Ctx | 51.1 | Control (Path) 4 Occipital Ctx | 37.9 |
| AD 6 Sup Temporal Ctx | 51.8 | Control 1 Parietal Ctx | 8.2 |
| Control 1 Temporal Ctx | 6.7 | Control 2 Parietal Ctx | 53.6 |
| Control 2 Temporal Ctx | 34.2 | Control 3 Parietal Ctx | 26.2 |
| Control 3 Temporal Ctx | 26.8 | Control (Path) 1 Parietal Ctx | 56.3 |
| Control 4 Temporal Ctx | 17.0 | Control (Path) 2 Parietal Ctx | 33.7 |
| Control (Path) 1 Temporal Ctx | 62.0 | Control (Path) 3 Parietal Ctx | 6.0 |
| Control (Path) 2 Temporal Ctx | 52.9 | Control (Path) 4 Parietal Ctx | 49.7 |

**Table RE. General_screening_panel_v1.6**

| **Tissue Name** | **Rel. Exp.(%) Ag6953, Run 278388895** | **Tissue Name** | **Rel. Exp.(%) Ag6953, Run 278388895** |
|---|---|---|---|
| Adipose | 8.2 | Renal ca. TK-10 | 0.0 |
| Melanoma* Hs688(A).T | 0.0 | Bladder | 30.8 |
| Melanoma* Hs688(B).T | 0.0 | Gastric ca. (liver met.) NCI-N87 | 55.1 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 9.3 | Colon ca. SW480 | 0.0 |
| Squamous cell carcinoma SCC-4 | 26.6 | Colon ca.* (SW480 met) SW620 | 0.0 |
| Testis Pool | **100.0** | Colon ca. HT29 | 0.0 |
| Prostate ca.* (bone met) PC-3 | 9.7 | Colon ca. HCT-116 | 0.0 |
| Prostate Pool | 17.7 | Colon ca. CaCo-2 | 10.0 |
| Placenta | 0.0 | Colon cancer tissue | 0.0 |
| Uterus Pool | 3.1 | Colon ca. SW1116 | 9.7 |
| Ovarian ca. OVCAR-3 | 0.0 | Colon ca. Colo-205 | 0.0 |
| Ovarian ca. SK-OV-3 | 10.1 | Colon ca. SW-48 | 0.0 |
| Ovarian ca. OVCAR-4 | 0.0 | Colon Pool | 40.6 |
| Ovarian ca. OVCAR-5 | 24.8 | Small Intestine Pool | 44.1 |
| Ovarian ca. IGROV-1 | 0.0 | Stomach Pool | 0.0 |
| Ovarian ca. OVCAR-8 | 0.0 | Bone Marrow Pool | 16.7 |
| Ovary | 10.3 | Fetal Heart | 10.2 |
| Breast ca. MCF-7 | 0.0 | Heart Pool | 8.1 |
| Breast ca. MDA-MB-231 | 92.0 | Lymph Node Pool | 47.3 |
| Breast ca. BT 549 | 0.0 | Fetal Skeletal Muscle | 35.4 |
| Breast ca. T47D | 0.0 | Skeletal Muscle Pool | 0.0 |
| Breast ca. MDA-N | 0.0 | Spleen Pool | 0.0 |
| Breast Pool | 19.5 | Thymus Pool | 48.3 |
| Trachea | 0.0 | CNS cancer (glio/astro) U87-MG | 10.1 |
| Lung | 0.0 | CNS cancer (glio/astro) U-118-MG | 0.0 |
| Fetal Lung | 7.7 | CNS cancer (neuro;met) SK-N-AS | 2.8 |
| Lung ca. NCI-N417 | 0.0 | CNS cancer (astro) SF-539 | 0.0 |
| Lung ca. LX-1 | 8.3 | CNS cancer (astro) SNB-75 | 0.0 |
| Lung ca. NCI-H146 | 0.0 | CNS cancer (glio) SNB-19 | 0.0 |
| Lung ca. SHP-77 | 0.0 | CNS cancer (glio) SF-295 | 12.1 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 0.0 | Brain (fetal) | 9.5 |
| Lung ca. NCI-H460 | 0.0 | Brain (Hippocampus) Pool | 0.0 |
| Lung ca. HOP-62 | 0.0 | Cerebral Cortex Pool | 0.0 |
| Lung ca. NCI-H522 | 0.0 | Pool (Substantia nigra) | 7.3 |
| Liver | 0.0 | Brain (Thalamus) Pool | 8.6 |
| Fetal Liver | 0.0 | Brain (whole) | 0.0 |
| Liver ca. HepG2 | 0.0 | Spinal Cord Pool | 0.0 |
| Kidney Pool | 13.7 | Adrenal Gland | 0.0 |
| Fetal Kidney | 8.1 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 37.9 |
| Renal ca. UO-31 | 0.0 | Pancreas Pool | 0.0 |

**Table RF. Panel 4.1D**

| **Tissue Name** | **Rel. Exp.(%) Ag4348, Run 186362432** | **Tissue Name** | **Rel. Exp.(%) Ag4348, Run 186362432** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 1.6 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1 beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 1.5 | Small airway epithelium TNFalpha + IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 0.0 |
| CD4 lymphocyte none | 1.7 | KU-812 (Basophil) PMA/ionomycin | 0.0 |
| 2ry Th1/Th2/Tr1_anti- CD95 CH11 | 2.3 | CCD1106 (Keratinocytes) none | 0.0 |
| LAK cells rest | 0.0 | CCD1106 (Keratinocytes) TNFalpha + IL- 1beta | 0.0 |
| LAK cells IL-2 | 0.0 | Liver cirrhosis | 0.0 |
| LAK cells IL-2+IL-12 | 0.0 | NCI-H292 none | 0.0 |
| LAK cells IL-2+IFN gamma | 0.0 | NCI-H292 IL-4 | 0.0 |
| LAK cells IL-2+ IL-18 | 0.0 | NCI-H292 IL-9 | 0.0 |
| LAK cells PMA/ionomycin | 0.0 | NCI-H292 IL-13 | 0.0 |
| NK Cells IL-2 rest | 0.0 | NCI-H292 IFN gamma | 0.0 |
| Two Way MLR 3 day | 0.0 | HPAEC none | 1.7 |
| Two Way MLR 5 day | 0.0 | HPAEC TNF alpha + IL-1 beta | 0.0 |
| Two Way MLR 7 day | 0.0 | Lung fibroblast none | 0.0 |
| PBMC rest | 0.0 | Lung fibroblast TNF alpha + IL-1 beta | 0.0 |
| PBMC PWM | 0.0 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 0.0 |
| Ramos (B cell) ionomycin | 0.0 | Lung fibroblast IFN gamma | 0.0 |
| B lymphocytes PWM | 0.0 | Dermal fibroblast CCD1070 rest | 0.0 |
| B lymphocytes CD40L and IL-4 | 0.0 | Dermal fibroblast CCD 1070 TNF alpha | 0.0 |
| EOL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 2.0 |
| Dendritic cells anti-CD40 | 0.0 | Neutrophils TNFa+LPS | 3.8 |
| Monocytes rest | 0.0 | Neutrophils rest | 0.0 |
| Monocytes LPS | 0.0 | Colon | 3.5 |
| Macrophages rest | 0.0 | Lung | 6.8 |
| Macrophages LPS | 0.0 | Thymus | 7.2 |
| HUVEC none | 0.0 | Kidney | **100.0** |
| HUVEC starved | 1.8 | | |

**CNS_neurodegeneration_v1.0 Summary:** Ag4348 This panel confirms the expression of the CG105491-01 gene at low levels in the brain in an independent group of individuals. This gene is found to be slightly down-regulated in the temporal cortex of Alzheimer's disease patients. Therefore, up-regulation of this gene or its protein product, or treatment with specific agonists for this protein may be of use in reversing the dementia/memory loss associated with this disease and neuronal death.

The CG105491-01 gene codes for a serine protease. Plasmin, a member of serine protease family, is shown to increase the processing of human APP preferentially at the alpha-cleavage site, and efficiently degrades secreted amyloidogenic and non-amyloidogenic APP fragments. Brain tissue from Alzheimer's disease patients was shown to contain reduced levels of plasmin, implying that plasmin downregulation may cause amyloid plaque deposition accompanying sporadic Alzheimer's disease (Ledesma et al., 2000, EMBO Rep 1(6):530-5, PMID: 11263499). Thus, based on functional homology and also, on expression pattern, the serine protease encoded by this gene may also play a role in degradation of amyloidogenic and non-amyloidogenic APP fragments. Therefore, therapeutic modulation of this gene product may be beneficial in the treatment of Alzheimer's disease.

**General_screening_panel_v1.6 Summary:** Ag6953 Highest expression of this gene is detected in testis and a breast cancer MDA-MB-231 cell line (CTs=33.1). Therefore, expression of this gene may be used to distinguish these samples from other samples in this panel. In addition, low expression of this gene is detected in pancreatic, a gastric, and squamous cell cancer cell lines. Therefore, expression of this gene may be used as diagnostic marker for detection of squamous cell carcinoma, breast, pancreatic, and gastric cancers. In additon, therapeutic modulation of this gene product may be useful in the treatment of these cancers.

In addition to testis, low levels of expression of this gene is also seen in normal tissues represented by thymus, lymphnode, bladder, colon and small intestine. Therefore, therapeutic modulation of this gene may be useful in the treatment of disease associated with these tissues.

Low levels of expression of this gene is also detected in fetal skeletal muscle. Interestingly, this gene is expressed at much higher levels in fetal (CT=34.5) when compared to adult skeletal muscle (CT=40). This observation suggests that expression of this gene can be used to distinguish fetal from adult skeletal muscle. In addition, the relative overexpression of this gene in fetal skeletal muscle suggests that the protein product may enhance muscular growth or development in the fetus and thus may also act in a regenerative capacity in the adult. Therefore, therapeutic modulation of the protein encoded by this gene could be useful in treatment of muscle related diseases. More specifically, treatment of weak or dystrophic muscle with the protein encoded by this gene could restore muscle mass or function.

**Panel 4.1D Summary:** Ag4348 Moderate level of expression of the CG105491-01 gene is detected only in kidney sample (CT=31.2). Therefore, expression of this gene may be used to distinguish kidney from other samples used in this panel. In addition, therapeutic modulation of this gene product may be beneficial in the treatment of autoimmune and inflammatory diseases that affect kidney, including lupus and glomerulonephritis.

### S. CG105954-01: human ortholog of chicken NEUROFASCIN PRECURSOR

Expression of gene CG105954-01 was assessed using the primer-probe set Ag4311, described in Table SA.

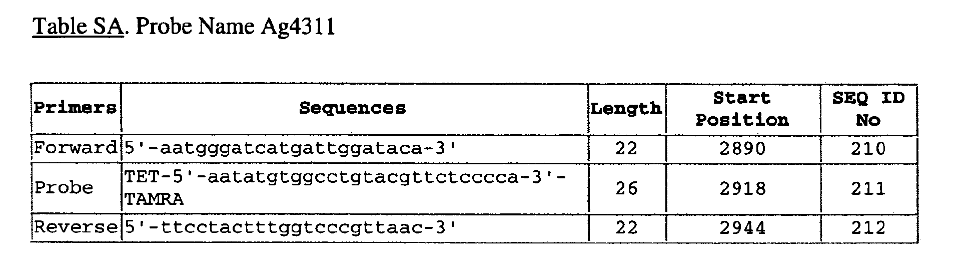

### T. CG105963-01: Novel cadherin

Expression of gene CG105963-01 was assessed using the primer-probe set Ag4312, described in Table TA. Results of the RTQ-PCR runs are shown in Tables TB and TC.

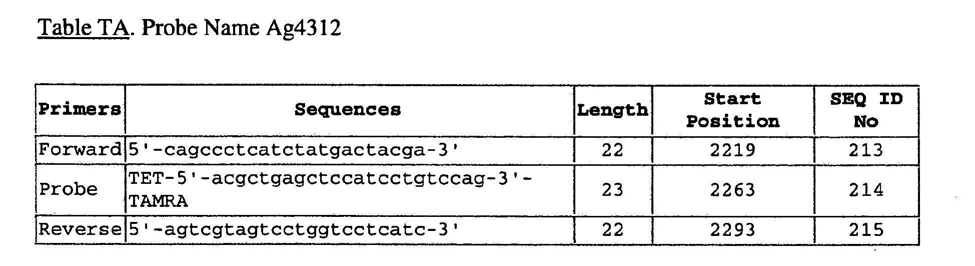

**Table TB. General_screening_panel_v1.4**

| **Tissue Name** | **Rel. Exp.(%) Ag4312, Run 222355477** | **Tissue Name** | **Rel. Exp.(%) Ag4312, Run 222355477** |
|---|---|---|---|
| Adipose | 1.4 | Renal ca. TK-10 | 7.3 |
| Melanoma* Hs688(A).T | 1.2 | Bladder | 0.3 |
| Melanoma* Hs688(B).T | 1.2 | Gastric ca. (liver met.) NCI-N87 | 2.1 |
| Melanoma* M14 | 0.0 | Gastric ca. KATO III | 0.0 |
| Melanoma* LOXIMVI | 0.7 | Colon ca. SW-948 | 0.0 |
| Melanoma* SK-MEL-5 | 0.0 | Colon ca. SW480 | 1.4 |
| Squamous cell carcinoma SCC-4 | 0.2 | Colon ca.* (SW480 met) SW620 | 0.5 |
| Testis Pool | 0.3 | Colon ca. HT29 | 0.0 |
| Prostate ca.* (bone met) PC-3 | 13.8 | Colon ca. HCT-116 | 8.1 |
| Prostate Pool | 0.6 | Colon ca. CaCo-2 | 0.0 |
| Placenta | 0.3 | Colon cancer tissue | 0.5 |
| Uterus Pool | 0.0 | Colon ca. SW1116 | 1.3 |
| Ovarian ca. OVCAR-3 | 0.5 | Colon ca. Colo-205 | 0.0 |
| Ovarian ca. SK-OV-3 | 0.4 | Colon ca. SW-48 | 0.0 |
| Ovarian ca. OVCAR-4 | 1.3 | Colon Pool | 0.4 |
| Ovarian ca. OVCAR-5 | 9.0 | Small Intestine Pool | 0.0 |
| Ovarian ca. IGROV-1 | 3.4 | Stomach Pool | 0.0 |
| Ovarian ca. OVCAR-8 | 7.7 | Bone Marrow Pool | 0.0 |
| Ovary | 0.0 | Fetal Heart | 0.0 |
| Breast ca. MCF-7 | 1.0 | Heart Pool | 0.1 |
| Breast ca. MDA-MB-231 | 1.1 | Lymph Node Pool | 0.3 |
| Breast ca. BT 549 | 0.6 | Fetal Skeletal Muscle | 38.7 |
| Breast ca. T47D | 17.4 | Skeletal Muscle Pool | 34.4 |
| Breast ca. MDA-N | 0.0 | Spleen Pool | 0.3 |
| Breast Pool | 0.0 | Thymus Pool | 0.3 |
| Trachea | 0.2 | CNS cancer (glio/astro) U87-MG | 9.6 |
| Lung | 0.1 | CNS cancer (glio/astro) U-118-MG | 0.1 |
| Fetal Lung | 1.3 | CNS cancer (neuro;met) SK-N-AS | 0.0 |
| Lung ca. NCI-N417 | 21.2 | CNS cancer (astro) SF-539 | 4.3 |
| Lung ca. LX-1 | 1.7 | CNS cancer (astro) SNB-75 | 14.4 |
| Lung ca. NCI-H146 | 0.0 | CNS cancer (glio) SNB-19 | 3.8 |
| Lung ca. SHP-77 | 1.2 | CNS cancer (glio) SF-295 | 6.7 |
| Lung ca. A549 | 0.1 | Brain (Amygdala) Pool | 0.0 |
| Lung ca. NCI-H526 | 0.0 | Brain (cerebellum) | **100.0** |
| Lung ca. NCI-H23 | 0.2 | Brain (fetal) | 0.0 |
| Lung ca. NCI-H460 | 0.0 | Brain (Hippocampus) Pool | 0.0 |
| Lung ca. HOP-62 | 8.8 | Cerebral Cortex Pool | 0.0 |
| Lung ca. NCI-H522 | 0.7 | Brain Pool (Substantia nigra) | 0.1 |
| Liver | 0.5 | Brain (Thalamus) Pool | 0.1 |
| Fetal Liver | 0.5 | Brain (whole) | 9.7 |
| Liver ca. HepG2 | 18.9 | Spinal Cord Pool | 0.0 |
| Kidney Pool | 0.2 | Adrenal Gland | 0.6 |
| Fetal Kidney | 1.8 | Pituitary gland Pool | 0.0 |
| Renal ca. 786-0 | 0.2 | Salivary Gland | 0.1 |
| Renal ca. A498 | 0.6 | Thyroid (female) | 0.0 |
| Renal ca. ACHN | 0.0 | Pancreatic ca. CAPAN2 | 0.0 |
| Renal ca. UO-31 | 0.0 | Pancreas Pool | 0.2 |

**Table TC. Panel 4.1D**

| **Tissue Name** | **Rel. Exp.(%) Ag4312, Run 182243751** | **Tissue Name** | **Rel. Exp.(%) Ag4312, Run 182243751** |
|---|---|---|---|
| Secondary Th1 act | 0.0 | HUVEC IL-1 beta | 0.3 |
| Secondary Th2 act | 0.0 | HUVEC IFN gamma | 0.3 |
| Secondary Tr1 act | 0.0 | HUVEC TNF alpha + IFN gamma | 1.5 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 4.6 |
| Primary Th1 act | 0.0 | Lung Microvascular EC TNFalpha + IL-1 beta | 0.4 |
| Primary Th2 act | 0.1 | Microvascular Dermal EC none | 0.2 |
| Primary Tr1 act | 0.0 | Microsvasular Dermal EC TNFalpha + IL-1 beta | 0.5 |
| Primary Th1 rest | 0.0 | Bronchial epithelium TNFalpha + IL 1 beta | 0.0 |
| Primary Th2 rest | 0.0 | Small airway epithelium none | 0.0 |
| Primary Tr1 rest | 0.0 | Small airway epithelium TNFalpha + IL-1beta | 0.0 |
| CD45RA CD4 act lymphocyte act | 0.1 | Coronery artery SMC rest | 1.2 |
| CD45RO CD4 lymphocyte act | 0.0 | Coronery artery SMC TNFalpha+IL-1beta | 2.1 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 | 0.1 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 0.0 |
| LAK cells IL-2+IL-12 | 0.0 | NCI-H292 none | 0.0 |
| LAK cells IL-2+IFN gamma | 0.0 | NCI-H292 IL-4 | 0.3 |
| LAK cells IL-2+ IL-18 | 0.0 | NCI-H292 IL-9 | 0.0 |
| LAK cells PMA/ionomycin | 0.0 | NCI-H292IL-13 | 0.0 |
| NK Cells IL-2rest | 0.0 | NCI-H292 IFN gamma | 0.0 |
| Two Way MLR 3 day | 0.0 | HPAEC none | 0.0 |
| Two Way MLR 5 day | 0.0 | HPAEC TNF alpha + IL-1 beta | 2.4 |
| Two Way MLR 7 day | 0.0 | Lung fibroblast none | 0.5 |
| PBMC rest | 0.0 | Lung fibroblast TNF alpha + IL-1 beta | 4.0 |
| PBMC PWM | 0.0 | Lung fibroblast IL-4 | 0.0 |
| PBMC PHA-L | 0.0 | Lung fibroblast IL-9 | 0.7 |
| Ramos (B cell) none | 0.0 | Lung fibroblast IL-13 | 1.3 |
| Ramos (B cell) ionomycin | 0.0 | Lung fibroblast IFN gamma | 0.0 |
| B lymphocytes PWM | 0.0 | Dermal fibroblast CCD1070 rest | 1.1 |
| B lymphocytes CD40L and IL-4 | 0.0 | Dermal fibroblast CCD 1070 TNF alpha | 0.0 |
| EOL-1 dbcAMP | 0.1 | Dermal fibroblast CCD1070 IL-1 beta | 0.1 |
| EOL-1 dbcAMP PMA/ionomycin | 0.6 | Dermal fibroblast IFN gamma | 0.4 |
| Dendritic cells none | 0.0 | Dermal fibroblast IL-4 | 0.0 |
| Dendritic cells LPS | 0.0 | Dermal Fibroblasts rest | 0.1 |
| Dendritic cells anti-CD40 | 0.0 | Neutrophils TNFa+LPS | 0.2 |
| Monocytes rest | 0.0 | Neutrophils rest | 0.7 |
| Monocytes LPS | 0.0 | Colon | 0.1 |
| Macrophages rest | 0.0 | Lung | 2.4 |
| Macrophages LPS | 0.0 | Thymus | 13.9 |
| HUVEC none | 0.1 | Kidney | **100.0** |
| HUVEC starved | 0.2 | | |

**General_screening_panel_v1.4 Summary:** Ag4312 Highest expression of the CG105963-01 gene is detected in brain (cerebellum) (Ct=28.8). In addition, moderate expression of this gene is also seen in whole brain sample. The CG105963-01 gene codes for a variant of cadherin-15 (M-cadherin). Cadherins are calcium-dependent, transmembrane intercellular adhesion proteins with morphoregulatory functions in the development and maintenance of tissues. Cadherins can act as axon guidance and cell adhesion proteins, specifically during development and in the response to injury (Ranscht B., 2000, Int. J. Dev. Neurosci. 18: 643-651, PMID: 10978842). In addition, M-cadherin is involved in muscle cell, Schwann cell, and motoneuron interactions and also in differentiation during neuromuscular development (Padilla et al., 1998, Mol Cell Neurosci 11(4):217-33, PMID: 9675053). Therefore, therapeutic modulation of this protein may be useful in inducing a compensatory synaptogenic response to neuronal death in Alzheimer's disease, Parkinson's disease, Huntington's disease, spinocerebellar ataxia, progressive supranuclear palsy, ALS, head trauma, stroke, or any other disease/condition associated with neuronal loss.

In addition, low to moderate levels of expression of this gene is also seen in number of cancer cell lines including CNS cancer, colon, renal, liver, lung, breast, ovarian and prostate cancer cell lines. Therefore, therapeutic modulation of this gene product may be useful in the treatment of these cancers.

Moderate expression of this gene is also seen in skeletal muscle. M-cadherin is shown to be important for skeletal muscle development, in particular the fusion of myoblasts into myotubes (Kaufmann et al., 1999, J Cell Sci 112:55-68, PMID: 9841904). Therefore, therapeutic modulation of this gene may be beneficial in the treatment of muscle related disease

**Panel 4.1D Summary:** Ag4312 Highest expression of the CG105963-01 gene is detected in kidney (CT=28.3). Therefore, expression of this gene may be used to distinguish kidney sample from other samples used in this panel. In addition, moderate to low expression of this gene is also seen in thymus, lung, TNF alpha + IL-1 beta treated lung fibroblasts and endothelial cells represent by HPAEC and HUVEC, coronery artery, and lung microvascular EC. Therefore, therapeutic modulation of this gene product may be beneficial in the treatment of autoimmune and inflammatory diseases affecting kidney and lung including lupus erythematosus, asthma, emphysema, Crohn's disease, ulcerative colitis, rheumatoid arthritis, osteoarthritis, and psoriasis.

### U. CG105973-01 and CG105973-02: INTEGRIN ALPHA-8

Expression of gene CG105973-01 and CG105973-02 was assessed using the primer-probe sets Ag4305 and Ag4313, described in Tables UA and UB. Results of the RTQ-PCR runs are shown in Tables UC, UD, UE, UF and UG. Please note that CG105973-02 represents a full-length physical clone of the CG105973-01 gene, validating the prediction of the gene sequence.

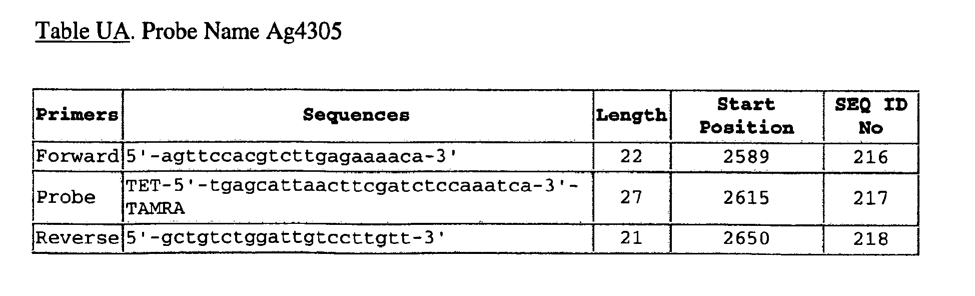

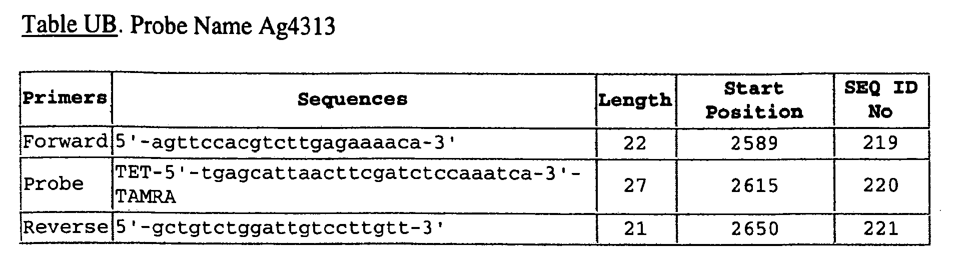

**Table UC. AI_comprehensive panel_v1.0**

| **Tissue Name** | **Rel. Exp.(%) Ag4305, Run 244570379** | **Tissue Name** | **Rel. Exp.(%) Ag4305, Run 244570379** |
|---|---|---|---|
| 110967 COPD-F | 17.0 | 112427 Match Control Psoriasis-F | 37.1 |
| 110980 COPD-F | 59.5 | 112418 Psoriasis-M | 14.5 |
| 110968 COPD-M | 15.7 | 112723 Match Control Psoriasis-M | 0.0 |
| 110977 COPD-M | 76.8 | 112419 Psoriasis-M | 33.0 |
| 110989 Emphysema-F | 22.8 | 112424 Match Control Psoriasis-M | 15.6 |
| 110992 Emphysema-F | 4.6 | 112420 Psoriasis-M | 27.2 |
| 110993 Emphysema-F | 21.6 | 112425 Match Control Psoriasis-M | 28.1 |
| 110994 Emphysema-F | 14.3 | 104689 (MF) OA Bone-Backus | 17.2 |
| 110995Emphysema-F | 10.2 | 104690 (MF) Adj "Normal" Bone-Backus | 18.7 |
| 110996 Emphysema-F | 0.0 | 104691 (MF) OA Synovium-Backus | 10.4 |
| 110997 Asthma-M | 13.9 | 104692 (BA) OA Cartilage-Backus | 0.0 |
| 111001 Asthma-F | 17.1 | 104694 (BA) OA Bone-Backus | 6.3 |
| 111002 Asthma-F | 14.8 | 104695 (BA) Adj "Normal" Bone-Backus | 16.8 |
| 111003 Atopic Asthma-F | 23.0 | 104696 (BA) OA Synovium-Backus | 6.0 |
| 111004 Atopic Asthma-F | 17.1 | 104700 (SS) OA Bone-Backus | 11.8 |
| 111005 Atopic Asthma-F | 12.8 | 104701 (SS) Adj "Normal" Bone-Backus | 10.2 |
| 111006 Atopic Asthma-F | 5.1 | 104702 (SS) OA Synovium-Backus | 23.3 |
| 111417 Allergy-M | 13.6 | 117093 OA Cartilage Rep7 | 18.8 |
| 112347 Allergy-M | 7.1 | 1112672 OA Bone5 | 36.9 |
| 112349 Normal Lung-F | 5.7 | 112673 OA Synovium5 | 17.6 |
| 112357 Normal Lung-F | 12.9 | 112674 OA Synovial Fluid cells5 | 17.0 |
| 112354 Normal Lung-M | 59.9 | 117100 OA Cartilage Rep14 | 5.0 |
| 112374 Crohns-F | 8.0 | 1112756 OA Bone9 | 0.2 |
| 112389 Match Control Crohns-F | 24.3 | 112757 OA Synovium9 | 3.3 |
| 112375 Crohns-F | 14.3 | 112758 OA Synovial Fluid Cells9 | 11.0 |
| 112732 Match Control Crohns-F | 6.2 | 117125 RA Cartilage Rep2 | 19.1 |
| 112725 Crohns-M | 11.3 | 113492 Bone2 RA | 40.9 |
| 112387 Match Control Crohns-M | 5.9 | 113493 Synovium2 RA | 21.6 |
| 112378 Crohns-M | 7.5 | 113494 Syn Fluid Cells RA | 34.4 |
| 112390 Match Control Crohns-M | 32.3 | 113499 Cartilage4 RA | 21.3 |
| 112726 Crohns-M | **100.0** | 113500 Bone4 RA | 26.8 |
| 112731 Match Control Crohns-M | 47.0 | 113501 Synovium4 RA | 24.3 |
| 112380 Ulcer Col-F | 17.1 | 113502 RA Syn Fluid Cells4 | 13.5 |
| 112734 Match Control Ulcer Col-F | 14.3 | 113495 Cartilage3 RA | 28.3 |
| 112384 Ulcer Col-F | 17.6 | 113496 Bone3 RA | 33.0 |
| 112737 Match Control Ulcer Col-F | 35.1 | 113497 Synovium3 RA | 15.7 |
| 112386 Ulcer Col-F RA | 3.0 | 113498 Syn Fluid Cells3 | 39.2 |
| 112738 Match Control Ulcer Col-F | 6.7 | 117106 Normal Cartilage Rep20 | 4.3 |
| 112381 Ulcer Col-M | 33.2 | 113663 Bone3 Normal | 12.7 |
| 112735 Match Control Ulcer Col-M | 40.9 | 113664 Synovium3 Normal | 1.5 |
| 112382 Ulcer Col-M | 37.9 | 113665 Syn Fluid Cells3 Normal | 7.5 |
| 112394 Match Control Ulcer Col-M | 0.2 | 117107 Normal Cartilage Rep22 | 10.0 |
| 112383 Ulcer Col-M | 3.5 | 113667 Bone4 Normal | 13.5 |
| 112736 Match Control Ulcer Col-M | 19.2 | 113668 Synovium4 Normal | 16.3 |
| 112423 Psoriasis-F | 39.2 | 113669 Syn Fluid Cells4 Normal | 20.2 |

**Table UD. CNS_neurodegeneration_v1.0**

| **Tissue Name** | **Rel. Exp.(%) Ag4305, Run 224074285** | **Rel. Exp.(%) Ag4313, Run 224064613** | **Tissue Name** | **Rel. Exp.(%) Ag4305, Run 224074285** | **Rel. Exp.(%) Ag4313, Run 224064613** |
|---|---|---|---|---|---|
| AD 1 Hippo | 24.0 | 29.1 | Control (Path) 3 Temporal Ctx | 17.0 | 28.5 |
| AD 2 Hippo | 36.9 | 39.0 | Control (Path) 4 Temporal Ctx | 37.1 | 39.0 |
| AD 3 Hippo | 25.9 | 34.2 | AD 1 Occipital Ctx | 20.4 | 18.4 |
| AD 4 Hippo | 9.2 | 9.4 | AD 2 Occipital Ctx (Missing) | 0.0 | 0.0 |
| AD 5 Hippo | 55.9 | 76.8 | AD 3 Occipital Ctx | 15.2 | 9.9 |
| AD 6 Hippo | **100.0** | **100.0** | AD 4 Occipital Ctx | 20.9 | 25.9 |
| Control 2 Hippo | 37.6 | 32.3 | AD 5 Occipital Ctx | 50.7 | 22.7 |
| Control 4 Hippo | 27.9 | 25.7 | AD 6 Occipital Ctx | 32.1 | 59.5 |
| Control (Path) 3 Hippo | 44.4 | 54.7 | Control 1 Occipital Ctx | 9.6 | 13.2 |
| AD 1 Temporal Ctx | 30.8 | 25.2 | Control 2 Occipital Ctx | 32.3 | 40.9 |
| AD 2 Ctx Temporal Ctx | 43.5 | 52.1 | Control 3 Occipital Ctx | 25.0 | 26.2 |
| AD 3 Temporal Ctx | 15.3 | 20.4 | Control 4 Occipital Ctx | 14.9 | 27.5 |
| AD 4 Temporal Ctx | 28.3 | 37.4 | Control (Path) 1 Occipital Ctx | 47.3 | 64.2 |
| AD 5 Inf Temporal Ctx | 66.0 | 70.7 | Control (Path) 2 Occipital Ctx | 12.7 | 18.0 |
| AD 5 Sup Temporal Ctx | 73.2 | 69.7 | Control (Path) 3 Occipital Ctx | 9.0 | 12.8 |
| AD 6 Inf Temporal Ctx | 40.3 | 43.5 | Control (Path) 4 Occipital Ctx | 25.2 | 37.1 |
| AD 6 Sup Temporal Ctx | 62.9 | 62.4 | Control 1 Parietal Ctx | 14.3 | 13.1 |
| Control 1 Temporal Ctx | 13.4 | 20.0 | Control 2 Parietal Ctx | 60.7 | 51.8 |
| Control 2 Temporal Ctx | 28.5 | 27.4 | Control 3 Parietal Ctx | 40.9 | 33.7 |
| Control 3 Temporal Ctx | 25.3 | 22.4 | Control (Path) Parietal Ctx | 42.0 | 62.9 |
| Control 3 Temporal Ctx | 24.7 | 20.2 | Control (Path) 2 Parietal Ctx | 42.6 | 38.2 |
| Control (Path) 1 Temporal Ctx | 54.7 | 66.0 | Control (Path) 3 Parietal Ctx | 21.6 | 19.6 |
| Control (Path) 2 Temporal Ctx | 36.6 | 33.9 | Control (Path) 4 Parietal Ctx | 59.5 | 77.9 |

**Table UE. General_screening_panel_v1.4**

| **Tissue Name** | **Rel. Exp.(%) Ag4305, Run 222261511** | **Rel. Exp.(%) Ag4313, Run 222360603** | **Tissue Name** | **Rel. Exp.(%) Ag4305, Run 222261511** | **Rel. Exp.(%) Ag4313, Run 222360603** |
|---|---|---|---|---|---|
| Adipose | 8.7 | 9.5 | Renal ca. TK-10 | 0.0 | 0.0 |
| Melanoma* Hs688(A).T | 19.8 | 20.0 | Bladder | 2.7 | 3.0 |
| Melanoma* Hs688(B).T | 18.0 | 15.0 | Gastric ca. (liver met.) NCI-N87 | 0.0 | 0.0 |
| Melanoma* M14 | 0.0 | 0.0 | Gastric ca. KATO III | 0.0 | 0.0 |
| Melanoma* LOXIMVI | 0.0 | 0.0 | Colon ca. SW-948 | 0.0 | 0.0 |
| Melanoma* SK-MEL-5 | 0.0 | 0.0 | Colon ca. SW480 | 0.0 | 0.0 |
| Squamous cell carcinoma SCC-4 | 0.0 | 0.0 | Colon ca.* (SW480 Colon ca.* (SW480 met) SW620 | 0.0 | 0.0 |
| Testis Pool | 10.8 | 8.9 | Colon ca. HT29 | 0.0 | 0.0 |
| Prostate ca.* (bone met) PC-3 | 0.0 | 0.0 | Colon ca. HCT-116 | 0.0 | 0.0 |
| Prostate Pool | 27.9 | 24.0 | Colon ca. CaCo-2 | 0.0 | 0.0 |
| Placenta | 0.0 | 0.0 | Colon cancer tissue | 2.2 | 1.7 |
| Uterus Pool | 13.2 | 10.4 | Colon ca. SW1116 | 0.0 | 0.0 |
| Ovarian ca. OVCAR-3 | 0.0 | 0.0 | Colon ca. Colo-205 | 0.0 | 0.0 |
| Ovarian ca. SK-OV-3 | 0.0 | 0.0 | Colon ca. SW-48 | 0.0 | 0.0 |
| Ovarian ca. OVCAR-4 | 0.0 | 0.0 | Colon Pool | 18.3 | 19.8 |
| Ovarian ca. OVCAR-5 | 0.0 | 0.0 | Small Intestine Pool | 13.9 | 11.7 |
| Ovarian ca. IGROV-1 | 0.0 | 0.0 | Stomach Pool | 10.0 | 10.6 |
| Ovarian ca. OVCAR-8 | 0.0 | 0.0 | Bone Marrow Pool | 20.9 | 20.2 |
| Ovary | 1.9 | 1.3 | Fetal Heart | 3.7 | 2.7 |
| Breast ca. MCF-7 | 0.0 | 0.0 | Heart Pool | 17.0 | 15.4 |
| Breast ca. MDA-MB-231 | 0.0 | 0.0 | Lymph Node Pool | 29.5 | 24.1 |
| Breast ca. BT 549 | 0.0 | 0.0 | Fetal Skeletal Muscle | 5.6 | 4.2 |
| Breast ca. T47D | 0.0 | 0.0 | Skeletal Muscle Pool | 5.7 | 4.7 |
| Breast ca. MDA-N | 0.0 | 0.0 | Spleen Pool | 37.4 | 31.2 |
| Breast Pool | 20.9 | 17.6 | Thymus Pool | 8.2 | 7.6 |
| Trachea | 14.1 | 12.7 | CNS cancer (glio/astro) U87-MG | 0.1 | 0.0 |
| Lung | 21.5 | 18.6 | CNS cancer (glio/astro) U-118-MG | 28.9 | 25.5 |
| Fetal Lung | **100.0** | **100.0** | CNS cancer (neuro;met) SK-N-AS | 83.5 | 69.7 |
| Lung ca. NCI-N417 | 0.0 | 0.0 | CNS cancer (astro) SF-539 | 0.3 | 0 0 |
| Lung ca. LX-1 | 0.0 | 0.0 | CNS cancer (astro) SNB-75 | 0.3 | 0.0 |
| Lung ca. NCI-H146 | 0.0 | 0.0 | CNS cancer (glio) SNB-19 | 0.0 | 0.0 |
| Lung ca. SHP-77 | 0.0 | 0.0 | CNS cancer (glio) SF-295 | 40.6 | 35.1 |
| Lung ca. A549 | 0.0 | 0.0 | Brain (Amygdala) Pool | 2.2 | 2.3 |
| Lung ca. NCI- H526 | 0.0 | 0.0 | Brain (cerebellum) | 5.2 | 3.7 |
| Lung ca. NCI- H23 | 0.5 | 0.1 | Brain (fetal) | 3.2 | 3.9 |
| Lung ca. NCI-H460 | 0.0 | 0.0 | Brain (Hippocampus) Pool | 6.4 | 5.0 |
| Lung ca. HOP-62 | 0.0 | 0.0 | Cerebral Cortex Pool | 3.6 | 3.2 |
| Lung ca. NCI-H522 | 0.0 | 0.0 | Brain (Substantia nigra) Pool | 2.2 | 2.4 |
| Liver | 0.1 | 0.0 | Brain (Thalamus) Pool | 5.7 | 5.9 |
| Fetal Liver | 3.7 | 3.0 | Brain (whole) | 2.5 | 2.6 |
| Liver ca. | 0.0 | 0.0 | Spinal Cord Pool | 3.6 | 2.3 |
| Kidney Pool | 52.9 | 42.0 | Adrenal Gland | 17.0 | 21.2 |
| Fetal Kidney | 45.1 | 40.3 | Pool | 3.7 | 2.7 |
| Renal ca. 786-0 | 0.0 | 0.0 | Salivary Gland | 1.3 | 0.2 |
| Renal ca. A498 | 0.0 | 0.0 | Thyroid (female) | 2.9 | 2.7 |
| Renal ca. ACHN | 0.0 | 0.0 | Pancreatic ca. CAPAN2 | 0.0 | 0.0 |
| Renal ca. UO- | 0.0 | 0.0 | Pancreas Pool | 8.4 | 8.9 |

**Table UF. Panel 4.1D**

| **Tissue Name** | **Rel. Exp.(%) Ag4305, Run 182086760** | **Rel. Exp.(%) Ag4313, Run 182244195** | **Tissue Name** | **Rel. Exp.(%) Ag4305, Run 182086760** | **Rel. Exp.(%) Ag4313, Run 182244195** |
|---|---|---|---|---|---|
| Secondary Th1 act | 0.0 | 0.0 | HUVEC IL-1beta | 0.6 | 0.0 |
| Secondary Th2 act | 0.0 | 0.0 | HUVEC IFN gamma | 0.0 | 0.0 |
| Secondary Tr1 act | 0.0 | 0.0 | HUVEC TNF alpha + IFN gamma | 0.0 | 0.0 |
| Secondary Th1 rest | 0.0 | 0.0 | HUVEC TNF alpha + IL4 | 0.0 | 0.0 |
| Secondary Th2 rest | 0.0 | 0.0 | HUVEC IL-11 | 0.0 | 0.0 |
| Secondary Tr1 rest | 0.0 | 0.0 | Lung Microvascular EC none | 0.3 | 0.0 |
| Primary Th1 act | 0.0 | 0.0 | Lung Microvascular EC TNFalpha + IL-1beta | 3.9 | 0.0 |
| Primary Th2 act | 0.0 | 0.0 | Microvascular Dermal EC none | 0.0 | 0.0 |
| Primary Tr1 act | 0.0 | 0.0 | Microsvasular | 0.0 | 1.0 |
| | | | Dermal EC TNFalpha + IL-1beta | | |
| Primary Th1 rest | 0.0 | 0.0 | Bronchial epithelium TNFalpha + IL1beta | 0.0 | 0.0 |
| Primary Th2 rest | 0.0 | 0.0 | Small airway epithelium none | 0.0 | 0.0 |
| Primary Tr1 rest | 0.0 | 0.0 | Small airway epithelium TNFalpha + IL-1 beta | 0.0 | 0.0 |
| CD45RA CD4 lymphocyte act | 3.8 | 8.7 | Coronery artery SMC rest | 0.0 | 0.0 |
| CD45RO CD4 lymphocyte act | 0.0 | 0.0 | Coronery artery SMC TNFalpha + IL-1 beta | 0.0 | 0.0 |
| CD8 lymphocyte act | 0.0 | 0.0 | Astrocytes rest | 0.0 | 0.0 |
| Secondary CD8 lymphocyte rest | 0.0 | 0.0 | Astrocytes TNFalpha + IL-1 beta | 0.0 | 0.0 |
| Secondary CD8 lymphocyte act | 0.0 | 0.0 | KU-812 (Basophil) rest | 0.0 | 0.0 |
| CD4 lymphocyte none | 0.0 | 0.0 | KU-812 (Basophil) PMA/ionomycin | 0.0 | 0.0 |
| 2ry Th1/Th2/Tr1_anti-CD95 CH11 | 0.0 | 0.0 | CCD1106 (Keratinocytes) none | 0.0 | 0.0 |
| LAK cells rest | 0.0 | 0.0 | CCD1106 (Keratinocytes) TNFalpha + IL-1beta | 0.0 | 0.0 |
| LAK cells IL-2 | 0.0 | 0.0 | Liver cirrhosis | 13.9 | 31.0 |
| LAK cells IL-2+IL-12 | 0.0 | 0.0 | NCI-H292 none | 0.0 | 0.0 |
| LAK cells IL-2+IFN gamma | 0.0 | 0.0 | NCI-H292 IL-4 | 0.0 | 0.0 |
| LAK cells IL-2+ IL-18 | 0.0 | 0.0 | NCI-H292 IL-9 | 0.0 | 0.0 |
| LAK cells PMA/ionomycin | 0.0 | 0.0 | NCI-H292 IL-13 | 0.4 | 0.0 |
| NK Cells IL-2 rest | 0.0 | 0.0 | NCI-H292 IFN gamma | 0.0 | 0.0 |
| Two Way MLR 3 day | 0.0 | 0.0 | HPAEC none | 0.0 | 0.0 |
| Two Way MLR 5 day | 0.0 | 0.0 | HPAEC TNF alpha + IL-1 beta | 0.0 | 1.2 |
| Two Way MLR 7 day | 0.0 | 0.0 | Lung fibroblast none | 3.5 | 0.0 |
| PBMC rest | 0.0 | 0.0 | Lung fibroblast TNF alpha + IL-1 beta | 0.6 | 1.9 |
| PBMC PWM | 0.0 | 0.0 | Lung fibroblast IL-4 | 0.0 | 1.4 |
| PBMC PHA-L | 0.0 | 0.0 | Lung fibroblast IL-9 | 1.8 | 2.1 |
| Ramos (B cell) none | 0.0 | 0.0 | Lung fibroblast IL-13 | 2.4 | 0.0 |
| Ramos (B cell) ionomycin | 0.0 | 0.0 | Lung fibroblast IFN gamma | 0.0 | 4.1 |
| B lymphocytes | 1.9 | 0.0 | Dermal fibroblast | 3.9 | 9.7 |
| PWM | | | CCD1070 rest | | |
| B lymphocytes CD40L and IL-4 | 0.0 | 0.0 | Dermal fibroblast CCD 1070 TNF alpha | 6.0 | 6.8 |
| EOL-1 dbcAMP | 0.0 | 0.0 | Dermal fibroblast CCD1070 IL-1 beta | 16.0 | 10.5 |
| EOL-1 dbcAMP PMA/ionomycin | 0.0 | 0.0 | Dermal fibroblast IFN gamma | 26.2 | 57.4 |
| Dendritic cells none | 0.0 | 0.0 | Dermal fibroblast IL-4 | 30.4 | 51.8 |
| Dendritic cells LPS | 0.0 | 0.0 | Dermal Fibroblasts rest | 45.1 | 66.0 |
| Dendritic cells anti-CD40 | 0.0 | 0.0 | Neutrophils TNFa+LPS | 0.0 | 4.3 |
| Monocytes rest | 0.0 | 0.0 | Neutrophils rest | 1.2 | 5.0 |
| Monocytes LPS | 0.0 | 0.0 | Colon | 14.5 | 23.5 |
| Macrophages rest | 0.0 | 0.0 | Lung | **100.0** | **100.0** |
| Macrophages LPS | 0.0 | 0.0 | Thymus | 16.4 | 22.8 |
| HUVEC none | 0.4 | 0.0 | Kidney | 48.0 | 71.2 |
| HUVEC starved | 0.0 | 0.0 | | | |

**Table UG. Panel 5 Islet**

| **Tissue Name** | **Rel. Exp.(%) Ag4305, Run 248029384** | **Tissue Name** | **Rel. Exp.(%) Ag4305, Run 248029384** |
|---|---|---|---|
| 97457_Patient-02go_adipose | 16.4 | 94709_Donor 2 AM - A_adipose | 30.1 |
| 97476_Patient-07sk_skeletal muscle | 52.5 | 94710_Donor 2 AM - B_adipose | 17.8 |
| 97477_Patient-07ut_uterus | 67.8 | 94711_Donor 2 AM - C_adipose | 6.3 |
| 97478_Patient-07p1_placenta | 0.5 | 94712_Donor 2 AD-A_adipose | 55.1 |
| 99167_Bayer Patient 1 | 13.0 | 94713_Donor 2 AD - B_adipose | 88.3 |
| 97482_Patient-08ut_uterus | 81.2 | 94714_Donor2AD-C_adipose | 81.8 |
| 97483_Patient-08pl_placenta | 0.0 | 94742_Donor 3 U - A_Mesenchymal Stem Cells | 11.3 |
| 97486_Patient-09sk_skeletal muscle | 15.4 | 94743_Donor 3 U - B_Mesenchymal Stem Cells | 12.5 |
| 97487_Patient-09ut_uterus | **100.0** | 94730_Donor 3 AM - A_adipose | 47.0 |
| 97488_Patient-09pl_placenta | 0.0 | 94731_Donor 3 AM - B_adipose | 12.5 |
| 97492_Patient-10ut_uterus | 63.7 | 94732_Donor 3 AM - C_adipose | 20.6 |
| 97493_Patient-10pl_placenta | 4.6 | 94733_Donor 3 AD - A_adipose | 76.8 |
| 97495_Patient-11go_adipose | 26.4 | 94734_Donor 3 AD - B_adipose | 27.2 |
| 97496_Patient-11sk_skeletal muscle | 33.2 | 94735_Donor 3 AD - C_adipose | 39.0 |
| 97497_Patient-11ut_uterus | 62.9 | 77138_Liver_HepG2untreated | 0.0 |
| 97498_Patient-11pl_placenta | 0.0 | 73556_Heart_Cardiac stromal cells (primary) | 0.0 |
| 97500_Patient- | 26.6 | 81735_Small Intestine | 50.3 |
| 9750t_Patient-12sk_skeletal muscle | 28.7 | 72409_Kidney_Proximal Convoluted Tubule | 0.0 |
| 97502_Patient-12ut_uterus | 73.7 | 82685_Small intestine_Duodenum | 41.5 |
| 97503_Patient-12p1_placenta | 0.0 | 90650_Adrenal_Adrenocortical adenoma | 12.9 |
| 94721_Donor 2 U -A_Mesenchymal Stem Cells | 1.7 | 72410_Kidney_HRCE | 0.0 |
| 94722_Donor 2 U -B_Mesenchymal Stem Cells | 0.0 | 72411_Kidney_HRE | 2.4 |
| 94723_Donor 2 U -C_Mesenchymal Stem Cells | 2.1 | 73139_Uterus_Uterine smooth smooth muscle cells | 11.5 |

**AI_comprehensive panel_v1.0 Summary:** Ag4305 Highest expression of the CG105973-01 gene is detected in Crohn's sample (CT=28.8). Low to moderate levels of expression of this gene are detected in samples derived from osteoarthritic (OA) bone and adjacent bone as well as OA cartilage, OA synovium and OA synovial fluid samples, and in cartilage, bone, synovium and synovial fluid samples from rheumatoid arthritis patients. Low level expression is also detected in samples derived from normal lung samples, COPD lung, emphysema, atopic asthma, asthma, allergy, Crohn's disease (normal matched control and diseased), ulcerative colitis(normal matched control and diseased), and psoriasis (normal matched control and diseased). Therefore, therapeutic modulation of this gene product may ameliorate symptoms/conditions associated with autoimmune and inflammatory disorders including psoriasis, allergy, asthma, inflammatory bowel disease, rheumatoid arthritis and osteoarthritis

**CNS_neurodegeneration_v1.0 Summary:** Ag4305/Ag4313 Two experiments with same primer and probe set are in excellent agreements, with highest expression of the CG105973-01 gene in a hippocampus sample from Alzheimer's patient (CTs=31). This panel confirms the expression of this gene at low levels in the brains of an independent group of individuals. However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4305/Ag4313 Two experiments with same primer and probe set are in excellent agreements, with highest expression of the CG105973-01 gene in fetal lung (Cts=27.7). Although, this gene appears to be expressed mainly in the normal tissues used in this panel, significant expression of this gene is also seen in two melanoma and three CNS cancer cell lines and colon cancer tissue. Therefore, therapeutic modulation of this gene product may be beneficial in the treatment of these cancers.

Among tissues with metabolic or endocrine function, this gene is expressed at moderate levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

In addition, this gene is expressed at moderate levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression and therapeutic modulation of this gene product may be useful in the treatment of these neurological disorders.

**Panel 4.1D Summary:** Ag4305/Ag4313 Two experiments with same primer and probe set are in excellent agreements, with highest expression of the CG105973-01 gene in lung (CTs=31-32). In addition, moderate to low levels of expression of this gene is also seen in liver cirrhosis, dermal fibroblasts and normal tissues represented by colon, thymus, and kidney. Therefore, therapeutic modulation of this gene may be useful in the treatment of autoimmune and inflammatory diseases that affect lung,colon and kidney, such as lupus erythematosus, asthma, emphysema, Crohn's disease, ulcerative colitis, and psoriasis.

The CG105973-01 gene codes for a variant of integrin alpha-8. In the kidney, the alpha8 integrin chain is expressed in glomerular mesangial cells and it plays a role in early nephrogenesis. In mice the alpha8 integrin chain maintains the integrity of the glomerular capillary tuft during mechanical stress, eg, in hypertension (Hartner et al., 2002, Am J Pathol 160 :861-7, PMID: 11891185). Therefore, therapeutic modulation of this gene may be useful in the treatment of glomerular mesangial cell related diseases such as glomerulonephritis.

**Panel 5 Islet Summary:** Ag4305 Highest expression of the CG105973-01 gene is detected in uterus (CT=31). This gene is expressed at moderate to low levels in tissues with metabolic or endocrine function including adipose, uterus, small intestine and kidney. Therefore, therapeutic modulation of the activity of this gene may prove useful in the treatment of endocrine/metabolically related diseases, such as obesity and diabetes.

In addition, this gene is expressed at low levels (CT=34) in human islets. Integrins are found at the insulin-secreting beta cell surface in situ. Insulin secretagogues upregulate the beta cell-surface expression of some classes of integrins (Bosco et al., 2000, Diabetes 49(2):233-43, PMID: 10868940). Thus, therapeutic modulation of this gene product may increase beta cell insulin secretion and may be useful in the treatment of Type 2 diabetes.

### V. CG106915-01 and CG106924-01: Novel Nogo receptor isoform-2

Expression of gene CG106924-01 was assessed using the primer-probe sets Ag4329, Ag4330 and Ag6865, described in Tables VA, VB and VC. Results of the RTQ-PCR runs are shown in Tables VD, VE and VF. Please note that probe Ag4330 is specific for the variant CG106924-01.

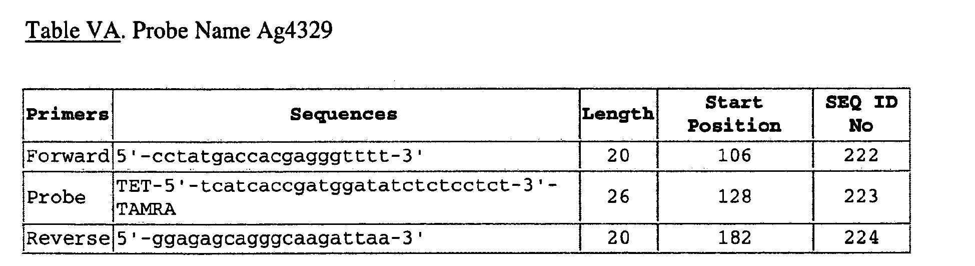

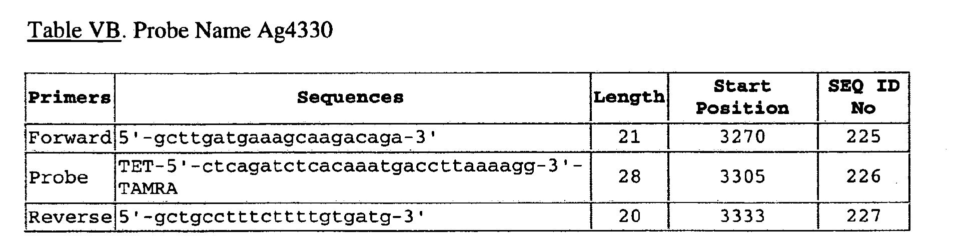

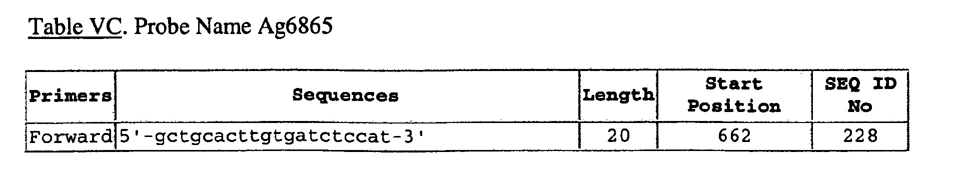

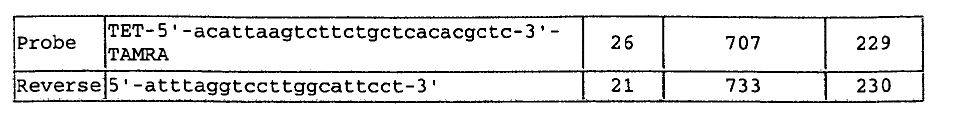

**Table VD. CNS_neurodegeneration_v1.0**

| **Tissue Name** | **Rel. Exp.(%) Ag4329, Run 224344077** | **Rel. Exp.(%) Ag4330, Run 224344731** | **Tissue Name** | **Rel. Exp.(%) Ag4329, Run 224344077** | **Rel. Exp.(%) Ag4330, Run 224344731** |
|---|---|---|---|---|---|
| AD 1 Hippo | 21.9 | 16.5 | Control (Path) 3 Temporal Ctx | 3.9 | 0.0 |
| AD 2 Hippo | 36.9 | 8.2 | Control (Path) 4 Temporal Ctx | 40.3 | 35.6 |
| AD 3 Hippo | 13.6 | 16.7 | AD 1 Occipital Ctx | 28.1 | 15.8 |
| AD 4 Hippo | 11.7 | 2.1 | AD 2 Occipital Ctx (Missing) | 0.0 | 0.0 |
| AD 5 hippo | 96.6 | 40.9 | AD 3 Occipital Ctx | 6.2 | 4.0 |
| AD 6 Hippo | **100.0** | 17.6 | AD 4 Occipital Ctx | 25.0 | 19.1 |
| Control 2 Hippo | 11.4 | 29.9 | AD 5 Occipital Ctx | 60.7 | 43.2 |
| Control 4 Hippo | 18.0 | 13.5 | AD 6 Occipital Ctx | 39.2 | 19.2 |
| Control (Path) 3 Hippo | 6.0 | 5.8 | Control 1 Occipital Ctx | 0.0 | 1.1 |
| AD 1 Temporal Ctx | 7.2 | 5.4 | Control 2 Occipital Ctx | 72.2 | 51.4 |
| AD 2 Temporal Ctx | 18.8 | 47.3 | Control 3 Occipital Ctx | 33.4 | 16.0 |
| AD 3 Temporal Ctx | 0.0 | 6.0 | Control 4 Occipital Ctx | 9.7 | 1.3 |
| AD 4 Temporal Ctx | 19.8 | 16.6 | Control (Path) 1 Occipital Ctx | 85.9 | 81.8 |
| AD 5 Inf Temporal Ctx | 53.6 | **100.0** | Control (Path) 2 Occipital Ctx | 17.3 | 19.3 |
| AD 5 SupTemporal Ctx | 23.8 | 33.7 | Control (Path) 3 Occipital Ctx | 0.0 | 1.9 |
| AD 6 Inf Temporal Ctx Temporal Ctx | 72.2 | 27.2 | Control (Path) 4 Occipital Ctx | 23.0 | 23.5 |
| AD 6 Sup Temporal Ctx | 54.7 | 25.9 | Control 1 Parietal Ctx | 4.9 | 10.6 |
| Control 1 Temporal Ctx | 6.8 | 7.0 | Control 2 Parietal Ctx | 46.0 | 26.1 |
| Control 2 Temporal Ctx | 49.0 | 44.4 | Control 3 Parietal Ctx | 39.2 | 11.0 |
| Control 3 Temporal Ctx | 28.7 | 3.3 | Control (Path) 1 Parietal Ctx | 95.9 | 54.3 |
| Control 4 Temporal Ctx | 9.5 | 1.6 | Control (Path) 2 Parietal Ctx | 53.2 | 42.0 |
| Control (Path) 1 Temporal Ctx | 40.9 | 50.3 | Control (Path) 3 Parietal Ctx | 8.1 | 1.9 |
| Control (Path) 2 Temporal Ctx | 48.0 | 23.8 | Control (Path) 4 Parietal Ctx | 64.6 | 37.9 |

**Table VE. General_screening_panel_v1.4**

| **Tissue Name** | **Rel. Exp.(%) Ag4329, Run 222550606** | **Rel. Exp.(%) Ag4330, Run 222550615** | **Tissue Name** | **Rel. Exp.(%) Ag4329, Run 222550606** | **Rel. Exp.(%) Ag4330, Run 222550615** |
|---|---|---|---|---|---|
| Adipose | 0.0 | 0.0 | Renal ca. TK-10 | 0.0 | 0.0 |
| Melanoma* Hs688(A).T | 0.0 | 0.0 | Bladder | 0.7 | 2.7 |
| Melanoma* Hs688(B).T | 0.0 | 0.0 | Gastric ca. (liver met.) NCI-N87 | 1.9 | 0.0 |
| Melanoma* M14 | 0.0 | 0.0 | Gastric ca. KATO III | 0.0 | 0.0 |
| Melanoma* LOXIMVI | 0.0 | 0.0 | Colon ca. SW-948 | 0.0 | 0.0 |
| Melanoma* SK-MEL-5 | 0.0 | 0.0 | Colon ca. SW480 | 0.0 | 0.0 |
| Squamous cell carcinoma SCC- | 0.0 | 0.0 | Colon ca.* (SW480 met) SW620 | 0.0 | 0.0 |
| Testis Pool | 3.2 | 5.1 | Colon ca. HT29 | 0.0 | 3.0 |
| Prostate ca.* (bone met) PC-3 | 0.0 | 0.0 | Colon ca. HCT-116 | 0.0 | 0.0 |
| Prostate Pool | 0.4 | 1.7 | Colon ca. CaCo-2 | 0.0 | 0.0 |
| Placenta | 0.0 | 0.0 | Colon cancer tissue | 0.0 | 0.0 |
| Uterus Pool | 0.0 | 1.0 | Colon ca. SW 1116 | 0.0 | 0.0 |
| Ovarian ca. OVCAR-3 | 0.0 | 0.6 | Colon ca. Colo-205 | 0.0 | 0.0 |
| Ovarian ca. SK-OV-3 | 0.0 | 1.0 | Colon ca. SW-48 | 0.0 | 0.0 |
| Ovarian ca. OVCAR-4 | 0.0 | 0.0 | Colon Pool | 0.8 | 1.4 |
| Ovarian ca. OVCAR-5 | 0.0 | 0.0 | Small Intestine Pool | 1.2 | 2.6 |
| Ovarian ca. IGROV-1 | 0.0 | 0.9 | Stomach Pool | 0.4 | 2.5 |
| Ovarian ca. OVCAR-8 | 0.0 | 0.0 | Bone Marrow Pool | 0.3 | 0.8 |
| Ovary | 0.9 | 0.6 | Fetal Heart | **100.0** | **100.0** |
| Breast ca. MCF-7 | 0.0 | 0.0 | Heart Pool | 10.2 | 16.5 |
| Breast ca. MDA-MB-231 | 0.0 | 0.0 | Lymph Node Pool | 5.0 | 2.3 |
| Breast ca. BT 549 | 0.4 | 0.0 | Fetal Skeletal Muscle | 0.0 | 0.0 |
| Breast ca. T47D | 0.0 | 1.6 | Skeletal Muscle Pool | 1.7 | 1.1 |
| Breast ca. MDA-N | 0.0 | 0.0 | Spleen Pool | 1.2 | 1.0 |
| Breast Pool | 1.8 | 3.1 | Thymus Pool | 1.2 | 4.3 |
| Trachea | 0.0 | 0.0 | CNS cancer (glio/astro) U87-MG | 0.0 | 0.0 |
| Lung | 1.0 | 1.6 | CNS cancer (glio/astro) U-118-MG | 0.0 | 0.0 |
| Fetal Lung | 0.4 | 0.4 | CNS cancer (neuro;met) SK-N-AS | 0.0 | 1.2 |
| Lung ca. NCI-N417 | 0.0 | 0.0 | CNS cancer (astro) SF-539 | 0.0 | 0.0 |
| Lung ca. LX-1 | 0.0 | 0.0 | CNS cancer (astro) SNB-75 | 0.5 | 0.0 |
| Lung ca. NCI-H146 | 2.4 | 5.4 | CNS cancer (glio) SNB-19 | 0.0 | 0.0 |
| Lung ca. SHP-77 | 17.0 | 47.3 | CNS cancer (glio) SF-295 | 0.0 | 0.0 |
| Lung ca. A549 | 0.0 | 0.0 | Brain (Amygdala) Pool | 2.8 | 2.9 |
| Lung ca. NCI-H526 | 0.0 | 0.0 | Brain (cerebellum) | 0.9 | 1.5 |
| Lung ca. NCI-H23 | 0.0 | 0.0 | Brain (fetal) | 2.3 | 3.1 |
| Lung ca. NCI-H460 | 0.5 | 0.7 | Brain (Hippocampus) Pool | 3.7 | 6.7 |
| Lung ca. HOP-62 | 0.3 | 3.1 | Cerebral Cortex Pool | 7.7 | 16.2 |
| Lung ca. NCI-H522 | 0.2 | 0.0 | Brain (Substantia nigra) Pool | 4.6 | 8.1 |
| Liver | 0.0 | 0.0 | Brain (Thalamus) | 8.8 | 17.9 |
| Fetal Liver | 0.0 | 0.0 | Brain (whole) | 3.0 | 2.6 |
| Liver ca. HepG2 | 0.0 | 0.0 | Spinal Cord Pool | 2.2 | 2.7 |
| Kidney Pool | 6.3 | 6.5 | Adrenal Gland | 0.0 | 0.0 |
| Fetal Kidney | 2.1 | 0.5 | Pituitary gland Pool | 0.0 | 0.4 |
| Renal ca. 786-0 | 0.0 | 0.0 | Salivary Gland | 0.0 | 0.0 |
| Renal ca. A498 | 0.3 | 0.7 | Thyroid (female) | 0.0 | 0.6 |
| Renal ca. ACHN | 0.0 | 0.0 | Pancreatic ca. CAPAN2 | 0.0 | 0.0 |
| Renal ca. UO-31 | 0.0 | 0.0 | Pancreas Pool | 1.4 | 4.7 |

**Table VF. General_screening_panel_v1.6**

| **Tissue Name** | **Rel. Exp.(%) Ag6865, Run 278387549** | **Tissue Name** | **Rel. Exp.(%) Ag6865, Run 278387549** |
|---|---|---|---|
| Adipose | 4.4 | Renal ca. TK-10 | 0.0 |
| Melanoma* Hs688(A).T | 0.0 | Bladder | 1.0 |
| Melanoma* Hs688(B).T | 0.0 | Gastric ca. (liver met.) NCI-N87 | 1.2 |
| Melanoma* M 14 | 0.0 | Gastric ca. KATO III | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 6.3 | Colon ca. HT29 | 0.5 |
| Prostate ca.* (bone met) PC-3 | 0.4 | Colon ca. HCT-116 | 0.0 |
| Prostate Pool | 0.0 | Colon ca. CaCo-2 | 0.0 |
| Placenta | 0.0 | Colon cancer tissue | 0.0 |
| Uterus Pool | 1.3 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 3.1 |
| Ovarian ca. OVCAR-5 | 0.0 | Small Intestine Pool | 2.4 |
| Ovarian ca. IGROV-1 | 0.0 | Stomach Pool | 0.0 |
| Ovarian ca. OVCAR-8 | 0.0 | Bone Marrow Pool | 0.6 |
| Ovary | 0.9 | Fetal Heart | **100.0** |
| Breast ca. MCF-7 | 0.0 | Heart Pool | 12.1 |
| Breast ca. NMA-MB-231 | 0.0 | Lymph Node Pool | 1.7 |
| Breast ca. BT 549 | 0.0 | Fetal Skeletal Muscle | 0.7 |
| Breast ca. T47D | 0.0 | Skeletal Muscle Pool | 1.8 |
| Breast ca.-MDA-N | 0.0 | Spleen Pool | 1.5 |
| Breast Pool | 0.0 | Thymus Pool | 1.6 |
| Trachea | 0.7 | CNS cancer (glio/astro) U87-MG | 0.0 |
| Lung | 0.7 | CNS cancer (glio/astro) U-118-MG | 0.0 |
| Fetal Lung | 0.6 | CNS cancer (neuro;met) SK-N-AS | 0.7 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 2.5 | CNS cancer (glio) SNB-19 | 0.0 |
| Lung ca. SHP-77 | 16.5 | CNS cancer (glio) SF-295 | 0.0 |
| Lung ca. A549 | 0.0 | Brain (Amygdala) Pool | 3.6 |
| Lung ca. NCI-H526 | 0.0 | Brain (cerebellum) | 4.2 |
| Lung ca. NCI-H23 | 0.0 | Brain (fetal) | 3.6 |
| Lung ca. NCI-H460 | 0.0 | Brain (Hippocampus) Pool | 2.7 |
| Lung ca. HOP-62 | 1.5 | Cerebral Cortex Pool | 8.4 |
| Lung ca. NCI-H522 | 0.0 | Brain (Substantia nigra) Pool | 3.3 |
| Liver | 0.0 | Brain (Thalamus) Pool | 7.9 |
| Fetal Liver | 0.6 | Brain (whole) | 1.1 |
| Liver ca. HepG2 | 0.0 | Spinal Cord Pool | 2.4 |
| Kidney Pool | 5.9 | Adrenal Gland | 0.0 |
| Fetal Kidney | 1.8 | Pituitary gland Pool | 1.2 |
| Renal ca. 786-0 | 0.0 | Salivary Gland | 0.0 |
| Renal ca. A498 | 1.7 | Thyroid (female) | 0.0 |
| Renal ca. ACHN | 0.0 | Pancreatic ca. CAPAN2 | 0.0 |
| Renal ca. UO-31 | 0.0 | Pancreas Pool | 0.0 |

**CNS_neurodegeneration_v1.0 Summary:** Ag4329/Ag4330 Two experiments with two different probe and primer sets are in good agreement with significant expression of the CG106924-01 gene in the brains of an independent group of individuals. However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4329/Ag4330 Two experiment with different probe and primer sets are in excellent agreements with highest expression of the CG106924-01 gene in fetal heart (CT=30.5-32). Interestingly, expression of this gene is higher in fetal as compared to the adult heart (CT=33.8-34.6). Therefore, expression of this gene may be used to distinguish fetal heart from adult tissue and also from other samples used in this panel. In addition, the relative overexpression of this gene in fetal heart suggests that the protein product may enhance heart growth or development in the fetus and thus may also act in a regenerative capacity in the adult. Therefore, therapeutic modulation of the protein encoded by this gene could be useful in treatment of heart related diseases.

In addition, this gene is expressed at low levels in some of the regions of the central nervous system examined, including substantia nigra, thalamus, and cerebral cortex. This gene encodes a leucine-rich repeat protein. Leucine rich repeats (LRR) mediate reversible protein-protein interactions and have diverse cellular functions, including cellular adhesion and signaling. Several of these proteins, such as connectin, slit, chaoptin, and Toll have been shown to have a pivotal role in neuronal development in Drosophila, as well as a distinct role in neural development and in the adult nervous system of humans (Battye R., 2001, J. Neurosci. 21: 4290-4298; Itoh A., 1998, Brain Res. Mol. Brain Res. 62: 175-186). In Drosophilia, the LRR region of axon guidance proteins has been shown to be critical for their function (especially in axon repulsion). Since the leucine-rich-repeat protein encoded by this gene shows significant expression in the cerebral cortex, it is an excellent candidate neuronal guidance protein for axons, dendrites and/or growth cones in general. Therefore, therapeutic modulation of the levels of this protein, or possible signaling via this protein, may be of utility in enhancing/directing compensatory synaptogenesis and fiber growth in the CNS in response to neuronal death (stroke, head trauma), axon lesion (spinal cord injury), or neurodegeneration (Alzheimer's, Parkinson's, Huntington's, vascular dementia or any neurodegenerative disease).

**General_screening_panel_v1.6 Summary:** Ag6865 Highest expression of the CG106915-01 gene is detected in fetal heart (CT=30.2). Interestingly, expression of this gene is higher in fetal as compared to the adult heart (CT=33.3). Therefore, expression of this gene may be used to distinguish fetal heart from adult tissue and also from other samples used in this panel. In addition, the relative overexpression of this gene in fetal heart suggests that the protein product may enhance heart growth or development in the fetus and thus may also act in a regenerative capacity in the adult. Therefore, therapeutic modulation of the protein encoded by this gene could be useful in treatment of heart related diseases.

In addition, this gene is expressed at low levels in some of the regions of the central nervous system examined, including thalamus, and cerebral cortex. Please see Panel 1.4 for a discussion of the potential use of this gene in treatment of central nervous system disorders.

Low expression of this gene is also seen in a lung cancer SHP-77 cell line. Therefore, expression of this gene may be used as a marker to detect the presence of lung cancer and therapeutic modulation of this gene may be useful in the treatment of this cancer.

Low expression of this gene is also seen in kidney, testis and adipose tissue. Therefore, therapeutic modulation of this gene may be useful in the treatment of diseases associated with these tissues including obesity, diabetes, lupus, glomerulonephritis, fertility and hypogonadism.

### W. CG106942-01: Nramp-like membrane protein

Expression of gene CG106942-01 was assessed using the primer-probe set Ag4331, described in Table WA. Results of the RTQ-PCR runs are shown in Tables WB, WC and WD.

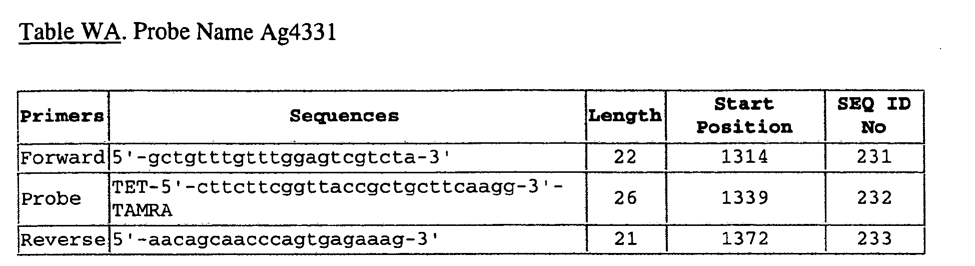

**Table WB. CNS_neurodegeneration_v1.0**

| **Tissue Name** | **Rel. Exp.(%) Ag4331, Run 224344734** | **Tissue Name** | **Rel. Exp.(%) Ag4331, Run 224344734** |
|---|---|---|---|
| AD 1 Hippo | 8.8 | Control (Path) 3 Temporal Ctx | 8.2 |
| AD 2 Hippo | 19.2 | Control (Path) 4 Temporal Ctx | 18.8 |
| AD 3 Hippo | 13.2 | AD I Occipital Ctx | 1.2 |
| AD 4 Hippo | 10.9 | AD 2 Occipital Ctx (Missing) | 0.0 |
| AD 5 hippo | 56.6 | AD 3 Occipital Ctx | 5.1 |
| AD 6 Hippo | 40.9 | AD 4 Occipital Ctx | 13.3 |
| Control 2 Hippo | 29.3 | AD 5 Occipital Ctx | 8.5 |
| Control 4 Hippo | 13.4 | AD 6 Occipital Ctx | 36.3 |
| Control (Path) 3 Hippo | 1.8 | Control 1 Occipital Ctx | 2.8 |
| AD 1 Temporal Ctx | 7.1 | Control 2 Occipital Ctx | 59.5 |
| AD 2 Temporal Ctx | 20.2 | Control 3 Occipital Ctx | 2.2 |
| AD 3 Temporal Ctx | 5.8 | Control 4 Occipital Ctx | 8.9 |
| AD 4 Temporal Ctx | 9.5 | Control (Path) 1 Occipital Ctx | 62.0 |
| AD 5 Inf Temporal Ctx | 68.8 | Control (Path) 2 Occipital Ctx | 5.6 |
| AD 5 SupTemporal Ctx | 28.3 | Control (Path) 3 Occipital Ctx | 2.1 |
| AD 6 Inf Temporal Ctx | 39.5 | Control (Path) 4 Occipital Ctx | 13.8 |
| AD 6 Sup Temporal Ctx | 21.2 | Control 1 Parietal Ctx | 4.6 |
| Control 1 Temporal Ctx | 2.2 | Control 2 Parietal Ctx | 15.9 |
| Control 2 Temporal Ctx | 91.4 | Control 3 Parietal Ctx | 17.8 |
| Control 3 Temporal Ctx | 8.2 | Control (Path) 1 Parietal Ctx | **100.0** |
| Control 4 Temporal Ctx | 8.4 | Control (Path) 2 Parietal Ctx | 14.1 |
| Control (Path) 1 Temporal Ctx | 37.1 | Control (Path) 3 Parietal Ctx | 2.3 |
| Control (Path) 2 Temporal Ctx | 4.1 | Control (Path) 4 Parietal Ctx | 21.3 |

**Table WC. General_screening_panel_v1.4**

| **Tissue Name** | **Rel. Exp.(%) Ag4331, Run 222556053** | **Tissue Name** | **Rel. Exp.(%) Ag4331, Run 222556053** |
|---|---|---|---|
| Adipose | 0.2 | Renal ca. TK-10 | 31.0 |
| Melanoma* Hs688(A).T | 2.4 | Bladder | 3.8 |
| Melanoma* Hs688(B).T | 1.2 | Gastric ca. (liver met.) NCI-N87 | 4.2 |
| Melanoma* M14 | 24.7 | Gastric ca. KATO III | 1.7 |
| Melanoma* LOXIMVI | 7.8 | Colon ca. SW-948 | 0.7 |
| Melanoma* SK-MEL-5 | 3.5 | Colon ca. SW480 | 83.5 |
| Squamous cell carcinoma SCC-4 | 3.1 | Colon ca.* (SW480 met) SW620 | 10.7 |
| Testis Pool | 3.8 | Colon ca. HT29 | 1.2 |
| Prostate ca.* (bone met) PC-3 | 2.1 | Colon ca. HCT-116 | 4.4 |
| Prostate Pool | 2.0 | Colon ca. CaCo-2 | 29.7 |
| Placenta | 4.0 | Colon cancer tissue | 24.0 |
| Uterus Pool | 0.2 | Colon ca. SW1116 | 4.2 |
| Ovarian ca. OVCAR-3 | 1.9 | Colon ca. Colo-205 | 2.2 |
| Ovarian ca. SK-OV-3 | 2.0 | Colon ca. SW-48 | 17.9 |
| Ovarian ca. OVCAR-4 | 7.6 | Colon Pool | 1.3 |
| Ovarian ca. OVCAR-5 | 16.8 | Small Intestine Pool | 0.9 |
| Ovarian ca. IGROV-1 | 12.8 | Stomach Pool | 2.0 |
| Ovarian ca. OVCAR-8 | 7.1 | Bone Marrow Pool | 0.1 |
| Ovary | 4.7 | Fetal Heart | 1.8 |
| Breast ca. MCF-7 | 11.7 | Heart Pool | 0.5 |
| Breast ca. MDA-MB-231 | 4.3 | Lymph Node Pool | 2.2 |
| Breast ca. BT 549 | 10.4 | Fetal Skeletal Muscle | 0.8 |
| Breast ca. T47D | 51.8 | Skeletal Muscle Pool | 0.1 |
| Breast ca. MDA-N | 31.4 | Spleen Pool | 2.5 |
| Breast Pool | 1.7 | Thymus Pool | 1.6 |
| Trachea | 1.2 | CNS cancer (glio/astro) U87-MG | 8.5 |
| Lung | 1.0 | CNS cancer (glio/astro) U-118-MG | 6.8 |
| FetalLung | 1.6 | CNS cancer (neuro;met) SK-N-AS | 21.6 |
| Lung ca. NCI-N417 | 60.3 | CNS cancer (astro) SF-539 | 2.5 |
| Lung ca. LX-1 | 13.6 | CNS cancer (astro) SNB-75 | 32.8 |
| Lung ca. NCI-H146 | 42.9 | CNS cancer (glio) SNB-19 | 8.7 |
| Lung ca. SHP-77 | **100.0** | CNS cancer (glio) SF-295 | 2.0 |
| Lung ca. A549 | 0.8 | Brain (Amygdala) Pool | 10.7 |
| Lung ca. NCI-H526 | 58.2 | Brain (cerebellum) | 39.5 |
| Lung ca. NCI-H23 | 4.0 | Brain (fetal) | 37.4 |
| Lung ca. NCI-H460 | 9.5 | Brain (Hippocampus) Pool | 32.8 |
| Lung ca. HOP-62 | 0.6 | Cerebral Cortex Pool | 26.6 |
| Lung ca. NCI-H522 | 16.0 | Brain (Substantia nigra) Pool | 34.9 |
| Liver | 0.3 | Brain (Thalamus) Pool | 21.2 |
| Fetal Liver | 2.6 | Brain (whole) | 34.9 |
| Liver ca. HepG2 | 64.6 | Spinal Cord Pool | 6.8 |
| Kidney Pool | 2.5 | Adrenal Gland | 8.2 |
| Fetal Kidney | 1.7 | Pituitary gland Pool | 3.6 |
| Renal ca. 786-0 | 8.0 | Salivary Gland | 0.8 |
| Renal ca. A498 | 18.4 | Thyroid (female) | 4.7 |
| Renal ca. ACHN | 11.3 | Pancreatic ca. CAPAN2 | 1.6 |
| Renal ca. UO-31 | 2.5 | Pancreas Pool | 4.3 |

**Table WD. Panel 4.1D**

| **Tissue Name** | **Rel. Exp.(%) Ag4331, Run 183718671** | **Tissue Name** | **Rel. Exp.(%) Ag4331, Run 183718671** |
|---|---|---|---|
| Secondary Th1 act | 19.2 | HUVEC IL-1beta | 0.0 |
| Secondary Th2 act | 63.7 | HUVEC IFN gamma | 0.0 |
| Secondary Tr1 act | 13.2 | HUVEC TNF alpha + IFN gamma | 0.0 |
| Secondary Th1 rest | 4.0 | HUVEC TNF alpha + IL4 | 0.0 |
| Secondary Th2 rest | 0.0 | HUVEC IL-11 | 0.0 |
| Secondary Tr1 rest | 1.5 | Lung Microvascular EC none | 0.0 |
| Primary Th1 act | 24.0 | Lung Microvascular EC TNFalpha + IL-1 beta | 0.0 |
| Primary Th2 act | 47.3 | Microvascular Dermal EC none | 0.0 |
| Primary Tr1 act | 7.0 | Microsvasular Dermal EC TNFalpha + IL-1beta | 0.0 |
| Primary Th1 rest | 0.0 | Bronchial epithelium TNFalpha + IL1beta | 10.1 |
| Primary Th2 rest | 4.7 | Small airway epithelium none | 0.0 |
| Primary Tr1 rest | 11.8 | Small airway epithelium TNFalpha + IL-1 beta | 7.9 |
| CD45RA CD4 lymphocyte act | 55.5 | Coronery artery SMC rest | 8.9 |
| CD45RO CD4 lymphocyte act | 25.3 | Coronery artery SMC TNFalpha + IL-1beta | 8.0 |
| CD8 lymphocyte act | 21.3 | Astrocytes rest | 39.0 |
| Secondary CD8 lymphocyte rest | 18.6 | Astrocytes TNFalpha + IL-1beta | 25.3 |
| Secondary CD8 lymphocyte act | 12.9 | KU-812 (Basophil) rest | 0.0 |
| CD4 lymphocyte none | 0.0 | KU-812 (Basophil) PMA/ionomycin | 1.4 |
| 2ry Th1/Th2/Tr1_anti-CD95 CH11 | 14.2 | CCD1106 (Keratinocytes) none | 11.6 |
| LAK cells rest | 12.0 | CCD1106 (Keratinocytes) TNFalpha + IL-1 beta | 8.7 |
| LAK cells IL-2 | 25.3 | Liver cirrhosis | 6.9 |
| LAK cells IL-2+IL-12 | 15.3 | NCI-H292 none | 67.8 |
| LAK cells IL-2+IFN gamma | 27.2 | NCI-H292 IL-4 | **100.0** |
| LAK cells IL-2+ IL-18 | 6.6 | NCI-H292 IL-9 | 75.3 |
| LAK cells PMA/ionomycin | 0.0 | NCI-H292 IL-13 | 50.7 |
| NK Cells IL-2 rest | 22.4 | NCI-H292 IFN gamma | 77.9 |
| Two Way MLR 3 day | 8.9 | HPAEC none | 0.0 |
| Two Way MLR 5 day | 23.5 | HPAEC TNF alpha + IL-1 beta | 0.0 |
| Two Way MLR 7 day | 10.5 | Lung fibroblast none | 9.9 |
| PBMC rest | 0.0 | Lung fibroblast TNF alpha + IL-1 beta | 50.0 |
| PBMC PWM | 52.5 | Lung fibroblast IL-4 | 7.7 |
| PBMC PHA-L | 33.7 | Lung fibroblast IL-9 | 13.3 |
| Ramos (B cell) none | 5.9 | Lung fibroblast IL-13 | 17.4 |
| Ramos (B cell) ionomycin | 10.4 | Lung fibroblast IFN gamma | 26.6 |
| B lymphocytes PWM | 28.3 | Dermal fibroblast CCD1070 rest | 28.9 |
| B lymphocytes CD40L and IL-4 | 18.0 | Dermal fibroblast CCD 1070 TNF alpha | 19.1 |
| EOL-1 dbcAMP | 0.0 | Dermal fibroblast CCD1070 IL-1 beta | 16.6 |
| EOL-1 dbcAMP PMA/ionomycin | 2.3 | Dermal fibroblast IFN gamma | 13.0 |
| Dendritic cells none | 0.0 | Dermal fibroblast IL-4 | 26.6 |
| Dendritic cells LPS | 0.0 | Dermal Fibroblasts rest | 22.1 |
| Dendritic cells anti-CD40 | 0.0 | Neutrophils TNFa+LPS | 0.0 |
| Monocytes rest | 0.0 | Neutrophils rest | 0.0 |
| Monocytes LPS | 0.0 | Colon | 1.6 |
| Macrophages rest | 0.0 | Lung | 0.0 |
| Macrophages LPS | 0.0 | Thymus | 14.7 |
| HUVEC none | 0.0 | Kidney | 24.5 |
| HUVEC starved | 0.0 | | |

**CNS_neurodegeneration_v1.0 Summary:** Ag4331 This panel confirms the expression of the CG106942-01 gene at low levels in the brains of an independent group of individuals. However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4331 Highest expression of the CG106942-01 gene is detected in a lung cancer SHP-77 cell line (CT=28.5). High to moderate levels of expression of this gene is also seen in cluster of cancer cell lines including CNS, colon, renal, liver, breast, ovarian and melanoma cancer cell lines. Therefore, expression of this gene may be used as diagnostic marker for detection of these cancers and therapeutic modulation of this gene product may be beneficial in the treatment of these cancers.

Among tissues with metabolic or endocrine function, this gene is expressed at moderate to low levels in pancreas, adrenal gland, thyroid, pituitary gland, fetal heart, fetal liver and the gastrointestinal tract. Therefore, therapeutic modulation of the activity of this gene may prove useful in the treatment of endocrine/metabolically related diseases, such as obesity and diabetes.

Interestingly, expression of this gene is higher in fetal (CT=33.8) as compared to adult liver (CT=37). Therefore, expression of this gene may be used to distinguish the fetal tissue from the adult liver. In addition, the relative overexpression of this gene in fetal liver suggests that the protein product may enhance liver growth or development in the fetus and thus may also act in a regenerative capacity in the adult. Therefore, therapeutic modulation of the protein encoded by this gene could be useful in treatment of liver related diseases.

In addition, this gene is expressed at high to moderate levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

**Panel 4.1D Summary:** Ag4331 Highest expression of the CG106942-01 gene is detected in IL4 treated NCI-H292 cells (CT=33.4). In addition, moderate to low expression of this gene is also seen in activated primary and secondary Th2 cells, activated CD45RA CD4 lymphocytes, PWM/PHA-L treated PBMC cells, resting astrocytes, untreated and cytokine treated NCI-H292 cells, TNF alpha + IL-1 beta treated lung fibroblasts. Therefore, therapeutic modulation of this gene product may be beneficial in the treatment of T cells and B cells mediated diseases such as systemic lupus erythematosus, Crohn's disease, ulcerative colitis, multiple sclerosis, chronic obstructive pulmonary disease, asthma, emphysema, rheumatoid arthritis, or psoriasis.

### X. CG107513-01: Syntaxin domain containing protein

Expression of gene CG107513-01 was assessed using the primer-probe set Ag4339, described in Table XA. Results of the RTQ-PCR runs are shown in Tables XB, XC and XD.

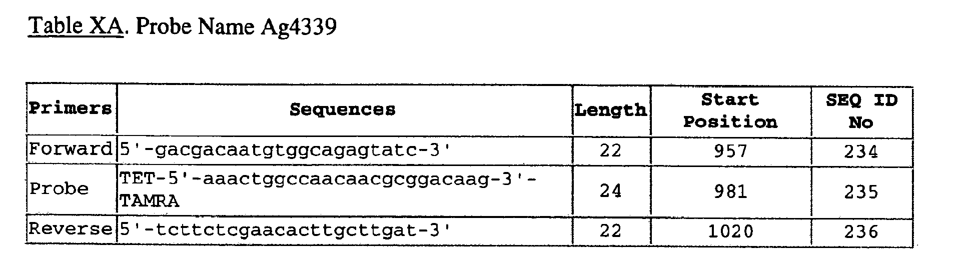

**Table XB. CNS_neurodegeneration_v1.0**

| **Tissue Name** | **Rel. Exp.(%) Ag4339, Run 224358973** | **Tissue Name** | **Rel. Exp.(%) Ag4339, Run 224358973** |
|---|---|---|---|
| AD 1 Hippo | 7.7 | Control (Path) 3 Temporal Ctx | 2.7 |
| AD 2 Hippo | 16.5 | Control (Path) 4 Temporal Ctx | 29.5 |
| AD 3 Hippo | 2.2 | AD 1 Occipital Ctx | 4.8 |
| AD 4 Hippo | 2.4 | AD 2 Occipital Ctx (Missing) | 0.0 |
| AD 5 Hippo | 97.3 | AD 3 Occipital Ctx | 2.0 |
| AD 6 Hippo | 33.2 | AD 4 Occipital Ctx | 18.2 |
| Control 2 Hippo | 20.7 | AD 5 Occipital Ctx | 40.9 |
| Control 4 Hippo | 2.5 | AD 6 Occipital Ctx | 19.6 |
| Control (Path) 3 Hippo | 2.4 | Control 1 Occipital Ctx | 0.8 |
| AD 1 Temporal Ctx | 8.5 | Control 2 Occipital Ctx | 60.3 |
| AD 2 Temporal Ctx | 23.8 | Control 3 Occipital Ctx | 17.4 |
| AD 3 Temporal Ctx | 2.4 | Control 4 Occipital Ctx | 2.2 |
| AD 4 Temporal Ctx | 17.0 | Control (Path) 1 Occipital Ctx | **100.0** |
| AD 5 Inf Temporal Ctx | 86.5 | Control (Path) 2 Occipital Ctx | 15.7 |
| AD 5 Sup Temporal Ctx | 28.5 | Control (Path) 3 Occipital Ctx | 1.4 |
| AD 6 Inf Temporal Ctx | 31.4 | Control (Path) 4 Occipital Ctx | 19.8 |
| AD 6 Sup Temporal Ctx | 33.7 | Control 1 Parietal Ctx | 2.4 |
| Control 1 Temporal Ctx | 1.8 | Control 2 Parietal Ctx | 31.0 |
| Control 2 Temporal Ctx | 41.2 | Control 3 Parietal Ctx | 21.2 |
| Control 3 Temporal Ctx | 16.6 | Control (Path) 1 Parietal Ctx | 94.6 |
| Control 3 Temporal Ctx | 4.3 | Control (Path) 2 Parietal Ctx | 22.7 |
| Control (Path) 1 Temporal Ctx | 72.7 | Control (Path) 3 Parietal Ctx | 2.2 |
| Control (Path) 2 Temporal Ctx | 40.6 | Control (Path) 4 Parietal Ctx | 47.6 |

**Table XC. General_screening_panel_v1.4**

| **Tissue Name** | **Rel. Exp.(%) Ag4339, Run 222523508** | **Tissue Name** | **Rel. Exp.(%) Ag4339, Run 222523508** |
|---|---|---|---|
| Adipose | 0.7 | Renal ca. TK-10 | 0.7 |
| Melanoma* Hs688(A).T | 0.9 | Bladder | 0.2 |
| Melanoma* Hs688(B).T | 0.8 | Gastric ca. (liver met.) NCI-N87 | 0.1 |
| Melanoma* M14 | 12.1 | Gastric ca. KATO III | 0.0 |
| Melanoma* LOXIMVI | 0.1 | Colon ca. SW-948 | 0.0 |
| Melanoma* SK-MEL-5 | 31.2 | Colon ca. SW480 | 3.5 |
| Squamous cell carcinoma SCC-4 | 1.2 | Colon ca.* (SW480 met) SW620 | 0.4 |
| Testis Pool | 0.4 | Colon ca. HT29 | 0.0 |
| Prostate ca.* (bone met) PC-3 | 4.3 | Colon ca. HCT-116 | 1.4 |
| Prostate Pool | 0.3 | Colon ca. CaCo-2 | 0.2 |
| Placenta | 0.8 | Colon cancer tissue | 0.4 |
| Uterus Pool | 0.3 | Colon ca. SW1116 | 0.7 |
| Ovarian ca. OVCAR-3 | 1.9 | Colon ca. Colo-205 | 0.1 |
| Ovarian ca. SK-OV-3 | 1.6 | Colon ca. SW-48 | 0.0 |
| Ovarian ca. OVCAR-4 | 0.7 | Colon Pool | 0.5 |
| Ovarian ca. OVCAR-5 | 1.1 | Small Intestine Pool | 1.6 |
| Ovarian ca. IGROV-1 | 1.3 | Stomach Pool | 0.3 |
| Ovarian ca. OVCAR-8 | 1.2 | Bone Marrow Pool | 0.3 |
| Ovary | 0.8 | Fetal Heart | 1.3 |
| Breast ca. MCF-7 | 0.1 | Heart Pool | 0.2 |
| Breast ca. MDA-MB-231 | 1.6 | Lymph Node Pool | 0.6 |
| Breast ca. BT 549 | 5.3 | Fetal Skeletal Muscle | 1.3 |
| Breast ca. T47D | 1.7 | Skeletal Muscle Pool | 2.6 |
| Breast ca. MDA-N | 7.3 | Spleen Pool | 4.9 |
| Breast Pool | 0.6 | Thymus Pool | 0.8 |
| Trachea | 0.8 | CNS cancer (glio/astro) U87-MG | 1.3 |
| Lung | 0.2 | CNS cancer (glio/astro) U- | 1.5 |
| | | 118-MG | |
| Fetal Lung | 11.7 | CNS cancer (neuro;met) SK-N-AS | 3.2 |
| Lung ca. NCI-N417 | 0.7 | CNS cancer (astro) SF-539 | 0.6 |
| Lung ca. LX-1 | 0.2 | CNS cancer (astro) SNB-75 | 1.4 |
| Lung ca. NCI-H146 | 2.1 | CNS cancer (glio) SNB-19 | 1.1 |
| Lung ca. SHP-77 | 3.0 | CNS cancer (glio) SF-295 | 2.9 |
| Lung ca. A549 | 0.7 | Brain (Amygdala) Pool | 13.4 |
| Lung ca. NCI-H526 | 0.9 | Brain (cerebellum) | 51.1 |
| Lung ca. NCI-H23 | 1.3 | Brain (fetal) | 20.3 |
| Lung ca. NCI-H460 | 0.0 | Brain (Hippocampus) Pool | 11.1 |
| Lung ca. HOP-62 | 0.6 | Cerebral Cortex Pool | 16.8 |
| Lung ca. NCI-H522 | 4.5 | Brain (Substantia nigra) Pool | 13.8 |
| Liver | 0.1 | Brain (Thalamus) Pool | 20.0 |
| Fetal Liver | **100.0** | Brain (whole) | 17.6 |
| Liver ca. HepG2 | 0.0 | Spinal Cord Pool | 15.7 |
| Kidney Pool | 1.8 | Adrenal Gland | 2.4 |
| Fetal Kidney | 1.4 | Pituitary gland Pool | 1.7 |
| Renal ca. 786-0 | 0.6 | Salivary Gland | 0.2 |
| Renal ca. A498 | 1.2 | Thyroid (female) | 0.4 |
| Renal ca. ACHN | 2.0 | Pancreatic ca. CAPAN2 | 0.1 |
| Renal ca. UO-31 | 0.8 | Pancreas Pool | 0.4 |

**Table XD. Panel 4.1D**

| **Tissue Name** | **Rel. Exp.(%) Ag4339, Run 184798183** | **Tissue Name** | **Rel. Exp.(%) Ag4339, Run 184798183** |
|---|---|---|---|
| Secondary Th1 act | **100.0** | HUVEC IL-1beta | 15.7 |
| Secondary Th2 act | 75.8 | HUVEC IFN gamma | 18.2 |
| Secondary Tr1 act | 59.0 | HUVEC TNF alpha + IFN gamma | 53.2 |
| Secondary Th1 rest | 23.0 | HUVEC TNF alpha + IL4 | 9.9 |
| Secondary Th2 rest | 9.1 | HUVEC IL-11 | 7.0 |
| Secondary Tr1 rest | 9.6 | Lung Microvascular EC none | 4.2 |
| Primary Th1 act | 92.0 | Lung Microvascular EC TNFalpha + IL-1beta | 38.2 |
| Primary Th2 act | 19.9 | Microvascular Dermal EC none | 8.7 |
| Primary Tr1 act | 33.0 | Microsvasular Dermal EC TNFalpha + IL-1beta | 30.1 |
| Primary Th1 rest | 3.1 | Bronchial epithelium TNFalpha + IL1beta | 11.7 |
| Primary Th2 rest | 1.9 | Small airway epithelium none | 2.7 |
| Primary Tr1 rest | 1.8 | Small airway epithelium TNFalpha + IL-1beta | 1.2 |
| CD45RA CD4 lymphocyte act | 38.4 | Coronery artery SMC rest | 8.1 |
| CD45RO CD4 | 72.2 | Coronery artery SMC | 13.3 |
| lymphocyte act | | TNFalpha + IL-1beta | |
| CD8 lymphocyte act | 29.7 | Astrocytes rest | 16.2 |
| Secondary CD8 lymphocyte rest | 18.3 | Astrocytes TNFalpha + IL-1beta | 25.9 |
| Secondary CD8 lymphocyte act | 16.7 | KU-812 (Basophil) rest | 47.0 |
| CD4 lymphocyte none | 4.9 | KU-812 (Basophil) PMA/ionomycin | 61.6 |
| 2ry Th1/Th2/Tr1_anti-CD95 CH11 | 25.2 | CCD1106 (Keratinocytes) none | 19.6 |
| LAK cells rest | 3.1 | CCD1106 (Keratinocytes) TNFalpha + IL-1 beta | 12.7 |
| LAK cells IL-2 | 12.9 | Liver cirrhosis | 1.9 |
| LAK cells IL-2+IL-12 | 21.0 | NCI-H292 none | 5.0 |
| LAK cells IL-2+IFN gamma | 10.1 | NCI-H292 IL-4 | 11.8 |
| LAK cells IL-2+ IL-18 | 14.2 | NCI-H292 IL-9 | 5.9 |
| LAK cells PMA/ionomycin | 8.2 | NCI-H292 IL-13 | 7.4 |
| NK Cells IL-2 rest | 14.3 | NCI-H292 IFN gamma | 2.2 |
| Two Way MLR 3 day | 13.7 | HPAEC none | 5.8 |
| Two Way MLR 5 day | 31.6 | HPAEC TNF alpha + IL-1 beta | 35.4 |
| Two Way MLR 7 day | 14.4 | Lung fibroblast none | 25.0 |
| PBMC rest | 6.1 | Lung fibroblast TNF alpha + IL-1 beta | 12.5 |
| PBMC PWM | 69.7 | Lung fibroblast IL-4 | 11.4 |
| PBMC PHA-L | 33.4 | Lung fibroblast IL-9 | 24.0 |
| Ramos (B cell) none | 8.8 | Lung fibroblast IL-13 | 10.7 |
| Ramos (B cell) ionomycin | 8.6 | Lung fibroblast IFN gamma | 2.5 |
| B lymphocytes PWM | 58.6 | Dermal fibroblast CCD 1070 rest | 29.5 |
| B lymphocytes CD40L and IL-4 | 12.9 | Dermal fibroblast CCD1070 TNF alpha | 12.8 |
| EOL-1 dbcAMP | 6.5 | Dermal fibroblast CCD1070 IL-1 beta | 16.6 |
| EOL-1 dbcAMP PMA/ionomycin | 3.2 | Dermal fibroblast IFN gamma | 2.9 |
| Dendritic cells none | 15.3 | Dermal fibroblast IL-4 | 12.7 |
| Dendritic cells LPS | 85.3 | Dermal Fibroblasts rest | 30.4 |
| Dendritic cells anti-CD40 | 22.8 | Neutrophils TNFa+LPS | 0.0 |
| Monocytes rest | 0.0 | Neutrophils rest | 6.9 |
| Monocytes LPS | 31.4 | Colon | 1.9 |
| Macrophages rest | 11.0 | Lung | 8.4 |
| Macrophages LPS | 17.8 | Thymus | 13.8 |
| HUVEC none | 12.1 | Kidney | 16.2 |
| HUVEC starved | 10.0 | | |

**CNS_neurodegeneration_v1.0** Summary: Ag4339 This panel confirms the expression of the CG107513-01 gene at low levels in the brains of an independent group of individuals. However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4339 Highest expression of the CG107513-01 gene is detected in fetal liver (CT=24). Interestingly, this gene is expressed at much higher levels in fetal (CT= 24) when compared to adult liver(CT=34.9). Thus expression of this gene can be used to distinguish fetal from adult liver and also from other samples used in this panel. In addition, the relative overexpression of this gene in fetal liver suggests that the protein product may enhance liver growth or development in the fetus and thus may also act in a regenerative capacity in the adult. Therefore, therapeutic modulation of the membrane protein encoded by this gene may be useful in treatment of liver related diseases.

Among tissues with metabolic or endocrine function, this gene is expressed at moderate levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

In addition, this gene is expressed at high levels in all regions of the central nervous system examined, including amygdala, hippocampus, substantia nigra, thalamus, cerebellum, cerebral cortex, and spinal cord. Therefore, therapeutic modulation of the membrane protein encoded by this gene may be useful in the treatment of central nervous system disorders such as Alzheimer's disease, Parkinson's disease, epilepsy, multiple sclerosis, schizophrenia and depression.

**Panel 4.1D Summary:** Ag4339 Highest expression of the CG107513-01 gene is detected in activated secondary Th1 cells (CT=30.7). This gene is expressed at high to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. This pattern is in agreement with the expression profile in General_screening_panel_v1.4 and also suggests a role for the gene product in cell survival and proliferation. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### Y. CG107533-02: TUMOR NECROSIS FACTOR (LIGAND) SUPERFAMILY, MEMBER 7

Expression of gene CG107533-02 was assessed using the primer-probe set Ag6859, described in Table YA. Results of the RTQ-PCR runs are shown in Table YB.

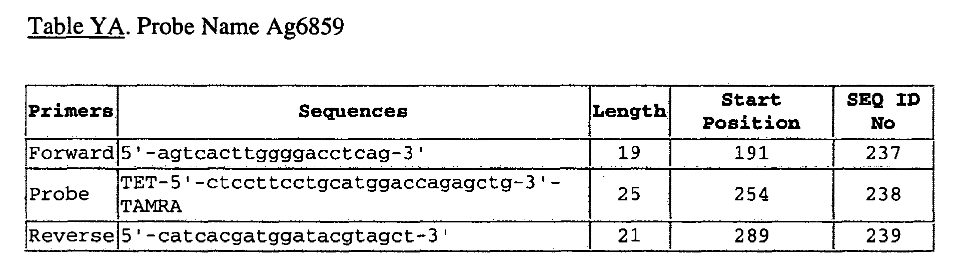

**Table YB. General_screening_panel_v1.6**

| **Tissue Name** | **Rel. Exp.(%) Ag6859, Run 278387508** | **Tissue Name** | **Rel. Exp.(%) Ag6859, Run 278387508** |
|---|---|---|---|
| Adipose | 0.3 | Renal ca. TK-10 | 4.5 |
| Melanoma* Hs688(A).T | 0.0 | Bladder | 0.0 |
| Melanoma* Hs688(B).T | 0.0 | Gastric ca. (liver met.) NCI-N87 | 0.0 |
| Melanoma* M14 | 0.7 | Gastric ca. KATO III | 0.2 |
| Melanoma* LOXIMVI | 3.0 | Colon ca. SW-948 | 5.8 |
| Melanoma* SK-MEL-5 | 0.0 | Colon ca. SW480 | 2.5 |
| Squamous cell carcinoma SCC-4 | 0.2 | Colon ca.* (SW480 met) SW620 | 0.0 |
| Testis Pool | 0.0 | Colon ca. HT29 | 0.0 |
| Prostate ca.* (bone met) PC-3 | 0.0 | Colon ca. HCT-116 | 0.1 |
| Prostate Pool | 0.0 | Colon ca. CaCo-2 | 0.0 |
| Placenta | 0.0 | Colon cancer tissue | 0.5 |
| Uterus Pool | 0.0 | Colon ca. SW1116 | 0.0 |
| Ovarian ca. OVCAR-3 | 0.2 | Colon ca. Colo-205 | 0.0 |
| Ovarian ca. SK-OV-3 | 57.8 | Colon ca. SW-48 | 0.0 |
| Ovarian ca. OVCAR-4 | 0.1 | Colon Pool | 0.0 |
| Ovarian ca. OVCAR-5 | 0.0 | Small Intestine Pool | 0.2 |
| Ovarian ca. IGROV-1 | 2.4 | Stomach Pool | 0.0 |
| Ovarian ca. OVCAR-8 | 2.0 | Bone Marrow Pool | 0.0 |
| Ovary | 0.0 | Fetal Heart | 0.0 |
| Breast ca. MCF-7 | 0.0 | Heart Pool | 0.0 |
| Breast ca. MDA-MB-231 | 0.0 | Lymph Node Pool | 0.0 |
| Breast ca. BT 549 | **100.0** | Fetal Skeletal Muscle | 0.0 |
| Breast ca. T47D | 0.0 | Skeletal Muscle Pool | 0.0 |
| Breast ca. MDA-N | 0.4 | Spleen Pool | 0.2 |
| Breast Pool | 0.0 | Thymus Pool | 0.0 |
| Trachea | 0.0 | CNS cancer (glio/astro) U87-MG | 22.4 |
| Lung | 0.1 | CNS cancer (glio/astro) U-118-MG | 21.5 |
| Fetal Lung | 0.0 | CNS cancer (neuro;met) SK-N-AS | 0.0 |
| Lung ca. NCI-N417 | 0.0 | CNS cancer (astro) SF-539 | 1.0 |
| Lung ca. LX-1 | 0.0 | 75 cancer (astro) SNB- | 1.2 |
| Lung ca. NCI-H146 | 0.0 | CNS cancer (glio) SNB-19 | 1.9 |
| Lung ca. SHP-77 | 0.0 | CNS cancer (glio) SF-295 | 7.1 |
| Lung ca. A549 | 0.3 | Brain (Amygdala) Pool | 0.0 |
| Lung ca. NCI-H526 | 1.3 | Brain (cerebellum) | 0.0 |
| Lung ca. NCI-H23 | 6.8 | Brain (fetal) | 0.0 |
| Lung ca. NCI-H460 | 13.6 | Brain (Hippocampus) Pool | 0.0 |
| Lung ca. HOP-62 | 0.1 | Cerebral Cortex Pool | 0.0 |
| Lung ca. NCI-H522 | 0.0 | Brain (Substantia nigra) Pool | 0.4 |
| Liver | 0.0 | Brain (Thalamus) Pool | 0.2 |
| Fetal Liver | 0.0 | Brain (whole) | 0.0 |
| Liver ca. HepG2 | 0.0 | Spinal Cord Pool | 0.0 |
| Kidney Pool | 0.0 | Adrenal Gland | 0.0 |
| Fetal Kidney | 0.0 | Pituitary gland Pool | 0.0 |
| Renal ca. 786-0 | 44.1 | Salivary Gland | 0.0 |
| Renal ca. A498 | 13.1 | Thyroid (female) | 0.0 |
| Renal ca. ACHN | 6.1 | Pancreatic ca. CAPAN2 | 0.0 |
| Renal ca. UO-31 | 3.8 | Pancreas Pool | 0.0 |

**General_screening_panel_v1.6 Summary:** Ag6859 Highest expression of the CG107533-02 gene is detected in breast cancer BT 549 cell line (CT=29.4). In addition, moderate to low levels of expression of this gene is also seen in number of cancer cell lines derived from colon, lung, renal, breast, ovarian, melanoma and brain cancers. Interestingly, expression of this gene is low or undectable in the samples derived from normal tissues.

Thus, expression of this gene may be used to differentiate between these samples and other samples on this panel and as a marker to detect the presence of these cancers. Furthermore, therapeutic modulation of the expression or function of this gene may be effective in the treatment of colon, lung, renal, breast, ovarian, melanoma and brain cancers.

### Z. CG107562-01 and CG107562-02: TM LRR Ig FnIII domains

Expression of gene CG107562-01 and CG107562-02 was assessed using the primer-probe set Ag4340, described in Table ZA. Results of the RTQ-PCR runs are shown in Tables ZB, ZC, ZD and ZE.

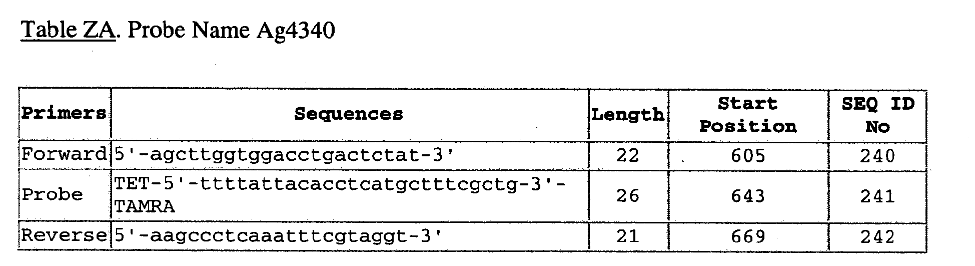

**Table ZB. CNS_neurodegeneration_v1.0**

| **Tissue Name** | **Rel. Exp.(%) Ag4340, Run 249266041** | **Rel. Exp.(%) Ag4340, Run 258587752** | **Tissue Name** | **Rel. Exp.(%) Ag4340, Run 249266041** | **Rel. Exp.(%) Ag4340, Run 258587752** |
|---|---|---|---|---|---|
| AD 1 Hippo | 4.4 | 3.7 | Control (Path) 3 Temporal Ctx | 1.6 | 1.0 |
| AD 2 Hippo | 14.0 | 10.4 | Control (path) 4 Temporal Ctx | 23.7 | 21.5 |
| AD 3 Hippo | 2.4 | 1.6 | AD 1 Occipital Ctx | 10.4 | 7.5 |
| AD 4 Hippo | 1.6 | 1.7 | AD 2 Occipital Ctx (Missing) | 0.0 | 0.0 |
| AD 5 hippo | **100.0** | **100.0** | AD 3 Occipital Ctx | 2.0 | 1.8 |
| AD 6 Hippo | 28.1 | 24.7 | AD 4 Occipital Ctx | 8.7 | 7.1 |
| Control 2 Hippo | 15.3 | 9.4 | AD 5 Occipital Ctx | 18.2 | 30.6 |
| Control 4 Hippo | 1.0 | 0.9 | AD 6 Occipital Ctx | 45.1 | 25.5 |
| Control (Path) 3 Hippo | 1.0 | 1.1 | Control 1 Occipital Ctx | 0.9 | 0.4 |
| AD 1 Temporal Ctx | 3.1 | 3.3 | Control 2 Occipital Ctx | 73.2 | 52.1 |
| AD 2 Temporal Ctx | 22.2 | 18.0 | Control 3 Occipital Ctx | 8.8 | 10.4 |
| AD 3 Temporal Ctx | 0.8 | 1.7 | Control 4 Occipital Ctx | 0.5 | 0.8 |
| AD 4 Temporal Ctx | 8.8 | 8.6 | Control (Path) 1 Occipital Ctx | 68.8 | 66.0 |
| AD 5 Inf Temporal Ctx | 70.7 | 73.2 | Control (Path) 2 Occipital Ctx | 8.5 | 9.6 |
| AD 5 SupTemporal Ctx | 17.7 | 19.1 | Control (Path) 3 Occipital Ctx | 0.8 | 0.4 |
| AD 6 Inf Temporal Ctx Temporal Ctx | 37.1 | 27.0 | Control (Path) 4 Occipital Ctx | 10.6 | 9.8 |
| AD 6 Sup Temporal Ctx | 43.5 | 40.6 | Control 1 Parietal Ctx | 2.0 | 1.2 |
| Control 1 Temporal Ctx | 1.4 | 0.9 | Control 2 Parietal Ctx | 27.4 | 20.3 |
| Control 2 Ctx Temporal Ctx | 35.8 | 29.9 | Control 3 Parietal Ctx | 13.5 | 12.2 |
| Control 3 Temporal Ctx | 8.2 | 9.7 | Control (Path) I Parietal Ctx | 71.2 | 70.7 |
| Control 4 Temporal Ctx | 2.1 | 2.5 | Control (Path) 2 Parietal Ctx | 17.4 | 15.1 |
| Control (Path) 1 Temporal Ctx | 53.2 | 48.6 | Control (Path) 3 Parietal Ctx | 1.1 | 0.8 |
| Control (Path) 2 Temporal Ctx | 28.9 | 24.5 | Control (Path) 4 Parietal Ctx | 33.9 | 35.6 |

**Table ZC. General_screening_panel_v1.4**

| **Tissue Name** | **Rel. Exp.(%) Ag4340, Run 222523509** | **Tissue Name** | **Rel. Exp.(%) Ag4340, Run 222523509** |
|---|---|---|---|
| Adipose | 3.7 | Renal ca. TK-10 | 7.6 |
| Melanoma* Hs688(A).T | 0.6 | Bladder | 1.5 |
| Melanoma* Hs688(B).T | 0.3 | Gastric ca. (liver met.) NCI-N87 | 0.0 |
| Melanoma* M14 | 0.0 | Gastric ca. KATO III | 0.0 |
| Melanoma* LOXIMVI | 24.0 | Colon ca. SW-948 | 0.0 |
| Melanoma* SK-MEL-5 | 0.3 | Colon ca. SW480 | 0.0 |
| Squamous cell carcinoma SCC-4 | 0.0 | Colon ca.* (SW480 met) SW620 | 0.0 |
| Testis Pool | 4.9 | Colon ca. HT29 | 0.0 |
| Prostate ca.* (bone met) PC-3 | 0.0 | Colon ca. HCT-116 | 0.0 |
| Prostate Pool | 9.0 | Colon ca. CaCo-2 | 0.2 |
| Placenta | 1.0 | Colon cancer tissue | 1.5 |
| Uterus Pool | 3.8 | Colon ca. SW1116 | 0.0 |
| Ovarian ca. OVCAR-3 | 0.2 | Colon ca. Colo-205 | 0.0 |
| Ovarian ca. SK-OV-3 | 0.5 | Colon ca. SW-48 | 0.0 |
| Ovarian ca. OVCAR-4 | 0.0 | Colon Pool | 2.0 |
| Ovarian ca. OVCAR-5 | 0.0 | Small Intestine Pool | 12.4 |
| Ovarian ca. IGROV-1 | 0.2 | Stomach Pool | 9.3 |
| Ovarian ca. OVCAR-8 | 1.5 | Bone Marrow Pool | 11.3 |
| Ovary | 1.5 | Fetal Heart | 1.6 |
| Breast ca. MCF-7 | 8.8 | Heart Pool | 2.4 |
| Breast ca. MDA-MB-231 | 0.0 | Lymph Node Pool | 37.6 |
| Breast ca. BT 549 | 2.4 | Fetal Skeletal Muscle | 4.8 |
| Breast ca. T47D | 0.0 | Skeletal Muscle Pool | 0.3 |
| Breast ca. MDA-N | 0.0 | Spleen Pool | 3.7 |
| Breast Pool | 9.9 | Thymus Pool | 14.2 |
| Trachea | 3.6 | CNS cancer (glio/astro) U87-MG | 3.4 |
| Lung | 1.8 | CNS cancer (glio/astro) U-118-MG | 76.8 |
| Fetal Lung | 11.9 | CNS cancer (neuro;met) SK-N-AS | 0.0 |
| Lung ca. NCI-N417 | 7.7 | CNS cancer (astro) SF-539 | 0.0 |
| Lung ca. LX-1 | 0.0 | CNS cancer (astro) SNB-75 | 0.0 |
| Lung ca. NCI-H146 | 4.2 | CNS cancer (glio) SNB-19 | 0.0 |
| Lung ca. SHP-77 | 1.8 | CNS cancer (glio) SF-295 | 8.8 |
| Lung ca. A549 | 0.0 | Brain (Amygdala) Pool | 22.1 |
| Lung ca. NCI-H526 | 0.0 | Brain (cerebellum) | 8.0 |
| Lung ca. NCI-H23 | 1.0 | Brain (fetal) | **100.0** |
| Lung ca. NCI-H460 | 3.3 | Brain (Hippocampus) Pool | 27.5 |
| Lung ca. HOP-62 | 0.8 | Cerebral Cortex Pool | 55.9 |
| Lung ca. NCI-H522 | 0.0 | Brain (Substantia nigra) Pool | 34.4 |
| Liver | 0.0 | Brain (Thalamus) Pool | 52.9 |
| Fetal Liver | 2.2 | Brain (whole) | 57.8 |
| Liver ca. HepG2 | 0.0 | Spinal Cord Pool | 5.6 |
| Kidney Pool | 7.5 | Adrenal Gland | 9.1 |
| Fetal Kidney | 12.2 | Pituitary gland Pool | 1.6 |
| Renal ca. 786-0 | 4.4 | Salivary Gland | 0.8 |
| Renal ca. A498 | 0.0 | Thyroid (female) | 1.3 |
| Renal ca. ACHN | 0.0 | Pancreatic ca. CAPAN2 | 0.0 |
| Renal ca. UO-31 | 0.0 | Pancreas Pool | 7.8 |

**Table ZD. Panel 4.1D**

| **Tissue Name** | **Rel. Exp.(%) Ag4340, Run 186362025** | **Tissue Name** | **Rel. Exp.(%) Ag4340, Run 186362025** |
|---|---|---|---|
| Secondary Th1 act | 0.0 | HUVEC IL-1beta | 0.6 |
| Secondary Th2 act | 0.0 | HUVEC IFN gamma | 0.0 |
| Secondary Tr1 act | 0.0 | HUVEC TNF alpha + IFN | 3.3 |
| | | gamma | |
| Secondary Th1 rest | 0.0 | HUVEC TNF alpha + IL4 | 0.0 |
| Secondary Th2 rest | 0.0 | HUVEC IL-11 | 0.5 |
| Secondary Tr1 rest | 0.0 | Lung Microvascular EC none | 0.0 |
| Primary Th1 act | 0.0 | Lung Microvascular EC TNFalpha + IL-1beta | 5.2 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL 1beta | 0.0 |
| Primary Th2 rest | 0.0 | Small airway epithelium none | 0.0 |
| Primary Tr1 rest | 0.0 | Small airway epithelium TNFalpha + IL-1beta | 0.5 |
| CD45RA CD4 lymphocyte act | 2.7 | Coronery artery SMC rest | 0.0 |
| CD45RO CD4 lymphocyte act | 0.0 | Coronery artery SMC TNFalpha + IL-1beta | 1.9 |
| CD8 lymphocyte act | 0.0 | Astrocytes rest | 0.0 |
| Secondary CD8 lymphocyte rest | 0.0 | Astrocytes TNFalpha + IL-1beta | |
| Secondary CD8 lymphocyte act | 0.0 | KU-812 (Basophil) rest | 0.7 |
| CD4 lymphocyte none | 1.0 | KU-812 (Basophil) PMA/ionomycin | 3.8 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 5.8 |
| LAK cells IL-2+IL-12 | 0.0 | NCI-H292 none | 0.0 |
| LAK cells IL-2+IFN gamma | 0.0 | NCI-H292 IL-4 | 0.0 |
| LAK cells IL-2+ IL-18 | 0.0 | NCI-H292 IL-9 | 0.0 |
| LAK cells PMA/ionomycin | 0.0 | NCI-H292 IL-13 | 0.0 |
| NK Cells IL-2 rest | 0.0 | NCI-H292 IFN gamma | 0.0 |
| Two Way MLR 3 day | 0.0 | HPAEC none | 1.2 |
| Two Way MLR 5 day | 0.0 | HPAEC TNF alpha + IL-1 beta | 21.9 |
| Two Way MLR 7 day | 0.0 | Lung fibroblast none | 10.2 |
| PBMC rest | 0.0 | Lung fibroblast TNF alpha + IL-1 beta | 11.5 |
| PBMC PWM | 0.0 | Lung fibroblast IL-4 | 46.7 |
| PBMC PHA-L | 0.0 | Lung fibroblast IL-9 | 48.3 |
| Ramos (B cell) none | 0.0 | Lung fibroblast IL-13 | 58.2 |
| Ramos (B cell) ionomycin | 0.0 | Lung fibroblast IFN gamma | 38.2 |
| B lymphocytes PWM | 0.0 | Dermal fibroblast CCD 1070 rest | 8.0 |
| B lymphocytes CD40L and IL-4 | 1.3 | Dermal fibroblast CCD 1070 TNF alpha | 19.5 |
| EOL-1 dbcAMP | 0.0 | Dermal fibroblast CCD1070 IL-1 beta | 14.2 |
| EOL-1 dbcAMP PMA/ionomycin | 0.0 | Dermal fibroblast IFN gamma | 3.7 |
| Dendritic cells none | 0.0 | Dermal fibroblast IL-4 | 5.9 |
| Dendritic cells LPS | 0.0 | Dermal Fibroblasts rest | 8.1 |
| Dendritic cells anti-CD40 | 0.0 | Neutrophils TNFa+LPS | 1.3 |
| Monocytes rest | 0.0 | Neutrophils rest | 0.0 |
| Monocytes LPS | 0.0 | Colon | 1.0 |
| Macrophages rest | 0.0 | Lung | 10.2 |
| Macrophages LPS | 0.0 | Thymus | 22.1 |
| HUVEC none | 0.0 | Kidney | **100.0** |
| HUVEC starved | 0.0 | | |

**Table ZE. general oncology screening panel_v_2.4**

| **Tissue Name** | **Rel. Exp.(%) Ag4340, Run 258689189** | **Rel. Exp.(%) Ag4340, Run 260280474** | **Tissue Name** | **Rel. Exp.(%) Ag4340, Run 258689189** | **Rel. Exp.(%) Ag4340, Run 260280474** |
|---|---|---|---|---|---|
| Colon cancer 1 | 0.7 | 1.7 | Bladder cancer NAT 2 | 0.0 | 0.9 |
| Colon NAT 1 | 0.9 | 1.3 | Bladder cancer NAT 3 | 0.0 | 0.2 |
| Colon cancer 2 | 0.8 | 3.9 | Bladder cancer NAT 4 | 37.1 | 14.1 |
| Colon cancer NAT 2 | 1.3 | 1.2 | Adenocarcinoma of the prostate 1 | 97.9 | **100.0** |
| Colon cancer 3 | 9.0 | 2.4 | Adenocarcinoma of the prostate 2 | 2.4 | 3.4 |
| Colon cancer NAT 3 | 16.0 | 5.7 | Adenocarcinoma of the prostate 3 | 14.2 | 5.5 |
| Colon malignant cancer 4 | 2.0 | 1.6 | Adenocarcmoma of the prostate 4 | 12.5 | 5.1 |
| Colon normal adjacent tissue | 1.7 | 1.2 | Prostate cancer NAT 5 | 2.9 | 0.8 |
| Lung cancer 1 | 1.1 | 0.7 | Adenocarcinoma of the prostate 6 | 5.6 | 0.9 |
| Lung NAT 1 | 0.0 | 0.7 | Adenocarcinoma of the prostate 7 | 13.5 | 5.8 |
| Lung cancer 2 | **100.0** | 51.1 | Adenocarcinoma of the prostate 8 | 4.4 | 0.9 |
| Lung NAT 2 | 1.7 | 0.8 | Adenocarcinoma of the prostate 9 | 65.5 | 57.4 |
| Squamous cell carcinoma 3 | 2.8 | 1.5 | Prostate cancer NAT 10 | 2.3 | 0.9 |
| Lung NAT 3 | 0.0 | 0.0 | Kidney cancer 1 | 1.5 | 1.2 |
| metastatic melanoma 1 | 24.1 | 8.8 | KidneyNAT 1 | 2.4 | 0.8 |
| Melanoma 2 | 2.4 | 0.9 | Kidney cancer 2 | 4.2 | 1.4 |
| Melanoma 3 | 3.4 | 0.9 | Kidney NAT 2 | 0.6 | 1.2 |
| metastatic melanoma 4 | 46.7 | 11.1 | Kidney cancer 3 | 0.0 | 0.3 |
| metastatic melanoma 5 | 17.6 | 10.6 | Kidney NAT 3 | 0.0 | 0.8 |
| Bladder cancer1 | 3.3 | 4.2 | Kidney cancer 4 | 0.7 | 0.3 |
| Bladder cancer NAT 1 | 0.0 | 0.0 | Kidney NAT 4 | 2.0 | 0.9 |
| Bladder cancer2 | 18.9 | 7.5 | | | |

**CNS_neurodegeneration_v1.0 Summary:** Ag4340 Two experiments with the same probe and primer set produce results that are in excellent agreement. Highest expression of this gene is seen in the hippocampus of a patient with Alzheimer's disease (CTs=30). The hippocampus is a critical brain region for the formation of long-term memory. This gene encodes a putative LRR/Ig/FNII containing protein. Fibronectin repeat regions are often involved in cell surface binding and in this protein may be involved in the formation and maintenance of specific neuronal networks in the brain. Therefore, this gene product is therefore an excellent drug target for the treatment of dementia (Alzheimer's, vascular, etc) or for memory enhancement.

**General_screening_panel_v1.4 Summary:** Ag4340 Highest expression of this gene is seen in the fetal brain (CT=28.7). In addition, this gene is expressed at moderate levels in thalamus, substantia nigra, cerebral cortex, hippocampus, and amygdala, with low, but significant expression in the cerebellum. This gene encodes a novel transmembrane protein that contains a putative leucine rich repeat region. Leucine rich repeats (LRR) mediate reversible protein-protein interactions and have diverse cellular functions, including cellular adhesion and signaling. Several of these proteins, such as connectin, slit, chaoptin, and Toll have pivotal roles in neuronal development in Drosophila and may play significant but distinct roles in neural development and in the adult nervous system of humans (Battye R. (2001) J. Neurosci. 21: 4290-4298. Itoh A. (1998) Brain Res. Mol. Brain Res. 62: 175-186). In Drosophilia, the LRR region of axon guidance proteins has been shown to be critical for their function (especially in axon repulsion). (Taniguchi H, Shishido E, Takeichi M, Nose A. (2000) J Neurobiol. 42:104-116.) Since the leucine-rich-repeat protein encoded by this gene shows high expression in the cerebral cortex, it is an excellent candidate neuronal guidance protein for axons, dendrites and/or growth cones in general. Therefore, therapeutic modulation of the levels of this protein, or possible signaling via this protein, may be of utility in enhancing/directing compensatory synaptogenesis and fiber growth in the CNS in response to neuronal death (stroke, head trauma), axon lesion (spinal cord injury), or neurodegeneration (Alzheimer's, Parkinson's, Huntington's, vascular dementia or any neurodegenerative disease).

Among tissues with metabolic function, this gene is expressed at low but significant levels in pituitary, adipose, adrenal gland, pancreas, fetal skeletal muscle, and adult and fetal heart, and liver. This widespread expression among these tissues suggests that this gene product may play a role in normal neuroendocrine and metabolic function and that disregulated expression of this gene may contribute to neuroendocrine disorders or metabolic diseases, such as obesity and diabetes.

Moderate levels of expression are also seen in colon cancer and some brain, breast, lung, renal, ovarian and melanoma cancer cell lines. Therefore, therapeutic modulation of this gene product may be useful in the treatment of these cancers.

**Panel 4.1D Summary:** Ag4340 Highest expression of this gene is seen in the kidney (CT=32). In addition, low but significant levels of expression are seen in activated lung and dermal fibroblasts, suggesting a role for this gene product in pathological and inflammatory conditions of the lung and skin.

**general oncology screening panel_v_2.4 Summary:** Ag4340 Two experiments with the same probe and primer set produce results that are in excellent agreement. Highest expression of the gene is seen in prostate and lung cancer (CTs=29.3-30). In addition, this gene is more highly expressed in lung and prostate cancer than in the corresponding normal adjacent tissue. Thus, expression of this gene could be used as a marker of these cancers. Furthemore, therapeutic modulation of the expression or function of this gene product may be useful in the treatment of lung and prostate cancer.

### AA. CG108184-01 and CG108184-02: novel transmembrane protein Tm7

Expression of gene CG108184-01 and CG108184-02 was assessed using the primer-probe set Ag4350, described in Table AAA. Results of the RTQ-PCR runs are shown in Tables AAB, AAC and AAD. Please note that CG108184-02 represents a full-length physical clone of the CG108184-01 gene, validating the prediction of the gene sequence.

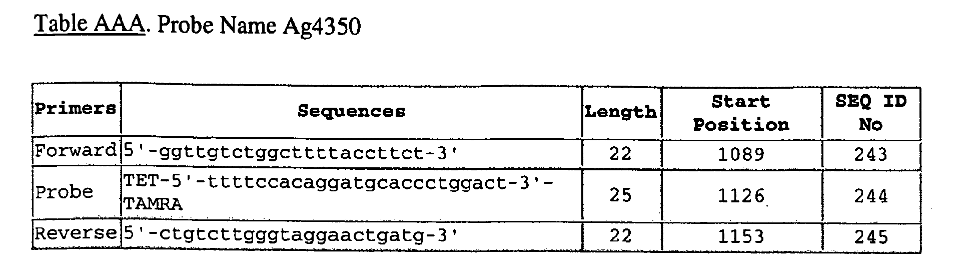

**Table AAB. CNS_neurodegeneration_v1.0**

| **Tissue Name** | **Rel. Exp.(%) Ag4350, Run 249266042** | **Tissue Name** | **Rel. Exp.(%) Ag4350, Run 249266042** |
|---|---|---|---|
| AD 1 Hippo | 11.0 | Control (Path) 3 Temporal Ctx | 5.2 |
| AD 2 Hippo | 22.4 | Control (Path) 4 Temporal Ctx | 24.3 |
| AD 3 Hippo | 6.3 | AD 1 Occipital Ctx | 6.3 |
| AD 4 Hippo | 5.7 | AD 2 Occipital Ctx (Missing) | 0.0 |
| AD 5 Hippo | 83.5 | AD 3 Occipital Ctx | 4.5 |
| AD 6 Hippo | 37.6 | AD 4 Occipital Ctx | 11.8 |
| Control 2 Hippo | 30.4 | AD 5 Occipital Ctx | 51.4 |
| Control 4 Hippo | 4.1 | AD 6 Occipital Ctx | 9.7 |
| Control (Path) 3 Hippo | 3.1 | Control Occipital Ctx | 2.0 |
| AD 1 Temporal Ctx | 5.9 | Control 2 Occipital Ctx | 71.2 |
| AD 2 Temporal Ctx | 25.3 | Control 3 Occipital Ctx | 11.1 |
| AD 3 Temporal Ctx | 4.4 | Control 4 Occipital Ctx | 3.1 |
| AD 4 Temporal Ctx | 12.3 | Control (Path) 1 Occipital Ctx | 80.7 |
| AD 5 Inf Temporal Ctx | 94.6 | Control (Path) 2 Occipital Ctx | 6.6 |
| AD 5 Sup Temporal Ctx | 63.3 | Control (Path) 3 Occipital Ctx | 1.3 |
| AD 6 Inf Temporal Ctx | 23.3 | Control (Path) 4 Occipital Ctx | 7.7 |
| AD 6 Sup Temporal | 26.4 | Control 1 Parietal Ctx | 2.7 |
| Control 1 Temporal Ctx | 2.4 | Control 2 Parietal Ctx | 29.1 |
| Control 2 Temporal Ctx | 45.1 | Control 3 Parietal Ctx | 16.3 |
| Control 3 Temporal Ctx | 10.2 | Control (Path) 1 Parietal Ctx | **100.0** |
| Control 3 Temporal Ctx | 6.0 | Control (Path) 2 Parietal Ctx | 13.7 |
| Control (Path) 1 Temporal Ctx | 51.8 | Control (Path) 3 Parietal Ctx | 2.3 |
| Control (Path) 2 Temporal Ctx | 25.7 | Control (Path) 4 Parietal Ctx | 32.5 |

**Table AAC. General_screening_panel_v1.4**

| **Tissue Name** | **Rel. Exp.(%) Ag4350, Run 222523514** | **Tissue Name** | **Rel. Exp.(%) Ag4350, Run 222523514** |
|---|---|---|---|
| Adipose | 0.9 | Renal ca. TK-10 | 2.5 |
| Melanoma* Hs688(A).T | 0.0 | Bladder | 12.3 |
| Melanoma* Hs688(B).T | 0.1 | Gastric ca. (liver met.) NCI-N87 | 8.5 |
| Melanoma* M14 | 0.2 | Gastric ca. KATO III | 7.4 |
| Melanoma* LOXIMVI | 0.0 | Colon ca. SW-948 | 0.9 |
| Melanoma* SK-MEL-5 | 1.3 | Colon ca. SW480 | 0.8 |
| Squamous cell carcinoma SCC-4 | 0.7 | Colon ca.* (SW480 met) SW620 | 0.8 |
| Testis Pool | 2.2 | Colon ca. HT29 | 1.5 |
| Prostate ca.* (bone met) PC-3 | 2.3 | Colon ca. HCT-116 | 4.0 |
| Prostate Pool | 1.2 | Colon ca. CaCo-2 | 1.4 |
| Placenta | 2.6 | Colon cancer tissue | 2.1 |
| Uterus Pool | 1.1 | Colon ca. SW1116 | 1.5 |
| Ovarian ca. OVCAR-3 | 1.5 | Colon ca. Colo-205 | 0.7 |
| Ovarian ca. SK-OV-3 | 1.5 | Colon ca. SW-48 | 2.4 |
| Ovarian ca. OVCAR-4 | 0.1 | Colon Pool | 1.0 |
| Ovarian ca. OVCAR-5 | 6.1 | Small Intestine Pool | 2.6 |
| Ovarian ca. IGROV-1 | 1.2 | Stomach Pool | 20.7 |
| Ovarian ca. OVCAR-8 | 0.5 | Bone Marrow Pool | 1.1 |
| Ovary | 4.6 | Fetal Heart | 0.3 |
| Breast ca. MCF-7 | 1.9 | Heart Pool | 0.4 |
| Breast ca. MDA-MB-231 | 0.0 | Lymph Node Pool | 1.9 |
| Breast ca. BT 549 | 0.9 | Fetal Skeletal Muscle | 0.3 |
| Breast ca. T47D | 9.0 | Skeletal Muscle Pool | 0.4 |
| Breast ca. MDA-N | 0.2 | Spleen Pool | 1.2 |
| Breast Pool | 1.2 | Thymus Pool | 1.1 |
| Trachea | 34.9 | CNS cancer (glio/astro) U87-MG | 0.7 |
| Lung | 1.5 | CNS cancer (glio/astro) U-118-MG | 0.0 |
| Fetal Lung | 16.3 | CNS cancer (neuro;met) SK-N-AS | 2.0 |
| Lung ca. NCI-N417 | 2.0 | CNS cancer (astro) SF-539 | 0.0 |
| Lung ca. LX-1 | 1.5 | CNS cancer (astro) SNB-75 | 0.2 |
| Lung ca. NCI-H146 | 0.3 | CNS cancer (glio) SNB-19 | 1.1 |
| Lung ca. SHP-77 | 1.4 | CNS cancer (glio) SF-295 | 0.4 |
| Lung ca. A549 | 2.9 | Brain (Amygdala) Pool | 74.7 |
| Lung ca. NCI-H526 | 1.1 | Brain (cerebellum) | 55.9 |
| Lung ca. NCI-H23 | 4.1 | Brain (fetal) | 10.5 |
| Lung ca. NCI-H460 | 0.4 | Brain (Hippocampus) Pool | 66.9 |
| Lung ca. HOP-62 | 0.1 | Cerebral Cortex Pool | 84.7 |
| Lung ca. NCI-H522 | 7.8 | Brain (Substantia nigra) Pool | 81.2 |
| Liver | 0.2 | Brain (Thalamus) Pool | **100.0** |
| Fetal Liver | 2.1 | Brain (whole) | 96.6 |
| Liver ca. HepG2 | 2.4 | Spinal Cord Pool | 7.9 |
| Kidney Pool | 3.7 | Adrenal Gland | 0.7 |
| Fetal Kidney | 10.4 | Pituitary gland Pool | 3.2 |
| Renal ca. 786-0 | 0.9 | Salivary Gland | 20.7 |
| Renal ca. A498 | 2.3 | Thyroid (female) | 1.9 |
| Renal ca. ACHN | 6.1 | Pancreatic ca. CAPAN2 | 2.7 |
| Renal ca. UO-31 | 5.5 | Pancreas Pool | 9.0 |

**Table AAD. Panel 4.1D**

| **Tissue Name** | **Rel. Exp.(%) Ag4350, Run 186362899** | **Tissue Name** | **Rel. Exp.(%) Ag4350, Run 186362899** |
|---|---|---|---|
| Secondary Th1 act | 0.2 | HUVEC IL-1beta | 0.2 |
| Secondary Th2 act | 0.4 | HUVEC IFN gamma | 0.0 |
| Secondary Tr1 act | 0.3 | HUVEC TNF alpha + IFN gamma | 0.1 |
| Secondary Th1 rest | 0.4 | HUVEC TNF alpha + IL4 | 0.0 |
| Secondary Th2 rest | 0.3 | HUVEC IL-11 | 0.2 |
| Secondary Tr1 rest | 0.3 | Lung Microvascular EC none | 0.2 |
| Primary Th1 act | 0.3 | Lung Microvascular EC TNFalpha + IL-1beta | 0.2 |
| Primary Th2 act | 0.7 | Microvascular Dermal EC none | 1.5 |
| Primary Tr1 act | 0.7 | Microsvasular Dermal EC TNFalpha + IL-1beta | 0.1 |
| Primary Th1 rest | 0.1 | Bronchial epithelium TNFalpha + IL1beta | 0.2 |
| Primary Th2 rest | 0.8 | Small airway epithelium none | 0.5 |
| Primary Tr1 rest | 0.2 | Small airway epithelium TNFalpha + IL-1 beta | 0.1 |
| CD45RACD4 lymphocyte act | 1.2 | Coronery artery SMC rest | 0.6 |
| CD45RO CD4 lymphocyte act | 0.3 | Coronery artery SMC TNFalpha + IL-1beta | 0.4 |
| CD8 lymphocyte act | 0.9 | Astrocytes rest | 0.0 |
| Secondary CD8 lymphocyte rest | 0.6 | Astrocytes TNFalpha + IL-1beta | 0.7 |
| Secondary CD8 lymphocyte act CD4 lymphocyte none | 0.5 | KU-812 (Basophil) rest rest | 0.5 |
| | 0.1 | KU-812 (Basophil) PMA/ionomycin | 0.3 |
| 2ry Th1/Th2/Tr1_anti-CD95 CH11 | 1.9 | CCD1106 (Keratinocytes) none | 0.0 |
| LAK cells rest | 0.3 | CCD1106 (Keratinocytes) TNFalpha + IL-1beta | 0.0 |
| LAK cells IL-2 | 0.8 | Liver cirrhosis | 0.3 |
| LAK cells IL-2+IL-12 | 0.6 | NCI-H292 none | 1.2 |
| LAK cells IL-2+IFN gamma | 0.6 | NCI-H292 IL-4 | 0.9 |
| LAK cells IL-2+ IL-18 | 0.1 | NCI-H292 IL-9 | 2.9 |
| LAK cells PMA/ionomycin | 0.2 | NCI-H292 IL-13 | 1.3 |
| NK Cells IL-2 rest | 1.2 | NCI-H292 IFN gamma | 1.7 |
| Two Way MLR 3 day | 0.1 | HPAEC none | 0.4 |
| Two Way MLR 5 day | 0.7 | HPAEC TNF alpha + IL-1 beta | 0.0 |
| Two Way MLR 7 day | 1.5 | Lung fibroblast none | 0.4 |
| PBMC rest | 0.0 | Lung fibroblast TNF alpha + IL-1 beta | 0.4 |
| PBMC PWM | 0.7 | Lung fibroblast IL-4 | 0.2 |
| PBMC PHA-L | 0.4 | Lung fibroblast IL-9 | 0.0 |
| Ramos (B cell) none | 0.0 | Lung fibroblast IL-13 | 0.1 |
| Ramos (B cell) ionomycin | 0.9 | Lung fibroblast IFN gamma | 0.2 |
| B lymphocytes PWM | 0.5 | Dermal fibroblast CCD1070 rest | 0.2 |
| B lymphocytes CD40L and IL-4 | 0.3 | Dermal fibroblast CCD1070 TNF alpha | 0.6 |
| EOL-1 dbcAMP | 0.2 | Dermal fibroblast CCD1070 IL-1 beta | 0.0 |
| EOL-1 dbcAMP PMA/ionomycin | 0.0 | Dermal fibroblast IFN gamma | 0.3 |
| Dendritic cells none | 0.2 | Dermal fibroblast IL-4 | 0.0 |
| Dendritic cells LPS | 0.0 | Dermal Fibroblasts rest | 0.3 |
| Dendritic cells anti-CD40 | 0.3 | Neutrophils TNFa+LPS | 0.0 |
| Monocytes rest | 0.2 | Neutrophils rest | 0.2 |
| Monocytes LPS | 0.0 | Colon | 2.3 |
| Macrophages rest | 0.4 | Lung | 5.5 |
| Macrophages LPS | 0.0 | Thymus | 4.9 |
| HUVEC none | 0.4 | Kidney | **100.0** |
| HUVEC starved | 0.1 | | |

**CNS_neurodegeneration_v1.0 Summary:** Ag4350 This panel confirms the expression of the CG108184-01 gene at low levels in the brains of an independent group of individuals. However, no differential expression of this gene was detected between Alzheimer's diseased postmortem brains and those of non-demented controls in this experiment. Please see Panel 1.4 for a discussion of the potential use of this gene in treatment of central nervous system disorders.

**General_screening_panel_v1.4 Summary:** Ag4350 Higest expression of the CG108184-01 gene is detected in brain (CTs=28.1). High expression of this gene is seen mainly in all the brain regions examined. Therefore, therapeutic modulation of this gene product may be useful in the treatment of central nervous system disorders such as Alzheimer's disease, Parkinson's disease, epilepsy, multiple sclerosis, schizophrenia and depression.

Moderate to low levels of expression of this gene is also seen in cluster of cancer cell lines derived from pancreas, gastric, colon, lung, renal, breast, ovarian, prostate, squamous cell carcinoma, melanoma and brain cancers. Thus, therapeutic modulation of the expression or function of this gene may be effective in the treatment of pancreas, gastric, colon, lung, renal, breast, ovarian, prostate, squamous cell carcinoma, melanoma and brain cancers.

Among tissues with metabolic or endocrine function, this gene is expressed at moderate to low levels in pancreas, adipose, thyroid, pituitary gland, fetal liver and the gastrointestinal tract. Therefore, therapeutic modulation of the activity of this gene may prove useful in the treatment of endocrine/metabolically related diseases, such as obesity and diabetes.

Interestingly, this gene is expressed at much higher levels in fetal (CTs=30.7-33.7) when compared to adult lung and liver (CTs=34-37). This observation suggests that expression of this gene can be used to distinguish fetal lung and liver from corresponding adult tissues. In addition, the relative overexpression of this gene in fetal tissue suggests that the protein product may enhance growth or development of lung and liver in the fetus and thus may also act in a regenerative capacity in the adult. Therefore, therapeutic modulation of the membrane protein encoded by this gene could be useful in treatment of lung and liver related diseases.

**Panel 4.1D Summary:** Ag4350 Higest expression of the CG108184-01 gene is detected in kidney (CT=27.8). Therefore, expression of this gene may be used to distinguish kidney from other samples used in this panel. Furthermore, therapeutic modulation of this gene product may be useful in the treatment of autoimmune and inflammatory disease that affect kidney, including lupus and glomerulonephritis.

In addition, moderate to low expression of this gene is also seen in CD45RA CD4 lymphocyte act, anti-CD95 CH11 treated secondary Th1/Th2/Tr1 cells, IL-2 treated LAK and NK cells, two way MLR, PWM treated PBMC, microvascular dermal EC, NCI-H292 and normal tissues represented by colon, lung and thymus. Therefore, therapeutic modulation of this gene product may be beneficial in the treatment of Crohn's disease, ulcerative colitis, multiple sclerosis, chronic obstructive pulmonary disease, asthma, emphysema, rheumatoid arthritis, lupus erythematosus, or psoriasis.

### AB. CG108238-01: Sialic acid binding immunoglobulin-like lectin

Expression of gene CG108238-01 was assessed using the primer-probe set Ag4352, described in Table ABA. Results of the RTQ-PCR runs are shown in Tables ABB, ABC and ABD.

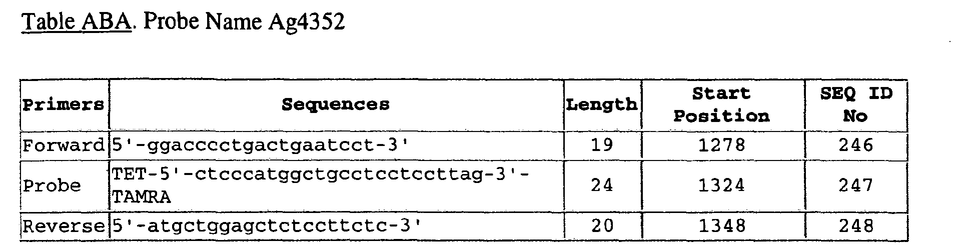

**Table ABB. CNS_neurodegeneration_v1.0**

| **Tissue Name** | **Rel. Exp.(%) Ag4352, Run 224367442** | **Tissue Name** | **Rel. Exp.(%) Ag4352, Run 224367442** |
|---|---|---|---|
| AD 1 Hippo | 6.3 | Control (Path) 3 Temporal Ctx | 1.7 |
| AD 2 Hippo | 12.2 | Control (Path) 4 Temporal Ctx | 31.0 |
| AD 3 Hippo | 2.9 | AD 1 Occipital Ctx | 10.2 |
| AD 4 Hippo | 1.3 | AD 2 Occipital Ctx (Missing) | 0.0 |
| AD 5 hippo | **100.0** | AD 3 Occipital Ctx | 2.4 |
| AD 6 Hippo | 33.4 | AD 4 Occipital Ctx | 12.2 |
| Control 2 Hippo | 15.3 | AD 5 Occipital Ctx | 31.2 |
| Control 4 Hippo | 2.2 | AD 6 Occipital Ctx | 46.7 |
| Control (Path) 3 Hippo | 2.0 | Control 1 Occipital | 0.8 |
| AD 1 Temporal Ctx | 3.5 | Control 2 Occipital Ctx | 95.9 |
| AD 2 Temporal Ctx | 25.0 | Control 3 Occipital | 8.4 |
| AD 3 Temporal Ctx | 1.8 | Control 4 Occipital Ctx | 0.5 |
| AD 4 Temporal Ctx | 10.4 | Control (Path) 1 Occipital Ctx | 82.9 |
| AD 5 Inf Temporal Ctx | 88.3 | Control (Path) 2 Occipital Ctx | 10.5 |
| AD 5 SupTemporal Ctx | 22.2 | Control (Path) 3 Control (Path) 3 Occipital Ctx | 0.4 |
| AD 6 Inf Temporal Ctx | 42.6 | Control (Path) 4 Occipital Ctx | 11.9 |
| AD 6 Sup Temporal Ctx | 57.4 | Control 1 Parietal Ctx | 3.2 |
| Control I Temporal Ctx | 0.9 | Control 2 Parietal Ctx | 31.0 |
| Control 2 Temporal Ctx | 41.8 | Control 3 Parietal Ctx | 15.1 |
| Control 3 Temporal Ctx | 9.0 | Control (Path) 1 Parietal Ctx | 95.3 |
| Control 4 Temporal Ctx | 4.0 | Control (Path) 2 Parietal Ctx | 18.2 |
| Control (Path) 1 Temporal Ctx | 59.0 | Control (Path) 3 Parietal Ctx | 1.8 |
| Control (Path) 2 Temporal Ctx | 29.9 | Control (Path) 4 Parietal Ctx | 36.3 |

**Table ABC. General_screening_panel_v1.4**

| **Tissue Name** | **Rel. Exp.(%) Ag4352, Run 222541798** | **Tissue Name** | **Rel. Exp.(%) Ag4352, Run 222541798** |
|---|---|---|---|
| Adipose | 6.8 | Renal ca. TK-10 | 0.0 |
| Melanoma* Hs688(A).T | 0.0 | Bladder | 7.7 |
| Melanoma* Hs688(B).T | 0.0 | Gastric ca. (liver met.) NCI-N87 | 0.0 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | **100.0** | Colon ca. HT29 | 0.0 |
| Prostate ca.* (bone met) PC-3 | 0.0 | Colon ca. HCT-116 | 0.0 |
| Prostate Pool | 9.7 | Colon ca. CaCo-2 | 0.0 |
| Placenta | 0.0 | Colon cancer tissue | 0.0 |
| Uterus Pool | 7.3 | Colon ca. SW 1116 | 0.0 |
| Ovarian ca. OVCAR-3 | 0.0 | Colon ca. Colo-205 | 8.8 |
| Ovarian ca. SK-OV-3 | 0.0 | Colon ca. SW-48 | 0.0 |
| Ovarian ca. OVCAR-4 | 0.0 | Colon Pool | 4.4 |
| Ovarian ca. OVCAR-5 | 8.3 | Small Intestine Pool | 0.0 |
| Ovarian ca. IGROV-1 | 0.0 | Stomach Pool | 0.0 |
| Ovarian ca. OVCAR-8 | 0.0 | Bone Marrow Pool | 3.4 |
| Ovary | 0.0 | Fetal Heart | 10.2 |
| Breast ca. MCF-7 | 0.0 | Heart Pool | 7.7 |
| Breast ca. MDA-MB-231 | 0.0 | Lymph Node Pool | 14.6 |
| Breast ca. BT 549 | 0.0 | Fetal Skeletal Muscle | 11.7 |
| Breast ca. T47D | 0.0 | Skeletal Muscle Pool | 0.0 |
| Breast ca. MDA-N | 0.0 | Spleen Pool | 23.3 |
| Breast Pool | 0.0 | Thymus Pool | 10.4 |
| Trachea | 5.3 | CNS cancer (glio/astro) U87-MG | 0.0 |
| Lung | 2.2 | CNS cancer (glio/astro) U-118-MG | 0.0 |
| Fetal Lung | 4.5 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.0 | CNS cancer (glio) SNB-19 | 0.0 |
| Lung ca. SHP-77 | 0.0 | CNS cancer (glio) SF-295 | 0.0 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 15.8 | Brain (fetal) | 0.0 |
| Lung ca. NCI-H460 | 0.0 | Brain (Hippocampus) Pool | 0.0 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 0.0 | Adrenal Gland | 0.0 |
| Fetal Kidney | 11.2 | Pituitary gland Pool | 0.0 |
| Renal ca. 786-0 | 33.4 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 10.6 |

**Table ABD. Panel 4.1D**

| **Tissue Name** | **Rel. Exp.(%) Ag4352, Run 186363976** | **Tissue Name** | **Rel. Exp.(%) Ag4352, Run 186363976** |
|---|---|---|---|
| Secondary Th 1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th 1 act | 0.0 | Lung Microvascular EC TNFatpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha + IL-1 beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 2.7 |
| CD4 lymphocyte none | 0.0 | KU-812 (Basophil) PMA/ionomycin | 0.0 |
| 2ry Th1/Th2/Tr1_anti-CD95 CH1I | 0.0 | CCD 1106 (Keratinocytes) none | 0.0 |
| LAK cells rest | 0.0 | CCD1106 (Keratinocytes) TNFalpha+IL-1beta | 0.0 |
| LAK cells IL-2 | 0.0 | Liver cirrhosis | 0.0 |
| LAK cells IL-2+IL-12 | 0.0 | NCI-H292 none | 0.0 |
| LAK cells IL-2+IFN gamma | 1.5 | NCI-H292 IL-4 | 0.0 |
| LAK cells IL-2+ IL-18 | 0.0 | NCI-H292 IL-9 | 0.0 |
| LAK cells PMA/ionomycin | 0.0 | NCI-H292 IL-13 | 0.0 |
| NK Cells IL-2 rest | 0.0 | NCI-H292 IFN gamma | 0.0 |
| Two Way MLR 3 day | 0.0 | HPAEC none | 0.0 |
| Two Way MLR 5 day | 0.0 | HPAEC TNF alpha + IL-1 beta | 0.0 |
| Two Way MLR 7 day | 0.0 | Lung fibroblast none | 0.0 |
| PBMC rest | 4.0 | Lung fibroblast TNF alpha + IL-1 beta | 0.0 |
| PBMC PWM | 0.0 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 7.6 |
| Ramos (B cell) ionomycin | 0.0 | Lung fibroblast IFN gamma | 1.9 |
| B lymphocytes PWM | 0.0 | Dermal fibroblast CCD 1070 rest | 0.0 |
| B lymphocytes CD40L and IL-4 | 0.0 | Dermal fibroblast CCD 1070 TNF alpha | 0.0 |
| EOL-1 dbcAMP | 1.6 | Dermal fibroblast CCD1070 IL-1 beta | 0.0 |
| EOL-1 dbcAMP PMA/ionomycin | 1.7 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 3.5 |
| Dendritic cells anti-CD40 | 0.0 | Neutrophils TNFa+LPS | 0.0 |
| Monocytes rest | 18.9 | Neutrophils rest | 0.0 |
| Monocytes LPS | 0.0 | Colon | 3.6 |
| Macrophages rest | 0.0 | Lung | 0.0 |
| Macrophages LPS | 0.0 | Thymus | 21.2 |
| HUVEC none | 0.0 | Kidney | **100.0** |
| HUVEC starved | 0.0 | | |

**CNS_neurodegeneration_v1.0 Summary:** Ag4352 This panel confirms the expression of the CG108238-01 gene at low levels in the brains of an independent group of individuals. However, no differential expression of this gene was detected between Alzheimer's diseased postmortem brains and those of non-demented controls in this experiment. Expression of this gene in brain suggests that this gene may play a role in central nervous system disorders such as Parkinson's disease, epilepsy, multiple sclerosis, schizophrenia and depression.

**General_screening_panel_v1.4 Summary:** Ag4352 Low levels of expression of the CG108238-01 gene is seen only in testis (CT=34.5). Therefore, expression of this gene may be used to distinguish testis from other samples used in the panel. In addition, therapeutic modulation of this gene product may a beneficial in the treatment of testis related diseases including fertility and hypogonadism.

**Panel 4.1D Summary:** Ag4352 Low levels of expression of the CG108238-01 gene is seen only in kidney (CT=33.6). Therefore, expression of this gene may be used to distinguish kidney from other samples used in the panel. In addition, therapeutic modulation of this gene product may a beneficial in the treatment of autoimmune and inflammatory diseases that affect kidney including lupus and glomerulonephritis.

### AC. CG109505-01: Aldehyde dehydrogenase

Expression of gene CG109505-01 was assessed using the primer-probe set Ag4387, described in Table ACA. Results of the RTQ-PCR runs are shown in Tables ACB and ACC.

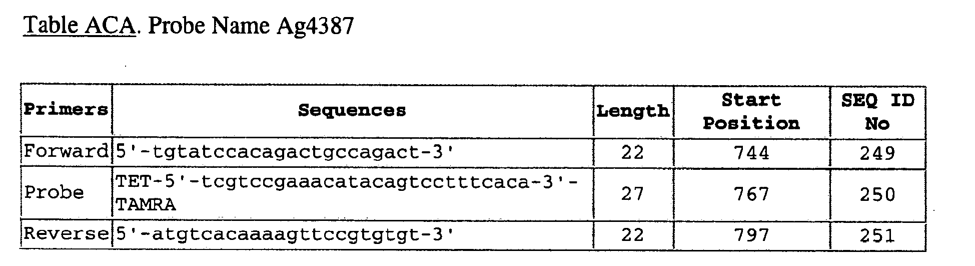

**Table ACB. General_screening_panel_v1.4**

| **Tissue Name** | **Rel. Exp.(%) Ag4387, Run 222567011** | **Tissue Name** | **Rel. Exp.(%) Ag4387, Run 222567011** |
|---|---|---|---|
| Adipose | 0.5 | Renal ca. TK-10 | 0.0 |
| Melanoma* Hs688(A).T | 0.0 | Bladder | 7.8 |
| Melanoma* Hs688(B).T | 0.0 | Gastric ca. (liver met.) NCI-N87 | 46 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 4.4 | Colon ca.* (SW480 met) SW620 | 0.0 |
| Testis Pool | 1.8 | Colon ca. HT29 | 0.0 |
| Prostate ca.* (bone met) PC-3 | 0.0 | Colon ca. HCT-116 | 1.2 |
| Prostate Pool | 0.0 | Colon ca. CaCo-2 | 0.0 |
| Placenta | 0.0 | Colon cancer tissue | 0.0 |
| Uterus Pool | 15.9 | Colon ca. SW1116 | 0.0 |
| Ovarian ca. OVCAR-3 | 2.8 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.0 |
| Ovarian ca. OVCAR-5 | 0.4 | Small Intestine Pool | 0.0 |
| Ovarian ca. IGROV-1 | 0.0 | Stomach Pool | 0.0 |
| Ovarian ca. OVCAR-8 | 0.0 | Bone Marrow Pool | **100.0** |
| Ovary | 0.0 | Fetal Heart | 0.0 |
| Breast ca. MCF-7 | 1.1 | Heart Pool | 0.0 |
| Breast ca. MDA-MB-231 | 0.0 | Lymph Node Pool | 0.0 |
| Breast ca. BT 549 | 0.0 | Fetal Skeletal Muscle | 0.0 |
| Breast ca. T47D | 0.7 | Skeletal Muscle Pool | 0.0 |
| Breast ca. MDA-N | 0.0 | Spleen Pool | 0.0 |
| Breast Pool | 0.0 | Thymus Pool | 1.3 |
| Trachea | 10.1 | CNS cancer (glio/astro) U87-MG | 0.0 |
| Lun | 0.0 | CNS cancer (glio/astro) U-118-MG | 0.6 |
| Fetal Lung | 0.4 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.0 | CNS cancer (glio) SNB-19 | 0.0 |
| Lung ca. SHP-77 | 0.0 | CNS cancer (glio) SF-295 | 0.0 |
| Lung ca. A549 | 1.7 | Brain (Amygdala) Pool | 0.5 |
| Lung ca. NCI-H526 | 0.0 | Brain (cerebellum) | 0.0 |
| Lung ca. NCI-H23 | 1.5 | Brain (fetal) | 0.0 |
| Lung ca. NCI-H460 | 0.0 | Brain (Hippocampus) Pool | 0.0 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 0.7 | Adrenal Gland | 0.0 |
| Fetal Kidney | 0.0 | Pituitary gland Pool | 0.0 |
| Renal ca. 786-0 | 0.5 | Salivary Gland | 2.4 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 0.0 |

**Table ACC. Panel 4.1D**

| **Tissue Name** | **Rel. Exp.(%) Ag4387, Run 186501500** | **Tissue Name** | **Rel. Exp.(%) Ag4387, Run 186501500** |
|---|---|---|---|
| Secondary Th act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th 1 rest | 0.0 | HUVEC TNF alpha + IL4 | 0.0 |
| Secondary Th2 rest | 0.0 | HUVEC IL-11 | 0.9 |
| Secondary Tr1 rest | 0.0 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1 beta | 0.0 |
| Primary Th1 rest | 0.3 | Bronchial epithelium TNFalpha + IL1 beta | 4.6 |
| Primary Th2 rest | 0.0 | Small airway epithelium none | 20.0 |
| Primary Tr1 rest | 0.0 | Small airway epithelium TNFalpha + IL-1beta | 22.4 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 6.7 |
| CD4 lymphocyte none | 0.0 | KU-812 (Basophil) PMA/ionomycin | 16.3 |
| 2ry Th1/Th2/Tr1_anti-CD95 CH11 | 0.0 | CCD1106 (Keratinocytes) none | 0.4 |
| LAK cells rest | 0.6 | CCD1106 (Keratinocytes) TNFalpha + IL-1beta | 2.5 |
| LAK cells IL-2 | 1.0 | Liver cirrhosis | 0.0 |
| LAK cells IL-2+IL-12 | 0.9 | NCI-H292 none | 0.4 |
| LAK cells IL-2+IFN gamma | 0.0 | NCI-H292 IL-4 | 0.9 |
| LAK cells IL-2+IL-18 | 0.9 | NCI-H292 IL-9 | 0.0 |
| PMA/ionomycin | | | |
| NK Cells IL-2 rest | 0.0 | NCI-H292 IFN gamma | 0.0 |
| Two Way MLR 3 day | 0.0 | HPAEC none | 0.0 |
| Two Way MLR 5 day | 0.0 | HPAEC TNF alpha + IL-1 beta | 0.0 |
| Two Way MLR 7 day | 0.0 | Lung fibroblast none | 1.0 |
| PBMC rest | 0.0 | Lung fibroblast TNF alpha + IL-1 beta | 0.4 |
| PBMC PWM | 0.9 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 0.8 |
| Ramos (B cell) ionomycin | 0.0 | Lung fibroblast IFN gamma | 0.0 |
| B lymphocytes PWM | 0.5 | Dermal fibroblast CCD1070 rest | 0.0 |
| B lymphocytes CD40L and IL-4 | 0.0 | Dermal fibroblast CCD 1070 TNF alpha | 0.0 |
| EOL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 0.0 |
| Dendritic cells anti-CD40 | 0.0 | Neutrophils TNFa+LPS | 0.0 |
| Monocytes rest | 0.0 | Neutrophils rest | 1.8 |
| Monocytes LPS | 0.0 | Colon | 2.0 |
| Macrophages rest | 0.0 | Lung | 2.1 |
| Macrophages LPS | 0.0 | Thymus | 18.4 |
| HUVEC none | 0.0 | Kidney | **100.0** |
| HUVEC starved | 0.0 | | |

**General_screening_panel_v1.4 Summary:** Ag4387 Highest expression of the CG109505-01 gene is detected in bone marrow (CT=30.6). Therefore, expression of this gene may be used to distinguish this sample from other samples used in this panel. In addition, therapeutic modulation of this gene product may be useful in the bone marrow related diseases such as leukemia.

**Panel 4.1D Summary:** Ag4387 Highest expression of the CG109505-01 gene is detected in kidney (CT=30.9). Therefore, expression of this gene may be used to distinguish kidney from other samples used in this panel. In addition, therapeutic modulation of this gene may be beneficial in the treatment of autoimmune of inflammatory disease that affect kidney including lupus and glomerulonephritis.

Moderate to low levels of expression of this gene is also seen in thymus, basophils, and small airway epithelium. Therefore, therapeutic modulation of this gene product may be beneficial in the treatment of asthma, allergies, COPD, and emphysema, inflammatory bowel disease, and autoimmune diseases.

### AD. CG109742-01: LATENT TRANSFORMING GROWTH FACTOR BETA BINDING PROTEIN 3 like

Expression of gene CG109742-01 was assessed using the primer-probe sets Ag1112 and Ag25, described in Tables ADA. Results of the RTQ-PCR runs are shown in Table ADB.

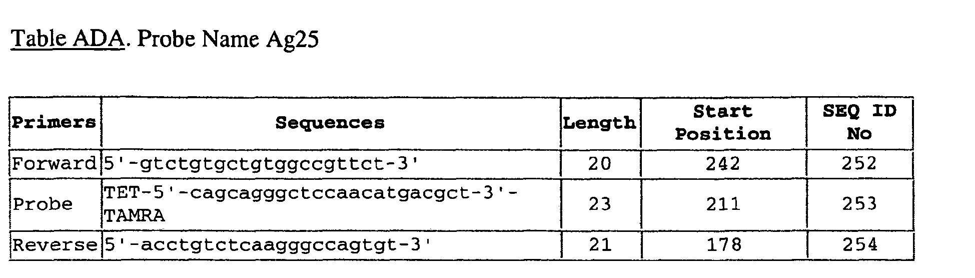

**Table ADB. Panel 1**

| **Tissue Name** | **Rel. Exp.(%) Ag25, Run 91010022** | **Tissue Name** | **Rel. Exp.(%) Ag25, Run 91010022** |
|---|---|---|---|
| Endothelial cells | 7.4 | Renal ca. 786-0 | 3.8 |
| Endothelial cells (treated) | 1.8 | Renal ca. A498 | 6.9 |
| Pancreas | 17.7 | Renal ca. RXF 393 | 30.1 |
| Pancreatic ca. CAPAN 2 | 9.6 | Renal ca. ACHN | 9.9 |
| Adrenal gland | 11.6 | Renal ca. UO-31 | 5.7 |
| Thyroid | 14.8 | Renal ca. TK-10 | 19.8 |
| Salivary gland | 6.7 | Liver | 34.4 |
| Pituitary gland | 7.7 | Liver (fetal) | 2.4 |
| Brain (fetal) | 5.3 | Liver ca. (hepatoblast) HepG2 | 8.8 |
| Brain (whole) | 19.2 | Lung | 15.0 |
| Brain (amygdala) | 7.2 | Lung (fetal) | 19.3 |
| Brain (cerebellum) | 52.9 | Lung ca. (small cell) LX- | 22.1 |
| Brain (hippocampus) | 7.6 | Lung ca. (small cell) NCI-H69 | 0.8 |
| Brain (substantia nigra) | 6.9 | Lung ca. (s.cell var.) SHP-77 | 1.8 |
| Brain (thalamus) | 6.8 | Lung ca. (large cell)NCI-H460 | 13.3 |
| Brain (hypothalamus) | 10.8 | Lung ca. (non-sm. cell) A549 | 15.0 |
| Spinal cord | 11.8 | Lung ca. (non-s.cell) NCI-H23 | 4.1 |
| glio/astro U87-MG | 7.1 | Lung ca. (non-s.cell) HOP-62 | 0.0 |
| glio/astro U-118-MG | 5.1 | Lung ca. (non-s.cl) NCI-H522 | 1.6 |
| astrocytoma SW1783 | 2.7 | Lung ca. (squam.) SW | **100.0** |
| neuro*; met SK-N-AS | 2.6 | Lung ca. (squam.) NCI-H596 | 0.4 |
| astrocytoma SF-539 | 1.9 | Mammary gland | 50.3 |
| astrocytoma SNB-75 | 5.1 | Breast ca.* (pl.ef) MCF-7 | 7.4 |
| glioma SNB-19 | 10.4 | Breast ca.* (pl.ef) MDA-MB-231 | 0.9 |
| glioma U251 | 18.3 | Breast ca.* (pl. ef) T47D | 12.0 |
| glioma SF-295 | 27.5 | Breast ca. BT-549 | 16.4 |
| Heart | 20.4 | Breast ca. MDA-N | 3.7 |
| Skeletal muscle | 5.1 | Ovary | 59.0 |
| Bone marrow | 3.2 | Ovarian ca. OVCAR-3 | 3.5 |
| Thymus | 17.7 | Ovarian ca. OVCAR-4 | 6.3 |
| Spleen | 6.9 | Ovarian ca. OVCAR-5 | 49.0 |
| Lymph node | 16.8 | Ovarian ca. OVCAR-8 | 7.6 |
| Colon (ascending) | 3.6 | Ovarian ca. IGROV-1 | 1.1 |
| Stomach | 20.2 | Ovarian ca. (ascites) SK-OV-3 | 1.2 |
| Small intestine | 8.5 | Uterus | 9.9 |
| Colon ca. SW480 | 0.5 | Placenta | 21.0 |
| Colon ca.* SW620 (SW480 met) | 1.9 | Prostate | 15.4 |
| Colon ca. HT29 | 2.5 | Prostate ca.* (bone met) PC-3 | 7.8 |
| Colon ca. HCT-116 | 0.0 | Testis | 25.7 |
| Colon ca. CaCo-2 | 4.3 | Melanoma Hs688(A).T | 4.4 |
| Colon ca. HCT-15 | 3.8 | Melanoma* (met) Hs688(B).T | 14.6 |
| Colon ca. HCC-2998 | 0.8 | Melanoma UACC-62 | 3.3 |
| Gastric ca. * (liver met) NCI-N87 | 39.8 | Melanoma M14 | 5.5 |
| Bladder | 18.6 | Melanoma LOX IMVI | 90.8 |
| Trachea | 21.3 | Melanoma* (met) SK-MEL-5 | 0.0 |
| Kidney | 16.4 | Melanoma SK-MEL-28 | 3.2 |
| Kidney (fetal) | 23.3 | | |

**Panel 1 Summary:** Ag25 Highest expression of the CG109742-01 gene is detected in a lung cancer SW 900 cell line (CT=21.5). High expression of this gene is seen in cluster of cancer cell lines including melanoma, ovarian, breast, lung, renal, colon, gastric, pancreatic and CNS cancer cell lines. Therefore, therapeutic modulation of this gene product may be beneficial in the treatment of these cancers.

Among tissues with metabolic or endocrine function, this gene is expressed at high levels in pancreas, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes.

In addition, this gene is expressed at high levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

### AE. CG109844-01: C4B-BINDING PROTEIN

Expression of gene CG109844-01 was assessed using the primer-probe sets Ag4406 and Ag4446, described in Tables AEA and AEB. Results of the RTQ-PCR runs are shown in Table AEC.

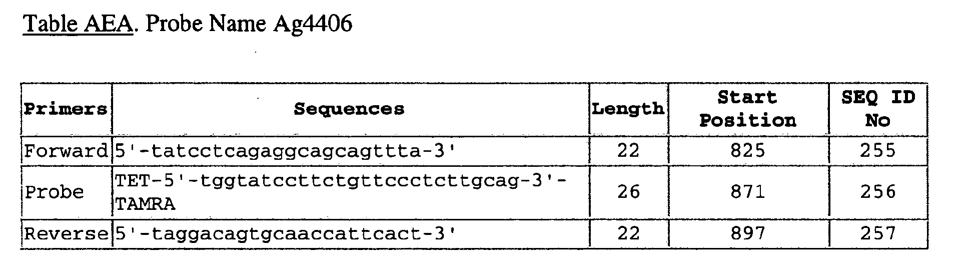

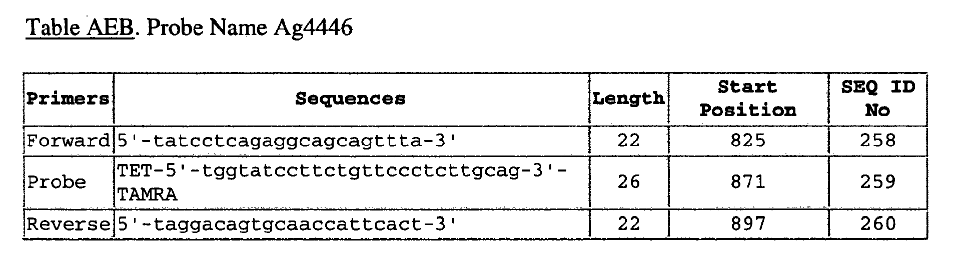

**Table AEC. General_screening_panel_v1.4**

| **Tissue Name** | **Rel. Exp.(%) Ag4446, Run 222693980** | **Tissue Name** | **Rel. Exp.(%) Ag4446, Run 222693980** |
|---|---|---|---|
| Adipose | 0.0 | Renal ca. TK-10 | 0.0 |
| Melanoma* Hs688(A).T | 0.0 | Bladder | 0.0 |
| Melanoma* Hs688(B).T | 0.0 | Gastric ca. (liver met.) NCI-N87 | 0.0 |
| Melanoma* M14 | 0.0 | Gastric ca. KATO III | 9.3 |
| Melanoma* LOXIMVI | 0.0 | Colon ca. SW-948 | 14.4 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 34.6 | Colon ca. HT29 | 0.0 |
| Prostate ca.* (bone met) PC-3 | 18.3 | Colon ca. HCT-116 | **100.0** |
| Prostate Pool | 0.0 | Colon ca. CaCo-2 | 0.0 |
| Placenta | 0.0 | Colon cancer tissue | 0.0 |
| Uterus Pool | 0.0 | Colon ca. SW1116 | 0.0 |
| Ovarian ca. OVCAR-3 | 13.4 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.0 |
| Ovarian ca. OVCAR-5 | 0.0 | Small Intestine Pool | 0.0 |
| Ovarian ca. IGROV-1 | 0.0 | Stomach Pool | 0.0 |
| Ovarian ca. OVCAR-8 | 0.0 | Bone Marrow Pool | 0.0 |
| Ovary | 0.0 | Fetal Heart | 0.0 |
| Breast ca. MCF-7 | 0.0 | Heart Pool | 0.0 |
| Breast ca. MDA-MB-231 | 0.0 | Lymph Node Pool | 0.0 |
| Breast ca. BT 549 | 0.0 | Fetal Skeletal Muscle | 0.0 |
| Breast ca. T47D | 11.2 | Skeletal Muscle Pool | 0.0 |
| Breast ca. MDA-N | 0.0 | Spleen Pool | 0.0 |
| Breast Pool | 0.0 | Thymus Pool | 0.0 |
| Trachea | 0.0 | CNS cancer (glio/astro) U87-MG | 10.2 |
| Lung | 0.0 | CNS cancer (glio/astro) U-118-MG | 56.3 |
| Fetal Lung | 0.0 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.0 | CNS cancer (glio) SNB-19 | 0.0 |
| Lung ca. SHP-77 | 0.0 | CNS cancer (glio) SF-295 | 0.0 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 0.0 | Brain (fetal) | 0.0 |
| Lung ca. NCI-H460 | 0.0 | Brain (Hippocampus) Pool | 0.0 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 0.0 | Adrenal Gland | 0.0 |
| Fetal Kidney | 0.0 | Pituitary gland Pool | 0.0 |
| Renal ca. 786-0 | 22.2 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 0.0 |

**General_screening_panel_v1.4 Summary:** Ag4406 Low levels of expression of the CG109844-01 gene is seen only in a colon cancer HCT-116 cell line (CT=34.9). Therefore, expression of this gene may be used to distinguish this sample from other samples used in this panel and also as diagnostic marker for colon cancer. In addition, therapeutic modulation of this gene product may be beneficial for the treatment of colon cancer.

### AF. CG110014-03: Protein Tyrosine Kinase-7

Expression of gene CG110014-03 was assessed using the primer-probe set Ag6098, described in Table AFA. Results of the RTQ-PCR runs are shown in Table AFB.

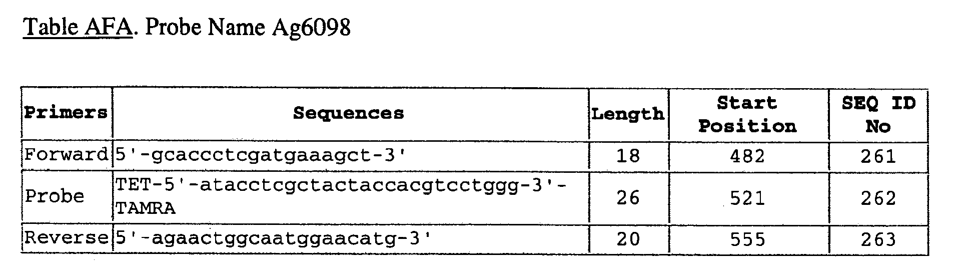

**Table AFB. General_screening_panel_v1.5**

| **Tissue Name** | **Rel. Exp.(%) Ag6098, Run 248491181** | **Tissue Name** | **Rel. Exp.(%) Ag6098, Run 248491181** |
|---|---|---|---|
| Adipose | 0.0 | Renal ca. TK-10 | 15.8 |
| Melanoma* Hs688(A).T | 46.7 | Bladder | 6.2 |
| Melanoma* Hs688(B).T | 19.6 | Gastric ca. (liver met.) NCI-N87 | 22.7 |
| Melanoma* M14 | 7.4 | Gastric ca. KATO III | 0.0 |
| Melanoma* LOXIMVI | 2.7 | Colon ca. SW-948 | 0.0 |
| Melanoma* SK-MEL-5 | 5.8 | Colon ca. SW480 | 20.7 |
| Squamous cell carcinoma SCC-4 | 20.4 | Colon ca.* (SW480 met) SW620 | 39.5 |
| Testis Pool | 5.6 | Colon ca. HT29 | 3.8 |
| Prostate ca.* (bone met) PC-3 | 10.7 | Colon ca. HCT-116 | **100.0** |
| Prostate Pool | 0.0 | Colon ca. CaCo-2 | 26.8 |
| Placenta | 21.5 | Colon cancer tissue | 10.0 |
| Uterus Pool | 5.7 | Colon ca. SW1116 | 16.8 |
| Ovarian ca. OVCAR-3 | 25.9 | Colon ca. Colo-205 | 4.8 |
| Ovarian ca. SK-OV-3 | 25.7 | Colon ca. SW-48 | 0.0 |
| Ovarian ca. OVCAR-4 | 9.9 | Colon Pool | 6.0 |
| Ovarian ca. OVCAR-5 | 8.6 | Small Intestine Pool | 9.3 |
| Ovarian ca. IGROV-1 | 11.0 | Stomach Pool | 4.3 |
| Ovarian ca. OVCAR-8 | 6.8 | Bone Marrow Pool | 2.2 |
| Ovary | 19.1 | Fetal Heart | 1.3 |
| Breast ca. MCF-7 | 3.4 | Heart Pool | 9.1 |
| Breast ca. MDA-MB-231 | 10.0 | Lymph Node Pool | 10.8 |
| Breast ca. BT 549 | 46.7 | Fetal Skeletal Muscle | 12.2 |
| Breast ca. T47D | 2.7 | Skeletal Muscle Pool | 0.0 |
| Breast ca. MDA-N | 5.8 | Spleen Pool | 5.2 |
| Breast Pool | 13.6 | Thymus Pool | 29.3 |
| Trachea | 1.6 | CNS cancer (glio/astro) U87-MG | 0.8 |
| Lung | 0.0 | CNS cancer (glio/astro) U-118-MG | 0.0 |
| Fetal Lung | 35.8 | CNS cancer (neuro;met) SK-N-AS | 20.0 |
| Lung ca. NCI-N417 | 4.5 | CNS cancer (astro) SF-539 | 80.7 |
| Lung ca. LX-1 | 40.1 | CNS cancer (astro) SNB-75 | 39.5 |
| Lung ca. NCI-H146 | 5.3 | CNS cancer (glio) SNB-19 | 3.7 |
| Lung ca. SHP-77 | 24.3 | CNS cancer (glio) SF-295 | 49.7 |
| Lung ca. A549 | 0.0 | Brain (Amygdala) Pool | 0.0 |
| Lung ca. NCI-H526 | 6.3 | Brain (cerebellum) | 32.5 |
| Lung ca. NCI-H23 | 27.5 | Brain (fetal) | 7.3 |
| Lung ca. NCI-H460 | 0.0 | Brain (Hippocampus) Pool | 0.0 |
| Lung ca. HOP-62 | 8.0 | Cerebral Cortex Pool | 0.0 |
| Lung ca. NCI-H522 | 7.8 | Brain (Substantia nigra) Pool | 1.1 |
| Liver | 0.0 | Brain (Thalamus) Pool | 2.8 |
| Fetal Liver | 1.1 | Brain (whole) | 5.3 |
| Liver ca. HepG2 | 34.6 | Spinal Cord Pool | 57.0 |
| Kidney Pool | 13.2 | Adrenal Gland | 0.7 |
| Fetal Kidney | 39.8 | Pituitary gland Pool | 2.7 |
| Renal ca. 786-0 | 0.0 | Salivary Gland | 1.4 |
| Renal ca. A498 | 0.0 | Thyroid (female) | 0.7 |
| Renal ca. ACHN | 4.6 | Pancreatic ca. CAPAN2 | 0.0 |
| Renal ca. UO-31 | 7.0 | Pancreas Pool | 6.0 |

**General_screening_panel_v1.5 Summary: Ag6098 Highest expression of the** CG110014-03 gene is detected in colon cancer HCT-116 cell line (CT=32.9). In addition, low to moderate expression of this gene is also seen in number of cancer cell lines including CNS, colon, liver, lung, breast, ovarain and melanoma cancer cell lines. This gene codes for a splice variant of tyrosine protein kinase -like 7 precursor (colon carcinoma kinase 4, CCK-4; PTK7), belonging to protein-tyrosine kinases (PTKs) family. PTKs play important role in regulating cell proliferation and differentiation during development. Mossie et al. (1995, Oncogene 11(10):2179-84, PMID: 7478540) showed a varied expression of CCK-4 in colon cancer cell lines and suggested a tumor-characteristic role for CCK-4 as a signal amplifier or modulator. Therefore, therapeutic modulation of this gene product may be beneficial in the treatment of melanoma, CNS, colon, liver, lung, breast, and ovarian cancers.

Moderate expression of this gene is also seen in spinal cord sample. Therefore, therapeutic modulation of this gene product may be useful in the treatment of spinal cord related diseases.

Low expression of this gene is also detected in fetal lung. Interestingly, expression of this gene is higher in fetal (CT=34.3) as compared to adult lung (CT=40). Therefore, the expression of this gene may be used to distinguish the fetal from adult lung. In addition, the relative overexpression of this gene in fetal lung suggests that the protein product may enhance lung growth or development in the fetus and thus may also act in a regenerative capacity in the adult. Therefore, therapeutic modulation of the CCK-4 protein encoded by this gene could be useful in treatment of lung related diseases.

### AG. CG110014-04: Protein Tyrosine Kinase-7

Expression of gene CG110014-04 was assessed using the primer-probe set Ag6687, described in Table AGA. Results of the RTQ-PCR runs are shown in Table AGB.

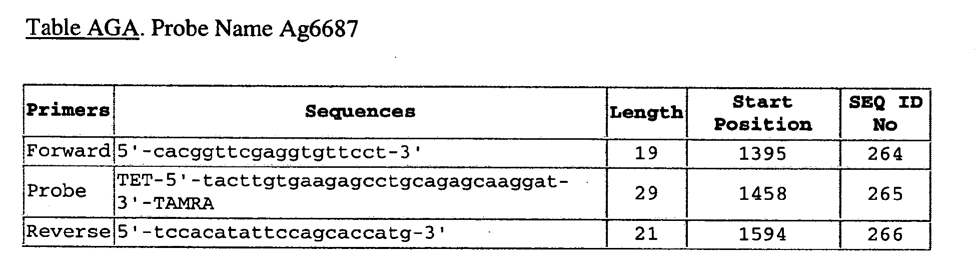

**Table AGB. General_screening_panel_v1.6**

| **Tissue Name** | **Rel. Exp.(%) Ag6687, Run 277259294** | **Tissue Name** | **Rel. Exp.(%) Ag6687, Run 277259294** |
|---|---|---|---|
| Adipose | 0.0 | Renal ca. TK-10 | 30.6 |
| Melanoma* Hs688(A).T | 73.7 | Bladder | 4.7 |
| Melanoma* Hs688(B).T | 63.7 | Gastric ca. (liver met.) NCI-N87 | 28.5 |
| Melanoma* M14 | 20.7 | Gastric ca. KATO III | 0.0 |
| Melanoma* LOXIMVI | 3.3 | Colon ca. SW-948 | 0.0 |
| Melanoma* SK-MEL-5 | 2.4 | Colon ca. SW480 | 34.9 |
| Squamous cell | 8.7 | Colon ca.* (SW480 met) | 36.3 |
| carcinoma SCC-4 | | SW620 | |
| Testis Pool | 1.4 | Colon ca. HT29 | 9.3 |
| Prostate ca.* (bone met) PC-3 | 12.2 | Colon ca. HCT-116 | 33.2 |
| Prostate Pool | 2.7 | Colon ca. CaCo-2 | 27.5 |
| Placenta | 6.2 | Colon cancer tissue | 14.0 |
| Uterus Pool | 0.7 | Colon ca. SW1116 | 37.6 |
| Ovarian ca. OVCAR-3 | 27.9 | Colon ca. Colo-205 | 0.8 |
| Ovarian ca. SK-OV-3 | 55.1 | Colon ca. SW-48 | 0.0 |
| Ovarian ca. OVCAR-4 | 21.2 | Colon Pool | 10.2 |
| Ovarian ca. OVCAR-5 | 2.7 | Small Intestine Pool | 7.1 |
| Ovarian ca. IGROV-1 | 13.7 | Stomach Pool | 8.4 |
| Ovarian ca. OVCAR-8 | 19.5 | Bone Marrow Pool | 1.1 |
| Ovary | 15.5 | Fetal Heart | 0.6 |
| Breast ca. MCF-7 | 5.9 | Heart Pool | 5.0 |
| Breast ca. MDA-MB-231 | 26.1 | Lymph Node Pool | 12.8 |
| Breast ca. BT 549 | 71.7 | Fetal Skeletal Muscle | 0.6 |
| Breast ca. T47D | 8.0 | Skeletal Muscle Pool | 0.0 |
| Breast ca. MDA-N | 0.0 | Spleen Pool | 4.0 |
| Breast Pool | 12.6 | Thymus Pool | 18.8 |
| Trachea | 7.3 | CNS cancer (glio/astro) U87-MG | 4.5 |
| Lung | 2.3 | CNS cancer (glio/astro) U-118-MG | 3.5 |
| Fetal Lung | 22.8 | CNS cancer (neuro;met) SK-N-AS | 33.4 |
| Lung ca. NCI-N417 | 3.0 | CNS cancer (astro) SF-539 | 64.6 |
| Lung ca. LX-1 | 59.9 | CNS cancer (astro) SNB-75 | **100.0** |
| Lung ca. NCI-H146 | 9.9 | CNS cancer (glio) SNB-19 | 22.4 |
| Lung ca. SHP-77 | 8.9 | CNS cancer (glio) SF-295 | 82.9 |
| Lung ca. A549 | 2.1 | Brain (Amygdala) Pool | 0.0 |
| Lung ca. NCI-H526 | 6.8 | Brain (cerebellum) | 1.7 |
| Lung ca. NCI-H23 | 16.5 | Brain (fetal) | 3.3 |
| Lung ca. NCI-H460 | 0.0 | Brain (Hippocampus) Pool | 0.0 |
| Lung ca. HOP-62 | 17.6 | Cerebral Cortex Pool | 0.0 |
| Lung ca. NCI-H522 | 15.4 | Brain (Substantia nigra) Pool | 0.0 |
| Liver | 0.0 | Brain (Thalamus) Pool | 0.0 |
| Fetal Liver | 1.1 | Brain (whole) | 2.2 |
| Liver ca. HepG2 | 12.2 | Spinal Cord Pool | 0.0 |
| Kidney Pool | 22.7 | Adrenal Gland | 0.0 |
| Fetal Kidney | 13.0 | Pituitary gland Pool | 0.0 |
| Renal ca. 786-0 | 0.0 | Salivary Gland | 3.8 |
| Renal ca. A498 | 0.0 | Thyroid (female) | 5.6 |
| Renal ca. ACHN | 14.1 | Pancreatic ca. CAPAN2 | 4.8 |
| Renal ca. UO-31 | 26.2 | Pancreas Pool | 0.0 |

**General_screening_panel_v1.6 Summary:** Ag6687 Highest expression of the CG110014-04 gene is detected in CNS cancer cell line SNB-75 (CT=32.6). In addition, moderate to low levels of expression of this gene is also seen in number of cancer cell lines derived from gastric, colon, lung, renal, breast, ovarian, melanoma and brain cancers. Thus, expression of this gene could be used to differentiate between these samples and other samples on this panel and as a marker to detect the presence of these cancers. Furthermore, therapeutic modulation of the expression or function of this gene may be effective in the treatment of gastric, colon, lung, renal, breast, ovarian, melanoma and brain cancers.

Low levels of expression of this gene is also seen in kidney and fetal lung. Interestingly, this gene is expressed at much higher levels in fetal (CT=34.7) when compared to adult lung (CT=38). This observation suggests that expression of this gene can be used to distinguish fetal from adult lung. In addition, the relative overexpression of this gene in fetal lung suggests that the protein product may enhance lung growth or development in the fetus and thus may also act in a regenerative capacity in the adult. Therefore, therapeutic modulation of the protein encoded by this gene could be useful in treatment of lung and kidney related diseases.

### AH. CG110187-01: Novel alpha C1-like protocadherin

Expression of gene CG110187-01 was assessed using the primer-probe set Ag4412, described in Table AHA. Results of the RTQ-PCR runs are shown in Tables AHB, AHC, AHD, AHE and AHF.

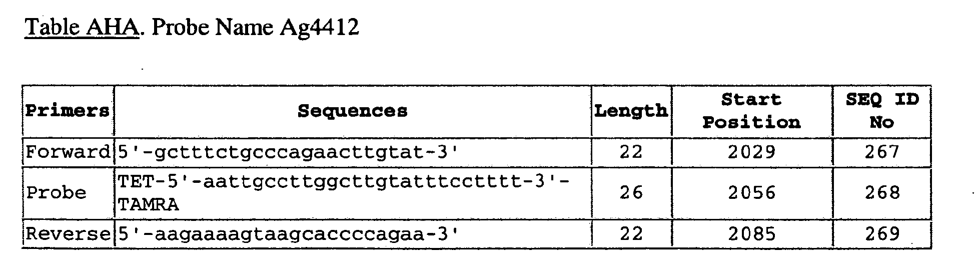

**Table AHB. CNS_neurodegeneration_v1.0**

| **Tissue Name** | **Rel. Exp.(%) Ag4412, Run 224505948** | **Tissue Name** | **Rel. Exp.(%) Ag4412, Run 224505948** |
|---|---|---|---|
| AD 1 Hippo | 8.1 | Control (Path) 3 Temporal Ctx | 2.0 |
| AD 2 Hippo | 11.9 | Control (Path) 4 | 21.3 |
| | | Temporal Ctx | |
| AD 3 Hippo | 4.9 | AD 1 Occipital Ctx | 10.2 |
| AD 4 Hippo | 1.2 | AD 2 Occipital Ctx (Missing) | 0.0 |
| AD 5 Hippo | 76.8 | AD 3 Occipital Ctx | 4.2 |
| AD 6 Hippo | 39.8 | AD 4 Occipital Ctx | 11.3 |
| Control 2 Hippo | 11.2 | AD 5 Occipital Ctx | 29.9 |
| Control 4 Hippo | 4.6 | AD 6 Occipital Ctx | 12.6 |
| Control (Path) 3 Hippo | 1.9 | Control I Occipital Ctx | 1.6 |
| AD 1 Temporal Ctx | 4.4 | Control 2 Occipital Ctx | 65.1 |
| AD 2 Temporal Ctx | 23.2 | Control 3 Occipital Ctx | 7.5 |
| AD 3 Temporal Ctx | 2.7 | Control 4 Occipital Ctx | 0.0 |
| AD 4 Temporal Ctx | 10.7 | Control (Path) 1 Occipital Ctx | 73.7 |
| AD 5 Inf Temporal Ctx | 66.0 | Control (Path) 2 Occipital Ctx | 15.5 |
| AD 5 Sup Temporal Ctx | 14.3 | Control (Path) 3 Occipital Ctx | 1.6 |
| AD 6 Inf Temporal Ctx | 33.7 | Control (Path) 4 Occipital Ctx | 9.2 |
| AD 6 Sup Temporal Ctx | 36.3 | Control 1 Parietal Ctx | 3.8 |
| Control 1 Temporal Ctx | 4.6 | Control 2 Parietal Ctx | 26.2 |
| Control 2 Temporal Ctx | 36.3 | Control 3 Parietal Ctx | 12.7 |
| Control 3 Temporal Ctx | 12.0 | Control (Path) 1 Parietal Ctx | **100.0** |
| Control 3 Temporal Ctx | 2.1 | Control (Path) 2 Parietal Ctx | 22.8 |
| Control (Path) 1 Temporal Ctx | 48.0 | Control (Path) 3 Parietal Ctx | 2.4 |
| Control (Path) 2 Temporal Ctx | 41.8 | Control (Path) 4 Parietal Ctx | 7.5 |

**Table AHC. General_screening_panel_v1.4**

| **Tissue Name** | **Rel. Exp.(%) Ag4412, Run 219923005** | **Tissue Name** | **Rel. Exp.(%) Ag4412, Run 219923005** |
|---|---|---|---|
| Adipose | 1.5 | Renal ca. TK-10 | 2.3 |
| Melanoma* Hs688(A).T | 0.3 | Bladder | 5.0 |
| Melanoma* Hs688(B).T | 0.0 | Gastric ca. (liver met.) NCI-N87 | 0.0 |
| Melanoma* M14 | 0.0 | Gastric ca. KATO III | 0.0 |
| Melanoma* LOXIMVI | 0.7 | Colon ca. SW-948 | 0.0 |
| Melanoma* SK-MEL-5 | 2.0 | Colon ca. SW480 | 0.4 |
| Squamous cell carcinoma SCC-4 | 0.0 | Colon ca.* (SW480 met) SW620 | 0.0 |
| Testis Pool | 6.7 | Colon ca. HT29 | 0.0 |
| Prostate ca.* (bone met) | 95.9 | Colon ca. HCT-116 | 3.9 |
| PC-3 | | | |
| Prostate Pool | 0.2 | Colon ca. CaCo-2 | 0.1 |
| Placenta | 0.0 | Colon cancer tissue | 0.9 |
| Uterus Pool | 0.5 | Colon ca. SW1116 | 0.5 |
| Ovarian ca. OVCAR-3 | 9.5 | Colon ca. Colo-205 | 0.0 |
| Ovarian ca. SK-OV-3 | 27.2 | Colon ca. SW-48 | 0.0 |
| Ovarian ca. OVCAR-4 | 0.3 | Colon Pool | 4.2 |
| Ovarian ca. OVCAR-5 | 1.5 | Small Intestine Pool | 6.4 |
| Ovarian ca. IGROV-1 | 2.0 | Stomach Pool | 3.3 |
| Ovarian ca. OVCAR-8 | 5.4 | Bone Marrow Pool | 0.7 |
| Ovary | 2.4 | Fetal Heart | 0.0 |
| Breast ca. MCF-7 | 12.6 | Heart Pool | 1.6 |
| Breast ca. MDA-MB-231 | 0.2 | Lymph Node Pool | 5.4 |
| Breast ca. BT 549 | 4.5 | Fetal Skeletal Muscle | 0.0 |
| Breast ca. T47D | 7.9 | Skeletal Muscle Pool | 0.3 |
| Breast ca. MDA-N | 2.1 | Spleen Pool | 1.0 |
| Breast Pool | 6.0 | Thymus Pool | 6.1 |
| Trachea | 3.3 | CNS cancer (glio/astro) U87-MG | 0.2 |
| Lun | 0.8 | CNS cancer (glio/astro) U-118-MG | 0.2 |
| Fetal Lung | 2.8 | CNS cancer (neuro;met) SK-N-AS | 31.6 |
| Lung ca. NCI-N417 | 1.1 | CNS cancer (astro) SF-539 | 1.7 |
| Lung ca. LX-1 | 0.3 | CNS cancer (astro) SNB-75 | 16.6 |
| Lung ca. NCI-H146 | 9.2 | CNS cancer (glio) SNB-19 | 1.6 |
| Lung ca. SHP-77 | 27.7 | CNS cancer (glio) SF-295 | 1.5 |
| Lung ca. A549 | 6.3 | Brain (Amygdala) Pool | 6.7 |
| Lung ca. NCI-H526 | 0.0 | Brain (cerebellum) | 25.3 |
| Lung ca. NCI-H23 | 20.2 | Brain (fetal) | **100.0** |
| Lung ca. NCI-H460 | 9.5 | Brain (Hippocampus) Pool | 4.5 |
| Lung ca. HOP-62 | 0.9 | Cerebral Cortex Pool | 8.6 |
| Lung ca. NCI-H522 | 37.6 | Brain (Substantia nigra) Pool | 6.0 |
| Liver | 0.0 | Brain (Thalamus) Pool | 8.7 |
| Fetal Liver | 0.2 | Brain (whole) | 17.0 |
| Liver ca. HepG2 | 0.4 | Spinal Cord Pool | 6.8 |
| Kidney Pool | 6.8 | Adrenal Gland | 1.1 |
| Fetal Kidney | 0.7 | Pituitary gland Pool | 3.6 |
| Renal ca. 786-0 | 0.0 | Salivary Gland | 0.5 |
| Renal ca. A498 | 2.4 | Thyroid (female) | 2.4 |
| Renal ca. ACHN | 0.0 | Pancreatic ca. CAPAN2 | 1.8 |
| Renal ca. UO-31 | 1.3 | Pancreas Pool | 6.3 |

**Table AHD. Oncology_cell_line_screening_panel_v3.2**

| **Tissue Name** | **Rel. Exp.(%) Ag4412, Run 268695315** | **Tissue Name** | **Rel. Exp.(%) Ag4412, Run 268695315** |
|---|---|---|---|
| 94905_Daoy_Medulloblastoma/Cerebellu m_sscDNA | 0.0 | 94954_Ca Ski_Cervical epidermoid carcinoma (metastasis)_sscDNA | 0.0 |
| 94906_TE671_Medulloblastom/Cerebell um_sscDNA | 1.3 | 94955_ES-2_Ovarian clear cell carcinoma_sscDNA | 0.0 |
| 94907_D283 Med_Medulloblastoma/Cerebellum_sscD NA | 12.4 | 94957_Ramos/6h stim_Stimulated with PMA/ionomycin 6h_sscDNA | 0.0 |
| 94908_PFSK-1_Primitive Neuroectodermal/Cerebellum_sscDNA | 0.9 | 94958_Ramos/14h stim_Stimulated with PMA/ionomycin 14h_sscDNA | 0.0 |
| 94909_XF-498_CNS_sscDNA | 2.9 | 94962_MEG-01_Chronic myelogenous leukemia (megokaryoblast)_sscDNA | 0.0 |
| 94910_SNB-78_CNS/glioma_sscDNA | 0.9 | 94963_Raji_Burkitt's lymphoma_sscDNA | 0.0 |
| 94911_SF-268_CNS/glioblastoma_sscDNA | 0.0 | 94964_Daudi_Burkitt's lymphoma_sscDNA | 0.0 |
| 94912_T98G_Glioblastoma_sscDNA | 0.0 | 94965_U266_B-cell plasmacytoma/myeloma_sscDNA | 0.0 |
| 96776_SK-N-SH_Neuroblastoma (metastasis)_sscDNA | 4.2 | 94968_CA46_Burkitt's lymphoma_sscDNA | 0.0 |
| 94913_SF-295_CNS/glioblastoma_sscDNA | 0.0 | 94970_RL_non-Hodgkin's B-cell lymphoma_sscDNA | 0.0 |
| 132565_NT2 pool_sscDNA | 11.2 | 94972_JM1_pre-B-cell lymphoma/leukemia_sscDNA | 0.0 |
| 94914 Cerebellum sscDNA | 24.5 | 94973_Jurkat_T cell leukemia-sscDNA | 0.0 |
| 96777_Cerebellum_sscDNA | 13.5 | 94974_TF-1_Erythroleukemia_sscDNA | 0.0 |
| 94916_NCI-H292_Mucoepidermoid lung carcinoma_sscDNA | 0.0 | 94975_HUT 78_T-cell lymphoma_sscDNA | 0.0 |
| 94917_DMS-114_Small cell lung cancer_sscDNA | 3.7 | 94977_U937_Histiocytic lymphoma_sscDNA | 0.0 |
| 94918_DMS-79_Small cell lung cancer/neuroendocrine_sscDNA | **100.0** | 94980_KU-812_Myelogenous leukemia_sscDNA | 0.0 |
| 94919_NCI-H146_Small cell lung cancer/neuroendocrine_sscDNA | 15.3 | 94981_769-P_Clear cell renal carcinoma_sscDNA | 0.0 |
| 94920_NCI-H526_Small cell lung cancer/neuroendocrine_sscDNA | 1.6 | 94983_Caki-2_Clear cell renal carcinoma_sscDNA | 1.9 |
| 94921_NCI-N417_Small cell lung cancer/neuroendocrine_sscDNA | 1.4 | 94984_SW 839_Clear cell renal carcinoma_sscDNA | 1.5 |
| 94923_NCI-H82_Small cell lung cancer/neuroendocrine_sscDNA | 1.4 | 94986_G401_Wilms' tumor_sscDNA | 4.0 |
| 94924_NCI-H157_Squamous cell lung cancer (metastasis)_sscDNA | 0.6 | 126768_293 cells_sscDNA | 0.0 |
| 94925_NCI-H1155_Large cell lung cancer/neuroendocrine_sscDNA | 19.5 | 94987_Hs766T_Pancreatic carcinoma (LN metastasis)_sscDNA | 0.0 |
| 94926_NCI-H1299_Large cell lung cancer/neuroendocrine_sscDNA | 5.0 | 94988_CAPAN-1_Pancreatic adenocarcinoma (liver metastasis)_sscDNA | 0.0 |
| 94927_NCI-H727_Lung | 39.0 | 94989_SU86.86_Pancreatic | 4.5 |
| carcinoid_sscDNA | | carcinoma (liver metastasis)_sscDNA | |
| 94928_NCI-UMC-11_Lung carcinoid_sscDNA | 18.3 | 94990_BxPC-3_Pancreatic adenocarcinoma_sscDNA | 0.0 |
| 94929_LX-1_Small cell lung cancer_sscDNA | 0.0 | 94991_HPAC_Pancreatic adenocarcinoma_sscDNA | 0.0 |
| 94930_Colo-205_Colon cancer_sscDNA | 0.0 | 94992_MIA PaCa-2_Pancreatic carcinoma_sscDNA | 0.0 |
| 94931_KM12_Colon cancer_sscDNA | 13.0 | 94993_CFPAC-1_Pancreatic ductal adenocarcinoma_sscDNA | 11.4 |
| 94932_KM20L2_Colon cancer_sscDNA | 0.0 | 94994_P ANC-1_Pancreatic epithelioid ductal carcinoma_sscDNA | 0.0 |
| 94933_NCI-H716_Colon cancer_sscDNA | 13.4 | 94996_T24_Bladder carcinma (transitional cell)_sscDNA | 0.0 |
| 94935_SW-48_Colon adenocarcinoma_sscDNA | 0.0 | 94997_5637_Bladder carcinoma_sscDNA | 0.0 |
| 94936_SW1116_Colon adenocarcinoma_sscDNA | 0.0 | 94998_HT-1197_Bladder carcinoma_sscDNA | 0.0 |
| 94937_LS 174T_Colon adenocarcinoma_sscDNA | 0.0 | 94999_UM-UC-3_Bladder carcinma (transitional cell)_sscDNA | 0.0 |
| 94938_SW-948_Colon adenocarcinoma_sscDNA | 0.0 | 95000_A204_Rhabdomyosarcoma_s scDNA | 2.4 |
| 94939_SW-480_Colon adenocarcinoma_sscDNA | 0.0 | 95001_HT-1080_Fibrosarcoma_sscDNA | 0.0 |
| 94940_NCI-SNU-5_Gastric carcinoma_sscDNA | 0.0 | 95002_MG-63_Osteosarcoma (bone)_sscDNA | 0.0 |
| 112197_KATO III_Stomach_sscDNA | 0.0 | 95003_SK-LMS-1_Leiomyosarcoma (vulva)_sscDNA | 4.6 |
| 94943_NCI-SNU-16_Gastric carcinoma_sscDNA | 0.7 | 95004_SJRH30_Rhabdomyosarcoma (met to bone marrow)_sscDNA | 0.0 |
| 94944_NCI-SNU-1_Gastric carcinoma_sscDNA | 0.0 | 95005_A431_Epidermoid carcinoma_sscDNA | 0.0 |
| 94946_RF-1_Gastric adenocarcinoma_sscDNA | 0.0 | 95007_WM266-4_Melanoma_sscDNA | 1.1 |
| 94947_RF-48_Gastric adenocarcinoma_sscDNA | 0.0 | 112195_DU 145_Prostate_sscDNA | 4.4 |
| 96778_MKN-45_Gastric carcinoma_sscDNA | 4.4 | 95012_MDA-MB-468_Breast adenocarcinoma_sscDNA | 0.5 |
| 94949_NCI-N87_Gastric carcinoma_sscDNA | 0.7 | 112196_SSC-4_Tongue_sscDNA | 0.0 |
| 94951_OVCAR-5_Ovarian carcinoma_sscDNA | 0.0 | 112194_SSC-9_Tongue_sscDNA | 0.0 |
| 94952_RL95-2_Uterine carcinoma_sscDNA | 1.4 | 112191_SSC-15_Tongue_sscDNA | 0.0 |
| 94953_HelaS3_Cervical adenocarcinoma_sscDNA | 0.0 | 95017_CAL 27_Squamous cell carcinoma of tongue_sscDNA | 0.0 |

**Table AHE. Panel 4.1D**

| **Tissue Name** | **Rel. Exp.(%) Ag4412, Run 190413471** | **Tissue Name** | **Rel. Exp.(%) Ag4412, Run 190413471** |
|---|---|---|---|
| Secondary Th1 act | 0.0 | HUVEC IL-1beta | 0.0 |
| Secondary Th2 act | 0.0 | HUVEC IFN gamma | 3.9 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 4.0 |
| Primary Th1 act | 0.0 | Lung Microvascular EC TNFalpha + IL-1 beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL 1beta | 11.4 |
| Primary Th2 rest | 0.0 | Small airway epithelium none | 3.2 |
| Primary Tr1 rest | 0.0 | Small airway epithelium TNFalpha + IL-1beta | 4.0 |
| CD45RA CD4 lymphocyte act | 0.0 | Coronery artery SMC rest | 0.0 |
| CD45RO CD4 lymphocyte act | 36.1 | Coronery artery SMC TNFalpha + IL-1beta | 0.0 |
| CD8 lymphocyte act | 0.0 | Astrocytes rest | 3.8 |
| Secondary CD8 lymphocyte rest | 0.0 | Astrocytes TNFalpha + IL-1beta | 4.9 |
| Secondary CD8 lymphocyte act | 0.0 | KU-812 (Basophil) rest | 6.6 |
| CD4 lymphocyte none | 0.0 | KU-812 (Basophil) PMA/ionomycin | 5.1 |
| 2ry Th1/Th2/Tr1_anti-CD95 CH11 | 0.0 | CCD1106 (Keratinocytes) none | 25.9 |
| LAK cells rest | 0.0 | CCD1106 (Keratinocytes) TNFalpha + IL-1beta | 20.9 |
| LAK cells IL-2 | 0.0 | Liver cirrhosis | 0.0 |
| LAK cells IL-2+IL-12 | 0.0 | NCI-H292 none | **100.0** |
| LAK cells IL-2+IFN gamma | 0.0 | NCI-H292 IL-4 | 0.0 |
| LAK cells IL-2+ IL-18 | 0.0 | NCI-H292 IL-9 | 0.0 |
| LAK cells PMA/ionomycin | 0.0 | NCI-H292 IL-13 | 0.0 |
| NK Cells IL-2 rest | 0.0 | NCI-H292 IFN gamma | 0.0 |
| Two Way MLR 3 day | 0.0 | HPAEC none | 0.0 |
| Two Way MLR 5 day | 4.2 | HPAEC TNF alpha + IL-1 beta | 6.3 |
| Two Way MLR 7 day | 0.0 | Lung fibroblast none | 5.7 |
| PBMC rest | 0.0 | Lung fibroblast TNF alpha + IL-1 beta | 0.0 |
| PBMC PWM | 0.0 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 6.0 |
| Ramos (B cell) ionomycin | 0.0 | Lung fibroblast IFN gamma | 0.0 |
| B lymphocytes PWM | 0.0 | Dermal fibroblast CCD1070 rest | 0.0 |
| B lymphocytes CD40L and IL-4 | 0.0 | Dermal fibroblast CCD1070 TNF alpha | 0.0 |
| EOL-1 dbcAMP | 0.0 | Dermal fibroblast CCD1070 IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 0.0 |
| Dendritic cells anti-CD40 | 0.0 | Neutrophils TNFa+LPS | 0.0 |
| Monocytes rest | 0.0 | Neutrophils rest | 0.0 |
| Monocytes LPS | 0.0 | Colon | 3.2 |
| Macrophages rest | 0.0 | Lung | 20.3 |
| Macrophages LPS | 4.9 | Thymus | 0.0 |
| HUVEC none | 0.0 | Kidney | 21.3 |
| HUVEC starved | 4.5 | | |

**Table AHF. general oncology screening panel_v_2.4**

| **Tissue Name** | **Rel. Exp.(%) Ag4412, Run 264979086** | **Tissue Name** | **Rel. Exp.(%) Ag4412, Run 264979086** |
|---|---|---|---|
| Colon cancer 1 | 0.0 | Bladder cancer NAT 2 | 0.0 |
| Colon NAT 1 | 1.5 | Bladder cancer NAT 3 | 0.0 |
| Colon cancer 2 | 1.3 | Bladder cancer NAT 4 | 0.0 |
| Colon cancer NAT 2 | 0.0 | Adenocarcinoma of the prostate 1 | 6.5 |
| Colon cancer 3 | 1.8 | Adenocarcinoma of the prostate 2 | 0.6 |
| Colon cancer NAT 3 | 3.1 | Adenocarcinoma of the prostate 3 | 1.6 |
| Colon malignant cancer 4 | 5.4 | Adenocarcinoma of the prostate 4 | 1.6 |
| Colon normal adjacent | 0.0 | Prostate cancer NAT 5 | 0.5 |
| Lung cancer 1 | 9.0 | Adenocarcinoma of the prostate 6 | 0.0 |
| Lung NAT 1 | 0.0 | Adenocarcinoma of the prostate 7 | 1.4 |
| Lung cancer 2 | 22.4 | Adenocarcinoma of the prostate 8 | 0.0 |
| Lung NAT 2 | 2.0 | Adenocarcinoma of the prostate 9 | 1.9 |
| Squamous cell carcinoma 3 | 4.1 | Prostate cancer NAT 10 | 0.0 |
| Lung NAT 3 | 0.0 | Kidney cancer 1 | 12.6 |
| metastatic melanoma 1 | 4.7 | KidneyNAT 1 | 4.8 |
| Melanoma 2 | 0.0 | Kidney cancer 2 | **100.0** |
| Melanoma 3 | 0.0 | Kidney NAT 2 | 5.3 |
| metastatic melanoma | 6.2 | Kidney cancer 3 | 2.3 |
| metastatic melanoma | 5.7 | Kidney NAT 3 | 1.2 |
| 5 | | | |
| Bladder cancer 1 | 0.0 | Kidney cancer 4 | 14.5 |
| Bladder cancer NAT 1 | 0.0 | Kidney NAT 4 | 2.4 |
| Bladder cancer 2 | 0.0 | | |

**CNS_neurodegeneration_v1.0 Summary:** Ag4412 This panel does not show differential expression of the CG110187-01 gene in Alzheimer's disease. However, this expression profile confirms the presence of this gene in the brain. Please see Panel 1.4 for discussion of use of this gene in the central nervous system.

**General_screening_panel_v1.4 Summary:** Ag4412 Highest expression of the CG110187-01 gene is seen in the fetal brain (CT=29.8). This gene is also expressed at moderate to low levels in all CNS regions examined, including the hippocampus, thalamus, substantia nigra, amygdala, cerebellum and cerebral cortex. The cadherins have been shown to be critical for CNS development, specifically for the guidance of axons, dendrites and/or growth cones in general. Therapeutic modulation of the levels of this protein, or possible signaling via this protein may be of utility in enhancing/directing compensatory synaptogenesis and fiber growth in the CNS in response to neuronal death (stroke, head trauma), axon lesion (spinal cord injury), or neurodegeneration (Alzheimer's, Parkinson's, Huntington's, vascular dementia or any neurodegenerative disease). Since protocadherins play an important role in synaptogenesis, this gene product may also be involved in depression, schizophrenia, which also involve synaptogeneisis. (Hilschmann N. Naturwissenschaften 2001 Jan;88(1):2-12)

Moderate levels of expression are also seen in prostate, ovarian, lung and brain cancer cell lines. Thus, expression of this gene could be used to as a marker to detect the presence of these cancers. This gene encodes a protien that is homologous to cadherin which is involved in cellular adhesion. Dysregulation of cadherins has been observed in cancer, including renal cell carcinomas (Stassar, MJ. Br J Cancer 2001 Nov 2;85(9):1372-82). Therefore, therapeutic modulation of the expression or function of this gene may be effective in the treatment of prostate, ovarian, lung and brain cancers.

**Oncology_cell_line_screening_panel_v3.2 Summary:** Ag4412 Significant expression of the CG110817-01 gene is restricted to lung cancer cell lines and the cerebellum, with highest expression in a small cell lung cancer cell line (CT=32.4). This expression is in agreement with expression in Panel 1.4, where significant levels of expression are detected in the brain and cancer cell lines. Thus, expression of this gene could be used as a marker for lung cancer. Furthermore, therapeutic modulation of the expression or function of this gene may be effective in the treatment of lung cancer.

**Panel 4.1D Summary:** Ag4412 Significant expression of the CG110817-01 gene is restricted to untreated muco-epidermoid NCI-H292 cells (CT=34.9). Thus, the protein could be used to identify certain lung tumors similar to NCI-H292. This expression is in agreement with the previous panels, where significant levels of expression are detected in lung cancer cell lines. The encoded protein may also contribute to the normal function of the goblet cells within the lung. Therefore, designing therapeutics to this protein may be important for the treatment of emphysema and asthma as well as other lung diseases in which goblet cells or the mucus they produce have pathological consequences.

**general oncology screening panel_v_2.4 Summary:** Ag4412 Highest expression of the CG110817-01 gene is seen in kidney cancer (CT=32.2). In addition, significant levels of expression are also seen in kidney cancer and lung cancer when compared to expression in the normal adjacent tissue. Thus, expression of this gene could be used as a marker of these cancers. Furthermore, therapeutic modulation of the expression or function of this gene may be effective in the treatment of lung cancer.

### AI. CG110205-01 and CG110205-02: A DISINTEGRIN-LIKE AND METALLOPROTEASE (REPROLYSIN TYPE) WITH THROMBOSPONDIN

Expression of gene CG110205-01 and CG110205-02 was assessed using the primer-probe sets Ag2430, Ag4413, Ag6546, Ag6645, Ag7012 and Ag7058, described in Tables AIA, AIB, AIC, AID, AIE and AIF. Results of the RTQ-PCR runs are shown in Tables AIG, AIH, AII, AIJ, AIK, AIL, AIM, AIN and AIO. Please note that CG110205-02 is the peptide containing reprolysin and thrombospondin type 1 domains of CG110205-01 and only recognized by probes Ag2430, and Ag4413.

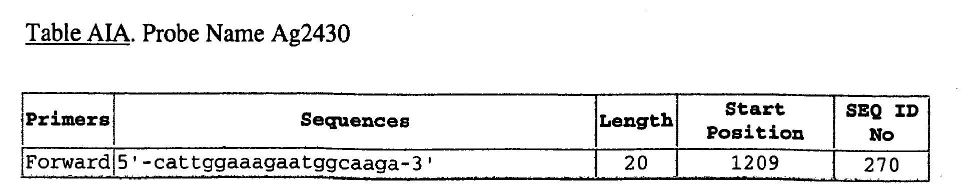

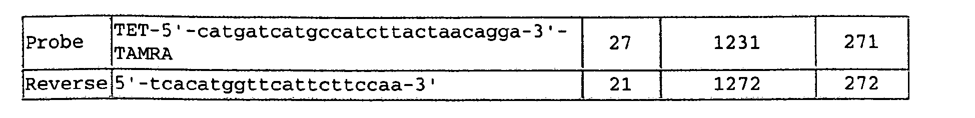

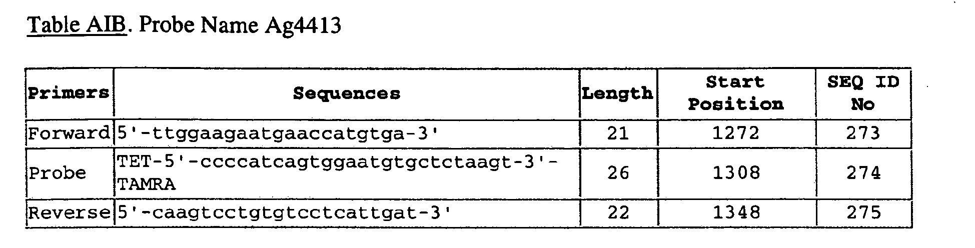

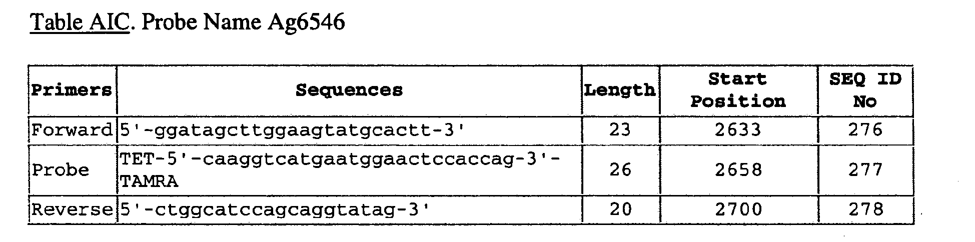

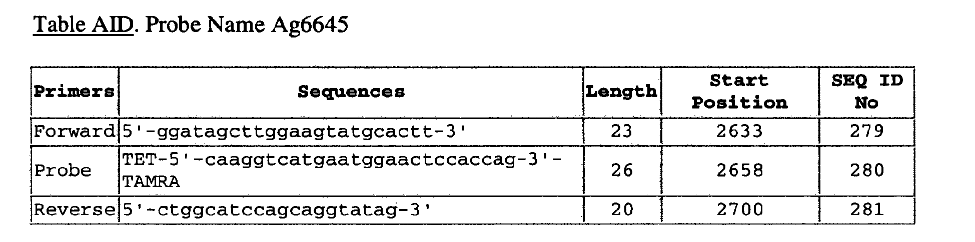

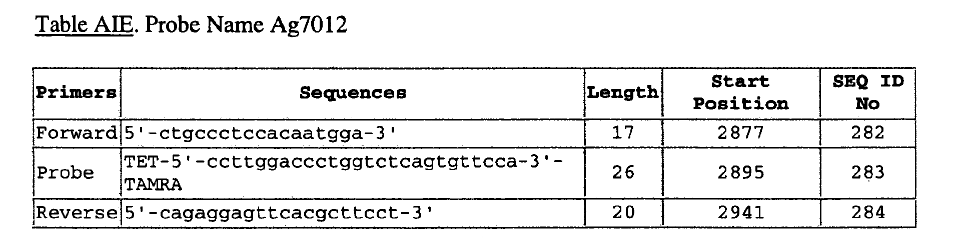

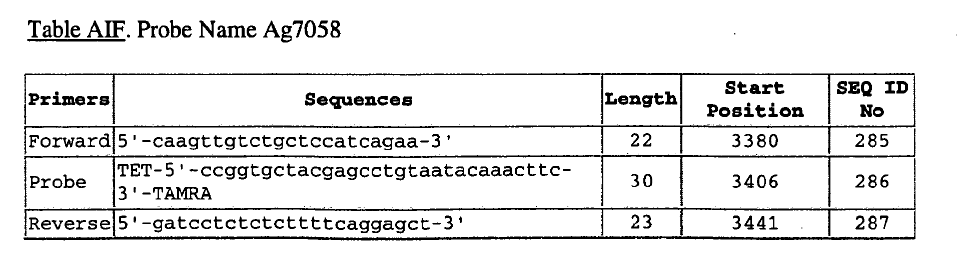

**Table AIG. AI_comprehensive panel_v1.0**

| **Tissue Name** | **Rel. Exp.(%) Ag4413, Run 248080021** | **Rel. Exp.(%) Ag4413, Run 251506632** | **Tissue Name** | **Rel. Exp.(%) Ag4413, Run 248080021** | **Rel. Exp.(%) Ag4413, Run 251506632** |
|---|---|---|---|---|---|
| 110967 COPD-F | 0.3 | 5.1 | 112427 Match Control Psoriasis-F | 0.8 | 1.4 |
| 110980 COPD-F | 0.2 | 0.0 | 112418 Psoriasis-M | 0.5 | 3.3 |
| 110968 COPD-M | 0.5 | 4.2 | 112723 Match Control Psoriasis-M | 2.3 | 22.8 |
| 110977 COPD-M | 0.2 | 0.5 | 112419 Psoriasis-M | 0.4 | 0.7 |
| 110989 Emphysema-F | 0.5 | 33.2 | 112424 Match Control Psoriasis-M | 0.3 | 6.4 |
| 110992 Emphysema-F | 0.7 | 3.5 | 112420 Psoriasis-M | 1.2 | 7.3 |
| 110993 Emphysema-F | 0.4 | 7.9 | 112425 Match Control Psoriasis-M | 0.2 | 10.8 |
| 110994 Emphysema-F | 0.2 | 3.4 | 104689 (MF) OA Bone-Backus | 7.5 | 17.1 |
| 110995 Emphysema-F | 0.7 | 4.0 | 104690 (MF) Adj "Normal" Bone-Backus | 0.3 | 1.5 |
| 110996 Emphysema-F | 0.3 | 0.9 | 104691 (MF) OA Synovium-Backus | 0.2 | 3.6 |
| 110997 Asthma-M | 0.3 | 3.0 | 104692 (BA) OA Cartilage-Backus | 0.0 | 0.0 |
| 111001 Asthma-F | 0.1 | 24.1 | 104694 (BA) OA Bone-Backus | 23.7 | 3.9 |
| 111002 Asthma-F | 0.3 | 22.4 | 104695 (BA) Adj "Normal" Bone-Backus | 2.6 | 6.3 |
| 111003 Atopic Asthma-F | 0.9 | 71.2 | 104696 (BA) OA Synovium-Backus | 1.0 | 16.6 |
| 111004 Atopic Asthma-F | 1.7 | 33.4 | 104700 (SS) OA Bone-Backus | 2.2 | 2.3 |
| 111005 Atopic Asthma-F | 1.6 | 38.2 | 104701 (SS) Adj "Normal" Bone-Backus | 5.9 | 4.1 |
| 111006 Atopic Asthma-F | 0.4 | 7.9 | 104702 (SS) OA Synovium-Backus | 1.1 | 60.3 |
| 111417 Allergy-M | 0.5 | 1.1 | 117093 OA Cartilage Rep7 | 0.2 | 3.2 |
| 112347 Allergy-M | 0.8 | 0.0 | 112672 OA Bone5 | 0,1 | 0.6 |
| 112349 Normal Lung-F | 0.8 | 0.0 | 112673 OA Synovium5 | 0.1 | 0.0 |
| 112357 Normal | 0.7 | 17.6 | 112674 OA | 0.1 | 0.6 |
| Lung-F | | | Synovial Fluid cells5 | | |
| 112354 Normal Lung-M | 0.2 | 0.7 | 117100 OA Cartilage Rep 14 | 0.1 | 0.5 |
| 112374 Crohns-F | 1.4 | 6.3 | 112756 OA Bone9 | 1.0 | 20.7 |
| 112389 Match Control Crohns-F | 0.5 | 0.0 | 112757 OA Synovium9 | 0.0 | 0.0 |
| 112375 Crohns-F | 1.8 | 5.1 | 112758 OA Synovial Fluid Cells9 | 0.5 | 12.9 |
| 112732 Match Control Crohns-F | 0.0 | 0.0 | 117125 RA Cartilage Rep2 | 0.5 | 24.3 |
| 112725 Crohns-M | 0.4 | 0.0 | 113492 Bone2 RA | 0.0 | 1.4 |
| 112387 Match Control Crohns-M | 0.2 | 0.6 | 113493 Synovium2 RA | 0.0 | 0.0 |
| 112378 Crohns-M | 0.6 | 0.0 | 113494 Syn Fluid Cells RA | 0.0 | 1.0 |
| 112390 Match Control Crohns- | 0.2 | 6.0 | 113499 Cartilage4 RA | 0.0 | 0.0 |
| 112726 Crohns-M | 1.9 | **100.0** | 113500 Bone4 RA | 0.0 | 0.0 |
| 112731 Match Control Crohns-M | 0.4 | 52.1 | 113501 Synovium4 RA RA | 0.0 | 0.7 |
| 112380 Ulcer Col-F | 0.6 | 29.3 | 113502 Syn Fluid Cells4 RA | 0.0 | 1.5 |
| 112734 Match Control Ulcer Col-F | **100.0** | 0.6 | 113495 Cartilage3 RA | 0.0 | 0.8 |
| 112384 Ulcer Col-F | 1.4 | 4.2 | 113496 Bone3 RA | 0.0 | 0.5 |
| 112737 Match Control Ulcer Col-F | 1.2 | 52.1 | 113497 Synovium3 RA | 0.0 | 0.6 |
| 112386 Ulcer Col-F | 0.1 | 0.7 | 113498 Syn Fluid Cells3 RA | 0.0 | 0.0 |
| 112738 Match Control Ulcer Col-F | 0.1 | 0.0 | 117106 Normal Cartilage Rep20 | 0.1 | 1.3 |
| 112381 Ulcer Col-M | 0.3 | 0.5 | 113663 Bone3 Normal | 0.7 | 0.1 |
| 112735 Match Control Ulcer Col-M | 1.9 | 0.9 | 113664 Synovium3 Normal | 0.1 | 0.0 |
| 112382 Ulcer Col-M | 0.4 | 0.0 | 113665 Syn Fluid Cells3 Normal | 0.4 | 0.0 |
| 112394 Match Control Ulcer Col-M | 0.1 | 0.6 | 117107 Normal Cartilage Rep22 | 0.0 | 1.3 |
| 112383 Ulcer Col-M | 1.8 | 18.7 | 113667 Bone4 Normal | 0.2 | 0.9 |
| 112736 Match Control Ulcer Col-M | 1.6 | 0.0 | 113668 Synovium4 Normal | 0.1 | 1.6 |
| 112423 Psoriasis-F | 1.0 | 11.2 | 113669 Syn Fluid Cells4 Normal | 0.2 | 1.9 |

**Table AIH. CNS_neurodegeneration_v1.0**

| **Tissue Name** | **Rel. Exp.(%) Ag2430, Run 208712834** | **Rel. Exp.(%) Ag4413, Run 224505949** | **Tissue Name** | **Rel. Exp.(%) Ag2430, Run 208712834** | **Rel. Exp.(%) Ag4413, Run 224505949** |
|---|---|---|---|---|---|
| AD 1 Hippo | 0.0 | 0.0 | Control (Path) 3 Temporal Ctx | 2.2 | 3.0 |
| AD 2 Hippo | 15.4 | 21.6 | Control (Path) 4 Temporal Ctx | 1.1 | 4.0 |
| AD 3 Hippo | 0.0 | 0.8 | AD 1 Occipital Ctx | 0.7 | 0.0 |
| AD 4 Hippo | 17.6 | 27.5 | AD 2 Occipital Ctx (Missing) | 0.0 | 0.0 |
| AD 5 Hippo | 13.9 | 22.5 | AD 3 Occipital | 0.0 | 0.0 |
| AD 6 Hippo | 43.2 | 0.0 | AD 4 Occipital | 52.5 | 94.0 |
| Control 2 Hippo | 62.9 | 85.9 | AD 5 Occipital Ctx | 27.7 | 25.5 |
| Control 4 Hippo | 0.0 | 1.6 | AD 6 Occipital Ctx | 24.0 | 27.7 |
| Control (Path) 3 Hippo | 3.2 | 14.5 | Control 1 Occipital Ctx | 0.0 | 0.0 |
| AD 1 Temporal Ctx | 0.9 | 0.7 | Control 2 Occipital Ctx | 52.1 | 66.0 |
| AD 2 Temporal Ctx | 21.9 | 37.6 | Control 3 Occipital Ctx | 3.1 | 11.1 |
| AD 3 Ctx Temporal Ctx | 0.0 | 0.0 | Control 4 Occipital Ctx | 0.0 | 0.0 |
| AD 4 Temporal Ctx | 57.0 | 97.9 | Control (Path) 1 Occipital Ctx | 59.9 | 73.2 |
| AD 5 Inf Temporal Ctx | 69.7 | 65.1 | Control (Path) 2 Occipital Ctx | 6.6 | 10.1 |
| AD 5 Sup Temporal Ctx | 38.2 | 26.2 | Control (Path) 3 Occipital Ctx | 5.4 | 4.7 |
| AD 6 Inf Temporal Ctx | **100.0** | **100.0** | Control (Path) 4 Occipital Ctx | 0.0 | 0.0 |
| AD 6 Sup Temporal Ctx | 36.6 | 37.6 | Control 1 Parietal Ctx | 0.0 | 0.0 |
| Control 1 Temporal Ctx | 0.0 | 0.8 | Control 2 Parietal Ctx | 37.6 | 38.4 |
| Control 2 Temporal Ctx | 36.6 | 45.7 | Control 3 Parietal Ctx | 6.3 | 6.6 |
| Control 3 Temporal Ctx | 9.3 | 12.5 | Control (Path) I Parietal Ctx | 19.5 | 26.1 |
| Control 3 Temporal Ctx | 0.0 | 1.4 | Control (Path) 2 Parietal Ctx | 7.2 | 9.1 |
| Control (Path) 1 Temporal Ctx | 18.0 | 28.1 | Control (Path) 3 Parietal Ctx | 0.0 | 4.6 |
| Control (Path) 2 Temporal Ctx | 5.1 | 0.0 | Control (Path) 4 Parietal Ctx | 0.0 | 0.9 |

**Table AII. General_screening_panel_v1.4**

| **Tissue Name** | **Rel. Exp.(%) Ag4413, Run 219923153** | **Tissue Name** | **Rel. Exp.(%) Ag4413, Run 219923153** |
|---|---|---|---|
| Adipose | 7.2 | Renal ca. TK-10 | 0.0 |
| Melanoma* Hs688(A).T | 0.0 | Bladder | 0.2 |
| Melanoma* Hs688(B).T | 0.0 | Gastric ca. (liver met.) NCI-N87 | 0.0 |
| Melanoma* M14 | 0.0 | Gastric ca. KATO III | 0.5 |
| Melanoma* LOXIMVI | 37.4 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 49.0 |
| Testis Pool | 1.8 | Colon ca. HT29 | 0.0 |
| Prostate ca.* (bone met) PC-3 | 0.0 | Colon ca. HCT-116 | 0.0 |
| Prostate Pool | 1.3 | Colon ca. CaCo-2 | 3.0 |
| Placenta | 19.5 | Colon cancer tissue | 2.6 |
| Uterus Pool | 0.9 | Colon ca. SW1116 | 0.0 |
| Ovarian ca. OVCAR-3 | 0.0 | Colon ca. Colo-205 | 0.0 |
| Ovarian ca. SK-OV-3 | 0.2 | Colon ca. SW-48 | 0.0 |
| Ovarian ca. OVCAR-4 | 0.0 | Colon Pool | 11.2 |
| Ovarian ca. OVCAR-5 | 0.0 | Small Intestine Pool | 1.7 |
| Ovarian ca. IGROV-1 | 0.1 | Stomach Pool | 6.3 |
| Ovarian ca. OVCAR-8 | 3.0 | Bone Marrow Pool | 2.7 |
| Ovary | 19.2 | Fetal Heart | 0.3 |
| Breast ca. MCF-7 | 0.0 | Heart Pool | 1.5 |
| Breast ca. MDA-MB-231 | 0.0 | Lymph Node Pool | 20.3 |
| Breast ca. BT 549 | 1.1 | Fetal Skeletal Muscle | 3.0 |
| Breast ca. T47D | 0.0 | Skeletal Muscle Pool | 0.0 |
| Breast ca. MDA-N | 0.0 | Spleen Pool | 0.0 |
| Breast Pool | 11.7 | Thymus Pool | 8.4 |
| Trachea | 0.9 | CNS cancer (glio/astro) U87-MG | 39.0 |
| Lung | 0.6 | CNS cancer (glio/astro) U-118-MG | 1.8 |
| Fetal Lung | 0.6 | CNS cancer (neuro;met) SK-N-AS | 0.2 |
| Lung ca. NCI-N417 | 0.0 | CNS cancer (astro) SF-539 | 0.0 |
| Lung ca. LX-1 | 9.7 | CNS cancer (astro) SNB-75 | 0.7 |
| Lung ca. NCI-H146 | 3.1 | CNS cancer (glio) SNB-19 | 0.2 |
| Lung ca. SHP-77 | 0.0 | CNS cancer (glio) SF-295 | 1.3 |
| Lung ca. A549 | 0.0 | Brain (Amygdala) Pool | 4.0 |
| Lung ca. NCI-H526 | 5.4 | Brain (cerebellum) | **100.0** |
| Lung ca. NCI-H23 | 0.9 | Brain (fetal) | 2.7 |
| Lung ca. NCI-H460 | 0.0 | Brain (Hippocampus) Pool | 2.0 |
| Lung ca. HOP-62 | 0.4 | Cerebral Cortex Pool | 4.3 |
| Lung ca. NCI-H522 | 0.0 | Brain (Substantia nigra) Pool | 5.5 |
| Liver | 0.0 | Brain (Thalamus) Pool | 8.7 |
| Fetal Liver | 0.7 | Brain (whole) | 11.8 |
| Liver ca. HepG2 | 0.2 | Spinal Cord Pool | 8.0 |
| Kidney Pool | 2.1 | Adrenal Gland | 0.0 |
| Fetal Kidney | 17.1 | Pituitary gland Pool | 3.2 |
| Renal ca. 786-0 | 0.0 | Salivary Gland | 6.8 |
| Renal ca. A498 | 0.0 | Thyroid (female) | 0.3 |
| Renal ca. ACHN | 3.1 | Pancreatic ca. CAPAN2 | 0.0 |
| Renal ca. UO-31 | 30.1 | Pancreas Pool | 8.2 |

**Table AIJ. Oncology_cell_line_screening_panel_v3.2**

| **Tissue Name** | **Rel. Exp.(%) Ag2430, Run 258381230** | **Tissue Name** | **Rel. Exp.(%) Ag2430, Run 258381230** |
|---|---|---|---|
| 94905_Daoy_Medulloblastoma/C erebellum_sscDNA | 0.0 | 94954_Ca Ski_Cervical epidermoid carcinoma (metastasis)_sscDNA | 0.0 |
| 94906_TE671_Medulloblastom/C erebellum_sscDNA | 0.0 | 94955_ES-2_Ovarian clear cell carcinoma_sscDNA | 0.0 |
| 94907_D283 Med_Medulloblastoma/Cerebellu m_sscDNA | 0.0 | 94957_Ramos/6h stim_ Stimulated with PMA/ionomycin 6h_sscDNA | 0.0 |
| 94908_PFSK-1_Primitive Neuroectodermal/Cerebellum_ssc DNA | 0.0 | 94958_Ramos/14h stim_ Stimulated with PMA/ionomycin 14h_sscDNA | 0.0 |
| 94909_XF-498_CNS_sscDNA | 0.0 | 94962_MEG-01_Chronic myelogenous leukemia (megokaryoblast)_sscDNA | 0.0 |
| 94910_SNB-78_CNS/glioma_sscDNA | 0.0 | 94963_Raji_Burkitt's lymphoma_sscDNA | 0.0 |
| 94911_SF-268_CNS/glioblastoma_sscDNA | 0.0 | 94964_Daudi_Burkitt's lymphoma_sscDNA | 0.0 |
| 94912_T98G_Glioblastoma_sscD NA | 0.0 | 94965_U266 B-cell plasmacytoma/myeloma_sscDNA | 0.0 |
| 96776_SK-N-SH_Neuroblastoma (metastasis)_sscDNA | 0.0 | 94968_CA46_Burkitt's lymphoma_sscDNA | 0.0 |
| 94913_SF-295_CNS/glioblastoma_sscDNA | 0.0 | 94970_RL_non-Hodgkin's B-cell lymphoma_sscDNA | 0.0 |
| 132565_NT2 pool_sscDNA | 0.1 | 94972_JM1_pre-B-cell lymphoma/leukemia_sscDNA | 0.0 |
| 94914_Cerebellum_sscDNA | 57.8 | 94973_Jurkat_T cell leukemia_sscDNA | 0.0 |
| 96777_Cerebellum_sscDNA | 25.5 | 94974_TF-1_Erythroleukemia_sscDNA | 0.0 |
| 94916_NCI-H292_Mucoepidermoid lung carcinoma_sscDNA | 0.0 | 94975_HUT 78_T-cell lymphoma_sscDNA | 0.0 |
| 94917_DMS-114_Small cell lung cancer_sscDNA | 17.9 | 94977_U937_Histiocytic lymphoma_sscDNA | 0.0 |
| 94918_DMS-79_Small cell lung cancer/neuroendocrine_sscDNA | **100.0** | 94980_KU-812_Myelogenous leukemia_sscDNA | 0.0 |
| 94919_NCI-H146_Small cell lung cancer/neuroendocrine_sscDNA | 2.9 | 94981_769-P_Clear cell renal carcinoma_sscDNA | 0.0 |
| 94920_NCI-H526_Small cell lung cancer/neuroendocrine_sscDNA | 10.9 | 94983_Caki-2_Clear cell renal carcinoma_sscDNA | 0.0 |
| 94921_NCI-N417_Small cell lung cancer/neuroendocrine_sscDNA | 0.0 | 94984_SW 839_Clear cell renal carcinoma_sscDNA | 0.0 |
| 94923_NCI-H82_Small cell lung cancer/neuroendocrine_sscDNA | 0.0 | 94986_G401_Wilms' tumor_sscDNA | 0.0 |
| 94924_NCI-H157_Squamous cell lung cancer (metastasis)_sscDNA | 0.0 | 126768_293 cells_sscDNA | 0.0 |
| 94925_NCI-H1155_Large cell lung cancer/neuroendocrine_sscDNA | 0.0 | 94987_Hs766T_Pancreatic carcinoma (LN metastasis)_sscDNA | 0.0 |
| 94926_NCI-H1299_Large cell lung cancer/neuroendocrine_sscDNA | 0.0 | 94988_CAPAN-1_Pancreatic adenocarcinoma (liver metastasis)_sscDNA | 0.0 |
| 94927_NCI-H727_Lung carcinoid_sscDNA | 0.3 | 94989_SU86.86_Pancreatic carcinoma (liver metastasis)_sscDNA | 0.0 |
| 94928_NCI-UMC-11_Lung carcinoid_sscDNA | 0.0 | 94990_BxPC-3_Pancreatic adenocarcinoma_sscDNA | 0.0 |
| 94929_LX-1_Small cell lung cancer_sscDNA | 3.8 | 94991_HPAC_Pancreatic adenocarcinoma_sscDNA | 0.0 |
| 94930_Colo-205_Colon cancer_sscDNA | 0.0 | 94992_MIA PaCa-2_Pancreatic carcinoma_sscDNA | 0.0 |
| 94931_KM12_Colon cancer_sscDNA | 0.0 | 94993_CFPAC-1 _Pancreatic ductal adenocarcinoma_sscDNA | 0.0 |
| 94932_KM20L2_Colon cancer_sscDNA | 0.0 | 94994_PANC-1_Pancreatic epithelioid ductal carcinoma_sscDNA | 0.0 |
| 94933_NCI-H716_Colon cancer_sscDNA | 0.0 | 94996_T24_Bladder carcinma (transitional cell)_sscDNA | 0.0 |
| 94935_SW-48_Colon adenocarcinoma_sscDNA | 0.0 | 94997_5637_Bladder carcinoma_sscDNA | 0.0 |
| 94936_SW1116_Colon adenocarcinoma_sscDNA | 0.0 | 94998_HT-1197_Bladder carcinoma_sscDNA | 0.0 |
| 94937_LS 174T_Colon adenocarcinoma_sscDNA | 0.0 | 94999_UM-UC-3_Bladder carcinma (transitional cell)_sscDNA | 0.0 |
| 94938_SW-948_Colon adenocarcinoma_sscDNA | 0.0 | 95000_A204_Rhabdomyosarcoma _sscDNA | 0.4 |
| 94939_SW-480_Colon adenocarcinoma_sscDNA | 0.0 | 95001_HT-1080_Fibrosarcoma_sscDNA | 0.2 |
| 94940_NCI-SNU-5_Gastric carcinoma_sscDNA | 0.0 | 95002_MG-63_Osteosarcoma (bone)_sscDNA | 0.0 |
| 112197_KATO III_Stomach_sscDNA | 0.0 | 95003_SK-LMS-1_Leiomyosarcoma (vulva)_sscDNA | 0.0 |
| 94943_NCI-SNU-16_Gastric carcinoma_sscDNA | 0.0 | 95004_SJRH30_Rhabdomyosarco ma (met to bone marrow)_sscDNA | 0.0 |
| 94944_NCI-SNU-1_Gastric carcinoma_sscDNA | 0.0 | 95005_A431_Epidermoid carcinoma_sscDNA | 0.0 |
| 94946_RF-1 _Gastric adenocarcinoma_sscDNA | 0.0 | 95007_WM266-4_Melanoma_sscDNA | 0.0 |
| 94947_RF-48_Gastric adenocarcinoma_sscDNA | 0.0 | 112195_DU 145_Prostate_sscDNA | 0.0 |
| 96778_MKN-45_Gastric carcinoma_sscDNA | 0.0 | 195012_MDA-MB-468_Breast adenocarcinoma_sscDNA | 0.0 |
| 94949_NCI-N87_Gastric carcinoma_sscDNA | 0.0 | 112196_SSC-4_Tongue_sscDNA | 0.0 |
| 94951_OVCAR-5_Ovarian carcinoma_sscDNA | 0.0 | 112194_SSC-9_Tongue_sscDNA | 0.0 |
| 94952_RL95-2_Uterine carcinoma_sscDNA | 0.0 | 112191_SSC-15_Tongue_sscDNA | 0.0 |
| 94953_HelaS3_Cervical adenocarcinoma_sscDNA | 0.0 | 95017_CAL 27_Squamous cell carcinoma of tongue_sscDNA | 0.0 |

**Table AIK. Panel 1.3D**

| **Tissue Name** | **Ret.Exp.(%)Ag2430, Run 159505456** | **Tissue Name** | **Rel. Exp.(%)Ag2430, Run 159505456** |
|---|---|---|---|
| Liver adenocarcinoma | 0.0 | Kidney (fetal) | 9.3 |
| Pancreas | 0.0 | Renal ca. 786-0 | 0.0 |
| Pancreatic ca. CAPAN 2 | 0.0 | Renal ca. A498 | 0.5 |
| Adrenal gland | 1.1 | Renal ca. RXF 393 | 0.0 |
| Thyroid | 0.2 | Renal ca. ACHN | 3.8 |
| Salivary gland | 15.0 | Renal ca. UO-31 | 13.9 |
| Pituitary gland | 13.2 | Renal ca. TK-10 | 0.0 |
| Brain (fetal) | 0.3 | Liver | 0.0 |
| Brain (whole) | 40.9 | Liver (fetal) | 0.0 |
| Brain (amygdala) | 9.7 | Liver ca. (hepatoblast) HepG2 | 0.0 |
| Brain (cerebellum) | 100.0 | Lung | 0.0 |
| Brain (hippocampus) | 37.9 | Lung (fetal) | 2.6 |
| Brain (substantia nigra) | 8.0 | Lung ca. (small cell) LX-1 | 9.9 |
| Brain (thalamus) | 12.2 | Lung ca. (small cell) NCI-H69 | 29.5 |
| Cerebral Cortex | 4.1 | Lung ca. (s.cell var.) SHP-77 | 0.0 |
| Spinal cord | 6.4 | Lung ca. (large cell)NCI-H460 | 0.0 |
| glio/astro U87-MG | 59.9 | Lung ca. (non-sm. cell) A549 | 0.0 |
| glio/astro U-118-MG | 6.7 | Lung ca. (non-s.cell) NCI-H23 | 2.0 |
| astrocytoma SW1783 | 0.0 | Lung ca. (non-s.cell) HOP-62 | 0.0 |
| neuro*; met SK-N-AS | 0.0 | Lung ca. (non-s.cl) NCI-H522 | 0.0 |
| astrocytoma SF-539 | 0.0 | Lung ca. (squam.) SW | 0.0 |
| astrocytoma SNB-75 | 0.6 | Lung ca. (squam.) NCI-H596 | 0.8 |
| glioma SNB-19 | 0.3 | Mammary gland | 30.8 |
| glioma U251 | 17.8 | Breast 7 ca.* (pl.ef) MCF- | 0.0 |
| glioma SF-295 | 0.5 | Breast ca.* (pl.ef) MDA-MB-231 | 0.0 |
| Heart (fetal) | 0.0 | Breast ca.* (pl.ef) T47D | 0.0 |
| Heart | 0.0 | Breast ca. BT-549 | 31.9 |
| Skeletal muscle (fetal) | 13.5 | Breast ca. MDA-N | 0.3 |
| Skeletal muscle | 0.0 | Ovary | 32.8 |
| Bone marrow | 0.0 | Ovarian ca. OVCAR-3 | 0.0 |
| Thymus | 3.1 | Ovarian ca. OVCAR-4 | 0.0 |
| Spleen | 0.0 | Ovarian ca. OVCAR-5 | 0.0 |
| Lymph node | 0.6 | Ovarian ca. OVCAR-8 | 0.0 |
| Colorectal | 0.9 | Ovarian ca. IGROV-1 | 0.2 |
| Stomach | 0.0 | Ovarian ca.* (ascites) SK-OV-3 | 0.0 |
| Small intestine | 0.3 | Uterus | 3.0 |
| Colon ca. SW480 | 0.0 | Placenta | 47.0 |
| Colon ca.* SW620(SW480 met) | 75.3 | Prostate | 1.4 |
| Colon ca. HT29 | 0.0 | Prostate ca.* (bone met)PC-3 | 0.0 |
| Colon ca. HCT-116 | 0.0 | Testis | 1.2 |
| Colon ca. CaCo-2 | 2.8 | Melanoma Hs688(A).T | 0.0 |
| Colon ca. tissue(ODO3866) | 5.1 | Melanoma* (met) Hs688(B).T | 0.0 |
| Colon ca. HCC-2998 | 0.0 | Melanoma UACC-62 | 0.0 |
| Gastric ca.* (liver met) NCI-N87 | 0.0 | Melanoma M14 | 0.0 |
| Bladder | 1.7 | Melanoma LOX IMVI | 45.4 |
| Trachea | 2.2 | Melanoma* (met) SK-MEL-5 | 0.0 |
| Kidney | 0.2 | Adipose | 18.6 |

**Table AIL. Panel 2D**

| **Tissue Name** | **Rel. Exp.(%) Ag2430, Run 159505825** | **Tissue Name** | **Rel. Exp.(%) Ag2430, Run 159505825** |
|---|---|---|---|
| Normal Colon | 15.4 | Kidney Margin 8120608 | 0.6 |
| CC Well to Mod Diff (OD03866) | 12.9 | Kidney Cancer 8120613 | 5.1 |
| CC Margin (OD03866) | 0.0 | Kidney Margin 8120614 | 6.1 |
| CC Gr.2 rectosigmoid (ODO3868) | 0.3 | Kidney Cancer 9010320 | 9.7 |
| CC Margin (OD03868) | 3.0 | Kidney Margin 9010321 | 2.7 |
| CC Mod Diff (OD03920) | 5.3 | Normal Uterus | 23.8 |
| CC Margin (ODO3920) | 2.3 | Uterus Cancer 064011 | 25.5 |
| CC Gr.2 ascend colon (ODO3921) | 12.7 | Normal Thyroid | 2.9 |
| CC Margin (ODO3921) | 4.6 | Thyroid Cancer 064010 | 23.8 |
| CC from Partial Hepatectomy (ODO4309) Mets | 0.0 | Thyroid Cancer A302152 | 14.3 |
| Liver Margin (ODO4309) | 0.0 | Thyroid Margin A302153 | 5.6 |
| Colon mets to lung (OD04451-01) | 0.0 | Normal Breast | 58.6 |
| Lung Margin (OD04451-02) | 0.0 | Breast Cancer (OD04566) | 2.3 |
| Normal Prostate 6546-1 | 1.0 | Breast Cancer (OD04590-01) | 30.6 |
| Prostate Cancer (OD04410) | 1.2 | Breast Cancer Mets (OD04590-03) | 17.0 |
| Prostate Margin (OD04410) | 47.0 | Breast Cancer Metastasis (OD04655-05) | 6.6 |
| Prostate Cancer (OD04720-01) | 7.9 | Breast Cancer 064006 | 7.1 |
| Prostate Margin (OD04720-02) | 0.0 | Breast Cancer 1024 | 59.0 |
| Normal Lung 061010 | 11.4 | Breast Cancer 9100266 | 33.0 |
| Lung Met to Muscle (ODO4286) | 1.2 | Breast Margin 9100265 | 26.6 |
| Muscle Margin (ODO4286) | 6.8 | Breast Cancer A209073 | 43.8 |
| Lung Malignant Cancer (OD03126) | 10.9 | Breast Margin A209073 | 68.3 |
| Lung Margin (OD03126) | 2.5 | Normal Liver | 0.0 |
| Lung Cancer (OD04404) | 2.3 | Liver Cancer 064003 | 0.8 |
| Lung Margin (OD04404) | 24.7 | Liver Cancer 1025 | 0.0 |
| Lung Cancer (OD04565) | 6.9 | Liver Cancer 1026 | 3.0 |
| Lung Margin (OD04565) | 1.2 | Liver Cancer 6004-T | 0.0 |
| Lung Cancer (OD04237-O1) | **100.0** | Liver Tissue 6004-N | 2.9 |
| Lung Margin (OD04237-02) | 9.8 | Liver Cancer 6005-T | 0.0 |
| Ocular Mel Met to Liver (OD04310) | 0.0 | Liver Tissue 6005-N | 0.0 |
| Liver Margin (OD04310) | 0.0 | Normal Bladder | 4.8 |
| Melanoma Mets to Lung (OD04321) | 1.5 | Bladder Cancer 1023 | 2.5 |
| Lung Margin (OD04321) | 0.0 | Bladder Cancer A302173 | 16.3 |
| Normal Kidney | 24.7 | Bladder Cancer (OD04718-01) | 6.1 |
| Kidney Ca, Nuclear grade 2 (OD04338) | 6.4 | Bladder Normal Adjacent (OD04718-03) | 53.2 |
| Kidney Margin (OD04338) | 10.5 | Normal Ovary | 18.2 |
| Kidney Ca Nuclear grade 1/2 (OD04339) | 5.5 | Ovarian Cancer 064008 | 30.1 |
| Kidney Margin (OD04339) | 3.8 | Ovarian Cancer (OD04768-07) | 2.7 |
| Kidney Ca, Clear cell type (OD04340) | 14.1 | Ovary Margin (OD04768-08) | 6.6 |
| Kidney Margin (OD04340) | 3.1 | Normal Stomach | 0.8 |
| Kidney Ca, Nuclear grade 3 (OD04348) | 2.0 | Gastric Cancer 9060358 | 3.9 |
| Kidney Margin (OD04348) | 5.0 | Stomach Margin 9060359 | 0.0 |
| Kidney Cancer (OD04622-01) | 1.0 | Gastric Cancer 9060395 | 8.0 |
| Kidney Margin (OD04622-03) | 0.0 | Stomach Margin 9060394 | 1.8 |
| Kidney Cancer (OD04450-01) | 6.5 | Gastric Cancer 9060397 | 12.2 |
| Kidney Margin (OD04450-03) | 11.3 | Stomach Margin 9060396 | 0.0 |
| Kidney Cancer 8120607 | 2.6 | Gastric Cancer 064005 | 10.7 |

**Table AIM. Panel 4.1D**

| **Tissue Name** | **Rel. Exp.(%) Ag4413, Run 190281896** | **Rel. Exp.(%) Ag4413, Run 249495488** | **Rel. Exp.(%) Ag7012, Run 279065633** | **Tissue Name** | **Rel. Exp.(%) Ag4413, Run 190281896** | **Rel. Exp.(%) Ag4413, Run 249495488** | **Rel. Exp.(%) Ag7012, Run 279065633** |
|---|---|---|---|---|---|---|---|
| Secondary Th1 act | 0.0 | 0.0 | 0.0 | HUVEC IL-1beta | 16.6 | 12.1 | 24.7 |
| Secondary Th2 act | 0.4 | 0.0 | 0.0 | HUVEC IFN gamma | 52.9 | 53.2 | 57.8 |
| Secondary Tr1 act | 0.6 | 0.0 | 0.0 | HUVEC TNF alpha + IFN gamma | 5.0 | 0.0 | 4.2 |
| Secondary Th1 rest | 1.1 | 0.0 | 0.0 | HUVEC TNF alpha + IL4 | 6.4 | 0.0 | 0.9 |
| Secondary Th2 rest | 0.0 | 0.0 | 0.0 | HUVEC IL-11 | 49.3 | 54.0 | 79.0 |
| Secondary Tr1 rest | 0.0 | 0.0 | 0.0 | Lung Microvascular EC none | 33.0 | 25.0 | 66.0 |
| Primary Th1 act | 0.0 | 0.0 | 0.0 | Lung Microvascular EC TNFalpha + IL-1beta | 42.9 | 8.7 | 17.2 |
| Primary Th2 act | 0.0 | 0.0 | 0.0 | Microvascular Dermal EC none | 15.7 | 0.4 | 4.8 |
| Primary Tr1 act | 0.0 | 0.0 | 0.0 | Microsvasular Dermal EC TNFalpha + IL-1 beta | 5.5 | 3.0 | 4.2 |
| Primary Th1 rest | 0.0 | 0.0 | 0.0 | Bronchial epithelium TNFalpha + IL1beta | 0.0 | 0.0 | 0.0 |
| Primary Th2 rest | 0.0 | 0.0 | 0.0 | Small airway epithelium none | 0.0 | 0.0 | 0.0 |
| Primary Tr1 rest | 0.0 | 0.0 | 0.0 | Small airway epithelium TNFalpha + IL-1beta | 0.0 | 0.0 | 0.0 |
| CD45RA CD4 lymphocyte act | 0.0 | 0.0 | 0.0 | Coronery artery SMC rest | 3.6 | 2.8 | 0.5 |
| CD45RO CD4 lymphocyte act | 0.0 | 0.0 | 0.0 | Coronery artery SMC TNFalpha + IL-1beta | 1.9 | 2.8 | 1.0 |
| CD8 lymphocyte act | 0.0 | 0.0 | 0.0 | Astrocytes rest | 0.0 | 0.9 | 0.0 |
| Secondary CD8 lymphocyte rest | 0.0 | 0.0 | 0.0 | Astrocytes TNFalpha + IL-1beta | 0.0 | 0.0 | 0.0 |
| Secondary CD8 lymphocyte act | 0.0 | 0.0 | 0.0 | KU-812 (Basophil) rest | 5.8 | 0.0 | 0.0 |
| CD4 lymphocyte none | 0.0. | 0.0 | 0.0 | KU-812 (Basophil) PMA/ionomycin | 0.9 | 0.0 | 0.0 |
| 2ry Th1/Th2/Tr1_anti-CD95 CH11 | 0.0 | 0.0 | 0.0 | CCD 1106 (Keratinocytes) none | 0.0 | 0.0 | 0.0 |
| LAK cells rest | 0.0 | 0.0 | 0.0 | CCD 1106 (Keratinocytes) TNFalpha + IL-1beta | 0.0 | 0.0 | 0.0 |
| LAK cells IL-2 | 0.0 | 0.0 | 0.0 | Liver cirrhosis | 0.4 | 0.0 | 0.0 |
| LAK cells IL-2+IL-12 | 0.0 | 0.0 | 0.0 | NCI-H292 none | 0.0 | 0.0 | 0.0 |
| LAK cells IL-2+IFN gamma | 0.0 | 0.0 | 0.0 | NCI-H292 IL-4 | 0.0 | 0.0 | 0.0 |
| LAK cells IL-2+ IL-18 | 0.0 | 0.0 | 0.0 | NCI-H292 IL-9 | 0.0 | 0.0 | 0.0 |
| LAK cells PMA/ionomycin | 0.0 | 0.0 | 0.0 | NCI-H292 IL-13 | 0.0 | 0.0 | 0.0 |
| NK Cells IL-2 rest | 0.0 | 0.0 | 0.0 | NCI-H292 IFN gamma | 0.0 | 0.0 | 0.0 |
| Two Way MLR 3 day | 0.0 | 0.0 | 0.0 | HPAEC none | 71.2 | 25.7 | 35.1 |
| Two Way MLR 5 day | 2.2 | 0.0 | 0.0 | HPAEC TNF alpha + IL-1 beta | **100.0** | **100.0** | **100.0** |
| Two Way MLR 7 day | 0.0 | 0.0 | 0.0 | Lung fibroblast none | 0.0 | 0.0 | 0.0 |
| PBMC rest | 0.0 | 0.0 | 0.0 | Lung fibroblast TNF alpha + IL-beta | 0.3 | 0.0 | 0.0 |
| PBMC PWM | 0.0 | 0.0 | 0.0 | Lung fibroblast | 0.3 | 0.0 | 0.0 |
| PBMC PHA-L | 0.0 | 0.0 | 0.0 | Lung fibroblast IL-9 | 0.0 | 0.0 | 0.0 |
| Ramos (B cell) none | 0.0 | 0.0 | 0.0 | Lung fibroblast IL-13 | 0.0 | 0.0 | 0.0 |
| Ramos (B cell) ionomycin | 0.0 | 0.0 | 0.0 | Lung fibroblast IFN gamma | 0.0 | 0.0 | 0.0 |
| B lymphocytes PWM | 0.0 | 0.0 | 0.0 | Dermal fibroblast CCD1070 rest | 0.0 | 0.0 | 0.0 |
| B lymphocytes CD40L and IL-4 | 0.0 | 0.0 | 0.0 | Dermal fibroblast CCD1070 TNF alpha | 0.0 | 0.0 | 0.0 |
| EOL-1 dbcAMP | 0.0 | 0.0 | 0.0 | Dermal fibroblast fibroblast beta | 0.0 | 0.0 | 0.0 |
| EOL-1 dbcAMP PMA/ionomycin | 0.0 | 0.0 | 0.0 | Dermal fibroblast IFN gamma | 0.0 | 0.0 | 0.0 |
| Dendritic cells none | 0.0 | 0.0 | 0.0 | Dermal fibroblast IL-4 | 0.0 | 0.0 | 0.0 |
| Dendritic cells LPS | 5.8 | 0.0 | 0.0 | Dermal Fibroblasts rest | 0.4 | 0.0 | 0.0 |
| Dendritic cells anti-CD40 | 0.0 | 0.0 | 0.0 | Neutrophils TNFa+LPS | 0.0 | 0.0 | 0.0 |
| Monocytes rest | 0.0 | 0.0 | 0.0 | Neutrophils rest | 0.0 | 0.0 | 0.0 |
| Monocytes LPS | 0.0 | 0.0 | 0.0 | Colon | 0.0 | 0.0 | 0.0 |
| Macrophages rest | 0.0 | 0.0 | 0.0 | Lung | 5.7 | 0.0 | 0.0 |
| Macrophages LPS | 0.0 | 0.0 | 0.0 | Thymus | 2.7 | 0.3 | 1.6 |
| HUVEC none | 33.4 | 14.9 | 27.5 | Kidney | 0.0 | 0.8 | 1.0 |
| HUVEC starved | 55.9 | 20.6 | 49.7 | | | | |

**Table AIN. Panel 4D**

| **Tissue Name** | **Rel. Exp.(%) Ag2430, Run 159506306** | **Tissue Name** | **Rel. Exp.(%) Ag2430, Run 159506306** |
|---|---|---|---|
| Secondary Th1 act | 0.0 | HUVEC IL-1beta | 5.9 |
| Secondary Th2 act | 0.6 | HUVEC IFN gamma | 40.3 |
| Secondary Tr1 act | 0.0 | HUVEC TNF alpha + IFN gamma | 5.1 |
| Secondary Th1 rest | 0.0 | HUVEC TNF alpha + IL4 | 4.0 |
| Secondary Th2 rest | 0.0 | HUVEC IL-11 | 47.3 |
| Secondary Tr1 rest | 0.0 | Lung Microvascular EC none | 22.1 |
| Primary Th1 act | 0.0 | Lung Microvascular EC TNFalpha + IL-1beta | 22.5 |
| Primary Th2 act | 0.0 | Microvascular Dermal EC none | 20.4 |
| Primary Tr1 act | 0.0 | Microsvasular Dermal EC TNFalpha + IL-1beta | 3.8 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha + IL-1beta | 0.0 |
| CD45RA CD4 lymphocyte act | 0.0 | Coronery artery SMC rest | 4.2 |
| CD45RO CD4 lymphocyte act | 0.0 | Coronery artery SMC TNFalpha + IL-1 beta | 2.1 |
| CD8 lymphocyte act | 0.0 | Astrocytes rest | 1.1 |
| Secondary CD8 lymphocyte rest | 0.0 | Astrocytes TNFalpha + IL-1beta | 0.9 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 0.4 |
| LAK cells IL-2+IL-12 | 0.0 | Lupus kidney | 0.2 |
| LAK cells IL-2+IFN gamma | 0.0 | NCI-H292 none | 0.0 |
| LAK cells IL-2+ IL-18 | 0.0 | NCI-H292 IL-4 | 0.0 |
| LAK cells PMA/ionomycin | 0.0 | NCI-H292 IL-9 | 0.0 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 0.0 | HPAEC none | 85.3 |
| Two Way MLR 7 day | 0.0 | HPAEC TNF alpha + IL-1 beta | 56.6 |
| PBMC rest | 0.0 | Lung fibroblast none | 0.0 |
| PBMC PWM | 0.0 | Lung fibroblast TNF alpha + IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytes rest | 0.0 | IBD Crohn's | 0.0 |
| Monocytes LPS | 0.0 | Colon | 0.7 |
| Macrophages rest | 0.0 | Lung | 3.1 |
| Macrophages LPS | 0.0 | Thymus | 2.6 |
| HUVEC none | 38.7 | Kidney | 7.2 |
| HUVEC starved | **100.0** | | |

**Table AIO. Panel CNS_1**

| **Tissue Name** | **Rel. Exp.(%) Ag2430, Run 171656292** | **Tissue Name** | **Rel. Exp.(%) Ag2430, Run 171656292** |
|---|---|---|---|
| BA4 Control | 0.0 | BA17 PSP | 2.9 |
| BA4 Control2 | 19.6 | BA17 PSP2 | 0.0 |
| BA4 Alzheimer's2 | 0.0 | Sub Nigra Control | 94.0 |
| BA4 Parkinson's | 0.0 | Sub Nigra Control2 | 25.9 |
| BA4 Parkinson's2 | 17.0 | Sub Nigra Alzheimer's2 | 15.7 |
| BA4 Huntington's | 9.8 | Sub Nigra Parkinson's2 | 36.6 |
| BA4 Huntington's2 | 0.0 | Sub Nigra Huntington's | **100.0** |
| BA4 PSP | 0.0 | Sub Nigra Huntington's2 | 2.8 |
| BA4 PSP2 | 11.0 | Sub Nigra PSP2 | 29.1 |
| BA4 Depression | 19.5 | Sub Nigra Depression | 62.4 |
| BA4 Depression2 | 0.0 | Sub Nigra Depression2 | 0.0 |
| BA7 Control | 40.1 | Glob Palladus Control | 51.4 |
| BA7 Control2 | 26.1 | Glob Palladus Control2 | 5.1 |
| BA7 Alzheimer's2 | 0.0 | Glob Palladus Alzheimer's | 21.3 |
| BA7 Parkinson's | 0.0 | Glob Palladus Alzheimer's2 | 0.0 |
| BA7 Parkinson's2 | 16.6 | Glob Palladus Parkinson's | 0.0 |
| BA7 Huntington's | 15.2 | Glob Palladus Parkinson's2 | 31.4 |
| BA7 Huntington's2 | 0.0 | Glob Palladus PSP | 0.0 |
| BA7 PSP | 0.0 | Glob Palladus PSP2 | 4.5 |
| BA7 PSP2 | 14.7 | Glob Palladus Depression | 44.4 |
| BA7 Depression | 4.3 | Temp Pole Control | 4.3 |
| BA9 Control | 7.1 | Temp Pole Control2 | 9.5 |
| BA9 Control2 | 30.8 | Temp Pole Alzheimer's | 3.6 |
| BA9 Alzheimer's | 0.0 | Temp Pole Alzheimer's2 | 0.0 |
| BA9 Alzheimer's2 | 0.0 | Temp Pole Parkinson's | 3.0 |
| BA9 Parkinson's | 0.0 | Temp Pole Parkinson's2 | 19.8 |
| BA9 Parkinson's2 | 33.9 | Temp Pole Huntington's | 10.7 |
| BA9 Huntington's | 37.4 | Temp Pole PSP | 0.0 |
| BA9 Huntington's2 | 0.0 | Temp Pole PSP2 | 1.7 |
| BA9 PSP | 0.0 | Temp Pole Depression2 | 2.5 |
| BA9 PSP2 | 1.9 | Cing Gyr Control | 86.5 |
| BA9 Depression | 5.2 | Cing Gyr Control2 | 26.6 |
| BA9 Depression2 | 0.0 | Cing Gyr Alzheimer's | 15.3 |
| BA17 Control | 28.3 | Cing Gyr Alzheimer's2 | 13.0 |
| BA17 Control2 | 20.6 | Cing Gyr Parkinson's | 0.0 |
| BA17 Alzheimer's2 | 7.0 | Cing Gyr Parkinson's2 | 49.3 |
| BA17 Parkinson's | 0.0 | Cing Gyr Huntington's | 58.6 |
| BA17 | 27.2 | Cing Gyr Huntington's2 | 0.0 |
| BA17 Huntington's | 23.7 | Cing Gyr PSP | 0.0 |
| BA17 | 0.0 | Cing Gyr PSP2 | 17.0 |
| BA17 Depression | 24.5 | Cing Gyr Depression | 49.3 |
| BA17 Depression2 | 27.9 | Cing Gyr Depression2 | 19.1 |

**AI_comprehensive panel_v1.0 Summary:** Ag4413 Highest expression of this gene is seen in a sample from a patient with Crohn's disease (CT=29.4). Moderate levels of expression are also seen in a cluster of tissues derived from patients with asthma and OA. This gene encodes a protein with homology to members of the ADAMTS family. ADAMTS proteins have been implicated in extracellular proteolysis and may play a critical role in the tissue degradation seen in arthritis and other inflammatory conditions. (Martel-Pelletier J. (2001) Best Pract Res Clin Rheumatol 15(5):805-29) Therefore, therapeutic modulation of the expression or function of this gene through the use of human monoclonal antibodies or small molecule drugs may be effective in the treatment of osteoarthritis and other autoimmune diseases.

**CNS_neurodegeneration_v1.0 Summary:** Ag2430/Ag4413 Two experiments with two different probe and primer sets produce results that are in excellent agreement, with highest expression in the temporal cortex of an Alzheimer's patient (CTs=30-32.7). These results confirm the expression of this gene at low levels in the brain in an independent group of individuals. This gene is found to be upregulated in the temporal cortex of Alzheimer's disease patients. Therefore, therapeutic modulation of the expression or function of this gene may decrease neuronal death and be of use in the treatment of this disease.

**General_screening_panel_v1.4 Summary:** Ag4413 Highest expression of this gene is seen in the cerebellum (CT=27). In addition, this gene is expressed at moderate to low levels in all regions of the CNS examined. The high levels of expression in the cerebellum suggest that this gene product may be a useful and specific target for the treatment of CNS disorders that originate in this region, such as autism and the ataxias.

Among tissues with metabolic function, this gene is expressed at moderate to low levels in adipose, pancreas, heart, and fetal skeletal muscle and liver. This expression suggests that this gene product may play a role in normal neuroendocrine and metabolic function and that disregulated expression of this gene may contribute to neuroendocrine disorders or metabolic diseases, such as obesity and diabetes.

In addition, this gene is expressed at much higher levels in fetal kidney tissue (CT=29.6) when compared to expression in the adult counterpart (CT=30.6). Thus, expression of this gene may be used to differentiate between the fetal and adult source of this tissue.

Moderate levels of expression are also seen in cell lines from brain, colon, lung, renal and melanoma cancers. Thus, expression of this gene may potentially be used as a marker of these cancers. Therapeutic modulation of this gene product may also be useful in the treatment of these cancers.

**Oncology_cell_line_screening_panel_v3.2 Summary:** Ag2430 Expression of the gene on this panel is limited to cerebellum and lung cancer cell lines. This is in agreement with the expression seen in Panels 1.3D and 1.4. Thus, expression of this gene could be used as a marker of cerebellar tissue and lung cancer and to differentiate these samples from other samples on this panel.

**Panel 1.3D Summary:** Ag2430 Expression of the gene in this panel is in agreement with expression in Panel 1.4. Highest expression is seen in the cerebellum (CT=31), with low but significant expression detected in the amygdala, hippocampus, substantia nigra and thalamus. Moderate to low levels of expression are seen in fetal skeletal muscle, adipose, and cancer cell lines derived from melanoma, breast, lung, renal, colon and brain cancers. Please see Panel 1.4 for further discussion of utility of this gene in human disease.

**Panel 2D Summary:** Ag2430 Highest expression of this gene is seen in lung cancer (CT=31). In addition, expression of this gene appears to be upregulated in lung, thyroid, gastric and ovarian cancer when compared to expression in the corresponding normal adjacent tissue. This protein is homologous to members of the family of ADAMTS proteins that are characterized by disintegrin, metalloproteinase and thrombospondin domains. This domain structure alone leads one to speculate that the expression of these genes in the context of cancer might play a role in the progression of the disease, as both metalloproteinases and thrombospondins have been demonstrated to be important to tumor progression. Specifically, the metalloproteinase domain may play a role in cell invasion and metastasis, and the thrombospondin domain may play a role in angiogenesis. (Masui T. Clin Cancer Res 2001 Nov;7(11):3437-4)

Based on the expression profile of this gene and the role played by ADAMTS proteins in tumor progression, this gene in the correct context might play a role in tumor angiogeneis. Furthermore, therapeutic targeting with antibodies or small molecule drugs directed against this gene product may block the angiogenic and invasion/metastasis promoting activities of this molecule especially in those cancer types where the gene is overexpressed in the tumor compared to the normal adjacent tissue.

**Panel 4.1D Summary:** Ag4413/Ag7012 Three experiments with two different probe and primer set produce results that are in excellent agreement. Highest expression is seen in TNF-a and IL-1 beta treated HPAECs. This gene appears to be preferentially expressed in endothelial cells, including microvascular dermal endothelial cells, microvascular lung endothelial cells, human pulmonary aortic endothelial cells and human umbilical vein endothelial cells. Endothelial cells are known to play important roles in inflammatory responses by altering the expression of surface proteins that are involved in activation and recruiting of effector inflammatory cells. The expression of this gene in dermal microvascular endothelial cells suggests that this protein product may be involved in inflammatory responses to skin disorders, including psoriasis. Expression in lung microvascular endothelial cells suggests that the protein encoded by this gene may also be involved in lung disorders including asthma, allergies, chronic obstructive pulmonary disease, and emphysema. The protein encoded by this gene has homology to ADAMTS family of molecules suggesting that it may function as an enzyme. Based on its homology, it may contribute to the tissue destruction and remodeling processes associated with asthma, ulcerative colitis, emphysema and osteoarthritis. (Kuno K. J Biol Chem 1997 Jan 3;272(1):556-62;) Therefore, blocking the function of the protein encoded by this gene with human nonoclonal antibody therapeutics or small molecule therapeutics may reduce or inhibit tissue destruction in the lungs, intestine, or joints due to emphysema, allergy, asthma, colitis, or osteoarthritis.

**Panel 4D Summary:** Ag2430 Highest expression of the gene in this panel is seen in HUVECs (CT=28). Expression in this panel is in agreement with expression in Panel 4.1D, with preferential expression seen in endothelial cells, including HPAECs, lung and dermal microvascular ECs, and a cluster of HUVEC samples. Please see Panel 4D for discussion of this gene in inflammation.

**Panel CNS_1 Summary:** Ag2430 This panel confirms the presence of this gene in the brain. Please see Panels 1.4 and CNS_neurodegeneration for discussion of this gene in the central nervous system.

### AJ. CG110242-01: Ebnerin

Expression of gene CG110242-01 was assessed using the primer-probe sets Ag1000 and Ag855, described in Tables AJA and AJB. Results of the RTQ-PCR runs are shown in Table AJC.

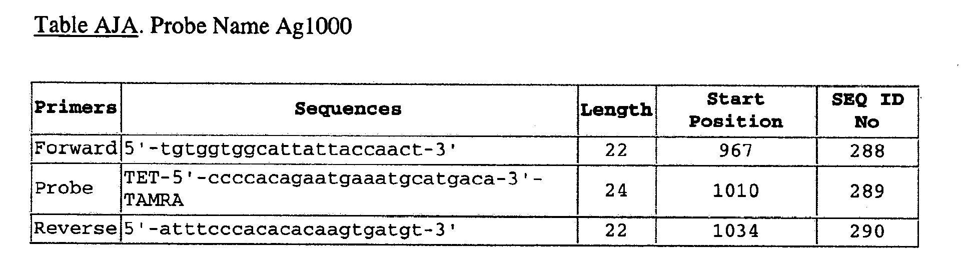

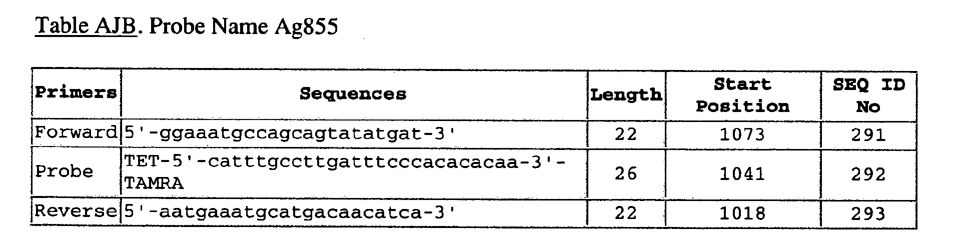

**Table AJC. General_screening_panel_v1.5**

| **Tissue Name** | **Rel. Exp.(%) Ag855, Run 258465524** | **Tissue Name** | **Rel. Exp.(%) Ag855, Run 258465524** |
|---|---|---|---|
| Adipose | 0.0 | Renal ca. TK-10 | 0.0 |
| Melanoma* Hs688(A).T | 0.0 | Bladder | **100.0** |
| Melanoma* Hs688(B).T | 0.0 | Gastric ca. (liver met.) NCI-N87 | 0.0 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 23.5 | Colon ca. HT29 | 0.0 |
| Prostate ca.* (bone met) PC-3 | 0.0 | Colon ca. HCT-116 | 0.0 |
| Prostate Pool | 2.1 | Colon ca. CaCo-2 | 0.0 |
| Placenta | 0.7 | Colon cancer tissue | 0.0 |
| Uterus Pool | 0.4 | Colon ca. SW 1116 | 0.0 |
| Ovarian ca. OVCAR-3 | 0.0 | Colon ca. Colo-205 | 0.0 |
| Ovarian ca. SK-OV-3 | 3.8 | Colon ca. SW-48 | 0.0 |
| Ovarian ca. OVCAR-4 | 0.0 | Colon Pool | 0.0 |
| Ovarian ca. OVCAR-5 | 0.0 | Small Intestine Pool | 0.9 |
| Ovarian ca. IGROV-1 | 0.6 | Stomach Pool | 0.0 |
| Ovarian ca. OVCAR-8 | 0.0 | Bone Marrow Pool | 0.0 |
| Ovary | 0.0 | Fetal Heart | 0.0 |
| Breast ca. MCF-7 | 0.0 | Heart Pool | 0.0 |
| Breast ca. MDA-MB-231 | 0.0 | Lymph Node Pool | 0.9 |
| Breast ca. BT 549 | 0.0 | Fetal Skeletal Muscle | 0.0 |
| Breast ca. T47D | 0.0 | Skeletal Muscle Pool | 0.4 |
| Breast ca. MDA-N | 0.0 | Spleen Pool | 0.0 |
| Breast Pool | 0.0 | Thymus Pool | 1.0 |
| Trachea | 0.3 | CNS cancer (glio/astro) U87-MG | 0.0 |
| Lun | 0.0 | CNS cancer (glio/astro) U-118-MG | 0.0 |
| Fetal Lung | 0.0 | CNS cancer (neuro;met) SK-N-AS | 1.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.0 | CNS cancer (glio) SNB-19 | 0.0 |
| Lung ca. SHP-77 | 0.5 | CNS cancer (glio) SF-295 | 0.0 |
| Lung ca. A549 | 0.0 | Brain (Amygdala) Pool | 2.4 |
| Lung ca. NCI-H526 | 0.0 | Brain (cerebellum) | 0.0 |
| Lung ca. NCI-H23 | 0.0 | Brain (fetal) | 5.0 |
| Lung ca. NCI-H460 | 0.0 | Brain (Hippocampus) Pool | 3.2 |
| Lung ca. HOP-62 | 0.0 | Cerebral Cortex Pool | 4.6 |
| Lung ca. NCI-H522 | 2.7 | Brain (Substantia nigra) Pool | 9.1 |
| Liver | 0.0 | Brain (Thalamus) Pool | 9.3 |
| Fetal Liver | 0.0 | Brain (whole) | 17.7 |
| Liver ca. HepG2 | 0.0 | Spinal Cord Pool | 2.0 |
| Kidney Pool | 0.0 | Adrenal Gland | 1.9 |
| Fetal Kidney | 0.0 | Pituitary gland Pool | 5.7 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 0.0 |

**General_screening_panel_v1.5 Summary:** Ag855 Highest expression of the CG 110242-01 gene is seen in the bladder (CT=31). Thus, expression of this gene could be used to differentiate this sample from other samples on this panel and as a marker of bladder tissue. In addition, low but significant levels of expression are also seen in testis, thalamus, substantia nigra, and whole brain samples. Thus, therapeutic modulation of the expression or function of this gene may be useful in the treatment of neurologic disorders, such as Alzheimer's disease, Parkinson's disease, schizophrenia, multiple sclerosis, stroke and epilepsy.

### AK. CG99598-01: Endosomal Glycoprotein

Expression of gene CG99598-01 was assessed using the primer-probe sets Ag4149 and Ag4806, described in Tables AKA and AKB. Results of the RTQ-PCR runs are shown in Table AKC.

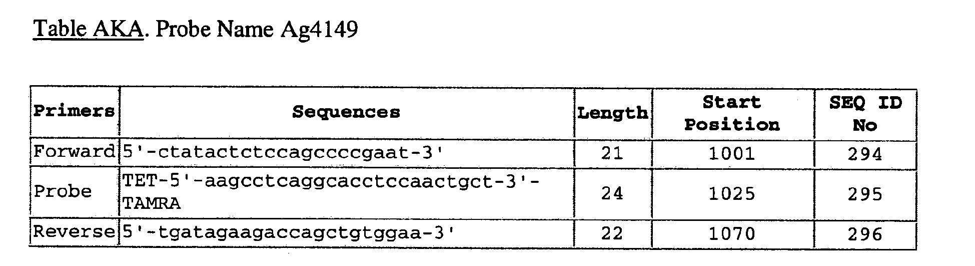

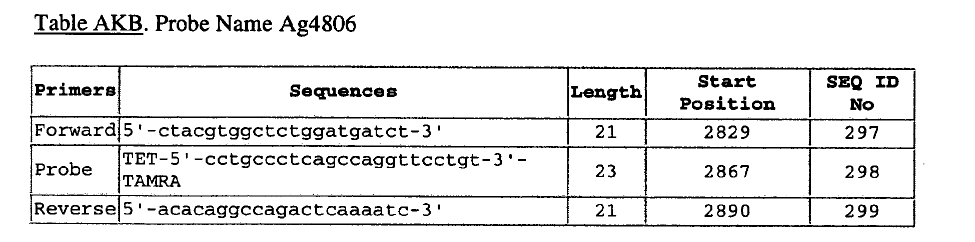

**Table AKC. General_screening_panel_v1.4**

| **Tissue Name** | **Rel. Exp.(%) Ag4806, Run 223204110** | **Tissue Name** | **Rel. Exp.(%) Ag4806, Run 223204110** |
|---|---|---|---|
| Adipose | 7.6 | Renal ca. TK-10 | 4.4 |
| Melanoma* Hs688(A).T | 4.6 | Bladder | 13.7 |
| Melanoma* Hs688(B).T | 12.7 | Gastric ca. (liver met.) NCI-N87 | 6.6 |
| Melanoma* M14 | 27.7 | Gastric ca. KATO III | 0.0 |
| Melanoma* LOXIMVI | 0.0 | Colon ca. SW-948 | 7.7 |
| Melanoma* SK-MEL-5 | 0.0 | Colon ca. SW480 | 9.6 |
| Squamous cell carcinoma SCC-4 | 0.0 | Colon ca.* (SW480 met) SW620 | 7.2 |
| Testis Pool | 0.0 | Colon ca. HT29 | 15.0 |
| Prostate ca.* (bone met) PC-3 | 0.0 | Colon ca. HCT-116 | 15.2 |
| Prostate Pool | 3.6 | Colon ca. CaCo-2 | 12.5 |
| Placenta | 4.7 | Colon cancer tissue | 15.8 |
| Uterus Pool | 0.0 | Colon ca. SW1116 | 16.8 |
| Ovarian ca. OVCAR-3 | 0.3 | Colon ca. Colo-205 | 13.9 |
| Ovarian ca. SK-OV-3 | 19.9 | Colon ca. SW-48 | 0.0 |
| Ovarian ca. OVCAR-4 | 0.0 | Colon Pool | 0.0 |
| Ovarian ca. OVCAR-5 | 14.2 | Small Intestine Pool | 0.0 |
| Ovarian ca. IGROV-1 | 24.1 | Stomach Pool | 1.3 |
| Ovarian ca. OVCAR-8 | 23.2 | Bone Marrow Pool | 0.0 |
| Ovary | 0.7 | Fetal Heart | 17.0 |
| Breast ca. MCF-7 | 6.7 | Heart Pool | 4.2 |
| Breast ca. MDA-MB-231 | 65.1 | Lymph Node Pool | 3.5 |
| Breast ca. BT 549 | 18.8 | Fetal Skeletal Muscle | 6.5 |
| Breast ca. T47D | **100.0** | Skeletal Muscle Pool | 11.8 |
| Breast ca. MDA-N | 8.7 | Spleen Pool | 18.7 |
| Breast Pool | 1.9 | Thymus Pool | 12.9 |
| Trachea | 0.0 | CNS cancer (glio/astro) U87-MG | 25.9 |
| Lung | 0.0 | CNS cancer (glio/astro) U-118-MG | 37.9 |
| Fetal Lung | 19.9 | CNS cancer (neuro;met) SK-N-AS | 9.3 |
| Lung ca. NCI-N417 | 0.0 | CNS cancer (astro) SF-539 | 3.2 |
| Lung ca. LX-1 | 11.0 | CNS cancer (astro) SNB-75 | 33.4 |
| Lung ca. NCI-H146 | 0.0 | CNS cancer (glio) SNB-19 | 11.0 |
| Lung ca. SHP-77 | 11.4 | CNS cancer (glio) SF-295 | 9.8 |
| Lung ca. A549 | 33.7 | Brain (Amygdala) Pool | 9.0 |
| Lung ca. NCI-H526 | 0.0 | Brain (cerebellum) | 22.4 |
| Lung ca. NCI-H23 | 7.0 | Brain (fetal) | 14.4 |
| Lung ca. NCI-H460 | 9.7 | Brain (Hippocampus) Pool | 8.4 |
| Lung ca. HOP-62 | 7.9 | Cerebral Cortex Pool | 0.0 |
| Lung ca. NCI-H522 | 0.0 | Brain (Substantia nigra) | 18.9 |
| Liver | 40.1 | Brain (Thalamus) Pool | 8.2 |
| Fetal Liver | 5.1 | Brain (whole) | 3.5 |
| Liver ca. HepG2 | 16.6 | Spinal Cord Pool | 7.8 |
| Kidney Pool | 11.5 | Adrenal Gland | 4.0 |
| Fetal Kidney | 16.7 | Pituitary gland Pool | 13.9 |
| Renal ca. 786-0 | 25.7 | Salivary Gland | 0.8 |
| Renal ca. A498 | 0.0 | Thyroid (female) | 1.8 |
| Renal ca. ACHN | 0.0 | Pancreatic ca. CAPAN2 | 0.5 |
| Renal ca. UO-31 | 8.8 | Pancreas Pool | 11.7 |

**General_screening_panel_v1.4 Summary:** Ag4806 Expression of this gene is highest in a breast cancer cell line (CT=31.5). This gene is also expressed in breast, ovarian and colon cancer cell lines at higher levels when compared to normal tissue samples. Hence, expression of this gene might be used as a marker to identify normal tissue from cancerous tissue in these organs.

There is relatively low level of expression in most endocrine (metabolic)-related tissues except for liver. Modulation of this gene or gene-product may therefore be beneficial in treating various abnormalities related to liver function. The higher levels of expression in adult liver (CT=32.7) when compared to fetal liver suggest that expression of this gene can also be used to differentiate fetal vs adult liver tissue. Conversely, higher levels of expression in fetal lung (CT=33) when compared to adult lung (CT=40) suggest involvement of this gene in the development of the lung. Expression of this gene could also therefore be used to differentiate between fetal and adult lung tissue.

### Example D: Identification of Single Nucleotide Polymorphisms in NOVX nucleic acid sequences

Variant sequences are also included in this application. A variant sequence can include a single nucleotide polymorphism (SNP). A SNP can, in some instances, be referred to as a "cSNP" to denote that the nucleotide sequence containing the SNP originates as a cDNA. A SNP can arise in several ways. For example, a SNP may be due to a substitution of one nucleotide for another at the polymorphic site. Such a substitution can be either a transition or a transversion. A SNP can also arise from a deletion of a nucleotide or an insertion of a nucleotide, relative to a reference allele. In this case, the polymorphic site is a site at which one allele bears a gap with respect to a particular nucleotide in another allele. SNPs occurring within genes may result in an alteration of the amino acid encoded by the gene at the position of the SNP. Intragenic SNPs may also be silent, when a codon including a SNP encodes the same amino acid as a result of the redundancy of the genetic code. SNPs occurring outside the region of a gene, or in an intron within a gene, do not result in changes in any amino acid sequence of a protein but may result in altered regulation of the expression pattern. Examples include alteration in temporal expression, physiological response regulation, cell type expression regulation, intensity of expression, and stability of transcribed message.

SeqCalling assemblies produced by the exon linking process were selected and extended using the following criteria. Genomic clones having regions with 98% identity to all or part of the initial or extended sequence were identified by BLASTN searches using the relevant sequence to query human genomic databases. The genomic clones that resulted were selected for further analysis because this identity indicates that these clones contain the genomic locus for these SeqCalling assemblies. These sequences were analyzed for putative coding regions as well as for similarity to the known DNA and protein sequences. Programs used for these analyses include Grail, Genscan, BLAST, HMMER, FASTA, Hybrid and other relevant programs.

Some additional genomic regions may have also been identified because selected SeqCalling assemblies map to those regions. Such SeqCalling sequences may have overlapped with regions defined by homology or exon prediction. They may also be included because the location of the fragment was in the vicinity of genomic regions identified by similarity or exon prediction that had been included in the original predicted sequence. The sequence so identified was manually assembled and then may have been extended using one or more additional sequences taken from CuraGen Corporation's human SeqCalling database. SeqCalling fragments suitable for inclusion were identified by the CuraTools™ program SeqExtend or by identifying SeqCalling fragments mapping to the appropriate regions of the genomic clones analyzed.

The regions defined by the procedures described above were then manually integrated and corrected for apparent inconsistencies that may have arisen, for example, from miscalled bases in the original fragments or from discrepancies between predicted exon junctions, EST locations and regions of sequence similarity, to derive the final sequence disclosed herein. When necessary, the process to identify and analyze SeqCalling assemblies and genomic clones was reiterated to derive the full length sequence (Alderbom et al., Determination of Single Nucleotide Polymorphisms by Real-time Pyrophosphate DNA Sequencing. Genome Research. 10 (8) 1249-1265, 2000).

Variants are reported individually but any combination of all or a select subset of variants are also included as contemplated NOVX embodiments of the invention.

### NOV1a SNP Data

One polymorphic variant of NOV1a has been identified and is shown in Table 42A.

**Table 42A**

| **Variant** | **Nucleotides** | | | **Amino Acids** | | |
|---|---|---|---|---|---|---|
| | Position | Initial | Modified | Position | Initial | Modified |
| 13379178 | 120 | A | G | 30 | Pro | Pro |

### NOV2a SNP Data

One polymorphic variant of NOV2a has been identified and is shown in Table 42B.

**Table 42B**

| **Variant** | **Nucleotides** | | | **Amino Acids** | | |
|---|---|---|---|---|---|---|
| | Position | Initial | Modified | Position | Initial | Modified |
| 13379271 | 2850 | C | T | 909 | Asp | Asp |

### NOV5a SNP Data

One polymorphic variant of NOV5a has been identified and is shown in Table 42C.

**Table 42C**

| **Variant** | **Nucleotides** | | | **Amino Acids** | | |
|---|---|---|---|---|---|---|
| | Position | Initial | Modified | Position | Initial | Modified |
| 13379181 | 1001 | G | C | Silent | N/A | N/A |

### NOV6a SNP Data

Seven polymorphic variants of NOV6a have been identified and are shown in Table 42D.

**Table 42D**

| **Variant** | **Nucleotides** | | | **Amino Acids** | | |
|---|---|---|---|---|---|---|
| | Position | Initial | Modified | Position | Initial | Modified |
| 13379177 | 1599 | C | T | Silent | N/A | N/A |
| 13379176 | 1665 | G | T | Silent | N/A | N/A |
| 13379175 | 1673 | G | A | Silent | N/A | N/A |
| 13379174 | 1732 | G | A | Silent | N/A | N/A |
| 13379173 | 1791 | G | T | Silent | N/A | N/A |
| 13379172 | 1795 | G | A | Silent | N/A | N/A |
| 13379171 | 1876 | T | C | Silent | N/A | N/A |

### NOV9a SNP Data

Two polymorphic variants of NOV9a have been identified and are shown in Table 42E.

**Table 42E**

| **Variant** | **Nucleotides** | | | **Amino Acids** | | |
|---|---|---|---|---|---|---|
| | Position | Initial | Modified | Position | Initial | Modified |
| 13379179 | 191 | A | G | 59 | Thr | Thr |
| 13379180 | 360 | G | C | 116 | Val | Leu |

### NOV9b SNP Data

Three polymorphic variants of NOV9b have been identified and are shown in Table 42F.

**Table 42F**

| **Variant No.** | **Nucleotides** | | | **Amino Acids** | | |
|---|---|---|---|---|---|---|
| | Base Position of SNP | Wild-type | Variant | Base Position of SNP | Wild-type | Variant |
| 13379251 | 971 | A | G | 315 | Ala | Ala |
| 13379250 | 1226 | C | T | 400 | Gly | Gly |
| 13379249 | 1712 | C | T | 562 | Tyr | Tyr |

### NOV10a SNP Data

Eight polymorphic variants of NOV10a have been identified and are shown in Table 42G.

**Table 42G**

| **Variant** | **Nucleotides** | | | **Amino Acids** | | |
|---|---|---|---|---|---|---|
| | Position | Initial | Modified | Position | Initial | Modified |
| 13376858 | 229 | A | G | 74 | Glu | Gly |
| 13376859 | 258 | G | A | 84 | Ala | Thr |
| 13376860 | 286 | A | G | 93 | Gln | Arg |
| 13376861 | 296 | T | C | 96 | Ala | Ala |
| 13376862 | 305 | A | G | 99 | Gln | Gln |
| 13376863 | 312 | A | G | 102 | Thr | Ala |
| 13376864 | 348 | A | G | 114 | Arg | Gly |
| 13376866 | 404 | G | A | 132 | Met | Ile |

### NOV11a SNP Data

Two polymorphic variants of NOV11a have been identified and are shown in Table 42H.

**Table 42H**

| **Variant** | **Nucleotides** | | | **Amino Acids** | | |
|---|---|---|---|---|---|---|
| | Position | Initial | Modified | Position | Initial | Modified |
| 13379186 | 346 | T | C | 96 | Ser | Ser |
| 13379187 | 539 | A | G | 161 | Ile | Val |

### NOV15a SNP Data

Three polymorphic variants of NOV15a have been identified and are shown in Table 42I.

**Table 42I**

| **Variant** | **Nucleotides** | | | **Amino Acids** | | |
|---|---|---|---|---|---|---|
| | Position | Initial | Modified | Position | Initial | Modified |
| 13377489 | 243 | T | C | 71 | Tyr | Tyr |
| 13377488 | 348 | C | T | 106 | Ile | Ile |
| 13377487 | 723 | T | G | 231 | Thr | Thr |

### NOV16a SNP Data

One polymorphic variant of NOV16a has been identified and is shown in Table 42J.

**Table 42J**

| **Variant** | **Nucleotides** | | | **Amino Acids** | | |
|---|---|---|---|---|---|---|
| | Position | Initial | Modified | Position | Initial | Modified |
| 13379185 | 1258 | A | G | 415 | Ser | Ser |

### NOV17a SNP Data

One polymorphic variant of NOV17a has been identified and is shown in Table 42K.

**Table 42K**

| **Variant** | **Nucleotides** | | | **Amino Acids** | | |
|---|---|---|---|---|---|---|
| | Position | Initial | Modified | Position | Initial | Modified |
| 13379184 | 229 | A | G | 76 | Met | Val |

### NOV19a SNP Data

Two polymorphic variants of NOV19a have been identified and are shown in Table 42L.

**Table 42L**

| **Variant** | **Nucleotides** | | | **Amino Acids** | | |
|---|---|---|---|---|---|---|
| | Position | Initial | Modified | Position | Initial | Modified |
| 13379188 | 3620 | T | C | 1187 | Val | Ala |
| 13379189 | 3725 | G | A | Silent | N/A | N/A |

### NOV27a SNP Data

One polymorphic variant of NOV27a has been identified and is shown in Table 42M.

**Table 42M**

| **Variant** | **Nucleotides** | | | **Amino Acids** | | |
|---|---|---|---|---|---|---|
| | Position | Initial | Modified | Position | Initial | Modified |
| 13379191 | 2068 | T | C | 364 | Pro | Pro |

### NOV29a SNP Data

One polymorphic variant of NOV29a has been identified and is shown in Table 42N.

**Table 42N**

| **Variant** | **Nucleotides** | | | **Amino Acids** | | |
|---|---|---|---|---|---|---|
| | Position | Initial | Modified | Position | Initial | Modified |
| 13379190 | 113 | C | T | 25 | Pro | Leu |

### NOV31a SNP Data

One polymorphic variant of NOV31a has been identified and is shown in Table 420.

**Table 42O**

| **Variant** | **Nucleotides** | | | **Amino Acids** | | |
|---|---|---|---|---|---|---|
| | Position | Initial | Modified | Position | Initial | Modified |
| 13379285 | 923 | C | T | 307 | Phe | Phe |

### NOV35a SNP Data

One polymorphic variant of NOV35a has been identified and is shown in Table 42P.

**Table 42P**

| **Variant** | **Nucleotides** | | | **Amino Acids** | | |
|---|---|---|---|---|---|---|
| | Position | Initial | Modified | Position | Initial | Modified |
| 13378787 | 1271 | C | T | 424 | Ala | Val |

### NOV39a SNP Data

Five polymorphic variants of NOV39a have been identified and are shown in Table 42Q.

**Table 42Q**

| **Variant** | **Nucleotides** | | | **Amino Acids** | | |
|---|---|---|---|---|---|---|
| | Position | Initial | Modified | Position | Initial | Modified |
| 13379193 | 655 | T | C | 191 | Tyr | His |
| 13379196 | 861 | A | G | 259 | Lys | Lys |
| 13379195 | 871 | A | G | 263 | Lys | Glu |
| 13374732 | 1425 | T | C | 447 | Asp | Asp |
| 13379194 | 1960 | T | A | 626 | Leu | Ile |

### Example E: Potential Role(s) of CG102615-01 in Tumorgenesis

The NOV13a gene (CG102615-01) is known to mediate chloride flow, affecting the membrane potential of the cell. Changes in membrane potential can affect tumor cell and associated smooth muscle cells (therefore tumor-induced vasculature) growth and motility. In this respect, the strong expression in fetal muscle is an indication of a role for NOV13a in muscle growth/development.

Therapeutic targeting of NOV13a with a human monoclonal antibody is anticipated to limit or block the extent of tumor cell growth and motility and tumor associated angiogenesis, preferably in breast, ovarian bladder, lung tumors.

SAGE data is present for NOV13a in Table 43.

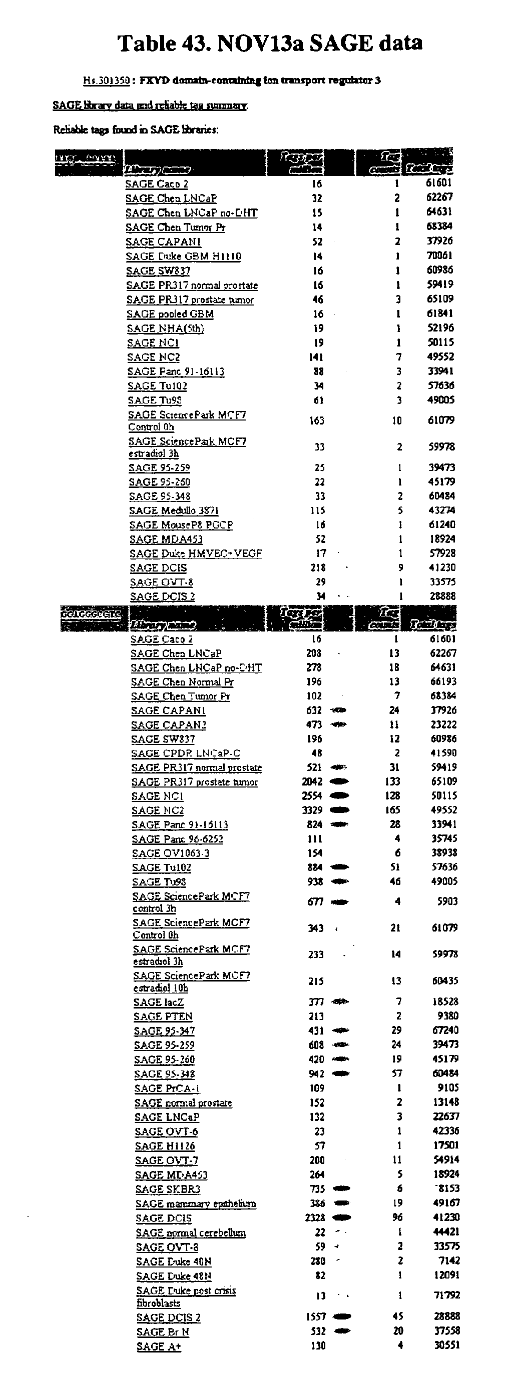

### OTHER EMBODIMENTS

Although particular embodiments have been disclosed herein in detail, this has been done by way of example for purposes of illustration only, and is not intended to be limiting with respect to the scope of the appended claims, which follow. In particular, it is contemplated by the inventors that various substitutions, alterations, and modifications may be made to the invention without departing from the spirit and scope of the invention as defined by the claims. The choice of nucleic acid starting material, clone of interest, or library type is believed to be a matter of routine for a person of ordinary skill in the art with knowledge of the embodiments described herein. The claims presented are representative of the inventions disclosed herein. Other, unclaimed inventions are also contemplated. Applicants reserve the right to pursue such inventions in later claims.

## Claims

1. An isolated polypeptide comprising the mature form of an amino acid sequence selected from the group consisting of SEQ ID NO:74, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO; 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, or SEQ ID NO:146.

2. An isolated polypeptide comprising an amino acid sequence selected from the group consisting of SEQ ID NO:74, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO; 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, or SEQ ID NO:146.

3. An isolated polypeptide comprising an amino acid sequence which is at least 95% identical to an amino acid sequence selected from the group consisting of SEQ ID NO:74, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO; 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID N0:102, SEQ ID NO:104, SEQ ID NO:106, SEQ ID NO:108, SEQ ID NO:110, SEQ ID NO:112, SEQ ID NO:114, SEQ ID NO:116, SEQ 10 NO:118, SEQ 10 NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, or SEQ NO:146.

4. An isolated polypeptide, wherein the polypeptide comprises an amino acid sequence comprising one or more conservative substitutions in the amino acid sequence selected from the group consisting of SEQ ID NO:74, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO; 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, or SEQ ID NO:146.

5. The polypeptide of claim 1 wherein said polypeptide is naturally occurring.

6. A composition comprising the polypeptide of claim 1 and a carrier.

7. A kit comprising, in one or more containers, the composition of claim 6.

8. The use of a therapeutic in the manufacture of a medicament for treating a syndrome associated with a human disease, the disease selected from a pathology associated with the polypeptide of claim 1, wherein the therapeutic comprises the polypeptide of claim 1.

9. A method for determining the presence or amount of the polypeptide of claim 1 in a sample, the method comprising:
(a) providing said sample;
(b) introducing said sample to an antibody that binds immunospecifically to the polypeptide; and
(3) determining the presence or amount of antibody bound to said polypeptide,
thereby determining the presence or amount of polypeptide in said sample.

10. A method for determining the presence of or predisposition to a disease associated with altered levels of expression of the polypeptide of claim 1 in a first mammalian subject, the method comprising:
a) measuring the level of expression of the polypeptide in a sample from the first mammalian subject; and
b) comparing the expression of said polypeptide in the sample of step (a) to the expression of the polypeptide present in a control sample from a second mammalian subject known not to have, or not to be predisposed to, said disease,
wherein an alteration in the level of expression of the polypeptide in the first subject as compared to the control sample indicates the presence of or predisposition to said disease.

11. A method of identifying an agent that binds to the polypeptide of claim 1, the method comprising:
(a) introducing said polypeptide to said agent; and
(b) determining whether said agent binds to said polypeptide.

12. The method of claim 11 wherein the agent is a cellular receptor or a downstream effector.

13. A method for identifying a potential therapeutic agent for use in treatment of a pathology, wherein the pathology is related to aberrant expression or aberrant physiological interactions of the polypeptide of claim 1, the method comprising:
(a) providing a cell expressing the polypeptide of claim 1 and having a property or function ascribable to the polypeptide;
(b) contacting the cell with a composition comprising a candidate substance; and
(c) determining whether the substance alters the property or function ascribable to the polypeptide;
whereby, if an alteration observed in the presence of the substance is not observed when the cell is contacted with a composition in the absence of the substance, the substance is identified as a potential therapeutic agent.

14. A method for screening for a modulator of activity of or of latency or predisposition to a pathology associated with the polypeptide of claim 1, said method comprising:
(a) administering a test compound to a test animal at increased risk for a pathology associated with the polypeptide of claim 1, wherein said test animal recombinantly expresses the polypeptide of claim 1;
(b) measuring the activity of said polypeptide in said test animal after administering the compound of step (a); and
(c) comparing the activity of said polypeptide in said test animal with the activity of said polypeptide in a control animal not administered said polypeptide, wherein a change in the activity of said polypeptide in said test animal relative to said control animal indicates the test compound is a modulator activity of or latency or predisposition to, a pathology associated with the polypeptide of claim 1.

15. The method of claim 14, wherein said test animal is a recombinant test animal that expresses a test protein transgene or expresses said transgene under the control of a promoter at an increased level relative to a wild-type test animal, and wherein said promoter is not the native gene promoter of said transgene.

16. A method for modulating the activity of the polypeptide of claim 1, the method comprising contacting a cell sample expressing the polypeptide of claim 1 with a compound that binds to said polypeptide in an amount sufficient to modulate the activity of the polypeptide.

17. The polypeptide of claim 1 for use in a method of treating or preventing a pathology associated with the polypeptide of claim 1 in a subject.

18. The use of claim 17, wherein the subject is a human.

19. A polypeptide having an amino acid sequence at least 95% identical to a polypeptide comprising the amino acid sequence selected from the group consisting of SEQ ID NO:74, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO; 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, SEQ ID NO:146 or a biologically active fragment thereof, for use in a method of treating a pathological state in a mammal.

20. An isolated nucleic acid molecule comprising a nucleic acid sequence selected from the group consisting of SEQ ID NO:73, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129; SEQ ID NO:131, SEQ ID NO:133, SEQ ID NO:135, SEQ ID NO:137, SEQ ID NO:139, SEQ ID NO:141, SEQ ID NO:143 or SEQ ID NO:145.

21. The nucleic acid molecule of claim 20, wherein the nucleic acid molecule is naturally occurring.

22. A nucleic acid molecule, wherein the nucleic acid molecule differs by a single nucleotide from a nucleic acid sequence selected from the group consisting of SEQ ID NO:73, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:75, SEQ ID NO:77, SEQ ID NO:79, SEQ ID N0:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129; SEQ ID NO:131, SEQ ID NO:133, SEQ ID NO:135, SEQ ID NO:137, SEQ ID NO:139, SEQ ID NO:141, SEQ ID NO:143 or SEQ ID NO:145.

23. An isolated nucleic acid molecule encoding the mature form of a polypeptide having an amino acid sequence selected from the group consisting of SEQ ID NO:73, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129; SEQ ID NO:131, SEQ ID NO:133, SEQ ID NO:135, SEQ ID NO:137, SEQ ID NO:139, SEQ ID NO:141, SEQ ID NO:143 or SEQ ID NO:145.

24. An isolated nucleic acid molecule comprising a nucleic acid selected from the group consisting of SEQ ID NO:73, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ 10 N0:61, SEQ ID NO:63, SEQ ID NO:65, SEQ ID NO:67, SEQ ID NO:69, SEQ ID NO:71, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129; SEQ ID NO:131, SEQ ID NO:133, SEQ ID NO:135, SEQ ID NO:137, SEQ ID NO:139, SEQ ID NO:141, SEQ ID NO:143 or SEQ ID NO:145.

25. The nucleic acid molecule of claim 20, wherein said nucleic acid molecule hybridizes under stringent conditions to the nucleotide sequence selected from the group consisting of SEQ ID NO: 73, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129; SEQ ID NO:131, SEQ ID NO:133, SEQ ID NO:135, SEQ ID NO:137, SEQ ID NO:139, SEQ ID NO:141, SEQ ID NO:143 or SEQ ID NO:145, or a complement of said nucleotide sequence.

26. A vector comprising the nucleic acid molecule of claim 20.

27. The vector of claim 26, further comprising a promoter operably linked to said nucleic acid molecule.

28. A cell comprising the vector of claim 26.

29. An antibody that immunospecifically binds to the polypeptide of claim 1.

30. The antibody of claim 29, wherein the antibody is a monoclonal antibody.

31. The antibody of claim 29, wherein the antibody is a humanized antibody.

32. The antibody of claim 29, wherein the antibody is a fully human antibody.

33. The antibody of claim 29, wherein the dissociation constant for the binding of the polypeptide to the antibody is less than 1 x 10⁻⁹ M.

34. The antibody of claim 29, wherein the antibody neutralizes an activity of the polypeptide.

35. The antibody of claim 29 for use in a method of treating or preventing a NOVX-associated disorder in a subject.

36. The use of claim 35, wherein the subject is human.

37. A method for determining the presence or amount of the nucleic acid molecule of claim 20 in a sample, the method comprising:
(a) providing said sample;
(b) introducing said sample to a probe that binds to said nucleic acid molecule; and
(c) determining the presence or amount of said probe bound to said nucleic acid molecule,
thereby determining the presence or amount of the nucleic acid molecule in said sample.

38. The method of claim 37 wherein presence or amount of the nucleic acid molecule is used as a marker for cell or tissue type.

39. The method of claim 38 wherein the cell or tissue type is cancerous.

40. A method for determining the presence of or predisposition to a disease associated with altered levels of expression of the nucleic acid molecule of claim 20 in a first mammalian subject, the method comprising:
a) measuring the level of expression of the nucleic acid in a sample from the first mammalian subject; and
b) comparing the level of expression of said nucleic acid in the sample of step (a) to the level of expression of the nucleic acid present in a control sample from a second mammalian subject known not to have or not be predisposed to, the disease;
wherein an alteration in the level of expression of the nucleic acid in the first subject as compared to the control sample indicates the presence of or predisposition to the disease.

41. A method of producing the polypeptide of claim 1, the method comprising culturing a cell under conditions that lead to expression of the polypeptide, wherein said cell comprises a vector comprising an isolated nucleic acid molecule comprising a nucleic acid sequence selected from the group consisting of SEQ ID NO:73, SEQ ID NO:1, SEQ ID NO:3, SEQ ID NO:5, SEQ ID NO:7, SEQ ID NO:9, SEQ ID N0:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129; SEQ ID NO:131, SEQ ID NO:133, SEQ ID NO:135, SEQ ID NO:137, SEQ ID NO:139, SEQ ID NO:141, SEQ ID NO:143 or SEQ ID NO:145.

42. The method of claim 41 wherein the cell is a bacterial cell.

43. The method of claim 41 wherein the cell is an insect cell.

44. The method of claim 41 wherein the cell is a yeast cell.

45. The method of claim 41 wherein the cell is a mammalian cell.

46. A method of producing the polypeptide of claim 2, the method comprising culturing a cell under conditions that lead to expression of the polypeptide, wherein said cell comprises a vector comprising an isolated nucleic acid molecule comprising a nucleic acid sequence selected from the group consisting of SEQ ID NO:73, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129; SEQ ID NO:131, SEQ ID NO:133, SEQ ID NO:135, SEQ ID NO:137, SEQ ID NO:139, SEQ ID NO:141, SEQ ID NO:143 or SEQ ID NO:145.

47. The method of claim 46 wherein the cell is a bacterial cell.

48. The method of claim 46 wherein the cell is an insect cell.

49. The method of claim 46 wherein the cell is a yeast cell.

50. The method of claim 46 wherein the cell is a mammalian cell.

51. A method of treating or preventing a pathology associated with the polypeptide of claim 1, the method comprising administering the polypeptide of claim 1 to a subject in which such treatment or prevention is desired in an amount sufficient to treat or prevent the pathology in the subject.

52. The method of claim 51, wherein the subject is a human.

53. A method of treating a pathological state in a mammal, the method comprising administering to the mammal a polypeptide in an amount that is sufficient to alleviate the pathological state, wherein the polypeptide is a polypeptide having an amino acid sequence at least 95% identical to a polypeptide comprising the amino acid sequence selected from the group consisting of SEQ ID NO:2n, wherein n is an integer between 1 and 73 or a biologically active fragment thereof.

54. A method of treating or preventing a NOVX-associated disorder, the method comprising administering to a subject in which such treatment or prevention is desired the antibody of claim 29 in an amount sufficient to treat or prevent the pathology in the subject.

55. The method of claim 54, wherein the subject is human.
